# EUROPEAN PATENT APPLICATION

(11) **EP 4 768 030 A2**
(43) Date of publication of application: **01.07.2026**
(21) Application number: 26162054.6
(22) Date of filing: 16.08.2024
(51) Int. Cl.: A61K 31/519

(54) **SPIROCYCLIC DIHYDROPYRANOPYRIMIDINE KRAS INHIBITORS**

(30) Priority: 17.08.2023 US 202363533354 P; 28.08.2023 US 202363535014 P; 03.10.2023 US 202363542178 P; 24.10.2023 US 202363545535 P; 22.12.2023 US 202363614248 P; 29.02.2024 US 202463559553 P; 19.03.2024 US 202463567306 P; 01.04.2024 US 202463572733 P; 21.05.2024 US 202463650285 P; 29.05.2024 US 202463653025 P; 25.07.2024 US 202463675568 P
(62) Divisional of application: 24768437.6
(71) Applicant: Treeline Biosciences, Inc., Watertown, Massachusetts 02472 (US)
(72) Inventor: CABRAL, Shawn, Watertown, MA, 02472 (US); MASCITTI, Vincent, Watertown, MA, 02472 (US); DECULTOT, Ludovic Jacky Gilbert, Watertown, MA, 02472 (US); HEROLD, Jens-Martin, Watertown, MA, 02472 (US); HESP, Kevin D., Watertown, MA, 02472 (US); LAJINESS, James Paul, San Diego, CA, 92121 (US); SEIM, Alexander E., Watertown, MA, 02472 (US); TOFOLEANU, Florentina, Watertown, MA, 02472 (US); TSAI, Andy, Watertown, MA, 02472 (US)
(74) Representative: Fish & Richardson P.C.

(57) **Abstract**

Provided herein are compounds of Formula **(II)** (e.g., Formula **(II-a), (II-b),** (**II-a1**), (**II-b1**), **(II-a2), (II-b2),** (**II-3**), **(II-a3),** (**II-4**), **(II-a4),** (**II-5**), **(II-a5),** (**II-6**), **(II-a6),** (**II-7**), (**II-a7**), **(II-7),** or **(II-a8)),** Formula **(III)** (e.g., Formula (**III-1**), (**III-2**), **(III-3),** (**III-4**), (**III-5**), (**III-6**), (**III-7**), or **(III-8)),** Formula **(IV)** (e.g., Formula **(IV-a), (IV-b), (IV-c), (IV-a1), (IV-b1**), **(IV-a2), (IV-b2), (IV-a3), (IV-b3), (IV-a4), (IV-b4), (IV-a5), (IV-b5), (IV-a6), (IV-b6), (IV-a7), (IV-b7), (IV-a8), or (IV-b8)),** Formula **(V)** (e.g., Formula **(V-a)** or **(V-b), (V-a1),** (V-**c), (V-d), (V-b1), (V-a2), (V-b2), (V-a3),** or **(V-b3)),** Formula **(VI)** (e.g., Formula **(VI-a), (VI-b), (VI-c), (VI-d),** or **(VI-e))),** or Formula **(A)** (e.g., Formula (**I-a1**)), or pharmaceutically acceptable salts thereof, that inhibit a KRas protein (e.g., a dysregulated KRas protein (e.g., a mutated or amplified KRas protein)). This disclosure also provides compositions containing the compounds as provided herein, or pharmaceutically acceptable salts thereof, as well as methods of using and making the same.

## Description

### CROSS-REFERENCE TO RELATED APPLICATIONS

This application claims priority to U.S. Provisional Application Serial Nos. 63/675,568, filed July 25, 2024; 63/653,025, filed May 29, 2024; 63/650,285, filed May 21, 2024; 63/572,733, filed April 1, 2024; 63/567,306, filed March 19, 2024; 63/559,553, filed February 29, 2024; 63/614,248, filed December 22, 2023; 63/545,535, filed October 24, 2023; 63/542,178, filed October 3, 2023; 63/535,014, filed August 28, 2023; and 63/533,354, filed August 17, 2023, each of which is incorporated by reference it its entirety herein.

### DESCRIPTION OF THE TEXT FILE SUBMITTED ELECTRONICALLY

This application contains a Sequence Listing which has been submitted electronically in XML format. The Sequence Listing XML is incorporated herein by reference. The XML file, created on August 13, 2024, is named TRLN-008-011WO1_ST26_SL.xml and is 2,079 bytes in size.

### TECHNICAL FIELD

This disclosure provides compounds of Formula **(II)** (e.g., Formula **(II-a),** (**II-b**), **(II-a1**), **(II-b1), (II-a2),** (**II-b2**), **(11-3),** (**II-a3**), **(11-4), (II-a4), (11-5), (II-a5),** (**II-6**), **(II-a6), (II-7), (II-a7), (11-7),** or (**II-a8**)), Formula (**III**) (e.g., Formula (**III-1**), (**III-2**), **(111-3),** (**III-4**), **(III-5),** (**III-6**), (**III-7**), or (**III-8**)), Formula **(IV)** (e.g., Formula **(IV-a), (IV-b), (IV-c),** (**IV-a1**), **(IV-b1), (IV-a2), (IV-b2), (IV-a3), (IV-b3), (IV-a4), (IV-b4), (IV-a5), (IV-b5), (IV-a6), (IV-b6), (IV-a7), (IV-b7), (IV-a8),** or **(IV-b8)),** Formula **(V)** (e.g., Formula **(V-a)** or **(V-b), (V-a1**), **(V-c), (V-d), (V-b1), (V-a2), (V-b2), (V-a3),** or **(V-b3)),** Formula **(VI)** (e.g., Formula **(VI-a), (VI-b), (VI-c), (VI-d),** or (**VI-e**))), or Formula **(A)** (e.g., Formula (**I-a1**)), or pharmaceutically acceptable salts thereof, that inhibit a KRas GTPase (e.g., a KRas GTPase that has a dysregulation (referred to herein as a dysregulated KRas protein)). In some embodiments, the KRas protein is a dysregulated KRas protein that has a mutation (referred to herein as a mutant KRas protein). These compounds are useful, for example, for treating a disease, disorder, or condition in which increased and/or sustained (e.g., excessive) KRas activation, such as KRas activation associated with a mutant KRas protein, contributes to the pathology and/or symptoms and/or progression of the disease, disorder, or condition (e.g., cancer) in a subject (e.g., a human). This disclosure also provides compositions containing compounds of Formula **(II)** (e.g., Formula (**II-a**), **(II-b),** (**II-a1**), (**II-b1**), (**II-a2**), (**II-b2**), (**II-3),** (**II-a3**), (**II-4**), (**II-a4**), **(11-5), (II-a5),** (**II-6**), **(II-a6),** (**II-7**), (**II-a7**), **(11-7),** or **(II-a8)),** Formula **(III)** (e.g., Formula (**III-1**), (**III-2**), (**III-3**), (**III-4**), (**III-5**), (**III-6**), (**III-7**), or (**III-8)),** Formula **(IV)** (e.g., Formula **(IV-a), (IV-b), (IV-c), (IV-al), (IV-b1), (IV-a2), (IV-b2), (IV-a3), (IV-b3), (IV-a4), (IV-b4), (IV-a5), (IV-b5), (IV-a6), (IV-b6), (IV-a7), (IV-b7), (IV-a8),** or **(IV-b8)),** Formula (V) (e.g., Formula **(V-a)** or **(V-b),** (**V-a1)**, **(V-c), (V-d), (V-b1), (V-a2), (V-b2), (V-a3),** or **(V-b3)),** Formula **(VI)** (e.g., Formula **(VI-a), (VI-b), (VI-c), (VI-d),** or **(VI-e))),** or Formula **(A)** (e.g., Formula (**I-a1**)), or pharmaceutically acceptable salts thereof, as well as methods of using and making the same.

### BACKGROUND

The *KRAS* gene is frequently dysregulated (e.g., mutated or amplified) in various human cancers. Oncogenic mutations in KRas typically occur at hotspots in the protein such as at amino acids positions 12, 13, and 61. In some cases, a mutation can lead to maintenance of KRas activation (GTP-bound state), e.g., due to a deficiency of intrinsic GTPase activity and/or insensitivity for GTPase-activating proteins (GAPs) and consequent increased KRas signaling. Specifically, some of the most common protein mutations include those at position 12 (referred to herein as G12X) such as G12A, G12C, G12D, G12R, G12S, and G12V; position 13 (referred to herein as G13X) such as G13C, G13D, and G13V; and Q61 (referred to herein as Q61X), such as Q61E, Q61H, Q61K, Q61L, Q61P, and Q61R.

KRas is widely recognized as a target for the design and development of therapies that can specifically bind and inhibit KRas signaling in cancer cells but had long been considered to be undruggable. Currently, there are few approved KRas-targeted therapies.

### SUMMARY

This disclosure provides compounds of Formula **(II)** (e.g., Formula **(II-a),** (**II-b**), (**II-a1**), **(II-b1), (II-a2),** (**II-b2**), **(11-3),** (**II-a3**), **(11-4),** (**II-a4**), **(11-5), (II-a5),** (**II-6**), **(II-a6),** (**II-7), (II-a7), (11-7),** or (**II-a8**)), Formula (**III**) (e.g., Formula (**III-1**), (**III-2**), **(111-3),** (**III-4**), (**III-5),** (**III-6**), (**III-7**), or (**III-8**)), Formula **(IV)** (e.g., Formula **(IV-a), (IV-b), (IV-c),** (**IV-a1**), **(IV-b1), (IV-a2), (IV-b2), (IV-a3), (IV-b3), (IV-a4), (IV-b4), (IV-a5), (IV-b5), (IV-a6), (IV-b6), (IV-a7), (IV-b7), (IV-a8),** or **(IV-b8)),** Formula **(V)** (e.g., Formula **(V-a)** or **(V-b), (V-a1**), **(V-c), (V-d),** (**V-b1)**, **(V-a2), (V-b2), (V-a3),** or **(V-b3)),** Formula **(VI)** (e.g., Formula **(VI-a), (VI-b), (VI-c), (VI-d),** or **(VI-e))),** or Formula **(A)** (e.g., Formula (**I-a1**)), or pharmaceutically acceptable salts thereof, that inhibit a KRas protein (e.g., a dysregulated KRas protein, such as a mutant KRas protein). These compounds are useful, for example, for treating a disease, disorder, or condition in which increased KRas activation, such as KRas activation associated with a mutant KRas protein or KRas activation associated with KRas amplification, contributes to the pathology and/or symptoms and/or progression of the disease, disorder, or condition (e.g., cancer) in a subject (e.g., a human). This disclosure also provides compositions containing compounds of Formula **(II)** (e.g., Formula **(II-a), (II-b),** (**II-a1**), (**II-b1**), (**II-a2**), (**II-b2**), **(11-3),** (**II-a3**), (**II-4**), (**II-a4**), **(11-5), (II-a5),** (**II-6**), **(II-a6),** (**II-7**), (**II-a7),** (**II-7**), or **(II-a8)),** Formula **(III)** (e.g., Formula (**III-1**), **(III-2),** (**III-3**), **(III-4), (111-5),** (**III-6**), **(111-7),** or **(III-8)),** Formula **(IV)** (e.g., Formula **(IV-a), (IV-b), (IV-c), (IV-al), (IV-b1), (IV-a2), (IV-b2), (IV-a3), (IV-b3), (IV-a4), (IV-b4), (IV-a5), (IV-b5), (IV-a6), (IV-b6), (IV-a7), (IV-b7), (IV-a8),** or **(IV-b8)),** Formula **(V)** (e.g., Formula **(V-a)** or **(V-b),** (**V-a1**), **(V-c), (V-d), (V-b1), (V-a2), (V-b2), (V-a3),** or **(V-b3)),** Formula **(VI)** (e.g., Formula **(VI-a), (VI-b), (VI-c), (VI-d),** or **(VI-e))),** or Formula **(A)** (e.g., Formula (**I-a1**)), or pharmaceutically acceptable salts thereof, as well as methods of using and making the same.

Provided herein are compounds of Formula **(II):** 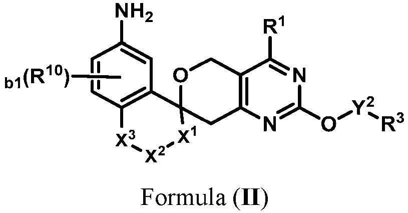 or pharmaceutically acceptable salts thereof, wherein:
**R¹** is selected from the group consisting of:
   **(i)** a 4-10 membered heterocyclyl optionally substituted with 1-4 **R⁷**;
   **(ii)** an 8-12 membered bicyclic heterocyclyl, wherein the heterocyclyl comprises an endocyclic group selected from the group consisting of C(=O)NH and S(O)₂NH, and wherein the heterocyclyl is further optionally substituted with 1-3 **R⁷** at one or more ring carbon atoms; and
   **(iii)** 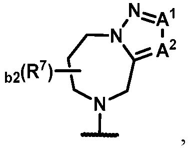 wherein **b2** is 0, 1, 2, or 3; and **A¹** and **A²** are independently selected from the group consisting of: N, CH, and **CR⁷**;
each **R⁷** is independently selected from the group consisting of **R^{a}** and **R^{b}**;
**X¹** is selected from the group consisting of a bond, S(O)₀₋₂, CH₂, CH**R^{L}**, C(**R^{L}**)₂, and O;
**X²** and **X³** are independently selected from the group consisting of: CH₂, CH**R^{L}**, C(**R^{L}**)₂, O, and S(O)₀₋₂, provided that no more than one of **X¹**, **X²**, and **X³** is selected from the group consisting of: O and S(O)₀₋₂;
**b1** is 1 or 2;
each **R¹⁰** is independently selected from the group consisting of **R^{a}** and **R^{b}**;
each **R^{L}** is independently selected from the group consisting of C₁₋₃ alkoxy, -F, -CN, and C₁₋₃ alkyl optionally substituted with 1-3 **R^{c}**; or
one pair of **R^{L}** on the same or different ring carbon atom(s) taken together with the ring atom(s) connecting them form a C₃₋₆ cycloalkyl ring;
**Y²** is a bond or a straight-chain C₁₋₆ alkylene optionally substituted with 1-6 **R^{Y}**;
each **R^{Y}** is independently selected from the group consisting of: halo, cyano, -OH, oxo, C₁₋₆ alkoxy, C₁₋₆ haloalkoxy, C₁₋₆ alkyl, and C₁₋₆ haloalkyl, or
one pair of **R^{Y}** on the same or different carbon atom(s) taken together with the atom(s) connecting them forms a C₃₋₆ cycloalkyl ring or 4-6 membered heterocyclyl ring, each of which is optionally substituted with 1-3 independently selected C₁₋₃ alkyl;
**R³** is selected from the group consisting of:
   **(a)** a 4-15 membered heterocyclyl optionally substituted with 1-6 substituents independently selected from the group consisting of: **R^{a}** and **R^{b}**; and
   **(b)** -N**R^{d}R^{e}**;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   **(f)** -C₁₋₆ haloalkoxy;
   (**g**) -N**R^{d}R^{e}**;
   **(h)** C(=O)C₁₋₆ alkyl;
   **(i)** C(=O)C₁₋₆ haloalkyl;
   **(j)** C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   **(o)** S(O)₀₋₂(C₁₋₆ haloalkyl);
   **(p)** S(O)₁₋₂N(**R^{f}**)₂; and
   **(q)** C₁₋₆ alkyl, C₂₋₆ alkenyl, or C₂₋₆ alkynyl, each optionally substituted with 1-6 **R^{c};**
each **R^{b}** is independently selected from the group consisting of: -(**L^{b}**)**_{b}**-**R^{b1}** and -**R^{b1}**, wherein:
   **b is** 1, 2, or 3;
   each -**L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e}**, C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)₁₋₂N(**R^{f}**)₂;
   each **R^{d}** and **R^{e}** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂-.

Also provided herein are compounds of Formula (III): 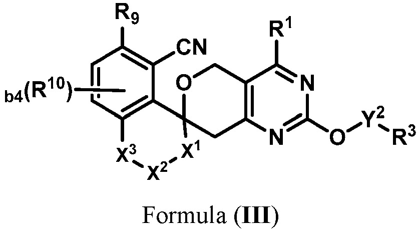 or pharmaceutically acceptable salts thereof, wherein:
**R¹** is selected from the group consisting of:
   (i) a 4-10 membered heterocyclyl optionally substituted with 1-4 **R⁷**;
   (ii) an 8-12 membered bicyclic heterocyclyl, wherein the heterocyclyl comprises an endocyclic group selected from the group consisting of C(=O)NH and S(O)₂NH, and wherein the heterocyclyl is further optionally substituted with 1-3 **R⁷** at one or more ring carbon atoms; and
   **(iii)** 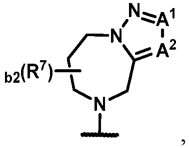 wherein **b2** is 0, 1, 2, or 3; and **A¹** and **A²** are independently selected from the group consisting of: N, CH, and **CR⁷**;
each **R⁷** is independently selected from the group consisting of **R^{a}** and **R^{b}**;
**X¹** is selected from the group consisting of a bond, S(O)₀₋₂, CH₂, CH**R^{L}**, C(**R^{L}**)₂, and O;
**X²** and **X³** are independently selected from the group consisting of: CH₂, CH**R^{L}**, C(**R^{L}**)₂, O, and S(O)₀₋₂, provided that no more than one of **X¹**, **X²**, and **X³** is selected from the group consisting of: O and S(O)₀₋₂;
**R⁹** is selected from the group consisting of: H, N**R^{d}R^{e}**, -OH, and halo;
**b4** is 0 or 1;
**R¹⁰** is selected from the group consisting of **R^{a}** and **R^{b}**;
each **R^{L}** is independently selected from the group consisting of C₁₋₃ alkoxy, -F, CN, and C₁₋₃ alkyl optionally substituted with 1-3 **R^{c}**; or
one pair of **R^{L}** on the same or different ring carbon atom(s) taken together with the ring atom(s) connecting them form a C₃₋₆ cycloalkyl ring;
**Y²** is a bond or a straight-chain C₁₋₆ alkylene optionally substituted with 1-6 **R^{Y}**;
each **R^{Y}** is independently selected from the group consisting of: halo, cyano, -OH, oxo, C₁₋₆ alkoxy, C₁₋₆ haloalkoxy, C₁₋₆ alkyl, and C₁₋₆ haloalkyl, or
one pair of **R^{Y}** on the same or different carbon atom(s) taken together with the atom(s) connecting them forms a C₃₋₆ cycloalkyl ring or 4-6 membered heterocyclyl ring, each of which is optionally substituted with 1-3 independently selected C₁₋₃ alkyl;
**R³** is selected from the group consisting of:
   **(a)** a 4-15 membered heterocyclyl optionally substituted with 1-6 substituents independently selected from the group consisting of: **R^{a}** and **R^{b}**; and
   (**b**) -N**R^{d}R^{e}**;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   **(f)** -C₁₋₆ haloalkoxy;
   **(g)** -N**R^{d}R^{e}**;
   **(h)** C(=O)C₁₋₆ alkyl;
   **(i)** C(=O)C₁₋₆ haloalkyl;
   **(j)** C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   (**o**) S(O)₀₋₂(C₁₋₆ haloalkyl);
   **(p)** S(O)₁₋₂N(**R^{f}**)₂; and
   **(q)** C₁₋₆ alkyl, C₂₋₆ alkenyl, or C₂₋₆ alkynyl, each optionally substituted with 1-6 **R^{c};**
each **R^{b}** is independently selected from the group consisting of: -(**L^{b}**)**_{b}**-**R^{b1}** and -**R^{b1}**, wherein:
   **b is** 1, 2, or 3;
   each -**L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e}**, C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)₁₋₂N(**R^{f}**)₂;
   each **R^{d}** and **R^{c};** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂-.

Also provided herein are compounds of Formula **(IV):** 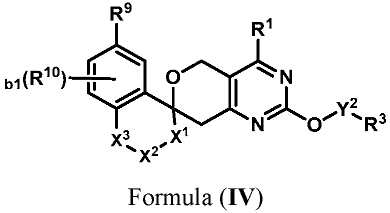 or pharmaceutically acceptable salts thereof, wherein:
**X¹** is selected from the group consisting of a bond, S(O)₀₋₂, CH₂, CH**R^{L}**, C(**R^{L}**)₂, and O;
**X²** and **X³** are independently selected from the group consisting of: CH₂, CH**R^{L}**, C(**R^{L}**)₂, O, and S(O)₀₋₂, provided that at least one of **X¹**, **X²**, and **X³** is CH**R^{L}** or C(**R^{L}**)₂;
further provided that no more than one of **X¹**, **X²**, and **X³** is selected from the group consisting of: O and S(O)₀₋₂;
**b1** is 0, 1 or 2;
**R⁹** is selected from the group consisting of: H, OH, N**R^{d}R^{e}**, and halo;
each **R¹⁰** is independently selected from the group consisting of **R^{a}** and **R^{b}**;
each **R^{L}** is independently selected from the group consisting of C₁₋₃ alkoxy, -F, CN, and C₁₋₃ alkyl optionally substituted with 1-3 **R^{c}**;
**R¹** is selected from the group consisting of:
   **(i)** a 4-10 membered heterocyclyl optionally substituted with 1-4 **R⁷**;
   **(ii)** an 8-12 membered bicyclic heterocyclyl, wherein the heterocyclyl comprises an endocyclic group selected from the group consisting of C(=O)NH and S(O)₂NH, and wherein the heterocyclyl is further optionally substituted with 1-3 **R⁷** at one or more ring carbon atoms; and
   **(iii)** 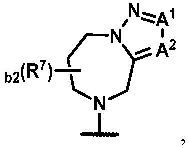 wherein **b2** is 0, 1, 2, or 3; and **A¹** and **A²** are independently selected from the group consisting of: N, CH, and **CR⁷**;
each **R⁷** is independently selected from the group consisting of **R^{a}** and **R^{b}**;
**Y²** is a bond or a straight-chain C₁₋₆ alkylene optionally substituted with 1-6 **R^{Y}**;
each **R^{Y}** is independently selected from the group consisting of: halo, cyano, -OH, oxo, C₁₋₆ alkoxy, C₁₋₆ haloalkoxy, C₁₋₆ alkyl, and C₁₋₆ haloalkyl, or
one pair of **R^{Y}** on the same or different carbon atom(s) taken together with the atom(s) connecting them forms a C₃₋₆ cycloalkyl ring or 4-6 membered heterocyclyl ring, each of which is optionally substituted with 1-3 independently selected C₁₋₃ alkyl;
**R³** is selected from the group consisting of:
   **(a)** a 4-15 membered heterocyclyl optionally substituted with 1-6 substituents independently selected from the group consisting of: **R^{a}** and **R^{b}**; and
   (**b**) -N**R^{d}R^{e}**;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   **(f)** -C₁₋₆ haloalkoxy;
   (**g**) -N**R^{d}R^{e}**;
   **(h)** C(=O)C₁₋₆ alkyl;
   **(i)** C(=O)C₁₋₆ haloalkyl;
   **(j)** C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   (**o**) S(O)₀₋₂(C₁₋₆ haloalkyl);
   (**p)** S(O)₁₋₂N(**R^{f}**)2; and
   **(q)** C₁₋₆ alkyl, C₂₋₆ alkenyl, or C₂₋₆ alkynyl, each optionally substituted with 1-6 **R^{c};**
each **R^{b}** is independently selected from the group consisting of: -(**L^{b}**)**_{b}**-**R^{b1}** and -**R^{b1}**, wherein:
   **b** is 1, 2, or 3;
   each -**L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e}**, C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)₁₋₂N(**R^{f}**)₂;
   each **R^{d}** and **R^{e}** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂-.

Also provided herein are pharmaceutical compositions comprising a compound of Formula (**II**) (e.g., Formula (**II-a**), **(II-b),** (**II-a1**), **(II-b1), (II-a2), (II-b2), (11-3),** (**II-a3**), (**II-4), (II-a4),** (**II-5**), (**II-a5**), (**II-6**), **(II-a6),** (**II-7**), (**II-a7**), **(11-7),** or (**II-a8**)), Formula (**III**) (e.g., Formula (**III-1**), **(111-2), (111-3),** (**III-4**), (**III-5**), (**III-6**), (**III-7**), or (**III-8**)), Formula **(IV)** (e.g., Formula **(IV-a), (IV-b), (IV-c), (IV-al), (IV-b1), (IV-a2), (IV-b2), (IV-a3), (IV-b3), (IV-a4), (IV-b4), (IV-a5), (IV-b5), (IV-a6), (IV-b6), (IV-a7), (IV-b7), (IV-a8),** or **(IV-b8)),** Formula **(V)** (e.g., Formula **(V-a)** or **(V-b),** (**V-a1**), **(V-c), (V-d),** (**V-b1)**, **(V-a2), (V-b2), (V-a3),** or **(V-b3)),** Formula **(VI)** (e.g., Formula **(VI-a), (VI-b), (VI-c), (VI-d),** or **(VI-e))),** or Formula **(A)** (e.g., Formula (**I-a1**)), or a pharmaceutically acceptable salt thereof, and a pharmaceutically acceptable carrier.

Provided herein are methods for treating cancer in a subject in need thereof, the methods comprising administering to the subject a therapeutically effective amount a compound of Formula (**II**) (e.g., Formula (**II-a**), **(II-b),** (**II-a1**), **(II-b1), (II-a2), (II-b2), (11-3),** (**II-a3**), (**II-4), (II-a4),** (**II-5**), (**II-a5**), (**II-6**), **(II-a6),** (**II-7**), (**II-a7**), **(11-7),** or (**II-a8**)), Formula (**III**) (e.g., Formula (**III-1**), **(111-2), (111-3),** (**III-4**), (**III-5**), (**III-6**), (**III-7**), or (**III-8**)), Formula **(IV)** (e.g., Formula **(IV-a), (IV-b), (IV-c), (IV-al), (IV-b1), (IV-a2), (IV-b2), (IV-a3), (IV-b3), (IV-a4), (IV-b4), (IV-a5), (IV-b5), (IV-a6), (IV-b6), (IV-a7), (IV-b7), (IV-a8),** or **(IV-b8)),** Formula **(V)** (e.g., Formula **(V-a)** or **(V-b),** (**V-a1**), **(V-c), (V-d),** (**V-b1)**, **(V-a2), (V-b2), (V-a3),** or **(V-b3)),** Formula **(VI)** (e.g., Formula **(VI-a), (VI-b), (VI-c), (VI-d),** or **(VI-e))),** or Formula **(A)** (e.g., Formula (**I-a1**)), or a pharmaceutically acceptable salt thereof, or a pharmaceutical composition as provided herein.

Also provided herein are methods for treating cancer in a subject in need thereof, the methods comprising (a) determining that the cancer has a KRas dysregulation (e.g., a KRas mutation (e.g., a KRas G12A mutation, a KRas G12C mutation, a KRas G12D mutation, a KRas G12R mutation, a KRas G12S mutation, or a KRas G12V mutation)); and (b) administering to the subject a therapeutically effective amount of a compound of Formula (**II**) (e.g., Formula (**II-a**), (**II-b**), (**II-a1**), (**II-b1**), (**II-a2**), **(II-b2), (II-3),** (**II-a3**), (**II-4**), (**II-a4**), **(11-5), (II-a5), (II-6), (II-a6),** (**II-7**), (**II-a7**), (**II-7**), or (**II-a8**)), Formula (**III**) (e.g., Formula (**III-1**), (**III-2**), (**III-3**), (**III-4**), **(111-5), (111-6),** (**III-7**), or **(111-8)),** Formula **(IV)** (e.g., Formula **(IV-a), (IV-b), (IV-c), (IV-a1), (IV-b1), (IV-a2), (IV-b2), (IV-a3), (IV-b3), (IV-a4), (IV-b4), (IV-a5), (IV-b5), (IV-a6), (IV-b6), (IV-a7), (IV-b7), (IV-a8),** or **(IV-b8)),** Formula **(V)** (e.g., Formula **(V-a)** or **(V-b),** (**V-a1**), **(V-c), (V-d),** (**V-b1)**, **(V-a2), (V-b2), (V-a3),** or **(V-b3)),** Formula **(VI)** (e.g., Formula **(VI-a), (VI-b), (VI-c), (VI-d),** or **(VI-e))),** or Formula **(A)** (e.g., Formula (**I-a1**)), or a pharmaceutically acceptable salt thereof, or a pharmaceutical composition as provided herein.

Provided herein are methods of treating a KRas-associated disease or disorder (e.g., a mutant KRas-associated disease or disorder (e.g., a KRas G12A-associated cancer, a KRas G12C-associated cancer, a KRas G12D-associated cancer, a KRas G12R-associated cancer, a KRas G12S-associated cancer, or a KRas G12V-associated cancer)) in a subject, the methods comprising administering to a subject identified or diagnosed as having a KRas-associated disease or disorder a therapeutically effective amount of a compound of Formula **(II)** (e.g., Formula (**II-a**), **(II-b),** (**II-a1**), **(II-b1), (II-a2), (II-b2), (11-3),** (**II-a3**), **(11-4),** (**II-a4**), **(II-5),** (**II-a5**), (**II-6**), **(II-a6), (11-7),** (**II-a7**), (**II-7**), or (**II-a8**)), Formula (**III**) (e.g., Formula (**III-1**), (**III-2**), (**III-3**), (**III-4**), (**III-5**), (**III-6**), (**III-7**), or (**III-8**)), Formula **(IV)** (e.g., Formula **(IV-a), (IV-b), (IV-c), (IV-al), (IV-b1), (IV-a2), (IV-b2), (IV-a3), (IV-b3), (IV-a4), (IV-b4), (IV-a5), (IV-b5), (IV-a6), (IV-b6), (IV-a7), (IV-b7), (IV-a8),** or **(IV-b8)),** Formula **(V)** (e.g., Formula **(V-a)** or **(V-b),** (**V-a1**), **(V-c), (V-d), (V-b1), (V-a2), (V-b2), (V-a3),** or **(V-b3)),** Formula **(VI)** (e.g., Formula **(VI-a), (VI-b), (VI-c), (VI-d),** or **(VI-e))),** or Formula **(A)** (e.g., Formula (**I-a1**)), or a pharmaceutically acceptable salt thereof, or a pharmaceutical composition as provided herein.

This disclosure also provides methods of treating a KRas-associated disease or disorder (e.g., a mutant KRas-associated disease or disorder (e.g., a KRas G12A-associated disease or disorder, a KRas G12C-associated disease or disorder, a KRas G12D-associated disease or disorder, a KRas G12R-associated disease or disorder, a KRas G12S-associated disease or disorder, or a KRas G12V-associated disease or disorder)) in a subject, the methods comprising: determining that the disease or disorder in the subject is a KRas-associated disease or disorder (e.g., a mutant KRas-associated disease or disorder (e.g., a KRas G12A-associated disease or disorder, a KRas G12C-associated disease or disorder, a KRas G12D-associated disease or disorder, a KRas G12R-associated disease or disorder, a KRas G12S-associated disease or disorder, or a KRas G12V-associated disease or disorder)); and administering to the subject a therapeutically effective amount of a compound of Formula **(II)** (e.g., Formula **(II-a), (II-b), (II-a1), (II-b1),** (**II-a2**), **(II-b2),** (**II-3**), (**II-a3**), (**II-4**), (**II-a4**), (**II-5**), (**II-a5**), (**II-6), (II-a6), (11-7), (II-a7), (11-7),** or **(II-a8)),** Formula (**III**) (e.g., Formula (**III-1**), **(111-2),** (**III-3), (111-4), (111-5),** (**III-6**), **(111-7),** or (**III-8**)), Formula **(IV)** (e.g., Formula **(IV-a), (IV-b), (IV-c), (IV-a1), (IV-b1), (IV-a2), (IV-b2), (IV-a3), (IV-b3), (IV-a4), (IV-b4), (IV-a5), (IV-b5), (IV-a6), (IV-b6), (IV-a7), (IV-b7), (IV-a8),** or **(IV-b8)),** Formula **(V)** (e.g., Formula **(V-a)** or **(V-b), (V-a1), (V-c), (V-d), (V-b1), (V-a2), (V-b2), (V-a3),** or **(V-b3)),** Formula **(VI)** (e.g., Formula **(VI-a), (VI-b), (VI-c), (VI-d),** or **(VI-e))),** or Formula **(A)** (e.g., Formula (**I-a1**)), or a pharmaceutically acceptable salt thereof, or a pharmaceutical composition as provided herein.

Further provided herein are methods of treating a KRas-associated cancer (e.g., a mutant KRas-associated cancer (e.g., a KRas G12A-associated cancer, a KRas G12C-associated cancer, a KRas G12D-associated cancer, a KRas G12R-associated cancer, a KRas G12S-associated cancer, or a KRas G12V-associated cancer)) in a subject, the methods comprising administering to a subject identified or diagnosed as having a KRas-associated cancer (e.g., a KRas G12A-associated cancer, a KRas G12C-associated cancer, a mutant KRas-associated cancer (e.g., a KRas G12D-associated cancer, a KRas G12R-associated cancer, a KRas G12S-associated cancer, or a KRas G12V-associated cancer)) a therapeutically effective amount of a compound of Formula **(II)** (e.g., Formula (**II-a**), (**II-b**), (**II-a1**), (**II-b1**), (**II-a2**), (**II-b2**), (**II-3**), (**II-a3**), (**II-4**), (**II-a4**), (**II-5**), **(II-a5), (II-6), (II-a6), (11-7), (II-a7),** (**II-7**), or **(II-a8)),** Formula (**III**) (e.g., Formula (**III-1**), **(III-2), (III-3), (111-4), (III-5), (111-6), (111-7),** or **(III-8)),** Formula **(IV)** (e.g., Formula **(IV-a), (IV-b), (IV-c), (IV-al), (IV-b1), (IV-a2), (IV-b2), (IV-a3), (IV-b3), (IV-a4), (IV-b4), (IV-a5), (IV-b5), (IV-a6), (IV-b6), (IV-a7), (IV-b7), (IV-a8),** or **(IV-b8)),** Formula **(V)** (e.g., Formula **(V-a)** or **(V-b), (V-a1), (V-c), (V-d), (V-b1), (V-a2), (V-b2), (V-a3),** or **(V-b3)),** Formula **(VI)** (e.g., Formula **(VI-a), (VI-b), (VI-c), (VI-d),** or **(VI-e))),** or Formula **(A)** (e.g., Formula (**I-a1**)), or a pharmaceutically acceptable salt thereof, or a pharmaceutical composition as provided herein.

This disclosure also provides methods of treating a KRas-associated cancer (e.g., a mutant KRas-associated cancer (e.g., a KRas G12A-associated cancer, a KRas G12C-associated cancer, a KRas G12D-associated cancer, a KRas G12R-associated cancer, a KRas G12S-associated cancer, or a KRas G12V-associated cancer)) in a subject, the methods comprising: determining that the cancer in the subject has a KRas dysregulation (e.g., a KRas G12A-associated cancer, a KRas G12C-associated cancer, a KRas G12D-associated cancer, a KRas G12R-associated cancer, a KRas G12S-associated cancer, or a KRas G12V-associated cancer)); and administering to the subject a therapeutically effective amount of a compound of Formula (**II**) (e.g., Formula (**II-a**), **(II-b), (II-a1), (II-b1), (II-a2), (II-b2), (11-3),** (**II-a3**), (**II-4), (II-a4),** (**II-5**), (**II-a5**), (**II-6**), **(II-a6),** (**II-7**), (**II-a7**), **(11-7),** or (**II-a8**)), Formula (**III**) (e.g., Formula (**III-1**), **(111-2), (111-3),** (**III-4**), (**III-5**), (**III-6**), (**III-7**), or (**III-8**)), Formula **(IV)** (e.g., Formula **(IV-a), (IV-b), (IV-c), (IV-al), (IV-b1), (IV-a2), (IV-b2), (IV-a3), (IV-b3), (IV-a4), (IV-b4), (IV-a5), (IV-b5), (IV-a6), (IV-b6), (IV-a7), (IV-b7), (IV-a8),** or **(IV-b8)),** Formula **(V)** (e.g., Formula **(V-a)** or **(V-b),** (**V-a1**), **(V-c), (V-d),** (**V-b1)**, **(V-a2), (V-b2), (V-a3),** or **(V-b3)),** Formula **(VI)** (e.g., Formula **(VI-a), (VI-b), (VI-c), (VI-d),** or **(VI-e))),** or Formula **(A)** (e.g., Formula (**I-a1**)), or a pharmaceutically acceptable salt thereof, or a pharmaceutical composition as provided herein.

To facilitate understanding of the disclosure set forth herein, a number of terms are provided. Generally, the nomenclature used herein and the laboratory procedures in organic chemistry, medicinal chemistry, and pharmacology described herein are those well-known and commonly employed in the art. Unless defined otherwise, all technical and scientific terms used herein generally have the same meaning as commonly understood by one of ordinary skill in the art to which this disclosure belongs. Each of the patents, applications, published applications, and other publications that are mentioned throughout the specification and the attached appendices are incorporated herein by reference in their entireties. In the case of conflict between the present disclosure and any content incorporated by reference, the present disclosure controls.

The details of one or more embodiments of the invention are set forth in the accompanying drawings and the description below. Other features and advantages of the invention will be apparent from the description and drawings, and from the claims.

### DETAILED DESCRIPTION

This disclosure provides compounds of Formula **(II)** (e.g., Formula **(II-a),** (**II-b**), (**II-a1**), **(II-b1), (II-a2),** (**II-b2**), **(11-3),** (**II-a3**), **(11-4), (II-a4), (11-5), (II-a5),** (**II-6**), **(II-a6),** (**II-7), (II-a7), (11-7),** or (**II-a8**)), Formula (**III**) (e.g., Formula (**III-1**), (**III-2**), **(111-3),** (**III-4**), (**III-5),** (**III-6**), (**III-7**), or (**III-8**)), Formula **(IV)** (e.g., Formula **(IV-a), (IV-b), (IV-c),** (**IV-a1**), **(IV-b1), (IV-a2), (IV-b2), (IV-a3), (IV-b3), (IV-a4), (IV-b4), (IV-a5), (IV-b5), (IV-a6), (IV-b6), (IV-a7), (IV-b7), (IV-a8),** or **(IV-b8)),** Formula **(V)** (e.g., Formula **(V-a)** or **(V-b), (V-a1**), **(V-c), (V-d),** (**V-b1)**, **(V-a2), (V-b2), (V-a3),** or **(V-b3)),** Formula **(VI)** (e.g., Formula **(VI-a), (VI-b), (VI-c), (VI-d),** or **(VI-e))),** or Formula **(A)** (e.g., Formula (**I-a1**)), or pharmaceutically acceptable salts thereof, that inhibit a KRas protein (e.g., a dysregulated KRas protein, such as a mutant KRas protein). These compounds are useful, e.g., for treating a disease, disorder, or condition associated with a KRas dysregulation (e.g., a KRas mutation or amplification) in which increased and/or sustained (e.g., excessive) KRas activation contributes to the pathology and/or symptoms and/or progression of the disease, disorder, or condition (e.g., cancer) in a subject (e.g., a human). These compounds can also be useful, e.g., for treating a disease, disorder, or condition in which a mutant KRas protein (e.g., a resistance mutation) confers intrinsic resistance to one or more KRas inhibitors (e.g., a KRas inhibitor selective for a KRas G12C mutant protein), or to a non-KRas-targeted therapeutic agent. See, e.g., Misale, et al., Nature 486.7404 (2012): 532-536 and Awad, et al., New England Journal of Medicine 384.25 (2021): 2382-2393. This disclosure also provides compositions containing the compounds provided herein as well as methods of using and making the same.

Ras family genes (e.g., *KRAS, NRAS,* and *HRAS)* were the first oncogenes identified and are some of the most commonly mutated of all discovered oncogenes. See, e.g., Hunter et al. Mol Cancer Res. 2015;13(9):1325-35. The Ras family are guanine nucleotide binding proteins generally found at the inner leaflet of the cell membrane. A wild type Ras protein becomes activated when bound to GTP, but it is inactive when bound to GDP. Normally, growth factors bind to extracellular receptors to induce nucleotide exchange with the help of guanine nucleotide exchange factors (GEF) (e.g., Son of sevenless homolog 1 (SOS1)). These GEFs allow GDP to dissociate from a Ras protein and GTP to bind. Ras proteins can interact with effector proteins such as cRAF when bound to GTP. Hydrolysis of GTP to form GDP can deactivate Ras proteins, and the hydrolysis can be achieved through the intrinsic GTPase activity, which may be enhanced by binding to a GTPase activating protein (GAP). There are 3 major RAS proteins in humans: KRas, HRas, and NRas.

Some oncogenic KRas missense mutations can prevent or slow GTP hydrolysis and result in the accumulation of KRas in the active state. Signaling pathways associated with KRas are persistently activated in many cancers, where they participate in cellular growth and proliferation, differentiation, protein synthesis, glucose metabolism, cell survival, and inflammation.

Mutant KRas proteins often have altered Raf affinity and/or altered intrinsic GTPase activity. See, for example, Table 1 reproduced from Hunter et al. Mol Cancer Res. 2015;13(9):1325-35. These changes and other factors can contribute to increased KRas signaling in mutant KRas proteins.

**Table 1**

| | | **Raf Affinity** | |
|---|---|---|---|
| | | **High** | **Low** |
| **Intrinsic GTPase activity** | **Low** | G12A | G12R |
| | | Q61L | G12V |
| | **High** | Wild type | G12D |
| | | G12C | |
| | | G13D | |

KRas inhibitors are described in, for example, International Publication Nos. WO 2024/112654; WO 2023/154766; WO 2023/143623; WO 2022/240971; WO 2020/236940; WO 2022/115439; WO 2023/086383; WO 2021/093758; WO 2022/135546; WO 2021/139748; WO 2022/251576; and WO 2023/025116.

Additional examples of KRas inhibitors are described in, for example, International Publication Nos. WO 2022/132200; WO 2022/133038; WO 2023/150284; WO 2022/261154; WO 2023/183585; WO 2023/099592; WO 2023/099623; WO 2023/099624; WO 2023/099608; WO 2022/250170; WO 2022/173870; WO 2022/236578; WO 2022/237649; WO 2022/248885; WO 2022/256459; WO 2022/258974; WO 2022/266015; WO 2023/018809; WO 2023/018810; WO 2023/018812; WO 2023/020518; WO 2023/020519; WO 2023/020521; WO 2023/020523; WO 2023/046135; WO 2023/061294; WO 2023/097227; WO 2023/114733; WO 2023/137223; WO 2023/141300; WO 2023/138583; WO 2023/159086; WO 2023/159087; WO 2023/173016; WO 2023/173017; WO 2023/179703; WO 2023/125627; WO 2022/216762; WO 2024/030633; WO 2023/230190; and CN 116143806.

### Compound Embodiments

Provided herein are compounds of Formula **(II):** 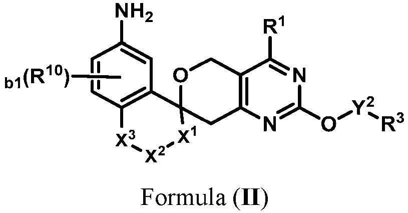 or pharmaceutically acceptable salts thereof, wherein:
**R¹** is selected from the group consisting of:
   **(i)** a 4-10 membered heterocyclyl optionally substituted with 1-4 **R⁷**;
   **(ii)** an 8-12 membered bicyclic heterocyclyl, wherein the heterocyclyl comprises an endocyclic group selected from the group consisting of C(=O)NH and S(O)₂NH, and wherein the heterocyclyl is further optionally substituted with 1-3 **R⁷** at one or more ring carbon atoms; and
   **(iii)** 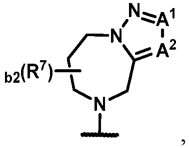 wherein **b2** is 0, 1, 2, or 3; and **A¹** and **A²** are independently selected from the group consisting of: N, CH, and **CR⁷**;
each **R⁷** is independently selected from the group consisting of **R^{a}** and **R^{b}**;
**X¹** is selected from the group consisting of a bond, S(O)₀₋₂, CH₂, CH**R^{L}**, C(**R^{L}**)₂, and O;
**X²** and **X³** are independently selected from the group consisting of: CH₂, CH**R^{L}**, C(**R^{L}**)₂, O, and S(O)₀₋₂, provided that no more than one of **X¹**, **X²**, and **X³** is selected from the group consisting of: O and S(O)₀₋₂;
**b1** is 1 or 2;
each **R¹⁰** is independently selected from the group consisting of **R^{a}** and **R^{b}**;
each **R^{L}** is independently selected from the group consisting of C₁₋₃ alkoxy, -F, -CN, and C₁₋₃ alkyl optionally substituted with 1-3 **R^{c}**; or
one pair of **R^{L}** on the same or different ring carbon atom(s) taken together with the ring atom(s) connecting them form a C₃₋₆ cycloalkyl ring;
**Y²** is a bond or a straight-chain C₁₋₆ alkylene optionally substituted with 1-6 **R^{Y}**;
each **R^{Y}** is independently selected from the group consisting of: halo, cyano, -OH, oxo, C₁₋₆ alkoxy, C₁₋₆ haloalkoxy, C₁₋₆ alkyl, and C₁₋₆ haloalkyl, or
one pair of **R^{Y}** on the same or different carbon atom(s) taken together with the atom(s) connecting them forms a C₃₋₆ cycloalkyl ring or 4-6 membered heterocyclyl ring, each of which is optionally substituted with 1-3 independently selected C₁₋₃ alkyl;
**R³** is selected from the group consisting of:
   **(a)** a 4-15 membered heterocyclyl optionally substituted with 1-6 substituents independently selected from the group consisting of: **R^{a}** and **R^{b}**; and
   **(b)** -N**R^{d}R^{e}**;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   **(f)** -C₁₋₆ haloalkoxy;
   (**g**) -N**R^{d}R^{e}**;
   **(h)** C(=O)C₁₋₆ alkyl;
   **(i)** C(=O)C₁₋₆ haloalkyl;
   **(j)** C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   **(o)** S(O)₀₋₂(C₁₋₆ haloalkyl);
   **(p)** S(O)₁₋₂N(**R^{f}**)₂; and
   **(q)** C₁₋₆ alkyl, C₂₋₆ alkenyl, or C₂₋₆ alkynyl, each optionally substituted with 1-6 **R^{c};**
each **R^{b}** is independently selected from the group consisting of: -(**L^{b}**)**_{b}**-**R^{b1}** and -**R^{b1}**, wherein:
   **b** is 1, 2, or 3;
   each -**L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e}**, C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)₁₋₂N(**R^{f}**)₂;
   each **R^{d}** and **R^{c};** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂-.

In some embodiments, the compounds of Formula (**II**) are other than Compound Nos. **R139, R139a, R139b, R139c, R158, R158a, R158b, R158c, R160, R160a, R161, R161a, R161b, R161c, R164, R164a, R164b, R170, R170a, R171, R171a, R176, R176a, R176b, R176c, R176d, R176e, R178, R178a, R178b, R179, R179a, R179b, R179d, R179e, R179f, R180, R180a, R181,** and **R181a** as depicted in **Table C1,** or pharmaceutically acceptable salts thereof.

In some embodiments, the compounds of Formula **(II)** are other than the compounds depicted in **Table C1** of International Patent Application PCT/US2023/080513 (published as WO 2024/112654), or pharmaceutically acceptable salts thereof.

In some embodiments of Formula (**II**), **b1** is 1 or 2; and each **R¹⁰** is independently selected from the group consisting of: -Cl, -F, -CN, and C₁₋₃ alkyl optionally substituted with 1-3 **R^{c}.**

In some embodiments of Formula (**II**), **b1** is 1; and **R¹⁰** is CN. In some embodiments, **b1** is 1; **R¹⁰** is CN; and **R¹⁰** is *ortho* to the NH₂ group (i.e., *meta* to **X³**).

In some embodiments of Formula **(II), b1** is 2; one **R¹⁰** is CN; and the other **R¹⁰** is independently selected from the group consisting of: -Cl, -F, -CN, and C₁₋₃ alkyl optionally substituted with 1-3 **R^{c};**

In some embodiments, the compounds of Formula **(II)** are compounds of Formula **(II-**
**a):** 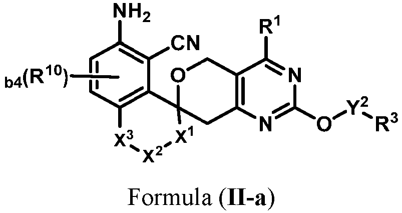 or pharmaceutically acceptable salts thereof, wherein:
   **b4** is 0 or 1; and
   **R¹⁰** is selected from the group consisting of: -Cl, -F, -CN, and C₁₋₃ alkyl optionally substituted with 1-3 **R^{c}.**

In some embodiments, the compounds of Formula **(II)** are compounds of Formula **(II-**
**b):** 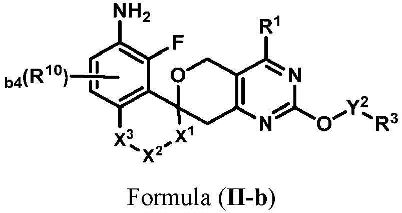 or pharmaceutically acceptable salts thereof, wherein:
**b4** is 0 or 1; and
**R¹⁰** is selected from the group consisting of: -Cl, -F, -CN, and C₁₋₃ alkyl optionally substituted with 1-3 **R^{c}.**

In some embodiments of Formula **(II-a)** or **(II-b), b4** is 0.

In some embodiments of Formula **(II-a)** or **(II-b), b4** is 1.

In some embodiments of Formula **(II-a)** or **(II-b), b4** is 1; and **R¹⁰** is *ortho* to **X³**.

Also provided herein are compounds of Formula (III): 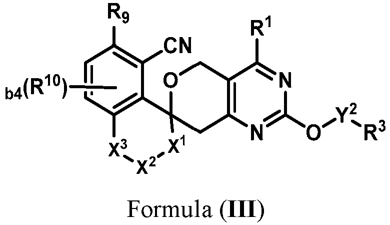 or pharmaceutically acceptable salts thereof, wherein:
**R¹** is selected from the group consisting of:
   **(i)** a 4-10 membered heterocyclyl optionally substituted with 1-4 **R⁷**;
   **(ii)** an 8-12 membered bicyclic heterocyclyl, wherein the heterocyclyl comprises an endocyclic group selected from the group consisting of C(=O)NH and S(O)₂NH, and wherein the heterocyclyl is further optionally substituted with 1-3 **R⁷** at one or more ring carbon atoms; and
   **(iii)** 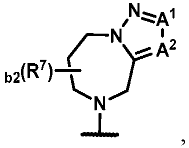 wherein **b2** is 0, 1, 2, or 3; and **A¹** and **A²** are independently selected from the group consisting of: N, CH, and **CR⁷**;
each **R⁷** is independently selected from the group consisting of **R^{a}** and **R^{b}**;
**X¹** is selected from the group consisting of a bond, S(O)₀₋₂, CH₂, CH**R^{L}**, C(**R^{L}**)₂, and O;
**X²** and **X³** are independently selected from the group consisting of: CH₂, CH**R^{L}**, C(**R^{L}**)₂, O, and S(O)₀₋₂, provided that no more than one of **X¹**, **X²**, and **X³** is selected from the group consisting of: O and S(O)₀₋₂;
**R⁹** is selected from the group consisting of: H, N**R^{d}R^{e}**, -OH, and halo;
**b4 is 0 or** 1;
**R¹⁰** is selected from the group consisting of **R^{a}** and **R^{b}**;
each **R^{L}** is independently selected from the group consisting of C₁₋₃ alkoxy, -F, CN, and C₁₋₃ alkyl optionally substituted with 1-3 **R^{c}**; or
one pair of **R^{L}** on the same or different ring carbon atom(s) taken together with the ring atom(s) connecting them form a C₃₋₆ cycloalkyl ring;
**Y²** is a bond or a straight-chain C₁₋₆ alkylene optionally substituted with 1-6 **R^{Y}**;
each **R^{Y}** is independently selected from the group consisting of: halo, cyano, -OH, oxo, C₁₋₆ alkoxy, C₁₋₆ haloalkoxy, C₁₋₆ alkyl, and C₁₋₆ haloalkyl, or
one pair of **R^{Y}** on the same or different carbon atom(s) taken together with the atom(s) connecting them forms a C₃₋₆ cycloalkyl ring or 4-6 membered heterocyclyl ring, each of which is optionally substituted with 1-3 independently selected C₁₋₃ alkyl;
**R³** is selected from the group consisting of:
   **(a)** a 4-15 membered heterocyclyl optionally substituted with 1-6 substituents independently selected from the group consisting of: **R^{a}** and **R^{b}**; and
   (**b**) -N**R^{d}R^{e}**;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   **(f)** -C₁₋₆ haloalkoxy;
   **(g)** -N**R^{d}R^{e}**;
   **(h)** C(=O)C₁₋₆ alkyl;
   **(i)** C(=O)C₁₋₆ haloalkyl;
   **(j)** C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   (**o**) S(O)₀₋₂(C₁₋₆ haloalkyl);
   **(p)** S(O)₁₋₂N(**R^{f}**)₂; and
   **(q)** C₁₋₆ alkyl, C₂₋₆ alkenyl, or C₂₋₆ alkynyl, each optionally substituted with 1-6 **R^{c};**
each **R^{b}** is independently selected from the group consisting of: -(**L^{b}**)**_{b}**-**R^{b1}** and -**R^{b1}**, wherein:
   **b** is 1, 2, or 3;
   each -**L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e}**, C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)₁₋₂N(**R^{f}**)₂;
   each **R^{d}** and **R^{c};** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂-.

In some embodiments, the compounds of Formula (**III**) are other than Compound Nos. **R158, R158a, R158b, R158c, R161, R161a, R161b, R161c, R176, R176a, R176b, R176c, R176d, R176e, R177, R177a, R178, R178a, R178b, R179, R179a, R179b, R179d, R179e, R179f, R180,** and **R180a** as depicted in **Table C1,** or pharmaceutically acceptable salts thereof.

In some embodiments, the compounds of Formula **(III)** are other than the compounds depicted in **Table C1 of** International Patent Application PCT/US2023/080513 (published as WO 2024/112654), or pharmaceutically acceptable salts thereof.

In some embodiments of Formula **(III), b4** is 0.

In some embodiments of Formula **(III), b4** is 1.

In some embodiments of Formula **(III), b4** is 1; and **R¹⁰** is *ortho* to **X³**.

In some embodiments of Formula **(III), R⁹** is selected from the group consisting of: N**R^{d}R^{e}**, -OH, and halo. In some embodiments of Formula (**III**), **R⁹** is N**R^{d}R^{e}**. For example, **R⁹** can be -NH₂.

In some embodiments of Formula (**III**), **R⁹** is -NH₂; and **R¹⁰** is selected from the group consisting of: -Cl, -F, -CN, and C₁₋₃ alkyl optionally substituted with 1-3 **R^{c}**.

In some embodiments of Formula **(III), R⁹** is -NH₂; and **b4** is 0.

In some embodiments of Formula **(II)** (e.g., Formula **(II-a)** or **(II-b))** or Formula **(III), X¹** is CH₂ or CH**R^{L}**.

In some embodiments of Formula **(II)** (e.g., Formula **(II-a)** or **(II-b))** or Formula **(III), X²** and **X³** are independently selected from the group consisting of: CH₂, CH**R^{L}**, and C(**R^{L}**)₂.

In some embodiments of Formula **(II)** (e.g., Formula **(II-a)** or **(II-b))** or Formula **(III), X¹** is CH₂; and **X²** and **X³** are both CH₂.

In some embodiments of Formula **(II)** (e.g., Formula **(II-a)** or **(II-b))** or Formula **(III),** at least one of **X¹**, **X²**, and **X³** is selected from the group consisting of: CH**R^{L}** and C(**R^{L}**)₂.

In some embodiments of Formula **(II)** (e.g., Formula **(II-a)** or **(II-b))** or Formula **(III),** one of **X¹**, **X²**, and **X³** (e.g., **X³**) is selected from the group consisting of: CH**R^{L}** and C(**R^{L}**)₂.

In some embodiments of Formula **(II)** (e.g., Formula **(II-a)** or **(II-b))** or Formula **(III), X¹** is CH₂; and **X²** and **X³** are independently selected from the group consisting of: CH₂, CH**R^{L}**, and C(**R^{L}**)₂, provided that 1-2 (e.g., one) of **X²** and **X³** is independently CH**R^{L}** or C(**R^{L}**)₂.

In some embodiments of Formula **(II)** (e.g., Formula **(II-a)** or **(II-b))** or Formula **(III), X¹** is CH₂; **X²** is CH₂; and **X³** is CH**R^{L}**. In some embodiments, each **R^{L}** is independently selected from the group consisting of: CH₃, CF₃, CHF₂, and CH₂F (e.g., each **R^{L}** is CH₃).

In some embodiments of Formula **(II)** (e.g., Formula **(II-a)** or **(II-b))** or Formula **(III), X¹** is CH₂; **X²** is CH₂; and **X³** is CH**R^{L}**, wherein **R^{L}** is C₁₋₃ alkyl optionally substituted with 1-3 **R^{c}** (e.g., **R^{L}** can be C₁₋₃ alkyl optionally substituted with 1-3 F).

In some embodiments of Formula **(II)** (e.g., Formula **(II-a)** or **(II-b))** or Formula **(III), X¹** is CH₂; **X²** is CH₂; and **X³** is C(H)CF₃.

In some embodiments of Formula **(II)** (e.g., Formula **(II-a)** or **(II-b))** or Formula **(III), X¹** is CH₂; **X²** is CH₂; and **X³** is C(H)CH₃.

In some embodiments of Formula **(II)** (e.g., Formula **(II-a)** or **(II-b))** or Formula **(III),** one of **X²** and **X³** is -O-; and the other of **X²** and **X³** is selected from the group consisting of: CH₂, CH**R^{L}**, and C(**R^{L}**)₂.

In some embodiments of Formula **(II)** (e.g., Formula **(II-a)** or **(II-b))** or Formula **(III), X²** is -O-; and **X³** is selected from the group consisting of: CH₂, CH**R^{L}**, and C(**R^{L}**)₂. In some embodiments, each **R^{L}** is independently selected from the group consisting of: CH₃, CF₃, CHF₂, and CH₂F (e.g., each **R^{L}** is CH₃).

In some embodiments of Formula **(II)** (e.g., Formula **(II-a)** or **(II-b))** or Formula **(III),** each **R^{L}** is independently C₁₋₃ alkyl optionally substituted with 1-3 **R^{c}.**

In some embodiments of Formula **(II)** (e.g., Formula **(II-a)** or **(II-b))** or Formula **(III),** each **R^{L}** is independently selected from the group consisting of: CH₃, CF₃, CHF₂, and CH₂F (e.g., each **R^{L}** is CH₃).

In some embodiments of Formula (II), the 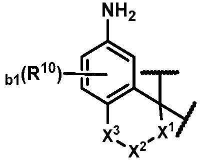 moiety is selected from the group consisting of: 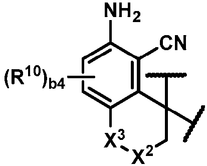 and 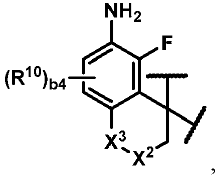 wherein:
**b4** is 0 or 1;
**X²** is -O- or -CH₂-;
**X³** is -CH₂- or -CH**R^{L}**-, wherein **R^{L}** is C₁₋₃ alkyl (e.g., methyl); and
each **R¹⁰** is independently selected from the group consisting of: -Cl, -F, -CN, and C₁₋₃ alkyl optionally substituted with 1-3 **R^{c}**.

In some embodiments of Formula **(II),** the wherein: 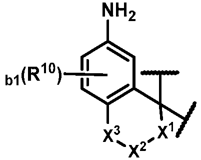 moiety is 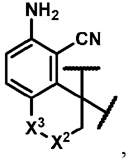
**X²** is -O- or -CH₂-; and
**X³** is -CH₂- or -CH**R^{L}**-, wherein **R^{L}** is C₁₋₃ alkyl optionally substituted with 1-3 F (e.g., methyl, methyl, or CF₃).

In some embodiments of Formula (II), the 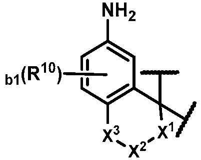 moiety is selected from the group consisting of: 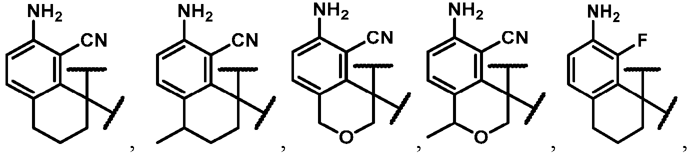 and

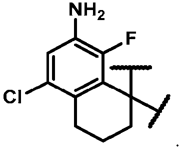

In some embodiments of Formula (II), the 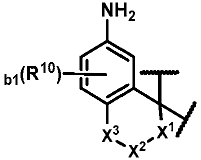 moiety is selected from the group consisting of:

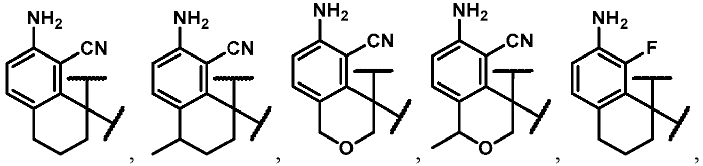

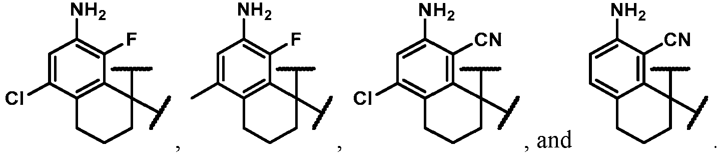

In some embodiments of Formula **(II),** the 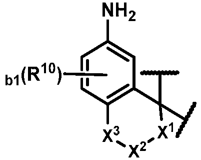 moiety is selected from the group consisting of: 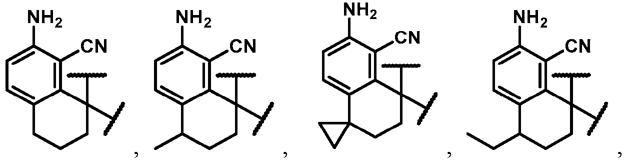 and 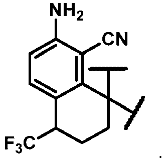 For example, the 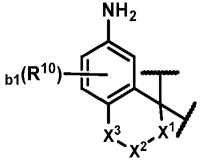 moiety can be 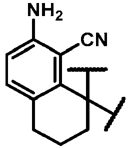 or

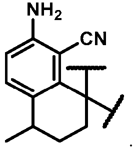

In some embodiments of Formula (III), the 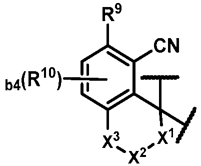 moiety is 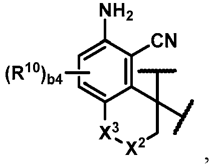 wherein:
**b4** is 0 or 1;
**X²** is -O- or -CH₂-;
**X³** is -CH₂- or -CH**R^{L}**-, wherein **R^{L}** is C₁₋₃ alkyl (e.g., methyl); and
**R¹⁰** is selected from the group consisting of: -Cl, -F, -CN, and C₁₋₃ alkyl optionally substituted with 1-3 **R^{c}**.

For example, the 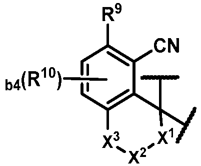 moiety can be:

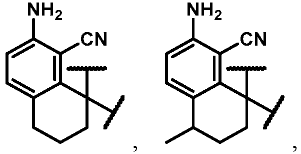

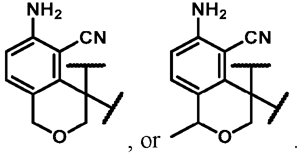

In some embodiments of Formula (III), the 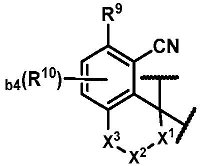 moiety is selected from the group consisting of: 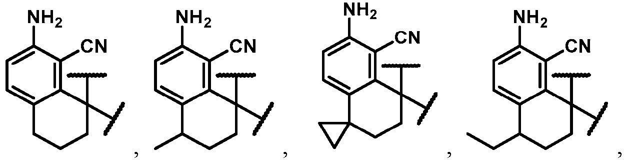 and 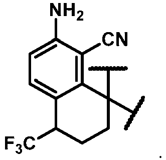 For example, the 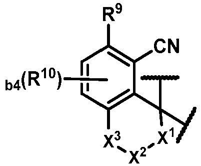 moiety can be 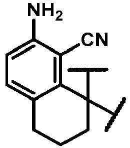 or

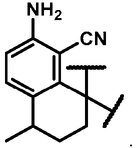

Also provided herein are compounds of Formula (IV): 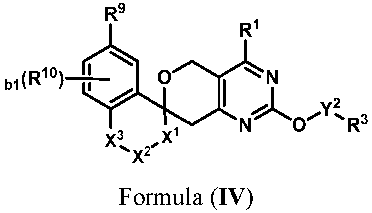 or pharmaceutically acceptable salts thereof, wherein:
**X¹** is selected from the group consisting of a bond, S(O)₀₋₂, CH₂, CH**R^{L}**, C(**R^{L}**)₂, and O;
**X²** and **X³** are independently selected from the group consisting of: CH₂, CH**R^{L}**, C(**R^{L}**)₂, O, and S(O)₀₋₂, provided that at least one of **X¹**, **X²**, and **X³** is CH**R^{L}** or C(**R^{L})**₂;
further provided that no more than one of **X¹**, **X²**, and **X³** is selected from the group consisting of: O and S(O)₀₋₂;
**b1** is 0, 1 or 2;
**R⁹** is selected from the group consisting of: H, OH, N**R^{d}R^{e}**, and halo;
each **R¹⁰** is independently selected from the group consisting of **R^{a}** and **R^{b}**;
each **R^{L}** is independently selected from the group consisting of C₁₋₃ alkoxy, -F, CN, and C₁₋₃ alkyl optionally substituted with 1-3 **R^{c}**;
**R¹** is selected from the group consisting of:
   (i) a 4-10 membered heterocyclyl optionally substituted with 1-4 **R⁷**;
   (ii) an 8-12 membered bicyclic heterocyclyl, wherein the heterocyclyl comprises an endocyclic group selected from the group consisting of C(=O)NH and S(O)₂NH, and wherein the heterocyclyl is further optionally substituted with 1-3 **R⁷** at one or more ring carbon atoms; and
   (iii) 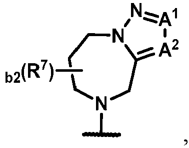 wherein b2 is 0, 1, 2, or 3; and **A¹** and **A²** are independently selected from the group consisting of: N, CH, and C**R⁷**;
each **R⁷** is independently selected from the group consisting of **R^{a}** and **R^{b}**;
**Y²** is a bond or a straight-chain C₁₋₆ alkylene optionally substituted with 1-6 **R^{Y}**;
each **R^{Y}** is independently selected from the group consisting of: halo, cyano, -OH, oxo, C₁₋₆ alkoxy, C₁₋₆ haloalkoxy, C₁₋₆ alkyl, and C₁₋₆ haloalkyl, or
one pair of **R^{Y}** on the same or different carbon atom(s) taken together with the atom(s) connecting them forms a C₃₋₆ cycloalkyl ring or 4-6 membered heterocyclyl ring, each of which is optionally substituted with 1-3 independently selected C₁₋₃ alkyl;
**R³** is selected from the group consisting of:
   **(a)** a 4-15 membered heterocyclyl optionally substituted with 1-6 substituents independently selected from the group consisting of: **R^{a}** and **R^{b}**; and
   **(b)** -N**R^{d}R^{e}**;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   **(f)** -C₁₋₆ haloalkoxy;
   **(g)** -N**R^{d}R^{e}**;
   **(h)** C(=O)C₁₋₆ alkyl;
   **(i)** C(=O)C₁₋₆ haloalkyl;
   (**j**) C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   **(o)** S(O)₀₋₂(C₁₋₆ haloalkyl);
   **(p)** S(O)₁₋₂N(**R^{f}**)₂; and
   **(q)** C₁₋₆ alkyl, C₂₋₆ alkenyl, or C₂₋₆ alkynyl, each optionally substituted with 1-6 **R^{c}**;
each **R^{b}** is independently selected from the group consisting of: -(**L^{b}**)**_{b}-R^{b1}** and -**R^{b1}**, wherein:
   **b is** 1, 2, or 3;
   each -**L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e}**, C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)₁₋₂N(**R^{f}**)₂;
   each **R^{d}** and **R^{e}** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂₋.

In some embodiments, the compounds of Formula **(IV)** are other than Compound Nos. **R124, R124a, R124b, R124c, R124d, R124e, R124f, R125, R125a, R130, R130a, R131, R131a, R133, R133a, R133b, R134, R134a, R138, R138a, R148, R148a, R162, R162a, R163, R163a, R175, R175a, R176, R176a, R176b, R176c, R176d, R176e, R179, R179a, R179b, R179d, R179e,** and **R179f,** as depicted in **Table C1,** or pharmaceutically acceptable salts thereof.

In some embodiments, the compounds of Formula **(IV)** are other than the compounds depicted in **Table C1** of International Patent Application PCT/US2023/080513 (published as WO 2024/112654), or pharmaceutically acceptable salts thereof.

In some embodiments of Formula **(IV), R⁹** is selected from the group consisting of: OH, N**R^{d}R^{e}**, and halo. For example, **R⁹** can be N**R^{d}R^{e}** (e.g., -NH₂).

In some embodiments of Formula **(IV), b1** is 1 or 2; and each **R¹⁰** is independently selected from the group consisting of: -Cl, -F, -CN, and C₁₋₃ alkyl optionally substituted with 1-3 **R^{c}**.

In some embodiments of Formula **(IV), b1** is 1; and **R¹⁰** is CN.

In some embodiments of Formula **(IV), b1** is 2; one **R¹⁰** is CN; and the other **R¹⁰** is independently selected from the group consisting of: -Cl, -F, -CN, and C₁₋₃ alkyl optionally substituted with 1-3 **R^{c}**.

In some embodiments, the compounds of Formula **(IV)** are compounds of Formula **(IV-a):** 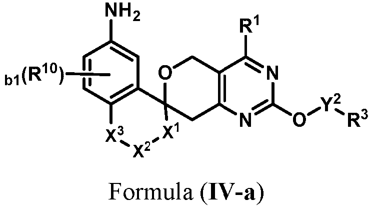 or pharmaceutically acceptable salts thereof, wherein:
each **R¹⁰** is independently selected from the group consisting of: -Cl, -F, -CN, and C₁₋₃ alkyl optionally substituted with 1-3 **R^{c}**.

In some embodiments, the compounds of Formula **(IV)** are compounds of Formula **(IV-**b): 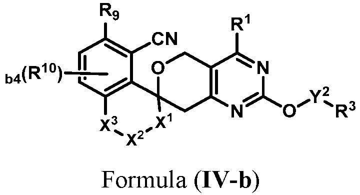 or pharmaceutically acceptable salts thereof, wherein:
**b4** is 0 or 1; and
**R¹⁰** is selected from the group consisting of: -Cl, -F, -CN, and C₁₋₃ alkyl optionally substituted with 1-3 **R^{c}.**

In some embodiments of Formula **(IV-b), R⁹** is -N**R^{d}R^{e}**. For example, **R⁹** can be -NH₂.

In some embodiments of Formula **(IV-b), b4** is 0.

In some embodiments of Formula **(IV-b), b4** is 1.

In some embodiments of Formula **(IV-b), b4** is 1; and **R¹⁰** is *ortho* to **X³.**

In some embodiments of Formula **(IV)** (e.g., Formula **(IV-a) or (IV-b)), X¹** is CH₂.

In some embodiments of Formula **(IV)** (e.g., Formula **(IV-a) or (IV-b)), X²** is CH₂; and **X³** is CH**R^{L}**.

In some embodiments of Formula **(IV)** (e.g., Formula **(IV-a)** or **(IV-b)), X²** is -O-; and **X³** is selected from the group consisting of: CH**R^{L}** and C(**R^{L}**)₂.

In some embodiments of Formula **(IV)** (e.g., Formula **(IV-a)** or **(IV-b)),** each **R^{L}** is independently C₁₋₃ alkyl optionally substituted with 1-3 **R^{c}** (e.g., C₁₋₃ alkyl optionally substituted with 1-3 F).

In some embodiments of Formula **(IV)** (e.g., Formula **(IV-a)** or **(IV-b)),** each **R^{L}** is independently selected from the group consisting of: CH₃, CF₃, CHF₂, and CH₂F (e.g., each **R^{L}** is CH₃).

In some embodiments of Formula **(IV)** (e.g., Formula **(IV-a)** or **(IV-b)),** one **R^{L}** is CF₃.

In some embodiments of Formula **(IV)** (e.g., Formula **(IV-a)** or **(IV-b)),** one **R^{L}** is CH₃.

In some embodiments, the compounds of Formula **(IV)** are compounds of Formula **(IV-**c): 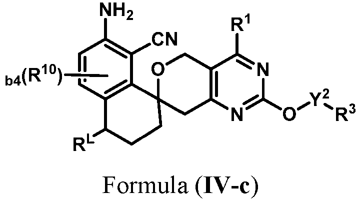 or pharmaceutically acceptable salts thereof, wherein:
**b4** is 0 or 1; and
**R¹⁰** is selected from the group consisting of: -Cl, -F, -CN, and C₁₋₃ alkyl optionally substituted with 1-3 **R^{c}**.

In some embodiments of Formula **(IV-c), b4** is 0.

In some embodiments of Formula **(IV-c), b4** is 1.

In some embodiments of Formula **(IV-c), b4** is 1; and **R¹⁰** *is para* to the CN group.

In some embodiments of Formula **(IV-c), R^{L}** is C₁₋₃ alkyl optionally substituted with 1-3 **R^{c}** (e.g., C₁₋₃ alkyl optionally substituted with 1-3 F).

In some embodiments of Formula **(IV-c), R^{L}** is CF₃.

In some embodiments of Formula **(IV-c), R^{L}** is CH₃.

Also provided herein are compounds of Formula (V): 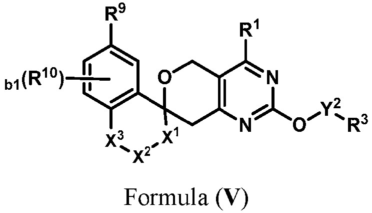 or pharmaceutically acceptable salts thereof, wherein:
**X¹** is selected from the group consisting of a bond, S(O)₀₋₂, CH₂, CH**R^{L}**, C(**R^{L}**)₂, and O;
**X²** and **X³** are independently selected from the group consisting of: CH₂, CH**R^{L}**, C(**R^{L}**)₂, O, and S(O)₀₋₂, provided that 2-3 of **X¹**, **X²**, and **X³** are independently CH**R^{L}** or C(**R^{L}**)₂;
one pair of **R^{L}** on the same or different ring carbon atom(s) taken together with the ring atom(s) connecting them form a C₃₋₆ cycloalkyl ring; and
each additional **R^{L}** is independently selected from the group consisting of: C₁₋₃ alkoxy, -F, CN, and C₁₋₃ alkyl optionally substituted with 1-3 **R^{c}**;
**b1** is 0, 1 or 2;
**R⁹** is selected from the group consisting of: H, OH, N**R^{d}R^{e}**, and halo;
each **R¹⁰** is independently selected from the group consisting of **R^{a}** and **R^{b}**;
**R¹** is selected from the group consisting of:
   **(i) a** 4-10 membered heterocyclyl optionally substituted with 1-4 **R⁷**;
   **(ii)** an 8-12 membered bicyclic heterocyclyl, wherein the heterocyclyl comprises an endocyclic group selected from the group consisting of C(=O)NH and S(O)₂NH, and wherein the heterocyclyl is further optionally substituted with 1-3 **R⁷** at one or more ring carbon atoms; and
   (iii) 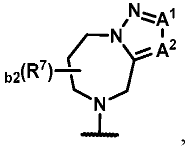 wherein **b2** is 0, 1, 2, or 3; and **A¹** and **A²** are independently selected from the group consisting of: N, CH, and C**R⁷**;
each **R⁷** is independently selected from the group consisting of **R^{a}** and **R^{b}**;
**Y²** is a bond or a straight-chain C₁₋₆ alkylene optionally substituted with 1-6 **R^{Y}**;
each **R^{Y}** is independently selected from the group consisting of: halo, cyano, -OH, oxo, C₁₋₆ alkoxy, C₁₋₆ haloalkoxy, C₁₋₆ alkyl, and C₁₋₆ haloalkyl, or
one pair of **R^{Y}** on the same or different carbon atom(s) taken together with the atom(s) connecting them forms a C₃₋₆ cycloalkyl ring or 4-6 membered heterocyclyl ring, each of which is optionally substituted with 1-3 independently selected C₁₋₃ alkyl;
**R³** is selected from the group consisting of:
   **(a)** a 4-15 membered heterocyclyl optionally substituted with 1-6 substituents independently selected from the group consisting of: **R^{a}** and **R^{b}**; and
   **(b)** -N**R^{d}R^{e}**;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   (**f**) -C₁₋₆ haloalkoxy;
   (**g**) -N**R^{d}R^{e}**;
   **(h)** C(=O)C₁₋₆ alkyl;
   **(i)** C(=O)C₁₋₆ haloalkyl;
   **(j)** C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   (**o**) S(O)₀₋₂(C₁₋₆ haloalkyl);
   **(p)** S(O)₁₋₂N(**R^{f}**)₂; and
   **(q)** C₁₋₆ alkyl, C₂₋₆ alkenyl, or C₂₋₆ alkynyl, each optionally substituted with 1-6 **R^{c}**;
each **R^{b}** is independently selected from the group consisting of: -(**L^{b}**)**_{b}**-**R^{b1}** and -**R^{b1}**, wherein:
   **b is** 1, 2, or 3;
   each -**L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e}**, C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)₁₋₂N(**R^{f}**)₂;
   each **R^{d}** and **R^{e}** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂₋.

In some embodiments, the compounds of Formula (V) are other than Compound Nos. **R172** and **R172a** as depicted in **Table C1,** or a pharmaceutically acceptable salt thereof.

In some embodiments, the compounds of Formula **(V)** are other than the compounds depicted in **Table C1** of International Patent Application PCT/US2023/080513 (published as WO 2024/112654), or pharmaceutically acceptable salts thereof.

In some embodiments of Formula (V), **R⁹** is -N**R^{d}R^{e}**. For example, **R⁹** can be -NH₂.

In some embodiments of Formula **(V), b1** is 1; and **R¹⁰** is CN.

In some embodiments of Formula **(V), b1** is 2; one **R¹⁰** is CN; and the other **R¹⁰** is independently selected from the group consisting of: -Cl, -F, -CN, and C₁₋₃ alkyl optionally substituted with 1-3 **R^{c}**.

In some embodiments, the compounds of Formula **(V)** are compounds of Formula **(V-**
**a):** 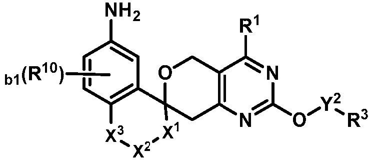 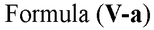 or pharmaceutically acceptable salts thereof, wherein:
   each **R¹⁰** is independently selected from the group consisting of: -Cl, -F, -CN, and C₁₋₃ alkyl optionally substituted with 1-3 **R^{c}**.

In some embodiments of Formula (V-a), **b1** is 1; and **R¹⁰** is CN.

In some embodiments of Formula (V-a), **b1** is 2; one **R¹⁰** is CN; and the other **R¹⁰** is selected from the group consisting of: -Cl, -F, -CN, and C₁₋₃ alkyl optionally substituted with 1-3 **R^{c}**.

In some embodiments, the compounds of Formula **(V)** are compounds of Formula **(V-b):** 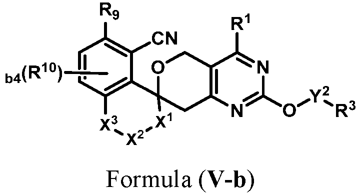 or pharmaceutically acceptable salts thereof, wherein:
**b4** is 0 or 1; and
**R¹⁰** is selected from the group consisting of: -Cl, -F, -CN, and C₁₋₃ alkyl optionally substituted with 1-3 **R^{c}**.

In some embodiments of Formula **(V-b), R⁹** is -N**R^{d}R^{e}**. For example, **R⁹** can be -NH₂.

In some embodiments of Formula **(V-b), b4** is 1.

In some embodiments of Formula **(V-b), b4** is 1; and **R¹⁰** is *ortho* to **X³.**

In some embodiments of Formula **(V)** (e.g., Formula **(V-a)** or **(V-b)), X²** is -O- or - CH₂- (e.g., -CH₂-).

In some embodiments of Formula **(V)** (e.g., Formula **(V-a)** or **(V-b)), X¹** is CH**R^{L};** and **X³** is CH**R^{L}**, wherein the pair of **R^{L}** on different ring carbon atom(s) taken together with the ring atom(s) connecting them form a C₃₋₄ cycloalkyl ring.

In some embodiments of Formula **(V)** (e.g., Formula **(V-a)** or **(V-b)), X¹** is CH**R^{L}**; and **X³** is C(**R^{L}**)₂, wherein the pair of **R^{L}** on different ring carbon atom(s) taken together with the ring atom(s) connecting them form a C₃₋₄ cycloalkyl ring, and the remaining **R^{L}** is C₁₋₂ alkyl optionally substituted with 1-3 F.

In some embodiments, the compounds of Formula (V) are compounds of Formula (V-**c)** or Formula **(V-d):** 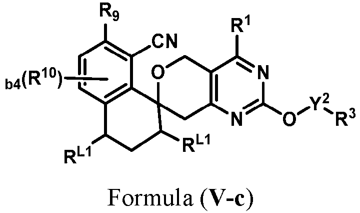 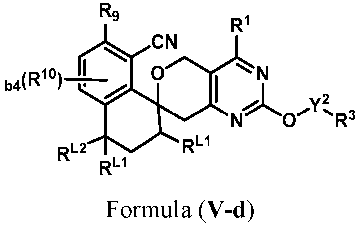 or a pharmaceutically acceptable salt thereof, wherein:
**b4** is 0 or 1; and
**R¹⁰** is selected from the group consisting of: -Cl, -F, -CN, and C₁₋₃ alkyl optionally substituted with 1-3 **R^{c}**;
the pair of **R^{L1}** taken together with the ring atom(s) connecting them form a C₃₋₄ cycloalkyl ring, and
**R^{L2}** is C₁₋₂ alkyl optionally substituted with 1-3 F.

In some embodiments of Formula **(V-c)** or **(V-d), R⁹** is -N**R^{d}R^{e}**. For example, **R⁹** can be -NH₂.

In some embodiments of Formula **(V-d), b4** is 1; and **R¹⁰** is *ortho* to **X³.**

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), Y²** is -CH₂-; and **R³** is a 4-10 membered heterocyclyl having one ring nitrogen atom and 0-1 additional ring heteroatom selected from the group consisting of oxygen and nitrogen, wherein the heterocyclyl is optionally substituted with 1-6 **R^{a}**.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), Y²** is -CH₂-; and **R³** is 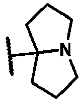 optionally substituted with 1-2 **R^{a}** (e.g., optionally substituted with 1-2 -F).

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), Y²** is -CH₂-; and **R³** is 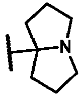 optionally substituted with 1-2 substituents each independently selected from the group consisting of: -F, -C₁₋₃ alkoxy, and - C₁₋₃ haloalkoxy (e.g., **R³** is 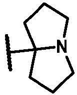 optionally substituted with 1-2 -F). For example, **R³** can be 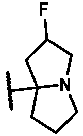 (e.g., 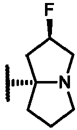 ). For example, **R³** can be 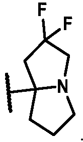 For example, **R³** can be

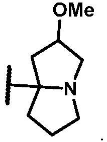

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)),** or Formula **(V)** (e.g., Formula **(V-a)** or **(V-b)), R³** is 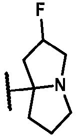 (e.g.,

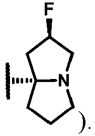

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), Y²** is CH₂; and **R³** is 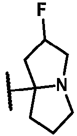 (e.g., 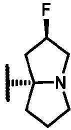 ).

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is 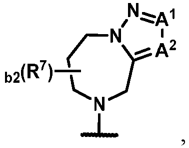 wherein **b2** is 0, 1, or 2, and **A¹** and **A²** are independently selected from the group consisting of: N, CH, and C**R⁷**.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is 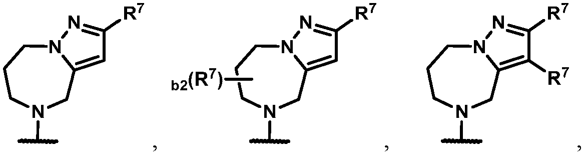 or 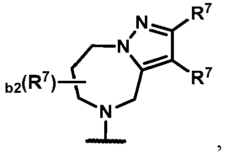 wherein **b2** is 1 or 2.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is

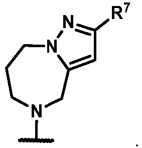

In some embodiments of Formula **(II)** (e.g., Formula **(II-a)** or **(II-b)),** Formula (III), Formula **(IV)** (e.g., Formula **(IV-a), (IV-b),** or **(IV-c)),** or Formula **(V)** (e.g., Formula **(V-a), (V-b), (V-c),** or **(V-d)), R¹** is 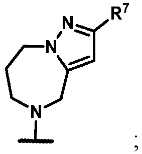 and **R⁷** is selected from the group consisting of: C(=O)N(**R^{f}**)₂, C(=O)N(C₁₋₃ alkyl)**R^{b1}**, -C(=O)N(H)**R^{b1}**, **R^{b1}**, and C(=O)**R^{b1}**.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R⁷** is selected from the group consisting of:
**(a)** C(=O)N(**R^{f}**)₂, wherein each **R^{f}** is independently H or C₁₋₃ alkyl optionally substituted with 1-3 **R^{h}**;
**(b)** C(=O)N(C₁₋₃ alkyl)**R^{b1}** or C(=O)N(H)**R^{b1}**, wherein: **R^{b1}** is a C₃₋₆ cycloalkyl or 4-6 membered heterocyclyl, each of which is optionally substituted with 1-3 **R^{g}**; and
(**c**) C(=O)**R^{b1}**, wherein **R^{b1}** is a 4-10 membered heterocyclyl optionally substituted with 1-3 **R^{g}**, wherein **R^{b1}** is attached to the C(=O) via a ring nitrogen atom. In some embodiments, **R⁷** is C(=O)N(**R^{f}**)₂, wherein each **R^{f}** is independently H or C₁₋₃ alkyl optionally substituted with 1-3 **R^{h}**. In some embodiments, **R⁷** is C(=O)N(**R^{f}**)₂, wherein each **R^{f}** is independently selected C₁₋₃ alkyl (e.g., **R⁷** can be C(=O)N(Me)₂). In some embodiments, **R⁷** is **R^{b1}**, wherein the **R^{b1}** is a 5-6 membered heteroaryl or 4-6 membered heterocyclyl, each of which is optionally substituted with 1-2 **R^{g}**.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is 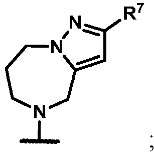 and **R⁷** is **R^{b1}**, wherein the **R^{b1}** is a 5-6 membered heteroaryl optionally substituted with 1-2 **R^{g}**.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is 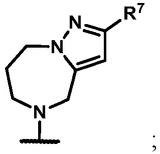 and **R⁷** is **R^{b1}**,wherein the **R^{b1}** is oxetanyl optionally substituted with **R^{g}**.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is 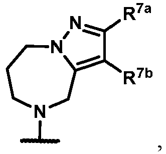 wherein **R^{7a}** and **R^{7b}** are independently selected **R⁷**.

In some embodiments, **R^{7a}** is selected from the group consisting of: C(=O)N(**R^{f}**)₂, C(=O)N(C₁₋₃ alkyl)**R^{b1}**, C(=O)N(H)**R^{b1}**, **R^{b1}**, and C(=O)**R^{b1}**; and
**R^{7b}** is -halo, -CN, or C₁₋₃ alkyl optionally substituted with 1-3 **R^{c}**.

In some embodiments, **R^{7a}** is selected from the group consisting of:
**(a)** C(=O)N(**R^{f}**)₂, wherein each **R^{f}** is independently H or C₁₋₃ alkyl optionally substituted with 1-3 **R^{h}**;
**(b)** C(=O)N(C₁₋₃ alkyl)**R^{b1}** or -C(=O)N(H)**R^{b1}**, wherein: **R^{b1}** is a C₃₋₆ cycloalkyl or 4-6 membered heterocyclyl, each of which is optionally substituted with 1-3 **R^{g}**; and
(**c**) C(=O)**R^{b1}**, wherein **R^{b1}** is a 4-10 membered heterocyclyl optionally substituted with 1-3 **R^{g}**, wherein **R^{b1}** is attached to the C(=O) via a ring nitrogen atom.

In some embodiments, **R^{7a}** is **R^{b1}**, wherein the **R^{b1}** is a 5-6 membered heteroaryl or 4-6 membered heterocyclyl, each of which is optionally substituted with 1-2 **R^{g}**.

In some embodiments, **R^{7a}** is **R^{b1}**, wherein the **R^{b1}** is a 5-6 membered heteroaryl optionally substituted with 1-2 **R^{g}**. In some embodiments, **R^{7a}** is **R^{b1}**, wherein the **R^{b1}** is oxetanyl optionally substituted with **R^{g}**.

In some embodiments, **R^{7b}** is selected from the group consisting of: -halo, -CN, and C₁₋₃ alkyl optionally substituted with 1-3 -F.

In some embodiments, **R^{7b}** is halo (e.g., -Cl).

In some embodiments, **R^{7a}** is -C(=O)N(**R^{f}**)₂, wherein each **R^{f}** is independently H or C₁₋₃ alkyl optionally substituted with 1-3 **R^{h}** (e.g., each **R^{f}** present on **R^{7a}** is an independently selected C₁₋₃ alkyl) (e.g., **R^{7a}** is C(=O)N(Me)₂); and **R^{7b}** is -Cl, -F, or methyl. For example, **R^{7a}** can be C(=O)N(Me)₂; and **R^{7b}** can be -Cl. For example, **R^{7a}** can be C(=O)N(Me)₂; and **R^{7b}** can be -F. For example, **R^{7a}** can be C(=O)N(Me)₂; and **R^{7b}** can be methyl.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is 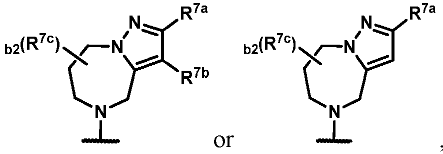 wherein **b2** is 1 or 2; and each **R^{7a}**, **R^{7b}**, and **R^{7c}** is an independently selected **R⁷**. In some embodiments, **R^{7a}** is C(=O)N(**R^{f}**)₂, wherein each **R^{f}** is independently H or C₁₋₃ alkyl optionally substituted with 1-3 **R^{h}**; and **R^{7b}** is -halo, -CN, or C₁₋₃ alkyl optionally substituted with 1-3 **R^{c}**.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is 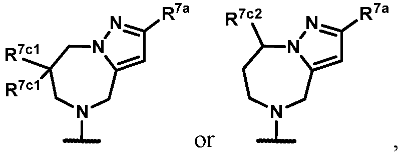 wherein **R^{7a}** is selected from the group consisting of: C(=O)N(**R^{f}**)₂, C(=O)N(C₁₋₃ alkyl)**R^{b1}**, C(=O)N(H)**R^{b1}**, **R^{b1}**, and C(=O)**R^{b1}**;
one **R**^{**7c**1} is -CN, and the other **R^{7c1}** is C₁₋₃ alkyl optionally substituted with 1-3 **R^{c}**; and
**R^{7c2}** is C₁₋₃ alkyl substituted with CN and further optionally substituted with 1-3 **R^{c}**. In some embodiments, **R^{7a}** is C(=O)N(**R^{f}**)₂, wherein each **R^{f}** is independently H or C₁₋₃ alkyl optionally substituted with 1-3 **R^{h}**; one **R^{7c1}** is -CN, and the other **R^{7c1}** is C₁₋₃ alkyl optionally substituted with 1-3 F; and **R^{7c2}** is C₁₋₃ alkyl substituted with CN (e.g., CH₂CN).

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is a 4-10 membered heterocyclyl optionally substituted with 1-4 **R⁷**. In some embodiments, **R¹** is a 4-5 membered heterocyclyl optionally substituted with 1-4 **R⁷**. In some embodiments, **R¹** is a spirocyclic bicyclic 7-10 (e.g., 8-10) membered heterocyclyl optionally substituted with 1-4 **R⁷**.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)),** each **R⁷** is independently selected from the group consisting of: halo; cyano; -OH; oxo; -C₁₋₆ alkoxy; C(=O)N(**R^{f}**)₂; **R^{b1}**; -(C₁₋₃ alkylene)-**R^{b1}**; -O-**R^{b1}**; and C₁₋₆ alkyl optionally substituted with 1-3 **R^{c7}**, wherein:
each **R^{b1}** is independently selected from the group consisting of: C₃₋₆ cycloalkyl, phenyl, 4-6 membered heterocyclyl, and 5-6 membered heteroaryl, each of which is optionally substituted with 1-3 **R^{g}** (e.g., each **R^{g}** can be independently selected from the group consisting of: halo and C₁₋₃ alkyl); and
each **R^{c7}** is independently selected from the group consisting of: halo, cyano, -OH, and -C₁₋₆ alkoxy.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is a 4-10 membered heterocyclyl optionally substituted with 1-4 **R⁷**; and each **R⁷** is independently selected from the group consisting of: halo; cyano; -OH; oxo; - C₁₋₆ alkoxy; C(=O)N(**R^{f}**)₂; **R^{b1}**; -(C₁₋₃ alkylene)-**R^{b1}**; -O-**R^{b1}**; and C₁₋₆ alkyl optionally substituted with 1-3 **R^{c7}**, wherein: each **R^{b1}** is independently selected from the group consisting of: C₃₋₆ cycloalkyl, phenyl, 4-6 membered heterocyclyl, and 5-6 membered heteroaryl, each of which is optionally substituted with 1-3 **R^{g}** (e.g., each **R^{g}** can be independently selected from the group consisting of: halo and C₁₋₃ alkyl); and each **R^{c7}** is independently selected from the group consisting of: halo, cyano, -OH, and -C₁₋₆ alkoxy. In some embodiments, at least one **R⁷** is C₁₋₃ alkyl substituted with -OH.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is a 7-10 (e.g., 7) membered heterocyclyl having one ring nitrogen atom, one ring oxygen atom, and no additional ring heteroatoms, wherein the 7-10 membered heterocyclyl is optionally substituted with 1-4 **R⁷**.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is a 7-10 (e.g., 7) membered monocyclic heterocyclyl having one ring nitrogen atom, one ring oxygen atom, and no additional ring heteroatoms, wherein the 7-10 membered monocyclic heterocyclyl is optionally substituted with 1-4 (e.g., 1-2) **R⁷**.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula (V) (e.g., Formula **(V-a), (V-b), (V-c),** or (V-d)), **R¹** is 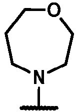 optionally substituted with 1-4 **R⁷** at one or more ring carbon atoms. For example, **R¹** can be 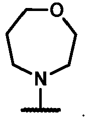 For example, **R¹** can be

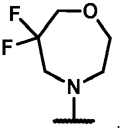

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)),** each **R⁷** is independently selected from the group consisting of: -OH; -CN; -F; and C₁₋₃ alkyl optionally substituted with 1-3 **R^{c}**, wherein each **R^{c}** is independently selected from the group consisting of: -F, -OH, and -CN.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is 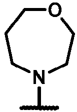 optionally substituted with 1-4 **R⁷** at one or more ring carbon atoms, wherein each **R⁷** is independently selected from the group consisting of: -OH; - CN; -F; and C₁₋₃ alkyl optionally substituted with 1-3 **R^{c}**, wherein each **R^{c}** is independently selected from the group consisting of: -F, -OH, and -CN.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is 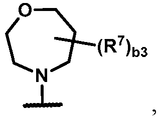 wherein **b3** is 1, 2, or 3.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is 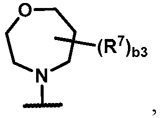 wherein **b3** is 1, 2, or 3; and one occurrence of **R⁷** is **R^{b}** (e.g., one occurrence of **R⁷** is **R^{b1}**). In some embodiments, the one occurrence of **R⁷** is a 5-6 membered heteroaryl optionally substituted with 1-3 **R^{g}**. In some embodiments, the one occurrence of **R⁷** is a 5-membered heteroaryl optionally substituted with 1-3 **R^{g}**. In some embodiments, the one occurrence of **R⁷** is selected from the group consisting of pyrazolyl and oxazolyl, each of which is optionally substituted with 1-2 **R^{g}** (e.g., the one occurrence of **R⁷** is 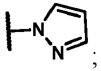 or the one occurrence of **R⁷** is 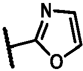 ).

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is 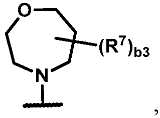 and **b3** is 1. In some embodiments, **R⁷** is a 5-6 membered heteroaryl optionally substituted with 1-3 **R^{g}**. In some embodiments, **R⁷** is a 5-membered heteroaryl optionally substituted with 1-3 **R^{g}**. In some embodiments, **R⁷** is selected from the group consisting of pyrazolyl and oxazolyl, each of which is optionally substituted with 1-2 **R^{g}** (e.g., **R⁷** is 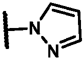 ).

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is 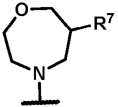 (e.g., 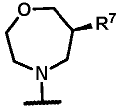 ), wherein **R⁷** is a 5-membered heteroaryl optionally substituted with 1-3 **R^{g}**. In some embodiments, **R⁷** is selected from the group consisting of pyrazolyl and oxazolyl, each of which is optionally substituted with 1-2 **R^{g}**. In some embodiments, **R⁷** is pyrazolyl optionally substituted with 1-2 **R^{g}** (e.g., **R⁷** is 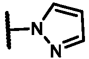 optionally substituted with one **R^{g}**). For example, **R⁷** can be 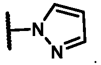 In some embodiments, **R⁷** is oxazolyl optionally substituted with one **R^{g}** (e.g., **R⁷** is 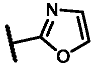 optionally substituted with one **R^{g}**). For example, **R⁷** can be

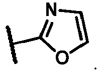

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is a 7-10 (e.g., 7; or e.g., 9) membered bicyclic heterocyclyl having one ring nitrogen atom, one ring oxygen atom, and no additional ring heteroatoms, wherein the 7-10 membered bicyclic heterocyclyl is optionally substituted with 1-4 (e.g., 1-2) **R⁷**.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is a 7-10 (e.g., 7) membered spirocyclic bicyclic heterocyclyl having one ring nitrogen atom, one ring oxygen atom, and no additional ring heteroatoms, wherein the 7-10 membered spirocyclic bicyclic heterocyclyl is optionally substituted with 1-4 (e.g., 1-2) **R⁷**.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is 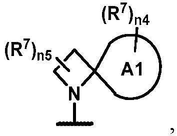 wherein: **Ring A1** is a 4-7 membered heterocyclyl ring having one ring oxygen atom and no additional ring heteroatoms; **n4** is 0, 1, or 2; and **n5** is 0, 1, or 2, provided that **n4** + **n5** is 0, 1, or 2. In some embodiments, **n4** is 0. In some embodiments, **n5** is 0. In some embodiments **n4** is 0; and **n5** is 0. In some embodiments, each **R⁷** is independently selected from the group consisting of: -F, -OH, oxo, -**R^{b1}**, and C₁₋₃ alkyl optionally substituted with 1-3 **R^{c}**, wherein the **R^{b1}** is a 5-6 membered heteroaryl optionally substituted with 1-2 **R^{g}**.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is 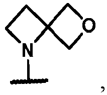 which is optionally substituted with 1-2 **R⁷**.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is

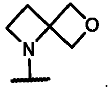

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is 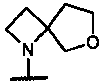 (e.g., 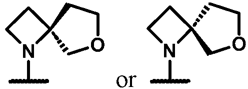 ), which is optionally substituted with 1-2 **R⁷**.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is 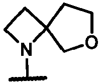 (e.g., 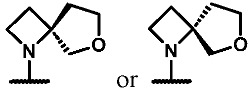 ).

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is 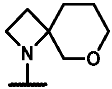 (e.g., 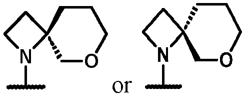 ), which is optionally substituted with 1-2 **R⁷**. In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is 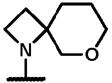 (e.g., 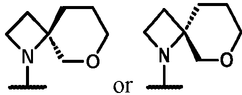 ).

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is a 4-7 membered heterocyclyl (e.g., azetidinyl or pyrrolidinyl) optionally substituted with 1-4 (e.g., 1-2) **R⁷**. In some embodiments, each **R⁷** is independently selected from the group consisting of: halo; cyano; -OH; oxo; -C₁₋₆ alkoxy; C(=O)N(**R^{f}**)₂; **R^{b1}**, -(C₁₋₃ alkylene)-**R^{b1}**; -O-**R^{b1}**; and C₁₋₆ alkyl optionally substituted with 1-3 **R^{c7}**, wherein: each **R^{b1}** is independently selected from the group consisting of: C₃₋₆ cycloalkyl, phenyl, 4-6 membered heterocyclyl, and 5-6 membered heteroaryl, each of which is optionally substituted with 1-3 **R^{g}** (e.g., each **R^{g}** can be independently selected from the group consisting of: halo and C₁₋₃ alkyl); and each **R^{c7}** is independently selected from the group consisting of: halo, cyano, - OH, and -C₁₋₆ alkoxy. In some embodiments, at least one **R⁷** is C₁₋₃ alkyl substituted with -OH.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is a 4-5 membered heterocyclyl (e.g., azetidinyl or pyrrolidinyl) optionally substituted with 1-4 (e.g., 1-2) **R⁷**. In some embodiments, each **R⁷** is independently selected from the group consisting of: halo; cyano; -OH; oxo; -C₁₋₆ alkoxy; C(=O)N(**R^{f}**)₂; **R^{b1}**; -(C₁₋₃ alkylene)-**R^{b1}**; -O-**R^{b1}**; and C₁₋₆ alkyl optionally substituted with 1-3 **R^{c7}**, wherein: each **R^{b1}** is independently selected from the group consisting of: C₃₋₆ cycloalkyl, phenyl, 4-6 membered heterocyclyl, and 5-6 membered heteroaryl, each of which is optionally substituted with 1-3 **R^{g}** (e.g., each **R^{g}** can be independently selected from the group consisting of: halo and C₁₋₃ alkyl); and each **R^{c7}** is independently selected from the group consisting of: halo, cyano, - OH, and -C₁₋₆ alkoxy. In some embodiments, at least one **R⁷** is C₁₋₃ alkyl substituted with -OH.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is a 4-membered heterocyclyl optionally substituted with 1-4 **R⁷**. In some embodiments, each **R⁷** is independently selected from the group consisting of: -F, - OH, oxo, -**R^{b1}**, and C₁₋₃ alkyl optionally substituted with 1-3 **R^{c}**, wherein the **R^{b1}** is a 5-6 membered heteroaryl optionally substituted with 1-2 **R^{g}**. In some embodiments, each **R⁷** is independently selected from the group consisting of: halo; cyano; -OH; oxo; -C₁₋₆ alkoxy; C(=O)N(**R^{f}**)₂; **R^{b1}**; -(C₁₋₃ alkylene)-**R^{b1}**; -O-**R^{b1}**; and C₁₋₆ alkyl optionally substituted with 1-3 **R^{c7}**, wherein: each **R^{b1}** is independently selected from the group consisting of: C₃₋₆ cycloalkyl, phenyl, 4-6 membered heterocyclyl, and 5-6 membered heteroaryl, each of which is optionally substituted with 1-3 **R^{g}** (e.g., each **R^{g}** can be independently selected from the group consisting of: halo and C₁₋₃ alkyl); and each **R^{c7}** is independently selected from the group consisting of: halo, cyano, -OH, and -C₁₋₆ alkoxy. In some embodiments, at least one **R⁷** is C₁₋₃ alkyl substituted with -OH.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is a 4-membered heterocyclyl optionally substituted with 1-4 (e.g., 1-2) **R⁷**. In some embodiments, each **R⁷** is independently selected from the group consisting of: -F, -OH, -**R^{b1}**, and C₁₋₃ alkyl optionally substituted with 1-3 F, wherein the **R^{b1}** is a 5-6 membered heteroaryl optionally substituted with 1-2 **R^{g}**. In some embodiments, each **R⁷** is independently selected from the group consisting of: halo; cyano; -OH; oxo; -C₁₋₆ alkoxy; C(=O)N(**R^{f}**)₂; **R^{b1}**; -(C₁₋₃ alkylene)-**R^{b1}**; -O-**R^{b1}**; and C₁₋₆ alkyl optionally substituted with 1-3 **R^{c7}**, wherein: each **R^{b1}** is independently selected from the group consisting of: C₃₋₆ cycloalkyl, phenyl, 4-6 membered heterocyclyl, and 5-6 membered heteroaryl, each of which is optionally substituted with 1-3 **R^{g}** (e.g., each **R^{g}** can be independently selected from the group consisting of: halo and C₁₋₃ alkyl); and each **R^{c7}** is independently selected from the group consisting of: halo, cyano, -OH, and -C₁₋₆ alkoxy. In some embodiments, at least one **R⁷** is C₁₋₃ alkyl substituted with -OH.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or (V-d)), **R¹** is 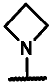 optionally substituted with 1-4 (e.g., 1-2) **R⁷**. In some embodiments, each **R⁷** is independently selected from the group consisting of: -F, oxo, -OH, - **R^{b1}**, and C₁₋₃ alkyl optionally substituted with 1-3 **R^{c}**, wherein the **R^{b1}** is a 5-6 membered heteroaryl optionally substituted with 1-2 **R^{g}**. In some embodiments, each **R⁷** is independently selected from the group consisting of: halo; cyano; -OH; oxo; -C₁₋₆ alkoxy; C(=O)N(**R^{f}**)₂; **R^{b1}**; -(C₁₋₃ alkylene)-**R^{b1}**; -O-**R^{b1}**; and C₁₋₆ alkyl optionally substituted with 1-3 **R^{c7}**, wherein: each **R^{b1}** is independently selected from the group consisting of: C₃₋₆ cycloalkyl, phenyl, 4-6 membered heterocyclyl, and 5-6 membered heteroaryl, each of which is optionally substituted with 1-3 **R^{g}** (e.g., each **R^{g}** can be independently selected from the group consisting of: halo and C₁₋₃ alkyl); and each **R^{c7}** is independently selected from the group consisting of: halo, cyano, - OH, and -C₁₋₆ alkoxy. In some embodiments, at least one **R⁷** is C₁₋₃ alkyl substituted with -OH.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula (V-a), **(V-b), (V-c),** or (V-d)), **R¹** is 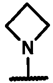 optionally substituted with 1-2 **R⁷**. In some embodiments, each **R⁷** is independently selected from the group consisting of: -F, -OH, -**R^{b1}**, and C₁₋₃ alkyl optionally substituted with 1-3 F, wherein the **R^{b1}** is a 5-6 membered heteroaryl optionally substituted with 1-2 **R^{g}**. In some embodiments, each **R⁷** is independently selected from the group consisting of: halo; cyano; -OH; oxo; -C₁₋₆ alkoxy; C(=O)N(**R^{f}**)₂; **R^{b1}**; -(C₁₋₃ alkylene)-**R^{b1}**; -O-**R^{b1}**; and C₁₋₆ alkyl optionally substituted with 1-3 **R^{c7}**, wherein: each **R^{b1}** is independently selected from the group consisting of: C₃₋₆ cycloalkyl, phenyl, 4-6 membered heterocyclyl, and 5-6 membered heteroaryl, each of which is optionally substituted with 1-3 **R^{g}** (e.g., each **R^{g}** can be independently selected from the group consisting of: halo and C₁₋₃ alkyl); and each **R^{c7}** is independently selected from the group consisting of: halo, cyano, -OH, and -C₁₋₆ alkoxy. In some embodiments, at least one **R⁷** is C₁₋₃ alkyl substituted with -OH.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b),** (V-c), or (V-d)), **R¹** is selected from the group consisting of: 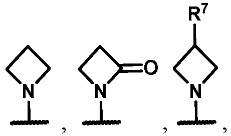 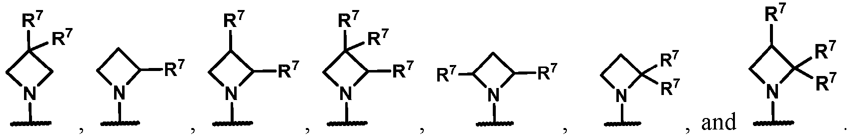 In some embodiments, each **R⁷** is independently selected from the group consisting of: halo; cyano; - OH; oxo; -C₁₋₆ alkoxy; C(=O)N(**R^{f}**)₂; **R^{b1}**; -(C₁₋₃ alkylene)-**R^{b1}**; -O-**R^{b1}**; and C₁₋₆ alkyl optionally substituted with 1-3 **R^{c7}**, wherein: each **R^{b1}** is independently selected from the group consisting of: C₃₋₆ cycloalkyl, phenyl, 4-6 membered heterocyclyl, and 5-6 membered heteroaryl, each of which is optionally substituted with 1-3 **R^{g}** (e.g., each **R^{g}** can be independently selected from the group consisting of: halo and C₁₋₃ alkyl); and each **R^{c7}** is independently selected from the group consisting of: halo, cyano, -OH, and -C₁₋₆ alkoxy. In some embodiments, at least one **R⁷** is C₁₋₃ alkyl substituted with -OH.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is selected from the group consisting of: 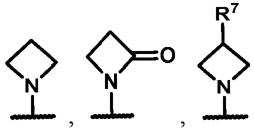 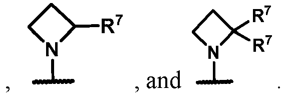 In some embodiments, each **R⁷** is independently selected from the group consisting of: -F; cyano; -OH; -**R^{b1}**, wherein the **R^{b1}** is a 5-6 membered heteroaryl optionally substituted with 1-2 **R^{g}**; C₁₋₃ alkyl optionally substituted with 1-3 F; C₁₋₃ alkyl substituted with -OH or C₁₋₃ alkoxy; and C(=O)N(**R^{f}**)₂. In some embodiments, each **R⁷** is independently selected from the group consisting of: halo; cyano; -OH; oxo; -C₁₋₆ alkoxy; C(=O)N(**R^{f}**)₂; **R^{b1}**; -(C₁₋₃ alkylene)-**R^{b1}**; -O-**R^{b1}**; and C₁₋₆ alkyl optionally substituted with 1-3 **R^{c7}**, wherein: each **R^{b1}** is independently selected from the group consisting of: C₃₋₆ cycloalkyl, phenyl, 4-6 membered heterocyclyl, and 5-6 membered heteroaryl, each of which is optionally substituted with 1-3 **R^{g}** (e.g., each **R^{g}** can be independently selected from the group consisting of: halo and C₁₋₃ alkyl); and each **R^{c7}** is independently selected from the group consisting of: halo, cyano, -OH, and -C₁₋₆ alkoxy. In some embodiments, at least one **R⁷** is C₁₋₃ alkyl substituted with -OH.

In some embodiments of Formula **(II)** (e.g., Formula **(II-a)** or **(II-b)),** Formula **(III),** Formula **(IV)** (e.g., Formula **(IV-a), (IV-b),** or **(IV-c)),** or Formula **(V)** (e.g., Formula **(V-a), (V-b), (V-c),** or **(V-d)), R¹** is selected from the group consisting of: 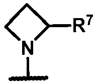 (e.g., 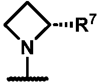 ), 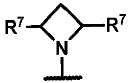 (e.g., 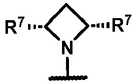 ), or 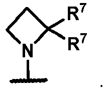 In some embodiments, each **R⁷** is independently selected from the group consisting of: -F; cyano; -OH; -**R^{b1}**, wherein the **R^{b1}** is a 5-6 membered heteroaryl optionally substituted with 1-2 **R^{g}**; C₁₋₃ alkyl optionally substituted with 1-3 F; C₁₋₃ alkyl substituted with -OH or C₁₋₃ alkoxy; and C(=O)N(**R^{f}**)₂. In some embodiments, each **R⁷** is independently selected from the group consisting of: halo; cyano;-OH; oxo; -C₁₋₆ alkoxy; C(=O)N(**R^{f}**)₂; **R^{b1}**; -(C₁₋₃ alkylene)-**R^{b1}**; -O-**R^{b1}**; and C₁₋₆ alkyl optionally substituted with 1-3 **R^{c7}**, wherein: each **R^{b1}** is independently selected from the group consisting of: C₃₋₆ cycloalkyl, phenyl, 4-6 membered heterocyclyl, and 5-6 membered heteroaryl, each of which is optionally substituted with 1-3 **R^{g}** (e.g., each **R^{g}** can be independently selected from the group consisting of: halo and C₁₋₃ alkyl); and each **R^{c7}** is independently selected from the group consisting of: halo, cyano, -OH, and -C₁₋₆ alkoxy. In some embodiments, at least one **R⁷** is C₁₋₃ alkyl substituted with -OH.

In some embodiments of Formula (**II**) (e.g., Formula (**II-a**) or (**II-b**)), Formula (**III**), Formula (**IV**) (e.g., Formula (**IV-a**), (**IV-b**), or (**IV-c**)), or Formula (**V**) (e.g., Formula (**V-a**), (**V-b**), (**V-c**), or (**V-d**)), **R¹** is selected from the group consisting of: 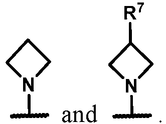 In some embodiments, each **R⁷** is independently selected from the group consisting of: -F, -OH, -**R^{b1}**, and C₁₋₃ alkyl optionally substituted with 1-3 F, wherein the **R^{b1}** is a 5-6 membered heteroaryl optionally substituted with 1-2 **R^{g}.**

In some embodiments of Formula (**II**) (e.g., Formula (**II-a**) or (**II-b**)), Formula (**III**), Formula (**IV**) (e.g., Formula (**IV-a**), (**IV-b**), or (**IV-c**)), or Formula (**V**) (e.g., Formula (**V-a**), (**V-b**), (**V-c**), or (**V-d**)), **R¹** is 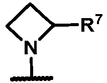 (e.g., 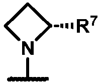 ). In some embodiments, each **R⁷** is independently selected from the group consisting of: -F; cyano; -OH; -**R^{b1}**, wherein the **R^{b1}** is a 5-6 membered heteroaryl optionally substituted with 1-2 **R^{g}**; C₁₋₃ alkyl optionally substituted with 1-3 F; C₁₋₃ alkyl substituted with -OH or C₁₋₃ alkoxy; and C(=O)N(**R^{f}**)₂.

In some embodiments of Formula (**II**) (e.g., Formula (**II-a**) or (**II-b**)), Formula (**III**), Formula (**IV**) (e.g., Formula (**IV-a**), (**IV-b**), or (**IV-c**)), or Formula (**V**) (e.g., Formula (**V-a**), (**V-b**), (**V-c**), or (**V-d**)), **R¹** is 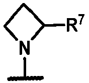 (e.g., 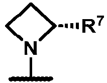 ), wherein **R⁷** is C₁₋₃ alkyl substituted with -OH or C₁₋₃ alkoxy (e.g., C₁₋₃ alkyl substituted with -OH).

In some embodiments of Formula (**II**) (e.g., Formula (**II-a**) or (**II-b**)), Formula (**III**), Formula (**IV**) (e.g., Formula (**IV-a**), (**IV-b**), or (**IV-c**)), or Formula (**V**) (e.g., Formula (**V-a**), (**V-b**), (**V-c**), or (**V-d**)), **R¹** is 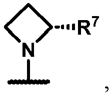 wherein **R⁷** is C₁₋₃ alkyl substituted with -OH.

In some embodiments of Formula (**II**) (e.g., Formula (**II-a**) or (**II-b**)), Formula (**III**), Formula (**IV**) (e.g., Formula (**IV-a**), (**IV-b**), or (**IV-c**)), or Formula (**V**) (e.g., Formula (**V-a**), **(V-b), (V-c),** or (**V-d**)), **R¹** is 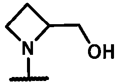 (e.g., 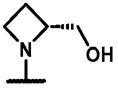 ).

In some embodiments of Formula (**II**) (e.g., Formula (**II-a**) or (**II-b**)), Formula (**III**), Formula (**IV**) (e.g., Formula (**IV-a**), (**IV-b**), or (**IV-c**)), or Formula (**V**) (e.g., Formula (**V-a**), (**V-b**), (**V-c**), or (**V-d**)), **R¹** is 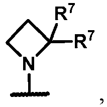 wherein each **R⁷** is independently selected from the group consisting of: C₁₋₃ alkyl optionally substituted with 1-3 F; and C₁₋₃ alkyl substituted with -OH or C₁₋₃ alkoxy.

In some embodiments of Formula (II) (e.g., Formula (II-a) or (II-b)), Formula (III), Formula (IV) (e.g., Formula (IV-a), (IV-b), or (IV-c)), or Formula (V) (e.g., Formula (V-a), **(V-b), (V-c),** or (V-d)), R¹ is 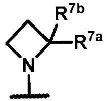 (e.g., 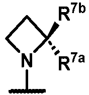 ), wherein **R^{7a}** is C₁₋₃ alkyl substituted with -OH (e.g., -CH₂OH); and R^{7b} is selected from the group consisting of: C₁₋₃ alkyl optionally substituted with 1-3 F (e.g., methyl); and C₁₋₃ alkyl substituted with -OH or C₁₋₃ alkoxy. For example, **R¹** can be 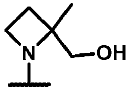 (e.g., 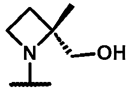 ). For example, **R¹** can be

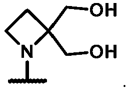

In some embodiments of Formula (**II**) (e.g., Formula (**II-a**) or (**II-b**)), Formula (**III**), Formula (**IV**) (e.g., Formula (**IV-a**), (**IV-b**), or (**IV-c**)), or Formula (**V**) (e.g., Formula (**V-a**), (**V-b**), (**V-c**), or (**V-d**)), **R¹** is 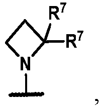 wherein each **R⁷** is independently C₁₋₃ alkyl optionally substituted with -OH or C₁₋₃ alkoxy (e.g., each **R⁷** is independently C₁₋₃ alkyl substituted with -OH (e.g., each **R⁷** is -CH₂OH)). For example, **R¹** can be

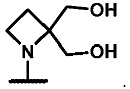

In some embodiments of Formula (**II**) (e.g., Formula (**II-a**) or (**II-b**)), Formula (**III**), Formula (**IV**) (e.g., Formula (**IV-a**), (**IV-b**), or (**IV-c**)), or Formula (**V**) (e.g., Formula (**V-a**), (**V-b**), (**V-c**), or (**V-d**)), **R¹** is 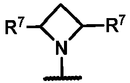 (e.g., 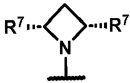 ), wherein each **R⁷** is independently selected from the group consisting of: C₁₋₃ alkyl optionally substituted with 1-3 F; and C₁₋₃ alkyl substituted with -OH or C₁₋₃ alkoxy.

In some embodiments of Formula (**II**) (e.g., Formula (**II-a**) or (**II-b**)), Formula (**III**), Formula (**IV**) (e.g., Formula (**IV-a**), (**IV-b**), or (**IV-c**)), or Formula (**V**) (e.g., Formula (**V-a**), (**V-b**), (**V-c**), or (**V-d**)), **R¹** is 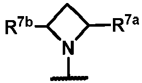 (e.g., 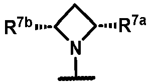 ), wherein **R^{7a}** is C₁₋₃ alkyl substituted with -OH (e.g., -CH₂OH); and **R^{7b}** is selected from the group consisting of: C₁₋₃ alkyl optionally substituted with 1-3 F (e.g., methyl); and C₁₋₃ alkyl substituted with -OH or C₁₋₃ alkoxy.

In some embodiments of Formula (**II**) (e.g., Formula (**II-a**) or (**II-b**)), Formula (**III**), Formula (**IV**) (e.g., Formula (**IV-a**), (**IV-b**), or (**IV-c**)), or Formula (**V**) (e.g., Formula (**V-a**), (**V-b**), (**V-c**), or (**V-d**)), **R¹** is a 4-10 (e.g., 4-6) membered heterocyclyl having one ring oxygen atom and no additional ring heteroatoms, wherein the 4-10 (e.g., 4-6) membered heterocyclyl is optionally substituted with 1-4 **R⁷.** In some embodiments, each **R⁷** is independently selected from the group consisting of: -OH; -CN; -F; and C₁₋₃ alkyl optionally substituted with 1-3 **R^{c},** wherein each **R^{c}** is independently selected from the group consisting of: -F, -OH, and -CN. In some embodiments, **R¹** is selected from the group consisting of:

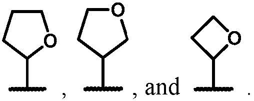

In some embodiments, the compounds of Formula (**II**) are compounds of Formula (**II-a1**) or (**II-b1**): 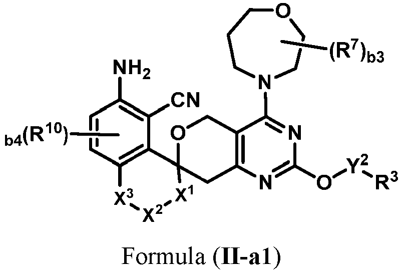 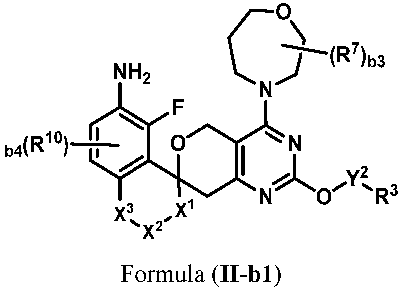 or pharmaceutically acceptable salts thereof, wherein:
**b4** is 0 or 1;
**R¹⁰** is selected from the group consisting of: -Cl, -F, -CN, and C₁₋₃ alkyl optionally substituted with 1-3 **R^{c}**;
**b3** is 0, 1, 2, or 3;
**X¹** is CH₂; and
**X²** and **X³** are independently selected from the group consisting of: O, CH₂, CH**R^{L}**, and C(**R^{L}**)₂.

In some embodiments of Formula (**II**-**a1**) or (**II-b1**), **b4** is 0.

In some embodiments of Formula (**II-a1**) or (**II-b1**), **b4** is 1; and **R¹⁰** is *ortho* to **X³**.

In some embodiments, the compounds of Formula (**III**) are compounds of compound of Formula (**III-1**): 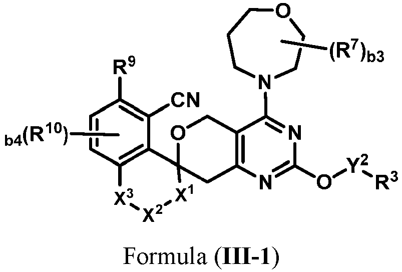 or pharmaceutically acceptable salts thereof, wherein:
**b4** is 0 or 1;
**R¹⁰** is selected from the group consisting of: -Cl, -F, -CN, and C₁₋₃ alkyl optionally substituted with 1-3 **R^{c};**
**b3** is 0, 1, 2, or 3;
**X¹** is CH₂; and
**X²** and **X³** are independently selected from the group consisting of: O, CH₂, CH**R^{L}**, and C(**R^{L}**)₂.

In some embodiments of Formula (**III-1**), **R⁹** is N**R^{d}R^{e}** (e.g., -NH₂).

In some embodiments of Formula (**III-1**), **b4** is 0.

In some embodiments of Formula (**III-1**), **b4** is 1; and **R¹⁰** is *ortho* to **X³**.

In some embodiments, the compounds of Formula (**IV**) are compounds of Formula (**IV-a1**) or (**IV-b1**): 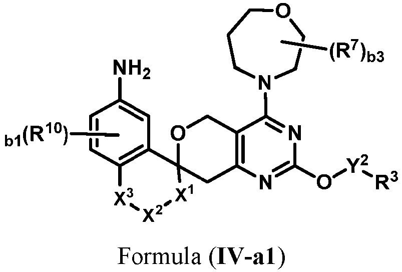 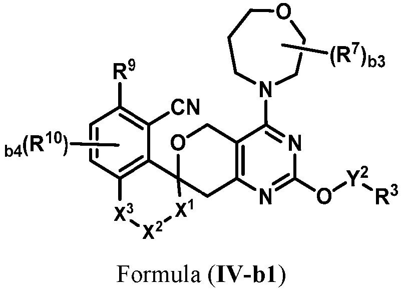 or pharmaceutically acceptable salts thereof, wherein:
**b4** is 0 or 1;
**b1** is 0, 1, or 2;
each **R¹⁰** is independently selected from the group consisting of: -Cl, -F, -CN, and C₁₋₃ alkyl optionally substituted with 1-3 **R^{c}**;
**b3** is 0, 1, 2, or 3;
**X¹** is CH₂;
one of **X²** and **X³** is independently selected from the group consisting of: CH**R^{L}** and C(**R^{L}**)₂; and
the other of **X²** and **X³** is CH₂ or O.

In some embodiments of Formula (**IV-a1**), **b1** is 1 or 2; and the 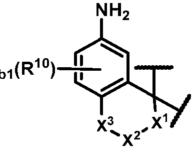 moiety is

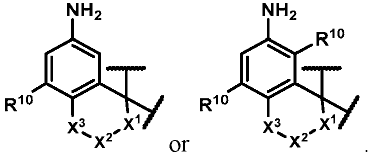

In some embodiments of Formula (**IV-b1**), **b4** is 1; and **R¹⁰** is *ortho* to **X³**.

In some embodiments of Formula (**IV-b1**), **b4** is 0.

In some embodiments of Formula (**IV-b1**), **R⁹** is N**R^{d}R^{e}** (e.g., -NH₂).

In some embodiments of Formula (**IV-a1**) or (**IV-b1**), **X²** is CH₂; and **X³** is CH**R^{L}**. In some embodiments, **R^{L}** is CF₃. In some embodiments, **R^{L}** is CH₃.

In some embodiments, the compounds of Formula (**V**) are compounds of Formula (**V-a1**) or (**V-b1**): 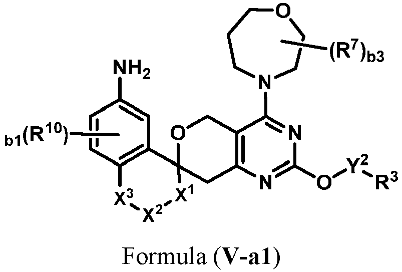 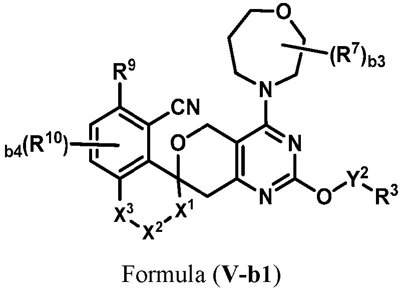 or pharmaceutically acceptable salts thereof, wherein:
**b4** is 0 or 1;
**b1** is 0, 1, or 2;
each **R¹⁰** is independently selected from the group consisting of: -Cl, -F, -CN, and C₁₋₃ alkyl optionally substituted with 1-3 **R^{c}**;
**b3** is 0, 1, 2, or 3;
**X²** is -O- or -CH₂-;
**X¹** is CH**R^{L}**; and **X³** is CH**R^{L}** or C(**R^{L}**)₂, wherein:
   one pair of **R^{L}** on different ring carbon atom(s) taken together with the ring atom(s) connecting them form a C₃₋₄ cycloalkyl ring; and
   the remaining **R^{L}** if present is C₁₋₂ alkyl optionally substituted with 1-3 F.

In some embodiments of Formula (**V-a1**), **b1** is 1 or 2; and the 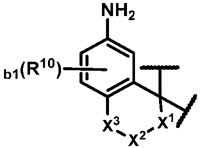 moiety is

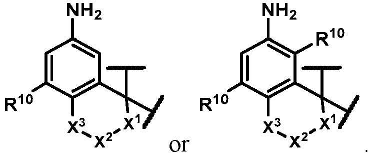

In some embodiments of Formula (**V-b1**), b4 is 0.

In some embodiments of Formula (**V-b1**), b4 is 1; and **R¹⁰** is *ortho* to **X³**.

In some embodiments of Formula (**V-b1**), **R⁹** is N**R^{d}R^{e}** (e.g., -NH₂).

In some embodiments of Formula (**II-a1**), (**II-b1**), (**III-1**), (**IV-a1**), (**IV-b1**), (**V-a1**), or (**V-b1**), **b3** is 0.

In some embodiments of Formula (**II-a1**), (**II-b1**), (**III-1**), (**IV-a1**), (**IV-b1**), (**V-a1**), or (**V-b1**), **b3** is 1 or 2; and each **R⁷** is independently selected from the group consisting of: -OH; -CN; -F; and C₁₋₃ alkyl optionally substituted with 1-3 **R^{c}**, wherein each **R^{c}** is independently selected from the group consisting of: -F, -OH, and -CN.

In some embodiments of Formula (**II-a1**), (**II-b1**), (**III-1**), (**IV-a1**), (**IV-b1**), (**V-a1**), or (**V-b1**), one occurrence of **R⁷** is **R^{b}**; and each remaining **R⁷** is an independently selected **R^{a}**. In some embodiments, one occurrence of **R⁷** is a 5-membered heteroaryl optionally substituted with 1-3 **R^{g}**; and each remaining **R⁷** is independently selected from the group consisting of: - OH; -CN; -F; and C₁₋₃ alkyl optionally substituted with 1-3 **R^{c}**, wherein each **R^{c}** is independently selected from the group consisting of: -F, -OH, and -CN.

In some embodiments of Formula (**II-a1**), (**II-b1**), (**III-1**), (**IV-a1**), (**IV-b1**), (**V-a1**), or **(V-b1), b3** is 1; and the 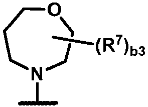 moiety is 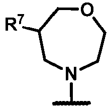 (e.g., 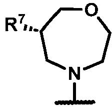 ), wherein **R⁷** is a 5-membered heteroaryl optionally substituted with 1-3 **R^{g}**.

In some embodiments of Formula (**II-a1**), (**II-b1**), (**III-1**), (**IV-a1**), (**IV-b1**), (**V-a1**), or **(V-b1), b3** is 1; and the 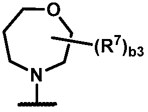 moiety is 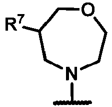 (e.g., 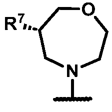 ), wherein **R⁷** is pyrazolyl optionally substituted with 1-2 **R^{g}** (e.g., **R⁷** is 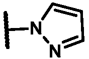 optionally substituted with one **R^{g}**). For example, **R⁷** can be

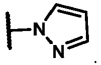

In some embodiments, the compounds of Formula (**II**) are compounds of Formula (**II-a2**) or (**II-b2**): 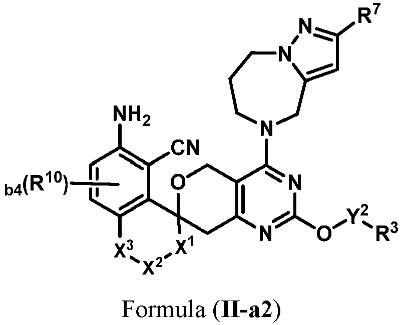 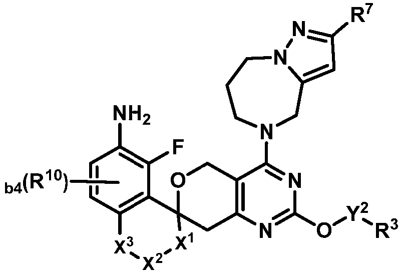 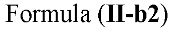 or pharmaceutically acceptable salts thereof, wherein:
**b4** is 0 or 1;
**R¹⁰** is selected from the group consisting of: -Cl, -F, -CN, and C₁₋₃ alkyl optionally substituted with 1-3 **R^{c}**;
**R⁷** is selected from the group consisting of: C(=O)N(**R^{f}**)₂, C(=O)N(C₁₋₃ alkyl)**R^{b1}**, - C(=O)N(R)**R^{b1}**, **R^{b1}**, and C(=O)**R^{b1};**
**X¹** is CH₂; and
**X²** and **X³** are independently selected from the group consisting of: O, CH₂, CH**R^{L}**, and C(**R^{L}**)₂.

In some embodiments of Formula (**II-a2**) or (**II-b2**), **b4** is 0.

In some embodiments of Formula (**II-a2**) or (**II-b2**), **b4** is 1; and **R¹⁰** is *ortho* to **X³**.

In some embodiments, the compounds of Formula (**III**) are compounds of Formula (**III-2**): 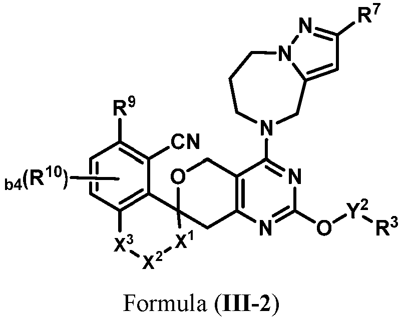 or pharmaceutically acceptable salts thereof, wherein:
**b4** is 0 or 1;
**R¹⁰** is selected from the group consisting of: -Cl, -F, -CN, and C₁₋₃ alkyl optionally substituted with 1-3 **R^{c}**;
**R⁷** is selected from the group consisting of: C(=O)N(**R^{f}**)₂, C(=O)N(C₁₋₃ alkyl)**R^{b1}**, - C(=O)N(R)**R^{b1}**, **R^{b1}**, and C(=O)**R^{b1}**;
**X¹** is CH₂; and
**X²** and **X³** are independently selected from the group consisting of: O, CH₂, CH**R^{L}**, and C(**R^{L}**)₂.

In some embodiments of Formula (**III-2**)**, R⁹** is N**R^{d}R^{e}** (e.g., -NH₂).

In some embodiments of Formula (**III-2**), **b4** is 0.

In some embodiments of Formula (**III-2**), **b4** is 1; and **R¹⁰** is *ortho* to **X³**.

In some embodiments, the compounds of Formula (**IV**) are compounds of compound of Formula (**IV-a2**) or (**IV-b2**): 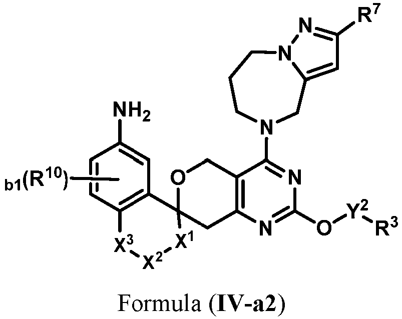 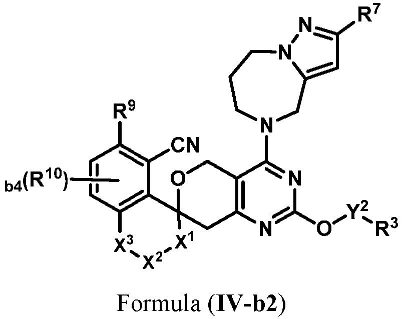 or pharmaceutically acceptable salts thereof, wherein:
**b4** is 0 or 1;
**b1** is 0, 1, or 2;
each **R¹⁰** is independently selected from the group consisting of: -Cl, -F, -CN, and C₁₋₃ alkyl optionally substituted with 1-3 **R^{c}**;
**R⁷** is selected from the group consisting of: C(=O)N(**R^{f}**)₂, C(=O)N(C₁₋₃ alkyl)**R^{b1}**, - C(=O)N(H)**R^{b1}**, **R^{b1}**, and C(=O)**R^{b1}**;
**X¹** is CH₂;
one of **X²** and **X³** is independently selected from the group consisting of: CH**R^{L}** and C(**R^{L}**)₂; and
the other of **X²** and **X³** is CH₂ or O.

In some embodiments of Formula (**IV-a2**) or (**IV-b2**), **X²** is CH₂; and **X³** is CH**R^{L}**. In some embodiments, **R^{L}** is CF₃. In some embodiments, **R^{L}** is CH₃.

In some embodiments of Formula (**IV-a2**), **b1** is 1 or 2; and the 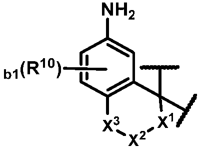 moiety is

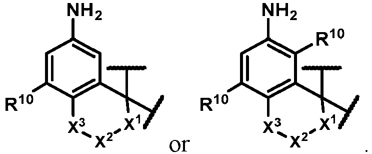

In some embodiments of Formula (**IV-b2**), **b4** is 0.

In some embodiments of Formula (**IV-b2**), **b4** is 1; and **R¹⁰** is *ortho* to **X³**.

In some embodiments of Formula (**IV-b2**), **R⁹** is -N**R^{d}R^{e}**. For example, **R⁹** can be - NH₂.

In some embodiments, the compounds of Formula (V) are compounds of Formula (**V-a2**) or (**V-b2**): 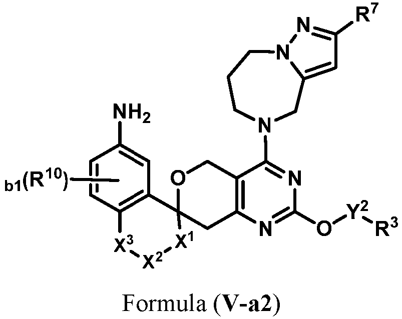 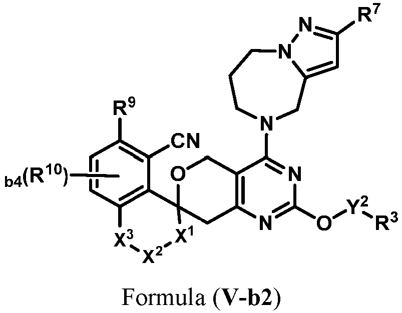 or pharmaceutically acceptable salts thereof, wherein:
**b4** is 0 or 1;
**b1** is 0, 1, or 2;
each **R¹⁰** is independently selected from the group consisting of: -Cl, -F, -CN, and C₁₋₃ alkyl optionally substituted with 1-3 **R^{c}**;
**R⁷** is selected from the group consisting of: C(=O)N(**R^{f}**)₂, C(=O)N(C₁₋₃ alkyl)**R^{b1}**, - C(=O)N(H)**R^{b1}**, **R^{b1}**, and C(=O)**R^{b1}**;
**X²** is -O- or -CH₂-;
**X¹** is CH**R^{L}**; and **X³** is CH**R^{L}** or C(**R^{L}**)₂, wherein:
   one pair of **R^{L}** on different ring carbon atom(s) taken together with the ring atom(s) connecting them form a C₃₋₄ cycloalkyl ring; and
   the remaining **R^{L}** if present is C₁₋₂ alkyl optionally substituted with 1-3 F.

In some embodiments of Formula (**V-a2**), **b1** is 1 or 2; and the 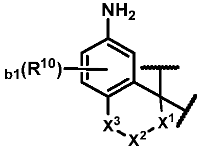 moiety is

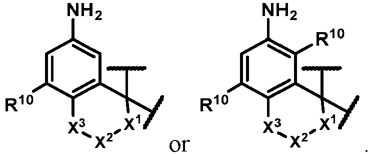

In some embodiments of Formula (**V-b2**), **b4** is 0.

In some embodiments of Formula (**V-b2**), **b4** is 1; and **R¹⁰** is *ortho* to **X³**.

In some embodiments of Formula (**II-a2**), (**II-b2**), (**III-2**), (**IV-a2**), (**IV-b2**), (**V-a2**), or (**V-b2**), **R⁷** is selected from the group consisting of:
**(a)** C(=O)N(**R^{f}**)₂, wherein each **R^{f}** is independently H or C₁₋₃ alkyl optionally substituted with 1-3 **R^{h}**;
**(b)** C(=O)N(C₁₋₃ alkyl)**R^{b1}** or C(=O)N(H)**R^{b1}**, wherein: **R^{b1}** is a C₃₋₆ cycloalkyl or 4-6 membered heterocyclyl, each of which is optionally substituted with 1-3 **R^{g}**; and
**(c)** C(=O)**R^{b1}**, wherein **R^{b1}** is a 4-10 membered heterocyclyl optionally substituted with 1-3 **R^{g}**, wherein **R^{b1}** is attached to the C(=O) via a ring nitrogen atom.

In some embodiments of Formula (**II-a2**), (**II-b2**), (**III-2**), (**IV-a2**), (**IV-b2**), (**V-a2**), or (**V-b2**), **R⁷** is **R^{b1}**, wherein the **R^{b1}** is a 5-6 membered heteroaryl or 4-6 membered heterocyclyl, each of which is optionally substituted with 1-2 **R^{g}**.

In some embodiments, the compounds of Formula (**II**) are compounds of Formula (**II-3**): 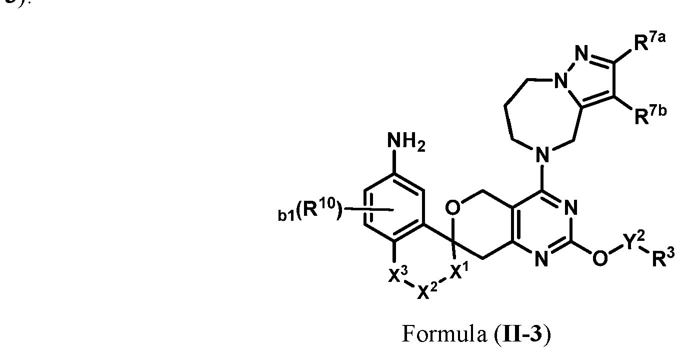 or pharmaceutically acceptable salts thereof, wherein:
**b1** is 1 or 2;
each **R¹⁰** is independently selected from the group consisting of: -Cl, -F, -CN, and C₁₋₃ alkyl optionally substituted with 1-3 **R^{c}**;
**R^{7a}** is selected from the group consisting of:
   **(a)** C(=O)N(**R^{f}**)₂, wherein each **R^{f}** is independently H or C₁₋₃ alkyl optionally substituted with 1-3 **R^{h}**;
   **(b)** C(=O)N(C₁₋₃ alkyl)**R^{b1}** or C(=O)N(H)**R^{b1}**, wherein: **R^{b1}** is a C₃₋₆ cycloalkyl or 4-6 membered heterocyclyl, each of which is optionally substituted with 1-3 **R^{g}**; and
   **(c)** C(=O)**R^{b1}**, wherein **R^{b1}** is a 4-10 membered heterocyclyl optionally substituted with 1-3 **R^{g}**, wherein **R^{b1}** is attached to the C(=O) via a ring nitrogen atom;
**R^{7b}** is -halo or C₁₋₃ alkyl (e.g., **R^{7b}** is -halo);
**X¹** is CH₂; and
**X²** and **X³** are independently selected from the group consisting of: O, CH₂, CH**R^{L}**, and C(**R^{L}**)₂.

In some embodiments of Formula (**II-3**), **b1** is 1.

In some embodiments, the compounds of Formula (**II**) are compounds of Formula (**II-a3**): 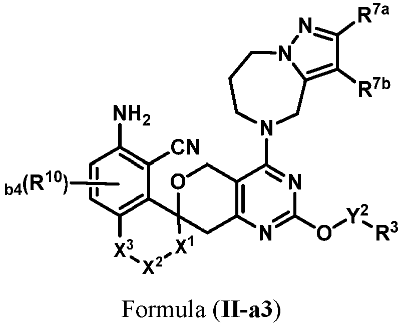 or pharmaceutically acceptable salts thereof, wherein:
**b4** is 0 or 1;
**R¹⁰** is selected from the group consisting of: -Cl, -F, -CN, and C₁₋₃ alkyl optionally substituted with 1-3 **R^{c}**;
**R^{7a}** is selected from the group consisting of:
   **(a)** C(=O)N(**R^{f}**)₂, wherein each **R^{f}** is independently H or C₁₋₃ alkyl optionally substituted with 1-3 **R^{h}**;
   **(b)** C(=O)N(C₁₋₃ alkyl)**R^{b1}** or C(=O)N(H)**R^{b1}**, wherein: **R^{b1}** is a C₃₋₆ cycloalkyl or 4-6 membered heterocyclyl, each of which is optionally substituted with 1-3 **R^{g}**; and
   **(c)** C(=O)**R^{b1}**, wherein **R^{b1}** is a 4-10 membered heterocyclyl optionally substituted with 1-3 **R^{g}**, wherein **R^{b1}** is attached to the C(=O) via a ring nitrogen atom;
**R^{7b}** is -halo or C₁₋₃ alkyl (e.g., **R^{7b}** is -halo);
**X¹** is CH₂; and
**X²** and **X³** are independently selected from the group consisting of: O, CH₂, CH**R^{L}**, and C(**R^{L}**)₂.

In some embodiments of Formula (**II-a3**), **b4** is 0.

In some embodiments of Formula (**II-a3**), **b4** is 1; and **R¹⁰** is *ortho* to **X³**.

In some embodiments, the compounds of Formula (**III**) are compounds of Formula (**III-3**): 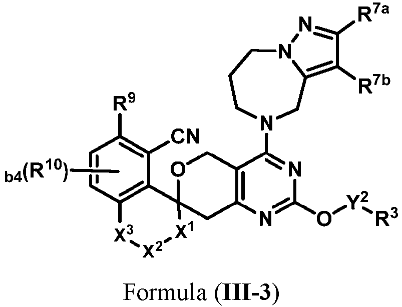 or pharmaceutically acceptable salts thereof, wherein:
**b4** is 0 or 1;
**R¹⁰** is selected from the group consisting of: -Cl, -F, -CN, and C₁₋₃ alkyl optionally substituted with 1-3 **R^{c}**;
**R^{7a}** is selected from the group consisting of:
   **(a)** C(=O)N(**R^{f}**)₂, wherein each **R^{f}** is independently H or C₁₋₃ alkyl optionally substituted with 1-3 **R^{h}**;
   **(b)** C(=O)N(C₁₋₃ alkyl)**R^{b1}** or C(=O)N(H)**R^{b1}**, wherein: **R^{b1}** is a C₃₋₆ cycloalkyl or 4-6 membered heterocyclyl, each of which is optionally substituted with 1-3 **R^{g}**; and
   **(c)** C(=O)**R^{b1}**, wherein **R^{b1}** is a 4-10 membered heterocyclyl optionally substituted with 1-3 **R^{g}**, wherein **R^{b1}** is attached to the C(=O) via a ring nitrogen atom;
**R^{7b}** is -halo or C₁₋₃ alkyl (e.g., -halo);
**X¹** is CH₂; and
**X²** and **X³** are independently selected from the group consisting of: O, CH₂, CH**R^{L}**, and C(**R^{L}**)₂.

In some embodiments of Formula (**III-3**), **b4** is 0.

In some embodiments of Formula (**III-3**), **b4** is 1; and **R¹⁰** is *ortho* to **X³**.

In some embodiments of Formula (**III-3**), **R⁹** is N**R^{d}R^{e}** (e.g., -NH₂).

In some embodiments, the compounds of Formula (**IV**) are compounds of Formula (**IV-a3**) or (**IV-b3**): 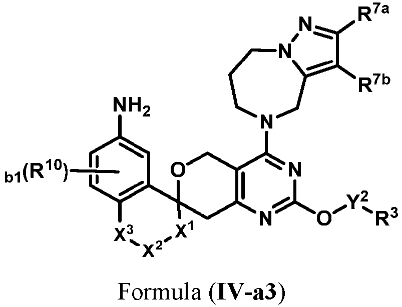 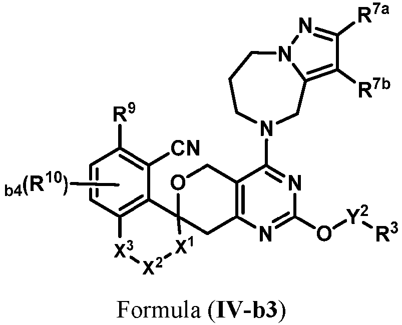 or pharmaceutically acceptable salts thereof, wherein:
**b1** is 0, 1, or 2;
**b4** is 0 or 1;
each **R¹⁰** is independently selected from the group consisting of: -Cl, -F, -CN, and C₁₋₃ alkyl optionally substituted with 1-3 **R^{c}**;
**R^{7a}** is selected from the group consisting of:
   **(a)** C(=O)N(**R^{f}**)₂, wherein each **R^{f}** is independently H or C₁₋₃ alkyl optionally substituted with 1-3 **R^{h}**;
   **(b)** C(=O)N(C₁₋₃ alkyl)**R^{b1}** or C(=O)N(H)**R^{b1}**, wherein: **R^{b1}** is a C₃₋₆ cycloalkyl or 4-6 membered heterocyclyl, each of which is optionally substituted with 1-3 **R^{g}**; and
   **(c)** C(=O)**R^{b1}**, wherein **R^{b1}** is a 4-10 membered heterocyclyl optionally substituted with 1-3 **R^{g}**, wherein **R^{b1}** is attached to the C(=O) via a ring nitrogen atom;
**R^{7b}** is -halo or C₁₋₃ alkyl (e.g., -halo);
**X¹** is CH₂;
one of **X²** and **X³** is independently selected from the group consisting of: CH**R^{L}** and C(**R^{L}**)₂; and
the other of **X²** and **X³** is CH₂ or O.

In some embodiments, the compounds are compounds of Formula (**IV-b3**), or pharmaceutically acceptable salts thereof, wherein **b4** is 1; and **R¹⁰** is *ortho* to **X³**.

In some embodiments, the compounds are compounds of Formula (**IV-b3**), or pharmaceutically acceptable salts thereof, wherein **b4** is 0.

In some embodiments, the compounds are compounds of Formula (**IV-a3**), or pharmaceutically acceptable salts thereof, wherein **b1** is 1 or 2; and the 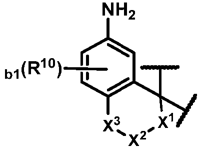 moiety is

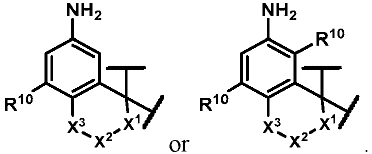

In some embodiments, the compounds are compounds of Formula (**IV-a3**), or pharmaceutically acceptable salts thereof, wherein **b1** is 1; and the 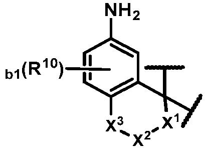 moiety is

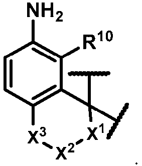

In some embodiments of Formula (**IV-a3**) or (**IV-b3**), **X²** is CH₂; and **X³** is CH**R^{L}**. In some embodiments, **R^{L}** is CF₃ or CH₃. In some embodiments, **R^{L}** is CH₃.

In some embodiments, the compounds of Formula (**V**) are compounds of Formula (**V-a3**) or (**V-b3**): 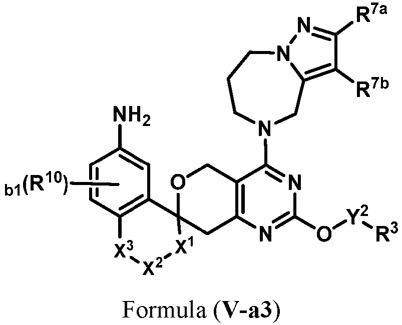 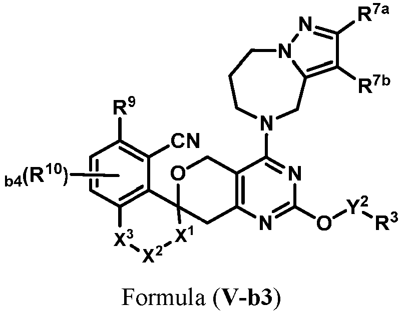 or a pharmaceutically acceptable salt thereof, wherein:
**b1** is 0, 1, or 2;
**b4** is 0 or 1;
each **R¹⁰** is independently selected from the group consisting of: -Cl, -F, -CN, and C₁₋₃ alkyl optionally substituted with 1-3 **R^{c}**;
**R^{7a}** is selected from the group consisting of:
   **(a)** C(=O)N(**R^{f}**)₂, wherein each **R^{f}** is independently H or C₁₋₃ alkyl optionally substituted with 1-3 **R^{h}**;
   **(b)** C(=O)N(C₁₋₃ alkyl)**R^{b1}** or C(=O)N(H)**R^{b1}**, wherein: **R^{b1}** is a C₃₋₆ cycloalkyl or 4-6 membered heterocyclyl, each of which is optionally substituted with 1-3 **R^{g}**; and
   **(c)** C(=O)**R^{b1}**, wherein **R^{b1}** is a 4-10 membered heterocyclyl optionally substituted with 1-3 **R^{g}**, wherein **R^{b1}** is attached to the C(=O) via a ring nitrogen atom;
**R^{7b}** is -halo or C₁₋₃ alkyl (e.g., -halo);
**X²** is -O- or -CH₂-;
**X¹** is CH**R^{L}**; and **X³** is CH**R^{L}** or C(**R^{L}**)₂, wherein:
   one pair of **R^{L}** on different ring carbon atom(s) taken together with the ring atom(s) connecting them form a C₃₋₄ cycloalkyl ring; and
   the remaining **R^{L}** if present is C₁₋₂ alkyl optionally substituted with 1-3 F.

In some embodiments, the compounds are compounds of Formula (**V-b3**), or pharmaceutically acceptable salts thereof, wherein **b4** is 1; and **R¹⁰** is *ortho* to **X³**.

In some embodiments, the compounds are compounds of Formula (**V-b3**), or pharmaceutically acceptable salts thereof, wherein **b4** is 0.

In some embodiments, the compounds are compounds of Formula (**V-a3**), or pharmaceutically acceptable salts thereof, wherein **b1** is 1 or 2; and the 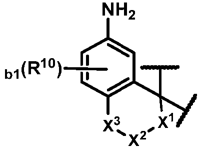 moiety is

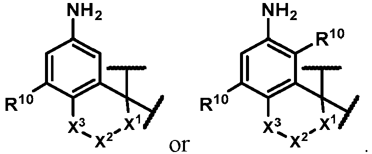

In some embodiments, the compounds are compounds of Formula (**V-a3**), or pharmaceutically acceptable salts thereof, wherein **b1** is 1; and the 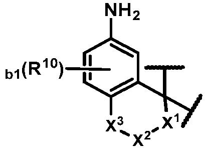 moiety is

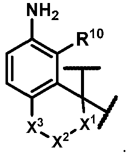

In some embodiments of Formula (**II-3**), (**II-a3**), (**III-3**), (**IV-a3**), (**IV-b3**), (**V-a3**), or (**V-b3**), **R^{7a}** is C(=O)N(**R^{f}**)₂, wherein each **R^{f}** is independently C₁₋₃ alkyl optionally substituted with 1-3 **R^{h}**. For example, **R^{7a}** can be C(=O)NMe₂.

In some embodiments of Formula (**II-3**), (**II-a3**), (**III**-3), (**IV-a3**), (**IV-b3**), (**V-a3**), or (**V-b3**), **R^{7b}** is -Cl, -F, or methyl.

In some embodiments of Formula (**II-3**), (**II-a3**), (**III-3**), (**IV-a3**), (**IV-b3**), (**V-a3**), or (**V-b3**), **R^{7b}** is -halo.

In some embodiments of Formula (**II-3**), (**II-a3**), (**III-3**), (**IV-a3**), (**IV-b3**), (**V-a3**), or (**V-b3**), **R^{7b}** is -Cl.

In some embodiments of Formula (**II-3**), (**II-a3**), (**III-3**), (**IV-a3**), (**IV-b3**), (**V-a3**), or (**V-b3**), **R^{7b}** is -F.

In some embodiments of Formula (**II-3**), (**II-a3**), (**III-3**), (**IV-a3**), (**IV-b3**), (**V-a3**), or (**V-b3**), **R^{7b}** is -methyl.

In some embodiments of Formula (**II-3**), (**II-a3**), (**III-3**), (**IV-a3**), (**IV-b3**), (**V-a3**), or (**V-b3**), the 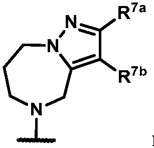 moiety is

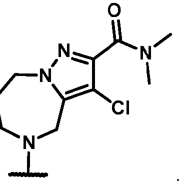

In some embodiments, the compounds of Formula (**II**) are compounds of Formula (**II-**
**4**): 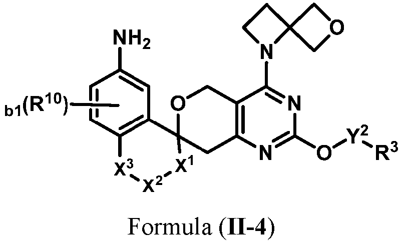 or pharmaceutically acceptable salts thereof, wherein:
**b1** is 1 or 2;
each **R¹⁰** is independently selected from the group consisting of: -Cl, -F, -CN, and C₁₋₃ alkyl optionally substituted with 1-3 **R^{c}**;
**X¹** is CH₂; and
**X²** and **X³** are independently selected from the group consisting of: O, CH₂, CH**R^{L}**, and C(**R^{L}**)₂.

In some embodiments of Formula (**II-4**), **b1** is 1.

In some embodiments of Formula (**II-4**), **b1** is 1; and **R¹⁰** is -CN.

In some embodiments, the compounds of Formula (**II**) are compounds of Formula (**II-**
**a4**): 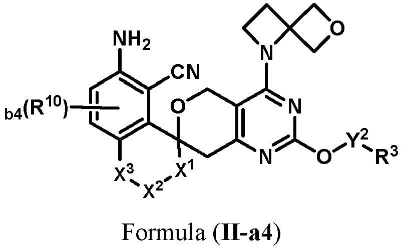 or pharmaceutically acceptable salts thereof, wherein:
**b4** is 0 or 1;
each **R¹⁰** is independently selected from the group consisting of: -Cl, -F, -CN, and C₁₋₃ alkyl optionally substituted with 1-3 **R^{c}**;
**X¹ is** CH₂; and
**X²** and **X³** are independently selected from the group consisting of: O, CH₂, CH**R^{L}**, and C(**R^{L}**)₂.

In some embodiments of Formula (**II-a4**), **b4** is 0.

In some embodiments, the compounds of Formula (**III**) are compounds of Formula (III**-4**): 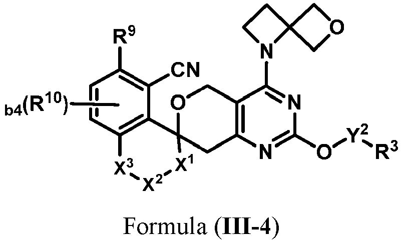 or pharmaceutically acceptable salts thereof, wherein:
**b4** is 0 or 1;
**R¹⁰** is selected from the group consisting of: -Cl, -F, -CN, and C₁₋₃ alkyl optionally substituted with 1-3 **R^{c}**;
**X¹** is CH₂; and
**X²** and **X³** are independently selected from the group consisting of: O, CH₂, CH**R^{L}**, and C(**R^{L}**)₂.

In some embodiments of Formula (**III-4**), **b4** is 0.

In some embodiments of Formula (**III-4**), **R⁹** is N**R^{d}R^{e}** (e.g., -NH₂).

In some embodiments of Formula (**III-4**), **b4** is 0; and **R⁹** is N**R^{d}R^{e}** (e.g., -NH₂).

In some embodiments, the compounds of Formula (**IV**) are compounds of Formula (**IV-a4**) or Formula (**IV-b4**): 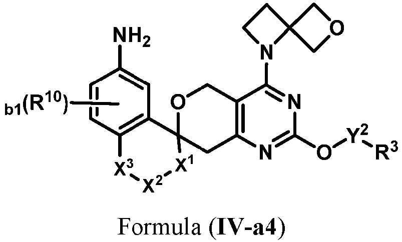 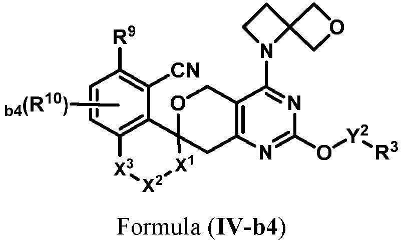 or pharmaceutically acceptable salts thereof, wherein:
**b1** is 0, 1, or 2;
**b4** is 0 or 1;
each **R¹⁰** is independently selected from the group consisting of: -Cl, -F, -CN, and C₁₋₃ alkyl optionally substituted with 1-3 **R^{c}**;
**X¹** is CH₂;
one of **X²** and **X³** is independently selected from the group consisting of: CH**R^{L}** and C(**R^{L}**)₂; and
the other of **X²** and **X³** is CH₂ or O.

In some embodiments of Formula (**IV-a4**), **b1** is 1; and the 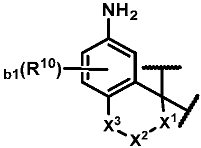 moiety is 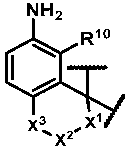 (e.g., 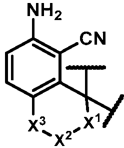 ).

In some embodiments of Formula (**IV-b4**), **b4** is 0.

In some embodiments of Formula (**II-4**), (**II-a4**), (**III-4**), (**IV-a4**), or (**IV-b4**), **X²** is CH₂; and **X³** is CH**R^{L}** (e.g., CH(CH₃)).

In some embodiments of Formula (**II-4**), (**II-a4**), or (**III-4**), **X²** is CH₂; and **X³** is CH₂.

In some embodiments, the compounds of Formula (**II**) are compounds of Formula (**II-5**): 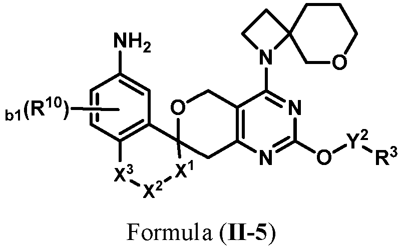 or pharmaceutically acceptable salts thereof, wherein:
**b1** is 1 or 2;
each **R¹⁰** is independently selected from the group consisting of: -Cl, -F, -CN, and C₁₋₃ alkyl optionally substituted with 1-3 **R^{c}**;
**X¹** is CH₂; and
**X²** and **X³** are independently selected from the group consisting of: O, CH₂, CHR^{L}, and C(**R^{L}**)₂.

In some embodiments of Formula (**II-5**), **b1** is 1.

In some embodiments of Formula (**II-5**), **b1** is 1; and **R¹⁰** is -CN.

In some embodiments, the compounds of Formula (**II**) are compounds of Formula (**II-**
**a5**): 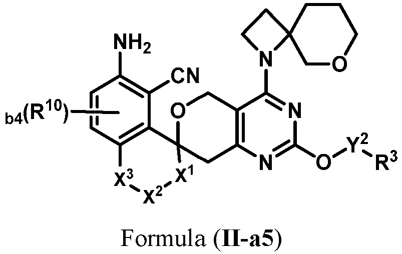 or pharmaceutically acceptable salts thereof, wherein:
**b4** is 0 or 1;
**R¹⁰** is selected from the group consisting of: -Cl, -F, -CN, and C₁₋₃ alkyl optionally substituted with 1-3 **R^{c}**;
**X¹** is CH₂; and
**X²** and **X³** are independently selected from the group consisting of: O, CH₂, CH**R^{L}**, and C(**R^{L}**)₂.

In some embodiments of Formula (**II-a5**), **b4** is 0.

In some embodiments, the compounds of Formula (**III**) are compounds of Formula (**III-5)**: 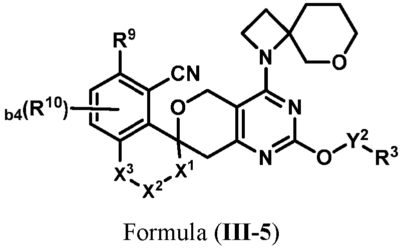 or pharmaceutically acceptable salts thereof, wherein:
**b4** is 0 or 1;
**R¹⁰** is selected from the group consisting of: -Cl, -F, -CN, and C₁₋₃ alkyl optionally substituted with 1-3 **R^{c}**;
**X¹** is CH₂; and
**X²** and **X³** are independently selected from the group consisting of: O, CH₂, CH**R^{L}**, and C(**R^{L}**)₂.

In some embodiments of Formula (**III-5**), **b4** is 0.

In some embodiments of Formula (**III-5**), **R⁹** is N**R^{d}R^{e}** (e.g., -NH₂).

In some embodiments of Formula (**III-5**), **b4** is 0; and **R⁹** is N**R^{d}R^{e}** (e.g., -NH₂).

In some embodiments, the compounds of Formula (**IV**) are compounds of Formula (**IV-a5**) or Formula (**IV-b5**): 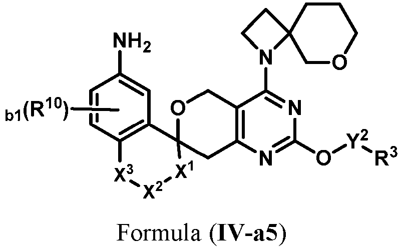 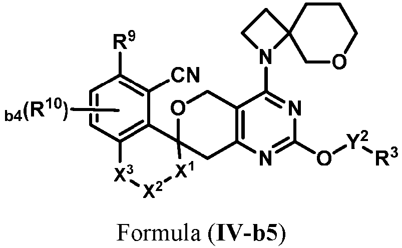 or pharmaceutically acceptable salts thereof, wherein:
**b1** is 0, 1, or 2;
**b4** is 0 or 1;
each **R¹⁰** is independently selected from the group consisting of: -Cl, -F, -CN, and C₁₋₃ alkyl optionally substituted with 1-3 **R^{c}**;
**X¹** is CH₂;
one of **X²** and **X³** is independently selected from the group consisting of: CH**R^{L}** and C(**R^{L}**)₂; and
the other of **X²** and **X³** is CH₂ or O.

In some embodiments of Formula (**IV-a5**), **b1** is 1; and the 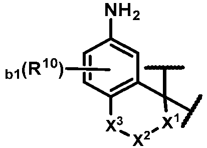 moiety is 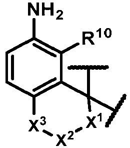 (e.g., 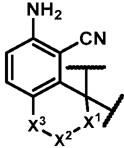 ).

In some embodiments of Formula (**IV-b5**), **b4** is 0.

In some embodiments of Formula (**II-5**), (**II-a5**), (**III-5**), (**IV-a5**), or (**IV-b5**), **X²** is CH₂; and **X³** is CH**R^{L}** (e.g., CH(CH₃)).

In some embodiments of Formula (**II-5**), (**II-a5**), or (**III-5**), **X²** is CH₂; and **X³** is CH₂.

In some embodiments, the compounds of Formula (**II**) are compounds of Formula (**II-**
**6**): 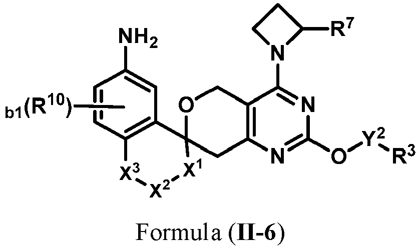 or pharmaceutically acceptable salts thereof, wherein:
**R⁷** is C₁₋₃ alkyl substituted with -OH or C₁₋₃ alkoxy;
**b1** is 1 or 2;
each **R¹⁰** is independently selected from the group consisting of: -Cl, -F, -CN, and C₁₋₃ alkyl optionally substituted with 1-3 **R^{c}**;
**X¹** is CH₂; and
**X²** and **X³** are independently selected from the group consisting of: O, CH₂, CH**R^{L}**, and C(**R^{L}**)₂.

In some embodiments of Formula (**II-6**), **b1** is 1.

In some embodiments of Formula (**II-6**), **b1** is 1; and **R¹⁰** is -CN.

In some embodiments, the compounds of Formula (**II**) are compounds of Formula (**II-**
**a6**): 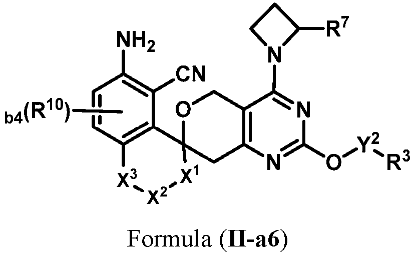 or pharmaceutically acceptable salts thereof, wherein:
**R⁷** is C₁₋₃ alkyl substituted with -OH or C₁₋₃ alkoxy;
**b4** is 0 or 1;
each **R¹⁰** is independently selected from the group consisting of: -Cl, -F, -CN, and C₁₋₃ alkyl optionally substituted with 1-3 **R^{c}**;
**X¹** is CH₂; and
**X²** and **X³** are independently selected from the group consisting of: O, CH₂, CH**R^{L}**, and C(**R^{L}**)₂.

In some embodiments of Formula (**II-a6**), **b4** is 0.

In some embodiments, the compounds of Formula (**III**) are compounds of Formula (**III-6**): 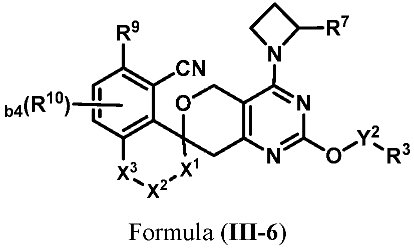 or pharmaceutically acceptable salts thereof, wherein:
**R⁷** is C₁₋₃ alkyl substituted with -OH or C₁₋₃ alkoxy;
**b4** is 0 or 1;
**R¹⁰** is selected from the group consisting of: -Cl, -F, -CN, and C₁₋₃ alkyl optionally substituted with 1-3 **R^{c}**;
**X¹** is CH₂; and
**X²** and **X³** are independently selected from the group consisting of: O, CH₂, CH**R^{L}**, and C(**R^{L}**)₂.

In some embodiments of Formula (**III-6**), **b4** is 0.

In some embodiments of Formula (**III-6**), **R⁹** is N**R^{d}R^{e}** (e.g., -NH₂).

In some embodiments of Formula (**III-6**), **b4** is 0; and **R⁹** is N**R^{d}R^{e}** (e.g., -NH₂).

In some embodiments, the compounds of Formula (**IV**) are compounds of Formula (**IV-a6**) or (**IV-b6**): 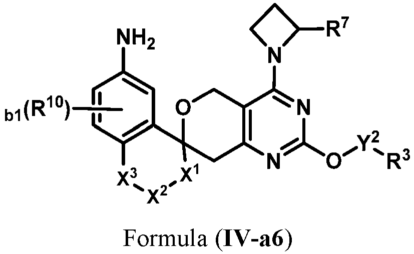 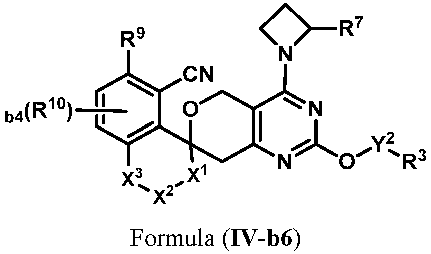 or a pharmaceutically acceptable salt thereof, wherein:
**R⁷** is C₁₋₃ alkyl substituted with -OH or C₁₋₃ alkoxy;
**b1** is 0, 1, or 2;
**b4** is 0 or 1;
each **R¹⁰** is independently selected from the group consisting of: -Cl, -F, -CN, and C₁₋₃ alkyl optionally substituted with 1-3 **R^{c}**;
**X¹** is CH₂;
one of **X²** and **X³** is independently selected from the group consisting of: CH**R^{L}** and C(**R^{L}**)₂; and
the other of **X²** and **X³** is CH₂ or O.

In some embodiments of Formula (**IV-a6**), **b1** is 1; and the 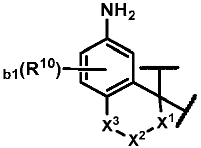 moiety is 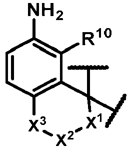 (e.g., 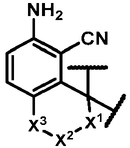 ).

In some embodiments of Formula (**IV-b6**), **b4** is 0.

In some embodiments of Formula (**IV-b6**), **R⁹** is N**R^{d}R^{e}** (e.g., -NH₂).

In some embodiments of Formula (**II-6**), (**II-a6**), (**III-6**), (**IV-a6**), or (**IV-b6**), the 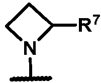 moiety is

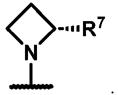

In some embodiments of Formula (**II-6**), (**II-a6**), (**III-6**), (**IV-a6**), or (**IV-b6**), **R⁷** is C₁₋₃ alkyl substituted with -OH. For example, **R⁷** can be -CH₂OH.

In some embodiments of Formula (**II-6**), (**II-a6**), (**III-6**), (**IV-a6**), or (**IV-b6**), the 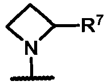 moiety is 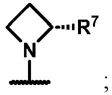 and **R⁷** is C₁₋₃ alkyl substituted with -OH. For example, **R⁷** can be -CH₂OH.

In some embodiments of Formula (**II-6**), (**II-a6**), (**III-6**), (**IV-a6**), or (**IV-b6**), **X²** is CH₂; and **X³** is CH**R^{L}** (e.g., CH(CH₃)).

In some embodiments of Formula (**II-6**), (**II-a6**), or (**III-6**), **X²** is CH₂; and **X³** is CH₂.

In some embodiments, the compounds of Formula (**II**) are compounds of Formula (**II-**
**7**): 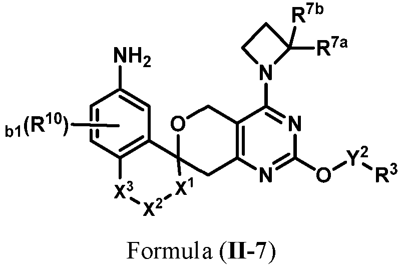 or pharmaceutically acceptable salts thereof, wherein:
**R^{7a}** is C₁₋₃ alkyl substituted with -OH (e.g., -CH₂OH);
**R^{7b}** is selected from the group consisting of:
   C₁₋₃ alkyl optionally substituted with 1-3 F (e.g., methyl), and
   C₁₋₃ alkyl substituted with -OH or C₁₋₃ alkoxy;
   **b1** is 1 or 2;
   each **R¹⁰** is independently selected from the group consisting of: -Cl, -F, -CN, and C₁₋₃ alkyl optionally substituted with 1-3 **R^{c}**;
   **X¹** is CH₂; and
   **X²** and **X³** are independently selected from the group consisting of: O, CH₂, CHR^{L}, and C(**R^{L}**)₂.

In some embodiments of Formula (**II-7**), **b1** is 1.

In some embodiments of Formula (**II-7**), **b1** is 1; and **R¹⁰** is -CN.

In some embodiments, the compounds of Formula (II) are compounds of Formula (**II-**
**a7**): 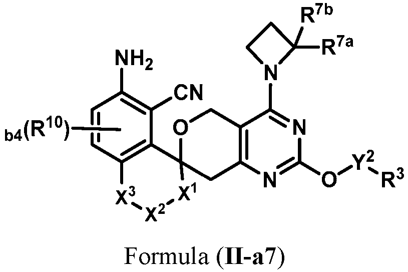 or pharmaceutically acceptable salts thereof, wherein:
**R^{7a}** is C₁₋₃ alkyl substituted with -OH (e.g., -CH₂OH);
**R^{7b}** is selected from the group consisting of:
   C₁₋₃ alkyl optionally substituted with 1-3 F (e.g., methyl), and
   C₁₋₃ alkyl substituted with -OH or C₁₋₃ alkoxy;
   **b4** is 0 or 1;
   **R¹⁰** is selected from the group consisting of: -Cl, -F, -CN, and C₁₋₃ alkyl optionally substituted with 1-3 **R^{c}**;
   **X¹** is CH₂; and
   **X²** and **X³** are independently selected from the group consisting of: O, CH₂, CH**R^{L}**, and C(**R^{L}**)₂.

In some embodiments of Formula (**II-a7**), **b4** is 0.

In some embodiments, the compounds of Formula (**III**) are compounds of Formula (**III-7**): 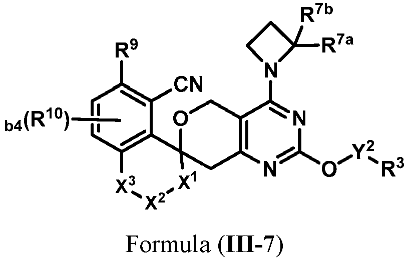 or pharmaceutically acceptable salts thereof, wherein:
**R^{7a}** is C₁₋₃ alkyl substituted with -OH (e.g., -CH₂OH);
**R^{7b}** is selected from the group consisting of:
   C₁₋₃ alkyl optionally substituted with 1-3 F (e.g., methyl), and
   C₁₋₃ alkyl substituted with -OH or C₁₋₃ alkoxy;
   **b4** is 0 or 1;
   **R¹⁰** is selected from the group consisting of: -Cl, -F, -CN, and C₁₋₃ alkyl optionally substituted with 1-3 **R^{c}**;
   **X¹** is CH₂; and
   **X²** and **X³** are independently selected from the group consisting of: O, CH₂, CH**R^{L}**, and C(**R^{L}**)₂.

In some embodiments of Formula (**III-7**), **b4** is 0.

In some embodiments of Formula (**III-7**), **R⁹** is N**R^{d}R^{e}** (e.g., -NH₂).

In some embodiments, the compounds of Formula (**IV**) are compounds of Formula (**IV- a7**), or (**IV-b7**): 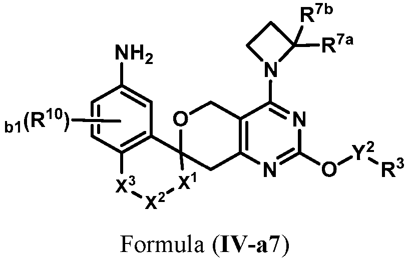 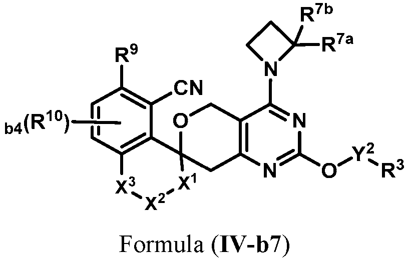 or pharmaceutically acceptable salts thereof, wherein:
**R^{7a}** is C₁₋₃ alkyl substituted with -OH (e.g., -CH₂OH);
**R^{7b}** is selected from the group consisting of:
   C₁₋₃ alkyl optionally substituted with 1-3 F (e.g., methyl), and
   C₁₋₃ alkyl substituted with -OH or C₁₋₃ alkoxy;
   **b1** is 0, 1, or 2;
   **b4** is 0 or 1;
   each **R¹⁰** is independently selected from the group consisting of: -Cl, -F, -CN, and C₁₋₃ alkyl optionally substituted with 1-3 **R^{c}**;
   **X¹** is CH₂;
   one of **X²** and **X³** is independently selected from the group consisting of: CH**R^{L}** and C(**R^{L}**)₂; and
   the other of **X²** and **X³** is CH₂ or O.

In some embodiments of Formula (**IV-a7**), **b1** is 1; and the 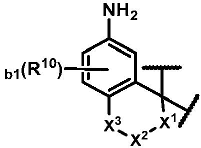 moiety is 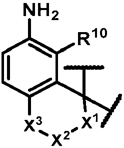 (e.g., 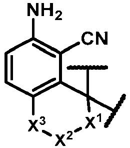 ).

In some embodiments of Formula **(IV-b7), b4** is 0.

In some embodiments of Formula (**II-7**), **(II-a7),** (**III-7**), **(IV-a7),** or **(IV-b7),** the 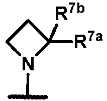 moiety is

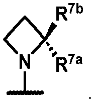

In some embodiments of Formula (**II-7**), **(II-a7), (III-7**), (**IV-a7**), or **(IV-b7), R^{7a}** is - CH₂OH.

In some embodiments of Formula (**II-7**), **(II-a7), (111-7), (IV-a7),** or **(IV-b7), R^{7b}** is C₁₋₃ alkyl optionally substituted with 1-3 -F. In some embodiments of Formula (**II-7**), **(II-a7),** (**III-7**), **(IV-a7),** or **(IV-b7), R^{7b}** is C₁₋₃ alkyl. For example, **R^{7b}** can be C₁₋₃ alkyl (e.g., methyl).

In some embodiments of Formula (**II-7**), **(II-a7), (III-7), (IV-a7),** or **(IV-b7), X²** is CH₂; and **X³** is CH**R^{L}** (e.g., CH(CH₃)).

In some embodiments of Formula (**II-7**), (**II-a7**), or (**III-7**), **X²** is CH₂; and **X³** is CH₂.

In some embodiments, the compounds of Formula **(II)** are compounds of Formula **(II-**
**8):** 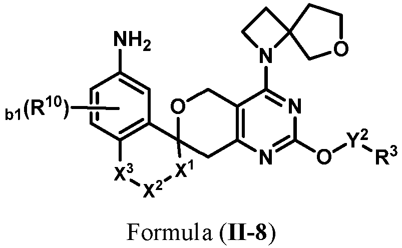 or pharmaceutically acceptable salts thereof, wherein:
**b4** is 0 or 1;
**R¹⁰** is selected from the group consisting of: -Cl, -F, -CN, and C₁₋₃ alkyl optionally substituted with 1-3 **R^{c}**;
**X¹** is CH₂; and
**X²** and **X³** are independently selected from the group consisting of: O, CH₂, CH**R^{L}**, and C(**R^{L}**)₂.

In some embodiments of Formula (**II-8**), **b1** is 1.

In some embodiments of Formula (**II-8**), **b1** is 1; and **R¹⁰** is -CN.

In some embodiments, the compounds of Formula **(II)** are compounds of Formula **(II-**
**a8):** 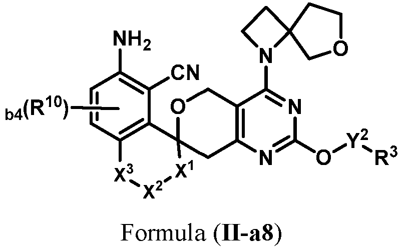 or pharmaceutically acceptable salts thereof, wherein:
**b4** is 0 or 1;
**R¹⁰** is selected from the group consisting of: -Cl, -F, -CN, and C₁₋₃ alkyl optionally substituted with 1-3 **R^{c}**;
**X¹** is CH₂; and
**X²** and **X³** are independently selected from the group consisting of: O, CH₂, CH**R^{L}**, and C(**R^{L}**)₂.

In some embodiments of Formula **(II-a8), b4** is 0.

In some embodiments, the compounds of Formula **(III)** are compounds of Formula **(111-8):** 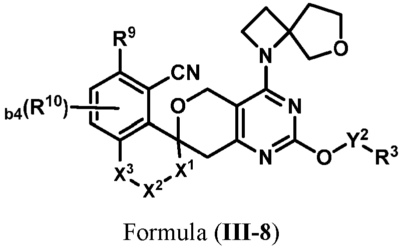 or pharmaceutically acceptable salts thereof, wherein:
**b1** is 1 or 2;
each **R¹⁰** is independently selected from the group consisting of: -Cl, -F, -CN, and C₁₋₃ alkyl optionally substituted with 1-3 **R^{c}**;
**X¹** is CH₂; and
**X²** and **X³** are independently selected from the group consisting of: O, CH₂, CH**R^{L}**, and C(**R^{L}**)₂.

In some embodiments of Formula (**III-8**), **b4** is 0.

In some embodiments of Formula (**III-8**), **R⁹** is N**R^{d}R^{e}** (e.g., -NH₂).

In some embodiments of Formula (**III-8**), **b4** is 0; and **R⁹** is N**R^{d}R^{e}** (e.g., -NH₂).

In some embodiments, the compounds of Formula (IV) are compounds of Formula **(IV-a8)** or Formula **(IV-b8):** 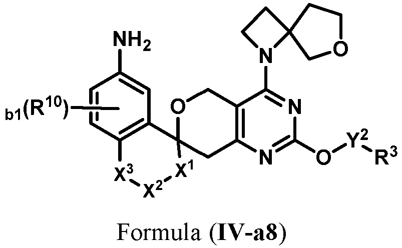 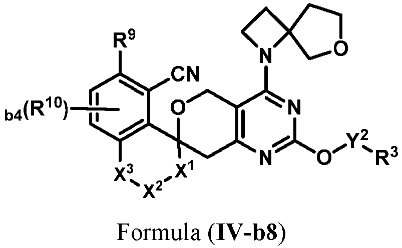 or pharmaceutically acceptable salts thereof, wherein:
**b1** is 0, 1, or 2;
**b4** is 0 or 1;
each **R¹⁰** is independently selected from the group consisting of: -Cl, -F, -CN, and C₁₋₃ alkyl optionally substituted with 1-3 **R^{c}**;
**X¹** is CH₂;
one of **X²** and **X³** is independently selected from the group consisting of: CH**R^{L}** and C(**R^{L}**)2; and
the other of **X²** and **X³** is CH₂ or O.

In some embodiments of Formula **(IV-a8), b1** is 1; and the 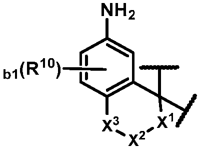 moiety is 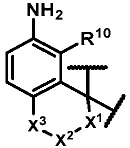 (e.g., 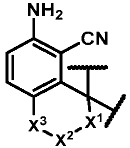 ).

In some embodiments of Formula **(IV-b8), b4** is 0.

In some embodiments of Formula (**II-8**), **(II-a8),** (**III-8**), **(IV-a8),** or **(IV-b8), X²** is CH₂; and **X³** is CH**R^{L}** (e.g., CH(CH₃)).

In some embodiments of Formula (**II-8**), (**II-a8**), or (**III-8**), **X²** is CH₂; and **X³** is CH₂.

In some embodiments of Formula (**II-a1**), (**II-b1**), (**III-1**), (**IV-a1)**, **(IV-b1),** (**V-a1**), **(V-b1), (II-a2), (II-b2), (III-2), (IV-a2), (IV-b2), (V-a2), (V-b2), (11-3), (II-a3), (III-3), (IV-**a3), **(IV-b3), (V-a3), (V-b3), (11-4), (II-a4), (III-4), (IV-a4), (IV-b4), (II-5), (II-a5), (111-5), (IV-a5), (IV-b5), (II-6), (II-a6), (111-6), (IV-a6), (IV-b6), (11-7), (II-a7), (111-7), (IV-a7), (IV-b7), (II-8), (II-a8), (III-8), (IV-a8),** or **(IV-b8), Y²** is -CH₂-; and **R³** is a 4-10 membered heterocyclyl having one ring nitrogen atom and 0-1 additional ring heteroatom selected from the group consisting of oxygen and nitrogen, wherein the heterocyclyl is optionally substituted with 1-6 **R^{a}**.

In some embodiments of Formula (**II-a1**), (**II-b1**), **(III-1), (IV-a1), (IV-b1), (V-a1), (V-b1), (II-a2), (II-b2), (III-2), (IV-a2), (IV-b2), (V-a2), (V-b2), (11-3), (II-a3),** (**III-3**), **(IV-a3), (IV-b3), (V-a3), (V-b3), (11-4), (II-a4),** (**III-4**), **(IV-a4), (IV-b4),** (**II-5**), **(II-a5), (111-5), (IV-a5), (IV-b5),** (**II-6**), **(II-a6), (111-6), (IV-a6), (IV-b6), (11-7), (II-a7), (111-7), (IV-a7), (IV-b7),** (**II-8**), **(II-a8),** (**III-8**), **(IV-a8),** or **(IV-b8), Y²** is -CH₂-; and **R³** is 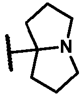 optionally substituted with 1-2 substituents each independently selected from the group consisting of: -F, -C₁₋₃ alkoxy, and -C₁₋₃ haloalkoxy.

In some embodiments of Formula (**II-a1**), (**II-b1**), **(III-1), (IV-a1), (IV-b1), (V-a1), (V-b1), (II-a2), (II-b2),** (**III-2**), **(IV-a2), (IV-b2), (V-a2), (V-b2), (11-3), (II-a3),** (**III-3**), **(IV-a3), (IV-b3), (V-a3), (V-b3), (11-4), (II-a4), (111-4), (IV-a4), (IV-b4), (11-5), (II-a5), (III-5), (IV-a5), (IV-b5), (II-6), (II-a6), (111-6), (IV-a6), (IV-b6), (11-7), (II-a7), (111-7), (IV-a7), (IV-b7),** (**II-8**), **(II-a8),** (**III-8**), **(IV-a8),** or **(IV-b8), Y²** is -CH₂-; and **R³** is 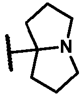 optionally substituted with 1-2 -F (e.g., **R³** is 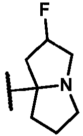 or 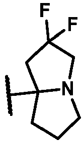 ).

In some embodiments of Formula (**II-a1**), (**II-b1**), **(III-1), (IV-a1), (IV-b1), (V-a1), (V-b1), (II-a2), (II-b2), (111-2), (IV-a2), (IV-b2), (V-a2), (V-b2), (11-3), (II-a3), (III-3), (IV-a3), (IV-b3), (V-a3), (V-b3), (11-4), (II-a4),** (**III-4**), **(IV-a4), (IV-b4), (II-5),** (**II-a5**), **(111-5),** (**IV-a5**), **(IV-b5), (II-6), (II-a6), (111-6), (IV-a6), (IV-b6), (11-7), (II-a7), (111-7), (IV-a7), (IV-b7), (11-8), (II-a8), (III-8), (IV-a8),** or **(IV-b8), R³** is 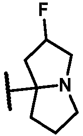 (e.g., 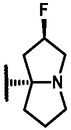 ). In some embodiments, **Y²** is CH₂.

In some embodiments of Formula (**II**) (e.g., Formula (**II-a**), **(II-b),** (**II-a1**), (**II-b1**), (**II-a2),** (**II-b2**), **(11-3), (II-a3), (11-4), (II-a4),** (**II-5**), **(II-a5),** (**II-6**), **(II-a6), (11-7), (II-a7),** (**II-8**), or **(II-a8)),** Formula **(III)** (e.g., Formula (**III-1**), **(111-2),** (**III-3**), (**III-4**), **(III-5), (111-6),** (**III-**7), or **(III-8)),** Formula **(IV)** (e.g., Formula **(IV-a), (IV-b), (IV-c), (IV-a1),** (**IV-b1**), **(IV-a2), (IV-b2), (IV-a3), (IV-b3), (IV-a4), (IV-b4), (IV-a5), (IV-b5), (IV-a6), (IV-b6), (IV-a7), (IV-b7), (IV-a8),** or **(IV-b8)),** Formula **(V)** (e.g., Formula **(V-a)** or **(V-b), (V-a1), (V-c), (V-d), (V-b1**), **(V-a2), (V-b2), (V-a3),** or **(V-b3)),** the 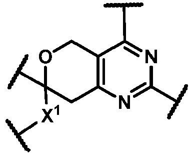 moiety is

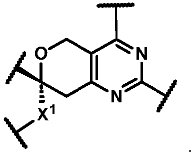

Also provided herein are compounds of Formula **(VI):** 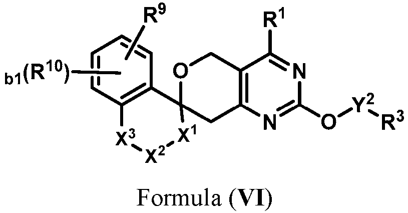 or pharmaceutically acceptable salts thereof, wherein:
**R¹** is a 4-10 membered heterocyclyl substituted with -CN, -(C₁₋₃ alkylene)-CN, or - (C₃₋₆ cycloalkylene)-CN on a ring carbon atom, wherein the heterocyclyl is further optionally substituted with 1-3 **R⁷**;
wherein each **R⁷** is independently selected from the group consisting of **R^{a}** and **R^{b}**;
**X¹** is selected from the group consisting of S(O)₀₋₂, CH₂, CH**R^{L}**, C(**R^{L}**)₂, and O;
**X²** and **X³** are independently selected from the group consisting of: CH₂, CH**R^{L}**, C(**R^{L}**)₂, O, and S(O)₀₋₂, provided that no more than one of **X¹**, **X²**, and **X³** is selected from the group consisting of: O and S(O)₀₋₂;
**b1** is 0, 1, or 2;
**R⁹** is selected from the group consisting of: H, N**R^{d}R^{e}**, -OH, and halo;
each **R¹⁰** is independently selected from the group consisting of **R^{a}** and **R^{b}**;
each **R^{L}** is independently selected from the group consisting of C₁₋₃ alkoxy, -F, -CN, and C₁₋₃ alkyl optionally substituted with 1-3 **R^{c}**; or
one pair of **R^{L}** on the same or different ring carbon atom(s) taken together with the ring atom(s) connecting them form a C₃₋₆ cycloalkyl ring;
**Y²** is a bond or a straight-chain C₁₋₆ alkylene optionally substituted with 1-6 **R^{Y}**;
each **R^{Y}** is independently selected from the group consisting of: halo, cyano, -OH, oxo, C₁₋₆ alkoxy, C₁₋₆ haloalkoxy, C₁₋₆ alkyl, and C₁₋₆ haloalkyl, or
one pair of **R^{Y}** on the same or different carbon atom(s) taken together with the atom(s) connecting them forms a C₃₋₆ cycloalkyl ring or 4-6 membered heterocyclyl ring, each of which is optionally substituted with 1-3 independently selected C₁₋₃ alkyl;
**R³** is selected from the group consisting of:
   **(a)** a 4-15 membered heterocyclyl optionally substituted with 1-6 substituents independently selected from the group consisting of: **R^{a}** and **R^{b}**; and
   **(b)** -N**R^{d}R^{e}**;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   **(f)** -C₁₋₆ haloalkoxy;
   **(g)** -N**R^{d}R^{e}**;
   **(h)** C(=O)C₁₋₆ alkyl;
   **(i)** C(=O)C₁₋₆ haloalkyl;
   **(j)** C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   **(o)** S(O)₀₋₂(C₁₋₆ haloalkyl);
   **(p)** S(O)₁₋₂N(**R^{f}**)₂; and
   **(q)** C₁₋₆ alkyl, C₂₋₆ alkenyl, or C₂₋₆ alkynyl, each optionally substituted with 1-6 **R^{c}**;
each **R^{b}** is independently selected from the group consisting of: **-(L^{b})_{b}-R^{b1}** and **-R^{b1}**, wherein:
   b is 1, 2, or 3;
   each **-L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e}**, C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)₁₋₂N(**R^{f}**)₂;
   each **R^{d}** and **R^{e}** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂₋.

In some embodiments, the compounds of Formula **(VI)** are other than Compound Nos. **R149, R149a, R149b, R149c, R173, R173a, R174,** and **R174a**.

In some embodiments, the compounds of Formula **(VI)** are other than compounds depicted in **Table C1** of International Patent Application PCT/US2023/080513 (published as WO 2024/112654), or pharmaceutically acceptable salts thereof.

In some embodiments of Formula **(VI), R¹** is a 4-10 membered heterocyclyl substituted with -CN or -(C₁₋₃ alkylene)-CN on a ring carbon atom, wherein the heterocyclyl is further optionally substituted with 1-3 **R⁷**.

In some embodiments of Formula **(VI), R¹** is a 6-8 membered heterocyclyl substituted with -CN or -(C₁₋₃ alkylene)-CN on a ring carbon atom, wherein:
the heterocyclyl has one ring nitrogen atom and 0-1 ring oxygen atom; and
the heterocyclyl is further optionally substituted with 1-3 **R⁷**.

In some embodiments of Formula **(VI),** each **R⁷** is independently selected from the group consisting of: -OH; -CN; -F; and C₁₋₃ alkyl optionally substituted with 1-3 **R^{c}**.

In some embodiments of Formula **(VI), R¹** is selected from the group consisting of: 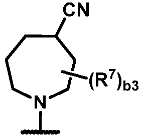 and 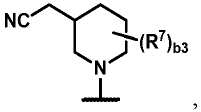 wherein **b3** is 0, 1, or 2. In some embodiments, each **R⁷** is independently selected from the group consisting of: -OH; -CN; -F; and C₁₋₃ alkyl optionally substituted with 1-3 **R^{c}** (e.g., C₁₋₃ alkyl optionally substituted with 1-3 -F). In some embodiments, **b3** is 0.

For example, **R¹** can be 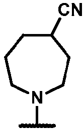 or 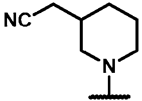 in Formula **(VI).**

In some embodiments of Formula **(VI), R⁹** is -N**R^{d}R^{e}** or OH (e.g., -NH₂).

In some embodiments of Formula **(VI), b1** is 2.

In some embodiments, the compounds of Formula **(VI)** are compounds of Formula **(VIa):** 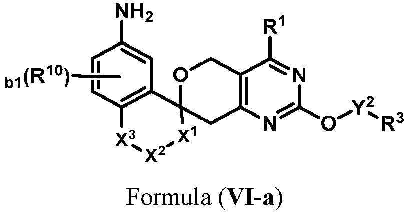 or pharmaceutically acceptable salts thereof, wherein:
each **R¹⁰** is independently selected from the group consisting of: -Cl, -F, -CN, and C₁₋₃ alkyl optionally substituted with 1-3 **R^{c}.**

In some embodiments of Formula **(VI-a), b1** is 1 or 2. For example, **b1** can be 2.

In some embodiments, the compounds of Formula **(VI)** are compounds of Formula **(VI-b):** 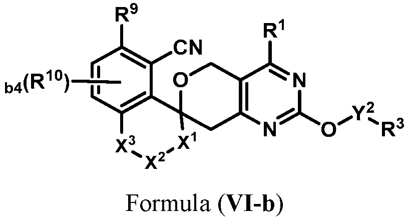 or pharmaceutically acceptable salts thereof, wherein:
**b4** is 0 or 1 (e.g., 1); and
**R¹⁰** is selected from the group consisting of: -Cl, -F, -CN, and C₁₋₃ alkyl optionally substituted with 1-3 **R^{c}.**

In some embodiments of Formula **(VI-b), R⁹** is -N**R^{d}R^{e}**.

In some embodiments, the compounds of Formula **(VI)** are compounds of Formula **(VI-c):** 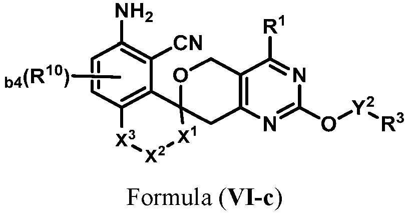 or pharmaceutically acceptable salts thereof, wherein:
**b4** is 0 or 1 (e.g., 1); and
**R¹⁰** is selected from the group consisting of: -Cl, -F, -CN, and C₁₋₃ alkyl optionally substituted with 1-3 **R^{c}.**

In some embodiments of Formula **(VI)** (e.g., Formula **(VI-a), (VI-b),** or **(VI-c)), X¹** is CH₂ or CH**R^{L}**.

In some embodiments of Formula **(VI)** (e.g., Formula **(VI-a), (VI-b),** or (V**I-c**)), **X²** and **X³** are independently selected from the group consisting of: CH₂, CH**R^{L}**, and C(**R^{L}**)₂.

In some embodiments of Formula **(VI)** (e.g., Formula **(VI-a), (VI-b),** or **(VI-c)), X¹** is CH₂; and **X²** and **X³** are both CH₂.

In some embodiments of Formula **(VI)** (e.g., Formula **(VI-a), (VI-b),** or **(VI-c)),** at least one (e.g., one) of **X¹**, **X²**, and **X³** is selected from the group consisting of: CH**R^{L}** and C(**R^{L}**)₂.

In some embodiments of Formula **(VI)** (e.g., Formula **(VI-a), (VI-b),** or **(VI-c)), X¹** is CH₂; and **X²** and **X³** are independently selected from the group consisting of: CH₂, CH**R^{L}**, and C(**R^{L}**)₂, provided that 1-2 of **X²** and **X³** is independently CH**R^{L}** or C(**R^{L}**)₂.

In some embodiments of Formula **(VI)** (e.g., Formula **(VI-a), (VI-b),** or **(VI-c)), X¹** is CH₂; **X²** is CH₂; and **X³** is CH**R^{L}**.

In some embodiments of Formula **(VI)** (e.g., Formula **(VI-a), (VI-b),** or **(VI-c)), X¹** is CH₂; one of **X²** and **X³** is -O-; and the other of **X²** and **X³** is selected from the group consisting of: CH₂, CH**R^{L}**, and C(**R^{L}**)₂.

In some embodiments of Formula **(VI)** (e.g., Formula **(VI-a), (VI-b),** or **(VI-c)), X¹** is CH₂; **X²** is -O-; and **X³** is selected from the group consisting of: CH₂, CH**R^{L}**, and C(**R^{L}**)₂.

In some embodiments of Formula **(VI)** (e.g., Formula **(VI-a), (VI-b),** or **(VI-c)),** each **R^{L}** is independently selected from the group consisting of: CH₃, CF₃, CHF₂, and CH₂F (e.g., each **R^{L}** is CH₃).

In some embodiments, the compounds of Formula **(VI)** are compounds of Formula **(VI-**d): 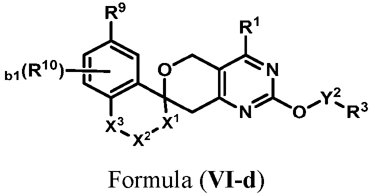 or pharmaceutically acceptable salts thereof, wherein:
**X¹** is selected from the group consisting of S(O)₀₋₂, CH₂, CH**R^{L}**, C(**R^{L}**)₂, and O;
**X²** and **X³** are independently selected from the group consisting of: CH₂, CH**R^{L}**, C(**R^{L}**)₂, O, and S(O)₀₋₂, provided that at least one of **X¹**, **X²**, and **X³** is CH**R^{L}** or C(**R^{L}**)2; and
further provided that no more than one of **X¹**, **X²**, and **X³** is selected from the group consisting of: O and S(O)₀₋₂; and
each **R¹⁰** is independently selected from the group consisting of: -Cl, -F, -CN, and C₁₋₃ alkyl optionally substituted with 1-3 **R^{c}.**

In some embodiments, the compounds of Formula (VI) are compounds of Formula **(VI-** 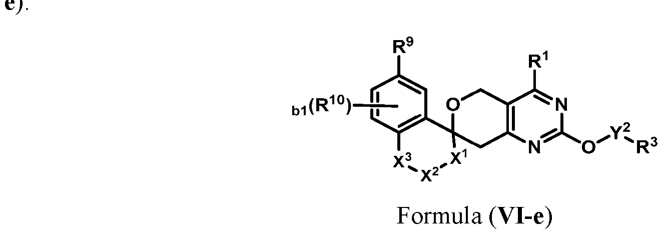 or pharmaceutically acceptable salts thereof, wherein:
**X¹** is selected from the group consisting of S(O)₀₋₂, CH₂, CH**R^{L}**, C(**R^{L}**)₂, and O;
**X²** and **X³** are independently selected from the group consisting of: CH₂, CH**R^{L}**, C(**R^{L}**)₂, O, and S(O)₀₋₂, provided that 2-3 of **X¹**, **X²**, and **X³** are independently CH**R^{L}** or C(**R^{L}**)₂;
one pair of **R^{L}** on the same or different ring carbon atom(s) taken together with the ring atom(s) connecting them form a C₃₋₆ cycloalkyl ring; and
each additional **R^{L}** is independently selected from the group consisting of: C₁₋₃ alkoxy, -F, -CN, and C₁₋₃ alkyl optionally substituted with 1-3 **R^{c}**; and
each **R¹⁰** is independently selected from the group consisting of: -Cl, -F, -CN, and C₁₋₃ alkyl optionally substituted with 1-3 **R^{c}.**

In some embodiments of Formula **(VI-d)** or **(VI-e), R⁹** is NH₂.

In some embodiments of Formula **(VI-d)** or **(VI-e), b1** is 1 or 2. For example, **b1** can
be 2.

In some embodiments of Formula **(VI-d)** or **(VI-e),** the 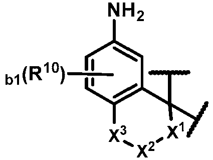 moiety is selected from the group consisting of: 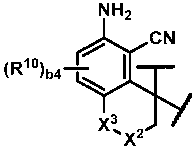 and 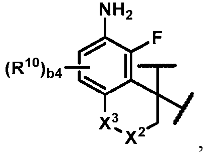 wherein:
**b4** is 0 or 1;
**X²** is -O- or -CH₂-;
**X³** is -CH₂- or -CH**R^{L}-**, wherein **R^{L}** is C₁₋₃ alkyl (e.g., methyl); and
**R¹⁰** is selected from the group consisting of: -Cl, -F, -CN, and C₁₋₃ alkyl optionally substituted with 1-3 **R^{c}**.

In some embodiments of Formula **(VI)** (e.g., Formula **(VI-a), (VI-b), (VI-c), (VI-d),** or **(VI-e)), Y²** is -CH₂-; and **R³** is a 4-10 membered heterocyclyl having one ring nitrogen atom and 0-1 additional ring heteroatom selected from the group consisting of oxygen and nitrogen, wherein the heterocyclyl is optionally substituted with 1-6 **R^{a}**.

In some embodiments of Formula **(VI)** (e.g., Formula **(VI-a), (VI-b), (VI-c), (VI-d),** or **(VI-e)), Y²** is -CH₂-; and **R³** is 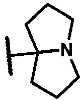 optionally substituted with 1-2 -F.

In some embodiments of Formula **(VI)** (e.g., Formula **(VI-a), (VI-b), (VI-c), (VI-d),** or **(VI-e)), R³** is 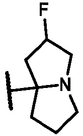 (e.g., 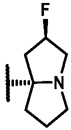 ).

In some embodiments of Formula **(VI)** (e.g., Formula **(VI-a), (VI-b), (VI-c), (VI-d),** or **(VI-e)),** the 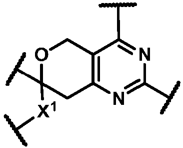 moiety is

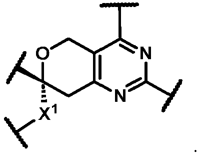

In some embodiments, compounds of Formula **(II), (III), (IV), (V),** and/or **(VI)** are selected from the group consisting of the compounds in **Table C3,** or pharmaceutically acceptable salts thereof.

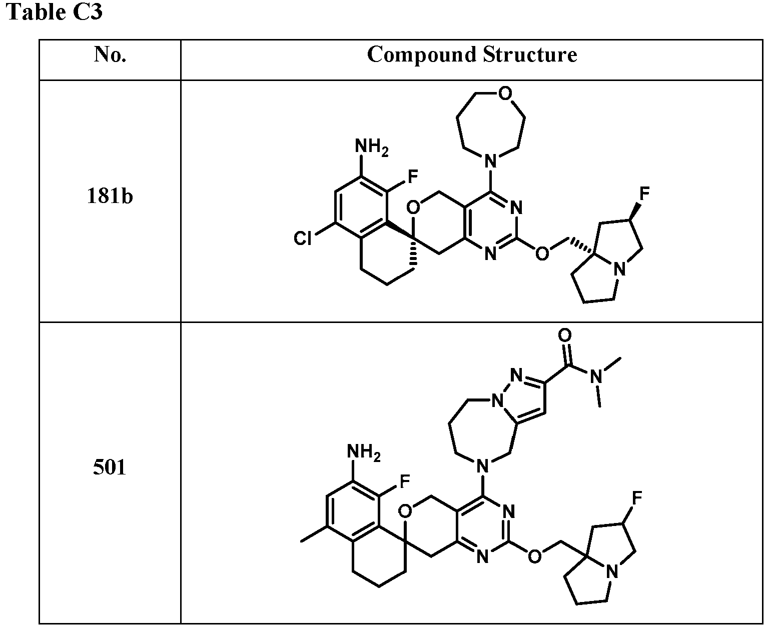

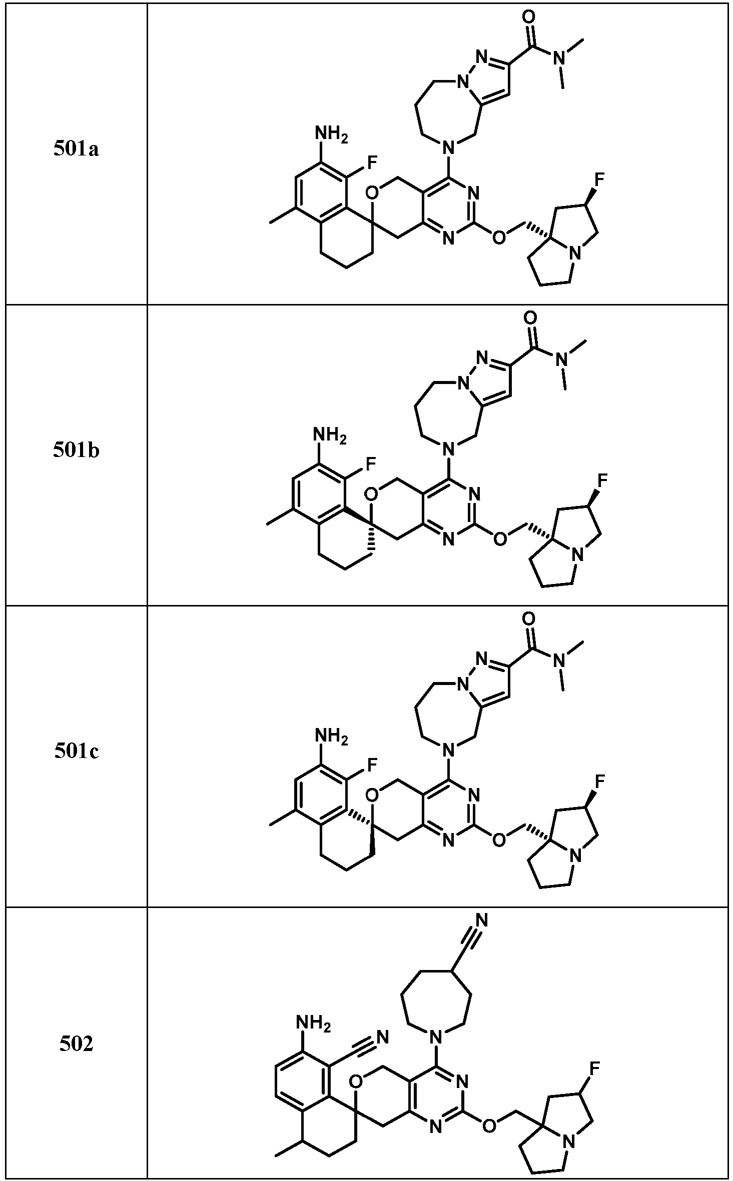

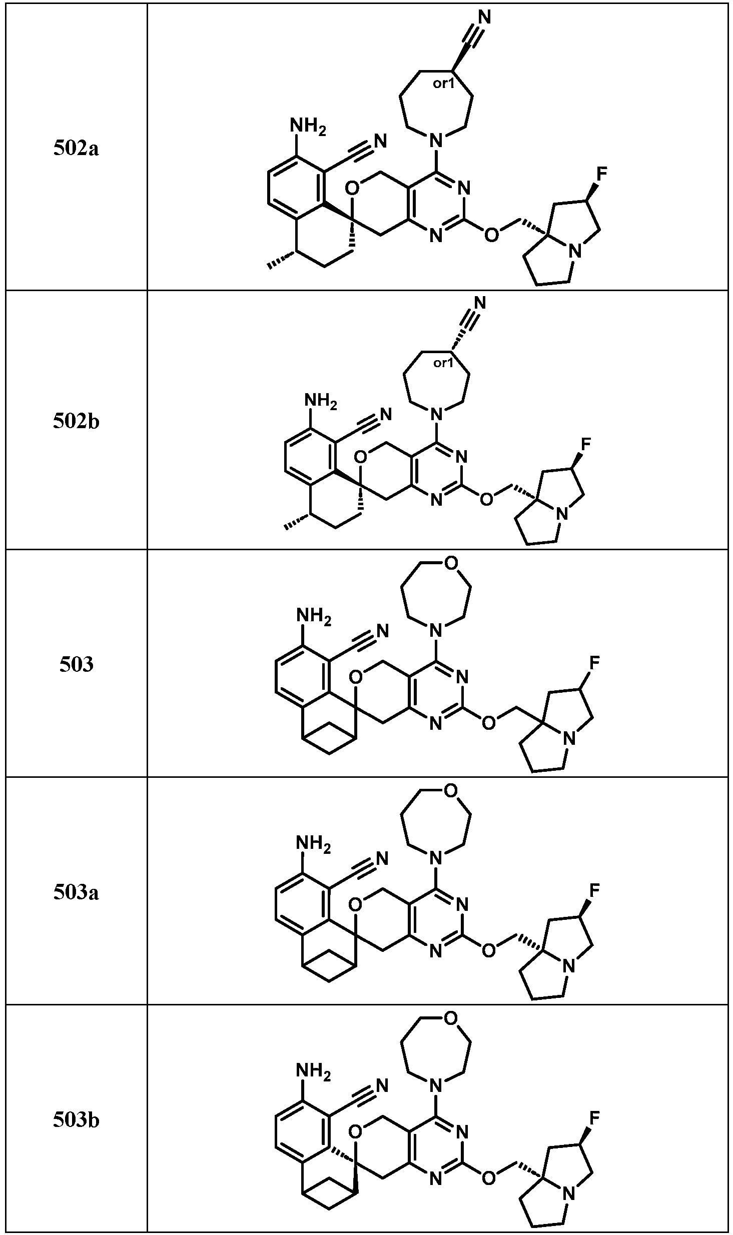

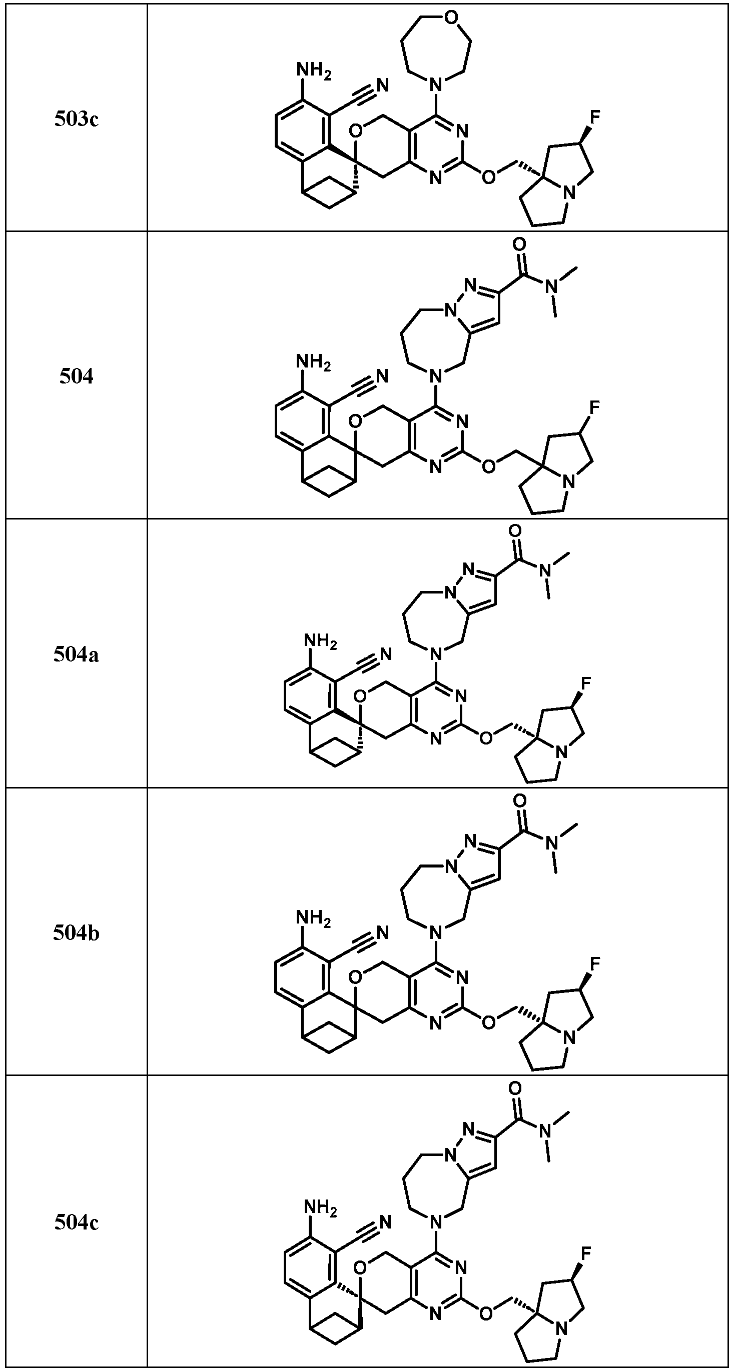

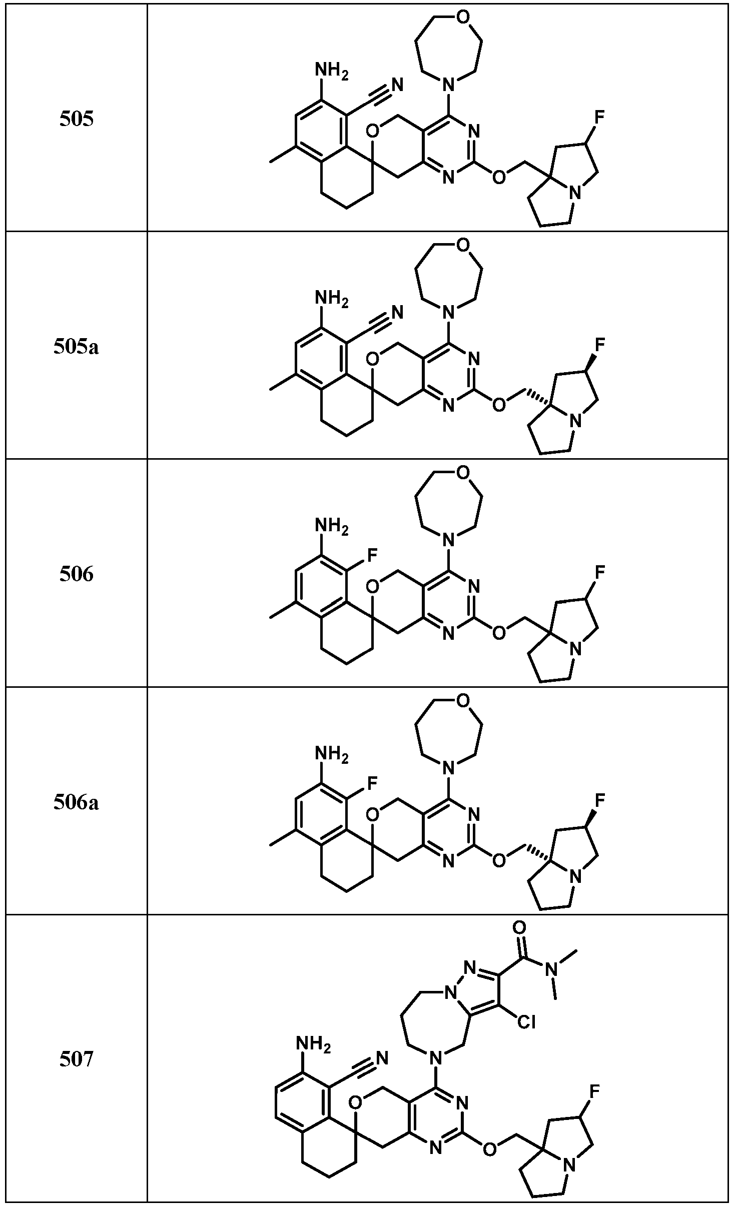

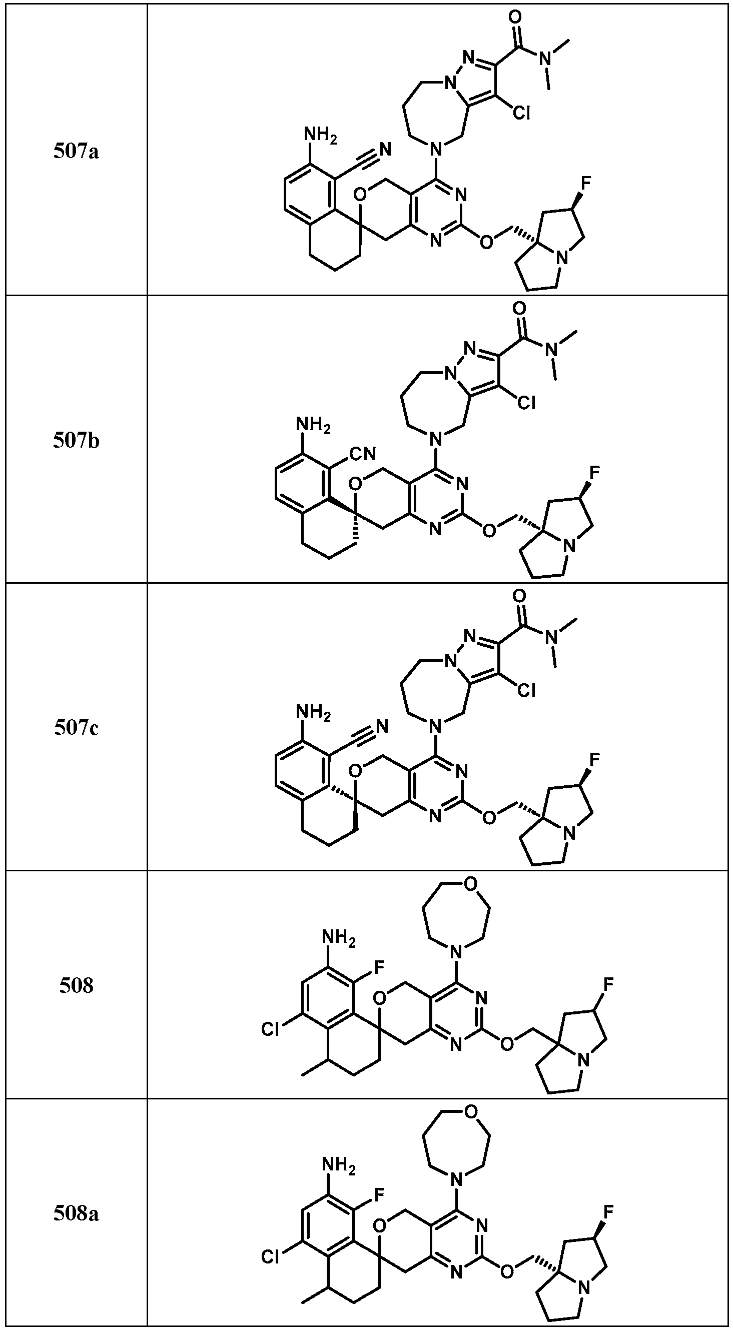

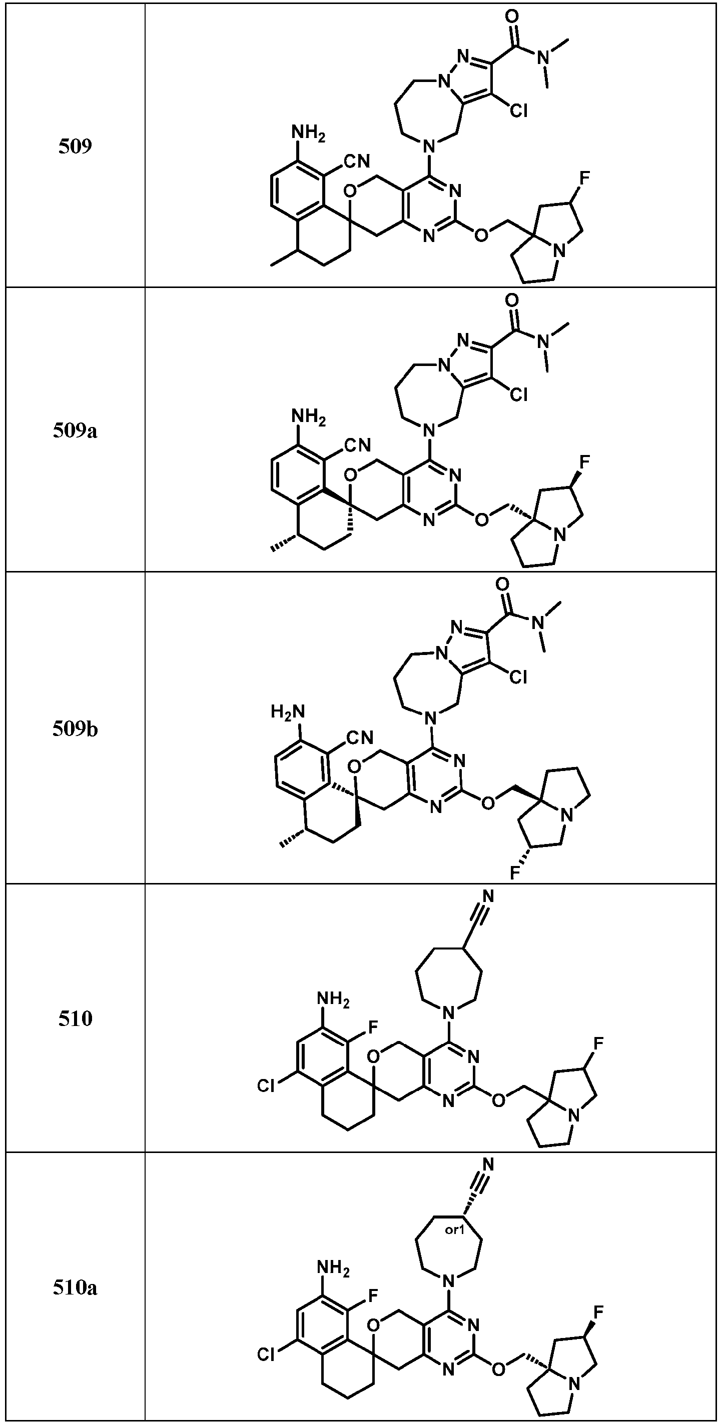

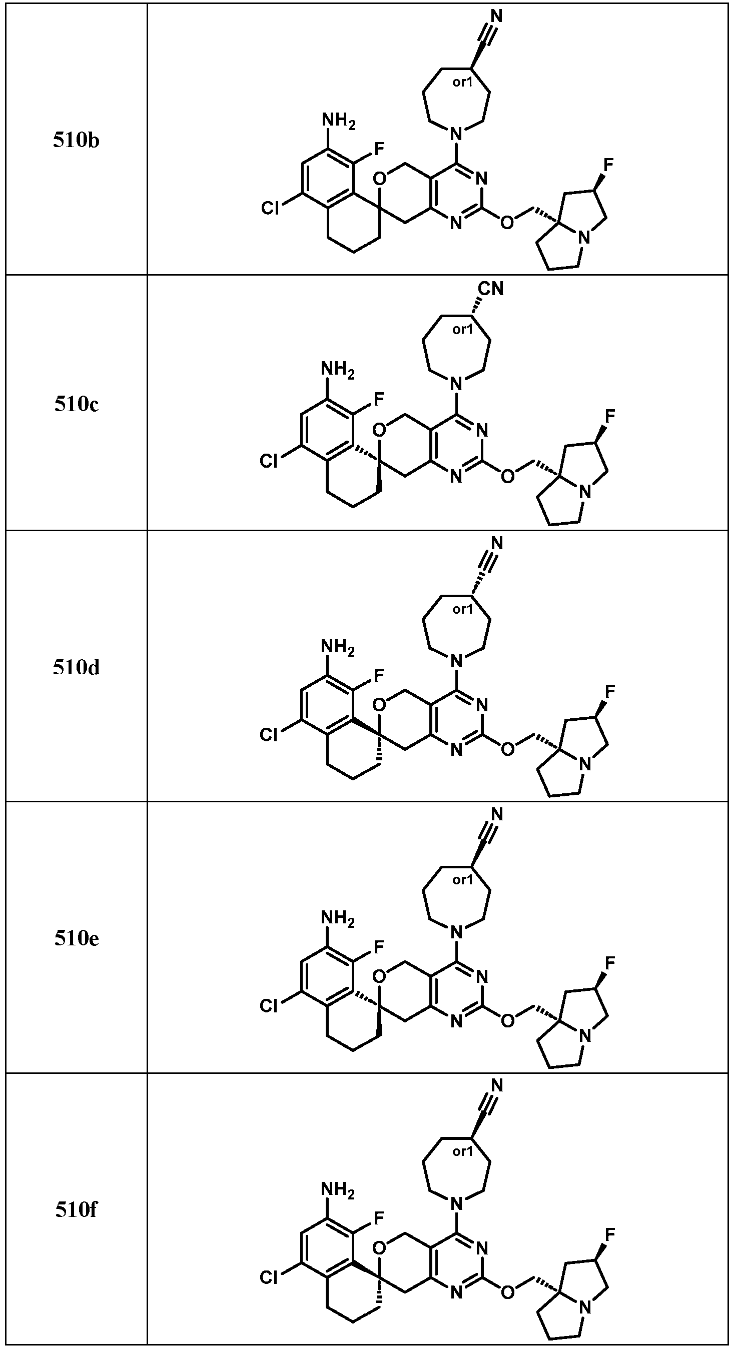

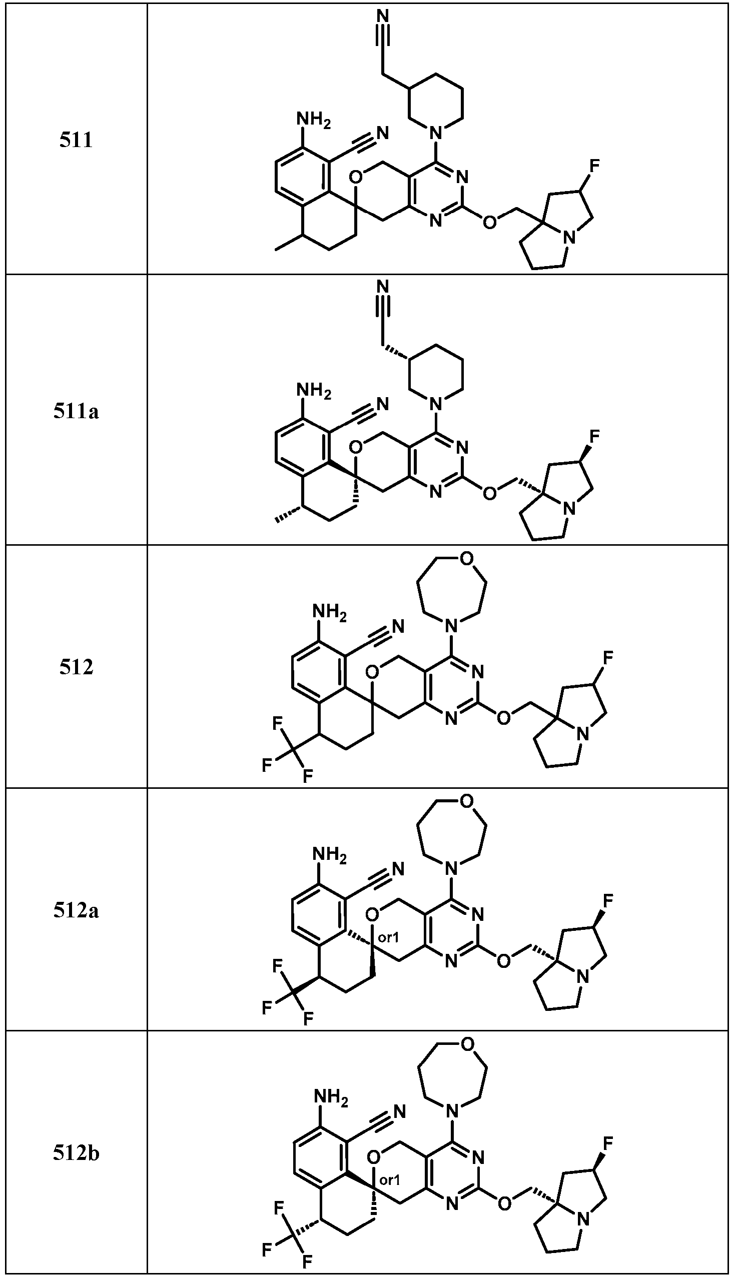

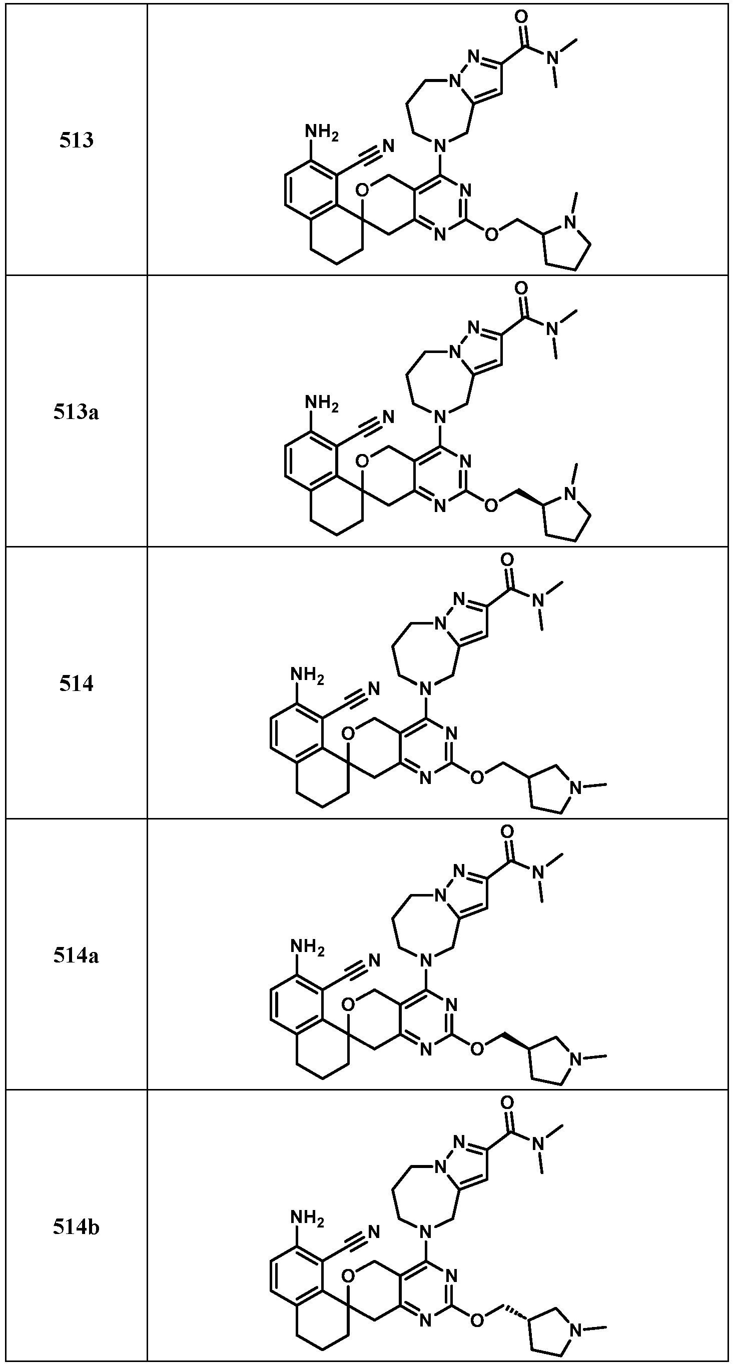

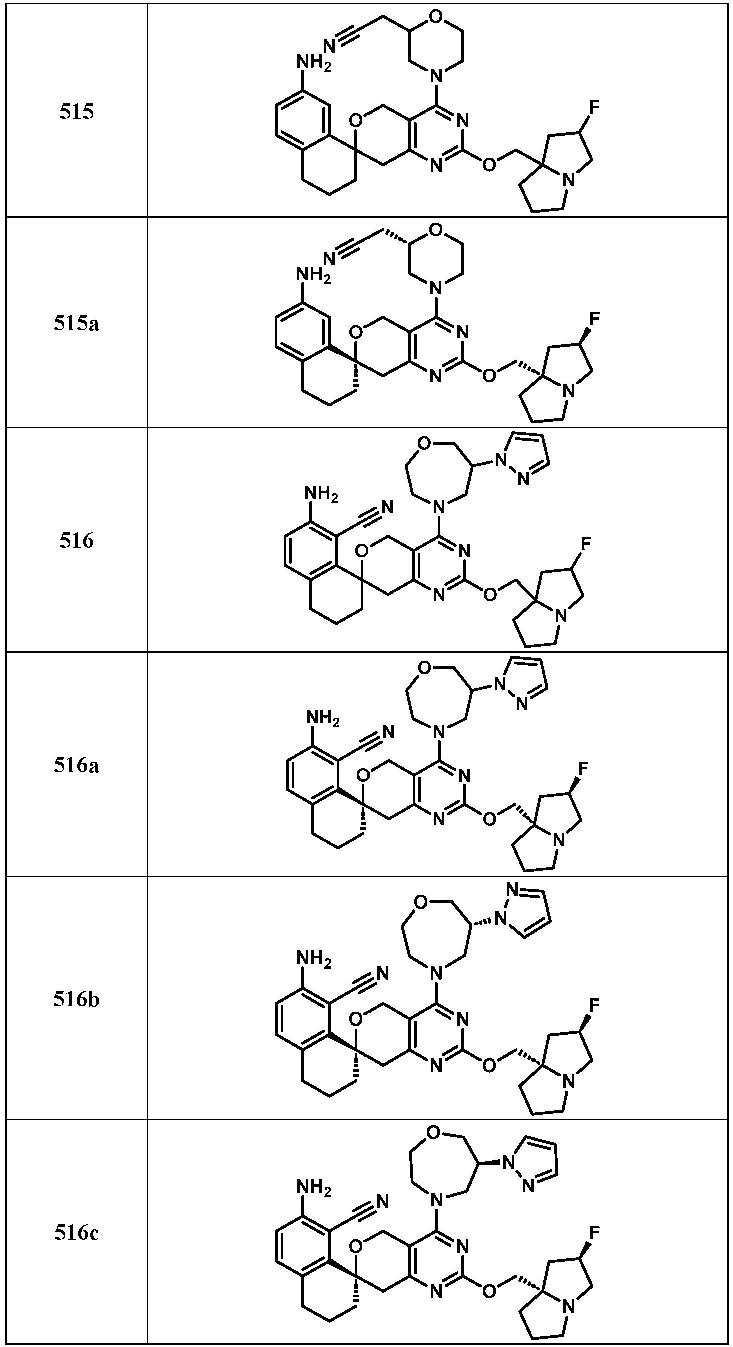

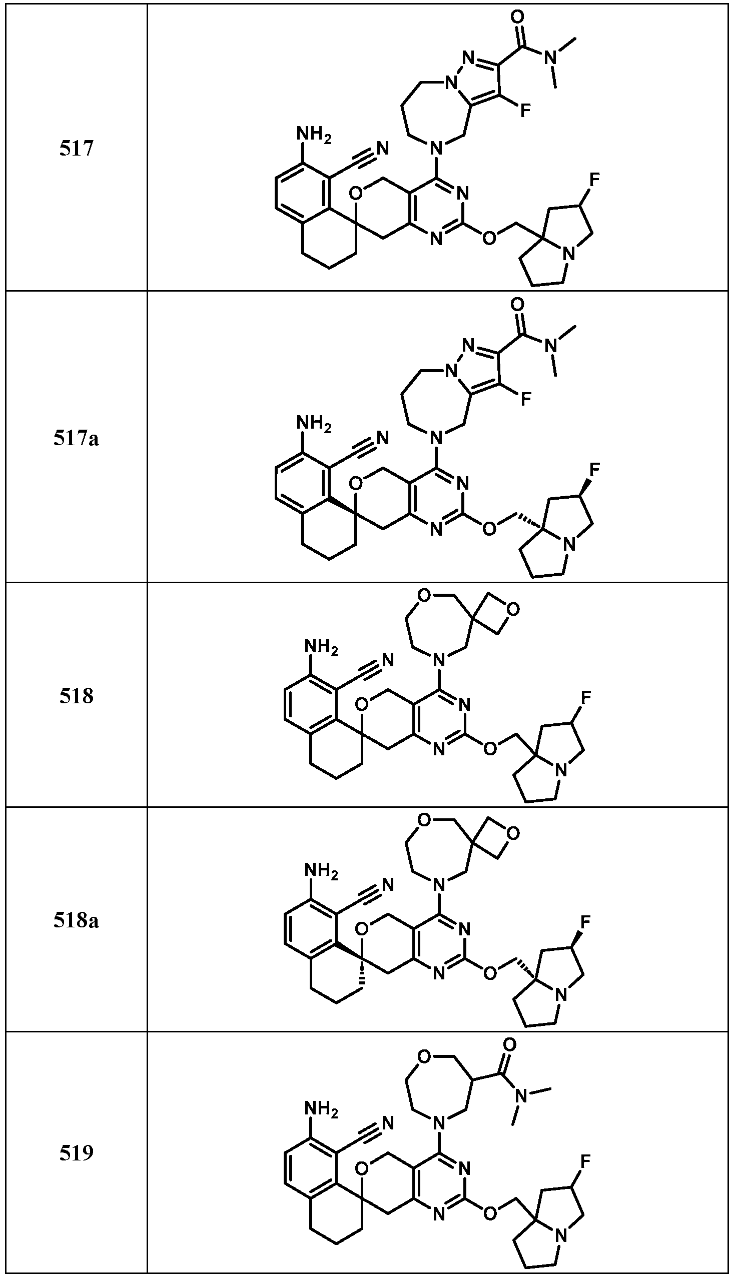

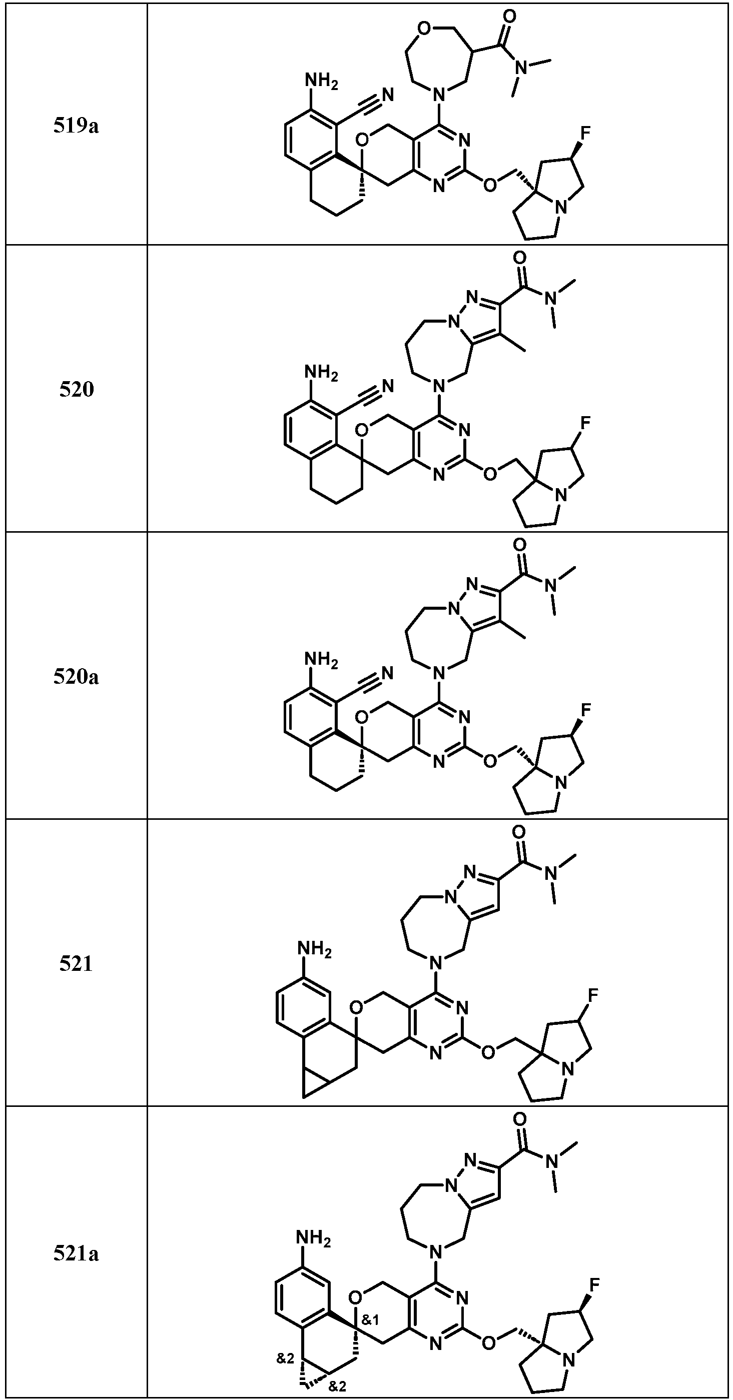

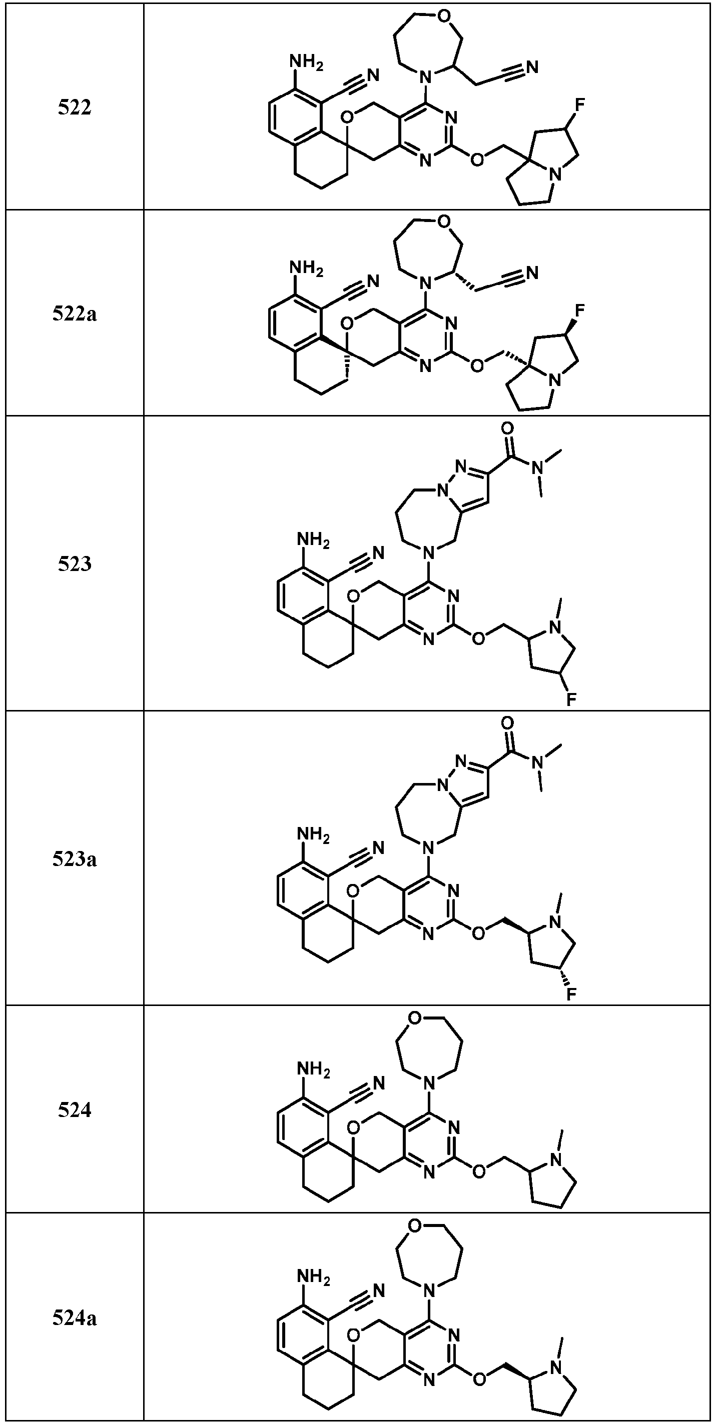

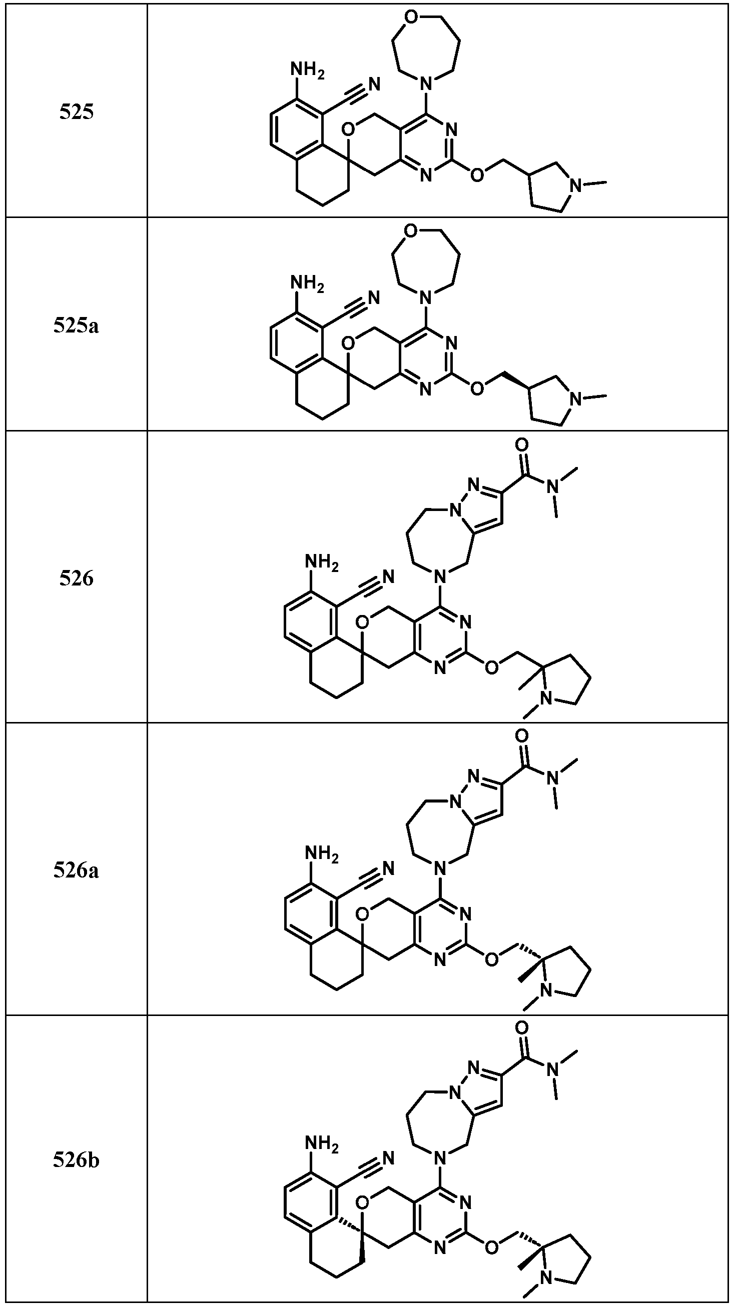

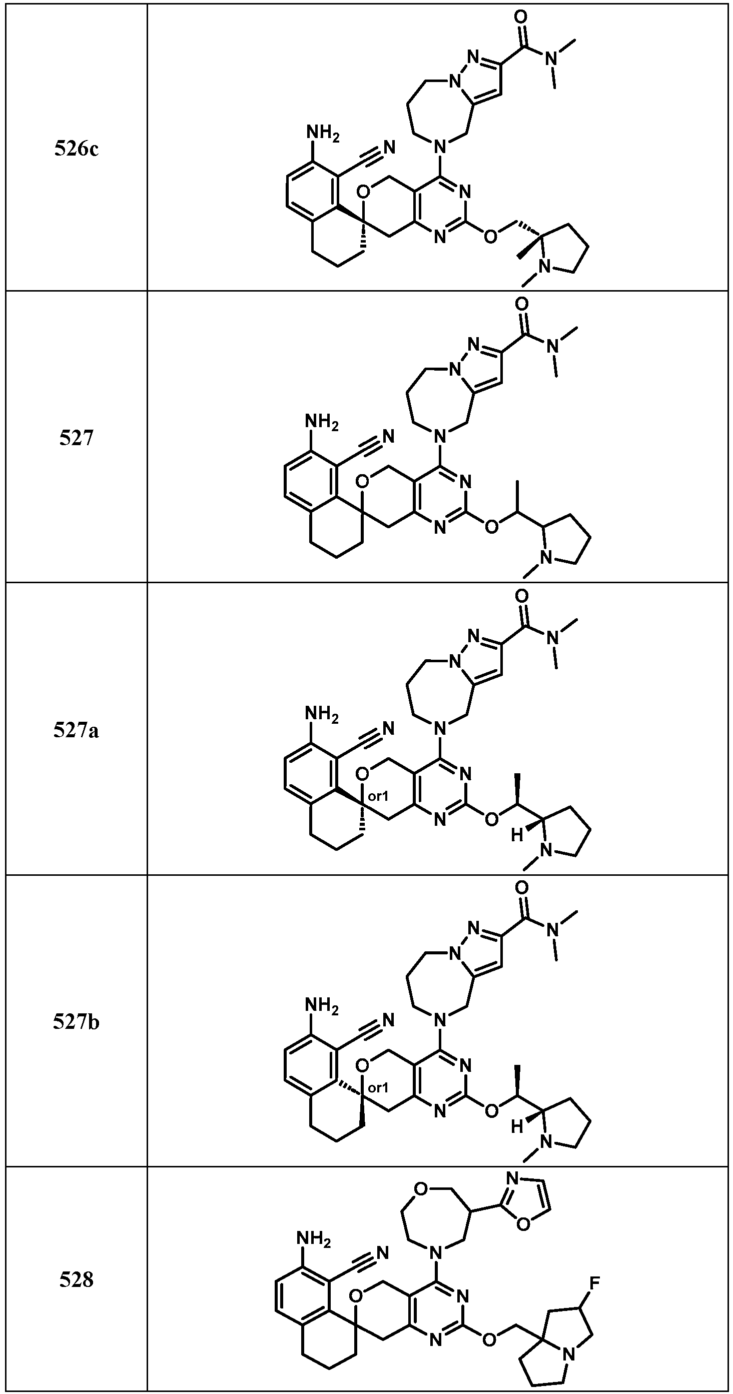

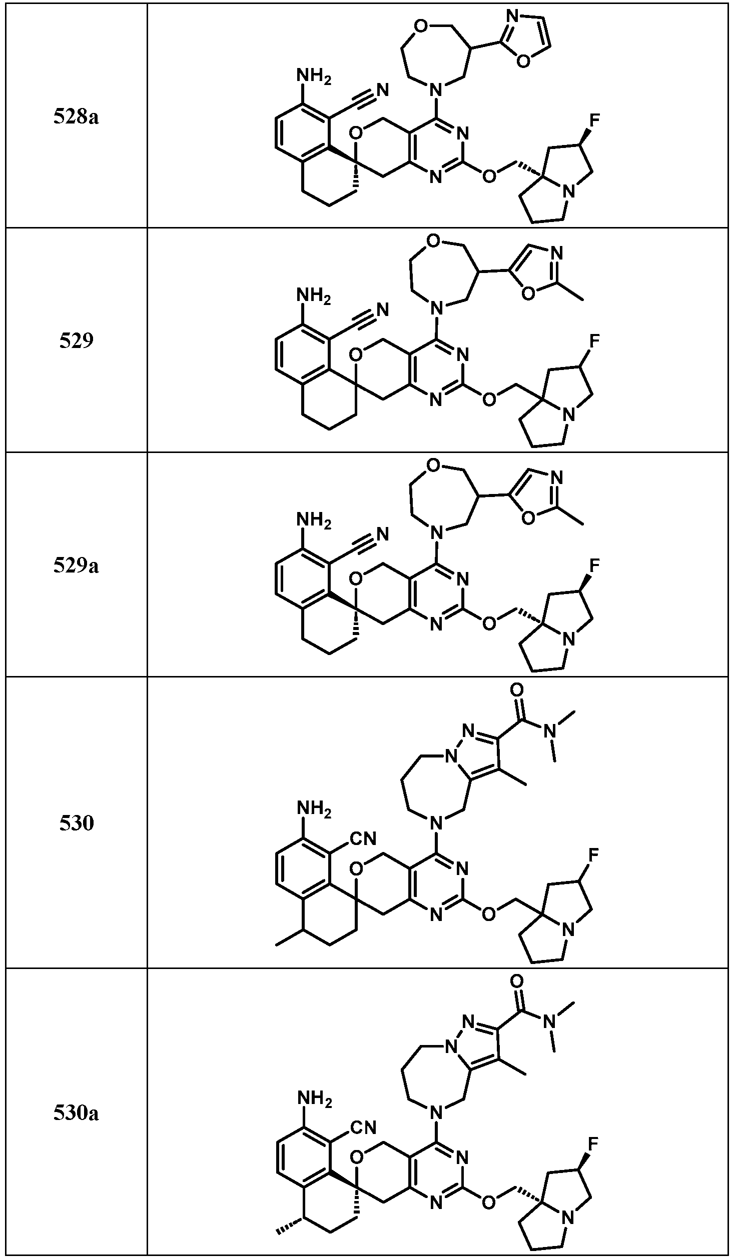

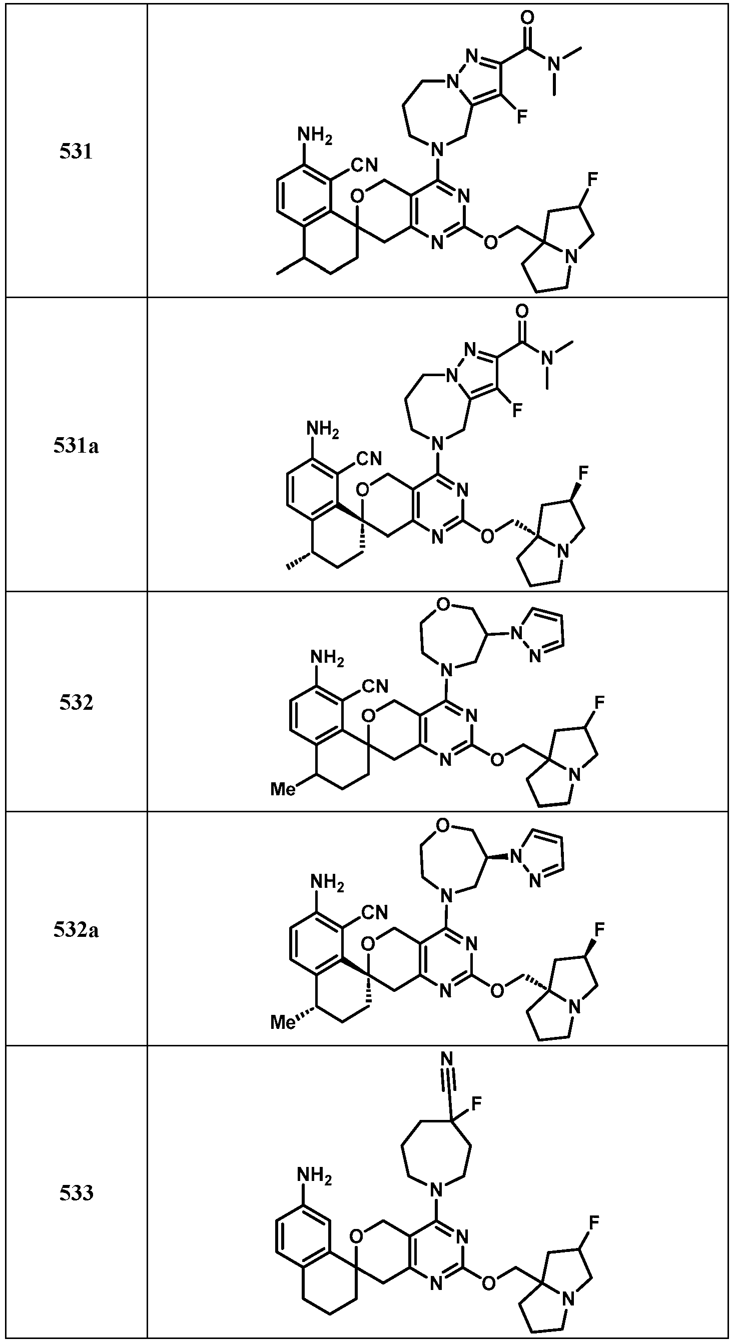

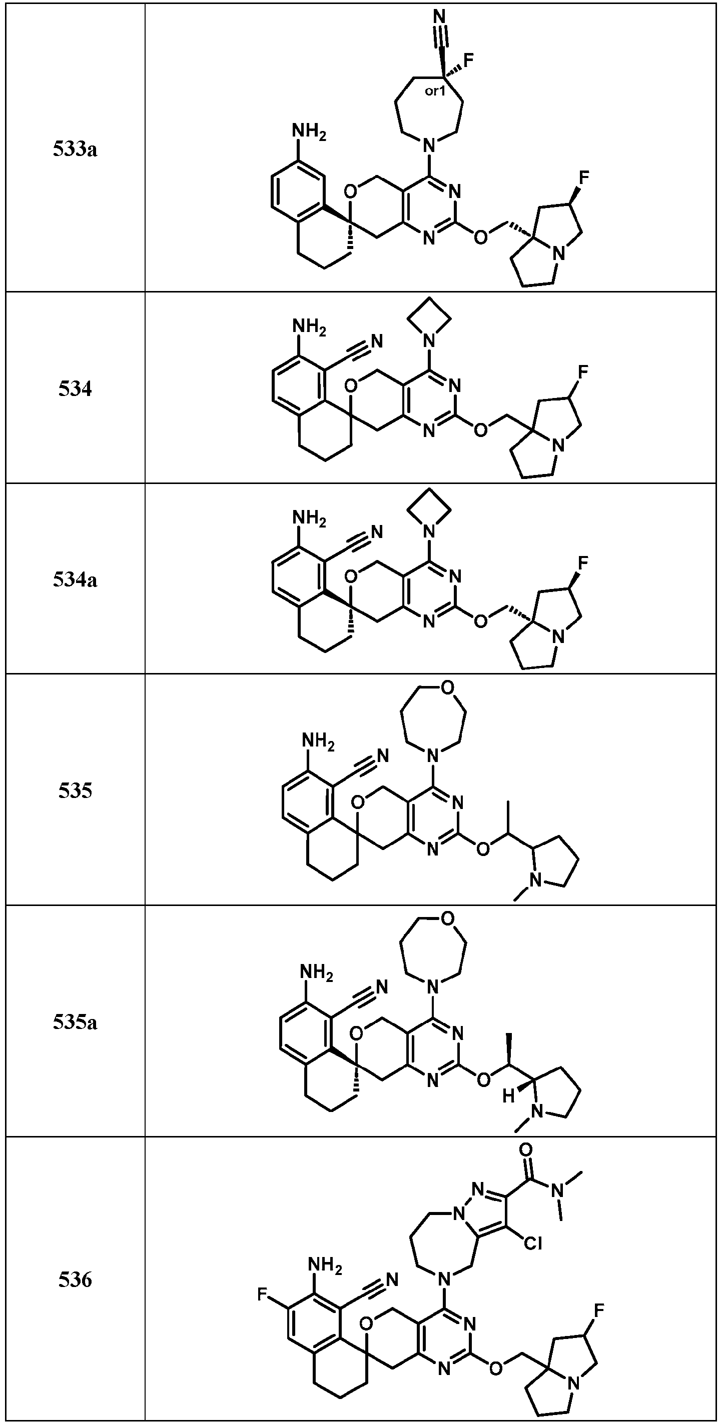

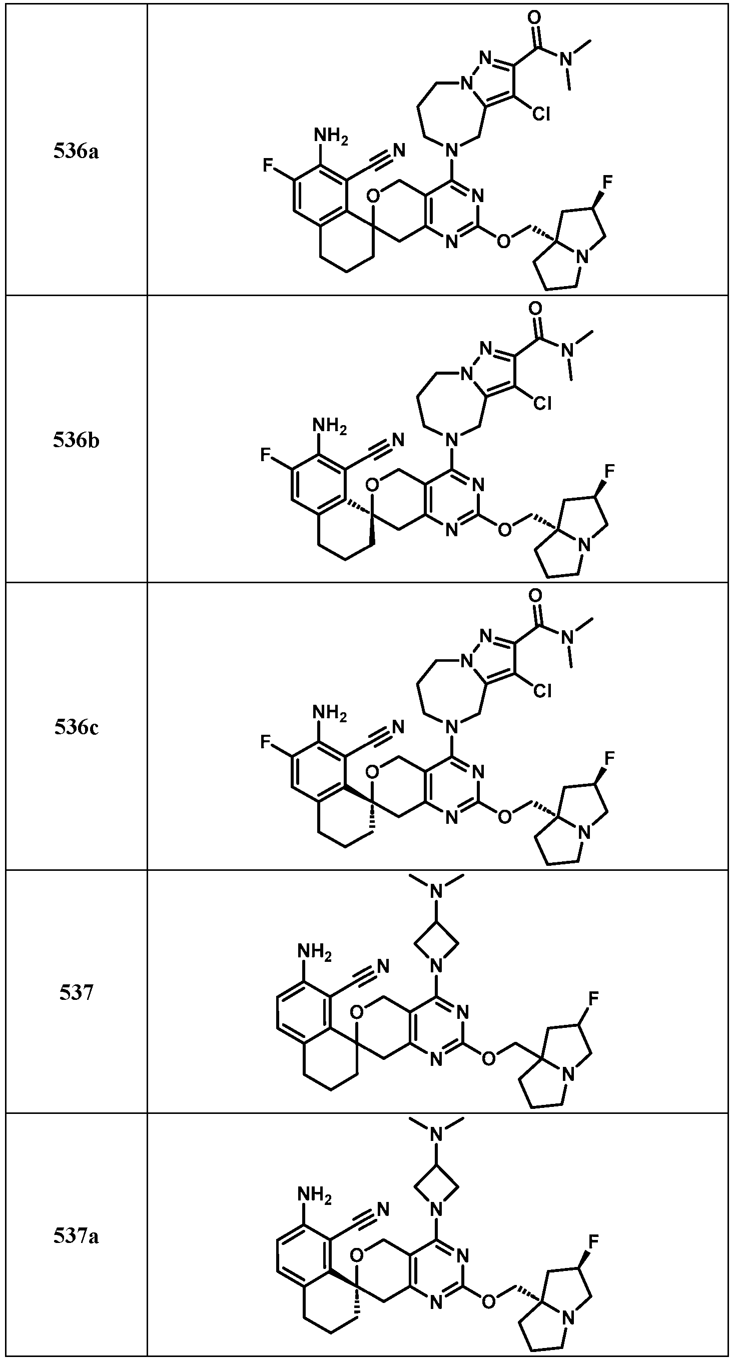

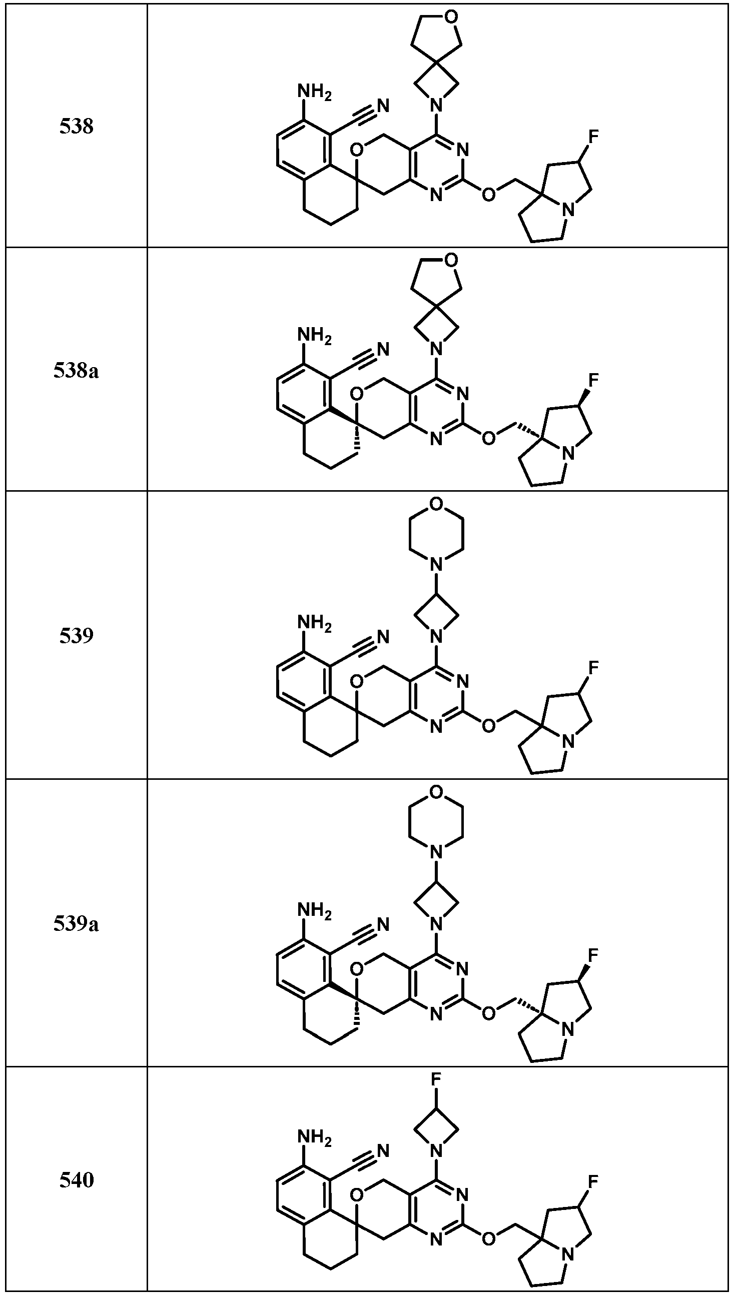

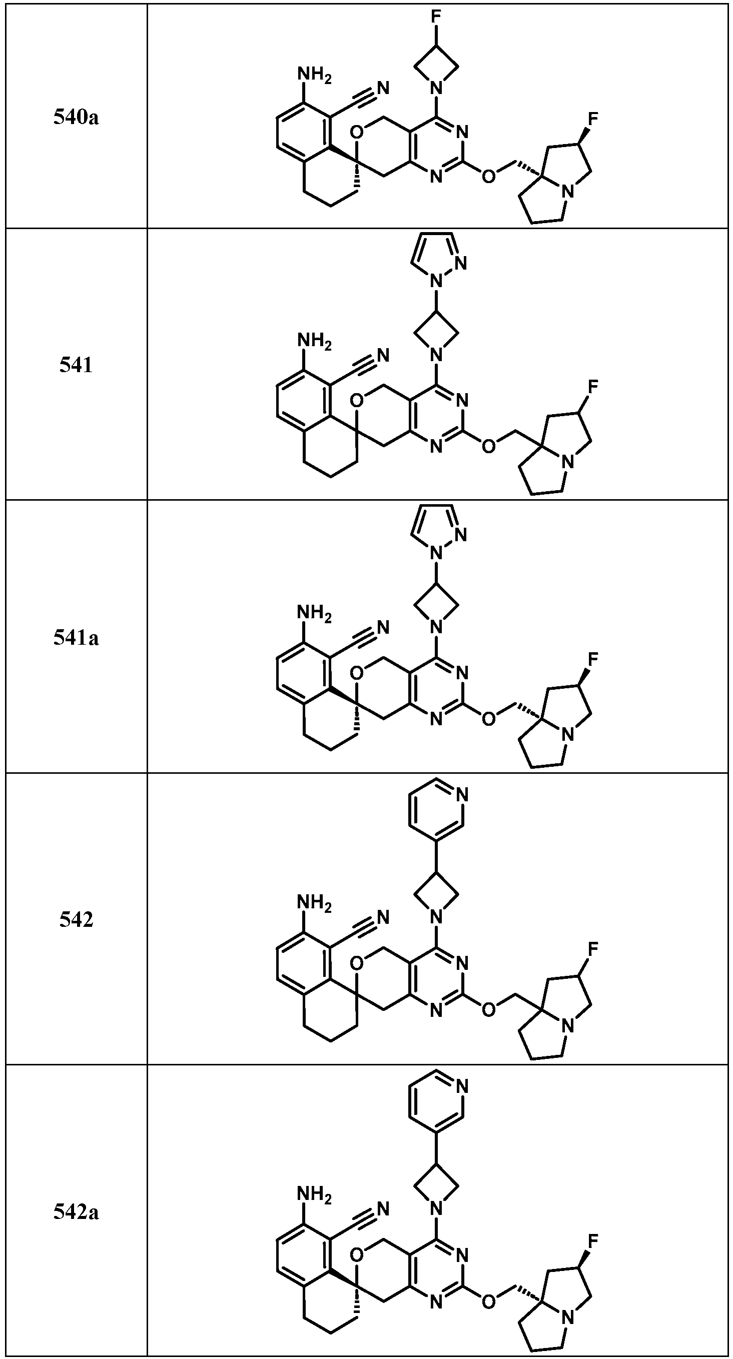

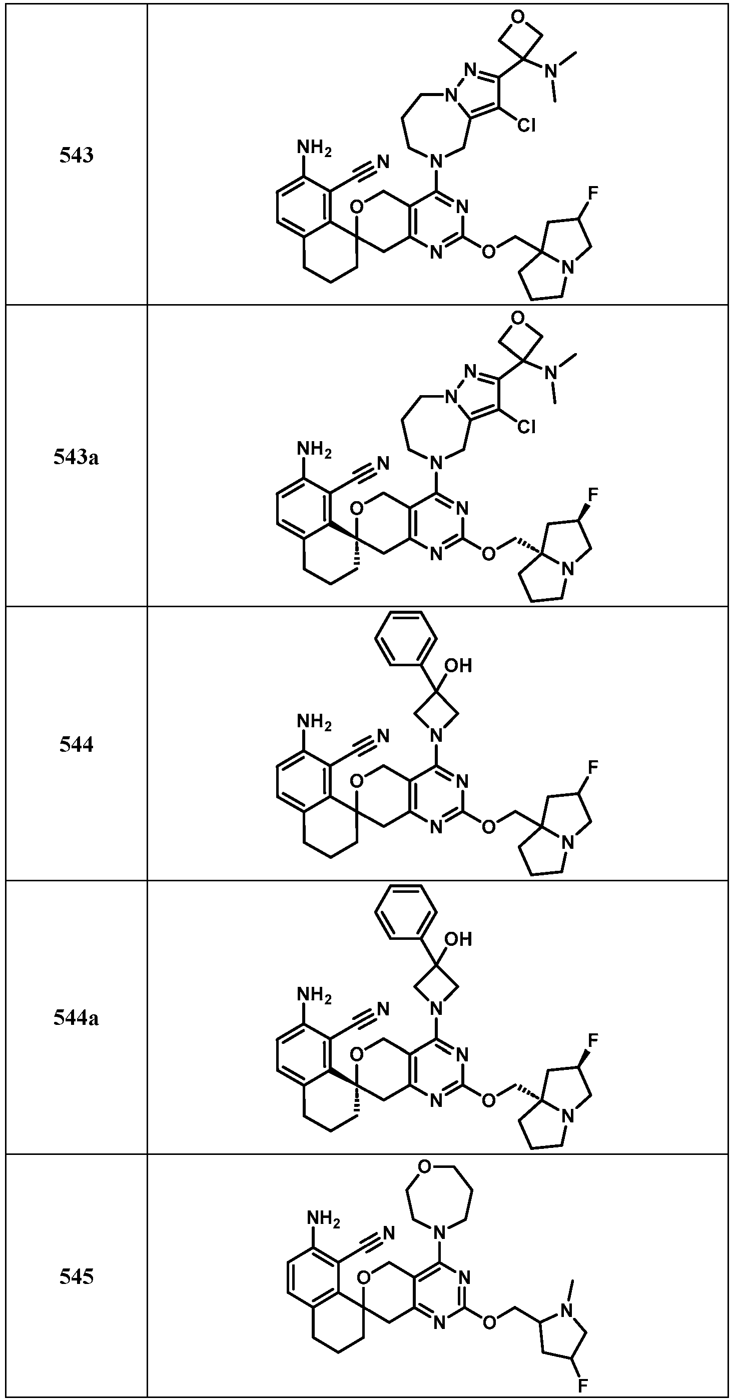

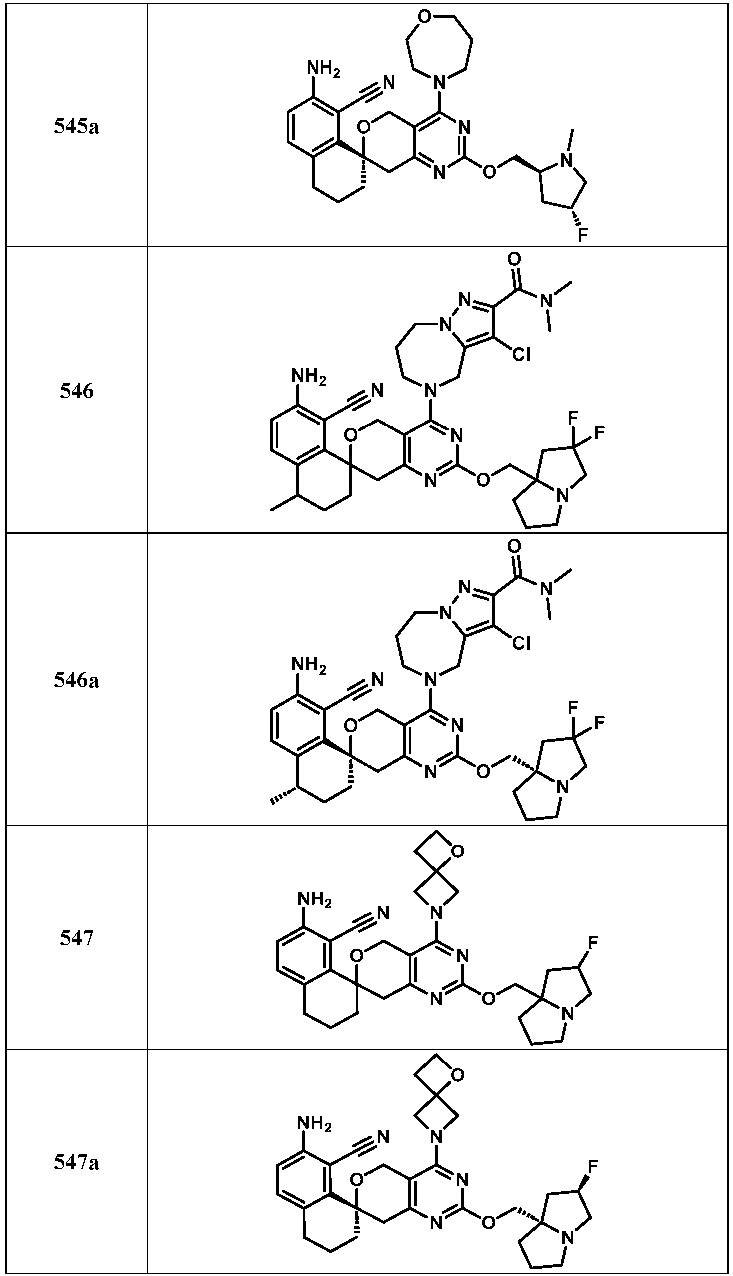

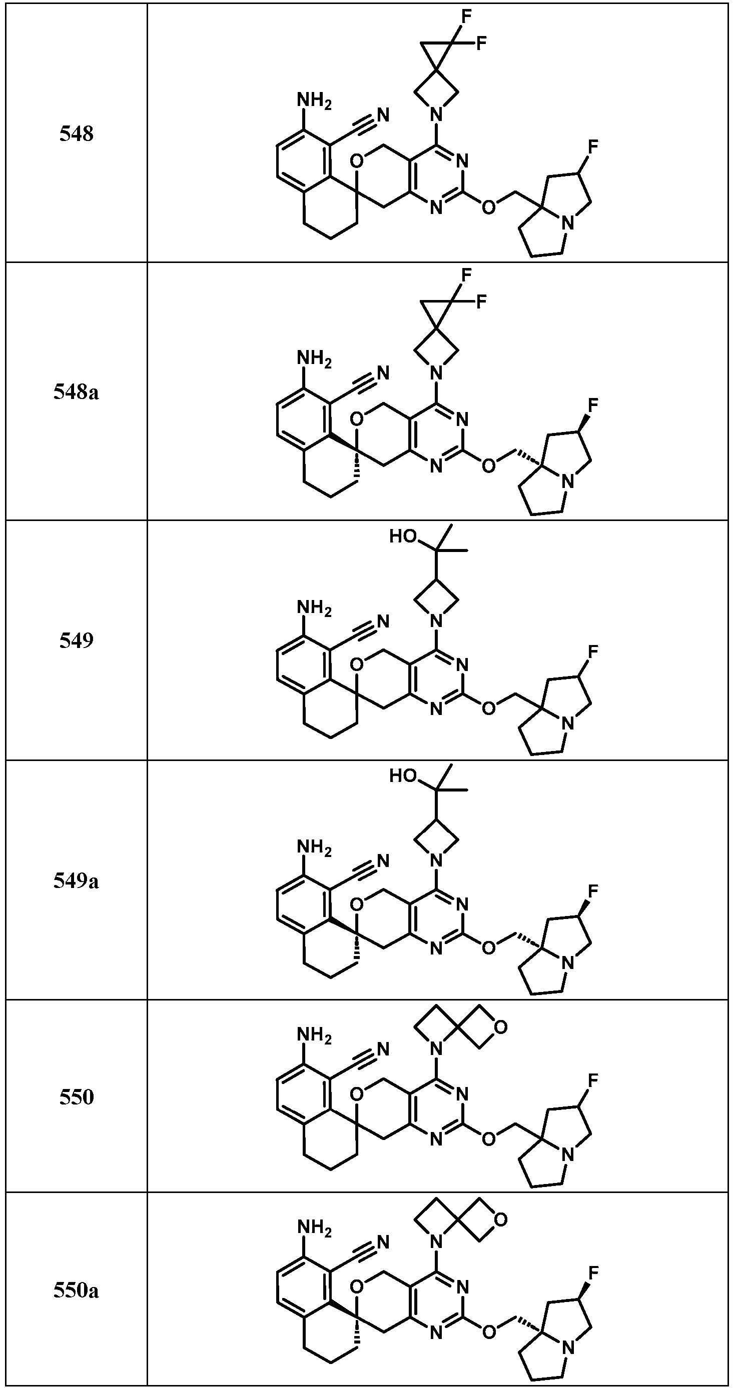

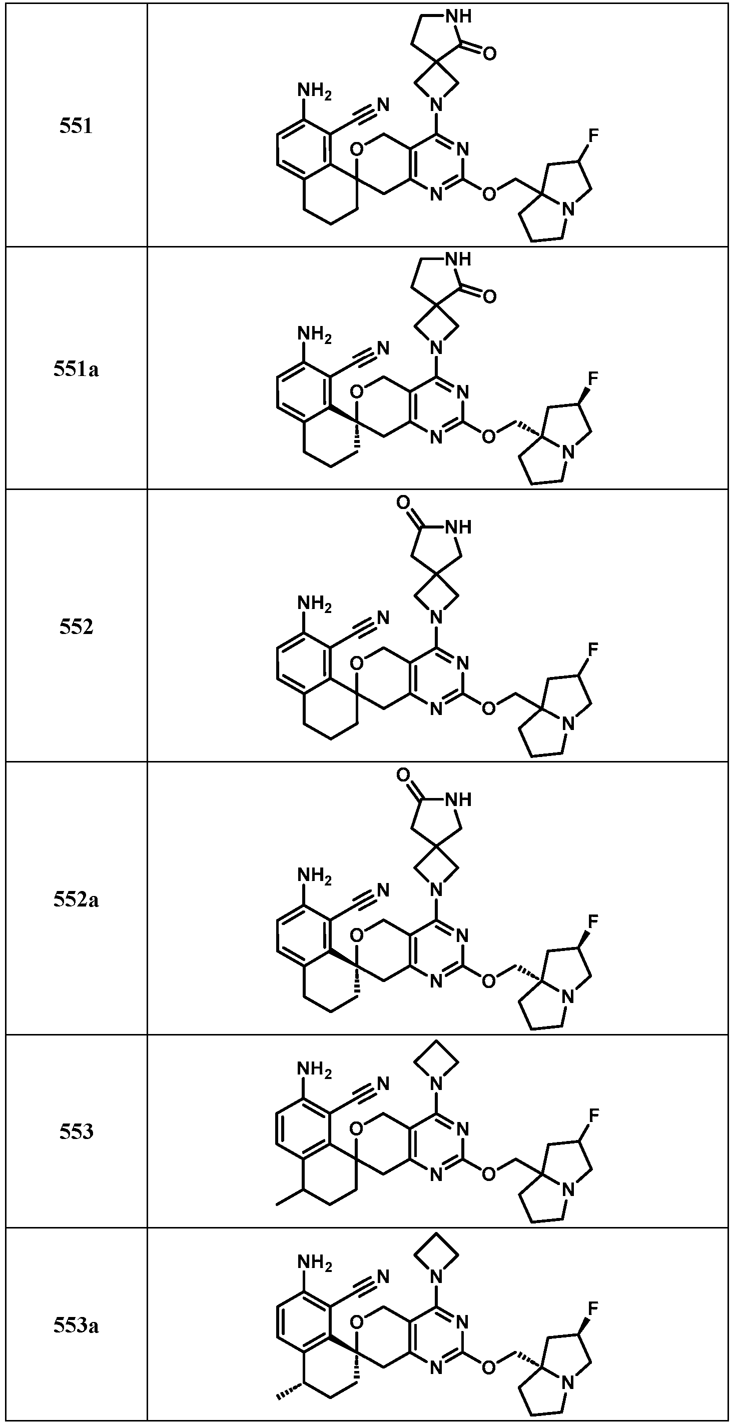

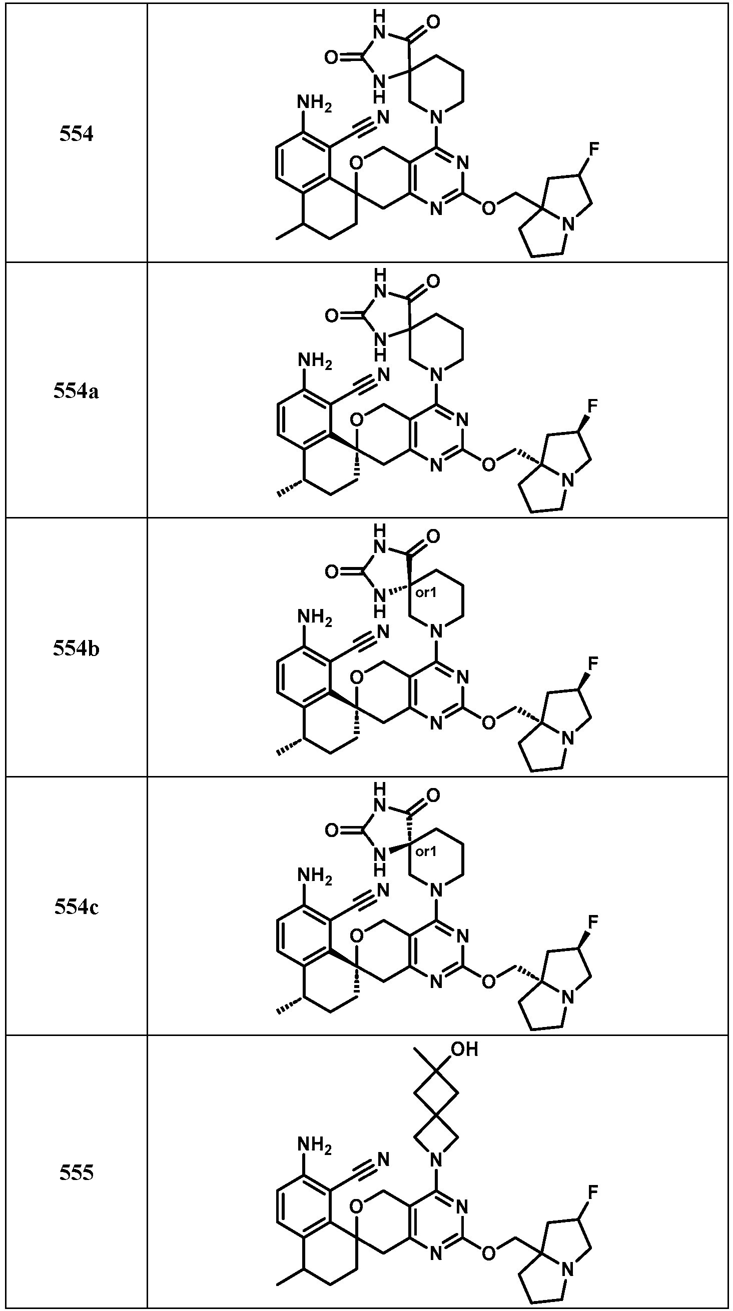

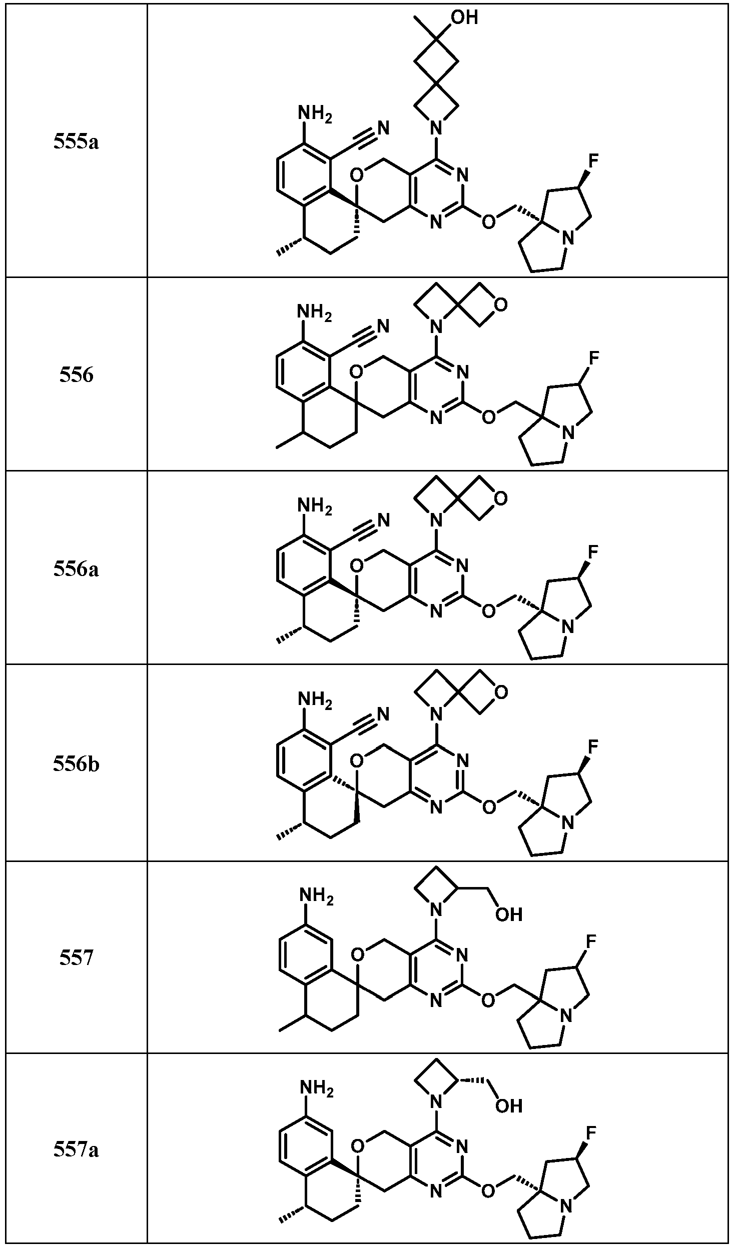

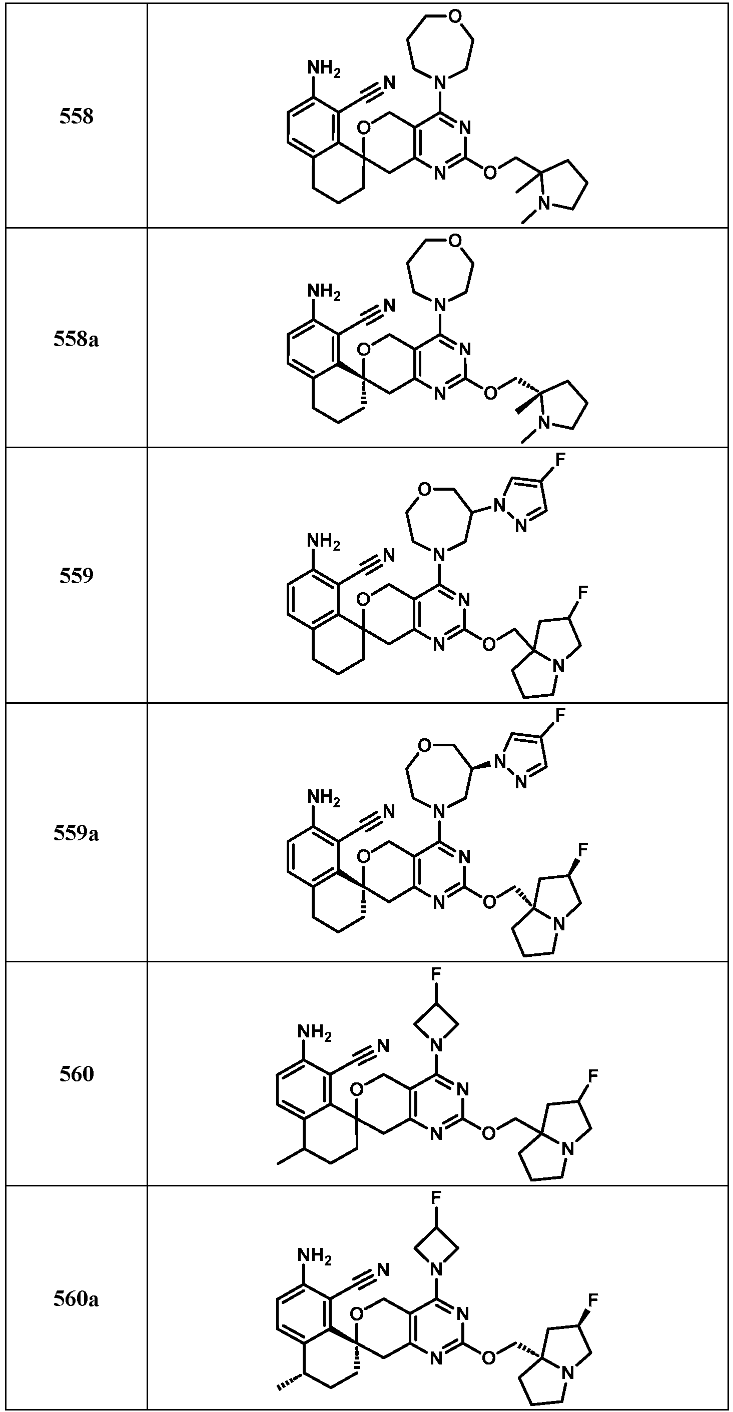

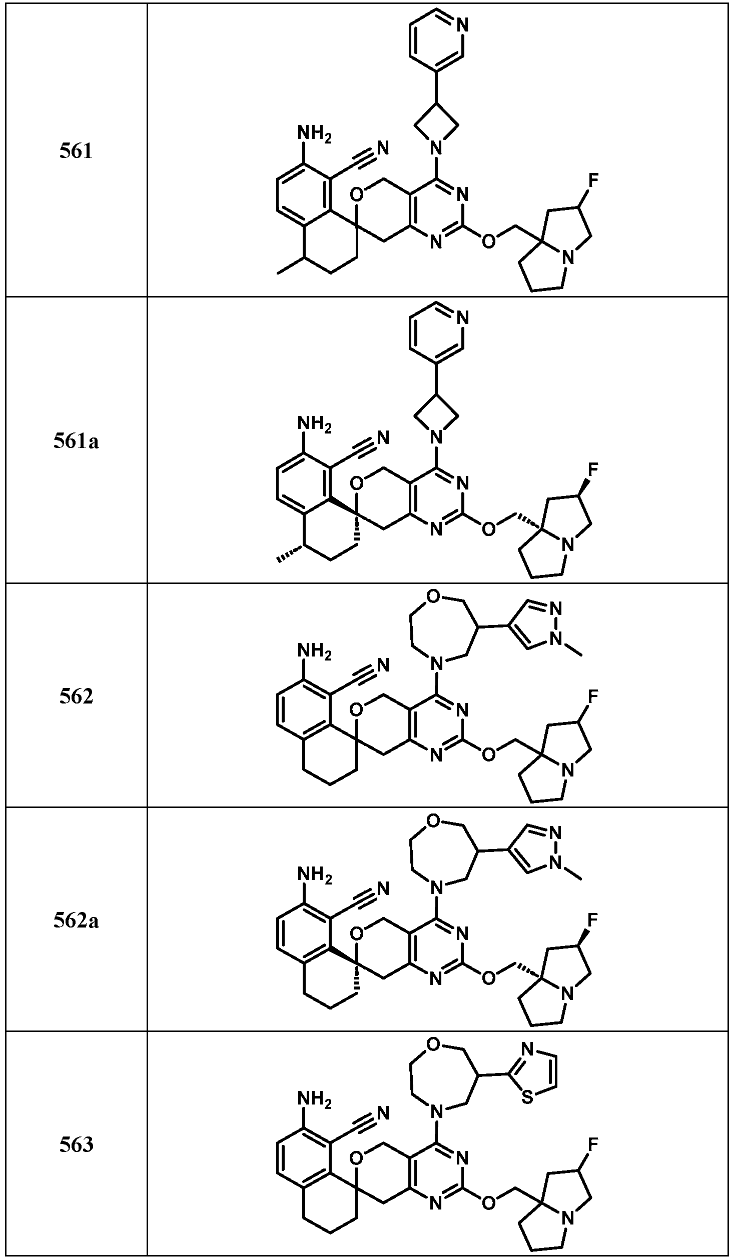

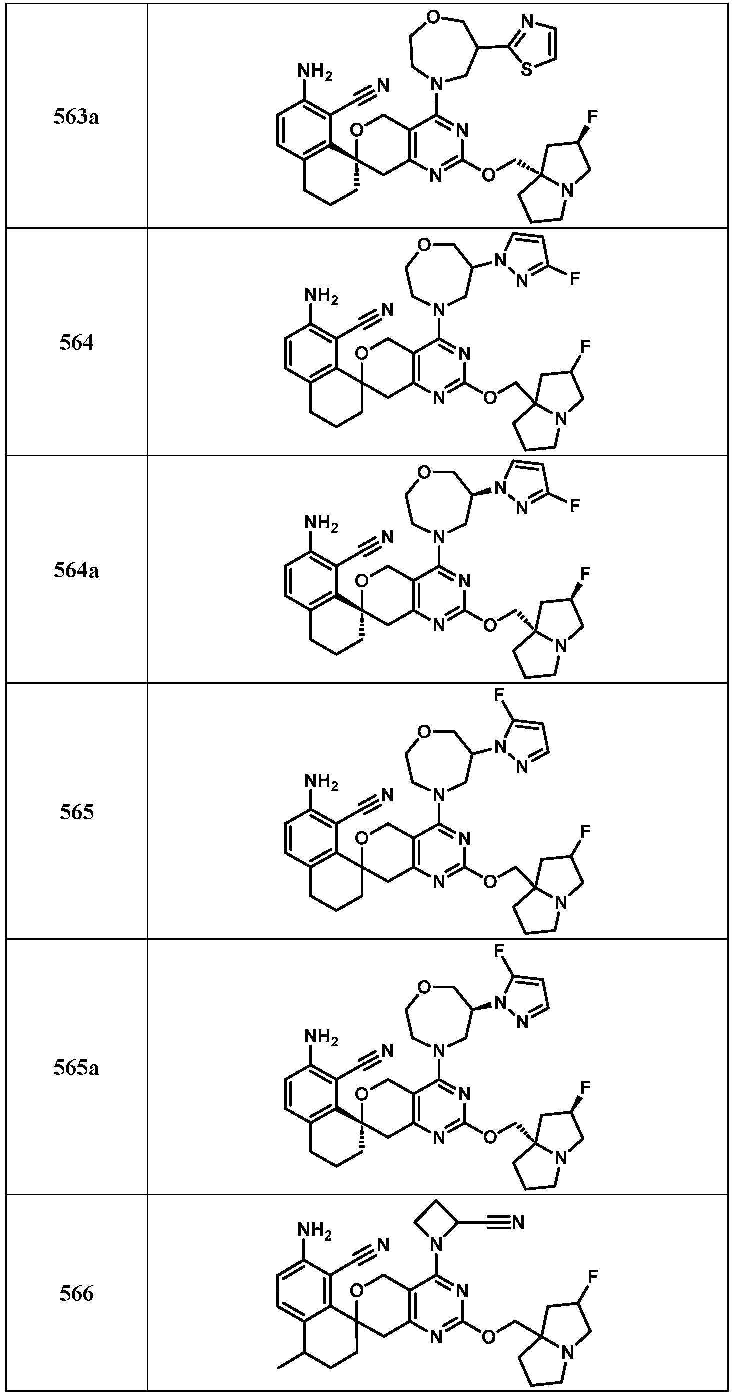

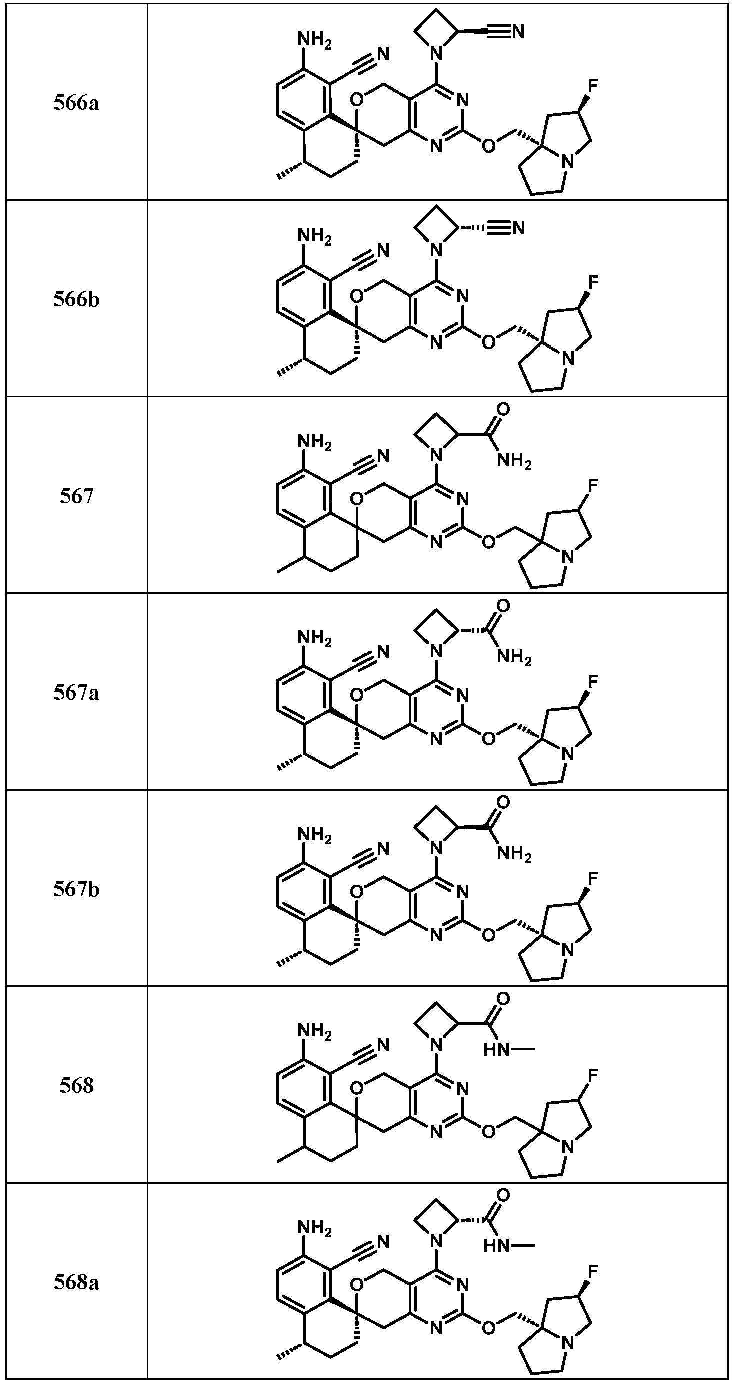

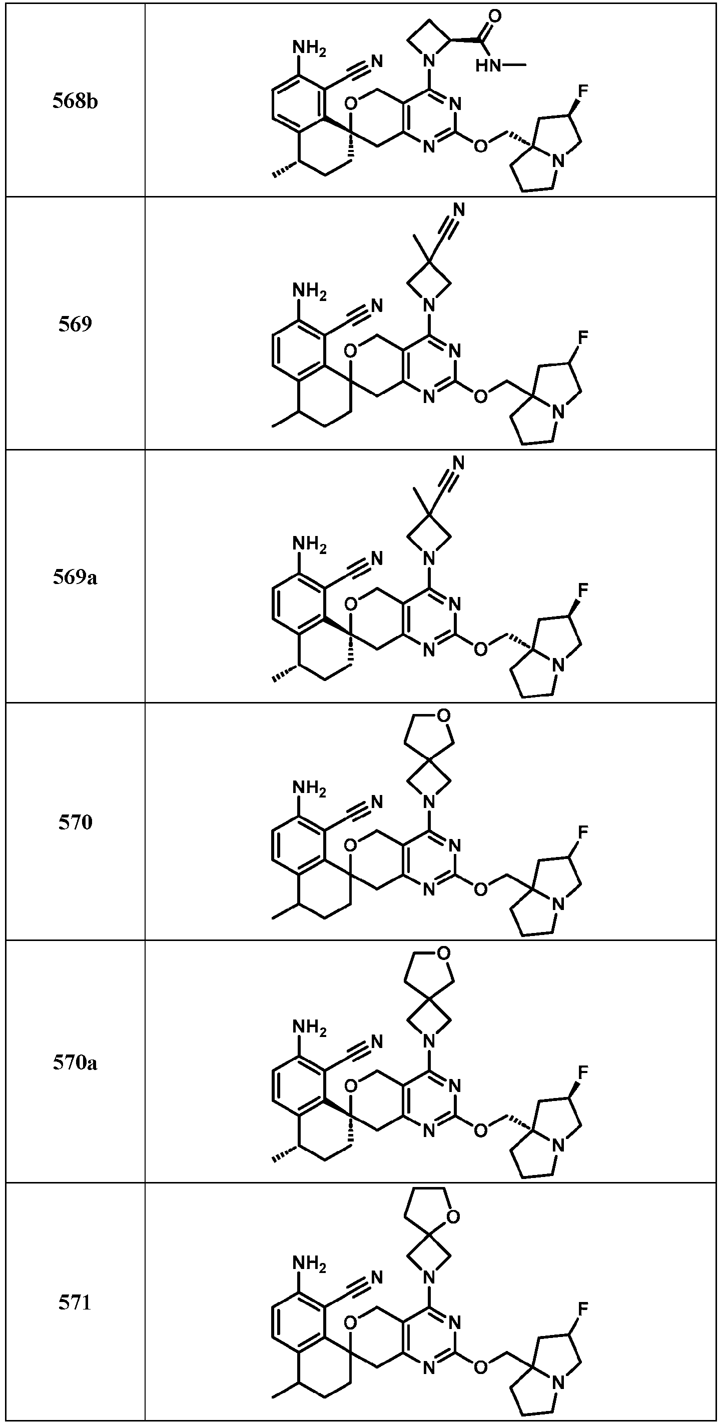

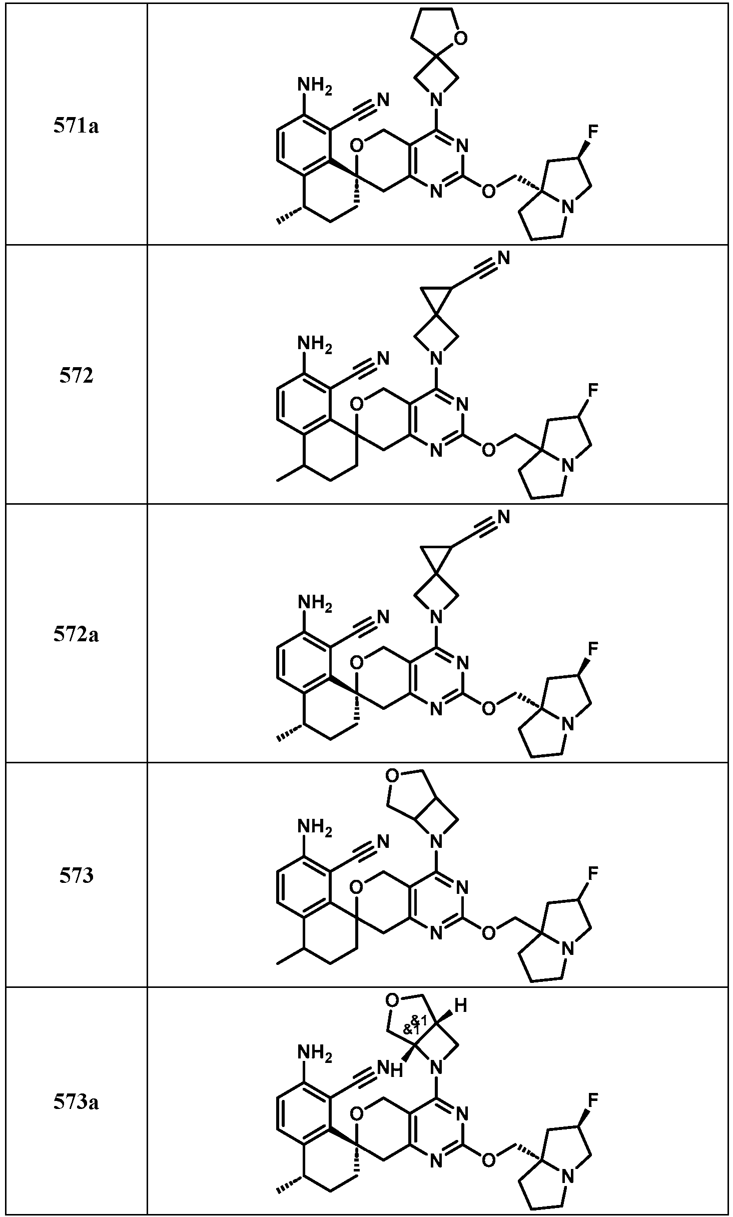

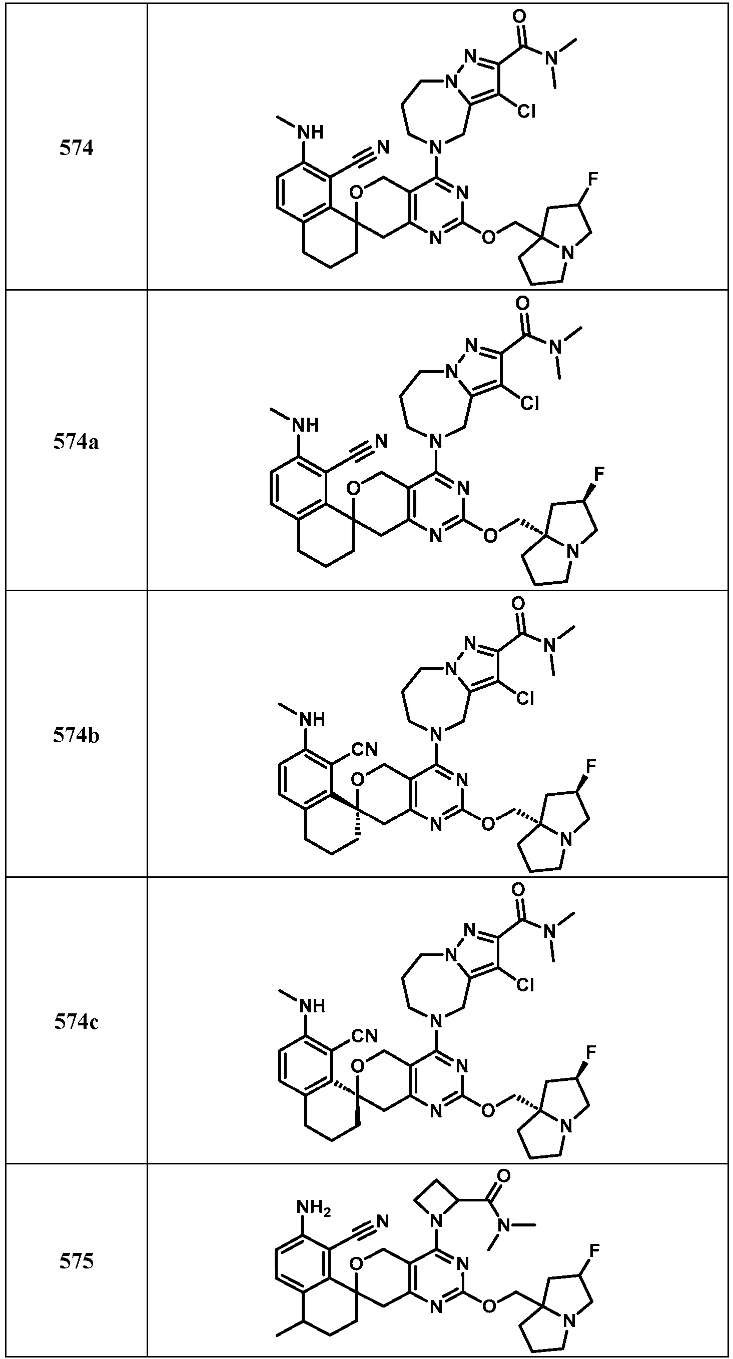

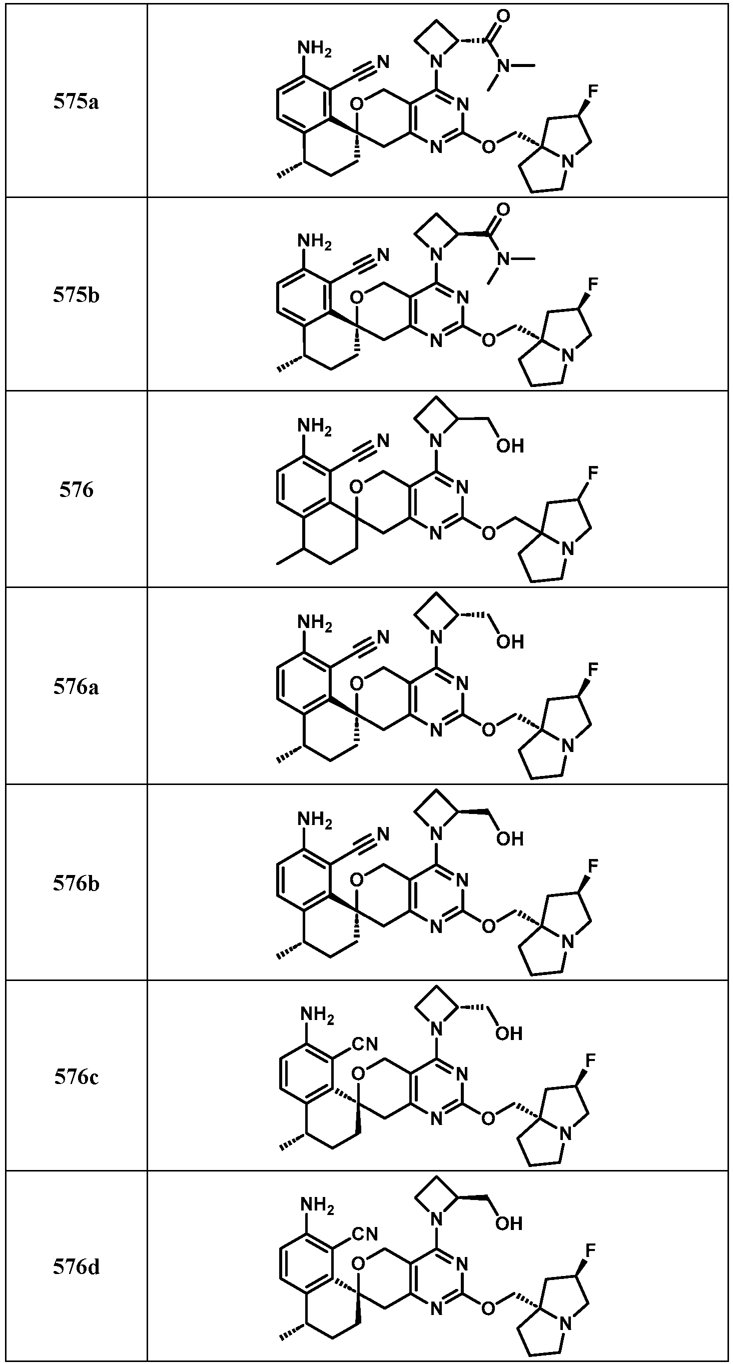

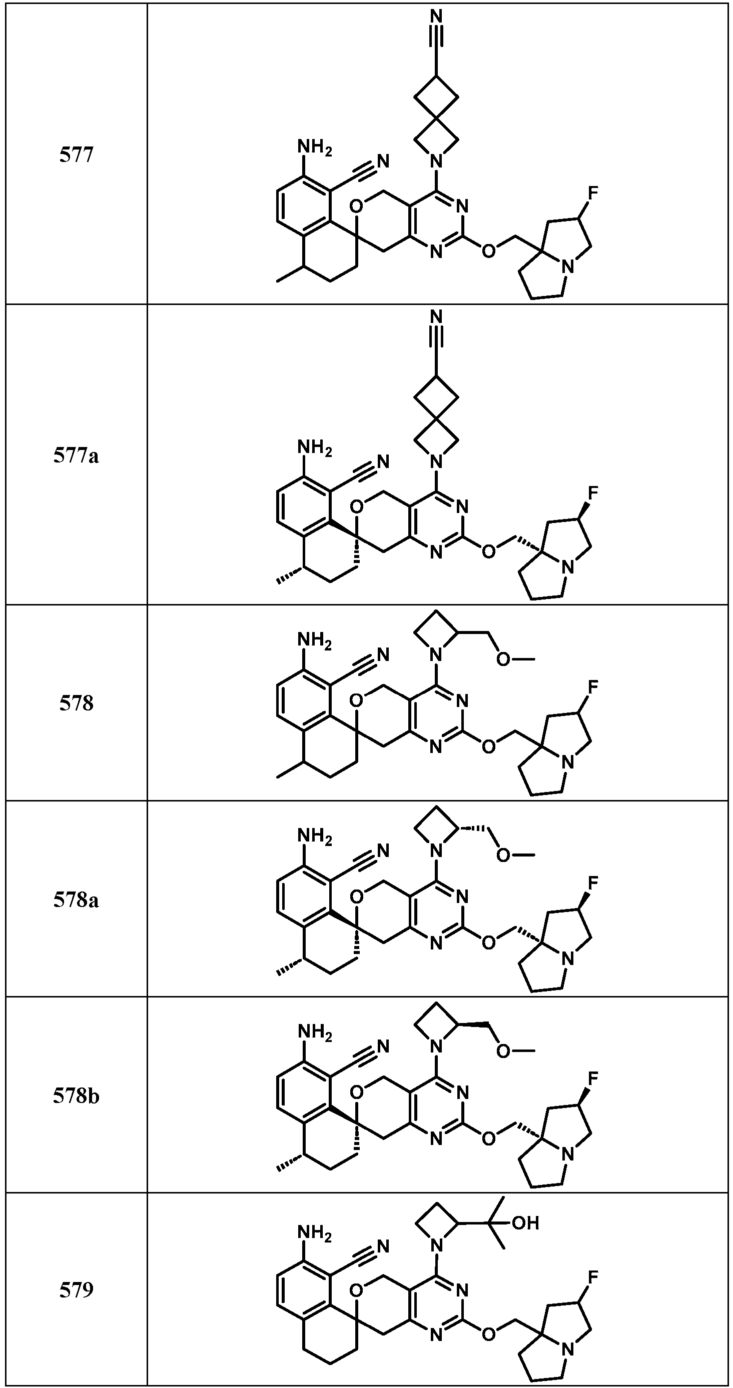

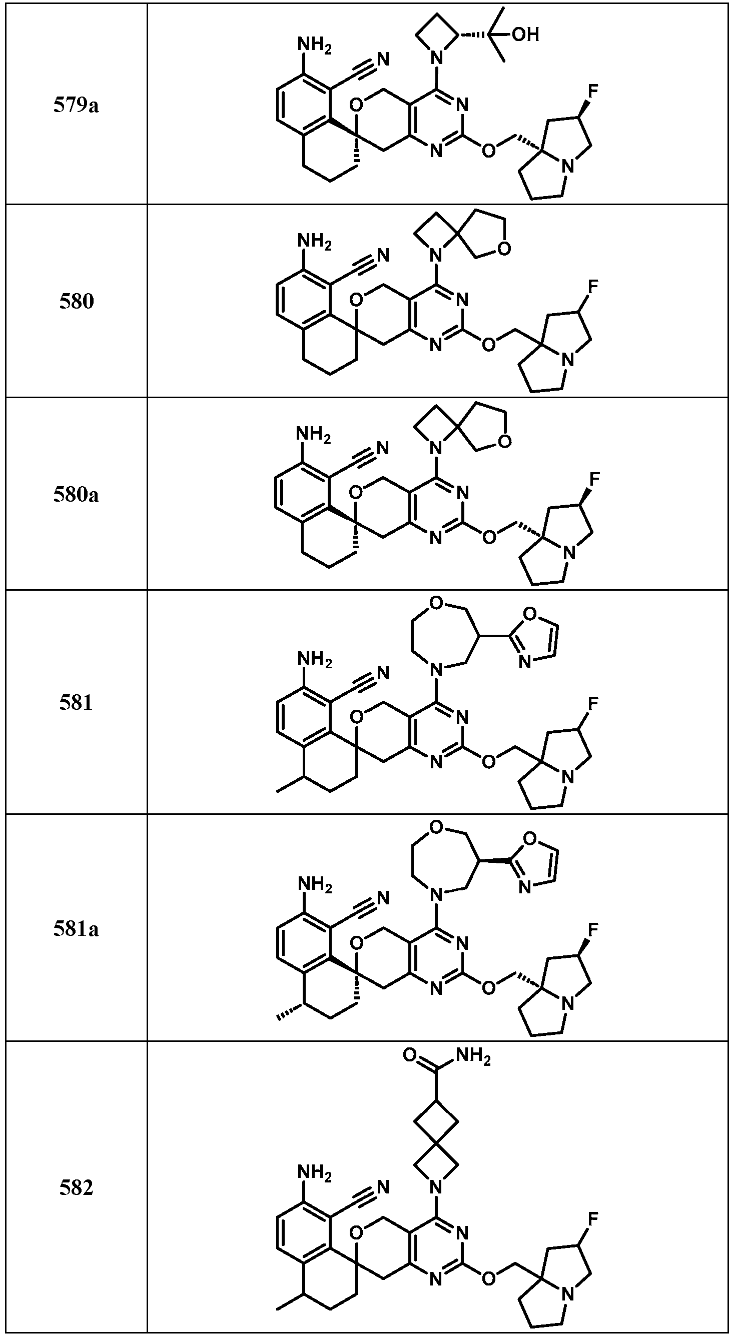

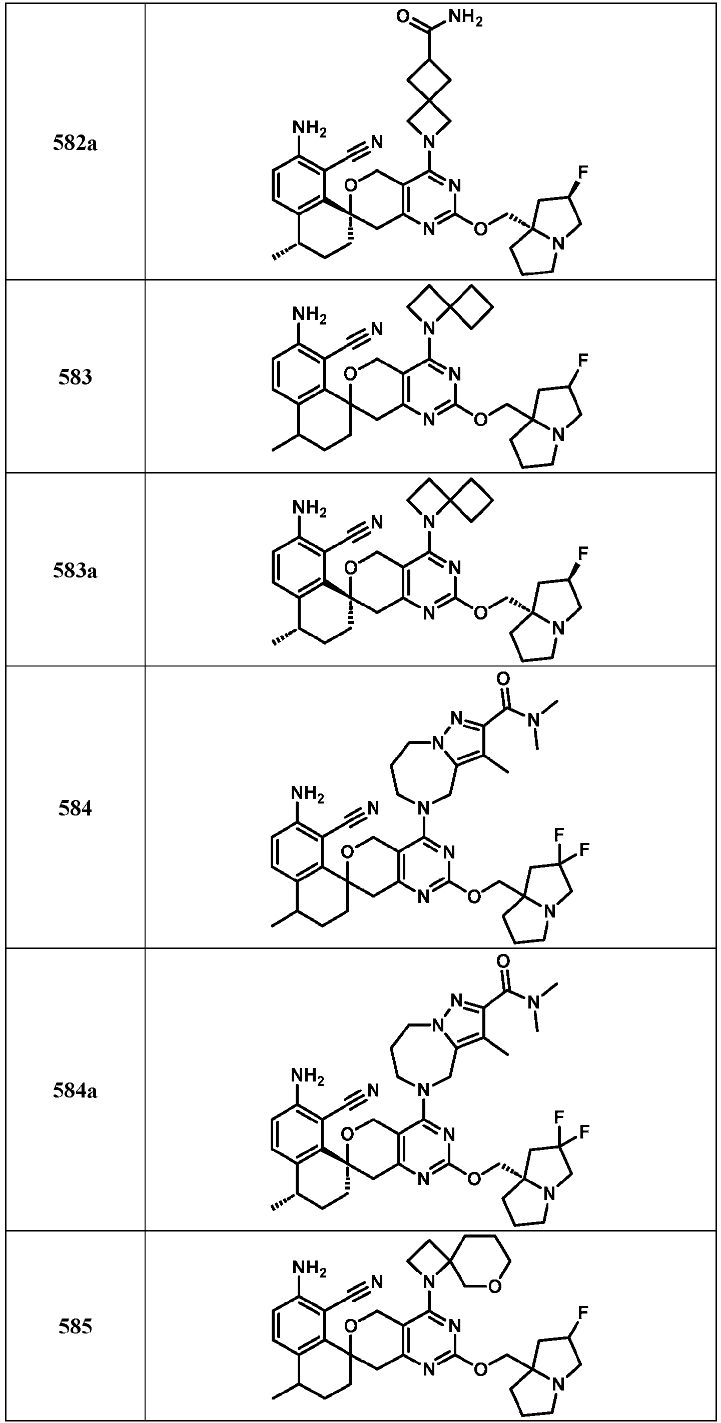

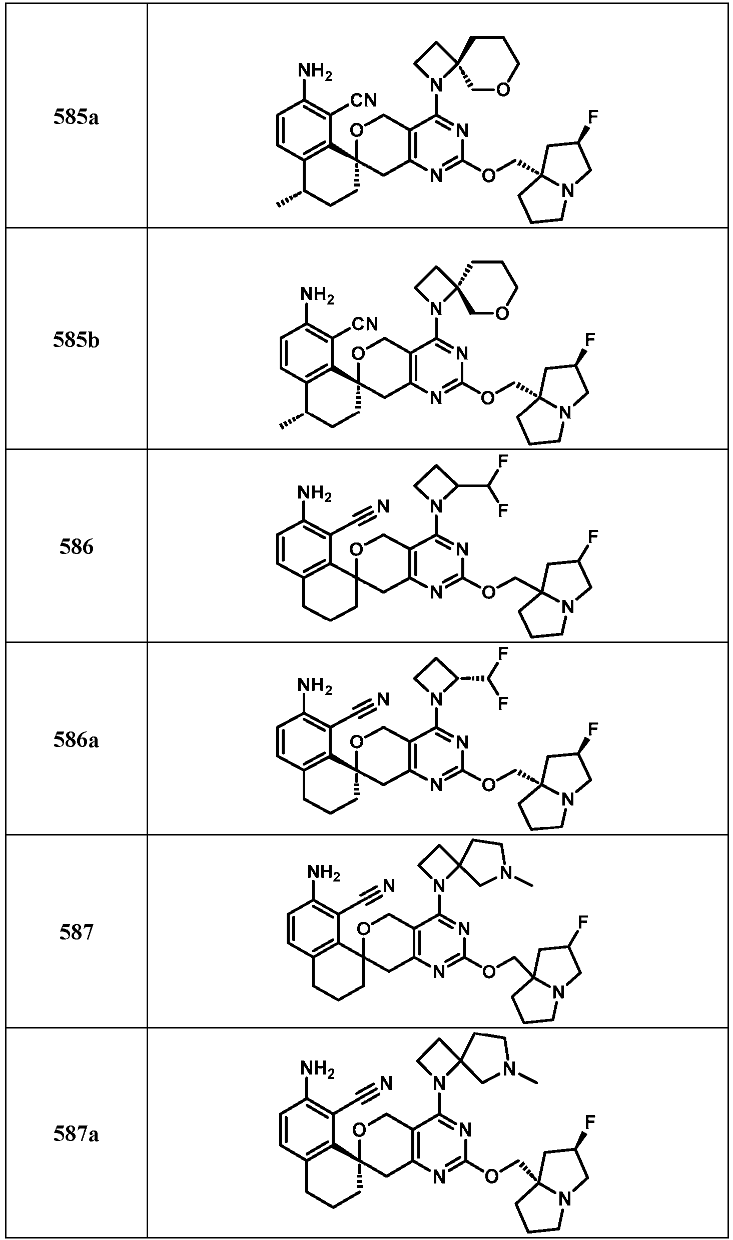

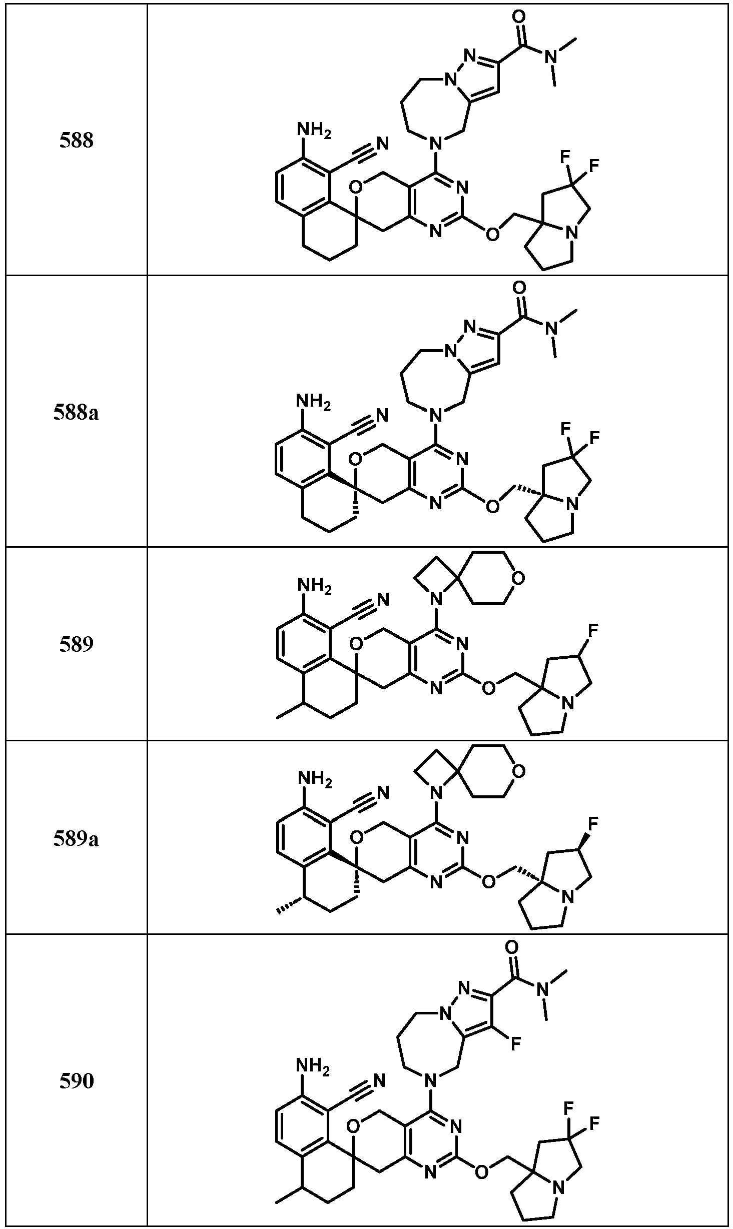

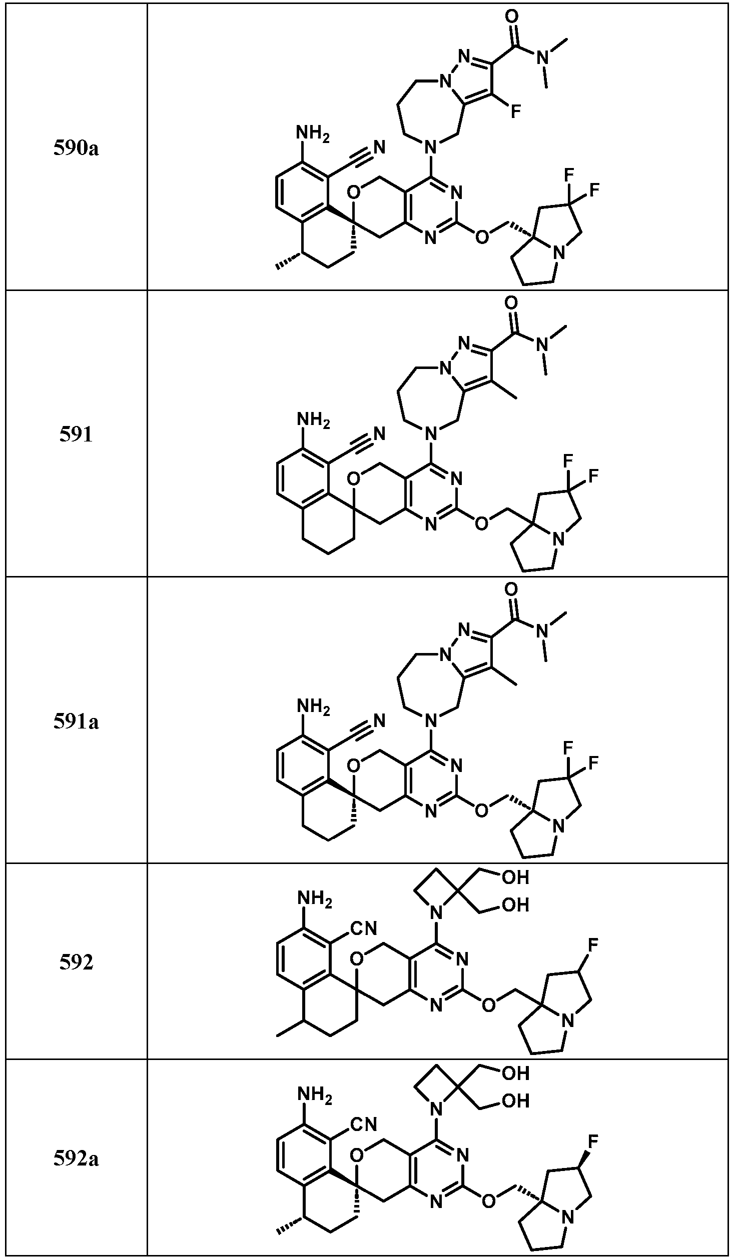

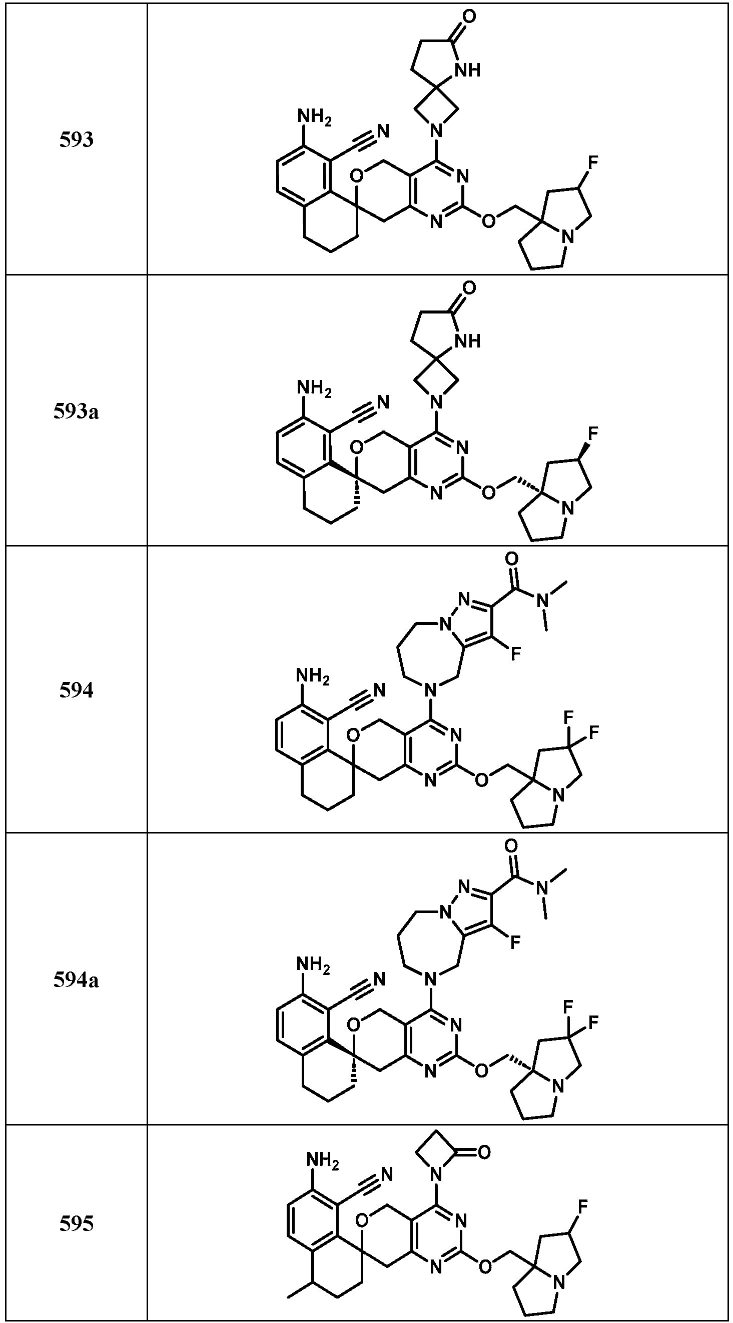

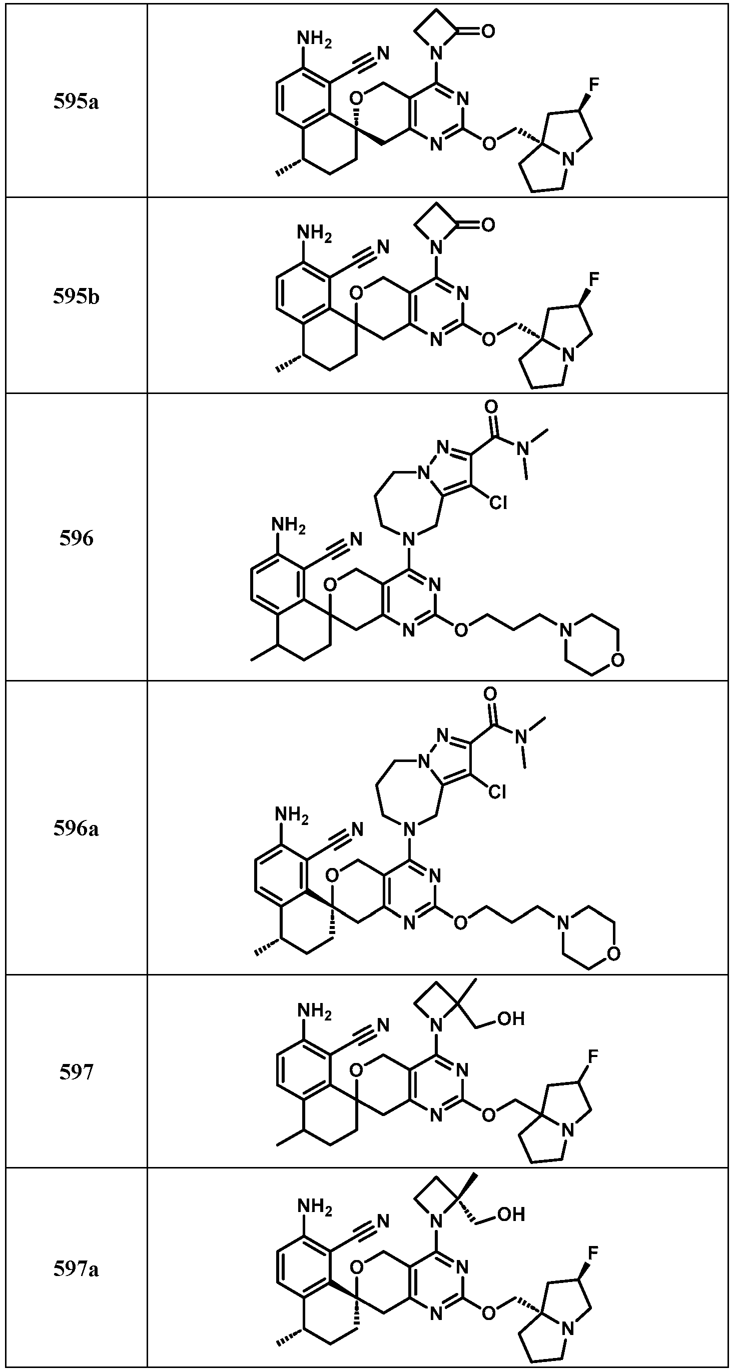

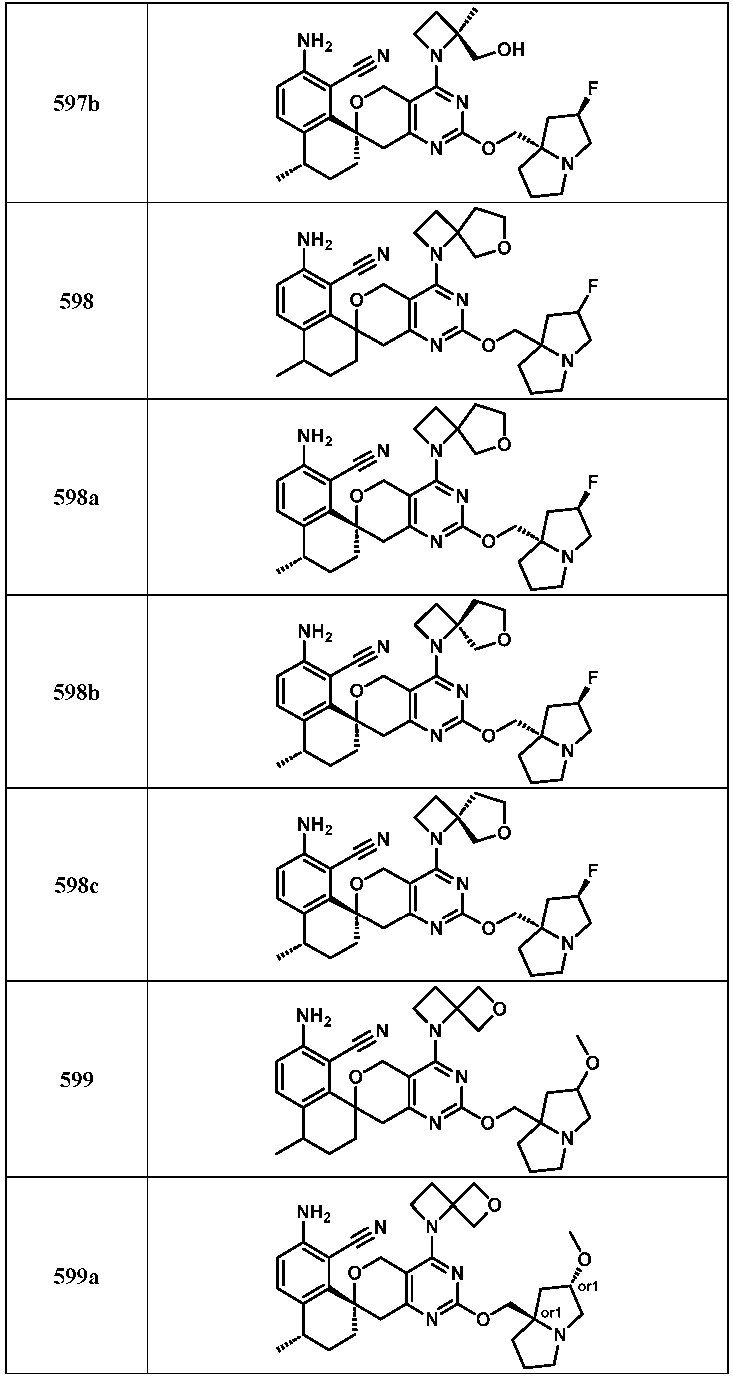

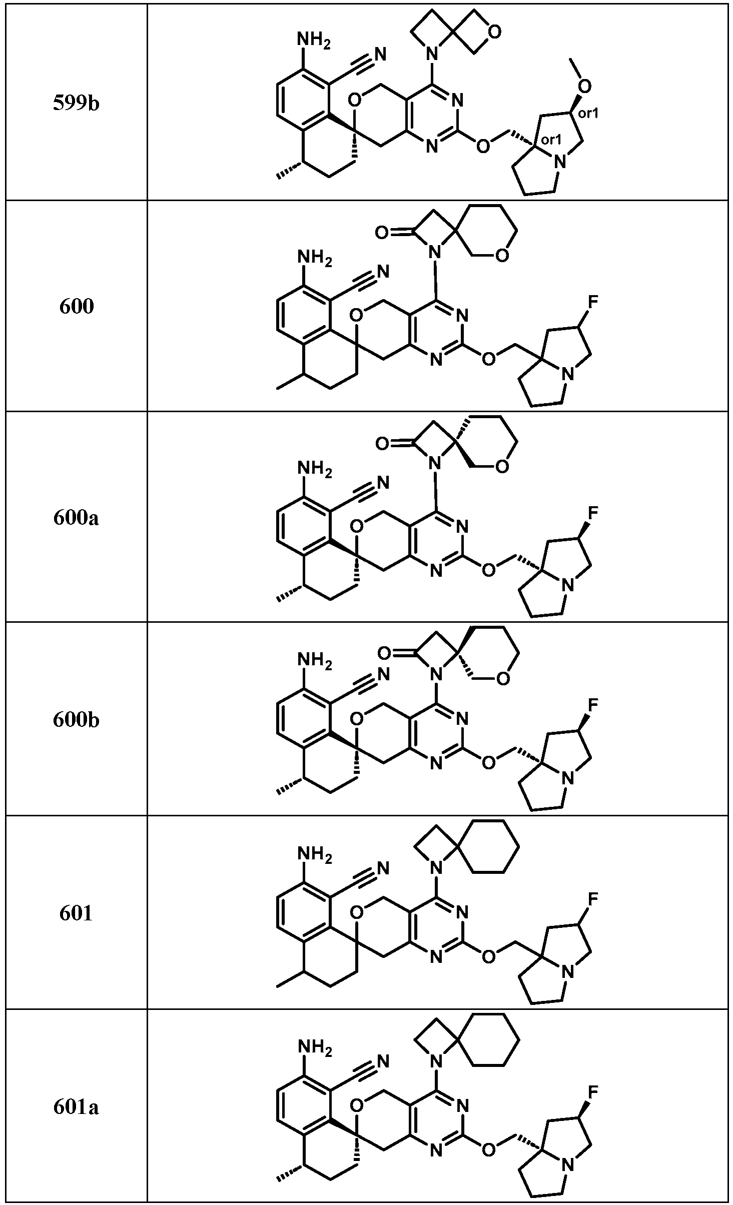

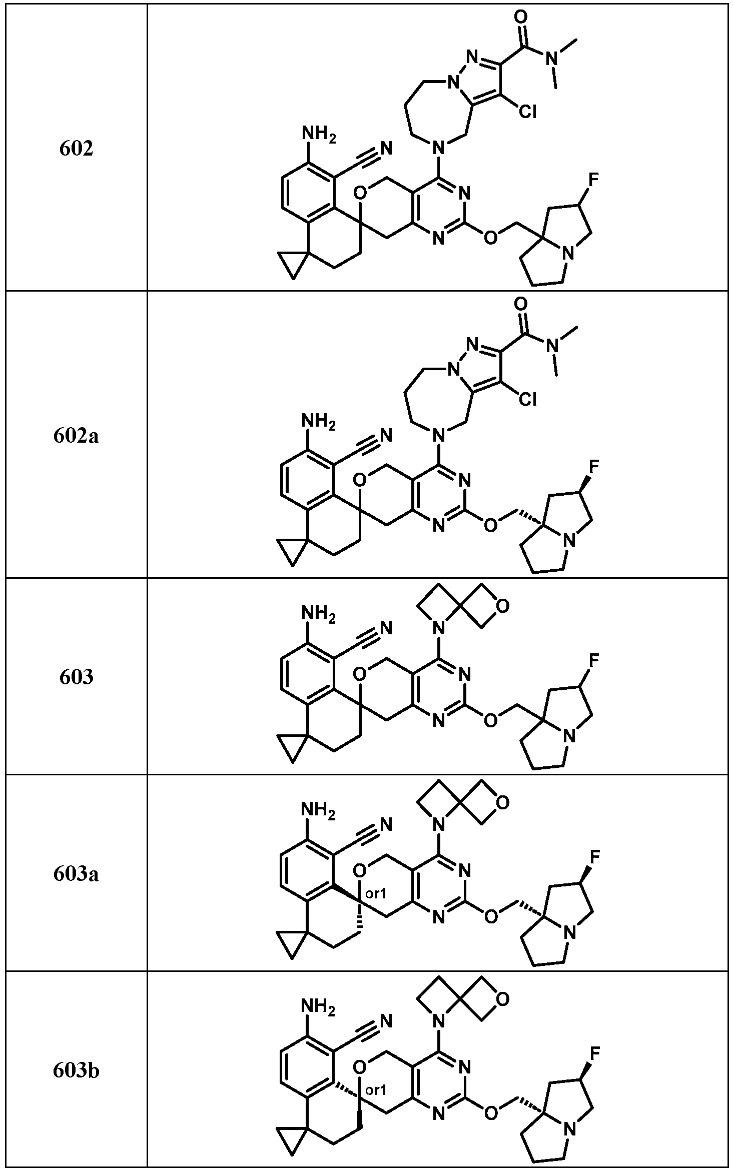

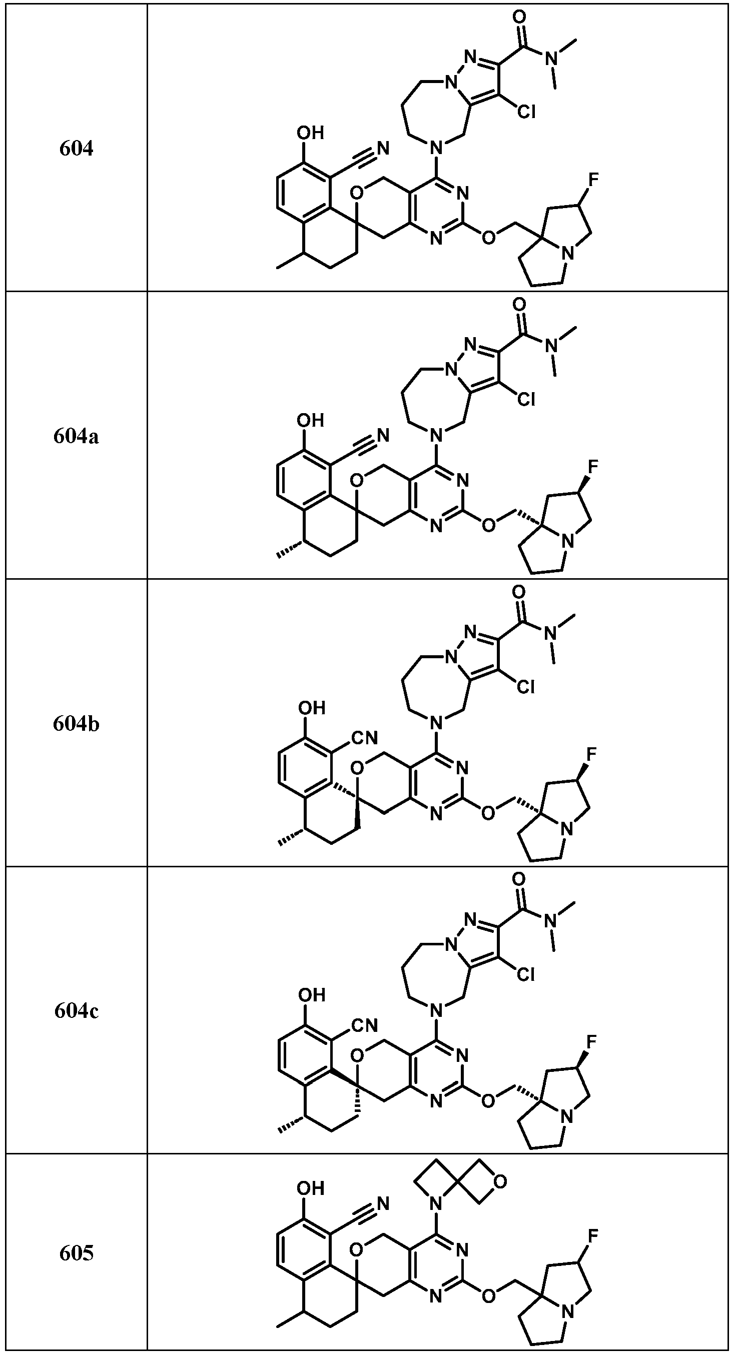

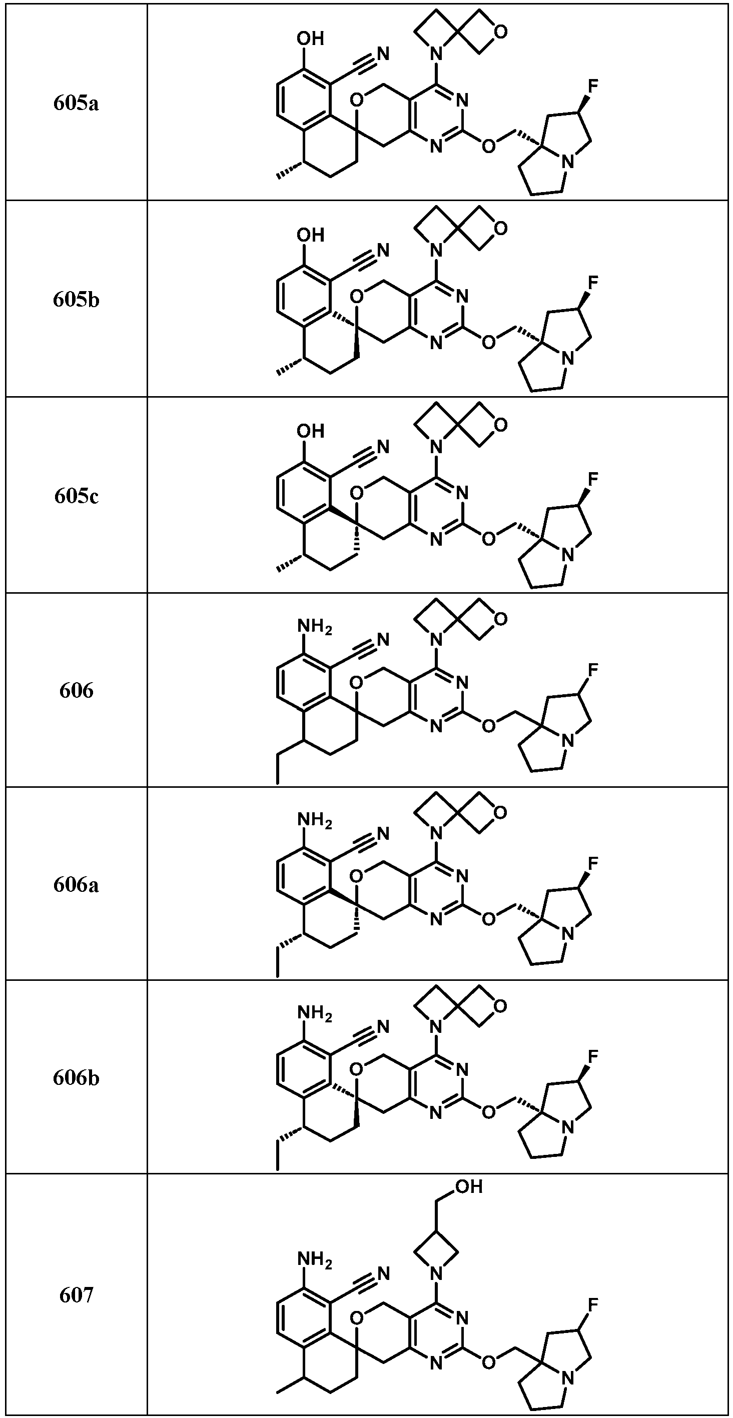

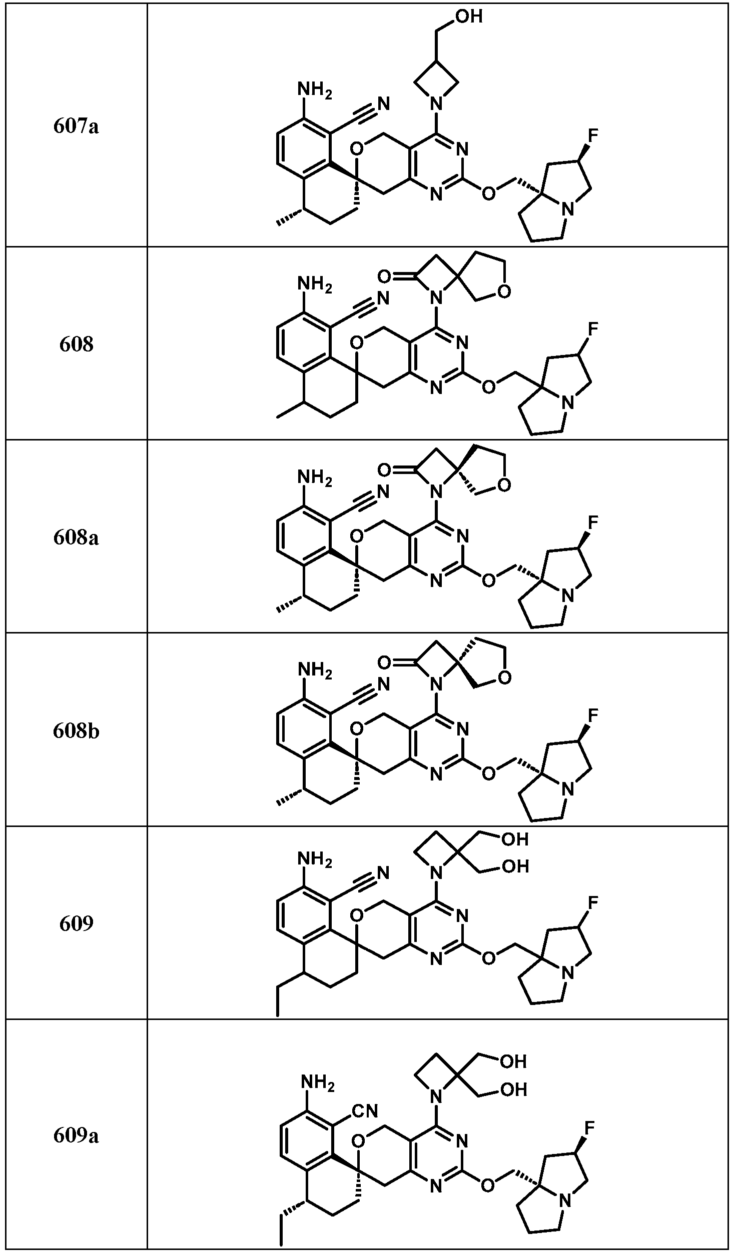

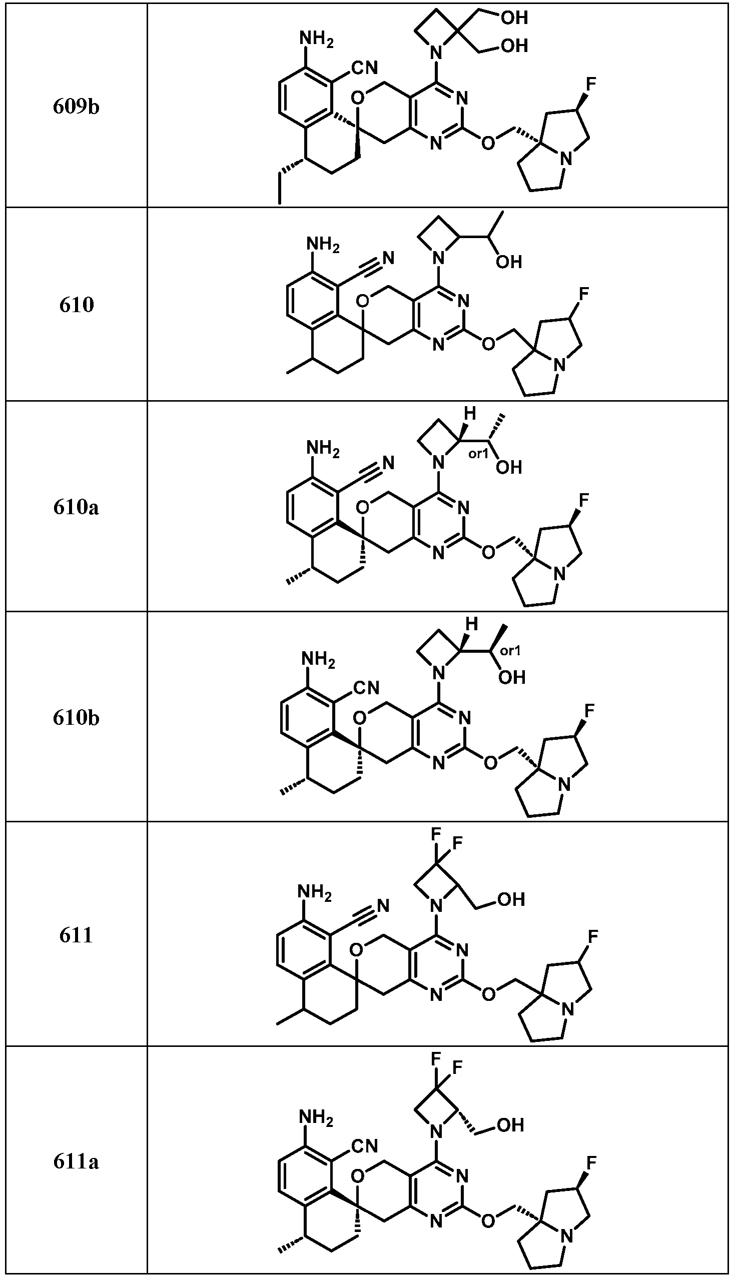

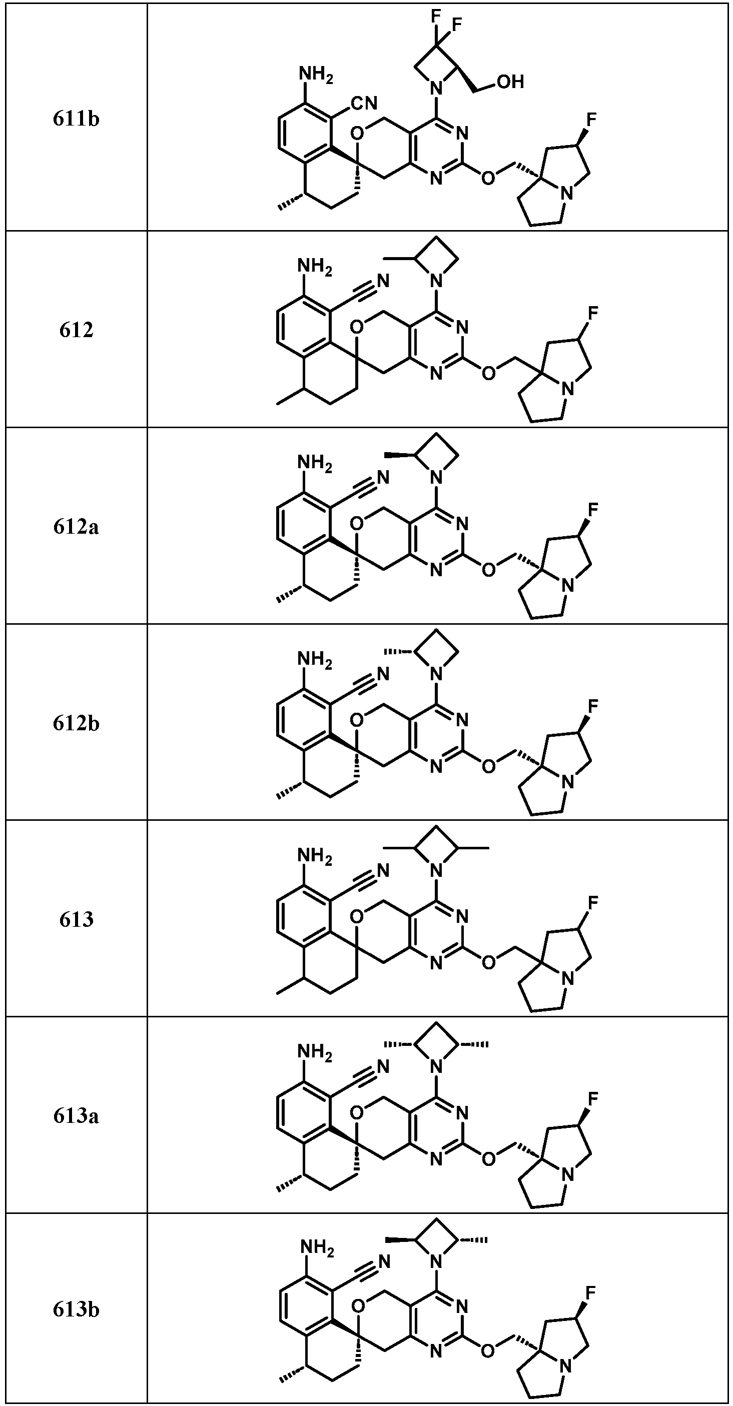

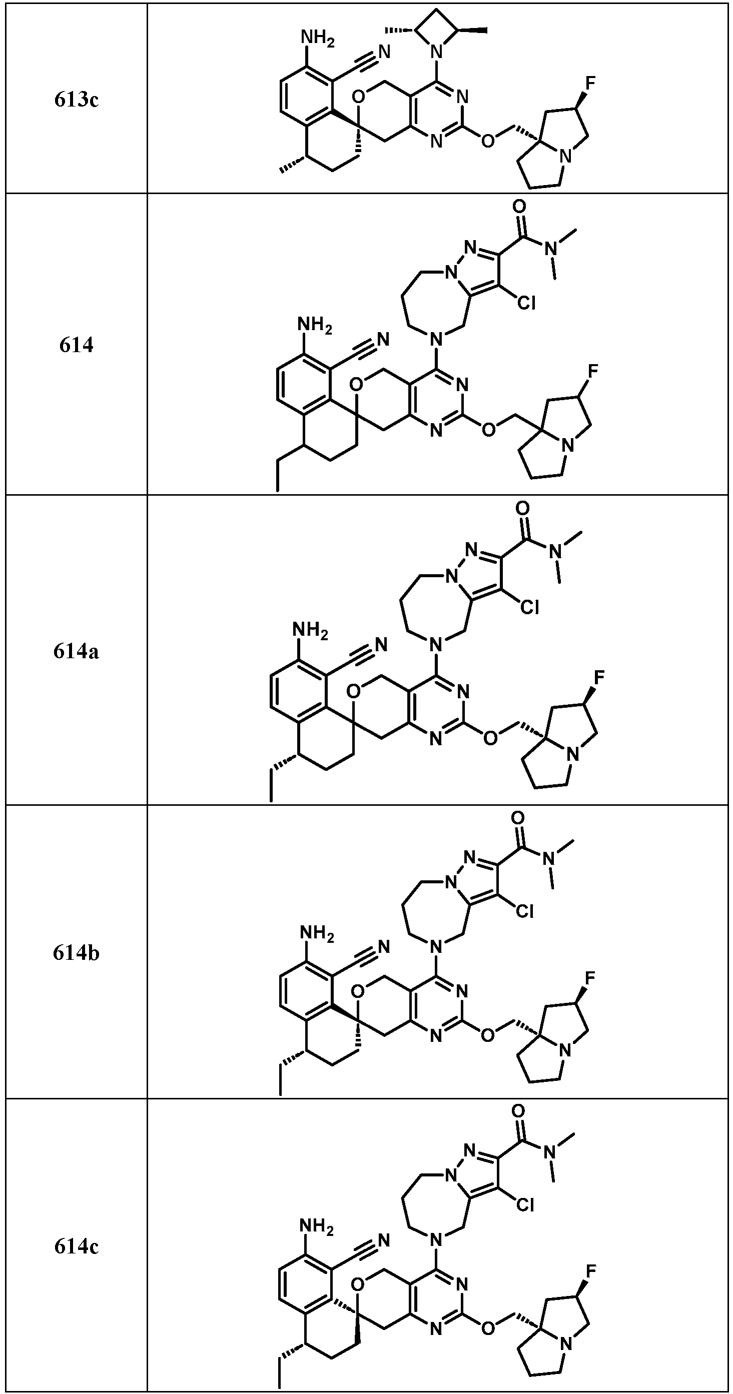

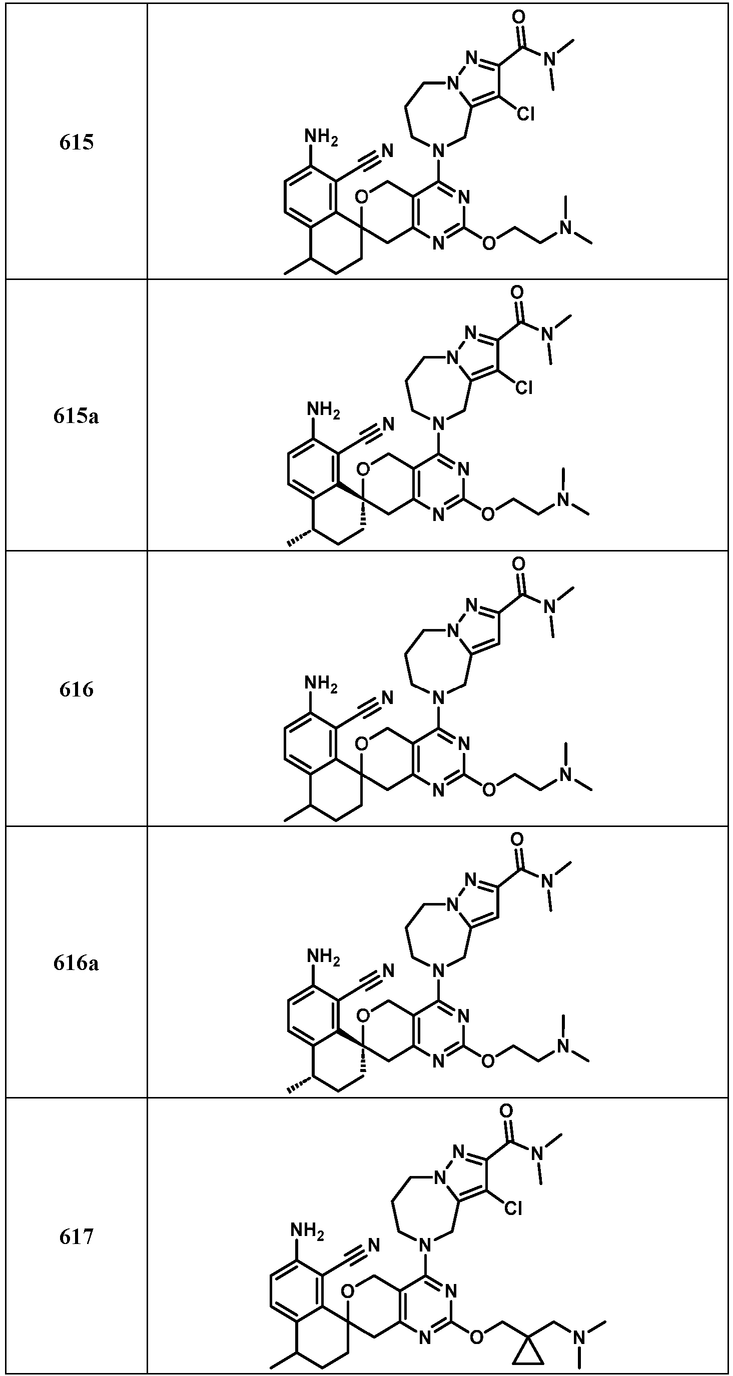

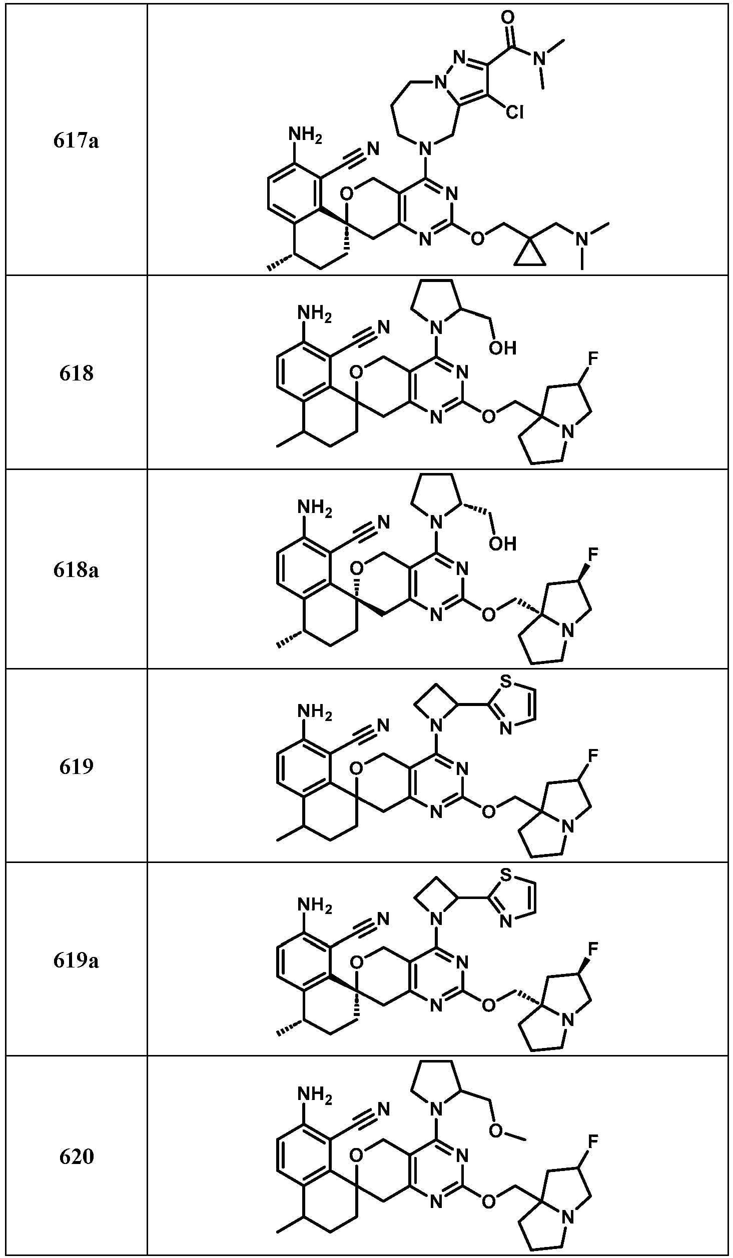

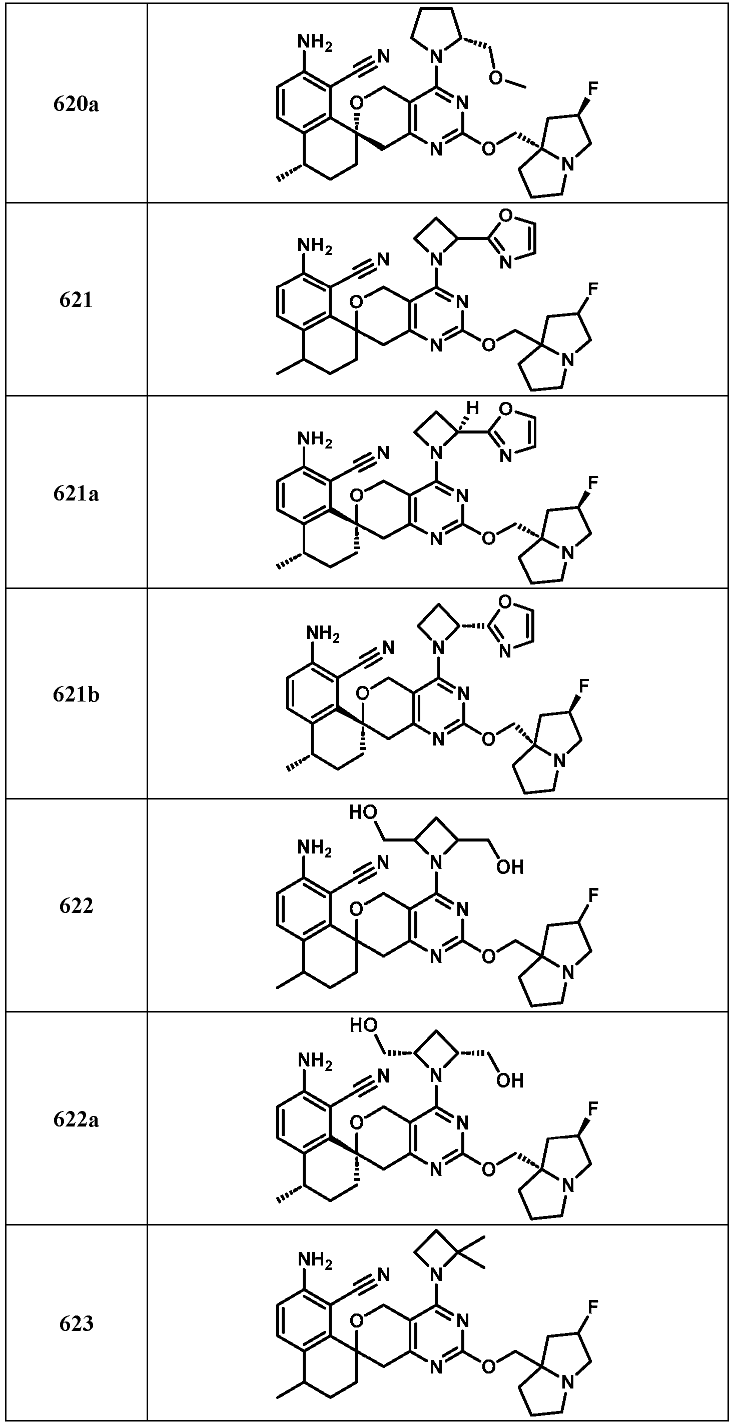

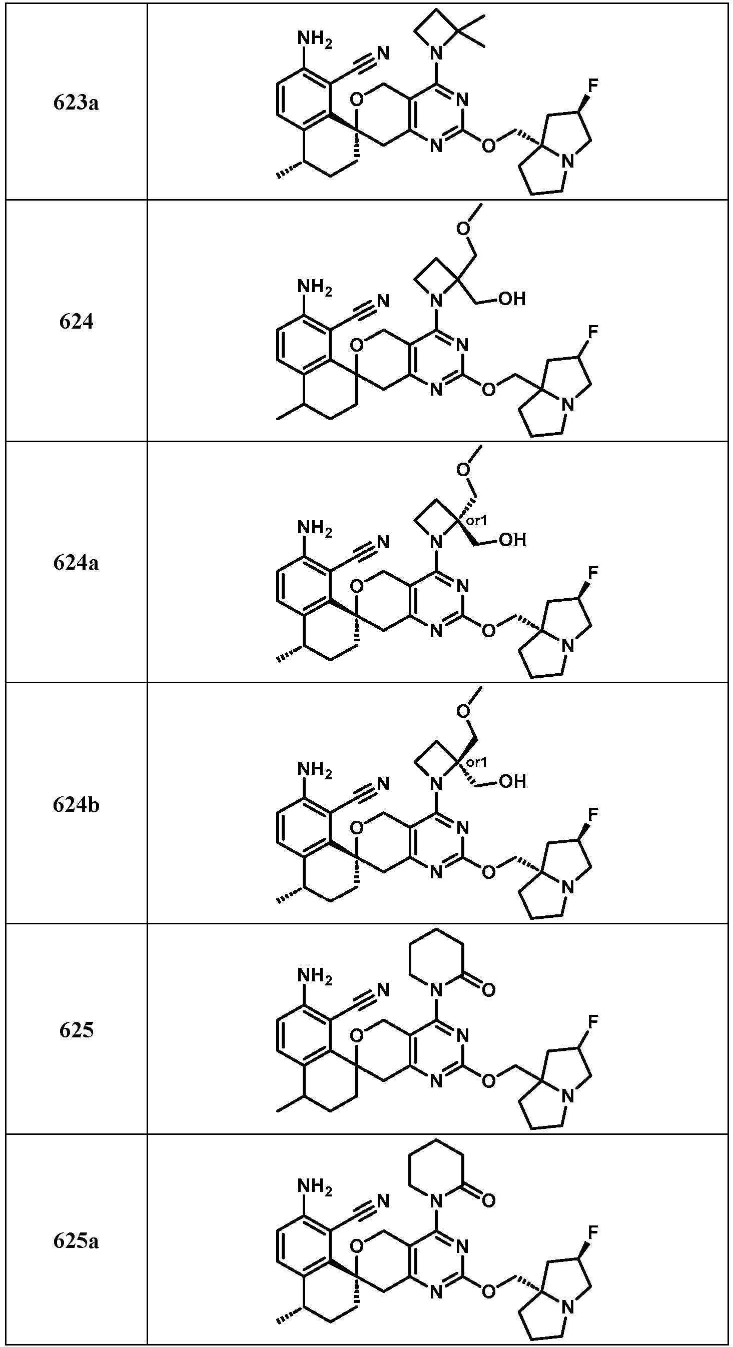

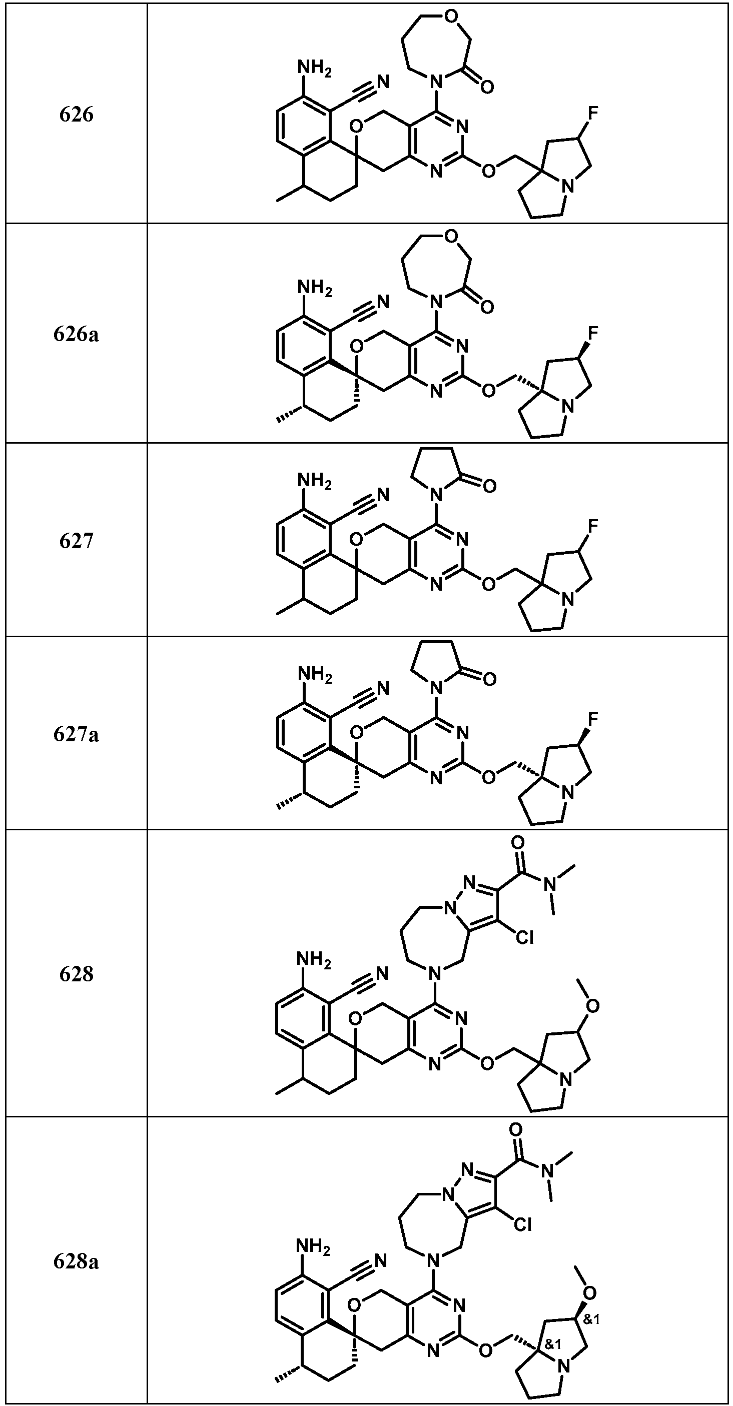

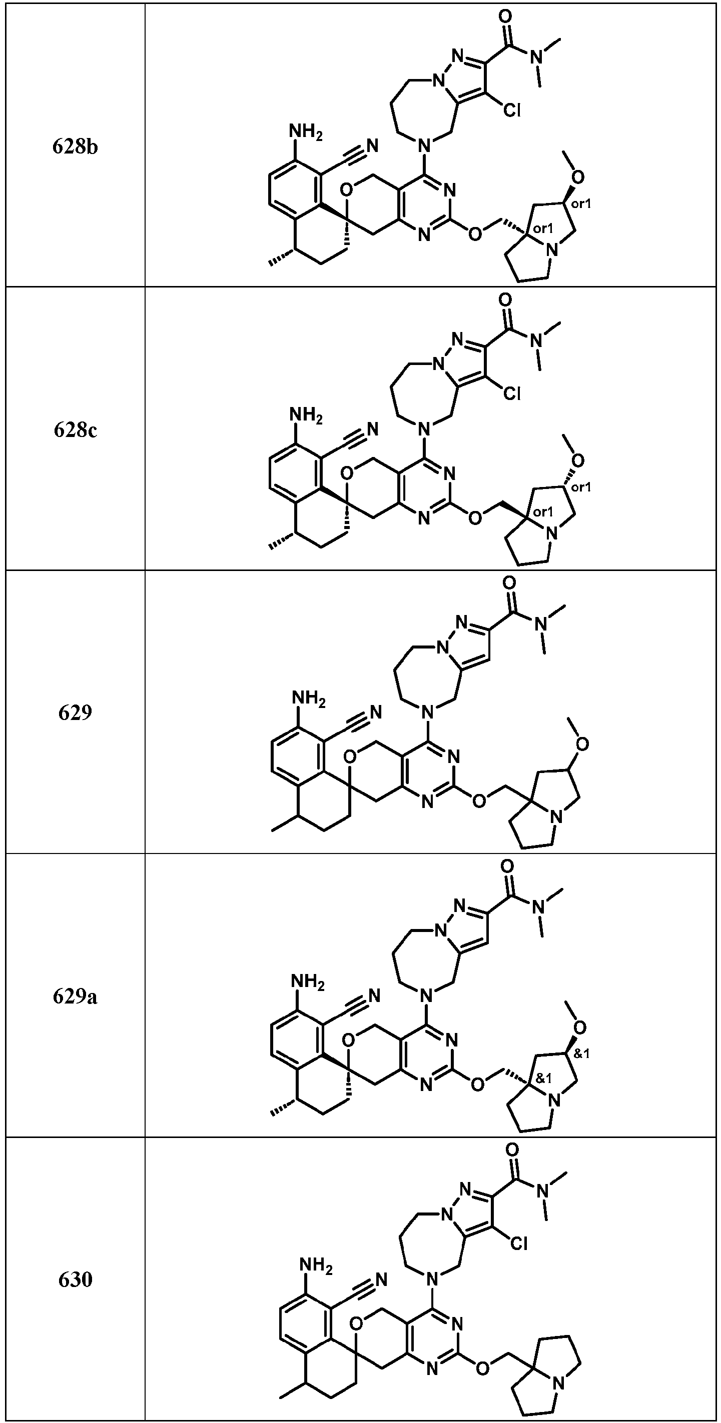

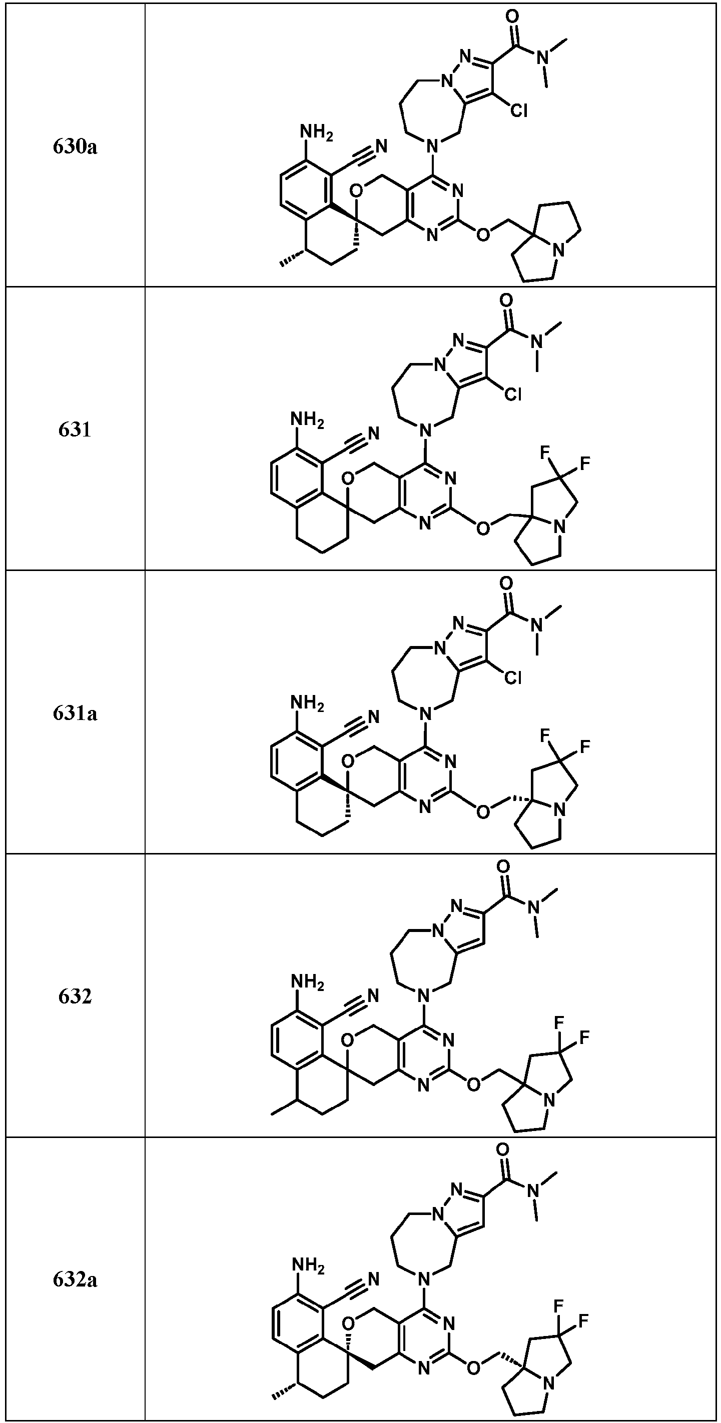

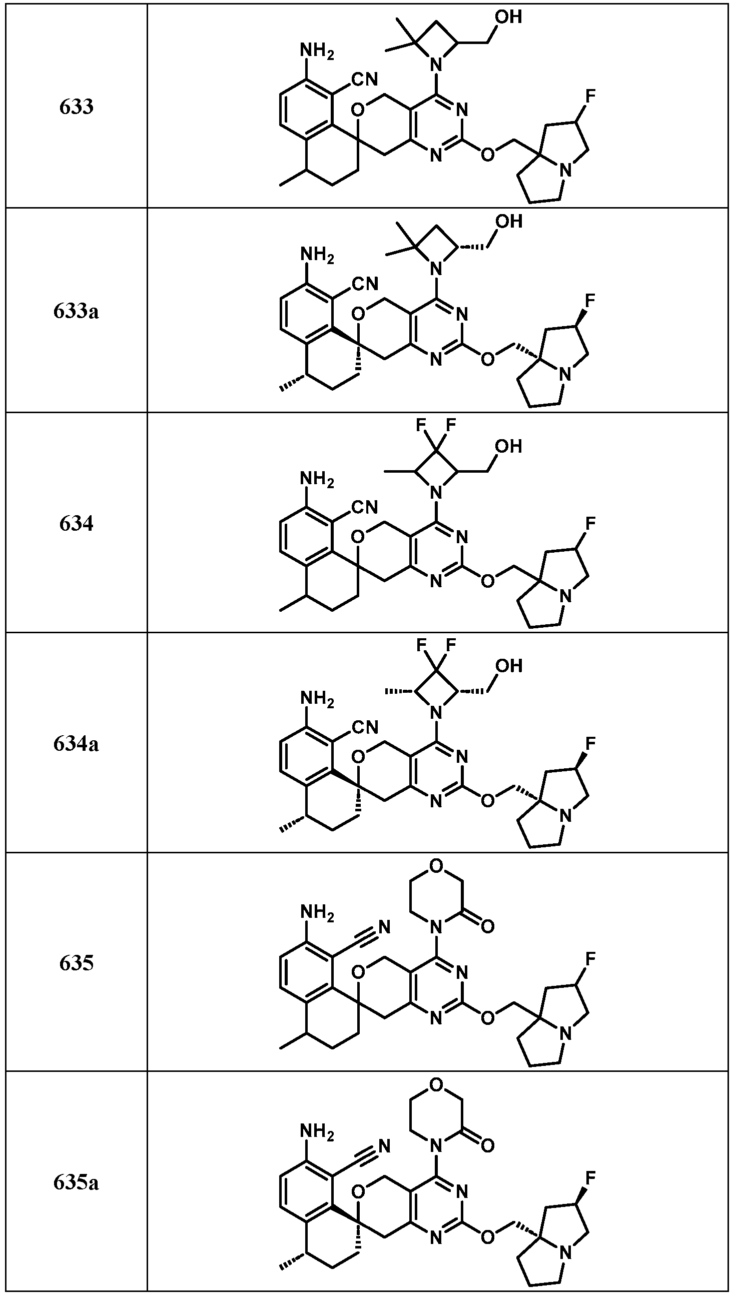

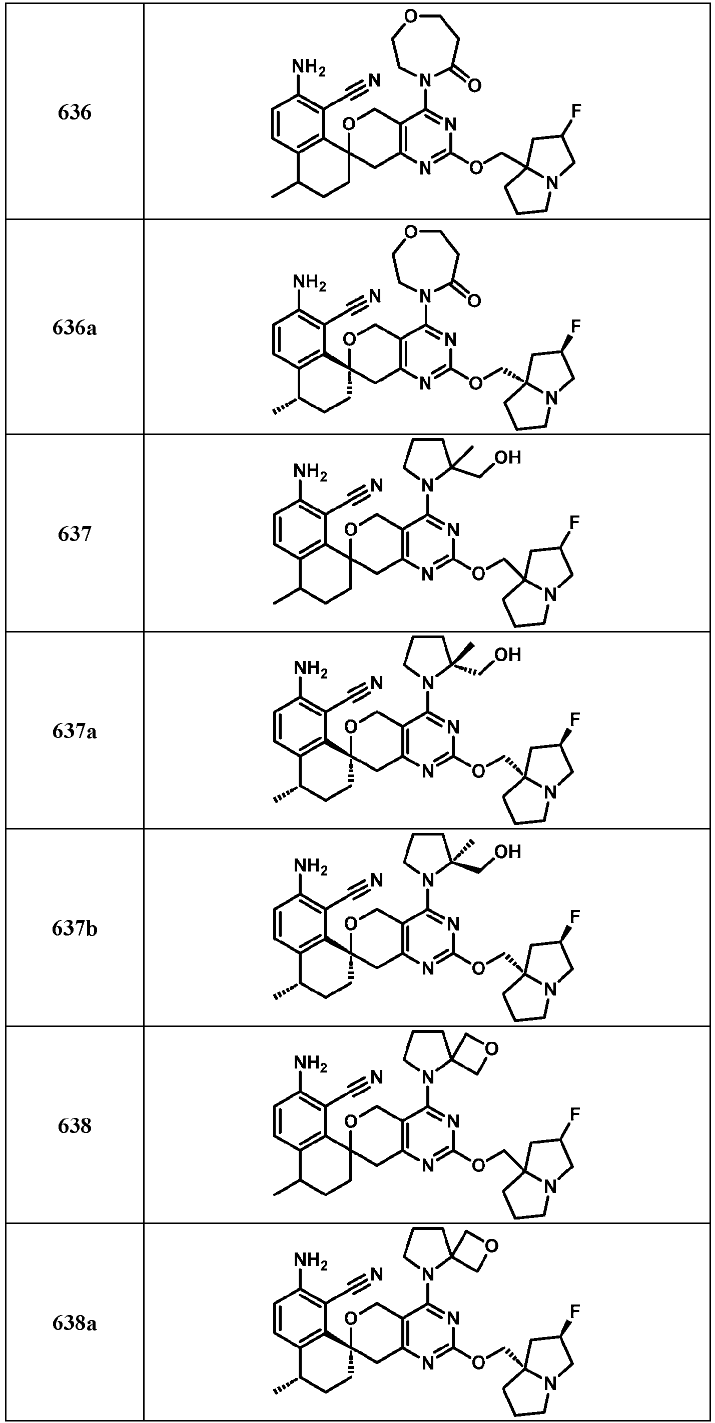

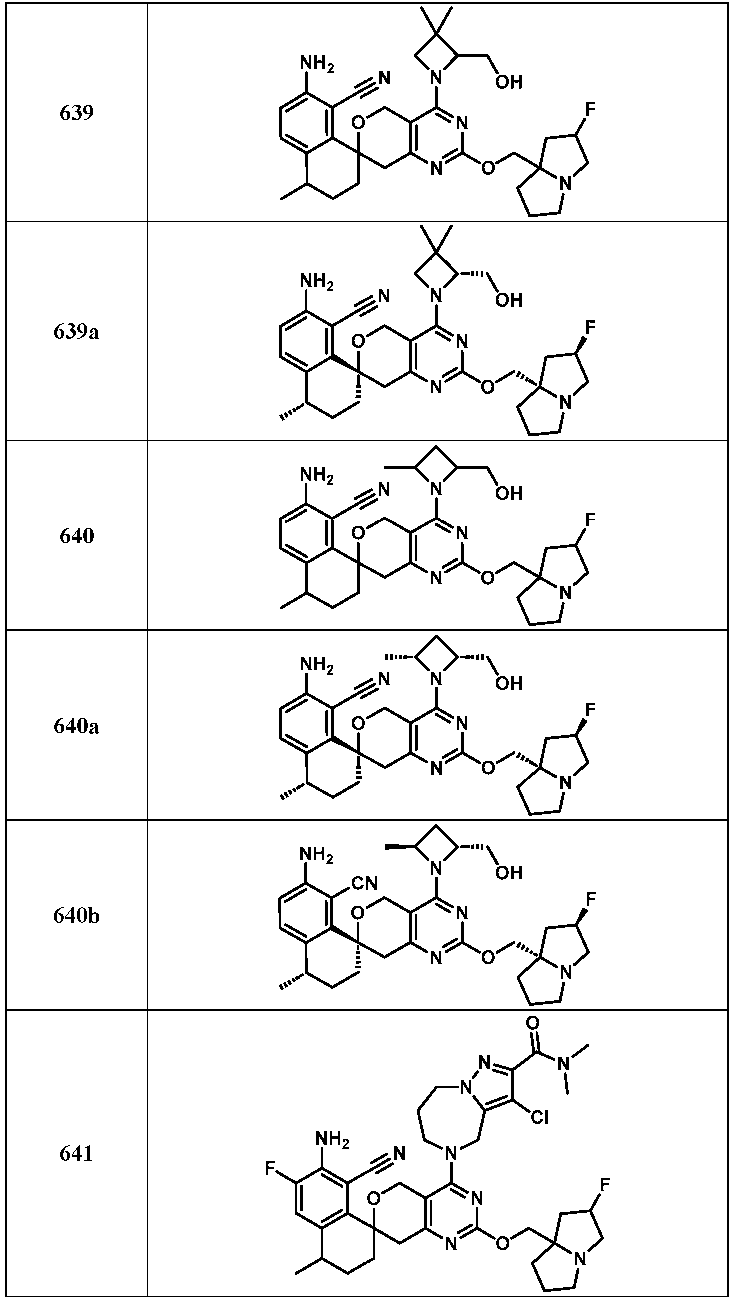

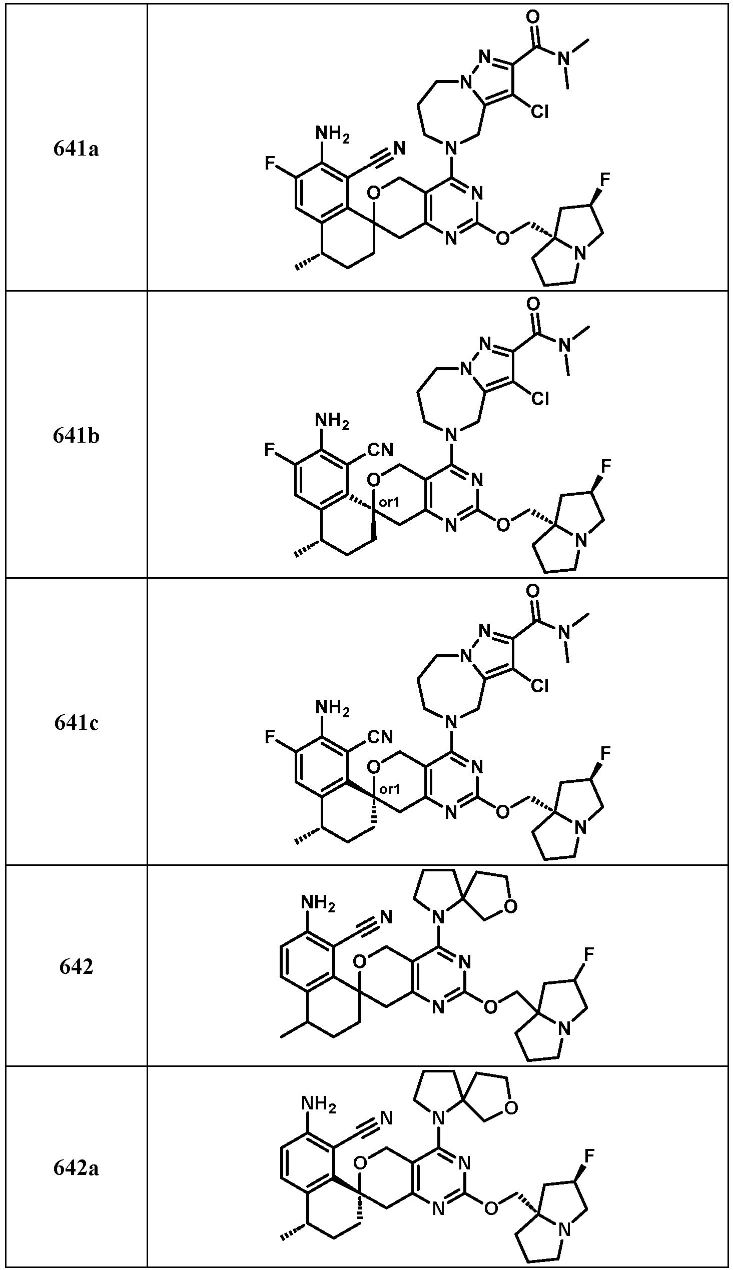

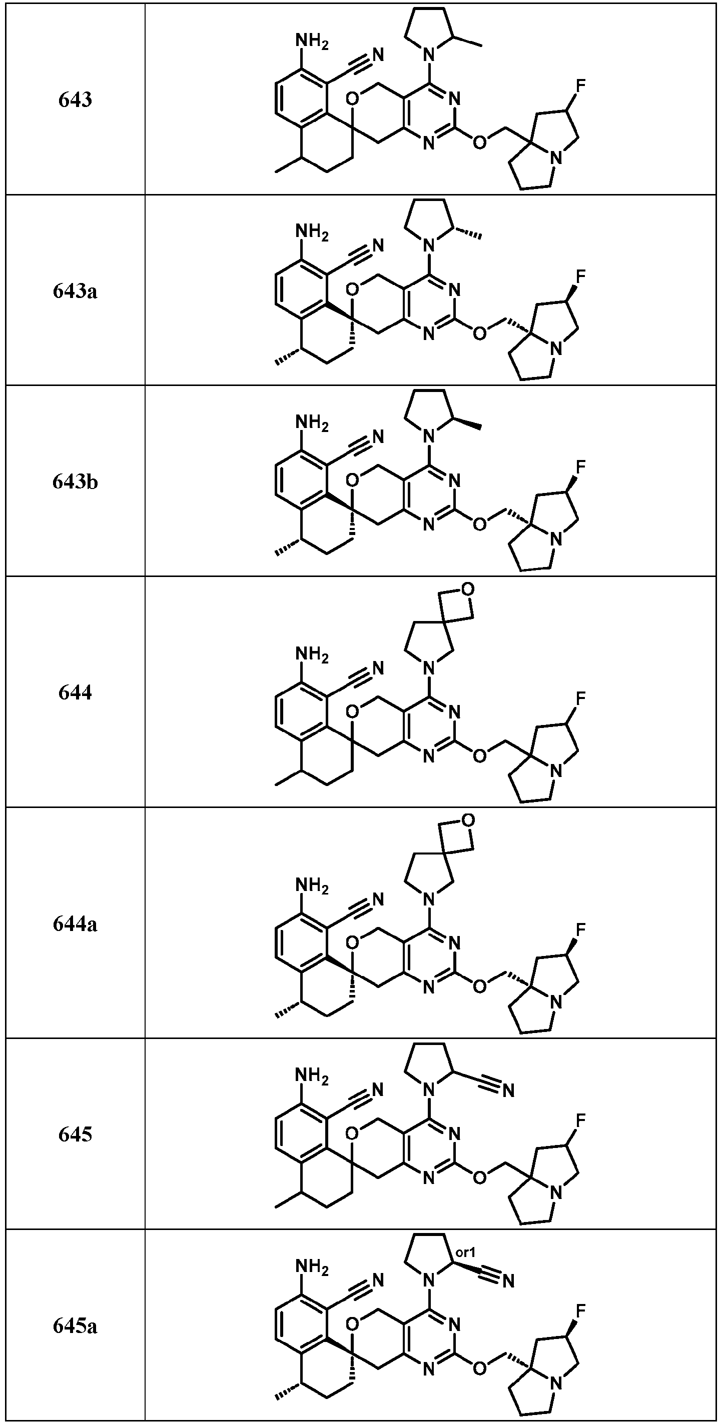

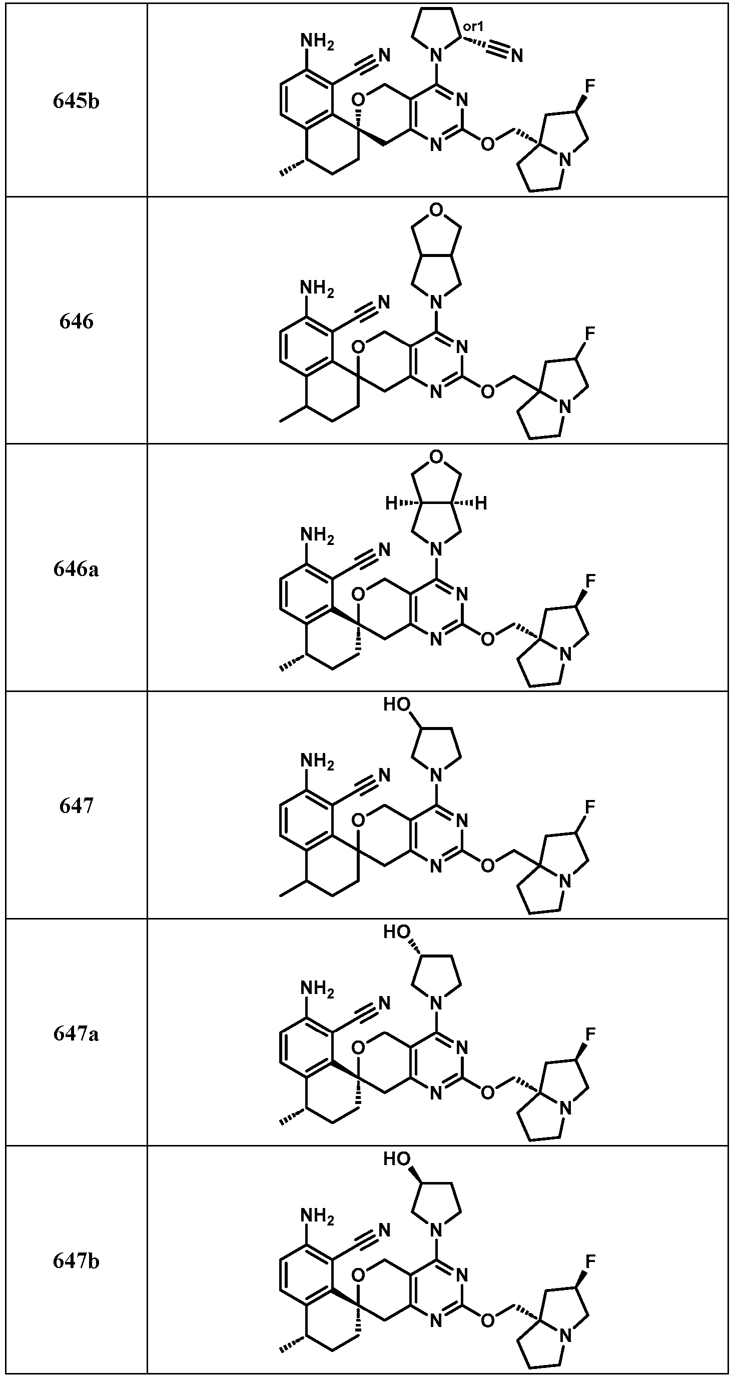

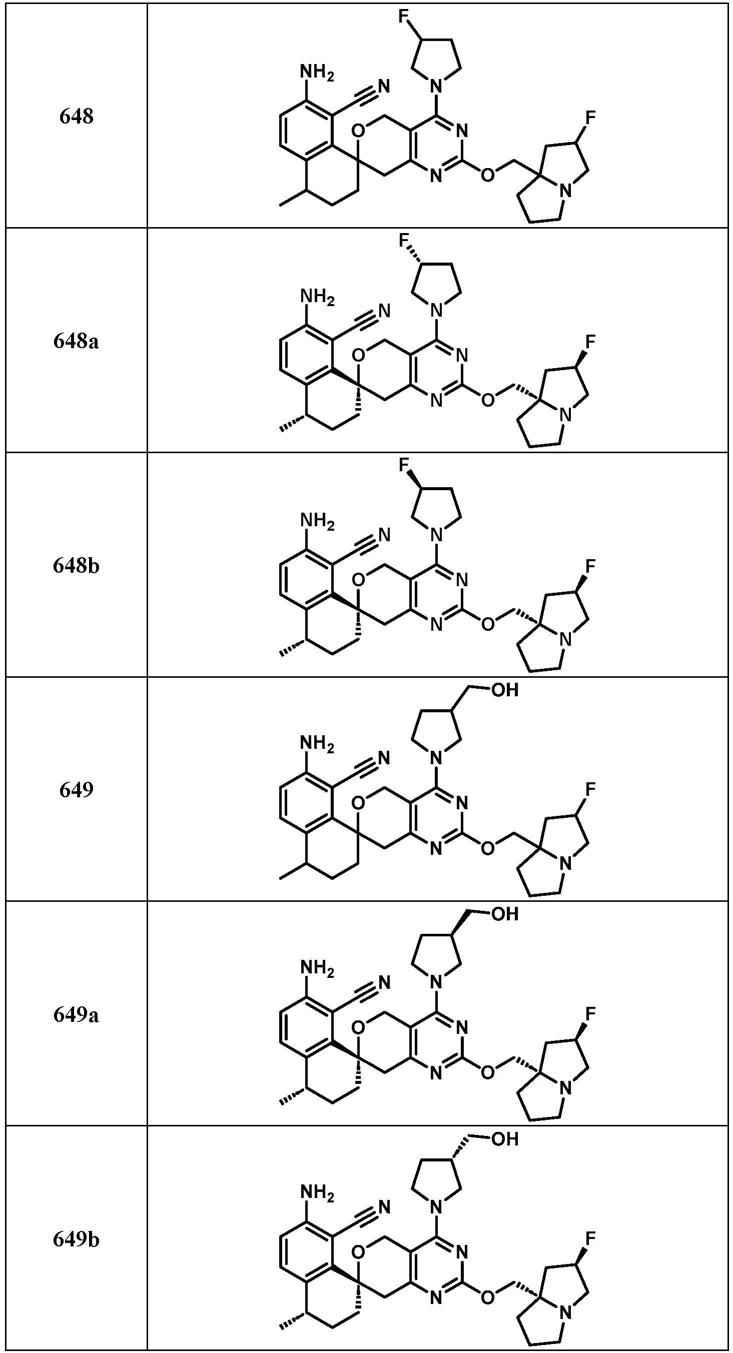

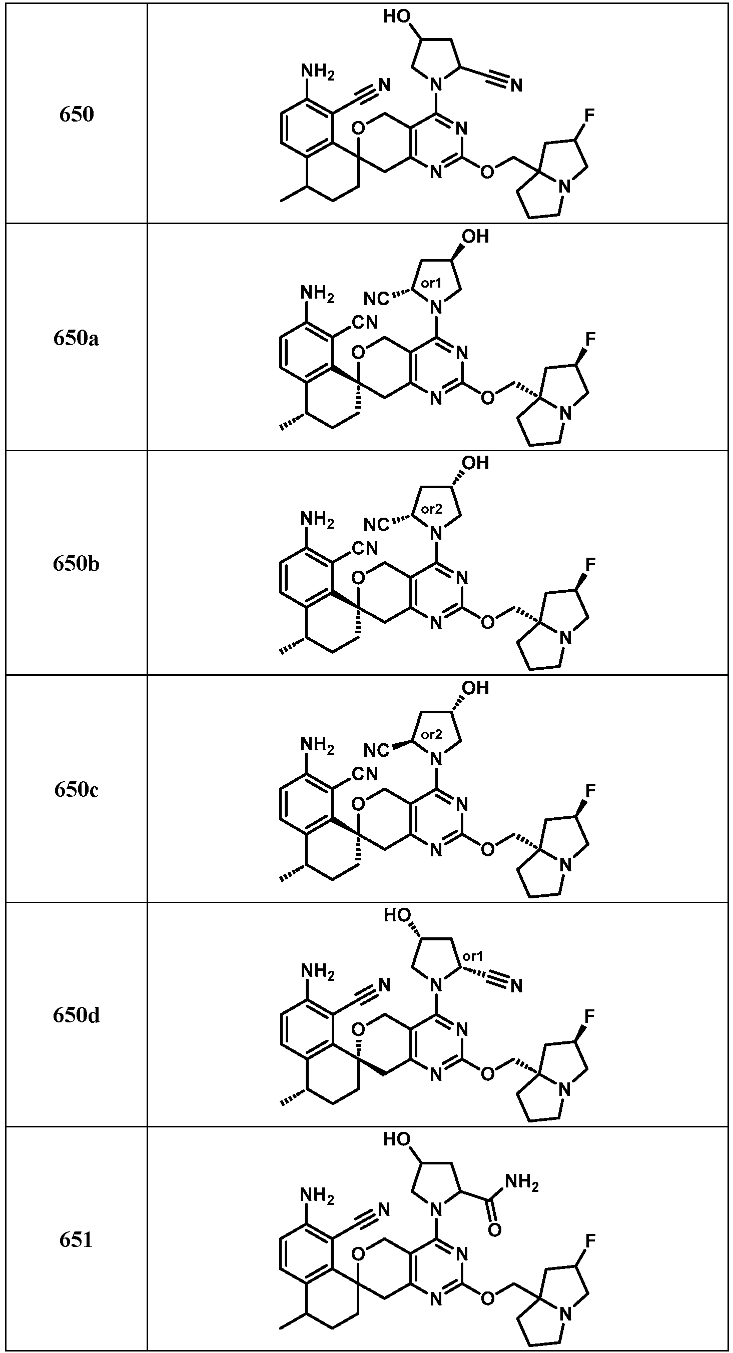

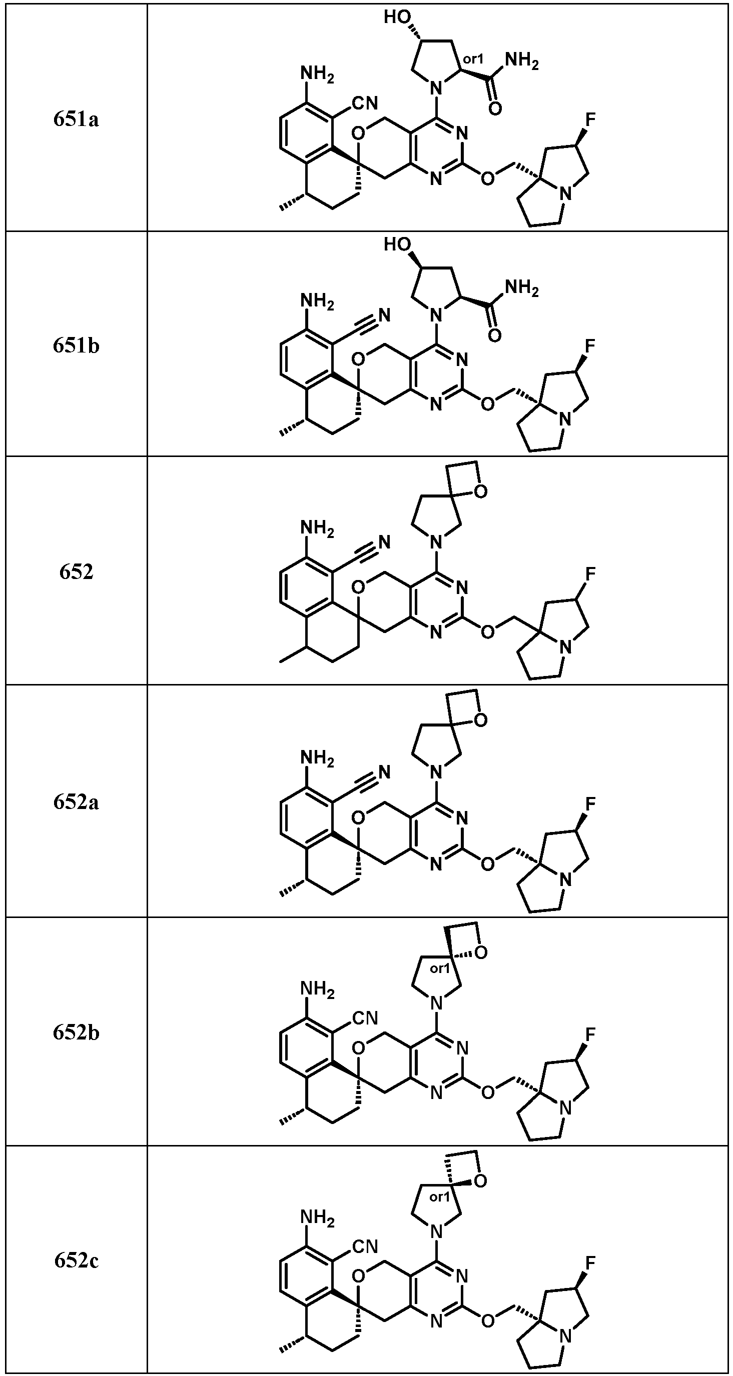

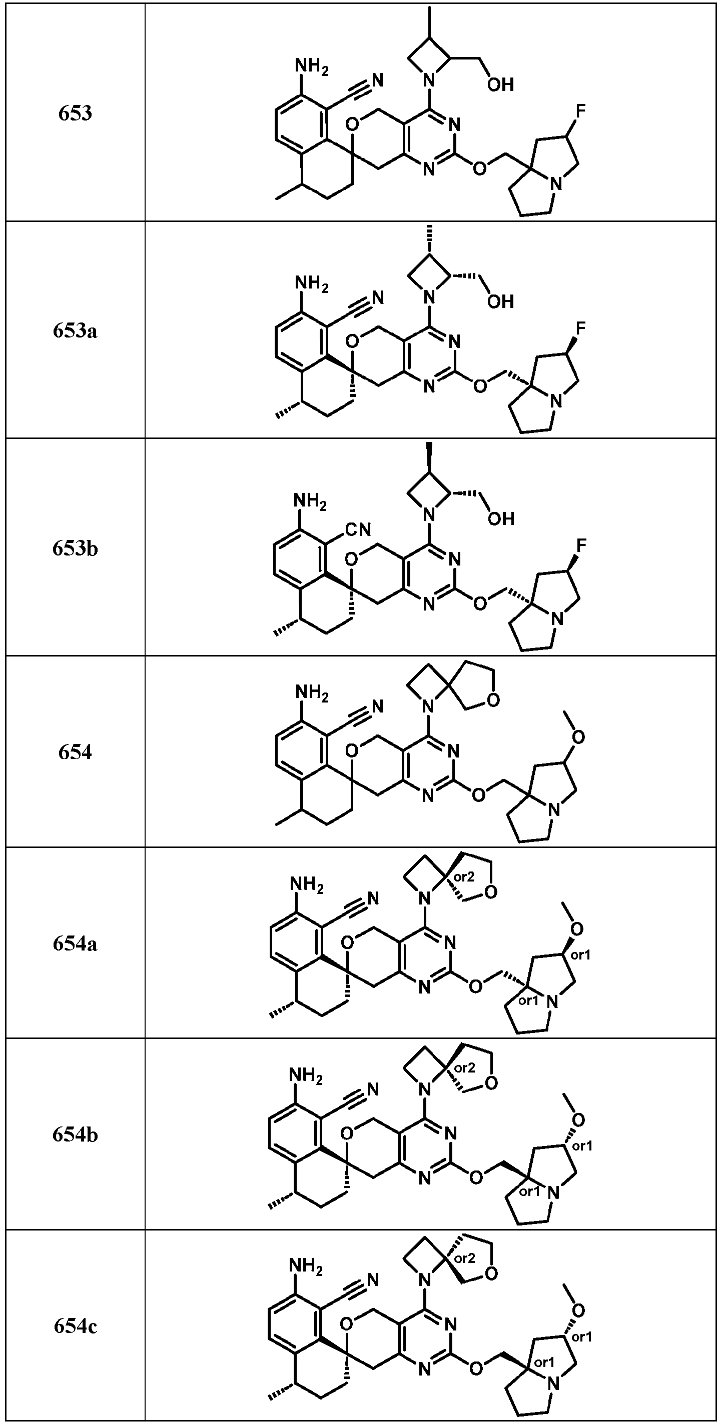

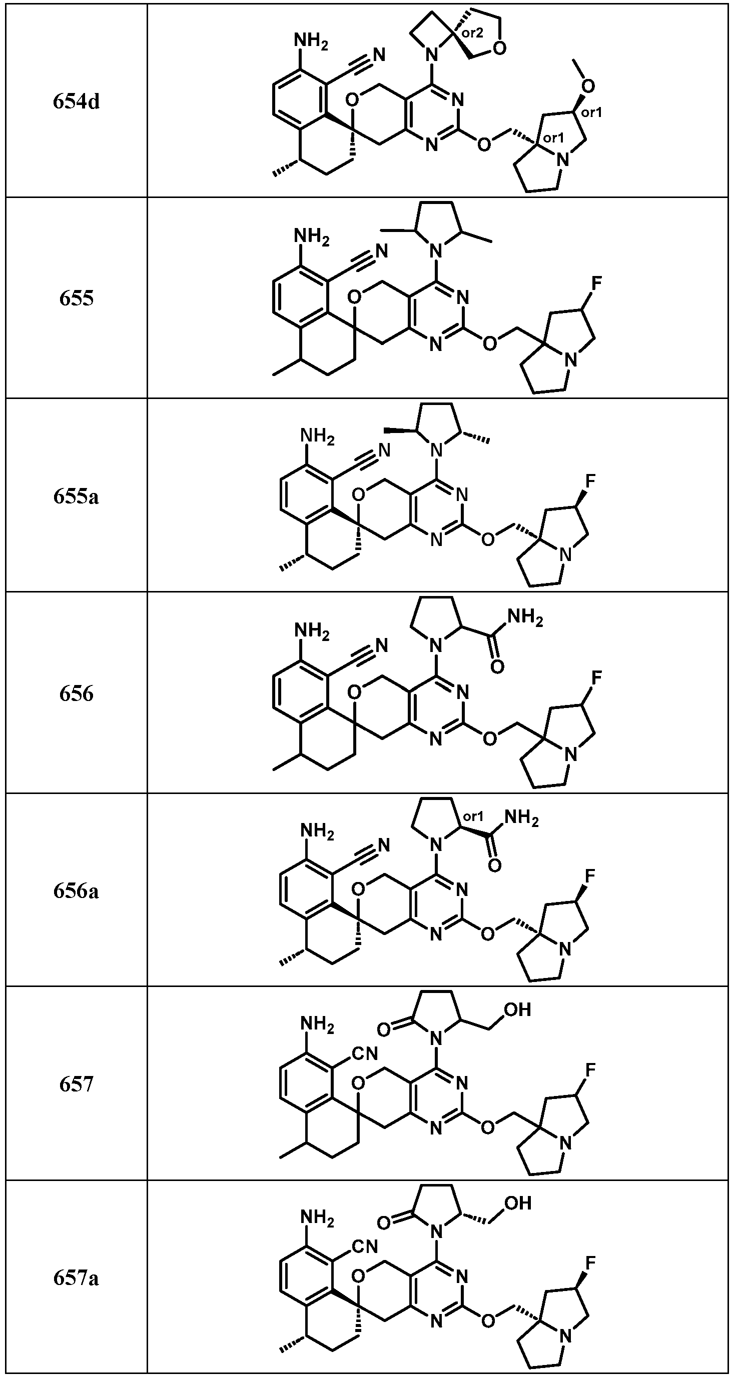

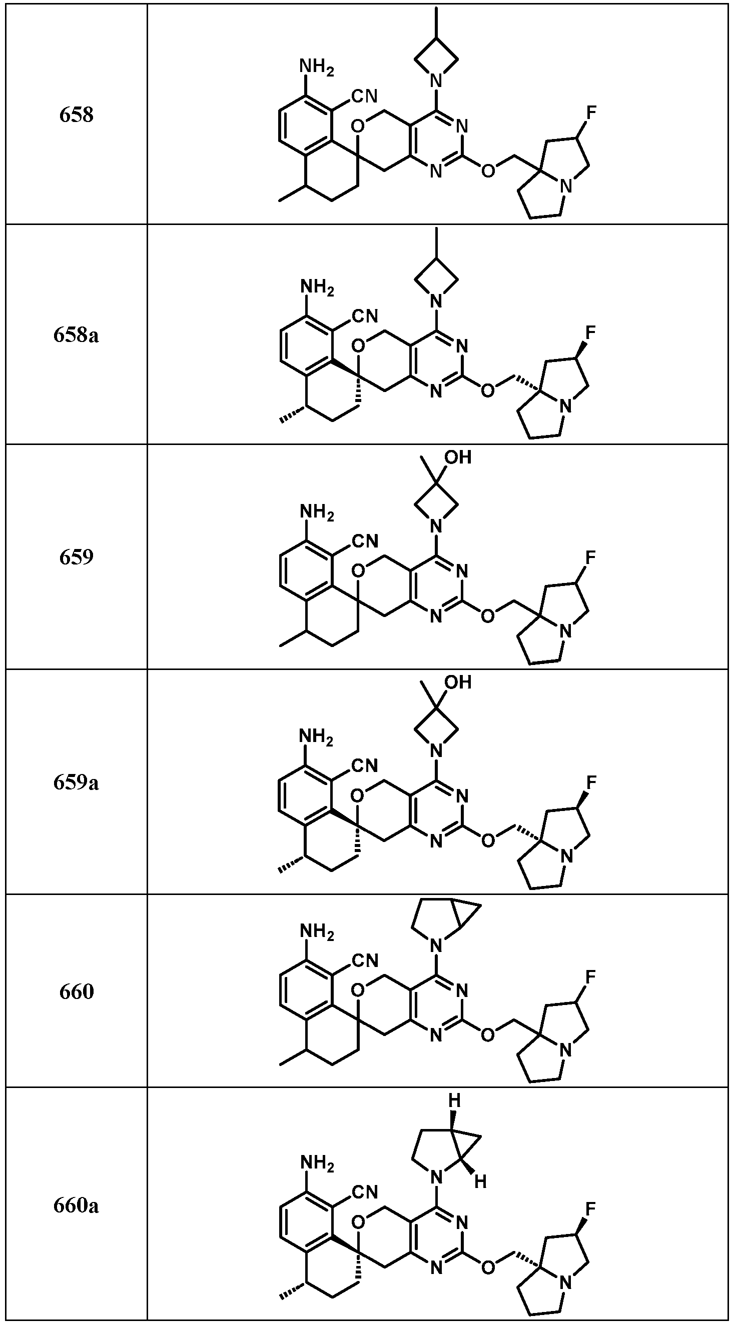

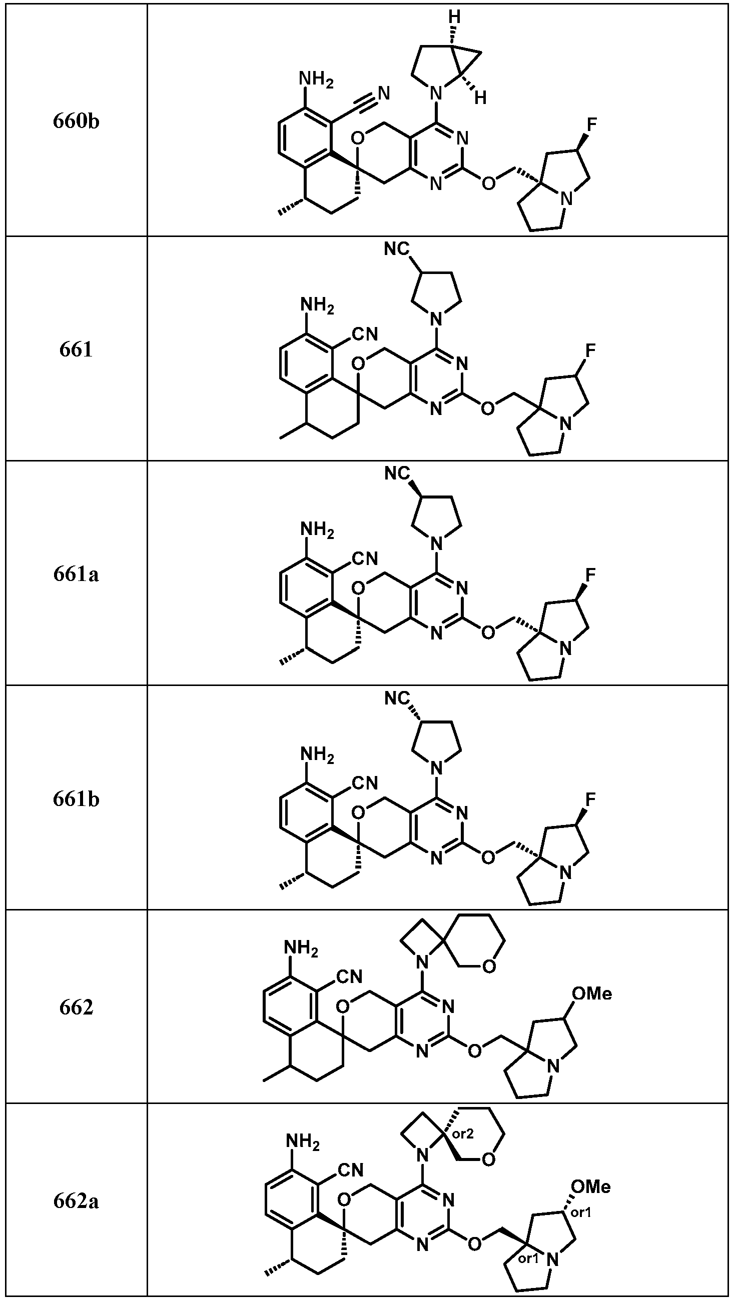

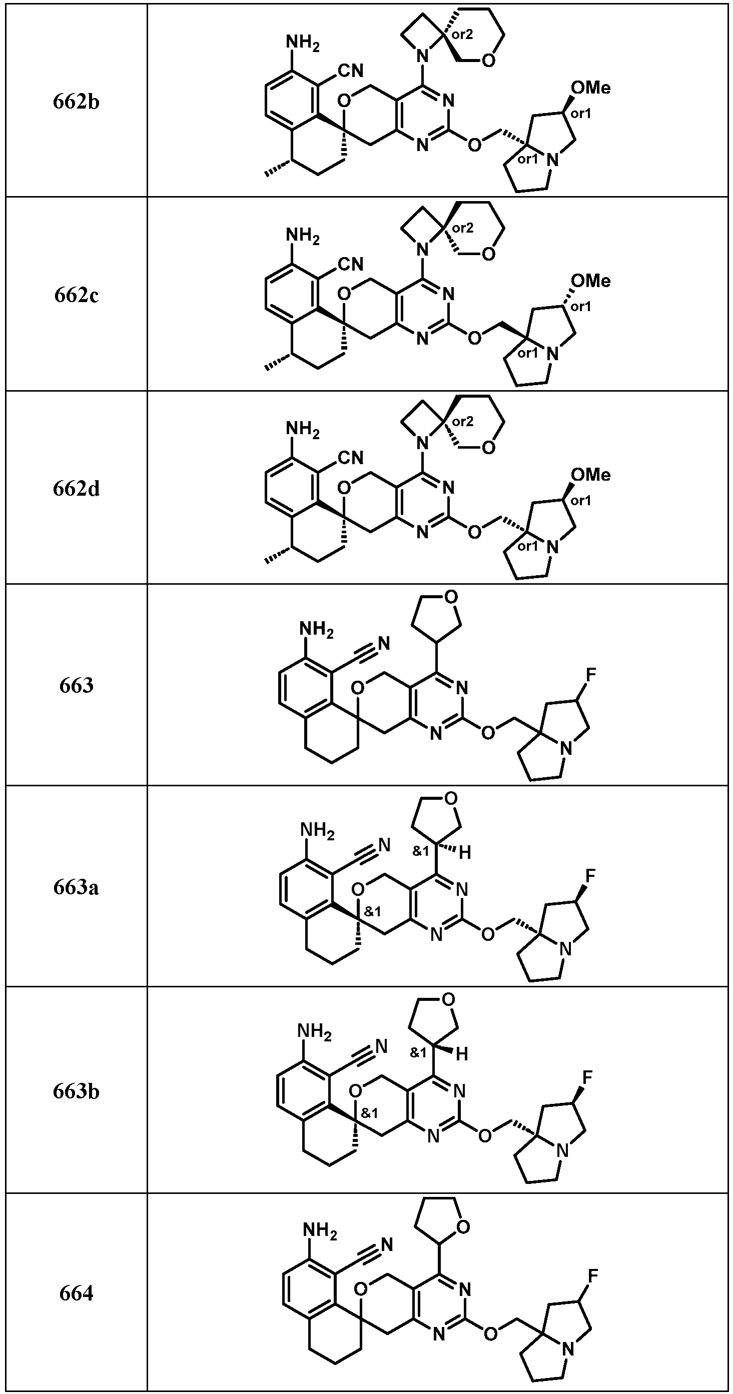

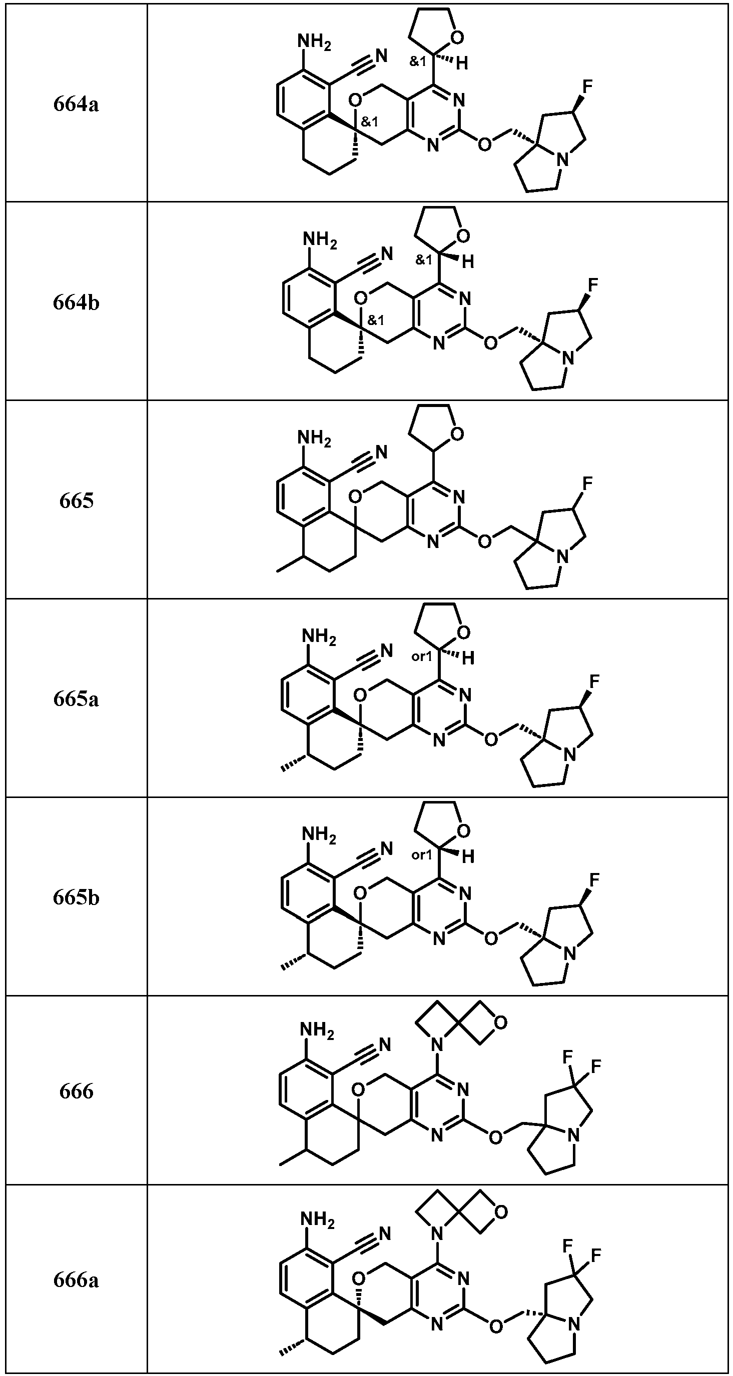

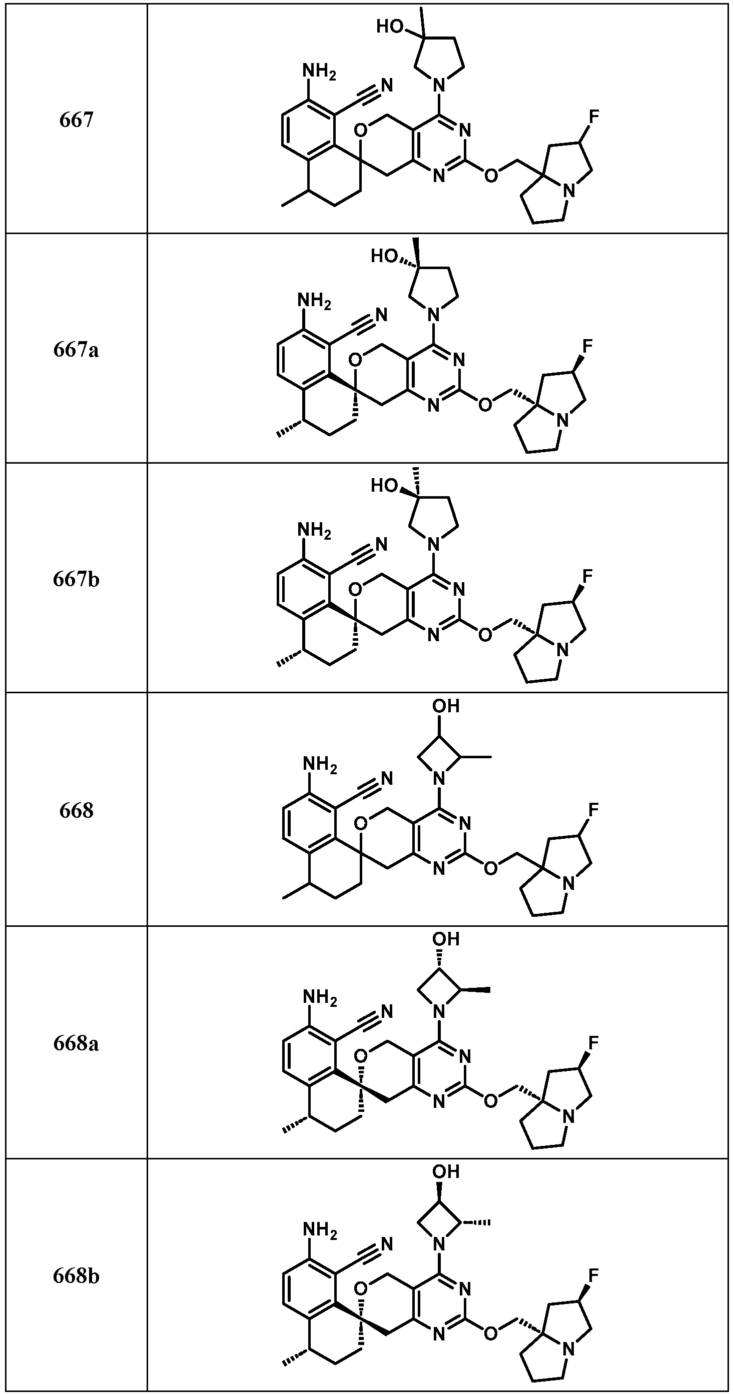

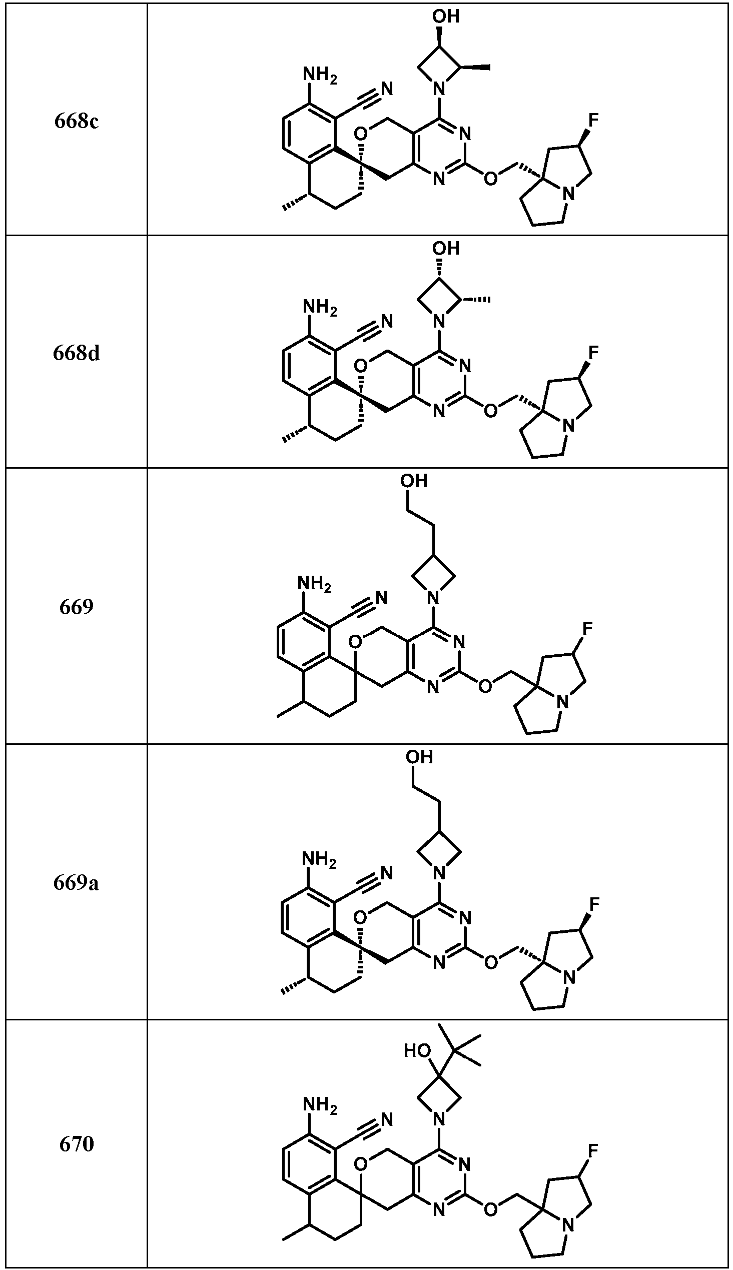

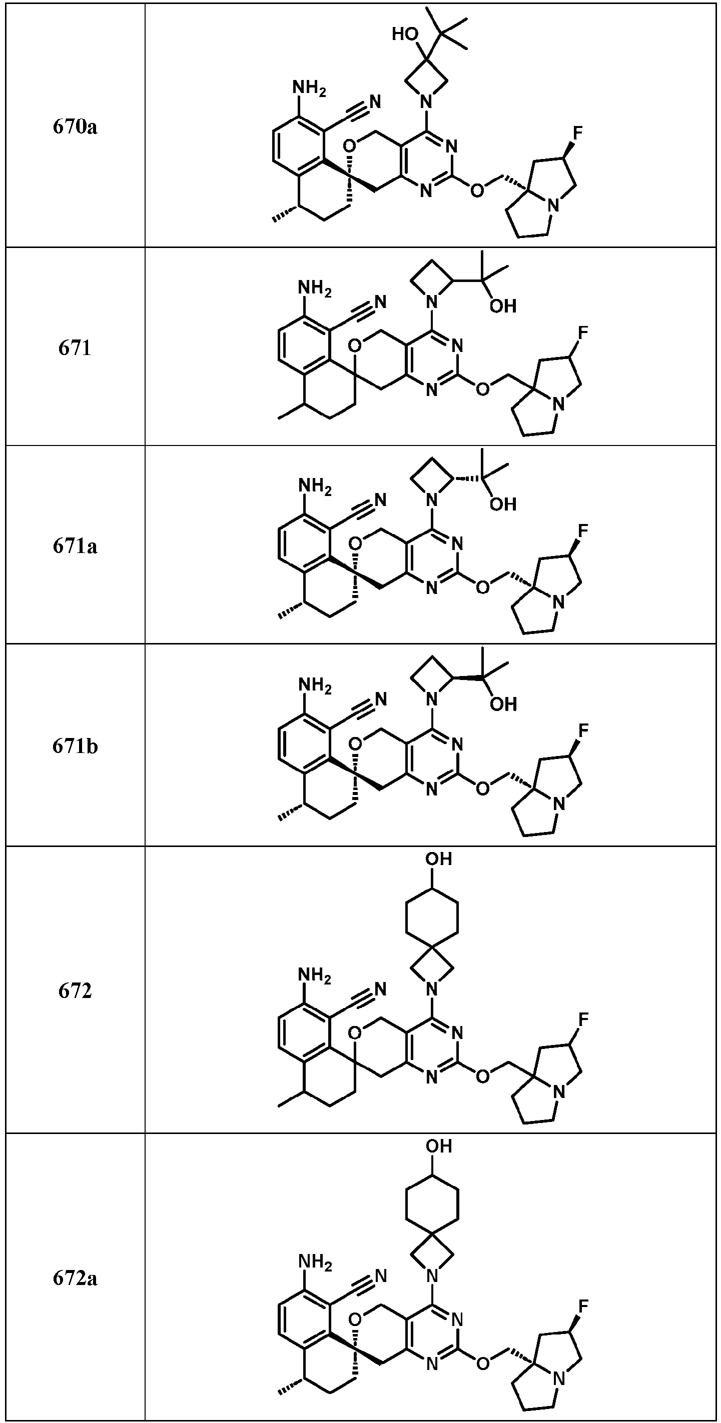

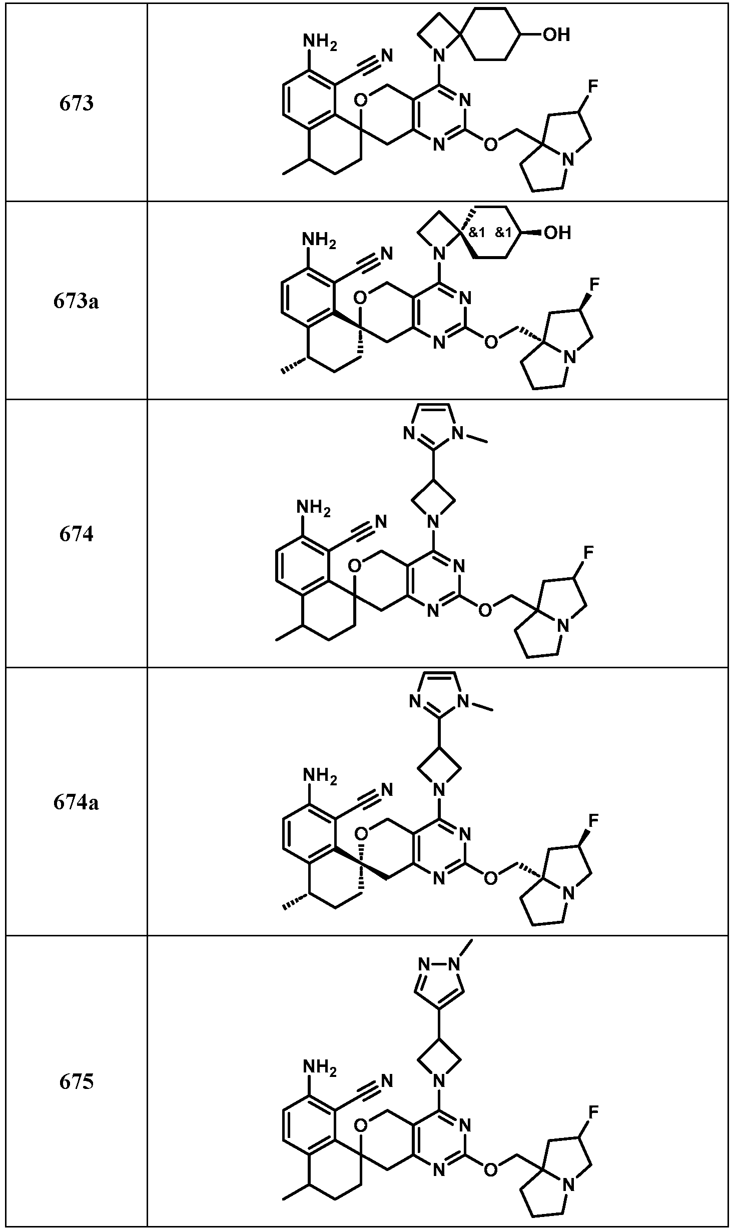

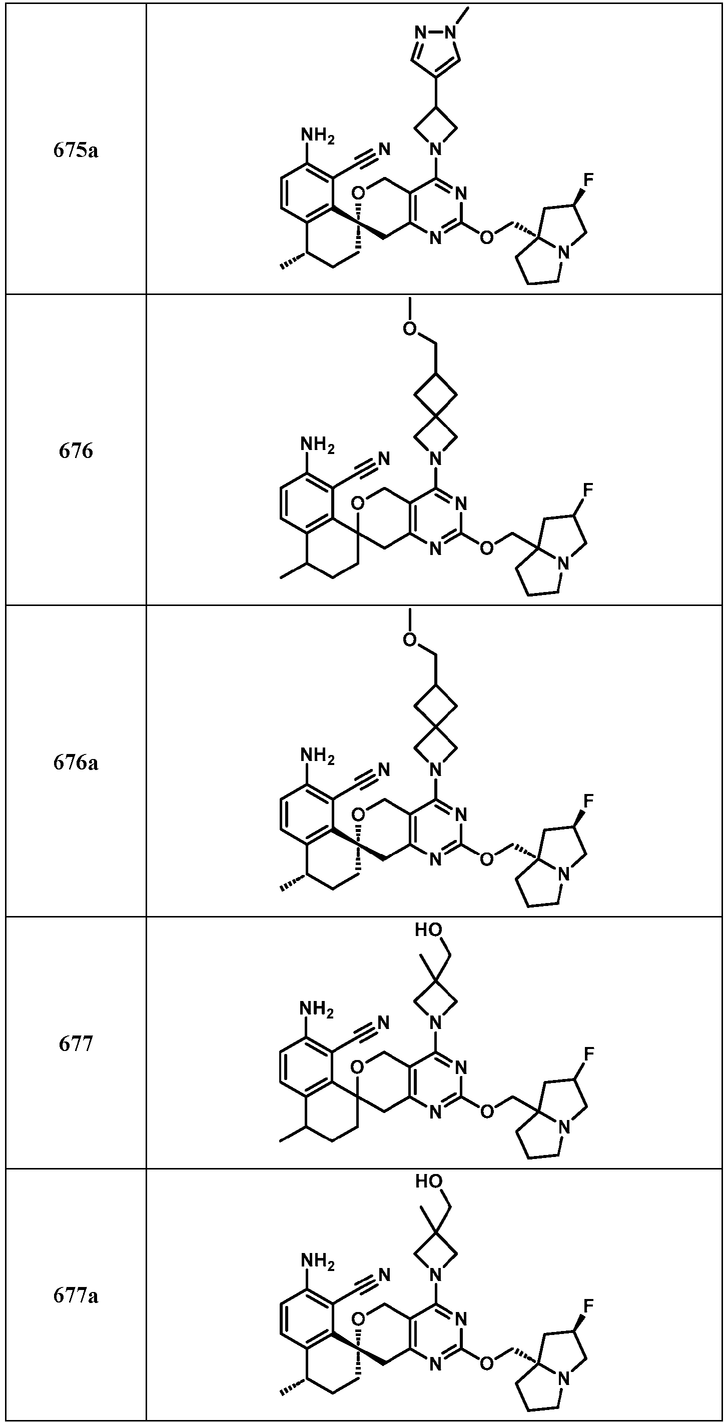

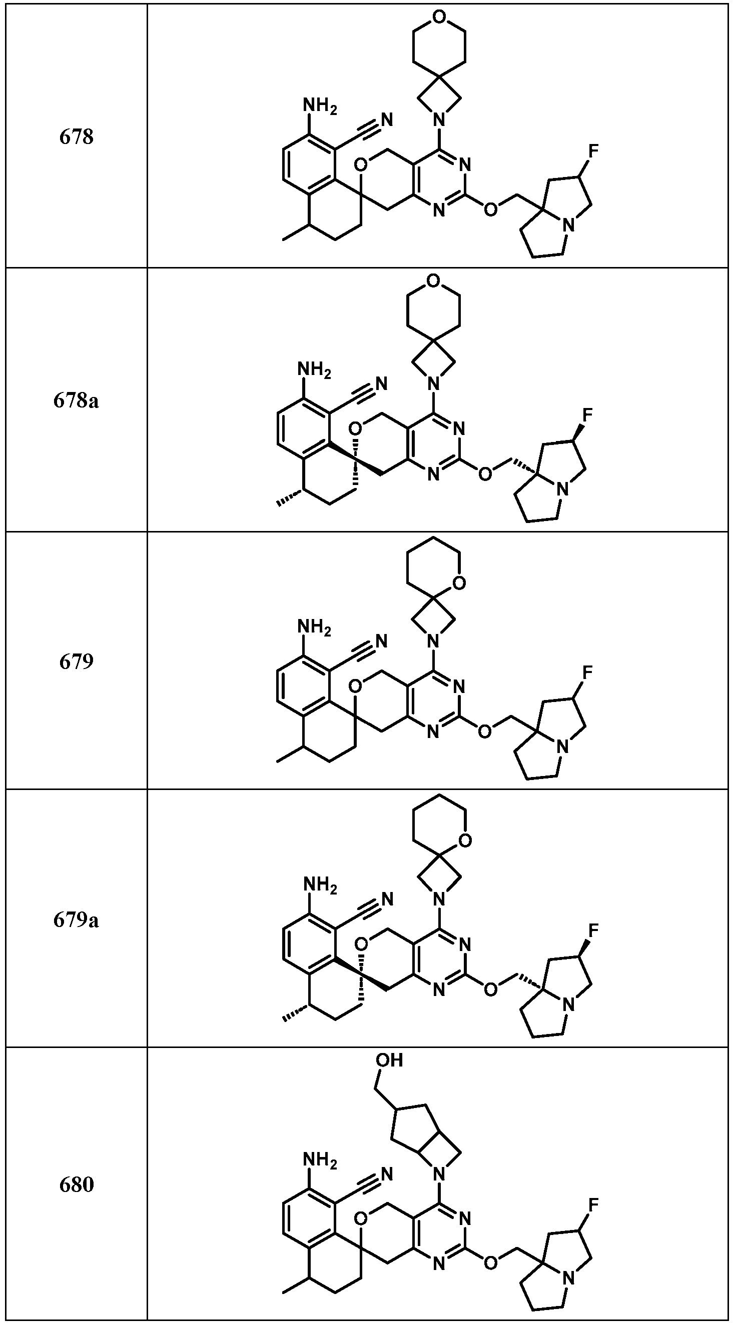

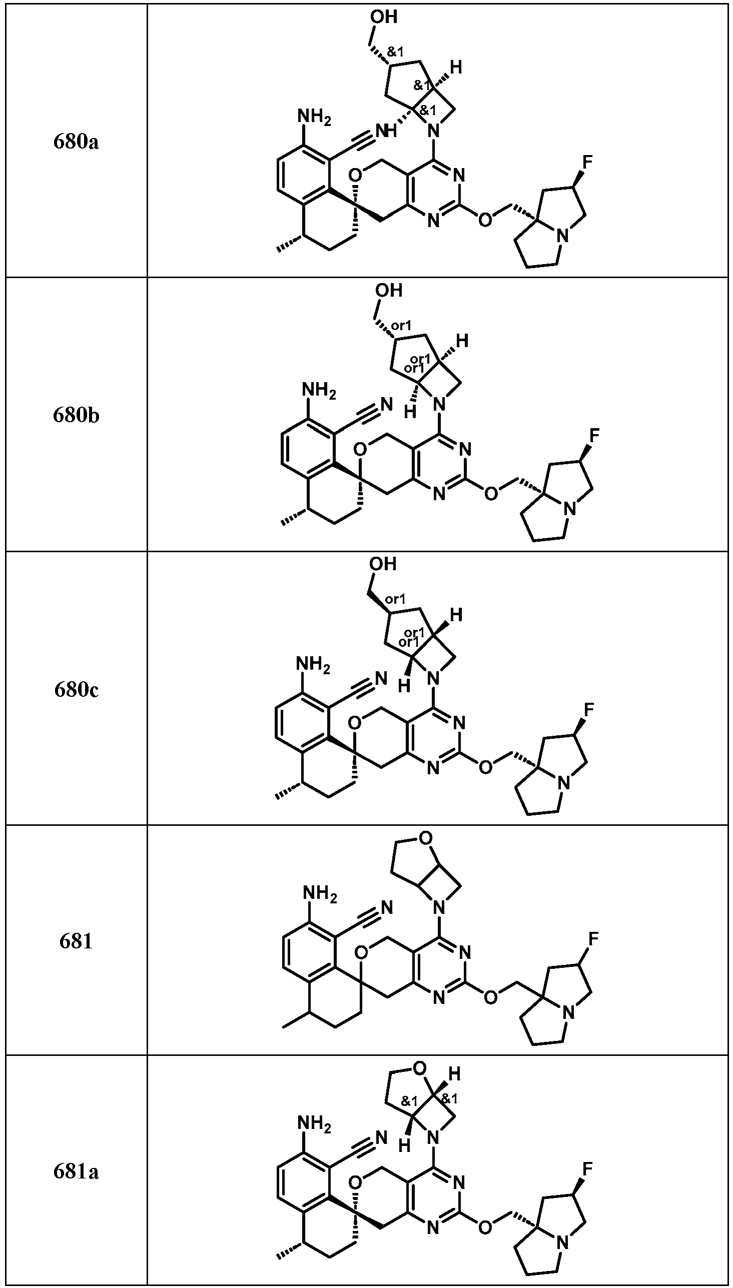

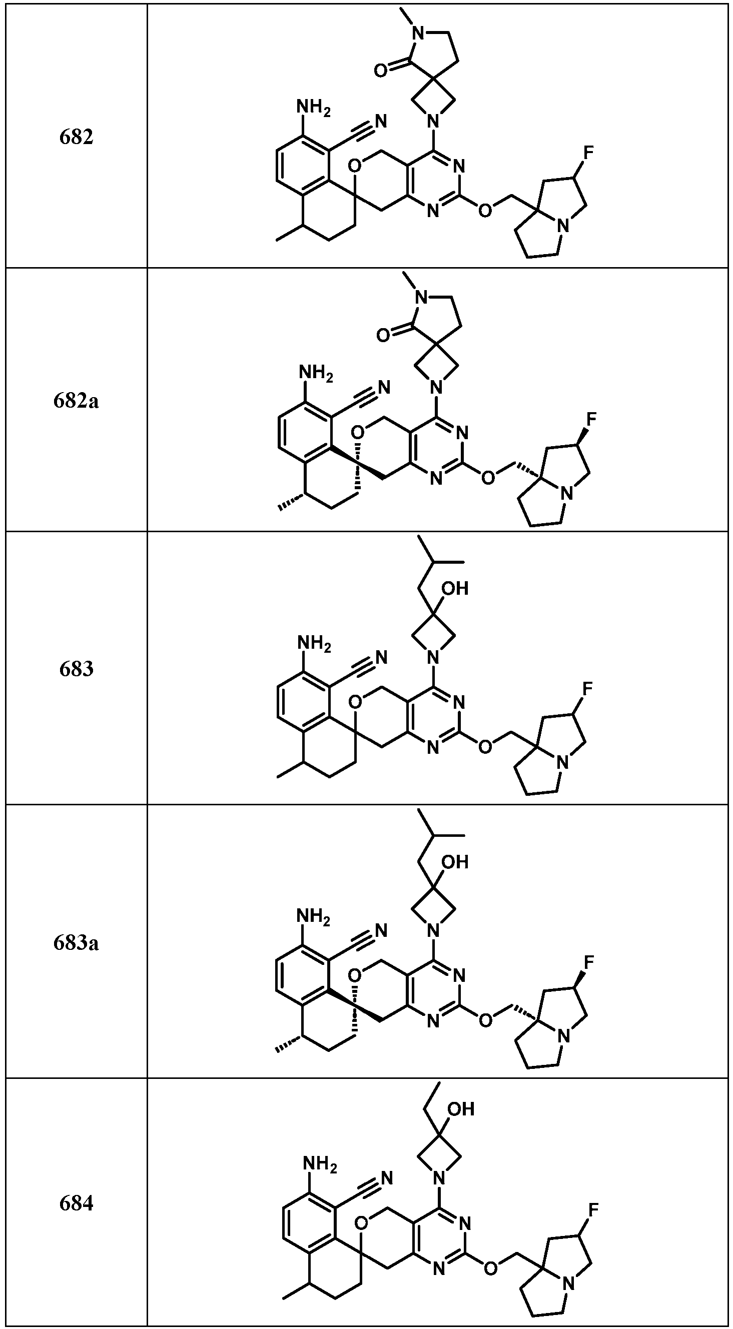

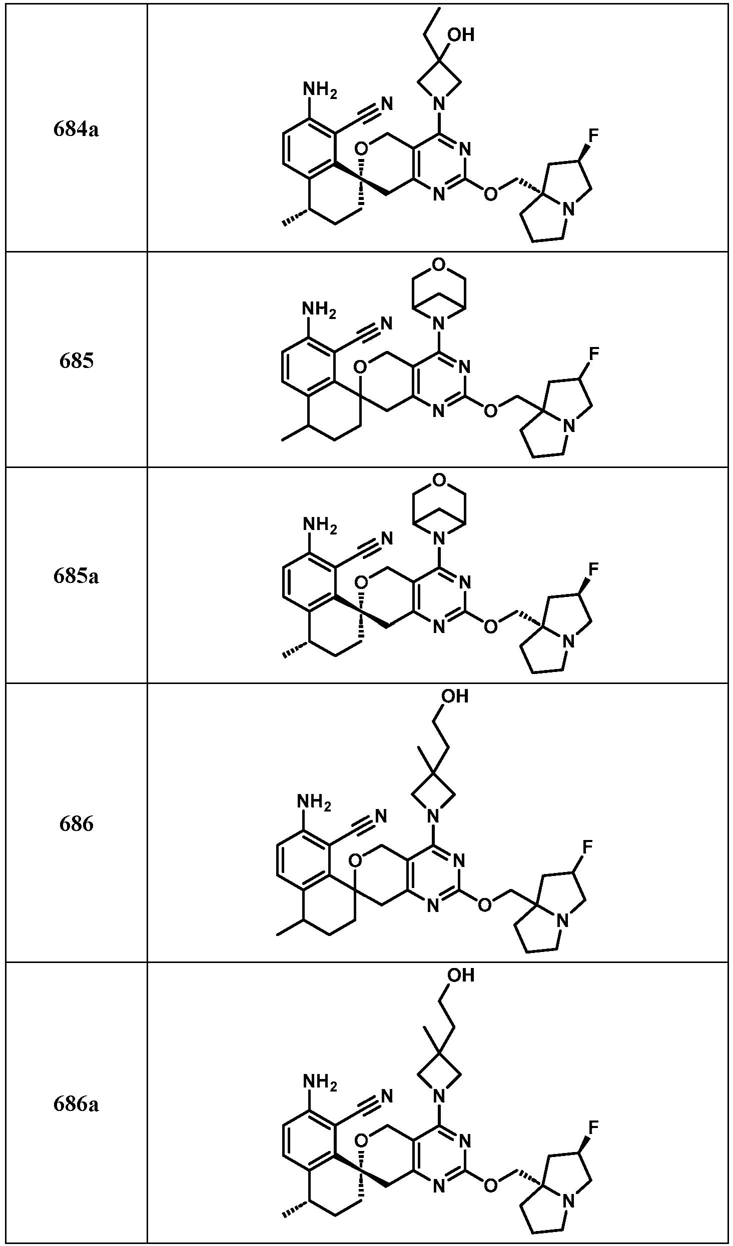

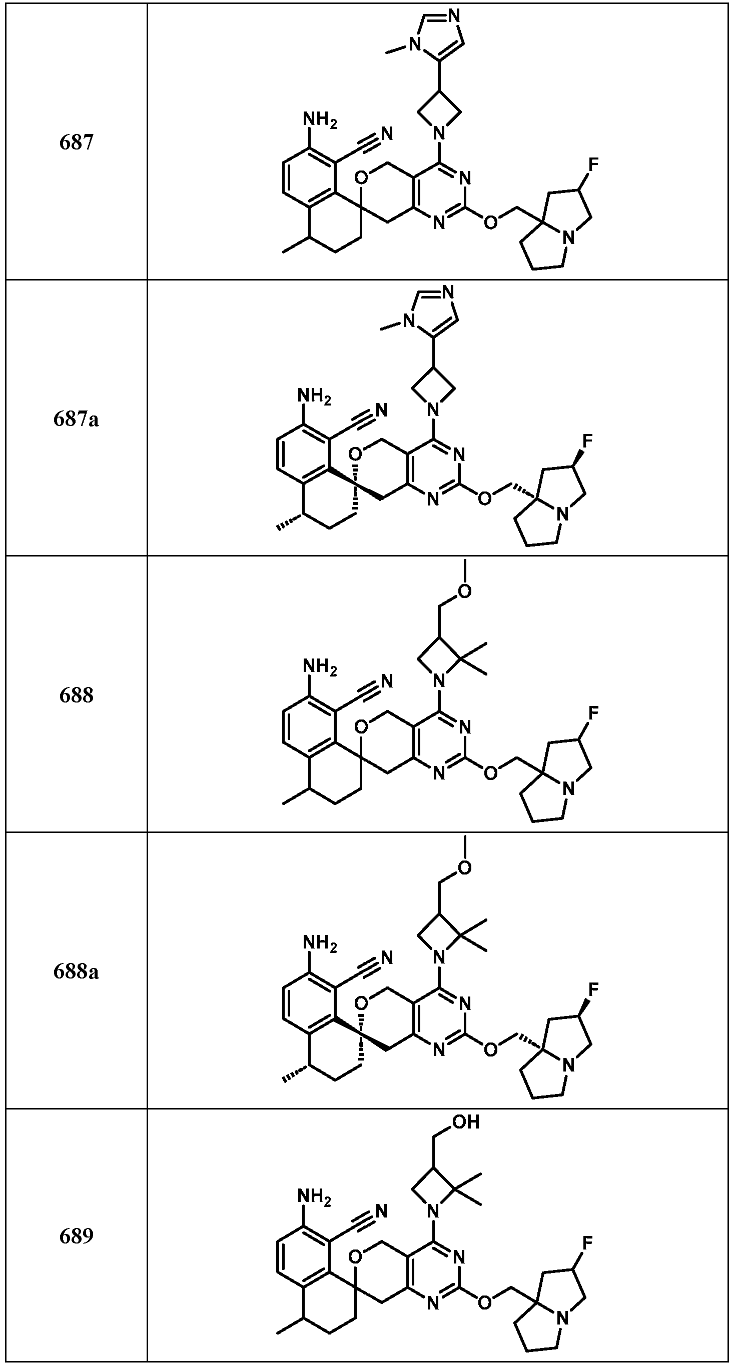

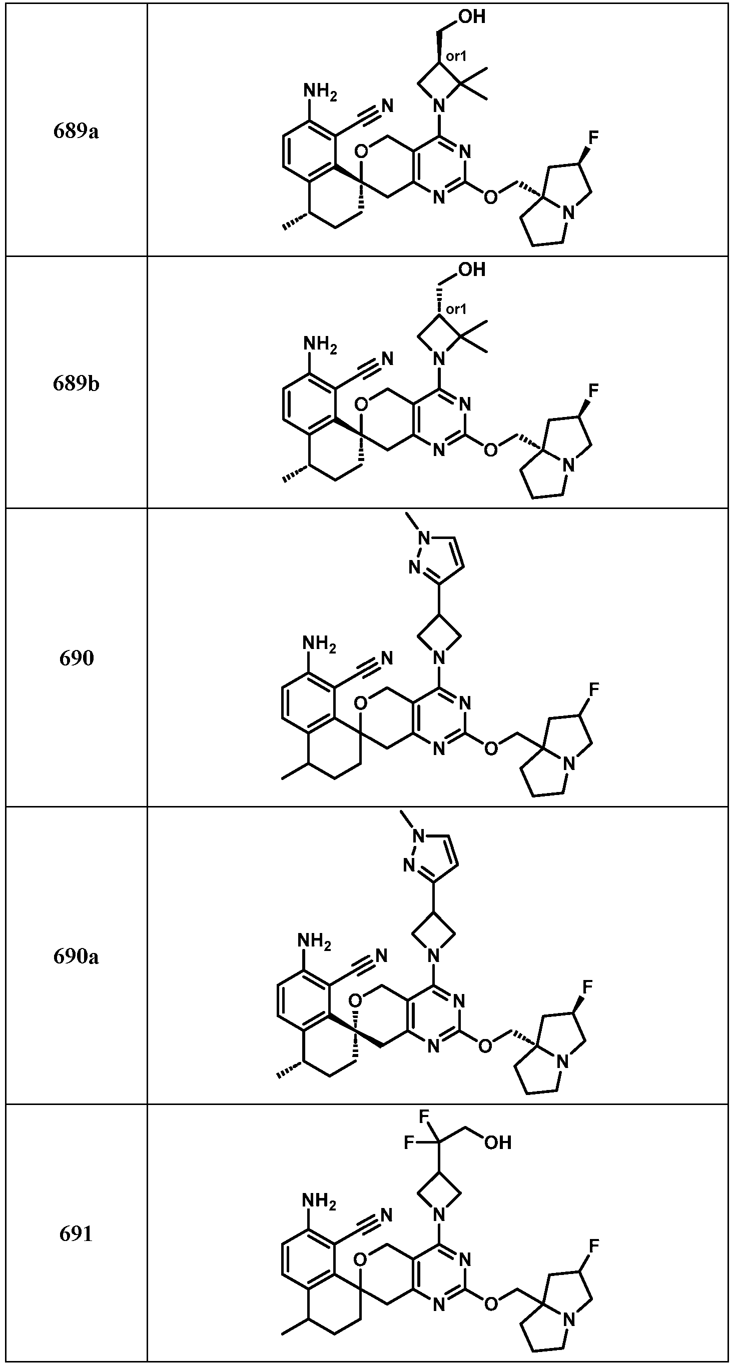

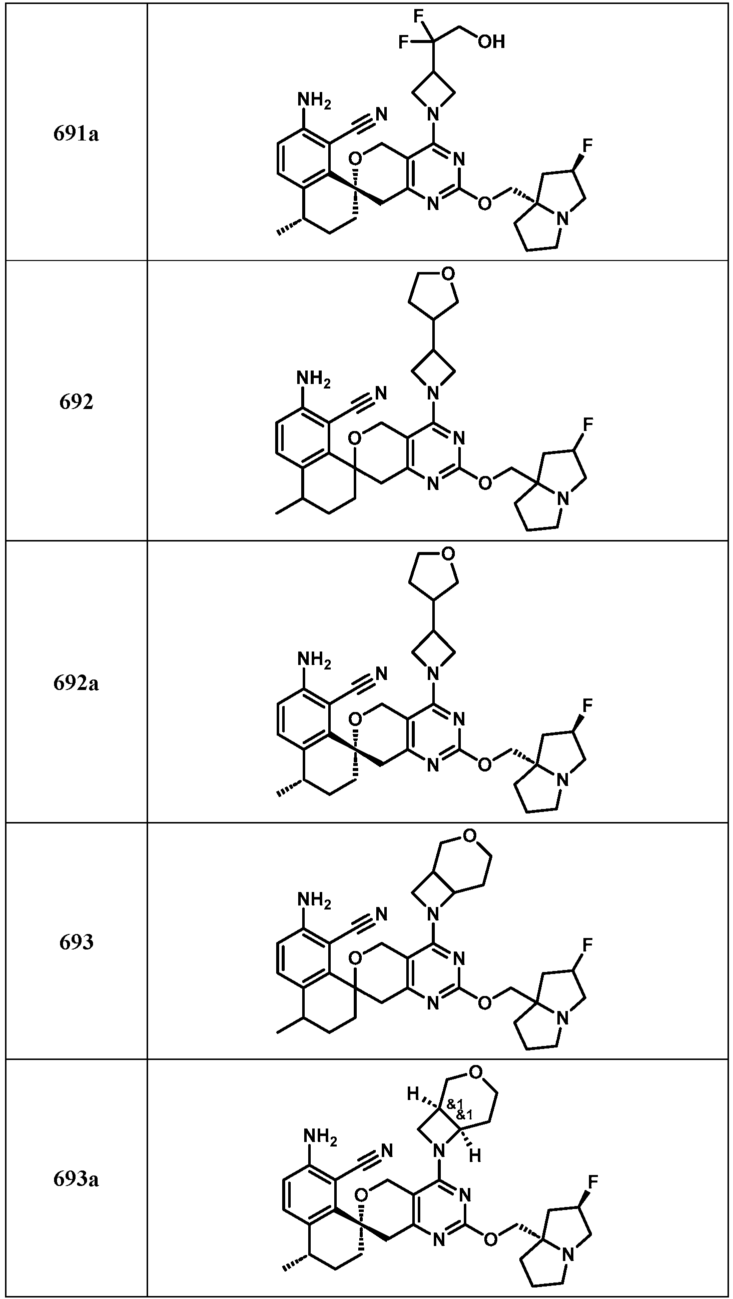

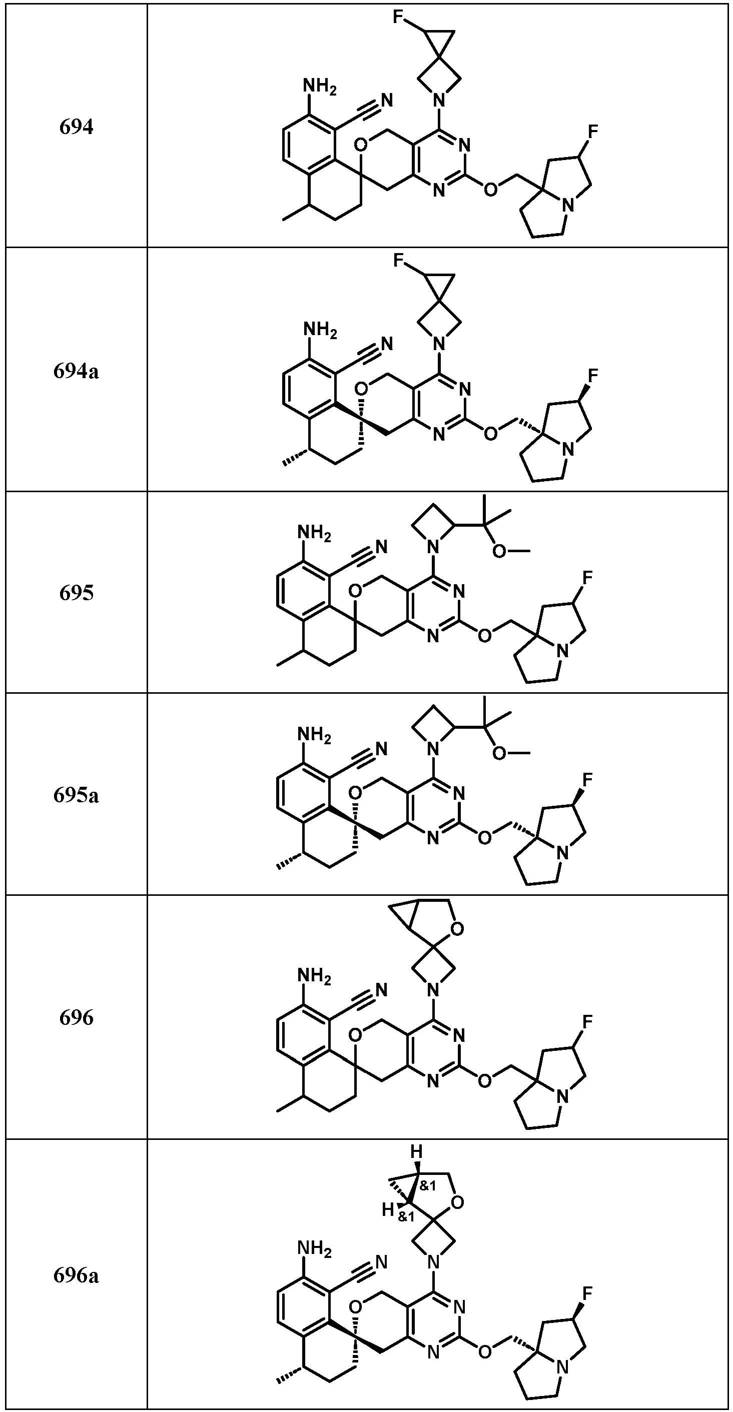

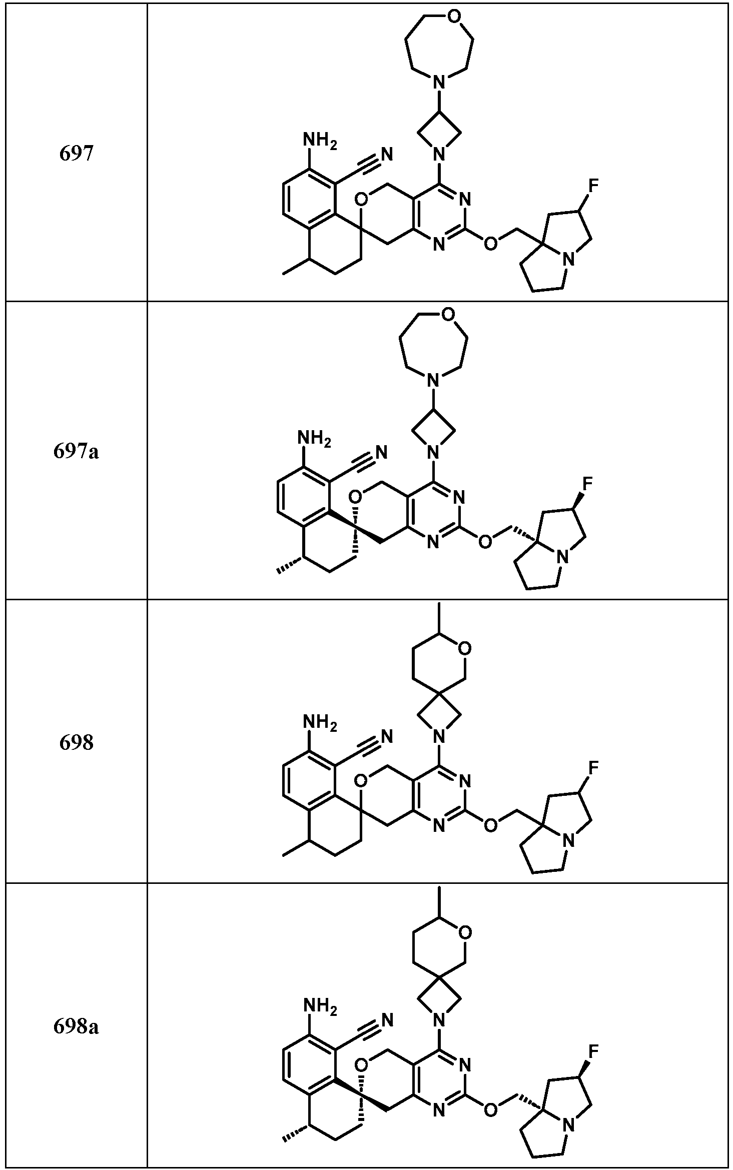

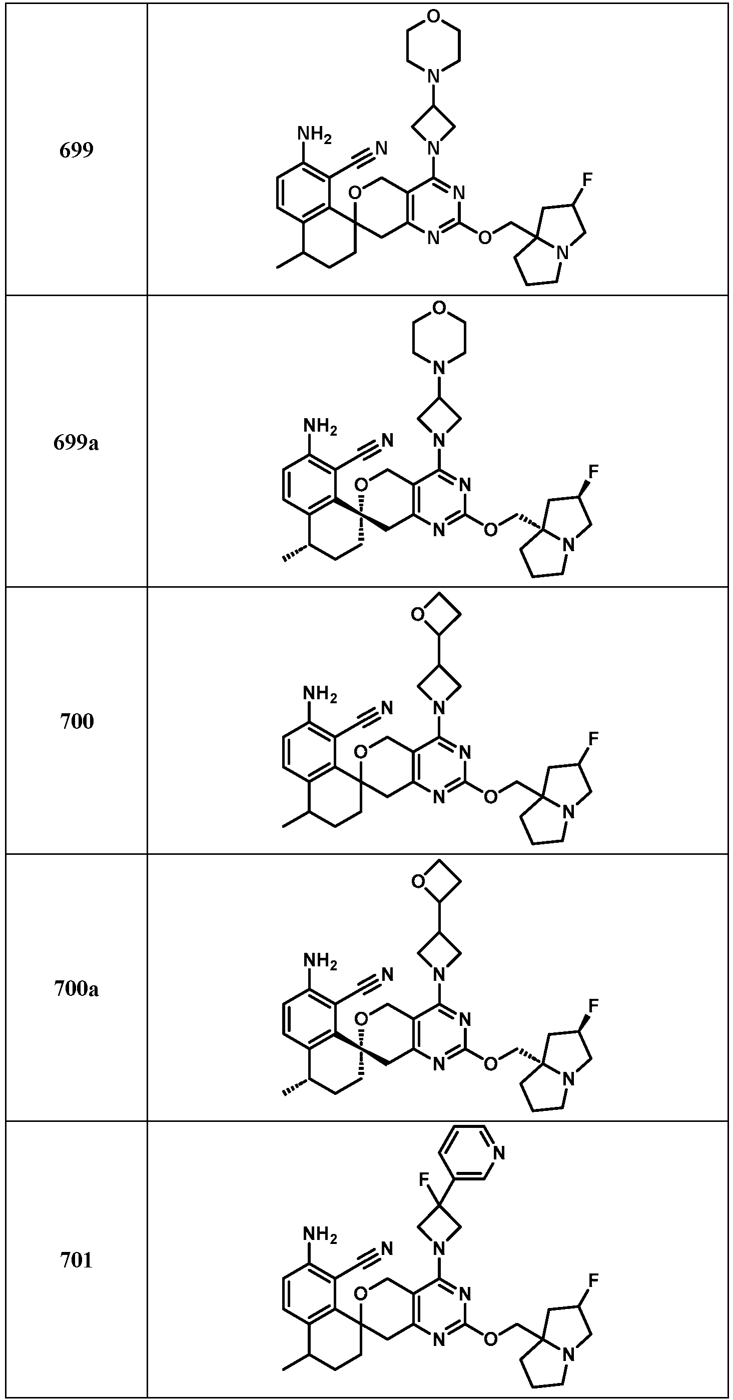

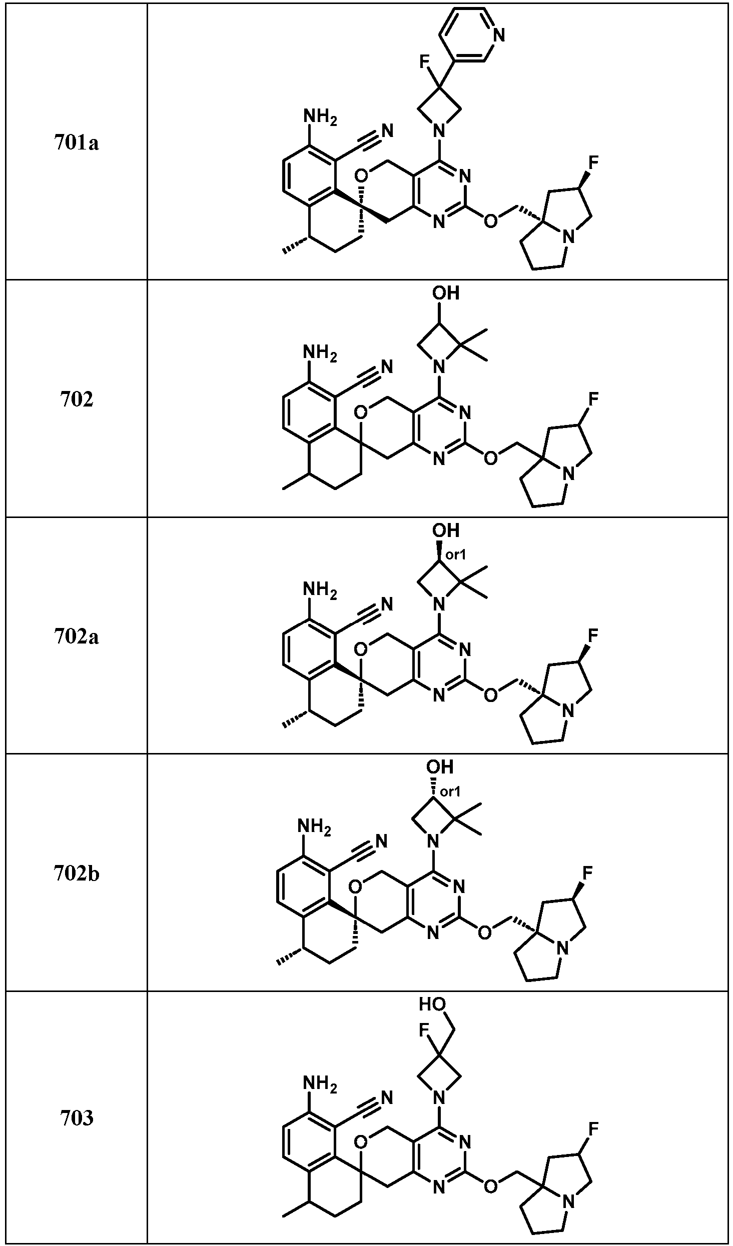

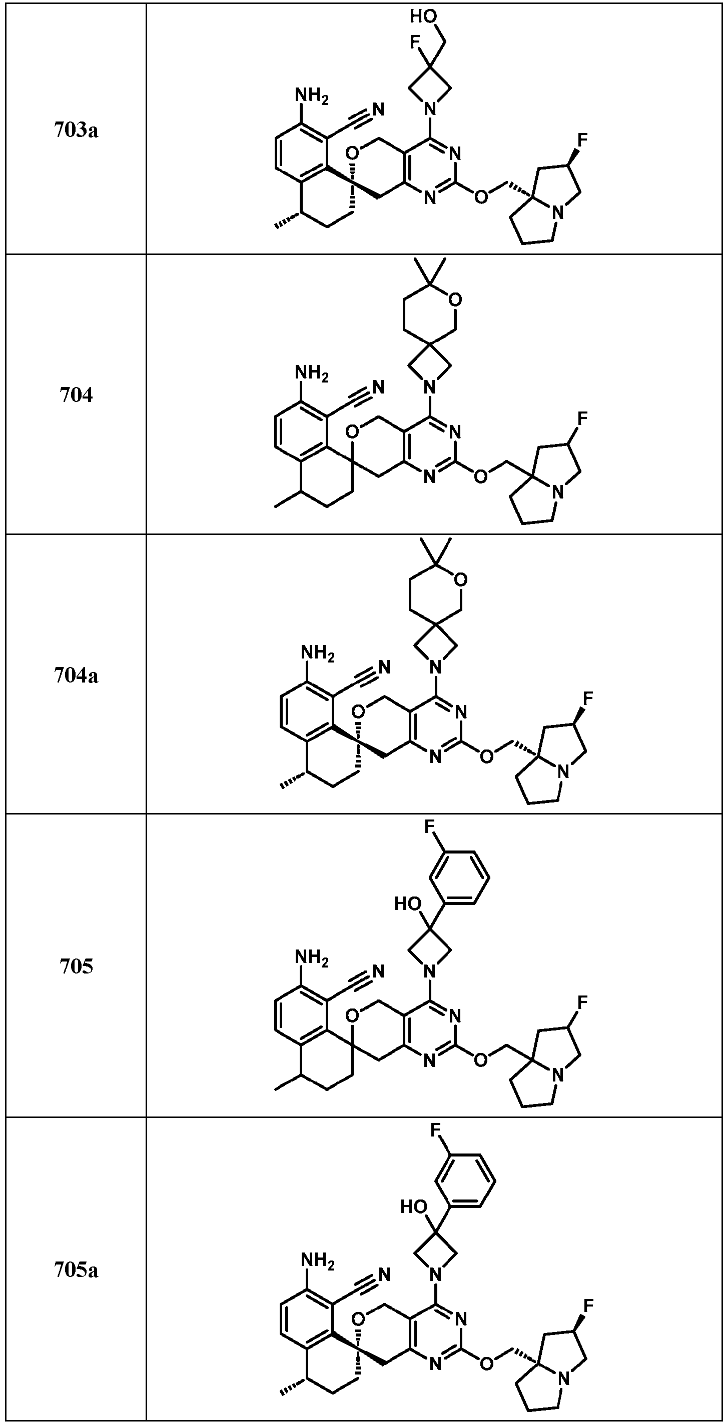

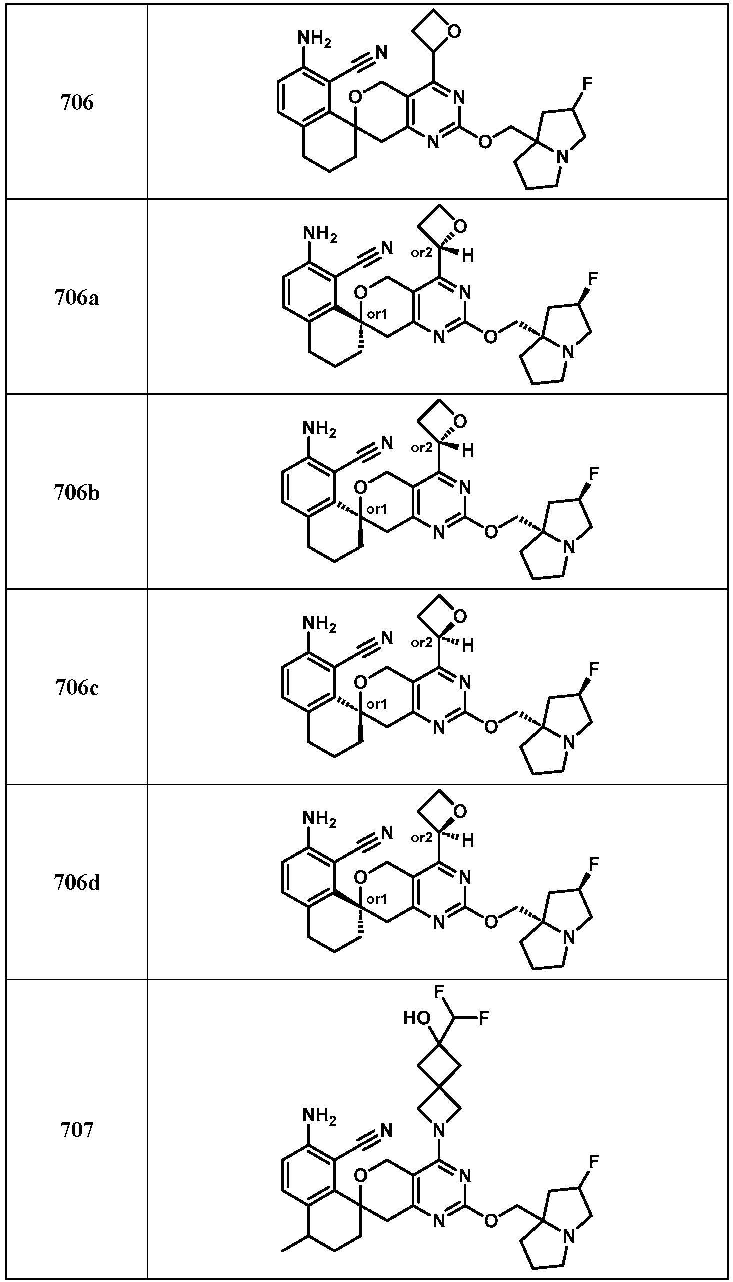

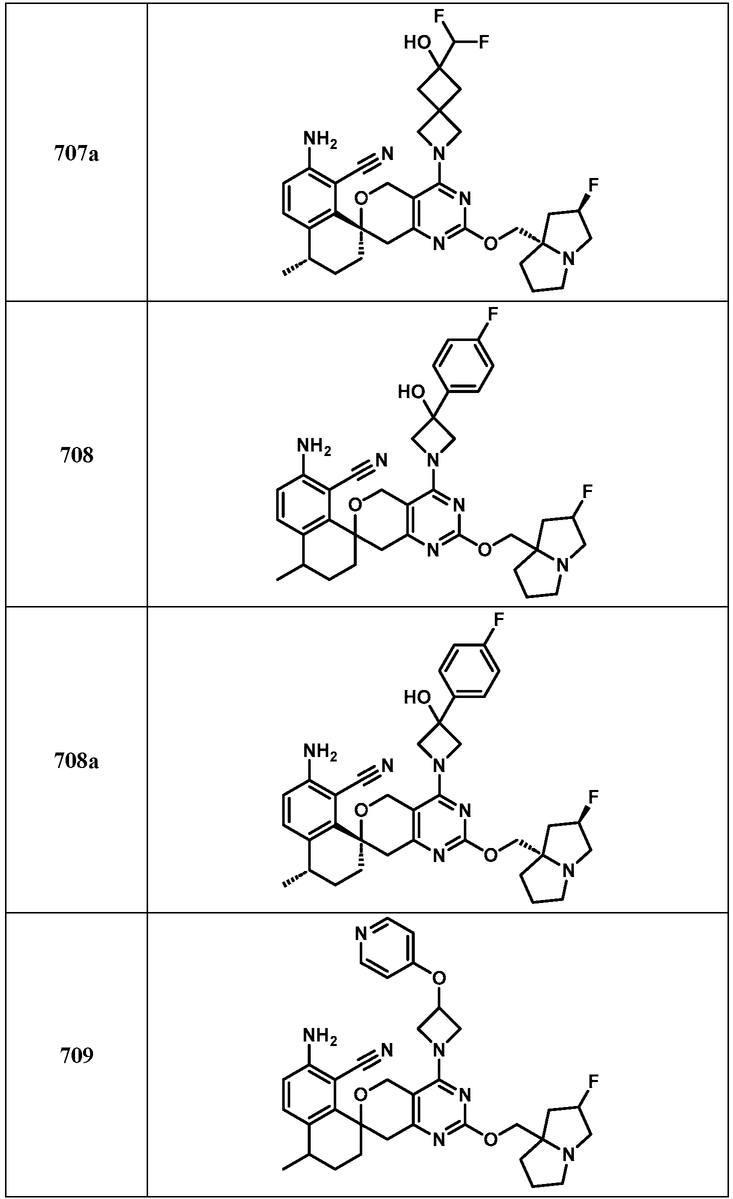

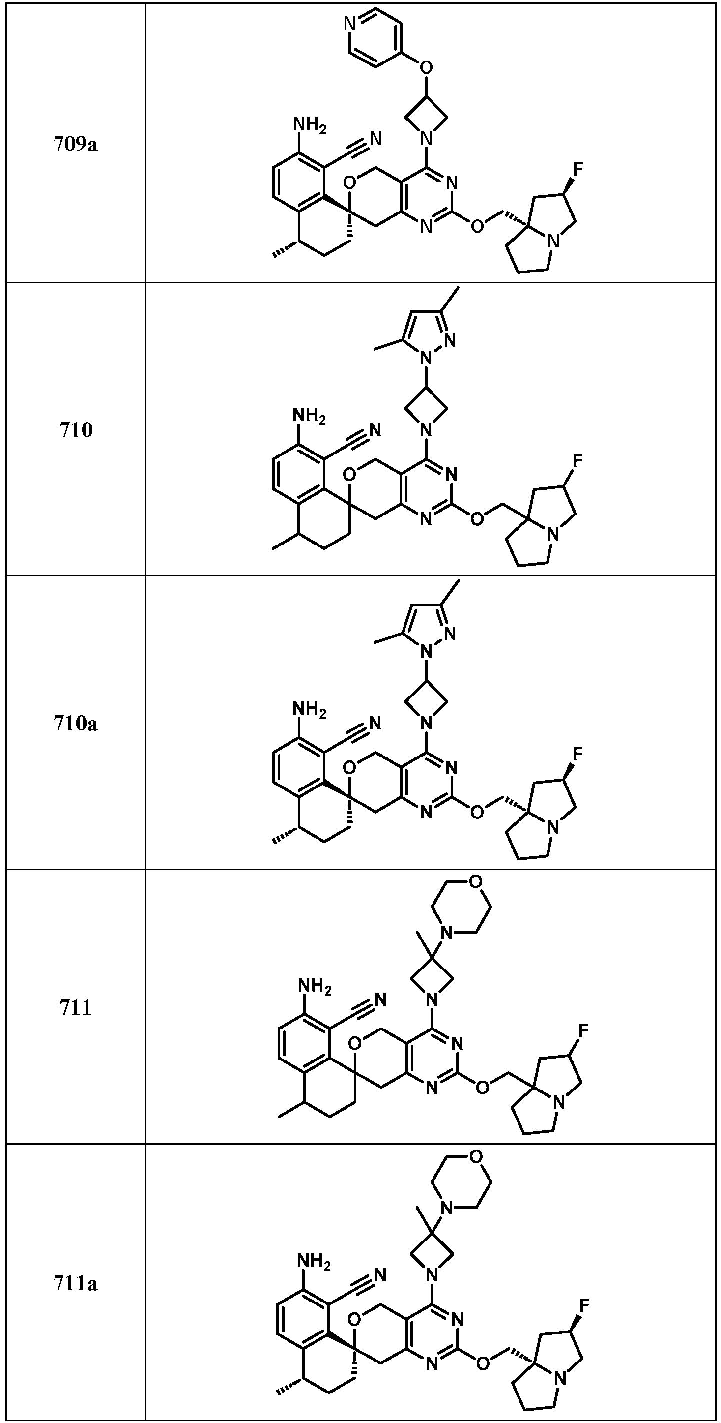

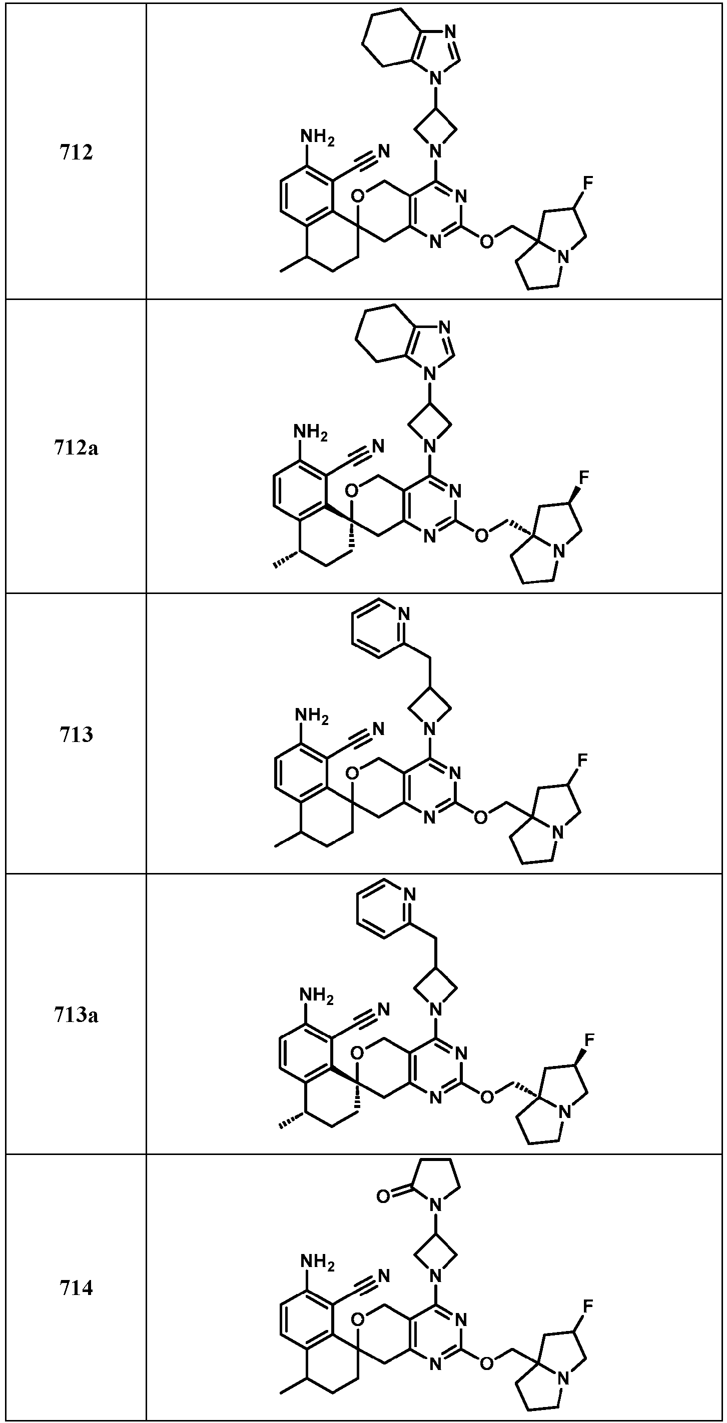

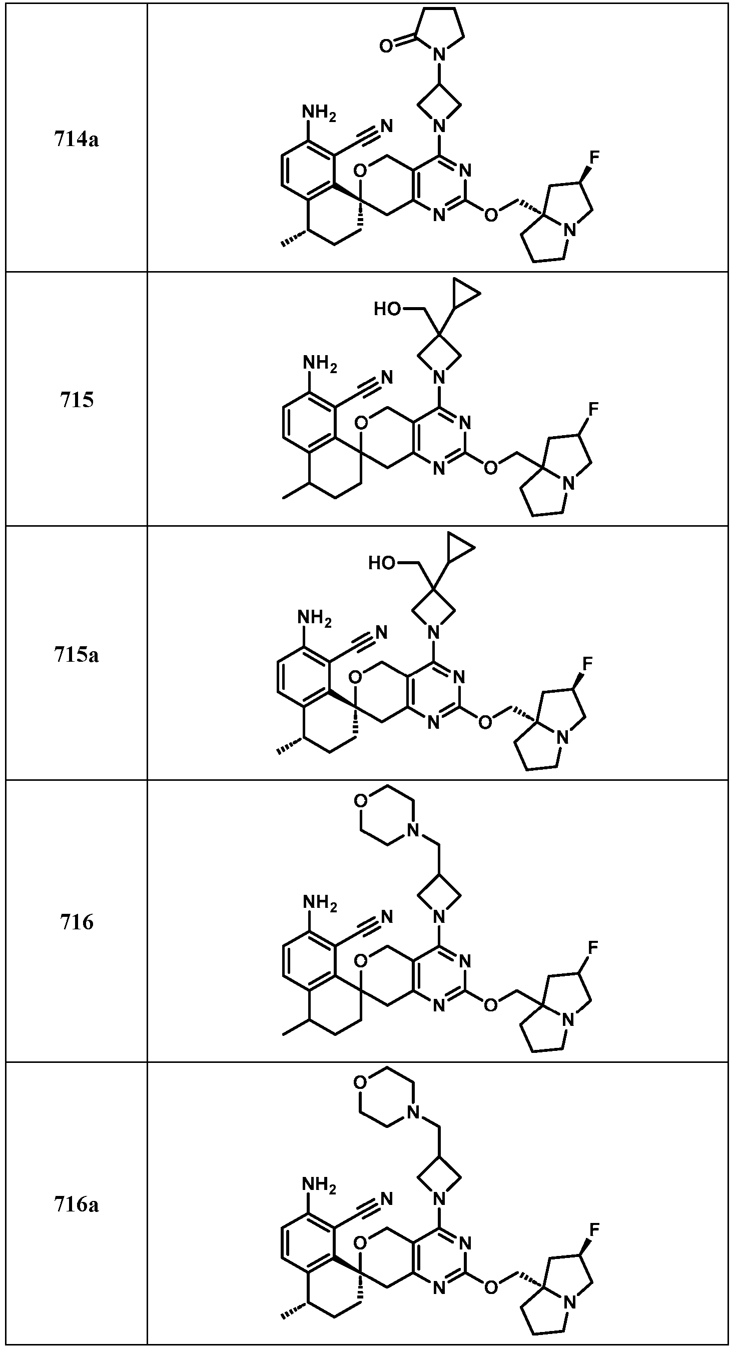

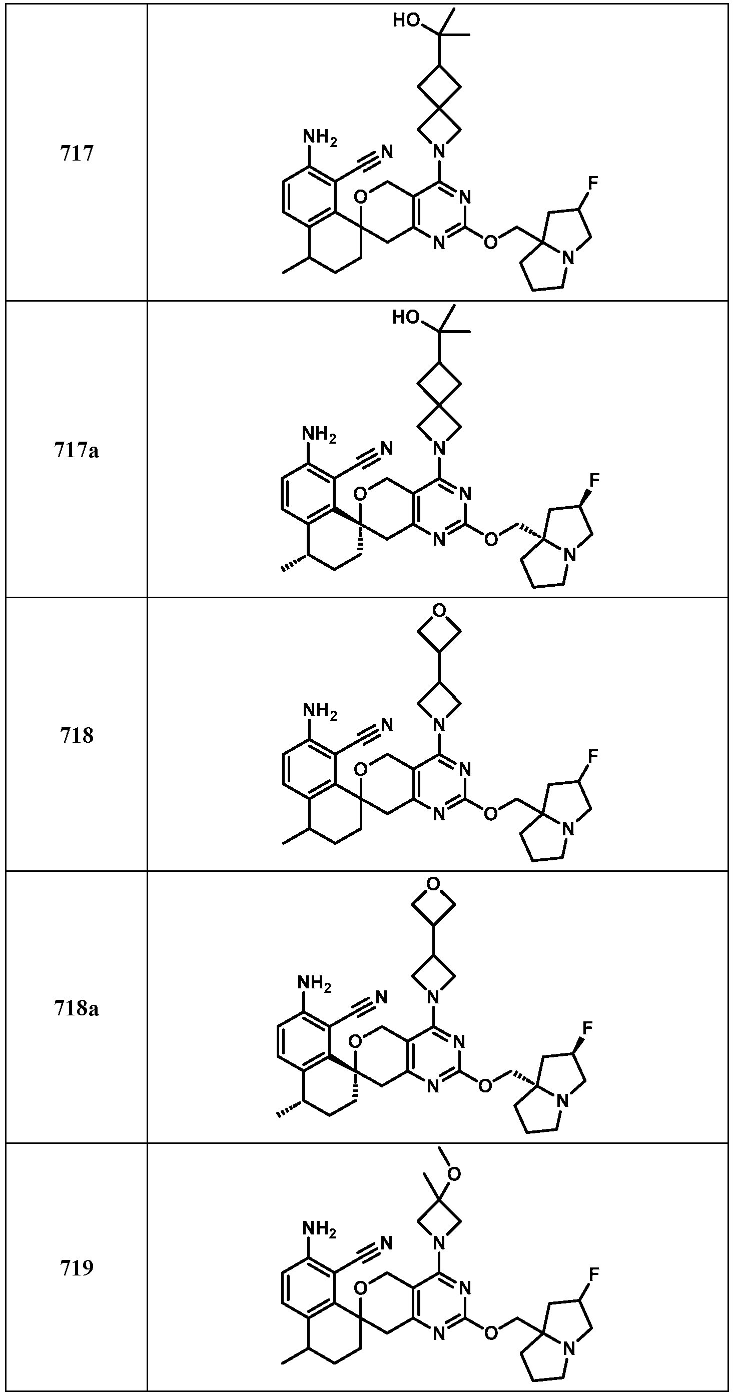

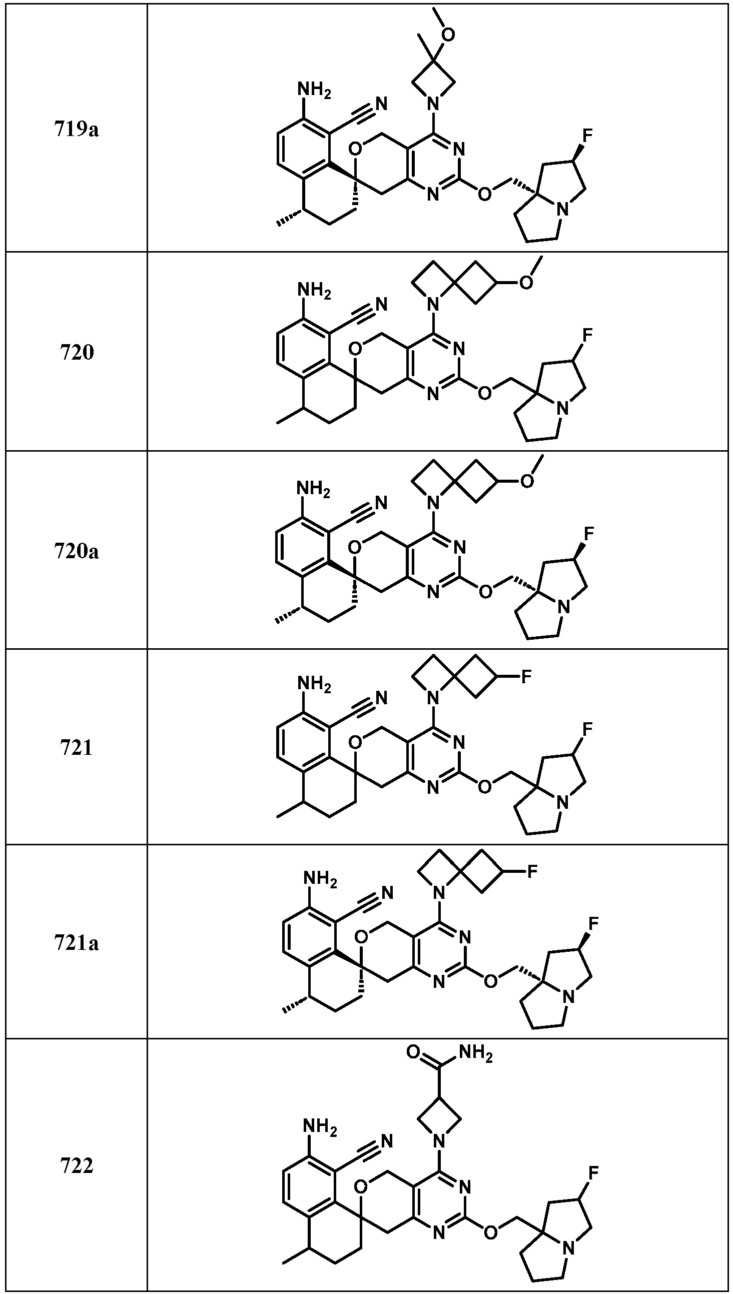

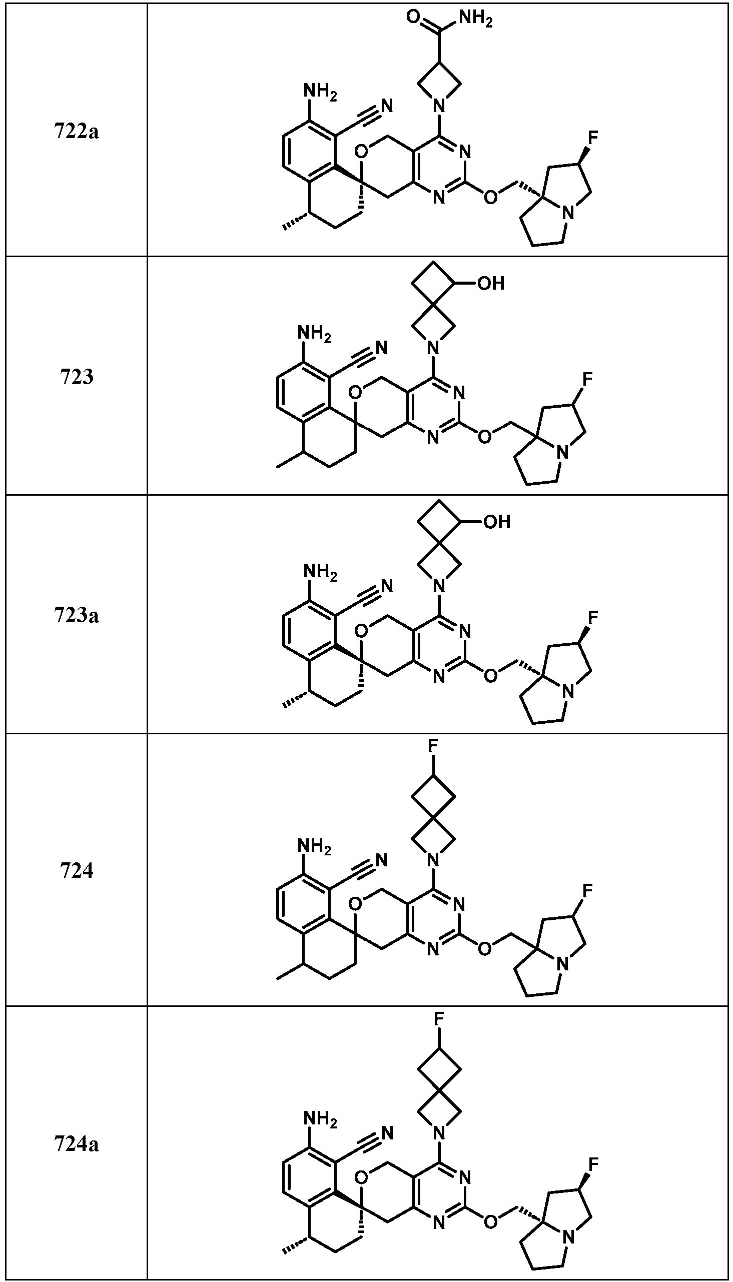

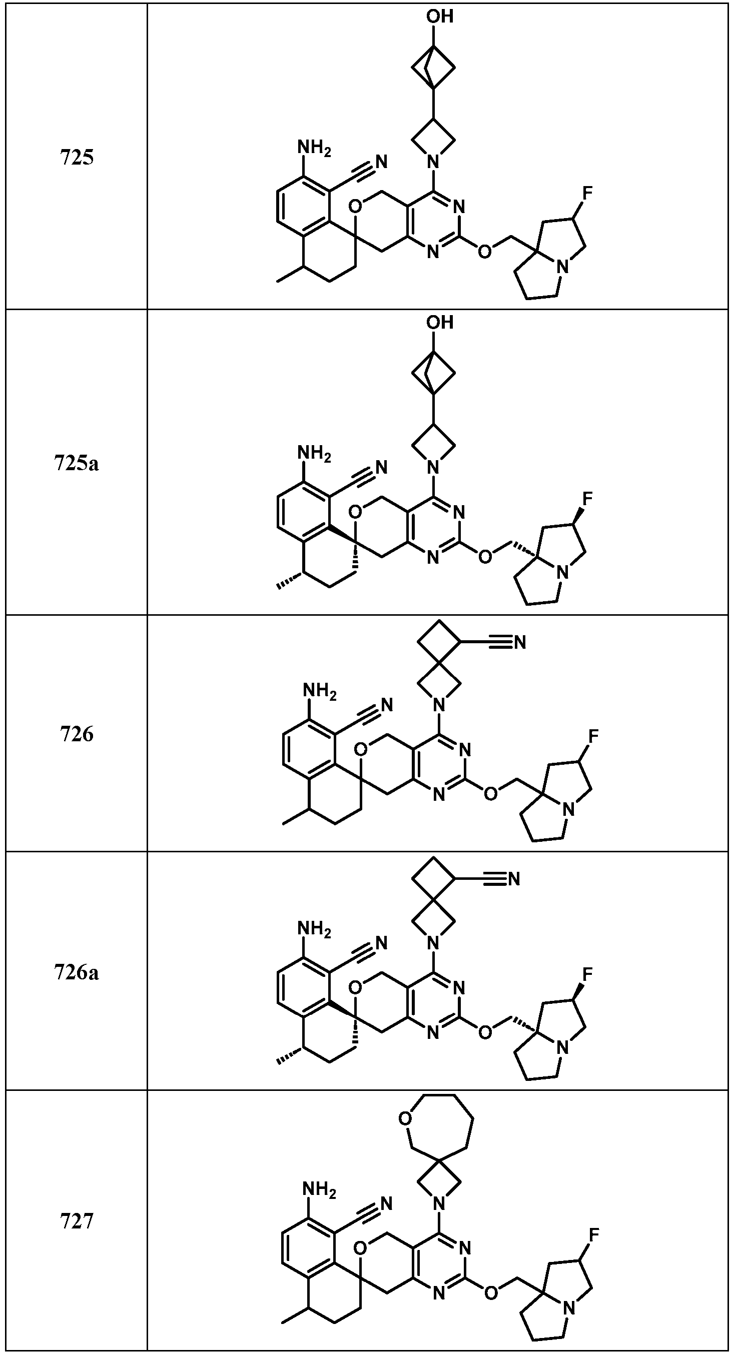

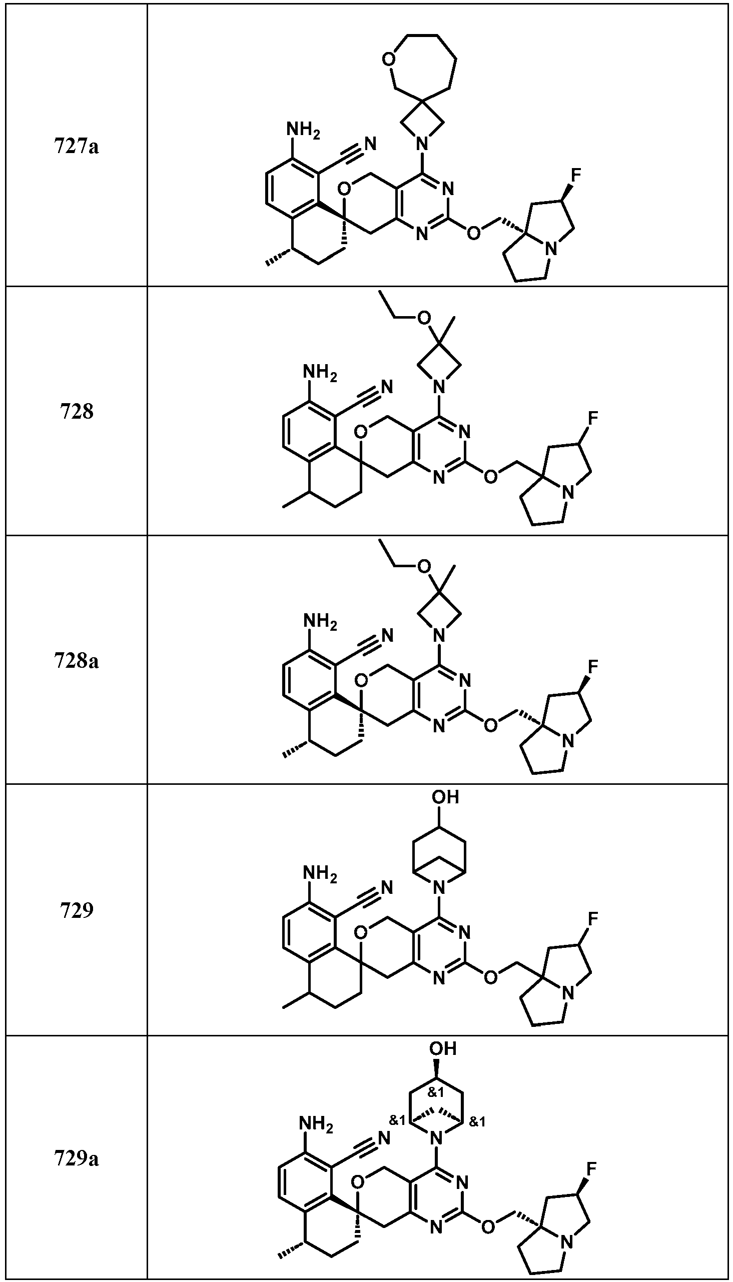

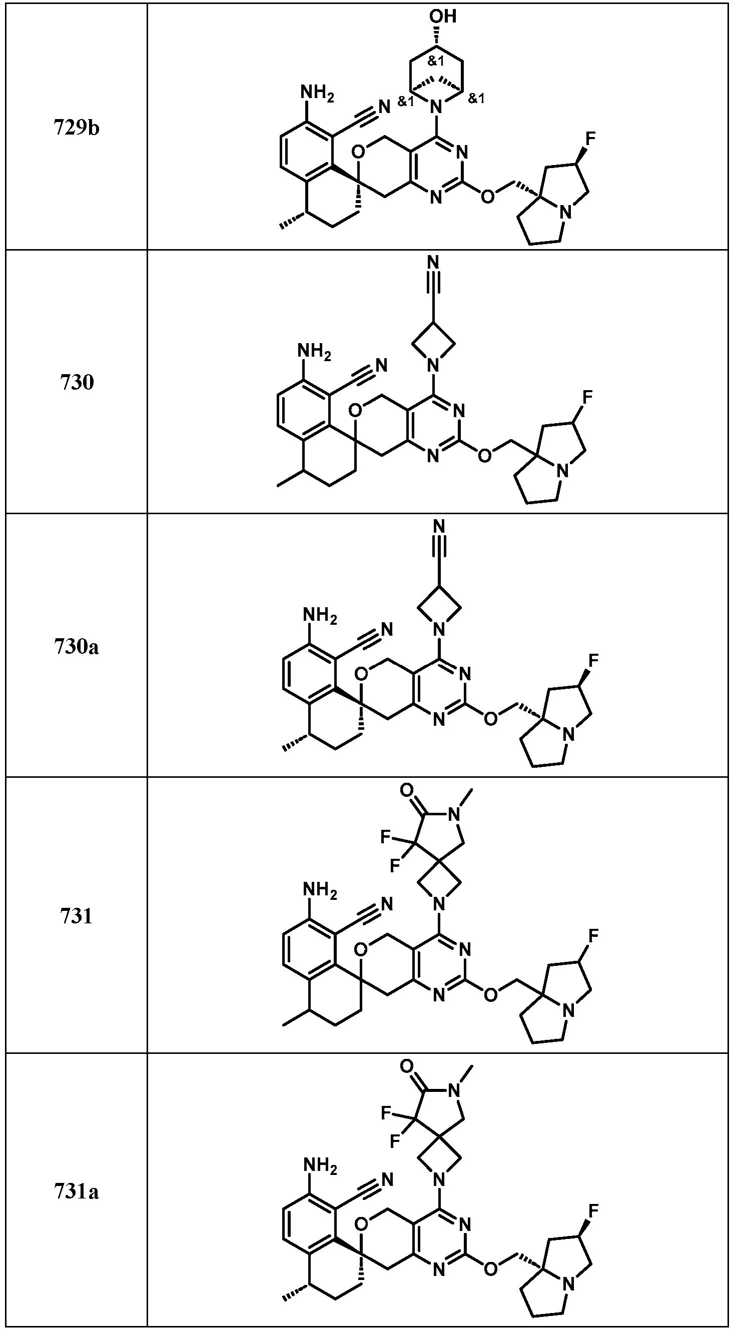

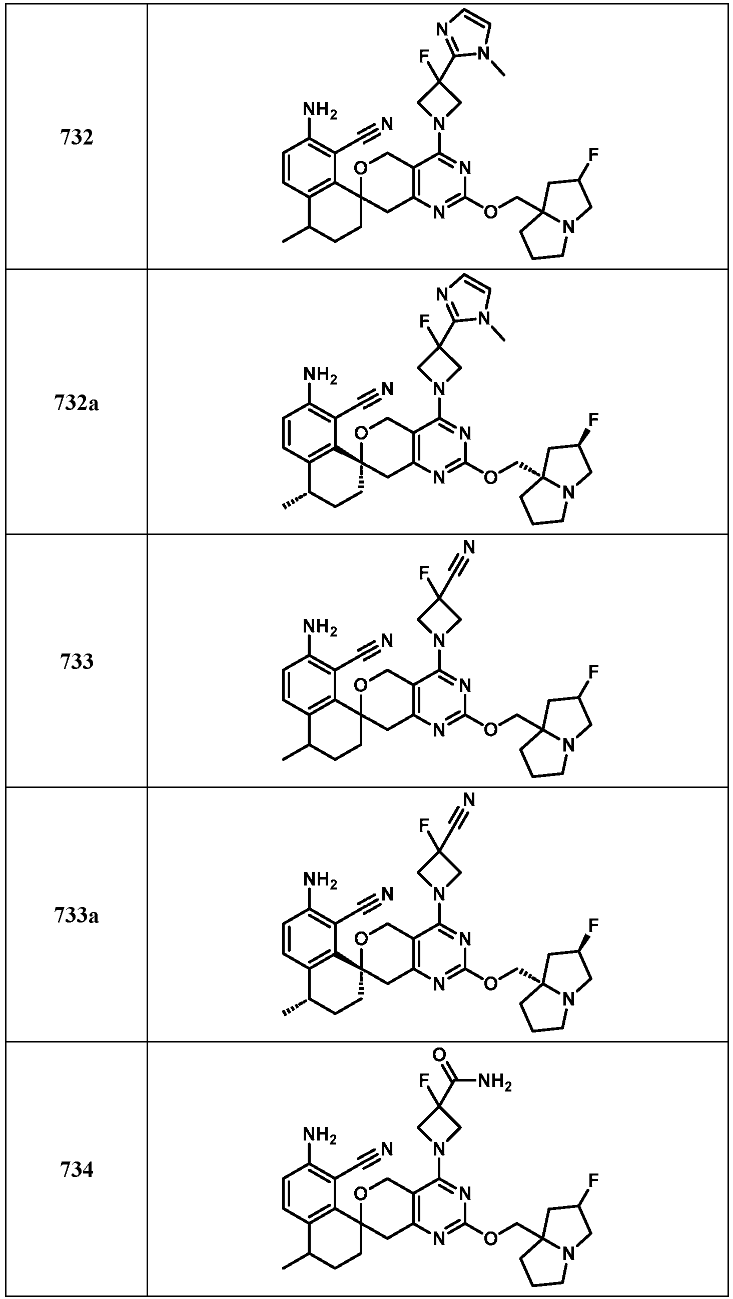

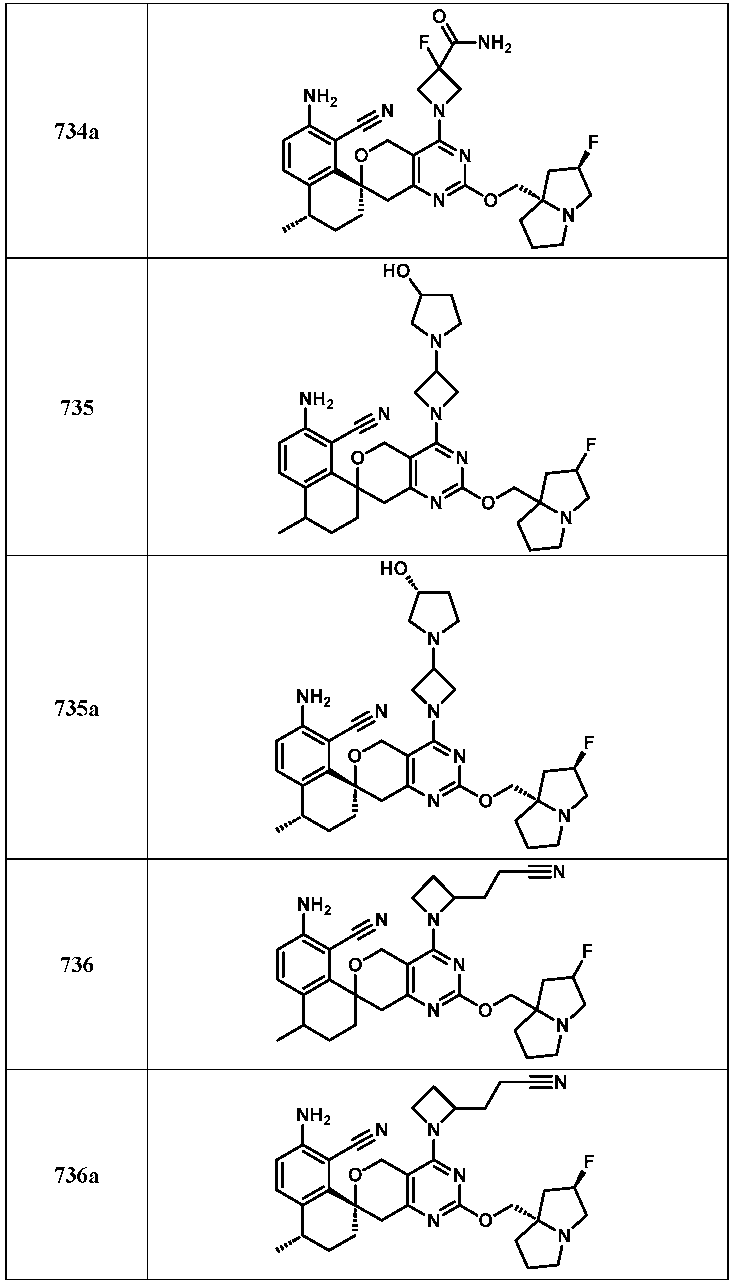

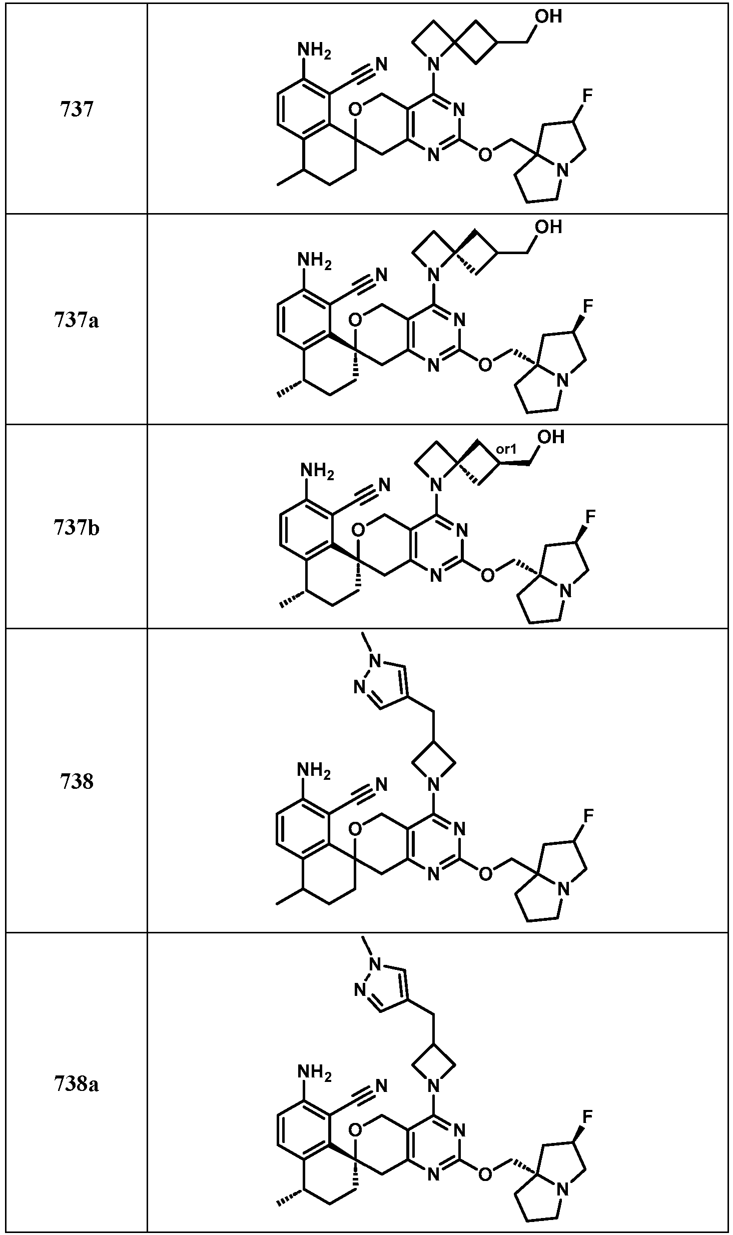

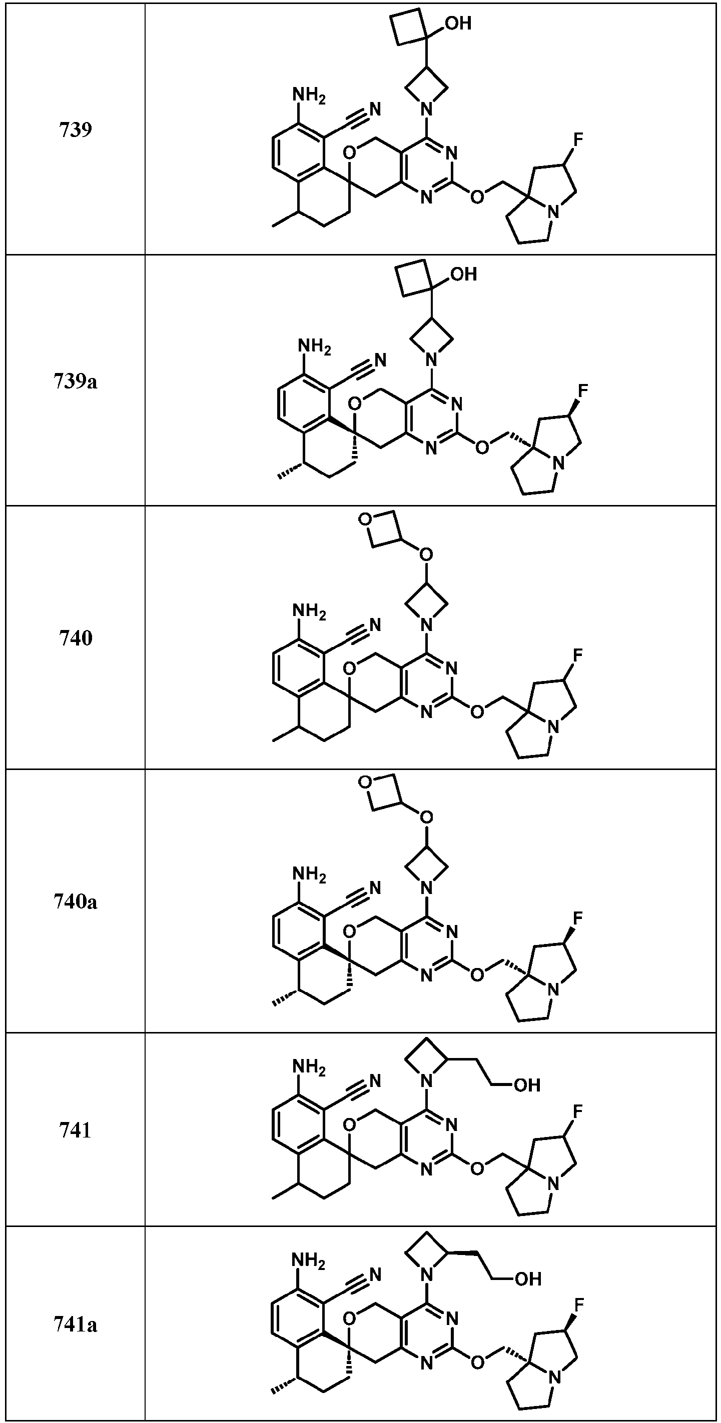

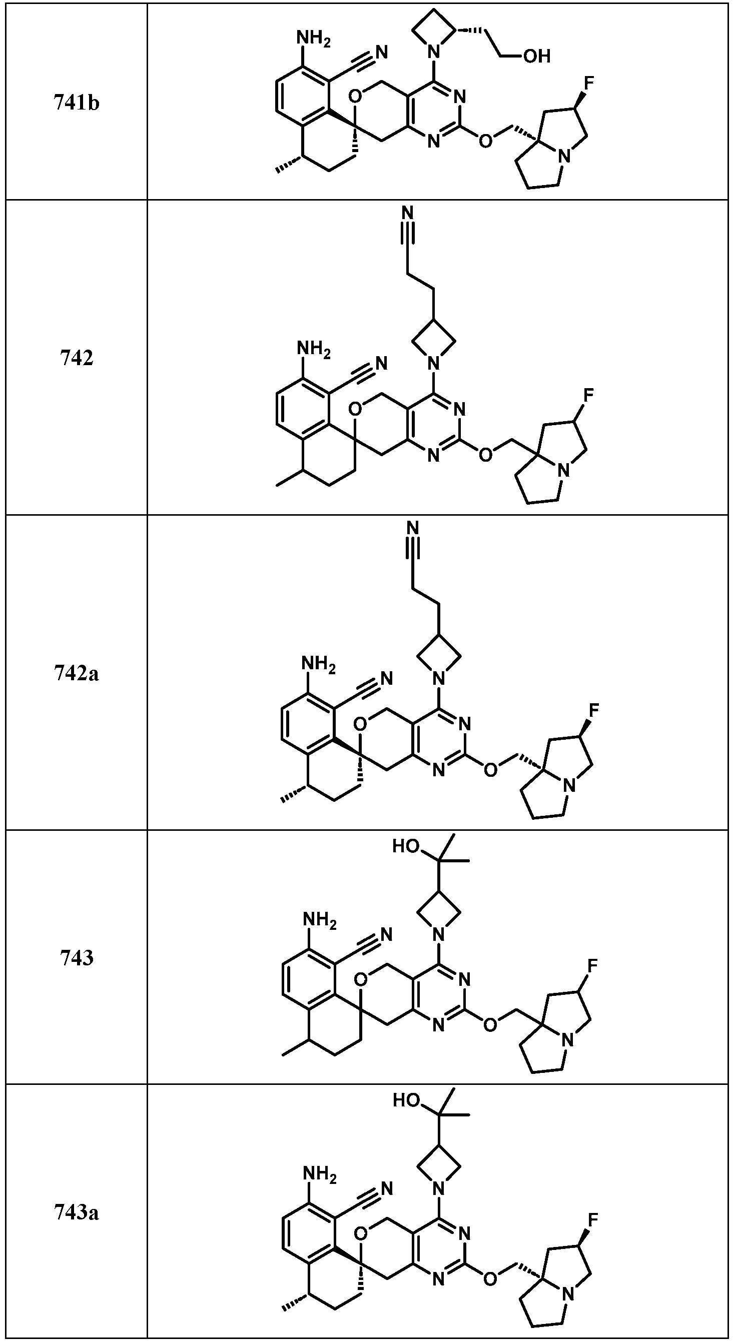

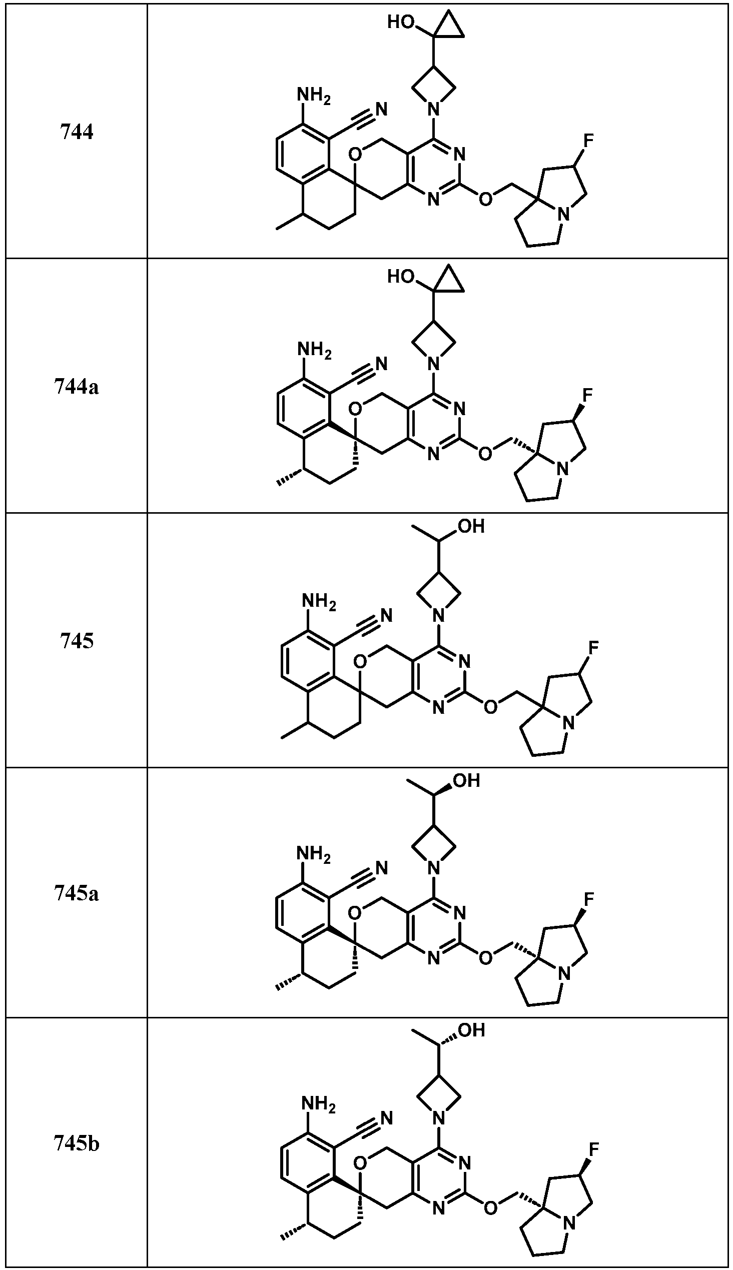

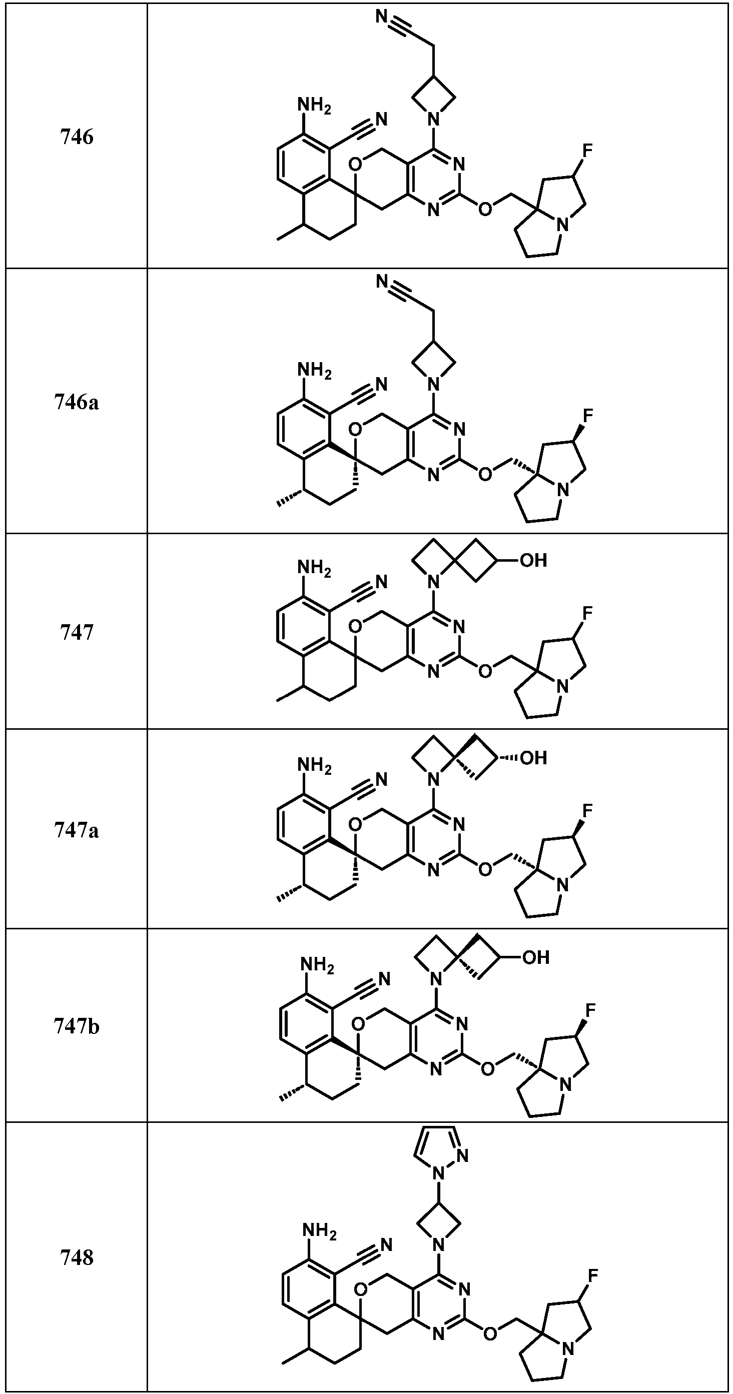

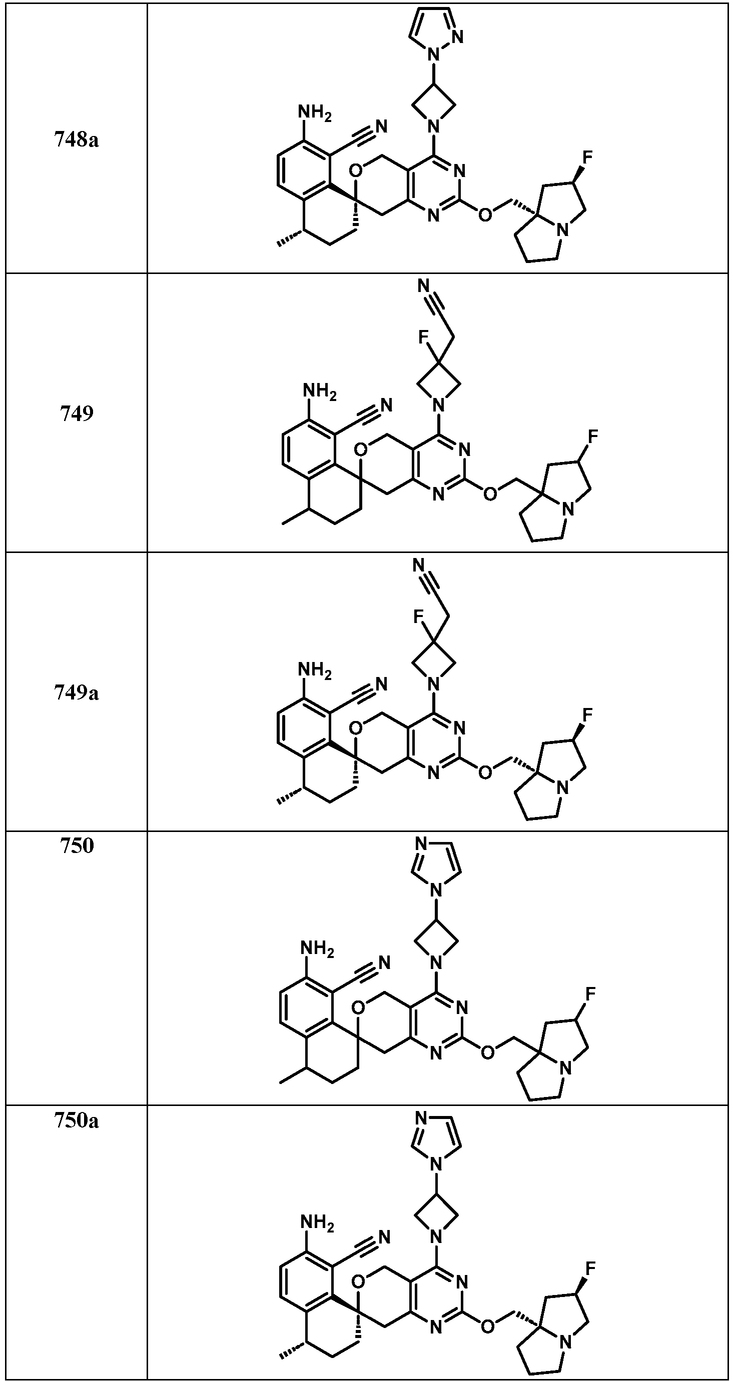

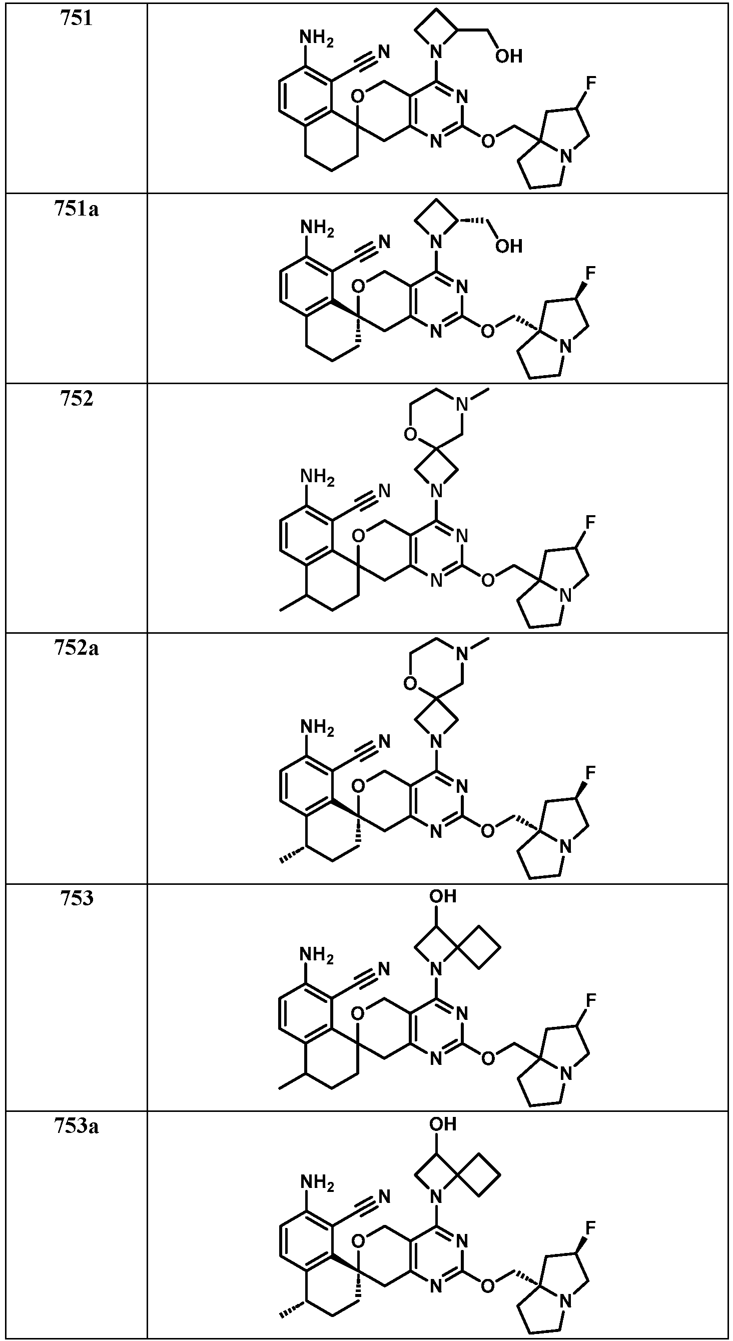

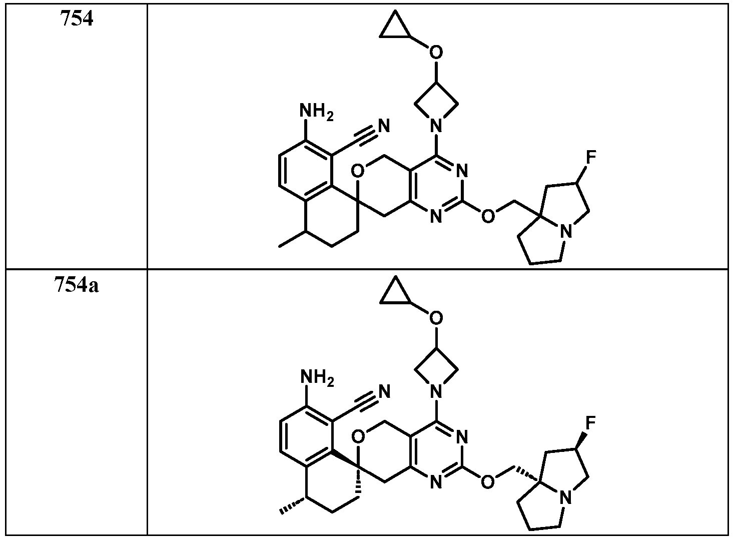

### Note:

In certain compounds of **Table C3 or Table C1,** one or more stereogenic centers are denoted with the "V3000 enhanced stereochemical notation" (*see*: support.collaborativedrug.com/hc/en-us/articles/360020872171-Advanced-Stereochemistry-Registration-Atropisomers-Mixtures-Unknowns-and-Non-Tetrahedral-Chirality, accessed on December 23, 2022 and Accelrys Chemical Representation Guide, Accelrys Software Inc., 2014, each of which is incorporated by reference herein in its entirety). Using this stereochemical notation, certain stereogenic centers are denoted with "*abs*", *"&x",* or *"orx",* wherein x is an integer (e.g., 1 or 2). For avoidance of doubt, the stereochemical notations in **Table C3 or Table C1** have the following meaning:
When a structure does not contain any wedged or hashed bonds (i.e., each stereogenic center is undefined), then each stereogenic center can independently adopt a (*R*) or (*S*) stereochemical configuration. For avoidance of doubt, such structures also encompass mixtures
of stereoisomers. For example, 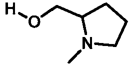 represents 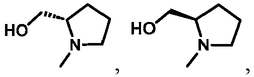 or a mixture of 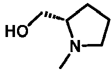 and

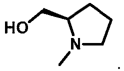

When a structure contains a stereogenic center or a plurality of stereogenic centers that is depicted with wedges and hashes (i.e., one or more stereogenic center is defined), the following notations are used:
**(1)** When a defined stereogenic center is denoted with "*abs*" or when the defined stereogenic center is not denoted with an enhanced stereochemical notation (e.g., "*abs*", *"&x",* or *"orx*"), the defined stereogenic center has the absolute configuration as depicted by the structural formula. For example, both of the structures 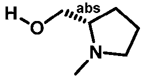 and 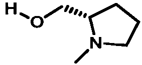 refer to (*S*)-(1-methylpyrrolidin-2-yl)methanol.
**(2)** When a defined stereogenic center is denoted with *"orx"* in a structural formula, the defined stereogenic center has been resolved but the configuration at the defined stereogenic center has not been determined. For example, the structure 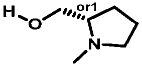 refers to one stereoisomer selected from the group consisting of (*S*)-(1-methylpyrrolidin-2-yl)methanol and (*R*)-(1-methylpyrrolidin-2-yl)methanol.
**(3)** When a stereogenic center is undefined (i.e., no wedged or hashed bonds attached to the undefined stereogenic center) in a structural formula having at least one defined stereogenic center (i.e., having a wedged and/or hashed bond attached to the at least one defined stereogenic center), a mixture of stereoisomers differing at the undefined stereogenic center is represented. For example, the structure 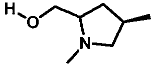 represents a mixture of 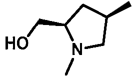 and 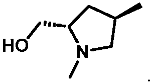 As another example, the structure 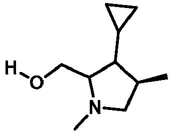 represents a mixture of 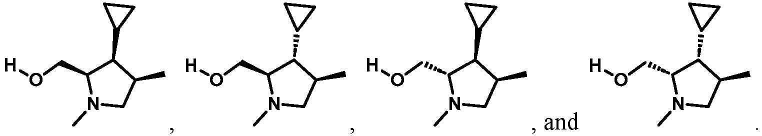
**(4)** When two or more defined stereogenic centers are denoted with *"orx"* in a structural formula, each of these defined stereogenic centers has been resolved but the configurations at the defined stereogenic centers have not been determined. Specifically:
   **a.** For any pair of defined stereogenic centers denoted with *"orx"* in a structural formula, when the numerical parts in the notation are different (e.g., two defined stereogenic centers denoted with *"or1"* and *"or2"* respectively), each defined stereogenic center should be independently interpreted according to "**(2)**" *supra.* For example, the structure 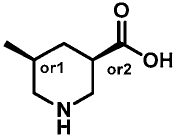 refers to one stereoisomer selected from the group consisting of: 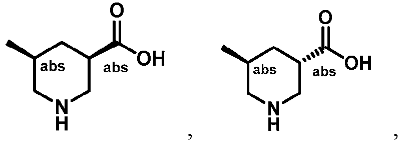 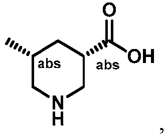 and 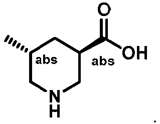
   **b.** For any pair of defined stereogenic centers denoted with *"orx"* in a structural formula, when the numerical part in the notation is identical (e.g., two defined stereogenic centers are each denoted with *"or1"*), the structural formula refers to one stereoisomer having the relative stereochemistry at these stereogenic centers as depicted in the structural formula, but the absolute configurations of these stereogenic centers have not been determined. For example, the structure 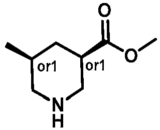 refers to one of the two *"syn"* stereoisomers: 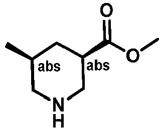 or 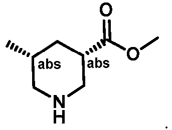 As another example, the structure 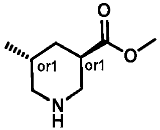 refers to one of the *"anti"* stereoisomers: 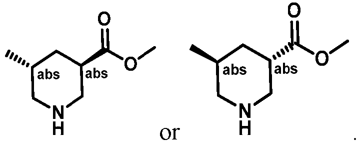
**(5)** When two or more defined stereogenic centers are denoted with "&x" in a structural formula, the structural formula refers to a mixture of stereoisomers that differ in the configuration at the defined stereogenic centers. Specifically:
   **a.** For any pair of defined stereogenic centers denoted with "&x" in a structural formula, when the numerical parts in the notation are different (e.g., two defined stereogenic centers denoted with "*&1*" and *"&2"* respectively), the structural formula refers to a mixture of stereoisomers at these two defined stereogenic centers, wherein the configuration at each of the defined stereogenic centers can vary independently of one another. For example, the structure 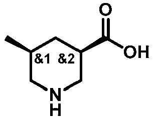 refers to a mixture of four stereoisomers: 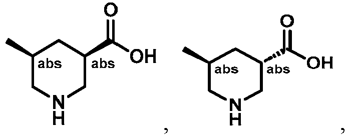 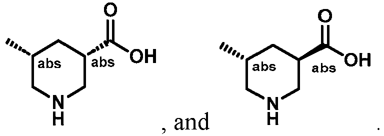
   **b.** For any pair of defined stereogenic centers denoted with "*&x*" in a structural formula, when the numerical part in the notation is identical (e.g., two defined stereogenic centers are each denoted with *"&1*"), the structural formula refers to a mixture of stereoisomers at these two defined stereogenic centers, wherein the relative configurations are as depicted in the structural formula. For example, the structure 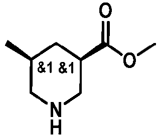 refers to a mixture of *"syn"* stereoisomers: 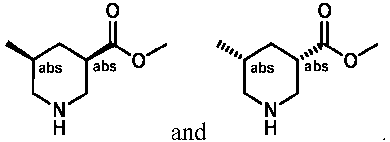 As another example, the structure 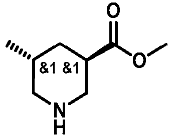 refers to a mixture of *"anti"* stereoisomers:

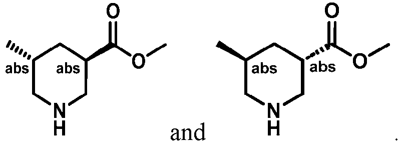

In some embodiments, the compound of Formula **(II)** is selected from the group consisting of Compound Nos. **181b, 501, 501a, 501b, 501c, 502, 502a, 502b, 503, 503a, 503b, 503c, 504, 504a, 504b, 504c, 505, 505a, 506, 506a, 507, 507a, 507b, 507c, 508, 508a, 509, 509a, 509b, 510, 510a, 510b, 510c, 510d, 510e, 510f, 511, 511a, 512, 512a, 512b, 513, 513a, 514, 514a, 514b, 516, 516a, 516b, 516c, 517, 517a, 518, 518a, 519, 519a, 520, 520a, 522, 522a, 523, 523a, 524, 524a, 525, 525a, 526, 526a, 526b, 526c, 527, 527a, 527b, 528, 528a, 529, 529a, 530, 530a, 531, 531a, 532, 532a, 534, 534a, 535, 535a, 536, 536a, 536b, 536c, 537, 537a, 538, 538a, 539, 539a, 540, 540a, 541, 541a, 542, 542a, 543, 543a, 544, 544a, 545, 545a, 546, 546a, 547, 547a, 548, 548a, 549, 549a, 550, 550a*,* 551, 551a, 552, 552a, 553, 553a, 554, 554a, 554b, 554c, 555, 555a, 556, 556a, 556b, 558, 558a, 559, 559a, 560, 560a, 561, 561a, 562, 562a, 563, 563a, 564, 564a, 565, 565a, 566, 566a, 566b, 567, 567a, 567b, 568, 568a, 568b, 569, 569a, 570, 570a, 571, 571a, 572, 572a, 573, 573a, 575, 575a, 575b, 576, 576a, 576b, 576c, 576d, 577, 577a, 578, 578a, 578b, 579, 579a, 580, 580a, 581, 581a, 582, 582a, 583, 583a, 584, 584a, 585, 585a, 585b, 586, 586a, 587, 587a, 588, 588a, 589, 589a, 590, 590a, 591, 591a, 592, 592a, 593, 593a, 594, 594a, 595, 595a, 595b, 596, 596a, 597, 597a, 597b, 598, 598a, 598b, 598c, 599, 599a, 599b, 600, 600a, 600b, 601, 601a, 602, 602a, 603, 603a, 603b, 606, 606a, 606b, 607, 607a, 608, 608a, 608b, 609, 609a, 609b, 610, 610a, 610b, 611, 611a, 611b, 612, 612a, 612b, 613, 613a, 613b, 613c, 614, 614a, 614b, 614c, 615, 615a, 616, 616a, 617, 617a, 618, 618a, 619, 619a, 620, 620a, 621, 621a, 621b, 622, 622a, 623, 623a, 624, 624a, 624b, 625, 625a, 626, 626a, 627, 627a, 628, 628a, 628b, 628c, 629, 629a, 630, 630a, 631, 631a, 632, 632a, 633, 633a, 634, 634a, 635, 635a, 636, 636a, 637, 637a, 637b, 638, 638a, 639, 639a, 640, 640a, 641, 641a, 641b, 641c, 642, 642a, 643, 643a, 643b, 644, 644a, 645, 645a, 645b, 646, 646a, 647, 647a, 647b, 648, 648a, 648b, 649, 649a, 649b, 650, 650a, 650b, 650c, 650d, 651, 651a, 651b, 652, 652a, 652b, 652c, 653, 653a, 653b, 654, 654a, 654b, 654c, 654d, 655, 655a, 656, 656a, 657, 657a, 658, 658a, 659, 659a, 660, 660a, 660b, 661, 661a, 661b, 662, 662a, 662b, 662c, 662d, 663, 663a, 663b, 664, 664a, 664b, 665, 665a, 665b, 666, 666a, 667, 667a, 667b, 668, 668a, 668b, 668c, 668d, 669, 669a, 670, 670a, 671, 671a, 671b, 672, 672a, 673, 673a, 674, 674a, 675, 675a, 676, 676a, 677, 677a, 678, 678a, 679, 679a, 680, 680a, 680b, 680c, 681, 681a, 682, 682a, 683, 683a, 684, 684a, 685, 685a, 686, 686a, 687, 687a, 688, 688a, 689, 689a, 689b, 690, 690a, 691, 691a, 692, 692a, 693, 693a, 694, 694a, 695, 695a, 696, 696a, 697, 697a, 698, 698a, 699, 699a, 700, 700a, 701, 701a, 702, 702a, 702b, 703, 703a, 704, 704a, 705, 705a, 706, 706a, 706b, 706c, 706d, 707, 707a, 708, 708a, 709, 709a, 710, 710a, 711, 711a, 712, 712a, 713, 713a, 714, 714a, 715, 715a, 716, 716a, 717, 717a, 718, 718a, 719, 719a, 720, 720a, 721, 721a, 722, 722a, 723, 723a, 724, 724a, 725, 725a, 726, 726a, 727, 727a, 728, 728a, 729, 729b, 729a, 730, 730a, 731, 731a, 732, 732a, 733, 733a, 734, 734a, 735, 735a, 736, 736a, 737, 737a, 737b, 738, 738a, 739, 739a, 740, 740a, 741, 741a, 741b, 742, 742a, 743, 743a, 744, 744a, 745, 745a, 745b, 746, 746a, 747, 747a, 747b, 748, 748a, 749, 749a, 750, 750a, 751, 751a, 752, 752a, 753, 753a, 754,** and **754a as** depicted in **Table C3,** or a pharmaceutically acceptable salt thereof. For example, the compound of Formula (**II**) can be selected from the group consisting of Compound Nos. **181b, 501, 501a, 501b, 501c, 502, 502a, 502b, 503, 503a, 504, 504a, 504b, 504c, 505, 505a, 506, 506a, 507, 507a, 507b, 508, 508a, 509,** and **509a** as depicted in **Table C3,** or a pharmaceutically acceptable salt thereof. For example, the compound of Formula (**II**) can be selected from the group consisting of Compound Nos. **181b, 501, 501a, 501b, 501c, 502, 502a, 502b, 503, 503a, 503b, 503c, 504, 504a, 504b, 504c, 505, 505a, 506, 506a, 507, 507a, 507b, 507c, 508, 508a, 509, 509a, 510, 510a, 510b, 511, 511a, 512, 512a,** and **512b,** as depicted in **Table C3,** or a pharmaceutically acceptable salt thereof.

In some embodiments, the compound of Formula (**III**) is selected from the group consisting of Compound Nos. **502, 502a, 502b, 503, 503a, 503b, 503c, 504, 504a, 504b, 504c, 505, 505a, 507, 507a, 507b, 507c, 509, 509a, 509b, 511, 511a, 512, 512a, 512b, 513, 513a, 514, 514a, 514b, 516, 516a, 516b, 516c, 517, 517a, 518, 518a, 519, 519a, 520, 520a, 522, 522a, 523, 523a, 524, 524a, 525, 525a, 526, 526a, 526b, 526c, 527, 527a, 527b, 528, 528a, 529, 529a, 530, 530a, 531, 531a, 532, 532a, 534, 534a, 535, 535a, 536, 536a, 536b, 536c, 537, 537a, 538, 538a, 539, 539a, 540, 540a, 541, 541a, 542, 542a, 543, 543a, 544, 544a, 545, 545a, 546, 546a, 547, 547a, 548, 548a, 549, 549a, 550, 550a, 551, 551a, 552, 552a, 553, 553a, 554, 554a, 554b, 554c, 555, 555a, 556, 556a, 556b, 558, 558a, 559, 559a, 560, 560a, 561, 561a, 562, 562a, 563, 563a, 564, 564a, 565, 565a, 566, 566a, 566b, 567, 567a, 567b, 568, 568a, 568b, 569, 569a, 570, 570a, 571, 571a, 572, 572a, 573, 573a, 574, 574a, 574b, 574c, 575, 575a, 575b, 576, 576a, 576b, 576c, 576d, 577, 577a, 578, 578a, 578b, 579, 579a, 580, 580a, 581, 581a, 582, 582a, 583, 583a, 584, 584a, 585, 585a, 585b, 586, 586a, 587, 587a, 588, 588a, 589, 589a, 590, 590a, 591, 591a, 592, 592a, 593 , 593a, 594, 594a, 595, 595a, 595b, 596, 596a, 597, 597a, 597b, 598, 598a, 598b, 598c, 599, 599a, 599b, 600, 600a, 600b, 601, 601a, 602, 602a , 603, 603a, 603b, 604, 604a, 604b, 604c, 605, 605a, 605b, 605c, 606, 606a, 606b, 607, 607a, 608, 608a, 608b, 609, 609a, 609b, 610, 610a, 610b, 611, 611a, 611b, 612, 612a, 612b, 613, 613a, 613b, 613c, 614, 614a, 614b, 614c, 615, 615a, 616, 616a, 617, 617a, 618, 618a, 619, 619a, 620, 620a, 621, 621a, 621b, 622, 622a, 623, 623a, 624, 624a, 624b, 625, 625a, 626, 626a, 627, 627a, 628, 628a, 628b, 628c, 629, 629a, 630, 630a, 631, 631a, 632, 632a, 633, 633a, 634, 634a, 635, 635a, 636, 636a, 637, 637a, 637b, 638, 638a, 639, 639a, 640, 640a, 641, 641a, 641b, 641c, 642, 642a, 643, 643a, 643b, 644, 644a, 645, 645a, 645b, 646, 646a, 647, 647a, 647b, 648, 648a, 648b, 649, 649a, 649b, 650, 650a, 650b, 650c, 650d, 651, 651a, 651b, 652, 652a, 652b, 652c, 653, 653a, 653b, 654, 654a, 654b, 654c, 654d, 655, 655a, 656, 656a, 657, 657a, 658, 658a, 659, 659a, 660, 660a, 660b, 661, 661a, 661b, 662, 662a, 662b, 662c, 662d, 663, 663a, 663b, 664, 664a, 664b, 665, 665a, 665b, 666, 666a, 667, 667a, 667b, 668, 668a, 668b, 668c, 668d, 669, 669a, 670, 670a, 671, 671a, 671b, 672, 672a, 673, 673a, 674, 674a, 675, 675a, 676, 676a, 677, 677a, 678, 678a, 679, 679a, 680, 680a, 680b, 680c, 681, 681a, 682, 682a, 683, 683a, 684, 684a, 685, 685a, 686, 686a, 687, 687a, 688, 688a, 689, 689a, 689b, 690, 690a, 691, 691a, 692, 692a, 693, 693a, 694, 694a, 695, 695a, 696, 696a, 697, 697a, 698, 698a, 699, 699a, 700, 700a, 701, 701a, 702, 702a, 702b, 703, 703a, 704, 704a, 705, 705a, 706, 706a, 706b, 706c, 706d, 707, 707a, 708, 708a, 709, 709a, 710, 710a, 711, 711a, 712, 712a, 713, 713a, 714, 714a, 715, 715a, 716, 716a, 717, 717a, 718, 718a, 719, 719a, 720, 720a, 721, 721a, 722, 722a, 723, 723a, 724, 724a, 725, 725a, 726, 726a, 727, 727a, 728, 728a, 729, 729a, 729b, 730, 730a, 731, 731a, 732, 732a, 733, 733a, 734, 734a, 735, 735a, 736, 736a, 737, 737a, 737b, 738, 738a, 739, 739a, 740, 740a, 741, 741a, 741b, 742, 742a, 743, 743a, 744, 744a, 745, 745a, 745b, 746, 746a, 747, 747a, 747b, 748, 748a, 749, 749a, 750, 750a, 751, 751a, 752, 752a, 753, 753a, 754,** and **754a** as depicted in **Table C3,** or a pharmaceutically acceptable salt thereof. For example, the compound of Formula (**III**) can be selected from the group consisting of Compound Nos. **502, 502a, 502b, 503, 503a, 504, 504a, 504b, 504c, 505, 505a, 507, 507a, 507b, 509,** and **509a** as depicted in **Table C3,** or a pharmaceutically acceptable salt thereof. For example, the compound of Formula (**III**) can be selected from the group consisting of Compound Nos. **502, 502a, 502b, 503, 503a, 503b, 503c, 504, 504a, 504b, 504c, 505, 505a, 507, 507a, 507b, 507c, 509, 509a, 511, 511a, 512, 512a,** and **512b,** as depicted in **Table C3,** or a pharmaceutically acceptable salt thereof.

In some embodiments, the compound of Formula **(IV)** is selected from the group consisting of Compound Nos. **502, 502a, 502b, 508, 508a, 509, 509a, 509b, 511, 511a, 512, 512a, 512b, 530, 530a, 531, 531a, 532, 532a, 546, 546a, 553, 553a, 554, 554a, 554b, 554c, 555, 555a, 556, 556a, 556b, 560, 560a, 561, 561a, 566, 566a, 566b, 567, 567a, 567b, 568, 568a, 568b, 569, 569a, 570, 570a, 571, 571a, 572, 572a, 573, 573a, 575, 575a, 575b, 576, 576a, 576b, 576c, 576d, 577, 577a, 578, 578a, 578b, 581, 581a, 582, 582a, 583, 583a, 584, 584a, 585, 585a, 585b, 589, 589a, 590, 590a, 592, 592a, 595, 595a, 595b, 596, 596a, 597, 597a, 597b, 598, 598a, 598b, 598c, 599, 599a, 599b, 600, 600a, 600b, 601, 601a, 604, 604a, 604b, 604c, 605, 605a, 605b, 605c, 606, 606a, 606b, 607, 607a, 608, 608a, 608b, 609, 609a, 609b, 610, 610a, 610b, 611, 611a, 611b, 612, 612a, 612b, 613, 613a, 613b, 613c, 614, 614a, 614b, 614c, 615, 615a, 616, 616a, 617, 617a, 618, 618a, 619, 619a, 620, 620a, 621, 621a, 621b, 622, 622a, 623, 623a, 624, 624a, 624b, 625, 625a, 626, 626a, 627, 627a, 628, 628a, 628b, 628c, 629, 629a, 630, 630a, 632, 632a, 633, 633a, 634, 634a, 635, 635a, 636, 636a, 637, 637a, 637b, 638, 638a, 639, 639a, 640, 640a, 641, 641a, 641b, 641c, 642, 642a, 643, 643a, 643b, 644, 644a, 645, 645a, 645b, 646, 646a, 647, 647a, 647b, 648, 648a, 648b, 649, 649a, 649b, 650, 650a, 650b, 650c, 650d, 651, 651a, 651b, 652, 652a, 652b, 652c, 653, 653a, 653b, 654, 654a, 654b, 654c, 654d, 655, 655a, 656, 656a, 657, 657a, 658, 658a, 659, 659a, 660, 660a, 660b, 661, 661a, 661b, 662, 662a, 662b, 662c, 662d, 665, 665a, 665b, 666, 666a, 667, 667a, 667b, 668, 668a, 668b, 668c, 668d, 669, 669a, 670, 670a, 671, 671a, 671b, 672, 672a, 673, 673a, 674, 674a, 675, 675a, 676, 676a, 677, 677a, 678, 678a, 679, 679a, 680, 680a, 680b, 680c, 681, 681a, 682, 682a, 683, 683a, 684, 684a, 685, 685a, 686, 686a, 687, 687a, 688, 688a, 689, 689a, 689b, 690, 690a, 691, 691a, 692, 692a, 693, 693a, 694, 694a, 695, 695a, 696, 696a, 697, 697a, 698, 698a, 699, 699a, 700, 700a, 701, 701a, 702, 702a, 702b, 703, 703a, 704, 704a, 705, 705a, 707, 707a, 708, 708a, 709, 709a, 710, 710a, 711, 711a, 712, 712a, 713, 713a, 714, 714a, 715, 715a, 716, 716a, 717, 717a, 718, 718a, 719, 719a, 720, 720a, 721, 721a, 722, 722a, 723, 723a, 724, 724a, 725, 725a, 726, 726a, 727, 727a, 728, 728a, 729, 729a, 729b, 730, 730a, 731, 731a, 732, 732a, 733, 733a, 734, 734a, 735, 735a, 736, 736a, 737, 737a, 737b, 738, 738a, 739, 739a, 740, 740a, 741, 741a, 741b, 742, 742a, 743, 743a, 744, 744a, 745, 745a, 745b, 746, 746a, 747, 747a, 747b, 748, 748a, 749, 749a, 750, 750a, 752, 752a, 753, 753a, 754,** and **754a,** as depicted in **Table C3,** or a pharmaceutically acceptable salt thereof. For example, the compound of Formula **(IV)** can be selected from the group consisting of Compound Nos. **502, 502a, 502b, 508, 508a, 509,** and **509a** as depicted in **Table C3,** or a pharmaceutically acceptable salt thereof. For example, the compound of Formula **(IV)** can be selected from the group consisting of Compound Nos. **502, 502a, 502b, 508, 508a, 509, 509a, 511, 511a, 512, 512a,** and **512b,** as depicted in **Table C3,** or a pharmaceutically acceptable salt thereof.

In some embodiments, the compound of Formula **(V)** is selected from the group consisting of Compound Nos. **503, 503a, 503b, 503c, 504, 504a, 504b, 504c, 521,** and **521a** as depicted in **Table C3,** or a pharmaceutically acceptable salt thereof. For example, the compound of Formula **(V)** can be selected from the group consisting of Compound Nos. **503, 503a, 504, 504a, 504b,** and **504c** as depicted in **Table C3,** or a pharmaceutically acceptable salt thereof. For example, the compound of Formula **(V)** can be selected from the group consisting of Compound Nos. **503, 503a, 503b, 503c, 504, 504a, 504b,** and **504c** as depicted in **Table C3,** or a pharmaceutically acceptable salt thereof.

In some embodiments, the compound of Formula **(VI)** is selected from the group consisting of Compound Nos. **502, 502a, 502b, 510, 510a, 510b, 510c, 510d, 510e, 510f, 511, 511a, 515, 515a, 522, 522a, 533, 533a, 566, 566a, 566b, 569, 569a, 572, 572a, 577, 577a, 645, 645a, 645b, 650, 650a, 650b, 650c, 650d, 726, 726a, 730, 730a, 733,** and **733a** as depicted in **Table C3,** or a pharmaceutically acceptable salt thereof. For example, the compound of Formula **(VI)** can be selected from the group consisting of Compound Nos. **502, 502a,** and **502b,** as depicted in **Table C3,** or a pharmaceutically acceptable salt thereof. For example, the compound of Formula **(VI)** can be selected from the group consisting of Compound Nos. **502, 502a, 502b, 510, 510a, 510b, 511,** and **511a,** as depicted in **Table C3,** or a pharmaceutically acceptable salt thereof.

In some embodiments, the compounds of Formula **(II),** (**III**), or **(IV)** are selected from the group consisting of:

| | |
|---|---|
| **507b** | 5-((*S*)-7-Amino-8-cyano-2'-(((2R*,*7a*S*)-2-fluorotetrahydro-1*H*-pyrrolizin-7a(5*H*)-yl)methoxy)-3,4,5',8'-tetrahydro-2*H*-spiro[naphthalene-1,7'-pyrano[4,3-*d*]pyrimidin]-4'-yl)-3-chloro-*N,N*-dimethyl-5,6,7,8-tetrahydro-4*H*-pyrazolo[1,5-*a*][1,4]diazepine-2-carboxamide |
| **509a** | 5-((1*S*,4*S*)-7-Amino-8-cyano-2'-(((2*R*,7a*S*)-2-fluorotetrahydro-1*H*-pyrrolizin-7a(5*H*)-yl)methoxy)-4-methyl-3,4,5',8'-tetrahydro-2*H*-spiro[naphthalene-1,7'-pyrano[4,3-*d*]pyrimidin]-4'-yl)-3-chloro-*N,N*-dimethyl-5,6,7,8-tetrahydro-4*H*-pyrazolo[1,5-*a*][1,4]diazepine-2-carboxamide |
| **517a** | 5-((*S*)-7-amino-8-cyano-2'-(((2*R*,7a*S*)-2-fluorotetrahydro-1*H*-pyrrolizin-7a(5*H*)-yl)methoxy)-3,4,5',8'-tetrahydro-2*H*-spiro[naphthalene-1,7'-pyrano[4,3-*d*]pyrimidin]-4'-yl)-3-fluoro-*N,N*-dimethyl-5,6,7,8-tetrahydro-4*H*-pyrazolo[1,5-*a*][1,4]diazepine-2-carboxamide |
| **520a** | 5-((*S*)-7-amino-8-cyano-2'-(((2*R*,7a*S*)-2-fluorotetrahydro-1*H*-pyrrolizin-7a(5*H*)-yl)methoxy)-3,4,5',8'-tetrahydro-2*H*-spiro[naphthalene-1,7'-pyrano[4,3-*d*]pyrimidin]-4'-yl)-*N,N*,3-trimethyl-5,6,7,8-tetrahvdro-4*H*-pyrazolo[1,5-*a*][1,4]diazepine-2-carboxamide |
| **530a** | 5-((1*S*,4*S*)-7-amino-8-cyano-2'-(((2*R*,7a*S*)-2-fluorotetrahydro-1*H*-pyrrolizin-7a(5*H*)-yl)methoxy)-4-methyl-3,4,5',8'-tetrahydro-2*H*-spiro[naphthalene-1,7'-pyrano[4,3-*d*]pyrimidin]-4'-yl)-*N,N*,3-trimethyl-5,6,7,8-tetrahydro-4*H*-pyrazolo[1,5-*a*][1,4]diazepine-2-carboxamide |
| **531a** | 5-((1*S*,4*S*)-7-amino-8-cyano-2'-(((2*R*,7a*S*)-2-fluorotetrahydro-1*H*-pyrrolizin-7a(5*H*)-yl)methoxy)-4-methyl-3,4,5',8'-tetrahydro-2*H*-spiro[naphthalene-1,7'-pyrano[4,3-*d*]pyrimidin]-4'-yl)-3-fluoro-*N,N*-dimethyl-5,6,7,8-tetrahydro-4*H*-pyrazolo[1,5-*a*][1,4]diazepine-2-carboxamide |
| **546a** | 5-((1*S*,4*S*)-7-amino-8-cyano-2'-(((*S*)-2,2-difluorotetrahydro-1*H*-pyrrolizin-7a(5*H*)-yl)methoxy)-4-methyl-3,4,5',8'-tetrahydro-2*H*-spiro[naphthalene-1,7'-pyrano[4,3-*d*]pyrimidin]-4'-yl)-3-chloro-*N,N*-dimethyl-5,6,7,8-tetrahydro-4*H*-pyrazolo[1,5-*a*][1,4]diazepine-2-carboxamide |
| 628b | 5-((1*S*,4*S*)-7-amino-8-cyano-2'-(((2*R*,7a*S*)-2-methoxytetrahydro-1*H*-pyrrolizin-7a(5*H*)-yl)methoxy)-4-methyl-3,4,5',8'-tetrahydro-2*H*-spiro[naphthalene-1,7'-pyrano[4,3-*d*]pyrimidin]-4'-yl)-3-chloro-*N,N*-dimethyl-5,6,7,8-tetrahydro-4*H*-pyrazolo[1,5-*a*][1,4]diazepine-2-carboxamide |
| **628c** | 5-((1*S*,4*S*)-7-amino-8-cyano-2'-(((2*S*,7a*R*)-2-methoxytetrahydro-1*H*-pyrrolizin-7a(5*H*)-yl)methoxy)-4-methyl-3,4,5',8'-tetrahydro-2*H*-spiro[naphthalene-1,7'-pyrano[4,3-*d*]pyrimidin]-4'-yl)-3-chloro-*N,N*-dimethyl-5,6,7,8-tetrahydro-4*H*-pyrazolo[1,5-*a*][1,4]diazepine-2-carboxamide |
| **576a** | (1*S*,4*S*)-7-Amino-2'-(((2*R*,7a*S*)-2-fluorotetrahydro-1*H*-pyrrolizin-7a(5*H*)-yl)methoxy)-4'-((*R*)-2-(hydroxymethyl)azetidin-1-yl)-4-methyl-3,4,5',8'-tetrahydro-2H-spiro[naphthalene-1,7'-pyrano[4,3-*d*]pyrimidine]-8-carbonitrile |
| **576b** | (1*S*,4*S*)-7-Amino-2'-(((2*R*,7a*S*)-2-fluorotetrahydro-1*H*-pyrrolizin-7a(5*H*)-yl)methoxy)-4'-((*S*)-2-(hydroxymethyl)azetidin-1-yl)-4-methyl-3,4,5',8'-tetrahydro-2*H-*spiro[naphthalene-1,7'-pyrano[4,3-*d*]pyrimidine]-8-carbonitrile |
| **592a** | (1*S*,4*S*)-7-Amino-4'-(2,2-bis(hydroxymethyl)azetidin-1-yl)-2'-(((2*R*,7a*S*)-2-fluorotetrahydro-1*H*-pyrrolizin-7a(5*H*)-yl)methoxy)-4-methyl-3,4,5',8'-tetrahydro-2*H-*spiro[naphthalene-1,7'-pyrano[4,3-*d*]pyrimidine]-8-carbonitrile |
| **597a** | (1*S*,4*S*)-7-Amino-2'-(((2*R*,7a*S*)-2-fluorotetrahydro-1*H*-pyrrolizin-7a(5*H*)-yl)methoxy)-4'-((*R*)-2-(hydroxymethyl)-2-methylazetidin-1-yl)-4-methyl-3,4,5',8'-tetrahydro-2*H-*spiro[naphthalene-1,7'-pyrano[4,3-*d*]pyrimidine]-8-carbonitrile |
| **597b** | (1*S*,4*S*)-7-Amino-2'-(((2*R*,7a*S*)-2-fluorotetrahydro-1*H*-pyrrolizin-7a(5*H*)-yl)methoxy)-4'-((*S*)-2-(hydroxymethyl)-2-methylazetidin-1-yl)-4-methyl-3,4,5',8'-tetrahydro-2*H-*spiro[naphthalene-1,7'-pyrano[4,3-*d*]pyrimidine]-8-carbonitrile |
| **610a** | (1*S*,4*S*)-7-amino-2'-(((2*R*,7a*S*)-2-fluorotetrahydro-1*H*-pyrrolizin-7a(5*H*)-yl)methoxy)-4'-((*R*)-2-((*S*)-1-hydroxyethyl)azetidin-1-yl)-4-methyl-3,4,5',8'-tetrahydro-2*H-*spiro[naphthalene-1,7'-pyrano[4,3-*d*]pyrimidine]-8-carbonitrile |
| **610b** | (1*S*,4*S*)-7-amino-2'-(((2*R*,7a*S*)-2-fluorotetrahydro-1*H*-pyrrolizin-7a(5*H*)-yl)methoxy)-4'-((*R*)-2-((*R*)-1-hydroxyethyl)azetidin-1-yl)-4-methyl-3,4,5',8'-tetrahydro-2H-spiro[naphthalene-1,7'-pyrano[4,3-*d*]pyrimidine]-8-carbonitrile |
| **611a** | (1*S*,4*S*)-7-amino-4'-((*S*)-3,3-difluoro-2-(hydroxymethyl)azetidin-1-yl)-2'-(((2*R*,7a*S*)-2-fluorotetrahydro-1*H*-pyrrolizin-7a(5*H*)-yl)methoxy)-4-methyl-3,4,5',8'-tetrahydro-2*H-*spiro[naphthalene-1,7'-pyrano[4,3-*d*]pyrimidine]-8-carbonitrile |
| **611b** | (1*S*,4*S*)-7-amino-4'-((*R*)-3,3-difluoro-2-(hydroxymethyl)azetidin-1-yl)-2'-(((2*R*,7a*S*)-2-fluorotetrahydro-1*H*-pyrrolizin-7a(5*H*)-yl)methoxy)-4-methyl-3,4,5',8'-tetrahydro-2*H-*spiro[naphthalene-1,7'-pyrano[4,3-*d*]pyrimidine]-8-carbonitrile |
| **623a** | (1*S*,4*S*)-7-amino-4'-(2,2-dimethylazetidin-1-yl)-2'-(((2*R*,7a*S*)-2-fluorotetrahydro-1*H-*pyrrolizin-7a(5*H*)-yl)methoxy)-4-methyl-3,4,5',8'-tetrahydro-2*H*-spiro[naphthalene-1,7'-pyrano[4,3-*d*]pyrimidine]-8-carbonitrile |
| **624a** | (1*S*,4*S*)-7-amino-2'-(((2*R*,7a*S*)-2-fluorotetrahydro-1*H*-pyrrolizin-7a(5*H*)-yl)methoxy)-4'-((*S*)-2-(hydroxymethyl)-2-(methoxymethyl)azetidin-1-yl)-4-methyl-3,4,5',8'-tetrahydro-2*H*-spiro[naphthalene-1,7'-pyrano[4,3-*d*\|pyrimidine]-8-carbonitrile |
| **624b** | (1*S*,4*S*)-7-amino-2'-(((2*R*,7a*S*)-2-fluorotetrahydro-1*H*-pyrrolizin-7a(5*H*)-yl)methoxy)-4'-((*R*)-2-(hydroxymethyl)-2-(methoxymethyl)azetidin-1-yl)-4-methyl-3,4,5',8'-tetrahydro-2*H*-spiro[naphthalene-1,7'-pyrano[4,3-*d*]pyrimidine]-8-carbonitrile |
| **640a** | (1*S*,4*S*)-7-Amino-2'-(((2*R*,7a*S*)-2-fluorotetrahydro-1*H*-pyrrolizin-7a(5*H*)-yl)methoxy)-4'-((2*R*,4*R*)-2-(hydroxymethyl)-4-methylazetidin-1-yl)-4-methyl-3,4,5', 8'-tetrahydro-2*H-*spiro[naphthalene-1,7'-pyrano[4,3-*d*]pyrimidine]-8-carbonitrile |
| **653a** | (1*S*,4*S*)-7-amino-2'-(((2*R*,7a*S*)-2-fluorotetrahydro-1*H*-pyrrolizin-7a(5*H*)-yl)methoxy)-4'-((2*R*,3*S*)-2-(hydroxymethyl)-3-methylazetidin-1-yl)-4-methyl-3,4,5',8'-tetrahydro-2*H-*spiro[naphthalene-1,7'-pyrano[4,3-*d*]pyrimidine]-8-carbonitrile |
| **653b** | (1*S*,4*S*)-7-amino-2'-(((2*R*,7a*S*)-2-fluorotetrahydro-1*H*-pyrrolizin-7a(5*H*)-yl)methoxy)-4'-((2*R*,3*R*)-2-(hydroxymethyl)-3-methylazetidin-1-yl)-4-methyl-3,4,5', 8'-tetrahydro-2*H-*spiro[naphthalene-1,7'-pyrano[4,3-*d*]pyrimidine]-8-carbonitrile |
| **751a** | (*S*)-7-amino-2'-(((2*R*,7a*S*)-2-fluorotetrahydro-1*H*-pyrrolizin-7a(5*H*)-yl)methoxy)-4'-((*R*)-2-(hydroxymethyl)azetidin-1-yl)-3,4,5',8'-tetrahydro-2*H*-spiro[naphthalene-1,7'-pyrano[4,3-*d*]pyrimidine]-8-carbonitrile |
| **550a** | (*S*)-7-Amino-2'-(((2*R*,7a*S*)-2-fluorotetrahydro-1*H*-pyrrolizin-7a(5*H*)-yl)methoxy)-4'-(6-oxa-1-azaspiro[3.3]heptan-1-yl)-3,4,5',8'-tetrahydro-2*H*-spiro[naphthalene-1,7'-pyrano[4,3-*d*]pyrimidine]-8-carbonitrile |
| **556a** | (1*S*,4*S*)-7-Amino-2'-(((2*R*,7a*S*)-2-fluorotetrahydro-1*H*-pyrrolizin-7a(5*H*)-yl)methoxy)-4-methyl-4'-(6-oxa-1-azaspiro[3.3]heptan-1-yl)-3,4,5',8'-tetrahydro-2*H*-spiro[naphthalene-1,7'-pyrano[4,3-*d*]pyrimidine]-8-carbonitrile |
| **599a** | (1*S*,4*S*)-7-amino-2'-(((2*S*,7a*R*)-2-methoxytetrahydro-1*H*-pyrrolizin-7a(5*H*)-yl)methoxy)-4-methyl-4'-(6-oxa-1-azaspiro[3 .3]heptan-1-yl)-3,4,5',8'-tetrahydro-2*H-*spiro[naphthalene-1,7'-pyrano[4,3-*d*]pyrimidine]-8-carbonitrile |
| **599b** | (1*S*,4*S*)-7-amino-2'-(((2*R*,7a*S*)-2-methoxytetrahydro-1*H*-pyrrolizin-7a(5*H*)-yl)methoxy)-4-methyl-4'-(6-oxa-1-azaspiro[3.3]heptan-1-yl)-3,4,5',8'-tetrahydro-2*H-*spiro[naphthalene-1,7'-pyrano[4,3-*d*]pyrimidine]-8-carbonitrile |
| **666a** | (1*S*,4*S*)-7-amino-2'-(((*S*)-2,2-difluorotetrahydro-1*H*-pyrrolizin-7a(5*H*)-yl)methoxy)-4-methyl-4'-(6-oxa-1-azaspiro[3.3]heptan-1-yl)-3,4,5',8'-tetrahydro-2*H*-spiro[naphthalene-1,7'-pyrano[4,3-*d*]pyrimidine]-8-carbonitrile |
| **580a** | (1*S*)-7-amino-2'-(((2*R*,7a*S*)-2-fluorotetrallydro-1*H*-pyrrolizin-7a(5*H*)-yl)methoxy)-4'-(6-oxa-1-azaspiro[3.4]octan-1-yl)-3,4,5',8'-tetrahydro-2*H*-spiro[naphthalene-1,7'-pyrano[4,3-*d*]pyrimidine]-8-carbonitrile |
| **598b** | (1*S*,4*S*)-7-amino-2'-(((2*R*,7a*S*)-2-fluorotetrahydro-1*H*-pyrrolizin-7a(5*H*)-yl)methoxy)-4-methyl-4'-((*S*)-6-oxa-1-azaspiro[3.4]octan-1-yl)-3,4,5',8'-tetrahydro-2*H-*spiro[naphthalene-1,7'-pyrano[4,3-*d*]pyrimidine]-8-carbonitrile |
| **598c** | (1*S*,4*S*)-7-amino-2'-(((2*R*,7a*S*)-2-fluorotetrahydro-1*H*-pyrrolizin-7a(5*H*)-yl)methoxy)-4-methyl-4'-((*R*)-6-oxa-1-azaspiro[3.4]octan-1-yl)-3,4,5',8'-tetrahydro-2*H-*spiro[naphthalene-1,7'-pyrano[4,3-*d*]pyrimidine]-8-carbonitrile |
| **654a** | (1*S*,4*S*)-7-amino-2'-(((2*R*,7a*S*)-2-methoxytetrahydro-1*H*-pyrrolizin-7a(5*H*)-yl)methoxy)-4-methyl-4'-((*S*)-6-oxa-1-azaspiro[3.4]octan-1-yl)-3,4,5',8'-tetrahydro-2H-spiro[naphthalene-1,7'-pyrano[4,3-*d*]pyrimidine]-8-carbonitrile |
| **654b** | (1*S*,4*S*)-7-amino-2'-(((2*S*,7a*R*)-2-methoxytetrahydro-1*H*-pyrrolizin-7a(5*H*)-yl)methoxy)-4-methyl-4'-((*S*)-6-oxa-1-azaspiro[3.4]octan-1-yl)-3,4,5',8'-tetrahydro-2H-spiro[naphthalene-1,7'-pyrano[4,3-*d*]pyrimidine]-8-carbonitrile |
| **654c** | (1*S*,4*S*)-7-amino-2'-(((2*S*,7a*R*)-2-methoxytetrahydro-1*H*-pyrrolizin-7a(5*H*)-yl)methoxy)-4-methyl-4'-((*R*)-6-oxa-1-azaspiro[3.4]octan-1-yl)-3,4,5',8'-tetrahydro-2H-spiro[naphthalene-1,7'-pyrano[4,3-*d*]pyrimidine]-8-carbonitrile |
| **654d** | (1*S*,4*S*)-7-amino-2'-(((2*R*,7a*S*)-2-methoxytetrahydro-1*H*-pyrrolizin-7a(5*H*)-yl)methoxy)-4-methyl-4'-((*R*)-6-oxa-1-azaspiro[3.4]octan-1-yl)-3,4,5',8'-tetrahydro-2H-spiro[naphthalene-1,7'-pyrano[4,3-*d*]pyrimidine]-8-carbonitrile |
| **585a** | (1*S*,4*S*)-7-amino-2'-(((2*R*,7a*S*)-2-fluorotetrahydro-1*H*-pyrrolizin-7a(5*H*)-yl)methoxy)-4-methyl-4'-((*R*)-6-oxa-1-azaspiro[3.5]nonan-1-yl)-3,4,5',8'-tetrahydro-2*H-*spiro[naphthalene-1,7'-pyrano[4,3-*d*]pyrimidine]-8-carbonitrile |
| **585b** | (1*S*,4*S*)-7-amino-2'-(((2*R*,7a*S*)-2-fluorotetrahydro-1*H*-pyrrolizin-7a(5*H*)-yl)methoxy)-4-methyl-4'-((*S*)-6-oxa-1-azaspiro[3.5]nonan-1-yl)-3,4,5',8'-tetrahydro-2*H-*spiro[naphthalene-1,7'-pyrano[4,3-*d*]pyrimidine]-8-carbonitrile |
| **662a** | (1*S*,4*S*)-7-amino-2'-(((2*S*,7a*R*)-2-methoxytetrahydro-1*H*-pyrrolizin-7a(5*H*)-yl)methoxy)-4-methyl-4'-((*S*)-6-oxa-1-azaspiro[3.5]nonan-1-yl)-3,4,5',8'-tetrahydro-2*H-*spiro[naphthalene-1,7'-pyrano[4,3-*d*]pyrimidine]-8-carbonitrile |
| **662b** | (1*S*,4*S*)-7-amino-2'-(((2*R*,7a*S*)-2-methoxytetrahydro-1*H*-pyrrolizin-7a(5*H*)-yl)methoxy)-4-methyl-4'-((*S*)-6-oxa-1-azaspiro[3.5]nonan-1-yl)-3,4,5',8'-tetrahydro-2*H-*spiro[naphthalene-1,7'-pyrano[4,3-*d*]pyrimidine]-8-carbonitrile |
| **662c** | (1*S*,4*S*)-7-amino-2'-(((2*S*,7a*R*)-2-methoxytetrahydro-1*H*-pyrrolizin-7a(5*H*)-yl)methoxy)-4-methyl-4'-((*R*)-6-oxa-1-azaspiro[3.5]nonan-1-yl)-3,4,5',8'-tetrahydro-2*H-*spiro[naphthalene-1,7'-pyrano[4,3-*d*]pyrimidine]-8-carbonitrile |
| **662d** | (1*S*,4*S*)-7-amino-2'-(((2*R*,7a*S*)-2-methoxytetrahydro-1*H*-pyrrolizin-7a(5*H*)-yl)methoxy)-4-methyl-4'-((*R*)-6-oxa-1-azaspiro[3.5]nonan-1-yl)-3,4,5',8'-tetrahydro-2*H-*spiro[naphthalene-1,7'-pyrano[4,3-*d*]pyrimidine]-8-carbonitrile |

or pharmaceutically acceptable salts thereof.

In some embodiments, compounds of Formula **(II),** (**III**), **(IV), (V),** and/or **(VI)** are selected from the group consisting of the compounds in **Table C3** of U.S. Provisional Application Serial Nos. 63/675,568, filed July 25, 2024; 63/653,025, filed May 29, 2024; 63/650,285, filed May 21, 2024; 63/572,733, filed April 1, 2024; 63/567,306, filed March 19, 2024; 63/559,553, filed February 29, 2024; 63/614,248, filed December 22, 2023; 63/545,535, filed October 24, 2023; 63/542,178, filed October 3, 2023; 63/535,014, filed August 28, 2023; and 63/533,354, filed August 17, 2023, or pharmaceutically acceptable salts thereof, each Table **C3** is incorporated by reference it its entirety herein.

Certain examples of Formula **(II), (III), (IV), (V),** and/or **(VI)** were synthesized using methods involving resolution of stereoisomeric mixture(s) (e.g., SFC separation of stereoisomers). In **Table C3,** the resolved stereogenic centers in these compounds are labelled with the "*or1*" and/or *"or2"* enhanced stereochemical notations. In some instances, the stereoisomeric resolutions were performed during the last step of the synthesis, thereby providing the individual stereoisomers of the compounds. Alternatively, in some other instances, the resolutions were performed on an intermediate or starting material, wherein each of the constituent stereoisomers of the intermediate or starting material could be separately subjected to the subsequent steps of the synthesis to provide the respective compounds as separate stereoisomers. A person of ordinary skill in the art would understand that, under either approach for stereoisomeric resolution, stereoisomers having both (*R*)- and (*S*)-configurations at a resolved stereogenic center are provided. *See* **Table C5,** wherein **Table C3** compounds whose stereoisomers contain the *or1* and/or *or2* stereochemical notations are provided in non-stereogenic form, followed by the respective stereoisomers having the (*R*)- and (*S*)-configurations.

**Table C5**

| **No.** | **Compound Structure** |
|---|---|
| 502 | 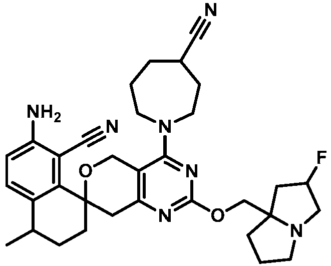 |
| | 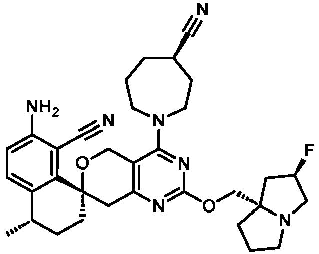 |
| | 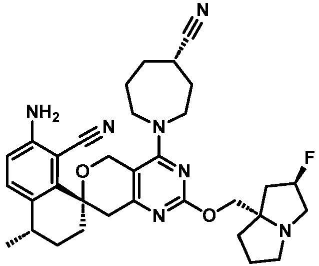 |
| 510 | 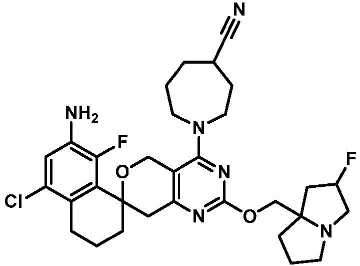 |
| | 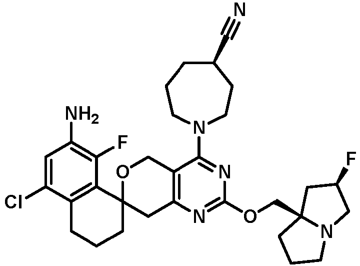 |
| | 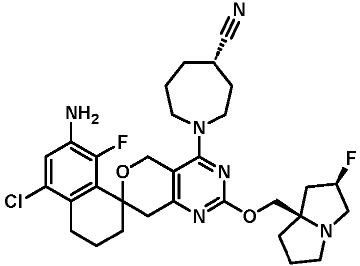 |
| 512 | 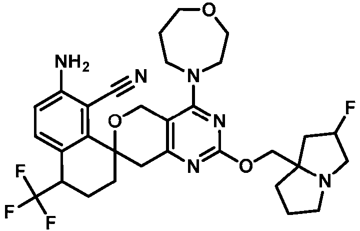 |
| | 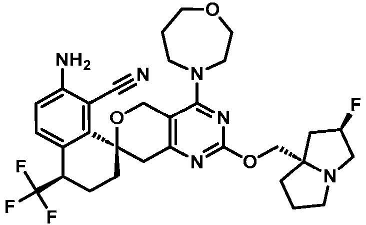 |
| | 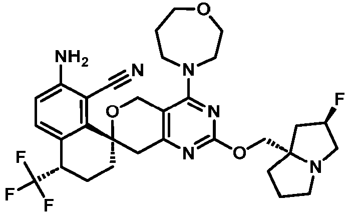 |
| **527** | 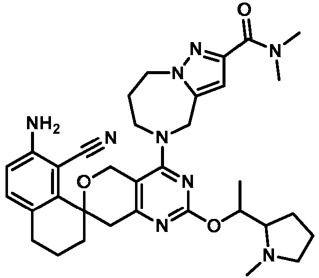 |
| | 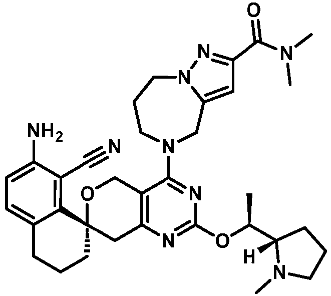 |
| | 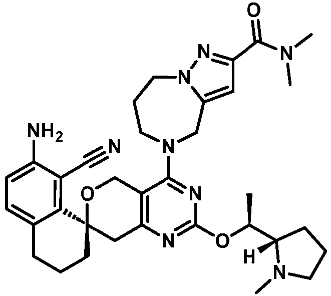 |
| **533** | 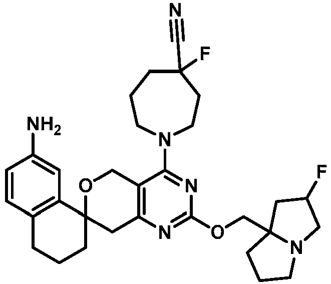 |
| | 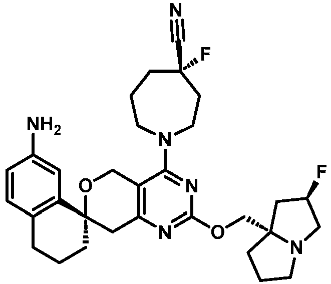 |
| | 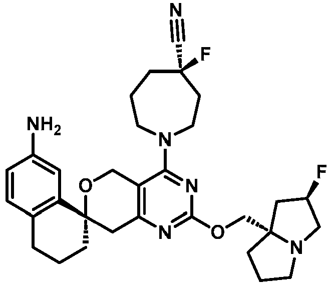 |
| **554** | 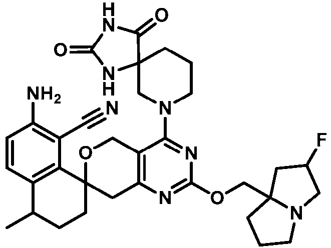 |
| | 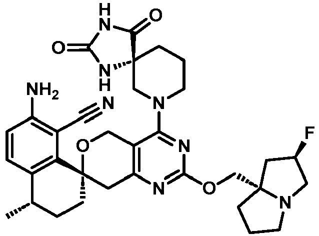 |
| | 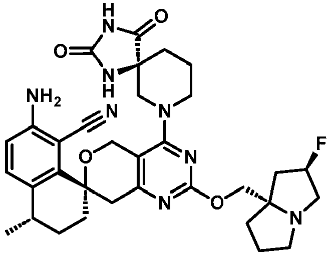 |
| **599** | 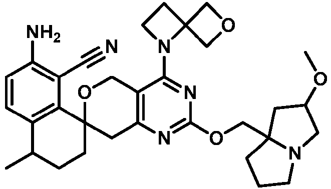 |
| | 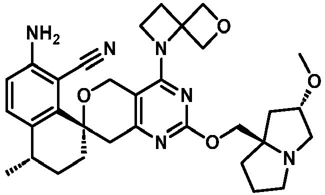 |
| | 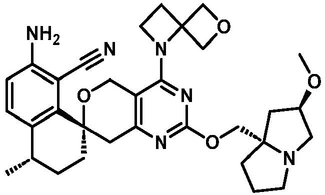 |
| **603** | 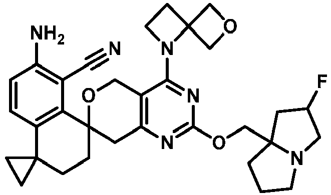 |
| | 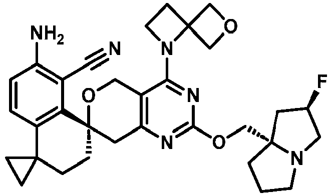 |
| | 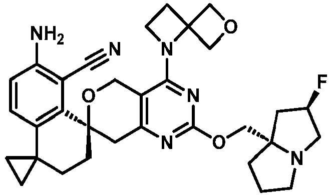 |
| **610** | 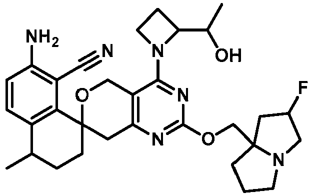 |
| | 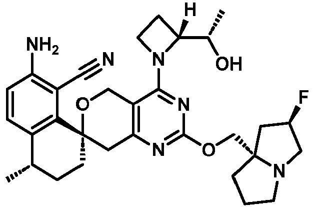 |
| | 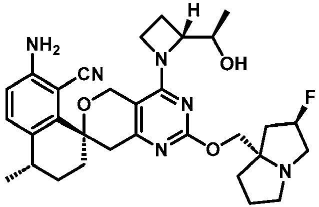 |
| **624** | 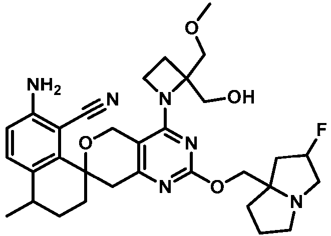 |
| | 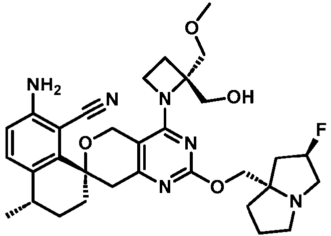 |
| | 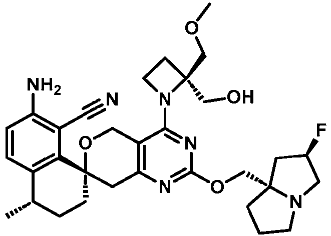 |
| **628** | 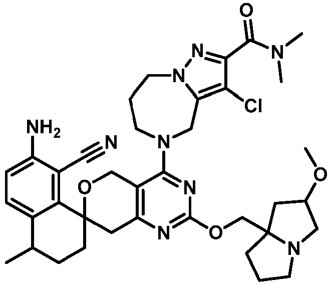 |
| | 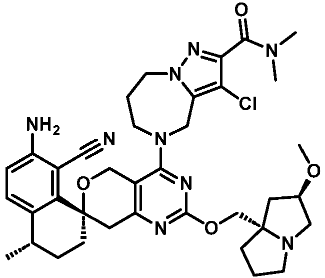 |
| | 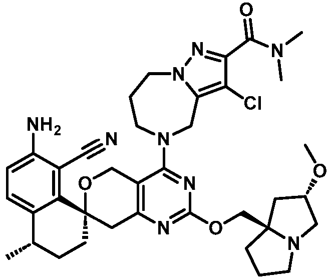 |
| **641** | 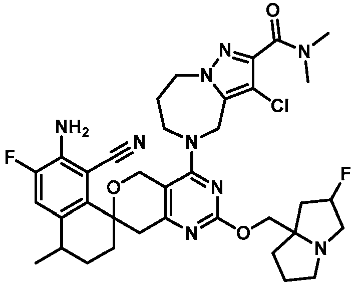 |
| | 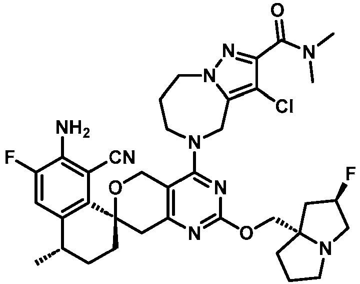 |
| | 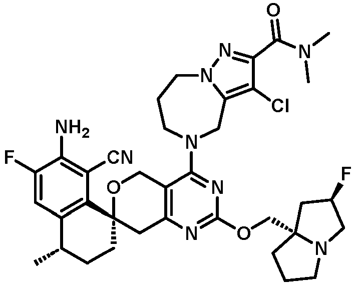 |
| **645** | 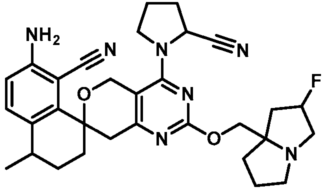 |
| | 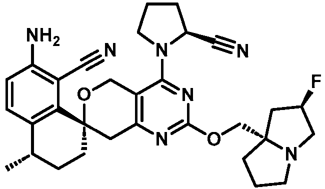 |
| | 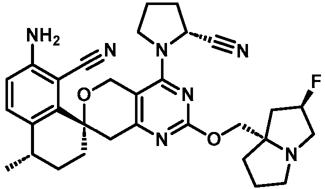 |
| **650** | 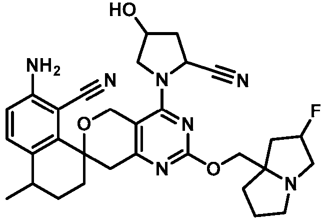 |
| | 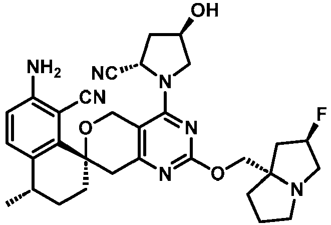 |
| | 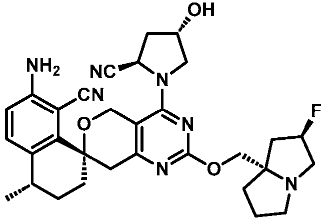 |
| | 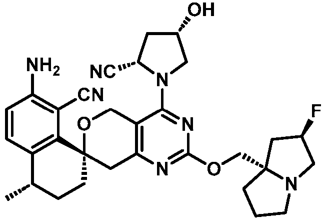 |
| | 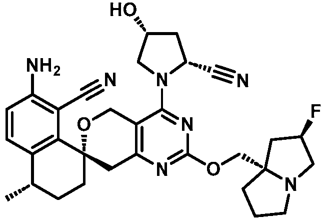 |
| **651** | 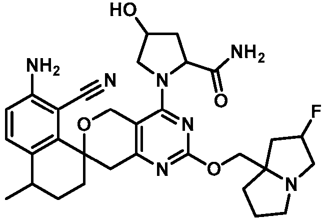 |
| | 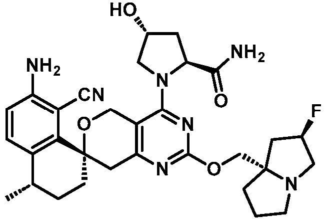 |
| | 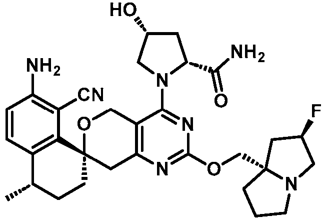 |
| **652** | 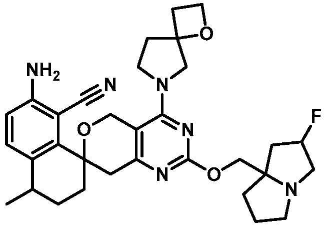 |
| | 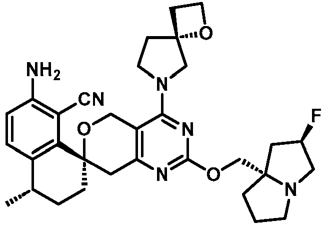 |
| | 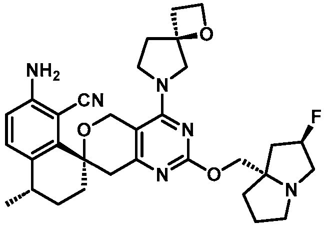 |
| **654** | 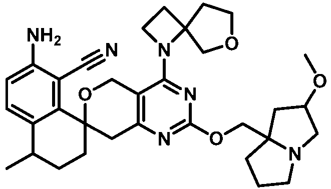 |
| | 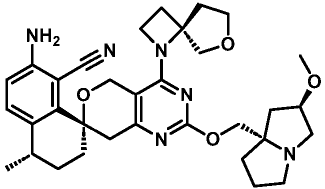 |
| | 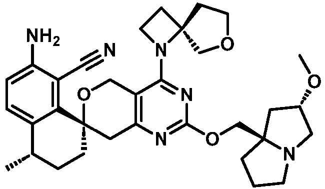 |
| | 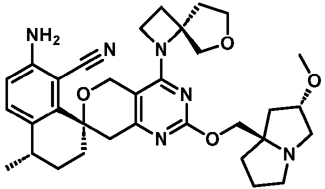 |
| | 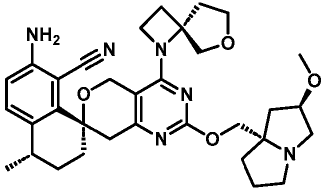 |
| **656** | 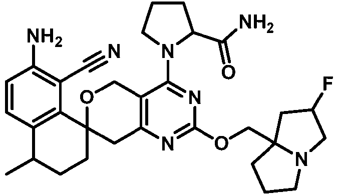 |
| | 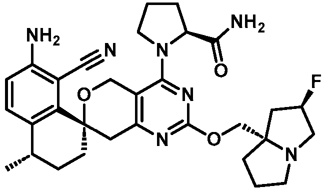 |
| | 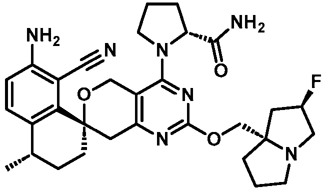 |
| **662** | 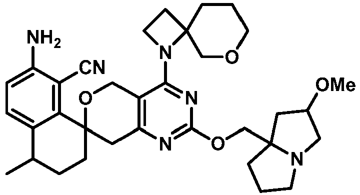 |
| | 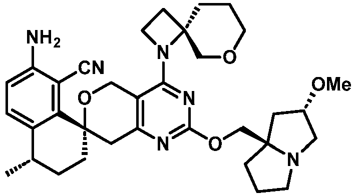 |
| | 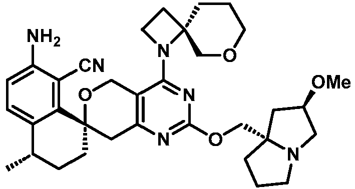 |
| | 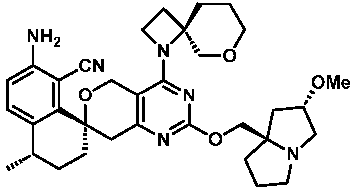 |
| | 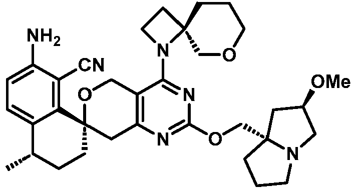 |
| **665** | 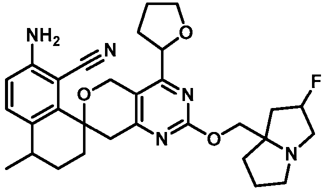 |
| | 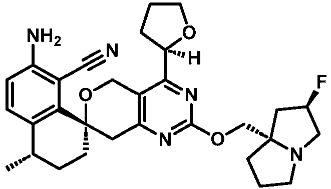 |
| | 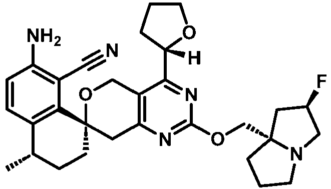 |
| **680** | 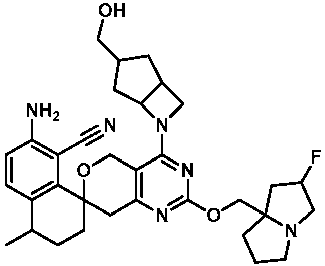 |
| | 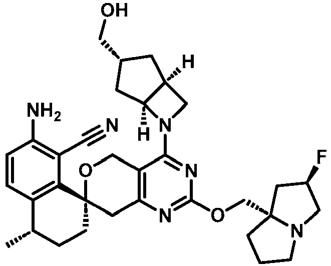 |
| | 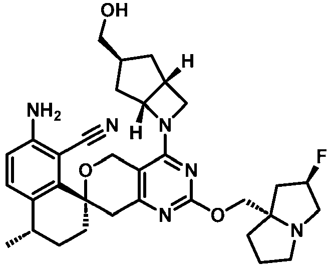 |
| **689** | 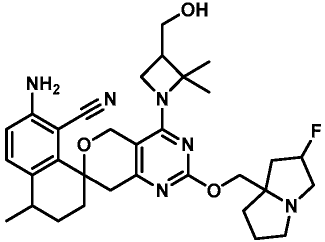 |
| | 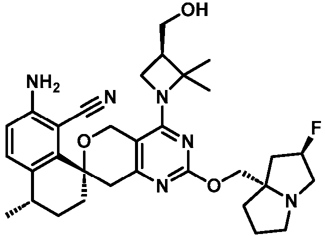 |
| | 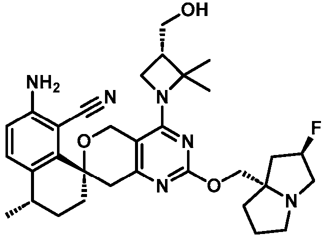 |
| **702** | 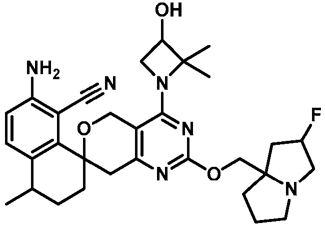 |
| | 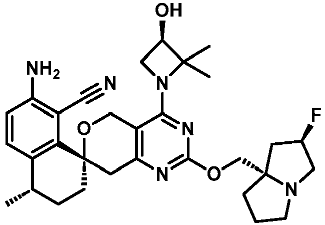 |
| | 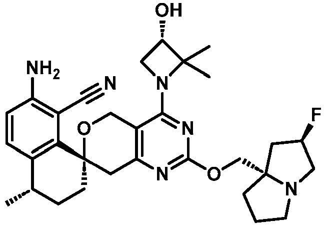 |
| **706** | 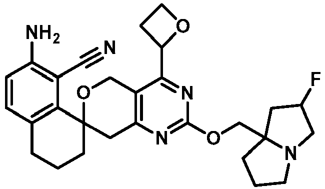 |
| | 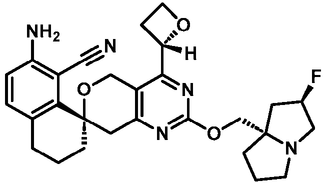 |
| | 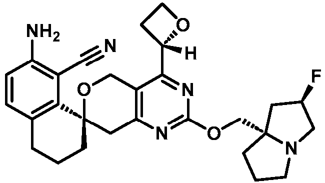 |
| | 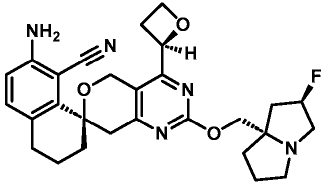 |
| | 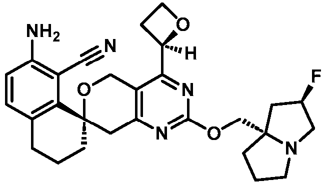 |
| **737** | 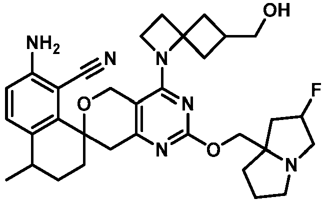 |
| | 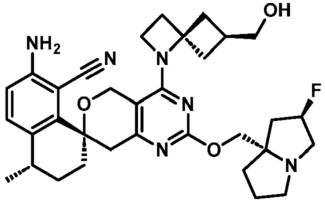 |
| | 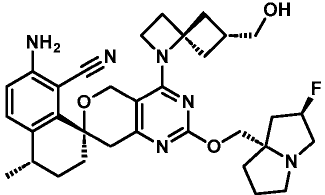 |

In some embodiments, compounds of Formula **(II),** (**III**), **(IV),** and/or **(V)** are selected from the group consisting of the compounds in **Table C2,** or pharmaceutically acceptable salts thereof.

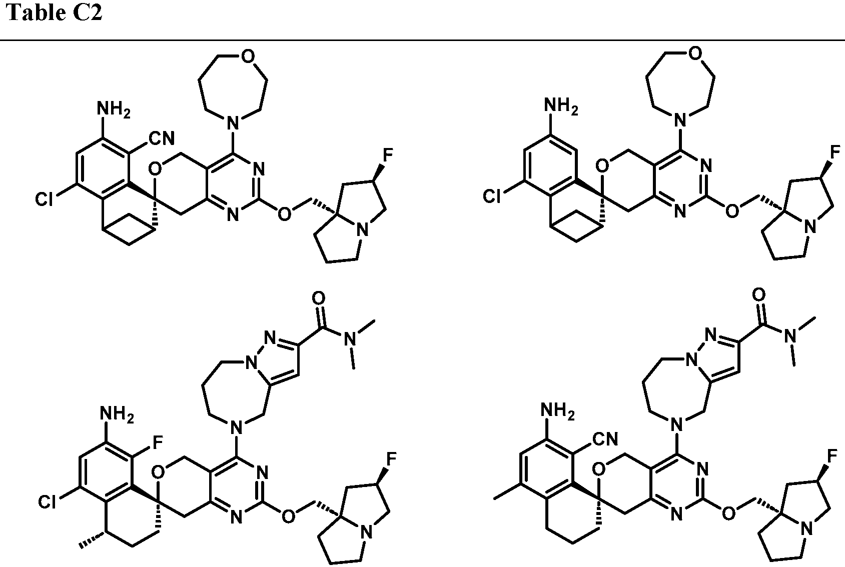

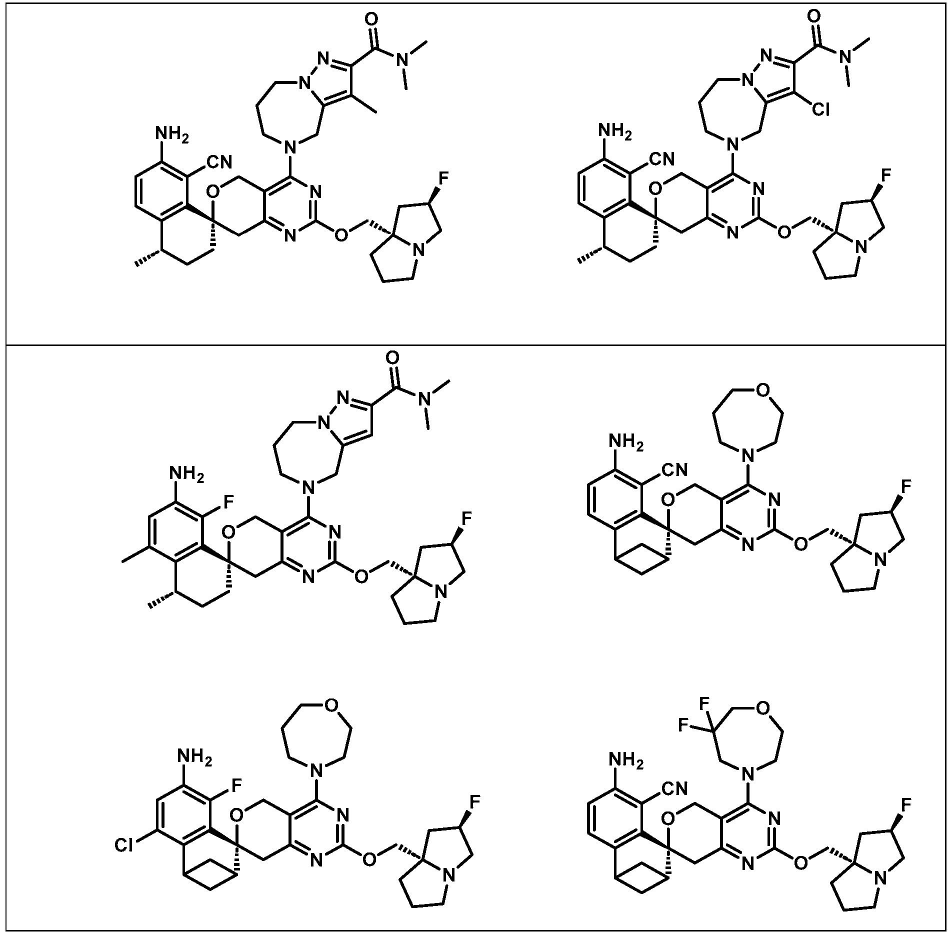

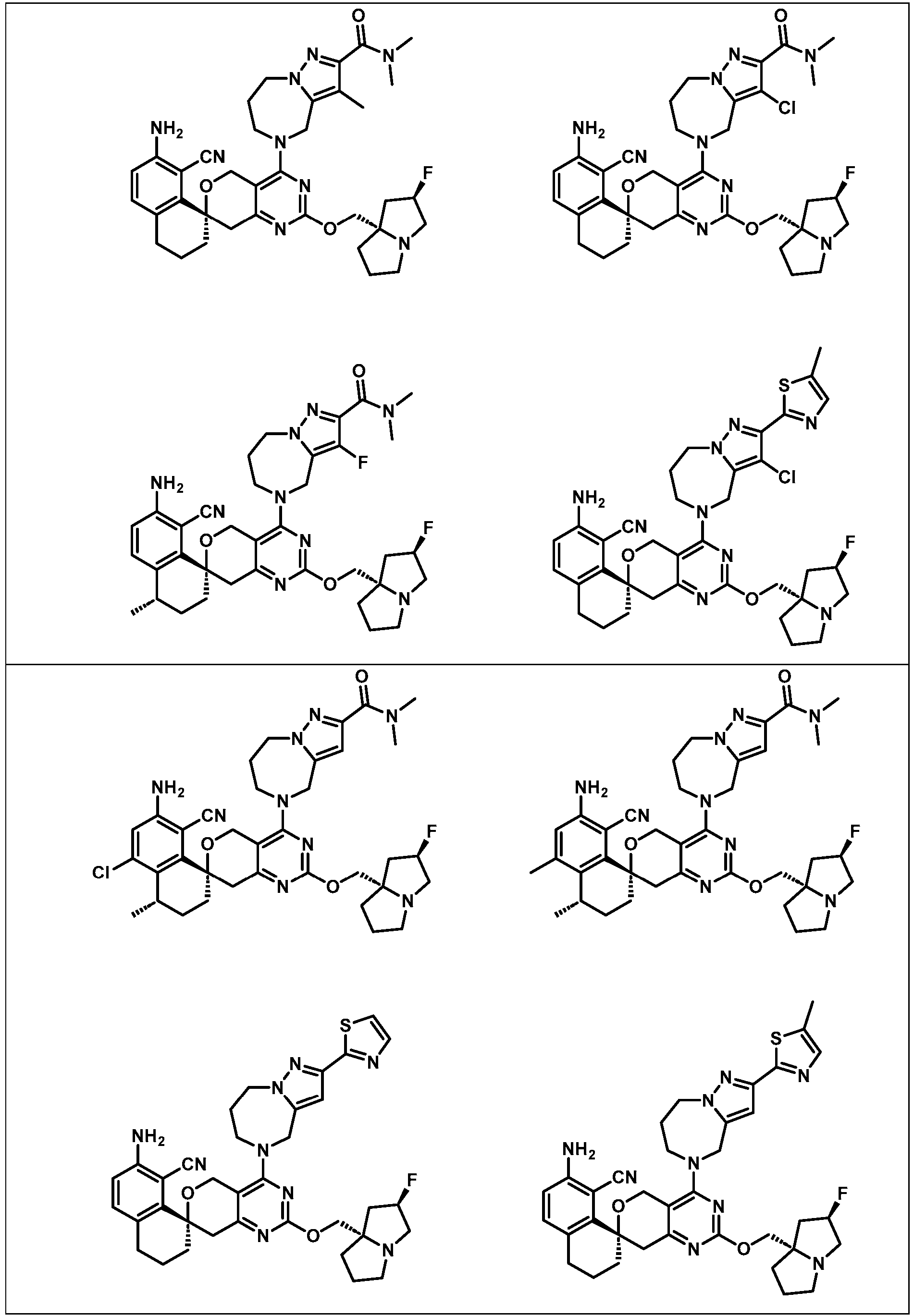

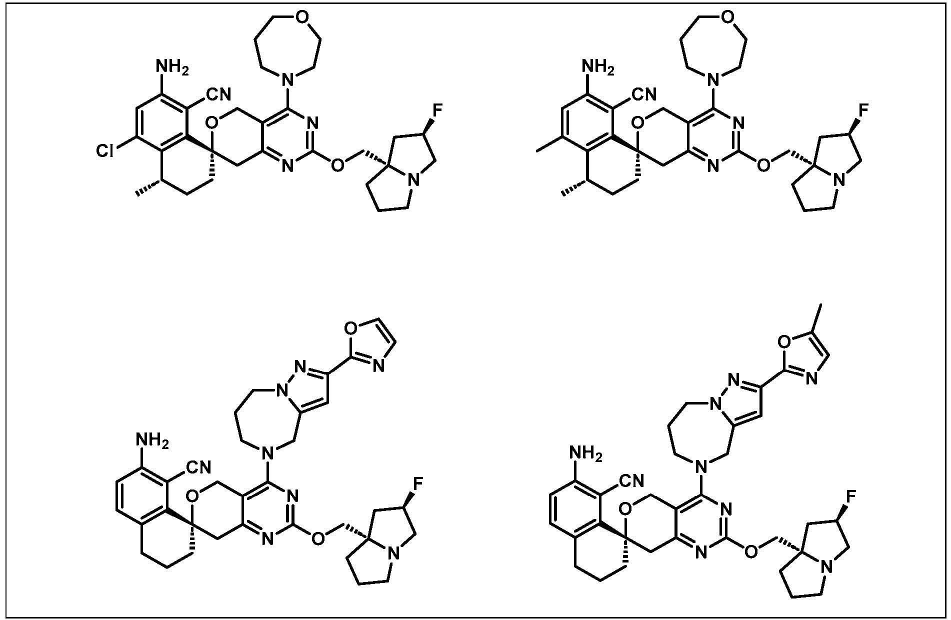

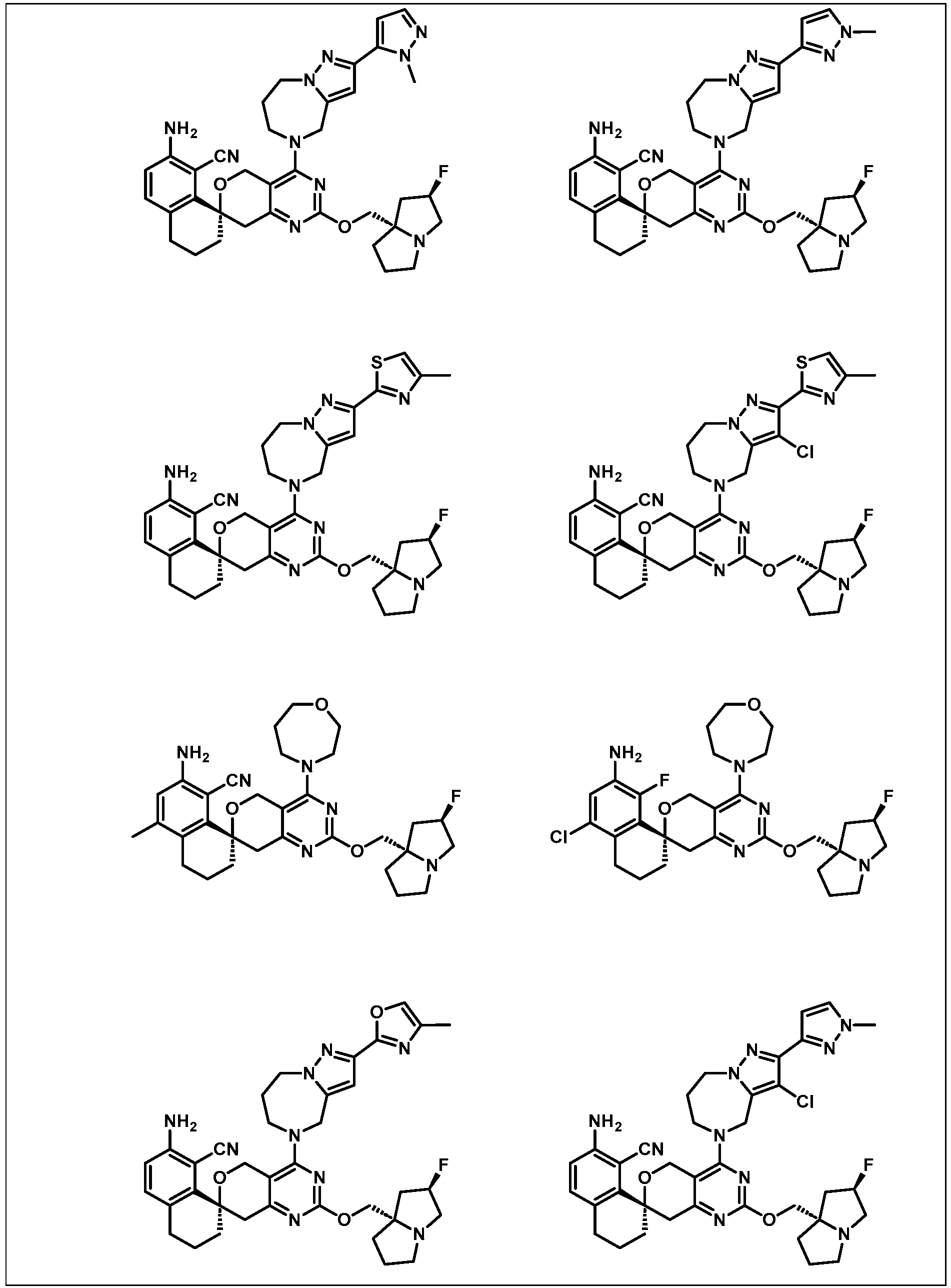

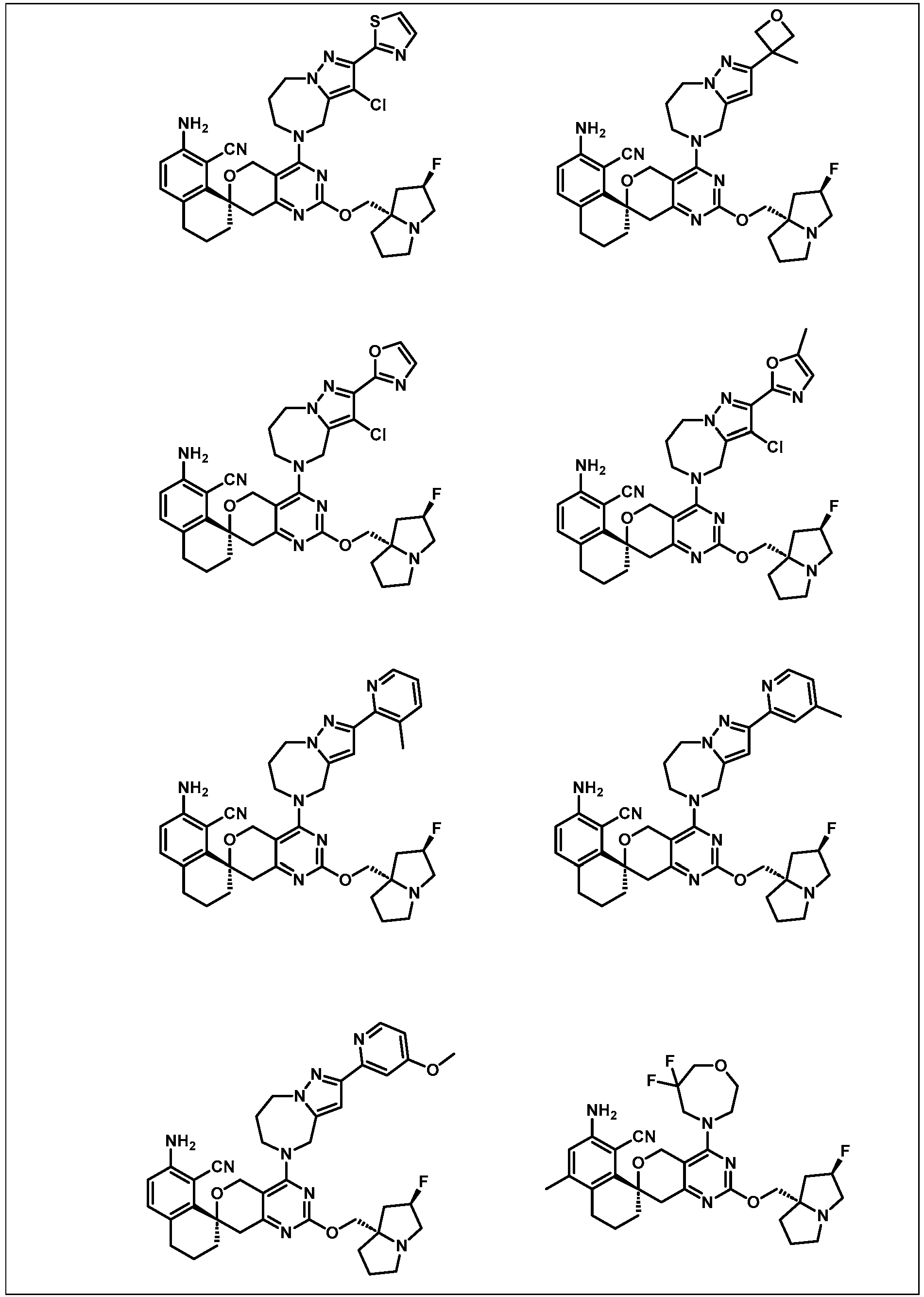

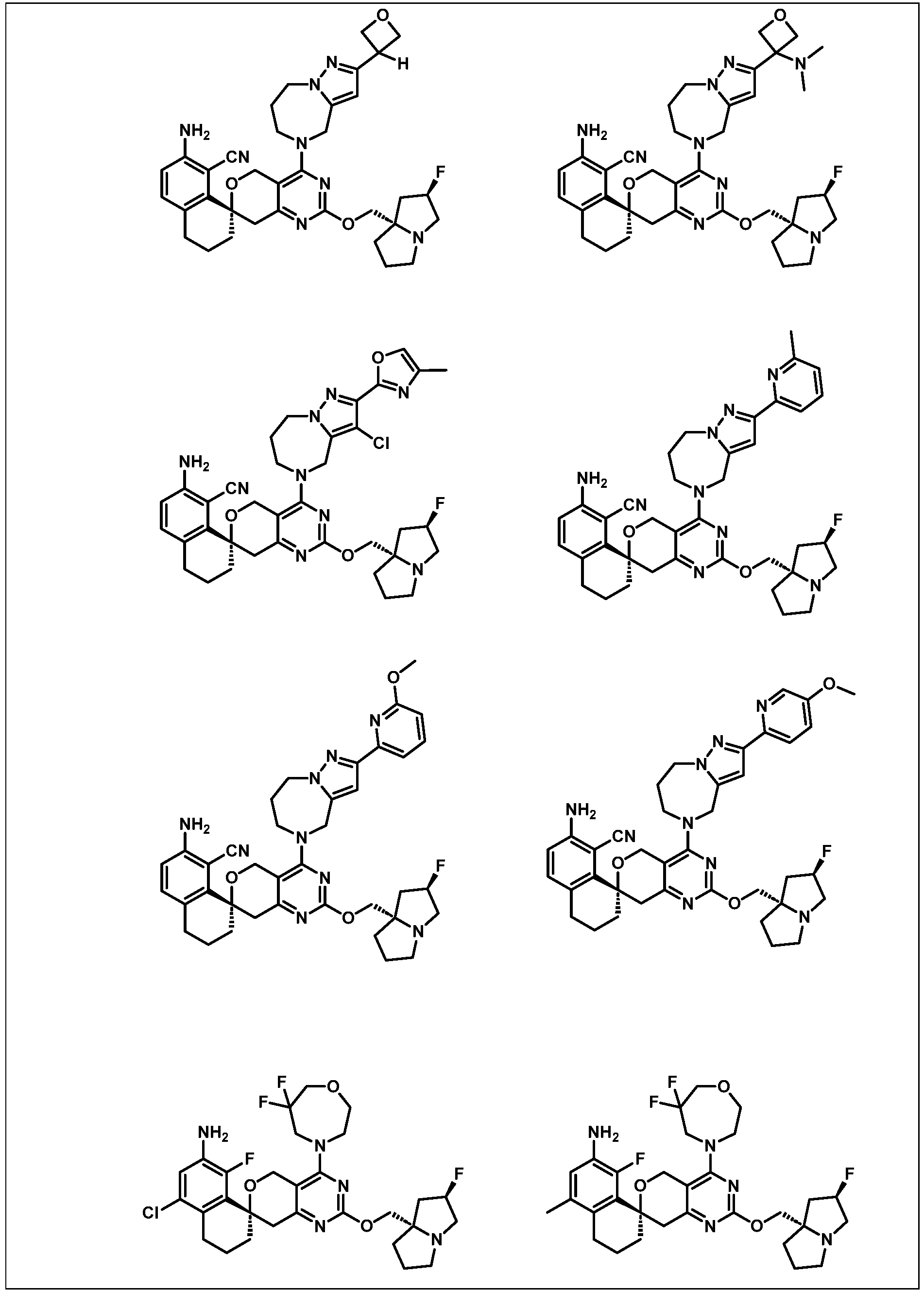

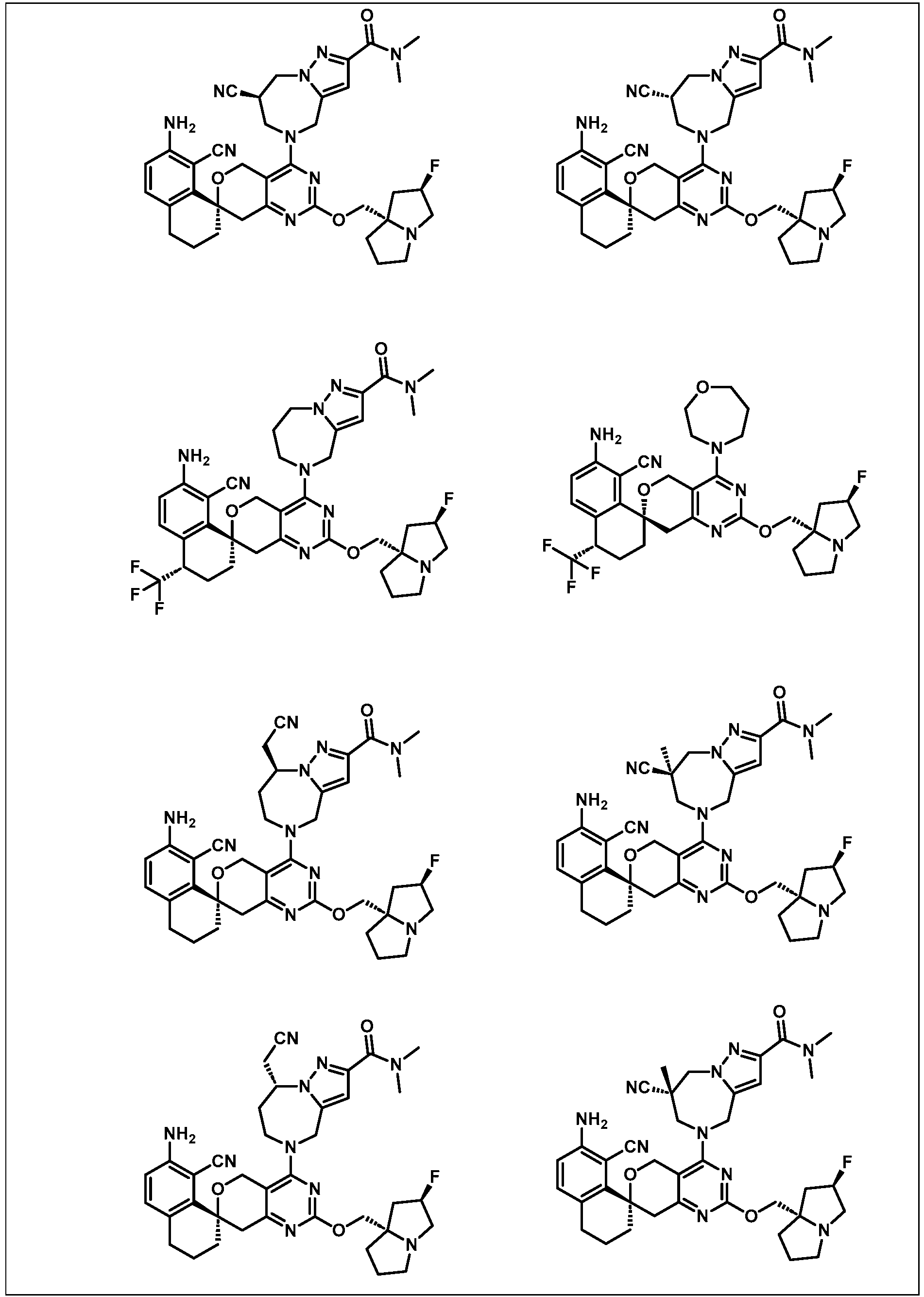

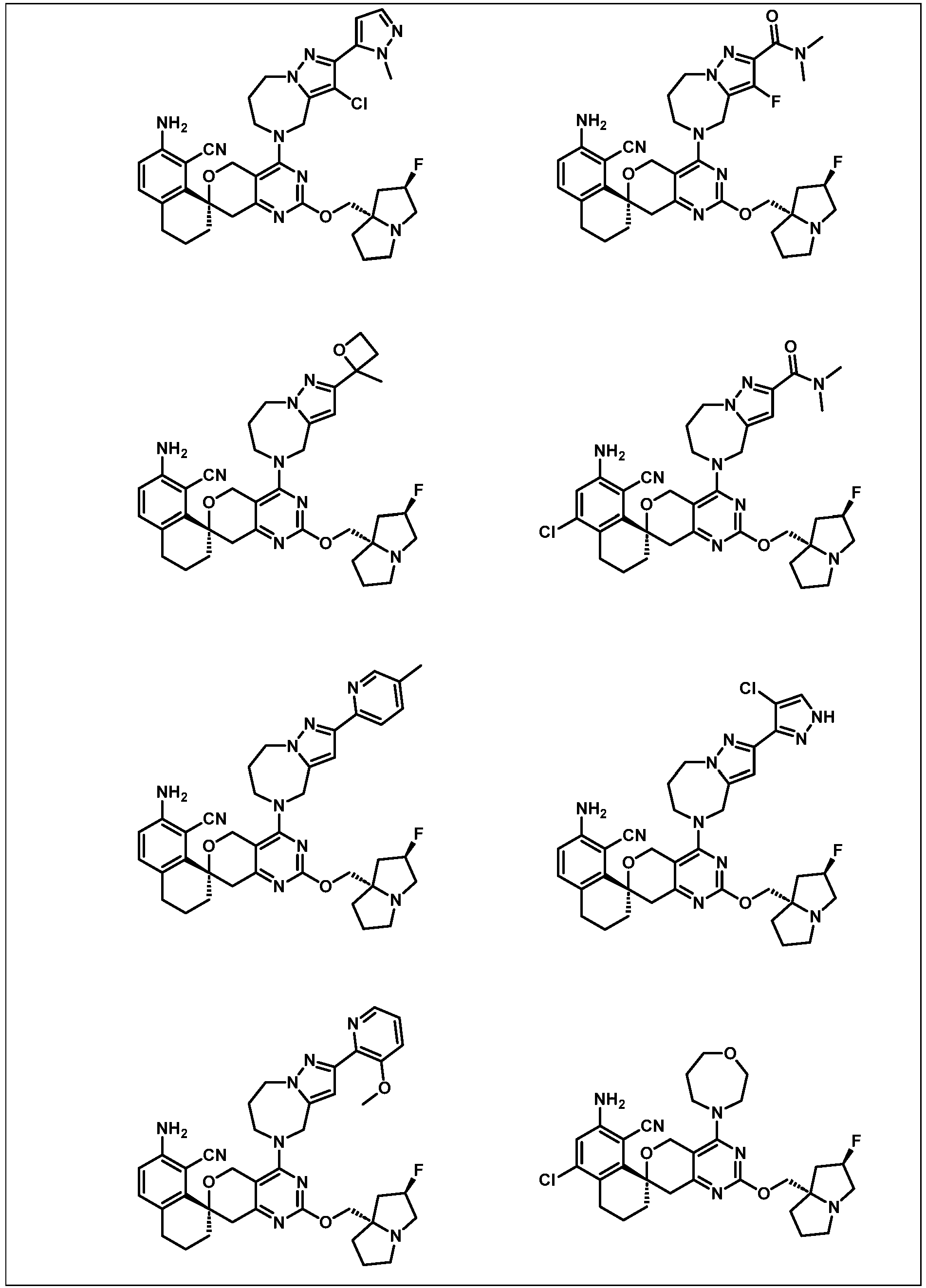

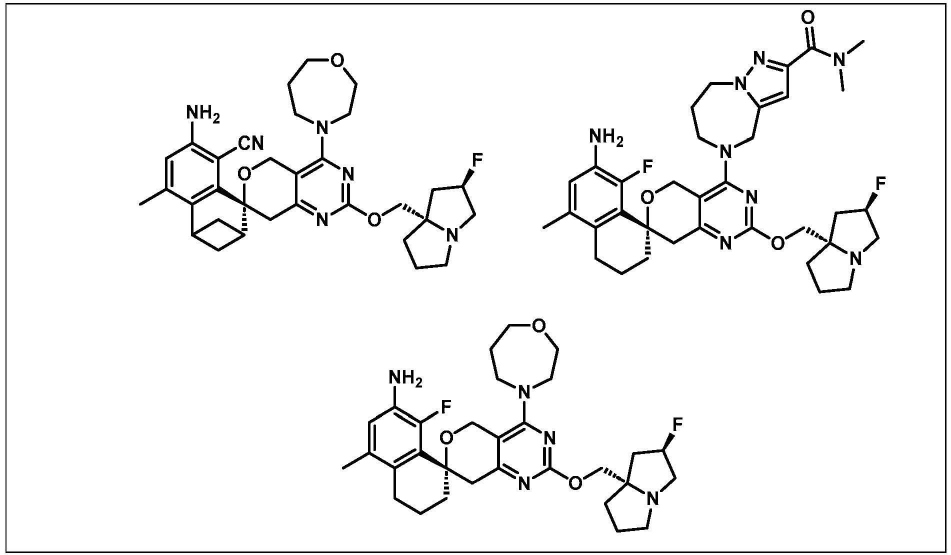

Also provided herein are compounds of Formula **(A):** 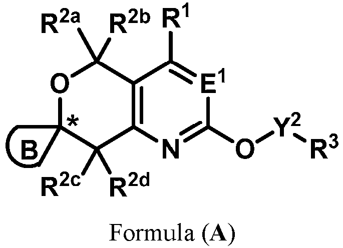 or pharmaceutically acceptable salts thereof or prodrugs thereof, wherein:
**E¹** is selected from the group consisting of N, CH, and C**R⁴**, wherein **R⁴** is selected from the group consisting of: -CN, halo, C₁₋₃ alkyl, C₁₋₃ haloalkyl, and C₃₋₆ cycloalkyl;
**R¹** is selected from the group consisting of:
   **(i)** a 4-10 membered heterocyclyl optionally substituted with 1-4 **R⁷**;
   **(ii)** an 8-12 membered bicyclic heterocyclyl, wherein the heterocyclyl comprises an endocyclic group selected from the group consisting of C(=O)NH and S(O)₂NH, and wherein the heterocyclyl is further optionally substituted with 1-3 **R⁷** at one or more ring carbon atoms; and
   **(iii)** 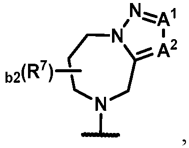 wherein b2 is 0, 1, 2, or 3; and **A¹** and **A²** are independently selected from the group consisting of: N, CH, and C**R⁷**;
each **R⁷** is independently selected from the group consisting of **R^{a}** and **R^{b}**;
**R^{2a}** and **R^{2b}** are independently selected from the group consisting of: -H, C₁₋₃ alkyl, C₁₋₃ haloalkyl, and C₃₋₆ cycloalkyl; or
**R^{2a}** and **R^{2b}** taken together with the ring carbon atom to which each is attached form a C₃₋₆ cycloalkyl ring or 4-6 membered heterocyclyl ring;
**R^{2c}** and **R^{2d}** are independently selected from the group consisting of: -H, halo, -CN, C₁₋₃ alkyl, C₁₋₃ haloalkyl, and C₃₋₆ cycloalkyl; or
**R^{2c}** and **R^{2d}** taken together with the ring carbon atom to which each is attached form a C₃₋₆ cycloalkyl ring or 4-6 membered heterocyclyl ring;
**Ring B** is selected from the group consisting of: 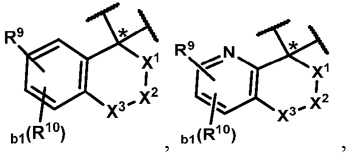 and 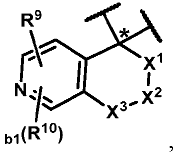 wherein:
the * marks the ring carbon atom common to both **Ring B** and 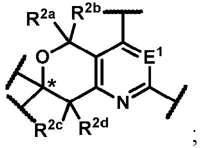
**X¹** is selected from the group consisting of a bond, S(O)₀₋₂, CH₂, CH**R^{L}**, C(**R^{L}**)₂, and O;
**X²** and **X³** are independently selected from the group consisting of: CH₂, CH**R^{L}**, C(**R^{L}**)₂, O, and S(O)₀₋₂, provided that no more than one of **X¹**, **X²**, and **X³** is selected from the group consisting of: O and S(O)₀₋₂;
**b1** is 0, 1, or 2;
**R⁹** is selected from the group consisting of: -H, -OH, -N**R^{d}R^{e}**, and halo;
each **R¹⁰** is independently selected from the group consisting of **R^{a}** and **R^{b}**;
each **R^{L}** is independently selected from the group consisting of C₁₋₃ alkoxy, -F, -CN, and C₁₋₃ alkyl optionally substituted with 1-3 **R^{c}**; or
a pair of **R^{L}** on the same or different ring carbon atom(s) taken together with the ring atom(s) connecting them form a C₃₋₆ cycloalkyl ring;
**Y²** is a bond or a straight-chain C₁₋₆ alkylene optionally substituted with 1-6 **R^{Y}**;
each **R^{Y}** is independently selected from the group consisting of: halo, cyano, -OH, oxo, C₁₋₆ alkoxy, C₁₋₆ haloalkoxy, C₁₋₆ alkyl, C₁₋₆ haloalkyl, and 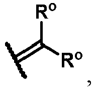 or
a pair of **R^{Y}** on the same or different carbon atom(s) taken together with the atom(s) connecting them forms a C₃₋₆ cycloalkyl ring or 4-6 membered heterocyclyl ring, each of which is optionally substituted with 1-3 substituents independently selected from the group consisting of F and C₁₋₃ alkyl;
**R³** is selected from the group consisting of:
   **(a)** a 4-15 membered heterocyclyl optionally substituted with 1-6 substituents independently selected from the group consisting of: **R^{a}**, **R^{b}**, 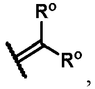 and 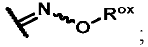 and
   **(b)** -N**R^{d}R^{e}**;
each **R^{o}** is independently selected from the group consisting of: -H, -F, C₁₋₃ alkyl, and C₁₋₃ haloalkyl;
**R^{ox}** is -H or C₁₋₃ alkyl;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   **(f)** -C₁₋₆ haloalkoxy;
   **(g)** -N**R^{d}R^{e}**;
   **(h)** C(=O)C₁₋₆ alkyl;
   **(i)** C(=O)C₁₋₆ haloalkyl;
   **(j)** C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   **(o)** S(O)₀₋₂(C₁₋₆ haloalkyl);
   **(p)** S(O)₁₋₂N(**R^{f}**)₂; and
   **(q)** C₁₋₆ alkyl, C₂₋₆ alkenyl, or C₂₋₆ alkynyl, each optionally substituted with 1-6 **R^{c}**;
each **R^{b}** is independently selected from the group consisting of: -**(L^{b})_{b}-R^{b1}** and **-R^{b1}**, wherein:
   **b** is 1, 2, or 3;
   each **-L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-12 (e.g., 4-10) membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e}**, C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)₁₋₂N(**R^{f}**)₂;
   each **R^{d}** and **R^{e}** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂-.

The term "prodrug" as used herein refers to a derivative of a compound of Formula **(A)** which releases the Formula **(A)** compound under appropriate conditions (e.g., under *in vivo* conditions) via non-enzymatic (e.g., chemical reduction, oxidation, or hydrolysis (e.g., acid catalyzed hydrolysis)) or enzymatic (e.g., esterase, nuclease, lipase, amidase, or protease catalyzed reactions) processes. A prodrug can be used to change the biological distribution of Formula **(A)** compounds or its pharmacokinetics. A variety of groups have been used to modify compounds to form prodrugs, such as esters (e.g., benzoates, acetates, etc.), ethers, carbamates, carbonates, *N*,*O*-acetals, phosphate esters/salts, etc. A compound of Formula **(A)** may form prodrugs at -NH₂ (e.g., at **R⁹** when **R⁹** is -NH2) or -OH (e.g., at **R⁷** when **R⁷** is -OH or C₁₋₃ alkyl substituted with -OH) functionalities. Further information on the use of prodrugs may be found in WO 2024/050640; ACS Omega 2023, 8, 7, 7211-7221, doi: 10.1021/acsomega.3c00329; Nat Rev Drug Discov 7, 255-270 (2008), doi: 10.1038/nrd246; Chem Biol Drug Des 82: 643-668 (2013), doi: 10.1111/cbdd.12224; Prodrugs as Novel Delivery Systems, Vol.14, ACS Symposium Series; Bioreversible Carriers in Drug Design, Pergamon Press, 1987 (ed. E. B. Roche, American Pharmaceutical Association.

In some embodiments of Formula **(A), Ring B** is: 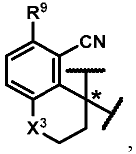 wherein **X³** is -CH₂-, - CH**R^{L}-**, or -C(**R^{L}**)₂-. In some embodiments, **R⁹** is selected from the group consisting of: OH, N**R^{d}R^{e}** (e.g., OH and NH₂). In some embodiments, each **R^{L}** is independently C₁₋₃ alkyl optionally substituted with 1-3 F.

In some embodiments of Formula **(A), Ring B** is: 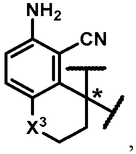 wherein **X³** is -CH₂-, - CH**R^{L}-**, or -C(**R^{L}**)₂-. In some embodiments, each **R^{L}** is independently C₁₋₃ alkyl optionally substituted with 1-3 F.

In some embodiments of Formula **(A), Ring B** is 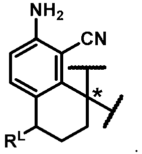 For example, **R^{L}** can be methyl, ethyl, or CF₃. For example, **R^{L}** can be methyl.

In some embodiments of Formula (A), **Ring B** is

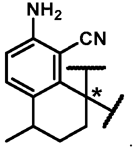

In some embodiments of Formula (A), **Ring** B is

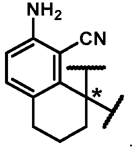

In some embodiments of Formula (A), **Ring B** is

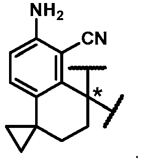

In some embodiments of Formula (A), **R^{2a}**, **R^{2b}**, **R^{2c}**, and **R^{2d}** are each H.

In some embodiments, the compounds of Formula (A) are compounds of Formula **(I-a1**): 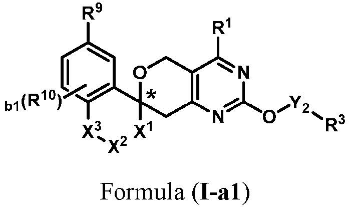 or pharmaceutically acceptable salts thereof.

In some embodiments of Formula (**I-a1**), **X¹** is CH₂; **X²** is CH₂; and **X³** is CH₂ or CH**R^{L}**.

In some embodiments of Formula (**I-a1**), the 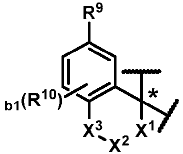 moiety is

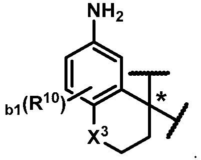

In some embodiments of Formula (**I-a1**), the 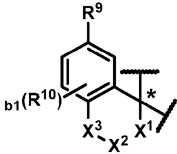 moiety is

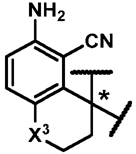

In some embodiments of Formula (**I-a1**), **X³** is CHMe, CHEt, or CHCF₃. For example, **X³** can be CHMe.

In some embodiments of Formula (**I-a1**), **X³** is CH₂.

In some embodiments of Formula **(A)** (e.g., Formula (**I-a1**)), **R¹** is a 7-10 (e.g., 7) membered spirocyclic bicyclic heterocyclyl having one ring nitrogen atom, one ring oxygen atom, and no additional ring heteroatoms, wherein the 7-10 membered spirocyclic bicyclic heterocyclyl is optionally substituted with 1-4 (e.g., 1-2) **R⁷**. In some embodiments, each **R⁷** is independently selected from the group consisting of: -F, -OH, oxo, **-R^{b1}**, and C₁₋₃ alkyl optionally substituted with 1-3 **R^{c}**, wherein the **R^{b1}** is a 5-6 membered heteroaryl optionally substituted with 1-2 **R^{g}**.

In some embodiments of Formula **(A)** (e.g., Formula (**I-a1**)), **R¹** is 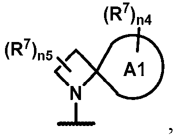 wherein: **Ring A1** is a 4-7 membered heterocyclyl ring having one ring oxygen atom and no additional ring heteroatoms; **n4** is 0, 1, or 2; and **n5** is 0, 1, or 2, provided that **n4** + **n5 is** 0, 1, or 2. In some embodiments, **n4** is 0. In some embodiments, **n5** is 0. In some embodiments **n4** is 0; and **n5** is 0. In some embodiments, each **R⁷** is independently selected from the group consisting of: -F, -OH, oxo, **-R^{b1}**, and C₁₋₃ alkyl optionally substituted with 1-3 **R^{c}**, wherein the **R^{b1}** is a 5-6 membered heteroaryl optionally substituted with 1-2 **R^{g}**.

In some embodiments of Formula **(A)** (e.g., Formula (**I-a1**)), **R¹** is selected from the group consisting of: 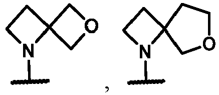 (e.g., 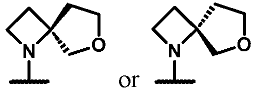 ), and 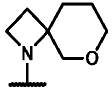 (e.g., 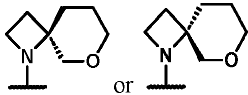 ), each of which is optionally substituted with 1-2 **R⁷**.

In some embodiments of Formula **(A)** (e.g., Formula (**I-a1**)), **R¹** is a 4-membered heterocyclyl optionally substituted with 1-4 **R⁷**. In some embodiments, each **R⁷** is independently selected from the group consisting of: -F, -OH, oxo, **-R^{b1}**, and C₁₋₃ alkyl optionally substituted with 1-3 **R^{c}**, wherein the **R^{b1}** is a 5-6 membered heteroaryl optionally substituted with 1-2 **R^{g}**.

In some embodiments of Formula **(A)** (e.g., Formula (**I-a1**)), **R¹** is 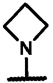 optionally substituted with 1-4 (e.g., 1-2) **R⁷**. In some embodiments, each **R⁷** is independently selected from the group consisting of: -F, oxo, -OH, -**R^{b1}**, and C₁₋₃ alkyl optionally substituted with 1-3 **R^{c}**, wherein the **R^{b1}** is a 5-6 membered heteroaryl optionally substituted with 1-2 **R^{g}**.

In some embodiments of Formula (A) (e.g., Formula (**I-a1**)), **R¹** is selected from the group consisting of: 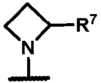 and 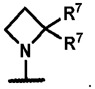 In some embodiments, each **R⁷** is independently selected from the group consisting of:
-F;
-cyano;
-OH;
-**R^{b1}**, wherein the **R^{b1}** is a 5-6 membered heteroaryl optionally substituted with 1-2 **R^{g}**;
C₁₋₃ alkyl optionally substituted with 1-3 F; and
C₁₋₃ alkyl substituted with -OH or C₁₋₃ alkoxy.

In some embodiments of Formula **(A)** (e.g., Formula (**I-a1**)), **R¹** is 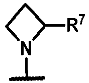 (e.g., 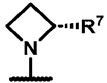 ), wherein **R⁷** is C₁₋₃ alkyl substituted with -OH or C₁₋₃ alkoxy (e.g., C₁₋₃ alkyl substituted with -OH). In some embodiments, **R¹** is 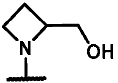 (e.g., 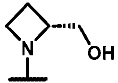 ).

In some embodiments of Formula **(A)** (e.g., Formula (**I-a1**)), **R¹** is 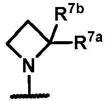 (e.g., 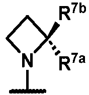 ), wherein **R^{7a}** is C₁₋₃ alkyl substituted with -OH (e.g., -CH₂OH); and **R^{7b}** is selected from the group consisting of: C₁₋₃ alkyl optionally substituted with 1-3 F (e.g., methyl), and C₁₋₃ alkyl substituted with -OH or C₁₋₃ alkoxy. For example, **R¹** can be 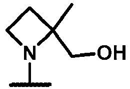 (e.g., 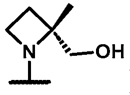 ). For example, **R¹** can be

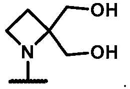

In some embodiments of Formula **(A)** (e.g., Formula (**I-a1**)), **R¹** is 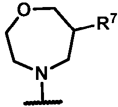 (e.g., 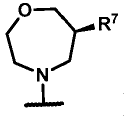 ), wherein **R⁷** is a 5-membered heteroaryl optionally substituted with 1-3 **R^{g}**. In some embodiments, **R⁷** is selected from the group consisting of pyrazolyl and oxazolyl, each of which is optionally substituted with 1-2 **R^{g}**. In some embodiments, **R⁷** is pyrazolyl optionally substituted with 1-2 **R^{g}** (e.g., **R⁷** is 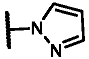 optionally substituted with one **R^{g}**). For example, **R⁷** can be 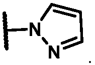 In some embodiments, **R⁷** is oxazolyl optionally substituted with one **R^{g}** (e.g., **R⁷** is 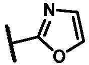 optionally substituted with one **R^{g}**). For example, **R⁷** can be

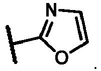

In some embodiments of Formula **(A)** (e.g., Formula (**I-a1**)), **Y²** is -CH₂- or -CD₂- (e.g., -CH₂-).

In some embodiments of Formula (A) (e.g., Formula (**I-a1**)), **Y²** is -CH₂- or -CD₂- (e.g., -CH₂-); and **R³** is 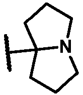 optionally substituted with 1-2 substituents each independently selected from the group consisting of: -F, -C₁₋₃ alkoxy, and -C₁₋₃ haloalkoxy.

In some embodiments of Formula **(A)** (e.g., Formula (**I-a1**)), **Y²** is -CH₂- or -CD₂- (e.g., -CH₂-); and **R³** is a 9-14 (e.g., 9-12) membered heterocyclyl optionally substituted with 1-3 substituents independently selected from the group consisting of: **R^{a}**, **R^{b}**, and 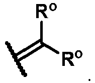 In some embodiments, **R³** is a 9-12 membered heterocyclyl optionally substituted with 1-3 **R^{a}**. In some embodiments, **R³** is a 9-12 membered heterocyclyl optionally substituted with 1-3 substituents independently selected from the group consisting of: -F, C₁₋₃ alkyl, and C₁₋₃ alkoxy. For example, **R³** can be selected from the group consisting of:

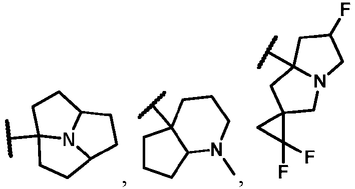

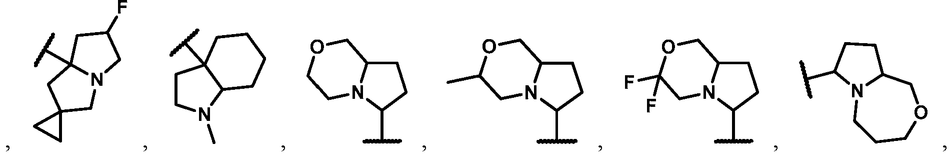

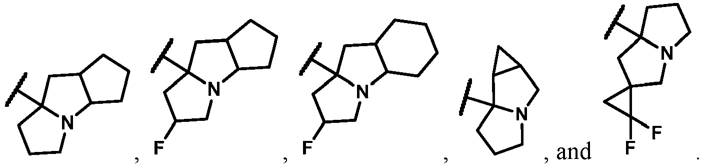

In some embodiments of Formula (A) (e.g., Formula (**I-a1**)), **Y²** is -CH₂- or -CD₂- (e.g., -CH₂-); and **R³** is an 8-12 membered heterocyclyl substituted with 1-2 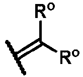 and further optionally substituted with 1-2 independently selected **R^{a}**. In some embodiments, **R³** is selected from the group consisting of: 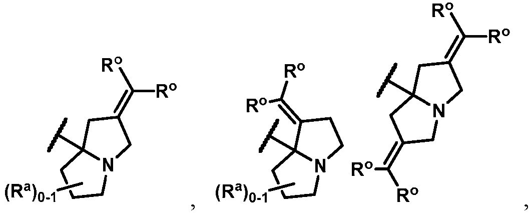 and 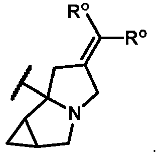 For example, **R³** can be selected from the group consisting of:

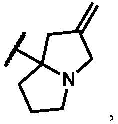

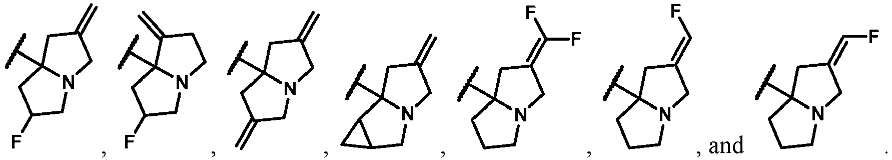

In some embodiments of Formula (A) (e.g., Formula (**I-a1**)), **Y²** is -CH₂- or -CD₂- (e.g., -CH₂-); and **R³** is an 8-12 membered heterocyclyl substituted with **R^{b}** and further optionally substituted with 1-2 substituents independently selected from the group consisting of: **R^{a}** and

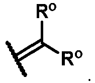

In some embodiments, **R³** is selected from the group consisting of: 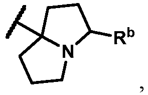 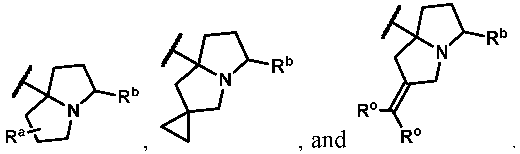 For example, **R³** can be selected from the group consisting of: 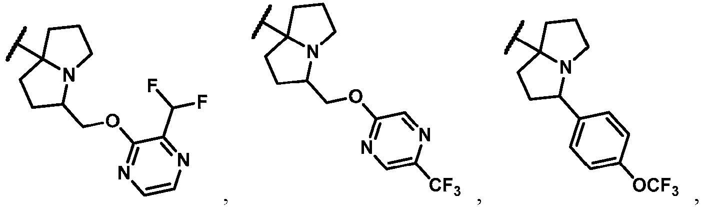 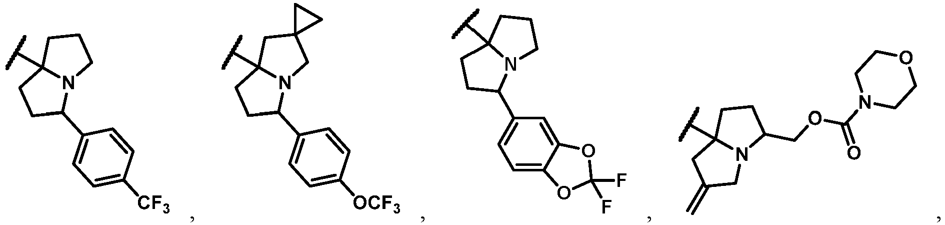 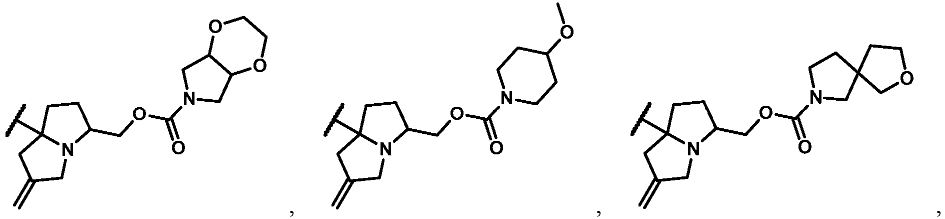 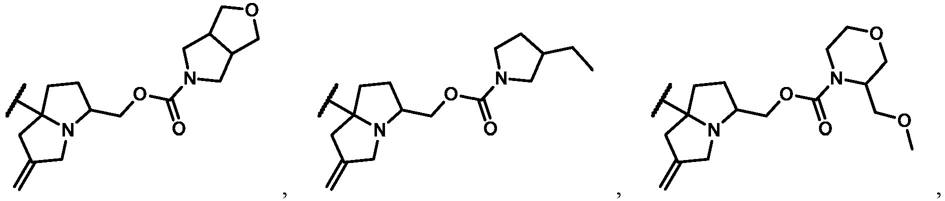 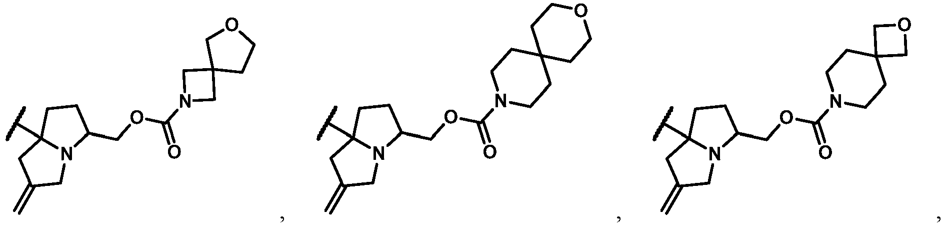 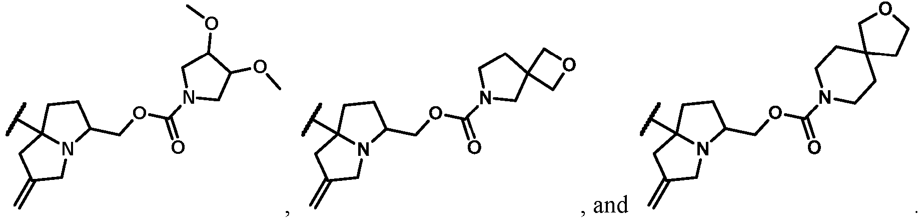 In some embodiments of Formula (**A**) (e.g., Formula (**I-a1**)), **Y²** is -CH₂- or -CD₂- (e.g., -CH₂-); and **R³** is selected from the group consisting of: 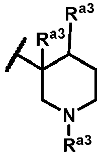 (e.g., 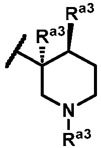 ) or 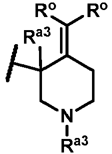 (e.g., 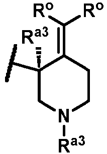 ), wherein each **R^{a3}** is an independently selected C₁₋₃ alkyl optionally substituted with 1-3 F. For example, **R³** can be selected from the group consisting of:

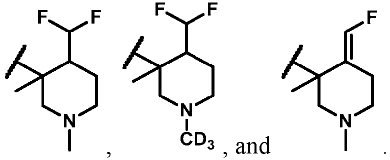

In some embodiments of Formula (A) (e.g., Formula (**I-a1**)), **Y²** is -CH₂- or -CD₂- (e.g., -CH₂-); and **R³** is selected from the group consisting of: 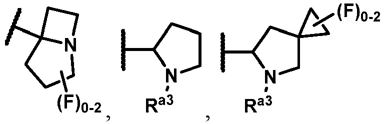 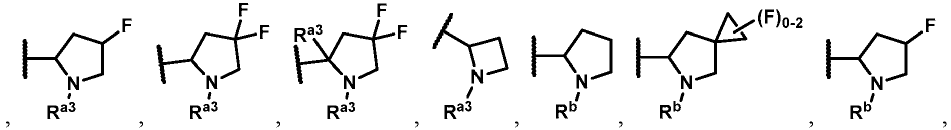 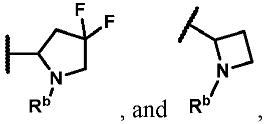 wherein each **R^{a3}** is an independently selected C₁₋₃ alkyl optionally substituted with 1-3 F. For example, **R³** can be selected from the group consisting of:

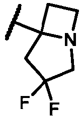

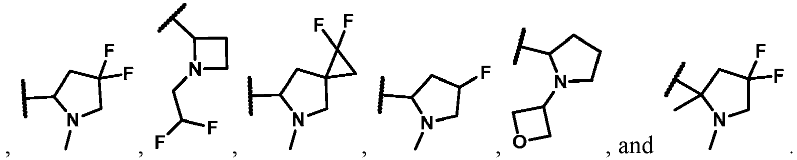

In some embodiments of Formula (A) (e.g., Formula (**I-a1**)), **Y²** is a straight-chain C₃₋₆ alkylene optionally substituted with 1-6 **R^{Y}**. In some embodiments, **Y²** is selected from the group consisting of:

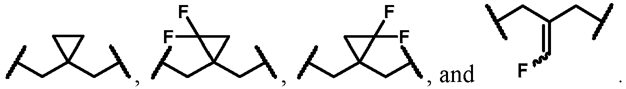

In some embodiments of Formula (A) (e.g., Formula (**I-a1**)), **Y²** is a straight-chain C₃₋₆ alkylene optionally substituted with 1-6 **R^{Y}**; and **R³** is -N**R^{d}R^{e}**. In some embodiments, **Y²** is selected from the group consisting of: 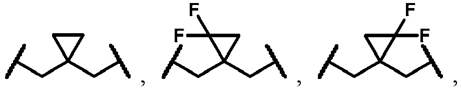 and 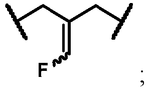 and **R³** is -N(C₁₋₃ alkyl)₂. For example, -O-**Y²-R³** can be:

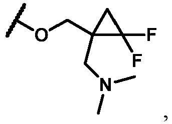

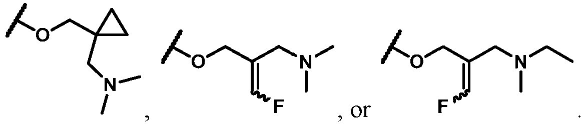

In some embodiments of Formula (**A**) (e.g., Formula (**I-a1**)), **Y²** is a straight-chain C₃₋₆ alkylene optionally substituted with 1-6 **R^{Y}**; and **R³** is a 4-8 membered heterocyclyl optionally substituted with 1-3 substituents independently selected from the group consisting of: **R^{a}**, 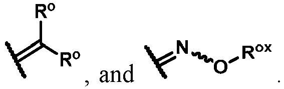 In some embodiments, **Y²** is selected from the group consisting of: 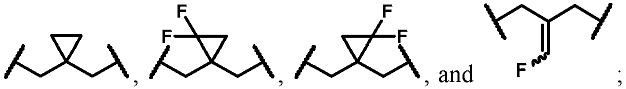 and **R³** is selected from the group consisting of: 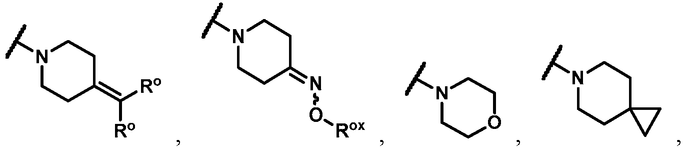 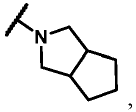 and 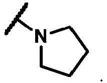 For example, -O-**Y²**-**R³** can be: 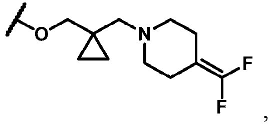 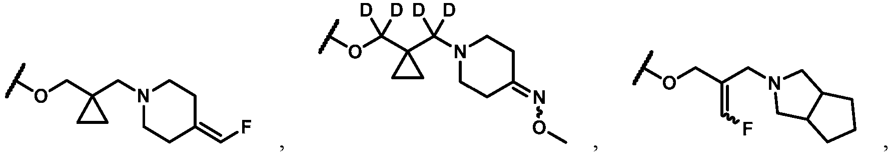 or

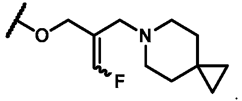

Also provided herein are reference compounds selected from the compounds depicted in **Table C1**, or pharmaceutically acceptable salts thereof.

**Table C1**

| **No.** | **Compound Structure** |
|---|---|
| **R101** | 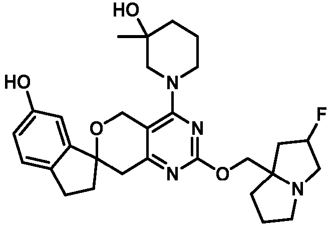 |
| **R101a** | 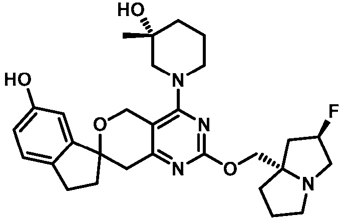 |
| **R101b** | 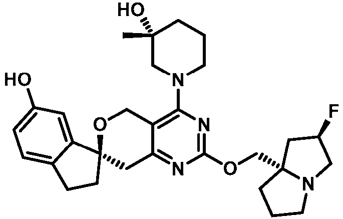 |
| **R101c** | 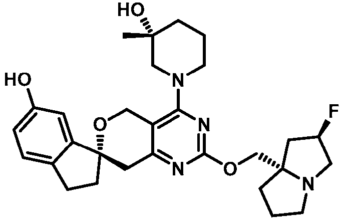 |
| **R102** | 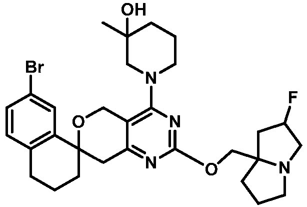 |
| **R102a** | 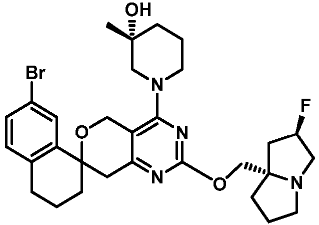 |
| **R105** | 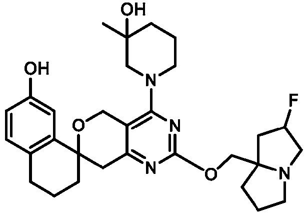 |
| **R105a** | 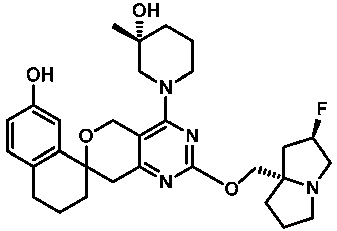 |
| **R105b** | 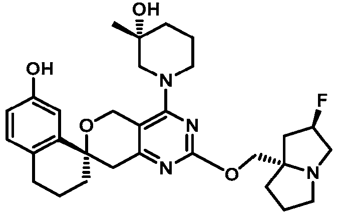 |
| **R105c** | 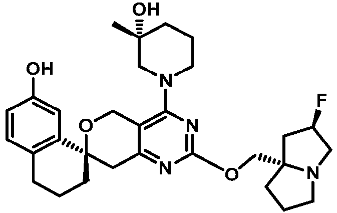 |
| **R113** | 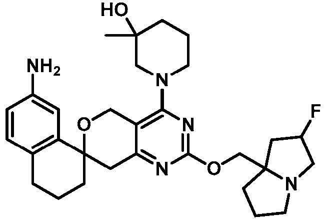 |
| **R113a** | 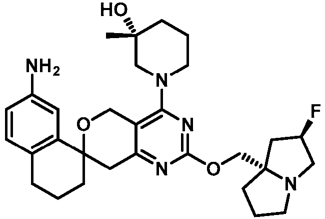 |
| **R113b** | 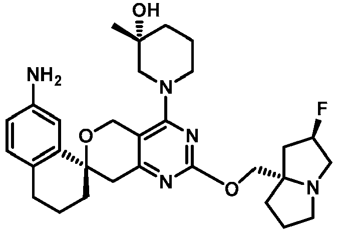 |
| **R113c** | 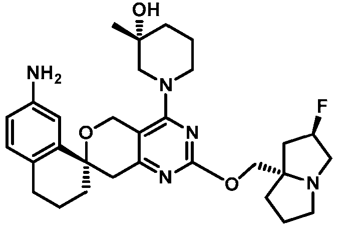 |
| **R114** | 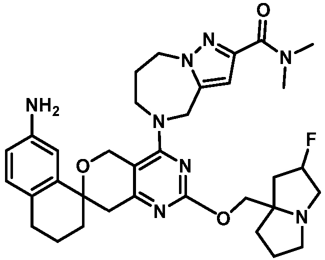 |
| **R114a** | 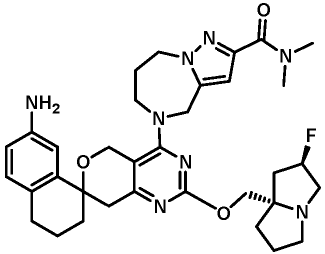 |
| **R114b** | 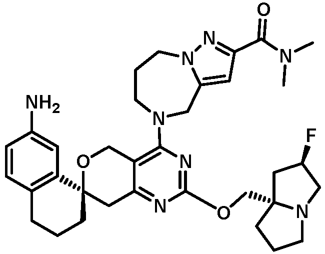 |
| **R114c** | 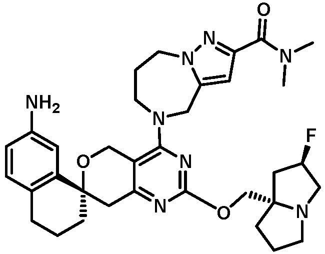 |
| **R115** | 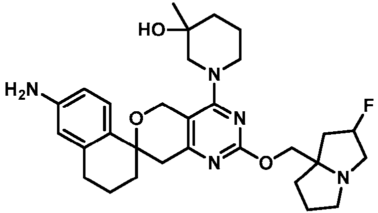 |
| **R115a** | 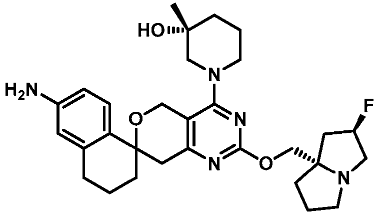 |
| **R116** | 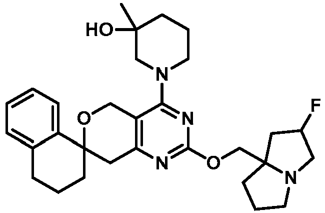 |
| **R116a** | 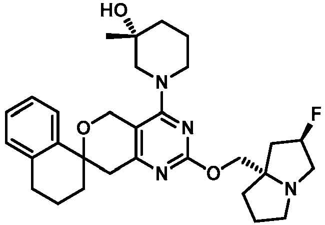 |
| **R116b** | 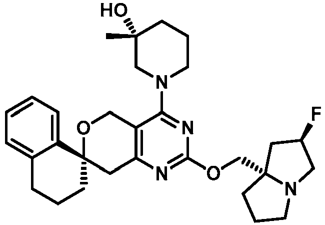 |
| **R120** | 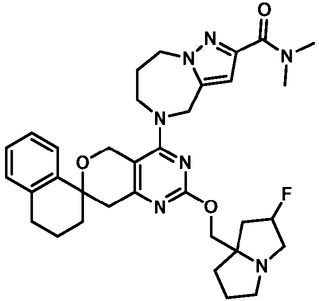 |
| **R120a** | 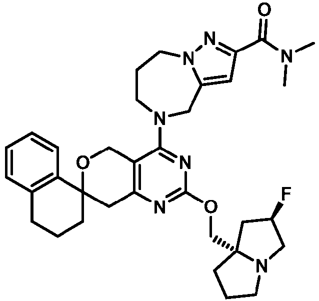 |
| **R120b** | 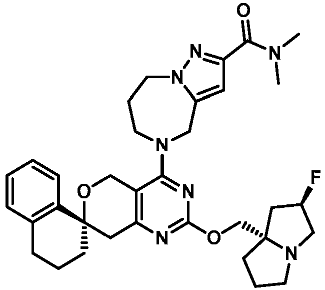 |
| **R124** | 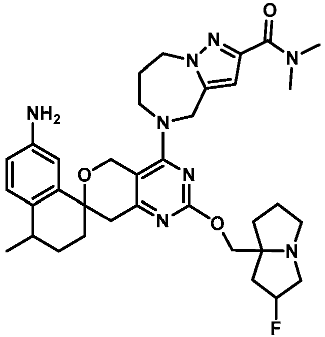 |
| **R124a** | 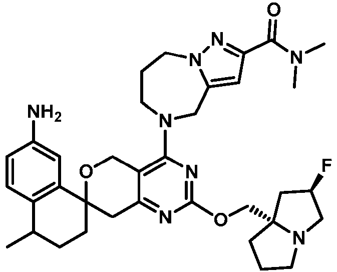 |
| **R124b** | 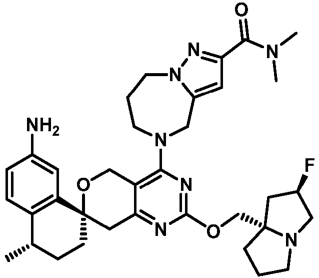 |
| **R124c** | 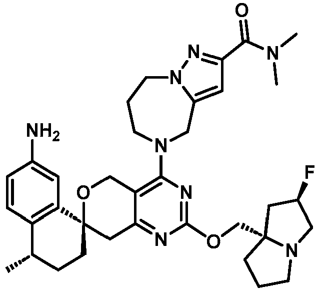 |
| **R124d** | 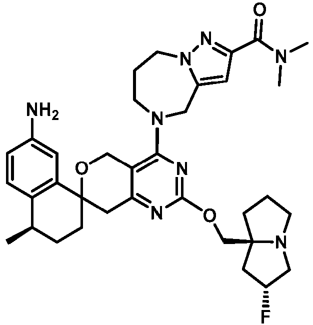 |
| **R124e** | 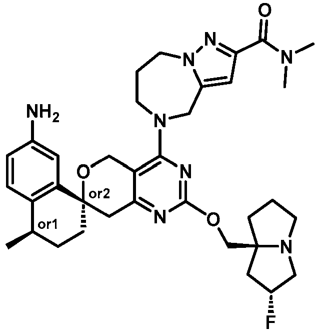 |
| **R124f** | 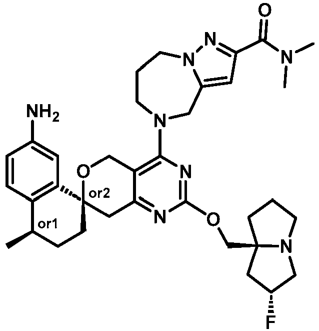 |
| **R125** | 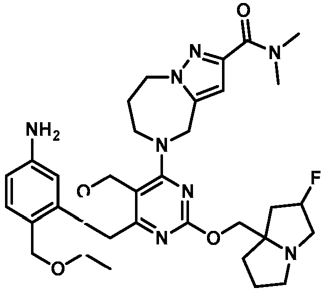 |
| **R125a** | 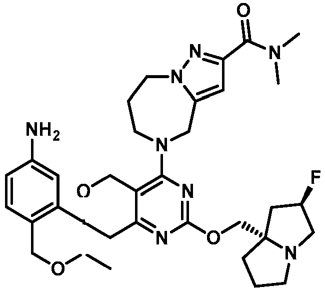 |
| **R127** | 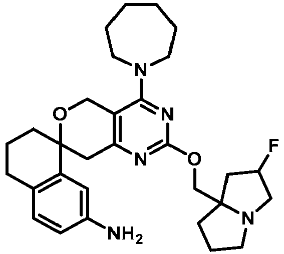 |
| **R127a** | 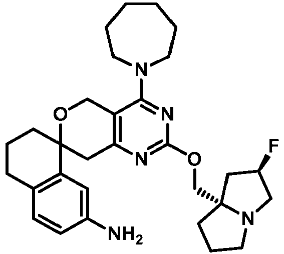 |
| **R128** | 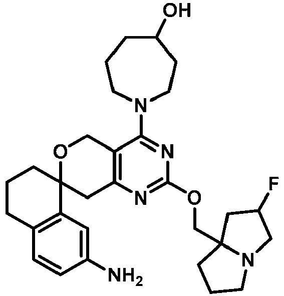 |
| **R128a** | 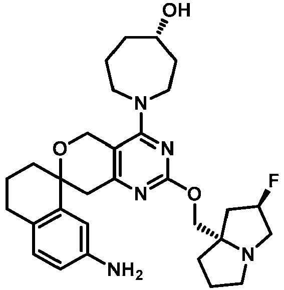 |
| **R128b** | 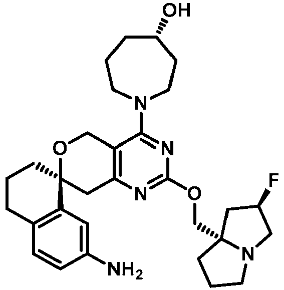 |
| **R130** | 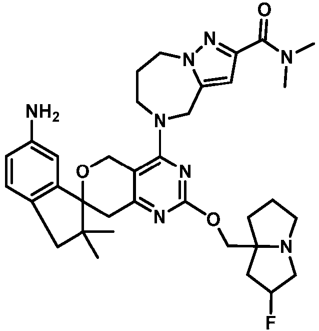 |
| **R130a** | 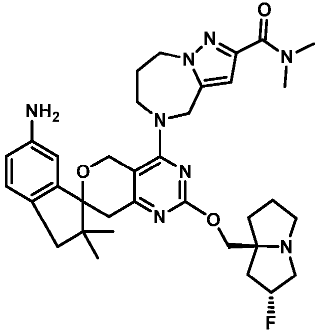 |
| **R131** | 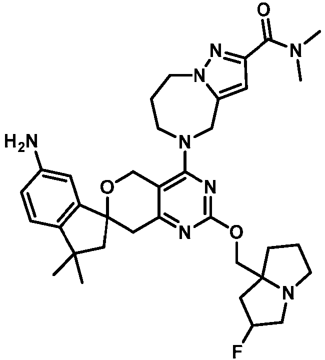 |
| **R131a** | 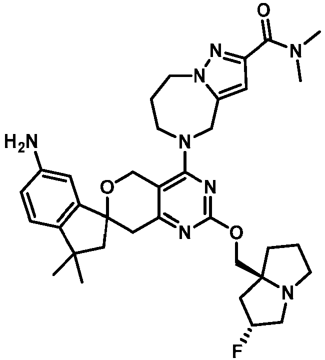 |
| **R133** | 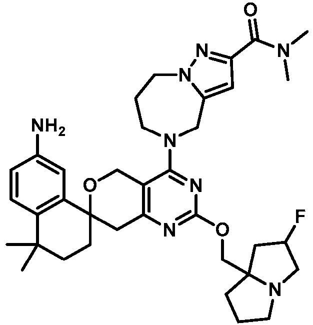 |
| **R133a** | 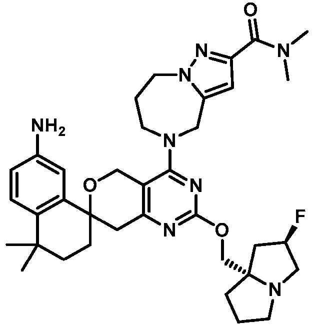 |
| **R133b** | 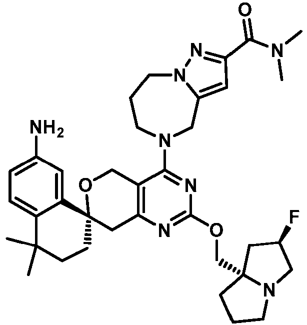 |
| **R134** | 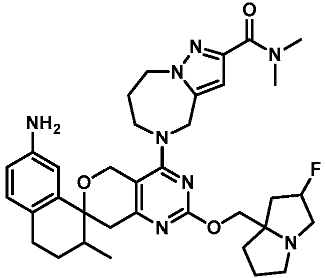 |
| **R134a** | 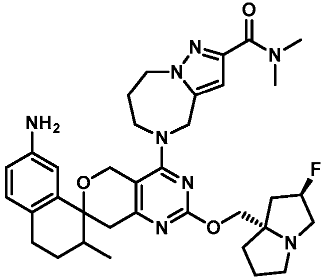 |
| **R136** | 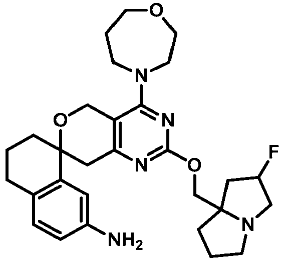 |
| **R136a** | 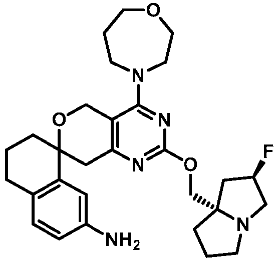 |
| **R136b** | 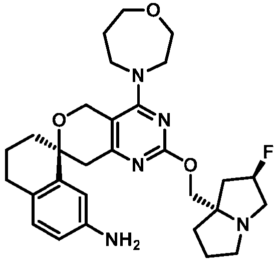 |
| **R138** | 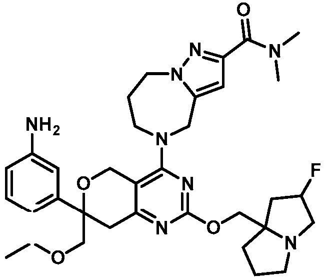 |
| **R138a** | 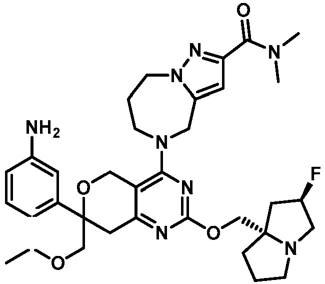 |
| **R139** | 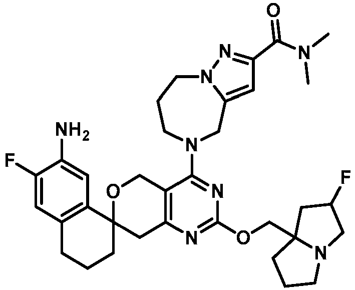 |
| **R139a** | 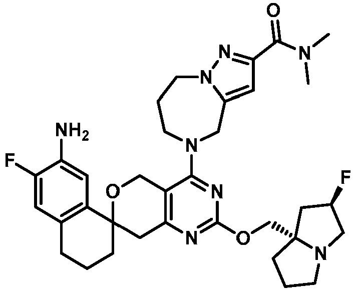 |
| **R139b** | 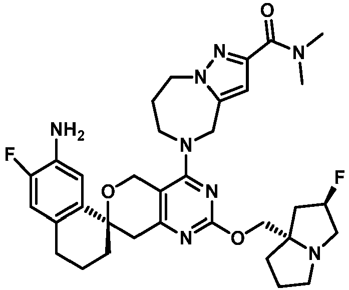 |
| **R139c** | 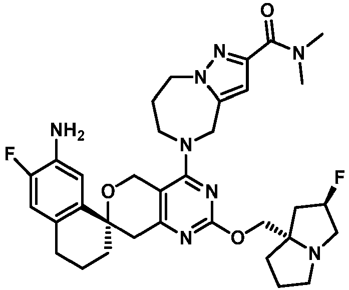 |
| **R148** | 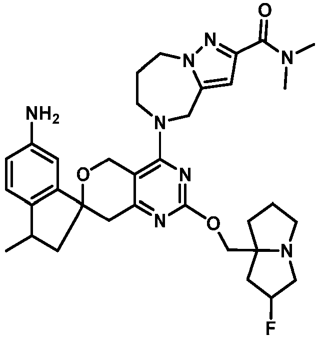 |
| **R148a** | 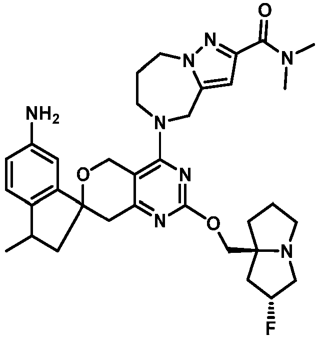 |
| **R149** | 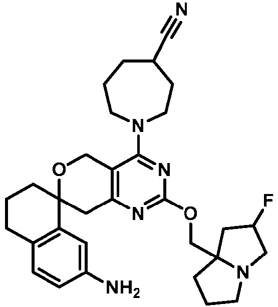 |
| **R149a** | 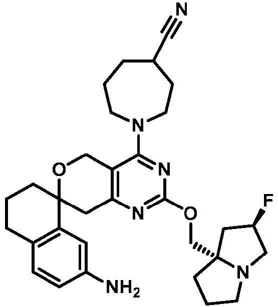 |
| **R149b** | 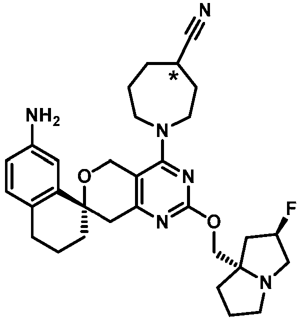 |
| **R149c** | 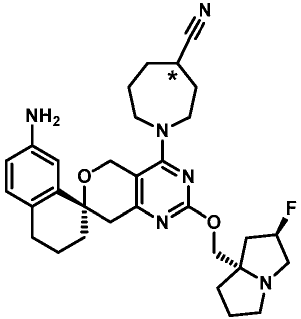 |
| **R158** | 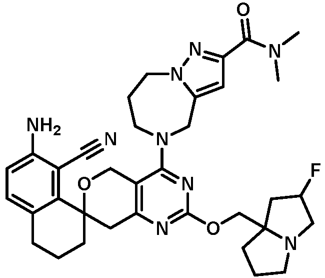 |
| **R158a** | 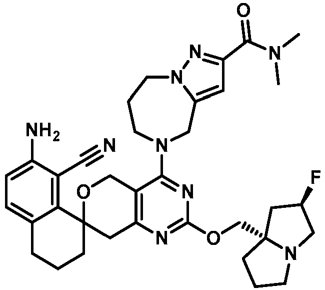 |
| **R158b** | 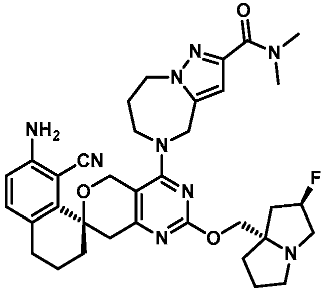 |
| **R158c** | 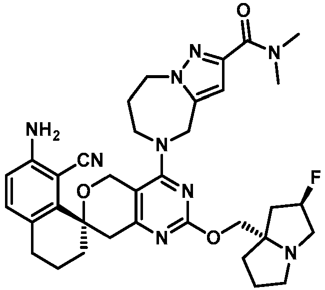 |
| **R160** | 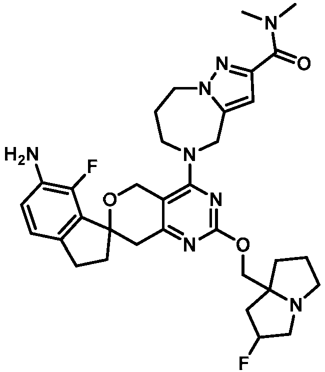 |
| **R160a** | 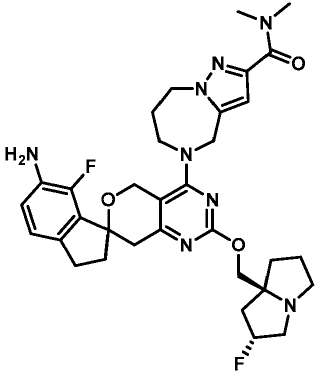 |
| **R161** | 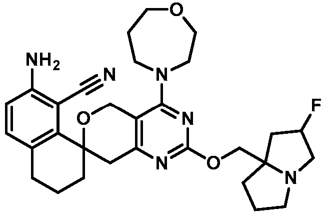 |
| **R161a** | 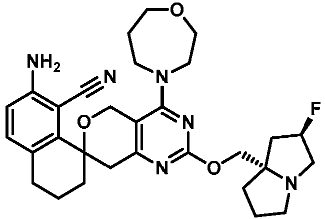 |
| **R161b** | 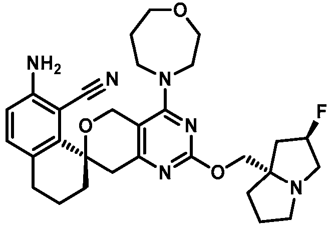 |
| **R161c** | 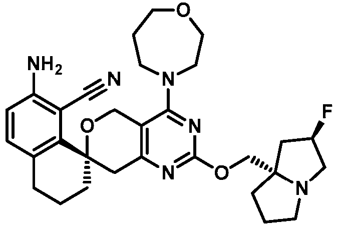 |
| **R162** | 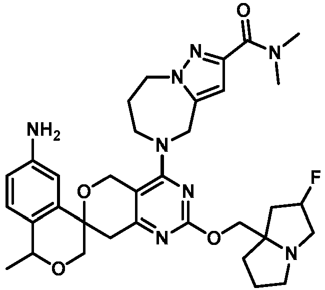 |
| **R162a** | 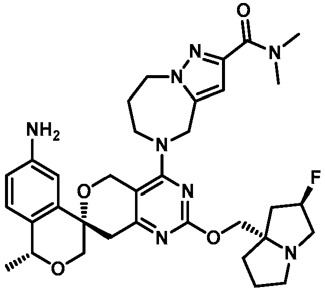 |
| **R163** | 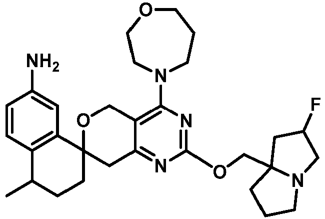 |
| **R163a** | 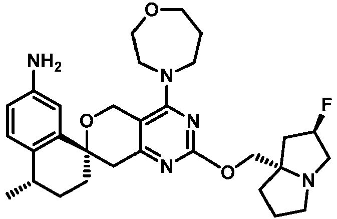 |
| **R164** | 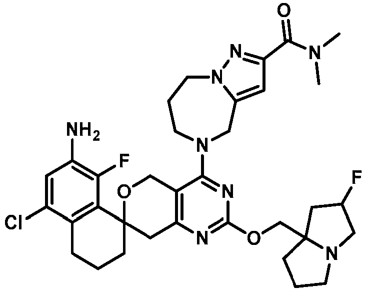 |
| **R164a** | 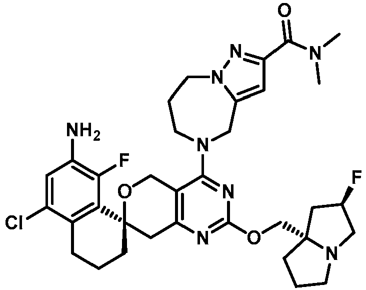 |
| **R164b** | 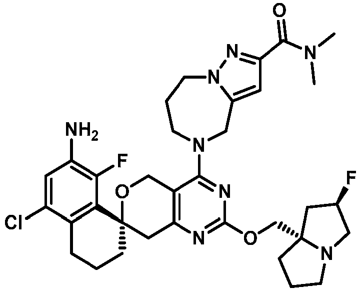 |
| **R165** | 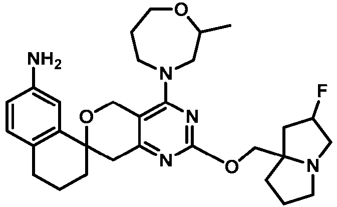 |
| **R165a** | 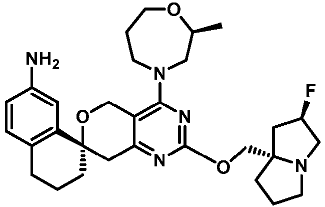 |
| **R165b** | 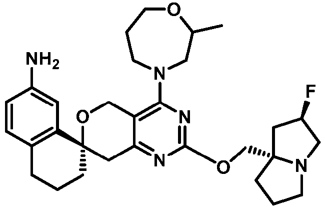 |
| **R166** | 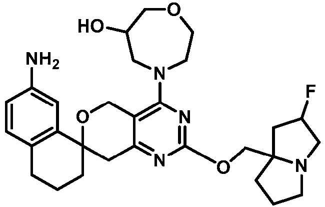 |
| **R166a** | 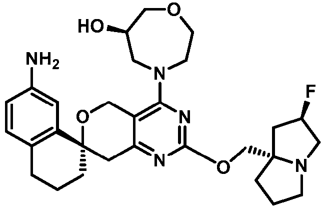 |
| **R170** | 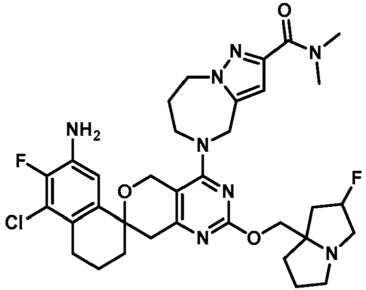 |
| **R170a** | 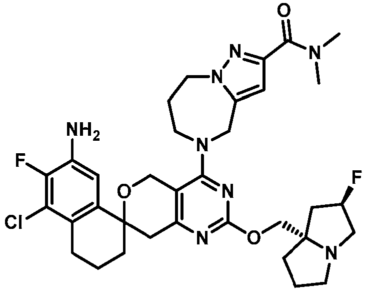 |
| **R171** | 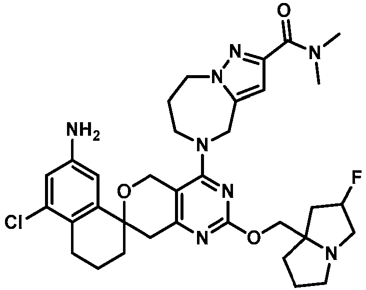 |
| **R171a** | 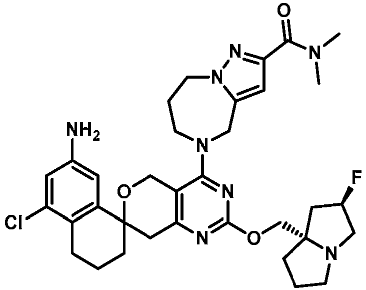 |
| **R172** | 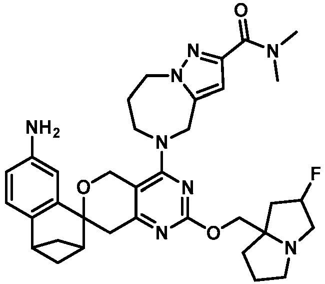 |
| **R172a** | 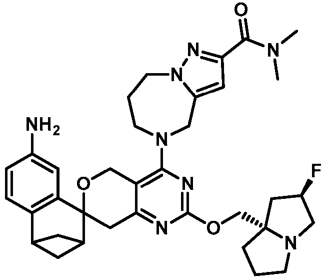 |
| **R173** | 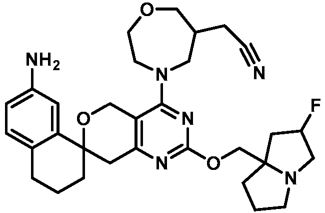 |
| **R173a** | 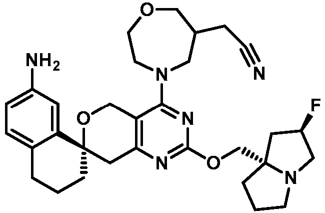 |
| **R174** | 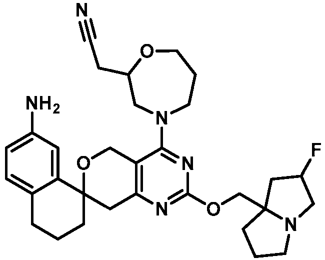 |
| **R174a** | 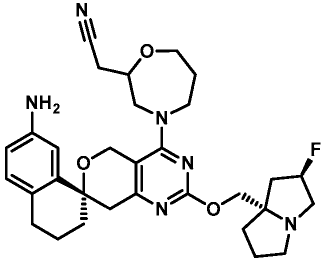 |
| **R175** | 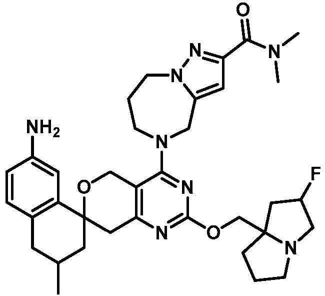 |
| **R175a** | 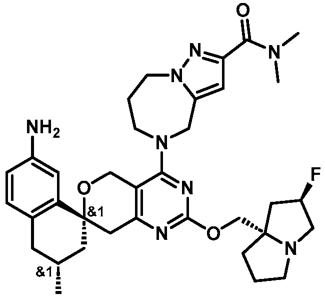 |
| **R176** | 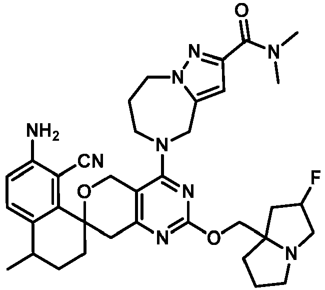 |
| **R176a** | 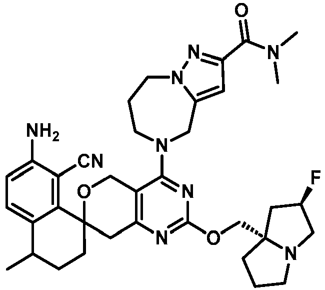 |
| **R176b** | 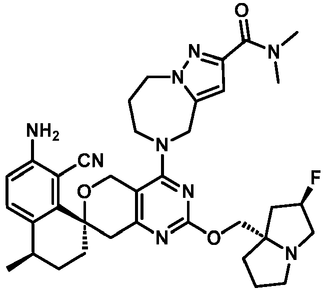 |
| **R176c** | 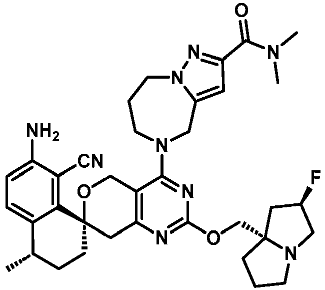 |
| **R176d** | 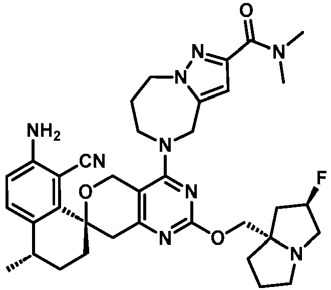 |
| **R176e** | 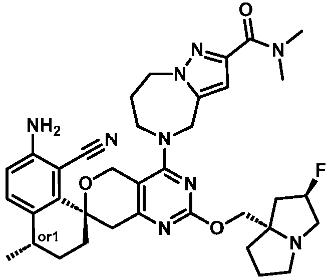 |
| **R177** | 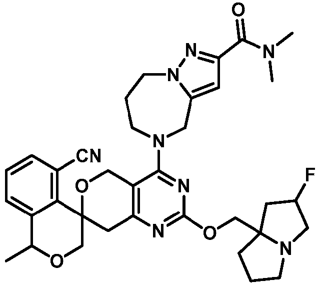 |
| **R177a** | 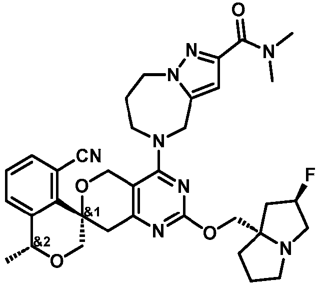 |
| **R178** | 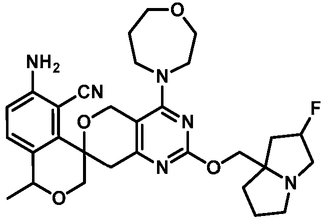 |
| **R178a** | 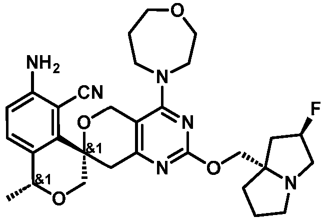 |
| **R178b** | 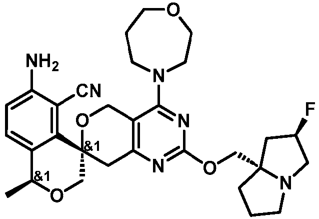 |
| **R179** | 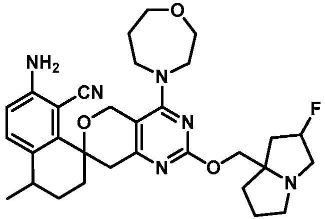 |
| **R179a** | 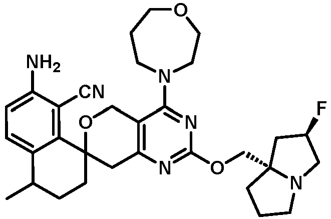 |
| **R179b** | 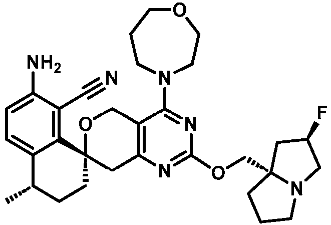 |
| **R179d** | 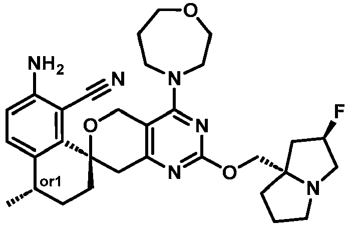 |
| **R179e** | 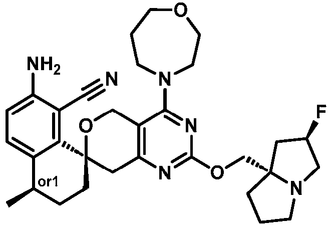 |
| **R179f** | 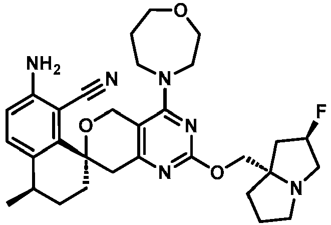 |
| **R180** | 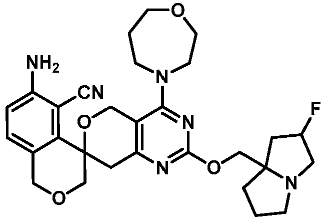 |
| **R180a** | 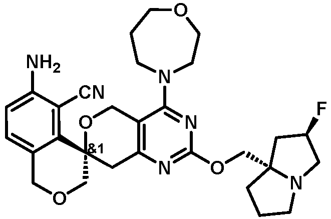 |
| **R181** | 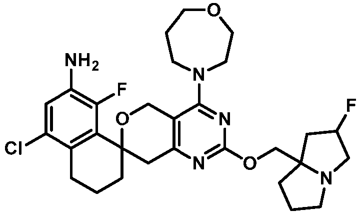 |
| **R181a** | 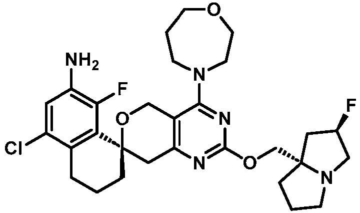 |
| **R181b** | 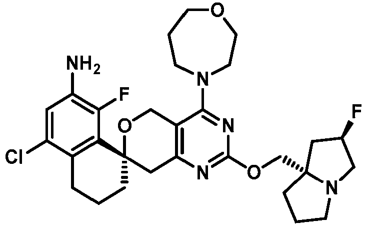 |
| **R184** | 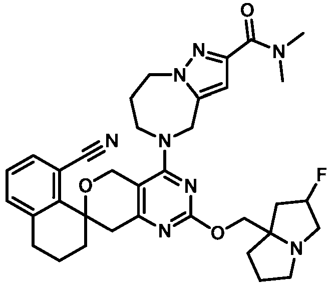 |
| **R184a** | 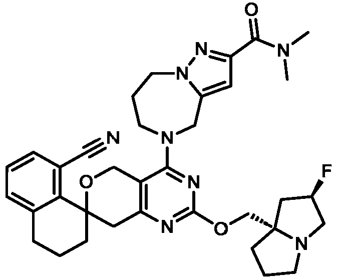 |
| **R184b** | 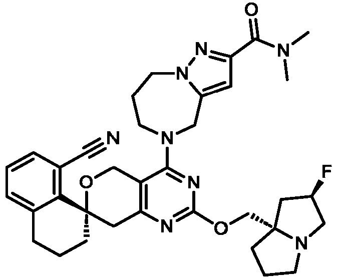 |
| **R184c** | 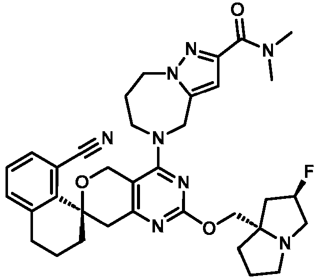 |
| **R185** | 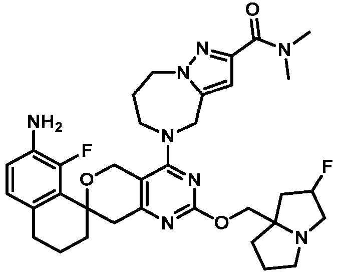 |
| **R185a** | 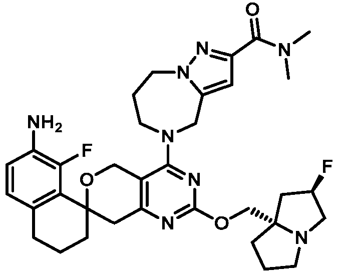 |
| **R194** | 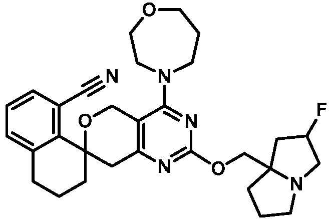 |
| **R194a** | 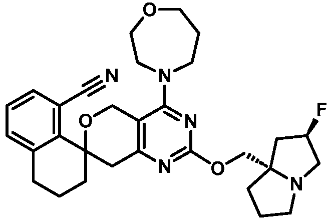 |
| **R183** | 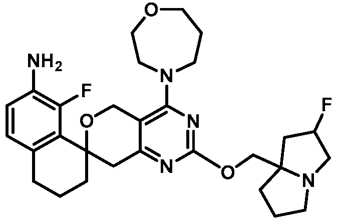 |
| **R183a** | 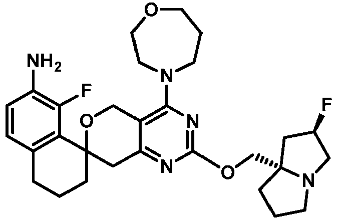 |

In some embodiments, the compounds of Formula (**II**), (**III**), (**IV**), (**V**), (**VI**), or (**A**) are other than compounds depicted in **Table C1**, or pharmaceutically acceptable salts thereof.

In some embodiments, the compounds of Formula (**II**), (**III**), (**IV**), (**V**), (**VI**), or (**A**) are other than compounds depicted in **Table C1** of International Patent Application PCT/US2023/080513 (published as WO 2024/112654), or pharmaceutically acceptable salts thereof, wherein the **Table C1** of International Patent Application PCT/US2023/080513 is incorporated herein by reference in its entirety.

### Chemical definitions

The term "halo" refers to fluoro (F), chloro (Cl), bromo (Br), or iodo (I).

The term "oxo" refers to a divalent doubly bonded oxygen atom (i.e., "=O"). As used herein, oxo groups are attached to carbon atoms to form carbonyls.

The term "alkyl" refers to a saturated acyclic hydrocarbon radical that may be a straight chain or branched chain, containing the indicated number of carbon atoms. For example, C₁₋₁₀ indicates that the group may have from 1 to 10 (inclusive) carbon atoms in it. Alkyl groups can either be unsubstituted or substituted with one or more substituents. Non-limiting examples include methyl, ethyl, *iso*-propyl, *tert*-butyl, *n*-hexyl. The term "saturated" as used in this context means only single bonds present between constituent carbon atoms and other available valences occupied by hydrogen and/or other substituents as defined herein.

The term "haloalkyl" refers to an alkyl, in which one or more hydrogen atoms is/are replaced with an independently selected halo (e.g., -CF₃, -CHF₂, or -CH₂F).

The term "alkoxy" refers to an -O-alkyl radical (e.g., -OCH₃). The term "haloalkoxy" refers to an -O-haloalkyl radical (e.g., -OCF₃, -OCHF₂, or -OCH₂F).

The term "alkylene" refers to a divalent alkyl (e.g., -CH₂-). Similarly, terms such as "cycloalkylene" and "heterocyclylene" refer to divalent cycloalkyl and heterocyclyl respectively. For avoidance of doubt, in "cycloalkylene" and "heterocyclylene", the two radicals can be on the same ring carbon atom (e.g., a geminal diradical such as 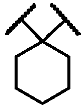 or 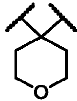 ) or on different ring atoms (e.g., ring carbon and/or nitrogen atoms (e.g., vicinal ring carbon and/or nitrogen atoms)) (e.g., 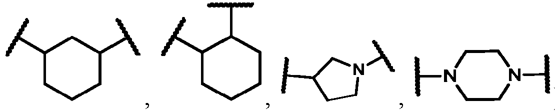 ).

The term "alkenyl" refers to an acyclic hydrocarbon chain that may be a straight chain or branched chain having one or more carbon-carbon double bonds. The alkenyl moiety contains the indicated number of carbon atoms. For example, C₂₋₆ indicates that the group may have from 2 to 6 (inclusive) carbon atoms in it. Alkenyl groups can either be unsubstituted or substituted with one or more substituents.

The term "alkynyl" refers to an acyclic hydrocarbon chain that may be a straight chain or branched chain having one or more carbon-carbon triple bonds. The alkynyl moiety contains the indicated number of carbon atoms. For example, C₂₋₆ indicates that the group may have from 2 to 6 (inclusive) carbon atoms in it. Alkynyl groups can either be unsubstituted or substituted with one or more substituents.

The term "aryl" refers to a 6-20 carbon mono-, bi-, tri- or polycyclic group wherein at least one ring in the system is aromatic (e.g., 6-carbon monocyclic, 10-carbon bicyclic, or 14-carbon tricyclic aromatic ring system); and wherein 0, 1, 2, 3, or 4 atoms of each ring may be substituted by a substituent. Examples of aryl groups include phenyl, naphthyl, tetrahydronaphthyl, and the like.

The term "cycloalkyl" as used herein refers to mono-, bi-, tri-, or polycyclic saturated or partially unsaturated hydrocarbon groups having, e.g., 3 to 20 ring carbons, preferably 3 to 15 ring carbons, and more preferably 3 to 12 ring carbons or 3 to 10 ring carbons or 3 to 6 ring carbons, wherein the cycloalkyl group may be optionally substituted. The term "saturated" as used in this context means only single bonds present between constituent carbon atoms. Examples of saturated cycloalkyl groups include, without limitation, cyclopropyl, cyclobutyl, cyclopentyl, cyclohexyl, cycloheptyl, and cyclooctyl. Partially unsaturated cycloalkyl may have any degree of unsaturation provided that one or more double bonds is present in the cycloalkyl, none of the rings in the ring system are aromatic, and the partially unsaturated cycloalkyl group is not fully saturated overall. Examples of partially unsaturated cycloalkyl include, without limitation, cyclopentenyl, cyclohexenyl, cycloheptenyl, and cyclooctenyl. Cycloalkyl may include multiple fused and/or bridged rings. Non-limiting examples of fused/bridged cycloalkyl includes: bicyclo[1.1.0]butyl, bicyclo[2.1.0]pentyl, bicyclo[1.1.1]pentyl, bicyclo[3.1.0]hexyl, bicyclo[2.1.1]hexyl, bicyclo[3.2.0]heptyl, bicyclo[4.1.0]heptyl, bicyclo[2.2.1]heptyl, bicyclo[3.1.1]heptyl, bicyclo[4.2.0]octyl, bicyclo[3.2.1]octyl, bicyclo[2.2.2]octyl, and the like. Cycloalkyl also includes spirocyclic rings (e.g., spirocyclic bicycle wherein two rings are connected through just one atom). Non-limiting examples of spirocyclic cycloalkyls include spiro[2.2]pentyl, spiro[2.5]octyl, spiro[3.5]nonyl, spiro[3.5]nonyl, spiro[3.5]nonyl, spiro[4.4]nonyl, spiro[2.6]nonyl, spiro[4.5]decyl, spiro[3.6]decyl, spiro[5.5]undecyl, and the like.

The term "heteroaryl", as used herein, means a mono-, bi-, tri- or polycyclic group having 5 to 20 ring atoms, alternatively 5, 6, 9, 10, or 15 ring atoms; wherein at least one ring in the system contains one or more heteroatoms independently selected from the group consisting of N, O, S (inclusive of oxidized forms such as: 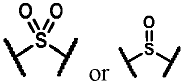 ), and P (inclusive of oxidized forms such as: 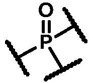 ) (e.g., N, O, and S (inclusive of oxidized forms such as: 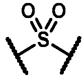 or 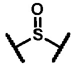 )) and at least one ring in the system is aromatic (but does not have to be a ring which contains a heteroatom, e.g. tetrahydroisoquinolinyl, e.g., tetrahydroquinolinyl). Heteroaryl groups can either be unsubstituted or substituted with one or more substituents. Examples of heteroaryl include thienyl, pyridinyl, furyl, oxazolyl, oxadiazolyl, pyrrolyl, imidazolyl, triazolyl, thiodiazolyl, pyrazolyl, isoxazolyl, thiadiazolyl, pyranyl, pyrazinyl, pyrimidinyl, pyridazinyl, triazinyl, thiazolyl benzothienyl, benzoxadiazolyl, benzofuranyl, benzimidazolyl, benzotriazolyl, cinnolinyl, indazolyl, indolyl, isoquinolinyl, isothiazolyl, naphthyridinyl, purinyl, thienopyridinyl, pyrido[2,3-*d*]pyrimidinyl, pyrrolo[2,3-*b*]pyridinyl, quinazolinyl, quinolinyl, thieno[2,3-*c*]pyridinyl, pyrazolo[3,4-*b*]pyridinyl, pyrazolo[3,4-*c*]pyridinyl, pyrazolo[4,3-*c*]pyridinyl, pyrazolo[4,3-*b*]pyridinyl, tetrazolyl, chromanyl, 2,3-dihydrobenzo[*b*][1,4]dioxinyl, benzo[*d*][1,3]dioxolyl, 2,3-dihydrobenzofuranyl, tetrahydroquinolinyl, 2,3-dihydrobenzo[*b*][1,4]oxathiinyl, isoindolinyl, and others. In some embodiments, the heteroaryl is selected from thienyl, pyridinyl, furyl, pyrazolyl, imidazolyl, isoindolinyl, pyranyl, pyrazinyl, and pyrimidinyl. For purposes of clarification, heteroaryl also includes aromatic lactams, aromatic cyclic ureas, or vinylogous analogs thereof, in which each ring nitrogen adjacent to a carbonyl is tertiary (i.e., all three valences are occupied by non-hydrogen substituents), such as one or more of pyridonyl (e.g., 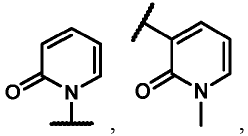 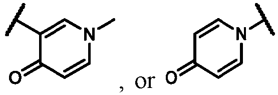 ), pyrimidonyl (e.g., 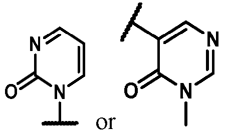 ), pyridazinonyl (e.g., 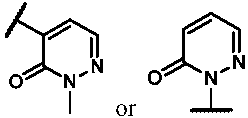 ), pyrazinonyl (e.g., 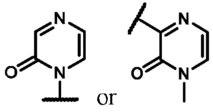 ), and imidazolonyl (e.g., 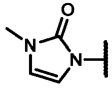 ), wherein each ring nitrogen adjacent to a carbonyl is tertiary (i.e., the oxo group (i.e., "=O") herein is a constituent part of the heteroaryl ring).

The term "heterocyclyl" refers to a mono-, bi-, tri-, or polycyclic saturated or partially unsaturated ring system with 3-15 ring atoms (e.g., 4-15 membered, 4-10 membered, 5-8 membered monocyclic, 8-12 membered bicyclic, or 11-15 membered tricyclic ring system) having 1-3 heteroatoms if monocyclic, 1-6 heteroatoms if bicyclic, or 1-9 heteroatoms if tricyclic or polycyclic, said heteroatoms selected from O, N, S (inclusive of oxidized forms such as: 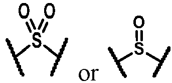 ), and P (inclusive of oxidized forms such as: 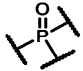 ) (e.g., N, O, and S (inclusive of oxidized forms such as: 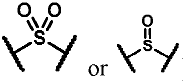 )) (e.g., carbon atoms and 1-3, 1-6, or 1-9 heteroatoms of N, O, S, or P if monocyclic, bicyclic, or tricyclic, respectively), wherein 0, 1, 2 or 3 atoms of each ring may be substituted by a substituent. The term "saturated" as used in this context means only single bonds present between constituent ring atoms and other available valences occupied by hydrogen and/or other substituents as defined herein. Examples of saturated heterocyclyl groups include azetidinyl, piperidinyl, piperazinyl, pyrrolidinyl, dioxanyl, morpholinyl, tetrahydrofuranyl, and the like. Partially unsaturated heterocyclyl groups may have any degree of unsaturation provided that one or more double bonds is present in the heterocyclyl, none of the rings in the ring system are aromatic, and the partially unsaturated heterocyclyl group is not fully saturated overall. Examples of partially unsaturated heterocyclyl groups include, without limitation, tetrahydropyridyl, dihydropyrazinyl, dihydropyridyl, dihydropyrrolyl, dihydrofuranyl, dihydrothiophenyl.

Heterocyclyl may include multiple fused and bridged rings. Non-limiting examples of fused/bridged heteorocyclyl includes: 2-azabicyclo[1.1.0]butyl, 2-azabicyclo[2.1.0]pentyl, 2-azabicyclo[1.1.1]pentyl, 3-azabicyclo[3.1.0]hexyl, 5-azabicyclo[2.1.1]hexyl, 3-azabicyclo[3.2.0]heptyl, octahydrocyclopenta[c]pyrrole, 3-azabicyclo[4.1.0]heptyl, 7-azabicyclo[2.2.1]heptyl, 6-azabicyclo[3.1.1]heptyl, 7-azabicyclo[4.2.0]octyl, 2-azabicyclo[2.2.2]octyl, 3-azabicyclo[3.2.1]octyl, 2-oxabicyclo[1.1.0]butyl, 2-oxabicyclo[2.1.0]pentyl, 2-oxabicyclo[1.1.1]pentyl, 3-oxabicyclo[3.1.0]hexyl, 5-oxabicyclo[2.1.1]hexyl, 3-oxabicyclo[3.2.0]heptyl, 3-oxabicyclo[4.1.0]heptyl, 7-oxabicyclo[2.2.1]heptyl, 6-oxabicyclo[3.1.1]heptyl, 7-oxabicyclo[4.2.0]octyl, 2-oxabicyclo[2.2.2]octyl, 3-oxabicyclo[3.2.1]octyl, and the like. Heterocyclyl also includes spirocyclic rings (e.g., spirocyclic bicycle wherein two rings are connected through just one atom). Non-limiting examples of spirocyclic heterocyclyl include 2-azaspiro[2.2]pentyl, 4-azaspiro[2.5]octyl, 1-azaspiro[3.5]nonyl, 2-azaspiro[3.5]nonyl, 7-azaspiro[3.5]nonyl, 2-azaspiro[4.4]nonyl, 6-azaspiro[2.6]nonyl, 1,7-diazaspiro[4.5]decyl, 7-azaspiro[4.5]decyl 2,5-diazaspiro[3.6]decyl, 3-azaspiro[5.5]undecyl, 2-oxaspiro[2.2]pentyl, 4-oxaspiro[2.5]octyl, 1-oxaspiro[3.5]nonyl, 2-oxaspiro[3.5]nonyl, 7-oxaspiro[3.5]nonyl, 2-oxaspiro[4.4]nonyl, 6-oxaspiro[2.6]nonyl, 1,7-dioxaspiro[4.5]decyl, 2,5-dioxaspiro[3.6]decyl, 1-oxaspiro[5.5]undecyl, 3-oxaspiro[5.5]undecyl, 3-oxa-9-azaspiro[5.5]undecyl and the like.

As used herein, when a ring is described as being "partially unsaturated", it means said ring has one or more additional degrees of unsaturation (in addition to the degree of unsaturation attributed to the ring itself; e.g., one or more double or triple bonds between constituent ring atoms), provided that the ring is not aromatic. Examples of such rings include: cyclopentene, cyclohexene, cycloheptene, dihydropyridine, tetrahydropyridine, dihydropyrrole, dihydrofuran, dihydrothiophene, and the like.

For the avoidance of doubt, and unless otherwise specified, for rings and cyclic groups (e.g., aryl, heteroaryl, heterocyclyl, cycloalkyl, and the like described herein) containing a sufficient number of ring atoms to form bicyclic or higher order ring systems (e.g., tricyclic, polycyclic ring systems), it is understood that such rings and cyclic groups encompass those having fused rings, including those in which the points of fusion are located (i) on adjacent ring atoms (e.g., [x.x.0] ring systems, in which 0 represents a zero atom bridge (e.g., 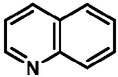 )); (ii) a single ring atom (spiro-fused ring systems) (e.g., 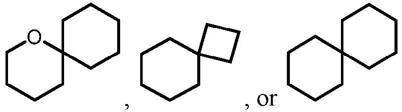 ), or (iii) a contiguous array of ring atoms (bridged ring systems having all bridge lengths > 0) (e.g., 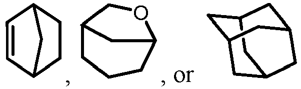 ).

In addition, atoms making up the compounds of the present embodiments are intended to include all isotopic forms of such atoms. Isotopes, as used herein, include those atoms having the same atomic number but different mass numbers. By way of general example and without limitation, isotopes of hydrogen include tritium and deuterium, and isotopes of carbon include ¹³C and ¹⁴C.

In addition, the compounds generically or specifically disclosed herein are intended to include all tautomeric forms. Thus, by way of example, a compound containing the moiety: 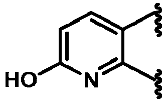 encompasses the tautomeric form containing the moiety: 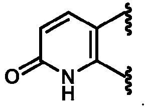 Similarly, a pyridinyl or pyrimidinyl moiety that is described to be optionally substituted with hydroxyl encompasses pyridone or pyrimidone tautomeric forms.

The compounds provided herein may encompass various stereochemical forms. The compounds also encompass diastereomers as well as optical isomers, e.g., mixtures of enantiomers including racemic mixtures, as well as individual enantiomers and diastereomers, which arise as a consequence of structural asymmetry in certain compounds. Unless otherwise indicated, when a disclosed compound is named or depicted by a structure without specifying the stereochemistry and has one or more chiral centers, it is understood to represent all possible stereoisomers of the compound.

Certain combinations of heteroatoms (e.g., N, O, S, or halo) define compounds which are less stable under physiological conditions. Examples include (**1**) compounds containing acetal or aminal linkages; (**2**) compounds containing acyclic N-O, N-N, or N-S(O)₀ bonds; and (**3**) compounds containing O-O, O-S(O)₀₋₂, N-halo, O-halo, and S(O)₀₋₂-halo bonds. Accordingly, such compounds are less preferred. As used herein, "acyclic bonds" mean chemical bonds that are not part of a ring. Examples include the N-O bond in 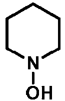 and 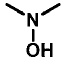 . For avoidance of doubt, acyclic N-O, N-N, or N-S(O)₀ bonds (i.e., those bonds that are not part of a ring (e.g., in 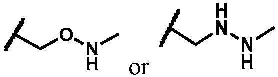 )) are less preferred, but compounds provided herein can include N-O, N-N, or N-S(O)₀ bonds that form part of a ring (e.g., the N-N bond in 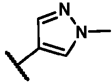 ).

### Methods of Treatment

### Indications

Provided herein are methods for inhibiting a KRas protein. For example, provided herein are inhibitors of a KRas protein (e.g., a dysregulated KRas protein (e.g., a mutant KRas protein (e.g., a KRas G12D mutant protein, a KRas G12R mutant protein, or a KRas G12V mutant protein))) useful for treating or preventing diseases or disorders associated with the KRas dysregulation (i.e., a KRas-associated disease or disorder), such as a cardiovascular disease, an inflammatory and/or autoimmune disease, or a cancer (e.g., a KRas-associated cancer).

The term "KRas-associated disease or disorder" as used herein refers to diseases or disorders associated with or having a dysregulation of a *KRAS* gene, a KRas protein, or the expression or activity or level of any (e.g., one or more) of the same (e.g., any of the types of dysregulations of a *KRAS* gene, a KRas protein, or the expression or activity or level of any of the same described herein). Non-limiting examples of a KRas-associated disease or disorder include, for example, cancer, a cardiovascular disease (e.g., arteriovenous malformations), endometriosis, and an inflammatory and/or autoimmune disease (e.g., a nonmalignant syndrome of autoimmunity and abnormal leukocyte homeostasis). See, e.g., Adashek et al. Genome Med. 2020; 12: 16, doi: 10.1186/s13073-020-0714-y; Niemela et al. Blood. 2011; 117(10):2883-6, doi: 10.1182/blood-2010-07-295501; Nosan et al. Croat Med J. 2013; 54(6): 574-578, doi: 10.3325/cmj.2013.54.574; and Messina et al. Small GTPases 11.5 (2020): 312-319, 10.1080/21541248.2018.1502591.

The term "mutant KRas-associated disease or disorder" as used herein refers to diseases or disorders associated with or having a KRas mutation (e.g., a *KRAS* gene having a mutation corresponding to a mutation in a KRas protein and/or a KRas protein having a mutation). Non-limiting examples of a mutant KRas-associated disease or disorder include, for example, cancer, a cardiovascular disease (e.g., arteriovenous malformations), endometriosis, and an inflammatory and/or autoimmune disease (e.g., a nonmalignant syndrome of autoimmunity and abnormal leukocyte homeostasis). See, e.g., Adashek et al. Genome Med. 2020; 12: 16, doi: 10.1186/s13073-020-0714-y; Niemela et al. Blood. 2011; 117(10):2883-6, doi: 10.1182/blood-2010-07-295501; Nosan et al. Croat Med J. 2013; 54(6): 574-578, doi: 10.3325/cmj.2013.54.574; and Messina et al. Small GTPases 11.5 (2020): 312-319, 10.1080/21541248.2018.1502591.

The phrase "dysregulation of a *KRAS* gene, a KRas protein, or the expression or activity or level of any of the same" refers to a genetic mutation (e.g., a mutation in a *KRAS* gene that results in the expression of a KRas protein that includes a deletion of at least one amino acid as compared to a wild type KRas protein, a mutation in a *KRAS* gene that results in the expression of a KRas protein with one or more point mutations as compared to a wild type KRas protein, a mutation in a *KRAS* gene that results in the expression of a KRas protein with at least one inserted amino acid as compared to a wild type KRas protein, a gene duplication that results in an increased level of KRas protein in a cell, or a mutation in a regulatory sequence (e.g., a promoter and/or enhancer) that results in an increased level of KRas protein in a cell); an alternative spliced version of a KRas mRNA that results in a KRas protein having a deletion of at least one amino acid in the KRas protein as compared to the wild type KRas protein; or increased expression (e.g., increased levels) of a wild type KRas protein in a mammalian cell due to aberrant cell signaling and/or dysregulated autocrine/paracrine signaling (e.g., as compared to a control non-cancerous cell). As an example, a dysregulation of a *KRAS* gene, a KRas protein, or expression or activity, or level of any of the same, can be a mutation in a *KRAS* gene that encodes a KRas protein that has low GTPase activity and/or has increased signaling activity as compared to a protein encoded by a *KRAS* gene that does not include the mutation. As another example, a dysregulation of a *KRAS* gene, a KRas protein, or expression or activity, or level of any of the same, can be a KRas amplification. In some embodiments, a KRas amplification is an amplification of the wild type KRas. In some embodiments, a KRas amplification is an amplification of a mutant KRas.

A "dysregulated KRas protein" as used herein refers to (i) a KRas protein having a mutation (e.g., a deletion of at least one amino acid as compared to a wild type KRas protein, one or more point mutations as compared to a wild type KRas protein, or an insertion of at least one amino acid as compared to a wild type KRas protein); (ii) a KRas protein resulting from a gene duplication event, e.g., of the gene encoding the KRas protein (e.g., the wild type KRas protein), thus resulting in an increased level and/or activity of the KRas protein (e.g., the wild type KRas protein) in a cell; (iii) a KRas protein resulting from a mutation in a regulatory sequence (e.g., a promoter and/or enhancer) that can also result in an increased level and/or activity of the KRas protein (e.g., the wild type KRas protein) in a cell); (iv) a KRas protein resulting from an alternative spliced version of a KRas mRNA that results in a KRas protein having a deletion of at least one amino acid in the KRas protein as compared to the wild type KRas protein); or (v) a KRas protein resulting from increased expression (e.g., increased levels) of a wild type KRas protein in a mammalian cell due to aberrant cell signaling and/or dysregulated autocrine/paracrine signaling (e.g., as compared to a control non-cancerous cell). In some embodiments, a dysregulated KRas protein is a dysregulated human KRas protein.

A "mutant KRas protein" as used herein refers to a KRas protein including a substitution, an insertion, a deletion, a truncation and/or a fusion relative to the wild type human KRas sequence shown in SEQ ID NO: 1. For example, a mutant human KRas protein includes a substitution at any amino acid position (relative to SEQ ID NO: 1).

A "KRas G12X mutant protein" as used herein refers to a KRas protein including substitution of a glycine to any other amino acid at the twelfth amino acid position (relative to SEQ ID NO: 1).

A "KRas G12A mutant protein" as used herein refers to a KRas protein including a glycine to alanine substitution at the twelfth amino acid position (relative to SEQ ID NO: 1).

A "KRas G12C mutant protein" as used herein refers to a KRas protein including a glycine to cysteine substitution at the twelfth amino acid position (relative to SEQ ID NO: 1).

A "KRas G12D mutant protein" as used herein refers to a KRas protein including a glycine to aspartic acid substitution at the twelfth amino acid position (relative to SEQ ID NO: 1).

A "KRas G12R mutant protein" as used herein refers to a KRas protein including a glycine to arginine substitution at the twelfth amino acid position (relative to SEQ ID NO: 1).

A "KRas G12S mutant protein" as used herein refers to a KRas protein including a glycine to serine substitution at the twelfth amino acid position (relative to SEQ ID NO: 1).

A "KRas G12V mutant protein" as used herein refers to a KRas protein including a glycine to valine substitution at the twelfth amino acid position (relative to SEQ ID NO: 1).

A "KRas G13X mutant protein" as used herein refers to a KRas protein including substitution of a glycine to any other amino acid at the thirteenth amino acid position (relative to SEQ ID NO: 1).

A "KRas G13C mutant protein" as used herein refers to a KRas protein including a glycine to cysteine substitution at the thirteenth amino acid position (relative to SEQ ID NO: 1).

A "KRas G13D mutant protein" as used herein refers to a KRas protein including a glycine to aspartic acid substitution at the thirteenth amino acid position (relative to SEQ ID NO: 1).

A "KRas G13V mutant protein" as used herein refers to a KRas protein including a glycine to valine substitution at the thirteenth amino acid position (relative to SEQ ID NO: 1).

A "KRas Q61X mutant protein" as used herein refers to a KRas protein including substitution of a glutamine to any other amino acid at the sixty-first amino acid position (relative to SEQ ID NO: 1).

A "KRas Q61E mutant protein" as used herein refers to a KRas protein including a glutamine to glutamic acid substitution at the sixty-first amino acid position (relative to SEQ ID NO: 1).

A "KRas Q61H mutant protein" as used herein refers to a KRas protein including a glutamine to histidine substitution at the sixty-first amino acid position (relative to SEQ ID NO: 1).

A "KRas Q61K mutant protein" as used herein refers to a KRas protein including a glutamine to lysine substitution at the sixty-first amino acid position (relative to SEQ ID NO: 1).

A "KRas Q61L mutant protein" as used herein refers to a KRas protein including a glutamine to leucine substitution at the sixty-first amino acid position (relative to SEQ ID NO: 1).

A "KRas Q61P mutant protein" as used herein refers to a KRas protein including a glutamine to proline substitution at the sixty-first amino acid position (relative to SEQ ID NO: 1).

A "KRas Q61R mutant protein" as used herein refers to a KRas protein including a glutamine to arginine substitution at the sixty-first amino acid position (relative to SEQ ID NO: 1).

A "KRas inhibitor" as used herein includes any compound exhibiting KRas protein inactivation activity (e.g., inhibiting or decreasing KRas signaling activity). In some embodiments, a KRas inhibitor as described herein has an IC₅₀ value of 1 µM or less in a nucleotide exchange assay as described herein, an IC₅₀ value of 1 µM or less in a Raf kinase interaction assay as described herein, or both. In some embodiments, a KRas inhibitor inhibits the signaling activity of a wild type KRas protein. In some embodiments, a KRas inhibitor inhibits the signaling activity of a dysregulated KRas protein, for example, resulting in a decrease in activated Raf or other downstream effectors, such as ERK. In some embodiments, a KRas inhibitor inhibits the signaling activity of a mutant KRas protein. In some embodiments, a KRas inhibitor inhibits both the signaling activity of a wild-type KRas protein and the signaling activity of one or more mutant KRas proteins and can be termed a "pan KRas inhibitor". In some embodiments, a KRas inhibitor inhibits one or more mutant KRas proteins, and such a KRas inhibitor can be termed a "mutant KRas inhibitor", and also termed by the mutant(s) it inhibits. For example, a KRas inhibitor that inhibits KRas G12R mutant protein could be termed a "KRas G12R inhibitor". As another example, a KRas inhibitor that inhibits both KRas G12C mutant protein and KRas G12D mutant protein could be termed a "KRas G12C inhibitor" and/or a "KRas G12D inhibitor". In some embodiments, a "mutant KRas inhibitor" inhibits two or more mutant KRas proteins and can be termed a "pan mutant KRas inhibitor". In some embodiments, a pan mutant KRas inhibitor inhibits two or more mutant KRas proteins selected from the group consisting of: a KRas G12A mutant protein, a KRas G12C mutant protein, a KRas G12D mutant protein, a KRas G12R mutant protein, a KRas G12S mutant protein, a KRas G12V mutant protein, a KRas G13C mutant protein, a KRas G13D mutant protein, a KRas G13V mutant protein, a KRas Q61E mutant protein, a KRas Q61H mutant protein, a KRas Q61K mutant protein, a KRas Q61L mutant protein, a KRas Q61P mutant protein, and a KRas Q61R mutant protein. For example, a "KRas G12X inhibitor" can inhibit two or more mutant KRas proteins selected from the group consisting of: a KRas G12A mutant protein, a KRas G12C mutant protein, a KRas G12D mutant protein, a KRas G12R mutant protein, a KRas G12S mutant protein, and a KRas G12V mutant protein. As yet another example, a KRas inhibitor that inhibits a KRas G13D mutant protein could be termed a "KRas G13D inhibitor". In some embodiments, a KRas inhibitor can inhibit a KRas protein having one or more mutations, and such a KRas inhibitor can be termed a "mutant KRas inhibitor" whether or not the mutant KRas inhibitor also inhibits wild type KRas protein. In some embodiments, a KRas inhibitor is a mutant KRas inhibitor. In some embodiments, a KRas inhibitor is an allosteric inhibitor.

The term "compound(s) provided herein" refers to compound(s) of Formula (**II**) (e.g., Formula (**II-a**), (**II-b**), (**II**-**a1**), (**II-b1**), (**II-a2**), (**II-b2**), (**II-3**), (**II-a3**), (**II-4**), (**II-a4**), (**II-5**), (**II-a5**), (**II-6**), (**II-a6**), (**II-7**), (**II-a7**), (**II-7**), or (**II-a8**)), Formula (**III**) (e.g., Formula (**III-1**), (**III-2**), (**III-3**), (**III-4**), (**III-5**), (**III-6**), (**III-7**), or (**III-8**)), Formula (**IV**) (e.g., Formula (**IV-a**), (**IV-b**), (**IV-c**), (**IV-a1**), (**IV-b1**), (**IV-a2**), (**IV-b2**), (**IV-a3**), (**IV-b3**), (**IV-a4**), (**IV-b4**), (**IV-a5**), (**IV-b5**), (**IV-a6**), (**IV-b6**), (**IV-a7**), (**IV-b7**), (**IV-a8**), or (**IV-b8**)), Formula (**V**) (e.g., Formula (**V-a**) or (**V-b**), (**V-a1**), (**V-c**), (**V-d**), (**V-b1**), (**V-a2**), (**V-b2**), (**V-a3**), or (**V-b3**)), Formula (**VI**) (e.g., Formula (**VI-a**), (**VI-b**), (**VI-c**), (**VI-d**), or (**VI-e**))), or Formula (**A**) (e.g., Formula (**I-a1**)) as disclosed herein.

The compounds provided herein, or pharmaceutically acceptable salts thereof, are KRas inhibitors. In some embodiments, a compound provided herein, or a pharmaceutically acceptable salt thereof, is a mutant KRas inhibitor. In some embodiments, a compound provided herein, or a pharmaceutically acceptable salt thereof, inhibits two or more mutant KRas proteins selected from the group consisting of: a KRas G12A mutant protein, a KRas G12C mutant protein, a KRas G12D mutant protein, a KRas G12R mutant protein, a KRas G12S mutant protein, a KRas G12V mutant protein, a KRas G13C mutant protein, a KRas G13D mutant protein, a KRas G13V mutant protein, a KRas Q61E mutant protein, a KRas Q61H mutant protein, a KRas Q61K mutant protein, a KRas Q61L mutant protein, a KRas Q61P mutant protein, and a KRas Q61R mutant protein. In some embodiments, a compound provided herein, or a pharmaceutically acceptable salt thereof, inhibits a KRas G12A mutant protein, a KRas G12C mutant protein, a KRas G12D mutant protein, a KRas G12R mutant protein, a KRas G12S mutant protein, a KRas G12V mutant protein, or a combination thereof. In some embodiments, a compound provided herein, or a pharmaceutically acceptable salt thereof, inhibits a KRas G12D mutant protein, a KRas G12R mutant protein, a KRas G12V mutant protein, or a combination thereof. In some embodiments, a compound provided herein, or a pharmaceutically acceptable salt thereof, inhibits a KRas G12D mutant protein, a KRas G12V mutant protein, or both. In some embodiments, a compound provided herein, or a pharmaceutically acceptable salt thereof, inhibits a KRas G12R mutant protein, a KRas G12V mutant protein, or both. In some embodiments, a compound provided herein, or a pharmaceutically acceptable salt thereof, inhibits a KRas G12A mutant protein. In some embodiments, a compound provided herein, or a pharmaceutically acceptable salt thereof, inhibits a KRas G12C mutant protein. In some embodiments, a compound provided herein, or a pharmaceutically acceptable salt thereof, inhibits a KRas G12D mutant protein. In some embodiments, a compound provided herein, or a pharmaceutically acceptable salt thereof, inhibits a KRas G12R mutant protein. In some embodiments, a compound provided herein, or a pharmaceutically acceptable salt thereof, inhibits a KRas G12S mutant protein. In some embodiments, a compound provided herein, or a pharmaceutically acceptable salt thereof, inhibits a KRas G12V mutant protein.

In some embodiments, a compound provided herein, or a pharmaceutically acceptable salt thereof, is a KRas G12X inhibitor. In some embodiments, a compound provided herein, or a pharmaceutically acceptable salt thereof, inhibits one or more mutant KRas proteins selected from the group consisting of: a KRas G12A mutant protein, a KRas G12C mutant protein, a KRas G12D mutant protein, a KRas G12R mutant protein, a KRas G12S mutant protein, and a KRas G12V mutant protein. In some embodiments, a compound provided herein, or a pharmaceutically acceptable salt thereof, inhibits two or more mutant KRas proteins selected from the group consisting of: a KRas G12A mutant protein, a KRas G12C mutant protein, a KRas G12D mutant protein, a KRas G12R mutant protein, a KRas G12S mutant protein, and a KRas G12V mutant protein. In some embodiments, a compound provided herein, or a pharmaceutically acceptable salt thereof, inhibits three or more mutant KRas proteins selected from the group consisting of: a KRas G12A mutant protein, a KRas G12C mutant protein, a KRas G12D mutant protein, a KRas G12R mutant protein, a KRas G12S mutant protein, and a KRas G12V mutant protein. In some embodiments, a compound provided herein, or a pharmaceutically acceptable salt thereof, inhibits four or more mutant KRas proteins selected from the group consisting of: a KRas G12A mutant protein, a KRas G12C mutant protein, a KRas G12D mutant protein, a KRas G12R mutant protein, a KRas G12S mutant protein, and a KRas G12V mutant protein. In some embodiments, a compound provided herein, or a pharmaceutically acceptable salt thereof, inhibits five or more mutant KRas proteins selected from the group consisting of: a KRas G12A mutant protein, a KRas G12C mutant protein, a KRas G12D mutant protein, a KRas G12R mutant protein, a KRas G12S mutant protein, and a KRas G12V mutant protein. In some embodiments, a compound provided herein, or a pharmaceutically acceptable salt thereof, inhibits a KRas G12A mutant protein, a KRas G12C mutant protein, a KRas G12D mutant protein, a KRas G12R mutant protein, a KRas G12S mutant protein, and a KRas G12V mutant protein. In some embodiments, a compound provided herein, or a pharmaceutically acceptable salt thereof, inhibits one or more mutant KRas proteins selected from the group consisting of: a KRas G12A mutant protein, a KRas G12D mutant protein, a KRas G12R mutant protein, a KRas G12S mutant protein, and a KRas G12V mutant protein. In some embodiments, a compound provided herein, or a pharmaceutically acceptable salt thereof, inhibits two or more mutant KRas proteins selected from the group consisting of: a KRas G12A mutant protein, a KRas G12D mutant protein, a KRas G12R mutant protein, a KRas G12S mutant protein, and a KRas G12V mutant protein. In some embodiments, a compound provided herein, or a pharmaceutically acceptable salt thereof, inhibits three or more mutant KRas proteins selected from the group consisting of: a KRas G12A mutant protein, a KRas G12D mutant protein, a KRas G12R mutant protein, a KRas G12S mutant protein, and a KRas G12V mutant protein. In some embodiments, a compound provided herein, or a pharmaceutically acceptable salt thereof, inhibits four or more mutant KRas proteins selected from the group consisting of: a KRas G12A mutant protein, a KRas G12D mutant protein, a KRas G12R mutant protein, a KRas G12S mutant protein, and a KRas G12V mutant protein. In some embodiments, a compound provided herein, or a pharmaceutically acceptable salt thereof, inhibits a KRas G12A mutant protein, a KRas G12D mutant protein, a KRas G12R mutant protein, a KRas G12S mutant protein, and a KRas G12V mutant protein. In some embodiments, a compound provided herein, or a pharmaceutically acceptable salt thereof, inhibits a KRas G12D mutant protein, a KRas G12V mutant protein, or both. In some embodiments, a compound provided herein, or a pharmaceutically acceptable salt thereof, inhibits a KRas G12A mutant protein. In some embodiments, a compound provided herein, or a pharmaceutically acceptable salt thereof, inhibits a KRas G12C mutant protein. In some embodiments, a compound provided herein, or a pharmaceutically acceptable salt thereof, does not inhibit a KRas G12C mutant protein. In some embodiments, a compound provided herein, or a pharmaceutically acceptable salt thereof, inhibits a KRas G12D mutant protein. In some embodiments, a compound provided herein, or a pharmaceutically acceptable salt thereof, does not inhibit a KRas G12D mutant protein. In some embodiments, a compound provided herein, or a pharmaceutically acceptable salt thereof, inhibits a KRas G12R mutant protein. In some embodiments, a compound provided herein, or a pharmaceutically acceptable salt thereof, inhibits a KRas G12S mutant protein. In some embodiments, a compound provided herein, or a pharmaceutically acceptable salt thereof, inhibits a KRas G12V mutant protein.

In some embodiments, a compound provided herein, or a pharmaceutically acceptable salt thereof, is a KRas G13X inhibitor. In some embodiments, a compound provided herein, or a pharmaceutically acceptable salt thereof, inhibits one or more mutant KRas proteins selected from the group consisting of: a KRas G13C mutant protein, a KRas G13D mutant protein, and a KRas G13V mutant protein. In some embodiments, a compound provided herein, or a pharmaceutically acceptable salt thereof, inhibits two or more mutant KRas proteins selected from the group consisting of: a KRas G13C mutant protein, a KRas G13D mutant protein, and a KRas G13V mutant protein. In some embodiments, a compound provided herein, or a pharmaceutically acceptable salt thereof, inhibits a KRas G13C mutant protein, a KRas G13D mutant protein, and a KRas G13V mutant protein. In some embodiments, a compound provided herein, or a pharmaceutically acceptable salt thereof, inhibits a KRas G13C mutant protein. In some embodiments, a compound provided herein, or a pharmaceutically acceptable salt thereof, inhibits a KRas G13D mutant protein. In some embodiments, a compound provided herein, or a pharmaceutically acceptable salt thereof, inhibits a KRas G13V mutant protein.

In some embodiments, a compound provided herein, or a pharmaceutically acceptable salt thereof, is a KRas Q61X inhibitor. In some embodiments, a compound provided herein, or a pharmaceutically acceptable salt thereof, inhibits one or more mutant KRas proteins selected from the group consisting of: a KRas Q61E mutant protein, a KRas Q61H mutant protein, a KRas Q61K mutant protein, a KRas Q61L mutant protein, a KRas Q61P mutant protein, and a KRas Q61R mutant protein. In some embodiments, a compound provided herein, or a pharmaceutically acceptable salt thereof, inhibits two or more mutant KRas proteins selected from the group consisting of: a KRas Q61E mutant protein, a KRas Q61H mutant protein, a KRas Q61K mutant protein, a KRas Q61L mutant protein, a KRas Q61P mutant protein, and a KRas mutant protein. In some embodiments, a compound provided herein, or a pharmaceutically acceptable salt thereof, inhibits three or more mutant KRas proteins selected from the group consisting of: a KRas Q61E mutant protein, a KRas Q61H mutant protein, a KRas Q61K mutant protein, a KRas Q61L mutant protein, a KRas Q61P mutant protein, and a KRas Q61R mutant protein. In some embodiments, a compound provided herein, or a pharmaceutically acceptable salt thereof, inhibits four or more mutant KRas proteins selected from the group consisting of: a KRas Q61E mutant protein, a KRas Q61H mutant protein, a KRas Q61K mutant protein, a KRas Q61L mutant protein, a KRas Q61P mutant protein, and a KRas Q61R mutant protein. In some embodiments, a compound provided herein, or a pharmaceutically acceptable salt thereof, inhibits five or more mutant KRas proteins selected from the group consisting of: a KRas Q61E mutant protein, a KRas Q61H mutant protein, a KRas Q61K mutant protein, a KRas Q61L mutant protein, a KRas Q61P mutant protein, and a KRas Q61R mutant protein. In some embodiments, a compound provided herein, or a pharmaceutically acceptable salt thereof, inhibits a KRas Q61E mutant protein, a KRas Q61H mutant protein, a KRas Q61K mutant protein, a KRas Q61L mutant protein, a KRas Q61P mutant protein, and a KRas Q61R mutant protein. In some embodiments, a compound provided herein, or a pharmaceutically acceptable salt thereof, inhibits a KRas Q61E mutant protein. In some embodiments, a compound provided herein, or a pharmaceutically acceptable salt thereof, inhibits a KRas Q61H mutant protein. In some embodiments, a compound provided herein, or a pharmaceutically acceptable salt thereof, inhibits a KRas Q61K mutant protein. In some embodiments, a compound provided herein, or a pharmaceutically acceptable salt thereof, inhibits a KRas Q61L mutant protein. In some embodiments, a compound provided herein, or a pharmaceutically acceptable salt thereof, inhibits a KRas Q61P mutant protein. In some embodiments, a compound provided herein, or a pharmaceutically acceptable salt thereof, inhibits a KRas Q61R mutant protein.

In some embodiments, a compound provided herein, or a pharmaceutically acceptable salt thereof, inhibits one or more mutant KRas proteins selected from the group consisting of: a KRas G12X mutant protein, a KRas G13X mutant protein, and a KRas Q61X mutant protein. In some embodiments, a compound provided herein, or a pharmaceutically acceptable salt thereof, inhibits two or more mutant KRas proteins selected from the group consisting of: a KRas G12X mutant protein, a KRas G13X mutant protein, and a KRas Q61X mutant protein. In some embodiments, a compound provided herein, or a pharmaceutically acceptable salt thereof, inhibits three or more mutant human KRas proteins selected from the group consisting of: a KRas G12X mutant protein, a KRas G13X mutant protein, and a KRas Q61X mutant protein. In some embodiments, a compound provided herein, or a pharmaceutically acceptable salt thereof, inhibits a KRas G12X mutant protein, a KRas G13X mutant protein, and a KRas Q61X mutant protein. In some embodiments, a compound provided herein, or a pharmaceutically acceptable salt thereof, inhibits one or more mutant KRas proteins selected from the group consisting of: a KRas G12A mutant protein, a KRas G12C mutant protein, a KRas G12D mutant protein, a KRas G12R mutant protein, a KRas G12S mutant protein, a KRas G12V mutant protein, a KRas G13C mutant protein, a KRas G13D mutant protein, a KRas G13V mutant protein, a KRas Q61E mutant protein, a KRas Q61H mutant protein, a KRas Q61K mutant protein, a KRas Q61L mutant protein, a KRas Q61P mutant protein, and a KRas Q61R mutant protein. In some embodiments, a compound provided herein, or a pharmaceutically acceptable salt thereof, inhibits two or more mutant KRas proteins selected from the group consisting of: a KRas G12A mutant protein, a KRas G12C mutant protein, a KRas G12D mutant protein, a KRas G12R mutant protein, a KRas G12S mutant protein, a KRas G12V mutant protein, a KRas G13C mutant protein, a KRas G13D mutant protein, a KRas G13V mutant protein, a KRas Q61E mutant protein, a KRas Q61H mutant protein, a KRas Q61K mutant protein, a KRas Q61L mutant protein, a KRas Q61P mutant protein, and a KRas Q61R mutant protein. In some embodiments, a compound provided herein, or a pharmaceutically acceptable salt thereof, inhibits three or more mutant KRas proteins selected from the group consisting of: a KRas G12A mutant protein, a KRas G12C mutant protein, a KRas G12D mutant protein, a KRas G12R mutant protein, a KRas G12S mutant protein, a KRas G12V mutant protein, a KRas G13C mutant protein, a KRas G13D mutant protein, a KRas G13V mutant protein, a KRas Q61E mutant protein, a KRas Q61H mutant protein, a KRas Q61K mutant protein, a KRas Q61L mutant protein, a KRas Q61P mutant protein, and a KRas Q61R mutant protein. In some embodiments, a compound provided herein, or a pharmaceutically acceptable salt thereof, inhibits four or more mutant KRas proteins selected from the group consisting of: a KRas G12A mutant protein, a KRas G12C mutant protein, a KRas G12D mutant protein, a KRas G12R mutant protein, a KRas G12S mutant protein, a KRas G12V mutant protein, a KRas G13C mutant protein, a KRas G13D mutant protein, a KRas G13V mutant protein, a KRas Q61E mutant protein, a KRas Q61H mutant protein, a KRas Q61K mutant protein, a KRas Q61L mutant protein, a KRas Q61P mutant protein, and a KRas Q61R mutant protein. In some embodiments, a compound provided herein, or a pharmaceutically acceptable salt thereof, five or more mutant KRas proteins selected from the group consisting of: a KRas G12A mutant protein, a KRas G12C mutant protein, a KRas G12D mutant protein, a KRas G12R mutant protein, a KRas G12S mutant protein, a KRas G12V mutant protein, a KRas G13C mutant protein, a KRas G13D mutant protein, a KRas G13V mutant protein, a KRas Q61E mutant protein, a KRas Q61H mutant protein, a KRas Q61K mutant protein, a KRas Q61L mutant protein, a KRas Q61P mutant protein, and a KRas Q61R mutant protein. In some embodiments, a compound provided herein, or a pharmaceutically acceptable salt thereof, inhibits a KRas G12A mutant protein, a KRas G12C mutant protein, a KRas G12D mutant protein, a KRas G12R mutant protein, a KRas G12S mutant protein, a KRas G12V mutant protein, a KRas G13C mutant protein, a KRas G13D mutant protein, a KRas G13V mutant protein, a KRas Q61E mutant protein, a KRas Q61H mutant protein, a KRas Q61K mutant protein, a KRas Q61L mutant protein, a KRas Q61P mutant protein, and a KRas Q61R mutant protein. In some embodiments, a compound provided herein, or a pharmaceutically acceptable salt thereof, inhibits a KRas G12A mutant protein, a KRas G12D mutant protein, a KRas G12R mutant protein, a KRas G12S mutant protein, a KRas G12V mutant protein, a KRas G13C mutant protein, a KRas G13D mutant protein, a KRas G13V mutant protein, a KRas Q61E mutant protein, a KRas Q61H mutant protein, a KRas Q61K mutant protein, a KRas Q61L mutant protein, a KRas Q61P mutant protein, and a KRas Q61R mutant protein.

In some embodiments, a compound provided herein, or a pharmaceutically acceptable salt thereof, inhibits one or more mutant KRas proteins selected from the group consisting of: a KRas G12C mutant protein, a KRas G12D mutant protein, a KRas G12R mutant protein, a KRas G12V mutant protein, a KRas G13D mutant protein, and a KRas Q61H mutant protein. In some embodiments, a compound provided herein, or a pharmaceutically acceptable salt thereof, inhibits two or more mutant KRas proteins selected from the group consisting of: a KRas G12C mutant protein, a KRas G12D mutant protein, a KRas G12R mutant protein, a KRas G12V mutant protein, a KRas G13D mutant protein, and a KRas Q61H mutant protein. In some embodiments, a compound provided herein, or a pharmaceutically acceptable salt thereof, inhibits three or more mutant KRas proteins selected from the group consisting of: a KRas G12C mutant protein, a KRas G12D mutant protein, a KRas G12R mutant protein, a KRas G12V mutant protein, a KRas G13D mutant protein, and a KRas Q61H mutant protein. In some embodiments, a compound provided herein, or a pharmaceutically acceptable salt thereof, inhibits one or more mutant KRas proteins selected from the group consisting of: a KRas G12C mutant protein, a KRas G12D mutant protein, a KRas G12R mutant protein, and a KRas G12V mutant protein. In some embodiments, a compound provided herein, or a pharmaceutically acceptable salt thereof, inhibits two or more mutant KRas proteins selected from the group consisting of: a KRas G12C mutant protein, a KRas G12D mutant protein, a KRas G12R mutant protein, and a KRas G12V mutant protein. In some embodiments, a compound provided herein, or a pharmaceutically acceptable salt thereof, inhibits three or more mutant KRas proteins selected from the group consisting of: a KRas G12C mutant protein, a KRas G12D mutant protein, a KRas G12R mutant protein, and a KRas G12V mutant protein. In some embodiments, a compound provided herein, or a pharmaceutically acceptable salt thereof, inhibits one or more mutant KRas proteins selected from the group consisting of: a KRas G12D mutant protein, a KRas G12R mutant protein, and a KRas G12V mutant protein. In some embodiments, a compound provided herein, or a pharmaceutically acceptable salt thereof, inhibits two or more mutant KRas proteins selected from the group consisting of: a KRas G12D mutant protein, a KRas G12R mutant protein, and a KRas G12V mutant protein. In some embodiments, a compound provided herein, or a pharmaceutically acceptable salt thereof, inhibits a KRas G12D mutant protein, a KRas G12R mutant protein, and a KRas G12V mutant protein. In some such embodiments, the compounds provided herein, or pharmaceutically acceptable salts thereof, are useful for treating a bladder cancer.

In some embodiments, a compound provided herein, or a pharmaceutically acceptable salt thereof, inhibits one or more mutant KRas proteins selected from the group consisting of: a KRas G12C mutant protein, a KRas G12D mutant protein, a KRas G12V mutant protein, and a KRas G13D mutant protein. In some embodiments, a compound provided herein, or a pharmaceutically acceptable salt thereof, inhibits two or more mutant KRas proteins selected from the group consisting of: a KRas G12C mutant protein, a KRas G12D mutant protein, a KRas G12V mutant protein, and a KRas G13D mutant protein. In some embodiments, a compound provided herein, or a pharmaceutically acceptable salt thereof, inhibits three or more mutant KRas proteins selected from the group consisting of: a KRas G12C mutant protein, a KRas G12D mutant protein, a KRas G12V mutant protein, and a KRas G13D mutant protein. In some embodiments, a compound provided herein, or a pharmaceutically acceptable salt thereof, inhibits one or more mutant KRas proteins selected from the group consisting of: a KRas G12C mutant protein, a KRas G12D mutant protein, and a KRas G12V mutant protein. In some embodiments, a compound provided herein, or a pharmaceutically acceptable salt thereof, inhibits two or more mutant KRas proteins selected from the group consisting of: a KRas G12C mutant protein, a KRas G12D mutant protein, and a KRas G12V mutant protein. In some embodiments, a compound provided herein, or a pharmaceutically acceptable salt thereof, inhibits a KRas G12C mutant protein, a KRas G12D mutant protein, and a KRas G12V mutant protein. In some embodiments, a compound provided herein, or a pharmaceutically acceptable salt thereof, inhibits a KRas G12D mutant protein, a KRas G12V mutant protein, or both. In some such embodiments, the compounds provided herein, or pharmaceutically acceptable salts thereof, are useful for treating a cervical cancer.

In some embodiments, a compound provided herein, or a pharmaceutically acceptable salt thereof, inhibits one or more mutant KRas proteins selected from the group consisting of: a KRas G12A mutant protein, a KRas G12C mutant protein, a KRas G12D mutant protein, a KRas G12R mutant protein, a KRas G12S mutant protein, a KRas G12V mutant protein, a KRas G13C mutant protein, a KRas G13D mutant protein, a KRas G13V mutant protein, a KRas Q61E mutant protein, a KRas Q61H mutant protein, a KRas Q61K mutant protein, a KRas Q61L mutant protein, a KRas Q61P mutant protein, and a KRas Q61R mutant protein. In some embodiments, a compound provided herein, or a pharmaceutically acceptable salt thereof, inhibits one or more mutant KRas proteins selected from the group consisting of: a KRas G12A mutant protein, a KRas G12C mutant protein, a KRas G12D mutant protein, a KRas G12R mutant protein, a KRas G12S mutant protein, and a KRas G12V mutant protein. In some embodiments, a compound provided herein, or a pharmaceutically acceptable salt thereof, inhibits two or more mutant KRas proteins selected from the group consisting of: a KRas G12A mutant protein, a KRas G12C mutant protein, a KRas G12D mutant protein, a KRas G12R mutant protein, a KRas G12S mutant protein, and a KRas G12V mutant protein. In some embodiments, a compound provided herein, or a pharmaceutically acceptable salt thereof, inhibits three or more mutant KRas proteins selected from the group consisting of: a KRas G12A mutant protein, a KRas G12C mutant protein, a KRas G12D mutant protein, a KRas G12R mutant protein, a KRas G12S mutant protein, and a KRas G12V mutant protein. In some embodiments, a compound provided herein, or a pharmaceutically acceptable salt thereof, inhibits one or more mutant KRas proteins selected from the group consisting of: a KRas G12A mutant protein, a KRas G12D mutant protein, a KRas G12R mutant protein, and a KRas G12V mutant protein. In some embodiments, a compound provided herein, or a pharmaceutically acceptable salt thereof, inhibits two or more mutant KRas proteins selected from the group consisting of: a KRas G12A mutant protein, a KRas G12D mutant protein, a KRas G12R mutant protein, and a KRas G12V mutant protein. In some embodiments, a compound provided herein, or a pharmaceutically acceptable salt thereof, inhibits three or more mutant KRas proteins selected from the group consisting of: a KRas G12A mutant protein, a KRas G12D mutant protein, a KRas G12R mutant protein, and a KRas G12V mutant protein. In some such embodiments, the compounds provided herein, or pharmaceutically acceptable salts thereof, are useful for treating a colorectal cancer.

In some embodiments, a compound provided herein, or a pharmaceutically acceptable salt thereof, inhibits one or more mutant KRas proteins selected from the group consisting of: a KRas G12A mutant protein, a KRas G12C mutant protein, a KRas G12D mutant protein, a KRas G12S mutant protein, a KRas G12V mutant protein, a KRas G13C mutant protein, a KRas G13D mutant protein, a KRas G13V mutant protein, a KRas Q61H mutant protein, and a KRas Q61L mutant protein. In some embodiments, a compound provided herein, or a pharmaceutically acceptable salt thereof, inhibits two or more mutant KRas proteins selected from the group consisting of: a KRas G12A mutant protein, a KRas G12C mutant protein, a KRas G12D mutant protein, a KRas G12S mutant protein, a KRas G12V mutant protein, a KRas G13C mutant protein, a KRas G13D mutant protein, a KRas G13V mutant protein, a KRas Q61H mutant protein, and a KRas Q61L mutant protein. In some embodiments, a compound provided herein, or a pharmaceutically acceptable salt thereof, inhibits three or more mutant KRas proteins selected from the group consisting of: a KRas G12A mutant protein, a KRas G12C mutant protein, a KRas G12D mutant protein, a KRas G12S mutant protein, a KRas G12V mutant protein, a KRas G13C mutant protein, a KRas G13D mutant protein, a KRas G13V mutant protein, a KRas Q61H mutant protein, and a KRas Q61L mutant protein. In some embodiments, a compound provided herein, or a pharmaceutically acceptable salt thereof, inhibits one or more mutant KRas proteins selected from the group consisting of: a KRas G12A mutant protein, a KRas G12C mutant protein, a KRas G12D mutant protein, a KRas G12S mutant protein, and a KRas G12V mutant protein. In some embodiments, a compound provided herein, or a pharmaceutically acceptable salt thereof, inhibits two or more mutant KRas proteins selected from the group consisting of: a KRas G12A mutant protein, a KRas G12C mutant protein, a KRas G12D mutant protein, a KRas G12S mutant protein, and a KRas G12V mutant protein. In some embodiments, a compound provided herein, or a pharmaceutically acceptable salt thereof, inhibits three or more mutant KRas proteins selected from the group consisting of: a KRas G12A mutant protein, a KRas G12C mutant protein, a KRas G12D mutant protein, a KRas G12S mutant protein, and a KRas G12V mutant protein. In some embodiments, a compound provided herein, or a pharmaceutically acceptable salt thereof, inhibits one or more mutant KRas proteins selected from the group consisting of: a KRas G12A mutant protein, a KRas G12D mutant protein, and a KRas G12V mutant protein. In some embodiments, a compound provided herein, or a pharmaceutically acceptable salt thereof, inhibits two or more mutant KRas proteins selected from the group consisting of: a KRas G12A mutant protein, a KRas G12D mutant protein, and a KRas G12V mutant protein. In some embodiments, a compound provided herein, or a pharmaceutically acceptable salt thereof, inhibits a KRas G12A mutant protein, a KRas G12D mutant protein, and a KRas G12V mutant protein. In some such embodiments, the compounds provided herein, or pharmaceutically acceptable salts thereof, are useful for treating an endometrial cancer.

In some embodiments, a compound provided herein, or a pharmaceutically acceptable salt thereof, inhibits one or more mutant KRas proteins selected from the group consisting of: a KRas G12C mutant protein, a KRas G12D mutant protein, a KRas G12S mutant protein, a KRas G12V mutant protein, a KRas G13C mutant protein, a KRas G13D mutant protein, and a KRas Q61H mutant protein. In some embodiments, a compound provided herein, or a pharmaceutically acceptable salt thereof, inhibits two or more mutant KRas proteins selected from the group consisting of: a KRas G12C mutant protein, a KRas G12D mutant protein, a KRas G12S mutant protein, a KRas G12V mutant protein, a KRas G13C mutant protein, a KRas G13D mutant protein, and a KRas Q61H mutant protein. In some embodiments, a compound provided herein, or a pharmaceutically acceptable salt thereof, inhibits three or more mutant KRas proteins selected from the group consisting of: a KRas G12C mutant protein, a KRas G12D mutant protein, a KRas G12S mutant protein, a KRas G12V mutant protein, a KRas G13C mutant protein, a KRas G13D mutant protein, and a KRas Q61H mutant protein. In some embodiments, a compound provided herein, or a pharmaceutically acceptable salt thereof, inhibits one or more mutant KRas proteins selected from the group consisting of: a KRas G12C mutant protein, a KRas G12D mutant protein, a KRas G12S mutant protein, and a KRas G12V mutant protein. In some embodiments, a compound provided herein, or a pharmaceutically acceptable salt thereof, inhibits two or more mutant KRas proteins selected from the group consisting of: a KRas G12C mutant protein, a KRas G12D mutant protein, a KRas G12S mutant protein, and a KRas G12V mutant protein. In some embodiments, a compound provided herein, or a pharmaceutically acceptable salt thereof, inhibits three or more mutant KRas proteins selected from the group consisting of: a KRas G12C mutant protein, a KRas G12D mutant protein, a KRas G12S mutant protein, and a KRas G12V mutant protein. In some embodiments, a compound provided herein, or a pharmaceutically acceptable salt thereof, inhibits a KRas G12D mutant protein, a KRas G12V mutant protein, or both. In some such embodiments, the compounds provided herein, or pharmaceutically acceptable salts thereof, are useful for treating an esophageal or stomach cancer.

In some embodiments, a compound provided herein, or a pharmaceutically acceptable salt thereof, inhibits one or more mutant KRas proteins selected from the group consisting of: a KRas G12A mutant protein, a KRas G12C mutant protein, a KRas G12D mutant protein, a KRas G12R mutant protein, a KRas G12S mutant protein, a KRas G12V mutant protein, a KRas G13C mutant protein, a KRas G13D mutant protein, a KRas G13V mutant protein, a KRas Q61E mutant protein, a KRas Q61H mutant protein, a KRas Q61K mutant protein, a KRas Q61L mutant protein, a KRas Q61P mutant protein, and a KRas Q61R mutant protein. In some embodiments, a compound provided herein, or a pharmaceutically acceptable salt thereof, inhibits two or more mutant KRas proteins selected from the group consisting of: a KRas G12A mutant protein, a KRas G12C mutant protein, a KRas G12D mutant protein, a KRas G12R mutant protein, a KRas G12S mutant protein, a KRas G12V mutant protein, a KRas G13C mutant protein, a KRas G13D mutant protein, a KRas G13V mutant protein, a KRas Q61E mutant protein, a KRas Q61H mutant protein, a KRas Q61K mutant protein, a KRas Q61L mutant protein, a KRas Q61P mutant protein, and a KRas Q61R mutant protein. In some embodiments, a compound provided herein, or a pharmaceutically acceptable salt thereof, inhibits three or more mutant KRas proteins selected from the group consisting of: a KRas G12A mutant protein, a KRas G12C mutant protein, a KRas G12D mutant protein, a KRas G12R mutant protein, a KRas G12S mutant protein, a KRas G12V mutant protein, a KRas G13C mutant protein, a KRas G13D mutant protein, a KRas G13V mutant protein, a KRas Q61E mutant protein, a KRas Q61H mutant protein, a KRas Q61K mutant protein, a KRas Q61L mutant protein, a KRas Q61P mutant protein, and a KRas Q61R mutant protein. In some embodiments, a compound provided herein, or a pharmaceutically acceptable salt thereof, inhibits one or more mutant KRas proteins selected from the group consisting of: a KRas G12A mutant protein, a KRas G12C mutant protein, a KRas G12D mutant protein, a KRas G12R mutant protein, a KRas G12S mutant protein, and a KRas G12V mutant protein. In some embodiments, a compound provided herein, or a pharmaceutically acceptable salt thereof, inhibits two or more mutant KRas proteins selected from the group consisting of: a KRas G12A mutant protein, a KRas G12C mutant protein, a KRas G12D mutant protein, a KRas G12R mutant protein, a KRas G12S mutant protein, and a KRas G12V mutant protein. In some embodiments, a compound provided herein, or a pharmaceutically acceptable salt thereof, inhibits three or more mutant KRas proteins selected from the group consisting of: a KRas G12A mutant protein, a KRas G12C mutant protein, a KRas G12D mutant protein, a KRas G12R mutant protein, a KRas G12S mutant protein, and a KRas G12V mutant protein. In some embodiments, a compound provided herein, or a pharmaceutically acceptable salt thereof, inhibits one or more mutant KRas proteins selected from the group consisting of: a KRas G12A mutant protein, a KRas G12D mutant protein, a KRas G12R mutant protein, and a KRas G12V mutant protein. In some embodiments, a compound provided herein, or a pharmaceutically acceptable salt thereof, inhibits two or more mutant KRas proteins selected from the group consisting of: a KRas G12A mutant protein, a KRas G12D mutant protein, a KRas G12R mutant protein, and a KRas G12V mutant protein. In some embodiments, a compound provided herein, or a pharmaceutically acceptable salt thereof, inhibits three or more mutant KRas proteins selected from the group consisting of: a KRas G12A mutant protein, a KRas G12D mutant protein, a KRas G12R mutant protein, and a KRas G12V mutant protein. In some such embodiments, the compounds provided herein, or pharmaceutically acceptable salts thereof, are useful for treating a leukemia.

In some embodiments, a compound provided herein, or a pharmaceutically acceptable salt thereof, inhibits one or more mutant KRas proteins selected from the group consisting of: a KRas G12C mutant protein, a KRas G12D mutant protein, a KRas G12R mutant protein, a KRas G13D mutant protein, a KRas G13V mutant protein, a KRas Q61K mutant protein, a KRas Q61L mutant protein, and a KRas Q61R mutant protein. In some embodiments, a compound provided herein, or a pharmaceutically acceptable salt thereof, inhibits two or more mutant KRas proteins selected from the group consisting of: a KRas G12C mutant protein, a KRas G12D mutant protein, a KRas G12R mutant protein, a KRas G13D mutant protein, a KRas G13V mutant protein, a KRas Q61K mutant protein, a KRas Q61L mutant protein, and a KRas Q61R mutant protein. In some embodiments, a compound provided herein, or a pharmaceutically acceptable salt thereof, inhibits three or more mutant KRas proteins selected from the group consisting of: a KRas G12C mutant protein, a KRas G12D mutant protein, a KRas G12R mutant protein, a KRas G13D mutant protein, a KRas G13V mutant protein, a KRas Q61K mutant protein, a KRas Q61L mutant protein, and a KRas Q61R mutant protein. In some embodiments, a compound provided herein, or a pharmaceutically acceptable salt thereof, inhibits one or more mutant KRas proteins selected from the group consisting of: a KRas G12C mutant protein, a KRas G12D mutant protein, and a KRas G12R mutant protein. In some embodiments, a compound provided herein, or a pharmaceutically acceptable salt thereof, inhibits two or more mutant KRas proteins selected from the group consisting of: a KRas G12C mutant protein, a KRas G12D mutant protein, and a KRas G12R mutant protein. In some embodiments, a compound provided herein, or a pharmaceutically acceptable salt thereof, inhibits a KRas G12C mutant protein, a KRas G12D mutant protein, and a KRas G12R mutant protein. In some embodiments, a compound provided herein, or a pharmaceutically acceptable salt thereof, inhibits a KRas G12D mutant protein, a KRas G12R mutant protein, or both. In some such embodiments, the compounds provided herein, or pharmaceutically acceptable salts thereof, are useful for treating a melanoma.

In some embodiments, a compound provided herein, or a pharmaceutically acceptable salt thereof, inhibits one or more mutant KRas proteins selected from the group consisting of: a KRas G12A mutant protein, a KRas G12C mutant protein, a KRas G12D mutant protein, a KRas G12R mutant protein, a KRas G12S mutant protein, a KRas G13D mutant protein, and a KRas Q61L mutation. In some embodiments, a compound provided herein, or a pharmaceutically acceptable salt thereof, inhibits two or more mutant KRas proteins selected from the group consisting of: a KRas G12A mutant protein, a KRas G12C mutant protein, a KRas G12D mutant protein, a KRas G12R mutant protein, a KRas G12S mutant protein, a KRas G13D mutant protein, and a KRas Q61L mutation. In some embodiments, a compound provided herein, or a pharmaceutically acceptable salt thereof, inhibits three or more mutant KRas proteins selected from the group consisting of: a KRas G12A mutant protein, a KRas G12C mutant protein, a KRas G12D mutant protein, a KRas G12R mutant protein, a KRas G12S mutant protein, a KRas G13D mutant protein, and a KRas Q61L mutation. In some embodiments, a compound provided herein, or a pharmaceutically acceptable salt thereof, inhibits one or more mutant KRas proteins selected from the group consisting of: a KRas G12A mutant protein, a KRas G12C mutant protein, a KRas G12D mutant protein, a KRas G12R mutant protein, and a KRas G12S mutant protein. In some embodiments, a compound provided herein, or a pharmaceutically acceptable salt thereof, inhibits two or more mutant KRas proteins selected from the group consisting of: a KRas G12A mutant protein, a KRas G12C mutant protein, a KRas G12D mutant protein, a KRas G12R mutant protein, and a KRas G12S mutant protein. In some embodiments, a compound provided herein, or a pharmaceutically acceptable salt thereof, inhibits three or more mutant KRas proteins selected from the group consisting of: a KRas G12A mutant protein, a KRas G12C mutant protein, a KRas G12D mutant protein, a KRas G12R mutant protein, and a KRas G12S mutant protein. In some embodiments, a compound provided herein, or a pharmaceutically acceptable salt thereof, inhibits a KRas G12C mutant protein, a KRas G12D mutant protein, and a KRas G12V mutant protein. In some embodiments, a compound provided herein, or a pharmaceutically acceptable salt thereof, inhibits a KRas G12D mutant protein, a KRas G12V mutant protein, or both. In some such embodiments, the compounds provided herein, or pharmaceutically acceptable salts thereof, are useful for treating an ovarian cancer.

In some embodiments, a compound provided herein, or a pharmaceutically acceptable salt thereof, inhibits one or more mutant KRas proteins selected from the group consisting of: a KRas G12A mutant protein, a KRas G12C mutant protein, a KRas G12D mutant protein, a KRas G12S mutant protein, a KRas G12V mutant protein, a KRas G13C mutant protein, a KRas G13D mutant protein, a KRas Q61H mutant protein, and a KRas Q61L mutant protein. In some embodiments, a compound provided herein, or a pharmaceutically acceptable salt thereof, inhibits two or more mutant KRas proteins selected from the group consisting of: a KRas G12A mutant protein, a KRas G12C mutant protein, a KRas G12D mutant protein, a KRas G12S mutant protein, a KRas G12V mutant protein, a KRas G13C mutant protein, a KRas G13D mutant protein, a KRas Q61H mutant protein, and a KRas Q61L mutant protein. In some embodiments, a compound provided herein, or a pharmaceutically acceptable salt thereof, inhibits three or more mutant KRas proteins selected from the group consisting of: a KRas G12A mutant protein, a KRas G12C mutant protein, a KRas G12D mutant protein, a KRas G12S mutant protein, a KRas G12V mutant protein, a KRas G13C mutant protein, a KRas G13D mutant protein, a KRas Q61H mutant protein, and a KRas Q61L mutant protein. In some embodiments, a compound provided herein, or a pharmaceutically acceptable salt thereof, inhibits one or more mutant KRas proteins selected from the group consisting of: a KRas G12A mutant protein, a KRas G12C mutant protein, a KRas G12D mutant protein, a KRas G12S mutant protein, and a KRas G12V mutant protein. In some embodiments, a compound provided herein, or a pharmaceutically acceptable salt thereof, inhibits two or more mutant KRas proteins selected from the group consisting of: a KRas G12A mutant protein, a KRas G12C mutant protein, a KRas G12D mutant protein, a KRas G12S mutant protein, and a KRas G12V mutant protein. In some embodiments, a compound provided herein, or a pharmaceutically acceptable salt thereof, inhibits three or more mutant KRas proteins selected from the group consisting of: a KRas G12A mutant protein, a KRas G12C mutant protein, a KRas G12D mutant protein, a KRas G12S mutant protein, and a KRas G12V mutant protein. In some embodiments, a compound provided herein, or a pharmaceutically acceptable salt thereof, inhibits one or more mutant KRas proteins selected from the group consisting of: a KRas G12A mutant protein, a KRas G12D mutant protein, and a KRas G12V mutant protein. In some embodiments, a compound provided herein, or a pharmaceutically acceptable salt thereof, inhibits two or more mutant KRas proteins selected from the group consisting of: a KRas G12A mutant protein, a KRas G12D mutant protein, and a KRas G12V mutant protein. In some embodiments, a compound provided herein, or a pharmaceutically acceptable salt thereof, inhibits a KRas G12A mutant protein, a KRas G12D mutant protein, and a KRas G12V mutant protein. In some such embodiments, the compounds provided herein, or pharmaceutically acceptable salts thereof, are useful for treating a lung cancer (e.g., non-small cell lung cancer).

In some embodiments, a compound provided herein, or a pharmaceutically acceptable salt thereof, inhibits one or more mutant KRas proteins selected from the group consisting of: a KRas G12A mutant protein, a KRas G12C mutant protein, a KRas G12D mutant protein, a KRas G12R mutant protein, a KRas G12S mutant protein, a KRas G12V mutant protein, a KRas G13C mutant protein, a KRas Q61H mutant protein, and a KRas Q61R mutant protein. In some embodiments, a compound provided herein, or a pharmaceutically acceptable salt thereof, inhibits two or more mutant KRas proteins selected from the group consisting of: a KRas G12A mutant protein, a KRas G12C mutant protein, a KRas G12D mutant protein, a KRas G12R mutant protein, a KRas G12S mutant protein, a KRas G12V mutant protein, a KRas G13C mutant protein, a KRas Q61H mutant protein, and a KRas Q61R mutant protein. In some embodiments, a compound provided herein, or a pharmaceutically acceptable salt thereof, inhibits three or more mutant KRas proteins selected from the group consisting of: a KRas G12A mutant protein, a KRas G12C mutant protein, a KRas G12D mutant protein, a KRas G12R mutant protein, a KRas G12S mutant protein, a KRas G12V mutant protein, a KRas G13C mutant protein, a KRas Q61H mutant protein, and a KRas Q61R mutant protein. In some embodiments, a compound provided herein, or a pharmaceutically acceptable salt thereof, inhibits one or more mutant KRas proteins selected from the group consisting of: a KRas G12A mutant protein, a KRas G12C mutant protein, a KRas G12D mutant protein, a KRas G12R mutant protein, a KRas G12S mutant protein, and a KRas G12V mutant protein. In some embodiments, a compound provided herein, or a pharmaceutically acceptable salt thereof, inhibits two or more mutant KRas proteins selected from the group consisting of: a KRas G12A mutant protein, a KRas G12C mutant protein, a KRas G12D mutant protein, a KRas G12R mutant protein, a KRas G12S mutant protein, and a KRas G12V mutant protein. In some embodiments, a compound provided herein, or a pharmaceutically acceptable salt thereof, inhibits three or more mutant KRas proteins selected from the group consisting of: a KRas G12A mutant protein, a KRas G12C mutant protein, a KRas G12D mutant protein, a KRas G12R mutant protein, a KRas G12S mutant protein, and a KRas G12V mutant protein. In some embodiments, a compound provided herein, or a pharmaceutically acceptable salt thereof, inhibits one or more mutant KRas proteins selected from the group consisting of: a KRas G12A mutant protein, a KRas G12D mutant protein, a KRas G12R mutant protein, and a KRas G12V mutant protein. In some embodiments, a compound provided herein, or a pharmaceutically acceptable salt thereof, inhibits two or more mutant KRas proteins selected from the group consisting of: a KRas G12A mutant protein, a KRas G12D mutant protein, a KRas G12R mutant protein, and a KRas G12V mutant protein. In some embodiments, a compound provided herein, or a pharmaceutically acceptable salt thereof, inhibits three or more mutant KRas proteins selected from the group consisting of: a KRas G12A mutant protein, a KRas G12D mutant protein, a KRas G12R mutant protein, and a KRas G12V mutant protein. In some such embodiments, the compounds provided herein, or pharmaceutically acceptable salts thereof, are useful for treating a pancreatic cancer.

In some embodiments, a compound provided herein, or a pharmaceutically acceptable salt thereof, inhibits one or more mutant KRas proteins selected from the group consisting of: a KRas G12A mutant protein, a KRas G12R mutant protein, a KRas G12S mutant protein, a KRas G12V mutant protein, a KRas Q61L mutant protein, a KRas Q61P mutant protein, and a KRas Q61R mutant protein. In some embodiments, a compound provided herein, or a pharmaceutically acceptable salt thereof, inhibits two or more mutant KRas proteins selected from the group consisting of: a KRas G12A mutant protein, a KRas G12R mutant protein, a KRas G12S mutant protein, a KRas G12V mutant protein, a KRas Q61L mutant protein, a KRas Q61P mutant protein, and a KRas Q61R mutant protein. In some embodiments, a compound provided herein, or a pharmaceutically acceptable salt thereof, inhibits three or more mutant KRas proteins selected from the group consisting of: a KRas G12A mutant protein, a KRas G12R mutant protein, a KRas G12S mutant protein, a KRas G12V mutant protein, a KRas Q61L mutant protein, a KRas Q61P mutant protein, and a KRas Q61R mutant protein. In some embodiments, a compound provided herein, or a pharmaceutically acceptable salt thereof, inhibits one or more mutant KRas proteins selected from the group consisting of: a KRas G12A mutant protein, a KRas G12R mutant protein, a KRas G12S mutant protein, and a KRas G12V mutant protein. In some embodiments, a compound provided herein, or a pharmaceutically acceptable salt thereof, inhibits two or more mutant KRas proteins selected from the group consisting of: a KRas G12A mutant protein, a KRas G12R mutant protein, a KRas G12S mutant protein, and a KRas G12V mutant protein. In some embodiments, a compound provided herein, or a pharmaceutically acceptable salt thereof, inhibits three or more mutant KRas proteins selected from the group consisting of: a KRas G12A mutant protein, a KRas G12R mutant protein, a KRas G12S mutant protein, and a KRas G12V mutant protein. In some embodiments, a compound provided herein, or a pharmaceutically acceptable salt thereof, inhibits one or more mutant KRas proteins selected from the group consisting of: a KRas G12A mutant protein, a KRas G12R mutant protein, and a KRas G12V mutant protein. In some embodiments, a compound provided herein, or a pharmaceutically acceptable salt thereof, inhibits two or more mutant KRas proteins selected from the group consisting of: a KRas G12A mutant protein, a KRas G12R mutant protein, and a KRas G12V mutant protein. In some embodiments, a compound provided herein, or a pharmaceutically acceptable salt thereof, inhibits a KRas G12A mutant protein, a KRas G12R mutant protein, and a KRas G12V mutant protein. In some such embodiments, the compounds provided herein, or pharmaceutically acceptable salts thereof, are useful for treating a testicular cancer (e.g., seminoma).

In some embodiments, a compound provided herein, or a pharmaceutically acceptable salt thereof, can bind to a KRas protein in the GTP-bound state. In some embodiments, a compound provided herein, or a pharmaceutically acceptable salt thereof, can bind selectively to a KRas protein in the GTP-bound state.

In some embodiments, a compound provided herein, or a pharmaceutically acceptable salt thereof, can bind to a KRas protein in the GDP-bound state. In some embodiments, a compound provided herein, or a pharmaceutically acceptable salt thereof, can bind selectively to a KRas protein in the GDP-bound state.

An exemplary sequence of mature human KRas protein is shown below (UniProtKB entry P01116) (SEQ ID NO: 1)

As used herein, "selective" or "selectively", when referring to an assayed compound, indicates at least a 5-fold (e.g., at least a 10-fold, at least a 25-fold, at least a 50-fold, or at least a 100-fold) superior performance in an assay (e.g., binding affinity and/or potency) for a specified condition with reference to a comparator protein variant in the assay. For example, if a compound provided herein, or a pharmaceutically acceptable salt thereof, binds "selectively" to a KRas G12X mutant protein over the wild type KRas protein as determined by a surface plasmon resonance (SPR) assay, then the compound provided herein, or a pharmaceutically acceptable salt thereof, has at least a 5-fold (e.g., at least a 10-fold, at least a 25-fold, at least a 50-fold, or at least a 100-fold) smaller K_{D} value for any one or more KRas mutant proteins selected from the group consisting of the KRas G12X mutant proteins than for the wild type KRas protein when measured by the SPR assay. As a further example, if a compound provided herein, or a pharmaceutically acceptable salt thereof, "selectively" reduces the viability of the KRas G12V mutant protein-expressing cells over the cells expressing KRas G12C protein as determined by a cell proliferation assay, then the compound has at least a 5-fold (e.g., at least a 10-fold, at least a 25-fold, at least a 50-fold, or at least a 100-fold) smaller EC₅₀ value for the KRas G12V mutant protein-expressing cells than for the KRas G12C protein-expressing cells when measured by the cell proliferation assay. In another example, if a compound provided herein, or a pharmaceutically acceptable salt thereof, "selectively" inhibits a KRas G13X mutant protein over the wild type KRas protein as determined by a Raf kinase interaction assay, then the compound provided herein, or a pharmaceutically acceptable salt thereof, has at least a 5-fold (e.g., at least a 10-fold, at least a 25-fold, at least a 50-fold, or at least a 100-fold) smaller IC₅₀ value for the KRas G13X protein than for the wild type KRas protein when measured by the Raf kinase interaction assay. As a further example, if a compound provided herein, or a pharmaceutically acceptable salt thereof, "selectively" inhibits the KRas G12R mutant protein over the wild type KRas protein as determined by a nucleotide exchange assay, then the compound provided herein, or a pharmaceutically acceptable salt thereof, has at least a 5-fold (e.g., at least a 10-fold, at least a 25-fold, at least a 50-fold, or at least a 100-fold) smaller IC₅₀ value for the KRas G12R mutant protein than for the wild type KRas protein when measured by the nucleotide exchange assay.

In some embodiments, a compound provided herein, or a pharmaceutically acceptable salt thereof, is a pan mutant KRas inhibitor (i.e., can inhibit two or more mutant KRas proteins (e.g., two or more of a KRas G12A mutant protein, a KRas G12D mutant protein, a KRas G12R mutant protein, and a KRas G12V mutant protein)). For example, such a compound can inhibit each mutant KRas protein (e.g., two or more mutant KRas proteins) with an IC₅₀ of less than 1 µM (e.g., less than 750 nM, less than 500 nM, or less than 200 nM). As another example, such a compound can inhibit ERK phosphorylation in cell lines each expressing a mutant KRas protein with an independent IC₅₀ of less than 1 µM (e.g., less than 750 nM, less than 500 nM, or less than 200 nM) in at least two of the cell lines. For example, a compound provided herein, or a pharmaceutically acceptable salt thereof, can inhibit ERK phosphorylation in a cell line expressing a KRas G12R mutant protein with an IC₅₀ of less than 1 µM, and the compound provided herein, or a pharmaceutically acceptable salt thereof, can inhibit ERK phosphorylation in a cell line expressing a KRas G12V mutant protein with an IC₅₀ of less than 1 µM. In some embodiments, a compound provided herein, or a pharmaceutically acceptable salt thereof, is a pan KRas inhibitor (i.e., the compound can inhibit wild type KRas and one or more mutant KRas proteins). In some embodiments, a compound provided herein, or a pharmaceutically acceptable salt thereof, does not inhibit certain KRas proteins (e.g., wild type KRas or one or more dysregulated KRas proteins). For example, such a compound can inhibit the interaction between a KRas protein (e.g., a dysregulated KRas protein) and one or more Raf proteins with an IC₅₀ of 1 µM or greater than 1 µM (e.g., greater than 2 µM, greater than 5 µM, greater than 10 µM, or greater than 30 µM). As another example, such a compound can inhibit ERK phosphorylation in cell lines expressing the KRas protein (e.g., a dysregulated KRas protein) with an IC₅₀ of 1 µM or greater than 1 µM (e.g., greater than 2 µM, greater than 5 µM, greater than 10 µM, or greater than 30 µM).

In some embodiments, a compound provided herein, or a pharmaceutically acceptable salt thereof, inhibits a KRas G12D mutant protein and a KRas G12V mutant protein. In some such embodiments, a compound provided herein, or a pharmaceutically acceptable salt thereof, inhibits ERK phosphorylation in a cell line expressing a KRas G12D mutant protein (e.g., AGS, ASPC1, GP2D, LS180, Panc04.03, HPAFII, Panc02.03, A427, and HPAC) with an IC₅₀ that is within about 10-fold, i.e., within about 10-fold less or within about 10-fold more (e.g., within about 9-fold less or within about 9-fold more, within about 8-fold less or within about 8-fold more, within about 7-fold less or within about 7-fold more, within about 6-fold less or within about 6-fold more, within about 5-fold less or within about 5-fold more, or within about 2-fold less or within about 2-fold more) of the IC₅₀ measured for inhibition of ERK phosphorylation by the compound in a cell line expressing a KRas G12V mutant protein (e.g., SW620, H727, CFPAC1, CAPAN1, CAPAN2, RKN, H441, and SW480). For example, if the compound provided herein, or a pharmaceutically acceptable salt thereof, inhibits ERK phosphorylation in a cell line expressing a KRas G12D mutant protein with an IC₅₀ of about 150 nM, then the IC₅₀ measured for inhibition of ERK phosphorylation by the compound in a cell line expressing a KRas G12V mutant protein would be within about 10-fold more than about 150 nM, thus ranging from about 150 nM to about 1500 nM, or within about 10-fold less than 150 nM, thus ranging from about 15 nM to about 150 nM. In some embodiments, a compound provided herein, or a pharmaceutically acceptable salt thereof, can inhibit ERK phosphorylation in a GP2d cell line with an IC₅₀ that is within about 10-fold, i.e., within about 10-fold less or within about 10-fold more (e.g., within about 9-fold less or within about 9-fold more, within about 8-fold less or within about 8-fold more, within about 7-fold less or within about 7-fold more, within about 6-fold less or within about 6-fold more, within about 5-fold less or within about 5-fold more, or within about 2-fold less or within about 2-fold more) of the IC₅₀ measured for inhibition of ERK phosphorylation by the compound in a SW620 cell line. In some embodiments, a compound provided herein, or a pharmaceutically acceptable salt thereof, inhibits ERK phosphorylation in a cell line expressing a KRas G12D mutant protein (e.g., AGS, ASPC1, GP2D, LS180, Panc04.03, HPAFII, Panc02.03, A427, and HPAC) with an IC₅₀ that is within about 10-fold less (e.g., within about 9-fold less, within about 8-fold less, within about 7-fold less, within about 6-fold less, within about 5-fold less, or within about 2-fold less) than the IC₅₀ measured for inhibition of ERK phosphorylation by the compound in a cell line expressing a KRas G12V mutant protein (e.g., SW620, H727, CFPAC1, CAPAN1, CAPAN2, RKN, H441, and SW480). For example, a compound provided herein, or a pharmaceutically acceptable salt thereof, can inhibit ERK phosphorylation in a GP2d cell line with an IC₅₀ that within about 10-fold less (e.g., within about 9-fold less, within about 8-fold less, within about 7-fold less, within about 6-fold less, within about 5-fold less, or within about 2-fold less) than the IC₅₀ measured for inhibition of ERK phosphorylation by the compound in a SW620 cell line. In some embodiments, a compound provided herein, or a pharmaceutically acceptable salt thereof, inhibits ERK phosphorylation in a cell line expressing a KRas G12D mutant protein (e.g., AGS, ASPC1, GP2D, LS180, Panc04.03, HPAFII, Panc02.03, A427, and HPAC ) with an IC₅₀ that is within about 10-fold more (e.g., within about 9-fold more, within about 8-fold more, within about 7-fold more, within about 6-fold more, within about 5-fold more, or within about 2-fold more) than the IC₅₀ measured for inhibition of ERK phosphorylation by the compound in a cell line expressing a KRas G12V mutant protein (e.g., SW620, H727, CFPAC1, CAPAN1, CAPAN2, RKN, H441, and SW480). For example, a compound provided herein, or a pharmaceutically acceptable salt thereof, can inhibit ERK phosphorylation in a GP2d cell line with an IC₅₀ that is within about 10-fold more (e.g., within about 9-fold more, within about 8-fold more, within about 7-fold more, within about 6-fold more, within about 5-fold more, or within about 2-fold more) than the IC₅₀ measured for inhibition of ERK phosphorylation by the compound in a SW620 cell line.

In some such embodiments, a compound provided herein, or a pharmaceutically acceptable salt thereof, inhibits ERK phosphorylation in a cell line expressing a KRas G12V mutant protein (e.g., SW620, H727, CFPAC1, CAPAN1, CAPAN2, RKN, H441, and SW480) with an IC₅₀ of less than 250 nM (e.g., less than 200 nM, less than 150 nM, less than 125 nM, less than 100 nM, less than 75 nM, less than 50 nM, less than 30 nM). For example, a compound provided herein, or a pharmaceutically acceptable salt thereof, can inhibit ERK phosphorylation in a SW620 cell line with an IC₅₀ of less than 250 nM (e.g., less than 200 nM, less than 150 nM, less than 125 nM, less than 100 nM, less than 75 nM, less than 50 nM, less than 30 nM). In some such further embodiments, the compound provided herein, or a pharmaceutically acceptable salt thereof, inhibits ERK phosphorylation in a cell line expressing a KRas G12D mutant protein (e.g., AGS, ASPC1, GP2D, LS180, Panc04.03, HPAFII, Panc02.03, A427, and HPAC) with an IC₅₀ of less than 250 nM (e.g., less than 200 nM, less than 150 nM, less than 125 nM, less than 100 nM, less than 75 nM, less than 50 nM, less than 30 nM). For example, the compound provided herein, or a pharmaceutically acceptable salt thereof, can inhibit ERK phosphorylation in a GP2d cell line with an IC₅₀ of less than 250 nM (e.g., less than 200 nM, less than 150 nM, less than 125 nM, less than 100 nM, less than 75 nM, less than 50 nM, less than 30 nM).

In some embodiments, a compound provided herein, or a pharmaceutically acceptable salt thereof, inhibits ERK phosphorylation in a cell line expressing a KRas G12D mutant protein (e.g., AGS, ASPC1, GP2D, LS180, Panc04.03, HPAFII, Panc02.03, A427, and HPAC) with an IC₅₀ that is within about 10-fold, i.e., within about 10-fold less or within about 10-fold more (e.g., within about 9-fold less or within about 9-fold more, within about 8-fold less or within about 8-fold more, within about 7-fold less or within about 7-fold more, within about 6-fold less or within about 6-fold more, within about 5-fold less or within about 5-fold more, or within about 2-fold less or within about 2-fold more) of the IC₅₀ measured for inhibition of ERK phosphorylation by the compound in a cell line expressing a KRas G12V mutant protein (e.g., SW620, H727, CFPAC1, CAPAN1, CAPAN2, RKN, H441, and SW480), wherein the compound provided herein, or a pharmaceutically acceptable salt thereof, inhibits ERK phosphorylation in the cell line expressing a KRas G12V mutant protein (e.g., SW620, H727, CFPAC1, CAPAN1, CAPAN2, RKN, H441, and SW480) with an IC₅₀ of less than 250 nM (e.g., less than 200 nM, less than 150 nM, less than 125 nM, less than 100 nM, less than 75 nM, less than 50 nM, less than 30 nM). In some such embodiments, the compound provided herein, or a pharmaceutically acceptable salt thereof, inhibits ERK phosphorylation in the cell line expressing a KRas G12D mutant protein (e.g., AGS, ASPC1, GP2D, LS180, Panc04.03, HPAFII, Panc02.03, A427, and HPAC) with an IC₅₀ of less than 250 nM (e.g., less than 200 nM, less than 150 nM, less than 125 nM, less than 100 nM, less than 75 nM, less than 50 nM, less than 30 nM).

For example, a compound provided herein, or a pharmaceutically acceptable salt thereof, can inhibit ERK phosphorylation in a GP2d cell line with an IC₅₀ that is within about 10-fold, i.e., within about 10-fold less or within about 10-fold more (e.g., within about 9-fold less or within about 9-fold more, within about 8-fold less or within about 8-fold more, within about 7-fold less or within about 7-fold more, within about 6-fold less or within about 6-fold more, within about 5-fold less or within about 5-fold more, or within about 2-fold less or within about 2-fold more) of the IC₅₀ measured for inhibition of ERK phosphorylation by the compound in a SW620 cell line, wherein the compound provided herein, or a pharmaceutically acceptable salt thereof, inhibits ERK phosphorylation in a SW620 cell line with an IC₅₀ of less than 250 nM (e.g., less than 200 nM, less than 150 nM, less than 125 nM, less than 100 nM, less than 75 nM, less than 50 nM, less than 30 nM). In some such embodiments, the compound provided herein, or a pharmaceutically acceptable salt thereof, inhibits ERK phosphorylation in a GP2d cell line with an IC₅₀ of less than 250 nM (e.g., less than 200 nM, less than 150 nM, less than 125 nM, less than 100 nM, less than 75 nM, less than 50 nM, less than 30 nM). In some embodiments, a compound provided herein, or a pharmaceutically acceptable salt thereof, inhibits ERK phosphorylation in a cell line expressing a KRas G12D mutant protein (e.g., AGS, ASPC1, GP2D, LS180, Panc04.03, HPAFII, Panc02.03, A427, and HPAC) with an IC₅₀ that is within about 10-fold less (e.g., within about 9-fold less, within about 8-fold less, within about 7-fold less, within about 6-fold less, within about 5-fold less, or within about 2-fold less) than the IC₅₀ measured for inhibition of ERK phosphorylation by the compound in a cell line expressing a KRas G12V mutant protein (e.g., SW620, H727, CFPAC1, CAPAN1, CAPAN2, RKN, H441, and SW480), wherein the compound provided herein, or a pharmaceutically acceptable salt thereof, inhibits ERK phosphorylation in the cell line expressing a KRas G12V mutant protein (e.g., SW620, H727, CFPAC1, CAPAN1, CAPAN2, RKN, H441, and SW480) with an IC₅₀ of less than 250 nM (e.g., less than 200 nM, less than 150 nM, less than 125 nM, less than 100 nM, less than 75 nM, less than 50 nM, less than 30 nM). In some such embodiments, the compound provided herein, or a pharmaceutically acceptable salt thereof, inhibits ERK phosphorylation in the cell line expressing a KRas G12D mutant protein (e.g., AGS, ASPC1, GP2D, LS180, Panc04.03, HPAFII, Panc02.03, A427, and HPAC) with an IC₅₀ of less than 250 nM (e.g., less than 200 nM, less than 150 nM, less than 125 nM, less than 100 nM, less than 75 nM, less than 50 nM, less than 30 nM).

For example, a compound provided herein, or a pharmaceutically acceptable salt thereof, can inhibit ERK phosphorylation in a GP2d cell line with an IC₅₀ that is within about 10-fold less (e.g., within about 9-fold less, within about 8-fold less, within about 7-fold less, within about 6-fold less, within about 5-fold less, or within about 2-fold less) than the IC₅₀ measured for inhibition of ERK phosphorylation by the compound in a SW620 cell line, wherein the compound provided herein, or a pharmaceutically acceptable salt thereof, inhibits ERK phosphorylation in a SW620 cell line with an IC₅₀ of less than 250 nM (e.g., less than 200 nM, less than 150 nM, less than 125 nM, less than 100 nM, less than 75 nM, less than 50 nM, less than 30 nM). In some such embodiments, the compound provided herein, or a pharmaceutically acceptable salt thereof, inhibits ERK phosphorylation in a GP2d cell line with an IC₅₀ of less than 250 nM (e.g., less than 200 nM, less than 150 nM, less than 125 nM, less than 100 nM, less than 75 nM, less than 50 nM, less than 30 nM).

In some embodiments, a compound provided herein, or a pharmaceutically acceptable salt thereof, inhibits ERK phosphorylation in a cell line expressing a KRas G12D mutant protein (e.g., AGS, ASPC1, GP2D, LS180, Panc04.03, HPAFII, Panc02.03, A427, and HPAC) with an IC₅₀ that is within about 10-fold more (e.g., within about 9-fold more, within about 8-fold more, within about 7-fold more, within about 6-fold more, within about 5-fold more, or within about 2-fold more) than the IC₅₀ measured for inhibition of ERK phosphorylation by the compound in a cell line expressing a KRas G12V mutant protein (e.g., SW620, H727, CFPAC1, CAPAN1, CAPAN2, RKN, H441, and SW480), wherein the compound provided herein, or a pharmaceutically acceptable salt thereof, inhibits ERK phosphorylation in the cell line expressing a KRas G12V mutant protein (e.g., SW620, H727, CFPAC1, CAPAN1, CAPAN2, RKN, H441, and SW480) with an IC₅₀ of less than 250 nM (e.g., less than 200 nM, less than 150 nM, less than 125 nM, less than 100 nM, less than 75 nM, less than 50 nM, less than 30 nM). In some such embodiments, the compound provided herein, or a pharmaceutically acceptable salt thereof, inhibits ERK phosphorylation in the cell line expressing a KRas G12D mutant protein (e.g., AGS, ASPC1, GP2D, LS180, Panc04.03, HPAFII, Panc02.03, A427, and HPAC) with an IC₅₀ of less than 250 nM (e.g., less than 200 nM, less than 150 nM, less than 125 nM, less than 100 nM, less than 75 nM, less than 50 nM, less than 30 nM).

For example, a compound provided herein, or a pharmaceutically acceptable salt thereof, can inhibit ERK phosphorylation in a GP2d cell line with an IC₅₀ that is within about 10-fold more (e.g., within about 9-fold more, within about 8-fold more, within about 7-fold more, within about 6-fold more, within about 5-fold more, or within about 2-fold more) than the IC₅₀ measured for inhibition of ERK phosphorylation by the compound in a SW620 cell line, wherein the compound provided herein, or a pharmaceutically acceptable salt thereof, inhibits ERK phosphorylation in a SW620 cell line with an IC₅₀ of less than 250 nM (e.g., less than 200 nM, less than 150 nM, less than 125 nM, less than 100 nM, less than 75 nM, less than 50 nM, less than 30 nM). In some such embodiments, the compound provided herein, or a pharmaceutically acceptable salt thereof, inhibits ERK phosphorylation in a GP2d cell line with an IC₅₀ of less than 250 nM (e.g., less than 200 nM, less than 150 nM, less than 125 nM, less than 100 nM, less than 75 nM, less than 50 nM, less than 30 nM).

The ability of a compound provided herein, or a pharmaceutically acceptable salt thereof, to bind to a KRas protein can be measured, for example, by a direct determination method (e.g., surface plasmon resonance or isothermal titration calorimetry); by radio labelling the compound prior to binding, isolating the compound/protein complex, and determining the amount of radio label bound; or by running a competition experiment where new compounds are incubated with the protein bound to known radioligands. As another example, the occupancy of a compound provided herein, or a pharmaceutically acceptable salt thereof, can be determined using a proximity-based technique, such as time-resolved Fluorescence Resonance Energy Transfer (FRET); for instance, using a labeled probe that binds mutually exclusively with the inhibitor, and using an antibody that binds to a position on the protein separate from where the compound provided herein, or a pharmaceutically acceptable salt thereof, binds (for example, an antibody that binds to an N-terminal tag). It will be understood that the antibody and probe can be tagged with any appropriate FRET pair. See, e.g., International Publication Nos. WO 2021/041671, WO 2021/120890, and U.S. Publication No. US 2021/0179633.

In some cases, binding affinities (e.g., as measured by dissociation constant K_{D}) of the compounds provided herein, or pharmaceutically acceptable salts thereof with a KRas protein (e.g., a wild type KRas protein or a mutant KRas protein) in the GDP-bound and/or GTP-bound state can be measured using methods known in the art (e.g., using SPR (e.g., using one or more methods described herein (e.g., using the methods described in **Example B1** or in **Example B5** herein))). Binding affinity with the KRas protein in the GDP-bound state can be measured by loading the KRas protein with GDP (e.g., at the concentrations described in **Example B1** or in **Example B5).** Binding affinity with the KRas protein in the GTP-bound state can be measured by loading the KRas protein with GMPPNP (e.g., at the concentrations described in **Example B1).**

Another exemplary assay for determining the potency of a compound provided herein, or a pharmaceutically acceptable salt thereof, includes measuring the effect of the compound provided herein, or a pharmaceutically acceptable salt thereof, on cell proliferation. Cell proliferation assays can be performed in a number of formats, including 2D and 3D. Similarly, a cell proliferation assay can be performed with any appropriate cell line, including, for example, A375, A427, A549, AGS, ASPC1, CAL62, CALU1, CAPAN1, CAPAN2, CFPAC1, GP2D, H358, H441, H460, H727, HCT116, HKA1, HPAC, HPAFII, HTK, HUPT3, KMS20, KP2, LS123, LS180, MIAPaCa-2, MKN1, NCI-H1993, NCI-H211, NCI-H424, NCI-H526, Panc02.03, Panc04.03, PATC50, PC9, PK8, PSN1, RKN, SW480, SW620, and/or TCCPAN2. In some embodiments, the cell line can be AGS, A375, A427, ASPC1, H727, H441, RKN, and/or SW620. As an illustrative example, a 3D cell proliferation assay can include growing cells in a 3D medium, contacting the cells with a compound provided herein, or a pharmaceutically acceptable salt thereof, measuring the cellular proliferation using an appropriate reagent (e.g., CELLTITERGLO^{®} 3D), and then comparing the signal from the experiment with the compound provided herein, or a pharmaceutically acceptable salt thereof, to the signal from a control experiment (e.g., lacking a compound provided herein). As another illustrative example, a 2D cell proliferation assay can include plating cells onto a growth surface, optionally letting the cells grow for a period of time, contacting the cells with a compound provided herein, or a pharmaceutically acceptable salt thereof, measuring the cellular proliferation using an appropriate reagent (e.g., CELLTITERGLO^{®}), and then comparing the signal from the experiment with a compound provided herein, or a pharmaceutically acceptable salt thereof, to the signal from a control experiment (e.g., lacking a compound provided herein, or a pharmaceutically acceptable salt thereof). See, e.g., **Example B7** herein. In some embodiments, cellular proliferation can be assessed using a platform for live cell imaging (e.g., an INCUCYTE^{®} SX5 Live-Cell Analysis Instrument). See also, e.g., U.S. Publication No. US 2021/0179633, US 2021/0230142, and US 2019/0284144.

As another example, the potency and/or efficacy of a compound provided herein, or a pharmaceutically acceptable salt thereof, can be evaluated in an animal model, for example, a xenograft model (e.g., using an established cancer cell line such as AGS, A375, A427, ASPC1, H727, H441, RKN, and/or SW620, or a patient-derived xenograft (PDX) model). See, e.g., U.S. Publication No. US 2021/0179633.

In some embodiments, the potency and/or efficacy of a compound provided herein, or a pharmaceutically acceptable salt thereof can be evaluated in a cell-derived xenograft (CDX) model. In some embodiments, a compound provided herein, or a pharmaceutically acceptable salt thereof, is assessed in a CDX (e.g., H727, RKN, or SW620) mouse model. For example, mice can be implanted with a cell line of interest (e.g., H727, RKN, or SW620) and the tumor allowed to grow for a period of time, then the mice can be administered a compound provided herein, or a pharmaceutically acceptable salt thereof. The effect of the compound provided herein, or a pharmaceutically acceptable salt thereof, can be determined by measuring tumor growth (or regression). An exemplary protocol follows.

All the procedures related to animal handling, care, and treatment in the efficacy study are performed according to guidelines approved by the Institutional Animal Care and Use Committee (IACUC) following the guidance of the Association for Assessment and Accreditation of Laboratory Animal Care (AAALAC). 6-8 week old BALB/c nude female mice are inoculated subcutaneously on the right flank with 5 × 10⁶ H727, RKN, or SW620 tumor cells in 0.1 mL of 1:1 medium/Matrigel for tumor development. Treatments start and groupings are assigned when the mean tumor volume reaches about 175-225 mm³. Based on the tumor volume, mice are randomly assigned to respective groups such that the average starting tumor size is the same for each treatment group. Tumor-bearing mice are treated orally twice daily with a compound provided herein, or a pharmaceutically acceptable salt thereof (e.g., a dose of about 1 mg/kg to about 200 mg/kg, such as 1 mg/kg, 3 mg/kg, 5 mg/kg, 10 mg/kg, 20 mg/kg, 30 mg/kg, 50 mg/kg, 75 mg/kg, 100 mg/kg, 150 mg/kg, or 200 mg/kg). The body weight of each animal is measured and recorded twice weekly throughout the study. The measurement of tumor size is conducted twice weekly with a caliper and recorded. The tumor volume (mm³) is estimated using the formula: TV=a × b²/2, where "a" and "b" are long and short diameters of a tumor, respectively. In some embodiments, the tumor volume is plotted as a function of time.

Additional assays can include, for example, assays based on hydrogen exchange (HX) mass spectrometry. Such assays can be useful, for example, to evaluate whether a compound (e.g., a compound provided herein, or a pharmaceutically acceptable salt thereof) stabilizes the GTP-bound state or GDP-bound state of a KRas protein (e.g., a dysregulated KRas protein, e.g., a mutant KRas protein (e.g., a KRas G12D mutant protein, a KRas G12R mutant protein, or a KRas G12V mutant protein)). In such assays, the rate of hydrogen exchange of the backbone amide hydrogens can be measured for a KRas protein (e.g., a dysregulated KRas protein, e.g., a mutant KRas protein (e.g., a KRas G12D mutant protein, a KRas G12R mutant protein, or a KRas G12V mutant protein)) bound to a non-hydrolyzable GTP mimic (GMPPNP), GDP, or a compound provided herein, or a pharmaceutically acceptable salt thereof. See, e.g., Lim et al. Angew Chem Int Ed Engl. 2014; 53(1): 199-204, doi: 10.1002/anie.201307387.

In some embodiments, potency of a compound provided herein, or a pharmaceutically acceptable salt thereof, as provided herein can be determined by EC₅₀ value. A compound with a lower EC₅₀ value, as determined under substantially similar conditions, is a more potent inhibitor relative to a compound with a higher EC₅₀ value. In some embodiments, an EC₅₀ value can be determined (e.g., using a KRas-dependent phosphorylation level (e.g., a phosphoERK level (sometimes called a "pERK" level)) or using a cell viability assay) in cells (e.g., in tumor cells, (e.g., cell lines such as A375, A427, A549, AGS, ASPC1, CAL62, CALU1, CAPAN1, CAPAN2, CFPAC1, GP2D, H358, H441, H460, H727, HCT116, HKA1, HPAC, HPAFII, HTK, HUPT3, KMS20, KP2, LS123, LS180, MIAPaCa-2, MKN1, NCI-H1993, NCI-H211, NCI-H424, NCI-H526, Panc02.03, Panc04.03, PATC50, PC9, PK8, PSN1, RKN, SW480, SW620, and/or TCCPAN2) expressing a KRas protein, such as a dysregulated KRas protein (e.g., a mutant KRas protein or an amplified KRas protein), or a fragment thereof).

In some embodiments, potency of a compound provided herein, or a pharmaceutically acceptable salt thereof, as provided herein can also be determined by IC₅₀ value. A compound with a lower IC₅₀ value, as determined under substantially similar conditions, is a more potent inhibitor relative to a compound with a higher IC₅₀ value. In some embodiments, an IC₅₀ value can be determined (e.g., using a KRas-dependent phosphorylation level (e.g., a phosphoERK level) or using a cell viability assay), in cells (e.g., in tumor cells, (e.g., cell lines such as A375, A427, A549, AGS, ASPC1, CAL62, CALU1, CAPAN1, CAPAN2, CFPAC1, GP2D, H358, H441, H460, H727, HCT116, HKA1, HPAC, HPAFII, HTK, HUPT3, KMS20, KP2, LS123, LS180, MIAPaCa-2, MKN1, NCI-H1993, NCI-H211, NCI-H424, NCI-H526, Panc02.03, Panc04.03, PATC50, PC9, PK8, PSN1, RKN, SW480, SW620, and/or TCCPAN2) expressing a KRas protein, such as a dysregulated KRas protein (e.g., a mutant KRas protein or an amplified KRas protein), or a fragment thereof).

In some embodiments, measuring the potency of a compound provided herein, or a pharmaceutically acceptable salt thereof, includes measuring the phosphorylation of a downstream kinase, such as ERK (e.g., ERK1 and/or ERK2) or MEK. Such assays can be used to measure the inhibition of KRas signaling activity, for instance, in a cell line (e.g., A375, A427, A549, AGS, ASPC1, CAL62, CALU1, CAPAN1, CAPAN2, CFPAC1, GP2D, H358, H441, H460, H727, HCT116, HKA1, HPAC, HPAFII, HTK, HUPT3, KMS20, KP2, LS123, LS180, MIAPaCa-2, MKN1, NCI-H1993, NCI-H211, NCI-H424, NCI-H526, Panc02.03, Panc04.03, PATC50, PC9, PK8, PSN1, RKN, SW480, SW620, and/or TCCPAN2 (e.g., AGS, A375, A427, ASPC1, H727, H441, RKN, and/or SW620)). For example, cells can be contacted with a compound provided herein, or a pharmaceutically acceptable salt thereof for a period of time, then lysed or permeabilized, and total ERK or MEK and phosphoERK or phosphoMEK content can be determined (e.g., using antibodies, or a kit, such as Invitrogen InstantOne ERK1/ERK2 (Phospho) [pT202/pY204]/[pT185/pY187] ELISA, MesoScale Discovery p/t ERK1/2, AlphaScreen SUREFIRE^{®} p-ERK1/2 (Thr202/Tyr204), or an HTRF^{®} PhosphoERK (Thr202/Tyr204) cellular kit (CisBio)). In some embodiments, multiple concentrations of a compound provided herein, or a pharmaceutically acceptable salt thereof can be used to construct a dose response curve. See, e.g., **Example B6** herein. See, e.g., International Publication No. WO 2021/041671, U.S. Publication Nos. US 2021/0122764, US 2018/0334454, US 2021/0179633, US 2018/0334454, and US 2019/0144444.

An exemplary ERK phosphorylation protocol follows. In some embodiments, an ERK phosphorylation assay can be carried out using the AlphaLisa SUREFIRE^{®} Ultra Multiplex Phospho/Total ERK1/2 (Thr202/Tyr204) Assay Kit. In a plate (e.g., a white, opaque-bottom Perkin Elmer CulturPlate-384 (product number 6007680)), cells are seeded at the desired concentration one day prior to treatment with compounds provided herein, or pharmaceutically acceptable salts thereof, and incubated overnight in a standard 37 °C, 5% CO₂ humidified incubator. The cells can be any cells of interest, such as MIAPaCa-2 (KRas G12C), H358 (KRas G12C), AGS (KRas G12D), ASPC1 (KRas G12D), GP2D (KRas G12D), LS180 (KRas G12D), Panc04.03 (KRas G12D), HPAFII (KRas G12D), Panc02.03 (KRas G12D), A427 (KRas G12D), HPAC (KRas G12D), TCCPAN2 (KRas G12R), PSN1 (KRas G12R), KP2 (KRas G12R), LS123 (KRas G12S), SW620 (KRas G12V), H727 (KRas G12V), CFPAC1 (KRas G12V), CAPAN1 (KRas G12V), CAPAN2 (KRas G12V), RKN (KRas G12V), H441 (KRas G12V), SW480 (KRas G12V), PACADD159 (KRas G12V/G12S), HS766T (KRas Q61H), H460 (KRas Q61H), PANC0213 (KRas Q61R), or A3735 (KRas WT). The day after seeding, compounds provided herein, or pharmaceutically acceptable salts thereof, are dispensed into the treatment plates (e.g., using a Tecan D300e compound printer in 9-point DRC format (1:3 dilution), 10-µM top concentration, in triplicate). Treatment plates are then returned to a standard 37 °C, 5% CO₂ humidified incubator for the pre-determined treatment time. Following compound treatment, all media is removed from the treatment plate(s), and the cells are subsequently lysed (e.g., using 1X Lysis Buffer in accordance with manufacturer protocol). Next, the *Acceptor Mix* (prepared in accordance with manufacturer's protocol) is added to each well of the assay plate and incubated on an orbital shaker at room temperature for 2 hours. Following incubation with the *Acceptor Mix,* the *Donor Mix* (prepared in accordance with manufacturer protocol) is added to each well of the assay plate, covered to protect from light, and incubated on an orbital shaker at room temperature overnight. Assay plates are read the following day (e.g., on a BMG Labtech PHERAstar FSX microplate reader). Data are then analyzed by calculating the ratio of ERK1/2-phosphorylation relative to Total ERK1/2 for each individual well. $Ratio pERK 1 / 2 = \frac{615 nm Signal \left(pERK1/2\right)}{545 nm Signal \left(Total ERK1/2\right)}$

The replicate ratios for each concentration are averaged and normalized to a DMSO control or other corresponding co-treatment before performing a variable slope (4-parameter), non-linear regression curve fit for each compound of interest. Data can be reported as IC₅₀ values.

In some embodiments, the compounds provided herein, or pharmaceutically acceptable salts thereof, inhibit ERK phosphorylation in a cell line expressing a KRas protein (e.g., a dysregulated KRas protein (e.g., a mutant KRas protein (e.g., a KRas G12D mutant protein, a KRas G12R mutant protein, or a KRas G12V mutant protein))) with an IC₅₀ of less than 1 µM (e.g., less than 750 nM, less than 500 nM, or less than 200 nM). In some embodiments, the compounds inhibit ERK phosphorylation in a cell line expressing the KRas protein (e.g., a dysregulated KRas protein (e.g., a mutant KRas protein (e.g., a KRas G12D mutant protein, a KRas G12R mutant protein, or a KRas G12V mutant protein))) with an IC₅₀ of less than 200 nM (e.g., less than 150 nM, less than 200 nM, less than 100 nM, less than 10 nM, less than 1 nM). For example, the compounds can inhibit ERK phosphorylation in a cell line expressing the KRas protein (e.g., a dysregulated KRas protein (e.g., a mutant KRas protein (e.g., a KRas G12D mutant protein, a KRas G12R mutant protein, or a KRas G12V mutant protein))) with an IC₅₀ of 0.1 nM to 100 nM, 0.1 nM to 50 nM, 1 nM to 50 nM, or 1 nM to 20 nM.

In some cases, a KRas A59G mutant protein (e.g., as a single mutant or as a double mutant with another mutation of interest, e.g., KRas G12X) can be used to "lock" the KRas protein in the GTP-bound state (e.g., by abrogating the GTPase activity of the protein); such an assay can be useful, for example, to determine the affinity of a compound provided herein, or a pharmaceutically acceptable salt thereof for the GTP-bound state and/or to determine the effect of the compound on downstream signaling (e.g., interaction with an RBD and/or the phosphorylation of a downstream kinase, such as ERK), potentially independent of the GTP cycling of the KRas protein. See, e.g., Hall, et al. Proceedings of the National Academy of Sciences 99.19 (2002): 12138-12142, doi: 10.1073/pnas.192453199; Lu, et al. Biochemistry 57.3 (2018): 324-333, doi: 10.1021/acs.biochem.7b00974; and Lim, Shuhui, et al. Chemical Science 12.48 (2021): 15975-15987, doi: 10.1039/D1SC05187C.

In some embodiments, the potency of a compound provided herein, or a pharmaceutically acceptable salt thereof, as a KRas inhibitor can be evaluated by its effect on the nucleotide exchange of GDP for GTP. For example, nucleotide exchange can be measured via the increase in fluorescence of protein-bound N-methylanthraniloyl (MANT)-GDP upon the addition of an excess amount of a non-hydrolyzable GTP analog such as guanosine-5'-[(β,γ)-imido]triphosphate (GppNHp, sometimes also referred to as GMPPNP), when exchange is inhibited. See, e.g., Kanie and Jackson, Bio Protoc. 2018; 8(7): e2795, doi: 10.21769/BioProtoc.2795. As another example, nucleotide exchange can be measured via the decrease in fluorescence of an incubated mixture of KRas protein-bound fluorophore-tagged GDP (e.g., Bodipy-GDP (e.g., EDA-GTP-DY-647P1)) and a compound provided herein, or a pharmaceutically acceptable salt thereof, followed by treatment with unlabeled GTP. In such an assay, an exchange of fluorophore-tagged GDP (e.g., Bodipy-GDP) for unlabeled GTP results in a reduced TR-FRET signal. As another example, nucleotide exchange can be measured via the increase in fluorescence of an incubated mixture of KRas protein-bound GDP and a compound provided herein, or a pharmaceutically acceptable salt thereof, followed by treatment with labeled GTP. In such an assay, an exchange of GDP for labeled GTP results in an increased FRET signal. See, e.g., International Publication No. WO 2020/085493 and U.S. Publication Nos. US 2021/0122764, US 2021/0269434, and US 2018/0334454. In some embodiments of nucleotide exchange assays, a guanine nucleotide exchange factor (e.g., SOS1) can be added to accelerate nucleotide exchange.

Inhibition of SOS1-catalyzed exchange of GDP for GTP on the KRas protein by compounds provided herein, or pharmaceutically acceptable salts thereof can be measured using methods known in the art (e.g., using one or more methods described herein (e.g., using methods described in **Example B2** herein)). Additional examples of in vitro assays include assays that determine inhibition of the GTPase activity of KRas protein. In some embodiments, the potency of a compound provided herein, or a pharmaceutically acceptable salt thereof, can be evaluated by its effect on GTPase activity (or lack thereof, as a decrease in GTPase activity is generally believed to be associated with aberrant signaling). For example, GTPase activity of a KRas protein can be measured using a phosphate assay system that continuously measures phosphate release. In some embodiments, a purine nucleoside phosphorylase-based (PNP) assay can be used to measure GTPase activity of a KRas protein. See, e.g., Hunter et al. Mol Cancer Res. 2015; 13(9):1325-35, doi: 10.1158/1541-7786.MCR-15-0203. In some embodiments, an enzyme-linked immunosorbent assay (ELISA) can be used to measure the effect of a compound provided herein, or a pharmaceutically acceptable salt thereof, on the GTPase activity of a KRas protein (e.g., a dysregulated KRas protein, e.g., a mutant KRas protein (e.g., a KRas G12D mutant protein, a KRas G12R mutant protein, or a KRas G12V mutant protein)), for example, by detecting a change in the amount of GST-Ras-RBD that binds to the KRas protein following pull-down and antibody detection of the complex. See, e.g., US 2021/0179633.

An exemplary SOS1-catalyzed nucleotide exchange assay protocol follows. GST-KRas G12R (1-169) loaded with GDP nucleotide is mixed with Anti-GST (Cisbio) antibody in assay buffer (20 mM HEPES pH 7.4, 150 mM NaCl, 5 mM MgCl₂, 1 mM DTT, 0.005% NP40, 1% DMSO) to produce a 1.5x solution. 10 µL of the 1.5x KRas-Ab solution is added to wells of a black, low-volume 384-well assay plate. Compounds provided herein, or pharmaceutically acceptable salts thereof, are added to wells using acoustic transfer technology. A 10-point dose response of each compound is performed with a 30 µM top dose. The KRas/Ab-compound mixture is incubated 1 hour at room temperature. A 3x solution of SOS1 (564-1049) and EDA-GTP-DY-647P1 (Jena Bioscience) is prepared in assay buffer. 5 µL of the SOS1-labeled GTP solution is added to the wells to initiate the nucleotide exchange reaction. The final concentration of KRas G12R and SOS1 are 10 nM and 200 nM, respectively. Time resolved fluorescence is read on a PHERAstar plate reader equipped with a filter module with excitation = 337 nm and emission 1 = 620 nm, emission 2 = 665 nm. The HTRF signal is calculated as the ratio of fluorescence intensity [emission 665 nm]/[emission 620 nm]. IC₅₀ values are calculated using a four-parameter, variable response sigmoidal dose response curve fit in Graphpad Prism software.

In some embodiments, a compound provided herein, or a pharmaceutically acceptable salt thereof, inhibits SOS1-catalyzed exchange of GDP for GTP on the KRas protein with an IC₅₀ of less than 1 µM (e.g., less than 750 nM, less than 500 nM, or less than 200 nM). In some embodiments, the compound inhibits SOS1-catalyzed exchange of GDP for GTP on the KRas protein with an IC₅₀ of less than 200 nM (e.g., less than 150 nM, less than 200 nM, less than 100 nM, less than 10 nM, less than 1 nM, less than 0.1 nM, or less than 0.01 nM). For example, the compound can inhibit SOS1-catalyzed exchange of GDP for GTP on the KRas protein with an IC₅₀ of 0.001 nM to 500 nM, 0.005 nM to 100 nM, 0.025 nM to 100 nM, 0.1 nM to 50 nM, or 0.1 nM to 10 nM.

Additional assays for evaluating the potency of a compound provided herein, or a pharmaceutically acceptable salt thereof, can also include, for example, a RAF kinase interaction assay. Such assays can be used to measure the affinity of KRas:nucleotide complexes for the Ras Binding Domain (RBD) of a RAF protein kinase (e.g., as impacted by a compound provided herein, or a pharmaceutically acceptable salt thereof). For example, FLAG tagged KRas protein can be preloaded with the GTP analog GppNHp and then incubated with biotinylated Raf-RBD to form complexes. A competition assay can then be performed by adding untagged KRas protein preloaded with GppNHp, which had been preloaded with various test molecules, over a range of concentrations. The proximity-dependent signal after addition of streptavidin donor and anti-flag acceptor beads (e.g., ALPHASCREEN^{®} beads) can be measured to determine the affinity of the KRas protein for the Raf kinase. See, e.g., Hunter et al. Mol Cancer Res. 2015; 13(9): 1325-35, doi: 10.1158/1541-7786.MCR-15-0203; Lim et al. Angew Chem Int Ed Engl. 2014; 53(1): 199-204, doi: 10.1002/anie.201307387; and Durrant, et al. Molecular Cancer Therapeutics 20.9 (2021): 1743-1754, doi: 10.1158/1535-7163.MCT-21-0175. As another example, for compounds provided herein, or pharmaceutically acceptable salts thereof, that may bind selectively to the GTP-bound state, His-tagged KRas protein can be preloaded with the GTP analog GppNHp and then incubated with a compound provided herein, or a pharmaceutically acceptable salt thereof, to form complexes. A competition assay can then be performed by adding Raf-RBD. The proximity-dependent signal after addition of Alpha detection reagents, compared to the signal from the same experiment using GDP instead of GppNHP, can be used to determine an IC₅₀ value. See, e.g., International Publication No. WO 2021/085653. It will be understood that in many cases, tagging technologies (e.g., FLAG tag, His tag, biotinylation) may be altered in an assay by one of skill in the art. In some embodiments, a RAF kinase interaction assay can be coupled with a nucleotide exchange assay; for example, a compound provided herein, or a pharmaceutically acceptable salt thereof, can be incubated with a KRas protein (e.g., a dysregulated KRas protein, e.g., a mutant KRas protein (e.g., a KRas G12D mutant protein, a KRas G12R mutant protein, or a KRas G12V mutant protein)) and GDP, then GTP (and optionally, a GEF such as SOS1) can be introduced. Then, RAF (e.g., cRAF) acceptor beads (e.g., GST-tagged acceptor beads) can be incubated with the KRas mixture, followed by introduction of donor beads (e.g., glutathione donor beads) and measurement using ALPHASCREEN^{®} technology. As an alternative to ALPHASCREEN^{®} technology, any appropriate FRET pair can be used to perform homogenous time resolved fluorescence. See, e.g., U.S. Publication Nos. US 2018/0334454 and US 2021/0230142. Another exemplary assay to measure the affinity of KRas:nucleotide complex for a RBD is to incubate cells with a compound provided herein, or a pharmaceutically acceptable salt thereof, lyse the cells, then pull down non-RBD-bound KRas using an immobilized RBD. See, e.g., U.S. Publication No. US 2019/0233440. As another example, the effect of a compound provided herein, or a pharmaceutically acceptable salt thereof, on the interaction between KRas and Raf-RBD can be evaluated using HiBiT and/or NANOBIT^{™} technology, wherein two parts of an enzyme are fused to or inserted into two proteins of interest (e.g., KRas and Raf-RBD); when the two proteins of interest are in proximity, the two parts of the enzyme complement each other to complete an enzyme that has signaling activity (e.g., that produces luminescence). In some such assays, the affinity of the two parts of the enzyme can be tuned, for example, to reduce or eliminate signal based on proximity driven by the two parts of the enzyme. See, e.g., Schwinn, et al. ACS Chemical Biology 13.2 (2018): 467-474, doi: 10.1021/acschembio.7b00549. Similarly, the effect of a compound provided herein, or a pharmaceutically acceptable salt thereof, on the interaction between KRas and Raf-RBD can be evaluated using NANOBRET^{™} technology, wherein two parts of signaling system (e.g., a protein and a ligand) are fused to or inserted into two proteins of interest (e.g., KRas and Raf-RBD); when the two proteins of interest are in proximity, the two parts of the signaling system have signaling activity (e.g., producing fluorescence). See, e.g., Durrant, et al. Molecular Cancer Therapeutics 20.9 (2021): 1743-1754, doi: 10.1158/1535-7163.MCT-21-0175. In some embodiments, a RAF kinase interaction assay can be used to determine if a compound provided herein, or a pharmaceutically acceptable salt thereof, is selective for a KRas protein (e.g., a dysregulated KRas protein, e.g., a mutant KRas protein (e.g., a KRas G12D mutant protein, a KRas G12R mutant protein, or a KRas G12V mutant protein)) in the GDP-bound state or the GTP-bound state.

Inhibition of the interaction between the KRas protein and Raf-RBD by compounds provided herein, or pharmaceutically acceptable salts thereof, can be measured using methods known in the art (e.g., using one or more methods described herein (e.g., using methods described in **Example B3** or **Example B4** herein)).

In some embodiments, a compound provided herein, or a pharmaceutically acceptable salt thereof, modulates the interaction between the KRas protein and one or more Raf proteins. In some embodiments, the compound inhibits the interaction between the KRas protein and Raf-RBD with an IC₅₀ of less than 1 µM (e.g., less than 750 nM, less than 500 nM, or less than 200 nM). In some embodiments, the compound inhibits the interaction between the KRas protein and Raf-RBD with an IC₅₀ of less than 200 nM (e.g., e.g., less than 150 nM, less than 200 nM, less than 100 nM, less than 10 nM, less than 1 nM, less than 0.1 nM, or less than 0.01 nM). For example, the compound inhibits the interaction between the KRas protein and Raf-RBD with an IC₅₀ from 0.001 nM to 500 nM, from 0.005 nM to 100 nM, from 0.025 nM to 100 nM, from 0.1 nM to 50 nM, or from 0.1 nM to 10 nM.

In some embodiments, a compound provided herein, or a pharmaceutically acceptable salt thereof, inhibits the interaction between the KRas protein and Raf-RBD with an IC₅₀ of less than 1 µM in the absence of cyclophilin A (e.g., less than 750 nM, less than 500 nM, or less than 200 nM). In some embodiments, the compounds inhibit the interaction between the KRas protein and Raf-RBD with an IC₅₀ of less than 200 nM in the absence of cyclophilin A (e.g., e.g., less than 150 nM, less than 200 nM, less than 100 nM, less than 10 nM, less than 1 nM, less than 0.1 nM, or less than 0.01 nM). For example, the compounds inhibit the interaction between the KRas protein and Raf-RBD with an IC₅₀ from 0.001 nM to 500 nM, from 0.005 nM to 100 nM, from 0.025 nM to 100 nM, from 0.1 nM to 50 nM, or from 0.1 nM to 10 nM in the absence of cyclophilin A.

Another exemplary assay for evaluating the potency of a compound provided herein, or a pharmaceutically acceptable salt thereof, includes measuring the phosphorylation of a downstream kinase, such as ERK (e.g., ERK1 and/or ERK2) or MEK. Such assays can be used to measure the inhibition of KRas signaling activity, for instance, in a cell line (e.g., A375, A427, A549, AGS, ASPC1, CAL62, CALU1, CAPAN1, CAPAN2, CFPAC1, GP2D, H358, H441, H460, H727, HCT116, HKA1, HPAC, HPAFII, HTK, HUPT3, KMS20, KP2, LS123, LS180, MIAPaCa-2, MKN1, NCI-H1993, NCI-H211, NCI-H424, NCI-H526, Panc02.03, Panc04.03, PATC50, PC9, PK8, PSN1, RKN, SW480, SW620, and/or TCCPAN2). For example, cells can be contacted with a compound provided herein, or a pharmaceutically acceptable salt thereof, for a period of time, then lysed or permeabilized, and total ERK or MEK and phosphoERK or phosphoMEK content can be determined (e.g., using antibodies, or a kit, such as Invitrogen InstantOne ERK1/ERK2 (Phospho) [pT202/pY204]/[pT185/pY187] ELISA or MesoScale Discovery p/t ERK1/2). In some embodiments, multiple concentrations of a compound provided herein, or a pharmaceutically acceptable salt thereof, can be used to construct a dose response curve.

In some embodiments, the compounds provided herein, or pharmaceutically acceptable salts thereof, inhibit ERK phosphorylation in a cell line expressing a KRas protein (e.g., a mutant KRas protein (e.g., a KRas G12D mutant protein, a KRas G12R mutant protein, or a KRas G12V mutant protein)) with an IC₅₀ of less than 1 µM (e.g., less than 750 nM, less than 500 nM, or less than 200 nM). In some embodiments, the compounds inhibit ERK phosphorylation in a cell line expressing a KRas protein (e.g., a mutant KRas protein (e.g., a KRas G12D mutant protein, a KRas G12R mutant protein, or a KRas G12V mutant protein)) with an IC₅₀ of less than 200 nM (e.g., less than 150 nM, less than 200 nM, less than 100 nM, less than 10 nM, less than 1 nM). For example, the compounds can inhibit ERK phosphorylation in a cell line expressing a KRas protein (e.g., a mutant KRas protein (e.g., a KRas G12D mutant protein, a KRas G12R mutant protein, or a KRas G12V mutant protein)) with an IC₅₀ from 0.1 nM to 100 nM, from 0.1 nM to 50 nM, from 1 nM to 50 nM, or from 1 nM to 20 nM.

In some embodiments, a compound provided herein, or a pharmaceutically acceptable salt thereof, can selectively inhibit one or more mutant KRas proteins over wild type KRas protein. The selectivity between wild type KRas protein and a mutant KRas protein as described herein can be measured using cellular proliferation assays where cell proliferation is dependent on signaling activity. For example, HEK293 cells transfected with a suitable version of wild type KRas, or HEK293 cells transfected with KRas containing one or more mutations as described herein (e.g., a G12D mutation, a G12R mutation, or a G12V mutation) can be used. Proliferation assays are performed at a range of inhibitor concentrations (e.g., 10 µM, 3 µM, 1.1 µM, 330 nM, 110 nM, 33 nM, 11 nM, 3 nM, 1 nM) and an EC₅₀ is calculated.

See also the assays described in International Publication Nos. WO 2021/120890; WO 2021/041671; and U.S. Publication Nos. US 2021/0130369; US 2021/0179633; US 2018/0334454; and US 2021/0122764.

The pharmacokinetic parameters of a compound provided herein, or a pharmaceutically acceptable salt thereof, can be evaluated in an animal model, for instance, a mouse model, a rat model, a dog model, or a nonhuman primate (e.g., cynomolgus monkey) model. Pharmacokinetics parameters, including clearance (CL), volume of distribution (V_{d}), maximum plasma concentration (Cₘₐₓ), time of maximum plasma concentration (tₘₐₓ), half-life (t_{1/2}), area under the curve (AUC), and oral bioavailability (%F) can be calculated using, e.g., a non-compartmental model. In some embodiments, a reference compound (e.g., a first KRas inhibitor (e.g., MRTX1133)) may be used as a comparator. See, e.g., Example 3 ("Pharmacokinetic experiments in mice") of International Publication No. WO 2023/098425.

Certain pharmacokinetic parameters of a compound provided herein, or a pharmaceutically acceptable salt thereof, can be evaluated in hepatocytes, such as in mouse, rat, dog, nonhuman primate (e.g., cynomolgus monkey), or human hepatocytes. Pharmacokinetics parameters, including clearance (CL) and half-life (t_{1/2}), can be calculated. In some embodiments, a reference compound (e.g., a first KRas inhibitor (e.g., MRTX1133)) may be used as a comparator. See, e.g., Example VI ("Liver microsomal metabolically stability") of International Publication No. WO 2023/284881.

In some embodiments, a compound provided herein, or a pharmaceutically acceptable salt thereof can be assessed for its pharmacokinetic parameters and/or its ability to cause toxicity (e.g., skin toxicity) in an animal model. For example, a compound provided herein, or a pharmaceutically acceptable salt thereof, can be administered to an animal (e.g., rat), and body fluid (e.g., blood) samples can be taken at various time points and analyzed for the amount of the compound provided herein, or a pharmaceutically acceptable salt thereof, remaining. As another example, a compound provided herein, or a pharmaceutically acceptable salt thereof, can be administered to an animal (e.g., rat), and the skin of the animal can be assessed for redness, scaling, and/or thickness. An exemplary protocol follows.

Rats (e.g., male RNU Nude Rat or Sprague-Dawley IGS rats, 6-8 weeks of age) are dosed with a compound provided herein, or a pharmaceutically acceptable salt thereof, once per day orally (e.g., via gavage) for several days (e.g., 14 days). The compound provided herein, or a pharmaceutically acceptable salt thereof, is administered to the rat at a given dose level (e.g., 1, 3, 5, 10, 25, 30, 50, or 100 mg/kg) in a solution or suspension formulation (e.g., 10% DMSO/90% hydroxypropyl methylcellulose). The rats have access to food and water ad libitum.

Blood samples (e.g., 200 µL per sample) from the rats are taken at predetermined intervals, such as 4, 8, or 24 hours after the first dose is administered. The blood is sampled via jugular vein puncture, then the blood samples (e.g., with K₂EDTA as anticoagulant) are temporarily put on ice and then centrifuged (e.g., at 4 °C and 4600 RPM for 5 minutes) within 30 minutes. Plasma samples can be diluted 1:1 v/v with acidified phosphate buffer and are put on dry ice. After the completion of the last sampling, all samples are stored at -80 °C or analyzed in a short time following collection. The concentration of the compound provided herein, or a pharmaceutically acceptable salt thereof, measured in an acidified plasma sample (e.g., diluted 1:1 v/v with pH 3 phosphate buffer) can be determined (e.g., via LC/MS/MS). For example, an acidified plasma sample is prepared for analysis using protein precipitation (e.g., by the addition of acetonitrile), and an internal standard is spiked in at a known concentration. The spiked sample is mixed, centrifuged, and the supernatant is used in an LC/MS/MS method. The LC/MS/MS method uses an ACQUITY UPLC BEH C18 1.7 µm (2.1*50 mm) column with a first mobile phase of 5 mM NH₄OAc (0.05% formic acid (FA) or 0.1% FA) and a second mobile phase of acetonitrile (0.1% FA). Multiple reaction monitoring is used to measure the analyte(s) of interest. Using the concentration of the compound provided herein, or a pharmaceutically acceptable salt thereof, in the plasma sample, pharmacokinetic parameters of t_{1/2} (hr), tₘₐₓ (hr), Cₘₐₓ (ng/mL), AUCₗₐₛₜ (hr*ng/mL), AUC_{Inf} (hr*ng/mL), AUC_{Extr} (%), MRT_{Inf} (hr), AUC_{Inf}/D (hr*kg*ng/mL/mg), F (%), are determined.

The clinical signs, body weight and food consumption of the rats are tracked during dosing. The potential for skin rash is tracked intermittently before and during dosing using one or more methodologies. A skin scoring system is used to evaluate skin redness (erythema) or skin scaling on a gradation of 0-4. Thickness of the back skin is measured using a micrometer (MITUTOYO ABSOLUTE Digimatic Micrometer Series 227-211). For the back skin, thickness measurement is performed by doing a skin folding of the applied area between the thumb and index fingers followed by measuring with the micrometer. The mice are then sacrificed and assessed for any abnormalities.

In some embodiments, a compound provided herein, or a pharmaceutically acceptable salt thereof, can be tested for its potency in inhibiting hERG potassium channels. The cardiac potassium channel hERG is responsible for a rapid delayed rectifier current (*I*_{Kr}) in human ventricle, and inhibition of *I*_{Kr} is the most common cause of cardiac action potential prolongation by non-cardiac drugs. Increased action potential duration causes prolongation of the QT interval that has been associated with a dangerous ventricular arrhythmia, torsade de pointes. There are several methods of testing hERG inhibition potency, including SyncroPatch hERG and manual patch clamp experiments.

In some embodiments, a compound provided herein, or a pharmaceutically acceptable salt thereof, is tested for its potency in inhibiting hERG potassium channels using a SyncroPatch hERG assay. For example, in some embodiments, solutions or suspension of a compound provided herein, or a pharmaceutically acceptable salt thereof, at several concentrations (0.30 µM, 1.00 µM, 3.00 µM, 10.00 µM and 30.00 µM) can be exposed to single CHO or HEK293 cells. The effect of the compound provided herein, or a pharmaceutically acceptable salt thereof, on the inhibition of hERG potassium channels can be measured in this system using an electrical pulse pattern, the data plotted, and an IC₅₀ value calculated for the inhibition of hERG by the compound provided herein, or a pharmaceutically acceptable salt thereof. An exemplary protocol follows.

hERG potassium channels are expressed in a Chinese Hamster Ovarian (CHO (Sophian Biosciences)) cell line that lacks endogenous *I*_{Kr}. See, e.g., Brown, Arthur M., and David Rampe. Pharmaceutical News 7.4 (2000): 15-20; Weirich, Jörg, and H. Antoni, Basic Research in Cardiology 93 (1998): s125-s132, doi: 10.1007/s003950050236; Yap, Yee Guan, and A. J. Camm. Clinical & Experimental Allergy 29 (1999): 174-181, doi: 10.1046/j.1365-2222.1999.0290s3174.x; Haraguchi, Yuji, et al. BMC Pharmacology and Toxicology 16 (2015): 1-6, doi: 10.1186/s40360-015-0042-9; and Walker, B. D., et al. British Journal of Pharmacology 127.1 (1999): 243-251, 10.1038/sj.bjp.0702502.

All chemicals used in solution preparations are purchased from a commercial supplier (e.g., Sigma-Aldrich) and are of ACS reagent grade purity or higher. Stock solutions of the compound provided herein, or a pharmaceutically acceptable salt thereof, positive control compound(s), and reference substance(s) are prepared in dimethyl sulfoxide (DMSO) and stored frozen. Solutions of each tested compound provided herein, or a pharmaceutically acceptable salt thereof, positive control compound(s), and reference substance(s) are prepared fresh daily by diluting stock solutions into HEPES-buffered physiological saline solution (HB-PS; 140 mM NaCl, 4 mM KCl, 2.0 mM CaCl₂, 1 mM MgCl₂, 10 mM HEPES, and 5 mM glucose, pH 7.4). Since previous results have shown that ≤ 0.3% DMSO does not affect channel current, all test and control solutions can contain up to 0.3% DMSO.

In some embodiments, a positive control compound can be included in the experiment. In some such embodiments, the positive control compound can be amitriptyline (Sigma-Aldrich), for example, in a HB-PS and 0.3% DMSO solution.

In some embodiments, a positive control compound can be included in the experiment. In some such embodiments, the positive control compound can be E-4031 (4'-[[1-[2-(6-Methyl-2-pyridinyl)ethyl-4-piperidinyl]carbonyl]methanesulfonanilide, 2HCl) (Sigma-Aldrich), for example, in a HB-PS and 0.3% DMSO solution.

If necessary, solutions are sonicated to facilitate dissolution. Visible precipitate observed during preparation or exposure of formulations to the test system is noted for reference.

The effect of compounds provided herein, or a pharmaceutically acceptable salt thereof, is initially evaluated at 1 µM. Subsequent concentrations are evaluated based on the inhibition observed at this initial concentration.

The CHO cells, which are at least two days after plating and more than 75% confluent, are used for experiments. Before testing, cells are harvested using TrypLE and resuspended in HB-PBS at room temperature. The HB-PBS and external solution (80 mM NaCl, 4 mM KCl, 2 mM CaCl₂, 1 mM MgCl₂, 60 mM *N*-Methyl-D-glucamine (NMDG), 10 mM HEPES, and 5 mM glucose, pH 7.4) are prepared and stored up to 1 month.

Voltage command protocol: From a holding potential of -80 mV, the voltage is first stepped to -50 mV for 80 ms for leak subtraction, and then stepped to +20 mV for 4800 ms to open hERG channels. After that, the voltage is stepped back down to -50 mV for 5000 ms, causing a "rebound" or tail current, which is measured and collected for data analysis. Finally, the voltage is stepped back to the holding potential (-80 mV, 1000 ms). This voltage command protocol is repeated every 20,000 msec. This command protocol is performed continuously during the experiment.

The hERG SyncroPatch assay is conducted at room temperature. The Setup, Prime Chip, Catch and Seal Cells, Amplifier Settings, Voltage and Application Protocols are established with Biomek Software (Nanion). A single cell per well is clamped with the formation of a gigaseal. On one side of the seal, the cell is bathed in external solution. On the opposite side, addition of 40 µL of the vehicle (e.g., HB-PBS) is applied, followed by a 300 s pause to create a baseline period. Then a dose of a compound provided herein, or a pharmaceutically acceptable salt thereof, is added (40 µL), and the process is repeated for different concentrations. The exposure of a compound provided herein, or a pharmaceutically acceptable salt thereof, at each concentration is no less than 300 s. The recording for the whole process must pass quality control criteria (e.g., seal quality, rundown attributes) or the well is abandoned, and the compound is retested, all automatically set by exclusion criteria determined prior to experiment start. Five concentrations (0.30 µM, 1.00 µM, 3.00 µM, 10.00 µM, and 30.00 µM) are tested for each tested compound provided herein, or a pharmaceutically acceptable salt thereof. A minimum of 2 replicates per concentration are obtained.

Data analysis is carried out using DataControl, Excel 2013 (Microsoft) and GraphPad Prism 5.0.

Within each well recording, a percent of control values is calculated for each concentration of the compound provided herein, or a pharmaceutically acceptable salt thereof, with current response based on peak current in presence of a reference compound (e.g., E-4031) (current response/peak current) × 100%. The Dose-Response curves are fit to the standard Hill equation as shown below: $\frac{{I}_{post compound}}{{I}_{pre compound}} = Bottom + \frac{\left(Top-Bottom\right)}{1 + {10}^{\left({LogIC}_{50}-X\right) ∗ HillSlope}}$ where X is the logarithm of concentration, *I*_{post compound}/*I*_{pre compound} is the normalized peak current amplitude, Top is 1, and Bottom is equal to 0.

Curve-fitting and IC₅₀ calculations are performed by GraphPad Prism 5.0. If the inhibition obtained at the lowest concentration tested is over 50%, or at the highest concentration tested is less than 50%, the IC₅₀ is reported as less than the lowest concentration, or higher than the highest concentration, respectively. In some embodiments, a positive control can be included in the experiment. In some such embodiments, the positive control compound can be cisapride (Tocris Bioscience), for example, in a 0.3% DMSO solution.

In some embodiments, a compound provided herein, or a pharmaceutically acceptable salt thereof, is not a hERG inhibitor. In some embodiments, a compound provided herein, or a pharmaceutically acceptable salt thereof, inhibits hERG with an IC₅₀ of greater than 60 nM (e.g., greater than 100 nM, 300 nM, 500 nM, 1 µM, 3 µM, 5 µM, 10 µM, 20 µM, or 30 µM). For example, a compound provided herein, or a pharmaceutically acceptable salt thereof, inhibits hERG with an IC₅₀ of greater than 500 nM (e.g., 1 µM, 3 µM, 5 µM, 10 µM, 20 µM, or 30 µM). In some embodiments, a compound provided herein, or a pharmaceutically acceptable salt thereof, inhibits hERG with an IC₅₀ of greater than 1 µM (e.g., greater than 3 µM, 5 µM, 10 µM, 20 µM, or 30 µM). In some embodiments, a compound provided herein, or a pharmaceutically acceptable salt thereof, inhibits hERG with an IC₅₀ of greater than 10 µM (e.g., greater than 20 µM or 30 µM). In some embodiments, a compound provided herein, or a pharmaceutically acceptable salt thereof, inhibits hERG with an IC₅₀ of greater than 30 µM.

In some embodiments, a compound provided herein, or a pharmaceutically acceptable salt thereof, can be assessed for its stability in hepatocytes (e.g., human hepatocytes). In some embodiments, a compound provided herein, or a pharmaceutically acceptable salt thereof, is assessed for stability in human hepatocytes, yielding a value for intrinsic hepatocyte clearance (CLᵢₙₜ(hep)), from which intrinsic liver clearance (CLᵢₙₜ (liver)) can be estimated. For example, human hepatocytes can be incubated with a compound provided herein, or a pharmaceutically acceptable salt thereof, and aliquots at various time points can be removed and analyzed for the amount of the compound provided herein, or a pharmaceutically acceptable salt thereof, remaining. First-order kinetics equations can then be used to determine half-life (t_{1/2}) and CLᵢₙₜ(hep). An exemplary protocol follows.

A 1000X stock solution of a compound provided herein is prepared to a final concentration (e.g., 1 mM in DMSO). Similarly, 1000X stock solution(s) of positive control compound(s) (e.g., 7-ethoxycoumarin and/or 7-hydroxycoumarin) is prepared to a final concentration (e.g., 3 mM in DMSO). 100X stock solutions are then made by dilution with acetonitrile.

To prepare a cell suspension (e.g., at 0.5 × 10⁶ cells/mL), cryopreserved hepatocytes (e.g., human hepatocytes) are thawed (e.g., in Williams' Medium E containing 5% fetal bovine serum and 30% Percoll solution and other supplements), isolated, and suspended in incubation medium (e.g., Williams' Medium E (no phenol red) containing 2 mM L-Glutamine and 25 mM HEPES), then diluted with pre-warmed incubation medium to 0.5 × 10⁶ cells/mL. Pre-warmed cell suspension (e.g., 198 µL) is added to a well in a testing plate (e.g., a 96-well plate).

A quenching plate is prepared by transferring stop solution (e.g., acetonitrile containing tolbutamide and labetalol as internal standards) (e.g., 125 µL) to a set of pre-labeled 96-well plates, with one quenching plate for T₀ and one plate per time point to be tested.

To begin the experiment, 2 µL of the 100X dosing solution of the compound provided herein, or a pharmaceutically acceptable salt thereof, or 2 µL of the 100X dosing solution of a control compound is added to a well of the testing plate. This is performed in duplicate.

For T₀ samples, the plate is mixed to achieve a homogenous suspension (e.g., mixed for about 1 min), then an aliquot (e.g., 25 µL) of each sample on the testing plates is immediately transferred into a well of a quenching plate containing ice-cold stop solution (e.g., 125 µL), followed by mixing.

The testing plate is incubated (e.g., at 37 °C in a 95% humidified incubator at 5% CO₂ with constant shaking) to start the reactions. At each time point of 15, 30, 60 and 90 minutes, the plate is mixed and then an aliquot of each sample (e.g., 25 µL) is transferred to a well in a quenching plate containing ice-cold stop solution (e.g., 125 µL) followed by mixing.

Medium Control (MC) sample plates (at least one for the first and last time point; additional time points are optional) are prepared in the same way as the testing plate except that incubation medium is used instead of cell suspension. At each corresponding time point as the testing plate, the reactions are stopped by removing the corresponding MC plate from the incubator and mixing with ice-cold stop solution (e.g., 125 µL).

Immediately after addition of stop solution to each plate, the plate is immediately vortexed (e.g., on a plate shaker at 600 RPM for 10 minutes). Then the plate is centrifuged (e.g., at 3220 x g for 20 min at 4 °C).

After centrifugation, supernatant from the plate (e.g., 80 µL/well) is transferred to another plate (e.g., a corresponding 96-well plate) which contains ultra pure water (240 µL per well) according to the plate map. This analytical plate is sealed and stored at 4 °C until LC-MS/MS analysis.

The percent remaining of the compound provided herein, or a pharmaceutically acceptable salt thereof, after incubation is calculated by the following equations. The equation of first order kinetics is used to calculate t_{1/2} and CLᵢₙₜ:
Equation of first order kinetics: ${C}_{t} = {C}_{0} \times {e}^{- k \times t}$ $When {C}_{t} = \frac{1}{2} {C}_{0} , then {t}_{\frac{1}{2}} = \frac{ln 2}{k}$ ${CL}_{int} \left(hep\right) = k / million cells per mL$ ${CL}_{int} \left(liver\right) = {CL}_{int} \left(hep\right) * liver weight \left(g/kg body weight\right) \times hepatocellularity$

**Table 2** shows reference values for liver weight, liver blood flow, and hepatocellularity for various species. See, e.g., Sohlenius-Sternbeck, Anna-Karin Toxicology in vitro 20.8 (2006): 1582-1586, doi: 10.1016/j.tiv.2006.06.003; Davies, Brian, and Tim Morris Pharmaceutical Research 10.7 (1993): 1093-1095; and Obach, R. Scott, et al. Journal of Pharmacology and Experimental Therapeutics 283.1 (1997): 46-58.

**Table 2.**

| **Species** | **Liver Weight (g/kg Body Weight)** | **Liver Blood Flow (ml/min/kg)** | **Hepatocellularity (million cells/g liver)** |
|---|---|---|---|
| **Mouse** | 88 | 90.0 | 135 |
| **Rat** | 40 | 55.2 | 117 |
| **Dog** | 32 | 30.9 | 215 |
| **Monkey** | 30 | 43.6 | 120 |
| **Human** | 20 | 20.7 | 139 |

**Table 3** shows floor and ceiling values for the described assay. For example, if CLᵢₙₜ (hep) falls below 6.4 µL/min/10⁶ cells, an experimental value may not be able to be accurately determined.

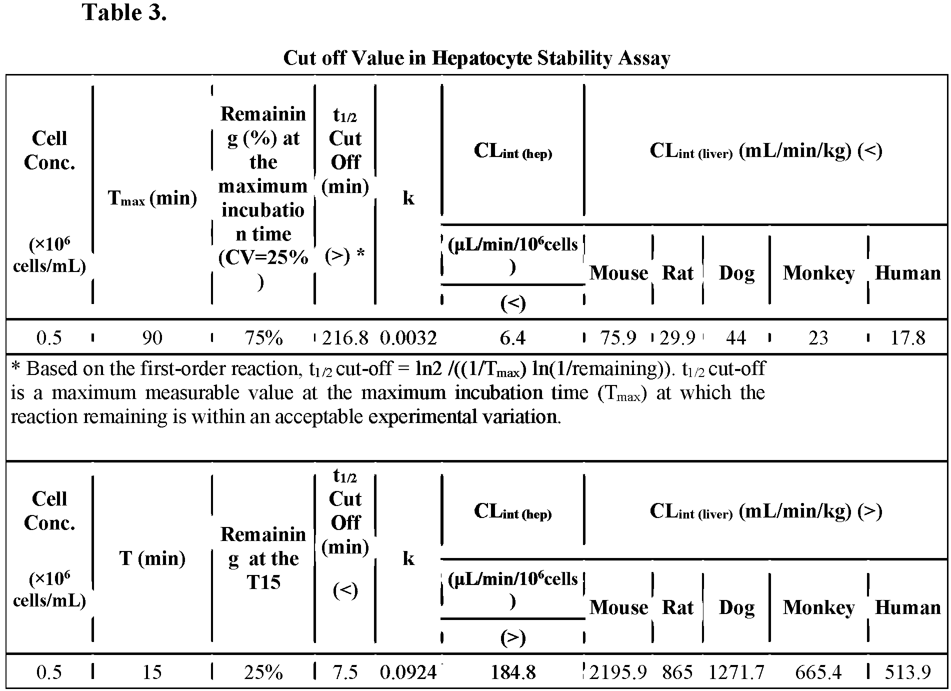

In some embodiments, a compound provided herein, or a pharmaceutically acceptable salt thereof can be assessed for its stability in simulated human fluids (e.g., simulated gastric fluid or simulated intestinal fluid). For example, a compound provided herein, or a pharmaceutically acceptable salt thereof can be incubated in a simulated human fluid (e.g., simulated gastric fluid (e.g., fed or fasted state) or simulated intestinal fluid (e.g., fed or fasted state)), and aliquots at various time points can be removed and analyzed for the amount of the compound provided herein, or a pharmaceutically acceptable salt thereof, remaining. An exemplary protocol follows.

Fasted state simulated gastric fluid (FaSSGF) is prepared (0.02 mM lecithin, 0.08 mM sodium taurocholate, 0.1 mg/mL pepsin, 34.2 mM sodium chloride, 25.1 mM hydrochloric acid, and deionized water, pH 1.6 ± 0.05).

A working solution of a compound provided herein, or a pharmaceutically acceptable salt thereof is prepared (e.g., 2 µM in DMSO). Similarly, a working solution of a control compound (e.g., omeprazole) is prepared (e.g. 2 µM in DMSO). An aliquot (e.g., 2 µL) of either working solution is transferred to a deep-well plate, one plate per time condition to be tested (e.g., 0 minutes, 60 minutes, 120 minutes, 360 minutes, and 1440 minutes). To the plates corresponding to the later time points (e.g., T₆₀, T₁₂₀, T₃₆₀, and T₁₄₄₀), an aliquot (e.g., 198 µL) of the FaSSGF is added, and the samples adjusted to have a final DMSO concentration of 1%. The plates are incubated at 37 °C with shaking at 600 RPM for the appointed amount of time. At the end of the appointed amount of time, the reaction is stopped by the addition of stop solution (e.g., 400 µL), and the resulting solution is mixed. A portion of this mixture (e.g., 200 µL) is removed and mixed with a further addition of stop solution (e.g., 400 µL). Similarly, the T₀ samples are prepared by adding stop solution (e.g., 400 µL), mixing, then adding the FaSSGF (e.g., 200 µL) and mixing again. The T₀ samples are further prepared by removing a portion of this mixture (e.g., 200 µL) and mixing with a further addition of stop solution (e.g., 400 µL). Following the second addition of stop solution at each time point, the samples are centrifuged (e.g., at 4000 RPM at 4 °C for 20 min). A portion of the supernatant (e.g., 60 µL) is removed and mixed with purified water (e.g., 180 µL) for LC-MS/MS analysis. The LC-MS/MS analysis is performed using an ACQUITY UPLC BEH C18 1.7 µm 2.1 * 50 mm (Part No.186002350) column, with a mobile phase A (0.1% formic acid in water) and a mobile phase B (0.1% formic acid in acetonitrile). The percent remaining of the compound provided herein, or a pharmaceutically acceptable salt thereof, at each incubation time is calculated based on peak area ratio of analyte to internal standard from the LC-MS/MS analysis using the equation below. $Percent remaining \left(at an appointed time\right) = \frac{{PAR}_{T}}{{PAR}_{0}} ∗ 100 %$

Where PAR is the peak area ratio, the ratio of peak area of the analyte of interest and an internal standard; PAR_{T} is the peak area ratio at the appointed time; and PAR₀ is the peak area ratio at T₀.

Fed state simulated intestinal fluid (FeSSIF) is prepared (0.282% (w/v) lecithin, 0.806% (w/v) sodium taurocholate, 0.865% (w/v) acetic acid, 1.52% (w/v) potassium chloride, and deionized water, pH 5.0 ± 0.05).

A working solution of a compound provided herein, or a pharmaceutically acceptable salt thereof is prepared (e.g. 2 µM in DMSO). Similarly, a working solution of a control compound (e.g., chlorambucil) is prepared (e.g. 2 µM in DMSO). An aliquot (e.g., 2 µL) of either working solution is transferred to a deep-well plate, one plate per time condition to be tested (e.g., 0 minutes, 60 minutes, 120 minutes, 360 minutes, and 1440 minutes). To the plates corresponding to the later time points (e.g., T₆₀, T₁₂₀, T₃₆₀, and T₁₄₄₀), an aliquot (e.g., 198 µL) of the FeSSIF is added, and the samples are adjusted to have a final DMSO concentration of 1%. The plates are incubated at 37 °C with shaking at 600 RPM for the appointed amount of time. At the end of the appointed amount of time, the reaction is stopped by the addition of stop solution (e.g., 400 µL), and the resulting solution is mixed. A portion of this mixture (e.g., 200 µL) is removed and mixed with a further addition of stop solution (e.g., 400 µL). Similarly, the T₀ samples are prepared by adding stop solution (e.g., 400 µL), mixing, then adding the FeSSIF (e.g., 200 µL) and mixing again. The T₀ samples are further prepared by removing a portion of this mixture (e.g., 200 µL) and mixing with a further addition of stop solution (e.g., 400 µL). Following the second addition of stop solution at each time point, the samples are centrifuged (e.g., at 4000 RPM at 4 °C for 20 min). A portion of the supernatant (e.g., 60 µL) is removed and mixed with purified water (e.g., 180 µL) for LC-MS/MS analysis. The LC-MS/MS analysis is performed using an ACQUITY UPLC HSS T3 1.8 µm 2.1 * 50 mm, (Part No.186003538) column, with a mobile phase A (0.1% formic acid in water) and a mobile phase B (0.1% formic acid in acetonitrile). The percent remaining of the compound provided herein, or a pharmaceutically acceptable salt thereof, at each incubation time is calculated based on peak area ratio of analyte to internal standard from the LC-MS/MS analysis using the equation below. $Percent remaining \left(at an appointed time\right) = \frac{{PAR}_{T}}{{PAR}_{0}} ∗ 100 %$

Where PAR is the peak area ratio, the ratio of peak area of the analyte of interest and an internal standard; PAR_{T} is the peak area ratio at the appointed time; and PAR₀ is the peak area ratio at T₀.

Fasted state simulated intestinal fluid (FaSSIF) is prepared (0.056% (w/v) lecithin, 0.161% (w/v) sodium taurocholate, 0.39% (w/v) monobasic potassium phosphate, 0.77% (w/v) potassium chloride, and deionized water, pH 6.5 ± 0.05).

A working solution of a compound provided herein, or a pharmaceutically acceptable salt thereof is prepared (e.g. 2 µM in DMSO). Similarly, a working solution of a control compound (e.g., chlorambucil) is prepared (e.g. 2 µM in DMSO). An aliquot (e.g., 2 µL) of either working solution is transferred to a deep-well plate, one plate per time condition to be tested (e.g., 0 minutes, 60 minutes, 120 minutes, 360 minutes, and 1440 minutes). To the plates corresponding to the later time points (e.g., T₆₀, T₁₂₀, T₃₆₀, and T₁₄₄₀), an aliquot (e.g., 198 µL) of the FaSSIF is added, and the samples adjusted to have a final DMSO concentration of 1%. The plates are incubated at 37 °C with shaking at 600 RPM for the appointed amount of time. At the end of the appointed amount of time, the reaction is stopped by the addition of stop solution (e.g., 400 µL), and the resulting solution is mixed. A portion of this mixture (e.g., 200 µL) is removed and mixed with a further addition of stop solution (e.g., 400 µL). Similarly, the T₀ samples are prepared by adding stop solution (e.g., 400 µL), mixing, then adding the FaSSIF (e.g., 200 µL) and mixing again. The T₀ samples are further prepared by removing a portion of this mixture (e.g., 200 µL) and mixing with a further addition of stop solution (e.g., 400 µL). Following the second addition of stop solution at each time point, the samples are centrifuged (e.g., at 4000 RPM at 4 °C for 20 min). A portion of the supernatant (e.g., 60 µL) is removed and mixed with purified water (e.g., 180 µL) for LC-MS/MS analysis. The LC-MS/MS analysis is performed using an ACQUITY UPLC HSS T3 1.8 µm 2.1 * 50mm, (Part No.186003538) column, with a mobile phase A (0.1% formic acid in water) and a mobile phase B (0.1% formic acid in acetonitrile). The percent remaining of the compound provided herein, or a pharmaceutically acceptable salt thereof, at each incubation time is calculated based on peak area ratio of analyte to internal standard from the LC-MS/MS analysis using the equation below. $Percent remaining \left(at an appointed time\right) = \frac{{PAR}_{T}}{{PAR}_{0}} ∗ 100 %$

Where PAR is the peak area ratio, the ratio of peak area of the analyte of interest and an internal standard; PAR_{T} is the peak area ratio at the appointed time; and PAR₀ is the peak area ratio at T₀.

In some embodiments, a compound provided herein, or a pharmaceutically acceptable salt thereof can be assessed for its solubility in simulated human fluids (e.g., simulated gastric fluid or simulated intestinal fluid) or aqueous solutions (e.g., water or pH 3.0 citrate buffer). For example, a compound provided herein, or a pharmaceutically acceptable salt thereof can be incubated in a simulated human fluid (e.g., simulated gastric fluid or simulated intestinal fluid) or an aqueous solution (e.g., water or pH 3.0 citrate buffer). For example, a compound provided herein, or a pharmaceutically acceptable salt thereof can be incubated in a simulated human fluid (e.g., simulated gastric fluid (e.g., fed or fasted state) or simulated intestinal fluid (e.g., fed or fasted state)) or an aqueous solution (e.g., water or pH 3.0 citrate buffer) , and aliquots at various time points can be removed and analyzed for the amount of the compound provided herein, or a pharmaceutically acceptable salt thereof, remaining. An exemplary protocol follows.

Fasted state simulated gastric fluid (FaSSGF) is prepared (0.02 mM lecithin, 0.08 mM sodium taurocholate, 0.1 mg/mL pepsin, 34.2 mM sodium chloride, 25.1 mM hydrochloric acid, and deionized water, pH 1.6 ± 0.05). The FaSSGF is equilibrated at 37 °C.

Fed state simulated intestinal fluid (FeSSIF) is prepared (0.282% (w/v) lecithin, 0.806% (w/v) sodium taurocholate, 0.865% (w/v) acetic acid, 1.52% (w/v) potassium chloride, and deionized water, pH 5.0 ± 0.05). The FeSSIF is equilibrated at 37 °C.

Fasted state simulated intestinal fluid (FaSSIF) is prepared (0.056% (w/v) lecithin, 0.161% (w/v) sodium taurocholate, 0.39% (w/v) monobasic potassium phosphate, 0.77% (w/v) potassium chloride, and deionized water, pH 6.5 ± 0.05). The FaSSIF is equilibrated at 37 °C.

Citrate buffer is prepared (100 mM sodium citrate, pH 3.07). Solubility in citrate buffer and water is determined at room temperature.

A compound provided herein, or a pharmaceutically acceptable salt thereof, (e.g., 6 mg) is combined with FaSSGF, FeSSIF, FaSSIF, citrate buffer, or water (e.g., 3 mL). The vials are stirred (at 37 °C for simulated human fluid or room temperature for citrate buffer and water), and at 30 minutes, 3 hours, and 24 hours, an aliquot is removed and filtered (e.g., using a 0.2 µm syringe filter), then analyzed with UPLC (e.g., HPLC is conducted using an Agilent 1290 Infinity LC System equipped with a VWD (Variable Wavelength Detector) and an Agilent 1260 ELSD (Evaporative Light Scattering Detector). Flow rate range of the instrument is 0.2-5.0 mL/min, operating pressure range is 0-1300 bar, temperature range is 5 °C above ambient to 60 °C, and wavelength range is 190-600 nm; mobile phase A of 0.1% trifluoroacetic acid (TFA) in distilled water; mobile phase B of 0.1% TFA in acetonitrile; column Waters Acuity UPLC CSH C-18, 2.1 x 150 mm, 1.7 µm) to determine the dissolved concentration of the compound provided herein, or a pharmaceutically acceptable salt thereof.

In some embodiments, the kinetic solubility of a compound provided herein, or a pharmaceutically acceptable salt thereof can be determined. In some embodiments, the kinetic solubility of a compound provided herein, or a pharmaceutically acceptable salt thereof, is determined at a physiologically relevant pH (e.g., 7.4). For example, a stock solution of a compound provided herein, or a pharmaceutically acceptable salt thereof, can be prepared, and any solids separated (e.g., by centrifugation). The resulting mixture (e.g., supernatant) can be subsequently filtered, and the dissolved concentration of the compound provided herein, or a pharmaceutically acceptable salt thereof, can be determined via liquid chromatography. An exemplary protocol follows.

A stock solution (e.g., a 200 µM stock solution) of a compound provided herein, or a pharmaceutically acceptable salt thereof, is diluted with buffer (e.g., phosphate buffer, pH 7.4) (e.g., 10 µL of the stock solution with 490 uL of the buffer). The diluted sample is vortexed for at least two minutes and then shaken (e.g., at 800 RPM) on a shaker for 24 hours at room temperature. The sample is then centrifuged (e.g., at 4000 RPM for 10 minutes at 25 °C). The supernatant is filtered (e.g., in a plate format by centrifugation for 5 minutes), and the concentration of the compound provided herein, or a pharmaceutically acceptable salt thereof, is determined by an LC-UV system (e.g., with a mobile phase A of 0.1% TFA and 5 mM NH₄OAc in water/MeCN (v:v, 95:5) and a mobile phase B of 0.1% TFA and 5 mM NH₄OAc in water/MeCN (v:v, 5:95)).

In some embodiments, the chemical stability of a compound provided herein, or a pharmaceutically acceptable salt thereof, can be determined. For example, an aliquot of a solution of a compound provided herein, or a pharmaceutically acceptable salt thereof, is added to an acidic solution (e.g., pH 1.5 or pH 5) and incubated for a period of time. A sample of the incubation mixture can be taken, and the remaining amount of the compound provided herein, or a pharmaceutically acceptable salt thereof, can be determined via liquid chromatography-mass spectrometry (LCMS) and/or nuclear magnetic resonance (NMR). Exemplary protocols follow.

To prepare a pH 1.5 HCl solution, 1 N hydrogen chloride solution (1 mL) is added to deionized water (99 mL) and stirred for 5 minutes. The pH is adjusted, while stirring and monitoring by pH meter, to the desired pH using concentrated hydrochloric acid. To prepare a pH 5 HCl solution, 1 N hydrogen chloride solution (1 mL) is added to deionized water. (99 mL) and stirred for 5 minutes. The pH is adjusted, while stirring and monitoring by pH meter, to the desired pH using 1N sodium hydroxide.

A portion (e.g., 10 µL) of a stock solution (e.g., 10 mM) of a compound provided herein, or a pharmaceutically acceptable salt thereof, is added the HCl solution at pH of 1.5 or pH 5 (e.g., 90 µL) in separate vials denoted for each timepoint (e.g., T₀, T₃₀ₘ, T₆₀ₘ, T₉₀ₘ, T₂₄₀ₘ, and T_{3d}). The samples are incubated at 37 °C for the desired timeframe and then immediately neutralized with 10 µL of HEPES buffer to pH of 7. The T₀ timepoint is neutralized immediately upon preparation of the sample. The samples are then analyzed by LCMS to determine the amount of the compound provided herein, or a pharmaceutically acceptable salt thereof, remaining.

To prepare pH 1.5 and pH 5 DCl solutions, deuterium oxide (5 mL) is adjusted to the desired pH of 1.5 or 5, monitoring by pH meter, using 20% DCl in D₂O and stirred at room temperature for 5 minutes.

A compound provided herein, or a pharmaceutically acceptable salt thereof, is dissolved in the pH 1.5 or pH 5 DCl solution to achieve a 3 mg/mL solution. The resulting solution is incubated at 37 °C to the desired timepoint (T₀, T₉₀ₘ, T₂₄₀ₘ, T_{5d}) then an aliquot (e.g., 0.3 mL) is taken and analyzed by ¹H NMR to determine the amount of the compound provided herein, or a pharmaceutically acceptable salt thereof, remaining and/or to determine the presence of a compound that is not the compound provided herein. At the same timepoints, 30 µL of the solution was neutralized with HEPES buffer to pH of 7 and analyzed by LCMS to determine the amount of the compound provided herein, or a pharmaceutically acceptable salt thereof, remaining and/or to determine the presence of a compound that is not the compound provided herein.

In some embodiments, a compound provided herein, or a pharmaceutically acceptable salt thereof, can be assessed as a potential substrate for efflux (e.g., as a potential substrate for p-glycoprotein (P-gp)). In some embodiments, a compound provided herein, or a pharmaceutically acceptable salt thereof, is assessed as a substrate for efflux using efflux pump-expressing cells, such as MDCKII cells. For example, a compound provided herein, or a pharmaceutically acceptable salt thereof, can be applied to one side of a cell monolayer, incubated, and then the recovery of the compound on the opposite side of the cell monolayer can be measured to determine the permeability of the compound to the cell monolayer in that direction. An exemplary protocol follows.

MDCKII cells (e.g., obtained from the Netherlands Cancer Institute) are seeded onto polycarbonate membranes (PC) in 96-well insert systems at 2.33 x 10⁵ cells/cm² for 4-7 days for confluent cell monolayer formation.

A solution of a compound provided herein, or a pharmaceutically acceptable salt thereof, is diluted with transport buffer (e.g., Hank's Balanced Salt Solution (HBSS) with 10 mM HEPES, pH 7.4) from a DMSO stock solution to a final concentration (e.g., of 2.00 µM (DMSO < 1%)) and applied to the apical or basolateral side of the cell monolayer. Permeation of the compound provided herein, or a pharmaceutically acceptable salt thereof, from the A to B direction and the B to A direction is determined in duplicate. For control data, digoxin is tested at 10.0 µM from A to B direction or B to A direction, while nadolol and metoprolol are tested (e.g., at 2.00 µM) in A to B direction in duplicate. The plate is incubated (e.g., for 2.5 hours in an incubator at 37.0 °C, with 5% CO₂ at saturated humidity without shaking). The efflux ratio of each compound is then determined. The compound provided herein, or pharmaceutically acceptable salt thereof, and control compounds are quantified by LC-MS/MS analysis based on the peak area ratio of analyte/internal standard (IS).

The apparent permeability coefficient Pₐₚₚ (cm/s) is calculated using the equation: ${P}_{app} = \left({dC}_{r}/dt\right) \times {V}_{r} / \left(A\times {C}_{0}\right)$

Where dCᵣ/dt is the cumulative concentration of compound in the receiver chamber as a function of time; Vᵣ is the solution volume in the receiver chamber (0.075 mL on the apical side, 0.25 mL on the basolateral side); A is the surface area for the transport (e.g., 0.143 cm² for the area of the monolayer); and C₀ is the initial concentration in the donor chamber.

The efflux ratio was calculated using the equation: $Efflux Ratio = {P}_{app} \left(BA\right) / {P}_{app} \left(AB\right)$

Without being bound by any particular theory, it is believed that the Efflux Ratio indicates how efficiently a tested compound is removed from the tested cell.

Percent recovery was calculated using the equation: $% Solution Recovery = 100 \times \left[\left({V}_{r}\times {C}_{r}\right)+\left({V}_{d}\times {C}_{d}\right)\right] / \left({V}_{d}\times {C}_{0}\right)$

Where V_{d} is the volume in the donor chamber (0.075 mL on the apical side, 0.25 mL on the basolateral side); C_{d} and Cᵣ are the final concentrations of transport compound in donor and receiver chambers, respectively.

Without being bound by any particular theory, it is believed that the percent recovery is informative of whether the tested compound could have a solubility issue, is stuck in the cellular membrane, metabolized, or a combination thereof.

After the transport assay, a Lucifer yellow rejection assay is used to determine the cell monolayer integrity (e.g., to determine whether the cell monolayer from the efflux study is intact). Buffers are removed from both apical and basolateral chambers, followed by the addition of lucifer yellow dye (e.g., 75 µL of a 100 µM solution in transport buffer) and transport buffer (e.g., 250 µL) in the apical and basolateral chambers, respectively. The plate is incubated (e.g., for 30 minutes at 37 °C with 5% CO₂ and 95% relative humidity without shaking). After incubation, a lucifer yellow (e.g., 20 µL) sample is taken from the apical side, and transport buffer (e.g., 60 µL) is added. A lucifer yellow sample (e.g., 80 µL) is taken from the basolateral side. The relative fluorescence unit (RFU) of lucifer yellow is measured at 425/528 nm (excitation/emission) with an Envision plate reader.

Percent of lucifer yellow in the basolateral well is calculated using the equation: $%Lucifer Yellow = \frac{{V}_{Basolateral} \times {RFU}_{Basolateral}}{\left({V}_{Apical}\times {RFU}_{Apical}\right) + \left({V}_{Basolateral}\times {RFU}_{Basolateral}\right)}$ where RFU_{Apical} and RFU_{Basolateral} are the relative fluorescence unit values of lucifer yellow in the apical and basolateral wells, respectively; V_{Apical} and V_{Basolateral} are the volume of apical and basolateral wells (0.075 mL and 0.25 mL), respectively. The %Lucifer Yellow should be less than 2.0 to indicate an intact cell monolayer.

In some embodiments, a compound provided herein, or a pharmaceutically acceptable salt thereof, can be assessed for formation of metabolites. For example, a compound provided herein, or a pharmaceutically acceptable salt thereof can be incubated with hepatocytes (e.g., rat hepatocytes, dog hepatocytes, or human hepatocytes), and at the end of incubation, the sample can be analyzed for the amount of the compound provided herein, or a pharmaceutically acceptable salt thereof, remaining, as well as for the amount of any metabolite from Phase 1 metabolism, Phase 2 metabolism, or a combination thereof. An exemplary protocol follows.

A working solution of a compound provided herein, or a pharmaceutically acceptable salt thereof, is prepared (e.g. 10 µM in DMSO). Similarly, a working solution of a control compound (e.g., 7-ethoxycoumarin) is prepared (e.g. 30 µM in DMSO). An aliquot of each working solution of a compound provided herein, or a pharmaceutically acceptable salt thereof, or the working solution of the control compound is incubated with hepatocytes (e.g., 1.0 × 10⁶ cells/mL) (e.g., rat hepatocytes, dog hepatocytes, or human hepatocytes) in incubation medium (e.g., Williams' Medium E with HEPES (e.g., 5.958 g/L) glutamine (e.g., 0.292 g/L)), to a total volume of 200 µL, for 0 minutes or 120 minutes at 37 °C in 5% CO₂/saturated humidity. After incubation, MeCN (800 µL) is added to each sample, and the samples are centrifuged. The supernatants are dried under N₂ gas, and the residue is reconstituted (e.g., with 200 µL of 10% MeCN with 0.1% FA). The reconstituted residue of the compound provided herein, or a pharmaceutically acceptable salt thereof, is subjected to LC-UV-MS (e.g., LC with a Mobile Phase A of 0.1% FA and 2 mM NH₄FA in H₂O/MeCN (v : v = 95 : 5), a Mobile Phase B of 0.1% FA and 2 mM NH₄FA in H₂O/MeCN (v : v = 5 : 95) using an ACQUITY UPLC^{®} HSS T3 2.1 × 100 mm, 1.8 µm column; a UV detector with λ: 190~500 nm; and MS with a Xevo G2 Q-TOF instrument in ESI⁺ mode with a scanning mode of MS^{E}/MS²). The reconstituted residue of the control compound is subjected to LC-UV-MS (e.g., LC with a Mobile Phase A of 0.1% FA in H₂O, a Mobile Phase B of 0.1% FA in MeCN using an ACQUITY UPLC^{®} HSS T3 2.1 × 100 mm, 1.8 µm column; a UV detector with λ: 190~500 nm; and MS with a Xevo G2 Q-TOF instrument in ESI⁺ mode with a scanning mode of MS^{E}). The data is then analyzed to identify and, if desired, quantify the metabolites.

In some embodiments, a compounds provide herein, or a pharmaceutically acceptable salt thereof, can exhibit potent and selective inhibition of a dysregulated KRas protein (e.g., a wild-type KRas protein and/or a mutant KRas protein (e.g., a KRas G12D mutant protein, a KRas G12R mutant protein, and/or a KRas G12V mutant protein (e.g., a KRas G12D mutant protein and/or a KRas G12V mutant protein))). In some embodiments, a compound provided herein, or a pharmaceutically acceptable salt thereof, can selectively inhibit a dysregulated KRas protein (e.g., a wild-type KRas protein and/or a mutant KRas protein (e.g., a KRas G12D mutant protein, a KRas G12R mutant protein, and/or a KRas G12V mutant protein (e.g., a KRas G12D mutant protein and/or a KRas G12V mutant protein))) over another GTPase or non-GTPase target. In some embodiments, a compound provided herein can exhibit nanomolar potency against a KRas protein (e.g., a wild-type KRas protein and/or a mutant KRas protein (e.g., a KRas G12D mutant protein, a KRas G12R mutant protein, and/or a KRas G12V mutant protein (e.g., a KRas G12D mutant protein and/or a KRas G12V mutant protein))) with minimal activity against related GTPases (e.g., wild-type NRas protein, and/or wild-type HRas protein).

In some embodiments, a compound provided herein, or a pharmaceutically acceptable salt thereof, can exhibit greater inhibition of a KRas protein (e.g., a wild-type KRas protein and/or a mutant KRas protein (e.g., a KRas G12D mutant protein, a KRas G12R mutant protein, and/or a KRas G12V mutant protein (e.g., a KRas G12D mutant protein and/or a KRas G12V mutant protein))) relative to inhibition of a related GTPase (e.g., wild-type NRas protein, and/or wild-type HRas protein). In some embodiments, a compound provided herein, or a pharmaceutically acceptable salt thereof, can exhibit at least 2-fold, 3-fold, 5-fold, 10-fold, 25-fold, 50-fold, or 100-fold greater inhibition of a KRas protein (e.g., a wild-type KRas protein and/or a mutant KRas protein (e.g., a KRas G12D mutant protein, a KRas G12R mutant protein, and/or a KRas G12V mutant protein (e.g., a KRas G12D mutant protein and/or a KRas G12V mutant protein))) relative to inhibition of a related GTPase (e.g., wild-type NRas protein, and/or wild-type HRas protein). In some embodiments, a compound provided herein, or a pharmaceutically acceptable salt thereof, can exhibit up to 10000-fold greater inhibition of a KRas protein (e.g., a wild-type KRas protein and/or a mutant KRas protein (e.g., a KRas G12D mutant protein, a KRas G12R mutant protein, and/or a KRas G12V mutant protein (e.g., a KRas G12D mutant protein and/or a KRas G12V mutant protein))) relative to inhibition of a related GTPase (e.g., wild-type NRas protein, and/or wild-type HRas protein).

In some embodiments, a compound provided herein, or a pharmaceutically acceptable salt thereof, can exhibit from about 2-fold to about 10-fold greater inhibition of a KRas protein (e.g., a wild-type KRas protein and/or a mutant KRas protein (e.g., a KRas G12D mutant protein, a KRas G12R mutant protein, and/or a KRas G12V mutant protein (e.g., a KRas G12D mutant protein and/or a KRas G12V mutant protein))) relative to inhibition of a related GTPase (e.g., wild-type NRas protein, and/or wild-type HRas protein). In some embodiments, a compound provided herein, or a pharmaceutically acceptable salt thereof, can exhibit from about 10-fold to about 100-fold greater inhibition of a KRas protein (e.g., a wild-type KRas protein and/or a mutant KRas protein (e.g., a KRas G12D mutant protein, a KRas G12R mutant protein, and/or a KRas G12V mutant protein (e.g., a KRas G12D mutant protein and/or a KRas G12V mutant protein))) relative to inhibition of a related GTPase (e.g., wild-type NRas protein, and/or wild-type HRas protein). In some embodiments, a compound provided herein, or a pharmaceutically acceptable salt thereof, can exhibit from about 100-fold to about 1000-fold greater inhibition of a KRas protein (e.g., a wild-type KRas protein and/or a mutant KRas protein (e.g., a KRas G12D mutant protein, a KRas G12R mutant protein, and/or a KRas G12V mutant protein (e.g., a KRas G12D mutant protein and/or a KRas G12V mutant protein))) relative inhibition of a related GTPase (e.g., wild-type NRas protein, and/or wild-type HRas protein). In some embodiments, a compound provided herein, or a pharmaceutically acceptable salt thereof, can exhibit from about 1000-fold to about 10000-fold greater inhibition of a KRas protein (e.g., a wild-type KRas protein and/or a mutant KRas protein (e.g., a KRas G12D mutant protein, a KRas G12R mutant protein, and/or a KRas G12V mutant protein (e.g., a KRas G12D mutant protein and/or a KRas G12V mutant protein))) relative to inhibition of a related GTPase (e.g., wild-type NRas protein, and/or wild-type HRas protein).

In some embodiments, the compounds provided herein, or pharmaceutically acceptable salts thereof, can exhibit potent and selective inhibition of a dysregulated KRas protein (e.g., a mutant KRas protein (e.g., a KRas G12D mutant protein, a KRas G12R mutant protein, and/or a KRas G12V mutant protein (e.g., a KRas G12D mutant protein and/or a KRas G12V mutant protein))). In some embodiments, a compound provided herein, or a pharmaceutically acceptable salt thereof, can selectively inhibit a dysregulated KRas protein (e.g., a mutant KRas protein (e.g., a KRas G12D mutant protein, a KRas G12R mutant protein, and/or a KRas G12V mutant protein (e.g., a KRas G12D mutant protein and/or a KRas G12V mutant protein))) over another GTPase or non-GTPase target. In some embodiments, the compounds provided herein can exhibit nanomolar potency against a mutant KRas protein (e.g., a KRas G12D mutant protein, a KRas G12R mutant protein, and/or a KRas G12V mutant protein (e.g., a KRas G12D mutant protein and/or a KRas G12V mutant protein))) with minimal activity against related GTPases (e.g., wild-type KRas protein, wild-type NRas protein, and/or wild-type HRas protein).

In some embodiments, a compound provided herein, or a pharmaceutically acceptable salt thereof, can exhibit greater inhibition of a mutant KRas protein (e.g., a KRas G12D mutant protein, a KRas G12R mutant protein, and/or a KRas G12V mutant protein (e.g., a KRas G12D mutant protein and/or a KRas G12V mutant protein)) relative to inhibition of wild-type KRas protein. In some embodiments, a compound provided herein, or a pharmaceutically acceptable salt thereof, can exhibit at least 2-fold, 3-fold, 5-fold, 10-fold, 25-fold, 50-fold, or 100-fold greater inhibition of a mutant KRas protein (e.g., a KRas G12D mutant protein, a KRas G12R mutant protein, and/or a KRas G12V mutant protein (e.g., a KRas G12D mutant protein and/or a KRas G12V mutant protein)) relative to inhibition of wild-type KRas protein. In some embodiments, a compound provided herein, or a pharmaceutically acceptable salt thereof, can exhibit up to 10000-fold greater inhibition of a mutant KRas protein (e.g., a KRas G12D mutant protein, a KRas G12R mutant protein, and/or a KRas G12V mutant protein (e.g., a KRas G12D mutant protein and/or a KRas G12V mutant protein)) relative to inhibition of wild-type KRas protein.

In some embodiments, a compound provided herein, or a pharmaceutically acceptable salt thereof, can exhibit from about 2-fold to about 10-fold greater inhibition of a mutant KRas protein (e.g., a KRas G12D mutant protein, a KRas G12R mutant protein, and/or a KRas G12V mutant protein (e.g., a KRas G12D mutant protein and/or a KRas G12V mutant protein)) relative to inhibition of wild-type KRas protein. In some embodiments, a compound provided herein, or a pharmaceutically acceptable salt thereof, can exhibit from about 10-fold to about 100-fold greater inhibition of a mutant KRas protein (e.g., a KRas G12D mutant protein, a KRas G12R mutant protein, and/or a KRas G12V mutant protein (e.g., a KRas G12D mutant protein and/or a KRas G12V mutant protein)) relative to inhibition of wild-type KRas protein. In some embodiments, a compound provided herein, or a pharmaceutically acceptable salt thereof, can exhibit from about 100-fold to about 1000-fold greater inhibition of a mutant KRas protein (e.g., a KRas G12D mutant protein, a KRas G12R mutant protein, and/or a KRas G12V mutant protein (e.g., a KRas G12D mutant protein and/or a KRas G12V mutant protein)) relative to inhibition of wild-type KRas protein. In some embodiments, a compound provided herein, or a pharmaceutically acceptable salt thereof, can exhibit from about 1000-fold to about 10000-fold greater inhibition of a mutant KRas protein (e.ga KRas G12D mutant protein, a KRas G12R mutant protein, and/or a KRas G12V mutant protein (e.g., a KRas G12D mutant protein and/or a KRas G12V mutant protein)) relative to inhibition of wild-type KRas protein.

In some embodiments, a compound provided herein, or a pharmaceutically acceptable salt thereof, can exhibit nanomolar potency against a dysregulated KRas protein (e.g., a mutant KRas protein (e.g., a KRas G12D mutant protein, a KRas G12R mutant protein, and/or a KRas G12V mutant protein (e.g., a KRas G12D mutant protein and/or a KRas G12V mutant protein))) with minimal activity against wild-type NRas protein and/or wild-type HRas protein In some embodiments, a compound provided herein, or a pharmaceutically acceptable salt thereof, can exhibit greater inhibition of a dysregulated KRas protein (e.g., a mutant KRas protein (e.g., a KRas G12D mutant protein, a KRas G12R mutant protein, and/or a KRas G12V mutant protein (e.g., a KRas G12D mutant protein and/or a KRas G12V mutant protein))) relative to inhibition of wild-type NRas protein and/or wild-type HRas protein. In some embodiments, a compound provided herein, or a pharmaceutically acceptable salt thereof, can exhibit at least 2-fold, 3-fold, 5-fold, 10-fold, 25-fold, 50-fold or 100-fold greater inhibition of a dysregulated KRas protein (e.g., a mutant KRas protein (e.g., a KRas G12D mutant protein, a KRas G12R mutant protein, and/or a KRas G12V mutant protein (e.g., a KRas G12D mutant protein and/or a KRas G12V mutant protein))) relative to inhibition of wild-type NRas protein and/or wild-type HRas protein. In some embodiments, a compound provided herein, or a pharmaceutically acceptable salt thereof, can exhibit up to 1000-fold greater inhibition of a dysregulated KRas protein (e.g., a mutant KRas protein (e.g., a KRas G12D mutant protein, a KRas G12R mutant protein, and/or a KRas G12V mutant protein (e.g., a KRas G12D mutant protein and/or a KRas G12V mutant protein))) relative to inhibition of wild-type NRas protein and/or wild-type HRas protein. In some embodiments, a compound provided herein, or a pharmaceutically acceptable salt thereof, can exhibit up to 10000-fold greater inhibition of a dysregulated KRas protein (e.g., a mutant KRas protein (e.g., a KRas G12D mutant protein, a KRas G12R mutant protein, and/or a KRas G12V mutant protein (e.g., a KRas G12D mutant protein and/or a KRas G12V mutant protein))) relative to inhibition of wild-type NRas protein and/or wild-type HRas protein.

In some embodiments, a compound provided herein, or a pharmaceutically acceptable salt thereof, can exhibit from about 2-fold to about 10-fold greater inhibition of a dysregulated KRas protein (e.g., a mutant KRas protein (e.g., a KRas G12D mutant protein, a KRas G12R mutant protein, and/or a KRas G12V mutant protein (e.g., a KRas G12D mutant protein and/or a KRas G12V mutant protein))) relative to inhibition of wild-type NRas protein and/or wild-type HRas protein. In some embodiments, a compound provided herein, or a pharmaceutically acceptable salt thereof, can exhibit from about 10-fold to about 100-fold greater inhibition of a dysregulated KRas protein (e.g., a mutant KRas protein (e.g., a KRas G12D mutant protein, a KRas G12R mutant protein, and/or a KRas G12V mutant protein (e.g., a KRas G12D mutant protein and/or a KRas G12V mutant protein))) relative to inhibition of wild-type HRas protein and/or wild-type NRas protein. In some embodiments, a compound provided herein, or a pharmaceutically acceptable salt thereof, can exhibit from about 100-fold to about 1000-fold greater inhibition of a dysregulated KRas protein (e.g., a mutant KRas protein (e.g., a KRas G12D mutant protein, a KRas G12R mutant protein, and/or a KRas G12V mutant protein (e.g., a KRas G12D mutant protein and/or a KRas G12V mutant protein))) relative to inhibition of wild-type NRas protein and/or wild-type HRas protein. In some embodiments, a compound provided herein, or a pharmaceutically acceptable salt thereof, can exhibit from about 1000-fold to about 10000-fold greater inhibition of a dysregulated KRas protein (e.g., a mutant KRas protein (e.g., a KRas G12D mutant protein, a KRas G12R mutant protein, and/or a KRas G12V mutant protein (e.g., a KRas G12D mutant protein and/or a KRas G12V mutant protein))) relative to inhibition of wild-type NRas protein and/or wild-type HRas protein.

Compounds provided herein, or pharmaceutically acceptable salts thereof, are useful for treating diseases and disorders including cardiovascular disease (e.g., arteriovenous malformations or Noonan syndrome), endometriosis, an inflammatory and/or autoimmune disease (e.g., a nonmalignant syndrome of autoimmunity and abnormal leukocyte homeostasis), proliferative disorders such as cancers, including hematological cancers and solid tumors (e.g., advanced solid tumors), and disorders of the MAPK pathway (e.g., neurofibromatosis type 1). In some embodiments, the diseases and disorders are KRas-associated diseases and disorders (e.g., mutant KRas-associated diseases or disorders (e.g., KRas G12D-, KRas G12R-, or G12V-associated diseases or disorders)).

In certain embodiments, compounds provided herein, or pharmaceutically acceptable salts thereof, are useful for preventing diseases and disorders as defined herein (for example, a cardiovascular disease, endometriosis, and an inflammatory and/or autoimmune disease, or cancer).

In some embodiments of any of the methods or uses described herein, the inflammatory and/or autoimmune disease is RAS-associated autoimmune leukoproliferative disease. See, e.g., Niemela et al. Blood. 2011; 117(10):2883-6, doi: 10.1182/blood-2010-07-295501.

In some embodiments, the subject has been identified or diagnosed as having a cancer with a KRas dysregulation (e.g., a KRas mutation or amplification) (e.g., as determined using a regulatory agency-approved, e.g., FDA-approved, assay or kit). In some embodiments, the subject has a cancer (e.g., a tumor sample) that has a KRas dysregulation (e.g., a KRas mutation or amplification) (e.g., as determined using a regulatory agency-approved assay or kit). The subject can be a subject with a cancer (e.g., one or more tumor samples) that is positive for a KRas dysregulation (e.g., a KRas mutation or amplification) (e.g., identified as positive using a regulatory agency-approved, e.g., FDA-approved, assay or kit). The subject can be a subject whose cancer (e.g., a tumor sample) has a KRas dysregulation (e.g., a KRas mutation or amplification) (e.g., where the cancer (e.g., tumor sample) is identified as such using a regulatory agency-approved, e.g., FDA-approved, kit or assay). In some embodiments, the subject is suspected of having a mutant KRas-associated cancer. In some embodiments, the subject has a clinical record indicating that the subject has a cancer (e.g., a tumor sample) that has a KRas dysregulation (e.g., a KRas mutation or amplification) (and optionally the clinical record indicates that the subject should be treated with any of the compounds and/or compositions provided herein).

In some such embodiments, the cancer (e.g., a tumor sample) has a KRas mutation selected from the group consisting of: a KRas G12X mutation, a KRas G13X mutation, and a KRas Q61X mutation. In some embodiments, a KRas mutation is selected from the group consisting of: a KRas G12A mutation, a KRas G12C mutation, a KRas G12D mutation, a KRas G12R mutation, a KRas G12S mutation, a KRas G12V mutation, a KRas G13C mutation, a KRas G13D mutation, a KRas G13V mutation, a KRas Q61E mutation, a KRas Q61H mutation, a KRas Q61K mutation, a KRas Q61L mutation, a KRas Q61P mutation, and a KRas Q61R mutation. In some embodiments, a KRas mutation is selected from the group consisting of: a KRas G12A mutation, a KRas G12C mutation, a KRas G12D mutation, a KRas G12R mutation, a KRas G12S mutation, and a KRas G12V mutation. In some embodiments, the cancer (e.g., a tumor sample) has a KRas mutation selected from the group consisting of: a KRas G12A mutation, a KRas G12D mutation, a KRas G12R mutation, and a KRas G12V mutation. In some embodiments, the cancer (e.g., a tumor sample) has a KRas G12A mutation. In some embodiments, the cancer (e.g., a tumor sample) has a KRas G12C mutation. In some embodiments, the cancer (e.g., a tumor sample) has a KRas G12D mutation. In some embodiments, the cancer (e.g., a tumor sample) has a KRas G12R mutation. In some embodiments, the cancer (e.g., a tumor sample) has a KRas G12S mutation. In some embodiments, the cancer (e.g., a tumor sample) has a KRas G12Vmutation.

In some embodiments, the cancer (e.g., a tumor sample) has a KRas G12D mutation, a KRas G12R mutation, or KRas G12V mutation (e.g., a KRas G12D mutation or a KRas G12V mutation). In some embodiments, the cancer (e.g., a tumor sample) has a KRas G12D mutation or a KRas G12V mutation. In some embodiments, the cancer (e.g., a tumor sample) has a KRas G12D mutation. In some embodiments, the cancer (e.g., a tumor sample) has a KRas G12R mutation. In some embodiments, the cancer (e.g., a tumor sample) has a KRas G12V mutation.

The term "KRas-associated cancer" as used herein refers to cancers associated with or having a dysregulation of a *KRAS* gene, a KRas protein, or the expression or activity or level of any (e.g., one or more) of the same (e.g., any of the types of dysregulations of a *KRAS* gene, a KRas protein, or the expression or activity or level of any of the same described herein). Non-limiting examples of a KRas-associated cancer are described herein.

The term "mutant KRas-associated cancer" as used herein refers to cancers associated with or having a KRas mutation (e.g., a *KRAS* gene having a mutation corresponding to a mutation in a KRas protein and/or a KRas protein having a mutation). Non-limiting examples of a mutant KRas-associated cancer are described herein.

The term "KRas G12X-associated cancer" as used herein refers to cancers associated with or having a KRas G12X mutation (e.g., a *KRAS* gene having a mutation corresponding to a G12X mutation in a KRas protein and/or a KRas protein having a G12X mutation). Non-limiting examples of a KRas G12X-associated cancer are described herein.

The term "KRas G12A-associated cancer" as used herein refers to cancers associated with or having a KRas G12A mutation (e.g., a *KRAS* gene having a mutation corresponding to a G12A mutation in a KRas protein and/or a KRas protein having a G12A mutation). Nonlimiting examples of a KRas G12A-associated cancer are described herein.

The term "KRas G12C-associated cancer" as used herein refers to cancers associated with or having a KRas G12C mutation (e.g., a *KRAS* gene having a mutation corresponding to a G12C mutation in a KRas protein and/or a KRas protein having a G12C mutation). Nonlimiting examples of a KRas G12C-associated cancer are described herein.

The term "KRas G12D-associated cancer" as used herein refers to cancers associated with or having a KRas G12D mutation (e.g., a *KRAS* gene having a mutation corresponding to a G12D mutation in a KRas protein and/or a KRas protein having a G12D mutation). Nonlimiting examples of a KRas G12D-associated cancer are described herein.

The term "KRas G12R-associated cancer" as used herein refers to cancers associated with or having a KRas G12R mutation (e.g., a *KRAS* gene having a mutation corresponding to a G12R mutation in a KRas protein and/or a KRas protein having a G12R mutation). Nonlimiting examples of a KRas G12R-associated cancer are described herein.

The term "KRas G12S-associated cancer" as used herein refers to cancers associated with or having a KRas G12S mutation (e.g., a *KRAS* gene having a mutation corresponding to a G12S mutation in a KRas protein and/or a KRas protein having a G12S mutation). Nonlimiting examples of a KRas G12S-associated cancer are described herein.

The term "KRas G12V-associated cancer" as used herein refers to cancers associated with or having a KRas G12V mutation (e.g., a *KRAS* gene having a mutation corresponding to a G12V mutation in a KRas protein and/or a KRas protein having a G12V mutation). Nonlimiting examples of a KRas G12V-associated cancer are described herein.

The term "KRas G13X-associated cancer" as used herein refers to cancers associated with or having a KRas G13X mutation (e.g., a *KRAS* gene having a mutation corresponding to a G13X mutation in a KRas protein and/or a KRas protein having a G13X mutation). Nonlimiting examples of a KRas G13X-associated cancer are described herein.

The term "KRas G13C-associated cancer" as used herein refers to cancers associated with or having a KRas G13C mutation (e.g., a *KRAS* gene having a mutation corresponding to a G13C mutation in a KRas protein and/or a KRas protein having a G13C mutation). Nonlimiting examples of a KRas G13C-associated cancer are described herein.

The term "KRas G13D-associated cancer" as used herein refers to cancers associated with or having a KRas G13D mutation (e.g., a *KRAS* gene having a mutation corresponding to a G13D mutation in a KRas protein and/or a KRas protein having a G13D mutation). Nonlimiting examples of a KRas G13D-associated cancer are described herein.

The term "KRas G13V-associated cancer" as used herein refers to cancers associated with or having a KRas G13V mutation (e.g., a *KRAS* gene having a mutation corresponding to a G13V mutation in a KRas protein and/or a KRas protein having a G13V mutation). Nonlimiting examples of a KRas G13V-associated cancer are described herein.

The term "KRas Q61X-associated cancer" as used herein refers to cancers associated with or having a KRas Q61X mutation (e.g., a *KRAS* gene having a mutation corresponding to a Q61X mutation in a KRas protein and/or a KRas protein having a Q61X mutation). Nonlimiting examples of a KRas Q61X-associated cancer are described herein.

The term "KRas Q61E-associated cancer" as used herein refers to cancers associated with or having a KRas Q61E mutation (e.g., a *KRAS* gene having a mutation corresponding to a Q61E mutation in a KRas protein and/or a KRas protein having a Q61E mutation). Nonlimiting examples of a KRas Q61E-associated cancer are described herein.

The term "KRas Q61H-associated cancer" as used herein refers to cancers associated with or having a KRas Q61H mutation (e.g., a *KRAS* gene having a mutation corresponding to a Q61H mutation in a KRas protein and/or a KRas protein having a Q61H mutation). Nonlimiting examples of a KRas Q61H-associated cancer are described herein.

The term "KRas Q61K-associated cancer" as used herein refers to cancers associated with or having a KRas Q61K mutation (e.g., a *KRAS* gene having a mutation corresponding to a Q61K mutation in a KRas protein and/or a KRas protein having a Q61K mutation). Nonlimiting examples of a KRas Q61K-associated cancer are described herein.

The term "KRas Q61L-associated cancer" as used herein refers to cancers associated with or having a KRas Q61L mutation (e.g., a *KRAS* gene having a mutation corresponding to a Q61L mutation in a KRas protein and/or a KRas protein having a Q61L mutation). Nonlimiting examples of a KRas Q61L-associated cancer are described herein.

The term "KRas Q61P-associated cancer" as used herein refers to cancers associated with or having a KRas Q61P mutation (e.g., a *KRAS* gene having a mutation corresponding to a Q61P mutation in a KRas protein and/or a KRas protein having a Q61P mutation). Nonlimiting examples of a KRas Q61P-associated cancer are described herein.

The term "KRas Q61R-associated cancer" as used herein refers to cancers associated with or having a KRas Q61R mutation (e.g., a *KRAS* gene having a mutation corresponding to a Q61R mutation in a KRas protein and/or a KRas protein having a Q61R mutation). Nonlimiting examples of a KRas Q61R-associated cancer are described herein.

Such mutations can be associated with the development of a variety of cancers. See, e.g., Hunter et al. Mol Cancer Res. 2015;13(9):1325-35, doi: 10.1158/1541-7786.MCR-15-0203.

Provided herein are methods of treating a cancer in a subject in need of such treatment, the methods comprising administering to the subject a therapeutically effective amount of a compound provided herein, or a pharmaceutically acceptable salt thereof, or a pharmaceutical composition thereof. In some embodiments, the subject is treatment naive with respect to the cancer. In some embodiments, the subject has received one or more lines of previous therapy for the cancer.

Also provided herein are methods of treating a cancer in a subject in need thereof, the methods comprising administering to the subject a therapeutically effective amount of a compound provided herein, or a pharmaceutically acceptable salt thereof, or a pharmaceutical composition thereof, as a monotherapy. In some embodiments, the subject is treatment naive with respect to the cancer. In some embodiments, the subject has received one or more lines of previous therapy for the cancer.

In some embodiments, the subject has received one or more lines of previous therapy for the cancer prior to administration of a compound provided herein. In some such embodiments, the subject has received previous chemotherapy for the cancer prior to administration of a compound provided herein. In some embodiments, the subject has received first- or second-line chemotherapy prior to administration of a compound provided herein. In some embodiments, the subject has received standard of care chemotherapy prior to administration of a compound provided herein.

In some embodiments, a subject with colorectal cancer has previously received one or more of capecitabine, fluorouracil (5-FU), leucovorin, and oxaliplatin. In some embodiments, a subject with colorectal cancer has previously received FOLFOX (fluorouracil, leucovorin, and oxaliplatin). In some embodiments, a subject with colorectal cancer has previously received FOLFIRI (fluorouracil, leucovorin, and irinotecan). In some embodiments, a subject with a colorectal cancer has previously received FOLFIRINOX (fluorouracil, leucovorin (e.g., leucovorin calcium), irinotecan (e.g., irinotecan hydrochloride), and oxaliplatin). In some embodiments, a subject with colorectal cancer has previously received CAPEOX (capecitabine and oxaliplatin). In some embodiments, a subject with colorectal cancer has previously received FOLFIRI and one or more of bevacizumab, ziv-aflibercept, and ramucirumab. In some embodiments, a subject with colorectal cancer has previously received FOLFIRI and one or more of cetuximab or panitumumab. In some embodiments, a subject with colorectal cancer has previously received FOLFOX and one or more of bevacizumab, ziv-aflibercept, and ramucirumab. In some embodiments, a subject with colorectal cancer has previously received FOLFOX and one or more of cetuximab or panitumumab. In some embodiments, a subject with colorectal cancer has previously received FOLFIRINOX and one or more of bevacizumab, ziv-aflibercept, and ramucirumab. In some embodiments, a subject with colorectal cancer has previously received FOLFIRINOX and one or more of cetuximab or panitumumab. In some embodiments, a subject with colorectal cancer has previously received CAPEOX and one or more of bevacizumab, ziv-aflibercept, and ramucirumab. In some embodiments, a subject with colorectal cancer has previously received CAPEOX and one or more of cetuximab or panitumumab.

In some embodiments, a subject with endometrial cancer has previously received one or more of cisplatin, carboplatin, paclitaxel, capecitabine, mitomycin, and gemcitabine. In some embodiments, a subject with endometrial cancer has previously received cisplatin and radiation therapy. In some embodiments, a subject with endometrial cancer has previously received cisplatin and radiation therapy followed by carboplatin and paclitaxel. In some embodiments, a subject with endometrial cancer has previously received capecitabine and mitomycin. In some embodiments, a subject with endometrial cancer has previously received gemcitabine. In some embodiments, a subject with endometrial cancer has previously received paclitaxel. In some embodiments, a subject with endometrial cancer has previously received carboplatin and paclitaxel. In some embodiments, a subject with endometrial cancer has previously received carboplatin, paclitaxel, and pembrolizumab. In some embodiments, a subject with endometrial cancer has previously received carboplatin, paclitaxel, and dostarlimab (e.g., dostarlimab-gxly). In some embodiments, a subject with endometrial cancer has previously received carboplatin, paclitaxel, and trastuzumab. In some embodiments, a subject with endometrial cancer has previously received carboplatin, paclitaxel, and bevacizumab. In some embodiments, a subject with endometrial carcinoma has previously received megestrol acetate and tamoxifen. In some embodiments, a subject with endometrial carcinoma has previously received everolimus and letrozole.

In some embodiments, a subject with lung cancer (e.g., NSCLC) has previously received one or more of pembrolizumab, atezolizumab, cemiplimab (e.g., cemiplimiab-rwlc), durvalumab, nivolumab, ipilimumab, tremelimumab (e.g., tremelimumab-actl), carboplatin, cisplatin, pemetrexed, paclitaxel (e.g., albumin-bound paclitaxel), and bevacizumab. In some embodiments, a subject with lung cancer (e.g., NSCLC) has previously received pembrolizumab. In some embodiments, a subject with lung cancer (e.g., NSCLC) has previously received carboplatin, pemetrexed, and pembrolizumab. In some embodiments, a subject with lung cancer (e.g., NSCLC) has previously received cisplatin, pemetrexed, and pembrolizumab. In some embodiments, a subject with lung cancer (e.g., NSCLC) has previously received cemiplimab. In some embodiments, a subject with lung cancer (e.g., NSCLC) has previously received cemiplimab, pemetrexed, and carboplatin. In some embodiments, a subject with lung cancer (e.g., NSCLC) has previously received cemiplimab, pemetrexed, and cisplatin. In some embodiments, a subject with lung cancer (e.g., NSCLC) has previously received carboplatin, paclitaxel, bevacizumab, and atezolizumab. In some embodiments, a subject with lung cancer (e.g., NSCLC) has previously received carboplatin, paclitaxel (e.g., albumin-bound paclitaxel), and atezolizumab. In some embodiments, a subject with lung cancer (e.g., NSCLC) has previously received cemiplimab, paclitaxel, and carboplatin. In some embodiments, a subject with lung cancer (e.g., NSCLC) has previously received cemiplimab, paclitaxel, and cisplatin. In some embodiments, a subject with lung cancer (e.g., NSCLC) has previously received tremelimumab, durvalumab, carboplatin, and paclitaxel (e.g., albumin-bound paclitaxel). In some embodiments, a subject with lung cancer (e.g., NSCLC) has previously received tremelimumab, durvalumab, carboplatin, and pemetrexed. In some embodiments, a subject with lung cancer (e.g., NSCLC) has previously received tremelimumab, durvalumab, cisplatin, and pemetrexed. In some embodiments, a subject with lung cancer (e.g., NSCLC) has previously received tremelimumab, durvalumab, carboplatin, and paclitaxel (e.g., albumin-bound paclitaxel). In some embodiments, a subject with lung cancer (e.g., NSCLC) has previously received tremelimumab, durvalumab, carboplatin, and gemcitabine. In some embodiments, a subject with lung cancer (e.g., NSCLC) has previously received tremelimumab, durvalumab, cisplatin, and gemcitabine. In some embodiments, a subject with lung cancer (e.g., NSCLC) has previously received nivolumab and ipilimumab. In some embodiments, a subject with lung cancer (e.g., NSCLC) has previously received carboplatin, paclitaxel (e.g., albumin-bound paclitaxel), and pembrolizumab. In some embodiments, a subject with lung cancer (e.g., NSCLC) has previously received nivolumab, ipilimumab, paclitaxel, and carboplatin.

In some embodiments, a subject with ovarian cancer has previously received one or more of paclitaxel, carboplatin, fluorouracil, leucovorin, oxaliplatin, capecitabine, docetaxel, and doxorubicin (e.g., liposomal doxorubicin). In some embodiments, a subject with ovarian cancer has previously received paclitaxel and carboplatin. In some embodiments, a subject with ovarian cancer has previously received fluorouracil, leucovorin, and oxaliplatin. In some embodiments, a subject with ovarian cancer has previously received hormone therapy (e.g., an aromatase inhibitor such as anastrozole, letrozole, or exemestane). In some embodiments, a subject with ovarian cancer has previously received docetaxel and carboplatin. In some embodiments, a subject with ovarian cancer has previously received carboplatin and doxorubicin (e.g., liposomal doxorubicin). In some embodiments, a subject with ovarian cancer has previously received paclitaxel, carboplatin, and bevacizumab. In some embodiments, a subject with ovarian cancer has previously received fluorouracil, leucovorin, oxaliplatin, and bevacizumab. In some embodiments, a subject with ovarian cancer has previously received capecitabine, oxaliplatin, and bevacizumab.

In some embodiments, a subject with pancreatic cancer has previously received one or more of capecitabine, fluorouracil, gemcitabine, irinotecan, leucovorin, paclitaxel (e.g., albumin-bound paclitaxel), and oxaliplatin. In some embodiments, a subject with pancreatic cancer has previously received FOLFIRINOX. In some embodiments, a subj ect with pancreatic cancer has previously received GEMOX (gemcitabine and oxaliplatin). In some embodiments, a subject with pancreatic cancer has previously received gemcitabine. In some embodiments, a subject with pancreatic cancer has previously received gemcitabine and paclitaxel (e.g., albumin-bound paclitaxel). In some embodiments, a subject with pancreatic cancer has previously received NALIRIFOX (liposomal irinotecan, fluorouracil, leucovorin, and oxaliplatin).

Provided herein is use of a compound provided herein, or a pharmaceutically acceptable salt thereof, or a pharmaceutical composition thereof, for the treatment of cancer, for example, any of the cancers provided herein.

Provided herein is use of a compound provided herein, or a pharmaceutically acceptable salt thereof, or a pharmaceutical composition thereof, as a medicament for the treatment of cancer, for example, any of the cancers provided herein.

Provided herein is use of a compound provided herein, or a pharmaceutically acceptable salt thereof, in the manufacture of a medicament for the treatment of cancer, for example, any of the cancers provided herein.

Provided herein is a compound provided herein, or a pharmaceutically acceptable salt thereof, or a pharmaceutical composition thereof, for use as a medicament. Also provided herein is a compound provided herein, or a pharmaceutically acceptable salt thereof, or a pharmaceutical composition thereof, for use as a medicament for the treatment of cancer, for example, any of the cancers provided herein.

Provided herein is a compound provided herein, or a pharmaceutically acceptable salt thereof, or a pharmaceutical composition thereof, for use in treating a cancer, for example, any of the cancers provided herein.

As used herein, "monotherapy", when referring to a compound provided herein, or a pharmaceutically acceptable salt thereof, means that the compound provided herein, or a pharmaceutically acceptable salt thereof, is the only therapeutic agent or therapy (e.g., anticancer agent or therapy) administered to the subject during the treatment cycle (e.g., no additional targeted therapeutics, anticancer agents, chemotherapeutics, or checkpoint inhibitors are administered to the subject during the treatment cycle). As a person of ordinary skill in the art would understand, monotherapy does not exclude the co-administration of medicaments for the treatment of side effects or general symptoms associated with the cancer or treatment, such as pain, rash, edema, photosensitivity, pruritis, skin discoloration, hair brittleness, hair loss, brittle nails, cracked nails, discolored nails, swollen cuticles, fatigue, weight loss, general malaise, shortness of breath, infection, anemia, or gastrointestinal symptoms, including nausea, diarrhea, and lack of appetite.

As used herein, "the subject has received one or more lines of previous therapy" means that the subject has been previously administered one or more therapeutic agents or therapies (e.g., anticancer agent or therapy, such as chemotherapy, radiation, or surgery) for the cancer other than a compound provided herein, or a pharmaceutically acceptable salt thereof, during a treatment cycle prior to administration of a compound provided herein. In some embodiments, the subject cannot tolerate the one or more therapeutic agents or therapies previously administered for the cancer. In some embodiments, the subject did not respond to the one or more therapeutic agents or therapies previously administered for the cancer. In some embodiments, the subject did not adequately respond to one or more therapeutic agents or therapies previously administered for the cancer. In some embodiments, the subject has stopped responding to the one or more therapeutic agents or therapies previously administered for the cancer. In some embodiments, a lack of response, an inadequate response, or a discontinued response can be determined by objective criteria (e.g., tumor volume, or by criteria such as RECIST 1.1). In some embodiments, a lack of response, an inadequate response, or a discontinued response can be determined by the subject's physician.

As used herein, "the subject is treatment naive with respect to the cancer" means that the subject has not been previously administered one or more therapeutic agents or therapies for the cancer.

For any of the solid tumors described herein, the solid tumors can be primary tumors or metastatic (or secondary) tumors. As used herein, "primary" tumors are those located at the site where the tumor began to grow (i.e., where it originated). As used herein, "metastatic" (or "secondary") tumors are those that have spread to other parts of body from the original tumor site. In some embodiments, the metastatic or secondary tumors are the same type of cancer as the primary tumor. In some embodiments, the metastatic or secondary tumors are not genetically identical to the primary tumor.

Provided herein is a method of treating a cancer in a subject in need of such treatment, the method comprising a) detecting a KRas dysregulation (e.g., a KRas mutation (e.g., a KRas G12A mutation, a KRas G12C mutation, a KRas G12D mutation, a KRas G12R mutation, a KRas G12S mutation, or a KRas G12V mutation (e.g., a KRas G12D mutation or a KRas G12V mutation)) or amplification) in a sample from the subject (e.g., detecting a *KRAS* gene having a mutation corresponding to a mutation in KRas protein and/or detecting a KRas protein having a mutation, a *KRAS* gene copy number increase, and/or an increase in KRas mRNA or protein expression); and b) administering a therapeutically effective amount of a compound provided herein, or a pharmaceutically acceptable salt thereof. Provided herein is a method of treating a cancer in a subject in need of such treatment, the method comprising a) detecting a KRas dysregulation (e.g., a KRas mutation (e.g., a KRas G12X mutation)) or amplification) in a sample from the subject (e.g., detecting a *KRAS* gene having a mutation corresponding to a mutation in KRas protein and/or detecting a KRas protein having a mutation, a *KRAS* gene copy number increase, and/or an increase in KRas mRNA or protein expression); and b) administering a therapeutically effective amount of a compound provided herein, or a pharmaceutically acceptable salt thereof. Also provided herein is a method of treating a KRas-associated cancer (e.g., a mutant KRas-associated cancer (e.g., a KRas G12A-associated cancer, a KRas G12C-associated cancer, a KRas G12D-associated cancer, a KRas G12R-associated cancer, a KRas G12S-associated cancer, or a KRas G12V-associated cancer (e.g., a KRas G12D-associated cancer or a KRas G12V-associated cancer))) in a subject in need of such treatment, the method comprising administering to the subject a therapeutically effective amount of a compound provided herein, or a pharmaceutically acceptable salt thereof, or a pharmaceutical composition as described herein. Also provided herein is a method of treating a KRas-associated cancer (e.g., a mutant KRas-associated cancer (e.g., a KRas G12X-associated cancer)) in a subject in need of such treatment, the method comprising administering to the subject a therapeutically effective amount of a compound provided herein, or a pharmaceutically acceptable salt thereof, or a pharmaceutical composition as described herein. For example, provided herein are methods for treating a KRas-associated cancer (e.g., a mutant KRas-associated cancer (e.g., a KRas G12A-associated cancer, a KRas G12C-associated cancer, a KRas G12D-associated cancer, a KRas G12R-associated cancer, or a KRas G12V-associated cancer (e.g., a KRas G12D-associated cancer or a KRas G12V-associated cancer))) in a subject in need of such treatment, the methods comprising a) detecting a KRas dysregulation (e.g., a KRas mutation (e.g., a KRas G12A mutation, a KRas G12C mutation, a KRas G12D mutation, a KRas G12R mutation, a KRas G12S mutation or a KRas G12V mutation (e.g., a KRas G12D mutation or a KRas G12V mutation)) or amplification) in a sample from the subject (e.g., detecting a *KRAS* gene having a mutation corresponding to a mutation in KRas protein and/or detecting a KRas protein having a mutation, a *KRAS* gene copy number increase, and/or an increase in KRas mRNA or protein expression); and b) administering a therapeutically effective amount of a compound provided herein, or a pharmaceutically acceptable salt thereof. For example, provided herein are methods for treating a KRas-associated cancer (e.g., a mutant KRas-associated cancer (e.g., a KRas G12X-associated cancer)) in a subject in need of such treatment, the methods comprising a) detecting a KRas dysregulation (e.g., a KRas mutation (e.g., a KRas G12X mutation) or amplification) in a sample from the subject (e.g., detecting a *KRAS* gene having a mutation corresponding to a mutation in KRas protein and/or detecting a KRas protein having a mutation, a *KRAS* gene copy number increase, and/or an increase in KRas mRNA or protein expression); and b) administering a therapeutically effective amount of a compound provided herein, or a pharmaceutically acceptable salt thereof.

In some embodiments of any of the methods or uses described herein, the cancer (e.g., KRas-associated cancer (e.g., mutant KRas-associated cancer (e.g., a KRas G12A-associated cancer, a KRas G12C-associated cancer, a KRas G12D-associated cancer, a KRas G12R-associated cancer, a KRas G12S-associated cancer, or a KRas G12V-associated cancer (e.g., a KRas G12D-associated cancer or a KRas G12V-associated cancer)))) is breast cancer (e.g., breast invasive carcinoma, breast invasive ductal carcinoma), central or peripheral nervous system tissue cancer (e.g., brain cancer (e.g., astrocytoma, glioblastoma, glioma, oligoastrocytoma)), endocrine or neuroendocrine cancer (e.g., adrenal cancer (e.g., adrenocortical carcinoma, pheochromocytoma, paraganglioma), multiple neuroendocrine type I and type II tumors, parathyroid cancer, pituitary tumors, thyroid cancer (e.g., papillary thyroid cancer)), eye cancer (e.g., uveal cancer (e.g., uveal melanoma)), gastrointestinal cancer (e.g., anal cancer, bile duct cancer (e.g., cholangiocarcinoma), colorectal cancer (e.g., colon adenocarcinoma, rectal adenocarcinoma, mucinous adenocarcinoma, mucinous carcinoma), esophageal cancer (e.g., esophageal adenocarcinoma), gallbladder cancer, gastrointestinal carcinoid tumor, gastrointestinal stromal tumor, liver cancer (e.g., hepatocellular carcinoma, intrahepatic bile duct cancer), pancreatic cancer (e.g., pancreatic adenocarcinoma, pancreatic islet cell cancer), small intestine cancer, or stomach cancer (e.g., stomach adenocarcinoma, signet ring cell carcinoma of the stomach)), genitourinary cancer (e.g., bladder cancer (e.g., bladder urothelial carcinoma), kidney cancer (e.g., renal clear cell carcinoma, renal papillary cell carcinoma, kidney chromophobe), prostate cancer (e.g., prostate adenocarcinoma), testicular cancer (e.g., testicular germ cell tumors, seminoma), or ureter cancer), gynecologic cancer (e.g., cervical cancer (e.g., cervical squamous cell carcinoma, endocervical adenocarcinoma, mucinous carcinoma), ovarian cancer (e.g., serous ovarian cancer, ovarian serous cystadenocarcinoma), uterine cancer (e.g., uterine carcinosarcoma, uterine endometrioid carcinoma, uterine serous carcinoma, uterine papillary serous carcinoma, uterine corpus endometrial carcinoma), or vulvar cancer), head and neck cancer (e.g., ear cancer (e.g., middle ear cancer), head and neck squamous cell carcinoma, nasal cavity cancer, oral cancer, pharynx cancer (e.g., hypopharynx cancer, nasopharynx cancer, oropharyngeal cancer), hematological cancer (e.g., leukemia (e.g., chronic lymphocytic leukemia (CLL), acute lymphocytic leukemia (ALL) (e.g., Philadelphia chromosome positive ALL), acute myeloid leukemia (AML) (e.g., acute promyelocytic leukemia (APL)), chronic myeloid leukemia (CML)), lymphoma (e.g., Hodgkin lymphoma (e.g., nodular lymphocyte predominant Hodgkin lymphoma (NLPHL)), non-Hodgkin lymphoma (e.g., Burkitt lymphoma (BL), diffuse large B-cell lymphoma (DLBCL), diffuse histiocytic lymphoma (DHL), follicular lymphoma (FL), intravascular large B-cell lymphoma (IVLBCL), mantle cell lymphoma (MCL), small lymphocytic lymphoma (SLL))), or myeloma (e.g., multiple myeloma)), Li-Fraumeni tumors, mesentery cancer (e.g., omentum cancer, peritoneal cancer), pleural cancer, respiratory cancer (e.g., larynx cancer, lung cancer (e.g., lung squamous cell carcinoma, lung adenocarcinoma, mesothelioma, non-small cell lung cancer (NSCLC)), tracheal cancer), sarcoma (e.g., bone cancer (e.g., osteosarcoma, chondrosarcoma) or soft tissue sarcoma (Ewing sarcoma, leiomyosarcoma, myxofibrosarcoma, rhabdomyosarcoma)), skin cancer (e.g., melanoma), thymus cancer (e.g., thymoma), or a combination thereof.

In some embodiments, the cancer (e.g., KRas-associated cancer (e.g., mutant KRas-associated cancer)) is a hematological cancer, a soft tissue cancer, bile duct cancer, bladder cancer, brain cancer, breast cancer, cervical cancer, colon cancer, endometrial cancer, esophageal cancer, kidney cancer, liver cancer, lung cancer, mucinous carcinoma, rectal cancer, ovarian cancer, pancreatic cancer, prostate cancer, rectal cancer, skin cancer, stomach cancer, testicular cancer, thymus cancer, thyroid cancer, urothelial cancer, or uterine cancer.

In some embodiments, the cancer (e.g., KRas-associated cancer (e.g., mutant KRas-associated cancer)) is colorectal cancer (e.g., a colon cancer or a rectal cancer), endometrial cancer, lung cancer (e.g., NSCLC), ovarian cancer, or pancreatic cancer.

In some embodiments, the cancer is a colorectal cancer (e.g., a colon cancer or a rectal cancer). In some such embodiments, the colorectal cancer has a KRas dysregulation (e.g., a KRas mutation (e.g., a KRas G12X mutation)) or amplification).

In some embodiments, the cancer is an endometrial cancer. In some such embodiments, the endometrial cancer has a KRas dysregulation (e.g., a KRas mutation (e.g., a KRas G12X mutation)) or amplification).

In some embodiments, the cancer is a lung cancer (e.g., NSCLC). In some such embodiments, the lung cancer (e.g., NSCLC) has a KRas dysregulation (e.g., a KRas mutation (e.g., a KRas G12X mutation)) or amplification).

In some embodiments, the cancer is an ovarian cancer. In some such embodiments, the ovarian cancer has a KRas dysregulation (e.g., a KRas mutation (e.g., a KRas G12X mutation)) or amplification).

In some embodiments, the cancer is a pancreatic cancer. In some such embodiments, the pancreatic cancer has a KRas dysregulation (e.g., a KRas mutation (e.g., a KRas G12X mutation)) or amplification).

In some embodiments, the cancer (e.g., a KRas-associated cancer (e.g., a mutant KRas-associated cancer (e.g., a KRas G12X-associated cancer))) is a hematological cancer, bile duct cancer, brain cancer, breast cancer, cervical cancer, colon cancer, endometrial cancer, esophageal cancer, kidney cancer, liver cancer, lung cancer, mucinous carcinoma, pancreatic cancer, prostate cancer, rectal cancer, testicular cancer (e.g., seminoma), skin cancer, stomach cancer, thymus cancer, thyroid cancer, urothelial cancer, or uterine cancer.

In some embodiments, the cancer is brain cancer, colon cancer, lung cancer, pancreatic cancer, rectal cancer, testicular cancer (e.g., seminoma), or uterine cancer. In some embodiments, the cancer is a KRas G12A-associated cancer.

In some embodiments, the cancer is bladder cancer, breast cancer, cervical cancer, colon cancer, lung cancer, kidney cancer, liver cancer, or rectal cancer. In some embodiments, the cancer is a KRas G12C-associated cancer.

In some embodiments, the cancer is a hematological cancer, brain cancer, cervical cancer, colon cancer, endometrial cancer, esophageal cancer, kidney cancer, liver cancer, lung cancer, mucinous carcinoma, ovarian cancer pancreatic cancer, prostate cancer, rectal cancer, skin cancer, stomach cancer, thymus cancer, urothelial cancer, or uterine cancer. In some embodiments, the cancer is a KRas G12D-associated cancer.

In some embodiments, the cancer is a hematological cancer, bladder cancer, bile duct cancer, colon cancer, ovarian cancer, pancreatic cancer, prostate cancer, rectal cancer, skin cancer, or testicular cancer. In some embodiments, the cancer is a KRas G12R-associated cancer.

In some embodiments, the cancer is colon cancer, lung cancer, pancreatic cancer, rectal cancer, stomach cancer, testicular cancer (e.g., seminoma), or uterine cancer. In some embodiments, the cancer is a KRas G12S-associated cancer

In some embodiments, the cancer is a hematological cancer, bladder cancer, bile duct cancer, brain cancer, breast cancer, cervical cancer, colon cancer, endometrial cancer, esophageal cancer, kidney cancer, liver cancer, lung cancer, mucinous carcinoma, ovarian cancer, pancreatic cancer, rectal cancer, skin cancer, stomach cancer, testicular cancer (e.g., seminoma), thymus cancer, or uterine cancer. In some embodiments, the cancer is a G12V-associated cancer.

In some embodiments, the cancer (e.g., a KRas-associated cancer (e.g., a mutant KRas-associated cancer (e.g., a KRas G13X-associated cancer))) is a hematological cancer, a soft tissue cancer, cervical cancer, colon cancer, endometrial cancer, liver cancer, lung cancer, pancreatic cancer, rectal cancer, skin cancer, stomach cancer, or urothelial cancer. In some embodiments, the cancer (e.g., a KRas-associated cancer (e.g., a mutant KRas-associated cancer (e.g., a KRas Q61X-associated cancer)) is bladder cancer, colon cancer, lung cancer, ovarian cancer, rectal cancer, thyroid cancer, or uterine cancer. In some embodiments, the cancer (e.g., a KRas-associated cancer (e.g., a cancer associated with KRas amplification (e.g., a cancer associated with wild-type KRas amplification)) is colorectal cancer, gastric cancer, gastroesophageal cancer, head and neck squamous carcinoma, or lung cancer (e.g., NSCLC). See, e.g., the public database cBioPortal.

In some embodiments, the cancer (e.g., a KRas-associated cancer (e.g., mutant KRas-associated cancer)) is pancreatic cancer or metastatic pancreatic cancer. In some embodiments, the cancer (e.g., a KRas-associated cancer (e.g., mutant KRas-associated cancer)) is pancreatic ductal adenocarcinoma (PDAC). In some such embodiments, the pancreatic cancer is a KRas G12R-associated cancer.

In some embodiments, the cancer (e.g., a KRas-associated cancer (e.g., mutant KRas-associated cancer)) is advanced-stage lung adenocarcinoma.

In some embodiments, the cancer is a solid tumor. In some embodiments, the solid tumor has a KRas dysregulation (e.g., a KRas mutation or amplification). For example, the solid tumor has a KRas mutation selected from the group consisting of: a KRas G12A mutation, a KRas G12C mutation, a KRas G12D mutation, a KRas G12R mutation, a KRas G12S mutation, and a KRas G12V mutation. In some embodiments, the solid tumor has a KRas G12D mutation, a KRas G12R mutation, or a KRas G12V mutation. In some embodiments, the solid tumor has a KRas G12D mutation or a KRas G12V mutation. In some embodiments, the solid tumor has a KRas G12A mutation. In some embodiments, the solid tumor has a KRas G12C mutation. In some embodiments, the solid tumor has a KRas G12D mutation. In some embodiments, the solid tumor has a KRas G12R mutation. In some embodiments, the solid tumor has a KRas G12S mutation. In some embodiments, the solid tumor has a KRas G12V mutation.

Also provided herein is a method of treating a solid tumor in a subject in need thereof, the method comprising: (a) detecting a KRas dysregulation (e.g., a KRas mutation or amplification) in a sample from the subject, and (b) administering to the subject a therapeutically effective amount of a compound provided herein, or a pharmaceutically acceptable salt thereof, or a pharmaceutical composition as described herein. Also provided herein is a method of treating a solid tumor in a subject in need thereof, the method comprising: (a) detecting a KRas G12A mutation, a KRas G12C mutation, a KRas G12D mutation, a KRas G12R mutation, a KRas G12S mutation, or a KRas G12V mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a solid tumor in a subject in need thereof, the method comprising: (a) detecting a KRas G12D or a KRas G12V mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a solid tumor in a subject in need thereof, the method comprising: (a) detecting a KRas G12A mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a solid tumor in a subject in need thereof, the method comprising: (a) detecting a KRas G12C mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a solid tumor in a subject in need thereof, the method comprising: (a) detecting a KRas G12D mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a solid tumor in a subject in need thereof, the method comprising: (a) detecting a KRas G12R mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a solid tumor in a subject in need thereof, the method comprising: (a) detecting a KRas G12S mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a solid tumor in a subject in need thereof, the method comprising: (a) detecting a KRas G12V mutation in a sample from the subject, and (b) administering to the subject a therapeutically effective amount of a compound provided herein, or a pharmaceutically acceptable salt thereof, or a pharmaceutical composition as described herein.

Also provided herein is a method of treating an advanced solid tumor in a subject in need thereof, the method comprising: (a) detecting a KRas dysregulation (e.g., a KRas mutation or amplification) in a sample from the subject, and (b) administering to the subject a therapeutically effective amount of a compound provided herein, or a pharmaceutically acceptable salt thereof, or a pharmaceutical composition as described herein. Also provided herein is a method of treating an advanced solid tumor in a subject in need thereof, the method comprising: (a) detecting a KRas G12A mutation, a KRas G12C mutation, a KRas G12D mutation, a KRas G12R mutation, a KRas G12S mutation, or a KRas G12V mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating an advanced solid tumor in a subject in need thereof, the method comprising: (a) detecting a KRas G12D or a KRas G12V mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating an advanced solid tumor in a subject in need thereof, the method comprising: (a) detecting a KRas G12A mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating an advanced solid tumor in a subject in need thereof, the method comprising: (a) detecting a KRas G12C mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating an advanced solid tumor in a subject in need thereof, the method comprising: (a) detecting a KRas G12D mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating an advanced solid tumor in a subject in need thereof, the method comprising: (a) detecting a KRas G12R mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating an advanced solid tumor in a subject in need thereof, the method comprising: (a) detecting a KRas G12S mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating an advanced solid tumor in a subject in need thereof, the method comprising: (a) detecting a KRas G12V mutation in a sample from the subject, and (b) administering to the subject a therapeutically effective amount of a compound provided herein, or a pharmaceutically acceptable salt thereof, or a pharmaceutical composition as described herein.

As used herein, an "advanced solid tumor" is a solid tumor that has spread extensively to other anatomic sites and/or that is no longer responding to treatment. In some embodiments, classification of an advanced solid tumor can be made by the subject's physician.

In some embodiments, the cancer is a bladder cancer. In some embodiments, the bladder cancer has a KRas dysregulation (e.g., a KRas mutation or amplification). For example, the bladder cancer has a KRas mutation selected from the group consisting of: a KRas G12C mutation, a KRas G12D mutation, a KRas G12R mutation, a KRas G12V mutation, a KRas G13D mutation, and a KRas Q61H mutation. As another example, the bladder cancer has a KRas mutation selected from the group consisting of: a KRas G12C mutation, a KRas G12D mutation, a KRas G12R mutation, and a KRas G12V mutation. In some embodiments, the bladder cancer has a KRas G12D mutation, a KRas G12R mutation, or a KRas G12V mutation. In some embodiments, the bladder cancer has a KRas G12D mutation or a KRas G12V mutation. In some embodiments, the bladder cancer has a KRas G12C mutation. In some embodiments, the bladder cancer has a KRas G12D mutation. In some embodiments, the bladder cancer has a KRas G12R mutation. In some embodiments, the bladder cancer has a KRas G12V mutation.

Also provided herein is a method of treating a bladder cancer in a subject in need thereof, the method comprising: (a) detecting a KRas dysregulation (e.g., a KRas mutation or amplification) in a sample from the subject, and (b) administering to the subject a therapeutically effective amount of a compound provided herein, or a pharmaceutically acceptable salt thereof, or a pharmaceutical composition as described herein. Also provided herein is a method of treating a bladder cancer in a subject in need thereof, the method comprising: (a) detecting a KRas G12C mutation, a KRas G12D mutation, a KRas G12R mutation, a KRas G12V mutation, a KRas G13D mutation, or a KRas Q61H mutation in a sample from the subject, and (b) administering to the subject a therapeutically effective amount of a compound provided herein, or a pharmaceutically acceptable salt thereof, or a pharmaceutical composition as described herein. Also provided herein is a method of treating a bladder cancer in a subject in need thereof, the method comprising: (a) detecting a KRas G12C mutation, a KRas G12D mutation, a KRas G12R mutation, or a KRas G12V mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a bladder cancer in a subject in need thereof, the method comprising: (a) detecting a KRas G12D or a KRas G12V mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a bladder cancer in a subject in need thereof, the method comprising: (a) detecting a KRas G12C mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a bladder cancer in a subject in need thereof, the method comprising: (a) detecting a KRas G12D mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a bladder cancer in a subject in need thereof, the method comprising: (a) detecting a KRas G12R mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a bladder cancer in a subject in need thereof, the method comprising: (a) detecting a KRas G12V mutation in a sample from the subject, and (b) administering to the subject a therapeutically effective amount of a compound provided herein, or a pharmaceutically acceptable salt thereof, or a pharmaceutical composition as described herein.

In some embodiments, the cancer is a cervical cancer. In some embodiments, the cervical cancer has a KRas dysregulation (e.g., a KRas mutation or amplification). For example, the cervical cancer has a KRas mutation selected from the group consisting of: a KRas G12C mutation, a KRas G12D mutation, a KRas G12V mutation, and a KRas G13D mutation. As another example, the cervical cancer has a KRas mutation selected from the group consisting of: a KRas G12C mutation, a KRas G12D mutation, and a KRas G12V mutation. In some embodiments, the cervical cancer has a KRas G12D mutation or a KRas G12V mutation. In some embodiments, the cervical cancer has a KRas G12C mutation. In some embodiments, the cervical cancer has a KRas G12D mutation. In some embodiments, the cervical cancer has a KRas G12V mutation.

Also provided herein is a method of treating a cervical cancer in a subject in need thereof, the method comprising: (a) detecting a KRas dysregulation (e.g., a KRas mutation or amplification) in a sample from the subject, and (b) administering to the subject a therapeutically effective amount of a compound provided herein, or a pharmaceutically acceptable salt thereof, or a pharmaceutical composition as described herein. Also provided herein is a method of treating a cervical cancer in a subject in need thereof, the method comprising: (a) detecting a KRas G12C mutation, a KRas G12D mutation, a KRas G12V mutation, or a KRas G13D mutation in a sample from the subject, and (b) administering to the subject a therapeutically effective amount of a compound provided herein, or a pharmaceutically acceptable salt thereof, or a pharmaceutical composition as described herein. Also provided herein is a method of treating a cervical cancer in a subject in need thereof, the method comprising: (a) detecting a KRas G12C mutation, a KRas G12D mutation, or a KRas G12V mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a cervical cancer in a subject in need thereof, the method comprising: (a) detecting a KRas G12D mutation or a KRas G12V mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a cervical cancer in a subject in need thereof, the method comprising: (a) detecting a KRas G12C mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a cervical cancer in a subject in need thereof, the method comprising: (a) detecting a KRas G12D mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a cervical cancer in a subject in need thereof, the method comprising: (a) detecting a KRas G12V mutation in a sample from the subject, and (b) administering to the subject a therapeutically effective amount of a compound provided herein, or a pharmaceutically acceptable salt thereof, or a pharmaceutical composition as described herein.

In some embodiments, the cancer is a colorectal cancer. In some embodiments, the colorectal cancer has a KRas dysregulation (e.g., a KRas mutation or amplification). For example, the colorectal cancer has a KRas mutation selected from the group consisting of: a KRas G12A mutation, a KRas G12C mutation, a KRas G12D mutation, a KRas G12R mutation, a KRas G12S mutation, a KRas G12V mutation, a KRas G13C mutation, a KRas G13D mutation, a KRas G13V mutation, a KRas Q61E mutation, a KRas Q61H mutation, a KRas Q61K mutation, a KRas Q61L mutation, a KRas Q61P mutation, and a KRas Q61R mutation. As another example, the colorectal cancer has a KRas mutation selected from the group consisting of: a KRas G12A mutation, a KRas G12C mutation, a KRas G12D mutation, a KRas G12R mutation, a KRas G12S mutation, and a KRas G12V mutation. In some embodiments, the colorectal cancer has a KRas G12D mutation, a KRas G12R mutation, or a KRas G12V mutation. In some embodiments, the colorectal cancer has a KRas G12D mutation or a KRas G12V mutation. In some embodiments, the colorectal cancer has a KRas G12A mutation. In some embodiments, the colorectal cancer has a KRas G12C mutation. In some embodiments, the colorectal cancer has a KRas G12D mutation. In some embodiments, the colorectal cancer has a KRas G12R mutation. In some embodiments, the colorectal cancer has a KRas G12S mutation. In some embodiments, the colorectal cancer has a KRas G12V mutation.

Also provided herein is a method of treating a colorectal cancer in a subject in need thereof, the method comprising: (a) detecting a KRas dysregulation (e.g., a KRas mutation or amplification) in a sample from the subject, and (b) administering to the subject a therapeutically effective amount of a compound provided herein, or a pharmaceutically acceptable salt thereof, or a pharmaceutical composition as described herein. Also provided herein is a method of treating a colorectal cancer in a subject in need thereof, the method comprising: (a) detecting a KRas G12A mutation, a KRas G12C mutation, a KRas G12D mutation, a KRas G12R mutation, a KRas G12S mutation, a KRas G12V mutation, a KRas G13C mutation, a KRas G13D mutation, a KRas G13V mutation, a KRas Q61E mutation, a KRas Q61H mutation, a KRas Q61K mutation, a KRas Q61L mutation, a KRas Q61P mutation, or a KRas Q61R mutation in a sample from the subject, and (b) administering to the subject a therapeutically effective amount of a compound provided herein, or a pharmaceutically acceptable salt thereof, or a pharmaceutical composition as described herein. Also provided herein is a method of treating a colorectal cancer in a subject in need thereof, the method comprising: (a) detecting a KRas G12A mutation, a KRas G12C mutation, a KRas G12D mutation, a KRas G12R mutation, a KRas G12S mutation, or a KRas G12V mutation, in a sample from the subject, and (b) administering to the subject a therapeutically effective amount of a compound provided herein, or a pharmaceutically acceptable salt thereof, or a pharmaceutical composition as described herein. Additionally provided herein is a method of treating a colorectal cancer in a subject in need thereof, the method comprising: (a) detecting a KRas G12D mutation or a KRas G12V mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a colorectal cancer in a subject in need thereof, the method comprising: (a) detecting a KRas G12A mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a colorectal cancer in a subject in need thereof, the method comprising: (a) detecting a KRas G12C mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a colorectal cancer in a subject in need thereof, the method comprising: (a) detecting a KRas G12D mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a colorectal cancer in a subject in need thereof, the method comprising: (a) detecting a KRas G12R mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a colorectal cancer in a subject in need thereof, the method comprising: (a) detecting a KRas G12S mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a colorectal cancer in a subject in need thereof, the method comprising: (a) detecting a KRas G12V mutation in a sample from the subject, and (b) administering to the subject a therapeutically effective amount of a compound provided herein, or a pharmaceutically acceptable salt thereof, or a pharmaceutical composition as described herein.

In some embodiments, the cancer is an endometrial cancer. In some embodiments, the endometrial cancer has a KRas dysregulation (e.g., a KRas mutation or amplification). For example, the endometrial cancer has a KRas mutation selected from the group consisting of: a KRas G12A mutation, a KRas G12C mutation, a KRas G12D mutation, a KRas G12S mutation, a KRas G12V mutation, a KRas G13C mutation, a KRas G13D mutation, a KRas G13V mutation, a KRas Q61H mutation, and a KRas Q61L mutation. As another example, the endometrial cancer has a KRas mutation selected from the group consisting of: a KRas G12A mutation, a KRas G12C mutation, a KRas G12D mutation, a KRas G12S mutation, and a KRas G12V mutation. In some embodiments, the endometrial cancer has a KRas G12D mutation or a KRas G12V mutation. In some embodiments, the endometrial cancer has a KRas G12A mutation. In some embodiments, the endometrial cancer has a KRas G12C mutation. In some embodiments, the endometrial cancer has a KRas G12D mutation. In some embodiments, the endometrial cancer has a KRas G12S mutation. In some embodiments, the endometrial cancer has a KRas G12V mutation.

Also provided herein is a method of treating an endometrial cancer in a subject in need thereof, the method comprising: (a) detecting a KRas dysregulation (e.g., a KRas mutation or amplification) in a sample from the subject, and (b) administering to the subject a therapeutically effective amount of a compound provided herein, or a pharmaceutically acceptable salt thereof, or a pharmaceutical composition as described herein. Also provided herein is a method of treating an endometrial cancer in a subject in need thereof, the method comprising: (a) detecting a KRas G12A mutation, a KRas G12C mutation, a KRas G12D mutation, a KRas G12S mutation, a KRas G12V mutation, a KRas G13C mutation, a KRas G13D mutation, a KRas G13V mutation, a KRas Q61H mutation, or a KRas Q61L mutation in a sample from the subject, and (b) administering to the subject a therapeutically effective amount of a compound provided herein, or a pharmaceutically acceptable salt thereof, or a pharmaceutical composition as described herein. Also provided herein is a method of treating an endometrial cancer in a subject in need thereof, the method comprising: (a) detecting a KRas G12A mutation, a KRas G12C mutation, a KRas G12D mutation, a KRas G12S mutation, or a KRas G12V mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating an endometrial cancer in a subject in need thereof, the method comprising: (a) detecting a KRas G12D mutation or a KRas G12V mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating an endometrial cancer in a subject in need thereof, the method comprising: (a) detecting a KRas G12A mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating an endometrial cancer in a subject in need thereof, the method comprising: (a) detecting a KRas G12C mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating an endometrial cancer in a subject in need thereof, the method comprising: (a) detecting a KRas G12D mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating an endometrial cancer in a subject in need thereof, the method comprising: (a) detecting a KRas G12S mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating an endometrial cancer in a subject in need thereof, the method comprising: (a) detecting a KRas G12V mutation in a sample from the subject, and (b) administering to the subject a therapeutically effective amount of a compound provided herein, or a pharmaceutically acceptable salt thereof, or a pharmaceutical composition as described herein.

In some embodiments, the cancer is an esophageal or stomach cancer. In some embodiments, the esophageal or stomach cancer has a KRas dysregulation (e.g., a KRas mutation or amplification). For example, the esophageal or stomach cancer has a KRas mutation selected from the group consisting of: a KRas G12C mutation, a KRas G12D mutation, a KRas G12S mutation, a KRas G12V mutation, a KRas G13C mutation, a KRas G13D mutation, and a KRas Q61H mutation. As another example, the esophageal or stomach cancer has a KRas mutation selected from the group consisting of: a KRas G12C mutation, a KRas G12D mutation, a KRas G12S mutation, and a KRas G12V mutation. In some embodiments, the esophageal or stomach cancer has a KRas G12D mutation or a KRas G12V mutation. In some embodiments, the esophageal or stomach cancer has a KRas G12C mutation. In some embodiments, the esophageal or stomach cancer has a KRas G12D mutation. In some embodiments, the esophageal or stomach cancer has a KRas G12S mutation. In some embodiments, the esophageal or stomach cancer has a KRas G12V mutation.

Also provided herein is a method of treating an esophageal or stomach cancer in a subject in need thereof, the method comprising: (a) detecting a KRas dysregulation (e.g., a KRas mutation or amplification) in a sample from the subject, and (b) administering to the subject a therapeutically effective amount of a compound provided herein, or a pharmaceutically acceptable salt thereof, or a pharmaceutical composition as described herein. Also provided herein is a method of treating an esophageal or stomach cancer in a subject in need thereof, the method comprising: (a) detecting a KRas G12C mutation, a KRas G12D mutation, a KRas G12S mutation, a KRas G12V mutation, a KRas G13C mutation, a KRas G13D mutation, or a KRas Q61H mutation in a sample from the subject, and (b) administering to the subject a therapeutically effective amount of a compound provided herein, or a pharmaceutically acceptable salt thereof, or a pharmaceutical composition as described herein. Also provided herein is a method of treating an esophageal or stomach cancer in a subject in need thereof, the method comprising: (a) detecting a KRas G12C mutation, a KRas G12D mutation, a KRas G12S mutation, or a KRas G12V mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating an esophageal or stomach cancer in a subject in need thereof, the method comprising: (a) detecting a KRas G12D mutation or a KRas G12V mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating an esophageal or stomach cancer in a subject in need thereof, the method comprising: (a) detecting a KRas G12C mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating an esophageal or stomach cancer in a subject in need thereof, the method comprising: (a) detecting a KRas G12D mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating an esophageal or stomach cancer in a subject in need thereof, the method comprising: (a) detecting a KRas G12S mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating an esophageal or stomach cancer in a subject in need thereof, the method comprising: (a) detecting a KRas G12V mutation in a sample from the subject, and (b) administering to the subject a therapeutically effective amount of a compound provided herein, or a pharmaceutically acceptable salt thereof, or a pharmaceutical composition as described herein.

In some embodiments, the cancer is a leukemia. In some embodiments, the leukemia has a KRas dysregulation (e.g., a KRas mutation or amplification). For example, the leukemia has a KRas mutation selected from the group consisting of: a KRas G12A mutation, a KRas G12C mutation, a KRas G12D mutation, a KRas G12R mutation, a KRas G12S mutation, a KRas G12V mutation, a KRas G13C mutation, a KRas G13D mutation, a KRas G13V mutation, a KRas Q61E mutation, a KRas Q61H mutation, a KRas Q61K mutation, a KRas Q61L mutation, a KRas Q61P mutation, and a KRas Q61R mutation. For example, the leukemia has a KRas mutation selected from the group consisting of: a KRas G12A mutation, a KRas G12C mutation, a KRas G12D mutation, a KRas G12R mutation, a KRas G12S mutation, and a KRas G12V mutation. In some embodiments, the leukemia has a KRas G12D mutation, a KRas G12R mutation, or a KRas G12V mutation. In some embodiments, the leukemia has a KRas G12D mutation or a KRas G12V mutation. In some embodiments, the leukemia has a KRas G12A mutation. In some embodiments, the leukemia has a KRas G12C mutation. In some embodiments, the leukemia has a KRas G12D mutation. In some embodiments, the leukemia has a KRas G12R mutation. In some embodiments, the leukemia has a KRas G12S mutation. In some embodiments, the leukemia has a KRas G12V mutation.

Also provided herein is a method of treating a leukemia in a subject in need thereof, the method comprising: (a) detecting a KRas dysregulation (e.g., a KRas mutation or amplification) in a sample from the subject, and (b) administering to the subject a therapeutically effective amount of a compound provided herein, or a pharmaceutically acceptable salt thereof, or a pharmaceutical composition as described herein. Also provided herein is a method of treating a leukemia in a subject in need thereof, the method comprising: (a) detecting a KRas G12A mutation, a KRas G12C mutation, a KRas G12D mutation, a KRas G12R mutation, a KRas G12S mutation, a KRas G12V mutation, a KRas G13C mutation, a KRas G13D mutation, a KRas G13V mutation, a KRas Q61E mutation, a KRas Q61H mutation, a KRas Q61K mutation, a KRas Q61L mutation, a KRas Q61P mutation, or a KRas Q61R mutation in a sample from the subject, and (b) administering to the subject a therapeutically effective amount of a compound provided herein, or a pharmaceutically acceptable salt thereof, or a pharmaceutical composition as described herein. Also provided herein is a method of treating a leukemia in a subject in need thereof, the method comprising: (a) detecting a KRas G12A mutation, a KRas G12C mutation, a KRas G12D mutation, a KRas G12R mutation, a KRas G12S mutation, or a KRas G12V mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a leukemia in a subject in need thereof, the method comprising: (a) detecting a KRas G12D mutation or a KRas G12V mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a leukemia in a subject in need thereof, the method comprising: (a) detecting a KRas G12A mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a leukemia in a subject in need thereof, the method comprising: (a) detecting a KRas G12C mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a leukemia in a subject in need thereof, the method comprising: (a) detecting a KRas G12D mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a leukemia in a subject in need thereof, the method comprising: (a) detecting a KRas G12R mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a leukemia in a subject in need thereof, the method comprising: (a) detecting a KRas G12S mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a leukemia in a subject in need thereof, the method comprising: (a) detecting a KRas G12V mutation in a sample from the subject, and (b) administering to the subject a therapeutically effective amount of a compound provided herein, or a pharmaceutically acceptable salt thereof, or a pharmaceutical composition as described herein.

In some embodiments, the cancer is a melanoma. In some embodiments, the melanoma has a KRas dysregulation (e.g., a KRas mutation or amplification). For example, the melanoma has a KRas mutation selected from the group consisting of: a KRas G12C mutation, a KRas G12D mutation, a KRas G12R mutation, a KRas G13D mutation, a KRas G13V mutation, a KRas Q61K mutation, a KRas Q61L mutation, and a KRas Q61R mutation. As another example, the melanoma has a KRas mutation selected from the group consisting of: a KRas G12C mutation, a KRas G12D mutation, and a KRas G12R mutation. In some embodiments, the melanoma has a KRas G12D mutation or a KRas G12R mutation. In some embodiments, the melanoma has a KRas G12C mutation. In some embodiments, the melanoma has a KRas G12D mutation. In some embodiments, the melanoma has a KRas G12R mutation.

Also provided herein is a method of treating a melanoma in a subject in need thereof, the method comprising: (a) detecting a KRas dysregulation (e.g., a KRas mutation or amplification) in a sample from the subject, and (b) administering to the subject a therapeutically effective amount of a compound provided herein, or a pharmaceutically acceptable salt thereof, or a pharmaceutical composition as described herein. Also provided herein is a method of treating a melanoma in a subject in need thereof, the method comprising: (a) detecting a KRas G12C mutation, a KRas G12D mutation, a KRas G12R mutation, a KRas G13D mutation, a KRas G13V mutation, a KRas Q61K mutation, a KRas Q61L mutation, or a KRas Q61R mutation in a sample from the subject, and (b) administering to the subject a therapeutically effective amount of a compound provided herein, or a pharmaceutically acceptable salt thereof, or a pharmaceutical composition as described herein. Also provided herein is a method of treating a melanoma in a subject in need thereof, the method comprising: (a) detecting a KRas G12C mutation, a KRas G12D mutation, a KRas G12R mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a melanoma in a subject in need thereof, the method comprising: (a) detecting a KRas G12C mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a melanoma in a subject in need thereof, the method comprising: (a) detecting a KRas G12D mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a melanoma in a subject in need thereof, the method comprising: (a) detecting a KRas G12R mutation in a sample from the subject, and (b) administering to the subject a therapeutically effective amount of a compound provided herein, or a pharmaceutically acceptable salt thereof, or a pharmaceutical composition as described herein.

In some embodiments, the cancer is a lung cancer (e.g., non-small cell lung cancer). In some embodiments, the lung cancer (e.g., non-small cell lung cancer) has a KRas dysregulation (e.g., a KRas mutation or amplification). For example, the lung cancer (e.g., non-small cell lung cancer) has a KRas mutation selected from the group consisting of: a KRas G12A mutation, a KRas G12C mutation, a KRas G12D mutation, a KRas G12S mutation, a KRas G12V mutation, a KRas G13C mutation, a KRas G13D mutation, a KRas Q61H mutation, and a KRas Q61L mutation. For example, the lung cancer (e.g., non-small cell lung cancer) has a KRas mutation selected from the group consisting of: a KRas G12A mutation, a KRas G12C mutation, a KRas G12D mutation, a KRas G12S mutation, and a KRas G12V mutation. In some embodiments, the lung cancer has a KRas G12D mutation or a KRas G12V mutation. In some embodiments, the lung cancer has a KRas G12A mutation. In some embodiments, the lung cancer has a KRas G12C mutation. In some embodiments, the lung cancer has a KRas G12D mutation. In some embodiments, the lung cancer has a KRas G12S mutation. In some embodiments, the lung cancer has a KRas G12V mutation.

Also provided herein is a method of treating a lung cancer (e.g., non-small cell lung cancer) in a subject in need thereof, the method comprising: (a) detecting a KRas dysregulation (e.g., a KRas mutation or amplification) in a sample from the subject, and (b) administering to the subject a therapeutically effective amount of a compound provided herein, or a pharmaceutically acceptable salt thereof, or a pharmaceutical composition as described herein. Also provided herein is a method of treating a lung cancer (e.g., non-small cell lung cancer) in a subject in need thereof, the method comprising: (a) detecting a KRas G12A mutation, a KRas G12C mutation, a KRas G12D mutation, a KRas G12S mutation, a KRas G12V mutation, a KRas G13C mutation, a KRas G13D mutation, a KRas Q61H mutation, or a KRas Q61L mutation in a sample from the subject, and (b) administering to the subject a therapeutically effective amount of a compound provided herein, or a pharmaceutically acceptable salt thereof, or a pharmaceutical composition as described herein. Also provided herein is a method of treating a lung cancer (e.g., non-small cell lung cancer) in a subject in need thereof, the method comprising: (a) detecting a KRas G12A mutation, a KRas G12C mutation, a KRas G12D mutation, a KRas G12S mutation, or a KRas G12V mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a lung cancer in a subject in need thereof, the method comprising: (a) detecting a KRas G12D mutation or a KRas G12V mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a lung cancer in a subject in need thereof, the method comprising: (a) detecting a KRas G12A mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a lung cancer in a subject in need thereof, the method comprising: (a) detecting a KRas G12C mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a lung cancer in a subject in need thereof, the method comprising: (a) detecting a KRas G12D mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a lung cancer in a subject in need thereof, the method comprising: (a) detecting a KRas G12S mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a lung cancer in a subject in need thereof, the method comprising: (a) detecting a KRas G12V mutation in a sample from the subject, and (b) administering to the subject a therapeutically effective amount of a compound provided herein, or a pharmaceutically acceptable salt thereof, or a pharmaceutical composition as described herein.

In some embodiments, the cancer is a pancreatic cancer. In some embodiments, the pancreatic cancer has a KRas dysregulation (e.g., a KRas mutation or amplification). For example, the pancreatic cancer has a KRas mutation selected from the group consisting of: a KRas G12A mutation, a KRas G12C mutation, a KRas G12D mutation, a KRas G12R mutation, a KRas G12S mutation, a KRas G12V mutation, a KRas G13C mutation, a KRas Q61H mutation, and a KRas Q61R mutation. For example, the pancreatic cancer has a KRas mutation selected from the group consisting of: a KRas G12A mutation, a KRas G12C mutation, a KRas G12D mutation, a KRas G12R mutation, a KRas G12S mutation, and a KRas G12V mutation. In some embodiments, the pancreatic cancer has a KRas G12D mutation, a KRas G12R mutation, or a KRas G12V mutation. In some embodiments, the pancreatic cancer has a KRas G12D mutation or a KRas G12V mutation. In some embodiments, the pancreatic cancer has a KRas G12A mutation. In some embodiments, the pancreatic cancer has a KRas G12C mutation. In some embodiments, the pancreatic cancer has a KRas G12D mutation. In some embodiments, the pancreatic cancer has a KRas G12R mutation. In some embodiments, the pancreatic cancer has a KRas G12S mutation. In some embodiments, the pancreatic cancer has a KRas G12V mutation.

Also provided herein is a method of treating a pancreatic cancer in a subject in need thereof, the method comprising: (a) detecting a KRas dysregulation (e.g., a KRas mutation or amplification) in a sample from the subject, and (b) administering to the subject a therapeutically effective amount of a compound provided herein, or a pharmaceutically acceptable salt thereof, or a pharmaceutical composition as described herein. Also provided herein is a method of treating a pancreatic cancer in a subject in need thereof, the method comprising: (a) detecting a KRas G12A mutation, a KRas G12C mutation, a KRas G12D mutation, a KRas G12R mutation, a KRas G12S mutation, a KRas G12V mutation, a KRas G13C mutation, a KRas Q61H mutation, or a KRas Q61R mutation in a sample from the subject, and (b) administering to the subject a therapeutically effective amount of a compound provided herein, or a pharmaceutically acceptable salt thereof, or a pharmaceutical composition as described herein. Also provided herein is a method of treating a pancreatic cancer in a subject in need thereof, the method comprising: (a) detecting a KRas G12A mutation, a KRas G12C mutation, a KRas G12D mutation, a KRas G12R mutation, a KRas G12S mutation, or a KRas G12V mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a pancreatic cancer in a subject in need thereof, the method comprising: (a) detecting a KRas G12D mutation, a KRas G12R mutation, or a KRas G12V mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a pancreatic cancer in a subject in need thereof, the method comprising: (a) detecting a KRas G12D mutation or a KRas G12V mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a pancreatic cancer in a subject in need thereof, the method comprising: (a) detecting a KRas G12A mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a pancreatic cancer in a subject in need thereof, the method comprising: (a) detecting a KRas G12C mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a pancreatic cancer in a subject in need thereof, the method comprising: (a) detecting a KRas G12D mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a pancreatic cancer in a subject in need thereof, the method comprising: (a) detecting a KRas G12R mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a pancreatic cancer in a subject in need thereof, the method comprising: (a) detecting a KRas G12S mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a pancreatic cancer in a subject in need thereof, the method comprising: (a) detecting a KRas G12V mutation in a sample from the subject, and (b) administering to the subject a therapeutically effective amount of a compound provided herein, or a pharmaceutically acceptable salt thereof, or a pharmaceutical composition as described herein.

In some embodiments, the cancer is a testicular cancer (e.g., seminoma). In some embodiments, the testicular cancer (e.g., seminoma) has a KRas dysregulation (e.g., a KRas mutation or amplification). For example, the testicular cancer (e.g., seminoma) has a KRas mutation selected from the group consisting of: a KRas G12A mutation, a KRas G12R mutation, a KRas G12S mutation, a KRas G12V mutation, a KRas Q61L mutation, a KRas Q61P mutation, and a KRas Q61R mutation. As another example, the testicular cancer (e.g., seminoma) has a KRas mutation selected from the group consisting of: a KRas G12A mutation, a KRas G12R mutation, a KRas G12S mutation, and a KRas G12V mutation. In some embodiments, the testicular cancer (e.g., seminoma) cancer has a KRas G12R mutation or a KRas G12V mutation. In some embodiments, the testicular cancer (e.g., seminoma) cancer has a KRas G12A mutation. In some embodiments, the testicular cancer (e.g., seminoma) cancer has a KRas G12R mutation. In some embodiments, the testicular cancer (e.g., seminoma) cancer has a KRas G12S mutation. In some embodiments, the testicular cancer (e.g., seminoma) cancer has a KRas G12V mutation.

Also provided herein is a method of treating a testicular cancer (e.g., seminoma) in a subject in need thereof, the method comprising: (a) detecting a KRas dysregulation (e.g., a KRas mutation or amplification) in a sample from the subject, and (b) administering to the subject a therapeutically effective amount of a compound provided herein, or a pharmaceutically acceptable salt thereof, or a pharmaceutical composition as described herein. Also provided herein is a method of treating a testicular cancer (e.g., seminoma) in a subject in need thereof, the method comprising: (a) detecting a KRas G12A mutation, a KRas G12R mutation, a KRas G12S mutation, a KRas G12V mutation, a KRas Q61L mutation, a KRas Q61P mutation, or a KRas Q61R mutation in a sample from the subject, and (b) administering to the subject a therapeutically effective amount of a compound provided herein, or a pharmaceutically acceptable salt thereof, or a pharmaceutical composition as described herein. Also provided herein is a method of treating a testicular cancer (e.g., seminoma) in a subject in need thereof, the method comprising: (a) detecting a KRas G12A mutation, a KRas G12R mutation, a KRas G12S mutation, or a KRas G12V mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a testicular cancer (e.g., seminoma) in a subject in need thereof, the method comprising: (a) detecting a KRas G12A mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a testicular cancer (e.g., seminoma) in a subject in need thereof, the method comprising: (a) detecting a KRas G12R mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a testicular cancer (e.g., seminoma) in a subject in need thereof, the method comprising: (a) detecting a KRas G12S mutation in a sample from the subject, and (b) administering to the subject a therapeutically effective amount of a compound provided herein, or a pharmaceutically acceptable salt thereof, or a pharmaceutical composition as described herein. Additionally provided herein is a method of treating a testicular cancer (e.g., seminoma) in a subject in need thereof, the method comprising: (a) detecting a KRas G12V mutation in a sample from the subject, and (b) administering to the subject a therapeutically effective amount of a compound provided herein, or a pharmaceutically acceptable salt thereof, or a pharmaceutical composition as described herein.

Also provided herein is a method of treating a bladder cancer in a subject in need of such treatment, the method comprising administering to the subject a therapeutically effective amount of a compound provided herein, or a pharmaceutically acceptable salt thereof, or a pharmaceutical composition as described herein. In some embodiments, the method further comprises determining that the bladder cancer has a KRas mutation selected from the group consisting of: a KRas G12C mutation, a KRas G12D mutation, a KRas G12R mutation, a KRas G12V mutation, a KRas G13D mutation, and a KRas Q61H mutation. In some embodiments, the method further comprises determining that the bladder cancer has a KRas mutation selected from the group consisting of: a KRas G12C mutation, a KRas G12D mutation, a KRas G12R mutation, and a KRas G12V mutation. In some such embodiments, the cancer is a KRas G12C-associated cancer, a KRas G12D-associated cancer, a KRas G12R-associated cancer, a KRas G12V-associated cancer, a KRas G13D-associated cancer, or a KRas Q61H-associated cancer. In some such embodiments, the cancer is a KRas G12C-associated cancer, a KRas G12D-associated cancer, a KRas G12R-associated cancer, or a KRas G12V-associated cancer. In some aspects of this embodiment, the cancer is a KRas G12D-associated cancer, a KRas G12R-associated cancer, or a KRas G12V-associated cancer. In some embodiments, the cancer is a KRas G12C-associated cancer. In some embodiments, the cancer is a KRas G12D-associated cancer. In some embodiments, the cancer is a KRas G12R-associated cancer. In some embodiments, the cancer is a KRas G12V-associated cancer. In some embodiments, the compound provided herein, or a pharmaceutically acceptable salt thereof, is a KRas G12C inhibitor, a KRas G12D inhibitor, a KRas G12R inhibitor, a KRas G12V inhibitor, a KRas G13D inhibitor, a KRas Q61H inhibitor, or two or more thereof. In some embodiments, the compound provided herein, or a pharmaceutically acceptable salt thereof, is a KRas G12C inhibitor, a KRas G12D inhibitor, a KRas G12R inhibitor, a KRas G12V inhibitor, or two or more thereof. In some aspects of this embodiment, the compound provided herein, or a pharmaceutically acceptable salt thereof, is a KRas G12D inhibitor, a KRas G12R inhibitor, and/or a KRas G12V inhibitor. In some aspects of this embodiment, the compound provided herein, or a pharmaceutically acceptable salt thereof, is a KRas G12D inhibitor, a KRas G12V inhibitor, or both.

Also provided herein is a method of treating a cervical cancer in a subject in need of such treatment, the method comprising administering to the subject a therapeutically effective amount of a compound provided herein, or a pharmaceutically acceptable salt thereof, or a pharmaceutical composition as described herein. In some embodiments, the method further comprises determining that the cervical cancer has a KRas mutation selected from the group consisting of: a KRas G12C mutation, a KRas G12D mutation, a KRas G12V mutation, and a KRas G13D mutation. In some embodiments, the method further comprises determining that the cervical cancer has a KRas mutation selected from the group consisting of: a KRas G12C mutation, a KRas G12D mutation, and a KRas G12V mutation. In some embodiments, the cancer is a KRas G12C-associated cancer, a KRas G12D-associated cancer, a KRas G12V-associated cancer, or a KRas G13D-associated cancer. In some embodiments, the cancer is a KRas G12C-associated cancer, a KRas G12D-associated cancer, or a KRas G12V-associated cancer. In some embodiments, the cancer is a KRas G12D-associated cancer or a KRas G12V-associated cancer. In some embodiments, the cancer is a KRas G12C-associated cancer. In some embodiments, the cancer is a KRas G12D-associated cancer. In some embodiments, the cancer is a KRas G12V-associated cancer. In some such embodiments, the compound provided herein, or a pharmaceutically acceptable salt thereof, is a KRas G12C inhibitor, a KRas G12D inhibitor, a KRas G12V inhibitor, a KRas G13D inhibitor, or two or more thereof. In some such embodiments, the compound provided herein, or a pharmaceutically acceptable salt thereof, is a KRas G12C inhibitor, a KRas G12D inhibitor, a KRas G12V inhibitor, or two or more thereof. In some aspects of this embodiment, the compound provided herein, or a pharmaceutically acceptable salt thereof, is a KRas G12D inhibitor, a KRas G12V inhibitor, or both.

Also provided herein is a method of treating a colorectal cancer in a subject in need of such treatment, the method comprising administering to the subject a therapeutically effective amount of a compound provided herein, or a pharmaceutically acceptable salt thereof, or a pharmaceutical composition as described herein. In some embodiments, the method further comprises determining that the colorectal cancer has a KRas mutation selected from the group consisting of: a KRas G12A mutation, a KRas G12C mutation, a KRas G12D mutation, a KRas G12R mutation, a KRas G12S mutation, a KRas G12V mutation, a KRas G13C mutation, a KRas G13D mutation, a KRas G13V mutation, a KRas Q61E mutation, a KRas Q61H mutation, a KRas Q61K mutation, a KRas Q61L mutation, a KRas Q61P mutation, and a KRas Q61R mutation. In some embodiments, the method further comprises determining that the colorectal cancer has a KRas mutation selected from the group consisting of: a KRas G12A mutation, a KRas G12C mutation, a KRas G12D mutation, a KRas G12R mutation, a KRas G12S mutation, and a KRas G12V mutation. In some embodiments, the cancer is a KRas G12A-associated cancer, a KRas G12C-associated cancer, a KRas G12D-associated cancer, a KRas G12R-associated cancer, a KRas G12S-associated cancer, a KRas G12V-associated cancer, a KRas G13C-associated cancer, a KRas G13D-associated cancer, a KRas G13V-associated cancer, a KRas Q61E-associated cancer, a KRas Q61H-associated cancer, a KRas Q61K-associated cancer, a KRas Q61L-associated cancer, a KRas Q61P-associated cancer, or a KRas Q61R-associated cancer. In some embodiments, the cancer is a KRas G12A-associated cancer, a KRas G12C-associated cancer, a KRas G12D-associated cancer, a KRas G12R-associated cancer, a KRas G12S-associated cancer, or a KRas G12V-associated cancer. In some aspects of this embodiment, the cancer is a KRas G12D-associated cancer, a KRas G12R-associated cancer, or a KRas G12V-associated cancer. In some embodiments, the cancer is a KRas G12D-associated cancer or a KRas G12V-associated cancer. In some embodiments, the cancer is a KRas G12A-associated cancer. In some embodiments, the cancer is a KRas G12C-associated cancer. In some embodiments, the cancer is a KRas G12D-associated cancer. In some embodiments, the cancer is a KRas G12R-associated cancer. In some embodiments, the cancer is a KRas G12S-associated cancer. In some embodiments, the cancer is a KRas G12V-associated cancer. In some embodiments, the compound provided herein, or a pharmaceutically acceptable salt thereof, is a KRas G12A inhibitor, a KRas G12C inhibitor, a KRas G12D inhibitor, a KRas G12R inhibitor, a KRas G12S inhibitor, a KRas G12V inhibitor, a KRas G13C inhibitor, a KRas G13D inhibitor, a KRas G13V inhibitor, a KRas Q61E inhibitor, a KRas Q61H inhibitor, a KRas Q61K inhibitor, a KRas Q61L inhibitor, a KRas Q61P inhibitor, a KRas Q61R inhibitor, or two or more thereof. In some embodiments, the compound provided herein, or a pharmaceutically acceptable salt thereof, is a KRas G12A inhibitor, a KRas G12C inhibitor, a KRas G12D inhibitor, a KRas G12R inhibitor, a KRas G12S inhibitor, a KRas G12V inhibitor, or two or more thereof. In some aspects of this embodiment, the compound provided herein, or a pharmaceutically acceptable salt thereof, is a KRas G12D inhibitor, a KRas G12R inhibitor, and/or a KRas G12V inhibitor. In some aspects of this embodiment, the compound provided herein, or a pharmaceutically acceptable salt thereof, is a KRas G12D inhibitor, a KRas G12V inhibitor, or both.

Also provided herein is a method of treating an endometrial cancer in a subject in need of such treatment, the method comprising administering to the subject a therapeutically effective amount of a compound provided herein, or a pharmaceutically acceptable salt thereof, or a pharmaceutical composition as described herein. In some embodiments, the method further comprises determining that the endometrial cancer has a KRas mutation selected from the group consisting of: a KRas G12A mutation, a KRas G12C mutation, a KRas G12D mutation, a KRas G12S mutation, a KRas G12V mutation, a KRas G13C mutation, a KRas G13D mutation, a KRas G13V mutation, a KRas Q61H mutation, and a KRas Q61L mutation. In some embodiments, the method further comprises determining that the endometrial cancer has a KRas mutation selected from the group consisting of: a KRas G12A mutation, a KRas G12C mutation, a KRas G12D mutation, a KRas G12S mutation, and a KRas G12V mutation. In some embodiments, the cancer is a KRas G12A-associated cancer, a KRas G12C-associated cancer, a KRas G12D-associated cancer, a KRas G12S-associated cancer, a KRas G12V-associated cancer, a KRas G13C-associated cancer, a KRas G13D-associated cancer, a KRas G13V-associated cancer, a KRas Q61H-associated cancer, or a KRas Q61L-associated cancer. In some embodiments, the cancer is a KRas G12A-associated cancer, a KRas G12C-associated cancer, a KRas G12D-associated cancer, a KRas G12S-associated cancer, or a KRas G12V-associated cancer. In some embodiments, the cancer is a KRas G12D-associated cancer or a KRas G12V-associated cancer. In some embodiments, the cancer is a KRas G12A-associated cancer. In some embodiments, the cancer is a KRas G12C-associated cancer. In some embodiments, the cancer is a KRas G12D-associated cancer. In some embodiments, the cancer is a KRas G12S-associated cancer. In some embodiments, the cancer is a KRas G12V-associated cancer. In some such embodiments, the compound provided herein, or a pharmaceutically acceptable salt thereof, is a KRas G12A inhibitor, a KRas G12C inhibitor, a KRas G12D inhibitor, a KRas G12S inhibitor, a KRas G12V inhibitor, a KRas G13C inhibitor, a KRas G13D inhibitor, a KRas G13V inhibitor, a KRas Q61H inhibitor, a KRas Q61L inhibitor, or two or more thereof. In some such embodiments, the compound provided herein, or a pharmaceutically acceptable salt thereof, is a KRas G12A inhibitor, a KRas G12C inhibitor, a KRas G12D inhibitor, a KRas G12S inhibitor, a KRas G12V inhibitor, or two or more thereof. In some aspects of this embodiment, the compound provided herein, or a pharmaceutically acceptable salt thereof, is a KRas G12D inhibitor, a KRas G12V inhibitor, or both.

Also provided herein is a method of treating an esophageal or stomach cancer in a subject in need of such treatment, the method comprising administering to the subject a therapeutically effective amount of a compound provided herein, or a pharmaceutically acceptable salt thereof, or a pharmaceutical composition as described herein. In some embodiments, the method further comprises determining that the esophageal or stomach cancer has a KRas mutation selected from the group consisting of: a KRas G12C mutation, a KRas G12D mutation, a KRas G12S mutation, a KRas G12V mutation, a KRas G13C mutation, a KRas G13D mutation, and a KRas Q61H mutation. In some embodiments, the method further comprises determining that the esophageal or stomach cancer has a KRas mutation selected from the group consisting of: a KRas G12C mutation, a KRas G12D mutation, a KRas G12S mutation, and a KRas G12V mutation. In some embodiments, the cancer is a KRas G12C-associated cancer, a KRas G12D-associated cancer, a KRas G12S-associated cancer, a KRas G12V-associated cancer, a KRas G13C-associated cancer, a KRas G13D-associated cancer, or a KRas Q61H-associated cancer. In some embodiments, the cancer is a KRas G12C-associated cancer, a KRas G12D-associated cancer, a KRas G12S-associated cancer, or a KRas G12V-associated cancer. In some embodiments, the cancer is a KRas G12D-associated cancer or a KRas G12V-associated cancer. In some embodiments, the cancer is a KRas G12C-associated cancer. In some embodiments, the cancer is a KRas G12D-associated cancer. In some embodiments, the cancer is a KRas G12S-associated cancer. In some embodiments, the cancer is a KRas G12V-associated cancer. In some embodiments, the compound provided herein, or a pharmaceutically acceptable salt thereof, is a KRas G12C inhibitor, a KRas G12D inhibitor, a KRas G12S inhibitor, a KRas G12V inhibitor, a KRas G13C inhibitor, a KRas G13D inhibitor, a KRas Q61H inhibitor, or two or more thereof. In some embodiments, the compound provided herein, or a pharmaceutically acceptable salt thereof, is a KRas G12C inhibitor, a KRas G12D inhibitor, a KRas G12S inhibitor, a KRas G12V inhibitor, or two or more thereof. In some aspects of this embodiment, the compound provided herein, or a pharmaceutically acceptable salt thereof, is a KRas G12D inhibitor, a KRas G12V inhibitor, or both.

Also provided herein is a method of treating a leukemia in a subject in need of such treatment, the method comprising administering to the subject a therapeutically effective amount of a compound provided herein, or a pharmaceutically acceptable salt thereof, or a pharmaceutical composition as described herein. In some embodiments, the method further comprises determining that the leukemia has a KRas mutation selected from the group consisting of: a KRas G12A mutation, a KRas G12C mutation, a KRas G12D mutation, a KRas G12R mutation, a KRas G12S mutation, a KRas G12V mutation, a KRas G13C mutation, a KRas G13D mutation, a KRas G13V mutation, a KRas Q61E mutation, a KRas Q61H mutation, a KRas Q61K mutation, a KRas Q61L mutation, a KRas Q61P mutation, and a KRas Q61R mutation. In some embodiments, the method further comprises determining that the leukemia has a KRas mutation selected from the group consisting of: a KRas G12A mutation, a KRas G12C mutation, a KRas G12D mutation, a KRas G12R mutation, a KRas G12S mutation, and a KRas G12V mutation. In some embodiments, the cancer is a KRas G12A-associated cancer, a KRas G12C-associated cancer, a KRas G12D-associated cancer, a KRas G12R-associated cancer, a KRas G12S-associated cancer, a KRas G12V-associated cancer, a KRas G13C-associated cancer, a KRas G13D-associated cancer, a KRas G13V-associated cancer, a KRas Q61E-associated cancer, a KRas Q61H-associated cancer, a KRas Q61K-associated cancer, a KRas Q61L-associated cancer, a KRas Q61P-associated cancer, or a KRas Q61R-associated cancer. In some embodiments, the cancer is a KRas G12A-associated cancer, a KRas G12C-associated cancer, a KRas G12D-associated cancer, a KRas G12R-associated cancer, a KRas G12S-associated cancer, and a KRas G12V-associated cancer. In some aspects of this embodiment, the cancer is a KRas G12D-associated cancer, a KRas G12R-associated cancer, or a KRas G12V-associated cancer. In some embodiments, the cancer is a KRas G12D-associated cancer or a KRas G12V-associated cancer. In some embodiments, the cancer is a KRas G12A-associated cancer. In some embodiments, the cancer is a KRas G12C-associated cancer. In some embodiments, the cancer is a KRas G12D-associated cancer. In some embodiments, the cancer is a KRas G12R-associated cancer. In some embodiments, the cancer is a KRas G12S-associated cancer. In some embodiments, the cancer is a KRas G12V-associated cancer. In some embodiments, the compound provided herein, or a pharmaceutically acceptable salt thereof, is a KRas G12A inhibitor, a KRas G12C inhibitor, a KRas G12D inhibitor, a KRas G12R inhibitor, a KRas G12S inhibitor, a KRas G12V inhibitor, a KRas G13C inhibitor, a KRas G13D inhibitor, a KRas G13V inhibitor, a KRas Q61E inhibitor, a KRas Q61H inhibitor, a KRas Q61K inhibitor, a KRas Q61L inhibitor, a KRas Q61P inhibitor, a KRas Q61R inhibitor, or two or more thereof. In some embodiments, the compound provided herein, or a pharmaceutically acceptable salt thereof, is a KRas G12A inhibitor, a KRas G12C inhibitor, a KRas G12D inhibitor, a KRas G12R inhibitor, a KRas G12S inhibitor, a KRas G12V inhibitor, or two or more thereof. In some aspects of this embodiment, the compound provided herein, or a pharmaceutically acceptable salt thereof, is a KRas G12D inhibitor, a KRas G12R inhibitor, and/or a KRas G12V inhibitor. In some aspects of this embodiment, the compound provided herein, or a pharmaceutically acceptable salt thereof, is a KRas G12D inhibitor, a KRas G12V inhibitor, or both.

Also provided herein is a method of treating a melanoma in a subject in need of such treatment, the method comprising administering to the subject a therapeutically effective amount of a compound provided herein, or a pharmaceutically acceptable salt thereof, or a pharmaceutical composition as described herein. In some embodiments, the method further comprises determining that the melanoma has a KRas mutation selected from the group consisting of: a KRas G12C mutation, a KRas G12D mutation, a KRas G12R mutation, a KRas G13D mutation, a KRas G13V mutation, a KRas Q61K mutation, a KRas Q61L mutation, and a KRas Q61R mutation. In some embodiments, the method further comprises determining that the melanoma has a KRas mutation selected from the group consisting of: a KRas G12C mutation, a KRas G12D mutation, and a KRas G12R mutation. In some embodiments, the cancer is a KRas G12C-associated cancer, a KRas G12D-associated cancer, a KRas G12R-associated cancer, a KRas G13D-associated cancer, a KRas G13V-associated cancer, a KRas Q61K-associated cancer, a KRas Q61L-associated cancer, or a KRas Q61R-associated cancer. In some embodiments, the cancer is a KRas G12C-associated cancer, a KRas G12D-associated cancer, or a KRas G12R-associated cancer. In some embodiments, the cancer is a KRas G12D-associated cancer or a KRas G12R-associted cancer. In some embodiments, the cancer is a KRas G12C-associated cancer. In some embodiments, the cancer is a KRas G12D-associated cancer. In some embodiments, the cancer is a KRas G12R-associated cancer. In some embodiments, the compound provided herein, or a pharmaceutically acceptable salt thereof, is a KRas G12C inhibitor, a KRas G12D inhibitor, a KRas G12R inhibitor, a KRas G13D inhibitor, a KRas G13V inhibitor, a KRas Q61K inhibitor, a KRas Q61L inhibitor, a KRas Q61R inhibitor, or two or more thereof. In some embodiments, the compound provided herein, or a pharmaceutically acceptable salt thereof, is a KRas G12C inhibitor, a KRas G12D inhibitor, a KRas G12R inhibitor, or two or more thereof. In some aspects of this embodiment, the compound provided herein, or a pharmaceutically acceptable salt thereof, is a KRas G12D inhibitor, a KRas G12R inhibitor, or both.

Also provided herein is a method of treating a lung cancer (e.g., NSCLC) in a subject in need of such treatment, the method comprising administering to the subject a therapeutically effective amount of a compound provided herein, or a pharmaceutically acceptable salt thereof, or a pharmaceutical composition as described herein. In some embodiments, the method further comprises determining that the lung cancer (e.g., NSCLC) has a KRas mutation selected from the group consisting of: KRas G12A mutation, a KRas G12C mutation, a KRas G12D mutation, a KRas G12S mutation, a KRas G12V mutation, a KRas G13C-asociated cancer, a KRas G13D mutation, a KRas Q61H mutation, and a KRas Q61L mutation. In some embodiments, the method further comprises determining that the lung cancer (e.g., NSCLC) has a KRas mutation selected from the group consisting of: KRas G12A mutation, a KRas G12C mutation, a KRas G12D mutation, a KRas G12S mutation, and a KRas G12V mutation. In some embodiments, the cancer is a KRas G12A-associated cancer, a KRas G12C-associated cancer, a KRas G12D-associated cancer, a KRas G12S-associated cancer, a KRas G12V-associated cancer, a KRas G13C-asociated cancer, a KRas G13D-associated cancer, a KRas Q61H-associated cancer, or a KRas Q61L-associated cancer. In some embodiments, the cancer is a KRas G12A-associated cancer, a KRas G12C-associated cancer, a KRas G12D-associated cancer, a KRas G12S-associated cancer, or a KRas G12V-associated cancer. In some embodiments, the cancer is a KRas G12D-associated cancer or a KRas G12V-associated cancer. In some embodiments, the cancer is a KRas G12A-associated cancer. In some embodiments, the cancer is a KRas G12C-associated cancer. In some embodiments, the cancer is a KRas G12D-associated cancer. In some embodiments, the cancer is a KRas G12S-associated cancer. In some embodiments, the cancer is a KRas G12V-associated cancer. In some embodiments, the compound provided herein, or a pharmaceutically acceptable salt thereof, is a KRas G12A inhibitor, a KRas G12C inhibitor, a KRas G12D inhibitor, a KRas G12S inhibitor, a KRas G12V inhibitor, a KRas G13C-asociated cancer, a KRas G13D inhibitor, a KRas Q61H inhibitor, a KRas Q61L inhibitor, or two or more thereof. In some embodiments, the compound provided herein, or a pharmaceutically acceptable salt thereof, is a KRas G12A inhibitor, a KRas G12C inhibitor, a KRas G12D inhibitor, a KRas G12S inhibitor, a KRas G12V inhibitor, or two or more thereof. In some aspects of this embodiment, the compound provided herein, or a pharmaceutically acceptable salt thereof, a KRas G12D inhibitor, a KRas G12V inhibitor, or both.

Also provided herein is a method of treating a pancreatic cancer in a subject in need of such treatment, the method comprising administering to the subject a therapeutically effective amount of a compound provided herein, or a pharmaceutically acceptable salt thereof, or a pharmaceutical composition as described herein. In some embodiments, the method further comprises determining that the pancreatic cancer has a KRas mutation selected from the group consisting of: a KRas G12A mutation, a KRas G12C mutation, a KRas G12D mutation, a KRas G12R mutation, a KRas G12S mutation, a KRas G12V mutation, a KRas G13C-asociated cancer, and a KRas Q61H mutation. In some embodiments, the method further comprises determining that the pancreatic cancer has a KRas mutation selected from the group consisting of: a KRas G12A mutation, a KRas G12C mutation, a KRas G12D mutation, a KRas G12R mutation, a KRas G12S mutation, and a KRas G12V mutation. In some embodiments, the cancer is a KRas G12A-associated cancer, a KRas G12C-associated cancer, a KRas G12D-associated cancer, a KRas G12R-associated cancer, a KRas G12S-associated cancer, a KRas G12V-associated cancer, a KRas G13C-asociated cancer, or a KRas Q61H-associated cancer. In some embodiments, the cancer is a KRas G12A-associated cancer, a KRas G12C-associated cancer, a KRas G12D-associated cancer, a KRas G12R-associated cancer, a KRas G12S-associated cancer, or a KRas G12V-associated cancer. In some aspects of this embodiment, the cancer is a KRas G12D-associated cancer, a KRas G12R-associated cancer, or a KRas G12V-associated cancer. In some embodiments, the cancer is a KRas G12D-associated cancer or a KRas G12V-associated cancer. In some embodiments, the cancer is a KRas G12A-associated cancer. In some embodiments, the cancer is a KRas G12C-associated cancer. In some embodiments, the cancer is a KRas G12D-associated cancer. In some embodiments, the cancer is a KRas G12R-associated cancer. In some embodiments, the cancer is a KRas G12S-associated cancer. In some embodiments, the cancer is a KRas G12V-associated cancer. In some embodiments, the compound provided herein, or a pharmaceutically acceptable salt thereof, is a KRas G12A inhibitor, a KRas G12C inhibitor, a KRas G12D inhibitor, a KRas G12R inhibitor, a KRas G12S inhibitor, a KRas G12V inhibitor, a KRas G13C inhibitor, a KRas Q61H inhibitor, or two or more thereof. In some embodiments, the compound provided herein, or a pharmaceutically acceptable salt thereof, is a KRas G12A inhibitor, a KRas G12C inhibitor, a KRas G12D inhibitor, a KRas G12R inhibitor, a KRas G12S inhibitor, a KRas G12V inhibitor, or two or more thereof. In some aspects of this embodiment, the compound provided herein, or a pharmaceutically acceptable salt thereof, is a KRas G12D inhibitor, a KRas G12R inhibitor, and/or a KRas G12V inhibitor. In some aspects of this embodiment, the compound provided herein, or a pharmaceutically acceptable salt thereof, is a KRas G12D inhibitor, a KRas G12V inhibitor, or both.

Also provided herein is a method of treating a testicular cancer (e.g., seminoma) in a subject in need of such treatment, the method comprising administering to the subject a therapeutically effective amount of a compound provided herein, or a pharmaceutically acceptable salt thereof, or a pharmaceutical composition as described herein. In some embodiments, the method further comprises determining that the testicular cancer (e.g., seminoma) has a KRas mutation selected from the group consisting of: a KRas G12A mutation, a KRas G12R mutation, a KRas G12S mutation, a KRas G12V mutation, a KRas Q61L mutation, a KRas Q61P mutation, and a KRas Q61R mutation. In some embodiments, the method further comprises determining that the testicular cancer (e.g., seminoma) has a KRas mutation selected from the group consisting of: a KRas G12A mutation, a KRas G12R mutation, a KRas G12S mutation, and a KRas G12V mutation. In some embodiments, the cancer is a KRas G12A-associated cancer, a KRas G12R-associated cancer, a KRas G12S-associated cancer, a KRas G12V-associated cancer, a KRas Q61L-associated cancer, a KRas Q61P-associated cancer, or a KRas Q61R-associated cancer. In some embodiments, the cancer is a KRas G12A-associated cancer, a KRas G12R-associated cancer, a KRas G12S-associated cancer, or a KRas G12V-associated cancer. In some aspects of this embodiment, the cancer is a KRas G12R-associated cancer or a KRas G12V-associated cancer. In some embodiments, the cancer is a KRas G12A-associated cancer. In some embodiments, the cancer is a KRas G12R-associated cancer. In some embodiments, the cancer is a KRas G12S-associated cancer. In some embodiments, the cancer is a KRas G12V-associated cancer. In some embodiments, the compound provided herein, or a pharmaceutically acceptable salt thereof, is a KRas G12A inhibitor, a KRas G12R inhibitor, a KRas G12S inhibitor, a KRas G12V inhibitor, a KRas Q61L inhibitor, a KRas Q61P inhibitor, a KRas Q61R inhibitor, or two or more thereof. In some embodiments, the compound provided herein, or a pharmaceutically acceptable salt thereof, is a KRas G12A inhibitor, a KRas G12R inhibitor, a KRas G12S inhibitor, a KRas G12V inhibitor, or two or more thereof. In some aspects of this embodiment, the compound provided herein, or a pharmaceutically acceptable salt thereof, is a KRas G12R inhibitor, a KRas G12V inhibitor, or both.

In some embodiments, the KRas-associated cancer is bladder cancer, breast cancer, cervical cancer, colorectal cancer, endometrial cancer, esophageal or stomach cancer, liver cancer, skin cancer (e.g., melanoma), lung cancer (e.g., NSCLC), or pancreatic cancer. In some embodiments, the cancer has a KRas G12C mutation or a KRas G12D mutation. In some such embodiments, the compound provided herein, or a pharmaceutically acceptable salt thereof, is a KRas G12C inhibitor, a KRas G12D inhibitor, or both.

Also provided herein is a method of treating a KRas G12C-associated cancer or a KRas G12D-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is bladder cancer, breast cancer, cervical cancer, colorectal cancer, endometrial cancer, esophageal or stomach cancer, liver cancer, skin cancer (e.g., melanoma), lung cancer (e.g., NSCLC), or pancreatic cancer. In some such embodiments, the compound provided herein, or a pharmaceutically acceptable salt thereof, is a KRas G12C inhibitor, a KRas G12D inhibitor, or both.

Also provided herein is a method of treating bladder cancer, breast cancer, cervical cancer, colorectal cancer, endometrial cancer, esophageal or stomach cancer, liver cancer, skin cancer (e.g., melanoma), lung cancer (e.g., NSCLC), or pancreatic cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12C-associated cancer or a KRas G12D-associated cancer, or both. In some such embodiments, the compound provided herein, or a pharmaceutically acceptable salt thereof, is a KRas G12C inhibitor, a KRas G12D inhibitor, or both.

In some embodiments, the KRas-associated cancer is bladder cancer, breast cancer, cervical cancer, colorectal cancer, endometrial cancer, esophageal or stomach cancer, leukemia, lung cancer (e.g., NSCLC), pancreatic cancer, or kidney cancer. In some embodiments, the cancer has a KRas G12D mutation or a KRas G12V mutation. In some such embodiments, the compound provided herein, or a pharmaceutically acceptable salt thereof, is a KRas G12D inhibitor, a KRas G12V inhibitor, or both.

Also provided herein is a method of treating a KRas G12D-associated cancer or a KRas G12V-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is bladder cancer, breast cancer, cervical cancer, colorectal cancer, endometrial cancer, esophageal or stomach cancer, leukemia, lung cancer (e.g., NSCLC), pancreatic cancer, or kidney cancer. In some such embodiments, the compound provided herein, or a pharmaceutically acceptable salt thereof, is a KRas G12D inhibitor, a KRas G12V inhibitor, or both.

Also provided herein is a method of treating bladder cancer, breast cancer, cervical cancer, colorectal cancer, endometrial cancer, esophageal or stomach cancer, leukemia, lung cancer (e.g., NSCLC), pancreatic cancer, or kidney cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12D-associated cancer or a KRas G12V-associated cancer. In some such embodiments, the compound provided herein, or a pharmaceutically acceptable salt thereof, is a KRas G12D inhibitor, a KRas G12V inhibitor, or both.

In some embodiments, the KRas-associated cancer is bladder cancer, cervical cancer, colorectal cancer, endometrial cancer, esophageal or stomach cancer, liver cancer, leukemia, skin cancer (e.g., melanoma), or lung cancer (e.g., NSCLC). In some embodiments, the cancer has a KRas G12D mutation or a KRas G13D mutation. In some such embodiments, the compound provided herein, or a pharmaceutically acceptable salt thereof, is a KRas G12D inhibitor, a KRas G13D inhibitor, or both.

Also provided herein is a method of treating a KRas G12D-associated cancer or a KRas G13D-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is bladder cancer, cervical cancer, colorectal cancer, endometrial cancer, esophageal or stomach cancer, liver cancer, leukemia, skin cancer (e.g., melanoma), or lung cancer (e.g., NSCLC). In some such embodiments, the compound provided herein, or a pharmaceutically acceptable salt thereof, is a KRas G12D inhibitor, a KRas G13D inhibitor, or both.

Also provided herein is a method of treating bladder cancer, cervical cancer, colorectal cancer, endometrial cancer, esophageal or stomach cancer, liver cancer, leukemia, skin cancer (e.g., melanoma), or lung cancer (e.g., NSCLC) in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12D-associated cancer or a KRas G13D-associated cancer. In some such embodiments, the compound provided herein, or a pharmaceutically acceptable salt thereof, is a KRas G12D inhibitor, a KRas G13D inhibitor, or both.

In some embodiments, the KRas-associated cancer is bladder cancer, breast cancer, cervical cancer, colorectal cancer, endometrial cancer, esophageal or stomach cancer, lung cancer (e.g., NSCLC), or pancreatic cancer. In some embodiments, the cancer has a KRas G12C mutation or a KRas G12V mutation. In some such embodiments, the compound provided herein, or a pharmaceutically acceptable salt thereof, is a KRas G12C inhibitor, a KRas G12V inhibitor, or both.

Also provided herein is a method of treating a KRas G12C-associated cancer or a KRas G12V-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is bladder cancer, breast cancer, cervical cancer, colorectal cancer, endometrial cancer, esophageal or stomach cancer, lung cancer (e.g., NSCLC), or pancreatic cancer. In some such embodiments, the compound provided herein, or a pharmaceutically acceptable salt thereof, is a KRas G12C inhibitor, a KRas G12V inhibitor, or both.

Also provided herein is a method of treating bladder cancer, breast cancer, cervical cancer, colorectal cancer, endometrial cancer, esophageal or stomach cancer, lung cancer (e.g., NSCLC), or pancreatic cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12C-associated cancer or a KRas G12V-associated cancer. In some such embodiments, the compound provided herein, or a pharmaceutically acceptable salt thereof, is a KRas G12C inhibitor, a KRas G12V inhibitor, or both.

In some embodiments, the KRas-associated cancer is bladder cancer, cervical cancer, colorectal cancer, endometrial cancer, esophageal or stomach cancer, liver cancer, skin cancer (e.g., melanoma), or lung cancer (e.g., NSCLC). In some embodiments, the cancer has a KRas G12C mutation or a KRas G13D mutation. In some such embodiments, the compound provided herein, or a pharmaceutically acceptable salt thereof, is a KRas G12C inhibitor, a KRas G13D inhibitor, or both.

Also provided herein is a method of treating a KRas G12C-associated cancer or a KRas G13D-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is bladder cancer, cervical cancer, colorectal cancer, endometrial cancer, esophageal or stomach cancer, liver cancer, skin cancer (e.g., melanoma), or lung cancer (e.g., NSCLC). In some such embodiments, the compound provided herein, or a pharmaceutically acceptable salt thereof, is a KRas G12C inhibitor, a KRas G13D inhibitor, or both.

Also provided herein is a method of treating bladder cancer, cervical cancer, colorectal cancer, endometrial cancer, esophageal or stomach cancer, liver cancer, skin cancer (e.g., melanoma), or lung cancer (e.g., NSCLC) in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12C-associated cancer or a KRas G13D-associated cancer. In some such embodiments, the compound provided herein, or a pharmaceutically acceptable salt thereof, is a KRas G12C inhibitor, a KRas G13D inhibitor, or both.

In some embodiments, the KRas-associated cancer is bladder cancer, colorectal cancer, endometrial cancer, esophageal or stomach cancer, leukemia, lung cancer (e.g., NSCLC), or pancreatic cancer. In some embodiments, the cancer has a KRas G12D mutation or a KRas Q61H mutation. In some such embodiments, the compound provided herein, or a pharmaceutically acceptable salt thereof, is a KRas G12D inhibitor, a KRas Q61H inhibitor, or both.

Also provided herein is a method of treating a KRas G12D-associated cancer or a KRas Q61H-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is bladder cancer, colorectal cancer, endometrial cancer, esophageal or stomach cancer, leukemia, lung cancer (e.g., NSCLC), or pancreatic cancer. In some such embodiments, the compound provided herein, or a pharmaceutically acceptable salt thereof, is a KRas G12D inhibitor, a KRas Q61H inhibitor, or both.

Also provided herein is a method of treating bladder cancer, colorectal cancer, endometrial cancer, esophageal or stomach cancer, leukemia, lung cancer (e.g., NSCLC), or pancreatic cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12D-associated cancer or a KRas Q61H-associated cancer. In some such embodiments, the compound provided herein, or a pharmaceutically acceptable salt thereof, is a KRas G12D inhibitor, a KRas Q61H inhibitor, or both.

In some embodiments, the KRas-associated cancer is bladder cancer, cervical cancer, colorectal cancer, endometrial cancer, esophageal or stomach cancer, leukemia, or lung cancer (e.g., NSCLC). In some embodiments, the cancer has a KRas G12V mutation or a KRas G13D mutation. In some such embodiments, the compound provided herein, or a pharmaceutically acceptable salt thereof, is a KRas G12V inhibitor, a KRas G13D inhibitor, or both.

Also provided herein is a method of treating a KRas G12V-associated cancer or a KRas G13D-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is bladder cancer, cervical cancer, colorectal cancer, endometrial cancer, esophageal or stomach cancer, leukemia, or lung cancer (e.g., NSCLC). In some such embodiments, the compound provided herein, or a pharmaceutically acceptable salt thereof, is a KRas G12V inhibitor, a KRas G13D inhibitor, or both.

Also provided herein is a method of treating bladder cancer, cervical cancer, colorectal cancer, endometrial cancer, esophageal or stomach cancer, leukemia, or lung cancer (e.g., NSCLC) in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12V-associated cancer or a KRas G13V-associated cancer. In some such embodiments, the compound provided herein, or a pharmaceutically acceptable salt thereof, is a KRas G12V inhibitor, a KRas G13D inhibitor, or both.

In some embodiments, the KRas-associated cancer is bladder cancer, colorectal cancer, endometrial cancer, esophageal or stomach cancer, leukemia, lung cancer (e.g., NSCLC), or pancreatic cancer. In some embodiments, the cancer has a KRas G12V mutation or a KRas Q61H mutation. In some such embodiments, the compound provided herein, or a pharmaceutically acceptable salt thereof, is a KRas G12V inhibitor, a KRas Q61H inhibitor, or both.

Also provided herein is a method of treating a KRas G12V-associated cancer or a KRas Q61H-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is bladder cancer, colorectal cancer, endometrial cancer, esophageal or stomach cancer, leukemia, lung cancer (e.g., NSCLC), or pancreatic cancer. In some such embodiments, the compound provided herein, or a pharmaceutically acceptable salt thereof, is a KRas G12V inhibitor, a KRas Q61H inhibitor, or both.

Also provided herein is a method of treating bladder cancer, colorectal cancer, endometrial cancer, esophageal or stomach cancer, leukemia, lung cancer (e.g., NSCLC), or pancreatic cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12V-associated cancer or a KRas Q61H-associated cancer. In some such embodiments, the compound provided herein, or a pharmaceutically acceptable salt thereof, is a KRas G12V inhibitor, a KRas Q61H inhibitor, or both.

In some embodiments, the KRas-associated cancer is bladder cancer, colorectal cancer, endometrial cancer, esophageal or stomach cancer, lung cancer (e.g., NSCLC), or pancreatic cancer. In some embodiments, the cancer has a KRas G12C mutation or a KRas Q61H mutation. In some such embodiments, the compound provided herein, or a pharmaceutically acceptable salt thereof, is a KRas G12C inhibitor, a KRas Q61H inhibitor, or both.

Also provided herein is a method of treating a KRas G12C-associated cancer or a KRas Q61H-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is bladder cancer, colorectal cancer, endometrial cancer, esophageal or stomach cancer, lung cancer (e.g., NSCLC), or pancreatic cancer. In some such embodiments, the compound provided herein, or a pharmaceutically acceptable salt thereof, is a KRas G12C inhibitor, a KRas Q61H inhibitor, or both.

Also provided herein is a method of treating bladder cancer, colorectal cancer, endometrial cancer, esophageal or stomach cancer, lung cancer (e.g., NSCLC), or pancreatic cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12C-associated cancer or a KRas Q61H-associated cancer. In some such embodiments, the compound provided herein, or a pharmaceutically acceptable salt thereof, is a KRas G12C inhibitor, a KRas Q61H inhibitor, or both.

In some embodiments, the KRas-associated cancer is colorectal cancer, endometrial cancer, esophageal or stomach cancer, lung cancer (e.g., NSCLC), pancreatic cancer, or testicular cancer (e.g., seminoma). In some embodiments, the cancer has a KRas G12S mutation or a KRas G12V mutation. In some such embodiments, the compound provided herein, or a pharmaceutically acceptable salt thereof, is a KRas G12S inhibitor, a KRas G12V inhibitor, or both.

Also provided herein is a method of treating a KRas G12S-associated cancer or a KRas G12V-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, endometrial cancer, esophageal or stomach cancer, lung cancer (e.g., NSCLC), pancreatic cancer, or testicular cancer (e.g., seminoma). In some such embodiments, the compound provided herein, or a pharmaceutically acceptable salt thereof, is a KRas G12S inhibitor, a KRas G12V inhibitor, or both.

Also provided herein is a method of treating colorectal cancer, endometrial cancer, esophageal or stomach cancer, lung cancer (e.g., NSCLC), pancreatic cancer, or testicular cancer (e.g., seminoma) in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12S-associated cancer or a KRas G12V-associated cancer. In some such embodiments, the compound provided herein, or a pharmaceutically acceptable salt thereof, is a KRas G12S inhibitor, a KRas G12V inhibitor, or both.

In some embodiments, the KRas-associated cancer is colorectal cancer, endometrial cancer, lung cancer (e.g., NSCLC), pancreatic cancer, or testicular cancer (e.g., seminoma). In some embodiments, the cancer has a KRas G12A mutation or a KRas G12S mutation. In some such embodiments, the compound provided herein, or a pharmaceutically acceptable salt thereof, is a KRas G12A inhibitor, a KRas G12S inhibitor, or both.

Also provided herein is a method of treating a KRas G12A-associated cancer or a KRas G12S-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, endometrial cancer, lung cancer (e.g., NSCLC), pancreatic cancer, or testicular cancer (e.g., seminoma). In some such embodiments, the compound provided herein, or a pharmaceutically acceptable salt thereof, is a KRas G12A inhibitor, a KRas G12S inhibitor, or both.

Also provided herein is a method of treating colorectal cancer, endometrial cancer, lung cancer (e.g., NSCLC), pancreatic cancer, or testicular cancer (e.g., seminoma) in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12A-associated cancer or a KRas G12S-associated cancer. In some such embodiments, the compound provided herein, or a pharmaceutically acceptable salt thereof, is a KRas G12A inhibitor, a KRas G12S inhibitor, or both.

In some embodiments, the KRas-associated cancer is colorectal cancer, endometrial cancer, lung cancer (e.g., NSCLC), pancreatic cancer, or testicular cancer (e.g., seminoma). In some embodiments, the cancer has a KRas G12A mutation or a KRas G12V mutation. In some such embodiments, the compound provided herein, or a pharmaceutically acceptable salt thereof, is a KRas G12A inhibitor, a KRas G12V inhibitor, or both.

Also provided herein is a method of treating a KRas G12A-associated cancer or a KRas G12V-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, endometrial cancer, lung cancer (e.g., NSCLC), pancreatic cancer, or testicular cancer (e.g., seminoma). In some such embodiments, the compound provided herein, or a pharmaceutically acceptable salt thereof, is a KRas G12A inhibitor, a KRas G12V inhibitor, or both.

Also provided herein is a method of treating colorectal cancer, endometrial cancer, lung cancer (e.g., NSCLC), pancreatic cancer, or testicular cancer (e.g., seminoma) in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12A-associated cancer or a KRas G12V-associated cancer. In some such embodiments, the compound provided herein, or a pharmaceutically acceptable salt thereof, is a KRas G12A inhibitor, a KRas G12V inhibitor, or both.

In some embodiments, the KRas-associated cancer is colorectal cancer, endometrial cancer, esophageal or stomach cancer, lung cancer (e.g., NSCLC), or pancreatic cancer. In some embodiments, the cancer has a KRas G12C mutation or a KRas G12S mutation. In some such embodiments, the compound provided herein, or a pharmaceutically acceptable salt thereof, is a KRas G12C inhibitor, a KRas G12S inhibitor, or both.

Also provided herein is a method of treating a KRas G12C-associated cancer or a KRas G12S-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, endometrial cancer, esophageal or stomach cancer, lung cancer (e.g., NSCLC), or pancreatic cancer. In some such embodiments, the compound provided herein, or a pharmaceutically acceptable salt thereof, is a KRas G12C inhibitor, a KRas G12S inhibitor, or both.

Also provided herein is a method of treating colorectal cancer, endometrial cancer, esophageal or stomach cancer, lung cancer (e.g., NSCLC), or pancreatic cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12C-associated cancer or a KRas G12S-associated cancer. In some such embodiments, the compound provided herein, or a pharmaceutically acceptable salt thereof, is a KRas G12C inhibitor, a KRas G12S inhibitor, or both.

In some embodiments, the KRas-associated cancer is bladder cancer, colorectal cancer, skin cancer (e.g., melanoma), pancreatic cancer, or prostate cancer. In some embodiments, the cancer has a KRas G12D mutation or a KRas G12R mutation. In some such embodiments, the compound provided herein, or a pharmaceutically acceptable salt thereof, is a KRas G12D inhibitor, a KRas G12R inhibitor, or both.

Also provided herein is a method of treating a KRas G12D-associated cancer or a KRas G12R-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is bladder cancer, colorectal cancer, skin cancer (e.g., melanoma), pancreatic cancer, or prostate cancer. In some such embodiments, the compound provided herein, or a pharmaceutically acceptable salt thereof, is a KRas G12D inhibitor, a KRas G12R inhibitor, or both.

Also provided herein is a method of treating bladder cancer, colorectal cancer, skin cancer (e.g., melanoma), pancreatic cancer, or prostate cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12D-associated cancer or a KRas G12R-associated cancer. In some such embodiments, the compound provided herein, or a pharmaceutically acceptable salt thereof, is a KRas G12D inhibitor, a KRas G12R inhibitor, or both.

In some embodiments, the KRas-associated cancer is colorectal cancer, endometrial cancer, esophageal or stomach cancer, lung cancer (e.g., NSCLC), or pancreatic cancer. In some embodiments, the cancer has a KRas G12D mutation or a KRas G12S mutation. In some such embodiments, the compound provided herein, or a pharmaceutically acceptable salt thereof, is a KRas G12D inhibitor, a KRas G12S inhibitor, or both.

Also provided herein is a method of treating a KRas G12D-associated cancer or a KRas G12S-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, endometrial cancer, esophageal or stomach cancer, lung cancer (e.g., NSCLC), or pancreatic cancer. In some such embodiments, the compound provided herein, or a pharmaceutically acceptable salt thereof, is a KRas G12D inhibitor, a KRas G12S inhibitor, or both.

Also provided herein is a method of treating colorectal cancer, endometrial cancer, esophageal or stomach cancer, lung cancer (e.g., NSCLC), or pancreatic cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12D-associated cancer or a KRas G12S-associated cancer. In some such embodiments, the compound provided herein, or a pharmaceutically acceptable salt thereof, is a KRas G12D inhibitor, a KRas G12S inhibitor, or both.

In some embodiments, the KRas-associated cancer is bladder cancer, colorectal cancer, ovarian cancer, pancreatic cancer, or testicular cancer (e.g., seminoma). In some embodiments, the cancer has a KRas G12R mutation or a KRas G12V mutation. In some such embodiments, the compound provided herein, or a pharmaceutically acceptable salt thereof, is a KRas G12R inhibitor, a KRas G12V inhibitor, or both.

Also provided herein is a method of treating a KRas G12R-associated cancer or a KRas G12V-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is bladder cancer, colorectal cancer, ovarian cancer, pancreatic cancer, or testicular cancer (e.g., seminoma). In some such embodiments, the compound provided herein, or a pharmaceutically acceptable salt thereof, is a KRas G12R inhibitor, a KRas G12V inhibitor, or both.

Also provided herein is a method of treating bladder cancer, colorectal cancer, ovarian cancer, pancreatic cancer, or testicular cancer (e.g., seminoma) in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12R-associated cancer or a KRas G12V-associated cancer. In some such embodiments, the compound provided herein, or a pharmaceutically acceptable salt thereof, is a KRas G12R inhibitor, a KRas G12V inhibitor, or both.

In some embodiments, the KRas-associated cancer is colorectal cancer, endometrial cancer, esophageal or stomach cancer, lung cancer (e.g., NSCLC), or pancreatic cancer. In some embodiments, the cancer has a KRas G12S mutation or a KRas Q61H mutation. In some such embodiments, the compound provided herein, or a pharmaceutically acceptable salt thereof, is a KRas G12S inhibitor, a KRas Q61H inhibitor, or both.

Also provided herein is a method of treating a KRas G12S-associated cancer or a KRas Q61H-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, endometrial cancer, esophageal or stomach cancer, lung cancer (e.g., NSCLC), or pancreatic cancer. In some such embodiments, the compound provided herein, or a pharmaceutically acceptable salt thereof, is a KRas G12S inhibitor, a KRas Q61H inhibitor, or both.

Also provided herein is a method of treating colorectal cancer, endometrial cancer, esophageal or stomach cancer, lung cancer (e.g., NSCLC), or pancreatic cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12S-associated cancer or a KRas Q61H-associated cancer. In some such embodiments, the compound provided herein, or a pharmaceutically acceptable salt thereof, is a KRas G12S inhibitor, a KRas Q61H inhibitor, or both.

In some embodiments, the KRas-associated cancer is colorectal cancer, endometrial cancer, lung cancer (e.g., NSCLC), ovarian cancer, or testicular cancer (e.g., seminoma). In some embodiments, the cancer has a KRas G12V mutation or a KRas Q61L mutation. In some such embodiments, the compound provided herein, or a pharmaceutically acceptable salt thereof, is a KRas G12V inhibitor, a KRas Q61L inhibitor, or both.

Also provided herein is a method of treating a KRas G12V-associated cancer or a KRas Q61L-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, endometrial cancer, lung cancer (e.g., NSCLC), ovarian cancer, or testicular cancer (e.g., seminoma). In some such embodiments, the compound provided herein, or a pharmaceutically acceptable salt thereof, is a KRas G12V inhibitor, a KRas Q61L inhibitor, or both.

Also provided herein is a method of treating colorectal cancer, endometrial cancer, lung cancer (e.g., NSCLC), ovarian cancer, or testicular cancer (e.g., seminoma) in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12V-associated cancer or a KRas Q61L-associated cancer. In some such embodiments, the compound provided herein, or a pharmaceutically acceptable salt thereof, is a KRas G12V inhibitor, a KRas Q61L inhibitor, or both.

In some embodiments, the KRas-associated cancer is colorectal cancer, endometrial cancer, lung cancer (e.g., NSCLC), or pancreatic cancer. In some embodiments, the cancer has a KRas G12A mutation or a KRas G12C mutation. In some such embodiments, the compound provided herein, or a pharmaceutically acceptable salt thereof, is a KRas G12A inhibitor, a KRas G12C inhibitor, or both.

Also provided herein is a method of treating a KRas G12A-associated cancer or a KRas G12C-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, endometrial cancer, lung cancer (e.g., NSCLC), or pancreatic cancer. In some such embodiments, the compound provided herein, or a pharmaceutically acceptable salt thereof, is a KRas G12A inhibitor, a KRas G12C inhibitor, or both.

Also provided herein is a method of treating colorectal cancer, endometrial cancer, lung cancer (e.g., NSCLC), or pancreatic cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12A-associated cancer or a KRas G12C-associated cancer. In some such embodiments, the compound provided herein, or a pharmaceutically acceptable salt thereof, is a KRas G12A inhibitor, a KRas G12C inhibitor, or both.

In some embodiments, the KRas-associated cancer is colorectal cancer, endometrial cancer, lung cancer (e.g., NSCLC), or pancreatic cancer. In some embodiments, the cancer has a KRas G12A mutation or a KRas G12D mutation. In some such embodiments, the compound provided herein, or a pharmaceutically acceptable salt thereof, is a KRas G12A inhibitor, a KRas G12D inhibitor, or both.

Also provided herein is a method of treating a KRas G12A-associated cancer or a KRas G12D-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, endometrial cancer, lung cancer (e.g., NSCLC), or pancreatic cancer. In some such embodiments, the compound provided herein, or a pharmaceutically acceptable salt thereof, is a KRas G12A inhibitor, a KRas G12D inhibitor, or both.

Also provided herein is a method of treating colorectal cancer, endometrial cancer, lung cancer (e.g., NSCLC), or pancreatic cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12A-associated cancer or a KRas G12D-associated cancer. In some such embodiments, the compound provided herein, or a pharmaceutically acceptable salt thereof, is a KRas G12A inhibitor, a KRas G12D inhibitor, or both.

In some embodiments, the KRas-associated cancer is colorectal cancer, endometrial cancer, lung cancer (e.g., NSCLC), or pancreatic cancer. In some embodiments, the cancer has a KRas G12A mutation or a KRas G13C mutation. In some such embodiments, the compound provided herein, or a pharmaceutically acceptable salt thereof, is a KRas G12A inhibitor, a KRas G13C inhibitor, or both.

Also provided herein is a method of treating a KRas G12A-associated cancer or a KRas G13C-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, endometrial cancer, lung cancer (e.g., NSCLC), or pancreatic cancer. In some such embodiments, the compound provided herein, or a pharmaceutically acceptable salt thereof, is a KRas G12A inhibitor, a KRas G13C inhibitor, or both.

Also provided herein is a method of treating colorectal cancer, endometrial cancer, lung cancer (e.g., NSCLC), or pancreatic cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12A-associated cancer or a KRas G13C-associated cancer. In some such embodiments, the compound provided herein, or a pharmaceutically acceptable salt thereof, is a KRas G12A inhibitor, a KRas G13C inhibitor, or both.

In some embodiments, the KRas-associated cancer is colorectal cancer, endometrial cancer, lung cancer (e.g., NSCLC), or pancreatic cancer. In some embodiments, the cancer has a KRas G12A mutation or a KRas Q61H mutation. In some such embodiments, the compound provided herein, or a pharmaceutically acceptable salt thereof, is a KRas G12A inhibitor, a KRas Q61H inhibitor, or both.

Also provided herein is a method of treating a KRas G12A-associated cancer or a KRas Q61H-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, endometrial cancer, lung cancer (e.g., NSCLC), or pancreatic cancer. In some such embodiments, the compound provided herein, or a pharmaceutically acceptable salt thereof, is a KRas G12A inhibitor, a KRas Q61H inhibitor, or both.

Also provided herein is a method of treating colorectal cancer, endometrial cancer, lung cancer (e.g., NSCLC), or pancreatic cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12A-associated cancer or a KRas Q61H-associated cancer. In some such embodiments, the compound provided herein, or a pharmaceutically acceptable salt thereof, is a KRas G12A inhibitor, a KRas Q61H inhibitor, or both.

In some embodiments, the KRas-associated cancer is colorectal cancer, endometrial cancer, lung cancer (e.g., NSCLC), or testicular cancer (e.g., seminoma). In some embodiments, the cancer has a KRas G12A mutation or a KRas Q61L mutation. In some such embodiments, the compound provided herein, or a pharmaceutically acceptable salt thereof, is a KRas G12A inhibitor, a KRas Q61L inhibitor, or both.

Also provided herein is a method of treating a KRas G12A-associated cancer or a KRas Q61L-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, endometrial cancer, lung cancer (e.g., NSCLC), or testicular cancer (e.g., seminoma). In some such embodiments, the compound provided herein, or a pharmaceutically acceptable salt thereof, is a KRas G12A inhibitor, a KRas Q61L inhibitor, or both.

Also provided herein is a method of treating colorectal cancer, endometrial cancer, lung cancer (e.g., NSCLC), or testicular cancer (e.g., seminoma) in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12A-associated cancer or a KRas Q61L-associated cancer. In some such embodiments, the compound provided herein, or a pharmaceutically acceptable salt thereof, is a KRas G12A inhibitor, a KRas Q61L inhibitor, or both.

In some embodiments, the KRas-associated cancer is bladder cancer, colorectal cancer, skin cancer (e.g., melanoma), or pancreatic cancer. In some embodiments, the cancer has a KRas G12C mutation or a KRas G12R mutation. In some such embodiments, the compound provided herein, or pharmaceutically acceptable salt thereof, is a KRas G12C inhibitor, a KRas G12R inhibitor, or both.

Also provided herein is a method of treating a KRas G12C-associated cancer or a KRas G12R-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is bladder cancer, colorectal cancer, skin cancer (e.g., melanoma), or pancreatic cancer. In some such embodiments, the compound provided herein, or a pharmaceutically acceptable salt thereof, is a KRas G12C inhibitor, a KRas G12R inhibitor, or both.

Also provided herein is a method of treating bladder cancer, colorectal cancer, skin cancer (e.g., melanoma), or pancreatic cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12C-associated cancer or a KRas G12R-associated cancer. In some such embodiments, the compound provided herein, or a pharmaceutically acceptable salt thereof, is a KRas G12C inhibitor, a KRas G12R inhibitor, or both.

In some embodiments, the KRas-associated cancer is colorectal cancer, endometrial cancer, lung cancer (e.g., NSCLC), or pancreatic cancer. In some embodiments, the cancer has a KRas G12C mutation or a KRas G13C mutation. In some such embodiments, the compound provided herein, or a pharmaceutically acceptable salt thereof, is a KRas G12C inhibitor, a KRas G13C inhibitor, or both.

Also provided herein is a method of treating a KRas G12C-associated cancer or a KRas G13C-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, endometrial cancer, lung cancer (e.g., NSCLC), or pancreatic cancer. In some such embodiments, the compound provided herein, or a pharmaceutically acceptable salt thereof, is a KRas G12C inhibitor, a KRas G13C inhibitor, or both.

Also provided herein is a method of treating colorectal cancer, endometrial cancer, lung cancer (e.g., NSCLC), or pancreatic cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12C-associated cancer or a KRas G13C-associated cancer. In some such embodiments, the compound provided herein, or a pharmaceutically acceptable salt thereof, is a KRas G12C inhibitor, a KRas G13C inhibitor, or both.

In some embodiments, the KRas-associated cancer is colorectal cancer, endometrial cancer, skin cancer (e.g., melanoma), or lung cancer (e.g., NSCLC). In some embodiments, the cancer has a KRas G12C mutation or a KRas Q61L mutation. In some such embodiments, the compound provided herein, or a pharmaceutically acceptable salt thereof, is a KRas G12C inhibitor, a KRas Q61L inhibitor, or both.

Also provided herein is a method of treating a KRas G12C-associated cancer or a KRas Q61L-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, endometrial cancer, skin cancer (e.g., melanoma), or lung cancer (e.g., NSCLC). In some such embodiments, the compound provided herein, or a pharmaceutically acceptable salt thereof, is a KRas G12C inhibitor, a KRas Q61L inhibitor, or both.

Also provided herein is a method of treating colorectal cancer, endometrial cancer, skin cancer (e.g., melanoma), or lung cancer (e.g., NSCLC) in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12C-associated cancer or a KRas Q61L-associated cancer. In some such embodiments, the compound provided herein, or a pharmaceutically acceptable salt thereof, is a KRas G12C inhibitor, a KRas Q61L inhibitor, or both.

In some embodiments, the KRas-associated cancer is colorectal cancer, endometrial cancer, lung cancer (e.g., NSCLC), or pancreatic cancer. In some embodiments, the cancer has a KRas G12D mutation or a KRas G13C mutation. In some such embodiments, the compound provided herein, or a pharmaceutically acceptable salt thereof, is a KRas G12D inhibitor, a KRas G13C inhibitor, or both.

Also provided herein is a method of treating a KRas G12D-associated cancer or a KRas G13C-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, endometrial cancer, lung cancer (e.g., NSCLC), or pancreatic cancer. In some such embodiments, the compound provided herein, or a pharmaceutically acceptable salt thereof, is a KRas G12D inhibitor, a KRas G13C inhibitor, or both.

Also provided herein is a method of treating colorectal cancer, endometrial cancer, lung cancer (e.g., NSCLC), or pancreatic cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12D-associated cancer or a KRas G13C-associated cancer. In some such embodiments, the compound provided herein, or a pharmaceutically acceptable salt thereof, is a KRas G12D inhibitor, a KRas G13C inhibitor, or both.

In some embodiments, the KRas-associated cancer is colorectal cancer, endometrial cancer, skin cancer (e.g., melanoma), or lung cancer (e.g., NSCLC). In some embodiments, the cancer has a KRas G12D mutation or a KRas Q61L mutation. In some such embodiments, the compound provided herein, or a pharmaceutically acceptable salt thereof, is a KRas G12D inhibitor, a KRas Q61L inhibitor, or both.

Also provided herein is a method of treating a KRas G12D-associated cancer or a KRas Q61L-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, endometrial cancer, skin cancer (e.g., melanoma), or lung cancer (e.g., NSCLC). In some such embodiments, the compound provided herein, or a pharmaceutically acceptable salt thereof, is a KRas G12D inhibitor, a KRas Q61L inhibitor, or both.

Also provided herein is a method of treating colorectal cancer, endometrial cancer, skin cancer (e.g., melanoma), or lung cancer (e.g., NSCLC) in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12D-associated cancer or a KRas Q61L-associated cancer. In some such embodiments, the compound provided herein, or a pharmaceutically acceptable salt thereof, is a KRas G12D inhibitor, a KRas Q61L inhibitor, or both.

In some embodiments, the KRas-associated cancer is colorectal cancer, skin cancer (e.g., melanoma), ovarian cancer, or testicular cancer (e.g., seminoma). In some embodiments, the cancer has a KRas G12R mutation or a KRas Q61L mutation. In some such embodiments, the compound provided herein, or a pharmaceutically acceptable salt thereof, is a KRas G12R inhibitor, a KRas Q61L inhibitor, or both.

Also provided herein is a method of treating a KRas G12R-associated cancer or a KRas Q61L-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, skin cancer (e.g., melanoma), ovarian cancer, or testicular cancer (e.g., seminoma). In some such embodiments, the compound provided herein, or a pharmaceutically acceptable salt thereof, is a KRas G12R inhibitor, a KRas Q61L inhibitor, or both.

Also provided herein is a method of treating colorectal cancer, skin cancer (e.g., melanoma), ovarian cancer, or testicular cancer (e.g., seminoma) in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12R-associated cancer or a KRas Q61L-associated cancer. In some such embodiments, the compound provided herein, or a pharmaceutically acceptable salt thereof, is a KRas G12R inhibitor, a KRas Q61L inhibitor, or both.

In some embodiments, the KRas-associated cancer is colorectal cancer, skin cancer (e.g., melanoma), pancreatic cancer, or testicular cancer (e.g., seminoma). In some embodiments, the cancer has a KRas G12R mutation or a KRas Q61R mutation. In some such embodiments, the compound provided herein, or a pharmaceutically acceptable salt thereof, is a KRas G12R inhibitor, a KRas Q61R inhibitor, or both.

Also provided herein is a method of treating a KRas G12R-associated cancer or a KRas Q61R-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, skin cancer (e.g., melanoma), pancreatic cancer, or testicular cancer (e.g., seminoma). In some such embodiments, the compound provided herein, or a pharmaceutically acceptable salt thereof, is a KRas G12R inhibitor, a KRas Q61R inhibitor, or both.

Also provided herein is a method of treating colorectal cancer, skin cancer (e.g., melanoma), pancreatic cancer, or testicular cancer (e.g., seminoma) in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12R-associated cancer or a KRas Q61R-associated cancer. In some such embodiments, the compound provided herein, or a pharmaceutically acceptable salt thereof, is a KRas G12R inhibitor, a KRas Q61R inhibitor, or both.

In some embodiments, the KRas-associated cancer is colorectal cancer, endometrial cancer, lung cancer (e.g., NSCLC), or pancreatic cancer. In some embodiments, the cancer has a KRas G12S mutation or a KRas G13C mutation. In some such embodiments, the compound provided herein, or a pharmaceutically acceptable salt thereof, is a KRas G12S inhibitor, a KRas G13C inhibitor, or both.

Also provided herein is a method of treating a KRas G12S-associated cancer or a KRas G13C-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, endometrial cancer, lung cancer (e.g., NSCLC), or pancreatic cancer. In some such embodiments, the compound provided herein, or a pharmaceutically acceptable salt thereof, is a KRas G12S inhibitor, a KRas G13C inhibitor, or both.

Also provided herein is a method of treating colorectal cancer, endometrial cancer, lung cancer (e.g., NSCLC), or pancreatic cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12S-associated cancer or a KRas G13C-associated cancer. In some such embodiments, the compound provided herein, or a pharmaceutically acceptable salt thereof, is a KRas G12S inhibitor, a KRas G13C inhibitor, or both.

In some embodiments, the KRas-associated cancer is colorectal cancer, endometrial cancer, esophageal or stomach cancer, or lung cancer (e.g., NSCLC). In some embodiments, the cancer has a KRas G12S mutation or a KRas G13D mutation. In some such embodiments, the compound provided herein, or a pharmaceutically acceptable salt thereof, is a KRas G12S inhibitor, a KRas G13D inhibitor, or both.

Also provided herein is a method of treating a KRas G12S-associated cancer or a KRas G13D-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, endometrial cancer, esophageal or stomach cancer, or lung cancer (e.g., NSCLC). In some such embodiments, the compound provided herein, or a pharmaceutically acceptable salt thereof, is a KRas G12S inhibitor, a KRas G13D inhibitor, or both.

Also provided herein is a method of treating colorectal cancer, endometrial cancer, esophageal or stomach cancer, or lung cancer (e.g., NSCLC) in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12S-associated cancer or a KRas G13D-associated cancer. In some such embodiments, the compound provided herein, or a pharmaceutically acceptable salt thereof, is a KRas G12S inhibitor, a KRas G13D inhibitor, or both.

In some embodiments, the KRas-associated cancer is colorectal cancer, endometrial cancer, lung cancer (e.g., NSCLC), or testicular cancer (e.g., seminoma). In some embodiments, the cancer has a KRas G12S mutation or a KRas Q61L mutation. In some such embodiments, a compound provided herein, or a pharmaceutically acceptable salt thereof, is a KRas G12S inhibitor, a KRas Q61L inhibitor, or both.

Also provided herein is a method of treating a KRas G12S-associated cancer or a KRas Q61L-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, endometrial cancer, lung cancer (e.g., NSCLC), or testicular cancer (e.g., seminoma). In some such embodiments, the compound provided herein, or a pharmaceutically acceptable salt thereof, is a KRas G12S inhibitor, a KRas Q61L inhibitor, or both.

Also provided herein is a method of treating colorectal cancer, endometrial cancer, lung cancer (e.g., NSCLC), or testicular cancer (e.g., seminoma) in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12S-associated cancer or a KRas Q61L-associated cancer. In some such embodiments, the compound provided herein, or a pharmaceutically acceptable salt thereof, is a KRas G12S inhibitor, a KRas Q61L inhibitor, or both.

In some embodiments, the KRas-associated cancer is colorectal cancer, endometrial cancer, lung cancer (e.g., NSCLC), or pancreatic cancer. In some embodiments, the cancer has a KRas G12V mutation or a KRas G13C mutation. In some such embodiments, the compound provided herein, or a pharmaceutically acceptable salt thereof, is a KRas G12V inhibitor, a KRas G13C inhibitor, or both.

Also provided herein is a method of treating a KRas G12V-associated cancer or a KRas G13C-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, endometrial cancer, lung cancer (e.g., NSCLC), or pancreatic cancer. In some such embodiments, the compound provided herein, or a pharmaceutically acceptable salt thereof, is a KRas G12V inhibitor, a KRas G13C inhibitor, or both.

Also provided herein is a method of treating colorectal cancer, endometrial cancer, lung cancer (e.g., NSCLC), or pancreatic cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12V-associated cancer or a KRas G13C-associated cancer. In some such embodiments, the compound provided herein, or a pharmaceutically acceptable salt thereof, is a KRas G12V inhibitor, a KRas G13C inhibitor, or both.

In some embodiments, the KRas-associated cancer is colorectal cancer, pancreatic cancer, testicular cancer (e.g., seminoma), or thyroid cancer. In some embodiments, the cancer has a KRas G12V mutation or a KRas Q61R mutation. In some such embodiments, the compound provided herein, or a pharmaceutically acceptable salt thereof, is a KRas G12V inhibitor, a KRas Q61R inhibitor, or both.

Also provided herein is a method of treating a KRas G12V-associated cancer or a KRas Q61R-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, pancreatic cancer, testicular cancer (e.g., seminoma), or thyroid cancer. In some such embodiments, the compound provided herein, or a pharmaceutically acceptable salt thereof, is a KRas G12V inhibitor, a KRas Q61R inhibitor, or both.

Also provided herein is a method of treating colorectal cancer, pancreatic cancer, testicular cancer (e.g., seminoma), or thyroid cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12V-associated cancer or a KRas Q61R-associated cancer. In some such embodiments, the compound provided herein, or a pharmaceutically acceptable salt thereof, is a KRas G12V inhibitor, a KRas Q61R inhibitor, or both.

In some embodiments, the KRas-associated cancer is colorectal cancer, pancreatic cancer, or testicular cancer (e.g., seminoma). In some embodiments, the cancer has a KRas G12A mutation or a KRas G12R mutation. In some such embodiments, the compound provided herein, or a pharmaceutically acceptable salt thereof, is a KRas G12A inhibitor, a KRas G12R inhibitor, or both.

Also provided herein is a method of treating a KRas G12A-associated cancer or a KRas G12R-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, pancreatic cancer, or testicular cancer (e.g., seminoma). In some such embodiments, the compound provided herein, or a pharmaceutically acceptable salt thereof, is a KRas G12A inhibitor, a KRas G12R inhibitor, or both.

Also provided herein is a method of treating colorectal cancer, pancreatic cancer, or testicular cancer (e.g., seminoma) in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12A-associated cancer or a KRas G12R-associated cancer. In some such embodiments, the compound provided herein, or a pharmaceutically acceptable salt thereof, is a KRas G12A inhibitor, a KRas G12R inhibitor, or both.

In some embodiments, the KRas-associated cancer is colorectal cancer, endometrial cancer, or lung cancer (e.g., NSCLC). In some embodiments, the cancer has a KRas G12A mutation or a KRas G13D mutation. In some such embodiments, the compound provided herein, or a pharmaceutically acceptable salt thereof, is a KRas G12A inhibitor, a KRas G13D inhibitor, or both.

Also provided herein is a method of treating a KRas G12A-associated cancer or a KRas G13D-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, endometrial cancer, or lung cancer (e.g., NSCLC). In some such embodiments, the compound provided herein, or a pharmaceutically acceptable salt thereof, is a KRas G12A inhibitor, a KRas G13D inhibitor, or both.

Also provided herein is a method of treating colorectal cancer, endometrial cancer, or lung cancer (e.g., NSCLC) in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12A-associated cancer or a KRas G13D-associated cancer. In some such embodiments, the compound provided herein, or a pharmaceutically acceptable salt thereof, is a KRas G12A inhibitor, a KRas G13D inhibitor, or both.

In some embodiments, the KRas-associated cancer is colorectal cancer, pancreatic cancer, or testicular cancer (e.g., seminoma). In some embodiments, the cancer has a KRas G12A mutation or a KRas Q61R mutation. In some such embodiments, the compound provided herein, or a pharmaceutically acceptable salt thereof, is a KRas G12A inhibitor, a KRas Q61R inhibitor, or both.

Also provided herein is a method of treating a KRas G12A-associated cancer or a KRas Q61R-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, pancreatic cancer, or testicular cancer (e.g., seminoma). In some such embodiments, the compound provided herein, or a pharmaceutically acceptable salt thereof, is a KRas G12A inhibitor, a KRas Q61R inhibitor, or both.

Also provided herein is a method of treating colorectal cancer, pancreatic cancer, or testicular cancer (e.g., seminoma) in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12A-associated cancer or a KRas Q61R-associated cancer. In some such embodiments, the compound provided herein, or a pharmaceutically acceptable salt thereof, is a KRas G12A inhibitor, a KRas Q61R inhibitor, or both.

In some embodiments, the KRas-associated cancer is colorectal cancer, skin cancer (e.g., melanoma), or pancreatic cancer. In some embodiments, the cancer has a KRas G12C mutation or a KRas Q61R mutation. In some such embodiments, the compound provided herein, or a pharmaceutically acceptable salt thereof, is a KRas G12C inhibitor, a KRas Q61R inhibitor, or both.

Also provided herein is a method of treating a KRas G12C-associated cancer or a KRas Q61R-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, skin cancer (e.g., melanoma), or pancreatic cancer. In some such embodiments, the compound provided herein, or a pharmaceutically acceptable salt thereof, is a KRas G12C inhibitor, a KRas Q61R inhibitor, or both.

Also provided herein is a method of treating colorectal cancer, skin cancer (e.g., melanoma), or pancreatic cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12C-associated cancer or a KRas Q61R-associated cancer. In some such embodiments, the compound provided herein, or a pharmaceutically acceptable salt thereof, is a KRas G12C inhibitor, a KRas Q61R inhibitor, or both.

In some embodiments, the KRas-associated cancer is colorectal cancer, skin cancer (e.g., melanoma), or pancreatic cancer. In some embodiments, the cancer has a KRas G12D mutation or a KRas Q61R mutation. In some such embodiments, the compound provided herein, or a pharmaceutically acceptable salt thereof, is a KRas G12D inhibitor, a KRas Q61R inhibitor, or both.

Also provided herein is a method of treating a KRas G12D-associated cancer or a KRas Q61R-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, skin cancer (e.g., melanoma), or pancreatic cancer. In some such embodiments, the compound provided herein, or a pharmaceutically acceptable salt thereof, is a KRas G12D inhibitor, a KRas Q61R inhibitor, or both.

Also provided herein is a method of treating colorectal cancer, skin cancer (e.g., melanoma), or pancreatic cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12D-associated cancer or a KRas Q61R-associated cancer. In some such embodiments, the compound provided herein, or a pharmaceutically acceptable salt thereof, is a KRas G12D inhibitor, a KRas Q61R inhibitor, or both.

In some embodiments, the KRas-associated cancer is colorectal cancer, pancreatic cancer, or testicular cancer (e.g., seminoma). In some embodiments, the cancer has a KRas G12R mutation or a KRas G12S mutation. In some such embodiments, the compound provided herein, or a pharmaceutically acceptable salt thereof, is a KRas G12R inhibitor, a KRas G12S inhibitor, or both.

Also provided herein is a method of treating a KRas G12R-associated cancer or a KRas G12S-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, pancreatic cancer, or testicular cancer (e.g., seminoma). In some such embodiments, the compound provided herein, or a pharmaceutically acceptable salt thereof, is a KRas G12R inhibitor, a KRas G12S inhibitor, or both.

Also provided herein is a method of treating colorectal cancer, pancreatic cancer, or testicular cancer (e.g., seminoma) in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12R-associated cancer or a KRas G12S-associated cancer. In some such embodiments, the compound provided herein, or a pharmaceutically acceptable salt thereof, is a KRas G12R inhibitor, a KRas G12S inhibitor, or both.

In some embodiments, the KRas-associated cancer is bladder cancer, colorectal cancer, or skin cancer (e.g., melanoma). In some embodiments, the cancer has a KRas G12R mutation or a KRas G13D mutation. In some such embodiments, the compound provided herein, or a pharmaceutically acceptable salt thereof, is a KRas G12R inhibitor, a KRas G13D inhibitor, or both.

Also provided herein is a method of treating a KRas G12R-associated cancer or a KRas G13D-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is bladder cancer, colorectal cancer, or skin cancer (e.g., melanoma). In some such embodiments, the compound provided herein, or a pharmaceutically acceptable salt thereof, is a KRas G12R inhibitor, a KRas G13D inhibitor, or both.

Also provided herein is a method of treating bladder cancer, colorectal cancer, or skin cancer (e.g., melanoma) in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12R-associated cancer or a KRas G13D-associated cancer. In some such embodiments, the compound provided herein, or a pharmaceutically acceptable salt thereof, is a KRas G12R inhibitor, a KRas G13D inhibitor, or both.

In some embodiments, the KRas-associated cancer is bladder cancer, colorectal cancer, or pancreatic cancer. In some embodiments, the cancer has a KRas G12R mutation or a KRas Q61H mutation. In some such embodiments, the compound provided herein, or a pharmaceutically acceptable salt thereof, is a KRas G12R inhibitor, a KRas Q61H inhibitor, or both.

Also provided herein is a method of treating a KRas G12R-associated cancer or a KRas Q61H-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is bladder cancer, colorectal cancer, or pancreatic cancer. In some such embodiments, the compound provided herein, or a pharmaceutically acceptable salt thereof, is a KRas G12R inhibitor, a KRas Q61H inhibitor, or both.

Also provided herein is a method of treating bladder cancer, colorectal cancer, or pancreatic cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12R-associated cancer or a KRas Q61H-associated cancer. In some such embodiments, the compound provided herein, or a pharmaceutically acceptable salt thereof, is a KRas G12R inhibitor, a KRas Q61H inhibitor, or both.

In some embodiments, the KRas-associated cancer is bile duct cancer (e.g., cholangiocarcinoma), colorectal cancer, or skin cancer (e.g., melanoma). In some embodiments, the cancer has a KRas G12R mutation or a KRas Q61K mutation. In some such embodiments, the compound provided herein, or a pharmaceutically acceptable salt thereof, is a KRas G12R inhibitor, a KRas Q61K inhibitor, or both.

Also provided herein is a method of treating a KRas G12R-associated cancer or a KRas Q61K-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is bile duct cancer (e.g., cholangiocarcinoma), colorectal cancer, or skin cancer (e.g., melanoma). In some such embodiments, the compound provided herein, or a pharmaceutically acceptable salt thereof, is a KRas G12R inhibitor, a KRas Q61K inhibitor, or both.

Also provided herein is a method of treating bile duct cancer (e.g., cholangiocarcinoma), colorectal cancer, or skin cancer (e.g., melanoma) in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12R-associated cancer or a KRas Q61K-associated cancer. In some such embodiments, the compound provided herein, or a pharmaceutically acceptable salt thereof, is a KRas G12R inhibitor, a KRas Q61K inhibitor, or both.

In some embodiments, the KRas-associated cancer is colorectal cancer, pancreatic cancer, or testicular cancer (e.g., seminoma). In some embodiments, the cancer has a KRas G12S mutation or a KRas Q61R mutation. In some such embodiments, the compound provided herein, or a pharmaceutically acceptable salt thereof, is a KRas G12S inhibitor, a KRas Q61R inhibitor, or both.

Also provided herein is a method of treating a KRas G12S-associated cancer or a KRas Q61R-associated cancer in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is colorectal cancer, pancreatic cancer, or testicular cancer (e.g., seminoma). In some such embodiments, the compound provided herein, or a pharmaceutically acceptable salt thereof, is a KRas G12S inhibitor, a KRas Q61R inhibitor, or both.

Also provided herein is a method of treating colorectal cancer, pancreatic cancer, or testicular cancer (e.g., seminoma) in a subject in need of such treatment, the method comprising administering to the subject a therapeutically effective amount of a compound provided herein, or a pharmaceutically acceptable salt thereof, or a pharmaceutical composition as described herein. In some such embodiments, the cancer is a KRas G12S-associated cancer or a KRas Q61R-associated cancer. In some such embodiments, the compound provided herein, or a pharmaceutically acceptable salt thereof, is a KRas G12S inhibitor, a KRas Q61R inhibitor, or both.

Also provided herein is a method for treating a subject diagnosed with or identified as having a KRas-associated cancer, e.g., any of the exemplary mutant KRas-associated cancers disclosed herein, comprising administering to the subject a therapeutically effective amount of a compound provided herein or a pharmaceutically acceptable salt thereof, or a pharmaceutical composition thereof, as defined herein. Also provided herein is a method for treating a subject diagnosed with or identified as having a cancer with a KRas dysregulation (e.g., a KRas mutation or amplification) comprising administering to the subject a therapeutically effective amount of a compound provided herein or a pharmaceutically acceptable salt thereof, or a pharmaceutical composition thereof, as defined herein.

Accordingly, provided herein are methods for treating a subject diagnosed with (or identified as having) a KRas-associated cancer (e.g., a mutant KRas-associated cancer (e.g., a KRas G12D-associated cancer, a KRas G12R-associated cancer, or a KRas G12V-associated cancer (e.g., a KRas G12D-associated cancer or a KRas G12V-associated cancer))) that include administering to the subject a therapeutically effective amount of a compound provided herein, or a pharmaceutically acceptable salt thereof. In some embodiments, the subject that has been identified or diagnosed as having a KRas-associated cancer (e.g., a mutant KRas-associated cancer (e.g., a KRas G12D-associated cancer, a KRas G12R-associated cancer, or a KRas G12V-associated cancer (e.g., a KRas G12D-associated cancer or a KRas G12V-associated cancer))) through the use of a regulatory agency-approved, e.g., FDA-approved test or assay for identifying a dysregulation associated with KRas (e.g., a *KRAS* gene having a mutation corresponding to a mutation in a KRas protein and/or a KRas protein having a mutation, a *KRAS* gene copy number increase, and/or an increase in KRas mRNA or protein expression), in a subject or a biopsy sample from the subject or by performing any of the non-limiting examples of assays described herein. Accordingly, provided herein are methods for treating a subject diagnosed with (or identified as having) a cancer having a KRas dysregulation (e.g., a KRas mutation or amplification) that include administering to the subject a therapeutically effective amount of a compound provided herein, or a pharmaceutically acceptable salt thereof. In some embodiments, the subject that has been identified or diagnosed as having a cancer with a KRas dysregulation (e.g., a KRas mutation or amplification) through the use of a regulatory agency-approved, e.g., FDA-approved test or assay for identifying a dysregulation associated with KRas (e.g., a *KRAS* gene having a mutation corresponding to a mutation in a KRas protein and/or a KRas protein having a mutation, a *KRAS* gene copy number increase, and/or an increase in KRas mRNA or protein expression), in a subject or a biopsy sample from the subject or by performing any of the non-limiting examples of assays described herein. In some embodiments, the subject that has been identified or diagnosed as having a KRas-associated cancer through the use of a regulatory agency-approved, e.g., FDA-approved test or assay for identifying a KRas dysregulation (e.g., KRas mutation) in a subject or a biopsy sample from the subject or by performing any of the non-limiting examples of assays described herein. In some embodiments, the test or assay is provided as a kit.

Also provided are methods for treating cancer in a subject in need thereof, the methods comprising: (a) detecting a KRas-associated cancer (e.g., a mutant KRas-associated cancer (e.g., a KRas G12D-associated cancer, a KRas G12R-associated cancer, or a KRas G12V-associated cancer (e.g., a KRas G12D-associated cancer or a KRas G12V-associated cancer))) in the subject; and (b) administering to the subject a therapeutically effective amount of a compound provided herein, or a pharmaceutically acceptable salt thereof, or a pharmaceutical composition thereof. Also provided are methods for treating cancer in a subject in need thereof, the method comprising: (a) detecting a cancer having a KRas dysregulation (e.g., a KRas mutation or amplification) in the subject; and (b) administering to the subject a therapeutically effective amount of a compound provided herein, or a pharmaceutically acceptable salt thereof, or a pharmaceutical composition thereof. Some embodiments of these methods further include administering to the subject another anticancer agent (e.g., a second compound provided herein, or a pharmaceutically acceptable salt thereof, an immunotherapy, or any of the other anticancer agents described herein). In some embodiments, the subject was previously treated with another anticancer treatment, e.g., chemotherapy or a kinase inhibitor (e.g., an EGFR inhibitor), at least partial resection of the tumor, radiation therapy, or a combination thereof. In some embodiments, the cancer in the subject is determined to have a KRas dysregulation (e.g., a KRas mutation (e.g., a KRas G12A mutation, a KRas G12C mutation, a KRas G12D mutation, a KRas G12R mutation, a KRas G12S mutation, or a KRas G12V mutation (e.g., a KRas G12D mutation or a KRas G12V mutation))) through the use of a regulatory agency-approved, e.g., FDA-approved test or assay for identifying a KRas mutation (e.g., a *KRAS* gene having a mutation corresponding to a mutation in a KRas protein and/or a KRas protein having a mutation, a *KRAS* gene copy number increase, and/or an increase in KRas mRNA or protein expression), in a subject or a sample (e.g., a tumor sample or blood sample) from the subject or by performing any of the non-limiting examples of assays described herein. In some embodiments, the subject that has been identified or diagnosed as having a cancer with a KRas dysregulation (e.g., a KRas mutation (e.g., a KRas G12D mutation, a KRas G12R mutation, or a KRas G12V mutation (e.g., a KRas G12D mutation or a KRas G12V mutation))) through the use of a regulatory agency-approved, e.g., FDA-approved test or assay for identifying a KRas dysregulation in a subject or a biopsy sample from the subject or by performing any of the non-limiting examples of assays described herein. In some embodiments, the test or assay is provided as a kit.

Also provided are methods of treating a subject that include performing an assay on a sample (e.g., a tumor sample or a blood sample) obtained from the subject to determine whether the cancer in the subject has a KRas dysregulation (e.g., a KRas mutation (e.g., a KRas G12A mutation, a KRas G12C mutation, a KRas G12D mutation, a KRas G12R mutation, a KRas G12S mutation, or a KRas G12V mutation (e.g., a KRas G12D mutation or a KRas G12V mutation))), and administering (e.g., specifically or selectively administering) a therapeutically effective amount of a compound provided herein, or a pharmaceutically acceptable salt thereof, or a pharmaceutical composition thereof, to the subject determined a cancer having a KRas dysregulation (e.g., a KRas mutation (e.g., a KRas G12A mutation, a KRas G12C mutation, a KRas G12D mutation, a KRas G12R mutation, a KRas G12S mutation, or a KRas G12V mutation (e.g., a KRas G12D mutation or a KRas G12V mutation))). In some embodiments, provided are methods of treating a subject that include performing an assay on a sample (e.g., a tumor sample or a blood sample) obtained from the subject to determine whether the cancer in the subject has a KRas dysregulation (e.g., a *KRAS* gene having a mutation corresponding to a mutation in a KRas protein and/or a KRas protein having a mutation, a *KRAS* gene copy number increase, and/or an increase in KRas mRNA or protein expression), and administering (e.g., specifically or selectively administering) a therapeutically effective amount of a compound provided herein, or a pharmaceutically acceptable salt thereof, or a pharmaceutical composition thereof, to the subject having a cancer determined to have a KRas dysregulation. Some embodiments of these methods further include administering to the subject another anticancer agent (e.g., a second compound provided herein, or a pharmaceutically acceptable salt thereof, chemotherapy, or immunotherapy). In some embodiments of these methods, the subject was previously treated with another anticancer treatment, e.g., a first KRas inhibitor, chemotherapy, a kinase inhibitor (e.g., an EGFR inhibitor), at least partial resection of a tumor, radiation therapy, or a combination thereof. In some embodiments, the subject is a subject suspected of having a KRas-associated cancer (e.g., a mutant KRas-associated cancer (e.g., a KRas G12A-associated cancer, a KRas G12C-associated cancer, a KRas G12D-associated cancer, a KRas G12R-associated cancer, a KRas G12S-assoicated cancer, or a KRas G12V-associated cancer (e.g., a KRas G12D-associated cancer or a KRas G12V-associated cancer))), a subject presenting with one or more symptoms of a KRas-associated cancer (e.g., a mutant KRas-associated cancer (e.g., a KRas G12A-associated cancer, a KRas G12C-associated cancer, a KRas G12D-associated cancer, a KRas G12R-associated cancer, a KRas G12S-associated cancer, or a KRas G12V-associated cancer (e.g., a KRas G12D-associated cancer or a KRas G12V-associated cancer))), or a subject having an elevated risk of developing a KRas-associated cancer (e.g., a mutant KRas-associated cancer (e.g., a KRas G12A-associated cancer, a KRas G12C-associated cancer, a KRas G12D-associated cancer, a KRas G12R-associated cancer, a KRas G12S-associated or a KRas G12V-associated cancer (e.g., a KRas G12D-associated cancer or a KRas G12V-associated cancer))). In some embodiments, the assay utilizes next generation sequencing, pyrosequencing, or immunohistochemistry. In some embodiments, the assay is a regulatory agency-approved assay, e.g., FDA-approved kit. In some embodiments, the assay is a liquid biopsy. Additional, non-limiting assays that may be used in these methods are described herein. Additional assays are also known in the art. Also provided is a compound provided herein, or a pharmaceutically acceptable salt thereof, or a pharmaceutical composition thereof, for use in treating a KRas-associated cancer (e.g., a mutant KRas-associated cancer (e.g., a KRas G12A-associated cancer, a KRas G12C-associated cancer, a KRas G12D-associated cancer, a KRas G12R-associated cancer, a KRas G12S-associated cancer, or a KRas G12V-associated cancer (e.g., a KRas G12D-associated cancer or a KRas G12V-associated cancer))) in a subject identified or diagnosed as having a KRas-associated cancer (e.g., a mutant KRas-associated cancer (e.g., a KRas G12A-associated cancer, a KRas G12C-associated cancer, a KRas G12D-associated cancer, a KRas G12R-associated cancer, a KRas G12S-associated cancer or a KRas G12V-associated cancer (e.g., a KRas G12D-associated cancer or a KRas G12V-associated cancer))) through a step of performing an assay (e.g., an in vitro assay) on a sample (e.g., a tumor sample or a blood sample) obtained from the subject to determine whether the cancer in the subject has a KRas dysregulation, where the presence of a KRas dysregulation (e.g., a *KRAS* gene having a mutation corresponding to a mutation in a KRas protein and/or a KRas protein having a mutation, a *KRAS* gene copy number increase, and/or an increase in KRas mRNA or protein expression), identifies that the cancer in the subject has a KRas-associated cancer (e.g., a mutant KRas-associated cancer (e.g., a KRas G12D-associated cancer, a KRas G12R-associated cancer, or a KRas G12V-associated cancer (e.g., a KRas G12D-associated cancer or a KRas G12V-associated cancer))). Also provided is a compound provided herein, or a pharmaceutically acceptable salt thereof, or a pharmaceutical composition thereof, for use in treating a cancer having a KRas dysregulation (e.g., a KRas mutation or amplification) in a subject identified or diagnosed as having a cancer having a KRas dysregulation (e.g., a KRas mutation or amplification) through a step of performing an assay (e.g., an in vitro assay) on a sample (e.g., a tumor sample or a blood sample) obtained from the subject to determine whether the cancer in the subject has a KRas dysregulation, where the presence of a KRas dysregulation (e.g., a *KRAS* gene having a mutation corresponding to a mutation in a KRas protein and/or a KRas protein having a mutation, a *KRAS* gene copy number increase, and/or an increase in KRas mRNA or protein expression), identifies that the cancer in the subject has a KRas dysregulation.

In some cases, the level (e.g., the protein expression level, the mRNA expression level, and/or the ctDNA level) of a biomarker of response can be determined following administration of a compound provided herein, or a pharmaceutically acceptable salt thereof, or a pharmaceutical composition comprising a compound provided herein, or a pharmaceutically acceptable salt thereof. For example, following administration of one or more doses (e.g., one dose, two doses, three doses, four doses, five doses, or more) of a compound provided herein, or a pharmaceutically acceptable salt thereof, or a pharmaceutical composition comprising a compound provided herein, or a pharmaceutically acceptable salt thereof, to a subject, a tumor sample (e.g., a biopsy) or a blood sample (e.g., a sample containing circulating tumor DNA (ctDNA), circulating cell-free tumor RNA (cfRNA), and/or circulating tumor cells (CTCs)) can be obtained from the subject, and an assay can be performed to determine the level (e.g., the protein expression level, the mRNA expression level, and/or the ctDNA level) of a biomarker of response. Any appropriate biomarker of response can be used, for instance, a mutant KRas protein (e.g., a KRas G12A mutant protein, a KRas G12C mutant protein, a KRas G12D mutant protein, a KRas G12R mutant protein, a KRas G12S mutant protein, or a KRas G12V mutant protein (e.g., a KRas G12D mutant protein or a KRas G12V mutant protein)), ERK1 and/or ERK2 (e.g., phosphoERK1 and/or phosphoERK2), DUSP6 (dual specificity protein phosphatase 6), and/or SPRY4 (protein sprouty homolog 4). See, e.g., Riely, Gregory J., et al. Journal of Thoracic Oncology 16.4 (2021): S751-S752, doi: 10.1016/S1556-0864(21)01941-9; and Hallin, Jill, et al. Molecular Cancer Research 21.5_Supplement (2023): B012-B012, doi: 10.1158/1557-3125.RAS23-B012. Determining the level (e.g., the protein expression level, the mRNA expression level, and/or the ctDNA level) of a biomarker of response can be performed using any appropriate method, including consulting the subject's medical record (i.e., if a level (e.g., a baseline level) of the biomarker of response was previously determined), and/or performing an assay, such as an immunohistochemical (IHC) assay, an immunofluorescence assay, a PCR assay (e.g., RT-qPCR assay or a digital droplet PCR assay), and/or a sequencing assay (e.g., a next-generation sequencing (NGS) assay). In some embodiments, the biomarker of response is a mutant KRas protein (e.g., a KRas G12D mutant protein, a KRas G12R mutant protein, or a KRas G12V mutant protein (e.g., a KRas G12D mutant protein or a KRas G12V mutant protein)), and the assay is an IHC assay, a PCR assay (e.g., RT-qPCR assay or a digital droplet PCR assay), or a sequencing assay (e.g., a next-generation sequencing assay). In some embodiments, the biomarker of response is ERK1 and/or ERK2 (e.g., phosphoERK1 and/or phosphoERK2), and the assay is an IHC assay or an immunofluorescence assay. In some embodiments, the biomarker of response is DUSP6, and the assay is a PCR assay (e.g., RT-qPCR assay or a digital droplet PCR assay). In some embodiments, the biomarker of response is SPRY4, and the assay is a PCR assay (e.g., RT-qPCR assay or a digital droplet PCR assay). See, e.g., Holm, Matilda, et al. PLoS One 15.11 (2020): e0239819, doi: 10.1371/journal.pone.0239819; Li, Jun, et al. Oncotarget 7.3 (2016): 2646, doi: 10.18632/oncotarget.6104; Van Herpen, Carla ML, et al. Oncotarget 10.19 (2019): 1850, doi: 10.18632/oncotarget.26753; Raez, L., et al. Journal of Thoracic Oncology 13.9 (2018): S153-S154, doi: 10.1016/j.jtho.2018.07.024; and Thatikonda, Venu, et al. bioRxiv (2023), doi: 10.1101/2023.01.23.525210.

Accordingly, in some embodiments of the methods provided herein, the method includes (a) administering a therapeutically effective amount of a compound provided herein, or a pharmaceutically acceptable salt thereof, or a pharmaceutical composition comprising a compound provided herein, or a pharmaceutically acceptable salt thereof, to a subject; and (b) determining the level (e.g., the protein expression level, the mRNA expression level, and/or the ctDNA level) of a biomarker of response (e.g., a mutant KRas protein (e.g., a KRas G12A mutant protein, a KRas G12C mutant protein, a KRas G12D mutant protein, a KRas G12R mutant protein, a KRas G12S mutant protein, or a KRas G12V mutant protein (e.g., a KRas G12D mutant protein or a KRas G12V mutant protein)), ERK1 and/or ERK2 (e.g., phosphoERK1 and/or phosphoERK2), DUSP6, and/or SPRY4). In some embodiments, determining the level (e.g., the protein expression level, the mRNA expression level, and/or the ctDNA level) of a biomarker of response includes performing an assay on a sample (e.g., a tumor sample or a blood sample) obtained from the subject. In some embodiments, prior to administering a therapeutically effective amount of a compound provided herein, or a pharmaceutically acceptable salt thereof, or a pharmaceutical composition comprising a compound provided herein, or a pharmaceutically acceptable salt thereof, to a subject, the level (e.g., the protein expression level, the mRNA expression level, and/or the ctDNA level) of a biomarker of response (e.g., a mutant KRas protein (e.g., a KRas G12A mutant protein, a KRas G12C mutant protein, a KRas G12D mutant protein, a KRas G12R mutant protein, a KRas G12S mutant protein, or a KRas G12V mutant protein (e.g., a KRas G12D mutant protein or a KRas G12V mutant protein)), ERK1 and/or ERK2 (e.g., phosphoERK1 and/or phosphoERK2), DUSP6, and/or SPRY4) is determined (e.g., by performing an assay or by consulting the subject's medical record); in some cases, this can be referred to as a baseline level. Thus, in some embodiments of the methods provided herein, the method includes (a) administering a therapeutically effective amount of a compound provided herein, or a pharmaceutically acceptable salt thereof, or a pharmaceutical composition comprising a compound provided herein, or a pharmaceutically acceptable salt thereof, to a subject; (b) after (a) determining the level (e.g., the protein expression level, the mRNA expression level, and/or the ctDNA level) of a biomarker of response (e.g., a mutant KRas protein (e.g., a KRas G12A mutant protein, a KRas G12C mutant protein, a KRas G12D mutant protein, a KRas G12R mutant protein, a KRas G12S mutant protein, or a KRas G12V mutant protein (e.g., a KRas G12D mutant protein or a KRas G12V mutant protein)), ERK1 and/or ERK2 (e.g., phosphoERK1 and/or phosphoERK2), DUSP6, and/or SPRY4; and (c) comparing the level of the biomarker(s) of response to a baseline level of the biomarker(s) of response. In some embodiments of the methods provided herein, the method includes (a) determining a first (e.g., baseline) level (e.g., the protein expression level, the mRNA expression level, and/or the ctDNA level) of a biomarker of response (e.g., a mutant KRas protein (e.g., a KRas G12A mutant protein, a KRas G12C mutant protein, a KRas G12D mutant protein, a KRas G12R mutant protein, a KRas G12S mutant protein, or a KRas G12V mutant protein (e.g., a KRas G12D mutant protein or a KRas G12V mutant protein)), ERK1 and/or ERK2 (e.g., phosphoERK1 and/or phosphoERK2), DUSP6, and/or SPRY4; (b) after (a), administering a therapeutically effective amount of a compound provided herein, or a pharmaceutically acceptable salt thereof, or a pharmaceutical composition comprising a compound provided herein, or a pharmaceutically acceptable salt thereof, to a subject; (c) after (b), determining a second level (e.g., the protein expression level, the mRNA expression level, and/or the ctDNA level) of the biomarker of response (e.g., a mutant KRas protein (e.g., a KRas G12A mutant protein, a KRas G12C mutant protein, a KRas G12D mutant protein, a KRas G12R mutant protein, a KRas G12S mutant protein or a KRas G12V mutant protein (e.g., a KRas G12D mutant protein or a KRas G12V mutant protein)), ERK1 and/or ERK2 (e.g., phosphoERK1 and/or phosphoERK2), DUSP6, and/or SPRY4; and (d) comparing the second level of the biomarker(s) of response to the first level of the biomarker(s) of response. In some such embodiments, step (a) is performed before the subject has received any doses of the compound provided herein, or a pharmaceutically acceptable salt thereof, or a pharmaceutical composition comprising a compound provided herein, or a pharmaceutically acceptable salt thereof. In some embodiments, the method includes (e) after (c), administering a therapeutically effective amount of a compound provided herein, or a pharmaceutically acceptable salt thereof, or a pharmaceutical composition comprising a compound provided herein, or a pharmaceutically acceptable salt thereof, to the subject; (f) after (e), determining a third level (e.g., the protein expression level, the mRNA expression level, and/or the ctDNA level) of the biomarker of response (e.g., a mutant KRas protein (e.g., a KRas G12A mutant protein, a KRas G12C mutant protein, a KRas G12D mutant protein, a KRas G12R mutant protein, a KRas G12S mutant protein, or a KRas G12V mutant protein (e.g., a KRas G12D mutant protein or a KRas G12V mutant protein)), ERK1 and/or ERK2 (e.g., phosphoERK1 and/or phosphoERK2), DUSP6, and/or SPRY4; and (g) comparing the third level of the biomarker(s) of response to a previous level of the biomarker(s) of response (e.g., the first level of the biomarker(s) of response and/or the second level of the biomarker(s) of response). In some embodiments, steps (e) through (g) are repeated one or more times (e.g., two or more times, three or more times, four or more times, five or more times, or more). In some embodiments, administering a therapeutically effective amount of a compound provided herein, or a pharmaceutically acceptable salt thereof, or a pharmaceutical composition comprising a compound provided herein, or a pharmaceutically acceptable salt thereof, to a subject comprises administration of one or more doses (e.g., one dose, two doses, three doses, four doses, five doses, or more) of a compound provided herein, or a pharmaceutically acceptable salt thereof, or a pharmaceutical composition comprising a compound provided herein, or a pharmaceutically acceptable salt thereof to the subject.

Also provided is the use of a compound provided herein, or a pharmaceutically acceptable salt thereof, for the manufacture of a medicament for treating a KRas-associated cancer (e.g., a mutant KRas-associated cancer (e.g., a KRas G12A-associated cancer, a KRas G12C-associated cancer, a KRas G12D-associated cancer, a KRas G12R-associated cancer, a KRas G12S-associated cancer, or a KRas G12V-associated cancer (e.g., a KRas G12D-associated cancer or a KRas G12V-associated cancer))) in a subject identified or diagnosed as having a KRas-associated cancer (e.g., a mutant KRas-associated cancer (e.g., a KRas G12A-associated cancer, a KRas G12C-associated cancer, a KRas G12D-associated cancer, a KRas G12R-associated cancer, a KRas G12S-associated cancer or a KRas G12V-associated cancer (e.g., a KRas G12D-associated cancer or a KRas G12V-associated cancer))) through a step of performing an assay on a sample obtained from the subject to determine whether the cancer in the subject has a KRas dysregulation (e.g., a *KRAS* gene having a mutation corresponding to a mutation in a KRas protein and/or a KRas protein having a mutation, a *KRAS* gene copy number increase, and/or an increase in KRas mRNA or protein expression) where the presence of a KRas dysregulation (e.g., a *KRAS* gene having a mutation corresponding to a mutation in a KRas protein and/or a KRas protein having a mutation, a *KRAS* gene copy number increase, and/or an increase in KRas mRNA or protein expression), identifies that the subject has a KRas-associated cancer (e.g., a mutant KRas-associated cancer (e.g., a KRas G12A-associated cancer, a KRas G12C-associated cancer, a KRas G12D-associated cancer, a KRas G12R-associated cancer, a KRas G12S-associated cancer, or a KRas G12V-associated cancer (e.g., a KRas G12D-associated cancer or a KRas G12V-associated cancer))). Also provided is the use of a compound provided herein, or a pharmaceutically acceptable salt thereof, for the manufacture of a medicament for treating a cancer having a KRas dysregulation (e.g., a KRas mutation or amplification) in a subject identified or diagnosed as having a cancer having a KRas dysregulation (e.g., a KRas mutation or amplification) through a step of performing an assay on a sample obtained from the subject to determine whether the cancer in the subject has a KRas dysregulation (e.g., a *KRAS* gene having a mutation corresponding to a mutation in a KRas protein and/or a KRas protein having a mutation, a *KRAS* gene copy number increase, and/or an increase in KRas mRNA or protein expression) where the presence of a KRas dysregulation (e.g., a *KRAS* gene having a mutation corresponding to a mutation in a KRas protein and/or a KRas protein having a mutation, a *KRAS* gene copy number increase, and/or an increase in KRas mRNA or protein expression), identifies that the subject has a cancer having a KRas dysregulation.

Some embodiments of any of the methods or uses described herein further include recording in the subject's clinical record (e.g., a computer readable medium) that the subject is determined to have a KRas dysregulation (e.g., a *KRAS* gene having a mutation corresponding to a mutation in a KRas protein and/or a KRas protein having a mutation, a *KRAS* gene copy number increase, and/or an increase in KRas mRNA or protein expression), through the performance of the assay, should be administered a compound provided herein, or a pharmaceutically acceptable salt thereof, or a pharmaceutical composition thereof. In some embodiments, the assay utilizes next generation sequencing, pyrosequencing, or immunohistochemistry. In some embodiments, the assay is a regulatory agency-approved assay, e.g., FDA-approved kit. In some embodiments, the assay is a liquid biopsy.

Also provided is a compound provided herein, or a pharmaceutically acceptable salt thereof, for use in the treatment of a cancer in a subject in need thereof, or a subject identified or diagnosed as having a KRas-associated cancer (e.g., a mutant KRas-associated cancer (e.g., a KRas G12A-associated cancer, a KRas G12C-associated cancer, a KRas G12D-associated cancer, a KRas G12R-associated cancer, a KRas G12S-associated cancer, or a KRas G12V-associated cancer (e.g., a KRas G12D-associated cancer or a KRas G12V-associated cancer))) (e.g., any of the KRas-associated cancers described herein). Also provided is a compound provided herein, or a pharmaceutically acceptable salt thereof, for use in the treatment of a cancer in a subject in need thereof, or a subject identified or diagnosed as having a cancer having a KRas dysregulation (e.g., a KRas mutation or amplification). Also provided is the use of a compound provided herein, or a pharmaceutically acceptable salt thereof, for the manufacture of a medicament for treating a cancer in a subject identified or diagnosed as having a KRas-associated cancer (e.g., a mutant KRas-associated cancer (e.g., a KRas G12A-associated cancer, a KRas G12C-associated cancer, a KRas G12D-associated cancer, a KRas G12R-associated cancer, a KRas G12S-associated cancer, or a KRas G12V-associated cancer (e.g., a KRas G12D-associated cancer or a KRas G12V-associated cancer))) (e.g., any of the KRas-associated cancers described herein). Also provided is the use of a compound provided herein, or a pharmaceutically acceptable salt thereof, for the manufacture of a medicament for treating a cancer in a subject identified or diagnosed as having a cancer having a KRas dysregulation (e.g., a KRas mutation or amplification) In some embodiments, a mutant KRas-associated cancer is a cancer that was previously identified as having no KRas mutation (e.g., KRas wild-type), for example, in a cancer that was previously identified as having no KRas mutation and then, later, a KRas mutation (e.g., a resistance mutation) was identified. In some embodiments, a subject is identified or diagnosed as having a KRas-associated cancer (e.g., a mutant KRas-associated cancer (e.g., a KRas G12A-associated cancer, a KRas G12C-associated cancer, a KRas G12D-associated cancer, a KRas G12R-associated cancer, a KRas G12S-associated cancer, or a KRas G12V-associated cancer (e.g., a KRas G12D-associated cancer or a KRas G12V-associated cancer))) through the use of a regulatory agency-approved, e.g., FDA-approved, kit for identifying a KRas dysregulation, in a subject or a biopsy sample from the subject. In some embodiments, a subject is identified or diagnosed as having a cancer having a KRas dysregulation (e.g., a KRas mutation or amplification) through the use of a regulatory agency-approved, e.g., FDA-approved, kit for identifying a KRas dysregulation, in a subject or a biopsy sample from the subject. As provided herein, a KRas-associated cancer (e.g., a mutant KRas-associated cancer (e.g., a KRas G12A-associated cancer, a KRas G12C-associated cancer, a KRas G12D-associated cancer, a KRas G12R-associated cancer, a KRas G12S-associated cancer, or a KRas G12V-associated cancer (e.g., a KRas G12D-associated cancer or a KRas G12V-associated cancer))) includes those described herein and known in the art.

In some embodiments of any of the methods or uses described herein, the subject has been identified or diagnosed as having a cancer with a KRas dysregulation (e.g., a KRas mutation (e.g., a KRas G12A mutation, a KRas G12C mutation, a KRas G12D mutation, a KRas G12R mutation, a KRas G12S mutation, or a KRas G12V mutation (e.g., a KRas G12D mutation or a KRas G12V mutation)) or amplification). In some embodiments of any of the methods or uses described herein, the subject has a cancer (e.g., a tumor sample) that is positive for a KRas dysregulation (e.g., a KRas mutation (e.g., a KRas G12A mutation, a KRas G12C mutation, a KRas G12D mutation, a KRas G12R mutation, a KRas G12S mutation or a KRas G12V mutation (e.g., a KRas G12D mutation or a KRas G12V mutation)) or amplification). In some embodiments of any of the methods or uses described herein, the subject can be a subject with a cancer (e.g., one or more tumor samples) that is positive for a KRas dysregulation (e.g., KRas mutation (e.g., a KRas G12A mutation, a KRas G12C mutation, a KRas G12D mutation, a KRas G12R mutation, a KRas G12S mutation, or a KRas G12V mutation (e.g., a KRas G12D mutation or a KRas G12V mutation))). In some embodiments of any of the methods or uses described herein, the subject is suspected of having a mutant KRas-associated cancer (e.g., a cancer that was previously identified a cancer having no KRas mutation (e.g., KRas wild type)). In some embodiments, provided herein are methods for treating a KRas-associated cancer (e.g., a mutant KRas-associated cancer (e.g., a KRas G12A-associated cancer, a KRas G12C-associated cancer, a KRas G12D-associated cancer, a KRas G12R-associated cancer, a KRas G12S-associated cancer, or a KRas G12V-associated cancer (e.g., a KRas G12D-associated cancer or a KRas G12V-associated cancer))) in a subject in need of such treatment, the method comprising a) detecting a KRas dysregulation (e.g., a *KRAS* gene having a mutation corresponding to a mutation in a KRas protein and/or a KRas protein having a mutation, a *KRAS* gene copy number increase, and/or an increase in KRas mRNA or protein expression) in a sample from the subject; and b) administering a therapeutically effective amount of a compound provided herein, or a pharmaceutically acceptable salt thereof. In some embodiments, provided herein are methods for treating cancer having a KRas dysregulation in a subject in need of such treatment, the method comprising a) detecting a KRas dysregulation (e.g., a *KRAS* gene having a mutation corresponding to a mutation in a KRas protein and/or a KRas protein having a mutation, a *KRAS* gene copy number increase, and/or an increase in KRas mRNA or protein expression) in a sample from the subject; and b) administering a therapeutically effective amount of a compound provided herein, or a pharmaceutically acceptable salt thereof.

In some embodiments, a mutant KRas-associated cancer is characterized by a mutation that arises from treatment with a first KRas inhibitor; for example, a mutant KRas-associated cancer as described herein can include one or more KRas mutations that confer resistance to treatment with a first KRas inhibitor. For example, a subject can acquire one or more of the following KRas mutations as a resistance mutation to a KRas G12C inhibitor: G12D, G12R, G12V, G12W, G13D, Q61H, R68S, H95D, H95Q, H95R, or Y96C. See, e.g., Awad et al. N Engl J Med. 2021 Jun 24;384(25):2382-2393, doi: 10.1056/NEJMoa2105281.

As used herein, a "first inhibitor of KRas" or "first KRas inhibitor" is a KRas inhibitor as defined herein, but which does not include a compound provided herein, or a pharmaceutically acceptable salt thereof, as defined herein. As used herein, a "second inhibitor of KRas" or a "second KRas inhibitor" is a KRas inhibitor as defined herein, but which does not include a compound provided herein, or a pharmaceutically acceptable salt thereof. When both a first and a second inhibitor of KRas are present in a method provided herein, the first and second inhibitors of KRas are different. In some embodiments, the first and/or second inhibitor of KRas bind in a different location than a compound provided herein, or a pharmaceutically acceptable salt thereof.

Exemplary first and second inhibitors of KRas are described herein. In some embodiments, a first or a second inhibitor of KRas can be a KRas G12C inhibitor. In some embodiments, a first or second inhibitor of KRas can be selected from the group consisting of sotorasib, adragrasib, ARS-853, ARS-1620, ARS-3248, ATG-012, BI 1823911, D-1553, ERAS-3490, GDC-6036, GFH925, JAB-21822, JDQ-443, LY3537982, MRTX-1257, RMC-6291, and combinations thereof. In some embodiments, a first or second inhibitor of KRas can be selected from the group consisting of sotorasib, adragrasib, ARS-853, ARS-1620, ARS-3248, ATG-012, BI 1823911, D-1553, ERAS-3490, GDC-6036, GFH925, JAB-21822, JDQ-443, LY3537982, MRTX-1133, MRTX-1257, RMC-6291, RMC-6236, and combinations thereof.

In some embodiments, the methods provided herein include performing an assay on a sample (e.g., a tumor sample or a blood sample) obtained from the subject to determine whether the cancer in the subject has a KRas dysregulation (e.g., a KRas mutation (e.g., a KRas G12A mutation, a KRas G12C mutation, a KRas G12D mutation, a KRas G12R mutation, a KRas G12S mutation or a KRas G12V mutation (e.g., a KRas G12D mutation or a KRas G12V mutation)) or amplification). In some such embodiments, the method also includes administering to a subject determined to have a KRas dysregulation (e.g., a KRas mutation (e.g., a KRas G12A mutation, a KRas G12C mutation, a KRas G12D mutation, a KRas G12R mutation, a KRas G12S mutation or a KRas G12V mutation (e.g., a KRas G12D mutation or a KRas G12V mutation)) or amplification) a therapeutically effective amount of a compound provided herein, or a pharmaceutically acceptable salt thereof. In some embodiments, the method includes determining that a cancer in a subject has a KRas dysregulation (e.g., a KRas mutation (e.g., a KRas G12A mutation, a KRas G12C mutation, a KRas G12D mutation, a KRas G12R mutation, a KRas G12S mutation, or a KRas G12V mutation (e.g., a KRas G12D mutation or a KRas G12V mutation)) or amplification) via an assay performed on a sample obtained from the subject. In such embodiments, the method also includes administering to a subject a therapeutically effective amount of a compound provided herein, or a pharmaceutically acceptable salt thereof. Some embodiments of these methods further include administering to the subject another anticancer agent (e.g., a second compound provided herein, or a pharmaceutically acceptable salt thereof, or immunotherapy).

In some embodiments of any of the methods or uses described herein, an assay is used to determine whether the cancer in the subject has a KRas dysregulation (e.g., a KRas mutation (e.g., a KRas G12A mutation, a KRas G12C mutation, a KRas G12D mutation, a KRas G12R mutation, a KRas G12S mutation, or a KRas G12V mutation (e.g., a KRas G12D mutation or a KRas G12V mutation)) or amplification), using a sample (e.g., a tumor sample or a blood sample) from a subject can include, for example, next generation sequencing, immunohistochemistry, fluorescence microscopy, Southern blotting, Western blotting, FACS analysis, Northern blotting, and PCR-based amplification (e.g., RT-PCR and quantitative real-time RT-PCR). As is well-known in the art, the assays are typically performed, e.g., with at least one labelled nucleic acid probe or at least one labelled antibody or antigen-binding fragment thereof. Assays can utilize other detection methods known in the art for detecting a KRas dysregulation (e.g., a KRas mutation (e.g., a KRas G12A mutation, a KRas G12C mutation, a KRas G12D mutation, a KRas G12R mutation, a KRas G12S mutation, or a KRas G12V mutation (e.g., a KRas G12D mutation or a KRas G12V mutation)) or amplification) (see, e.g., the references cited herein). In some embodiments, the sample is tumor biopsy sample (e.g., a paraffin-embedded biopsy sample) from the subject. In some embodiments, the subject is a subject suspected of having a KRas-associated cancer (e.g., mutant KRas-associated cancer (e.g., a KRas G12A-associated cancer, a KRas G12C-associated cancer, a KRas G12D-associated cancer, a KRas G12R-associated cancer, a KRas G12S-associateion, or a KRas G12V-associated cancer (e.g., a KRas G12D-associated cancer or a KRas G12V-associated cancer))), a subject having one or more symptoms of a KRas-associated cancer (e.g., mutant KRas-associated cancer (e.g., a KRas G12A-associated cancer, a KRas G12C-associated cancer, a KRas G12D-associated cancer, a KRas G12R-associated cancer, a KRas G12S-associated cancer, or a KRas G12V-associated cancer (e.g., a KRas G12D-associated cancer or a KRas G12V-associated cancer))), and/or a subject that has an increased risk of developing a KRas-associated cancer (e.g., mutant KRas-associated cancer (e.g., a KRas G12A-associated cancer, a KRas G12C-associated cancer, a KRas G12D-associated cancer, a KRas G12R-associated cancer, a KRas G12S-associated cancer, or a KRas G12V-associated cancer (e.g., a KRas G12D-associated cancer or a KRas G12V-associated cancer))). In some embodiments, the subject is a subject suspected of having a cancer having a KRas dysregulation (e.g., a KRas mutation or amplification), a subject having one or more symptoms of a cancer having a KRas dysregulation (e.g., a KRas mutation or amplification), and/or a subject that has an increased risk of developing a cancer having a KRas dysregulation (e.g., a KRas mutation or amplification).

In some embodiments, a KRas dysregulation (e.g., a KRas mutation (e.g., a KRas G12A mutation, a KRas G12C mutation, a KRas G12D mutation, a KRas G12R mutation, a KRas G12S mutation, or a KRas G12V mutation (e.g., a KRas G12D mutation or a KRas G12V mutation)) or amplification) can be identified using a liquid biopsy (variously referred to as a fluid biopsy or fluid phase biopsy). See, e.g., Karachialiou et al., "Real-time liquid biopsies become a reality in cancer treatment", Ann. Transl. Med., 3(3):36, 2016, doi: 10.3978/j.issn.2305-5839.2015.01.16. Liquid biopsy methods can be used to detect total tumor burden and/or the KRas dysregulation (e.g., the KRas mutation or amplification). Liquid biopsies can be performed on biological samples obtained relatively easily from a subject (e.g., via a simple blood draw) and are generally less invasive than traditional methods used to detect tumor burden and/or KRas dysregulation (e.g., a KRas mutation (e.g., a KRas G12A mutation, a KRas G12C mutation, a KRas G12D mutation, a KRas G12R mutation, a KRas G12S mutation, or a KRas G12V mutation (e.g., a KRas G12D mutation or a KRas G12V mutation)) or amplification). In some embodiments, liquid biopsies can be used to detect the presence of a KRas dysregulation (e.g., a KRas mutation or amplification) at an earlier stage than traditional methods. In some embodiments, the biological sample to be used in a liquid biopsy can include, blood, plasma, urine, cerebrospinal fluid, saliva, sputum, broncho-alveolar lavage, bile, lymphatic fluid, cyst fluid, stool, ascites, and combinations thereof. In some embodiments, a liquid biopsy can be used to detect circulating tumor cells (CTCs). In some embodiments, a liquid biopsy can be used to detect cell-free DNA. In some embodiments, cell-free DNA detected using a liquid biopsy is circulating tumor DNA (ctDNA) that is derived from tumor cells. Analysis of ctDNA (e.g., using sensitive detection techniques such as, without limitation, next-generation sequencing (NGS), traditional PCR, digital PCR, or microarray analysis) can be used to identify a KRas dysregulation (e.g., KRas mutation (e.g., a KRas G12A mutation, a KRas G12C mutation, a KRas G12D mutation, a KRas G12R mutation, a KRas G12S mutation, or a KRas G12V mutation (e.g., a KRas G12D mutation or a KRas G12V mutation))).

Also provided is a method for modulating (e.g., decreasing) KRas protein activity (e.g., dysregulated KRas protein activity (e.g., mutant KRas protein activity (e.g., KRas G12R mutant protein activity or G12V mutant protein activity))) in a cell, comprising contacting the cell with a compound provided herein, or a pharmaceutically acceptable salt thereof. In some embodiments, the contacting is in vitro. In some embodiments, the contacting is in vivo. In some embodiments, the contacting is ex vivo. In some embodiments, the contacting is in vivo, wherein the method comprises administering an effective amount of a compound provided herein, or a pharmaceutically acceptable salt thereof, to a subject having a cell having a KRas protein (e.g., a dysregulated KRas protein (e.g., a mutant KRas protein (e.g., a KRas G12A mutant protein, a KRas G12C mutant protein, a KRas G12D mutant protein, a KRas G12R mutant protein, a KRas G12S mutant protein, or a KRas G12V mutant protein (e.g., a KRas G12D mutant protein or a KRas G12V mutant protein)))). In some embodiments, the contacting is ex vivo, wherein the method comprises contacting a cell from a subject having a KRas protein (e.g., a dysregulated KRas protein (e.g., a mutant KRas protein (e.g., a KRas G12A mutant protein, a KRas G12C mutant protein, a KRas G12D mutant protein, a KRas G12R mutant protein, a KRas G12S mutant protein, or a KRas G12V mutant protein (e.g., a KRas G12D mutant protein or a KRas G12V mutant protein)))) with a compound provided herein, or a pharmaceutically acceptable salt thereof. In some embodiments, the cell is a cancer cell. In some embodiments, the cell is a mammalian cell. In some embodiments, the cell is a mammalian cancer cell. In some embodiments, the cancer cell is any cancer as described herein. In some embodiments, the cancer cell is a KRas-associated cancer cell (e.g., a mutant KRas-associated cancer cell (e.g., a KRas G12A-associated cancer cell, a KRas G12C-associated cancer cell, a KRas G12D-associated cancer cell, a KRas G12R-associated cancer cell, a KRas G12S-associated cancer cell, or a KRas G12V-associated cancer cell (e.g., a KRas G12D-associated cancer cell or a KRas G12V-associated cancer cell))).

As used herein, the term "contacting" refers to the bringing together of indicated moieties in an in vitro system, an in vivo system, or an ex vivo system. For example, "contacting" a KRas protein with a compound provided herein includes the administration of a compound provided herein to an individual or subject, such as a human, having a KRas protein, as well as, for example, introducing a compound provided herein into a sample containing a cellular or purified preparation containing the KRas protein.

Also provided herein is a method of inhibiting cell proliferation, in vitro, in vivo, or ex vivo, the method comprising contacting a cell with an effective amount of a compound provided herein, or a pharmaceutically acceptable salt thereof, or a pharmaceutical composition thereof as defined herein. In some embodiments, the cell has a KRas dysregulation. In some embodiments, the cell has a KRas mutation. In some embodiments, the cell has a KRas G12D mutation or a KRas G12V mutation. In some embodiments, the cell has a KRas G12A mutation. In some embodiments, the cell has a KRas G12C mutation. In some embodiments, the cell has a KRas G12D mutation. In some embodiments, the cell has a KRas G12R mutation. In some embodiments, the cell has a KRas G12S mutation. In some embodiments, the cell has a KRas G12V mutation. In some embodiments, the cell has a KRas amplification.

Further provided herein is a method of increasing cell death, in vitro, in vivo, or ex vivo, the method comprising contacting a cell with an effective amount of a compound provided herein, or a pharmaceutically acceptable salt thereof, or a pharmaceutical composition thereof as defined herein. Also provided herein is a method of increasing tumor cell death in a subject. The method comprises administering to the subject a compound provided herein, or a pharmaceutically acceptable salt thereof, in an amount effective to increase tumor cell death. In some embodiments, the cell has a KRas dysregulation. In some embodiments, the cell has a KRas mutation. In some embodiments, the cell has a KRas G12D mutation or a KRas G12V mutation. In some embodiments, the cell has a KRas G12A mutation. In some embodiments, the cell has a KRas G12C mutation. In some embodiments, the cell has a KRas G12D mutation. In some embodiments, the cell has a KRas G12R mutation. In some embodiments, the cell has a KRas G12S mutation. In some embodiments, the cell has a KRas G12V mutation. In some embodiments, the cell has a KRas amplification.

When employed as pharmaceuticals, the compounds provided herein, or pharmaceutically acceptable salts thereof, can be administered in the form of pharmaceutical compositions as described herein.

Also provided herein is a method for inhibiting a KRas protein in a mammalian cell, the method comprising contacting the mammalian cell with an effective amount of a compound provided herein, or a pharmaceutically acceptable salt thereof. Also provided herein is a method for inhibiting a dysregulated KRas protein (e.g., a mutant KRas protein (e.g., a KRas G12A mutant protein, a KRas G12C mutant protein, a KRas G12D mutant protein, a KRas G12R mutant protein, a KRas G12S mutant protein, or a KRas G12V mutant protein (e.g., a KRas G12D mutant protein or a KRas G12V mutant protein))) in a mammalian cell, the method comprising contacting the mammalian cell with an effective amount of a compound provided herein, or a pharmaceutically acceptable salt thereof. In some embodiments, the mammalian cell is ex vivo.

Also provided herein is a method of treating a subject having a cancer, wherein the method comprises:
administering a therapeutically effective amount of a compound provided herein, or a pharmaceutically acceptable salt thereof, as a monotherapy or in conjunction with a first anticancer agent to the subject who has been administered one or more doses of the first anticancer agent to the subject for a period of time.

Also provided herein is a method of treating a subject having a cancer, wherein the method comprises:
(a) administering one or more doses of a first anticancer agent to the subject for a period of time;
(b) after (a), determining whether a cancer cell in a sample (e.g., a tumor sample or a blood sample) obtained from the subject has a KRas dysregulation (e.g., a KRas mutation (e.g., a KRas G12A mutation, a KRas G12C mutation, a KRas G12D mutation, a KRas G12R mutation, a KRas G12S mutation, or a KRas G12V mutation (e.g., a KRas G12D mutation or a KRas G12V mutation)) or amplification); and
(c) administering a therapeutically effective amount of a compound provided herein, or a pharmaceutically acceptable salt thereof, as a monotherapy or in conjunction with a second anticancer agent to the subject if the subject has been determined to have a cancer cell that has a KRas dysregulation (e.g., a KRas mutation or amplification); or
(d) administering additional doses of the first anticancer agent of step (a) to the subject if the subject has not been determined to have a cancer cell that has a KRas dysregulation (e.g., a KRas mutation (e.g., a KRas G12A mutation, a KRas G12C mutation, a KRas G12D mutation, a KRas G12R mutation, a KRas G12S mutation, or a KRas G12V mutation (e.g., a KRas G12D mutation or a KRas G12V mutation)) or amplification).

Further provided herein is a method of treating a subject having a cancer, wherein the method comprises:
(a) determining whether a cancer cell in a sample (e.g., a tumor sample or a blood sample) obtained from a subject having a cancer and previously administered one or more doses of a first anticancer agent has a KRas dysregulation (e.g., a KRas mutation (e.g., a KRas G12A mutation, a KRas G12C mutation, a KRas G12D mutation, a KRas G12R mutation, a KRas G12S mutation, or a KRas G12V mutation (e.g., a KRas G12D mutation or a KRas G12V mutation)) or amplification); and
(b) administering a therapeutically effective amount of a compound provided herein, or a pharmaceutically acceptable salt thereof, as a monotherapy or in conjunction with a second anticancer agent to the subject if the subject has been determined to have a cancer cell that has a KRas dysregulation (e.g., a KRas mutation (e.g., a KRas G12A mutation, a KRas G12C mutation, a KRas G12D mutation, a KRas G12R mutation, a KRas G12S mutation, or a KRas G12V mutation (e.g., a KRas G12D mutation or a KRas G12V mutation)) or amplification); or
(c) administering additional doses of the first anticancer agent to the subject if the subject has not been determined to have a cancer cell that has a KRas dysregulation (e.g., KRas mutation (e.g., a KRas G12A mutation, a KRas G12C mutation, a KRas G12D mutation, a KRas G12R mutation, a KRas G12S mutation, or a KRas G12V mutation (e.g., a KRas G12D mutation or a KRas G12V mutation)) or amplification).

Also provided herein is a method of treating a subject having a cancer, wherein the method comprises:
(a) determining that a cancer cell in a sample (e.g., a tumor sample or a blood sample) obtained from a subject having a cancer and previously administered one or more doses of a first anticancer agent has a KRas dysregulation (e.g., a KRas mutation (e.g., a KRas G12A mutation, a KRas G12C mutation, a KRas G12D mutation, a KRas G12R mutation, a KRas G12S mutation, or a KRas G12V mutation (e.g., a KRas G12D mutation or a KRas G12V mutation)) or amplification); and
(b) administering a therapeutically effective amount of a compound provided herein, or a pharmaceutically acceptable salt thereof, as a monotherapy or in conjunction with a second anticancer agent to the subject.

In some embodiments of any of the methods described herein, the first anticancer agent can be a first KRas inhibitor.

When employed as pharmaceuticals, the compounds provided herein, or pharmaceutically acceptable salts thereof, can be administered in the form of pharmaceutical compositions as described herein.

### Combinations

In any of the indications described herein, a compound provided herein, or a pharmaceutically acceptable salt thereof, can be used as a monotherapy. In some embodiments, a compound provided herein, or a pharmaceutically acceptable salt thereof, can be used prior to administration of an additional therapeutic agent or additional therapy. For example, a subject in need thereof can be administered one or more doses of a compound provided herein, or a pharmaceutically acceptable salt thereof, for a period of time and then undergo at least partial resection of the tumor. In some embodiments, the treatment with one or more doses of a compound provided herein, or a pharmaceutically acceptable salt thereof, reduces the size of the tumor (e.g., the tumor burden) prior to the at least partial resection of the tumor.

In some embodiments, a subject in need thereof can be administered one or more doses of a compound provided herein, or a pharmaceutically acceptable salt thereof, for a period of time and under one or more rounds of radiation therapy. In some embodiments, the treatment with one or more doses of a compound provided herein, or a pharmaceutically acceptable salt thereof, reduces the size of the tumor (e.g., the tumor burden) prior to the one or more rounds of radiation therapy.

In some embodiments, a subject has a cancer (e.g., a locally advanced or metastatic tumor) that is refractory or intolerant to standard therapy (e.g., administration of a chemotherapeutic agent, such as a first KRas inhibitor, a kinase inhibitor, immunotherapy, or radiation). In some embodiments, a subject has a cancer (e.g., a locally advanced or metastatic tumor) that is refractory or intolerant to prior therapy (e.g., administration of a chemotherapeutic agent, such as a first KRas inhibitor, a kinase inhibitor, immunotherapy, or radiation). In some embodiments, a subject has a cancer (e.g., a locally advanced or metastatic tumor) that has no standard therapy. In some embodiments, a subject is KRas inhibitor naive. In some embodiments, a subject is not KRas inhibitor naive. In some embodiments, a subject has undergone prior therapy. For example, treatment with surgery, radiation, a chemotherapeutic agent, an immunotherapy, a multi-kinase inhibitor (MKI), a KRas inhibitor, a RAF/MEK/PI3K pathway inhibitor, a MEK inhibitor, a Raf inhibitor, a YAP inhibitor, a proteasome inhibitor, a PI3K-AKT-mTOR pathway inhibitor, an ERK inhibitor, a pan-ErbB inhibitor, a MET inhibitor, a farnesyl transferase inhibitor, a FAK inhibitor, a HSP90 inhibitor, or a combination thereof.

In some embodiments of any the methods described herein, the compound provided herein, or a pharmaceutically acceptable salt thereof, is administered in combination with a therapeutically effective amount of at least one additional therapeutic agent selected from one or more additional therapies or therapeutic (e.g., chemotherapeutic) agents.

Non-limiting examples of additional therapeutic agents include: Ras pathway targeted therapeutic agents (e.g., Ras/RAF/MEK/PI3K pathway inhibitors or degraders, (e.g., Ras inhibitors or degraders, KRas-targeted therapeutic agents, SOS1 inhibitors or degraders, SOS1/Ras protein-protein interaction inhibitors, SHP2 inhibitors or degraders, PI3K-AKT-mTOR pathway inhibitors or degraders)), kinase-targeted therapeutics (e.g., MEK inhibitors or degraders, ERK inhibitors or degraders, Raf inhibitors or degraders (e.g., BRaf inhibitors or degraders), PI3K inhibitors or degraders, AKT inhibitors or degraders, mTOR inhibitors or degraders, CDK4/5 inhibitors or degraders, CDK4/6 inhibitors or degraders, MET inhibitors or degraders, FAK inhibitors or degraders, ErbB family inhibitors or degraders (e.g., EGFR inhibitors or degraders, Her2 inhibitors or degraders), Src inhibitors or degraders), Bcl-X_{L} inhibitors or degraders, mTORC1 inhibitors or degraders, YAP inhibitors or degraders, TEAD inhibitors or degraders, proteasome inhibitors or degraders, farnesyl transferase inhibitors or degraders, HSP90 inhibitors or degraders, PTEN inhibitors or degraders, signal transduction pathway inhibitors or degraders, checkpoint inhibitors, modulators of the apoptosis pathway (e.g., venetoclax, navitoclax, obataclax), chemotherapeutics, angiogenesis-targeted therapies, immune-targeted agents including immunomodulatory imide drugs (sometimes called "IMiDs" or "CELMoDs"), immunotherapy (e.g., anti-PD1, anti-PD-L1, anti-CTLA4, anti-LAG3, anti-TIM3, anti-B7-H3, anti-VISTA therapies, including antibodies (e.g., single-targeted antibodies targeting one or more of PD1, PD-L1, CTLA4, LAG3, TIM3, B7-H3, or VISTA; bispecific antibodies (including bispecific T cell engagers (BiTEs)) targeting one or more of PD1, PD-L1, CTLA4, LAG3, TIM3, B7-H3, or VISTA; and antibody-drug conjugates (ADCs) incorporating one or more of PD1, PD-L1, CTLA4, LAG3, TIM3, B7-H3, or VISTA) or antigen-binding fragments thereof, a PD-1 inhibitor, a PD-L1 inhibitor, or an ADORA2A inhibitor), cell-based therapeutics (e.g., adoptive cell therapy (e.g., CAR T therapy, cytokine-induced killer cells (CIKs), natural killer cells (e.g., CAR-modified NK cells)) or antibody-armed cell therapy), and radiotherapy. See also, e.g., the therapeutic agents listed in U.S. Publication No. US 2021/0130303.

A "degrader" as used herein is a heterobifunctional molecule that induces degradation of a target protein, the degrader including a moiety that binds to the target protein and a moiety that binds to a ubiquitin E3 ligase (sometimes referred to as an E3 ligase or simply an E3), these two moieties being optionally separated by a linker. Such degraders are sometimes known as "PROTACs".

A "Ras pathway targeted therapeutic agent" as used herein includes any compound exhibiting inactivation activity of any protein in a Ras pathway (e.g., kinase inhibition, allosteric inhibition, inhibition of dimerization, and/or induction of degradation). Non-limiting examples of a protein in a Ras pathway include any one of the proteins in the Ras-RAF-MAPK pathway or PI3K/AKT pathway such as Ras (e.g., KRas, HRas, and NRas), RAF, BRAF, MEK, ERK, PI3K, AKT, and mTOR. In some embodiments, a Ras pathway modulator can be selective for a protein in a Ras pathway, e.g., the Ras pathway modulator can be selective for Ras (also referred to as a Ras modulator). In some embodiments, a Ras modulator is a covalent inhibitor. In some embodiments, a Ras pathway targeted therapeutic agent is a "KRas pathway modulator." A KRas pathway modulator includes any compound exhibiting inactivation activity of any protein in a KRas pathway (e.g., kinase inhibition, allosteric inhibition, inhibition of dimerization, and/or induction of degradation). Non-limiting examples of a protein in a KRas pathway include any one of the proteins in the KRas-RAF-MAPK pathway or PI3K/AKT pathway such as KRas, RAF, BRAF, MEK, ERK, PI3K, AKT, and mTOR. In some embodiments, a KRas pathway modulator is a KRas-targeted therapeutic agent. In some embodiments, the Ras pathway targeted therapeutic agent is a SOS1 inhibitor or a SHP2 inhibitor. Non-limiting examples of SOS1 inhibitors include MRTX-0902 and RMC-5845. Non-limiting examples of SHP2 inhibitors include batoprotafib (TNO-155), vociprotafib (RMC-4630), ARRY-558, BBP-398, ENT-03, ERAS-601, ET-0038, GDC-1971 (RLY-1971), GH-21, HS-10381, ICP-189, JAB-3068, JAB-3312, and SH-3809.

Non-limiting examples of KRas-targeted therapeutic agents (e.g., a first KRas inhibitor or a second KRas inhibitor) include a KRas-selective inhibitor, a Ras inhibitor, and an anti-KRas antibody. In some embodiments, the KRas inhibitor is a covalent inhibitor. In some embodiments, the KRas-targeted therapeutic agent is adagrasib, divarasib (GDC-6036), garsorasib (D-1553), glecirasib (JAB-21822), olomorasib (LY-3537982), sotorasib, ARS-1620, ARS-3248, ARS-853, ASP-3082, ATG-012, BI-1701963, BI-1823911, BPI-421286, ERAS-3490, GFH-925, GH-35, JDQ-443, MK-1084, MRTX-1133, MRTX-1257, RMC-6236, RMC-6291, RMC-7977, RMC-9805, RSC-1255, SHR-1127, or a combination thereof.

In some embodiments, the KRas-targeted therapeutic agent is an agent that inhibits the interaction between KRas and SOS 1 or SHP2. Non-limiting examples of an agent that inhibits the interaction between SOS1 and KRas include BI-3406, BI-1701963, and BAY 293.

Additional KRas-targeted therapeutic agents (e.g., a first KRas inhibitor or a second KRas inhibitor) include those disclosed in International Publication Nos. WO 2021/104431; WO WO2021/119343; WO2021/113595; WO 2021/107160; WO 2016/161361; WO 2016/17262; WO 2020/035031; WO 2021/041671; WO 2016/077793; WO 2020/180768; WO 2021/092115; WO 2020/180770; U.S. Patent Nos. US 10,898,487; US 10,829,487; US 10,858,359; US 10,561,655; US 10,532,042; U.S. Publication Nos. US 2021/0101870; US 2019/0231805; US 2020/0017517; US 2020/0017511; US 2020/0147058; US 2021/0009577; and Hillig et al. PNAS. 2019; 116(7): 2551-2560, doi: 10.1073/pnas.1812963116.

Further non-limiting examples of Ras pathway-targeted therapeutic agents include BRAF inhibitors, MEK inhibitors, ERK inhibitors, PI3K inhibitors, AKT inhibitors, and mTOR inhibitors.

In some embodiments, the BRAF inhibitor is avutometinib, dabrafenib (e.g., dabrafenib mesylate, TAFINLAR^{®}), encorafenib (BRAFTOVI^{™}), naporafenib, sorafenib (e.g., sorafenib tosylate), vemurafenib (ZELBORAF^{®}), ARQ 736, AZ304, BMS-908662 (XL281), C17071479-F, CHIR-265, FORE-8394, GDC-0879, GSK2118436, HLX-208, HM95573, LGX818, LXH254, PLX-3603, PLX-4720, PLX-8394, RAF265, RO5126766, RO5185426, or a combination thereof. In some embodiments, the BRAF inhibitor is avutometinib, dabrafenib (e.g., dabrafenib mesylate), encorafenib, naporafenib, sorafenib (e.g., sorafenib tosylate), vemurafenib, C17071479-F, CHIR-265, FORE-8394, HLX-208, or a combination thereof.

In some embodiments, the MEK inhibitor is avutometinib, binimetinib (MEKTOVI^{®}, MEK162), cobimetinib (e.g., cobimetinib fumarate, COTELLIC^{®}), mirdametinib, pimasertib, refametinib, selumetinib (e.g., selumetinib sulfate, AZD6244), trametinib (e.g., trametinib dimethyl sulfoxide, GSK-1120212 MEKINIST^{®}), zapnometinib, hypothemycin, CI1040 (PD184352), CS3006, FCN-159, MSC1936369B, NFX-179, PD0325901, PD98059,RO5126766, SHR7390, TAK-733, WX-554, or a combination thereof. In some embodiments, the MEK inhibitor is avutometinib, binimetinib, cobimetinib (e.g., cobimetinib fumarate), mirdametinib, nedometinib, pimasertib, refametinib, selumetinib (e.g., selumetinib sulfate), trametinib (e.g., trametinib dimethyl sulfoxide, GSK-1120212), tunlametinib, zapnometinib, FCN-159, NFX-179, TAK-733, or a combination thereof. In some embodiments, the MEK inhibitor is a MEK-Raf protein-protein interaction stabilizer, such as NST-628 or avutometinib (VS6766).

In some embodiments, the ERK inhibitor is 25-OH-D3-3-BE (B3CD, bromoacetoxycalcidiol), 5-7-Oxozeaenol, 5-iodotubercidin, AEZ-131 (AEZS-131), AEZS-136, ASN007, AZ-13767370, BL-EI-001, CC-90003, FR148083, FR-180204, FRI-20 (ON-01060), GDC0994, GDC-0994 (RG-7482), KO-947, KO-947, LTT-462, LY-3214996, MK-8353 (SCH900353), ONC201SCH772984, ulixertinib (BVD-523), VTX-11e, or a combination thereof. In some embodiments, the ERK inhibitor is rineterkib, ulixertinib, or a combination thereof.

In some embodiments, PI3K inhibitor is alpelisib (BYL719), apitolisib (GDC-0980), buparlisib (BKM120), copanlisib (ALIQOPA^{™}, BAY80-6946), dactolisib (NVP-BEZ235, BEZ-235), gedatolisib (PF-05212384, PKI-587), omipalisib (GSK2126458, GSK458), pictilisib (GDC-0941), pilaralisib (XL147, SAR245408), rigosertib, serabelisib (TAK-117, MLN1117, INK 1117), sonolisib (PX-866), taselisib (GDC-0032, RG7604), voxtalisib (XL756, SAR245409), wortmannin, AMG 511, AMG319, ASN003, AZD8835, BGT-226 (NVP-BGT226), CH5132799, CUDC-907, GDC-0077, GDC-0084 (RG7666), GS-9820, GSK1059615, GSK2636771, KIN-193 (AZD-6428), LY2023414, LY294002, PF-04691502, PI-103, PKI-402, PQR309, SAR260301, SF1126, VS-5584 (SB2343), WX-037, XL-765, ZSTK474, or a combination thereof. In some embodiments, the PI3K inhibitor is alpelisib, amdizalisib, apitolisib, bimiralisib, buparlisib, copanlisib (e.g., copanlisib dihydrochloride or a hydrate of copanlisib dihydrochloride), dactolisib, dezapelisib, dordaviprone, duvelisib (e.g., a hydrate of duvelisib), eganelisib, fimepinostat, gedatolisib, idelalisib, inavolisib, leniolisib (e.g., leniolisib phosphate), linperlisib, parsaclisib, paxalisib, risovalisib, seletalisib, serabelisib, sonolisib, tenalisib, umbralisib (e.g., umbralisib tosylate), zandelisib, PF-04691502, SHC-014748-M, TQ-B-3525, or a combination thereof.

In some embodiments, the AKT inhibitor is 2-[4-(2-aminoprop-2-yl)phenyl]-3-phenylquinoxaline, 3-oxo-tirucallic acid, A-443654, A-674563, afuresertib, API-1, ARQ092, AT13148, AT7867, AZD5363, BAY 1125976, boc-Phe-vinyl ketone, CCT128930, DC120, DM-PIT-1, edelfosine, erucylphophocholine, erufosine, GSK2141795, GSK690693, H-89, ipatasertib (GDC-0068, RG7440), lactoquinomycin, miltefosine (IMPADIVO^{®}), MK-2206, N-(4-(5-(3-acetamidophenyl)-2-(2-aminopyridin-3-yl)-3H-imidazo[4,5-b] pyridin-3-yl)benzyl)-3-fluorobenzamide, NL-71-101, ONC201, OSU-A9, Perifosine (D-21266), PH-316, PHT-427, PIT-1, SR13668, TCN, TCN-P, triciribine (Triciribine Phosphate Monohydrate), uprosertib, wortmannin, or a combination thereof. In some embodiments, the AKT inhibitor is afuresertib, capivasertib (AZD-5363), miransertib (e.g., miransertib mesylate), pifusertib, uprosertib, MK-2206, SM-020, or a combination thereof.

In some embodiments, the mTOR inhibitor is MLN0128, AZD-2014, CC-223, AZD2014, CC-115, everolimus (RAD001), temsirolimus (CCI-779), ridaforolimus (AP-23573), sirolimus (rapamycin), or a combination thereof. In some embodiments, the mTOR inhibitor is apitolisib, bimiralisib, dactolisib, everolimus, fosciclopirox (e.g., fosciclopirox sodium), gedatolisib, onatasertib, paxalisib, sapanisertib, sirolimus, sodium 2-hydroxylinoleate, temsirolimus, umirolimus, zandelisib, zotarolimus, BI-860585, CC-115, PF-04691502, or a combination thereof. In some embodiments, the mTOR inhibitor is everolimus, sirolimus, temsirolimus, umirolimus, zotarolimus, or a combination thereof. In some embodiments, the mTOR inhibitor is everolimus, sirolimus, temsirolimus, umirolimus, zotarolimus, RMC5552, or a combination thereof.

In some embodiments, the farnesyl transferase inhibitor is lonafarnib, tipifarnib, BMS-214662, L778123, L744832, FTI-277, or a combination thereof. In some embodiments, the farnesyl transferase inhibitor is lonafarnib, tipifarnib, BMS-214662, or a combination thereof.

In some embodiments, a chemotherapeutic agent includes a DNA replication inhibitor (e.g., a DNA intercalator (e.g., an anthracycline)), a DNA crosslinker (e.g., cyclophosphamide, a mitomycin (e.g., mitomycin C), a platinum complex), a ribonucleotide-diphosphate reductase inhibitor (e.g., gemcitabine), or a topoisomerase inhibitor), an anti-microtubule agent (e.g., a taxane, a vinca alkaloid, or eribulin), or a combination thereof.

Non-limiting examples of a taxane include paclitaxel, docetaxel, abraxane, and taxotere.

In some embodiments, the anthracycline is selected from daunorubicin, doxorubicin, epirubicin, idarubicin, and combinations thereof.

In some embodiments, the platinum-based agent is selected from carboplatin, cisplatin, oxaliplatin, nedplatin, triplatin tetranitrate, phenanthriplatin, picoplatin, satraplatin, and combinations thereof.

In some embodiments, the chemotherapy is a platinum complex, a microtubule inhibitor (e.g., a microtubule destabilizer or a microtubule stabilizer), a topoisomerase inhibitor, or an antibody-drug conjugate including any thereof. In some embodiments, the platinum complex is carboplatin, cisplatin, lobaplatin, miriplatin, oxaliplatin, or a combination thereof. In some embodiments, the microtubule inhibitor is cabazitaxel, colchicine, desoxyepothilone B, docetaxel, eribulin, ixabepilone, nab-paclitaxel, paclitaxel, plinabulin, sabizabulin, tirbanibulin, vinblastine, vinflunine, vinorelbine, or a combination thereof. In some embodiments, the microtubule inhibitor is cabazitaxel, docetaxel, nab-paclitaxel, paclitaxel, or a combination thereof. In some embodiments, the topoisomerase inhibitor is aclarubicin, amsacrine, belotecan, camptothecin, daunorubicin, dexrazoxane, elliptinium, epirubicin, etoposide, gepotidacin, idarubicin, mitoxantrone, nemonoxacin, pirarubicin, pixantrone, razoxane, rubitecan, sobuzoxane, temozolomide, teniposide, topotecan, SN-38, or a combination thereof. In some embodiments, the hypomethylating agent is azacitidine, decitabine, or a combination thereof. In some embodiments, the chemotherapy is a platinum complex and a topoisomerase inhibitor (e.g., cisplatin and etoposide). In some embodiments, the antibody-drug conjugate including the microtubule inhibitor is belantamab mafodotin, brentuximab vedotin, cofetuzumab pelidotin, disitamab vedotin, enfortumab vedotin (e.g., enfortumab vedotin-ejfv, or a biosimilar thereof), mirvetuximab soravtansine (e.g., mirvetuximab soravtansine-gynx, or a biosimilar thereof), polatuzumab vedotin, telisotuzumab vedotin, tisotumab vedotin, trastuzumab emtansine (e.g., ado-trastuzumab emtansine, or a biosimilar thereof), tusamitamab ravtansine, upifitamab rilsodotin, zilovertamab vedotin, Alpha-Her2-pAF1-AS-269, BAT-8001, TAA-013, biosimilars thereof, or a combination thereof. In some embodiments, the antibody-drug conjugate including the microtubule inhibitor is enfortumab vedotin (e.g., enfortumab vedotin-ejfv, or a biosimilar thereof). In some embodiments, the antibody-drug conjugate including the microtubule inhibitor is mirvetuximab soravtansine (e.g., mirvetuximab soravtansine-gynx, or a biosimilar thereof). In some embodiments, the antibody-drug conjugate including the microtubule inhibitor is trastuzumab emtansine (e.g., ado-trastuzumab emtansine, or a biosimilar thereof). In some embodiments, the antibody-drug conjugate including the topoisomerase inhibitor is datopotamab deruxtecan, patritumab deruxtecan, sacituzumab govitecan (e.g., sacituzumab govitecan-hziy, or a biosimilar thereof), trastuzumab deruxtecan (fam-trastuzumab deruxtecan-nxki, or a biosimilar thereof), or a combination thereof. In some embodiments, the antibody-drug conjugate including the topoisomerase inhibitor is sacituzumab govitecan (e.g., sacituzumab govitecan-hziy, or a biosimilar thereof). In some embodiments, the antibody-drug conjugate including the topoisomerase inhibitor is trastuzumab deruxtecan (e.g., fam-trastuzumab deruxtecan-nxki, or a biosimilar thereof).

In some embodiments, the chemotherapy includes one or more of capecitabine, carboplatin, cisplatin, docetaxel, doxorubicin (e.g., liposomal doxorubicin), fluorouracil, gemcitabine, leucovorin, mitomycin, oxaliplatin, paclitaxel (e.g., albumin-bound paclitaxel), and pemetrexed.

In some embodiments, the chemotherapy is FOLFOX. In some embodiments, the chemotherapy is FOLFIRI. In some embodiments, the chemotherapy is FOLFIRINOX. In some embodiments, the chemotherapy is CAPEOX. In some embodiments, the chemotherapy is GEMOX. In some embodiments, the chemotherapy is NALIRIFOX. In some embodiments, the chemotherapy is carboplatin and paclitaxel. In some embodiments, the chemotherapy is capecitabine and mitomycin. In some embodiments, the chemotherapy is fluorouracil, leucovorin, and oxaliplatin. In some embodiments, the chemotherapy is carboplatin and doxorubicin (e.g., liposomal doxorubicin). In some embodiments, the chemotherapy is gemcitabine. In some embodiments, the chemotherapy is gemcitabine and paclitaxel.

In some embodiments, the BCL-X_{L} inhibitor or degrader is foselutoclax (UBX-1325), , navitoclax, obatoclax, pelcitoclax, mirzotamab clezutoclax (ABBV-155), ABBV-637, APG-1252-12A, AZD-0466, DT-2216, PA-15227, UBX-1967, XZ-739, 753-B, or a combination thereof.

In some embodiments, the CDK4/6 inhibitor is abemaciclib, birociclib, dalpiciclib, lerociclib, milciclib, palbociclib, ribociclib (e.g., ribociclib succinate), riviciclib, roniciclib, trilaciclib (e.g., trilaciclib dihydrochloride), BPI-16350, FCN-437, SPH-4336, or a combination thereof. In some embodiments, the CDK4/6 inhibitor is palbociclib or ribociclib (e.g., ribociclib succinate).

In some embodiments, the EGFR inhibitor is abivertinib, afatinib (e.g., afatinib dimaleate), alflutinib (e.g., alflutinib mesylate), almonertinib (e.g., almonertinib mesylate), befotertinib, brigatinib, canertinib, dacomitinib (e.g., dacomitinib monohydrate), dovitinib, erlotinib (e.g., erlotinib hydrochloride), gefitinib, icotinib, lapatinib (e.g., lapatinib ditosylate monohydrate), larotinib, lazertinib, limertinib, mobocertinib (e.g., mobocertinib succinate), nazartinib, neratinib (e.g., neratinib maleate), olmutinib, osimertinib (e.g., osimertinib mesylate), pelitinib, poziotinib, pyrotinib (e.g., pyrotinib maleate), ruserontinib (SKLB-1028), sapitinib, sunvozertinib, sutetinib, tesevatinib, vandetanib, varlitinib, zipalertinib, zorifertinib, BIBW-2948, BPI-7711, HA-121-28, SH-1028, or a combination thereof. In some embodiments, the EGFR inhibitor is an EGFR-targeting biologic.

In some embodiments, the EGFR-targeting biologic is amivantamab (e.g., amivantamab-vmjw, or a biosimilar thereof), cetuximab (e.g., ERBITUX^{®} (cetuximab), or a biosimilar thereof (e.g., CMAB-009, CPGJ-602, or KL-140)), cetuximab sarotalocan (AKALUX^{®} (cetuximab sarotalocan), or a biosimilar thereof), depatuxizumab, duligotuzumab, futuximab, imgatuzumab, modotuximab, necitumumab (e.g., PORTRAZZA^{®} (necitumumab), or a biosimilar thereof), nimotuzumab (e.g., BIOMAb EGFR^{®} (nimotuzumab), or a biosimilar thereof), panitumumab (e.g., VECTIBIX^{®} (panitumumab), or a biosimilar thereof), tomuzotuximab, zalutumumab, EMD-55900, EMD-82633, GC-1118, HLX-07, ICR-62, SCT-200, SI-B-001, biosimilars thereof, or a combination thereof. In some embodiments, the EGFR-targeting biologic is cetuximab or panitumumab. In some embodiments, the EGFR-targeting biologic is cetuximab. In some embodiments, the EGFR-targeting biologic is panitumumab.

In some embodiments, the PARP inhibitor is iniparib, niraparib, olaparib (LYNPARZA^{®}), pamiparib (BGB-290), rucaparib, talazoparib, veliparib, 2X-121, ABT-767, BMN 673, BSI-201, CEP 9722, E7016, IMP4297, INO-1001, JPI-289, KU-0059436 (AZD2281), NOV1401, PF-01367338, and RBN-2397. In some embodiments, the PARP inhibitor is fuzuloparib (fluzoparib), niraparib (e.g., niraparib tosylate monohydrate), olaparib, pamiparib, rucaparib (e.g., rucaparib camsylate), saruparib (AZD5305), senaparib, stenoparib, talazoparib (e.g., talazoparib tosylate), veliparib, CEP-9722, JPI-289, NMS-03305293, or a combination thereof. In some embodiments, the PARP inhibitor is a PARP1 inhibitor. In some embodiments, the PARP1 inhibitor is saruparib (AZD5305), NMS-03305293, or a combination thereof.

Non-limiting examples of immunotherapy include immune checkpoint therapies. Non-limiting examples of immune checkpoint therapies include antibodies and/or inhibitors that target CTLA-4, PD-1, PD-L1, BTLA, LAG-3, ADORA2A, TIM-3, B7-H3, VISTA, IDO, and combinations thereof.

In some embodiments, the anti-CTLA4 therapy is abatacept (e.g., ORENCIA^{®} (abatacept), or a biosimilar thereof), botensilimab, cadonilimab, erfonrilimab, gotistobart, ipilimumab (e.g., YERVOY^{®} (ipilimumab), or a biosimilar thereof), nurulimab, quavonlimab, tremelimumab (ticilimumab) (e.g., IMIUDO^{®} (tremelimumab), or a biosimilar thereof), volrustomig, vudalimab, zalifrelimab, BMS-986218, PSB-205, biosimilars thereof, or a combination thereof. In some embodiments, the anti-CTLA4 therapy is ipilimumab or tremelimumab. In some embodiments, the anti-CTLA4 therapy is ipilimumab. In some embodiments, the anti-CTLA4 therapy is tremelimumab. In some embodiments, the anti-CTLA4 therapy is used in combination with anti-PD1 or anti-PD-L1 therapy.

In some embodiments, the anti-PD1 therapy is balstilimab, budigalimab, cadonilimab, camrelizumab, cemiplimab (e.g., cemiplimab-rwlc, or a biosimilar thereof), cetrelimab, dostarlimab (e.g., dostarlimab-gxly, or a biosimilar thereof), enlonstobart (SG-001), ezabenlimab, geptanolimab, ivonescimab, nivolumab (e.g., OPDIVO^{®} (nivolumab), or a biosimilar thereof), nofazinlimab, pembrolizumab (e.g., KEYTRUDA^{®} (pembrolizumab), or a biosimilar thereof), penpulimab, pidilizumab, pimivalimab, prolgolimab, pucotenlimab, retifanlimab (e.g., retifanlimab-dlwr, or a biosimilar thereof), rilvegostomig, rosnilimab, rulonilimab, sasanlimab, serplulimab, sintilimab (e.g., TYVYT^{®} (sintilimab), or a biosimilar thereof), spartalizumab, tebotelimab, tislelizumab, toripalimab, volrustomig, vudalimab, zimberelimab, QL-1604, HX-009, INCB-086550, RG-6139, BAT-1306, BAT-1308, biosimilars thereof, or a combination thereof. In some embodiments, the anti-PD1 therapy is cemiplimab, nivolumab, or pembrolizumab. In some embodiments, the anti-PD1 therapy is cemiplimab. In some embodiments, the anti-PD1 therapy is nivolumab. In some embodiments, the anti-PD1 therapy is pembrolizumab. In some embodiments, the anti-PD1 therapy is used in combination with anti-CTLA4 therapy.

In some embodiments, the anti-PD-L1 therapy is adebrelimab, atezolizumab (e.g., TECENTRIQ^{®} (atezolizumab), or a biosimilar thereof), avelumab (e.g., BAVENCIO^{®} (avelumab), or a biosimilar thereof), benmelstobart (APL-502), bintrafusp alfa, cosibelimab, danburstotug, durvalumab (e.g., IMFINZI^{®} (durvalumab), or a biosimilar thereof), envafolimab (e.g., ENWEIDA^{®} (envafolimab), or a biosimilar thereof), erfonrilimab, lesabelimab, pacmilimab, socazolimab, sugemalimab (e.g., CEJEMLY^{®} (sugemalimab), or a biosimilar thereof), tagitanlimab (A-167), AUPM-170, BNT-311, SHR-1701, biosimilars thereof, or a combination thereof. In some embodiments, the anti-PD-L1 therapy is atezolizumab or durvalumab. In some embodiments, the anti-PD-L1 therapy is atezolizumab. In some embodiments, the anti-PD-L1 therapy is durvalumab. In some embodiments, the anti-PD-L1 therapy is used in combination with anti-CTLA4 therapy.

In some embodiments, the PD-L1 inhibitor is INCB-086550 or INCB-099280.

In some embodiments, the anti-LAG3 therapy is eftilagimod alfa, favezelimab, fianlimab, ieramilimab, miptenalimab, negalstobart (IBI-110), relatlimab (e.g., relatlimab-rmbw, or a biosimilar thereof), tebotelimab, tobemstomig (RG-6139), tuparstobart (INCAGN-02385), HLX-26, LBL-007, SHR-1802, biosimilars thereof, or a combination thereof.

In some embodiments, the ADORA2A inhibitor is etrumadenant, inupadenant, istradefylline, mefloquine (e.g., mefloquine), taminadenant, CPI-444, PBF-999, or a combination thereof. In some embodiments, the ADORA2A inhibitor is etrumadenant, inupadenant, istradefylline, mefloquine (e.g., mefloquine), taminadenant, PBF-999, or a combination thereof.

In some embodiments, the anti-TIM3 therapy is cobolimab, sabatolimab (MBG-453), surzebiclimab, AZD-7789, INCAGN-02390, TQB-2618, or a combination thereof.

In some embodiments, the anti-B7-H3 therapy is omburtamab, enoblituzumab, or a combination thereof.

In some embodiments, the anti-VISTA therapy is onvatilimab (JNJ-61610588), HMBD-002, K01401-020, KVA-12.1, SNS-101, or a combination thereof.

In some embodiments, the IDO inhibitor (e.g., IDO1 and/or IDO2 inhibitor) is 3-deazaguanine, beta-lapachone, diindolylmethane, epacadostat, indole-3-carbinol, indoximod, linrodostat, sertaconazole (e.g., sertaconazole nitrate), or a combination thereof. See, for example, Marin-Acevedo, et al., J Hematol Oncol. 11: 39 (2018), doi: 10.1186/s13045-018-0582-8.

In some embodiments, the additional therapy or therapeutic agent is a combination of atezolizumab and nab-paclitaxel.

Accordingly, also provided herein is a method of treating cancer, comprising administering to a subject in need thereof (a) a compound provided herein, or a pharmaceutically acceptable salt thereof, (b) an additional therapeutic agent, and (c) optionally at least one pharmaceutically acceptable carrier for simultaneous, separate or sequential use for the treatment of cancer, wherein the amounts of the compound provided herein, or a pharmaceutically acceptable salt thereof, and the additional therapeutic agent are together effective in treating the cancer.

In some embodiments, the additional therapeutic agent(s) includes any one of the above listed therapies or therapeutic agents which are standards of care in cancers wherein the cancer has a KRas dysregulation (e.g., a KRas mutation or amplification). In some embodiments, the additional therapeutic agent(s) includes any one of the above listed therapies or therapeutic agents which are standards of care in a KRas-associated cancer (e.g., a mutant KRas-associated cancer (e.g., a KRas G12D-associated cancer, a KRas G12R-associated cancer, or a KRas G12V-associated cancer (e.g., a KRas G12D-associated cancer or a KRas G12V-associated cancer))).

These additional therapeutic agents may be administered with one or more doses of the compound provided herein, or a pharmaceutically acceptable salt thereof, or pharmaceutical composition thereof, as part of the same or separate dosage forms, via the same or different routes of administration, and/or on the same or different administration schedules according to standard pharmaceutical practice known to one skilled in the art.

Also provided herein is (i) a pharmaceutical composition for treating a cancer in a subject in need thereof, which comprises (a) a compound provided herein, or a pharmaceutically acceptable salt thereof, (b) at least one additional therapeutic agent (e.g., any of the exemplary additional therapeutic agents described herein or known in the art), and (c) optionally at least one pharmaceutically acceptable carrier for simultaneous, separate or sequential use for the treatment of cancer, wherein the amounts of the compound provided herein, or a pharmaceutically acceptable salt thereof, and of the additional therapeutic agent are together effective in treating the cancer; (ii) the use of such a composition for the preparation of a medicament for the treatment of cancer; and (iii) a commercial package or product comprising such a composition for simultaneous, separate or sequential use. In some embodiments, the cancer is a KRas-associated cancer (e.g., a mutant KRas-associated cancer (e.g., a KRas G12D-associated cancer, a KRas G12R-associated cancer, or a KRas G12V-associated cancer (e.g., a KRas G12D-associated cancer or a KRas G12V-associated cancer))).

Accordingly, also provided herein is a method of treating a cancer, comprising administering to a subject in need thereof (a) a compound provided herein, or a pharmaceutically acceptable salt thereof, and (b) an additional therapeutic agent, wherein the compound provided herein, or a pharmaceutically acceptable salt thereof, and the additional therapeutic agent are administered simultaneously, separately or sequentially, wherein the amounts of the compound provided herein, or a pharmaceutically acceptable salt thereof, and the additional therapeutic agent are together effective in treating the cancer. In some embodiments, the compound provided herein, or a pharmaceutically acceptable salt thereof, and the additional therapeutic agent are administered simultaneously as separate dosages. In some embodiments, a compound provided herein, or a pharmaceutically acceptable salt thereof, and the additional therapeutic agent are administered as separate dosages sequentially in any order, in jointly therapeutically effective amounts, e.g., in daily or intermittently dosages. In some embodiments, the compound provided herein, or a pharmaceutically acceptable salt thereof, and the additional therapeutic agent are administered simultaneously as a combined dosage. In some embodiments, the cancer is a KRas-associated cancer (e.g., a mutant KRas-associated cancer (e.g., a KRas G12D-associated cancer, a KRas G12R-associated cancer, or a KRas G12V-associated cancer (e.g., a KRas G12D-associated cancer or a KRas G12V-associated cancer))).

The term "wild type" or "wild-type" describes a nucleic acid (e.g., a *KRAS* gene or a KRas mRNA) or protein (e.g., a KRas protein) sequence that is typically found in a subject that does not have a disease or disorder related to the reference nucleic acid or protein. Although a wild type nucleic acid or protein sequence is the sequence that is typically found in a subject that does not have a disease or disorder related to the reference nucleic acid or protein, it is not necessarily the case that a subject that has a disease or disorder related to the reference nucleic acid or protein lacks wild type sequence. For example, a subject with a gene duplication of the reference gene may have the wild type sequence but could still have a disease or disorder related to the reference nucleic acid or protein due to the duplication event. As another example, a subject with a disease or disorder related to the reference nucleic acid or protein may have one allele that encodes wild type protein, and another allele that encodes a mutant protein.

The term "wild type KRas" or "wild-type KRas" describes a KRas nucleic acid (e.g., a *KRAS* gene or a KRas mRNA) or protein (e.g., a KRas protein) that is found in a subject that does not have a KRas-associated disease, e.g., a KRas-associated cancer (and optionally also does not have an increased risk of developing a KRas-associated disease and/or is not suspected of having a KRas-associated disease), or is found in a cell or tissue from a subject that does not have a KRas-associated disease, e.g., a KRas-associated cancer (and optionally also does not have an increased risk of developing a KRas-associated disease and/or is not suspected of having a KRas-associated disease). Although a wild type KRas nucleic acid or protein sequence is the sequence that is typically found in a subject that does not have a KRas-associated disease or disorder, it is not necessarily the case that a subject that has a KRas-associated disease or disorder lacks the wild type KRas sequence. For example, a subject with a KRas gene duplication may have the wild type sequence but could still have a KRas-associated disease or disorder due to the duplication event. As another example, a subject with a KRas-associated disease or disorder may have one allele that encodes wild type KRas protein, and another allele that encodes a mutant KRas protein.

As used herein, terms "treat" or "treatment" refer to therapeutic or palliative measures. Beneficial or desired clinical results include, but are not limited to, alleviation, in whole or in part, of symptoms associated with a disease or disorder or condition, diminishment of the extent of disease, stabilized (i.e., not worsening) state of disease, delay or slowing of disease progression, amelioration or palliation of the disease state (e.g., one or more symptoms of the disease), and remission (whether partial or total), whether detectable or undetectable. "Treatment" can also mean prolonging survival as compared to expected survival if not receiving treatment. As used herein, treatment of a cancer can include treatment of a primary tumor (i.e., non-metastatic cancer) (e.g., as first, second, third, or later line of therapy, including, but not limited to, the relapsed/refractory setting), treatment of a metastatic (or secondary) tumor, neoadjuvant therapy (e.g., before treatment with an additional therapy or therapeutic agent, such as surgery, radiation, chemotherapy, or a line of therapy), adjuvant therapy (e.g., following treatment with an additional therapy or therapeutic agent, such as surgery, radiation, chemotherapy, or a line of therapy), or maintenance therapy (e.g., treatment following response to an additional therapy or therapeutic agent, such as surgery, radiation, chemotherapy, or a line of therapy).

As used herein, the terms "subject," "individual," or "patient," are used interchangeably and refer to any animal, including mammals such as mice, rats, other rodents, rabbits, dogs, cats, swine, cattle, sheep, horses, primates, and humans. In some embodiments, the subject is a human. In some embodiments, the subject has experienced and/or exhibited at least one symptom of the disease or disorder to be treated and/or prevented.

In some embodiments, the subject is a pediatric subject.

The term "pediatric subject" as used herein refers to a subject under the age of 21 years at the time of diagnosis or treatment. The term "pediatric" can be further be divided into various subpopulations including: neonates (from birth through the first month of life); infants (1 month up to two years of age); children (two years of age up to 12 years of age); and adolescents (12 years of age through 21 years of age (up to, but not including, the twenty-second birthday)). Berhman RE, Kliegman R, Arvin AM, Nelson WE. Nelson Textbook of Pediatrics, 15th Ed. Philadelphia: W.B. Saunders Company, 1996; Rudolph AM, et al. Rudolph's Pediatrics, 21st Ed. New York: McGraw-Hill, 2002; and Avery MD, First LR. Pediatric Medicine, 2nd Ed. Baltimore: Williams & Wilkins; 1994. In some embodiments, a pediatric subject is from birth through the first 28 days of life, from 29 days of age to less than two years of age, from two years of age to less than 12 years of age, or 12 years of age through 21 years of age (up to, but not including, the twenty-second birthday). In some embodiments, a pediatric subject is from birth through the first 28 days of life, from 29 days of age to less than 1 year of age, from one month of age to less than four months of age, from three months of age to less than seven months of age, from six months of age to less than 1 year of age, from 1 year of age to less than 2 years of age, from 2 years of age to less than 3 years of age, from 2 years of age to less than seven years of age, from 3 years of age to less than 5 years of age, from 5 years of age to less than 10 years of age, from 6 years of age to less than 13 years of age, from 10 years of age to less than 15 years of age, or from 15 years of age to less than 22 years of age.

The term "activating mutation" in reference to KRas describes a mutation in a *KRas* gene that results in the expression of a KRas protein that has decreased GTPase activity and/or increased effector activation activity, e.g., as compared to a wild type KRas protein, e.g., when assayed under identical conditions. For example, an activating mutation can be a mutation in a *KRAS* gene that results in the expression of a KRas protein that has one or more (e.g., two, three, four, five, six, seven, eight, nine, or ten) amino acid substitutions (e.g., any combination of any of the amino acid substitutions described herein) that has decreased GTPase activity, e.g., as compared to a wild type KRas protein, e.g., when assayed under identical conditions. In another example, an activating mutation can be a mutation in a *KRAS* gene that results in the expression of a KRas protein that has one or more (e.g., two, three, four, five, six, seven, eight, nine, or ten) amino acids deleted, e.g., as compared to a wild type KRas protein, e.g., when assayed under identical conditions. In another example, an activating mutation can be a mutation in a *KRAS* gene that results in the expression of a KRas protein that has at least one (e.g., at least 2, at least 3, at least 4, at least 5, at least 6, at least 7, at least 8, at least 9, at least 10, at least 12, at least 14, at least 16, at least 18, or at least 20) amino acid inserted as compared to a wild type KRas protein, e.g., the exemplary wild type KRas protein described herein, e.g., when assayed under identical conditions. Additional examples of activating mutations are known in the art.

The term "preventing" as used herein means to delay the onset, recurrence or spread, in whole or in part, of the disease or condition as described herein, or a symptom thereof.

The term "regulatory agency" refers to a country's agency for the approval of the medical use of pharmaceutical agents with the country. For example, a non-limiting example of a regulatory agency is the U.S. Food and Drug Administration (FDA).

The phrase "therapeutically effective amount" means an amount of compound that, when administered to a subject in need of such treatment, is sufficient to (i) treat a KRas-associated disease or disorder (e.g., a mutant KRas-associated disease or disorder (e.g., a KRas G12D-associated cancer, a KRas G12R-associated cancer, or a KRas G12V-associated cancer (e.g., a KRas G12D-associated cancer or a KRas G12V-associated cancer)), (ii) attenuate, ameliorate, or eliminate one or more symptoms of the particular disease, disorder, or condition, or (iii) delay the onset of one or more symptoms of the particular disease, disorder, or condition described herein. The amount of a compound provided herein, or a pharmaceutically acceptable salt thereof, that will correspond to such an amount will vary depending upon factors such as the particular compound, disease condition and its severity, the identity (e.g., weight) of the subject in need of treatment, but can nevertheless be routinely determined by one skilled in the art.

As used herein, an "effective amount" refers to an amount of the compound sufficient to effect a beneficial or desired result. For example, an "effective amount" as used herein can refer to an amount of the compound sufficient to modulate (e.g., increase or decrease) (1) activity or amount of a protein; (2) proliferation of a cell (e.g., a cancer cell); and/or (3) one or more cellular signaling pathways associated with a protein's activity.

### Pharmaceutical Compositions and Administration

### General

In some embodiments, a compound provided herein, or a pharmaceutically acceptable salt thereof, is administered as a pharmaceutical composition that includes the compound, or a pharmaceutically acceptable salt thereof, and one or more pharmaceutically acceptable excipients, and optionally one or more additional therapeutic agents as described herein.

In some embodiments, the compounds provided herein, or pharmaceutically acceptable salts thereof, can be administered in combination with one or more conventional pharmaceutical excipients. Pharmaceutically acceptable excipients include, but are not limited to, ion exchangers, alumina, aluminum stearate, lecithin, self-emulsifying drug delivery systems (SEDDS) such as d-α-tocopherol polyethylene glycol 1000 succinate, surfactants used in pharmaceutical dosage forms such as Tweens, poloxamers or other similar polymeric delivery matrices, serum proteins, such as human serum albumin, buffer substances such as phosphates, tris, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium-chloride, zinc salts, colloidal silica, magnesium trisilicate, polyvinyl pyrrolidone, cellulose-based substances, polyethylene glycol, sodium carboxymethyl cellulose, polyacrylates, waxes, polyethylene-polyoxypropylene-block polymers, and wool fat. Cyclodextrins such as α-, β-, and γ-cyclodextrin, or chemically modified derivatives such as hydroxyalkylcyclodextrins, including 2- and 3-hydroxypropyl-β-cyclodextrins, or other solubilized derivatives can also be used to enhance delivery of compounds described herein. Dosage forms or compositions containing a compound provided herein, or a pharmaceutically acceptable salt thereof, as described herein in the range of 0.005% to 100% with the balance made up from non-toxic excipient may be prepared. The contemplated compositions may contain 0.001%-100% of a compound provided herein, or a pharmaceutically acceptable salt thereof, provided herein, in one embodiment 0.1-95%, in another embodiment 75-85%, in a further embodiment 20-80%. Actual methods of preparing such dosage forms are known, or will be apparent, to those skilled in this art; for example, see Remington: The Science and Practice of Pharmacy, 22nd Edition (Pharmaceutical Press, London, UK. 2012).

### Routes of Administration and Composition Components

In some embodiments, the compounds provided herein, or pharmaceutically acceptable salts thereof, or a pharmaceutical composition comprising a compound provided herein, or a pharmaceutically acceptable salt thereof, can be administered to a subject in need thereof by any accepted route of administration. Acceptable routes of administration include, but are not limited to, buccal, cutaneous, endocervical, endosinusial, endotracheal, enteral, epidural, interstitial, intra-abdominal, intra-arterial, intrabronchial, intrabursal, intracerebral, intracisternal, intracoronary, intradermal, intraductal, intraduodenal, intradural, intraepidermal, intraesophageal, intragastric, intragingival, intraileal, intralymphatic, intramedullary, intrameningeal, intramuscular, intraovarian, intraperitoneal, intraprostatic, intrapulmonary, intrasinal, intraspinal, intrasynovial, intratesticular, intrathecal, intratubular, intratumoral, intrauterine, intravascular, intravenous, nasal, nasogastric, oral, parenteral, percutaneous, peridural, rectal, respiratory (inhalation), subcutaneous, sublingual, submucosal, topical, transdermal, transmucosal, transtracheal, ureteral, urethral and vaginal. In certain embodiments, a preferred route of administration is parenteral (e.g., intratumoral).

In some embodiments, a compound provided herein, or a pharmaceutically acceptable salt thereof, as described herein, or a pharmaceutical composition thereof, can be administered orally to a subject in need thereof. Without being bound by any particular theory, it is believed that oral dosing (e.g., versus IV dosing) can be preferred by patients for convenience, perception of efficacy, and/or past experience.

Compositions can be formulated for parenteral administration, e.g., formulated for injection via the intravenous, intramuscular, sub-cutaneous, or even intraperitoneal routes. Typically, such compositions can be prepared as injectables, either as liquid solutions or suspensions; solid forms suitable for use to prepare solutions or suspensions upon the addition of a liquid prior to injection can also be prepared; and the preparations can also be emulsified. The preparation of such formulations will be known to those of skill in the art in light of the present disclosure.

The pharmaceutical forms suitable for injectable use include sterile aqueous solutions or dispersions; formulations including sesame oil, peanut oil, or aqueous propylene glycol; and sterile powders for the extemporaneous preparation of sterile injectable solutions or dispersions. In all cases the form must be sterile and must be fluid to the extent that it may be easily injected. It also should be stable under the conditions of manufacture and storage and must be preserved against the contaminating action of microorganisms, such as bacteria and fungi.

The carrier also can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion, and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques, which yield a powder of the active ingredient, plus any additional desired ingredient from a previously sterile-filtered solution thereof.

Intratumoral injections are discussed, e.g., in Lammers, et al., "Effect of Intratumoral Injection on the Biodistribution and the Therapeutic Potential of HPMA Copolymer-Based Drug Delivery Systems" Neoplasia. 2006, 10, 788-795, doi: 10.1593/neo.06436.

Pharmacologically acceptable excipients usable in the rectal composition as a gel, cream, enema, or rectal suppository, include, without limitation, any one or more of cocoa butter glycerides, synthetic polymers such as polyvinylpyrrolidone, PEG (like PEG ointments), glycerine, glycerinated gelatin, hydrogenated vegetable oils, poloxamers, mixtures of polyethylene glycols of various molecular weights and fatty acid esters of polyethylene glycol Vaseline, anhydrous lanolin, shark liver oil, sodium saccharinate, menthol, sweet almond oil, sorbitol, sodium benzoate, anoxid SBN, vanilla essential oil, aerosol, parabens in phenoxyethanol, sodium methyl p-oxybenzoate, sodium propyl p-oxybenzoate, diethylamine, carbomers, carbopol, methyloxybenzoate, macrogol cetostearyl ether, cocoyl caprylocaprate, isopropyl alcohol, propylene glycol, liquid paraffin, xanthan gum, carboxy-metabisulfite, sodium edetate, sodium benzoate, potassium metabisulfite, grapefruit seed extract, methyl sulfonyl methane (MSM) , lactic acid, glycine, vitamins, such as vitamin A and E and potassium acetate.

In certain embodiments, suppositories can be prepared by mixing a compound provided herein, or a pharmaceutically acceptable salt thereof, with suitable non-irritating excipients or carriers such as cocoa butter, polyethylene glycol or a suppository wax which are solid at ambient temperature but liquid at body temperature and therefore melt in the rectum and release the active compound. In other embodiments, compositions for rectal administration are in the form of an enema.

In other embodiments, the compounds described herein, or a pharmaceutical composition thereof, are suitable for local delivery to the digestive or GI tract by way of oral administration (e.g., solid or liquid dosage forms.).

Solid dosage forms for oral administration include capsules, tablets, pills, powders, and granules. In such solid dosage forms, the compound provided herein, or a pharmaceutically acceptable salt thereof, is mixed with one or more pharmaceutically acceptable excipients, such as sodium citrate or dicalcium phosphate and/or: a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents. Solid compositions of a similar type may also be employed as fillers in soft and hard-filled gelatin capsules using such excipients as lactose or milk sugar as well as high molecular weight polyethylene glycols and the like.

In one embodiment, the compositions will take the form of a unit dosage form such as a pill or tablet and thus the composition may contain, along with a compound provided herein, or a pharmaceutically acceptable salt thereof, provided herein, a diluent such as lactose, sucrose, dicalcium phosphate, or the like; a lubricant such as magnesium stearate or the like; and a binder such as starch, gum acacia, polyvinylpyrrolidine, gelatin, cellulose, cellulose derivatives, or the like. In another solid dosage form, a powder, marume, solution or suspension (e.g., in propylene carbonate, vegetable oils, PEGs, poloxamer 124 or triglycerides) is encapsulated in a capsule (gelatin or cellulose base capsule). Unit dosage forms in which one or more compounds provided herein, or pharmaceutically acceptable salts thereof, provided herein or additional active agents are physically separated are also contemplated; e.g., capsules with granules (or tablets in a capsule) of each drug; two-layer tablets; two-compartment gel caps, etc. Enteric coated or delayed release oral dosage forms are also contemplated.

Other physiologically acceptable compounds include wetting agents, emulsifying agents, dispersing agents or preservatives that are particularly useful for preventing the growth or action of microorganisms. Various preservatives are well known and include, for example, phenol and ascorbic acid.

In certain embodiments the excipients are sterile and generally free of undesirable matter. These compositions can be sterilized by conventional, well-known sterilization techniques. For various oral dosage form excipients, such as tablets and capsules, sterility is not required. The USP/NF standard is usually sufficient.

In certain embodiments, solid oral dosage forms can further include one or more components that chemically and/or structurally predispose the composition for delivery of the compound provided herein, or a pharmaceutically acceptable salt thereof, to the stomach or the lower GI; e.g., the ascending colon and/or transverse colon and/or distal colon and/or small bowel. Exemplary formulation techniques are described in, e.g., Filipski, K.J., et al., Current Topics in Medicinal Chemistry, 2013, 13, 776-802, doi: 10.2174/1568026611313070002.

Examples include upper-GI targeting techniques, e.g., Accordion Pill (Intec Pharma), floating capsules, and materials capable of adhering to mucosal walls.

Other examples include lower-GI targeting techniques. For targeting various regions in the intestinal tract, several enteric/pH-responsive coatings and excipients are available. These materials are typically polymers that are designed to dissolve or erode at specific pH ranges, selected based upon the GI region of desired drug release. These materials also function to protect acid labile drugs from gastric fluid or limit exposure in cases where the active ingredient may be irritating to the upper GI (e.g., hydroxypropyl methylcellulose phthalate series, Coateric (polyvinyl acetate phthalate), cellulose acetate phthalate, hydroxypropyl methylcellulose acetate succinate, Eudragit series (methacrylic acid-methyl methacrylate copolymers), and Marcoat). Other techniques include dosage forms that respond to local flora in the GI tract, Pressure-controlled colon delivery capsule, and Pulsincap.

Ocular compositions can include, without limitation, one or more of any of the following: viscogens (e.g., Carboxymethylcellulose, Glycerin, Polyvinylpyrrolidone, Polyethylene glycol); Stabilizers (e.g., Pluronic (triblock copolymers), Cyclodextrins); Preservatives (e.g., Benzalkonium chloride, ETDA, SofZia (boric acid, propylene glycol, sorbitol, and zinc chloride; Alcon Laboratories, Inc.), Purite (stabilized oxychloro complex; Allergan, Inc.)).

Topical compositions can include ointments and creams. Ointments are semisolid preparations that are typically based on petrolatum or other petroleum derivatives. Creams containing the selected active agent are typically viscous liquid or semisolid emulsions, often either oil-in-water or water-in-oil. Cream bases are typically water-washable, and contain an oil phase, an emulsifier, and an aqueous phase. The oil phase, also sometimes called the "internal" phase, is generally comprised of petrolatum and a fatty alcohol such as cetyl or stearyl alcohol; the aqueous phase usually, although not necessarily, exceeds the oil phase in volume, and generally contains a humectant. The emulsifier in a cream formulation is generally a nonionic, anionic, cationic, or amphoteric surfactant. As with other carriers or vehicles, an ointment base should be inert, stable, nonirritating, and non-sensitizing.

In any of the foregoing embodiments, pharmaceutical compositions described herein can include one or more one or more of the following: lipids, interbilayer crosslinked multilamellar vesicles, biodegradable poly(D,L-lactic-co-glycolic acid) [PLGA]-based or poly anhydride-based nanoparticles or microparticles, and nanoporous particle-supported lipid bilayers.

### Dosages

The dosages may be varied depending on the requirement of the patient, the severity of the condition being treated, and the particular compound being employed. Determination of the proper dosage for a particular situation can be determined by one skilled in the medical arts. The total daily dosage may be divided and administered in portions throughout the day or by means providing continuous delivery.

In some embodiments, the compounds described herein are administered at a dosage of from about 0.001 mg/kg to about 500 mg/kg (e.g., from about 0.001 mg/kg to about 200 mg/kg; from about 0.01 mg/kg to about 200 mg/kg; from about 0.01 mg/kg to about 150 mg/kg; from about 0.01 mg/kg to about 100 mg/kg; from about 0.01 mg/kg to about 50 mg/kg; from about 0.01 mg/kg to about 10 mg/kg; from about 0.01 mg/kg to about 5 mg/kg; from about 0.01 mg/kg to about 1 mg/kg; from about 0.01 mg/kg to about 0.5 mg/kg; from about 0.01 mg/kg to about 0.1 mg/kg; from about 0.1 mg/kg to about 200 mg/kg; from about 0.1 mg/kg to about 150 mg/kg; from about 0.1 mg/kg to about 100 mg/kg; from about 0.1 mg/kg to about 50 mg/kg; from about 0.1 mg/kg to about 10 mg/kg; from about 0.1 mg/kg to about 5 mg/kg; from about 0.1 mg/kg to about 1 mg/kg; from about 0.1 mg/kg to about 0.5 mg/kg).

### Regimens

The foregoing dosages can be administered on a daily basis (e.g., as a single dose or as two or more divided doses) or non-daily basis (e.g., every other day, every two days, every three days, once weekly, twice weeks, once every two weeks, once a month).

In some embodiments, the period of administration of a compound described herein is for 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 11 months, 12 months, or more. In a further embodiment, a period of during which administration is stopped is for 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 11 months, 12 months, or more. In an embodiment, a therapeutic compound is administered to an individual for a period of time followed by a separate period of time. In another embodiment, a therapeutic compound is administered for a first period and a second period following the first period, with administration stopped during the second period, followed by a third period where administration of the therapeutic compound is started and then a fourth period following the third period where administration is stopped. In an aspect of this embodiment, the period of administration of a therapeutic compound followed by a period where administration is stopped is repeated for a determined or undetermined period of time. In a further embodiment, a period of administration is for 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 11 months, 12 months, or more. In a further embodiment, a period of during which administration is stopped is for 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 11 months, 12 months, or more.

The term "acceptable" with respect to a formulation, composition, or ingredient, as used herein, means having no persistent detrimental effect on the general health of the subject being treated.

"API" refers to an active pharmaceutical ingredient.

The term "excipient" or "pharmaceutically acceptable excipient" means a pharmaceutically acceptable material, composition, or vehicle, such as a liquid or solid filler, diluent, carrier, solvent, or encapsulating material. In one embodiment, each component is "pharmaceutically acceptable" in the sense of being compatible with the other ingredients of a pharmaceutical formulation, and suitable for use in contact with the tissue or organ of humans and animals without excessive toxicity, irritation, allergic response, immunogenicity, or other problems or complications, commensurate with a reasonable benefit/risk ratio. *See, e.g.,* Remington: The Science and Practice of Pharmacy, 21st ed.; Lippincott Williams & Wilkins: Philadelphia, PA, 2005; Handbook of Pharmaceutical Excipients, 6th ed.; Rowe et al., Eds.; The Pharmaceutical Press and the American Pharmaceutical Association: 2009; Handbook of Pharmaceutical Additives, 3rd ed.; Ash and Ash Eds.; Gower Publishing Company: 2007; Pharmaceutical Preformulation and Formulation, 2nd ed.; Gibson Ed.; CRC Press LLC: Boca Raton, FL, 2009.

The term "pharmaceutically acceptable salt" refers to a formulation of a compound that does not cause significant irritation to an organism to which it is administered and does not abrogate the biological activity and properties of the compound. In certain instances, pharmaceutically acceptable salts are obtained by reacting a compound described herein, with acids such as hydrochloric acid, hydrobromic acid, sulfuric acid, nitric acid, phosphoric acid, methanesulfonic acid, ethanesulfonic acid, p-toluenesulfonic acid, salicylic acid, and the like. In some instances, pharmaceutically acceptable salts are obtained by reacting a compound having acidic group described herein with a base to form a salt such as an ammonium salt, an alkali metal salt, such as a sodium or a potassium salt, an alkaline earth metal salt, such as a calcium or a magnesium salt, a salt of organic bases such as dicyclohexylamine, N-methyl-D-glucamine, tris(hydroxymethyl)methylamine, and salts with amino acids such as arginine, lysine, and the like, or by other methods previously determined. The term "pharmacologically acceptable salts" is not specifically limited as far as it can be used in medicaments. Examples of a salt that the compounds described herein form with a base include the following: salts thereof with inorganic bases such as sodium, potassium, magnesium, calcium, and aluminum; salts thereof with organic bases such as methylamine, ethylamine, and ethanolamine; salts thereof with basic amino acids such as lysine and ornithine; and ammonium salt. The salts may be acid addition salts, which are specifically exemplified by acid addition salts with the following: mineral acids such as hydrochloric acid, hydrobromic acid, hydroiodic acid, sulfuric acid, nitric acid, and phosphoric acid; organic acids such as formic acid, acetic acid, propionic acid, oxalic acid, malonic acid, succinic acid, fumaric acid, maleic acid, lactic acid, malic acid, tartaric acid, citric acid, methanesulfonic acid, and ethanesulfonic acid; acidic amino acids such as aspartic acid and glutamic acid.

In some embodiments, a pharmaceutically acceptable salt is an anion salt selected from the group consisting of: a mesylate salt, a besylate salt, an acetate salt, a benzenesulfonate salt, a benzoate salt, a bicarbonate salt, a bitartrate salt, a bromide salt, a calcium edetate salt, a camsylate salt, a carbonate salt, a chloride salt, a citrate salt, a dihydrochloride salt, an edetate salt, an edisylate salt, an estolate salt, an esylate salt, a fumarate salt, a gluceptate salt, a gluconate salt, a glucuronate salt, a glutamate salt, a glycollylarsanilate salt, a hexylresorcinol salt, a hydramine salt, a hydrobromide salt, a hydrochloride salt, a hydroxynaphthoate salt, an iodide salt, an isethionate salt, a lactate salt, a lactobionate salt, a malate salt, a maleate salt, a mandelate salt, a mesylate salt, a methylbromide salt, a methylnitrate salt, a methylsulfate salt, a mucate salt, a napsylate salt, a nitrate salt, a pamoate (embonate) salt, a pantothenate salt, a phosphate/diphosphate salt, a polygalacturonate salt, a salicylate salt, a stearate salt, a subacetate salt, a succinate salt, a sulfate salt, an oleate salt, a tannate salt, a tartrate salt, a teoclate salt, and a triethiodide salt.

In some embodiments, a pharmaceutically acceptable salt is a cation salt selected from the group consisting of: a benzathine salt, a chloroprocaine salt, a choline salt, a tromethamine salt, a diethanolamine salt, an ethylenediamine salt, a meglumine salt, a procaine salt, an aluminum salt, a calcium salt, a lithium salt, a magnesium salt, a potassium salt, a sodium salt, and a zinc salt.

Examples of pharmaceutically acceptable salts also include those disclosed in e.g., Berge, Stephen M., Lyle D. Bighley, and Donald C. Monkhouse. "Pharmaceutical salts." Journal of pharmaceutical sciences 66.1 (1977): 1-19 doi: 10.1002/jps.2600660104, Bharate, Sonali S. "Recent developments in pharmaceutical salts: FDA approvals from 2015 to 2019." Drug Discovery Today 26.2 (2021): 384-398 doi: 10.1016/j.drudis.2020.11.016, and Bharate, Sonali S. "Modulation of biopharmaceutical properties of drugs using sulfonate counterions: A critical analysis of FDA-approved pharmaceutical salts." Journal of Drug Delivery Science and Technology 66 (2021): 102913 doi: 10.1016/j.jddst.2021.102913.

The term "pharmaceutical composition" refers to a mixture of a compound described herein with other chemical components (referred to collectively herein as "excipients"), such as carriers, stabilizers, diluents, dispersing agents, suspending agents, and/or thickening agents. The pharmaceutical composition facilitates administration of the compound to a subject. Multiple techniques of administering a compound exist in the art including, but not limited to: rectal, oral, intravenous, aerosol, parenteral, ophthalmic, pulmonary, and topical administration.

### Compound Preparation

The compounds disclosed herein can be prepared in a variety of ways using commercially available starting materials, compounds known in the literature, or from readily prepared intermediates, by employing standard synthetic methods and procedures either known to those skilled in the art, or in light of the teachings herein.

Standard synthetic methods and procedures for the preparation of organic molecules and functional group transformations and manipulations can be obtained from the relevant scientific literature or from standard textbooks in the field. Although not limited to any one or several sources, classic texts such as R. Larock, Comprehensive Organic Transformations, VCH Publishers (1989); L. Fieser and M. Fieser, Fieser and Fieser's Reagents for Organic Synthesis, John Wiley and Sons (1994); Smith, M. B., March, J., March' s Advanced Organic Chemistry: Reactions, Mechanisms, and Structure, 5th edition, John Wiley & Sons: New York, 2001 ; and Greene, T.W., Wuts, P.G. M., Protective Groups in Organic Synthesis, 3rd edition, John Wiley & Sons: New York, 1999, are useful and recognized reference textbooks of organic synthesis known to those in the art. The following descriptions of synthetic methods are designed to illustrate, but not to limit, general procedures for the preparation of compounds of the present disclosure.

The synthetic processes disclosed herein can tolerate a wide variety of functional groups; therefore, various substituted starting materials can be used. The processes generally provide the desired final compound at or near the end of the overall process, although it may be desirable in certain instances to further convert the compound to a pharmaceutically acceptable salt thereof.

Also provided herein are compounds useful (e.g., as intermediates) in the preparation of compounds provided herein.

Provided herein are compounds of Formula **(SVI-1):** 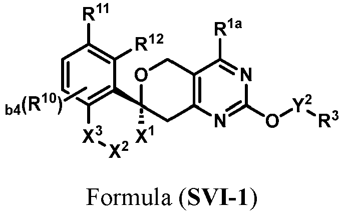 or salts thereof, wherein:
**R¹¹** is selected from the group consisting of: NH₂, NH**Pg**, and N(**Pg**)₂;
each **Pg** is an independently selected nitrogen protecting group;
**R¹²** is selected from the group consisting of: halo (e.g., -Br) and CN;
**b4** is 0 or 1;
**R¹⁰** is selected from the group consisting of: -Cl, -F, -CN, and C₁₋₃ alkyl optionally substituted with 1-3 **R^{c}**;
**X¹** is selected from the group consisting of a bond, S(O)₀₋₂, CH₂, CH**R^{L}**, C(**R^{L}**)₂, and
   O;
**X²** and **X³** are independently selected from the group consisting of: CH₂, CH**R^{L}**, C(**R^{L}**)₂, O, and S(O)₀₋₂, provided that no more than one of **X¹**, **X²**, and **X³** is selected from the group consisting of: O and S(O)₀₋₂;
each **R^{L}** is independently selected from the group consisting of C₁₋₃ alkoxy, -F, CN, and C₁₋₃ alkyl optionally substituted with 1-3 **R^{c}**; or
one pair of **R^{L}** on the same or different ring carbon atom(s) taken together with the ring atom(s) connecting them form a C₃₋₆ cycloalkyl ring;
**R^{1a}** is -Cl or -OH;
**Y²** is a bond or a straight-chain C₁₋₆ alkylene optionally substituted with 1-6 **R^{Y}**;
each **R^{Y}** is independently selected from the group consisting of: halo, cyano, -OH, oxo, C₁₋₆ alkoxy, C₁₋₆ haloalkoxy, C₁₋₆ alkyl, and C₁₋₆ haloalkyl, or
one pair of **R^{Y}** on the same or different carbon atom(s) taken together with the atom(s) connecting them forms a C₃₋₆ cycloalkyl ring or 4-6 membered heterocyclyl ring, each of which is optionally substituted with 1-3 independently selected C₁₋₃ alkyl;
**R³** is selected from the group consisting of:
   **(a)** a 4-15 membered heterocyclyl optionally substituted with 1-6 substituents independently selected from the group consisting of: **R^{a}** and **R^{b}**; and
   **(b)** -**NR^{d}R^{e}**;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   **(f)** -C₁₋₆ haloalkoxy;
   **(g)** -N**R^{d}R^{e}**;
   **(h)** C(=O)C₁₋₆ alkyl;
   **(i)** C(=O)C₁₋₆ haloalkyl;
   **(j)** C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   **(o)** S(O)₀₋₂(C₁₋₆ haloalkyl);
   **(p)** S(O)_{1**-**2}N(**R^{f}**)₂; and
   **(q)** C₁₋₆ alkyl, C₂₋₆ alkenyl, or C₂₋₆ alkynyl, each optionally substituted with 1-6 **R^{c};**
each **R^{b}** is independently selected from the group consisting of: -(**L^{b})_{b}**-**R**^{**b**1} and -**R^{b1}**, wherein:
   **b** is 1, 2, or 3;
   each -**L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e}**, C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)_{1**-**2}N(**R^{f}**)₂;
   each **R^{d}** and **R^{e}** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂₋.

In some embodiments of Formula **(SVI-1), R¹¹** is NH₂.

In some embodiments of Formula **(SVI-1), R¹¹** is NH**Pg** or N(**Pg**)₂, wherein each **Pg** is an independently selected nitrogen protecting group. Representative examples of nitrogen protecting groups include: acetyl (Ac), benzoyl (Bz), benzyl (Bn), benzyloxycarbonyl (Cbz), formyl, phenylsulfonyl, pivaloyl, tert-butoxycarbonyl (Boc), *tert-butyl* acetyl, ethyloxycarbonyl, trifluoroacetyl, triphenylmethyl (trityl), and triisopropylsilane. *See:* T. W. Greene and P. G. M. Wuts in "Protective groups in organic chemistry" John Wiley and Sons, 4th Edition, 2006.

In some embodiments of Formula **(SVI-1), R¹¹** is N(**Pg**)₂. For example, **R¹¹** can be N(Bn)₂. For example, **R¹¹** can be N(Boc)₂.

In some embodiments of Formula **(SVI-1), R¹²** is -Br.

In some embodiments of Formula **(SVI-1), R¹²** is -CN.

In some embodiments of Formula **(SVI-1),** the 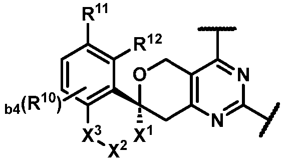 moiety is 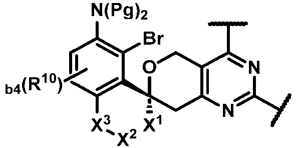 (e.g., 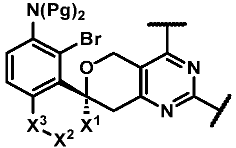 ) or 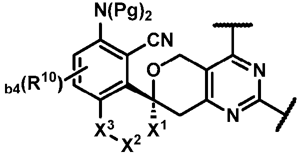 (e.g., 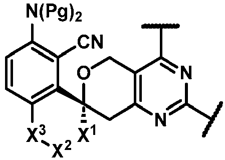 ).

In some embodiments of Formula **(SVI-1),** the 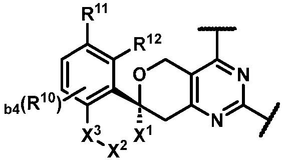 moiety is 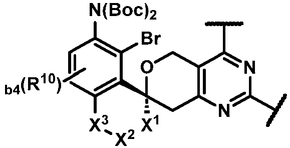 (e.g., 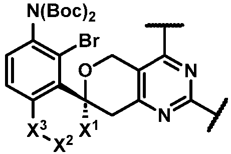 ) or 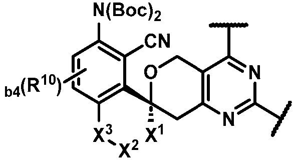 (e.g., 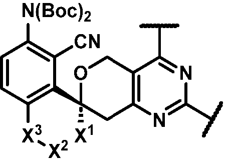 ).

In some embodiments of Formula **(SVI-1),** the 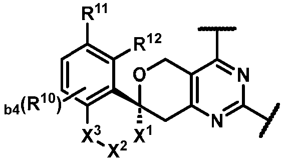 moiety is 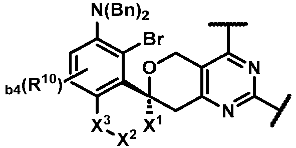 (e.g., 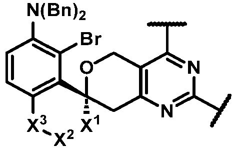 ) or 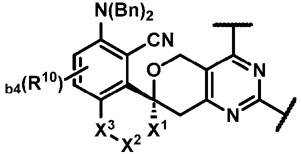 (e.g., 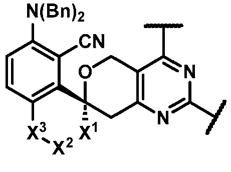 ).

In some embodiments of Formula **(SVI-1),** the 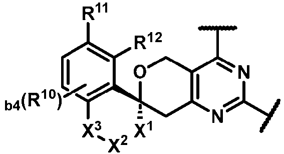 moiety is 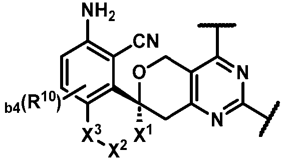 (e.g., 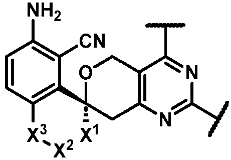 ).

In some embodiments of Formula **(SVI-1), X¹** is CH₂; **X²** is CH₂; and **X³** is CH**R^{L}** or CH₂ (e.g., CHMe or CH₂).

In some embodiments of Formula **(SVI-1),** the 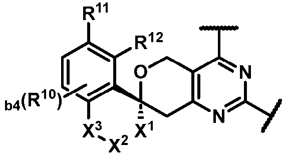 moiety is 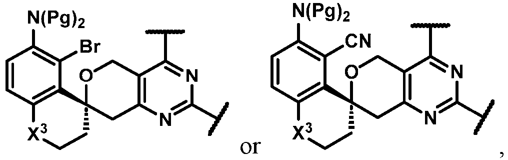 wherein **X³** is CH₂ or CH**R^{L}**. For example, **X³** can be CH₂. For example, **X³** can be CHMe.

In some embodiments of Formula (SVI-1), the 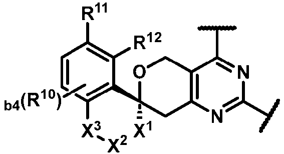 moiety is 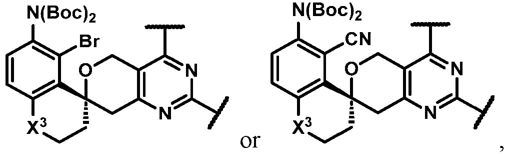 wherein **X³** is CH₂ or CH**R^{L}**. For example, **X³** can be CH₂. For example, **X³** can be CHMe.

In some embodiments of Formula (SVI-1), the 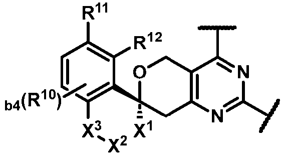 moiety is 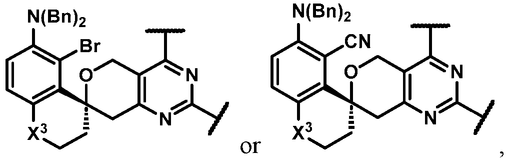 wherein **X³** is CH₂ or CH**R^{L}**. For example, **X³** can be CH₂. For example, **X³** can be CHMe.

In some embodiments of Formula (SVI-1), the 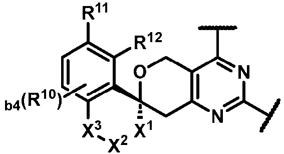 moiety is 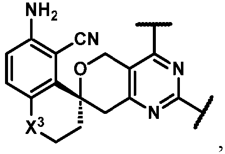 wherein **X³** is CH₂ or CH**R^{L}**. For example, **X³** can be CH₂. For example, **X³** can be CHMe.

In some embodiments of Formula **(SVI-1), R^{1a}** is -Cl.

In some embodiments of Formula **(SVI-1), R^{1a}** is OH.

In some embodiments of Formula **(SVI-1), Y²** is -CH₂-; and **R³** is 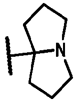 optionally substituted with 1-2 substituents each independently selected from the group consisting of: -F, -C₁₋₃ alkoxy, and -C₁₋₃ haloalkoxy. For example, **R³** can be 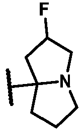 (e.g., 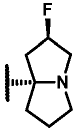 ). For example, **R³** can be 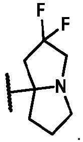 For example, **R³** can be

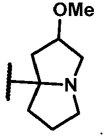

Also provided herein are compounds of Formula (SVI-2) 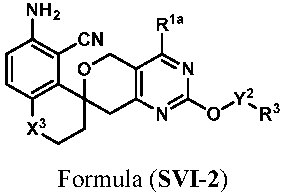 or salts thereof, wherein:
**X³** is CH**R^{L}** or C(**R^{L}**)₂;
each **R^{L}** is independently selected from the group consisting of C₁₋₃ alkoxy, -F, CN, and C₁₋₃ alkyl optionally substituted with 1-3 **R^{c}**; or
one pair of **R^{L}** on the same ring carbon atom taken together with the ring atom connecting them form a C₃₋₆ cycloalkyl ring;
**R^{1a}** is -Cl or -O^{H};
**Y²** is a bond or a straight-chain C₁₋₆ alkylene optionally substituted with 1-6 **R^{Y}**;
each **R^{Y}** is independently selected from the group consisting of: halo, cyano, -OH, oxo, C₁₋₆ alkoxy, C₁₋₆ haloalkoxy, C₁₋₆ alkyl, and C₁₋₆ haloalkyl, or
one pair of **R^{Y}** on the same or different carbon atom(s) taken together with the atom(s) connecting them forms a C₃₋₆ cycloalkyl ring or 4-6 membered heterocyclyl ring, each of which is optionally substituted with 1-3 independently selected C₁₋₃ alkyl;
**R³** is selected from the group consisting of:
   **(a)** a 4-15 membered heterocyclyl optionally substituted with 1-6 substituents independently selected from the group consisting of: **R^{a}** and **R^{b}**; and
   (**b**) -N**R^{d}R^{e}**;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   **(f)** -C₁₋₆ haloalkoxy;
   **(g)** -N**R^{d}R^{e}**;
   **(h)** C(=O)C₁₋₆ alkyl;
   **(i)** C(=O)C₁₋₆ haloalkyl;
   **(j)** C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   **(o)** S(O)₀₋₂(C₁₋₆ haloalkyl);
   **(p)** S(O)_{1**-**2}N(**R^{f}**)₂; and
   **(q)** C₁₋₆ alkyl, C₂₋₆ alkenyl, or C₂₋₆ alkynyl, each optionally substituted with 1-6 **R^{c}**;
each **R^{b}** is independently selected from the group consisting of: -**(L^{b})_{b}-R^{b1}** and -**R^{b1}**, wherein:
   **b is** 1, 2, or 3;
   each -**L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e}**, C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)_{1**-**2}N(**R^{f}**)₂;
   each **R^{d}** and **R^{e}** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂₋.

In some embodiments of Formula (SVI-2), the 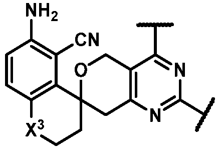 moiety is 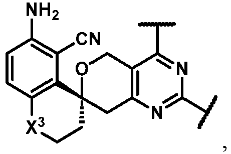 wherein **X³** is CH**R^{L}**. For example, **X³** can be CHMe.

In some embodiments of Formula (SVI-2), **R¹²** is -Cl.

In some embodiments of Formula (SVI-2), **R^{1a}** is OH.

In some embodiments of Formula **(SVI-2), Y²** is -CH₂-; and **R³** is 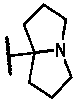 optionally substituted with 1-2 substituents each independently selected from the group consisting of: -F, -C₁₋₃ alkoxy, and -C₁₋₃ haloalkoxy. For example, **R³** can be 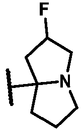 (e.g., 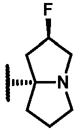 ). For example, **R³** can be 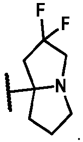 For example, **R³** can be

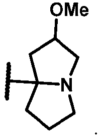

Also provided herein are compounds of Formula **(SIV-II-a),** (**SIII-II-a**), (**SII-II-a)**, or (**SI-II-a)**:

| | |
|---|---|
| 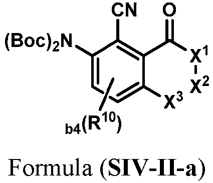 | 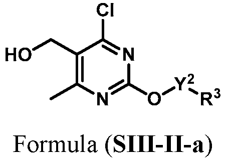 |
| 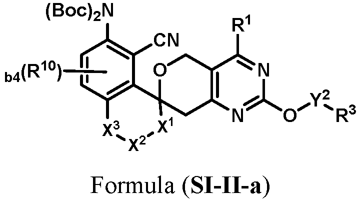 | 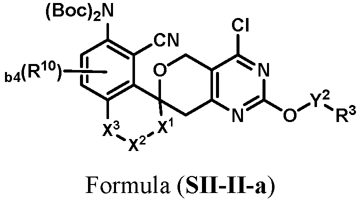 |

or salts thereof, wherein:
**R¹** is selected from the group consisting of:
   **(i)** a 4-10 membered heterocyclyl optionally substituted with 1-4 **R⁷**;
   **(ii)** an 8-12 membered bicyclic heterocyclyl, wherein the heterocyclyl comprises an endocyclic group selected from the group consisting of C(=O)NH and S(O)₂NH, and wherein the heterocyclyl is further optionally substituted with 1-3 **R⁷** at one or more ring carbon atoms; and
   **(iii)** 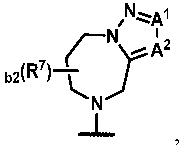 wherein **b2** is 0, 1, 2, or 3; and **A¹** and **A²** are independently selected from the group consisting of: N, CH, and **CR⁷**;
each **R⁷** is independently selected from the group consisting of **R^{a}** and **R^{b}**;
**X¹** is selected from the group consisting of a bond, S(O)₀₋₂, CH₂, CH**R^{L}**, C(**R^{L}**)₂, and O;
**X²** and **X³** are independently selected from the group consisting of: CH₂, CH**R^{L}**, C(**R^{L}**)₂, O, and S(O)₀₋₂, provided that no more than one of **X¹**, **X²**, and **X³** is selected from the group consisting of: O and S(O)₀₋₂;
**b4** is 0 or 1; and
each **R¹⁰** is independently selected from the group consisting of: -Cl, -F, -CN, and C₁₋₃ alkyl optionally substituted with 1-3 **R^{c}**;
each **R^{L}** is independently selected from the group consisting of C₁₋₃ alkoxy, -F, CN, and C₁₋₃ alkyl optionally substituted with 1-3 **R^{c}**; or
one pair of **R^{L}** on the same or different ring carbon atom(s) taken together with the ring atom(s) connecting them form a C₃₋₆ cycloalkyl ring;
**Y²** is a bond or a straight-chain C₁₋₆ alkylene optionally substituted with 1-6 **R^{Y}**;
each **R^{Y}** is independently selected from the group consisting of: halo, cyano, -OH, oxo, C₁₋₆ alkoxy, C₁₋₆ haloalkoxy, C₁₋₆ alkyl, and C₁₋₆ haloalkyl, or
one pair of **R^{Y}** on the same or different carbon atom(s) taken together with the atom(s) connecting them forms a C₃₋₆ cycloalkyl ring or 4-6 membered heterocyclyl ring, each of which is optionally substituted with 1-3 independently selected C₁₋₃ alkyl;
**R³** is selected from the group consisting of:
   **(a)** a 4-15 membered heterocyclyl optionally substituted with 1-6 substituents independently selected from the group consisting of: **R^{a}** and **R^{b}**; and
   **(b)** -N**R^{d}R^{e}**;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   **(f)** -C₁₋₆ haloalkoxy;
   **(g)** -N**R^{d}R^{e}**;
   **(h)** C(=O)C₁₋₆ alkyl;
   **(i)** C(=O)C₁₋₆ haloalkyl;
   **(j)** C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   **(o)** S(O)₀₋₂(C₁₋₆ haloalkyl);
   **(p)** S(O)_{1**-**2}N(**R^{f}**)₂; and
   **(q)** C₁₋₆ alkyl, C₂₋₆ alkenyl, or C₂₋₆ alkynyl, each optionally substituted with 1-6 **R^{c}**;
each **R^{b}** is independently selected from the group consisting of: -(**L^{b}**)**_{b}-R^{b1}** and -**R^{b1}**, wherein:
   **b** is 1, 2, or 3;
   each -**L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e}**, C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)_{1**-**2}N(**R^{f}**)₂;
   each **R^{d}** and **R^{e}** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂₋.

In some embodiments of Formula **(SIV-II-a), (SII-II-a),** or **(SI-II-a), X¹** is CH₂; **X²** is CH₂; and **X³** is CH**R^{L}** or CH₂ (e.g., CHMe or CH₂).

In some embodiments of Formula (**SII-II-a)** or **(SI-II-a),** the 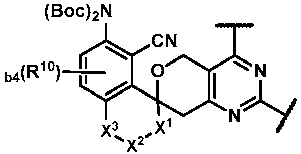 moiety is 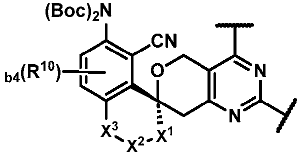 (e.g., 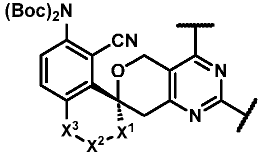 ).

In some embodiments of Formula (**SII-II-a)** or **(SI-II-a),** the 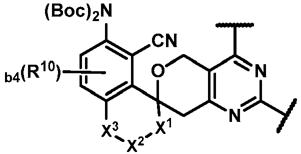 moiety is 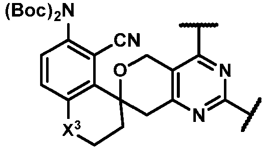 wherein **X³** is CH₂ or CH**R^{L}**. For example, **X³** can be CH₂. For example, **X³** can be CHMe.

In some embodiments of Formula (**SII-II-a)** or **(SI-II-a),** the 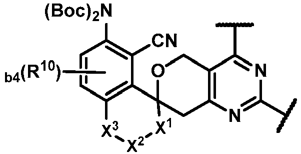 moiety is 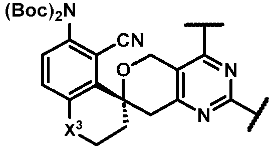 wherein **X³** is CH₂ or CH**R^{L}**. For example, **X³** can be CH₂. For example, **X³** can be CHMe.

In some embodiments of Formula **(SI-II-a), R¹** is a 7-10 (e.g., 7) membered heterocyclyl optionally substituted with 1-4 (e.g., 1-2) **R⁷**. In some embodiments, each **R⁷** is independently selected from the group consisting of: -F, -OH, oxo, -**R^{b1}**, and C₁₋₃ alkyl optionally substituted with 1-3 **R^{c}**, wherein the **R^{b1}** is a 5-6 membered heteroaryl optionally substituted with 1-2 **R^{g}**.

In some embodiments of Formula **(SI-II-a), R¹** is a 7-10 (e.g., 7) membered heterocyclyl having one ring nitrogen atom, one ring oxygen atom, and no additional ring heteroatoms, wherein the 7-10 membered heterocyclyl is optionally substituted with 1-4 (e.g., 1-2) **R⁷**. In some embodiments, each **R⁷** is independently selected from the group consisting of: -F, -OH, oxo, -**R^{b1}**, and C₁₋₃ alkyl optionally substituted with 1-3 **R^{c}**, wherein the **R^{b1}** is a 5-6 membered heteroaryl optionally substituted with 1-2 **R^{g}**.

In some embodiments of Formula (**SI-II-a**), **R¹** is a 7-10 (e.g., 7) membered spirocyclic bicyclic heterocyclyl having one ring nitrogen atom, one ring oxygen atom, and no additional ring heteroatoms, wherein the 7-10 membered spirocyclic bicyclic heterocyclyl is optionally substituted with 1-4 (e.g., 1-2) **R⁷**. In some embodiments, each **R⁷** is independently selected from the group consisting of: -F, -OH, oxo, -**R^{b1}**, and C₁₋₃ alkyl optionally substituted with 1-3 **R^{c}**, wherein the **R^{b1}** is a 5-6 membered heteroaryl optionally substituted with 1-2 **R^{g}**.

In some embodiments of Formula **(SI-II-a), R¹** is 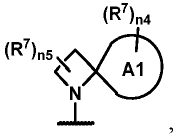 wherein: **Ring A1** is a 4-7 membered heterocyclyl ring having one ring oxygen atom and no additional ring heteroatoms; **n4** is 0, 1, or 2; and **n5** is 0, 1, or 2, provided that **n4** + **n5 is** 0, 1, or 2. In some embodiments, **n4** is 0. In some embodiments, **n5** is 0. In some embodiments **n4** is 0; and **n5** is 0. In some embodiments, each **R⁷** is independently selected from the group consisting of: -F, - OH, oxo, -**R^{b1}**, and C₁₋₃ alkyl optionally substituted with 1-3 **R^{c}**, wherein the **R^{b1}** is a 5-6 membered heteroaryl optionally substituted with 1-2 **R^{g}**.

In some embodiments of Formula **(SI-II-a), R¹** is selected from the group consisting of: 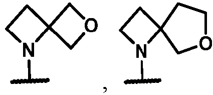 (e.g., 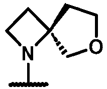 or 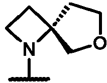 ), and 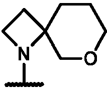 (e.g., 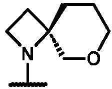 or 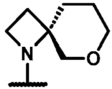 ), each of which is optionally substituted with 1-2 **R⁷**.

In some embodiments of Formula **(SI-II-a), R¹** is a 4-membered heterocyclyl optionally substituted with 1-4 **R⁷**. In some embodiments, each **R⁷** is independently selected from the group consisting of: -F, -OH, oxo, -**R^{b1}**, and C₁₋₃ alkyl optionally substituted with 1-3 **R^{c}**, wherein the **R^{b1}** is a 5-6 membered heteroaryl optionally substituted with 1-2 **R^{g}**.

In some embodiments of Formula (**SI-II-a**), **R¹** is 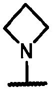 optionally substituted with 1-4 (e.g., 1-2) **R⁷**. In some embodiments, each **R⁷** is independently selected from the group consisting of: -F, oxo, -OH, -**R^{b1}**, and C₁₋₃ alkyl optionally substituted with 1-3 **R^{c}**, wherein the **R^{b1}** is a 5-6 membered heteroaryl optionally substituted with 1-2 **R^{g}**.

In some embodiments of Formula **(SI-II-a), R¹** is selected from the group consisting of: 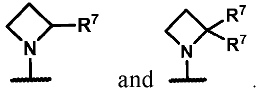 In some embodiments, each **R⁷** is independently selected from the group consisting of:
-F;
-cyano;
-OH;
-**R^{b1}**, wherein the **R^{b1}** is a 5-6 membered heteroaryl optionally substituted with 1-2 **R^{g}**;
C₁₋₃ alkyl optionally substituted with 1-3 F; and
C₁₋₃ alkyl substituted with -OH or C₁₋₃ alkoxy.

In some embodiments of Formula **(SI-II-a), R¹** is 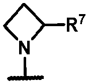 (e.g., 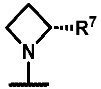 ), wherein **R⁷** is C₁₋₃ alkyl substituted with -OH or C₁₋₃ alkoxy (e.g., C₁₋₃ alkyl substituted with -OH). In some embodiments, **R¹** is 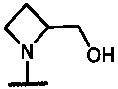 (e.g., 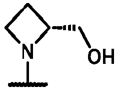 ).

In some embodiments of Formula **(SI-II-a), R¹** is 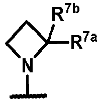 (e.g., 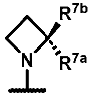 ), wherein **R^{7a}** is C₁₋₃ alkyl substituted with -OH (e.g., -CH₂OH); and **R^{7b}** is selected from the group consisting of: C₁₋₃ alkyl optionally substituted with 1-3 F (e.g., methyl), and C₁₋₃ alkyl substituted with -OH or C₁₋₃ alkoxy. For example, **R¹** can be 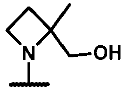 (e.g., 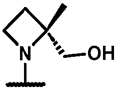 ). For example, **R¹** can be

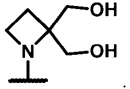

In some embodiments of Formula **(SI-II-a), R¹** is 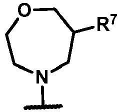 (e.g., 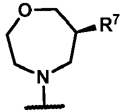 ), wherein **R⁷** is a 5-membered heteroaryl optionally substituted with 1-3 **R^{g}**. In some embodiments, **R⁷** is selected from the group consisting of pyrazolyl and oxazolyl, each of which is optionally substituted with 1-2 **R^{g}**. In some embodiments, **R⁷** is pyrazolyl optionally substituted with 1-2 **R^{g}** (e.g., **R⁷** is 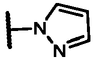 optionally substituted with one **R^{g}**). For example, **R⁷** can be 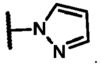 In some embodiments, **R⁷** is oxazolyl optionally substituted with one **R^{g}** (e.g., **R⁷** is 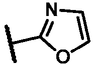 optionally substituted with one **R^{g}**). For example, **R⁷** can be

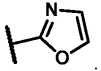

In some embodiments of Formula (**SIII-II-a**), **(SII-II-a),** or (**SI-II-a**), **Y²** is -CH₂-; and **R³** is 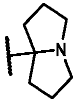 optionally substituted with 1-2 substituents each independently selected from the group consisting of: -F, -C₁₋₃ alkoxy, and -C₁₋₃ haloalkoxy. For example, **R³** can be 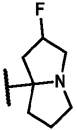 (e.g., 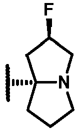 ). For example, **R³** can be 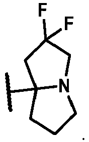 For example, **R³** can be

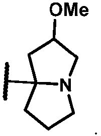

**Scheme 1** provides exemplary methods for preparing compounds of Formula (**II-a)**: 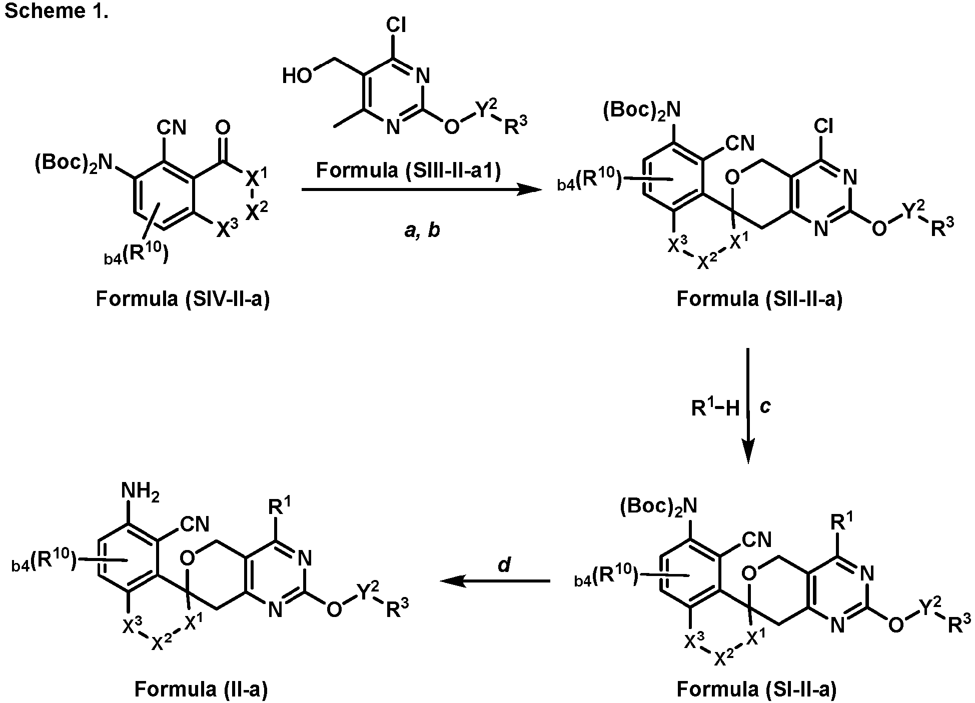 **Exemplary Conditions: a.** nBuLi, THF, -78°C; **b.** 4-nitrobenzene-1-sulfonyl chloride, THF; **c.** DIPEA or TEA, EtOH or 1,4-dioxane; **d.** HCI or TFA, MeOH or DCM or MeCN

Referring to **Scheme 1,** a compound of Formula **(SIII-II-a)** is treated with a base (e.g., nBuLi (e.g., in THF at -78°C)), followed by a compound of Formula (**SIV-II-a)** to provide an intermediate, which is converted into a compound of Formula (**SII-II-a)** under appropriate conditions (e.g., 4-nitrobenzene-1-sulfonyl chloride in THF; or TsCl and nBuLi in THF). The compound of Formula (**SII-II-a)** is then reacted with **R¹**-H, wherein **R¹** is as defined for Formula (**II-a**) (e.g., **R¹** is attached to the pyrimidine ring via a nitrogen atom), to provide a compound of Formula **(SI-II-a)** under appropriate conditions (e.g., triethylamine or *N,N-*diisopropylethylamine) in an appropriate solvent (e.g., EtOH, 1,4-dioxane, or DMF). The compound of Formula (**SI-II-a**) is then reacted with an acid (e.g., hydrochloric acid or trifluoracetic acid) in an appropriate solvent (e.g., MeOH, DCM, or MeCN), to provide a compound of Formula (**II-a**).

In some embodiments of the methods described in **Scheme 1,** provided herein are methods of preparing compounds of Formula **(II-aa),** as depicted in **Scheme 2.** 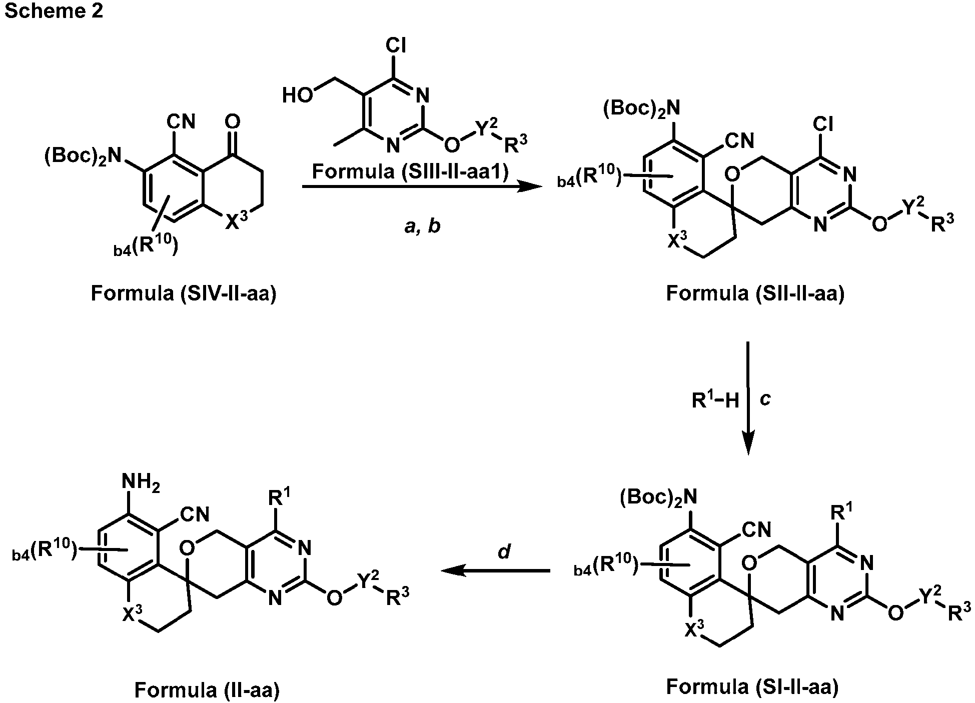 **Exemplary Conditions: a.** nBuLi, THF, -78°C; **b.** 4-nitrobenzene-1-sulfonyl chloride, THF; **c.** DIPEA or TEA, EtOH or 1,4-dioxane; **d.** HCI or TFA, MeOH or DCM or MeCN

Referring to **Scheme 2,** a compound of Formula (**SIII-II-aa**) is treated with a base (e.g., nBuLi (e.g., in THF at -78°C)), followed by a compound of Formula (**SIV-II-aa**) to provide an intermediate which is converted into a compound of Formula (**SII-II-aa**) under appropriate conditions (e.g., 4-nitrobenzene-1-sulfonyl chloride in THF; or TsCl and nBuLi in THF), wherein **X³**, **b4, R¹⁰**, **Y²**, and **R³** are as defined for Formula (**II-a**). The compound of Formula (**SII-II-aa**) is then reacted with **R¹**-H, wherein **R¹** is as defined for Formula (**II-a**) (e.g., **R¹** is attached to the pyrimidine ring via a nitrogen atom), to provide a compound of Formula **(SI-II-aa**) under appropriate conditions (e.g., triethylamine or N,N-diisopropylethylamine) in an appropriate solvent (e.g., EtOH, 1,4-dioxane, or DMF)). A compound of Formula **(SI-II-aa)** is then reacted with an acid (e.g., hydrochloric acid or trifluoracetic acid) in an appropriate solvent (e.g., MeOH, DCM, or MeCN), to provide a compound of Formula (**II-aa**) wherein **R¹⁰**, **b4, X³**, **R¹**, **Y²**, and **R³** are as defined for Formula (**II-a**).

**Scheme 3** provides exemplary methods for preparing compounds of Formula (**I-a1**): 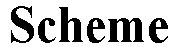 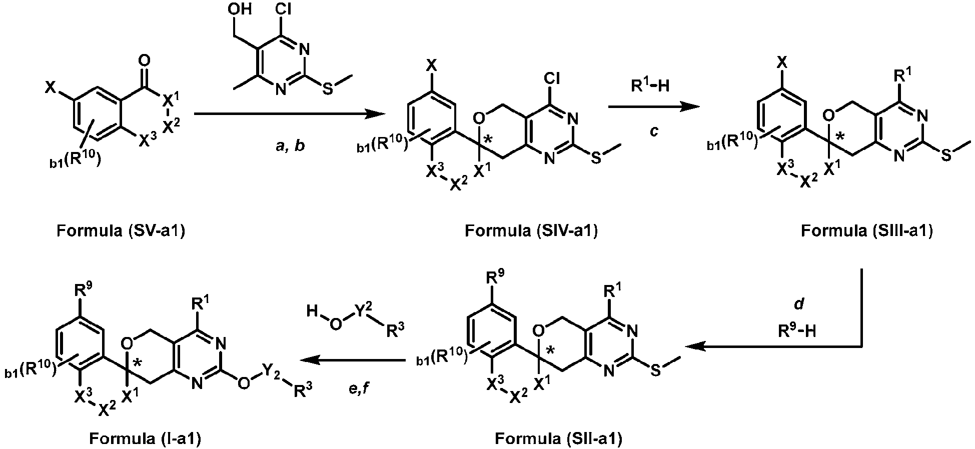 **Exemplary Conditions:** a. LDA, THF, -78°C; ***b***. H₃PO₄, toluene, reflux; or TPP, DIAD, THF or TsCl, nBuLi, THF; ***c***. DIPEA or TEA, EtOH or 1,4-dioxane; ***d*.** BrettPhos Pd G₄, Cs₂CO₃, 1,4-dioxane, 80°C; or Pd(OAc)₂, Xantphos, Cs₂CO₃, 1,4-dioxane, 80 °C; ***e*.** Oxone or mCPBA, DCM; ***f***. NaH or tBuONa or t-amylONa, THF

Referring to **Scheme 3,** (4-chloro-6-methyl-2-(methylthio)pyrimidin-5-yl)methanol is treated with a base (e.g., LDA (e.g., in THF at - 78°C)), followed by a compound of Formula (**SV-a1)** to provide an intermediate which is converted into a compound of Formula (**SIV-a1**) under appropriate conditions (e.g., H₃PO₄ in toluene under reflux; or TPP and DIAD in THF; or TsCl and nBuLi in THF), wherein **X¹**, **X²**, **X³**, **b1,** and **R¹⁰** are as defined for Formula (**I-a1**); and **X** is halo (e.g., -Br). A compound of Formula (**SIV-a1)** is then reacted with **R¹**-H, wherein **R¹** is as defined for Formula (**I-a1**) (e.g., **R¹** is attached to the pyrimidine ring via a nitrogen atom), to provide a compound of Formula **(SIII-a1)** under appropriate conditions (e.g., in the presence of a base (e.g., triethylamine or *N*,*N-*diisopropylethylamine) in an appropriate solvent (e.g., EtOH, 1,4-dioxane, or DMF)). A compound of Formula **(SIII-a1)** is then reacted with a compound having Formula **R⁹**-H, wherein **R⁹** is as defined for Formula (**I-a1**), to provide a compound of Formula **(SII-a1)** under appropriate conditions (e.g., in the presence of a palladium catalyst and optionally a ligand and/or a base (e.g., Pd(OAc)₂, XantPhos, Cs₂CO₃, in 1,4-dioxane at 80 °C; or BrettPhos Pd G4, Cs₂CO₃, in 1,4-dioxane at 80 °C)). Subsequently, the thioether group in the compound of Formula **(SII-a1)** is oxidized (e.g., with Oxone or mCPBA). This is followed by coupling with **R³-Y²**-OH, wherein **R³** and **Y²** are as defined for Formula (**I-a1**), to provide a compound of Formula (**I-a1**).

Variations to the reaction sequence in **Scheme 3 are also** contemplated. For example, to provide certain compounds of Formula (**I-a1**) wherein **R⁹** is halo, **step d** in **Scheme 2** may be bypassed (i.e., compounds of Formula **(SIII-a1)** are reacted with **R³-Y²**-OH under **steps e** and **f).** For example, in the preparation of certain compounds of Formula (**I-a1**) wherein **R⁹** is NH₂, the **R⁹** group can be protected with one or two nitrogen protecting groups (e.g., the NH₂ can be protected as NBn₂ or NHBoc) during the reaction sequence e.g., in Formula **(SII-a1)** and/or **R⁹**-H of **step d,** followed by the appropriate deprotection steps (e.g., HCl in 1,4-dioxane for NHBoc group; or Pd(OAc)₂/H₂ for NBn₂). Representative examples of nitrogen protecting groups include: acetyl (Ac), benzoyl (Bz), benzyl (Bn), benzyloxycarbonyl (Cbz), formyl, phenylsulfonyl, pivaloyl, *tert*-butoxycarbonyl (Boc), *tert*-butyl acetyl, ethyloxycarbonyl, trifluoroacetyl, triphenylmethyl (trityl), and triisopropylsilane. *See:* T. W. Greene and P. G. M. Wuts in "Protective groups in organic chemistry" John Wiley and Sons, 4th Edition, 2006.

The final product and/or intermediates in **Scheme 3** can be subjected to standard functional group transformation(s) to provide additional examples of compounds of Formula (**I-a1**). For example, a compound of Formula (**I-a1**) as provided in **Scheme 3** can be converted into a second compound of Formula (**I-a1**) having a different **R⁹** group. For example, a compound of Formula (**I-a1**) wherein **R⁹** is -NHC(=O)O^{t}Bu can be converted into a second compound of Formula (**I-a1**) wherein **R⁹** is -NH₂ upon treatment with an acid (e.g., HCl in 1,4-dioxane).

**Scheme 4** depicts an exemplary method for synthesizing certain intermediates useful in the preparation of compounds of Formula (A) (e.g., Formula (**I-a1**)): 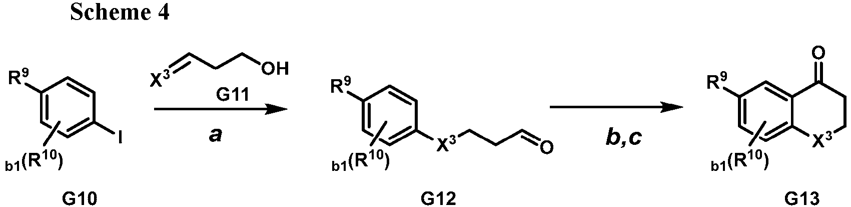 ***Exemplary Conditions: a.*** Pd(OAc)₂, LiCl, LiOAc, Bu₄N⁺Cl⁻, b. NaClO₂, NaH₂PO₄; c. AlCl₃, oxalyl chloride, DMF

Referring to **Scheme 4,** Compound **G10** is reacted with compound **G11** to provide compound **G12** under appropriate conditions (e.g., in the presence of a palladium catalyst (e.g., Pd(OAc)₂, LiCl, LiOAc, tetrabutylammonium chloride), wherein **R⁹**, **b1**, and **R¹⁰** are as defined for Formula **(A)** (e.g., Formula (**I-a1**)) (e.g., **R⁹** is halo such as -Br); and **X³** is CH₂, CH**R^{L}**, or C(**R^{L}**)₂ wherein **R^{L}** is as defined for Formula **(A)** (e.g., Formula (**I-a1**)) (e.g., **X³** is CH₂ or CH**R^{L}**). The aldehyde group in compound **G12** is subsequently oxidized (e.g., under Pinnick oxidation conditions (e.g., with NaClO₂, NaH₂PO₄)). The oxidized product is then cyclized to provide compound **G13** (e.g., with AlCl₃, oxalyl chloride, DMF).

### EXAMPLES

In some of the examples disclosed herein, one or more compounds in a described chemical reaction sequence (e.g., starting materials, intermediates, or products) is structurally depicted with enhanced stereochemical notation(s) at one or more defined stereogenic center(s). Examples of such notations include *or1, or2, &1,* &2, and the like. In some such examples, in the chemical name of the same compound, each of such defined stereogenic center(s) is assigned a tentative configuration (e.g., *(R)-* or (S)-) shown by the wedge/hash representation of its structural formula. However, the defined stereogenic center(s) should be understood to have configurations consistent with the enhanced stereochemical notation(s), as described herein, based e.g., on the conventions explained below. For avoidance of doubt, the chemical names of these compounds, having one or more enhanced stereochemical notation(s), adopt the following conventions:
When the chemical name of a compound having only one stereogenic center contains the prefix *"rel,"* the stereogenic center is resolved, but its absolute configuration is either (R) or (*S*), thereby corresponding to an *or1* enhanced stereochemical notation at the corresponding stereogenic center in its structural formula. For example, the chemical name *rel*-(*R*)-(1-methylpyrrolidin-2-yl)methanol represents one stereoisomer selected from the group consisting of:

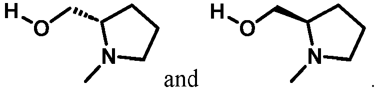

When one stereogenic center is labelled with an asterisk ("*") in the chemical name of a compound having more than one stereogenic centers (e.g., when a stereogenic center is denoted as (*R**)), the stereogenic center labeled with the asterisk is resolved, but its absolute configuration is either (*R*) or (*S*), thereby corresponding to an *or1* enhanced stereochemical notation at the corresponding stereogenic center in its structural formula. For example, the chemical name ((2*R**,4*R*)-1,4-dimethylpyrrolidin-2-yl)methanol represents one stereoisomer selected from the group consisting of:

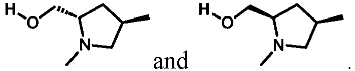

When the chemical name of a compound contains two stereogenic centers labelled with asterisks, these two stereogenic centers may have (*R*) or (*S*) configurations, either concertedly or independently, in conformity with the *orx* (e.g., *or1* or *or2)* notations in its structural formula in **Table C1** or **Table C3.**

When two stereogenic centers are labelled *or1* and *or1* in a compound structure, and they are labelled with asterisks in the chemical name, then the relative stereochemistry between the two stereogenic centers (e.g., *syn* or *anti* relationship) is as represented by the name, but the absolute configurations of the two stereogenic centers can vary concertedly (e.g., a compound designated (*R*,R**) is one stereoisomer selected from (*R,R*) and (*S,S*); for avoidance of doubt, the compound designated (*R**,*R**) does not have *(S,R)* or (*R,S*) configurations across these stereogenic centers). For example, the chemical name of 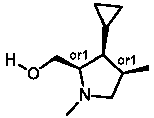 is ((2*R**,3*S*,4*R**)-3-cyclopropyl-1,4-dimethylpyrrolidin-2-yl)methanol. This name, taken together with the structural formula, represents one stereoisomer selected from the group consisting of:

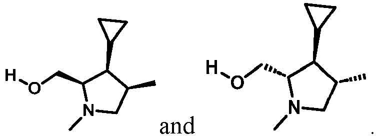

When two stereogenic centers are labelled *or1* and *or2* in a compound structure, and they are labelled with asterisks in the chemical name, then the configuration of the two stereogenic centers can vary independently (e.g., a compound designated (*R**,*R**) is one stereoisomer selected from *(R,R),* (S,S), *(R,S),* and *(S,R)).* For example, the chemical name of 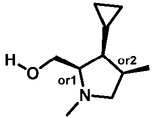 is ((2*R**,3*S*,4*R**)-3-cyclopropyl-1,4-dimethylpyrrolidin-2-yl)methanol. This name, taken together with the structural formula, represents one stereoisomer selected from the group consisting of: 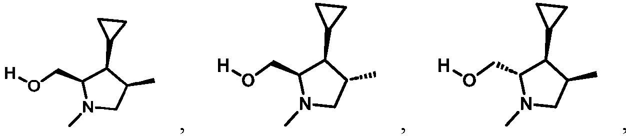 and

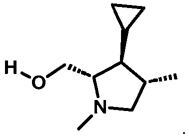

When the chemical name of a compound contains two stereogenic centers designated "*RS*" and/or *"SR,"* a mixture of stereoisomers is provided wherein among the constituent stereoisomers these two stereogenic centers differ either concertedly or independently, in conformity with the *&x* (e.g., *&1* or *&2)* notations in **Table C1** or **Table C3** for the corresponding compound.

When two defined stereogenic centers are labelled *&1* and *&1* in a chemical structure, and they are designated *"RS"* and/or *"SR"* in the corresponding chemical name, then a mixture of two stereoisomers is provided, wherein each constituent stereoisomer has the relative stereochemistry between these two stereogenic centers (e.g., *syn* or *anti* relationship) as depicted by the structural formula and by the name. As an example, a chemical name designated *(RS,SR)* represents a mixture of *(R,S)* and *(S,R)* stereoisomers, and this mixture does not include *(R,R)* or *(S,S)* stereoisomers. As a second example, a chemical name designated *(SR,SR)* represents a mixture of *(S,S)* and *(R,R)* stereoisomers, and this mixture does not include *(R,S)* or *(S,R)* stereoisomers. For example, the chemical name of 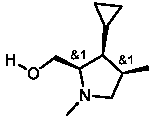 is ((2*RS*,3*S*,4*RS*)-3-cyclopropyl-1,4-dimethylpyrrolidin-2-yl)methanol. This name, taken together with the structural formula, represents a mixture of two stereoisomers:

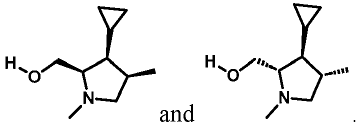

When two defined stereogenic centers are labelled *&1* and *&2* in a chemical structure, and they are designated *"RS"* and/or *"SR"* in the corresponding chemical name, then a mixture of four stereoisomers is provided each differing in configuration at one or both of these two stereogenic centers (e.g., a chemical name designated *(RS,SR)* represents a mixture of *(R,S), (S,R), (R,R),* and *(S,S)* stereoisomers). For example, the chemical name of 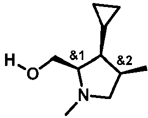 is ((2*RS*,3*S*,4*RS*)-3-cyclopropyl-1,4-dimethylpyrrolidin-2-yl)methanol. This name, taken together with the structural formula, represents a mixture of four stereoisomers:

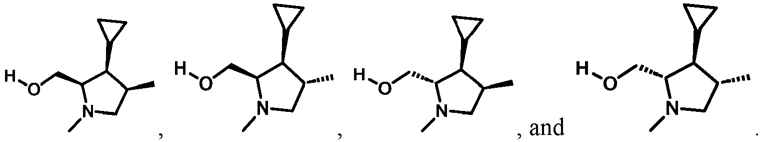

### General Analytical Methods:

**Method A:** VanGuard Pre-Column CSH C18, 1.7 µm, 2.1 × 5 mm, Pre-run: 1 mL/min for 0.7 minutes. Column: Acquity UPLC, CSH C18, 1.7 µm, 2.1 × 30 mm, Flow rate: 0.9 mL/min, 5 to 100% MeCN/ H₂O (+ 0.1% formic acid) for 3 minutes.

**Method B:** VanGuard Pre-Column CSH C18, 1.7 µm, 2.1 × 5 mm, Pre-run: 1 mL/min for 0.7 minutes. Column: Acquity UPLC, CSH C18, 1.7 µm, 2.1 × 30 mm, Flow rate: 0.9 mL/min, 5 to 100% MeCN/ H₂O (+ 10 mM ammonium bicarbonate) for 3 minutes.

**Method C:** Acquity UPLC, CSH C18, 1.7 µm, 2.1 × 30 mm, Mobile phase A: 0.1% formic acid in H₂O; Mobile phase B: 0.1% formic acid in MeCN (v/v); Gradient: 95% H₂O/5% MeCN linear to 5% H₂O/95% MeCN in 2.0 minutes, hold at 5% H₂O/95% MeCN to 2.50 minutes. Then 5% H₂O/95% MeCN linear to 95% H₂O/5% MeCN in 0.5 minutes. Flow rate: 0.6 mL/min.

**Method D:** Acquity UPLC, CSH C18, 1.7 µm, 2.1 × 30 mm, Mobile phase A: 0.1% ammonium hydroxide in H₂O; Mobile phase B: 0.1% ammonium hydroxide in MeCN (v/v); Gradient: 95% H₂O/5% MeCN linear to 5% H₂O/95% MeCN in 2.0 minutes, hold at 5% H₂O/95% MeCN to 2.50 minutes. Then 5% H₂O/95% MeCN linear to 95% H₂O/5% MeCN in 0.5 minutes. Flow rate: 0.6 mL/min.

**Method E:** Acquity UPLC, CSH C18, 1.7 µm, 2.1 × 30 mm, Mobile phase A: 0.1% formic acid in H₂O; Mobile phase B: 0.1% formic acid in MeCN (v/v); Gradient: 95% H₂O/5% MeCN linear to 5% H₂O/95% MeCN in 4.0 minutes, hold at 5% H₂O/95% MeCN to 4.50 minutes. Then 5% H₂O/95% MeCN linear to 95% H₂O/5% MeCN in 0.5 minutes. Flow rate: 0.6 mL/min.

**Method F:** Poroshell SB-C18, 4.6 × 150 mm, Mobile phase A: 0.1% TFA in H₂O (v/v), Mobile phase B: 0.1% TFA in MeCN (v/v), Gradient: 30-95% Mobile phase B over 15 minutes.

**Method G:** Chiralpak IB-U (3.0 × 100 mm, 1.6 mM, P/N 81U83).

**Method H:** Kinetex^{®} EVO C18 2.1 × 30 mm 5 µm, Mobile phase A: 0.0375% TFA in H₂O (v/v), Mobile phase B: 0.01875% TFA in MeCN (v/v), Gradient: 95% H₂O/5% MeCN linear to 5% H₂O/95% MeCN in 0.6 minutes, hold at 5% H₂O/95% MeCN to 0.78 minutes. Flow rate: 2.0 mL/min. Then 5% H₂O/95% MeCN linear to 95% H₂O/5% MeCN in 0.02 minutes. Flow rate: 2.0 mL/min.

**Method I:** VanGuard Pre-Column CSH C18, 1.7 µm, 3.0 × 50 mm, Pre-run: 0.65 mL/min for 2.5 minutes. Column: Acquity UPLC, CSH C18, 1.7 µm, 2.1 × 75 mm, Flow rate: 0.6 mL/min, 5 to 100% MeCN/ H₂O (+ 10 mM ammonium bicarbonate) for 7.5 minutes.

**Method J:** Column: Chiralcel OD-3R (2.1 × 100 mm, 3 µm, P/N 14893); Mobile phase A = 0.1% v/v ammonium hydroxide in H₂O, 0.1% v/v ammonium hydroxide in MeCN; Gradient: 95% H₂O/5% MeCN linear to 5% H₂O/95% MeCN in 8.0 minutes, hold at 5% H₂O/95% MeCN to 9.00 minutes. Then 5% H₂O/95% MeCN linear to 95% H₂O/5% MeCN in 1.0 minute.

**Method K:** Acquity UPLC, CSH C18, 1.7 µm, 2.1 x 30 mm, Mobile phase A: 0.1% TFA in H₂O; Mobile phase B: 0.1% TFA in MeCN (v/v); Gradient: 95% H₂O/5% MeCN linear to 5% H₂O/95% MeCN in 4.0 minutes, hold at 5% H₂O/95% MeCN to 4.50 minutes. Then 5% H₂O/95% MeCN linear to 95% H₂O/5% MeCN in 0.5 minutes. Flow rate: 0.6 mL/min.

**Method L:** Acquity UPLC, CSH C18, 1.7 µm, 2.1 × 30 mm, Mobile phase A: 0.1% TFA in H₂O; Mobile phase B: 0.1% TFA in MeCN (v/v); Gradient: 95% H₂O/5% MeCN linear to 5% H₂O/95% MeCN in 2.0 minutes, hold at 5% H₂O/95% MeCN to 2.50 minutes. Then 5% H₂O/95% MeCN linear to 95% H₂O/5% MeCN in 0.5 minutes. Flow rate: 0.6 mL/min.

**Method M:** VanGuard Pre-Column CSH C18, 17 µm, 21 x 5 mm, Pre-run: 1mL/min for 07 min Column: Acquity UPLC, CSH C18, 17 µm, 21 x 30 mm, Flow rate: 09 mL/min, 5 to 100% MeCN/water (+ 10 mM ammonium bicarbonate) for 7 minutes.

**Table of Abbreviations:**

| **Abbreviation** | **Definition** |
|---|---|
| Ac | Acyl |
| Bn | Benzyl |
| Boc | *tert*-Butoxycarbonyl |
| BOP | Benzotriazole-1-yl-oxy-tris-(dimethylamino)-phosphonium hexafluorophosphate |
| BrettPhos | 2-(Dicyclohexylphosphino)3,6-dimethoxy-2',4',6'-triisopropyl-1,1'-biphenyl |
| BrettPhos Pd G4 | (SP-4-3)-[Dicyclohexyl[3,6-dimethoxy-2',4',6'-tris(1-methylethyl)[1,1'-biphenyl]-2-yl]phosphine-κP](methanesulfonato-κO)[2'-(methylamino-κN)[1,1'-biphenyl]-2-yl-κC] |
| BSA | Bovine Serum Albumin |
| Bz | Benzoyl |
| Cbz | Benzyloxycarbonyl |
| CSH | Charged Surface Hybrid |
| Da | Daltons |
| DBU | 1,8-Diazabicyclo[5.4.0]undec-7-ene |
| DCM | Dicholoromethane |
| DEA | Diethylamine |
| DIAD | Diisopropyl azodicarboxylate |
| DIBAL-H | Diisobutylaluminium hydride |
| DIPEA | Diisopropylethylamine |
| DMA | Dimethylacetamide |
| DMF | Dimethylformamide |
| DMSO | Dimethylsulfoxide |
| DTT | Dithiothreitol |
| *ee* | Enantiomeric excess |
| eq | equivalents |
| ESI | Electrospray Ionization |
| EtOAc | Ethyl acetate |
| EtOH | Ethanol |
| GDP | Guanosine diphosphate |
| GMPPNP | 5'-Guanylyl imidodiphosphate |
| GTP | Guanosine triphosphate |
| HATU | 1-[Bis(dimethylamino)methylene]-1H-1,2,3-triazolo[4,5-b]pyridinium 3-oxide hexafluorophosphate |
| HEPES | 4-(2-Hydroxyethyl)-1-piperazineethanesulfonic acid |
| HPLC | High-performance liquid chromatography |
| IPA | Isopropyl alcohol |
| LCMS | Liquid chromatography mass spectroscopy |
| LDA | Lithium diisopropylamide |
| LiHMDS | Lithium bis(trimethylsilyl)amide |
| m/z | Mass to charge |
| *m*CPBA | *meta*-Chloroperoxybenzoic acid |
| MeCN | Acetonitrile |
| MeOD | Deuterated methanol (CD₃OD) |
| MeOH | Methanol |
| MsCl | Methanesulfonyl chloride |
| nBuLi | *n*-Butyl lithium |
| NMP | N-Methyl-2-pyrrolidone |
| NMR | Nuclear magnetic resonance |
| Pd(dppf)Cl₂ | [1,1'-Bis(diphenylphosphino)ferrocene]dichloropalladium(II) |
| Pd₂dba₃ | Tris(dibenzylideneacetone)dipalladium(0) |
| PDA | Photodiode array |
| psi | Pounds per square inch |
| PyBOP | Benzotriazol-1-yloxytripyrrolidinophosphonium hexafluorophosphate |
| RU | Resonance units |
| SFC | Supercritical fluid chromatography |
| SOS1 | Son of sevenless homolog 1 |
| tBuXPhos | 2-Di-*tert*-butylphosphino-2',4',6'-triisopropylbiphenyl |
| TCEP | Tris (2-carboxyethyl)phosphine |
| TEA | Triethylamine |
| TFA | Trifluoroacetic acid |
| THF | Tetrahydrofuran |
| TPP | Triphenylphosphine |
| t_{R} or T_{R} | Retention time |
| TR-FRET | Time-resolved fluorescence resonance energy transfer |
| Trityl | Triphenylmethyl |
| TsCl | *p*-Toluenesulfonyl chloride |
| UPLC | Ultra performance liquid chromatography |
| wt% | Percent by weight |
| XantPhos | (9,9-Dimethyl-9H-xanthene-4,5-diyl)bis(diphenylphosphane) |

### Example P. Synthesis of Intermediates

### Intermediate 1: (4-Chloro-6-methyl-2-(methylthio)pyrimidin-5-yl)methanol

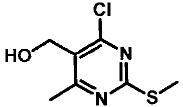

To ethyl 4-chloro-6-methyl-2-(methylthio)pyrimidine-5-carboxylate (535 g, 2.17 mol) in toluene (1.5 L) and THF (1.5 L) at -14 °C was added DIBAL-H (4 L of 1.03 M in THF and 666 g of 25 wt% in toluene, 5.29 mol) over 66 minutes. The mixture was gradually warmed to 20 °C, held for 2 hours, and then added to a solution of potassium sodium tartrate tetrahydrate (4.5 kg, 15.9 mol) in water (9 L) at 2 °C over 15 minutes. Gas evolution and exotherm continued after the transfer was complete with a maximum internal temperature of 31 °C. EtOAc (3.25 L) was added, and the layers were separated, and the organic layer was washed with brine (4 L). The aqueous layers were sequentially extracted with EtOAc (3.25 L). The combined organic layers were dried over magnesium sulfate, filtered through a pad of Magnesol (380 g), and rinsed with EtOAc (1.5 L). The filtrate was concentrated to a solid, triturated in EtOAc (800 mL) at 45-50 °C, diluted with n-heptane (2.4 L) over 20 minutes, and cooled to room temperature overnight. The slurry was filtered, washed with n-heptane, and the solids were dried under vacuum to afford (4-chloro-6-methyl-2-(methylthio)pyrimidin-5-yl)methanol (328 g) as a white solid.

¹H NMR (400 MHz, CDCl₃) δ 4.80 (d, 2H), 2.64 (s, 3H), 2.59 (s, 3H), 1.90 (t, 1H).

t_{R} = 4.84 minutes (Method F).

### Intermediate 2: 7-Bromo-4'-chloro-2'-(methylthio)-3,4,5',8'-tetrahydro-2H-spiro[naphthalene-1,7'-pyrano[4,3-d]pyrimidine]

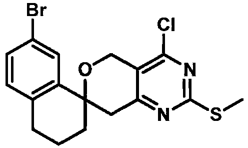

### Step 1: 7-Bromo-1-((6-chloro-5-(hydroxymethyl)-2-(methylthio)pyrimidin-4-yl)methyl)-1,2,3,4-tetrahydronaphthalen-1-ol

To a solution of diisopropylamine (355 mL, 2.53 mol) in THF (3.5 L) at -28 °C was added nBuLi in hexanes (1 L, 2.5 M, 2.5 mol) over 24 minutes. The mixture was held at -16 to -26 °C for 10 minutes then cooled to -71 °C. (4-Chloro-6-methyl-2-(methylthio)pyrimidin-5-yl)methanol (255 g, 1.25 mol) in THF (1.4 L) was added over 45 minutes, while maintaining the internal temperature below -66°C. The mixture was stirred for one hour at -70 °C. To this mixture, 7-bromo-3,4-dihydronaphthalen-1(2H)-one (280 g, 1.24 mol) in THF (1 L) was added over 37 minutes, while maintaining the temperature below -70 °C. The mixture was warmed to 2 °C over two hours and then quenched with ammonium chloride (500 g) in water (1.75 L). The layers were separated, and the organic layer was washed with brine (1.5 L). The aqueous layers were sequentially extracted with EtOAc (1 L). The combined organic layers were dried over magnesium sulfate and then concentrated under reduced pressure to a gummy residue. DCM (700 mL) was then added, and the resulting mixture was re-concentrated to a solid. The solid was triturated in DCM (2 L) at 35°C for 20 minutes, cooled to 15 °C for 30 minutes, filtered, and washed with DCM (400 mL). The solids were dried under vacuum at 35°C to afford 7-bromo-1-((6-chloro-5-(hydroxymethyl)-2-(methylthio)pyrimidin-4-yl)methyl)-1,2,3,4-tetrahydronaphthalen-1-ol (360 g) as a white solid.

¹H NMR (400 MHz, CDCl₃) δ7.70 (s, 1H), 7.30 (d, 1H), 7.00 (d, 1H), 4.80-4.70 (m, 2H), 4.33 (s, 1H), 3.40-3.25 (m, 3H), 2.80 (t, 2H), 2.55 (s, 3H), 2.15-1.85 (m, 3H), 1.75-1.65 (m, 1H).

t_{R} = 10.491 minutes (Method F).

### Step 2: 7-Bromo-4'-chloro-2'-(methylthio)-3,4,5',8'-tetrahydro-2H-spiro[naphthalene-1,7'-pyrano[4,3-d]pyrimidine]

7-Bromo-1-((6-chloro-5-(hydroxymethyl)-2-(methylthio)pyrimidin-4-yl)methyl)-1,2,3,4-tetrahydronaphthalen-1-ol (200 g, 465 mmol) was suspended in toluene (1 L, 5 vol). Phosphoric acid (86 wt%, 35 mL, 512 mmol) was then added and the resulting mixture was refluxed for 27 hours. The solution was cooled to room temperature before adjusting the pH from 1 to 7 using 4 N sodium hydroxide (150 mL, 600 mmol). The reaction mixture was then diluted with EtOAc (500 mL) and water (500 mL). The phases were separated, and the organic phase was washed with brine (500 mL). The aqueous phases were sequentially back-extracted with EtOAc (500 mL). The organic phases were combined, dried over sodium sulfate, and concentrated to a neat orange oil. The crude product was dissolved in EtOAc (140 mL, 1 vol) at 50 °C. Hexane (450 mL, 3 vol) was added to the solution over 5 minutes, and the resulting crystals were filtered and washed with EtOAc/hexane (1:4, 100 mL). This process was repeated three times to afford 7-bromo-4'-chloro-2'-(methylthio)-3,4,5',8'-tetrahydro-2H-spiro[naphthalene-1,7'-pyrano[4,3-d]pyrimidine] (113 g) as an off-white solid.

¹H NMR (400 MHz, CDCl₃) δ7.55 (s, 1H), 7.35 (d, 1H), 7.05 (d, 1H), 4.80 (q, 2H), 3.15 (q, 2H), 2.90-2.78 (m, 2H), 2.60 (s, 3H), 2.07-1.92 (m, 3H), 1.85-1.70 (m, 1H).

t_{R} = 13.149 minutes (Method F).

### Intermediate 2a: (S)-7-Bromo-4'-chloro-2'-(methylthio)-3,4,5',8'-tetrahydro-2H-spiro[naphthalene-1,7'-pyrano[4,3-d]pyrimidine]

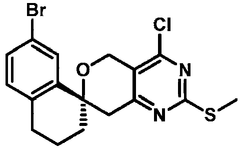

The enantiomers of 7-bromo-4'-chloro-2'-(methylthio)-3,4,5',8'-tetrahydro-2H-spiro[naphthalene-1,7'-pyrano[4,3-d]pyrimidine] (5.0 g) were separated by chiral SFC (Chiralpak IB-N 21mm ID × 250 mm, 5µm (serial number IBSSMJ-BV001; MeOH, 40%, backpressure = 120 bar).

The first eluting peak was isolated as **Intermediate 2a** (1.85 g).
LCMS: m/z (ESI) [M+H]⁺ 413.0, t_{R} = 4.00 minutes (Method E)
Chiral HPLC: t_{R} = 4.78 minutes. (Method G; Gradient 5-40% MeOH over 10 minutes)
¹H NMR (400 MHz, CDCl₃) δ 7.54 (d, 1H), 7.27 (dd, 1H), 6.93 (d, 1H), 4.70 (q, 2H), 3.17 - 2.85 (m, 2H), 2.82 - 2.61 (m, 2H), 2.50 (s, 3H), 1.99 - 1.79 (m, 3H), 1.78 - 1.62 (m, 1H).

### Intermediate 2b: (R)-7-Bromo-4'-chloro-2'-(methylthio)-3,4,5',8'-tetrahydro-2H-spiro[naphthalene-1,7'-pyrano[4,3-d]pyrimidine]

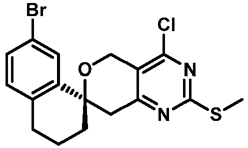

The second eluting peak was isolated as **Intermediate 2b** (2.05 g).
LCMS: m/z (ESI) [M+H]⁺ 413.0, t_{R} = 4.00 minutes (Method E)
Chiral HPLC: t_{R} = 6.12 minutes. (Method G; Gradient 5-40% MeOH over 10 minutes)
¹H NMR (400 MHz, CDCl₃) δ 7.61 (d, 1H), 7.34 (dd, 1H), 7.00 (d, 1H), 4.92 - 4.63 (m, 2H), 3.26 - 2.94 (m, 2H), 2.90 - 2.72 (m, 2H), 2.57 (s, 3H), 2.09 - 1.86 (m, 3H), 1.82 - 1.67 (m, 1H).

### Intermediate 3: tert-Butyl (2'-(((2R,7aS)-2-fluorotetrahydro-1H-pyrrolizin-7a(5H)-yl)methoxy)-4'-oxo-3,3',4,4',5',8'-hexahydro-2H-spiro[naphthalene-1,7'-pyrano[4,3-d]pyrimidin]-7-yl)carbamate

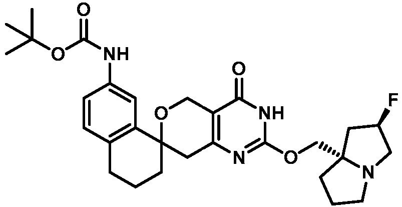

### Step 1: 4'-(Benzyloxy)-7-bromo-2'-(methylthio)-3,4,5',8'-tetrahydro-2H-spiro[naphthalene-1,7'-pyrano[4,3-d]pyrimidine]

Phenylmethanol (367 µL, 1.5 *equiv.,* 3.53 mmol) was added to a suspension of NaH (188 mg, 60 wt%, 2 *equiv.,* 4.71 mmol) in THF (6 mL). The mixture was stirred at room temperature for 5 minutes until gas evolution had ceased. A solution of 7-bromo-4'-chloro-2'-(methylthio)-3,4,5',8'-tetrahydro-2*H*-spiro[naphthalene-1,7'-pyrano[4,3-*d*]pyrimidine] (970 mg, *1 equiv.,* 2.36 mmol) in THF (6 mL) was added dropwise, and the resulting reaction mixture was stirred for 1 hour. The mixture was cooled in an ice bath and quenched by the addition of saturated aqueous ammonium chloride (20 mL). The mixture was extracted with EtOAc (2 × 50 mL) and the combined organic phases were washed with brine (50 mL), dried over sodium sulfate, and concentrated to a solid which was triturated with ethanol (60 mL) (sonication was used to break up the material). The solid was separated by filtration, washed with ethanol (10 mL), and dried to yield 4'-(benzyloxy)-7-bromo-2'-(methylthio)-3,4,5',8'-tetrahydro-2H-spiro[naphthalene-1,7'-pyrano[4,3-*d*]pyrimidine] (1.05 g).
LCMS: m/z (ESI) [M+H]⁺ 485.1, t_{R} = 2.24 minutes (Method A)

### Step 2: tert-Butyl (4'-(benzyloxy)-2'-(methylthio)-3,4,5',8'-tetrahydro-2H-spiro[naphthalene-1,7'-pyrano[4,3-d]pyrimidin]-7-yl)carbamate

A suspension of 4'-(benzyloxy)-7-bromo-2'-(methylthio)-3,4,5',8'-tetrahydro-2*H-*spiro[naphthalene-1,7'-pyrano[4,3-d]pyrimidine] (2.75 g, *1 equiv.,* 5.69 mmol), cesium carbonate (5.56 g, 3 *equiv.,* 17.1 mmol), and tert-butyl carbamate (2.00 g, 3 *equiv., 17.1* mmol) in 1,4-dioxane (80 mL) was degassed with nitrogen at room temperature for 5 minutes. BrettPhos Pd G4 (524 mg, 0.1 *equiv.,* 0.57 mmol) was added, and nitrogen was bubbled through the mixture at room temperature for another 5 minutes. The resulting reaction mixture was stirred for 5 hours, concentrated to a residue, and purified by flash chromatography (silica, 120 g silicycle cartridge, solid loading, gradient of EtOAc in DCM) to provide *tert-*butyl (4'-(benzyloxy)-2'-(methylthio)-3,4,5',8'-tetrahydro-2H-spiro[naphthalene-1,7'-pyrano[4,3-d]pyrimidin]-7-yl)carbamate (1.91 g).
LCMS: m/z (ESI) [M+H]⁺ 520.3, t_{R} = 2.14 minutes (Method A)

### Step 3: tert-Butyl (4'-(benzyloxy)-2'-(methylsulfonyl)-3,4,5',8'-tetrahydro-2H-spiro[naphthalene-1,7'-pyrano[4,3-d]pyrimidin]-7-yl)carbamate

Tetrabutylammonium hydrogen sulfate (199 mg, 0.16 *equiv.,* 0.59 mmol) and sodium tungstate dihydrate (121 mg, 0.1 *equiv.,* 0.37 mmol) were added to a solution of *tert-butyl* (4'-(benzyloxy)-2'-(methylthio)-3,4,5',8'-tetrahydro-2*H*-spiro[naphthalene-1,7'-pyrano[4,3-d]pyrimidin]-7-yl)carbamate (1.90 g, 1 *equiv.,* 3.66 mmol) in EtOAc (70 mL). The temperature was raised to 40 °C followed by the dropwise addition of Hydrogen peroxide (2.61 mL, 30 wt%, 7 *equiv.,* 25.6 mmol). The resulting reaction mixture was stirred for 100 minutes. The reaction mixture was diluted with EtOAc (100 mL), washed with water (100 mL) and brine (100 mL), dried over sodium sulfate, and concentrated. After the material was concentrated to dryness, DCM (20 mL) was added, and the resulting material was concentrated to dryness again to yield *tert-butyl* (4'-(benzyloxy)-2'-(methylsulfonyl)-3,4,5',8'-tetrahydro-2H-spiro[naphthalene-1,7'-pyrano[4,3-*d*]pyrimidin]-7-yl)carbamate (2.09 g).
LCMS: m/z (ESI) [M+H]⁺ 552.2, t_{R} = 1.86 minutes (Method A)

### Step 4: tert-Butyl (4'-(benzyloxy)-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d] pyrimidin]-7-yl)carbamate

NaH (359 mg, 60 wt%, 2.5 *equiv.,* 8.97 mmol) was added to a solution of ((2*R*,7a*S*)-2-fluorotetrahydro-1*H*-pyrrolizin-7a(5*H*)-yl)methanol (1.43 g, 2.5 *equiv.,* 8.97 mmol) in anhydrous THF (20 mL). This solution was stirred for 2 minutes until gas evolution had ceased, and then a solution of *tert*-butyl (4'-(benzyloxy)-2'-(methylsulfonyl)-3,4,5',8'-tetrahydro-2*H-*spiro[naphthalene-1,7'-pyrano[4,3-d]pyrimidin]-7-yl)carbamate (2.04 g, 1 *equiv., 3.59* mmol) in anhydrous THF (20 mL) was added over 1 minute. The mixture was stirred for 2 hours at room temperature, and then water (20 mL) and brine (20 mL) were added. The mixture was extracted with DCM:MeOH 9:1 (3 x 100 mL). The combined organic phases were washed with brine (100 mL), dried over sodium sulfate, and concentrated to a residue which was purified by flash chromatography (silica, 120 g silicycle cartridge, gradient of DCM:MeOH:NH₄OH 85:13.5:1.5 in DCM) to yield *tert-butyl* (4'-(benzyloxy)-2'-(((2R,7aS)-2-fluorotetrahydro-1*H*-pyrrolizin-7a(5*H*)-yl)methoxy)-3,4,5',8'-tetrahydro-2*H-*spiro[naphthalene-1,7'-pyrano[4,3-*d*]pyrimidin]-7-yl)carbamate (1.9 g).
LCMS: m/z (ESI) [M+H]⁺ 631.5, t_{R} = 1.43 minutes (Method A)

### Step 5: tert-Butyl (2'-(((2R,7aS)-2-fluorotetrahydro-1H-pyrrolizin-7a(5H)-yl)methoxy)-4'-oxo-3,3',4,4',5',8'-hexahydro-2H-spiro[naphthalene-1,7'-pyrano[4,3-d] pyrimidin]-7-yl)carbamate

To a solution of *tert*-butyl (4'-(benzyloxy)-2'-(((2*R*,7a*S*)-2-fluorotetrahydro-1*H-*pyrrolizin-7a(5*H*)-yl)methoxy)-3,4,5',8'-tetrahydro-2*H*-spiro[naphthalene-1,7'-pyrano[4,3-*d*]pyrimidin]-7-yl)carbamate (1.50 g, 1 *equiv.,* 2.14 mmol) in MeOH (60 mL) was added Pd on carbon (114 mg, 10 wt%, 0.05 *equiv.,* 0.11 mmol). The mixture was degassed with nitrogen, and then hydrogen was bubbled through the mixture at room temperature for 5 minutes. The reaction mixture was stirred at room temperature for 2 hours and then purged with nitrogen. The mixture was diluted with DCM (50 mL), and filtered through a syringe filter. The syringe filter was washed with DCM (3 × 10 mL). The combined liquid was concentrated to a residue which was purified by flash chromatography (silica, gradient of DCM:MeOH:NH₄OH 85:13.5:1.5 in DCM) to yield *tert*-butyl (2'-(((2*R*,7a*S*)-2-fluorotetrahydro-1*H*-pyrrolizin-7a(5*H*)-yl)methoxy)-4'-oxo-3,3',4,4',5',8'-hexahydro-2*H*-spiro[naphthalene-1,7'-pyrano[4,3-*d*]pyrimidin]-7-yl)carbamate (1.13 g).
LCMS: m/z (ESI) [M+H]⁺ 541.3, t_{R} = 1.04 minutes (Method A)
¹H NMR (400 MHz, DMSO-*d*₆): δ 12.29 (s, 1H), 9.17 (s, 1H), 7.51 (s, 1H), 7.23 (d, 1 H), 6.97 (d, 1H), 5.26 (d, 1H), 4.37-4.24 (m, 2H), 4.03-3.92 (m, 2H), 3.12-2.95 (m, 3H), 2.82-2.55 (m, 5H), 2.09-1.64 (m, 10H), 1.43 (s 9H).

### Intermediate 4: tert-Butyl ((S)-2'-(((2R,7aS)-2-fluorotetrahydro-1H-pyrrolizin-7a(5H)-yl)methoxy)-4'-oxo-3,3',4,4',5',8'-hexahydro-2H-spiro[naphthalene-1,7'-pyrano[4,3-d]pyrimidin]-7-yl)carbamate

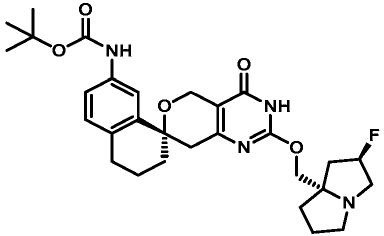

### Step 1: (S)-4'-(benzyloxy)-7-bromo-2'-(methylthio)-3,4,5',8'-tetrahydro-2H-spiro[naphthalene-1,7'-pyrano[4,3-d]pyrimidine]

Phenylmethanol (1.05 mL, 1.5 *equiv.,* 10.1 mmol) was added to a suspension of NaH (540 mg, 60 wt% dispersion in mineral oil, 2 *equiv.,* 13.5 mmol) and stirred for 5 minutes at room temperature until gas evolution had ceased. A solution of *(S)*-7-bromo-4'-chloro-2'-(methylthio)-3,4,5',8'-tetrahydro-2*H*-spiro[naphthalene-1,7'-pyrano[4,3-*d*]pyrimidine] (2.78 g, *1 equiv.,* 6.75 mmol) in THF (12 mL) was added dropwise, resulting in the formation of a hazy solution. After 50 minutes, the mixture was cooled in an ice bath and quenched by the addition of saturated aqueous ammonium chloride (40 mL). The mixture was extracted with EtOAc (2 × 100 mL). The combined organic phases were washed with brine (50 mL), dried over anhydrous sodium sulfate, and concentrated to a residue which was purified by flash column chromatography (silica, 80 g silicycle cartridge, DCM in hexanes (0-100%)) to afford (S)-4'-(benzyloxy)-7-bromo-2'-(methylthio)-3,4,5',8'-tetrahydro-2*H*-spiro[naphthalene-1,7'-pyrano[4,3-*d*]pyrimidine] (3.13 g).
LCMS: m/z (ESI) [M+H]⁺ 485.1, t_{R} = 2.25 minutes (Method A)
¹H NMR (400 MHz, CDCl₃): δ7.62 (s, 1 H), 7.41-7.31 (m, 6 H), 6.98 (d, 1H), 5.51-5.44 (m, 2 H), 4.78-4.65 (m, 2 H), 3.08-2.89 (m, 2 H), 2.78-2.75 (m, 2 H), 2.55 (s, 3 H), 1.97-1.89 (m, 3 H), 1.76-1.73 (m, 1 H).

### Step 2: tert-Butyl (S)-(4'-(benzyloxy)-2'-(methylthio)-3,4,5',8'-tetrahydro-2H-spiro[naphthalene-1,7'-pyrano[4,3-d]pyrimidin]-7-yl)carbamate

A suspension of (*S*)-4'-(benzyloxy)-7-bromo-2'-(methylthio)-3,4,5',8'-tetrahydro-2H-spiro[naphthalene-1,7'-pyrano[4,3-*d*]pyrimidine] (3.08 g, 1 *equiv.,* 6.37 mmol), cesium carbonate (6.23 g, 3 *equiv.,* 19.1 mmol), and *tert-*butyl carbamate (2.24 g, 3 *equiv.,* 19.1 mmol) in 1,4-dioxane (90 mL) was degassed for 5 minutes. BrettPhos Pd G4 (587 mg, 0.1 *equiv.,* 0.637 mmol) was added, and the resulting mixture was degassed at room temperature for another 5 minutes. The mixture was then stirred at 80 °C for 6 hours. The reaction mixture was concentrated to a residue which was purified by flash column chromatography (silica, 120 g silicycle cartridge, gradient of EtOAc in DCM, (0-100%) to afford tert-butyl (S)-(4'-(benzyloxy)-2'-(methylthio)-3,4,5',8'-tetrahydro-2*H*-spiro[naphthalene-1,7'-pyrano[4,3-*d*]pyrimidin]-7-yl)carbamate (2.27 g).
LCMS: m/z (ESI) [M+H]⁺ 520.3, t_{R} = 2.14 minutes (Method A)
¹H NMR (400 MHz, CDCl₃): δ 7.44-7.30 (m, 7 H), 7.03 (d, 1 H), 6.37 (s, 1 H), 5.47 (s, 2 H), 4.74-4.64 (m, 2 H), 3.14-2.90 (m, 2 H), 2.79-2.76 (m, 2 H), 2.55 (s, 3 H), 1.97-1.91 (m, 3 H), 1.74-1.72 (m, 1 H), 1.49 (s, 9 H).

### Step 3: tert-Butyl (S)-(4'-(benzyloxy)-2'-(methylsulfonyl)-3,4,5',8'-tetrahydro-2H-spiro[naphthalene-1,7'-pyrano[4,3-d]pyrimidin]-7-yl)carbamate

Tetrabutylammonium hydrogen sulfate (237 mg, 0.16 *equiv.,* 0.70 mmol) and sodium tungstate dihydrate (144 mg, 0.1 *equiv.,* 0.44 mmol) were added to a yellow solution of *tert-*butyl (*S*)-(4'-(benzyloxy)-2'-(methylthio)-3,4,5',8'-tetrahydro-2*H*-spiro[naphthalene-1,7'-pyrano[4,3-*d*]pyrimidin]-7-yl)carbamate (2.27 g, *1 equiv.,* 4.37 mmol) in EtOAc (90 mL). The temperature of the solution was raised to 40 °C followed by the dropwise addition of hydrogen peroxide (3.47 g, 3.12 mL, 30 wt%, 7 *equiv.,* 30.6 mmol). The mixture was stirred at 40 °C for 80 minutes. The crude reaction mixture was then diluted with EtOAc (100 mL), washed with water (100 mL), brine (100 mL), dried over anhydrous sodium sulfate, and concentrated. DCM (20 mL) was added, and the material was concentrated to dryness twice to yield tert-butyl (S)-(4'-(benzyloxy)-2'-(methylsulfonyl)-3,4,5',8'-tetrahydro-2*H*-spiro[naphthalene-1,7'-pyrano[4,3-d]pyrimidin]-7-yl)carbamate (2.39 g), which was used without further purification.
LCMS: m/z (ESI) [M-H]⁻ 550.3, t_{R} = 1.86 minutes (Method A)
¹H NMR (400 MHz, CDCl₃): δ 7.48-7.38 (m, 6 H), 7.19 (dd,1 H), 7.05 (d, 1H), 6.38 (s, 1H), 5.56 (s, 2H), 4.82-4.71 (m, 2H), 3.31-3.09 (m, 5H), 2.82-2.74 (m, 2H), 2.00-1.91 (m, 3H), 1.74-1.70 (m, 1H), 1.48 (s, 9H).

### Step 4: tert-Butyl ((S)-4'-(benzyloxy)-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-7-yl)carbamate

NaH (433 mg, 60 wt%, dispersion in mineral oil, 2.5 *equiv.,* 10.8 mmol) was added to a solution of ((2*R*,7a*S*)-2-fluorotetrahydro-1*H*-pyrrolizin-7a(5*H*)-yl)methanol (1.72 g, 2.5 *equiv.,* 10.8 mmol) in anhydrous THF (24 mL). The mixture was stirred for 2 minutes at room temperature until gas evolution ceased. A solution of *tert*-butyl (*S*)-(4'-(benzyloxy)-2'-(methylsulfonyl)-3,4,5',8'-tetrahydro-2*H*-spiro[naphthalene-1,7'-pyrano[4,3-*d*]pyrimidin]-7-yl)carbamate (2.39 g, 1 *equiv.,* 4.33 mmol) in anhydrous THF (24 mL) was added over 1 minute and the resulting mixture was stirred for 70 minutes. A mixture of water (20 mL) and brine (20 mL) was added, and the aqueous phase was extracted with DCM:MeOH 9:1 (3 × 100 mL). The combined organic phases were washed with brine (100 mL), dried over anhydrous sodium sulfate, filtered, and concentrated to a residue, which was purified using flash column chromatography (silica, 120 g silicycle cartridge, gradient of DCM:MeOH:NH₄OH 85:13.5:1.5/DCM (0-100%) to yield *tert*-butyl ((*S*)-4'-(benzyloxy)-2'-(((2*R*,7a*S*)-2-fluorotetrahydro-1*H*-pyrrolizin-7a(5*H*)-yl)methoxy)-3,4,5',8'-tetrahydro-2*H-***spiro[naphthalene-1,7'-pyrano[4,3-*d*]pyrimidin]-7-yl)carbamate** (1.89 g).
LCMS: m/z (ESI) [M+H]⁺ 631.5, t_{R} = 1.43 minutes (Method A)
¹H NMR (400 MHz, CDCl₃): δ 7.36-7.22 (m, 7 H), 7.04 (d, 1H), 6.37 (s, 1H), 5.44 (s, 2H), 5.26 (d, 1H), 4.72-4.63 (m, 2 H), 4.16-4.02 (m, 2 H), 3.27-2.71 (m, 8 H), 2.27-2.12 (m, 3 H), 1.98-1.82 (m, 6 H), 1.74-1.70 (m, 1 H), 1.49 (s, 9 H).
¹⁹F NMR (376 MHz, CDCl₃): δ -173.2.

### Step 5: tert-Butyl ((S)-2'-(((2R,7aS)-2-fluorotetrahydro-1H-pyrrolizin-7a(5H)-yl)methoxy)-4'-oxo-3,3',4,4',5',8'-hexahydro-2H-spiro[naphthalene-1,7'-pyrano[4,3-d]pyrimidin]-7-yl)carbamate

To a degassed solution of *tert-butyl* ((*S*)-4'-(benzyloxy)-2'-(((2*R*,7a*S*)-2-fluorotetrahydro-1*H*-pyrrolizin-7a(5*H*)-yl)methoxy)-3,4,5',8'-tetrahydro-2*H-*spiro[naphthalene-1,7'-pyrano[4,3-*d*]pyrimidin]-7-yl)carbamate (1.89 g, 1 *equiv.,* 3.00 mmol) in MeOH (60 mL) was added Pd on carbon (159 mg, 10 wt%, wet, 0.05 *equiv.,* 0.15 mmol). The suspension was degassed for 5 minutes. Hydrogen was then bubbled through the mixture at room temperature for 5 minutes. The mixture was stirred at room temperature for 2 hours. The reaction mixture was purged with nitrogen, diluted with DCM (50 mL), and filtered. The solid was washed with DCM (3 × 10 mL), and the combined filtrates were concentrated under reduced pressure to yield tert-butyl ((*S*)-2'-(((2*R*,7a*S*)-2-fluorotetrahydro-1*H*-pyrrolizin-7a(5*H*)-yl)methoxy)-4'-oxo-3,3',4,4',5',8'-hexahydro-2H-spiro[naphthalene-1,7'-pyrano[4,3-*d*]pyrimidin]-7-yl)carbamate (1.62 g).
LCMS: m/z (ESI) [M+H]⁺ 541.3, t_{R} = 1.05 minutes (Method A)
¹H NMR (400 MHz, DMSO-*d₆*): δ 12.29 (s, 1H), 9.17 (s, 1H), 7.51 (s, 1H), 7.23 (dd, 1H), 6.97 (d, 1H), 5.26 (d, 1H), 4.38-4.24 (m, 2H), 4.03-3.92 (m, 2H), 3.13-2.96 (m, 3H), 2.81-2.54 (m, 5H), 2.09-1.63 (m, 10H), 1.43 (s, 9 H).
¹⁹F NMR (376 MHz, DMSO): δ -172.0.

### Intermediate 5: tert-Butyl ((S)-4'-((1H-benzo[d][1,2,3]triazol-1-yl)oxy)-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-7-yl)carbamate

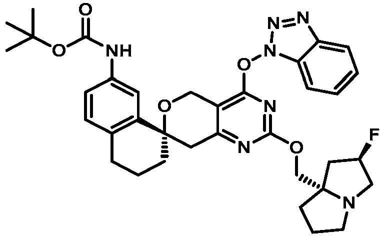

BOP (972 mg, 1.1 *equiv.,* 2.20 mmol) was slowly added to a solution of *tert*-butyl ((*S*)-2'-(((2*R*,7a*S*)-2-fluorotetrahydro-1*H*-pyrrolizin-7a(5*H*)-yl)methoxy)-4'-hydroxy-3,4,5',8'-tetrahydro-2*H*-spiro[naphthalene-1,7'-pyrano[4,3-*d*]pyrimidin]-7-yl)carbamate (1.08 g, 1 *equiv.,* 2.00 mmol) and DIPEA (516 mg, 2 *equiv.,* 4.00 mmol) in DMF (20 mL). The resulting colorless solution was stirred at room temperature for 3 hours. The mixture was poured into water (20 mL) and stirred for 5 minutes. The resulting precipitate was removed by filtration, washed with water (3 × 10 mL) and dried under vacuum to yield *tert*-butyl ((*S*)-4'-((1*H-*benzo[*d*][1,2,3]triazol-1-yl)oxy)-2'-(((2*R*,7a*S*)-2-fluorotetrahydro-1*H*-pyrrolizin-7a(5*H*)-yl)methoxy)-3,4,5',8'-tetrahydro-2*H*-spiro[naphthalene-1,7'-pyrano[4,3-*d*]pyrimidin]-7-yl)carbamate (1.19 g).
LCMS: m/z (ESI) [M+H]⁺ 658.4, t_{R} = 1.37 minutes (Method A).
¹H NMR (400 MHz, DMSO-*d₆*): δ 9.23 (s, 1H), 8.18 (d, 1H), 7.86 (d, 1H), 7.62-7.67 (m, 2H), 7.53 (t, 1H), 7.23 (dd, 1H), 7.02 (d, 1H), 5.12 (s, 1H), 4.91 (t, 2H), 3.49-3.61 (m, 2H), 2.97-3.09 (m, 2H), 2.87 (d, 1H), 2.80-2.82 (m, 1H), 2.76-2.79 (m, 1H), 2.71-2.74 (m, 2H), 2.62-2.68 (m, 2H), 1.99 (s, 2H), 1.90 (br s, 1H), 0.85-1.68 (m, 3H), 1.64 (dd, 2H), 1.50-1.58 (m, 1H), 1.40-1.43 (m, 9H).
¹⁹F NMR (376 MHz, DMSO): F -172.46 to -171.99 (m).

### Intermediate 6: N,N-Dibenzyl-8-bromo-4'-chloro-2'-(methylthio)-3,4,5',8'-tetrahydro-2H-spiro[naphthalene-1,7'-pyrano[4,3-d]pyrimidin]-7-amine

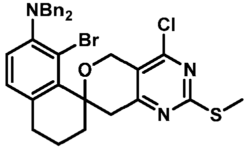

### Step 1: 7-Amino-8-bromo-3,4-dihydronaphthalen-1(2H)-one

A solution of 1-bromopyrrolidine-2,5-dione (2.86 g, 1 *equiv.,* 16.1 mmol) in DMF (20 mL) was added dropwise to a solution of 7-amino-3,4-dihydronaphthalen-1(2*H*)-one (2.59 g, 1 *equiv.,* 16.1 mmol) in DMF (20 mL) at 0 °C. The resulting mixture was stirred for 4 hours at room temperature and then poured into water. The mixture was extracted with DCM three times, and the combined extracts were washed with water. The combined organic phases were dried over sodium sulfate and passed through a Celite pad. The filtrate was concentrated to a residue which was purified by column chromatography (80 g, 0-20% hexane/EtOAc) to afford 7-amino-8-bromo-3,4-dihydronaphthalen-1(2H)-one (3.31 g).

LCMS: m/z (ESI) [M+H]⁺ 239.9, t_{R} = 1.02 minutes (Method A).

¹H NMR (400 MHz, CDCl₃): *δ* 7.01 (d, 1H), 6.88 (d, 1H), 4.30 (s, 2 H), 2.86 (t, 2 H), 2.66 (t, 2H), 2.03-2.09 (m, 2 H).

### Step 2: 8-Bromo-7-(dibenzylamino)-3,4-dihydronaphthalen-1(2H)-one

Potassium carbonate (5.36 g, 3.5 *equiv.,* 38.8 mmol) was added to 7-amino-8-bromo-3,4-dihydronaphthalen-1(2*H*)-one (2.66 g, 1 *equiv.,* 11.1 mmol) in anhydrous DMF (55.4 mL) followed by the dropwise addition of benzyl bromide (4.71 mL, 3.5 *equiv.,* 38.8 mmol) at room temperature. The resulting mixture was stirred at 70 °C for 16 hours. Water was added, and the aqueous layer was extracted with EtOAc three times. The combined extracts were washed with brine, dried over sodium sulfate, filtered, and evaporated to a residue, which was purified by column chromatography (80 g silica, 0-5% hexane/EtOAc) to afford 8-bromo-7-(dibenzylamino)-3,4-dihydronaphthalen-1(2H)-one (4.25 g).

LCMS: m/z (ESI) [M+H]⁺ 422.2, t_{R} = 1.99 minutes (Method A).

### Step 3: 8-Bromo-1-((6-chloro-5-(hydroxymethyl)-2-(methylthio)pyrimidin-4-yl)methyl)-7-(dibenzylamino)-1,2,3,4-tetrahydronaphthalen-1-ol

LDA (26.9 mL, 1 M, 2.5 *equiv.,* 26.9 mmol) was added to a solution of (4-chloro-6-methyl-2-(methylthio)pyrimidin-5-yl)methanol (2.20 g, *1 equiv.,* 10.8 mmol) in THF (108 mL) at -78 °C over 15 minutes. The mixture was stirred at that temperature for 1 hour, and then a solution of 8-bromo-7-(dibenzylamino)-3,4-dihydronaphthalen-1(2H)-one (4.970 g, 1.1 *equiv.,* 11.8 mmol) in THF (50 mL) was added over 15 minutes. The reaction mixture was stirred at -78 °C for 1 hour and then quenched with aqueous ammonium chloride. The reaction mixture was warmed slowly to room temperature, extracted with EtOAc, and washed with brine. The combined organic layers were dried over sodium sulfate, filtered, and concentrated to a residue which was purified by flash column chromatography (120 g silica, 0-20% hexane/EtOAc) to give 8-bromo-1-((6-chloro-5-(hydroxymethyl)-2-(methylthio)pyrimidin-4-yl)methyl)-7-(dibenzylamino)-1,2,3,4-tetrahydronaphthalen-1-ol (4 g).

LCMS: m/z (ESI) [M+H]⁺ 626.1, t_{R} = 2.07 minutes (Method B).

### Step 4: N,N-Dibenzyl-8-bromo-4'-chloro-2'-(methylthio)-3,4,5',8'-tetrahydro-2H-spiro[naphthalene-1,7'-pyrano[4,3-d]pyrimidin]-7-amine

n-Butyllithium (5.70 mL, 1.6 M, 2.5 *equiv.,* 9.12 mmol) was added dropwise to a solution of 8-bromo-1-((6-chloro-5-(hydroxymethyl)-2-(methylthio)pyrimidin-4-yl)methyl)-7-(dibenzylamino)-1,2,3,4-tetrahydronaphthalen-1-ol (2.28 g, *1 equiv.,* 3.65 mmol) in THF (25.6 mL) at -65 °C. The reaction mixture was stirred for 30 minutes. A solution of 4-methylbenzenesulfonyl chloride (1.04 g, 1.5 *equiv.,* 5.47 mmol) in THF (20 mL) was added dropwise at -65 °C, and the resulting mixture was warmed to room temperature and stirred for 2 hours. Saturated aqueous ammonium chloride solution was added, and the mixture was extracted with EtOAc three times. The organic phases were combined, washed with brine, dried over sodium sulfate, filtered, and concentrated under reduced pressure to give a residue, which was purified by flash chromatography (80 g, 0-10% hexane/EtOAc) to give *N,N*-dibenzyl-8-bromo-4'-chloro-2'-(methylthio)-3,4,5',8'-tetrahydro-2*H*-spiro[naphthalene-1,7'-pyrano[4,3-d]pyrimidin]-7-amine (1.7 g).

LCMS: m/z (ESI) [M+H]⁺ 608.0, t_{R} = 2.31 minutes (84%, Method B).

### Intermediate 7a: (R)-7-bromo-4-methyl-3,4-dihydronaphthalen-1(2H)-one

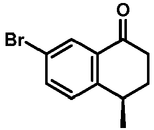

Aluminum chloride (20.8 g, 2.5 *equiv.,* 156 mmol) was stirred rapidly as 4-methyl-3,4-dihydronaphthalen-1(2H)-one (10.0 g, 1 *equiv.,* 62.4 mmol) was added dropwise by syringe over 20 minutes. The resulting dark-red mass was heated at 85 °C until it melted, at which point Br₂ (12.0 g, 3.86 mL, 1.2 *equiv.,* 74.9 mmol) was added dropwise by syringe over 40 minutes. The mixture was stirred for 1 hour at 85 °C. The reaction mixture was cooled to room temperature then added to ice-water (500 mL) and concentrated HCl (100 mL). The resulting mixture was extracted with EtOAc (4 × 300 mL), and the combined organic layers were filtered through Celite. The filtrate was washed with saturated aqueous sodium bicarbonate solution (250 mL), water (250 mL) and brine (250 mL), dried over sodium sulfate, filtered, and concentrated to give a residue. The residue was purified by reverse-phase HPLC (0.1% formic acid condition) to give 7-bromo-4-methyl-3,4-dihydronaphthalen-1(2*H*)-one (10.5 g).

LCMS: m/z (ESI) [M+H]⁺ 240.9, t_{R} = 0.462 minutes (Method H).

The enantiomers of 7-bromo-4-methyl-3,4-dihydronaphthalen-1(2*H*)-one (5 g, 20.9 mmol) were separated by chiral SFC (Column: Chiralpak AD-3 50 × 4.6 mm I.D., 3 µm Mobile phase: Phase A for CO₂, and Phase B for MeOH (0.05% DEA), Gradient elution: B in A from 5% to 40% Flow rate: 3 mL/min, Detector: PDA, Column Temp: 35 °C, Back Pressure: 100 bar) to afford (*R*)-7-bromo-4-methyl-3,4-dihydronaphthalen-1(2*H*)-one (**Intermediate 7a**) and (*S*)-7-bromo-4-methyl-3,4-dihydronaphthalen-1(2*H*)-one (**Intermediate 7b**).

### Intermediate 7a: First eluting peak, t_{R} = 1.027 minutes (1780 mg)

¹H NMR (400 MHz, CD₃OD) δ 8.03 (d, 1H), 7.69 (dd, 1H), 7.36 (d, 1H), 3.15 - 3.04 (m, 1H), 2.84 - 2.74 (m, 1H), 2.66 - 2.55 (m, 1H), 2.31 - 2.20 (m, 1H), 1.96 - 1.84 (m, 1H), 1.40 (d, 3H).

### Intermediate 7b: (S)-7-bromo-4-methyl-3,4-dihydronaphthalen-1(2H)-one

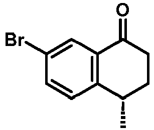

### Intermediate 7b: Second eluting peak, t_{R} = 1.167 minutes (1549 mg)

¹H NMR (400 MHz, CD₃OD) δ 8.03 (d, 1H), 7.69 (dd, 1H), 7.36 (d, 1H), 3.17 - 3.01 (m, 1H), 2.85 - 2.73 (m, 1H), 2.69 - 2.53 (m, 1H), 2.31 - 2.20 (m, 1H), 1.96 - 1.82 (m, 1H), 1.40 (d, 3H).

### Intermediate 8: 4-Chloro-2-(methylthio)-5,6',7',8-tetrahydro-5'H-spiro[pyrano[4,3-d]pyrimidine-7,8'-quinoline]

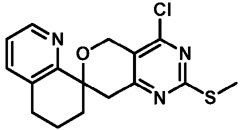

### Step 1: 8-((6-Chloro-5-(hydroxymethyl)-2-(methylthio)pyrimidin-4-yl)methyl)-5,6,7,8-tetrahydroquinolin-8-ol

LDA (2.889 g, 26.97 mL, 1 M, 2.3 *equiv.,* 26.97 mmol) was added dropwise to (4-chloro-6-methyl-2-(methylthio)pyrimidin-5-yl)methanol (2.400 g, 1 *equiv.,* 11.7 mmol) in THF (40 mL) at -78 °C, while keeping the internal temperature below -70 °C. The resulting mixture was kept at -78 °C for 1.5 hours. An orange solid formed, and additional THF (5 mL) was added. 6,7-Dihydroquinolin-8(5H)-one (2.071 g, 1.2 *equiv.,* 14.07 mmol) was added dropwise as a solution in THF (20 mL), and the resulting mixture was then stirred at - 78 °C for 1.5 hours. The reaction was quenched at -78 °C with saturated aqueous ammonium chloride and diluted with EtOAc. The aqueous phase was extracted with EtOAc four times. The combined organic layers were dried over sodium sulfate, filtered, and concentrated to a residue which was triturated with EtOAc to afford 8-((6-chloro-5-(hydroxymethyl)-2-(methylthio)pyrimidin-4-yl)methyl)-5,6,7,8-tetrahydroquinolin-8-ol as a yellow solid (2.66 g).

¹H NMR (400 MHz, CDCl₃) δ 8.36 (dd, 1H), 7.47 (dq, 1H), 7.17 (dd, 1H), 4.91 (s, 1H), 4.85 (d, 2H), 4.36 - 4.25 (m, 1H), 3.58 (d, 1H), 3.20 (d, 1H), 2.93 - 2.85 (m, 2H), 2.46 (s, 3H), 2.24 - 2.09 (m, 2H), 2.03 - 1.93 (m, 1H), 1.91 - 1.80 (m, 1H).

### Step 2: 4-Chloro-2-(methylthio)-5,6',7',8-tetrahydro-5'H-spiro[pyrano[4,3-d]pyrimidine-7,8'-quinoline]

Polymer-bound triphenylphosphine (2.38 g, 1.3 *equiv.,* 3.81 mmol) was added to 8-((6-chloro-5-(hydroxymethyl)-2-(methylthio)pyrimidin-4-yl)methyl)-5,6,7,8-tetrahydroquinolin-8-ol (1.03 g, 1 *equiv.,* 2.93 mmol) in THF (14 mL). The resulting mixture solidified and was cooled to -78 °C. DIAD (770 mg, 740 µL, 1.3 *equiv.,* 3.81 mmol) was added dropwise. The cold bath was removed, and the resulting reaction mixture was stirred for 1 hour. The reaction mixture was filtered to remove triphenylphosphine oxide and rinsed with DCM. The filtrate was concentrated to a residue which was purified by flash chromatography (24 g silica gel cartridge with gradient elution from 0:100 EtOAc:heptane to 100:0 EtOAc:heptane) to give 4-chloro-2-(methylthio)-5,6',7',8-tetrahydro-5'*H*-spiro[pyrano[4,3-*d*]pyrimidine-7,8'-quinoline] (570 mg).

¹H NMR (400 MHz, CDCl₃) δ 8.40 (dd, 1H), 7.48 (d, 1H), 7.16 (dd, 1H), 4.75 - 4.53 (m, 2H), 3.76 (dd, 1H), 3.00 - 2.75 (m, 3H), 2.58 (s, 3H), 2.26 - 2.16 (m, 1H), 2.16 - 2.06 (m, 1H), 1.93 - 1.77 (m, 2H).

### Intermediate 9: 6-Bromo-4'-chloro-2'-(methylthio)-5',8'-dihydrospiro[isochromane-4,7'-pyrano[4,3-d]pyrimidine]

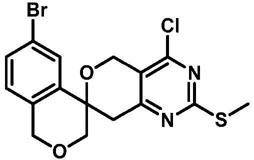

### Step 1: 6-bromo-4-((6-chloro-5-(hydroxymethyl)-2-(methylthio)pyrimidin-4-yl)methyl)isochroman-4-ol

To a dry flask was added (4-chloro-6-methyl-2-(methylthio)pyrimidin-5-yl)methanol (2.00 g, 1 *equiv.,* 9.77 mmol) and THF (40.0 mL). The mixture was cooled to -78 °C. LDA solution (2.62 g, 24.4 mL, 1.0 M, 2.5 *equiv.,* 24.4 mmol) was added dropwise at -78 °C, maintaining an internal temperature below -60 °C. After 1 hour, a degassed solution of 6-bromoisochroman-4-one (2.22 g, *1 equiv.,* 9.77 mmol) in THF (30.0 mL) was added dropwise, and the resulting mixture was stirred at -78 °C for 10 minutes. The reaction mixture, while still cold, was added into a round-bottom flask containing room temperature saturated aqueous ammonium chloride (80 mL). The mixture was then extracted with EtOAc (80 mL × 2) followed by DCM (80 mL × 2). The combined organic layers were dried over sodium sulfate, filtered, and concentrated under reduced pressure to give a residue which was purified by column chromatography (220 g SiO₂ cartridge, 0-40% EtOAc in heptane) to give 6-bromo-4-((6-chloro-5-(hydroxymethyl)-2-(methylthio)pyrimidin-4-yl)methyl)isochroman-4-ol (1.84 g).

¹H NMR (400 MHz, CDCl₃) δ 7.85 (m, 1H), 7.41 (m, 1H), 6.90 (m, 1H), 5.52 (s, 1H), 4.93 - 4.68 (m, 5H), 3.64 - 3.49 (m, 3H), 3.26 (m, 1H), 3.05 (m, 1H).

### Step 2: 6-Bromo-4'-chloro-2'-(methylthio)-5',8'-dihydrospiro[isochromane-4,7'-pyrano[4,3-d]pyrimidine]

To a vial of 6-bromo-4-((6-chloro-5-(hydroxymethyl)-2-(methylthio)pyrimidin-4-yl)methyl)isochroman-4-ol (736 mg, 1 *equiv.,* 1.70 mmol) was added triphenylphosphine (2.13 g, 2.0 *equiv.,* 3.41 mmol) (polymer supported, ~1.6 mmol/g loading). The vial was degassed and filled with N₂ followed by the addition of dry THF (20.0 mL) and dry DCM (20.0 mL). To this mixture was added dropwise diisopropyl (*E*)-diazene-1,2-dicarboxylate (345 mg, 1 *equiv.,* 1.70 mmol) at ambient temperature. The reaction mixture was stirred for 4 hours. The reaction mixture was then diluted with EtOAc (100 mL) and then DCM (100 mL). The mixture was then filtered over Celite and sodium sulfate and concentrated to a residue which was purified by column chromatography (50 g SiO₂ cartridge, 0-40% EtOAc in heptane) to give 6-bromo-4'-chloro-2'-(methylthio)-5',8'-dihydrospiro[isochromane-4,7'-pyrano[4,3-*d*]pyrimidine] (383 mg).

¹H NMR (400 MHz, DMSO*-d₆*) δ 7.69 (d, 1H), 7.51 (dd, 1H), 7.11 (d, 1H), 4.82 (m, 1H), 4.74 - 4.65 (m, 3H), 3.93 (m, 1H), 3.78 (m, 1H), 3.25 (m, 1H), 2.99 (m, 1H), 2.53 (s, 3H).

### Intermediate 10: (3R)-1-(6-Bromo-2'-(((2R,7aS)-2-fluorotetrahydro-1H-pyrrolizin-7a(5H)-yl)methoxy)-2,3,5',8'-tetrahydrospiro[indene-1,7'-pyrano[4,3-d]pyrimidin]-4'-yl)-3-methylpiperidin-3-ol

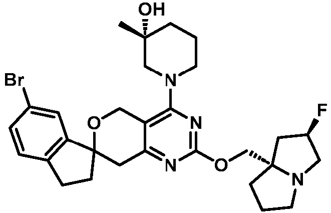

### Step 1: Methyl 4-(6-bromo-1-hydroxy-2,3-dihydro-1H-inden-1-yl)-3-oxobutanoate

NaH (1.00 g, 60 wt%) was suspended in THF (50 mL) and cooled to 0 °C. Methyl 3-oxobutanoate (4.00 g) was added, while maintaining an internal temperature below 10 °C. The solution was cooled to -75 °C, and LDA (4.00 g) was added dropwise, while maintaining a temperature below -68 °C. The reaction mixture was gradually warmed to 0 °C and cooled to - 78 °C followed by dropwise addition of 6-bromo-2,3-dihydro-1*H*-inden-1-one (5.00 g) in THF (10 mL), while maintaining a temperature below -68 °C. The reaction mixture was stirred and gradually warmed to 10 °C over the course of 4 hours. The reaction was quenched by the addition of aqueous ammonium chloride and EtOAc (50 mL). The mixture was and further extracted with EtOAc (2 × 40 mL). The combined organic layers were dried over sodium sulfate, filtered, and concentrated to a crude product, which was purified by column chromatography (SiO₂, heptanes/EtOAc = 100/0 to 10/6) to give methyl 4-(6-bromo-1-hydroxy-2,3-dihydro-1*H*-inden-1-yl)-3-oxobutanoate (4.50 g).

LCMS: m/z (ESI) [M-H]⁻ 325.2, t_{R} = 1.70 minutes (Method C).

¹H NMR (400 MHz, CDCl₃) δ 7.41-7.49 (m, 1H), 7.33-7.39 (m, 1H), 7.06-7.13 (m, 1H), 3.81 (s, 1H), 3.74 (s, 3H), 3.42-3.58 (m, 2H), 3.06-3.16 (m, 1H), 2.89-3.04 (m, 2H), 2.70-2.82 (m, 1H), 2.23-2.33 (m, 2H).

### Step 2: Methyl 6-bromo-4'-oxo-2,3,3',4'-tetrahydrospiro[indene-1,2'-pyran]-5'-carboxylate

DMA-DMF (1.8 g, 2.0 mL, 1.1 *equiv.,* 15 mmol) was added to methyl 4-(6-bromo-1-hydroxy-2,3-dihydro-1*H*-inden-1-yl)-3-oxobutanoate (4.50 g, 1 *equiv.,* 14 mmol) in DCM (100 mL) and the resulting mixture was stirred for 6 hours at room temperature. The mixture was cooled to 0 °C, followed by the dropwise addition of BF₃·OEt₂ (2.1 g, 1.9 mL, 1.1 *equiv.,* 15 mmol). The mixture was warmed to room temperature over 2 hours. The reaction mixture was quenched with aqueous sodium bicarbonate and extracted with EtOAc (2 × 30 mL). The combined organic extracts were dried over sodium sulfate, filtered, and concentrated to a crude product, which was purified by column chromatography (SiO₂, heptanes/EtOAc = 100/0 to 10/6) to give methyl 6-bromo-4'-oxo-2,3,3',4'-tetrahydrospiro[indene-1,2'-pyran]-5'-carboxylate (3.45 g).

¹H NMR (400 MHz, CDCl₃) δ 8.25-8.30 (m, 1H), 7.46-7.54 (m, 2H), 7.17-7.24 (m, 1H), 3.83 (s, 3H), 3.01-3.16 (m, 2H), 2.83-2.95 (m, 1H), 2.68-2.77 (m, 1H), 2.44-2.55 (m, 1H), 2.24-2.36 (m, 1H).

### Step 3: Methyl 6-bromo-4'-oxo-2,3,3',4',5',6'-hexahydrospiro[indene-1,2'-pyran]-5'-carboxylate

Methyl 6-bromo-4'-oxo-2,3,3',4'-tetrahydrospiro[indene-1,2'-pyran]-5'-carboxylate (5.2 g, 1 *equiv.,* 15 mmol) in THF (80 mL) was cooled to -78 °C followed by the addition of L-selectride (17 mL, 1 M, 1.1 *equiv.,* 17 mmol). The resulting mixture was stirred for 2 hours. The reaction was quenched with aqueous ammonium chloride and extracted with EtOAc (2 × 20 mL). The combined organic layers were dried with magnesium sulfate, filtered, and concentrated to a crude product, which was purified by column chromatography (SiO₂, heptanes/EtOAc = 100/0 to 10/6) to give methyl 6-bromo-4'-oxo-2,3,3',4',5',6'-hexahydrospiro[indene-1,2'-pyran]-5'-carboxylate (4.40 g).

¹H NMR (400 MHz, CDCl₃) δ 7.37-7.46 (m, 2H), 7.10-7.17 (m, 1H), 4.29-4.39 (m, 1H), 4.17-4.26 (m, 1H), 4.07-4.16 (m, 1H), 3.80 (s, 3H), 2.97-3.09 (m, 1H), 2.73-2.84 (m, 1H), 2.50-2.60 (m, 2H), 2.25-2.35 (m, 1H), 2.05-2.15 (m, 1H).

### Step 4: 6-Bromo-2'-(methylthio)-2,3,5',8'-tetrahydrospiro[indene-1,7'-pyrano[4,3-d]pyrimidin]-4'-ol

Methyl 6-bromo-4'-oxo-2,3,3',4',5',6'-hexahydrospiro[indene-1,2'-pyran]-5'-carboxylate (3.00 g, 1 *equiv.,* 9 mmol) in MeCN (50 mL) was treated with thiourea (0.9 g, 1.3 *equiv.,* 0.01 mol) and DBU (2.00 g, 1.6 *equiv.,* 0.01 mol). The mixture was heated to 75 °C for about 3 hours and then stirred at room temperature for 16 hours. Aqueous sodium hydroxide (0.6 g, 0.01 L, 1.0 M, 1.6 *equiv.,* 0.01 mol) and iodomethane (2.00 g, 1.4 *equiv.,* 0.01 mol) were added, and the resulting mixture was stirred at room temperature for 2 hours. The reaction was then quenched with aqueous ammonium chloride, diluted with DCM (40 mL), and extracted with DCM (2 × 30 mL). The organic layers were dried with magnesium sulfate, filtered, and concentrated to a crude product, which was purified by column chromatography (SiO₂, DCM/MeOH = 100/0 to 10/2). The product was precipitated from MeCN to give 6-bromo-2'-(methylthio)-2,3,5',8'-tetrahydrospiro[indene-1,7'-pyrano[4,3*-d*]pyrimidin]-4'-ol (550 mg).
LCMS: m/z (ESI) [M+H]⁺ 381.1, t_{R} = 1.88 minutes
¹H NMR (400 MHz, CDCl₃) δ 7.40 (dd, 1H), 7.33 (d, 1H), 7.16 (d, 1H), 4.60 (q, 2H), 3.03-3.11 (m, 1H), 2.83-2.92 (m, 3H), 2.59 (s, 3H), 2.31-2.38 (m, 1H), 2.08-2.17 (m, 1H).

### Step 5: 6-Bromo-4'-chloro-2'-(methylthio)-2,3,5',8'-tetrahydrospiro[indene-1,7'-pyrano[4,3-d]pyrimidine]

6-Bromo-2'-(methylthio)-2,3,5',8'-tetrahydrospiro[indene-1,7'-pyrano[4,3-d]pyrimidin]-4'-ol (550 mg, 1 *equiv.,* 1.45 mmol) in MeCN (5 mL) was treated with POCl₃ (1.78 g, 8 *equiv.,* 11.6 mmol) and heated to 80 °C for 1 hour. The reaction mixture was cooled to room temperature. The mixture was then quenched with aqueous sodium bicarbonate and extracted with EtOAc (3 × 15 mL). The organic layers were dried over sodium sulfate, filtered, and concentrated to give 6-bromo-4'-chloro-2'-(methylthio)-2,3,5',8'-tetrahydrospiro[indene-1,7' -pyrano[4,3-d]pyrimidine] which was used without further purification.

¹H NMR (400 MHz, CDCl₃) δ 7.42 (dd, 1H), 7.23 (d, 1H), 7.17 (d, 1H), 4.74 (q, 2H), 3.00-3.12 (m, 3H), 2.79-2.87 (m, 1H), 2.58 (s, 3H), 2.32-2.38 (m, 1H), 2.07-2.12 (m, 1H).

### Step 6: (3R)-1-(6-bromo-2'-(methylthio)-2,3,5',8'-tetrahydrospiro[indene-1,7'-pyrano[4,3-d]pyrimidin]-4'-yl)-3-methylpiperidin-3-ol

6-Bromo-4'-chloro-2'-(methylthio)-2,3,5',8'-tetrahydrospiro[indene-1,7'-pyrano[4,3-d]pyrimidine] (833 mg, 1 *equiv.,* 2.09 mmol) and (*R*)-3-methylpiperidin-3-ol hydrochloride (476 mg, 1.5 *equiv.,* 3.14 mmol) in EtOH (10 mL) was treated with DIPEA (406 mg, 1.5 *equiv.,* 3.14 mmol). The reaction mixture was heated to 70 °C and stirred for 2 hours. The reaction was quenched with aqueous ammonium chloride and extracted with EtOAc (2 × 20 mL). The combined organic layers were dried with magnesium sulfate, filtered, and concentrated to a crude product, which was purified by column chromatography (SiO₂, heptanes/EtOAc = 100/0 to 10/6) to give (3*R*)-1-(6-bromo-2'-(methylthio)-2,3,5',8'-tetrahydrospiro[indene-1,1'-pyrano[4,3-*d*]pyrimidin]-4'-yl)-3-methylpiperidin-3-ol (572 mg).
LCMS: m/z (ESI) [M+H]⁺ 477.7, t_{R} = 1.78 minutes (Method C)
¹H NMR (400 MHz, CDCl₃) δ 7.38-7.44 (m, 1H), 7.18-7.31 (m, 2H), 4.35-4.66 (m, 2H), 3.72-3.85 (m, 1H), 3.52 (dd, 1H), 2.94-3.17 (m, 5H), 2.76-2.89 (m, 1H), 2.54 (s, 3H), 2.31-2.42 (m, 1H), 2.13-2.25 (m, 1H), 1.76-1.96 (m, 2H), 1.49-1.65 (m, 3H), 1.25 (s, 3H).

### Step 7: (3R)-1-(6-bromo-2'-(((2R,7aS)-2-fluorotetrahydro-1H-pyrrolizin-7a(5H)-yl)methoxy)-2,3,5',8'-tetrahydrospiro[indene-1,7'-pyrano[4,3-d]pyrimidin]-4'-yl)-3-methylpiperidin-3-ol

(3*R*)-1-(6-Bromo-2'-(methylthio)-2,3,5',8'-tetrahydrospiro[indene-1,7'-pyrano[4,3-*d*]pyrimidin]-4'-yl)-3-methylpiperidin-3-ol (550 mg, 1 *equiv.,* 1.15 mmol) in MeOH (6 mL) and water (3 mL) was treated with Oxone (1.42 g, 2 *equiv.,* 2.31 mmol). The resulting mixture was stirred for 1 hour at room temperature. The reaction was quenched with aqueous sodium bicarbonate, diluted with EtOAc, and extracted with EtOAc (2 × 15 mL). The organic layers were dried with magnesium sulfate, filtered, and concentrated to give (3R)-1-(6-bromo-2'-(methylsulfonyl)-2,3,5',8'-tetrahydrospiro[indene-1,7'-pyrano[4,3-d]pyrimidin]-4'-yl)-3-methylpiperidin-3-ol, which was used without further purification.

NaH (160 mg, 3 *equiv.,* 4.01 mmol) was suspended in THF (3 mL) followed by the addition of (2*R*,7a*S*)-2-fluorotetrahydro-1*H*-pyrrolizin-7a(5*H*)-yl)methanol (639 mg, 3 *equiv.,* 4.01 mmol). (3*R*)-1-(6-bromo-2'-(methylsulfonyl)-2,3,5',8'-tetrahydrospiro[indene-1,7'-pyrano[4,3-*d*]pyrimidin]-4'-yl)-3-methylpiperidin-3-ol (680 mg, 1 *equiv.,* 1.34 mmol) in DMF (6 mL) was treated by the dropwise addition of the prepared alkoxide mixture and the resulting reaction mixture was stirred for 90 minutes at 50 °C. The reaction was quenched by addition of aqueous ammonium chloride, diluted with EtOAc, and extracted with EtOAc (3 × 1 mL). The organic layers were dried with sodium sulfate, filtered, and concentrated to a crude mixture, which was purified by column chromatography (SiO₂, DCM/MeOH = 100/0 to 10/2) to give (3*R*)-1-(6-bromo-2'-(((2*R*,7a*S*)-2-fluorotetrahydro-1*H*-pyrrolizin-7a(5*H*)-yl)methoxy)-2,3,5',8'-tetrahydrospiro[indene-1,7'-pyrano[4,3-*d*]pyrimidin]-4'-yl)-3-methylpiperidin-3-ol (626 mg).
LCMS: m/z (ESI) [M+H]⁺ 589.5, t_{R} = 1.43 minutes (Method C)

### Intermediate 11: (3R)-1-(2'-(((2R,7aS)-2-Fluorotetrahydro-1H-pyrrolizin-7a(5H)-yl)methoxy)-6-(4,4,5,5-tetramethyl-1,3,2-dioxaborolan-2-yl)-2,3,5',8'-tetrahydrospiro[indene-1,7'-pyrano[4,3-d]pyrimidin]-4'-yl)-3-methylpiperidin-3-ol

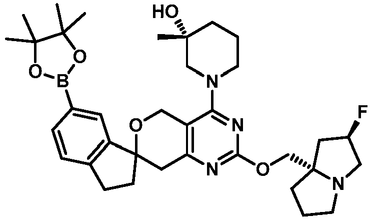

A suspension of (3*R*)-1-(6-bromo-2'-(((2*R,*7a*S*)-2-fluorotetrahydro-1*H*-pyrrolizin-7a(5*H*)-yl)methoxy)-2,3,5',8'-tetrahydrospiro[indene-1,1'-pyrano[4,3-*d*]pyrimidin]-4'-yl)-3-methylpiperidin-3-ol (105 mg, *1 equiv.,* 179 µmol), potassium acetate (26.3 mg, 1.5 *equiv.,* 268 µmol), and bis(pinacolato)diboron (81.7 mg, 1.8 *equiv.,* 322 µmol) in 1,4-dioxane (2 mL) was degassed with nitrogen, followed by the addition of Pd(dppf)Cl₂·CH₂Cl₂ (13.1 mg, 0.1 *equiv.,* 17.9 µmol). The mixture was heated to 95 °C for 1 hour and purified by column chromatography (SiO₂, DCM/MeOH = 100/0 to 10/1, DCM/MeOH with NH₄OH = 10/2) to give (3*R*)-1-(2'-(((2*R*,7a*S*)-2-fluorotetrahydro-1*H*-pyrrolizin-7a(5*H*)-yl)methoxy)-6-(4,4,5,5-tetramethyl-1,3,2-dioxaborolan-2-yl)-2,3,5',8'-tetrahydrospiro[indene-1,7'-pyrano[4,3-*d*]pyrimidin]-4'-yl)-3-methylpiperidin-3-ol (83 mg).
LCMS: m/z (ESI) [M+H]⁺ 635.8, t_{R} = 1.46 minutes (Method C)

### Intermediate 12: (3R)-1-(7-bromo-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)-3-methylpiperidin-3-ol

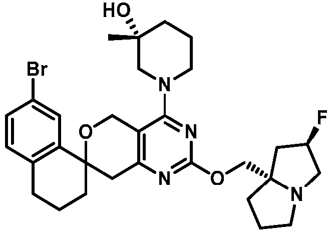

### Step 1: (3R)-1-(7-bromo-2'-(methylthio)-3,4,5',8'-tetrahydro-2H-spiro[naphthalene-1,7'-pyrano[4,3-d]pyrimidin]-4'-yl)-3-methylpiperidin-3-ol

7-Bromo-4'-chloro-2'-(methylthio)-3,4,5',8'-tetrahydro-2*H*-spiro[naphthalene- 1, 7'-pyrano[4,3-*d*]pyrimidine] (90 mg, 1 *equiv.,* 220 µmol) and (*R*)-3-methylpiperidin-3-ol hydrochloride (66 mg, 2 *equiv.,* 440 µmol) in EtOH (1.1 mL) was treated with DIPEA (57 mg, 2 *equiv.,* 440 µmol). The reaction mixture was heated to 80 °C and stirred for 1 hour. The reaction was quenched with aqueous ammonium chloride and extracted with EtOAc (3 × 15 mL). The organic layers were dried with magnesium sulfate, filtered, and concentrated to give (3*R*)-1-(7-bromo-2'-(methylthio)-3,4,5',8'-tetrahydro-2*H*-spiro[naphthalene-1,7'-pyrano[4,3-*d*]pyrimidin]-4'-yl)-3-methylpiperidin-3-ol (100 mg), which was used without further purification.
LCMS: m/z (ESI) [M+H]⁺ 491.8, t_{R} = 2.78 minutes (Method E)
¹H NMR (400 MHz, CDCl₃) δ 7.46 (dd, 1H), 7.18-7.27 (m, 1H), 6.87-6.96 (m 1H), 4.38-4.68 (m, 2H), 3.54-3.67 (m, 1H), 3.39-3.49 (m, 1H), 2.87-3.04 (m, 4H), 2.63-2.79 (m, 2H), 2.45 (s, 3H), 1.83-1.93 (m, 2H), 1.67-1.80 (m, 3H), 1.36-1.57 (m, 4H), 1.17 (s, 3H).

### Step 2: (3R)-1-(7-bromo-2'-(methylsulfonyl)-3,4,5',8'-tetrahydro-2H-spiro[naphthalene-1,7'-pyrano[4,3-d]pyrimidin]-4'-yl)-3-methylpiperidin-3-ol

7(3*R*)-1-(7-bromo-2'-(methylthio)-3,4,5',8'-tetrahydro-2*H*-spiro[naphthalene-1,7'-pyrano[4,3-*d*]pyrimidin]-4'-yl)-3-methylpiperidin-3-ol (100 mg, 1 *equiv.,* 184 µmol) and Oxone (226 mg, 2 *equiv.,* 367 µmol) in MeOH (1.22 mL) was treated with water (612 µL) and the resulting mixture was stirred for 2 hours. The reaction was quenched with aqueous ammonium chloride, diluted with EtOAc (15 mL), and extracted with EtOAc (3 × 15 mL). The organic layers were dried with magnesium sulfate, filtered, and concentrated to give (3R)-1-(7-bromo-2'-(methylsulfonyl)-3,4,5',8'-tetrahydro-2*H*-spiro[naphthalene-1,7'-pyrano[4,3-*d*]pyrimidin]-4'-yl)-3-methylpiperidin-3-ol (70 mg), which was used without further purification.
LCMS: m/z (ESI) [M+H]⁺ 524.0, t_{R} = 3.08 minutes (Method E)
¹H NMR (400 MHz, CDCl₃) δ 7.40 (d, 1H), 7.25 (d, 1H), 6.94 (dd, 1H), 4.47-4.75 (m, 2H), 3.80-3.59 (m, 2H), 3.23-3.16 (m, 3H), 3.15-2.91 (m, 4H), 2.83-2.62 (m, 3H), 2.06-1.79 (m, 4H), 1.77-1.64 (m, 2H), 1.64-1.48 (m, 2H), 1.17 (s, 3H).

### Step 3: (3R)-1-(7-bromo-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)-3-methylpiperidin-3-ol

NaH (16 mg, 3 *equiv.,* 0.40 mmol) was suspended in THF (0.35 mL) followed by the dropwise addition of (2*R*,7a*S*)-2-fluorotetrahydro-1*H*-pyrrolizin-7a(5*H*)-yl)methanol (64 mg, *3 equiv.,* 0.40 mmol) in THF (0.67 mL). (3*R*)-1-(7-Bromo-2'-(methylsulfonyl)-3,4,5',8'-tetrahydro-2*H*-spiro[naphthalene-1,7'-pyrano[4,3-*d*]pyrimidin]-4'-yl)-3-methylpiperidin-3-ol (70 mg, 1 *equiv.,* 0.13 mmol) in DMF (0.67 mL) was treated by the dropwise addition of the prepared alkoxide mixture. The resulting reaction mixture was stirred for 90 minutes at 50 °C. The reaction was quenched by the addition of aqueous ammonium chloride, diluted with EtOAc, and extracted with EtOAc (3 × 1 mL). The organic layers were dried with sodium sulfate, filtered, and concentrated to a crude product, which was purified by column chromatography (SiO₂, DCM/MeOH = 100/0 to 10/2) to give (3*R*)-1-(7-bromo-2'-(((2*R*,7a*S*)-2-fluorotetrahydro-1*H*-pyrrolizin-7a(5*H*)-yl)methoxy)-3,4,5',8'-tetrahydro-2*H-*spiro[naphthalene-1,7'-pyrano[4,3-*d*]pyrimidin]-4'-yl)-3-methylpiperidin-3-ol (53 mg).
LCMS: m/z (ESI) [M+H]⁺ 603.5, t_{R} = 1.43 minutes (Method C)
¹H NMR (400 MHz, CDCl₃) δ 7.54 (dd, 1H), 7.40 - 7.29 (m, 1H), 7.02 (t, 1H), 5.49 - 5.13 (m, 1H), 4.70 (dd, 1H), 4.53 (dd, 1H), 4.16 (dd, 1H), 4.04 (dd, 1H), 3.77 (dd, 1H), 3.63 - 3.45 (m, 1H), 3.41 - 3.16 (m, 3H), 3.16 - 2.91 (m, 5H), 2.91 - 2.69 (m, 2H), 2.43 - 2.09 (m, 3H), 2.09 - 1.72 (m, 9H), 1.73 - 1.48 (m, 2H), 1.27 (s, 3H).

### Intermediate 13a: (1R,4S)-7-bromo-4'-chloro-4-methyl-2'-(methylthio)-3,4,5',8'-tetrahydro-2H-spiro[naphthalene-1,7'-pyrano[4,3-d]pyrimidine]

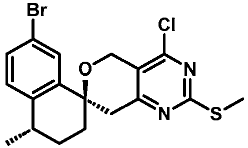

### Step 1: (4S)-7-Bromo-1-((6-chloro-5-(hydroxymethyl)-2-(methylthio)pyrimidin-4-yl)methyl)-4-methyl-1,2,3,4-tetrahydronaphthalen-1-ol

LDA solution (2.60 mL, 1 M, 2.3 *equiv.,* 2.60 mmol) was added dropwise to a solution of (4-chloro-6-methyl-2-(methylthio)pyrimidin-5-yl)methanol (231 mg, 1 *equiv.,* 1.13 mmol) in THF (4 mL) at -78 °C and stirred for 1.25 hours. To this mixture was added (S)-7-bromo-4-methyl-3,4-dihydronaphthalen-1(2*H*)-one (2^{nd} eluting peak) (**Intermediate 7b,** *supra*) (270 mg, 1 *equiv.,* 1.13 mmol) in THF (3 mL) dropwise, while keeping the temperature below -70 °C. The reaction mixture was stirred at -78 °C for 1 hour, at which point the reaction was quenched by aqueous ammonium chloride (10 mL) at -78 °C with vigorous stirring. The reaction mixture was then diluted with EtOAc (15 mL), and the aqueous phase was extracted with EtOAc (3 × 25 mL). The combined organics were dried with sodium sulfate and concentrated to a residue, which was purified via column chromatography (SiO₂; Gradient: 100% DCM - 100% EtOAc) to give (4*S*)-7-bromo-1-((6-chloro-5-(hydroxymethyl)-2-(methylthio)pyrimidin-4-yl)methyl)-4-methyl-1,2,3,4-tetrahydronaphthalen-1-ol (405 mg).

¹H NMR (400 MHz, CDCl₃) δ 7.67 (m, 1H), 7.36 (m, 1H), 7.11 (m, 1H), 4.81 - 4.67 (m, 2H), 3.40 - 3.16 (m, 2H), 2.93 (m, 1H), 2.55 (m, 3H), 2.18 (m, 1H), 2.06 - 1.92 (m, 1H), 1.91 - 1.62 (m, 2H), 1.32 (m, 3H).

### Step 2: (1R,4S)-7-bromo-4'-chloro-4-methyl-2'-(methylthio)-3,4,5',8'-tetrahydro-2H-spiro[naphthalene-1,7'-pyrano[4,3-d]pyrimidine]

Phosphoric acid (658 µL, 85 wt%, 1 *equiv.,* 9.62 mmol) was added to (4*S*)-7-bromo-1-((6-chloro-5-(hydroxymethyl)-2-(methylthio)pyrimidin-4-yl)methyl)-4-methyl-1,2,3,4-tetrahydronaphthalen-1-ol (4.27 g, 1 *equiv.,* 9.62 mmol) in toluene (19.2 mL). The resulting mixture was refluxed for 2 hours. Water (50 mL) and EtOAc (50 mL) were added, and the aqueous layer was extracted with EtOAc (3 × 50 mL). The combined organics were dried with sodium sulfate and concentrated to a residue, which was purified via chiral SFC (Column: Chiralpak IB-U (3.0 × 100 mm), 1.6 µm, P/N 81U83, flow rate: 1.2 mL/min, Mobile phase: (Methanol with 0.25% DEA)) to afford (1*R*,4*S*)-7-bromo-4'-chloro-4-methyl-2'-(methylthio)-3,4,5',8'-tetrahydro-2*H*-spiro[naphthalene-1,7'-pyrano[4,3-*d*]pyrimidine] (**Peak 1**) (2.02 g) and (1*S*,4*S*)-7-bromo-4'-chloro-4-methyl-2'-(methylthio)-3,4,5',8'-tetrahydro-2*H-*spiro[naphthalene-1,7'-pyrano[4,3-*d*]pyrimidine] (**Peak 2**) (1.78 g).
**Peak 1:** t_{R} = 1.34 minutes (Method G; 30% MeOH with 0.25% DEA)
LCMS: m/z (ESI) [M+H]⁺ 427.0 m/z; t_{R} = 4.14 minutes (Method E)

### Intermediate 13b: (1S,4S)-7-bromo-4'-chloro-4-methyl-2'-(methylthio)-3,4,5',8'-tetrahydro-2H-spiro[naphthalene-1,7'-pyrano[4,3-d]pyrimidine]

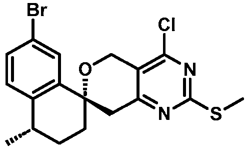

Phosphoric acid (658 µL, 85 wt%, 1 *equiv.,* 9.62 mmol) was added to a solution of (4*S*)-7-bromo-1-((6-chloro-5-(hydroxymethyl)-2-(methylthio)pyrimidin-4-yl)methyl)-4-methyl-1,2,3,4-tetrahydronaphthalen-1-ol (4.27 g, 1 *equiv.,* 9.62 mmol) in toluene (19.2 mL). The resulting mixture was refluxed for 2 hours. Water (50 mL) and EtOAc (50 mL) were added, and the aqueous layer was extracted with EtOAc (3 × 50 mL). The combined organics were dried with sodium sulfate and concentrated to a residue, which was purified via chiral SFC (Column: Chiralpak IB-U (3.0 × 100 mm), 1.6 µm, P/N 81U83, flow rate: 1.2 mL/min, Mobile phase: (Methanol with 0.25% DEA)) to afford (1*R*,4*S*)-7-bromo-4'-chloro-4-methyl-2'-(methylthio)-3,4,5',8'-tetrahydro-2*H*-spiro[naphthalene-1,7'-pyrano[4,3-d]pyrimidine] (**Peak 1**) (2.02 g) and (1*S*,4*S*)-7-bromo-4'-chloro-4-methyl-2'-(methylthio)-3,4,5',8'-tetrahydro-2*H-*spiro[naphthalene-1,7'-pyrano[4,3-*d*]pyrimidine] (**Peak 2**) (1.78 g).
**Peak 2:** t_{R} = 2.06 minutes (Method G; 30% MeOH with 0.25% DEA)
¹H NMR (400 MHz, CDCl₃) δ 7.63 (d, 1H), 7.41 (dd, 1H), 7.19 (d, 1H), 4.78 (q, 2H), 3.12 (q, 2H), 3.00 - 2.86 (m, 1H), 2.59 (s, 3H), 2.13 - 1.87 (m, 3H), 1.48 (qd, 1H), 1.34 (d, 3H).

### Intermediate 14: (S)-8-bromo-7-(dibenzylamino)-4-methyl-3,4-dihydronaphthalen-1(2H)-one

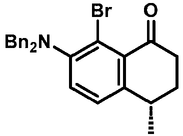

### Step 1: (S)-7-amino-4-methyl-3,4-dihydronaphthalen-1(2H)-one

NMP (27.9 mL) and ammonium hydroxide (29.3 g, 32.4 mL, 20 *equiv.,* 836 mmol) were delivered to a pressure vessel charged with (*S*)-7-bromo-4-methyl-3,4-dihydronaphthalen-1(2*H*)-one (**Intermediate 7b**) (10.0 g, 1.00 *equiv.,* 41.8 mmol) and copper(I) oxide (598 mg, 0.10 *equiv.,* 4.18 mmol) under nitrogen. The pressure vessel was sealed, and the reaction mixture was stirred at 80 °C for 17 hours. The reaction mixture was cooled to ambient temperature and poured into a separatory funnel charged with water (100 mL) and EtOAc (200 mL). The layers were separated, and the aqueous phase was extracted with EtOAc (3 x 150 mL). The combined organic layers were dried over sodium sulfate, filtered, and concentrated to a residue. The crude residue was purified by silica gel chromatography (2 to 45% heptane/EtOAc) to afford (*S*)-7-amino-4-methyl-3,4-dihydronaphthalen-1(2*H*)-one (6.79 g).
¹H NMR (400 MHz, CDCl₃) δ 7.34 (d, 1H), 7.16 (d, 1H), 6.94 - 6.86 (m, 1H), 3.68 (bs, 2H), 3.07 - 2.95 (m, 1H), 2.88 - 2.72 (m, 1H), 2.64 - 2.52 (m, 1H), 2.28 - 2.16 (m, 1H), 1.93 - 1.80 (m, 1H), 1.37 (d, 3H).
LCMS: m/z (ESI) [M+H]⁺ 176.2, t_{R} = 1.51 minutes (Method E)

### Step 2: (S)-7-amino-8-bromo-4-methyl-3,4-dihydronaphthalen-1(2H)-one

A solution of *N*-bromosuccinimide (6.90 g, 1.0 *equiv.,* 38.8 mmol) in DMF (25.9 mL) was delivered dropwise to a flask containing (S)-7-amino-4-methyl-3,4-dihydronaphthalen-1(2*H*)-one (6.79 g, 1.00 *equiv.,* 38.79 mmol) in DMF (51.7 mL) at 0 °C under nitrogen. The resulting mixture was stirred at 0 °C for 5 minutes. The mixture was then warmed to room temperature and stirred for 2 hours. The reaction mixture was quenched by the addition of water (150 mL) and EtOAc (200 mL). The layers were separated, and the aqueous phase was extracted with EtOAc (3 x 150 mL). The combined organic extracts were dried over sodium sulfate, filtered, and concentrated to give a residue, which was purified by silica gel chromatography (0 to 30% heptane/EtOAc) to afford (*S*)-7-amino-8-bromo-4-methyl-3,4-dihydronaphthalen-1(2*H*)-one (7.96 g).
¹H NMR (400 MHz, CDCl₃) δ 7.07 (d, 1H), 6.92 (d, 1H), 4.15 (bs, 2H), 3.05 - 2.91 (m, 1H), 2.86 - 2.74 (m, 1H), 2.68 - 2.56 (m, 1H), 2.25 - 2.12 (m, 1H), 1.90 - 1.77 (m, 1H), 1.31 (d, 3H).
LCMS: m/z (ESI) [M+H]⁺254.1, t_{R} = 2.47 minutes (Method E)

### Step 3. (S)-8-bromo-7-(dibenzylamino)-4-methyl-3,4-dihydronaphthalen-1(2H)-one

Benzyl bromide (16.1 g, 11.2 mL, 3 *equiv.,* 94.0 mmol) and potassium carbonate (15.2 g, 3.5 *equiv.,* 110 mmol) were sequentially delivered to a solution of (S)-7-amino-8-bromo-4-methyl-3,4-dihydronaphthalen-1(2H)-one (7.96 g, 1.0 *equiv.,* 31.3 mmol) in MeCN (313 mL) at room temperature. The resulting mixture was stirred vigorously at reflux for 18 hours at which point the reaction mixture was cooled to room temperature. The solids were filtered and rinsed with EtOAc. The filtrate was concentrated, and the remaining crude residue was purified by silica gel chromatography (1 to 35% heptane/EtOAc) to afford (S)-8-bromo-7-(dibenzylamino)-4-methyl-3,4-dihydronaphthalen-1(2*H*)-one (12.5 g).
1H NMR (400 MHz, CDCl₃) δ 7.36 - 7.19 (m, 10H), 7.09 - 6.97 (m, 2H), 4.24 - 4.10 (m, 4H), 3.03 - 2.90 (m, 1H), 2.89 - 2.76 (m, 1H), 2.73 - 2.61 (m, 1H), 2.24 - 2.12 (m, 1H), 1.90 - 1.77 (m, 1H), 1.30 (d, 3H).
LCMS: m/z (ESI) [M+H]⁺434.3, t_{R} = 3.78 minutes (Method E)

### Intermediate 15: (1S,4S)-N,N-dibenzyl-8-bromo-4'-chloro-4-methyl-2'-(methylthio)-3,4,5',8'-tetrahydro-2H-spiro[naphthalene-1,7'-pyrano[4,3-d]pyrimidin]-7-amine

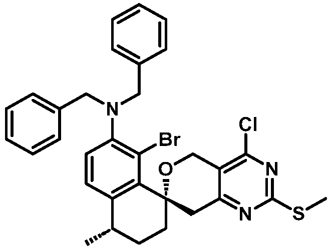

### Step 1: (4S)-8-bromo-1-((6-chloro-5-(hydroxymethyl)-2-(methylthio)pyrimidin-4-yl)methyl)-7-(dibenzylamino)-4-methyl-1,2,3,4-tetrahydronaphthalen-1-ol

LDA (1.0 M in THF/hexanes) (9.25 g, 86.3 mL, 1.0 M, 3.01 *equiv.,* 86.3 mmol) was delivered dropwise to a solution of (4-chloro-6-methyl-2-(methylthio)pyrimidin-5-yl)methanol (8.83 g, 1.51 *equiv.,* 43.1 mmol) in THF (191 mL) at -78 °C. The resulting mixture was stirred at -78 °C for 1.25 hours, followed by the dropwise addition of a solution of (*S*)-8-bromo-7-(dibenzylamino)-4-methyl-3,4-dihydronaphthalen-1(2*H*)-one (**Intermediate 14**) (12.5 g, 1.0 *equiv.,* 28.7 mmol) in THF (96 mL) keeping the internal temperature below -65 °C. The resulting solution was stirred for 1 hour at -78 °C. The reaction was quenched by the dropwise addition of HCl (2.0 M in Et₂O) (3.34 g, 45.9 mL, 3.2 *equiv.,* 91.7 mmol) at -78 °C, followed by water (200 mL). The mixture was warmed to room temperature, transferred to a separatory funnel, and the resulting layers were separated and the aqueous phase was extracted with EtOAc (1 x 200 mL) and DCM (2 x 200 mL). The combined organic extracts were dried over sodium sulfate, filtered, and concentrated to a residue which was purified by silica gel chromatography (2 → 50% CH₂Cl₂/EtOAc, (product elutes around 15% EtOAc)) to afford (4*S*)-8-bromo-1-((6-chloro-5-(hydroxymethyl)-2-(methylthio)pyrimidin-4-yl)methyl)-7-(dibenzylamino)-4-methyl-1,2,3,4-tetrahydronaphthalen-1-ol (16.05 g).
LCMS: m/z (ESI) [M+H]⁺ 640.2, t_{R} = 4.39 minutes (Method E)

### Step 2: (1S,4S)-N,N-dibenzyl-8-bromo-4'-chloro-4-methyl-2'-(methylthio)-3,4,5',8'-tetrahydro-2H-spiro[naphthalene-1,7'-pyrano[4,3-d]pyrimidin]-7-amine

n-Butyllithium (2.5 M in hexanes) (3.35 g, 20.9 mL, 2.2 *equiv.,* 52.3 mmol) was added dropwise to a solution of (4S)-8-bromo-1-((6-chloro-5-(hydroxymethyl)-2-(methylthio)pyrimidin-4-yl)methyl)-7-(dibenzylamino)-4-methyl-1,2,3,4-tetrahydronaphthalen-1-ol (16.0 g, 95 wt%, 1.0 *equiv.,* 23.8 mmol) in THF (198 mL) at -78 °C. The resulting solution was stirred at -78 °C for 35 minutes, followed by the dropwise addition of a solution of p-toluenesulfonyl chloride (6.80 g, 1.5 *equiv.,* 35.7 mmol) in THF (99 mL). The resulting mixture was stirred at -78 °C for 10 minutes, and the acetone/dry ice bath was removed. The resulting reaction mixture was stirred for another 45 minutes after reaching ambient temperature. The mixture was quenched by the addition of a 1:1 mixture of saturated aqueous ammonium chloride solution and water (250 mL). The reaction mixture was transferred to a separatory funnel. EtOAc (100 mL) was added, and the layers were separated and the aqueous phase was extracted with EtOAc (3 x 300 mL). The combined organic extracts were dried over sodium sulfate, filtered, and concentrated. The crude material was purified by silica gel chromatography (0 → 10% heptane/EtOAc). The material isolated from column chromatography was stirred in MeCN (200 mL) at 40 °C overnight. The mixture was filtered, and the solid rinsed with MeCN to afford a white solid. The filtrate was concentrated to a brown oil which was purified by silica gel chromatography (0 → 10% heptane/EtOAc) to give (1*S*,4*S*)-*N,N*-dibenzyl-8-bromo-4'-chloro-4-methyl-2'-(methylthio)-3,4,5',8'-tetrahydro-2*H-*spiro[naphthalene-1,7'-pyrano[4,3-*d*]pyrimidin]-7-amine (1.64 g)
LCMS: m/z (ESI) [M+H]⁺ 620.0, t_{R} = 4.63 minutes (Method E)

### Intermediate 15a: (1R,4S)-N,N-dibenzyl-8-bromo-4'-chloro-4-methyl-2'-(methylthio)-3,4,5',8'-tetrahydro-2H-spiro[naphthalene-1,7'-pyrano[4,3-d]pyrimidin]-7-amine

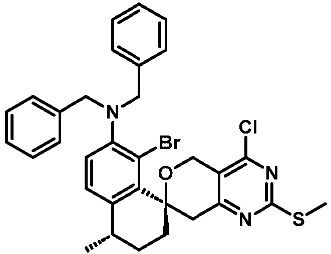

Diisopropylamine (14.6 mL, 103 mmol) in THF (90 mL) was cooled to -30°C in a 1 L 3-necked round bottom flask equipped with an overhead stirrer, addition funnel, thermocouple, and nitrogen line. To this mixture, n-Butyl lithium (2.5 M, 40 mL, 100 mmol) was added dropwise while maintaining the internal temperature below 20 °C. The addition funnel was washed with THF (10 mL) and the resulting pale-yellow solution was cooled to -10 °C for 10 minutes then cooled to -69 °C. (4-Chloro-6-methyl-2-(methylthio)pyrimidin-5-yl)methanol (10.0g, 49.0 mmol) in THF (100 mL) was added to the reaction mixture dropwise while maintaining the temperature below -65 °C. The resulting reaction was stirred at -78 °C for 40 minutes. (*S*)-8-Bromo-7-(dibenzylamino)-4-methyl-3,4-dihydronaphthalen-1(2*H*)-one (21.3 g, 49.0 mmol) in THF (60 mL) was added dropwise while maintaining the internal temperature below -73°C over 30 minutes. The reaction mixture was warmed to -40 °C over 45 minutes then cooled to -68 °C. To this mixture, TsCl (15.1g, 79.4 mmol) in THF (40 mL) was added over 10 minutes while maintaining the internal temperature below 63 °C. The cooling bath was removed, and the reaction mixture was warmed to 0 °C over 2 hours. To this mixture, saturated ammonium chloride (20 mL) was added at 0 °C. The reaction was stirred at room temperature for 30 minutes. Water (200 mL) was added, and the phases were separated and the aqueous layer was extracted with DCM (2 x 100 mL). The combined organic layers were washed with brine, dried over magnesium sulfate, filtered, concentrated, and dried under vacuum. The brown residue was suspended in DCM (60 mL) and sonicated for 30 minutes at 35 °C. To this mixture was added MeOH (240 mL). The resulting mixture was stirred and gradually heated to 55 °C over 35 minutes. The slurry was stirred at 55°C for 25 minutes then cooled to 20 °C and stirred for 1.75 hours. The mixture was filtered and washed with MeOH (80 mL) to give (4*S*)-*N,N*-dibenzyl-8-bromo-4'-chloro-4-methyl-2'-(methylthio)-3,4,5',8'-tetrahydro-2*H-*spiro[naphthalene-1,7'-pyrano[4,3-*d*]pyrimidin]-7-amine.

¹H NMR (400 MHz, CDCl₃) δ 7.40 - 7.17 (m, 10H), 7.05 m, 1H), 6.88 (m, 1H), 4.93 m, 1H), 4.75 (m, 1H), 4.26 - 3.99 (m, 5H), 3.02 - 2.84 (m, 2H), 2.58 (s, 3H), 2.24 - 1.81 (m, 3H), 1.72 - 1.36 (m, 1H), 1.29 (m, 3H).

The diastereomers of (4S)-*N,N*-dibenzyl-8-bromo-4'-chloro-4-methyl-2'-(methylthio)-3,4,5',8'-tetrahydro-2*H*-spiro[naphthalene-1,7'-pyrano[4,3-*d*]pyrimidin]-7-amine (13.5 g) were separated by chiral SFC (Daicel Chiralpak IG, 21mm ID x 250mmL, 5µm (part number 87445; MeOH, 40% with 0.25% DEA, backpressure = 120 bar)) to afford (1*R*,4*S*)-*N,N*-dibenzyl-8-bromo-4'-chloro-4-methyl-2'-(methylthio)-3,4,5',8'-tetrahydro-2*H*-spiro[naphthalene-1,7'-pyrano[4,3-*d*]pyrimidin]-7-amine (**Peak 1**) and (1*S*,4*S*)-*N,N*-dibenzyl-8-bromo-4'-chloro-4-methyl-2'-(methylthio)-3,4,5',8'-tetrahydro-2*H*-spiro[naphthalene-1,7'-pyrano[4,3-*d*]pyrimidin]-7-amine (**Peak 2**).

### Peak 1:

Analytical Chiral SFC: t_{R} = 2.09 minutes. (Daicel Chiralpak IG, 4.6mm ID x 100mmL, 5µm (part number 87423; MeOH, 40% with 0.25% DEA, backpressure = 137.5 bar); over 8 minutes)
¹H NMR (400 MHz, CDCl₃) δ 7.35 - 7.30 (m, 4H), 7.30 - 7.26 (m, 3H), 7.25 (s, 1H), 7.24 - 7.18 (m, 2H), 7.15 - 7.11 (m, 1H), 6.89 (d, *J =* 8.5 Hz, 1H), 4.92 (d, *J =* 16.5 Hz, 1H), 4.73 (d, *J =* 16.5 Hz, 1H), 4.20 - 4.06 (m, 5H), 2.97 - 2.86 (m, 2H), 2.58 (s, 3H), 2.11 - 2.02 (m, 1H), 2.00 - 1.89 (m, 2H), 1.28 (d, *J =* 6.8 Hz, 3H).

### Peak 2:

Analytical Chiral SFC: t_{R} = 2.67 minutes. (Daicel Chiralpak IG, 4.6mm ID x 100mmL, 5µm (part number 87423; MeOH, 40% with 0.25% DEA, backpressure = 137.5 bar); over 8 minutes)
¹H NMR (400 MHz, CDCl₃) δ 7.40 - 7.16 (m, 10H), 6.97 (d, *J =* 8.3 Hz, 1H), 6.86 (d, *J* = 8.3 Hz, 1H), 4.94 (d, *J =* 16.5 Hz, 1H), 4.82 - 4.73 (m, 1H), 4.21 - 4.00 (m, 5H), 3.09 - 2.80 (m, 2H), 2.58 (s, 3H), 2.17 (m, 1H), 1.92 (m, 2H), 1.71 - 1.60 (m, 1H), 1.30 (d, *J =* 7.1 Hz, 3H).

### Intermediate 16: 7-Bromo-4',5-dichloro-8-fluoro-2'-(methylthio)-3,4,5',8'-tetrahydro-2H-spiro[naphthalene-1,7'-pyrano[4,3-d]pyrimidine]

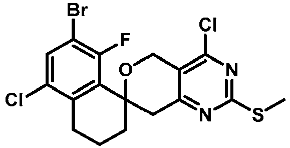

### Step 1: 4-(4-Bromo-2-chloro-5-fluorophenyl)butanal

A 200 mL round bottom flask was charged with 1-bromo-5-chloro-2-fluoro-4-iodobenzene (10 g, 1 *equiv.,* 30 mmol), palladium(II) acetate (0.40 g, 0.06 *equiv.,* 1.8 mmol), lithium acetate (4.9 g, 2.5 *equiv.,* 75 mmol), lithium chloride (1.3 g, 1 *equiv.,* 30 mmol), and tetrabutylammonium chloride hydrate (18 g, 2 *equiv.,* 60 mmol). The mixture was evacuated and backfilled with nitrogen three times. DMF (75 mL) and but-3-en-1-ol (2.6 g, 3.1 mL, 1.2 *equiv.,* 36 mmol) were then added via syringe and the resulting mixture was stirred overnight at 70 °C. The reaction was quenched with saturated ammonium chloride solution (50 mL) and 50 mL of heptanes. The aqueous and organic layers were separated and the aqueous was extracted two more times with 50 mL of heptanes. The combined organics were washed with 50 mL of 1 M HCl and 50 mL of brine. The organic layers were separated, combined, dried over magnesium sulfate, and filtered. The aqueous was re-extracted with EtOAc and the organic layer was washed with 1 M HCl and brine. The combined organic layers were concentrated, re-dissolved in heptanes, dried over magnesium sulfate, filtered, and concentrate. The combined crude oil was purified using a RediSep Rf Gold 220 g silica gel cartridge (eluting with a 100% heptane to 20% EtOAc in heptane gradient) to give 4-(4-bromo-2-chloro-5-fluorophenyl)butanal (3.83 g).

¹H NMR (400 MHz, CDCl₃) δ 9.79 (m, 1H), 7.54 (m, 1H), 7.00 (m, 1H), 2.71 (m, 2H), 2.51 (m, 2H), 1.94 (m, 2H).

### Step 2: 4-(4-Bromo-2-chloro-5-fluorophenyl)butanoic acid

Sodium chlorite (6.15 g, 5 *equiv.,* 68.0 mmol) was added to 4-(4-bromo-2-chloro-5-fluorophenyl)butanal (3.80 g, 1 *equiv.,* 13.6 mmol), 2-methyl-2-butene (38.1 g, 57.6 mL, 40 *equiv.,* 544 mmol), sodium dihydrogen phosphate hydrate (18.8 g, 10 *equiv.,* 136 mmol), water (45.3 mL) and t-BuOH (90.6 mL) at 0 °C. After 15 minutes, the reaction was brought to room temperature and stirred for 2.5 hours. The reaction mixture was then poured into a separatory funnel containing a 1:1 mixture of water and brine and CH₂Cl₂. The layers were separated and the aqueous phase was extracted with CH₂Cl₂ (3 x 50 mL). The combined organic extracts were dried over magnesium sulfate, filtered, and concentrated to afford 4-(4-bromo-2-chloro-5-fluorophenyl)butanoic acid (4.40 g, 14.9 mmol; contains residual *t*-BuOH), which was used without further purification.

¹H NMR (400 MHz, CDCl₃) δ 7.54 (m, 1H), 7.01 (m, 1H), 2.79 - 2.69 (m, 2H), 2.42 (m, 2H), 1.95 (m, 2H).

### Step 3: 7-Bromo-5-chloro-8-fluoro-3,4-dihydronaphthalen-1(2H)-one

Oxalyl chloride (2.3 mL, 2 *equiv.,* 27.1 mmol) was added dropwise to a solution of 4-(4-bromo-2-chloro-5-fluorophenyl)butanoic acid (4.00 g, 1 *equiv.,* 13.5 mmol) and *N,N-*dimethylformamide (105 µL, 0.1 *equiv.,* 1.35 mmol) in DCM (54 mL) at room temperature. The resulting mixture was stirred for 2 hours then concentrated. The residue was re-dissolved in DCM (54.1 mL), followed by the addition of aluminum chloride (3.61 g, 2 *equiv.,* 27.1 mmol) in one portion. The reaction mixture was stirred for 22 hours at ambient temperature. The reaction mixture was cooled, quenched with water, diluted with DCM, and extracted with DCM/brine (3 x 40 mL). The combined organic layers were collected, dried over magnesium sulfate, filtered, and concentrated to give a residue which was taken up in DCM and purified using a RediSep Rf Gold 120 g silica gel cartridge (eluting with 0-40% EtOAc in heptanes) to afford 7-bromo-5-chloro-8-fluoro-3,4-dihydronaphthalen-1(2*H*)-one (2.08 g).

¹H NMR (400 MHz, CDCl₃) δ 7.74 (m, 1H), 2.96 (m, 2H), 2.66 (m, 2H), 2.15 (m, 2H).

### Step 4: 7-Bromo-5-chloro-1-((6-chloro-5-(hydroxymethyl)-2-(methylthio)pyrimidin-4-yl)methyl)-8-fluoro-1,2,3,4-tetrahydronaphthalen-1-ol

To an oven-dried 125 mL round bottom flask was added (4-chloro-6-methyl-2-(methylthio)pyrimidin-5-yl)methanol (1.11 g, 1.5 *equiv.,* 5.41 mmol). The flask was then sealed and degassed with nitrogen. THF (27 mL) was then added, and the reaction was cooled to -78 °C. LDA (10.8 mL, 1 M, 3 *equiv.,* 10.8 mmol) was then added dropwise, while maintaining a temperature below -73 °C by internal probe. The solution was stirred at -78 °C for 1 hour. 7-Bromo-5-chloro-8-fluoro-3,4-dihydronaphthalen-1(2*H*)-one (1.00 g, 1 *equiv.,* 3.60 mmol) was then dissolved in THF (14 mL) and added dropwise to the reaction mixture, while maintaining a temperature below -73 ° C. The solution was stirred for 1 hour and the product mixture was then quenched with HCl (5.77 mL, 2.0 M, 3.2 *equiv.,* 11.5 mmol) slowly, while maintaining temperature below -75 ° C. The product mixture was removed from the dry ice bath warming to about -20 °C and was poured into a separatory funnel containing saturated aqueous sodium bicarbonate and EtOAc. The aqueous layer was separated and further extracted with EtOAc (2x). The organic layers were combined, dried over magnesium sulfate, filtered, and concentrated to give a yellow solid. The residue was purified on a 120 g RediSep column (0-100% EtOAc/heptanes; followed by 40% (20% MeOH, 2.5% ammonium hydroxide in DCM) in DCM) to afford 7-bromo-5-chloro-1-((6-chloro-5-(hydroxymethyl)-2-(methylthio)pyrimidin-4-yl)methyl)-8-fluoro-1,2,3,4-tetrahydronaphthalen-1-ol (1.27 g).
LCMS: m/z (ESI) [M+H]⁺ 481.0, t_{R} = 2.18 minutes (Method C)

### Step 5: 7-Bromo-4',5-dichloro-8-fluoro-2'-(methylthio)-3,4,5',8'-tetrahydro-2H-spiro[naphthalene-1,7'-pyrano[4,3-d]pyrimidine]

Phosphoric acid (170 µL, 85 wt%, *1 equiv.,* 2.49 mmol) was added to the white suspension of 7-bromo-5-chloro-1-((6-chloro-5-(hydroxymethyl)-2-(methylthio)pyrimidin-4-yl)methyl)-8-fluoro-1,2,3,4-tetrahydronaphthalen-1-ol (1.20 g, 1 *equiv.,* 2.49 mmol) in toluene (27 mL). The mixture was heated at 110 °C for 2 hours. The mixture was cooled to room temperature and was diluted with EtOAc. The product mixture was transferred to a separatory funnel and washed with saturated aqueous sodium bicarbonate and brine. The organic layer was dried over magnesium sulfate, filtered, and concentrated to give a crude residue which was purified by flash column chromatography (RediSep SiO₂ gold 80 g; eluting: EtOAc/heptanes, 0-60%) to give 7-bromo-4',5-dichloro-8-fluoro-2'-(methylthio)-3,4,5',8'-tetrahydro-2*H-*spiro[naphthalene-1,7'-pyrano[4,3-*d*]pyrimidine] (451 mg).
LCMS: m/z (ESI) [M+H]⁺465.0, t_{R} = 2.29 minutes (Method C)

### Intermediate 17a: rel-(S)-azepane-4-carbonitrile

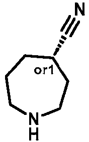

### Step 1: benzyl rel-(S)-4-cyanoazepane-1-carboxylate

Benzyl chloroformate (0.80 mL, 2.0 *equiv.,* 7.0 mmol) was added to a solution of azepane-4-carbonitrile (0.35 g, 1 *equiv.,* 2.8 mmol) and DIPEA (1.2 mL, 2.5 *equiv.,* 7.0 mmol) in DCM (1.5 mL) at room temperature. The reaction mixture was stirred for 16 hours. Afterward, the reaction mixture was concentrated to a residue, which was purified by flash chromatography (12 g Redisep Column using 25-30% hexanes in EtOAc) to afford benzyl 4-cyanoazepane-1-carboxylate (0.13 g). The enantiomers of benzyl 4-cyanoazepane-1-carboxylate were separated by chiral SFC (Column: i-Amylose-3; 21.2×250 mm; 5 mm; Mobile Phase A: 50% MeOH at 30 mL/min for 10 minutes) to afford benzyl *rel*-(*S*)-4-cyanoazepane-1-carboxylate (**Peak 1**) and benzyl *rel*-(*R*)-4-cyanoazepane-1-carboxylate (**Peak 2**).
**Peak 1:** HPLC: t_{R} = 1.74 minutes (Column: i-Amylose-3, 4.6x250 mm; 5 mm; Mobile Phase A: 50% MeOH @ 4 mL/min for 5 minutes)

### Step 2: rel-(S)-azepane-4-carbonitrile

Pd(OH)₂ on carbon (90 mg, 20 wt%, 0.3 *equiv.,* 0.13 mmol) was added to benzyl *rel-*(*S*)-4-cyanoazepane-1-carboxylate (**Peak 1**, 110 mg, 1 *equiv.,* 0.43 mmol) in THF (3 mL) at room temperature and stirred for 3 hours under hydrogen atmosphere. The reaction mixture was purged with nitrogen, filtered through a Celite bed, concentrated, and dried to afford *rel-*(S)-azepane-4-carbonitrile (45 mg).

¹H NMR (400 MHz, CDCl₃): δ 3.03-2.58 (m, 5H), 2.07-1.45 (m, 6 H).

### Intermediate 17b: rel-(R)-azepane-4-carbonitrile

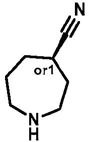

### Step 1: benzyl rel-(R)-4-cyanoazepane-1-carboxylate

Benzyl chloroformate (0.80 mL, 2.0 *equiv.,* 7.0 mmol) was added to a solution of azepane-4-carbonitrile (0.35 g, 1 *equiv.,* 2.8 mmol) and DIPEA (1.2 mL, 2.5 *equiv.,* 7.0 mmol) in DCM (1.5 mL) at room temperature. The reaction mixture was stirred for 16 hours. Afterward, the reaction mixture was concentrated to a residue, which was purified by flash chromatography (12 g RediSep Column using 25-30% hexanes in EtOAc) to afford benzyl 4-cyanoazepane-1-carboxylate (0.13 g). The enantiomers of benzyl 4-cyanoazepane-1-carboxylate were separated by chiral SFC (Column: i-Amylose-3; 21.2×250 mm; 5 mm; Mobile Phase A: 50% MeOH at 30 mL/min for 10 minutes) to afford benzyl *rel-*(*S*)-4-cyanoazepane-1-carboxylate (**Peak 1**) and benzyl *rel*-(*R*)-4-cyanoazepane-1-carboxylate (**Peak 2**).
**Peak 2:** HPLC: t_{R} = 2.16 minutes (Column: i-Amylose-3, 4.6x250 mm; 5 mm; Mobile Phase A: 50% MeOH @ 4 mL/min for 5 minutes)

### Step 2: rel-(R)-azepane-4-carbonitrile

Pd(OH)₂ on carbon (90 mg, 20 wt%, 0.3 *equiv.,* 0.13 mmol) was added to benzyl *rel-*(*R*)-4-cyanoazepane-1-carboxylate (**Peak 2**, 110 mg, 1 *equiv.,* 0.43 mmol) in THF (3 mL) at room temperature and stirred for 3 hours under hydrogen atmosphere. The reaction mixture was purged with nitrogen, filtered through a Celite bed, concentrated, and dried to afford *rel-*(*R*)-azepane-4-carbonitrile (45 mg).

¹H NMR (400 MHz, CDCl₃): δ 2.97-2.82 (m, 5H), 2.09-1.55 (m, 6 H).

### Intermediate 18: 3-Chloro-N,N-dimethyl-5,6,7,8-tetrahydro-4H-pyrazolo[1,5-a][1,4]diazepine-2-carboxamide

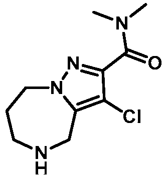

### Step 1: tert-Butyl 3-chloro-2-(dimethylcarbamoyl)-7,8-dihydro-4H-pyrazolo[1,5-a][1,4]diazepine-5(6H)-carboxylate

A solution of *tert*-butyl 2-(dimethylcarbamoyl)-7,8-dihydro-4*H*-pyrazolo[1,5-*a*][1,4]diazepine-5(6*H*)-carboxylate (1.7938 g, 1 *equiv.,* 5.8168 mmol) in DMF (12 mL) was treated with *N*-chlorosuccinimide (1.0 g, 1.3 *equiv.,* 7.5 mmol) at room temperature. Afterwards, the mixture was heated at 50 °C and stirred for 19 hours. The mixture was cooled to room temperature then treated with saturated aqueous sodium thiosulfate (20 mL) and stirred for 10 min. The mixture was extracted with EtOAc (3 x 10 mL). The organic phase was washed with 5% aqueous lithium chloride solution (3 x 20 mL), brine (20 mL), dried over magnesium sulfate, filtered, and concentrated to afford a clear oil. The mixture was redissolved in EtOAc and washed with 5% lithium chloride aqueous solution (2 x 20 mL). The organic phase was then dried over magnesium sulfate, filtered, and concentrated to afford *tert*-butyl 3-chloro-2-(dimethylcarbamoyl)-7,8-dihydro-4*H*-pyrazolo[1,5-*a*][1,4]diazepine-5(6*H*)-carboxylate (1.6 g) as a clear light yellow oil

¹H NMR (400 MHz, CDCl₃): δ 4.55 - 4.45 (m, 2H), 4.42 - 4.34 (m, 2H), 3.78 - 3.66 (m, 2H), 3.11 - 3.04 (m, 6H), 2.01 - 1.91 (m, 2H), 1.42 (s, 9H).

### Step 2: 3-Chloro-N,N-dimethyl-5,6,7,8-tetrahydro-4H-pyrazolo[1,5-a][1,4]diazepine-2-carboxamide, HCl

A solution of *tert*-butyl 3-chloro-2-(dimethylcarbamoyl)-7,8-dihydro-4*H-*pyrazolo[1,5-*a*][1,4]diazepine-5(6*H*)-carboxylate (488 mg, 1 *equiv.,* 1.42 mmol) in DCM (7 mL) was treated with HCl (519 mg, 7.12 mL, 2 M, 10 *equiv.,* 14.2 mmol) at room temperature. Afterwards, the mixture was stirred for 19 hours. The mixture was concentrated under reduced pressure to afford 3-chloro-*N,N*-dimethyl-5,6,7,8-tetrahydro-4*H*-pyrazolo[1,5-*a*][1,4]diazepine-2-carboxamide, HCl (400 mg).

¹H NMR (400 MHz, MeOD): δ 4.61 - 4.55 (m, 2H), 4.51 (s, 2H), 3.64 - 3.57 (m, 2H), 3.12 (s, 6H), 2.22 - 2.15 (m, 2H).

### Intermediate 19: 7-Amino-2'-(((2R,7aS)-2-fluorotetrahydro-1H-pyrrolizin-7a(5H)-yl)methoxy)-4'-hydroxy-3,4,5',8'-tetrahydro-2H-spiro[naphthalene-1,7'-pyrano[4,3-d]pyrimidine]-8-carbonitrile

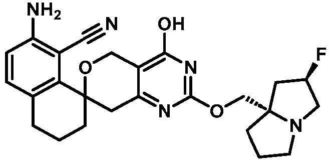

### Step 1: N,N-Dibenzyl-4'-(benzyloxy)-8-bromo-2'-(methylthio)-3,4,5',8'-tetrahydro-2H-spiro[naphthalene-1,7'-pyrano[4,3-d]pyrimidin]-7-amine

To a solution of phenylmethanol (511 µL, 4.94 mmol, 1.5 *equiv.*) in THF (32.9 mL), at room temperature, was added NaH (60% dispersion in mineral oil, 264 mg, 6.59 mmol, 2.0 *equiv.*)*.* The resulting mixture was stirred at room temperature for 10 minutes, followed by the portion-wise addition of *N,N-*dibenzyl-8-bromo-4'-chloro-2'-(methylthio)-3,4,5',8'-tetrahydro-2*H*-spiro[naphthalene-1,7'-pyrano[4,3-*d*]pyrimidin]-7-amine (2.00 g, 3.29 mmol). The reaction mixture was stirred at room temperature for 2 hours. The reaction was quenched by the addition of saturated aqueous ammonium chloride solution at 0 °C. The layers were separated, and the aqueous phase was extracted with EtOAc (3 x). The combined organic layers were washed with brine, dried over sodium sulfate, filtered, and concentrated under reduced pressure to give crude material, which was purified by silica gel chromatography (80 g cartridge, 80 mL/min, hexanes/DCM: 0 to 100%) to give *N,N*-dibenzyl-4'-(benzyloxy)-8-bromo-2'-(methylthio)-3,4,5',8'-tetrahydro-2*H*-spiro[naphthalene-1,7'-pyrano[4,3-*d*]pyrimidin]-7-amine (1.9 g) as an off white solid.
LCMS: m/z (ESI) [M+H]⁺ 678.4, t_{R} = 2.46 minutes, (Method B)

### Step 2: 4'-(Benzyloxy)-7-(dibenzylamino)-2'-(methylthio)-3,4,5',8'-tetrahydro-2H-spiro[naphthalene-1,7'-pyrano[4,3-d]pyrimidine]-8-carbonitrile

To a solution of *N,N*-dibenzyl-4'-(benzyloxy)-8-bromo-2'-(methylthio)-3,4,5',8'-tetrahydro-2*H*-spiro[naphthalene-1,7'-pyrano[4,3-*d*]pyrimidin]-7-amine (1.8 g, 2.7 mmol) in DMF (53 mL) was added Copper(I) cyanide (2.4 g, 27 mmol, 10 *equiv.*)*.* The reaction mixture was stirred at 120 °C for 4 hours. The reaction mixture was cooled to room temperature and concentrated. The residue was partitioned between DCM and water, upon which a precipitate formed. A solution of NH₄OH/MeOH (1:1) was added until no solid remained, and two clear layers were obtained. The biphasic mixture was stirred for 30 minutes, and the layers were separated and the aqueous phase was extracted with DCM (3 x). The combined organic extracts were washed with brine, dried over sodium sulfate, filtered, and concentrated under reduced pressure to give crude material, which was purified by silica gel chromatography (120 g cartridge, 110 mL/min, hexanes/EtOAc: 0 to 40%) to give 4'-(benzyloxy)-7-(dibenzylamino)-2'-(methylthio)-3,4,5',8'-tetrahydro-2*H*-spiro[naphthalene-1,7'-pyrano[4,3-*d*]pyrimidine]-8-carbonitrile (1.4 g) as an off white solid.
LCMS: m/z (ESI) [M+H]⁺ 625.4, t_{R} = 2.30 minutes, (Method B)
¹H NMR (400 MHz, DMSO*-d₆*) δ 7.51 - 7.46 (m, 2H), 7.43 - 7.37 (m, 2H), 7.37 - 7.32 (m, 5H), 7.31 - 7.25 (m, 4H), 7.24 - 7.18 (m, 3H), 7.15 (d, 1H), 5.50 (d, 1H), 5.47 (d, 1H), 4.75 (d, 1H), 4.67 (d, 1H), 4.26 (d, 2H), 4.21 (d, 2H), 3.35 - 3.28 (m, 1H), 2.94 (d, 1H), 2.73 - 2.63 (m, 2H), 2.50 (s, 3H), 2.09 - 2.01 (m, 1H), 1.82 - 1.62 (m, 3H).

### Step 3: 4'-(Benzyloxy)-7-(dibenzylamino)-2'-(methylsulfonyl)-3,4,5',8'-tetrahydro-2H-spiro[naphthalene-1,7'-pyrano[4,3-d]pyrimidine]-8-carbonitrile

To a suspension of 4'-(benzyloxy)-7-(dibenzylamino)-2'-(methylthio)-3,4,5',8'-tetrahydro-2*H*-spiro[naphthalene-1,7'-pyrano[4,3-*d*]pyrimidine]-8-carbonitrile (1.32 g, 2.11 mmol), tetrabutylammonium hydrogen sulfate (115 mg, 338 µmol, 0.16 *equiv.*)*,* and sodium tungstate dihydrate (70 mg, 0.21 mmol, 0.1 *equiv.*) in EtOAc (21.1 mL) was added hydrogen peroxide (1.51 mL, 30 wt%, 14.8 mmol, 7.0 *equiv.*)*.* The reaction mixture was stirred at 75 °C for 1 hour. The reaction was quenched by the addition of water and a 5% sodium bisulfate aqueous solution. EtOAc was added, and the layers were separated and the aqueous phase was extracted with EtOAc (3 x). The combined organic extracts were washed with brine, dried over sodium sulfate, filtered, and concentrated to yield 4'-(benzyloxy)-7-(dibenzylamino)-2'-(methylsulfonyl)-3,4,5',8'-tetrahydro-2*H*-spiro[naphthalene-1,7'-pyrano[4,3-*d*]pyrimidine]-8-carbonitrile (1.35 g), which was used in the next step without further purification.
LCMS: m/z (ESI) [M+H]⁺ 657.4, t_{R} = 2.07 minutes, (Method B)

### Step 4: 4'-(benzyloxy)-7-(dibenzylamino)-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidine]-8-carbonitrile

To a solution of 4'-(benzyloxy)-7-(dibenzylamino)-2'-(methylsulfonyl)-3,4,5',8'-tetrahydro-2*H*-spiro[naphthalene-1,7'-pyrano[4,3-*d*]pyrimidine]-8-carbonitrile (1.35 g, 2.06 mmol) in DMF (41.1 mL) at room temperature, was added ((2*R*,7a*S*)-2-fluorotetrahydro-1*H-*pyrrolizin-7a(5*H*)-yl)methanol (1.64 g, 10.3 mmol, 5 *equiv.*) under argon. The resulting solution was cooled to 0 °C, followed by the dropwise addition of lithium bis(trimethylsilyl)amide (1.0 M in THF, 3.08 mL, 3.08 mmol, 1.5 *equiv.*)*.* The ice/water bath was removed, and the reaction mixture was stirred for 1 hour. The reaction was quenched by the slow addition of a 1:1 mixture of saturated aqueous ammonium chloride solution and water at 0 °C. EtOAc was added, and the layers were separated and the aqueous phase was extracted with EtOAc. The combined organic extracts were dried over sodium sulfate, filtered, and concentrated under reduced pressure to give crude material, which was purified by silica gel chromatography (80 g cartridge, 60 mL/min, DCM /[DCM /MeOH/NH₄OH (80:18:2)], 0 to 60%) to give 4'-(benzyloxy)-7-(dibenzylamino)-2'-(((2*R*,7a*S*)-2-fluorotetrahydro-1*H-*pyrrolizin-7a(5*H*)-yl)methoxy)-3,4,5',8'-tetrahydro-2H-spiro[naphthalene-1,7'-pyrano[4,3-*d*]pyrimidine]-8-carbonitrile (1.20 g) as an orange solid.
LCMS: m/z (ESI) [M+H]⁺ 736.6, t_{R} = 2.24 minutes, (Method B)
¹H NMR (400 MHz, DMSO*-d₆*) δ 7.51 - 7.46 (m, 2H), 7.43 - 7.38 (m, 2H), 7.37 - 7.32 (m, 5H), 7.31 - 7.26 (m, 4H), 7.24 - 7.18 (m, 3H), 7.15 (d, 1H), 5.47 (d, 1H), 5.44 (d, 1H), 5.26 (d, 1H), 4.74 (d, 1H), 4.65 (d, 1H), 4.24 (d, 2H), 4.23 (d, 2H), 4.02 (dd, 1H), 3.93 (dd, 1H), 3.31 - 3.24 (m, 1H), 3.12 - 2.96 (m, 3H), 2.93 - 2.86 (m, 1H), 2.85 - 2.78 (m, 1H), 2.71 - 2.65 (m, 2H), 2.13 - 1.92 (m, 4H), 1.87 - 1.63 (m, 6H).

### Step 5: 7-Amino-2'-(((2R,7aS)-2-fluorotetrahydro-1H-pyrrolizin-7a(5H)-yl)methoxy)-4'-hydroxy-3,4,5',8'-tetrahydro-2H-spiro[naphthalene-1,7'-pyrano[4,3-d]pyrimidine]-8-carbonitrile

A flask was charged with 4'-(benzyloxy)-7-(dibenzylamino)-2'-(((2*R*,7a*S*)-2-fluorotetrahydro-1*H*-pyrrolizin-7a(5*H*)-yl)methoxy)-3,4,5',8'-tetrahydro-2*H-*spiro[naphthalene-1,7'-pyrano[4,3-*d*]pyrimidine]-8-carbonitrile (1.10 g, 1.49 mmol) and MeOH (29.9 mL). The flask headspace was sparged with argon, and Pd(OH)₂ on carbon (20 wt%, 2.62 g, 3.74 mmol, 2.5 *equiv.*) was added. The inert atmosphere was exchanged with hydrogen by degassing the reaction mixture under reduced pressure with a backflow of hydrogen from a balloon. The reaction mixture was stirred overnight under a hydrogen atmosphere at room temperature. Celite was added and the hydrogen atmosphere was exchanged with argon. The suspension was filtered over celite, and the solids were rinsed with MeOH. The filtrate was concentrated to provide crude 7-amino-2'-(((2*R*,7a*S*)-2-fluorotetrahydro-1*H*-pyrrolizin-7a(5*H*)-yl)methoxy)-4'-hydroxy-3,4,5',8'-tetrahydro-2*H-*spiro[naphthalene-1,7'-pyrano[4,3-*d*]pyrimidine]-8-carbonitrile (630 mg) as a white solid.
LCMS: m/z (ESI) [M+H]⁺ 466.4, t_{R} = 0.97 minutes, (Method B)
¹H NMR (400 MHz, DMSO-*d₆*): δ 7.04 (d, 1H), 6.73 (d, 1H), 5.75 (br s, 2H), 5.41 (br d, 1H), 4.51 (d, 1H), 4.42 (d, 1H), 4.29 - 4.10 (m, 2H), 3.63 - 3.11 (m, 6H), 3.03 - 2.98 (m, 2H), 2.63 - 2.57 (m, 3H), 2.30 - 1.71 (m, 6H), 1.68 - 1.58 (m, 2H).

### Intermediate 20: (1S,4S)-7-amino-2'-(((2R,7aS)-2-fluorotetrahydro-1H-pyrrolizin-7a(5H)-yl)methoxy)-4'-hydroxy-4-methyl-3,4,5',8'-tetrahydro-2H-spiro[naphthalene-1,7'-pyrano[4,3-d]pyrimidine]-8-carbonitrile

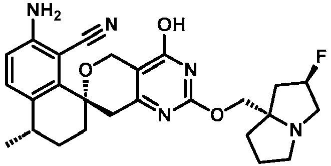

### Step 1: (1S,4S)-N,N-dibenzyl-4'-(benzyloxy)-8-bromo-4-methyl-2'-(methylthio)-3,4,5',8'-tetrahydro-2H-spiro[naphthalene-1,7'-pyrano[4,3-d]pyrimidin]-7-amine

To a solution of benzyl alcohol (666 mg, 640 µL, 1.01 *equiv.,* 6.15 mmol) in THF (10 mL) was added NaH as a dispersion in mineral oil (392 mg, 60 wt%, 1.61 *equiv.,* 9.80 mmol) in an ice bath. To this mixture, (1*S*,4*S*)-*N,N*-dibenzyl-8-bromo-4'-chloro-4-methyl-2'-(methylthio)-3,4,5',8'-tetrahydro-2*H*-spiro[naphthalene-1,7'-pyrano[4,3*-d*]pyrimidin]-7-amine (3.77 g, 1 *equiv.,* 6.07 mmol) in THF (10 mL) was added, and the ice bath was removed. The resulting mixture was stirred for 55 minutes at room temperature and then cooled back down in an ice bath. The reaction mixture was quenched with saturated aqueous ammonium chloride solution (10 mL). EtOAc was added (20 mL), and the layers that formed were separated and the aqueous layer was extracted with EtOAc (2 x 20 mL). The combined organic layers were washed with brine, dried over magnesium sulfate, filtered, and concentrated. The resulting residue was azeotroped with toluene (3 x 20 mL) at 50 °C to afford (1*S*,4*S*)-*N,N*-dibenzyl-4'-(benzyloxy)-8-bromo-4-methyl-2'-(methylthio)-3,4,5',8'-tetrahydro-2*H*-spiro[naphthalene-1,7'-pyrano[4,3-*d*]pyrimidin]-7-amine (4.26 g), which was used without further purification.
LCMS: m/z (ESI) [M+H]⁺ 692.6, t_{R} = 4.45 minutes (Method K)

### Step 2: (1S,4S)-4'-(benzyloxy)-7-(dibenzylamino)-4-methyl-2'-(methylthio)-3,4,5',8'-tetrahydro-2H-spiro[naphthalene-1,7'-pyrano[4,3-d]pyrimidine]-8-carbonitrile

Copper(I) cyanide (1.09 g, 2 *equiv.,* 12.1 mmol) was added to a solution of (1*S,*4*S*)-*N,N*-dibenzyl-4'-(benzyloxy)-8-bromo-4-methyl-2'-(methylthio)-3,4,5',8'-tetrahydro-2H-spiro[naphthalene-1,7'-pyrano[4,3-*d*]pyrimidin]-7-amine (4.20 g, 1 *equiv.,* 6.06 mmol) in DMF (30 mL) under a nitrogen atmosphere at room temperature. The resulting reaction mixture was stirred for 1.25 hours at 120 °C. The reaction mixture was allowed to cool to room temperature. To this mixture, DCM (50 mL) was added, and the resulting precipitate was filtered. Water (50 mL) and NH₄OH (20 mL) were added to the filtrate. The biphasic mixture was transferred to a separatory funnel, and the layers that formed were separated and the aqueous layer was extracted with DCM (50 mL). The combined organic layers were washed with 10% saturated LiCl solution (2 x 50 mL) and then concentrated. The residue obtained was re-dissolved in ether (40 mL) and washed with water (2 x 20 mL). The combined aqueous layers were extracted with ether (20 mL). The combined organic layers were dried over magnesium sulfate, filtered, and concentrated to yield (1*S*,4*S*)-4'-(benzyloxy)-7-(dibenzylamino)-4-methyl-2'-(methylthio)-3,4,5',8'-tetrahydro-2*H*-spiro[naphthalene-1,7'-pyrano[4,3-*d*]pyrimidine]-8-carbonitrile (3.68 g) which was used without further purification.
LCMS: m/z (ESI) [M+H]⁺ 639.9, t_{R} = 4.26 minutes (Method K)

### Step 3: (1S,4S)-4'-(benzyloxy)-7-(dibenzylamino)-4-methyl-2'-(methylsulfinyl)-3,4,5',8'-tetrahydro-2H-spiro-[naphthalene-1,7'-pyrano[4,3-d]pyrimidine]-8-carbonitrile and (1S,4S)-4'-(benzyloxy)-7-(dibenzylamino)-4-methyl-2'-(methylsulfonyl)-3,4,5',8'-tetrahydro-2H-spiro[naphthalene-1,7'-pyrano[4,3-d]pyrimidine]-8-carbonitrile

Oxone (7.08 g, 2 *equiv.,* 11.5 mmol) was added to a solution of (1*S*,4*S*)-4'-(benzyloxy)-7-(dibenzylamino)-4-methyl-2'-(methylthio)-3,4,5',8'-tetrahydro-2*H*-spiro[naphthalene-1,7'-pyrano[4,3-*d*]pyrimidine]-8-carbonitrile (3.68 g, 1 *equiv.,* 5.76 mmol) in THF (100 mL) and water (50 mL). The cloudy reaction mixture was stirred for 2 hours at room temperature. The product mixture was quenched with a 10% aqueous solution of sodium ascorbate (100 mL) and stirred for 5 minutes. The quenched mixture was transferred to a separatory funnel, and the aqueous layer was extracted with EtOAc (2 x 100 mL). The combined organic layers were washed with 1:1 brine:water (100 mL), dried over magnesium sulfate, filtered, and concentrated to provide a mixture of (1*S*,4*S*)-4'-(benzyloxy)-7-(dibenzylamino)-4-methyl-2'-(methylsulfinyl)-3,4,5',8'-tetrahydro-2*H*-spiro[naphthalene-1,7'-pyrano[4,3-*d*]pyrimidine]-8-carbonitrile and (1*S*,4*S*)-4'-(benzyloxy)-7-(dibenzylamino)-4-methyl-2'-(methylsulfonyl)-3,4,5',8'-tetrahydro-2*H*-spiro[naphthalene-1,7'-pyrano[4,3-*d*]pyrimidine]-8-carbonitrile (4.07 g, 6.22 mmol), which was used without further purification.
(1*S*,4*S*)-4'-(benzyloxy)-7-(dibenzylamino)-4-methyl-2'-(methylsulfinyl)-3,4,5',8'-tetrahydro-2*H*-spiro[naphthalene-1,7'-pyrano[4,3-*d*]pyrimidine]-8-carbonitrile LCMS: m/z (ESI) [M+H]⁺ 655.3, t_{R} = 2.29 minutes (Method L)
(1*S*,4*S*)-4'-(benzyloxy)-7-(dibenzylamino)-4-methyl-2'-(methylsulfonyl)-3,4,5',8'-tetrahydro-2*H*-spiro[naphthalene-1,7'-pyrano[4,3-*d*]pyrimidine]-8-carbonitrile LCMS: m/z (ESI) [M+H]⁺ 671.3, t_{R} = 2.32 minutes (Methos L)

### Step 4: (1S,4S)-4'-(benzyloxy)-7-(dibenzylamino)-2'-(((2R,7aS)-2-fluorotetrahydro-1H-pyrrolizin-7a(5H)-yl)methoxy)-4-methyl-3,4,5',8'-tetrahydro-2H-spiro[naphthalene-1,7'-pyrano[4,3-d]pyrimidine]-8-carbonitrile

A mixture of (1*S*,4*S*)-4'-(benzyloxy)-7-(dibenzylamino)-4-methyl-2'-(methylsulfinyl)-3,4,5',8'-tetrahydro-2*H*-spiro[naphthalene-1,7'-pyrano[4,3-*d*]pyrimidine]-8-carbonitrile and (1*S*,4*S*)-4'-(benzyloxy)-7-(dibenzylamino)-4-methyl-2'-(methylsulfonyl)-3,4,5',8'-tetrahydro-2*H*-spiro[naphthalene-1,7'-pyrano[4,3-*d*]pyrimidine]-8-carbonitrile (994 mg) and ((2*R*,7a*S*)-2-fluorotetrahydro-1*H*-pyrrolizin-7a(5*H*)-yl)methanol (252 mg, 1.04 *equiv.,* 1.58 mmol) in toluene (16 mL) was cooled in a -30 °C bath. To this mixture, sodium 2-methylbutan-2-olate (201 mg, 1.20 *equiv.,* 1.83 mmol) was added in one portion. The resulting reaction mixture was stirred for 15 minutes at -30 °C and then warmed to -20 °C. The mixture was stirred at this temperature for 5 minutes. The product mixture was quenched with a 4 M solution of HCl in dioxanes (0.5 mL) followed by a saturated aqueous sodium bicarbonate solution (10 mL). The aqueous layer was extracted with EtOAc (3 x 15 mL). The combined organic layers were washed with brine (30 mL), dried over magnesium sulfate, filtered, and concentrated to yield (1*S*,4*S*)-4'-(benzyloxy)-7-(dibenzylamino)-2'-(((2*R*,7a*S*)-2-fluorotetrahydro-1*H*-pyrrolizin-7a(5*H*)-yl)methoxy)-4-methyl-3,4,5',8'-tetrahydro-2*H*-spiro[naphthalene-1,7'-pyrano[4,3-*d*]pyrimidine]-8-carbonitrile (980 mg), which was used without further purification.
LCMS: m/z (ESI) [M+H]⁺ 750.4, t_{R} = 1.97 minutes (Method C)

### Step 5: (1S,4S)-7-amino-2'-(((2R,7aS)-2-fluorotetrahydro-1H-pyrrolizin-7a(5H)-yl)methoxy)-4'-hydroxy-4-methyl-3,4,5',8'-tetrahydro-2H-spiro[naphthalene-1,7'-pyrano[4,3-d]pyrimidine]-8-carbonitrile

Pd(OH)₂ on carbon (1.08 g, 20 wt%, 1.2 *equiv.,* 1.54 mmol) was added to a solution of (1*S*,4*S*)-4'-(benzyloxy)-7-(dibenzylamino)-2'-(((2*R*,7a*S*)-2-fluorotetrahydro-1*H*-pyrrolizin-7a(5*H*)-yl)methoxy)-4-methyl-3,4,5',8'-tetrahydro-2*H*-spiro[naphthalene-1,7'-pyrano[4,3-*d*]pyrimidine]-8-carbonitrile (958 mg, 1 *equiv.,* 1.28 mmol) in MeOH (33 mL) and DCM (6.2 mL) under a N₂ atmosphere. The mixture was sparged with H₂. The reaction mixture was then stirred under a hydrogen atmosphere for 25 hours. The reaction mixture was filtered over celite and concentrated. The resulting residue was purified by column chromatography (SiO₂, eluting with 5% MeOH/DCM +1% NH₄OH) to afford (1*S*,4*S*)-7-amino-2'-(((2*R*,7a*S*)-2-fluorotetrahydro-1*H*-pyrrolizin-7a(5*H*)-yl)methoxy)-4'-hydroxy-4-methyl-3,4,5',8'-tetrahydro-2*H*-spiro[naphthalene-1,7'-pyrano[4,3-*d*]pyrimidine]-8-carbonitrile (321 mg) as a white solid.
LCMS: m/z (ESI) [M+H]⁺ 480.3, t_{R} = 1.21 minutes (Method C)
¹H NMR (400 MHz, MeOD) δ 7.12 (d, 1H), 6.77 (d, 1H), 5.32 (d, 1H), 4.82 - 4.49 (m, 2H), 4.30 - 4.18 (m, 2H), 3.49 - 3.33 (m, 2H), 3.16 - 3.03 (m, 2H), 2.91 - 2.83 (m, 1H), 2.75 - 2.67 (m, 1H), 2.38 - 2.23 (m, 2H), 2.13 - 1.85 (m, 8H), 1.72 - 1.64 (m, 1H), 1.24 (d, 3H).

### Intermediate 21: (S)-7-amino-2'-(((2R,7aS)-2-fluorotetrahydro-1H-pyrrolizin-7a(5H)-yl)methoxy)-4'-hydroxy-3,4,5',8'-tetrahydro-2H-spiro[naphthalene-1,7'-pyrano[4,3-d]pyrimidine]-8-carbonitrile

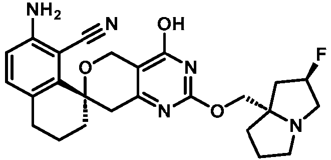

### Step 1: (S)-N,N-dibenzyl-8-bromo-4'-((4-methoxybenzyl)oxy)-2'-(methylthio)-3,4,5',8'-tetrahydro-2H-spiro[naphthalene-1,7'-pyrano[4,3-d]pyrimidin]-7-amine

Sodium 2-methylbutan-2-olate (9.3 g, 60 mL, 1.4 M, 1.7 *equiv.,* 84 mmol) was added dropwise to a stirred solution of *N,N*-dibenzyl-8-bromo-4'-chloro-2'-(methylthio)-3,4,5',8'-tetrahydro-2*H*-spiro[naphthalene-1,7'-pyrano[4,3-d]pyrimidin]-7-amine (30 g, 1 *equiv.,* 49 mmol) and 4-methoxybenzyl alcohol (7.5 g, 6.7 mL, 54 mmol) in THF (100 mL) over 25 minutes. The resulting dark brown mixture was stirred at room temperature for 2 hours. The reaction mixture was quenched by the addition of a saturated aqueous sodium bicarbonate (150 mL) and diluted with EtOAc (150 mL). The two phases were separated, and the aqueous layer was extracted with EtOAc (2 x 100 mL). The combined organic extracts were dried over sodium sulfate, filtered, and concentrated to give the crude product, which was dissolved in EtOAc (50 mL) and sonicated. To this mixture, ether was added to initiate further precipitation, and the mixture was filtered to afford N,N-dibenzyl-8-bromo-4'-((4-methoxybenzyl)oxy)-2'-(methylthio)-3,4,5',8'-tetrahydro-2*H*-spiro[naphthalene-1,7'-pyrano[4,3-*d*]pyrimidin]-7-amine.

¹H NMR (400 MHz, CDCl₃) δ 7.42 - 7.15 (m, 12H), 7.02 - 6.72 (m, 4H), 5.42 (s, 2H), 4.85 (d, *J =* 16.2 Hz, 1H), 4.65 (d, *J* = 16.2 Hz, 1H), 4.21 - 3.92 (m, 5H), 3.85 - 3.79 (m, 3H), 2.79 (m, 3H), 2.58 (d, *J =* 1.5 Hz, 3H), 2.11 (d, *J* = 12.6 Hz, 1H), 1.94 - 1.63 (m, 3H).

The stereoisomers of N,N-dibenzyl-8-bromo-4'-((4-methoxybenzyl)oxy)-2'-(methylthio)-3,4,5',8'-tetrahydro-2*H*-spiro[naphthalene-1,7'-pyrano[4,3*-d*]pyrimidin]-7-amine (14.2 g) were separated by chiral SFC (ChiralPak IC-H 21 x 250 mm, 5µm; EtOH, 35%, flow rate = 80 mL/min) to afford (*S*)-*N,N*-dibenzyl-8-bromo-4'-((4-methoxybenzyl)oxy)-2'-(methylthio)-3,4,5',8'-tetrahydro-2*H*-spiro[naphthalene-1,7'-pyrano[4,3*-d*]pyrimidin]-7-amine (5.82 g).

Analytical Chiral SFC: t_{R} = 2.61 minutes ChiralPak IC-H 21 x 250 mm, 5µm; EtOH, 35%, flow rate = 2.5 mL/min).

¹H NMR (400 MHz, CDCl₃) δ 7.43 - 7.15 (m, 12H), 6.96 - 6.86 (m, 3H), 6.80 (d, *J=* 8.2 Hz, 1H), 5.42 (s, 2H), 4.85 (d, *J* = 16.2 Hz, 1H), 4.65 (d, *J* = 16.4 Hz, 1H), 4.25 - 3.91 (m, 5H), 3.83 (s, 3H), 2.91 - 2.67 (m, 3H), 2.58 (s, 3H), 2.15 - 2.07 (m, 1H), 1.88 (m, 2H), 1.68 (m, 1H).

### Step 2: (S)-7-(dibenzylamino)-4'-hydroxy-2'-(methylthio)-3,4,5',8'-tetrahydro-2H-spiro[naphthalene-1,7'-pyrano[4,3-d]pyrimidine]-8-carbonitrile

Copper(I) cyanide (1.25 g, 428 µL, 10 *equiv.,* 13.9 mmol) was added in one portion to a stirred solution of (*S*)-*N,N*-dibenzyl-8-bromo-4'-((4-methoxybenzyl)oxy)-2'-(methylthio)-3,4,5',8'-tetrahydro-2*H*-spiro[naphthalene-1,7'-pyrano[4,3-*d*]pyrimidin]-7-amine (1.05 g, 94 wt%, 1 *equiv.,* 1.39 mmol) in DMF (27.9 mL). The resulting mixture was heated to 120 °C for approximately 8 hours. The reaction mixture was cooled to room temperature and concentrated under reduced pressure. The resulting residue was diluted with water, DCM, and a 1:1 mixture of NH₄OH and MeOH (200 mL). This mixture was stirred at room temperature for approximately 15 minutes until two layers were observed. The two layers were separated, and the aqueous layer was extracted with DCM (x 3). The combined organic extracts were dried over sodium sulfate, filtered, and concentrated to give the crude product, which was dissolved in EtOAc and washed with 10% LiCl aqueous solution (x 3). The organic layer was separated, dried over sodium sulfate, filtered, and concentrated to give the crude product. The crude product was purified via silica gel chromatography (0:100-A:B to 30:70-A:B where A is a premixed solution of 2.5% NH₄OH and 20% MeOH in DCM and B is DCM) to give (S)-7-(dibenzylamino)-4'-hydroxy-2'-(methylthio)-3,4,5',8'-tetrahydro-2*H*-spiro[naphthalene-1,7'-pyrano[4,3-d]pyrimidine]-8-carbonitrile (430 mg) as an orange solid.
LCMS: m/z (ESI) [M+H]⁺ 535.3, t_{R} = 2.17 minutes (Method C)

### Step 3: (S)-7-(dibenzylamino)-4'-hydroxy-2'-(methylsulfonyl)-3,4,5',8'-tetrahydro-2H-spiro[naphthalene-1,7'-pyrano[4,3-d]pyrimidine]-8-carbonitrile

To a light orange suspension of (*S*)-7-(dibenzylamino)-4'-hydroxy-2'-(methylthio)-3,4,5',8'-tetrahydro-2*H*-spiro[naphthalene-1,7'-pyrano[4,3-*d*]pyrimidine]-8-carbonitrile (430 mg, 1.00 *equiv.,* 804 µmol) in MeCN (10.7 mL) and water (5.36 mL) at room temperature was added potassium phosphate tribasic (427 mg, 2.50 *equiv.,* 2.01 mmol). The resulting mixture was stirred at 40 °C for 30 minutes. The reaction mixture was cooled to room temperature, and the orange organic layers were syringed out and filtered. The organic solution was cooled to 3 °C. To this mixture, hydrogen peroxide (195 mg, 173 µL, 35 wt%, 2.50 *equiv.,* 2.01 mmol) was added dropwise using a glass pipette. The resulting solution was stirred at 0 °C for 45 minutes. The reaction mixture was warmed to 10 °C, quenched by the addition of a saturated aqueous ammonium chloride solution, and diluted with EtOAc. The two layers were separated, and the aqueous layer was extracted with EtOAc (3 x 40 mL). The combined organic layers were dried over magnesium sulfate, filtered, and concentrated to give the crude product (*S*)-7-(dibenzylamino)-4'-hydroxy-2'-(methylsulfonyl)-3,4,5',8'-tetrahydro-2H-spiro[naphthalene-1,7'-pyrano[4,3-*d*]pyrimidine]-8-carbonitrile (334 mg) as a light yellow solid which was used in the next step without further purification.
LCMS: m/z (ESI) [M+H]⁺ 567.2, t_{R} = 2.14 minutes (Method C)

### Step 4: (S)-7-(dibenzylamino)-2'-(((2R,7aS)-2-fluorotetrahydro-1H-pyrrolizin-7a(5H)-yl)methoxy)-4'-hydroxy-3,4,5',8'-tetrahydro-2H-spiro[naphthalene-1,7'-pyrano[4,3-d]pyrimidine]-8-carbonitrile

To a flask charged with (S)-7-(dibenzylamino)-4'-hydroxy-2'-(methylsulfonyl)-3,4,5',8'-tetrahydro-2*H*-spiro[naphthalene-1,7'-pyrano[4,3-*d*]pyrimidine]-8-carbonitrile (334.2 mg, 1 *equiv.,* 589.8 µmol) was added ((2*R*,7a*S*)-2-fluorotetrahydro-1*H*-pyrrolizin-7a(5*H*)-yl)methanol (225.3 mg, 2.4 *equiv.,* 1.415 mmol). The reaction mixture was then cooled to 0 °C, and sodium 2-methylbutan-2-olate (155.9 mg, 2.4 *equiv.,* 1.415 mmol) was added in one portion. The reaction mixture was stirred at 0 °C for 30 minutes and the resulting mixture was stirred at room temperature for 20 minutes. The reaction mixture was then quenched by the addition of a 1:1 mixture of a saturated aqueous ammonium chloride solution and water and diluted with EtOAc (10 mL). The two layers were separated, and the aqueous phase was extracted with EtOAc (3 x 10 mL). The combined organic layers were dried over magnesium sulfate, filtered, and concentrated to give the crude product which was purified by silica gel chromatography (0:100-A:B to 50:50-A:B where A is a premixed solution of 2.5% NH₄OH and 22.5% MeOH in DCM, and B is DCM). The isolated product was repurified by silica gel chromatography (0:100-A:B to 30:70-A:B where A is a premixed solution of 2.5% NH₄OH and 22.5% MeOH in DCM, and B is DCM) to give (S)-7-(dibenzylamino)-2'-(((2R,7aS)-2-fluorotetrahydro-1*H-*pyrrolizin-7a(5*H*)-yl)methoxy)-4'-hydroxy-3,4,5',8'-tetrahydro-2*H-*spiro[naphthalene-1,7'-pyrano[4,3-*d*]pyrimidine]-8-carbonitrile (225 mg).
LCMS: m/z (ESI) [M+H]⁺ 646.3, t_{R} = 1.57 minutes (Method C)

### Step 5: (S)-7-amino-2'-(((2R,7aS)-2-fluorotetrahydro-1H-pyrrolizin-7a(5H)-yl)methoxy)-4'-hydroxy-3,4,5',8'-tetrahydro-2H-spiro[naphthalene-1,7'-pyrano[4,3-d]pyrimidine]-8-carbonitrile

Pd(OH)₂ on carbon (613.2 mg, 20 wt%, 2.5 *equiv.,* 873.4 µmol) was added to a solution of (*S*)-7-(dibenzylamino)-2'-(((2*R*,7a*S*)-2-fluorotetrahydro-1*H*-pyrrolizin-7a(5*H*)-yl)methoxy)-4'-hydroxy-3,4,5',8'-tetrahydro-2*H*-spiro[naphthalene-1,7'-pyrano[4,3-*d*]pyrimidine]-8-carbonitrile (225.6 mg, 1 *equiv.,* 349.3 µmol) in MeOH (7 mL). The resulting mixture was sparged with hydrogen for 5 minutes. The mixture was stirred under a hydrogen atmosphere for 2.5 hours. The reaction mixture was filtered, and the filtrate was concentrated to give (*S*)-7-amino-2'-(((2*R*,7a*S*)-2-fluorotetrahydro-1*H*-pyrrolizin-7a(5*H*)-yl)methoxy)-4'-hydroxy-3,4,5',8'-tetrahydro-2*H*-spiro[naphthalene-1,7'-pyrano[4,3-*d*]pyrimidine]-8-carbonitrile (82.6 mg) as an off-white solid.
LCMS: m/z (ESI) [M+H]⁺ 466.2, t_{R} = 1.16 minutes (Method C)
¹H NMR (400 MHz, DMSO*-d₆*) δ 7.03 (d, 1H), 6.72 (d, 1H), 5.75 (s, 2H), 5.27 (d, 1H), 4.53 - 4.36 (m, 2H), 4.11 - 3.91 (m, 2H), 3.14 - 2.94 (m, 4H), 2.86 - 2.76 (m, 1H), 2.65 - 2.54 (m, 3H), 2.08 - 1.89 (m, 4H), 1.86 - 1.72 (m, 4H), 1.69 - 1.58 (m, 2H).

### Intermediate 22: tert-Butyl (S)-(tert-butoxycarbonyl)(1-cyano-5-methyl-8-oxo-5,6,7,8-tetrahydronaphthalen-2-yl)carbamate

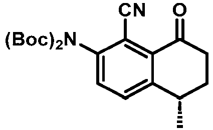

### Step 1: (S)-2-Amino-5-methyl-8-oxo-5,6,7,8-tetrahydronaphthalene-1-carbonitrile

A solution of (*S*)-7-amino-8-bromo-4-methyl-3,4-dihydronaphthalen-1(2*H*)-one (5.03g, 1.8 mmol) and copper(I) cyanide (8.88g, 99.2 mmol, 5 *equiv.*) in DMF (100 mL) was degassed with N₂ for 5 minutes. The reaction mixture was stirred at 100 °C for 1 hour, then cooled to room temperature. NH₄OH (50 mL) was then added and the resulting mixture was stirred for 5 minutes. The resulting suspension was filtered and the solids were washed with 5:1 DCM:IPA. The filtrate was further extracted with 5:1 DCM:IPA. The combined organics were treated with 5% aqueous LiCl and EtOAc (100 mL), washed with 5% aqueous LiCl (3 x 50 mL), and brine (50 mL). The layers were separated, and the organic layers were combined, dried over sodium sulfate, filtered, and concentrated to a residue. The residue was purified by flash silica gel chromatography (0-20% EtOAc/DCM) to give (S)-2-Amino-5-methyl-8-oxo-5,6,7,8-tetrahydronaphthalene-1-carbonitrile (3.22g) as a white solid.
¹H NMR (400 MHz, CDCl₃) δ 7.30 (d, 1H), 6.97 (d, 1H), 4.64 (s, 2H), 3.04 - 2.92 (m, 1H), 2.86 - 2.74 (m, 1H), 2.67 - 2.54 (m, 1H), 2.28 - 2.16 (m, 1H), 1.91 - 1.80 (m, 1H), 1.34 (d, 3H).

### Step 2: tert-Butyl (S)-(tert-butoxycarbonyl)(1-cyano-5-methyl-8-oxo-5,6,7,8-tetrahydronaphthalen-2-yl)carbamate

Dimethylaminopyridine (125 mg, 1.02 mmol, 0.2 *equiv.*) was added to a mixture of (S)-2-Amino-5-methyl-8-oxo-5,6,7,8-tetrahydronaphthalene-1-carbonitrile (102 g, 5.11 mmol, 1 *equiv.*) and di-*tert*-butyl dicarbonate (2.30 g, 10.5 mmol, 2 *equiv.*) in THF (10 mL). The mixture was stirred at room temperature for 4 hours. Water (10 mL) and EtOAc (25 mL) were added, and the phases were separated. The organic layer was washed with water (10 mL) and brine (30 mL) and the layers were separated once more. The combined organic layer was dried over sodium sulfate, filtered, and concentrated to a residue. The residue was purified by flash silica gel chromatography (0-30% EtOAc/heptanes) to afford *tert*-butyl (*S*)-(tert-butoxycarbonyl)(1-cyano-5-methyl-8-oxo-5,6,7,8-tetrahydronaphthalen-2-yl)carbamate (1.66 g) as a white solid.

¹H NMR (400 MHz, CDCl₃) δ 7.58 (dd, 1H), 7.44 (d, 1H), 3.22 - 3.07 (m, 1H), 2.93 - 2.80 (m, 1H), 2.76 - 2.63 (m, 1H), 2.34 - 2.19 (m, 1H), 2.05 - 1.84 (m, 1H), 1.47 (s, 18H), 1.42 (d, 3H).

### Intermediate 23: (4-Chloro-2-(((2R,7aS)-2-fluorotetrahydro-1H-pyrrolizin-7a(5H)-yl)methoxy)-6-methylpyrimidin-5-yl)methanol

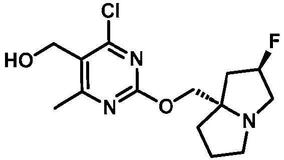

### Step 1: 5-(((tert-Butyldimethylsilyl)oxy)methyl)-4-chloro-6-methyl-2-(methylthio)pyrimidine

*Tert*-butyldimethylsilyl trifluoromethanesulfonate (33.8 mL, 147 mmol, 1.5 *equiv.*) was added dropwise to a solution of (4-chloro-6-methyl-2-(methylthio)pyrimidin-5-yl)methanol (20.1 g, 98.2 mmol, 1 *equiv.*)*,* 2,6-lutidine (22.9 mL, 196 mmol, 2 *equiv.*)*,* and DMAP (1.20g, 9.82 mmol, 0.1 *equiv.*) in DCM (500 mL) at -5 °C over 30 minutes, while maintaining the internal temperature below 0 °C. The reaction mixture was stirred at 0 °C for 70 minutes then quenched with saturated sodium bicarbonate (100 mL). The organic layer was washed with saturated sodium bicarbonate (100 mL), saturated ammonium chloride (3 x 100 mL), and brine (100 mL). The layers were separated and the organic layer was dried over sodium sulfate, filtered, and concentrated to a residue. The residue was purified by flash silica gel chromatography (ethyl ether/heptanes) to give 5-(((*tert-*butyldimethylsilyl)oxy)methyl)-4-chloro-6-methyl-2-(methylthio)pyrimidine (19.3g) as a white solid.

¹H NMR (400 MHz, CDCl₃) δ 4.76 (s, 2H), 2.60 - 2.51 (m, 6H), 0.89 (s, 9H), 0.11 (d, 6H).

### Step 2: 5-(((tert-Butyldimethylsilyl)oxy)methyl)-4-chloro-6-methyl-2-(methylsulfonyl)pyrimidine

*m*CPBA (40.3g, 180 mmol, 77 wt%, 3 *equiv.*) was added portion-wise over 1 hour to a solution of 5-(((*tert-*butyldimethylsilyl)oxy)methyl)-4-chloro-6-methyl-2-(methylthio)pyrimidine (19.1g, 59.9 mmol, 1 *equiv.*) in DCM (200 mL) at -5 °C, while ensuring the internal temperature remained below 0 °C. The reaction mixture was stirred at 0 °C for 30 minutes then at room temperature for 2 hours and 40 minutes. The mixture was then cooled to 0 °C and quenched with saturated sodium bicarbonate (400 mL). The organic layer was washed with saturated sodium thiosulfate (200 mL) and 1N sodium hydroxide (2 x 200 mL). The aqueous layer was back extracted with DCM (3x200 mL). The combined organic layers were dried over sodium sulfate, filtered, and concentrated to afford 5-(((*tert-*butyldimethylsilyl)oxy)methyl)-4-chloro-6-methyl-2-(methylsulfonyl)pyrimidine (21.2 g) as a white solid.

¹H NMR (400 MHz, CDCl₃) δ 4.88 (d, 2H), 3.36 (d, 3H), 2.77 (d, 3H), 0.90 (d, 9H), 0.14 (d, 6H).

### Step 3: (2R,7aS)-7a-(((5-(((tert-Butyldimethylsilyl)oxy)methyl)-4-chloro-6-methylpyrimidin-2-yl)oxy)methyl)-2-fluorohexahydro-1H-pyrrolizine

Sodium 2-methylbutan-2-olate (1.4 M, 39.6 mL, 55.4 mmol, 1 *equiv.*) was added to a degassed solution of 5-(((*tert*-butyldimethylsilyl)oxy)methyl)-4-chloro-6-methyl-2-(methylsulfonyl)pyrimidine (20.9 g, 55.4 mmol, 1 *equiv.*) and ((2*R*,7a*S*)-2-fluorotetrahydro-1*H*-pyrrolizin-7a(5*H*)-yl)methanol (7.94 g, 49.8 mmol, 0.9 *equiv.*) in THF (300 mL) at -5 °C. The resulting reaction mixture was stirred at 0 °C for 2 hours. To this mixture, saturated sodium bicarbonate (200 mL) and EtOAc (200 mL) were added. The aqueous layer was extracted with EtOAc (2 x 200 mL) and the combined organics were washed with brine (200 mL), dried over sodium sulfate, filtered, and concentrated to yield (2*R,7aS*)-7a*-*(((5-(((*tert-*butyldimethylsilyl)oxy)methyl)-4-chloro-6-methylpyrimidin-2-yl)oxy)methyl)-2-fluorohexahydro-1*H*-pyrrolizine (27.1 g) as a white solid.
LCMS: m/z (ESI) [M+H]⁺430.4, t_{R} = 1.30 minutes (Method A)

### Step 4: (4-Chloro-2-(((2R,7aS)-2-fluorotetrahydro-1H-pyrrolizin-7a(5H)-yl)methoxy)-6-methylpyrimidin-5-yl)methanol

TBAF (1 M, 72.1 mL, 72.1 mmol, 1.3 *equiv.*) was added over 30 minutes to a degassed solution of (2*R*,7a*S*)-7a-(((5-(((*tert*-butyldimethylsilyl)oxy)methyl)-4-chloro-6-methylpyrimidin-2-yl)oxy)methyl)-2-fluorohexahydro-1*H*-pyrrolizine (27.1 g, 55 mmol) in THF (300 mL) at room temperature. The reaction was stirred at room temperature for 2.5 hours. To this mixture, saturated sodium bicarbonate (200 mL) and EtOAc (200 mL) were added. The aqueous layer was extracted with EtOAc (4 x 200 mL) and the combined organic layers were dried over sodium sulfate, filtered, and concentrated to a residue which was purified twice by column chromatography (first with DCM in MeOH, then with EtOAc:EtOH:IPA:TEA 300:95:5:8 in hexanes) to afford (4-chloro-2-(((2*R*,7a*S*)-2-fluorotetrahydro-1*H*-pyrrolizin-7a(5H)-yl)methoxy)-6-methylpyrimidin-5-yl)methanol (7.95g) as a white solid.

¹H NMR (400 MHz, CDCl₃) δ 5.24 (d, 1H), 4.77 (s, 2H), 4.18 - 4.02 (m, 2H), 3.29 - 3.18 (m, 2H), 3.18 - 3.08 (m, 1H), 3.01-2.90 (m, 1H), 2.58 (s, 3H), 2.27 - 2.19 (m, 1H), 2.18 - 2.11 (m, 1H), 2.11 - 2.04 (m, 1H), 2.00 - 1.79 (m, 4H).

### Intermediate 24: Di-tert-butyl ((4S)-4'-chloro-8-cyano-2'-(((2S,7aR)-2-fluorotetrahydro-1H-pyrrolizin-7a(5H)-yl)methoxy)-4-methyl-3,4,5',8'-tetrahydro-2H-spiro[naphthalene-1,7'-pyrano[4,3-d]pyrimidin]-7-yl)iminodicarbonate

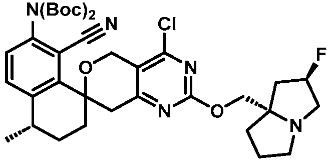

nBuLi (2.5 M, 10.5 mL, 26.3 mmol, 2.46 *equiv.*) was added to diisopropylamine (3.70 mL, 26.2 mmol, 2.46 *equiv.*) in THF at -78 °C, while maintaining the internal temperature below -45 °C. The solution was warmed to room temperature and stirred for 15 minutes. The mixture was cooled again to -78 °C followed by the addition of THF (6 mL). A solution of (4-chloro-2-(((2*R*,7a*S*)-2-fluorotetrahydro-1*H*-pyrrolizin-7a(5*H*)-yl)methoxy)-6-methylpyrimidin-5-yl)methanol (3.71g, 11.7 mmol, 1.1 *equiv.*) in THF (10 mL) was added at a rate to keep the internal temperature below -68 °C. The mixture was stirred at -78 °C for an additional 20 minutes. *tert*-Butyl (S)-(tert-butoxycarbonyl)(1-cyano-5-methyl-8-oxo-5,6,7,8-tetrahydronaphthalen-2-yl)carbamate (4.27 g, 10.7 mmol, 1 *equiv.*) in THF (6 mL) was then added to the reaction mixture at -75 °C, while maintaining the temperature below -70 °C. The reaction mixture was stirred at -75 °C for 20 minutes, after which 4-nitrobenzene-1-sulfonyl chloride (5.67 g, 25.6 mmol, 2.4 *equiv.*) in THF (10 mL) was added while keeping the temperature below -70 °C. The reaction was stirred at -70 °C for 20 minutes then quenched with saturated sodium bicarbonate (7 mL). The resulting mixture was warmed to room temperature and was extracted with DCM (3 x 5 mL). The combined organic layers were washed with saturated sodium bicarbonate (3x5 mL) and resulting layers were separated and the organic layer was dried over sodium sulfate, filtered, and concentrated to a residue. The residue was purified by column chromatography (5-10% of 2.5% NH₄OH in 20% MeOH in DCM/DCM) to afford di-*tert*-butyl ((4*S*)-4'-chloro-8-cyano-2'-(((2*S*,7a*R*)-2-fluorotetrahydro-1H-pyrrolizin-7a(5*H*)-yl)methoxy)-4-methyl-3,4,5',8'-tetrahydro-2*H*-spiro[naphthalene-1,7'-pyrano[4,3-*d*]pyrimidin]-7-yl)iminodicarbonate (3.69 g) as a white solid.
LCMS: m/z (ESI) [M+H]⁺ 698.5, t_{R} = 1.78 minutes (Method L)

**Intermediate 24a: *tert*-butyl (*tert*-butoxycarbonyl)((1*S*,4*S*)-4'-chloro-8-cyano-2'-(((2*R*,7a*S*)-2-fluorotetrahydro-1*H*-pyrrolizin-7a(5*H*)-yl)methoxy)-4-methyl-3,4,5',8'-tetrahydro-2*H*-spiro[naphthalene-1,7'-pyrano[4,3-*d*]pyrimidin]-7-yl)carbamate**

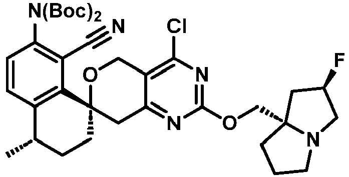

The diastereomers of *tert*-butyl (tert-butoxycarbonyl)((1R*S*,4*S*)-4'-chloro-8-cyano-2'-(((2*R*,7a*S*)-2-fluorotetrahydro-1*H*-pyrrolizin-7a(5*H*)-yl)methoxy)-4-methyl-3,4,5',8'-tetrahydro-2*H*-spiro[naphthalene-1,7'-pyrano[4,3-*d*]pyrimidin]-7-yl)carbamate were separated by SFC purification (Diacel Chiralpak IC, 30 x 250 mm; 5 µm; part number 83445; Mobile Phase A: CO₂; Mobile Phase B: MeOH with 0.1% TEA; 20%B isocratic) to afford *tert*-butyl (*tert*-butoxycarbonyl)((1*S*,4*S*)-4'-chloro-8-cyano-2'-(((2*R*,7a*S*)-2-fluorotetrahydro-1*H-*pyrrolizin-7a(5*H*)-yl)methoxy)-4-methyl-3,4,5',8'-tetrahydro-2*H*-spiro[naphthalene-1,7'-pyrano[4,3-*d*]pyrimidin]-7-yl)carbamate (**Peak 1**).

LCMS: m/z (ESI) [M+H]⁺ 698.4, t_{R} = 2.21 minutes (Diacel Chiralpak IC, 100 x 4.6 mm; 5 µm; part number 83423; Mobile Phase A: CO₂; Mobile Phase B: MeOH with 0.1% TEA; 20%B isocratic).

¹H NMR (400 MHz, CDCl₃) δ 7.37 (d, 1H), 7.17 (d, 1H), 5.36 - 5.15 (m, 1H; contains residual DCM), 5.01 (d, 1H), 4.78 (d, 1H), 4.15 - 4.05 (m, 2H), 3.37 (d, 1H), 3.28 - 2.90 (m, 6H), 2.30 - 2.04 (m, 4H), 2.04 - 1.79 (m, 5H), 1.73 (d, 1H), 1.52 - 1.39 (m, 18H), 1.32 (d, 3H).

### Intermediate 24b: tert-butyl (tert-butoxycarbonyl)((1R,4S)-4'-chloro-8-cyano-2'-(((2R,7aS)-2-fluorotetrahydro-1H-pyrrolizin-7a(5H)-yl)methoxy)-4-methyl-3,4,5',8'-tetrahydro-2H-spiro[naphthalene-1,7'-pyrano[4,3-d]pyrimidin]-7-yl)

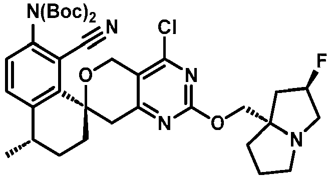

*tert-*butyl (*tert-*butoxycarbonyl)((1*R*,4S*)*-4'-chloro-8-cyano-2'-(((2*R*,7a*S*)-2-fluorotetrahydro-1*H*-pyrrolizin-7a(5*H*)-yl)methoxy)-4-methyl-3,4,5',8'-tetrahydro-2*H-*spiro[naphthalene-1,7'-pyrano[4,3-*d*]pyrimidin]-7-yl) was obtained from **Intermediate 24a as Peak 2.**

¹H NMR (400 MHz, CDCl₃) δ 7.53 (d, 1H), 7.20 (d, 1H), 5.35 - 5.13 (m, 1H), 4.99 (d, 1H), 4.74 (d, 1H), 4.18 - 4.03 (m, 2H), 3.46 (d, 1H), 3.32 - 3.01 (m, 4H), 2.94 (qd, 2H), 2.28 - 1.97 (m, 5H), 1.97 - 1.79 (m, 4H), 1.69 - 1.56 (m, 1H), 1.45 (d, 18H), 1.35 (d, 3H).

LCMS: m/z (ESI) [M+H]⁺ 698.4, t_{R} = 2.86 minutes (Diacel Chiralpak IC, 100 x 4.6 mm; 5 µm; part number 83423; Mobile Phase A: CO₂; Mobile Phase B: MeOH with 0.1% TEA; 20%B isocratic).

### General Procedures:

### General Procedure A:

A mixture of *tert*-butyl (2'-(((2*R*,7a*S*)-2-fluorotetrahydro-1*H*-pyrrolizin-7a(5*H*)-yl)methoxy)-4'-oxo-3,3',4,4',5',8'-hexahydro-2*H*-spiro[naphthalene-1,7'-pyrano[4,3-*d*]pyrimidin]-7-yl)carbamate (1.0 *equiv.*) and PyBOP (1.5 *equiv.*) in *N,N*-dimethylformamide (0.2-0.5 M) and DIPEA (2.5 *equiv.*) was stirred for 30 minutes at room temperature. The reaction was quenched with ice, and the resulting suspension was filtered. The resulting solid was dried to obtain *tert*-butyl (4'-((1*H*-benzo[d][1,2,3]triazol-1-yl)oxy)-2'-(((2*R*,7a*S*)-2-fluorotetrahydro-1*H*-pyrrolizin-7a(5*H*)-yl)methoxy)-3,4,5',8'-tetrahydro-2*H-*spiro[naphthalene-1,7'-pyrano[4,3-*d*]pyrimidin]-7-yl)carbamate which was used without further purification.

The freebase or salt form of the appropriate amine (1.5-3.0 *equiv.*) was added to a solution of *tert-*butyl (4'-((1*H*-benzo[d][1,2,3]triazol-1-yl)oxy)-2'-(((2*R*,7a*S*)-2-fluorotetrahydro-1*H*-pyrrolizin-7a(5*H*)-yl)methoxy)-3,4,5',8'-tetrahydro-2*H-*spiro[naphthalene-1,7'-pyrano[4,3-*d*]pyrimidin]-7-yl)carbamate (1.0 *equiv.*) and DIPEA (3.0 *equiv.*) in *N,N-*dimethylformamide or ethanol (0.2 M). The reaction mixture was stirred at 100 °C for 2 hours. The mixture was then cooled to room temperature and quenched with ice. The resulting solid was either filtered or purified by column chromatography to afford the Boc-protected title compound. MeCN (0.03 M) and *p*-toluenesulfonic acid (2.0 *equiv.*) was added, and the mixture stirred at 50 °C for 3 hours. Alternatively, the mixture was treated with HCl in diethyl ether at room temperature. The reaction mixture was concentrated to a residue, which was purified by flash chromatography or preparative HPLC to yield the title compound.

### General Procedure B:

The freebase or salt form of the appropriate amine (1.5-3.0 *equiv.*) was added to a solution of *tert-*butyl ((*S*)-4'-((1*H*-benzo[d][1,2,3]triazol-1-yl)oxy)-2'-(((2*R*,7a*S*)-2-fluorotetrahydro-1*H*-pyrrolizin-7a(5*H*)-yl)methoxy)-3,4,5',8'-tetrahydro-2*H-*spiro[naphthalene-1,7'-pyrano[4,3-*d*]pyrimidin]-7-yl)carbamate (1.0 *equiv.*) in *N,N-*dimethylformamide or ethanol (0.2 M) and DIPEA (3.0 *equiv.*)*.* The reaction mixture was stirred at 100 °C for 2 hours then quenched with ice. The resulting solid was either filtered or purified by column chromatography to afford the Boc-protected title compound. MeCN (0.03 M) and *p*-toluenesulfonic acid (2.0 *equiv.*) was added, and the mixture was stirred at 50 °C for 3 hours. Alternatively, the mixture was treated with HCl in diethyl ether at room temperature. The reaction mixture was concentrated to a residue which was purified by flash chromatography or preparative HPLC to yield the title compound.

### General Procedure C:

The appropriate amine (1.0-2.0 *equiv.*) is added to a solution of *tert*-butyl (*tert-*butoxycarbonyl)((1*S*,4*S*)-4'-chloro-8-cyano-2'-(((2*R*,7a*S*)-2-fluorotetrahydro-1*H*-pyrrolizin-7a(5*H*)-yl)methoxy)-4-methyl-3,4,5',8'-tetrahydro-2*H*-spiro[naphthalene-1,7'-pyrano[4,3-*d*]pyrimidin]-7-yl)carbamate (Intermediate 24a; 1.0 *equiv.*) and base such as DIPEA or TEA (2.0-5.0 *equiv.*) in EtOH (0.5-0.1 M) at room temperature. The resulting mixture is stirred at 70°C for 1-2 hours then cooled to room temperature. EtOAc is added and the mixture is washed with water and brine then dried over sodium sulfate, filtered, and concentrated to a residue which is purified by column chromatography. The resulting product is dissolved in an appropriate solvent such as MeOH, DCM, or MeCN and treated with an excess of acid (HCl or TFA) at 0°C and stirred for 0.5-2 hours. The pH is adjusted to 8 with saturated sodium bicarbonate and extracted with DCM. The combined organic layers are washed with saturated sodium bicarbonate, dried over sodium sulfate, and concentrated to a residue which may be purified by column chromatography to afford the title compound.

### Example 1: 2'-(((2R,7aS)-2-fluorotetrahydro-1H-pyrrolizin-7a(5H)-yl)methoxy)-4'-((R)-3-hydroxy-3-methylpiperidin-1-yl)-2,3,5',8'-tetrahydrospiro[indene-1,7'-pyrano[4,3-d]pyrimidin]-6-ol (Compound R101a)

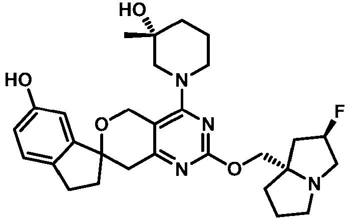

(3*R*)-1-(2'-(((2*R*,7a*S*)-2-Fluorotetrahydro-1*H*-pyrrolizin-7a(5*H*)-yl)methoxy)-6-(4,4,5,5-tetramethyl-1,3,2-dioxaborolan-2-yl)-2,3,5',8'-tetrahydrospiro[indene-1,7'-pyrano[4,3-*d*]pyrimidin]-4'-yl)-3-methylpiperidin-3-ol (80 mg, 1 *equiv.,* 130 µmol) in THF (1 mL) and MeOH (1 mL) was treated with aqueous sodium hydroxide (5.0 mg, 0.130 mL, 1 *equiv.,* 130 µmol) and hydrogen peroxide (24 mg, 22 µL, 35 wt%, 2 *equiv.,* 0.25 mmol) at room temperature for 30 minutes. The reaction was quenched with aqueous sodium bicarbonate and extracted with EtOAc (3 x 10 mL). The organic extracts were dried over sodium sulfate, filtered, and dried to a crude product, which was purified by reverse-phase column chromatography (ACCQPrep, Mobile phase A = 0.1% NH₄OH in water; Mobile phase B = 0.1% NH₄OH in MeCN; Gradient = 100/10 to 100/95) to give 2'-(((2*R*,7a*S*)-2-fluorotetrahydro-1*H*-pyrrolizin-7a(5*H*)-yl)methoxy)-4'-((*R*)-3-hydroxy-3-methylpiperidin-1-yl)-2,3,5',8'-tetrahydrospiro[indene-1,7'-pyrano[4,3-*d*]pyrimidin]-6-ol (10 mg).
LCMS: ESI [M+H]⁺ 525.8; t_{R} = 1.23 minutes. (Method C)
¹H NMR (400 MHz, CDCl₃) δ 7.14 (dd, 1H), 6.77 (dt, 1H), 6.61 (dd, 1H), 5.35 - 5.11 (m, 1H), 4.57 - 4.39 (m, 2H), 4.10 (dd, 1H), 4.00 (dd, 1H), 3.79 - 3.70 (m, 1H), 3.54 (dd, 1H), 3.28 - 2.86 (m, 8H), 2.82 - 2.68 (m, 1H), 2.42 - 2.28 (m, 1H), 2.26 - 2.05 (m, 3H), 1.96 - 1.71 (m, 5H), 1.69 - 1.44 (m, 4H), 1.27 - 1.13 (m, 3H).

### Example 3: (3R)-1-(6-amino-2'-(((2R,7aS)-2-fluorotetrahydro-1H-pyrrolizin-7a(5H)-yl)methoxy)-2,3,5',8'-tetrahydrospiro[indene-1,7'-pyrano[4,3-d]pyrimidin]-4'-yl)-3-methylpiperidin-3-ol (Compound 109a)

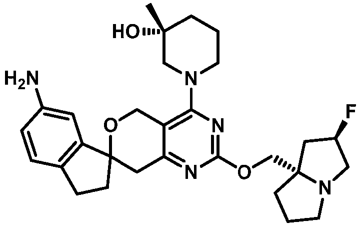

### Step 1: tert-butyl (2'-(((2R,7aS)-2-fluorotetrahydro-1H-pyrrolizin-7a(5H)-yl)methoxy)-4'-((R)-3-hydroxy-3-methylpiperidin-1-yl)-2,3,5',8'-tetrahydrospiro[indene-1,7'-pyrano[4,3-d]pyrimidin]-6-yl)carbamate

(3*R*)-1-(6-Bromo-2'-(((2*R*,7a*S*)-2-fluorotetrahydro-1*H*-pyrrolizin-7a(5*H*)-yl)methoxy)-2,3,5',8'-tetrahydrospiro[indene-1,7'-pyrano[4,3-d]pyrimidin]-4'-yl)-3-methylpiperidin-3-ol (300 mg, *1 equiv.,* 511 µmol), *tert-*butyl carbamate (150 mg, 2.5 *equiv.,* 1.28 mmol), and cesium carbonate (499 mg, 3 *equiv.,* 1.53 mmol) were taken up in 1,4-dioxane (3 mL). The vial was degassed with nitrogen and Pd(OAc)₂ (11.5 mg, 0.1 *equiv.,* 51.1 µmol) and XantPhos (35.5 mg, 0.12 *equiv.,* 61.3 µmol) were added. The reaction mixture was heated to 100 °C and purified via column chromatography (SiO₂, DCM/MeOH = 100/0 to 10/2, ammonia modifier) to give *tert*-butyl (2'-(((2*R*,7a*S*)-2-fluorotetrahydro-1*H*-pyrrolizin-7a(5*H*)-yl)methoxy)-4'-((*R*)-3-hydroxy-3-methylpiperidin-1-yl)-2,3,5',8'-tetrahydrospiro[indene-1,7'-pyrano[4,3-*d*]pyrimidin]-6-yl)carbamate (120 mg).
LCMS: ESI [M+H]⁺ 624.8; t_{R} = 1.43min. (Method C)

### Step 2: (3R)-1-(6-amino-2'-(((2R,7aS)-2-fluorotetrahydro-1H-pyrrolizin-7a(5H)-yl)methoxy)-2,3,5',8'-tetrahydrospiro[indene-1,7'-pyrano[4,3-d]pyrimidin]-4'-yl)-3-methylpiperidin-3-ol

*tert-*Butyl (2'-(((2*R*,7a*S*)-2-Fluorotetrahydro-1*H*-pyrrolizin-7a(5*H*)-yl)methoxy)-4'-((*R*)-3-hydroxy-3-methylpiperidin-1-yl)-2,3,5',8'-tetrahydrospiro[indene-1,7'-pyrano[4,3-*d*]pyrimidin]-6-yl)carbamate (120 mg, 1 *equiv.,* 192 µmol) in MeCN (1 mL) was treated with HCl in 1,4-dioxane (4 M, 56.1 mg, 8 *equiv.,* 1.54 mmol). The reaction mixture was stirred at room temperature for 1 hour, quenched with aqueous sodium bicarbonate, diluted with EtOAc, and extracted with EtOAc (2 x 10 mL). The organic extracts were dried over sodium sulfate, filtered, and concentrated to a crude product, which was purified via reverse phase chromatography (ACCQPrep, water/MeCN, 0.1% formic acid modifier = 100/10 to 100/95) to give (3*R*)-1-(6-amino-2'-(((2R,7a*S*)-2-fluorotetrahydro-1*H*-pyrrolizin-7a(5*H*)-yl)methoxy)-2,3,5',8'-tetrahydrospiro[indene-1,7'-pyrano[4,3-*d*]pyrimidin]-4'-yl)-3-methylpiperidin-3-ol (9 mg).
LCMS: ESI [M+H]⁺ 524.5; t_{R} = 1.27min. (Method C)
¹H NMR (400 MHz, CDCl₃) δ 8.31 (s, 1H), 7.08 (t, *J =* 6.9 Hz, 1H), 6.63 (d, *J =* 7.9 Hz, 1H), 6.51 (d, *J =* 10.4 Hz, 1H), 5.36 (d, *J =* 53.0 Hz, 1H), 4.63 - 4.45 (m, 2H), 4.45 - 4.23 (m, 2H), 3.91 - 3.50 (m, 5H), 3.30 (dd, *J =* 18.5, 14.5 Hz, 1H), 3.12 - 2.91 (m, 6H), 2.79 - 2.67 (m, 1H), 2.49 - 2.25 (m, 4H), 2.22 - 2.00 (m, 4H), 1.95 - 1.73 (m, 2H), 1.60 - 1.48 (m, 2H), 1.26 - 1.20 (m, 3H).

### Example 4: 4'-(7,8-dihydro-4H-[1,2,3]triazolo[1,5-a][1,4]diazepin-5(6H)-yl)-2'-(((2R,7aS)-2-fluorotetrahydro-1H-pyrrolizin-7a(5H)-yl)methoxy)-2,3,5',8'-tetrahydrospiro[indene-1,7'-pyrano[4,3-d]pyrimidin]-6-ol (Compound 106a)

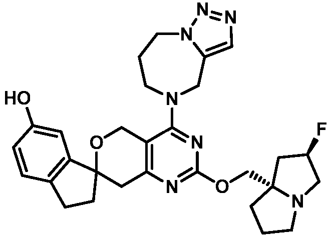

### Step 1: 6-bromo-4'-(7,8-dihydro-4H-[1,2,3]triazolo[1,5-a][1,4]diazepin-5(6H)-yl)-2'-(methylthio)-2,3,5',8'-tetrahydrospiro[indene-1,7'-pyrano[4,3-d]pyrimidine]

6-Bromo-4'-chloro-2'-(methylthio)-2,3,5',8'-tetrahydrospiro[indene-1,7'-pyrano[4,3-*d*]pyrimidine] (0.56 g, 1 *equiv.,* 1.4 mmol) and 5,6,7,8-tetrahydro-4*H*-[1,2,3]triazolo[1,5-*a*][1,4]diazepine, HCl (0.37 g, 1.5 *equiv.,* 2.1 mmol) in ethanol (5 mL) were treated with DIPEA (0.54 g, 3 *equiv.,* 4.2 mmol) followed by the addition of MeCN (1 mL). After 1 hour of stirring at room temperature, an additional portion of 5,6,7,8-tetrahydro-4*H-*[1,2,3]triazolo[1,5-*a*][1,4]diazepine, hydrochloride (200 mg) was added along with DIPEA (4 *equiv.*)*.* The resulting reaction mixture was stirred for two days. The reaction was quenched with aqueous ammonium chloride, diluted with EtOAc, and extracted with EtOAc (2 x 15 mL). The organic layers were dried over sodium sulfate, filtered, and concentrated to a crude product, which was then purified by column chromatography (SiO₂, heptanes/EtOAc = 100/5 to 100/100) to give 6-bromo-4'-(7,8-dihydro-4*H*-[1,2,3]triazolo[1,5-*a*][1,4]diazepin-5(6*H*)-yl)-2'-(methylthio)-2,3,5',8'-tetrahydrospiro[indene-1,7'-pyrano[4,3-*d*]pyrimidine] (262 mg).
LCMS: ESI [M+H]⁺ 501.0; t_{R} = 1.85 minutes. (Method C)
¹H NMR (400 MHz, CDCl₃) δ 7.56 (s, 1H), 7.44 (d, 1H), 7.20-7.24 (m, 2H), 4.46-4.78 (m, 6H), 3.89-3.95 (m, 1H), 3.75-3.81 (m, 1H), 2.98-3.14 (m, 3H), 2.79-2.84 (m, 1H), 2.51 (s, 3H), 2.32-2.38 (m, 1H), 2.11-2.24 (m, 3H).

### Step 2: 6-bromo-4'-(7,8-dihydro-4H-[1,2,3]triazolo[1,5-a][1,4]diazepin-5(6H)-yl)-2'-(methylsulfonyl)-2,3,5',8'-tetrahydrospiro[indene-1,7'-pyrano[4,3-d]pyrimidine]

6-Bromo-4'-(7,8-dihydro-4*H*-[1,2,3]triazolo[1,5-*a*][1,4]diazepin-5(6*H*)-yl)-2'-(methylthio)-2,3,5',8'-tetrahydrospiro[indene-1,7'-pyrano[4,3-*d*]pyrimidine] (260 mg, 1 *equiv.,* 521 µmol) in MeOH (4 mL) and water (2 mL) was treated with Oxone (640 mg, 2 *equiv.,* 1.04 mmol). The reaction mixture was stirred for 2 hours then quenched with aqueous sodium bicarbonate, diluted with EtOAc, and extracted with EtOAc (2 x 15 mL). The organic layers were dried over sodium sulfate, filtered, and concentrated to give 6-bromo-4'-(7,8-dihydro-4*H-*[1,2,3]triazolo[1,5-*a*][1,4]diazepin-5(6*H*)-yl)-2'-(methylsulfonyl)-2,3,5',8'-tetrahydrospiro[indene-1,7'-pyrano[4,3-*d*]pyrimidine] (277 mg), which was used in the next step without further purification.
LCMS: ESI [M+H]⁺ 533.4; t_{R} = 1.77 minutes. (Method C)

### Step 3: 6-bromo-4'-(7,8-dihydro-4H-[1,2,3]triazolo[1,5-a][1,4]diazepin-5(6H)-yl)-2'-(((2R,7aS)-2-fluorotetrahydro-1H-pyrrolizin-7a(5H)-yl)methoxy)-2,3,5',8'-tetrahydrospiro[indene-1,7'-pyrano[4,3-d]pyrimidine]

NaH (41.7 mg, 2 *equiv.,* 1.04 mmol) was suspended in THF (3 mL) followed by the dropwise addition of ((2*R*,7a*S*)-2-fluorotetrahydro-1*H*-pyrrolizin-7a(5*H*)-yl)methanol (64 mg, *3 equiv.,* 0.40 mmol) in THF (3 mL). 6-Bromo-4'-(7,8-dihydro-4*H*-[1,2,3]triazolo[1,5-*a*][1,4]diazepin-5(6*H*)-yl)-2'-(methylsulfonyl)-2,3,5',8'-tetrahydrospiro[indene-1,7'-pyrano[4,3-*d*]pyrimidine] (277 mg, *1 equiv.,* 512 µmol) in DMF (6 mL) was treated by the dropwise addition of the prepared alkoxide solution, and the mixture was stirred for 90 minutes at 50 °C. The reaction was quenched by the addition of aqueous ammonium chloride, diluted with EtOAc, and extracted with EtOAc (3 x 1 mL). The organic layers were dried with sodium sulfate, filtered, and concentrated to a crude product, which was purified by column chromatography ((SiO₂, DCM/methanol = 100/0 to 10/2, with ammonia modifier) to give 6-bromo-4'-(7,8-dihydro-4*H*-[1,2,3]triazolo[1,5-*a*][1,4]diazepin-5(6*H*)-yl)-2'-(((2*R*,7a*S*)-2-fluorotetrahydro-1*H*-pyrrolizin-7a(5*H*)-yl)methoxy)-2,3,5',8'-tetrahydrospiro[indene-1,7'-pyrano[4,3-*d*]pyrimidine] (230 mg).
LCMS: ESI [M+H]⁺ 612.3; t_{R} = 1.40 minutes. (Method C)
¹H NMR (400 MHz, CDCl₃) δ 7.57 (s, 1H), 7.44 (d, 1H), 7.18-7.23 (m, 2H), 5.26 (d, 1H), 4.61-4.79 (m, 4H), 4.51-4.60 (m, 1H), 4.43-4.52 (m, 1H), 4.03-4.09 (m, 1H), 3.83-3.99 (m, 2H), 3.74-3.83 (m, 1H), 3.19-3.29 (m, 2H), 3.06-3.18 (m, 3H), 2.92-3.03 (m, 2H), 2.77-2.86 (m, 1H), 2.28-2.39 (m, 1H), 2.06-2.29 (m, 6H), 1.81-1.99 (m, 3H).

### Step 4: 4'-(7,8-dihydro-4H-[1,2,3]triazolo[1,5-a][1,4]diazepin-5(6H)-yl)-2'-(((2R,7aS)-2-fluorotetrahydro-1H-pyrrolizin-7a(5H)-yl)methoxy)-6-(4,4,5,5-tetramethyl-1,3,2-dioxaborolan-2-yl)-2,3,5',8'-tetrahydrospiro[indene-1,7'-pyrano[4,3-d]pyrimidine]

6-Bromo-4'-(7,8-dihydro-4*H*-[1,2,3]triazolo[1,5-*a*][1,4]diazepin-5(6*H*)-yl)-2'-(((2*R*,7a*S*)-2-fluorotetrahydro-1*H*-pyrrolizin-7a(5*H*)-yl)methoxy)-2,3,5',8'-tetrahydrospiro[indene-1,7'-pyrano[4,3-d]pyrimidine] (130 mg, 1 *equiv.,* 213 µmol), potassium acetate (31.3 mg, 1.5 *equiv.,* 319 µmol), and bis(pinacolato)diboron (97.3 mg, 1.8 *equiv.,* 383 µmol) in 1,4-dioxane (2 mL) was degassed with nitrogen, followed by the addition of Pd(dppf)Cl₂·CH₂Cl₂ (15.6 mg, 0.1 *equiv.,* 21.3 µmol). The reaction mixture was heated to 90 °C for 45 minutes and then at 100 °C for 2 hours. The crude product was purified via column chromatography (SiO₂, DCM/methanol = 100/0 to 100/15, with ammonia modifier) to give 4'-(7,8-dihydro-4*H*-[1,2,3]triazolo[1,5-*a*][1,4]diazepin-5(6*H*)-yl)-2'-(((2*R*,7a*S*)-2-fluorotetrahydro-1*H*-pyrrolizin-7a(5*H*)-yl)methoxy)-6-(4,4,5,5-tetramethyl-1,3,2-dioxaborolan-2-yl)-2,3,5',8'-tetrahydrospiro[indene-1,7'-pyrano[4,3-*d*]pyrimidine] (140 mg).
LCMS: ESI [M+H]⁺ 658.8; t_{R} = 1.50 minutes. (Method C)

### Step 5: 4'-(7,8-dihydro-4H-[1,2,3]triazolo[1,5-a][1,4]diazepin-5(6H)-yl)-2'-(((2R,7aS)-2-fluorotetrahydro-1H-pyrrolizin-7a(5H)-yl)methoxy)-2,3,5',8'-tetrahydrospiro[indene-1,7'-pyrano[4,3-d]pyrimidin]-6-ol

4'-(7,8-dihydro-4*H*-[1,2,3]triazolo[1,5-*a*][1,4]diazepin-5(6*H*)-yl)-2'-(((2*R*,7a*S*)-2-fluorotetrahydro-1*H*-pyrrolizin-7a(5*H*)-yl)methoxy)-6-(4,4,5,5-tetramethyl-1,3,2-dioxaborolan-2-yl)-2,3,5',8'-tetrahydrospiro[indene-1,7'-pyrano[4,3-d*]*pyrimidine] (140 mg, 1 *equiv.,* 213 µmol) in MeOH (2 mL) and 1,4-dioxane (1 mL) was treated with aqueous sodium hydroxide (25.6 mg, 3 *equiv.,* 639 µmol, 1 M) and hydrogen peroxide (41.4 mg, 35 wt%, 2 *equiv.,* 426 µmol). The mixture was stirred at 0 °C for 30 minutes. The reaction was quenched with aqueous sodium bicarbonate, aqueous sodium thiosulfate, diluted with EtOAc, and extracted with EtOAc (3 x 10 mL). The crude product was purified via reverse-phase chromatography (ACCQPrep, water/MeCN = 100/0 to 100/60, with 0.1% formic acid modifier) to give 4'-(7,8-dihydro-4*H*-[1,2,3]triazolo[1,5-*a*][1,4]diazepin-5(6*H*)-yl)-2'-(((2*R*,7a*S*)-2-fluorotetrahydro-1*H*-pyrrolizin-7a(5*H*)-yl)methoxy)-2,3,5',8'-tetrahydrospiro[indene-1,7'-pyrano[4,3-*d*]pyrimidin]-6-ol (16 mg).
LCMS: ESI [M+H]⁺ 548.5; t_{R} = 1.25 minutes. (Method C)
¹H NMR (400 MHz, CDCl₃) δ 7.54 (d, 1H), 7.15 (dd, 1H), 6.88-6.84 (m, 1H), 6.52 (dd, 1H), 5.29 (d, 1H), 4.65-4.76 (m, 2H), 4.50-4.61 (m, 4H), 4.00-4.17 (m, 3H), 3.58-3.69 (m, 1H), 3.31-3.43 (m, 2H), 3.19-3.30 (m, 1H), 2.91- 3.18 (m, 4H), 2.71-2.79 (m, 1H), 1.90-2.37 (m, 10H).

### Example 6: 2'-(((2R,7aS)-2-fluorotetrahydro-1H-pyrrolizin-7a(5H)-yl)methoxy)-4'-((R)-3-hydroxy-3-methylpiperidin-1-yl)-3,4,5',8'-tetrahydro-2H-spiro[naphthalene-1,7'-pyrano[4,3-d]pyrimidin]-7-ol (Compound R105a)

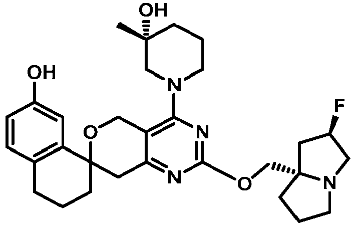

A solution of potassium hydroxide (15.4 mg, 85 wt%, 3 *equiv.,* 0.233 mmol) in a 4:1 mixture of 1,4-dioxane and water (1.5 mL) was degassed with nitrogen. (3*R*)-1-(7-Bromo-2'-(((2*R*,7a*S*)-2-fluorotetrahydro-1*H*-pyrrolizin-7a(5*H*)-yl)methoxy)-3,4,5',8'-tetrahydro-2*H-*spiro[naphthalene-1,7'-pyrano[4,3-*d*]pyrimidin]-4'-yl)-3-methylpiperidin-3-ol (**Intermediate 12,** 46.8 mg, *1 equiv.,* 0.078 mmol) was added along with Pd₂(dba)₃ (10.7 mg, 0.15 *equiv.,* 0.012 mmol) and t-BuXPhos (9.91 mg, 0.3 *equiv.,* 0.023 mmol). The reaction mixture was stirred at 95 °C for 6 hours and quenched with aqueous sodium bicarbonate, diluted with DCM (2 mL), and extracted with DCM (3 x 10 mL). Organic extracts were dried over sodium sulfate, filtered, and concentrated to a crude product, which was purified via reverse-phase chromatography (C18, water/MeCN with 0.1% formic acid = 100/0 to 0/100) to give 2'-(((2*R*,7a*S*)-2-fluorotetrahydro-1*H*-pyrrolizin-7a(5*H*)-yl)methoxy)-4'-((*R*)-3-hydroxy-3-methylpiperidin-1-yl)-3,4,5',8'-tetrahydro-2*H*-spiro[naphthalene-1,7'-pyrano[4,3-*d*]pyrimidin]-7-ol (16.2 mg).
LCMS: ESI [M+H]⁺ 539.3; t_{R} = 1.25 minutes. (Method C)
¹H NMR (400 MHz, CD₃OD) δ 6.98 (dd, 1H), 6.75 (dd, 1H), 6.72 - 6.64 (m, 1H), 5.71 - 5.45 (m, 1H), 4.87 - 4.58 (m, 2H), 4.50 (q, 3H), 4.04 - 3.64 (m, 5H), 3.57 - 3.38 (m, 2H), 3.38 - 3.30 (m, 2H), 3.30 - 3.17 (m, 2H), 3.04 (dd, 1H), 2.92 (d, 1H), 2.86 - 2.66 (m, 2H), 2.66 - 2.47 (m, 2H), 2.47 - 2.25 (m, 3H), 2.25 - 1.87 (m, 4H), 1.85 - 1.58 (m, 1H), 1.22 (d, 3H).

### Example 6a: (R)-2'-(((2R,7aS)-2-fluorotetrahydro-1H-pyrrolizin-7a(5H)-yl)methoxy)-4'-((R)-3-hydroxy-3-methylpiperidin-1-yl)-3,4,5',8'-tetrahydro-2H-spiro[naphthalene-1,7'-pyrano[4,3-d]pyrimidin]-7-ol (Compound R105c)

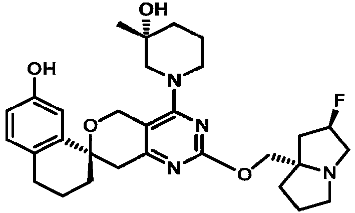

The diastereomers of 2'-(((2*R*,7a*S*)-2-fluorotetrahydro-1*H*-pyrrolizin-7a(5*H*)-yl)methoxy)-4'-((*R*)-3-hydroxy-3-methylpiperidin-1-yl)-3,4,5',8'-tetrahydro-2*H-*spiro[naphthalene-1,7'-pyrano[4,3-*d*]pyrimidin]-7-ol were separated via Chiralpak IC (20 × 250 mm, 5 µm, P/N 83345) on ACCQPrep under basic conditions (0.1% ammonium hydroxide in water, 0.1% ammonium hydroxide in MeCN), retention time 22 minutes, to provide (*R*)-2'-(((2*R*,7a*S*)-2-fluorotetrahydro-1*H*-pyrrolizin-7a(5*H*)-yl)methoxy)-4'-((R*)*-3-hydroxy-3-methylpiperidin-1-yl)-3,4,5',8'-tetrahydro-2*H*-spiro[naphthalene-1,7'-pyrano[4,3-*d*]pyrimidin]-7-ol (**Peak 1**) (4.4 mg) and (*S*)-2'-(((2*R*,7a*S*)-2-fluorotetrahydro-1*H*-pyrrolizin-7a(5*H*)-yl)methoxy)-4'-((*R*)-3-hydroxy-3-methylpiperidin-1-yl)-3,4,5',8'-tetrahydro-2*H-*spiro[naphthalene-1,7'-pyrano[4,3-*d*]pyrimidin]-7-ol (**Peak 2**) (5.2 mg).
LCMS: ESI [M+H]⁺ 539.3 m/z; t_{R} = 6.63 minutes. (Column: IC-U (3 × 100 mm, 1.6 µm, P/N 83U83; mobile phase A: 0.1% NH₄OH in MeCN; mobile phase B: 0.1% NH₄OH in water; 5% to 95% over 10 minutes)

### Example 6b: (S)-2'-(((2R,7aS)-2-fluorotetrahydro-1H-pyrrolizin-7a(5H)-yl)methoxy)-4'-((R)-3-hydroxy-3-methylpiperidin-1-yl)-3,4,5',8'-tetrahydro-2H-spiro[naphthalene-1,7'-pyrano[4,3-d]pyrimidin]-7-ol (Compound R105b)

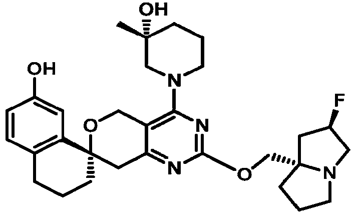

(*S*)-2'-(((2*R*,7a*S*)-2-fluorotetrahydro-1*H*-pyrrolizin-7a(5*H*)-yl)methoxy)-4'-((*R*)-3-hydroxy-3-methylpiperidin-1-yl)-3,4,5',8'-tetrahydro-2*H*-spiro[naphthalene-1,7'-pyrano[4,3-*d*]pyrimidin]-7-ol (5.2 mg) was obtained from **Example 6a** as **Peak 2:** Chiralpak IC (20 × 250 mm, 5 µm, P/N 83345) on ACCQPrep under basic conditions (0.1% ammonium hydroxide in water, 0.1% ammonium hydroxide in MeCN), retention time 26 minutes.
LCMS: ESI [M+H]⁺ 539.3 m/z; t_{R} = 7.93 minutes. (Column: IC-U (3 × 100 mm, 1.6 µm, P/N 83U83; mobile phase A: 0.1% NH₄OH in MeCN; mobile phase B: 0.1% NH₄OH in water; 5% to 95% over 10 minutes)

### Example 7: 4'-(7,8-dihydro-4H-[1,2,3]triazolo[1,5-a][1,4]diazepin-5(6H)-yl)-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-7-amine (Compound 107a)

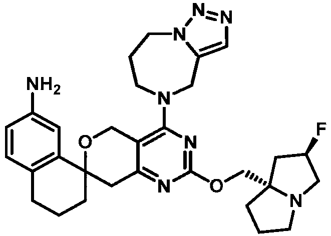

4'-(7,8-Dihydro-4H-[1,2,3]triazolo[1,5*-a*][1,4]diazepin-5(6*H*)-yl)-2'-(((2*R*,7a*S*)-2-fluorotetrahydro-1H-pyrrolizin-7a(5*H*)-yl)methoxy)-3,4,5',8'-tetrahydro-2*H-*spiro[naphthalene-1,7'-pyrano[4,3-*d*]pyrimidin]-7-amine was prepared according to **General Procedure A** with 5,6,7,8-tetrahydro-4*H*-[1,2,3]triazolo[1,5-*a*][1,4]diazepine (101 mg, 1 *equiv.,* 729 µmol) as the appropriate amine. The reaction mixture was purified via preparative HPLC (C18 column; Gradient: 5% to 60% MeCN in ammonium bicarbonate buffer) to provide 4'-(7,8-dihydro-4*H*-[1,2,3]triazolo[1,5-*a*][1,4]diazepin-5(6*H*)-yl)-2'-(((2*R*,7a*S*)-2-fluorotetrahydro-1*H*-pyrrolizin-7a(5*H*)-yl)methoxy)-3,4,5',8'-tetrahydro-2*H-*spiro[naphthalene-1,7'-pyrano[4,3-*d*]pyrimidin]-7-amine (47 mg).
LCMS: ESI [M+H]⁺ 561.4 m/z, t_{R} = 0.69 minutes. (Method A)
¹H NMR (400 MHz, DMSO-d₆): δ 7.58 (1H, s), 6.77 (1H, d), 6.54 (1H, d), 6.45 (1H, dd), 5.26 (1H, d), 4.71-4.83 (5H, m), 4.65 (2H, m), 4.48 (1H, d), 3.91 (1H, dd), 3.83 (3H, m), 3.00-3.08 (4H, m), 2.73-2.88 (3H, m), 2.60 (1H, m), 1.65-2.14 (12H, m).
¹⁹F NMR (DMSO, 376 MHz): δ -172.0.

### Example 8: 4'-(7,8-dihydro-4H-[1,2,3]triazolo[1,5-a][1,4]diazepin-5(6H)-yl)-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-7-ol (Compound 108a)

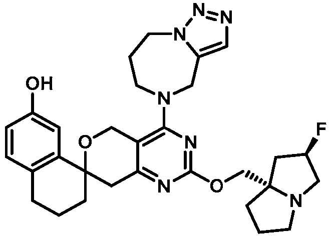

### Step 1: 7-bromo-4'-(7,8-dihydro-4H-[1,2,3]triazolo[1,5-a][1,4]diazepin-5(6H)-yl)-2'-(methylthio)-3,4,5',8'-tetrahydro-2H-spiro[naphthalene-1,7'-pyrano[4,3-d]pyrimidine]

7-Bromo-4'-chloro-2'-(methylthio)-3,4,5',8'-tetrahydro-2*H*-spiro[naphthalene-1,7'-pyrano[4,3-*d*]pyrimidine] (300 mg, *1 equiv.,* 729 µmol) and 5,6,7,8-tetrahydro-4H-[1,2,3]triazolo[1,5-*a*][1,4]diazepine (101 mg, *1 equiv.,* 729 µmol) in EtOH (3.64 mL) was treated with DIPEA (753 mg, 8 *equiv.,* 5.83 mmol). The reaction mixture was stirred at 80 °C for 1 hour. The reaction was quenched with aqueous ammonium chloride and extracted with EtOAc (3 x 15 mL). The organic layers were dried with sodium sulfate, filtered, and concentrated to give 7-bromo-4'-(7,8-dihydro-4*H*-[1,2,3]triazolo[1,5-*a*][1,4]diazepin-5(6*H*)-yl)-2'-(methylthio)-3,4,5',8'-tetrahydro-2*H*-spiro[naphthalene-1,7'-pyrano[4,3-*d*]pyrimidine] (256 mg), which was used without further purification.

LCMS: ESI [M+H]⁺ 515.0 m/z; t_{R} = 1.95 minutes (Method C).

### Step 2: 7-bromo-4'-(7,8-dihydro-4H-[1,2,3]triazolo[1,5-a][1,4]diazepin-5(6H)-yl)-2'-(methylsulfonyl)-3,4,5',8'-tetrahydro-2H-spiro[naphthalene-1,7'-pyrano[4,3-d]pyrimidine]

7-Bromo-4'-(7,8-dihydro-4*H*-[1,2,3]triazolo[1,5-*a*][1,4]diazepin-5(6*H*)-yl)-2'-(methylthio)-3,4,5',8'-tetrahydro-2*H*-spiro[naphthalene-1,7'-pyrano[4,3-*d*]pyrimidine] (250 mg, *1 equiv.,* 0.49 mmol) in MeOH (4 mL) and water (2 mL) was treated with Oxone (599 mg, *2 equiv.,* 0.97 mmol). The reaction mixture was stirred for 4 hours. The reaction was quenched with sodium bicarbonate, diluted with EtOAc, and extracted with EtOAc (3 x 15 mL). The organic layers were dried with sodium sulfate, filtered, and concentrated to give 7-bromo-4'-(7,8-dihydro-4*H*-[1,2,3]triazolo[1,5-*a*][1,4]diazepin-5(6*H*)-yl)-2'-(methylsulfonyl)-3,4,5',8'-tetrahydro-2*H*-spiro[naphthalene-1,7'-pyrano[4,3-*d*]pyrimidine] (277.6 mg), which was used without further purification.

LCMS: ESI [M+H]⁺ 547.3 m/z; t_{R} = 1.87 minutes (Method C).

### Step 3: 7-bromo-4'-(7,8-dihydro-4H-[1,2,3]triazolo[1,5-a][1,4]diazepin-5(6H)-yl)-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidine]

NaH (15 mg, 3 *equiv.,* 0.38 mmol) was suspended in THF (0.35 mL) followed by the dropwise addition of ((2*R*,7a*S*)-2-fluorotetrahydro-1*H*-pyrrolizin-7a(5*H*)-yl)methanol (60 mg, 0.38 mmol) in THF (0.63 mL). 7-Bromo-4'-(7,8-dihydro-4*H*-[1,2,3]triazolo[1,5-*a*][1,4]diazepin-5(6*H*)-yl)-2'-(methylsulfonyl)-3,4,5',8'-tetrahydro-2*H*-spiro[naphthalene-1,7'-pyrano[4,3-*d*]pyrimidine] (69 mg, *1 equiv.,* 100 µmol) in DMF (0.63 mL) was treated by the dropwise addition of the prepared alkoxide solution, and the resulting mixture was stirred for 90 minutes at 50 °C. The reaction was quenched by the addition of aqueous ammonium chloride then diluted with EtOAc, and extracted with EtOAc (3 x 1 mL). The organic layers were dried with sodium sulfate, filtered, and concentrated to a crude product, which was purified by column chromatography (SiO₂, DCM/MeOH = 100/0 to 10/2) to give 7-bromo-4'-(7,8-dihydro-4*H*-[1,2,3]triazolo[1,5-*a*][1,4]diazepin-5(6*H*)-yl)-2'-(((2*R*,7a*S*)-2-fluorotetrahydro-1*H*-pyrrolizin-7a(5*H*)-yl)methoxy)-3,4,5',8'-tetrahydro-2*H*-spiro[naphthalene-1,7'-pyrano[4,3-d]pyrimidine] (42 mg).
LCMS: ESI [M+H]⁺ 626.0 m/z; t_{R} = 1.46 minutes. (Method C)

### Step 4: 4'-(7,8-Dihydro-4H-[1,2,3]triazolo[1,5-a][1,4]diazepin-5(6H)-yl)-2'-(((2R,7aS)-2-fluorotetrahydro-1H-pyrrolizin-7a(5H)-yl)methoxy)-7-(4,4,5,5-tetramethyl-1,3,2-dioxaborolan-2-yl)-3,4,5',8'-tetrahydro-2H-spiro[naphthalene-1,7'-pyrano[4,3-d]pyrimidine]

7-Bromo-4'-(7,8-dihydro-4*H*-[1,2,3]triazolo[1,5-*a*][1,4]diazepin-5(6*H*)-yl)-2'-(((2*R*,7a*S*)-2-fluorotetrahydro-1*H*-pyrrolizin-7a(5*H*)-yl)methoxy)-3,4,5',8'-tetrahydro-2*H-*spiro[naphthalene-1,7'-pyrano[4,3-d]pyrimidine] (77 mg, *1 equiv.,* 120 µmol), potassium acetate (18 mg, 1.5 *equiv.,* 180 µmol), and bis(pinacolato)diboron (56 mg, 1.8 *equiv.*, 220 µmol) in 1,4-dioxane (1.2 mL) was degassed with nitrogen, followed by the addition of Pd(dppf)Cl₂·CH₂Cl₂ (9.0 mg, 0.1 *equiv.,* 12 µmol). The mixture was heated to 95 °C overnight and filtered through a silica plug to provide crude 4'-(7,8-dihydro-4*H*-[1,2,3]triazolo[1,5-*a*][1,4]diazepin-5(6*H*)-yl)-2'-(((2*R*,7a*S*)-2-fluorotetrahydro-1*H*-pyrrolizin-7a(5*H*)-yl)methoxy)-7-(4,4,5,5-tetramethyl-1,3,2-dioxaborolan-2-yl)-3,4,5',8'-tetrahydro-2*H-*spiro[naphthalene-1,7'-pyrano[4,3-*d*]pyrimidine] (150 mg).

LCMS: ESI [M+H]⁺ 673.0 m/z; t_{R} = 1.49 minutes (Method C).

### Step 5: 4'-(7,8-Dihydro-4H-[1,2,3]triazolo[1,5-a][1,4]diazepin-5(6H)-yl)-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-7-ol

4'-(7,8-Dihydro-4*H*-[1,2,3]triazolo[1,5-*a*][1,4]diazepin-5(6*H*)-yl)-2'-(((2*R*,7a*S*)-2-fluorotetrahydro-1*H*-pyrrolizin-7a(5*H*)-yl)methoxy)-7-(4,4,5,5-tetramethyl-1,3,2-dioxaborolan-2-yl)-3,4,5',8'-tetrahydro-2*H*-spiro[naphthalene-1,7'-pyrano[4,3-*d*]pyrimidine] (150 mg, 20 wt%, 1 *equiv.*, 0.045 mmol) in MeOH (425 µL) and 1,4-dioxane (213 µL) was cooled to 0 °C and treated with hydrogen peroxide (6.08 mg, 50 wt%, 2 *equiv.,* 0.089 mmol). The reaction mixture was stirred for 30 minutes. The reaction was quenched with aqueous sodium bicarbonate and sodium thiosulfate. The mixture was then diluted with EtOAc, and extracted with EtOAc (3 x 10 mL). The organic layers were dried and purified via ACCQPrep HPLC (0.1% formic acid in water, 0.1% formic acid in MeCN, 4 mL/min; Gradient: 25% to 35% B over 27 minutes then 100% B to 32 minutes), to provide 4'-(7,8-dihydro-4H-[1,2,3]triazolo[1,5-*a*][1,4]diazepin-5(6*H*)-yl)-2'-(((2*R*,7a*S*)-2-fluorotetrahydro-1*H*-pyrrolizin-7a(5*H*)-yl)methoxy)-3,4,5',8'-tetrahydro-2*H*-spiro[naphthalene-1,7'-pyrano[4,3-*d*]pyrimidin]-7-ol (4.5 mg).

LCMS: ESI [M+H]⁺ 563.3 m/z; t_{R} = 1.27 minutes (Method C).

¹H NMR (400 MHz, MeOD) δ 8.48 (s, 1H), 7.65 (s, 1H), 6.96-7.00 (m, 1H), 6.66-6.70 (m, 2H), 5.42 (d, 1H), 4.79-4.82 (m, 1H), 4.72 (m, 2H), 4.52-4.56 (m, 1H), 4.18-4.29 (m, 2H), 3.86-4.03 (m, 2H), 3.49-3.63 (m, 3H), 3.17-3.26 (m, 1H), 2.88-3.05 (m, 2H), 2.67-2.84 (m, 2H), 2.32-2.50 (m, 2H), 2.22-2.27 (m, 1H), 2.08-2.20 (m, 5H), 1.93-2.06 (m, 4H), 1.74 (m, 1H).

### Example 9: (3R)-1-(7-amino-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)-3-methylpiperidin-3-ol (Compound R113a)

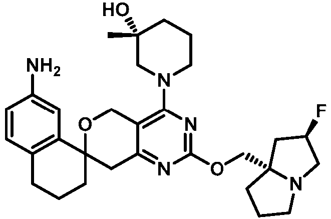

### Step 1: tert-butyl (4'-((R)-3-hydroxy-3-methylpiperidin-1-yl)-2'-(methylthio)-3,4,5',8'-tetrahydro-2H-spiro[naphthalene-1,7'-pyrano[4,3-d]pyrimidin]-7-yl)carbamate

(3*R*)-1-(7-bromo-2'-(methylthio)-3,4,5',8'-tetrahydro-2*H*-spiro[naphthalene-1,7'-pyrano[4,3-*d*]pyrimidin]-4'-yl)-3-methylpiperidin-3-ol (251 mg, *1 equiv.,* 0.51 mmol), *tert-*butyl carbamate (150 mg, 2.5 *equiv.,* 1.3 mmol), Pd(OAc)₂ (12 mg, 0.1 *equiv.,* 0.051 mmol), XantPhos (44 mg, 0.15 *equiv.,* 0.076 mmol), and cesium carbonate (500 mg, 3 *equiv.*, 1.54 mmol) were purged with nitrogen gas in a vial followed by the addition of 1,4-dioxane (2.1 mL). The sealed flask was heated to 100 °C for 1 hour. The reaction was quenched with aqueous sodium bicarbonate and extracted with EtOAc (3 x 20 mL). The organic layers were dried over sodium sulfate, filtered, and concentrated to a crude product, which was purified by column chromatography (SiO₂, heptanes/EtOAc = 100/0 to 0/100, 5% DCM modifier) to give *tert-*butyl (4'-((*R*)-3-hydroxy-3-methylpiperidin-1-yl)-2'-(methylthio)-3,4,5',8'-tetrahydro-2*H-*spiro[naphthalene-1,7'-pyrano[4,3-d]pyrimidin]-7-yl)carbamate (138 mg).

LCMS: ESI [M+H]⁺ 527.8 m/z; t_{R} = 2.65 minutes (Method E).

### Step 2: tert-butyl (2'-(((2R,7aS)-2-fluorotetrahydro-1H-pyrrolizin-7a(5H)-yl)methoxy)-4'-((R)-3-hydroxy-3-methylpiperidin-1-yl)-3,4,5',8'-tetrahydro-2H-spiro[naphthalene-1,7'-pyrano[4,3-d]pyrimidin]-7-yl)carbamate

*tert*-Butyl (4'-((*R*)-3-hydroxy-3-methylpiperidin-1-yl)-2'-(methylthio)-3,4,5',8'-tetrahydro-2*H*-spiro[naphthalene-1,7'-pyrano[4,3-*d*]pyrimidin]-7-yl)carbamate (138 mg, 90 wt%, 1 *equiv.,* 0.24 mmol) in MeOH (1.57 mL) and water (786 µL) was treated with Oxone (290 mg, 2 *equiv.,* 0.47 mmol). The mixture was stirred at room temperature for 2 hours. The reaction was quenched with aqueous ammonium chloride, diluted with EtOAc (15 mL), and extracted with EtOAc (3 x 15 mL). The organic phases were dried with sodium sulfate, filtered, and concentrated to give *tert*-butyl (4'-((*R*)-3-hydroxy-3-methylpiperidin-1-yl)-2'-(methylsulfonyl)-3,4,5',8'-tetrahydro-2*H*-spiro[naphthalene-1,7'-pyrano[4,3-*d*]pyrimidin]-7-yl)carbamate (130 mg), which was used without further purification.

NaH (28 mg, 60 wt%, 3 *equiv.,* 0.70 mmol) was suspended in THF (1.2 mL) followed by the dropwise addition of ((2*R*,7a*S*)-2-fluorotetrahydro-1*H*-pyrrolizin-7a(5*H*)-yl)methanol (111 mg, 3 *equiv.,* 0.70 mmol). *tert*-Butyl (4'-((*R*)-3-hydroxy-3-methylpiperidin-1-yl)-2'-(methylsulfonyl)-3,4,5',8'-tetrahydro-2*H*-spiro[naphthalene-1,7'-pyrano[4,3-*d*]pyrimidin]-7-yl)carbamate (130 mg, 1 *equiv.,* 0.23 mmol) in THF (1.2 mL) was treated by the dropwise addition of the prepared alkoxide solution, and the resulting mixture was stirred for 90 minutes at 50 °C. The reaction was quenched by the addition of aqueous ammonium chloride then diluted with EtOAc, and extracted with EtOAc (3 x 1 mL). The organic layers were dried with sodium sulfate, filtered, and concentrated to a crude product, which was purified by reverse-phase chromatography (ACCQPrep, XSelect CSH C18 OBD Prep Column, 130 Å, 5 µm, 19 mm × 250 mm, P/N: 186005492 with acidic mobile phases (Mobile phase A = 0.1% v/v ammonium hydroxide in water, Mobile phase B = 0.1% v/v ammonium hydroxide in acetonitrile)) to give *tert*-butyl (2'-(((2*R*,7a*S*)-2-fluorotetrahydro-1*H*-pyrrolizin-7a(5*H*)-yl)methoxy)-4'-((*R*)-3-hydroxy-3-methylpiperidin-1-yl)-3,4,5',8'-tetrahydro-2*H-*spiro[naphthalene-1,7'-pyrano[4,3-*d*]pyrimidin]-7-yl)carbamate (26.3 mg).

LCMS: ESI [M+H]⁺ 638.7 m/z; t_{R} = 1.42 minutes (Method C).

### Step 3: (3R)-1-(7-amino-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)-3-methylpiperidin-3-ol

*tert*-Butyl (2'-(((2*R*,7a*S*)-2-fluorotetrahydro-1*H*-pyrrolizin-7a(5*H*)-yl)methoxy)-4'-((*R*)-3-hydroxy-3-methylpiperidin-1-yl)-3,4,5',8'-tetrahydro-2*H*-spiro[naphthalene-1,7'-pyrano[4,3-*d*]pyrimidin]-7-yl)carbamate (33.2 mg, 1 *equiv.,* 52.1 µmol) in 1,4-dioxane (521 µL) was treated with HCl (19 mg, 4 N, 10 *equiv.,* 521 µmol). The reaction mixture was stirred overnight. The reaction mixture was concentrated under reduced pressure to obtain (3*R*)-1-(7-amino-2'-(((2*R*,7a*S*)-2-fluorotetrahydro-1*H*-pyrrolizin-7a(5*H*)-yl)methoxy)-3,4,5',8'-tetrahydro-2*H*-spiro[naphthalene-1,7'-pyrano[4,3-*d*]pyrimidin]-4'-yl)-3-methylpiperidin-3-ol as a crude product (30 mg).

LCMS: ESI [M+H]⁺ 538.5 m/z; t_{R} = 1.07 minutes (Method C).

¹H NMR (400 MHz, MeOD) δ 7.49 - 7.40 (d, 1H), 7.29 - 7.13 (m, 2H), 5.56 (d, 0.5H), 5.44 (d, 0.5H), 4.96 (d, 1H), 4.77 - 4.44 (m, 3H), 3.97 - 3.70 (m, 3H), 3.68 - 3.44 (m, 2H), 3.33 (dd, 2H), 3.15 (d, 1H), 3.02 (s, 2H), 2.80 (d, 2H), 2.73 - 2.31 (m, 4H), 2.29 - 2.07 (m, 3H), 2.07 - 1.82 (m, 2H), 1.82 - 1.58 (m, 4H), 1.26 - 1.10 (m, 4H).

### Example 9a: (R)-1-((R)-7-amino-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)-3-methylpiperidin-3-ol (Compound R113b)

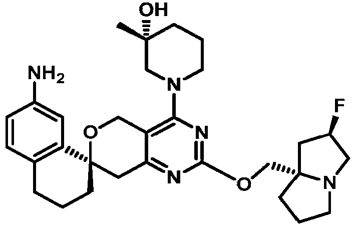

The diastereomers of (3*R*)-1-(7-amino-2'-(((2*R*,7a*S*)-2-fluorotetrahydro-1*H*-pyrrolizin-7a(5*H*)-yl)methoxy)-3,4,5',8'-tetrahydro-2*H*-spiro[naphthalene-1,7'-pyrano[4,3-*d*]pyrimidin]-4'-yl)-3-methylpiperidin-3-ol were separated under basic conditions (ACCQPrep, Mobile Phase A = 0.1% v/v ammonium hydroxide in water, 0.1% v/v ammonium hydroxide in acetonitrile) on Chiralcel OD (21 × 250 mm, 5 µm, P/N 14745)) to give product (*R*)-1-((*R*)-7-amino-2'-(((2*R*,7a*S*)-2-fluorotetrahydro-1*H*-pyrrolizin-7a(5*H*)-yl)methoxy)-3,4,5',8'-tetrahydro-2*H*-spiro[naphthalene-1,7'-pyrano[4,3-*d*]pyrimidin]-4'-yl)-3-methylpiperidin-3-ol **(Peak 1)** (9.4 mg) and (*R*)-1-((*S*)-7-amino-2'-(((2*R*,7a*S*)-2-fluorotetrahydro-1*H*-pyrrolizin-7a(5*H*)-yl)methoxy)-3,4,5',8'-tetrahydro-2*H*-spiro[naphthalene-1,7'-pyrano[4,3-*d*]pyrimidin]-4'-yl)-3-methylpiperidin-3-ol **(Peak 2)** (6.9 mg).
LCMS: ESI [M+H]⁺= 538.3 m/z; t_{R} = 2.26 minutes (Method J)
¹H NMR (400 MHz, CDCl₃) δ 6.92 (d, 1H), 6.71 (d, 1H), 6.58 (m, 1H), 5.25 (d, 1H), 4.66 (d, 1H), 4.48 (d, 1H), 4.11 (d, 1H), 3.98 (d, 1H), 3.78 (d, 1H), 3.60 (d, 1H), 3.30 - 3.09 (m, 4H), 3.07 - 2.88 (m, 4H), 2.83 - 2.66 (m, 2H), 2.21 - 2.09 (m, 3H), 2.07 - 1.72 (m, 10H), 1.62 - 1.46 (m, 2H). 1.26 (s, 3H).

### Example 9b: (R)-1-((S)-7-amino-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)-3-methylpiperidin-3-ol (Compound R113c)

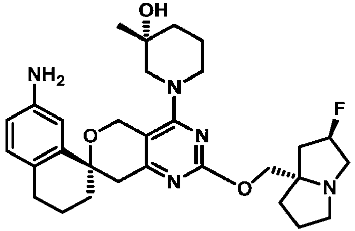

(*R*)-1-((*S*)-7-amino-2'-(((2*R*,7a*S*)-2-fluorotetrahydro-1*H*-pyrrolizin-7a(5*H*)-yl)methoxy)-3,4,5',8'-tetrahydro-2H-spiro[naphthalene-1,7'-pyrano[4,3-d]pyrimidin]-4'-yl)-3-methylpiperidin-3-ol (9.4 mg) was obtained from **Example 9a,** as **Peak 2:** (ACCQPrep, Mobile Phase A = 0.1% v/v ammonium hydroxide in water, 0.1% v/v ammonium hydroxide in acetonitrile) on Chiralcel OD (21 × 250 mm, 5 µm, P/N 14745)).
LCMS: ESI [M+H]⁺= 538.3 m/z; t_{R} = 1.82 minutes (Method J)
¹H NMR (400 MHz, CDCl₃) δ 6.92 (d, 1H), 6.71 (m, 1H), 6.59 (m, 1H), 5.25 (d, 1H), 4.66 (d, 1H), 4.48 (d, 1H), 4.11 (d, 1H), 3.98 (d, 1H), 3.78 (d, 1H), 3.60 (d, 1H), 3.19-3.30 (m, 2H), 3.01-3.18 (m, 2H), 2.89-3.01 (m, 4H), 2.64-2.82 (m, 2H), 2.09-2.27 (m, 3H), 1.65-2.08 (m, 10H), 1.46-1.62 (m, 2H), 1.23 (s, 3H).

### Example 10: 5-(7-amino-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d)pyrimidin]-4'-yl)-N,N-dimethyl-5,6,7,8-tetrahydro-4H-pyrazolo[1,5-a][1,4]diazepine-2-carboxamide (Compound R114a)

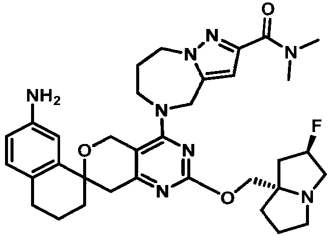

### Step 1: 5-(7-bromo-2'-(methylthio)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide

7-Bromo-4'-chloro-2'-(methylthio)-3,4,5',8'-tetrahydro-2*H*-spiro[naphthalene-1,7'-pyrano[4,3-d]pyrimidine] (266 mg, 1 *equiv.,* 0.65 mmol) and*N*,*N*-dimethyl-5,6,7,8-tetrahydro-4*H*-pyrazolo[1,5-*a*][1,4]diazepine-2-carboxamide (404 mg, 3 *equiv.,* 1.94 mmol) in ethanol (3.23 mL) were treated with DIPEA (418 mg, 5 *equiv.*, 3.23 mmol). The reaction mixture was heated to 80 °C for 9 hours. The reaction was quenched with aqueous ammonium chloride, diluted with EtOAc (15 mL), and extracted with EtOAc (3 x 40 mL). The organic extracts were dried over sodium sulfate, filtered, and concentrated. The crude product obtained was purified by column chromatography (SiO₂, DCM/MeOH = 100/0 to 100/20) to give 5-(7-bromo-2'-(methylthio)-3,4,5',8'-tetrahydro-2H-spiro[naphthalene-1,7'-pyrano[4,3-d]pyrimidin]-4'-yl)-*N*,*N*-dimethyl-5,6,7,8-tetrahydro-4*H*-pyrazolo[1,5-*a*][1,4]diazepine-2-carboxamide (251 mg).
LCMS: ESI [M+H]⁺ 585.0 m/z; t_{R} = 1.88 minutes. (Method C)

### Step 2: tert-butyl (4'-(2-(dimethylcarbamoyl)-7,8-dihydro-4H-pyrazolo[1,5-a][1,4]diazepin-5(6H)-yl)-2'-(methylthio)-3,4,5',8'-tetrahydro-2H-spiro[naphthalene-1,7'-pyrano[4,3-d]pyrimidin]-7-yl)carbamate

5-(7-Bromo-2'-(methylthio)-3,4,5',8'-tetrahydro-2*H*-spiro[naphthalene-1,7'-pyrano[4,3-*d*]pyrimidin]-4'-yl)-*N*,*N*-dimethyl-5,6,7,8-tetrahydro-4*H*-pyrazolo[1,5-*a*][1,4]diazepine-2-carboxamide (250.8 mg, 1 *equiv.,* 0.43 mmol), *tert*-butyl carbamate (126 mg, 3 *equiv.,* 1.07 mmol), Pd(OAc)₂ (9.65 mg, 0.1 *equiv.,* 0.043 mmol), XantPhos (37.30 mg, *0.15 equiv.,* 0.065 mmol) and cesium carbonate (420 mg, 3 *equiv.,* 1.29 mmol) in a flask were purged with nitrogen and taken up in 1,4-dioxane (4.3 mL). The reaction mixture was heated to 100 °C and stirred for 2.5 hours. Upon standard workup, the mixture was then purified by column chromatography (SiO₂, heptanes/EtOAc = 100/0 to 0/100, 1% MeOH modifier) to give *tert*-butyl (4'-(2-(dimethylcarbamoyl)-7,8-dihydro-4*H*-pyrazolo[1,5-*a*][1,4]diazepin-5(6*H*)-yl)-2'-(methylthio)-3,4,5',8'-tetrahydro-2*H*-spiro[naphthalene-1,7'-pyrano[4,3-*d*]pyrimidin]-7-yl)carbamate (111 mg).

¹H NMR (400 MHz, CDCl₃) δ 7.28 (m, 1H), 7.07 (d, 1H), 6.60 (s, 1H), 6.44 (s, 1H), 4.71 (d, 1H), 4.48-4.61 (m, 3H), 4.41-4.48 (bs, 2H), 3.85 (s, 2H), 3.34 (s, 3H), 3.04-3.18 (m, 4H), 2.93-3.01 (m, 1H), 2.70-2.89 (m, 2H), 2.50 (s, 3H), 2.16 (s, 2H), 1.93-2.10 (m, 3H), 1.69-1.81 (m, 1H), 1.60 (s, 1H), 1.45 (s, 9H).

### Step 3: tert-butyl (4'-(2-(dimethylcarbamoyl)-7,8-dihydro-4H-pyrazolo[1,5-a][1,4]diazepin-5(6H)-yl)-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-7-yl)carbamate

*tert*-Butyl (4'-(2-(dimethylcarbamoyl)-7,8-dihydro-4*H*-pyrazolo[1,5-*a*][1,4]diazepin-5(6*H*)-yl)-2'-(methylthio)-3,4,5',8'-tetrahydro-2*H*-spiro[naphthalene-1,7'-pyrano[4,3-*d*]pyrimidin]-7-yl)carbamate (100.5 mg, *1 equiv.,* 162 µmol) in MeOH (1.44 mL) and water (721 µL) was treated with Oxone (199 mg, 2 *equiv.,* 324 µmol). The mixture was stirred at room temperature for 2 hours. The reaction was quenched with aqueous ammonium chloride, diluted with EtOAc (15 mL), and extracted with EtOAc (3 x 15 mL). The organic phases were dried with sodium sulfate, filtered, and concentrated to give *tert*-butyl (4'-(2-(dimethylcarbamoyl)-7,8-dihydro-4*H*-pyrazolo[1,5-*a*][1,4]diazepin-5(6*H*)-yl)-2'-(methylsulfonyl)-3,4,5',8'-tetrahydro-2*H*-spiro[naphthalene-1,7'-pyrano[4,3-*d*]pyrimidin]-7-yl)carbamate (125 mg), which was used without further purification.

NaH (13.8 mg, 60 wt%, 3 *equiv.,* 0.57 mmol) was suspended in THF (1.16 mL) followed by the dropwise addition of ((2*R*,7a*S*)-2-fluorotetrahydro-1*H*-pyrrolizin-7a(5*H*)-yl)methanol (91.2 mg, 3 *equiv.,* 573 µmol). *tert*-Butyl (4'-(2-(dimethylcarbamoyl)-7,8-dihydro-4*H*-pyrazolo[1,5-*a*][1,4]diazepin-5(6*H*)-yl)-2'-(methylsulfonyl)-3,4,5',8'-tetrahydro-2*H-*spiro[naphthalene-1,7'-pyrano[4,3-d]pyrimidin]-7-yl)carbamate (125 mg, *1 equiv.,* 0.19 mmol) in THF (955 µL) was treated by the dropwise addition of the prepared alkoxide solution, and the reaction mixture was stirred for 90 minutes at 50 °C. The reaction was quenched by addition of aqueous ammonium chloride, diluted with EtOAc, and extracted with EtOAc (3 x 1 mL). The organic layers were dried with sodium sulfate, filtered, and concentrated to give a crude product, which was purified by reverse-phase chromatography (XSelect CSH C18 OBD Prep Column, 130 Å, 5 µm, 19 mm × 250 mm, P/N: 186005492 with basic mobile phases (Mobile phase A = 0.1% v/v ammonium hydroxide in water, Mobile phase B = 0.1% v/v ammonium hydroxide in acetonitrile)) to give *tert*-butyl (4'-(2-(dimethylcarbamoyl)-7,8-dihydro-4*H-*pyrazolo[1,5-*a*][1,4]diazepin-5(6*H*)-yl)-2'-(((2*R*,7a*S*)-2-fluorotetrahydro-1*H*-pyrrolizin-7a(5*H*)-yl)methoxy)-3,4,5',8'-tetrahydro-2*H*-spiro[naphthalene-1,7'-pyrano[4,3-*d*]pyrimidin]-7-yl)carbamate (57 mg).

¹H NMR (400 MHz, CDCl₃) δ 7.32 - 7.20 (m, 1H), 7.06 (d, 1H), 6.59 (s, 1H), 6.45 (s, 1H), 5.26 (d, 1H), 4.70 (d, 1H), 4.64 - 4.50 (m, 3H), 4.47 - 4.40 (m, 2H), 4.07 (d, 1H), 3.96 (d, 1H), 3.86 - 3.80 (m, 2H), 3.33 (s, 3H), 3.27 - 3.20 (m, 2H), 3.18 - 3.05 (m, 5H), 3.01 - 2.91 (m, 2H), 2.90 - 2.78 (m, 1H), 2.78 - 2.68 (m, 1H), 2.32 - 2.12 (m, 5H), 2.08 - 1.65 (m, 8H), 1.45 (d, 9H).

### Step 4: 5-(7-amino-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-dlpyrimidin]-4'-yl)-N,N-dimethyl-5,6,7,8-tetrahydro-4H-pyrazolo[1,5-a][1,4]diazepine-2-carboxamide

*tert*-Butyl (4'-(2-(dimethylcarbamoyl)-7,8-dihydro-4*H*-pyrazolo[1,5-*a*][1,4]diazepin-5(6*H*)-yl)-2'-(((2*R*,7a*S*)-2-fluorotetrahydro-1*H*-pyrrolizin-7a(5*H*)-yl)methoxy)-3,4,5',8'-tetrahydro-2H-spiro[naphthalene-1,7'-pyrano[4,3-*d*]pyrimidin]-7-yl)carbamate (57 mg, 1 *equiv.,* 0.078 mmol) in chloroform (0.78 mL) was treated with 4 N HCl (43 mg, 15 *equiv.*, 1.2 mmol). The mixture was stirred for 16 hours. The mixture was concentrated under reduced pressure to obtain 5-(7-amino-2'-(((2*R*,7a*S*)-2-fluorotetrahydro-1*H*-pyrrolizin-7a(5*H*)-yl)methoxy)-3,4,5',8'-tetrahydro-2*H*-spiro[naphthalene-1,7'-pyrano[4,3-*d*]pyrimidin]-4'-yl)-*N,N*-dimethyl-5,6,7,8-tetrahydro-4*H*-pyrazolo[1,5-*a*][1,4]diazepine-2-carboxamide (47.6 mg).
LCMS: ESI [M+H]⁺= 631.6 m/z; t_{R} = 1.10 minutes (Method C)
¹H NMR (400 MHz, MeOD) δ 7.42 (dd, 1H), 7.28 (d, 1H), 7.20 (dd, 1H), 6.39 (s, 1H), 5.50 (d, 1H), 5.07 - 4.83 (m, 3H), 4.73 - 4.60 (m, 3H), 4.58 - 4.44 (m, 3H), 4.34 - 4.19 (m, 1H), 3.97 - 3.70 (m, 4H), 3.39 - 3.30 (m, 1H), 3.21 (s, 3H), 3.04 (s, 2H), 2.99 (s, 3H), 2.92 - 2.73 (m, 2H), 2.72 - 2.50 (m, 2H), 2.50 - 2.35 (m, 1H), 2.31 - 1.92 (m, 9H), 1.80 - 1.68 (m, 1H).

### Example 10a: 5-((R)-7-amino-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide (Compound R114b)

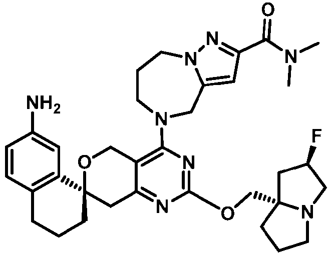

The diastereomers of 5-(7-amino-2'-(((2*R*,7a*S*)-2-fluorotetrahydro-1*H*-pyrrolizin-7a(5*H*)-yl)methoxy)-3,4,5',8'-tetrahydro-2*H*-spiro[naphthalene-1,7'-pyrano[4,3-*d*]|pyrimidin]-4'-yl)-*N*,*N*-dimethyl-5,6,7,8-tetrahydro-4*H*-pyrazolo[1,5-*a*][1,4]diazepine-2-carboxamide were separated under basic conditions (ACCQPrep, Mobile Phase A = 0.1% v/v ammonium hydroxide in water, Mobile phase B = 0.1% v/v ammonium hydroxide in acetonitrile, Chiralcel OD (21 × 250 mm, 5 µm, P/N 14745)) to give 5-((*R*)-7-amino-2'-(((2*R*,7a*S*)-2-fluorotetrahydro-1*H*-pyrrolizin-7a(5*H*)-yl)methoxy)-3,4,5',8'-tetrahydro-2*H-*spiro[naphthalene-1,7'-pyrano[4,3-*d*]pyrimidin]-4'-yl)-*N,N*-dimethyl-5,6,7,8-tetrahydro-4*H-*pyrazolo[1,5-*a*][1,4]diazepine-2-carboxamide **(Peak 1)** (5.2 mg, 86% *ee*) and 5-((*S*)-7-amino-2'-(((2*R*,7a*S*)-2-fluorotetrahydro-1*H*-pyrrolizin-7a(5*H*)-yl)methoxy)-3,4,5',8'-tetrahydro-2*H-*spiro[naphthalene-1,7'-pyrano[4,3-*d*]pyrimidin]-4'-yl)-*N*,*N*-dimethyl-5,6,7,8-tetrahydro-4*H-*pyrazolo[1,5-*a*][1,4]diazepine-2-carboxamide **(Peak 2)** (10.6 mg, 98% *ee*).
LCMS: ESI [M+H]⁺= 631.4 m/z; t_{R} = 6.04 minutes (Method J)
¹H NMR (400 MHz, MeOD) δ 6.91 (d, 1H), 6.69 (d, 1H), 6.65 (dd, 1H), 6.54 (s, 1H), 5.30 (d, 1H), 4.82 - 4.69 (m, 3H), 4.58 - 4.45 (m, 3H), 4.15 - 4.04 (m, 2H), 4.00 - 3.87 (m, 2H), 3.30 - 3.18 (m, 4H), 3.04 (d, 5H), 2.86 (d, 1H), 2.82 - 2.62 (m, 2H), 2.35 - 1.80 (m, 13H), 1.74 (dt, 1H).

### Example 10b: 5-((S)-7-amino-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide (Compound R114c)

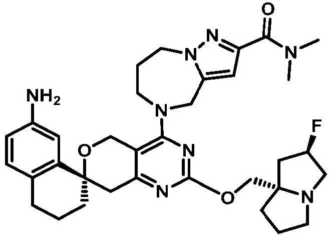

5-((*S*)-7-amino-2'-(((2*R*,7a*S*)-2-fluorotetrahydro-1*H*-pyrrolizin-7a(5*H*)-yl)methoxy)-3,4,5',8'-tetrahydro-2*H*-spiro[naphthalene-1,7'-pyrano[4,3-*d*]pyrimidin]-4'-yl)-*N*,*N*-dimethyl-5,6,7,8-tetrahydro-4*H*-pyrazolo[1,5-*a*][1,4]diazepine-2-carboxamide (10.6 mg, 98% *ee*) was obtained from **Example 10a,** as **Peak 2:** (ACCQPrep, Mobile phase A = 0.1% v/v ammonium hydroxide in water, Mobile phase B = 0.1% v/v ammonium hydroxide in acetonitrile, Chiralcel OD (21 × 250 mm, 5 µm, P/N 14745)).
LCMS: ESI [M+H]⁺= 631.4 m/z; t_{R} = 7.70 minutes (Method J)
¹H NMR (400 MHz, MeOD) δ 6.91 (d, 1H), 6.69 (d, 1H), 6.64 (dd, 1H), 6.54 (s, 1H), 5.29 (d, 1H), 4.83 - 4.70 (m, 4H), 4.62 - 4.43 (m, 3H), 4.12 (d, 1H), 4.05 (d, 1H), 3.94 (s, 2H), 3.29 - 3.15 (m, 2H), 3.04 (d, 6H), 2.86 (d, 1H), 2.81 - 2.62 (m, 2H), 2.31 - 1.83 (m, 13H), 1.74 (dt, 1H).

### Example 11: (3R)-1-(2'-Amino-2-(((2R,7aS)-2-fluorotetrahydro-1H-pyrrolizin-7a(5H)-yl)methoxy)-5,6',7',8-tetrahydro-5'H-spiro[pyrano[4,3-d]pyrimidine-7,8'-quinolin]-4-yl)-3-methylpiperidin-3-ol (Compound 110a)

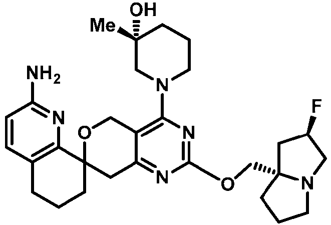

### Step 1: (3R)-3-methyl-1-(2-(methylthio)-5,6',7',8-tetrahydro-5'H-spiro[pyrano[4,3-d]pyrimidine-7,8'-quinolin]-4-yl)piperidin-3-ol

To a solution of 4-chloro-2-(methylthio)-5,6',7',8-tetrahydro-5'*H*-spiro[pyrano[4,3-d]pyrimidine-7,8'-quinoline] (260 mg, 1 *equiv.,* 0.78 mmol) in ethanol (3.1 mL) were added (*R*)-3-methylpiperidin-3-ol (179 mg, 2 *equiv.,* 1.56 mmol) and DIPEA (407 µL, 3 *equiv.,* 2.36 mmol). The mixture was stirred at 65 °C for 17 hours. The reaction mixture was cooled to room temperature and concentrated to a residue, which was diluted with DCM and water. The aqueous layer was extracted with DCM (x 4). The combined organic layers were dried over sodium sulfate, filtered, and concentrated to give (3*R*)-3-methyl-1-(2-(methylthio)-5,6',7',8-tetrahydro-5'*H*-spiro[pyrano[4,3-*d*]pyrimidine-7,8'-quinolin]-4-yl)piperidin-3-ol (280 mg) which was used without further purification.
LCMS: m/z (ESI) [M+H]⁺ 413.3, t_{R} = 1.25 minutes (Method C)

### Step 2: 4-((R)-3-Hydroxy-3-methylpiperidin-1-yl)-2-(methylsulfonyl)-5,6',7',8-tetrahydro-5'H-spiro[pyrano[4,3-d]pyrimidine-7,8'-quinoline]-1'-oxide

mCPBA (669 mg, 70 wt%, 4 *equiv.,* 2.72 mmol) was added to (3*R*)-3-methyl-1-(2-(methylthio)-5,6',7',8-tetrahydro-5'*H*-spiro[pyrano[4,3-*d*]pyrimidine-7,8'-quinolin]-4-yl)piperidin-3-ol (280 mg, 1 *equiv.,* 0.68 mmol) in DCM (3.4 mL) at 0 °C in one portion. The ice bath was removed, and the reaction mixture was stirred at room temperature for 1.5 hours. The reaction mixture was cooled to 0 °C, and additional mCPBA (669 mg, 70 wt%, 4 *equiv.,* 2.72 mmol) was added in one portion. The ice bath was removed, and the reaction mixture was stirred at room temperature for 1.5 hours. The reaction was quenched with a saturated aqueous solution of sodium thiosulfate, diluted with DCM, and then stirred for 10 minutes. The mixture was extracted with DCM. The combined organic layers were washed with saturated sodium carbonate, dried over sodium sulfate, filtered, and concentrated to a residue which was purified by flash column chromatography (RediSep Rf Gold 24 g silica gel cartridge; gradient elution from 0:100 MeOH:DCM to 15:85 MeOH:DCM) to give 4-((*R*)-3-hydroxy-3-methylpiperidin-1-yl)-2-(methylsulfonyl)-5,6',7',8-tetrahydro-5'*H*-spiro[pyrano[4,3-*d*]pyrimidine-7,8'-quinoline]-1'-oxide (208 mg).
LCMS: m/z (ESI) [M+H]⁺ 461.3, t_{R} = 1.38 minutes (Method C)

### Step 3: 2-(((2R,7aS)-2-Fluorotetrahydro-1H-pyrrolizin-7a(5H)-yl)methoxy)-4-((R)-3-hydroxy-3-methylpiperidin-1-yl)-5,6',7',8-tetrahydro-5'H-spiro[pyrano[4,3-d]pyrimidine-7,8'-quinoline]-1'-oxide

((2*R*,7a*S*)-2-Fluorotetrahydro-1*H*-pyrrolizin-7a(5*H*)-yl)methanol (62 mg, 0.9 *equiv.,* 0.39 mmol) was added to a stirred solution of 4-((*R*)-3-hydroxy-3-methylpiperidin-1-yl)-2-(methylsulfonyl)-5,6',7',8-tetrahydro-5'*H*-spiro[pyrano[4,3-*d*]pyrimidine-7,8'-quinoline]-1'-oxide (200 mg, 1 *equiv.,* 0.43 mmol) in THF (1.7 mL). The resulting mixture was cooled to-20 °C, and potassium tert-butoxide (868 µL, 1 M, 2 *equiv.*, 0.87 mmol) was added dropwise. The reaction mixture was stirred at -20 °C for 1 hour and at 0 °C for 1 hour. The reaction was quenched by saturated ammonium chloride and diluted with EtOAc. The aqueous layer was extracted with EtOAc (x 2) and with DCM (x 3). The combined organic layers were dried over sodium sulfate, filtered, and concentrated to a residue, which was purified by flash column chromatography (RediSep Rf Gold 24 g silica gel cartridge; gradient elution from 0:100-premixed solution made up of 2.5% NH₄OH in 20% MeOH/DCM:DCM to 60:40-premixed solution made up of 2.5% NH₄OH in 20% MeOH/DCM:DCM) to afford 2-(((2*R*,7a*S*)-2-fluorotetrahydro-1*H*-pyrrolizin-7a(5H)-yl)methoxy)-4-((R)-3-hydroxy-3-methylpiperidin-1-yl)-5,6',7',8-tetrahydro-5'*H*-spiro[pyrano[4,3-*d*]pyrimidine-7,8'-quinoline]-1'-oxide (160 mg).
LCMS: m/z (ESI) [M+H]⁺ 540.3, t_{R} = 1.05 minutes (Method C)

### Step 4: (3R)-1-(2'-Amino-2-(((2R,7aS)-2-fluorotetrahydro-1H-pyrrolizin-7a(5H)-yl)methoxy)-5,6',7',8-tetrahydro-5'H-spiro[pyrano[4,3-d]pyrimidine-7,8'-quinolin]-4-yl)-3-methylpiperidin-3-ol

Pyridine (72 µL, 10 *equiv.,* 0.89 mmol) was added to a stirred solution of 2-(((2*R*,7a*S*)-2-fluorotetrahydro-1*H*-pyrrolizin-7a(5*H*)-yl)methoxy)-4-((*R*)-3-hydroxy-3-methylpiperidin-1-yl)-5,6',7',8-tetrahydro-5'*H*-spiro[pyrano[4,3-*d*]pyrimidine-7,8'-quinoline]-1'-oxide (50 mg, 1 *equiv.,* 0.09 mmol) in DCM (0.8 mL). Trifluoroacetic anhydride (75 µL, 6 *equiv.*, 0.53 mmol) was added dropwise, and the resulting mixture was stirred at room temperature for 1 hour. The reaction mixture was concentrated to dryness and diluted with ethanol (0.8 mL) and ethanolamine (108 µL, 20 *equiv.,* 1.78 mmol). This mixture was stirred at room temperature for 16 hours. The reaction mixture was concentrated to remove ethanol, and the resulting residue was diluted with DCM and water. The aqueous layer was extracted with DCM (x 4). The combined organic layers were dried over sodium sulfate, filtered, and concentrated to a residue, which was purified by flash column chromatography (RediSep Rf Gold 12 g silica gel cartridge; gradient elution from 0:100-premixed solution made up of 2.5% NH₄OH in 20% MeOH/DCM:DCM to 20:80 to 40:60 premixed solution made up of 2.5% NH₄OH in 20% MeOH/DCM:DCM; desired product elutes around 30%) to give (3*R*)-1-(2'-amino-2-(((2*R*,7a*S*)-2-fluorotetrahydro-1*H*-pyrrolizin-7a(5*H*)-yl)methoxy)-5,6',7',8-tetrahydro-5'*H-*spiro[pyrano[4,3-d]pyrimidine-7,8'-quinolin]-4-yl)-3-methylpiperidin-3-ol (37 mg).
LCMS: m/z (ESI) [M+H]⁺ 539.3, t_{R} = 1.44 minutes (Method E)
¹H NMR (400 MHz, CDCl₃) δ 7.20 (d, 1H), 6.40 (d, 1H), 5.36 - 5.16 (m, 1H), 4.70 - 4.53 (m, 2H), 4.26 (d, 2H), 4.13 (dd, 1H), 4.01 (t, 1H), 3.80 - 3.58 (m, 3H), 3.49 (dd, 1H), 3.35 - 3.11 (m, 3H), 3.05 - 2.87 (m, 3H), 2.78 - 2.59 (m, 3H), 2.29 - 2.06 (m, 4H), 2.06 - 1.97 (m, 1H), 1.97 - 1.69 (m, 7H), 1.60 - 1.45 (m, 2H), 1.22 (d, 3H).

### Example 12: 5-(6-Amino-2'-(((2R,7aS)-2-fluorotetrahydro-1H-pyrrolizin-7a(5H)-yl)methoxy)-5',8'-dihydrospiro[isochromane-4,7'-pyrano[4,3-d]pyrimidin]-4'-yl)-N,N-dimethyl-5,6,7,8-tetrahydro-4H-pyrazolo[1,5-a][1,4]diazepine-2-carboxamide (Compound 118a)

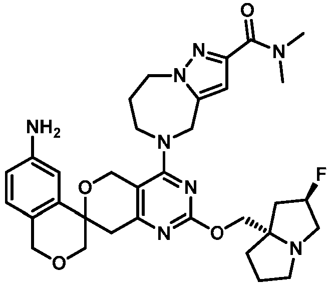

### Step 1: 5-(6-bromo-2'-(methylthio)-5',8'-dihydrospiro[isochromane-4,7'-pyrano[4,3-d]pyrimidin]-4'-yl)-N,N-dimethyl-5,6,7,8-tetrahydro-4H-pyrazolo[1,5-a][1,4]diazepine-2-carboxamide

A mixture of 6-bromo-4'-chloro-2'-(methylthio)-5',8'-dihydrospiro[isochromane-4,7'-pyrano[4,3-*d*]pyrimidine] (200 mg, *1 equiv.,* 0.48 mmol), N,N-dimethyl-5,6,7,8-tetrahydro-4*H*-pyrazolo[1,5-*a*][1,4]diazepine-2-carboxamide, HCl (296 mg, 2.5 *equiv.,* 1.21 mmol), MeCN (5.0 mL), and DIPEA (505 µL, 6.0 *equiv.,* 2.90 mmol) was stirred at 65 °C for 72 hours. The reaction temperature was then increased to 80 °C and stirred for 24 hours. The reaction mixture was diluted with EtOAc (100 mL) and washed with water (20 mL x 2). The organic layer was then filtered over sodium sulfate and concentrated under reduced pressure to give a residue, which was purified by column chromatography (10 g SiO₂ cartridge, 0-10% MeOH in dichloromethane) to afford 5-(6-bromo-2'-(methylthio)-5',8'-dihydrospiro[isochromane-4,7'-pyrano[4,3-*d*]pyrimidin]-4'-yl)-*N*,*N*-dimethyl-5,6,7,8-tetrahydro-4*H*-pyrazolo[1,5-*a*][1,4]diazepine-2-carboxamide (205 mg).

¹H NMR (400 MHz, CDCl₃) δ 7.50 (d, 1H), 7.42 (dd, 1H), 6.95 (d, 1H), 6.54 (s, 1H), 4.88 - 4.62 (m, 4H), 4.61 (s, 2H), 4.50 - 4.41 (m, 2H), 3.96 - 3.76 (m, 4H), 3.31 (s, 3H), 3.07 (s, 3H), 3.00 (d, 2H), 2.51 (s, 3H), 2.16 (dd, 2H), 1.59 (s, 2H).

### Step 2: 5-(6-Bromo-2'-(methylsulfonyl)-5',8'-dihydrospiro[isochromane-4,7'-pyrano[4,3-d]pyrimidin]-4'-yl)-N,N-dimethyl-5,6,7,8-tetrahydro-4H-pyrazolo[1,5-a][1,4]diazepine-2-carboxamide

Oxone (980 mg, 45 wt%, 2.05 *equiv.,* 0.718 mmol) was added to 5-(6-bromo-2'-(methylthio)-5',8'-dihydrospiro[isochromane-4,7'-pyrano[4,3-*d*]pyrimidin]-4'-yl)-*N*,*N-***dimethyl-5,6,7,8-tetrahydro-4*H*-pyrazolo[1,5-*a*][1,4]diazepine-2-carboxamide** (205 mg, 1.0 *equiv.,* 0.35 mmol) in MeOH (5 mL) and water (5 mL). The mixture was stirred at room temperature for 6 hours. The reaction mixture was extracted with EtOAc followed by dichloromethane. The combined organic layers were washed with water, dried over sodium sulfate, and concentrated to yield 5-(6-bromo-2'-(methylsulfonyl)-5',8'-dihydrospiro[isochromane-4,7'-pyrano[4,3-*d*]pyrimidin]-4'-yl)-*N*,*N*-dimethyl-5,6,7,8-tetrahydro-4*H*-pyrazolo[1,5-*a*][1,4]diazepine-2-carboxamide (197 mg), which was used without further purification.
LCMS: m/z (ESI) [M+H]⁺ 619.3, t_{R} = 2.46 minutes (Method E)

### Step 3: 5-(6-bromo-2'-(((2R,7aS)-2-fluorotetrahydro-1H-pyrrolizin-7a(5H)-yl)methoxy)-5',8'-dihydrospiro[isochromane-4,7'-pyrano[4,3-d]pyrimidin]-4'-yl)-N,N-dimethyl-5,6,7,8-tetrahydro-4H-pyrazolo[1,5-a][1,4]diazepine-2-carboxamide

NaH (51.0 mg, 60 wt%, 4 *equiv.,* 1.28 mmol) was added to ((2*R*,7a*S*)-2-fluorotetrahydro-1*H*-pyrrolizin-7a(5*H*)-yl)methanol (254 mg, 5 *equiv.,* 1.60 mmol) in dry THF (5 mL) and stirred for 10 minutes. 5-(6-Bromo-2'-(methylsulfonyl)-5',8'-dihydrospiro[isochromane-4,7'-pyrano[4,3-*d*]pyrimidin]-4'-yl)-*N*,*N*-dimethyl-5,6,7,8-tetrahydro-4*H*-pyrazolo[1,5-*a*][1,4]diazepine-2-carboxamide (197 mg, 1 *equiv.,* 0.319 mmol) in dry THF (5 mL) was added, and the resulting mixture was stirred for 2 hours at ambient temperature. The reaction mixture was diluted with EtOAc (100 mL) and washed with water (20 mL x 2). The organic layer was then filtered over sodium sulfate and concentrated under reduced pressure to a residue, which was purified by column chromatography (25 g SiO₂ cartridge, 0-20% MeOH in dichloromethane) to give 5-(6-bromo-2'-(((2*R*,7a*S*)-2-fluorotetrahydro-1*H*-pyrrolizin-7a(5*H*)-yl)methoxy)-5',8'-dihydrospiro[isochromane-4,7'-pyrano[4,3-*d*]pyrimidin]-4'-yl)-*N*,*N*-dimethyl-5,6,7,8-tetrahydro-4*H*-pyrazolo[1,5-*a*][1,4]diazepine-2-carboxamide (181 mg).

¹H NMR (400 MHz, CDCl₃) δ 7.49 (m, 1H), 7.43 - 7.39 (m, 1H), 6.94 (m, 1H), 6.55 (s, 1H), 5.27 (m, 1H), 4.85 - 4.75 (m, 2H), 4.72 (m, 1H), 4.67 - 4.60 (m, 3H), 4.48 - 4.43 (m, 2H), 4.06 (m, 2H), 3.93 - 3.80 (m, 4H), 3.30 - 3.17 (m, 6H), 3.07 (s, 3H), 2.98 (m, 3H), 2.29 - 2.09 (m, 5H), 1.96 (m, 3H).

¹⁹F NMR (376 MHz, CDCl₃) δ -173.00.

### Step 4: tert-Butyl (4'-(2-(dimethylcarbamoyl)-7,8-dihydro-4H-pyrazolo[1,5-a][1,4]diazepin-5(6H)-yl)-2'-(((2R,7aS)-2-fluorotetrahydro-1H-pyrrolizin-7a(5H)-yl)methoxy)-5',8'-dihydrospiro[isochromane-4,7'-pyrano[4,3-d]pyrimidin]-6-yl)carbamate

A degassed suspension of 5-(6-bromo-2'-(((2*R*,7a*S*)-2-fluorotetrahydro-1*H*-pyrrolizin-7a(5*H*)-yl)methoxy)-5',8'-dihydrospiro[isochromane-4,7'-pyrano[4,3-*d*]pyrimidin]-4'-yl)-*N*,*N-*dimethyl-5,6,7,8-tetrahydro-4*H*-pyrazolo[1,5-*a*][1,4]diazepine-2-carboxamide (30 mg, 1.0 *equiv.,* 0.043 mmol), *tert*-butyl carbamate (10 mg, 2.0 *equiv.,* 0.086 mmol), Pd(OAc)₂ (1.9 mg, *0.2 equiv.,* 0.086 mmol), cesium carbonate (42 mg, 3.0 *equiv.,* 0.130 mmol), and 2-(dicyclohexylphosphanyl)-2',4',6'-tris(isopropyl)biphenyl (8.2 mg, 0.4 *equiv.,* 0.017 mmol) in dry 1,4-dioxane (1.0 mL) was stirred at 100 °C for 16 hours. The reaction mixture was diluted with DCM (100 mL), filtered over sodium sulfate, and concentrated to a residue, which was purified by column chromatography (10 g SiO₂ cartridge, 0-20% MeOH in dichloromethane) to afford *tert*-butyl (4'-(2-(dimethylcarbamoyl)-7,8-dihydro-4*H*-pyrazolo[1,5-a][1,4]diazepin-5(6*H*)-yl)-2'-(((2*R*,7a*S*)-2-fluorotetrahydro-1*H*-pyrrolizin-7a(5*H*)-yl)methoxy)-5',8'-dihydrospiro[isochromane-4,7'-pyrano[4,3-d]pyrimidin]-6-yl)carbamate (36 mg).

¹H NMR (400 MHz, CDCl₃) δ 7.41 - 7.33 (m, 1H), 7.29 - 7.25 (m, 1H), 7.02 - 6.91 (m, 2H), 5.39 (s, 2H), 4.87 - 4.74 (m, 2H), 4.71 - 4.55 (m, 4H), 4.48 - 4.39 (m, 2H), 4.20 (m, 2H), 3.95 (m, 1H), 3.87 - 3.73 (m, 3H), 3.52 (s, 1H), 3.32 (m, 5H), 3.06 (s, 3H), 3.01 - 2.87 (m, 2H), 2.39 - 2.20 (m, 3H), 2.14 (m, 2H), 2.00 (m, 3H), 1.43 (m, 9H).

### Step 5: 5-(6-Amino-2'-(((2R,7aS)-2-fluorotetrahydro-1H-pyrrolizin-7a(5H)-yl)methoxy)-5',8'-dihydrospiro[isochromane-4,7'-pyrano[4,3-d]pyrimidin]-4'-yl)-N,N-dimethyl-5,6,7,8-tetrahydro-4H-pyrazolo[1,5-a][1,4]diazepine-2-carboxamide

HCl (150 µL, 4.0 M in 1,4-dioxane, 15 *equiv.,* 0.60 mmol) was added to *tert*-butyl (4'-(2-(dimethylcarbamoyl)-7,8-dihydro-4*H*-pyrazolo[1,5-*a*][1,4]diazepin-5(6*H*)-yl)-2'-(((2*R*,7a*S*)-2-fluorotetrahydro-1*H*-pyrrolizin-7a(5*H*)-yl)methoxy)-5',8'-dihydrospiro[isochromane-4,7'-pyrano[4,3-d]pyrimidin]-6-yl)carbamate (30 mg, 1 *equiv.*, 0.041 mmol) in CDCl₃ (1.0 mL). The reaction mixture was stirred at 25 °C for 5 hours and concentrated to give 5-(6-amino-2'-(((2*R*,7a*S*)-2-fluorotetrahydro-1*H*-pyrrolizin-7a(5*H*)-yl)methoxy)-5',8'-dihydrospiro[isochromane-4,7'-pyrano[4,3-*d*]pyrimidin]-4'-yl)-*N*,*N-*dimethyl-5,6,7,8-tetrahydro-4*H*-pyrazolo[1,5-*a*][1,4]diazepine-2-carboxamide (29 mg).
LCMS: m/z (ESI) [M+H]⁺ 633.5, t_{R} = 1.22 minutes (Method E)
¹H NMR (400 MHz, MeOD) δ 6.85 (m, 1H), 6.70 (m, 2H), 6.61 (s, 1H), 5.42 (s, 1H), 4.80 (m, 2H), 4.76 - 4.67 (m, 4H), 4.48 (m, 2H), 4.39 - 4.29 (m, 2H), 3.90 (m, 5H), 3.68 (m, 3H), 3.07 (m, 3H), 2.94 (m, 2H), 2.66 (s, 5H), 2.50 - 2.42 (m, 2H), 2.32 (m, 1H), 2.22 (m, 2H), 2.13 (m, 3H).

### Example 12a: 5-((R)-6-amino-2'-(((2R,7aS)-2-fluorotetrahydro-1H-pyrrolizin-7a(5H)-yl)methoxy)-5',8'-dihydrospiro[isochromane-4,7'-pyrano[4,3-d]pyrimidin]-4'-yl)-N,N-dimethyl-5,6,7,8-tetrahydro-4H-pyrazolo[1,5-a][1,4]diazepine-2-carboxamide (Compound 118c)

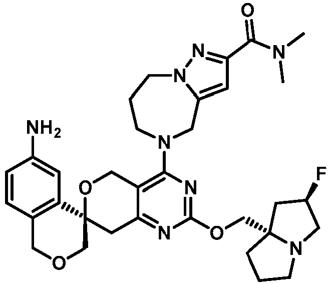

The diastereomers of 5-(6-amino-2'-(((2*R*,7a*S*)-2-fluorotetrahydro-1*H*-pyrrolizin-7a(5*H*)-yl)methoxy)-5',8'-dihydrospiro[isochromane-4,7'-pyrano[4,3-*d*]pyrimidin]-4'-yl)-*N,N-*dimethyl-5,6,7,8-tetrahydro-4*H*-pyrazolo[1,5-*a*][1,4]diazepine-2-carboxamide were separated by SFC using ChiralCel OJ-H 21 × 250 mm column. Isocratic mobile phase: 35% methanol, 0.25% triethylamine in CO₂ at a flow rate of 70 mL/min to afford 5-((*R*)-6-amino-2'-(((2*R*,7a*S*)-2-fluorotetrahydro-1*H*-pyrrolizin-7a(5*H*)-yl)methoxy)-5',8'-dihydrospiro[isochromane-4,7'-pyrano[4,3-*d*]pyrimidin]-4'-yl)-*N*,*N*-dimethyl-5,6,7,8-tetrahydro-4*H*-pyrazolo[1,5-*a*][1,4]diazepine-2-carboxamide **(Peak 1)** and 5-((*S*)-6-amino-2'-(((2*R*,7a*S*)-2-fluorotetrahydro-1*H*-pyrrolizin-7a(5*H*)-yl)methoxy)-5',8'-dihydrospiro[isochromane-4,7'-pyrano[4,3-*d*]pyrimidin]-4'-yl)-*N*,*N*-dimethyl-5,6,7,8-tetrahydro-4*H*-pyrazolo[1,5-*a*][1,4]diazepine-2-carboxamide **(Peak 2).**

LCMS: m/z (ESI) [M+H]⁺ 633.3, t_{R} = 1.25 minutes (ChiralCel OJ-H, 4.6x100 mm, Mobile phase: 35% MeOH with 0.25% DEA in CO₂; 2.5 mL/min).

¹H NMR (400 MHz, MeOD) δ 6.86 (m, 1H), 6.71 - 6.67 (m, 2H), 6.58 (s, 1H), 5.28 (m, 1H), 4.82 - 4.65 (m, 7H), 4.49 (m, 2H), 4.22 (m, 1H), 4.11 (m, 1H), 4.05 (m, 1H), 3.97 - 3.83 (m, 4H), 3.27 - 3.16 (m, 4H), 3.07 (s, 3H), 2.90 (m, 2H), 2.34 - 2.25 (m, 1H), 2.22 - 2.07 (m, 6H), 1.96 (m, 3H), 1.89 - 1.83 (m, 1H).

### Example 12b: 5-((S)-6-amino-2'-(((2R,7aS)-2-fluorotetrahydro-1H-pyrrolizin-7a(5H)-yl)methoxy)-5',8'-dihydrospiro[isochromane-4,7'-pyrano[4,3-d)pyrimidin]-4'-yl)-N,N-dimethyl-5,6,7,8-tetrahydro-4H-pyrazolo[1,5-a][1,4]diazepine-2-carboxamide (Compound 118b)

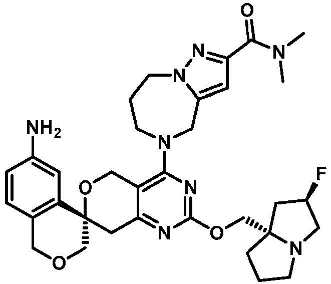

5-((*S*)-6-amino-2'-(((2*R*,7a*S*)-2-fluorotetrahydro-1*H*-pyrrolizin-7a(5*H*)-yl)methoxy)-5',8'-dihydrospiro[isochromane-4,7'-pyrano[4,3-*d*]pyrimidin]-4'-yl)-*N*,*N*-dimethyl-5,6,7,8-tetrahydro-4*H*-pyrazolo[1,5-*a*][1,4]diazepine-2-carboxamide was obtained from **Example 12a,** as **Peak 2:** by SFC using ChiralCel OJ-H 21 × 250 mm column. Isocratic mobile phase: 35% methanol, 0.25% triethylamine in CO₂ at a flow rate of 70 mL/min.

LCMS: m/z (ESI) [M+H]⁺ 633.3, t_{R} = 1.99 minutes. (ChiralCel OJ-H, 4.6x100 mm, Mobile phase: 35% MeOH with 0.25% DEA in CO₂; 2.5 mL/min).

¹H NMR (400 MHz, MeOD) δ 6.85 (m, 1H), 6.69 (m, 2H), 6.58 (s, 1H), 5.27 (m,1H), 4.82 - 4.65 (m, 7H), 4.49 (m, 2H), 4.13 - 4.02 (m, 2H), 3.88 (m, 5H), 3.26 - 3.14 (m, 3H), 3.07 (s, 3H), 3.03 - 2.87 (m, 4H), 2.28 - 2.06 (m, 6H), 2.01 - 1.83 (m, 4H).

### Example 13: 1-(7-Amino-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d)pyrimidin]-4'-yl)azepane-4-carbonitrile (Compound R149a)

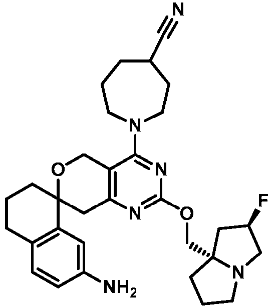

1-(7-Amino-2'-(((2*R*,7a*S*)-2-fluorotetrahydro-1*H*-pyrrolizin-7a(5*H*)-yl)methoxy)-3,4,5',8'-tetrahydro-2*H*-spiro[naphthalene-1,7'-pyrano[4,3-*d*]pyrimidin]-4'-yl)azepane-4-carbonitrile was prepared according to **General Procedure A** using the *p*-toluenesulfonic acid salt of azepane-4-carbonitrile (40 mg, 3 *equiv.*, 0.32 mmol). The material was purified by reverse-phase preparative purification with a C-18 Biotage column in basic buffer to afford 1-(7-amino-2'-(((2*R*,7a*S*)-2-fluorotetrahydro-1*H*-pyrrolizin-7a(5*H*)-yl)methoxy)-3,4,5',8'-tetrahydro-2*H*-spiro[naphthalene-1,7'-pyrano[4,3-*d*]pyrimidin]-4'-yl)azepane-4-carbonitrile (20 mg).
LCMS: m/z (ESI) [M+H]⁺ 547.3, t_{R} = 4.04 minutes (Method I)
¹H NMR (400 MHz, DMSO-*d₆*) δ 6.74 (d, 1H), 6.56 (t, 1H), 6.47 - 6.37 (m, 1H), 5.24 (d, 1H), 4.85 - 4.64 (m, 3H), 4.48 (dd, 1H), 3.99 - 3.90 (m, 1H), 3.89 - 3.78 (m, 1H), 3.68 - 3.41 (m, 4H), 3.14 - 2.93 (m, 4H), 2.90 - 2.69 (m, 3H), 2.65 - 2.51 (m, 2H), 2.14 - 2.04 (m, 2H), 2.02 - 1.67 (m, 13H), 1.62 (q, 1H).

### Example 13a: (S*)-1-((S)-7-amino-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)azepane-4-carbonitrile (Compound R149b)

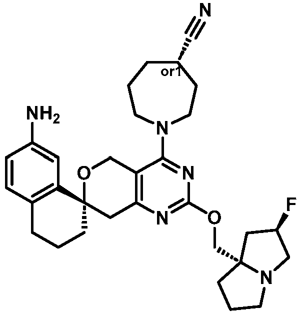

### Step 1: Benzyl rel-(S)-4-cyanoazepane-1-carboxylate

Benzyl chloroformate (0.80 mL, 2.0 *equiv.*, 7.0 mmol) was added to a solution of azepane-4-carbonitrile (0.35 g, 1 *equiv.,* 2.8 mmol) and DIPEA (1.2 mL, 2.5 *equiv.,* 7.0 mmol) in DCM (1.5 mL) at room temperature. The reaction mixture was stirred for 16 hours. Afterward, the reaction mixture was concentrated to a residue, which was purified by flash chromatography (12 g Redisep Column using 25-30% hexanes in EtOAc) to afford benzyl 4-cyanoazepane-1-carboxylate (0.13 g). The enantiomers of benzyl 4-cyanoazepane-1-carboxylate were separated by chiral SFC (Column: i-Amylose-3; 21.2×250 mm; 5 mm; Mobile Phase A: 50% MeOH at 30 mL/min for 10 minutes) to afford benzyl *rel*-(*S*)-4-cyanoazepane-1-carboxylate **(Peak 1)** and benzyl *rel-*(*R*)-4-cyanoazepane-1-carboxylate **(Peak 2).**
**Peak 1:** HPLC: t_{R} = 1.74 minutes (Column: i-Amylose-3, 4.6x250 mm; 5 mm; Mobile Phase A: 50% MeOH @ 4 mL/min for 5 minutes)

### Step 2: rel-(S)-azepane-4-carbonitrile

Pd(OH)₂ on carbon (90 mg, 20 wt%, 0.3 *equiv.,* 0.13 mmol) was added to benzyl *rel-*(*S*)-4-cyanoazepane-1-carboxylate **(Peak 1,** 110 mg, *1 equiv.,* 0.43 mmol) in THF (3 mL) at room temperature and stirred for 3 hours under hydrogen atmosphere. The reaction mixture was purged with nitrogen, filtered through a Celite bed, concentrated, and dried to afford *rel-*(S)-azepane-4-carbonitrile (45 mg).

¹H NMR (400 MHz, CDCl₃): δ 2.97-2.82 (m; 4H), 2.09-1.55 (m; 6 H).

### Step 3: (S*)-1-((S)-7-amino-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)azepane-4-carbonitrile

(*S**)-1-((*S*)-7-amino-2'-(((2*R*,7a*S*)-2-fluorotetrahydro-1*H*-pyrrolizin-7a(5*H*)-yl)methoxy)-3,4,5',8'-tetrahydro-2*H*-spiro[naphthalene-1,7'-pyrano[4,3-*d*]pyrimidin]-4'-yl)azepane-4-carbonitrile was prepared according to **General Procedure** A using *rel*-(*S*)-azepane-4-carbonitrile (40 mg, 0.32 mmol) to afford (*S**)-1-((*S*)-7-amino-2'-(((2*R*,7a*S*)-2-fluorotetrahydro-1*H*-pyrrolizin-7a(5*H*)-yl)methoxy)-3,4,5',8'-tetrahydro-2*H-*spiro[naphthalene-1,7'-pyrano[4,3-*d*]pyrimidin]-4'-yl)azepane-4-carbonitrile (15 mg).
LCMS: ESI [M+H]⁺ 547.3 m/z; t_{R} = 4.07 minutes. (Method I)
¹H NMR (400 MHz, DMSO-d₆): δ 6.74 (d, 1 H), 6.56 (d, 1 H), 6.42 (dd, 1 H), 5.24 (d, 1 H), 4.48-4.76 (m, 4 H), 3.88 (dd, 2 H), 3.40-3.66 (m, 3H), 2.70-3.04 (m, 6 H), 2.58 (s, 2 H), 1.61-2.05 (m, 16 H).

### Example 13b: (R*)-1-((S)-7-amino-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)azepane-4-carbonitrile (Compound R149c)

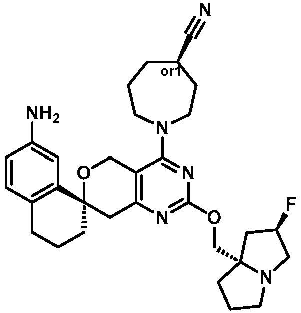

### Step 1: Benzyl rel-(R)-4-cyanoazepane-1-carboxylate

Benzyl chloroformate (0.80 mL, 2.0 *equiv.,* 7.0 mmol) was added to a solution of azepane-4-carbonitrile (0.35 g, 1 *equiv.,* 2.8 mmol) and DIPEA (1.2 mL, 2.5 *equiv.,* 7.0 mmol) in DCM (1.5 mL) at room temperature. The reaction mixture was stirred for 16 hours. Afterward, the reaction mixture was concentrated to a residue, which was purified by flash chromatography (12 g Redisep Column using 25-30% hexanes in EtOAc) to afford benzyl 4-cyanoazepane-1-carboxylate (0.13 g). The enantiomers of benzyl 4-cyanoazepane-1-carboxylate were separated by chiral SFC (Column: i-Amylose-3; 21.2×250 mm; 5 mm; Mobile Phase A: 50% MeOH at 30 mL/min for 10 minutes) to afford benzyl *rel*-(*S*)-4-cyanoazepane-1-carboxylate **(Peak 1)** and benzyl *rel*-(*R*)-4-cyanoazepane-1-carboxylate **(Peak 2).**
**Peak 2:** HPLC: t_{R} = 2.16 minutes (Column: i-Amylose-3, 4.6x250 mm; 5 mm; Mobile Phase A: 50% MeOH @ 4 mL/min for 5 minutes)

### Step 2: rel-(R)-azepane-4-carbonitrile

Pd(OH)₂ on carbon (90 mg, 20 wt%, 0.3 *equiv.,* 0.13 mmol) was added to benzyl *rel-*(R)-4-cyanoazepane-1-carboxylate **(Peak 2,** 110 mg, *1 equiv.,* 0.43 mmol) in THF (3 mL) at room temperature and stirred for 3 hours under hydrogen atmosphere. The reaction mixture was purged with nitrogen, filtered through a Celite bed, concentrated, and dried to afford *rel-*(R)-azepane-4-carbonitrile (45 mg).

¹H NMR (400 MHz, CDCl₃): δ 3.03-2.58 (m; 4H), 2.07-1.45 (m; 6 H).

### Step 3: (R*)-1-((S)-7-amino-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)azepane-4-carbonitrile

(*R**)-1-((*S*)-7-amino-2'-(((2*R*,7a*S*)-2-fluorotetrahydro-1*H*-pyrrolizin-7a(5*H*)-yl)methoxy)-3,4,5',8'-tetrahydro-2H-spiro[naphthalene-1,7'-pyrano[4,3-d]pyrimidin]-4'-yl)azepane-4-carbonitrile was prepared according to **General Procedure A** using *rel*-(*R*)-azepane-4-carbonitrile (35 mg, 0.28 mmol) to afford (*R**)-1-((*S*)-7-amino-2'-(((2*R*,7a*S*)-2-fluorotetrahydro-1*H*-pyrrolizin-7a(5*H*)-yl)methoxy)-3,4,5',8'-tetrahydro-2*H-*spiro[naphthalene-1,7'-pyrano[4,3-*d*]pyrimidin]-4'-yl)azepane-4-carbonitrile (2.5 mg).
LCMS: ESI [M+H]⁺ 547.4 m/z; t_{R} = 4.04 minutes. (Method I)
¹H NMR (400 MHz, CD₃OD): δ 6.89 (d, 1 H), 6.74 (d, 1 H), 6.63 (dd, 1 H), 5.34-5.49 (m, 1 H), 4.84-4.93 (m, 1 H), 4.61 (d, 1 H), 4.24-4.37 (m, 2 H), 3.49-3.85 (m, 6 H), 3.06-3.20 (m, 2 H), 2.84-3.05 (m, 2 H), 2.68-2.75 (m, 2 H), 1.81-2.34 (m, 17 H).

### Example 14: 2'-(((2R,7aS)-2-Fluorotetrahydro-1H-pyrrolizin-7a(5H)-yl)methoxy)-4'-(1,4-oxazepan-4-yl)-3,4,5',8'-tetrahydro-2H-spiro[naphthalene-1,7'-pyrano[4,3-d]pyrimidin]-7-amine (Compound R136a)

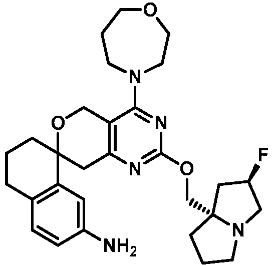

2'-(((2*R*,7a*S*)-2-Fluorotetrahydro-1*H*-pyrrolizin-7a(5*H*)-yl)methoxy)-4'-(1,4-oxazepan-4-yl)-3,4,5',8'-tetrahydro-2*H*-spiro[naphthalene-1,7'-pyrano[4,3-*d*]pyrimidin]-7-amine was prepared according to **General Procedure A** using *tert*-butyl (2'-(((2*R*,7a*S*)-2-fluorotetrahydro-1*H*-pyrrolizin-7a(5*H*)-yl)methoxy)-4'-oxo-3,3',4,4',5',8'-hexahydro-2*H-*spiro[naphthalene-1,7'-pyrano[4,3-*d*]pyrimidin]-7-yl)carbamate (150 mg, 0.23 mmol) and 1,4-oxazepane (35 mg, 0.34 mmol, 1.5 *equiv.*)*.* The reaction mixture was purified by preparative HPLC (Gemini 5 mm NX-C18, 110 Å, 100×30 mm, 45 mL/min; 10 mM NH₄CO₃/MeCN; 35-100%) to afford 2'-(((2*R*,7a*S*)-2-fluorotetrahydro-1*H*-pyrrolizin-7a(5*H*)-yl)methoxy)-4'-(1,4-oxazepan-4-yl)-3,4,5',8'-tetrahydro-2H-spiro[naphthalene-1,7'-pyrano[4,3-d]pyrimidin]-7-amine (20 mg).
LCMS: m/z (ESI) [M+H]⁺ 524.3, t_{R} = 1.57 minutes (Method B)
¹H NMR (400 MHz, DMSO-d₆): δ 6.76 (d, 1H); 6.57 (s, 1H), 6.43 (dd, 1H), 5.19-5.34 (m, 1H), 4.80 (s, 2H), 4.58 (dd, 2H), 3.93 (dd, 1H), 3.83 (dd, 1H), 3.59-3.70 (m, 8H), 3.06 (s, 2H), 2.99 (s, 1H), 2.74-2.89 (m, 3H), 2.55-2.67 (m, 3H), 1.69-2.04 (m, 11H).

### Example 14a: (S)-2'-(((2R,7aS)-2-Fluorotetrahydro-1H-pyrrolizin-7a(5H)-yl)methoxy)-4'-(1,4-oxazepan-4-yl)-3,4,5',8'-tetrahydro-2H-spiro[naphthalene-1,7'-pyrano[4,3-d]pyrimidin]-7-amine (Compound R136b)

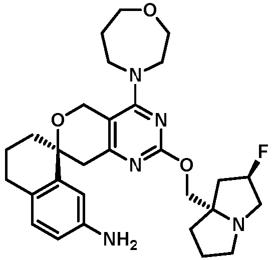

(*S*)-2'-(((2*R*,7a*S*)-2-Fluorotetrahydro-1*H*-pyrrolizin-7a(5*H*)-yl)methoxy)-4'-(1,4-oxazepan-4-yl)-3,4,5',8'-tetrahydro-2*H*-spiro[naphthalene-1,7'-pyrano[4,3-*d*]pyrimidin]-7-amine was prepared according to **General Procedure A** using *tert*-butyl ((*S*)-2'-(((2*R*,7a*S*)-2-fluorotetrahydro-1*H*-pyrrolizin-7a(5*H*)-yl)methoxy)-4'-oxo-3,3',4,4',5',8'-hexahydro-2*H-*spiro[naphthalene-1,7'-pyrano[4,3-*d*]pyrimidin]-7-yl)carbamate (200 mg, 0.30 mmol) and 1,4-oxazepane (46 mg, 0.45 mmol, 1.5 *equiv*.) and purified by preparative HPLC (C18 Biotage 12 g column; 10 mM NH₄CO₃/MeCN) to afford (*S*)-2'-(((2*R*,7a*S*)-2-fluorotetrahydro-1*H-*pyrrolizin-7a(5*H*)-yl)methoxy)-4'-(1,4-oxazepan-4-yl)-3,4,5',8'-tetrahydro-2*H-*spiro[naphthalene-1,7'-pyrano[4,3-*d*]pyrimidin]-7-amine (31 mg).
LCMS: ESI [M+H]⁺ 524.4 m/z, t_{R} = 3.85 minutes (Method I)
¹H NMR (400 MHz, DMSO-*d₆*) δ 6.73 (d, 1H), 6.54 (d, 1H), 6.41 (dd, 1H), 5.23 (d, 1H), 4.75 (s, 2H), 4.66 (d, 1H), 4.44 (d, 1H), 3.91 (d, 1H), 3.80 (d, 1H), 3.69 (d, 2H), 3.67 - 3.46 (m, 5H), 3.15 - 2.92 (m, 3H), 2.91 - 2.68 (m, 3H), 2.56 (t, 2H), 2.17 - 1.96 (m, 2H), 1.96 - 1.52 (m, 9H).

### Example 15: 5-(7-Amino-8-cyano-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide (Compound R158a)

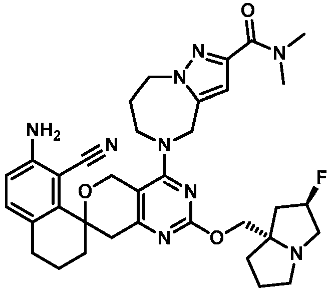

### Step 1: 5-(8-Bromo-7-(dibenzylamino)-2'-(methylthio)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide

DIPEA (1.70 g, 2.30 mL, 8 *equiv.,* 13.2 mmol) was added to a suspension of *N,N-*dibenzyl-8-bromo-4'-chloro-2'-(methylthio)-3,4,5',8'-tetrahydro-2*H*-spiro[naphthalene-1,7'-pyrano[4,3-d]pyrimidin]-7-amine (1.00 g, 1 *equiv.,* 1.65 mmol) and*N*,*N*-dimethyl-5,6,7,8-tetrahydro-4*H*-pyrazolo[1,5-*a*][1,4]diazepine-2-carboxamide hydrochloride (605 mg, 1.5 *equiv.,* 2.47 mmol) in DMF (33 mL) at room temperature. The mixture was heated at 120 °C for 18 hours, cooled to room temperature, and diluted with water. The aqueous layer was extracted with EtOAc three times, and the combined organic layers were washed with brine, dried over sodium sulfate, filtered, and concentrated to a residue, which was purified by flash column chromatography (80 g, 0-50% DCM/EtOAc to 0-10% DCM/methanol) to give 5-(8-bromo-7-(dibenzylamino)-2'-(methylthio)-3,4,5',8'-tetrahydro-2*H*-spiro[naphthalene-1,7'-pyrano[4,3-*d*]pyrimidin]-4'-yl)-*N*,*N*-dimethyl-5,6,7,8-tetrahydro-4*H*-pyrazolo[1,5-*a*][1,4]diazepine-2-carboxamide (719 mg).

LCMS: m/z (ESI) [M+H]⁺ 778.3, t_{R} = 2.15 minutes (Method B).

### Step 2: 5-(8-Cyano-7-(dibenzylamino)-2'-(methylthio)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide

Copper(I) cyanide (578 mg, 10 *equiv.,* 6.46 mmol) was added to a solution of 5-(8-bromo-7-(dibenzylamino)-2'-(methylthio)-3,4,5',8'-tetrahydro-2*H*-spiro[naphthalene-1,7'-pyrano[4,3-*d*]pyrimidin]-4'-yl)-*N*,*N*-dimethyl-5,6,7,8-tetrahydro-4*H*-pyrazolo[1,5-*a*][1,4]diazepine-2-carboxamide (503 mg, 1 *equiv.,* 0.646 mmol) in DMF (12.9 mL) and stirred at 120 °C for 5 hours. The reaction mixture was cooled to room temperature and then concentrated to a residue, which was treated with DCM/water. A solution of concentrated NH₄OH/MeOH (1:1) was added until a biphasic solution was observed. The mixture was stirred for 30 minutes, and the aqueous layer was extracted with DCM three times. The combined organic layers were washed with brine, dried over sodium sulfate, filtered, and concentrated to yield 5-(8-cyano-7-(dibenzylamino)-2'-(methylthio)-3,4,5',8'-tetrahydro-2*H-*spiro[naphthalene-1,7'-pyrano[4,3-*d*]pyrimidin]-4'-yl)-*N*,*N*-dimethyl-5,6,7,8-tetrahydro-4*H-*pyrazolo[1,5-*a*][1,4]diazepine-2-carboxamide (440 mg), which was used without further purification.

LCMS: m/z (ESI) [M+H]⁺ 725.5, t_{R} = 1.94 minutes (Method B).

### Step 3: 5-(8-cyano-7-(dibenzylamino)-2'-(methylsulfonyl)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide

3-Chlorobenzoperoxoic acid (370 mg, 85 wt%, 3 *equiv.,* 1.82 mmol) was added to a solution of 5-(8-cyano-7-(dibenzylamino)-2'-(methylthio)-3,4,5',8'-tetrahydro-2*H-*spiro[naphthalene-1,7'-pyrano[4,3-d]pyrimidin]-4'-yl)-*N*,*N*-dimethyl-5,6,7,8-tetrahydro-4*H-*pyrazolo[1,5-*a*][1,4]diazepine-2-carboxamide (440 mg, 1 *equiv.,* 0.61 mmol) in DCM (12.1 mL) at room temperature. The resulting yellow solution was stirred at room temperature for 1 hour and then diluted with DCM. A solution of saturated sodium bicarbonate and saturated sodium thiosulfate (10:1) was added. The resulting mixture was stirred at room temperature for 1 hour. The organic layer was separated, and the aqueous layer was extracted with DCM. The combined organic layers were washed with saturated potassium carbonate and brine, dried over sodium sulfate, filtered, and concentrated to give 5-(8-cyano-7-(dibenzylamino)-2'-(methylsulfonyl)-3,4,5',8'-tetrahydro-2*H*-spiro[naphthalene-1,7'-pyrano[4,3-*d*]pyrimidin]-4'-yl)-*N*,*N*-dimethyl-5,6,7,8-tetrahydro-4*H*-pyrazolo[1,5-*a*][1,4]diazepine-2-carboxamide (358 mg) as a foam, which was used without further purification.

LCMS: m/z (ESI) [M+H]⁺ 757.3, t_{R} = 1.70 minutes (Method B).

### Step 4: 5-(8-cyano-7-(dibenzylamino)-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide

((2*R*,7a*S*)-2-Fluorotetrahydro-1*H*-pyrrolizin-7a(5*H*)-yl)methanol (226 mg, 3 *equiv.,* 1.42 mmol) was added to a suspension of NaH (56.8 mg, 60 wt%, 3 *equiv.,* 1.42 mmol) in THF (9.5 mL) under argon at room temperature. The mixture was stirred at room temperature for 10 minutes. Then a solution of 5-(8-cyano-7-(dibenzylamino)-2'-(methylsulfonyl)-3,4,5',8'-tetrahydro-2*H*-spiro[naphthalene-1,7'-pyrano[4,3-*d*]pyrimidin]-4'-yl)-*N*,*N*-dimethyl-5,6,7,8-tetrahydro-4*H*-pyrazolo[1,5-*a*][1,4]diazepine-2-carboxamide (358 mg, *1 equiv.,* 0.47 mmol) in THF (2 mL) was added. The resulting orange solution was stirred at room temperature for 10 minutes. The mixture was then quenched with ice water and extracted with EtOAc three times. The combined organic layers were washed with brine, dried over sodium sulfate, filtered, and concentrated to a residue, which was purified by reverse-phase chromatography (30 g Biotage C18 silica, 0 - 60% (4 cv), 60% -100% (10 cv) MeCN/0.1% aqueous NH₄HCO₃) to provide 5-(8-cyano-7-(dibenzylamino)-2'-(((2*R*,7a*S*)-2-fluorotetrahydro-1*H*-pyrrolizin-7a(5*H*)-yl)methoxy)-3,4,5',8'-tetrahydro-2*H*-spiro[naphthalene-1,7'-pyrano[4,3-*d*]pyrimidin]-4'-yl)-*N,N*-dimethyl-5,6,7,8-tetrahydro-4*H*-pyrazolo[1,5-*a*][1,4]diazepine-2-carboxamide (273 mg).

LCMS: m/z (ESI) [M+H]⁺ 836.5, t_{R} = 1.79 minutes (Method B).

### Step 5: 5-(7-Amino-8-cyano-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide

Pd(OH)₂ on carbon (105 mg, 20 wt%, 2.5 *equiv.,* 0.15 mmol) was added to a solution of 5-(8-cyano-7-(dibenzylamino)-2'-(((2*R*,7a*S*)-2-fluorotetrahydro-1*H*-pyrrolizin-7a(5*H*)-yl)methoxy)-3,4,5',8'-tetrahydro-2*H*-spiro[naphthalene-1,7'-pyrano[4,3-*d*]pyrimidin]-4'-yl)-*N,N*-dimethyl-5,6,7,8-tetrahydro-4*H*-pyrazolo[1,5-*a*][1,4]diazepine-2-carboxamide (50 mg, 1 *equiv.,* 0.06 mmol) in MeOH (1.2 mL). The mixture was then sparged with argon. The inert atmosphere was exchanged with hydrogen by degassing the reaction mixture under reduced pressure with backflow of hydrogen from a balloon. Four drops of acetic acid were added, and the black reaction mixture was stirred overnight under hydrogen atmosphere at room temperature. Celite was added, and the atmosphere was exchanged for argon. The suspension was filtered over Celite with MeOH and concentrated. The crude residue was purified by reverse-phase chromatography (6 g Biotage C18 silica, 0 - 60% (4 cv), 60% -100% (10 cv) MeCN/0.1% aqueous NH₄HCO₃) to provide 5-(7-amino-8-cyano-2'-(((2*R*,7a*S*)-2-fluorotetrahydro-1*H*-pyrrolizin-7a(5*H*)-yl)methoxy)-3,4,5',8'-tetrahydro-2*H-*spiro[naphthalene-1,7'-pyrano[4,3-*d*]pyrimidin]-4'-yl)-*N*,*N*-dimethyl-5,6,7,8-tetrahydro-4*H-*pyrazolo[1,5-*a*][1,4]diazepine-2-carboxamide (17 mg).

LCMS: m/z (ESI) [M+H]⁺ 656.5, t_{R} = 1.26 minutes (Method B).

¹H NMR (400 MHz, DMSO-*d₆*) δ 7.01 (d, 1H), 6.70 (d, 1H), 6.51 (s, 1H), 5.70 (s, 2H), 5.21 (d, 1H), 4.95 (d, 1H), 4.79 - 4.52 (m, 3H), 4.43 (t, 2H), 3.95 - 3.70 (m, 4H), 3.22 (s, 3H), 3.09 - 2.97 (m, 3H), 2.98 - 2.73 (m, 6H), 2.61 - 2.51 (m, 2H), 2.21 - 1.86 (m, 6H), 1.84 - 1.54 (m, 4H).

### Example 15a: 5-((R)-7-amino-8-cyano-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide (Compound R158b)

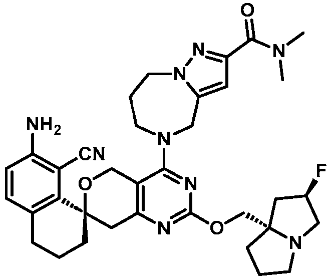

The diastereomers of 5-(7-amino-8-cyano-2'-(((2*R*,7a*S*)-2-fluorotetrahydro-1*H-*pyrrolizin-7a(5*H*)-yl)methoxy)-3,4,5',8'-tetrahydro-2*H*-spiro[naphthalene-1,7'-pyrano[4,3-*d*]pyrimidin]-4'-yl)-*N,N*-dimethyl-5,6,7,8-tetrahydro-4*H*-pyrazolo[1,5-*a*][1,4]diazepine-2-carboxamide were separated by chiral preparative HPLC (Column: Gemini 5 mm NX-C18; 30 × 100 mm; Mobile phase A: 10 mM ammonium bicarbonate pH = 10.0; Mobile phase B: MeOH; Gradient: 55% to 45% B) to afford 5-((*R*)-7-amino-8-cyano-2'-(((2*R*,7a*S*)-2-fluorotetrahydro-1*H*-pyrrolizin-7a(5*H*)-yl)methoxy)-3,4,5',8'-tetrahydro-2*H-*spiro[naphthalene-1,7'-pyrano[4,3-*d*]pyrimidin]-4'-yl)-*N,N*-dimethyl-5,6,7,8-tetrahydro-4*H-*pyrazolo[1,5-*a*][1,4]diazepine-2-carboxamide **(Peak 1)** (10 mg) and 5-((*S*)-7-amino-8-cyano-2'-(((2*R*,7a*S*)-2-fluorotetrahydro-1*H*-pyrrolizin-7a(5*H*)-yl)methoxy)-3,4,5',8'-tetrahydro-2*H-*spiro[naphthalene-1,7'-pyrano[4,3-*d*]pyrimidin]-4'-yl)-*N,N*-dimethyl-5,6,7,8-tetrahydro-4*H-*pyrazolo[1,5-*a*][1,4]diazepine-2-carboxamide **(Peak 2)** (8 mg).
**Peak 1:** Chiral HPLC: t_{R} = 5.12 minutes. (Column: Cellulose-1, 4.6 × 250 mm; 5 mM; Mobile Phase: 40% MeOH with 0.1% NH₄OH over 10 minutes.)
¹H NMR (400 MHz, DMSO-*d₆*): δ 7.04 (d, 1 H), 6.73 (d, 1 H), 6.54 (s, 1 H), 5.73 (s, 2 H), 5.34-5.10 (m, 1 H), 4.98 (d, 1 H), 4.72 (t, 2 H), 4.62 (t, 1 H), 4.45 (t, 2 H), 3.92 (d, 2 H), 3.83 (d, 2H), 3.11 (s, 1 H), 3.01-3.07 (m, 3 H), 2.99 (s, 1 H), 2.93 (s, 3 H), 2.78-2.86 (m, 2 H), 2.60 (s, 3 H), 2.14 (d, 2 H), 2.05-2.07 (m, 1 H), 1.96 (d, 3 H), 1.72-1.82 (m, 5 H).

### Example 15b: 5-((S)-7-amino-8-cyano-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d)pyrimidin]-4'-yl)-N,N-dimethyl-5,6,7,8-tetrahydro-4H-pyrazolo[1,5-a][1,4]diazepine-2-carboxamide (Compound R158c)

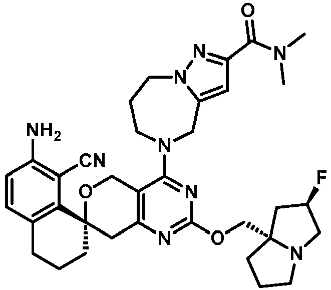

5-((*S*)-7-amino-8-cyano-2'-(((2*R*,7a*S*)-2-fluorotetrahydro-1*H*-pyrrolizin-7a(5*H*)-yl)methoxy)-3,4,5',8'-tetrahydro-2*H*-spiro[naphthalene-1,7'-pyrano[4,3-*d*]pyrimidin]-4'-yl)-*N,N*-dimethyl-5,6,7,8-tetrahydro-4*H*-pyrazolo[1,5-*a*][1,4]diazepine-2-carboxamide (8 mg) was obtained from **Example 15a,** as **Peak 2:** Chiral HPLC: t_{R} = 6.38 minutes. (Column: Cellulose-1, 4.6 × 250 mm; 5 mM; Mobile phase: 40% MeOH with 0.1% NH₄OH over 10 minutes.)
¹H NMR (400 MHz, DMSO-*d₆*): δ 7.04 (d, 1 H), 6.73 (d, 1 H), 6.54 (s, 1 H), 5.73 (s, 2 H), 5.34-5.07 (m, 1 H), 4.98 (d, 1 H), 4.72 (t, 2 H), 4.62 (t, 1 H), 4.45 (t, 2 H), 3.92 (d, 2 H), 3.83 (d, 2H), 3.11 (s, 1 H), 3.01-3.07 (m, 3 H), 2.99 (s, 1 H), 2.93 (s, 3 H), 2.78-2.86 (m, 2 H), 2.60 (s, 3 H), 2.14 (d, 2 H), 2.05-2.07 (m, 1 H), 1.96 (d, 3 H), 1.72-1.82 (m, 5 H).

### Example 16: 7-Amino-2'-(((2R,7aS)-2-fluorotetrahydro-1H-pyrrolizin-7a(5H)-yl)methoxy)-4'-(1,4-oxazepan-4-yl)-3,4,5',8'-tetrahydro-2H-spiro[naphthalene-1,7'-pyrano[4,3-d]pyrimidine]-8-carbonitrile (Compound R161a)

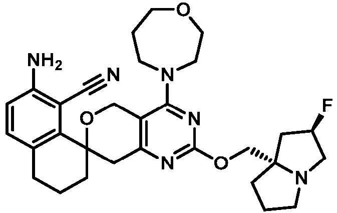

### Step 1: N,N-Dibenzyl-8-bromo-2'-(methylthio)-4'-(1,4-oxazepan-4-yl)-3,4,5',8'-tetrahydro-2H-spiro[naphthalene-1,7'-pyrano[4,3-d]pyrimidin]-7-amine

DIPEA (1.49 mL, 8 *equiv.,* 8.57 mmol) was added to a suspension of *N*,*N*-dibenzyl-8-bromo-4'-chloro-2'-(methylthio)-3,4,5',8'-tetrahydro-2*H*-spiro[naphthalene-1,7'-pyrano[4,3-*d*]pyrimidin]-7-amine (650 mg, 1 *equiv.,* 1.07 mmol) and 1,4-oxazepane (186 µL, 96 wt%, 1.5 *equiv.,* 1.61 mmol) in DMF (21.4 mL) at room temperature. The mixture was heated at 120 °C for 2 hours, cooled to room temperature, and diluted with water. The aqueous layer was extracted with EtOAc (3 x). The combined organic layers were washed with brine, dried over sodium sulfate, filtered, and concentrated. The resulting residue was purified by flash chromatography (24 g, 0-20% DCM/EtOAc) to give *N,N*-dibenzyl-8-bromo-2'-(methylthio)-4'-(1,4-oxazepan-4-yl)-3,4,5',8'-tetrahydro-2*H*-spiro[naphthalene-1,7'-pyrano[4,3-*d*]pyrimidin]-7-amine (235 mg).
LCMS: ESI [M+H]⁺ 673.2 m/z; t_{R} = 2.12 minutes. (Method B)

### Step 2: 7-(Dibenzylamino)-2'-(methylthio)-4'-(1,4-oxazepan-4-yl)-3,4,5',8'-tetrahydro-2H-spiro[naphthalene-1,7'-pyrano[4,3-d]pyrimidine]-8-carbonitrile

Copper(I) cyanide (256 mg, 10 *equiv.,* 2.86 mmol) was added to *N,N*-dibenzyl-8-bromo-2'-(methylthio)-4'-(1,4-oxazepan-4-yl)-3,4,5',8'-tetrahydro-2*H*-spiro[naphthalene-1,7'-pyrano[4,3-*d*]pyrimidin]-7-amine (192 mg, *1 equiv.,* 0.29 mmol) in DMF (5.7 mL) and stirred at 120 °C for 2 hours. The reaction mixture was cooled to room temperature and concentrated to a residue which was treated with DCM/water to form a precipitate. A solution of concentrated NH₄OH/MeOH (1:1) was added until no solid was visible, and the mixture was stirred for 30 minutes. The aqueous layer was extracted with DCM three times. The combined organic layers were washed with brine, dried over sodium sulfate, filtered, and concentrated to afford 7-(dibenzylamino)-2'-(methylthio)-4'-(1,4-oxazepan-4-yl)-3,4,5',8'-tetrahydro-2*H-*spiro[naphthalene-1,7'-pyrano[4,3-*d*]pyrimidine]-8-carbonitrile (162 mg), which was used in the next step without further purification.

LCMS: m/z (ESI) [M+H]⁺ 618.4, t_{R} = 1.93 minutes (Method B).

### Step 3: 7-(Dibenzylamino)-2'-(methylsulfonyl)-4'-(1,4-oxazepan-4-yl)-3,4,5',8'-tetrahydro-2H-spiro[naphthalene-1,7'-pyrano[4,3-d]pyrimidine]-8-carbonitrile

*mCPBA* (160 mg, 85 wt%, 3 *equiv.,* 0.79 mmol) was added to the solution of 7-(dibenzylamino)-2'-(methylthio)-4'-(1,4-oxazepan-4-yl)-3,4,5',8'-tetrahydro-2*H-*spiro[naphthalene-1,7'-pyrano[4,3-*d*]pyrimidine]-8-carbonitrile (162 mg, 1 *equiv*., 0.26 mmol) in DCM (5.2 mL) and stirred at room temperature for 1 hour. The reaction mixture was diluted with DCM, and a solution of saturated sodium bicarbonate and saturated sodium thiosulfate (10:1) was added. The mixture was stirred at room temperature for 1 hour. The organic layer was separated, and the aqueous layer was extracted with DCM. The combined organic layers were washed with saturated potassium carbonate followed by brine. The organic layers were dried over sodium sulfate, filtered, and concentrated to give 7-(dibenzylamino)-2'-(methylsulfonyl)-4'-(1,4-oxazepan-4-yl)-3,4,5',8'-tetrahydro-2*H*-spiro[naphthalene-1,7'-pyrano[4,3-*d*]pyrimidine]-8-carbonitrile (150 mg), which was used in the next step without further purification.

LCMS: m/z (ESI) [M+H]⁺ 650.4, t_{R} = 1.73 minutes (Method B).

### Step 4: 7-(dibenzylamino)-2'-(((2R,7aS)-2-fluorotetrahydro-1H-pyrrolizin-7a(5H)-yl)methoxy)-4'-(1,4-oxazepan-4-yl)-3,4,5',8'-tetrahydro-2H-spiro[naphthalene-1,7'-pyrano[4,3-d]pyrimidine]-8-carbonitrile

((2*R*,7a*S*)-2-Fluorotetrahydro-1*H*-pyrrolizin-7a(5*H*)-yl)methanol (110 mg, 3 *equiv.,* 0.69 mmol) was added to a suspension of NaH (27.7 mg, 60 wt%, 3 *equiv.,* 0.69 mmol) in THF (4.6 mL) and stirred at room temperature for 10 minutes. A solution of 7-(dibenzylamino)-2'-(methylsulfonyl)-4'-(1,4-oxazepan-4-yl)-3,4,5',8'-tetrahydro-2*H*-spiro[naphthalene-1,7'-pyrano[4,3-*d*]pyrimidine]-8-carbonitrile (150 mg, 1 *equiv.,* 0.23 mmol) in THF (0.5 mL) was added over 5 minutes. The mixture was then stirred at room temperature for 20 minutes. The reaction was quenched with ice water and extracted with EtOAc three times. The combined organic layers were washed with brine, dried over sodium sulfate, filtered, and concentrated to a residue, which was purified by reverse-phase chromatography (30 g Biotage C18 silica, 0 - 40% (4 cv), 40% -100% (8 cv) MeCN/0.1% aqueous NH₄HCO₃) to provide 7-(dibenzylamino)-2'-(((2*R*,7a*S*)-2-fluorotetrahydro-1*H*-pyrrolizin-7a(5*H*)-yl)methoxy)-4'-(1,4-oxazepan-4-yl)-3,4,5',8'-tetrahydro-2*H*-spiro[naphthalene-1,7'-pyrano[4,3-*d*]pyrimidine]-8-carbonitrile (40 mg).

LCMS: m/z (ESI) [M+H]⁺ 729.5, t_{R} = 1.88 minutes (Method B).

### Step 5: 7-Amino-2'-(((2R,7aS)-2-fluorotetrahydro-1H-pyrrolizin-7a(5H)-yl)methoxy)-4'-(1,4-oxazepan-4-yl)-3,4,5',8'-tetrahydro-2H-spiro[naphthalene-1,7'-pyrano[4,3-d]pyrimidine]-8-carbonitrile

Pd(OH)₂ on carbon (94 mg, 20 wt%, 2.5 *equiv.,* 0.13 mmol) was added to 7-(dibenzylamino)-2'-(((2*R*,7a*S*)-2-fluorotetrahydro-1*H*-pyrrolizin-7a(5*H*)-yl)methoxy)-4'-(1,4-oxazepan-4-yl)-3,4,5',8'-tetrahydro-2*H*-spiro[naphthalene-1,7'-pyrano[4,3-*d*]pyrimidine]-8-carbonitrile (39 mg, 1 *equiv.,* 0.05 mmol) in MeOH (1.1 mL). The mixture was sparged with argon. The inert atmosphere was exchanged with hydrogen by degassing the reaction mixture under reduced pressure and back-filled with hydrogen from a balloon. Four drops of acetic acid were added to the reaction mixture, and the black reaction mixture was stirred overnight under hydrogen atmosphere at room temperature. Celite was added, and the atmosphere was exchanged for argon. The suspension was filtered over Celite with MeOH and concentrated to a residue, which was purified by reverse-phase chromatography (6 g Biotage C18 silica, 0 - 60% (4 cv), 60% -100% (10 cv) MeCN/0.1% aqueous NH₄HCO₃) to provide 7-amino-2'-(((2*R*,7a*S*)-2-fluorotetrahydro-1*H*-pyrrolizin-7a(5*H*)-yl)methoxy)-4'-(1,4-oxazepan-4-yl)-3,4,5',8'-tetrahydro-2*H*-spiro[naphthalene-1,7'-pyrano[4,3-*d*]pyrimidine]-8-carbonitrile (16 mg).

LCMS: m/z (ESI) [M+H]⁺ 549.3, t_{R} = 1.32 minutes (Method B).

¹H NMR (400 MHz, DMSO-d₆): δ 7.04 (d, 1H), 6.73 (d, 1H), 5.74 (s, 2 H), 5.22 (d, 1 H), 4.88 (d, 1H), 4.66 (d, 1H), 3.84-3.93 (m, 2H), 3.72 (d, 6H), 3.60 (br s, 2H), 3.06-3.11 (m, 3H), 2.99 (s, 1H), 2.84 (d, 2H), 2.59 (s, 2H), 2.12 (d, 2H), 1.98 (d, 3H), 1.83 (br s, 3H), 1.71 (d, 4H).

### Example 16a: (S)-7-amino-2'-(((2R,7aS)-2-fluorotetrahydro-1H-pyrrolizin-7a(5H)-yl)methoxy)-4'-(1,4-oxazepan-4-yl)-3,4,5',8'-tetrahydro-2H-spiro[naphthalene-1,7'-pyrano[4,3-d]pyrimidine]-8-carbonitrile (Compound R161c)

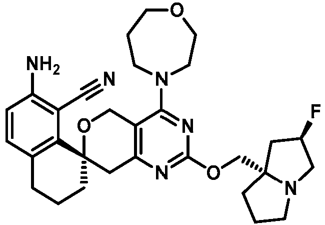

The diastereomers of 7-amino-2'-(((2*R*,7a*S*)-2-fluorotetrahydro-1*H*-pyrrolizin-7a(5*H*)-yl)methoxy)-4'-(1,4-oxazepan-4-yl)-3,4,5',8'-tetrahydro-2*H*-spiro[naphthalene-1,7'-pyrano[4,3-d]pyrimidine]-8-carbonitrile were separated by chiral preparative HPLC (Column: Cellulose-1 5 µm; 30 × 250 mm; Mobile phase A: CO₂; Mobile phase B: MeOH with 0.1% DEA; Isocratic: 35% B; 100 mL/min;) to afford (*S*)-7-amino-2'-(((2*R*,7a*S*)-2-fluorotetrahydro-1*H*-pyrrolizin-7a(5*H*)-yl)methoxy)-4'-(1,4-oxazepan-4-yl)-3,4,5',8'-tetrahydro-2*H-*spiro[naphthalene-1,7'-pyrano[4,3-*d*]pyrimidine]-8-carbonitrile **(Peak 1)** (49.2 mg) and (*R*)-7-amino-2'-(((2*R*,7a*S*)-2-fluorotetrahydro-1*H*-pyrrolizin-7a(5*H*)-yl)methoxy)-4'-(1,4-oxazepan-4-yl)-3,4,5',8'-tetrahydro-2*H*-spiro[naphthalene-1,7'-pyrano[4,3-*d*]pyrimidine]-8-carbonitrile **(Peak 2)** (64.7 mg).
**Peak 1:** Chiral HPLC: t_{R} = 7.31 minutes. (Column: Lux Cellulose-3, 4.6 × 150 mm; 5 µM; column temperature; 30 °C; Gradient:70% water with 10mM NH₄CO₃/30% MeOH over 12 minutes.)
¹H NMR (400 MHz, DMSO-d₆): *δ* 7.01 (d, 1H), 6.70 (d, 1H), 5.71 (s, 2H), 5.26 (d, 1H), 4.85 (d, 1H), 4.63 (d, 1H), 4.03 - 3.81 (m, 2H), 3.81 - 3.62 (m, 6H), 3.62 - 3.48 (m, 2H), 3.19 - 2.94 (m, 3H), 2.94 - 2.76 (m, 2H), 2.63 - 2.51 (m, 2H), 2.20 - 2.06 (m, 2H), 2.02 (s, 1H), 2.00 - 1.88 (m, 2H), 1.88 - 1.55 (m, 6H).

### Example 16b: (R)-7-amino-2'-(((2R,7aS)-2-fluorotetrahydro-1H-pyrrolizin-7a(5H)-yl)methoxy)-4'-(1,4-oxazepan-4-yl)-3,4,5',8'-tetrahydro-2H-spiro[naphthalene-1,7'-pyrano[4,3-d]pyrimidine]-8-carbonitrile (Compound R161b)

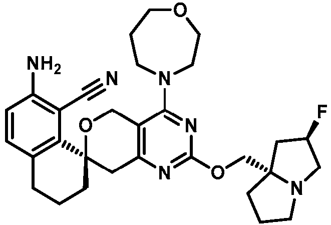

(*R*)-7-amino-2'-(((2*R*,7a*S*)-2-fluorotetrahydro-1*H*-pyrrolizin-7a(5*H*)-yl)methoxy)-4'-(1,4-oxazepan-4-yl)-3,4,5',8'-tetrahydro-2*H*-spiro[naphthalene-1,7'-pyrano[4,3-*d*]pyrimidine]-8-carbonitrile (64.7 mg) was obtained from Example 16a, as Peak 2: Chiral HPLC: t_{R} = 8.68 minutes. (Column: Lux Cellulose-3, 4.6 × 150 mm; 5 µM; column temperature; 30 °C; Gradient:70% water with 10mM NH₄CO₃/30% MeOH over 12 minutes.)

¹H NMR (400 MHz, DMSO-*d₆*): δ 7.01 (d, 1H), 6.69 (d, 1H), 5.72 (s, 2H), 5.22 (d, 1H), 4.85 (d, 1H), 4.63 (d, 1H), 3.90 (d, 1H), 3.81 (d, 1H), 3.78 - 3.61 (m, 4H), 3.61 - 3.48 (m, 2H), 3.10 - 3.00 (m, 3H), 3.00 - 2.93 (m, 1H), 2.84 (s, 1H), 2.82 - 2.72 (m, 1H), 2.61 - 2.52 (m, 2H), 2.20 - 2.08 (m, 1H), 2.08 - 2.02 (m, 1H), 2.02 - 1.87 (m, 3H), 1.88 - 1.60 (m, 6H), 1.56 (s, 1H).

### Example 17: 5-(7-Amino-2'-(((2R,7aS)-2-fluorotetrahydro-1H-pyrrolizin-7a(5H)-yl)methoxy)-4-methyl-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide (Compound R124a)

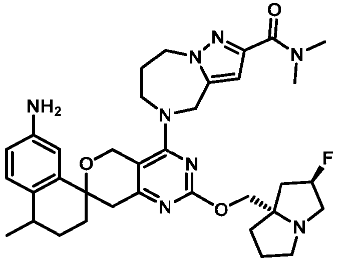

### Step 1: 7-Bromo-1-((6-chloro-5-(hydroxymethyl)-2-(methylthio)pyrimidin-4-yl)methyl)-4-methyl-1,2,3,4-tetrahydronaphthalen-1-ol

LDA solution (2.81 mL, 1 M, 2.3 *equiv.,* 2.81 mmol) was added dropwise to (4-chloro-6-methyl-2-(methylthio)pyrimidin-5-yl)methanol (250 mg, *1 equiv.,* 1.22 mmol) at -78 °C, maintaining a temperature below -74 °C. The solution was stirred at -78 °C for 30 minutes. 7-Bromo-4-methyl-3,4-dihydronaphthalen-1(2*H*)-one (321 mg, 1.1 *equiv.,* 1.34 mmol) was dissolved in THF (0.75 mL) and added dropwise to the solution, keeping the reaction mixture below -74 °C. After 30 minutes, an equal volume of saturated ammonium chloride solution was added, and the reaction mixture was stirred for 15 minutes. The reaction mixture was then extracted with EtOAc (3 x 100 mL), and then the organic layer was dried using sodium sulfate. The mixture was concentrated to a residue, which was purified by flash chromatography (ISCO silica Gold cartridge; Gradient: 100% DCM to 85:15 DCM:EtOAc) to afford 7-bromo-1-((6-chloro-5-(hydroxymethyl)-2-(methylthio)pyrimidin-4-yl)methyl)-4-methyl-1,2,3,4-tetrahydronaphthalen-1-ol (320 mg).

¹H NMR (400 MHz, CDCl₃) δ 7.71 (m, 1H), 7.39 (m, 1H), 7.13 (m, 1H), 4.86 - 4.72 (m, 2H), 4.05 (m, 1H), 3.49 - 3.16 (m, 2H), 3.06 - 2.81 (m, 2H), 2.57 (m, 3H), 2.29 - 2.14 (m, 1H), 2.09 - 1.96 (m, 1H), 1.93 - 1.64 (m, 2H), 1.34 (m, 3H).

### Step 2: 7-Bromo-4'-chloro-4-methyl-2'-(methylthio)-3,4,5',8'-tetrahydro-2H-spiro[naphthalene-1,7'-pyrano[4,3-d]pyrimidine]

To a 100 mL round bottom flask containing 7-bromo-1-((6-chloro-5-(hydroxymethyl)-2-(methylthio)pyrimidin-4-yl)methyl)-4-methyl-1,2,3,4-tetrahydronaphthalen-1-ol (320 mg, 1 *equiv.,* 0.72 mmol) in toluene (1.34 mL) was added phosphoric acid (49 µL, 85 wt%, 1 *equiv.*, 0.72 mmol). The reaction mixture was heated to reflux for 2 hours. The solvent was removed under reduced pressure, and the resulting residue was partitioned between EtOAc (8 mL) and water (8 mL). The organic phase was separated, and the aqueous was extracted with EtOAc (3 × 4 mL). The combined organics were dried with sodium sulfate, and solvent was removed under reduced pressure to give 7-bromo-4'-chloro-4-methyl-2'-(methylthio)-3,4,5',8'-tetrahydro-2*H*-spiro[naphthalene-1,7'-pyrano[4,3-*d*]pyrimidine] (285 mg).

¹H NMR (400 MHz, CDCl₃) δ 7.62 (m, 1H), 7.38 (m, 1H), 7.11 (m, 1H), 5.09 - 4.58 (m, 2H), 3.22 - 2.82 (m, 3H), 2.56 (m, 3H), 2.20 - 1.85 (m, 4H), 1.31 (m, 3H).

### Step 3: 5-(7-Bromo-4-methyl-2'-(methylthio)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide

DIPEA (475 µL, 4 *equiv.,* 2.72 mmol) was added to 7-bromo-4'-chloro-4-methyl-2'-(methylthio)-3,4,5',8'-tetrahydro-2*H*-spiro[naphthalene-1,7'-pyrano[4,3-*d*]pyrimidine] (290 mg, 1 *equiv.,* 0.68 mmol) and *N*,*N*-dimethyl-5,6,7,8-tetrahydro-4*H*-pyrazolo[1,5-*a*][1,4]diazepine-2-carboxamide, HCl (310 mg, 1.6 *equiv.,* 1.09 mmol) in ethanol (1.36 mL). The mixture was heated to 85 °C for 16 hours. The reaction mixture was concentrated, and EtOAc (10 mL) was added. The solution was washed with water (10 mL) and brine (10 mL). The combined aqueous layers were extracted with EtOAc (20 mL), and the combined organic layers were dried over sodium sulfate and concentrated to afford 5-(7-bromo-4-methyl-2'-(methylthio)-3,4,5',8'-tetrahydro-2*H*-spiro[naphthalene-1,7'-pyrano[4,3-*d*]pyrimidin]-4'-yl)-*N*,*N*-dimethyl-5,6,7,8-tetrahydro-4*H*-pyrazolo[1,5-*a*][1,4]diazepine-2-carboxamide (361.1 mg).

LCMS: ESI [M+H]⁺ 599.3; t_{R} = 1.98 minutes (Method C).

### Step 4: tert-Butyl (4'-(2-(dimethylcarbamoyl)-7,8-dihydro-4H-pyrazolo[1,5-a][1,4]diazepin-5(6H)-yl)-4-methyl-2'-(methylthio)-3,4,5',8'-tetrahydro-2H-spiro[naphthalene-1,7'-pyrano[4,3-d]pyrimidin]-7-yl)carbamate

A mixture of *tert*-butyl carbamate (142 mg, 2 *equiv.,* 1.21 mmol), 5-(7-bromo-4-methyl-2'-(methylthio)-3,4,5',8'-tetrahydro-2H-spiro[naphthalene-1,7'-pyrano[4,3-*d*]pyrimidin]-4'-yl)-*N*,*N*-dimethyl-5,6,7,8-tetrahydro-4*H*-pyrazolo[1,5-*a*][1,4]diazepine-2-carboxamide (361 mg, *1 equiv.,* 0.60 mmol), 2-(dicyclohexylphosphanyl)-2',4',6'-tris(isopropyl)biphenyl (25.9 mg, 0.09 *equiv.,* 0.05 mmol), and Pd(OAc)₂ (4 mg, 0.03 *equiv.,* 0.02 mmol) in 1,4-dioxane (4.8 mL) was sparged with nitrogen. The reaction mixture was heated to 100 °C for 60 minutes, cooled to room temperature, and filtered. The filtrate was concentrated to a residue, which was purified via column chromatography (SiO₂, 100% DCM to 85:15 DCM:MeOH) to give *tert*-butyl (4'-(2-(dimethylcarbamoyl)-7,8-dihydro-4H-pyrazolo[1,5-*a*][1,4]diazepin-5(6*H*)-yl)-4-methyl-2'-(methylthio)-3,4,5',8'-tetrahydro-2*H-*spiro[naphthalene-1,7'-pyrano[4,3-*d*]pyrimidin]-7-yl)carbamate (274 mg).

¹H NMR (400 MHz, CDCl₃) δ 7.36 - 7.09 (m, 3H), 6.73 - 6.25 (m, 2H), 4.89 - 4.37 (m, 6H), 3.87 (m, 2H), 3.34 (m, 3H), 3.21 - 2.85 (m, 6H), 2.50 (s, 3H), 2.29 - 1.82 (m, 6H), 1.45 (d, J = 1.9 Hz, 9H), 1.30 (m, 3H).

### Step 5: tert-Butyl (4'-(2-(dimethylcarbamoyl)-7,8-dihydro-4H-pyrazolo[1,5-a][1,4]diazepin-5(6H)-yl)-4-methyl-2'-(methylsulfonyl)-3,4,5',8'-tetrahydro-2H-spiro[naphthalene-1,7'-pyrano[4,3-d]pyrimidin]-7-yl)carbamate

Oxone (1.13 g, 45 wt%, 2.1 *equiv.,* 0.83 mmol) was added to *tert*-butyl (4'-(2-(dimethylcarbamoyl)-7,8-dihydro-4H-pyrazolo[1,5-*a*][1,4]diazepin-5(6*H*)-yl)-4-methyl-2'-(methylthio)-3,4,5',8'-tetrahydro-2*H*-spiro[naphthalene-1,7'-pyrano[4,3-*d*]pyrimidin]-7-yl)carbamate (250 mg, *1 equiv.,* 0.39 mmol) in water (4 mL) and MeOH (4 mL). The mixture was stirred for 30 minutes and then quenched by the addition of water (10 mL). EtOAc (10 mL) was added, and the layers were separated and the aqueous phase was extracted with EtOAc (3 x 5 mL). The combined organics were dried over sodium sulfate, filtered, and concentrated to afford tert-butyl (4'-(2-(dimethylcarbamoyl)-7,8-dihydro-4*H*-pyrazolo[1,5-*a*][1,4]diazepin-5(6*H*)-yl)-4-methyl-2'-(methylsulfonyl)-3,4,5',8'-tetrahydro-2*H*-spiro[naphthalene-1,7'-pyrano[4,3-*d*]pyrimidin]-7-yl)carbamate (109 mg).

LCMS: (ESI) m/z = 666.5 [M+H]⁺ ; t_{R} = 1.90 minutes. (Method C).

### Step 6: tert-Butyl (4'-(2-(dimethylcarbamoyl)-7,8-dihydro-4H-pyrazolo[1,5-a][1,4]diazepin-5(6H)-yl)-2'-(((2R,7aS)-2-fluorotetrahydro-1H-pyrrolizin-7a(5H)-yl)methoxy)-4-methyl-3,4,5',8'-tetrahydro-2H-spiro[naphthalene-1,7'-pyrano[4,3-d]pyrimidin]-7-yl)carbamate

Potassium *tert*-butoxide in THF (666 µL, 1 M, 2 *equiv.,* 0.67 mmol) was added dropwise to a solution of *tert*-butyl (4'-(2-(dimethylcarbamoyl)-7,8-dihydro-4*H*-pyrazolo[1,5-*a*][1,4]diazepin-5(6*H*)-yl)-4-methyl-2'-(methylsulfonyl)-3,4,5',8'-tetrahydro-2*H-*spiro[naphthalene-1,7'-pyrano[4,3-d]pyrimidin]-7-yl)carbamate (223 mg, 1 *equiv.*, 0.33 mmol) and ((2*R*,7a*S*)-2-fluorotetrahydro-1*H*-pyrrolizin-7a(5*H*)-yl)methanol (53.0 mg, 1 *equiv.,* 0.33 mmol) in THF (3.3 mL) at 0 °C. The reaction mixture was stirred for 40 minutes followed by the addition of water (1 mL) and EtOAc (2 mL). The aqueous layer was extracted with EtOAc (3 x 4 mL) and the combined organics were dried over sodium sulfate, filtered, and concentrated to a residue, which was purified by flash chromatography (SiO₂; Gradient: 100% DCM to 85:15 DCM:MeOH) to give *tert*-butyl (4'-(2-(dimethylcarbamoyl)-7,8-dihydro-4*H*-pyrazolo[1,5-*a*][1,4]diazepin-5(6*H*)-yl)-2'-(((2*R*,7a*S*)-2-fluorotetrahydro-1*H*-pyrrolizin-7a(5*H*)-yl)methoxy)-4-methyl-3,4,5',8'-tetrahydro-2*H*-spiro[naphthalene-1,7'-pyrano[4,3-*d*]pyrimidin]-7-yl)carbamate (77.6 mg).

¹H NMR (400 MHz, CDCl₃) δ 7.47 - 6.96 (m, 3H), 6.72 - 6.35 (m, 2H), 5.26 (d, J = 53.9 Hz, 1H), 4.86 - 4.34 (m, 6H), 4.13 - 3.71 (m, 4H), 3.42 - 2.86 (m, 13H), 2.29 - 1.77 (m, 12H), 1.48 - 1.38 (m, 9H), 1.30 (dd, J = 7.0, 4.8 Hz, 3H).

### Step 7: 5-(7-Amino-2'-(((2R,7aS)-2-fluorotetrahydro-1H-pyrrolizin-7a(5H)-yl)methoxy)-4-methyl-3,4,5',8'-tetrahydro-2H-spiro[naphthalene-1,7'-pyrano[4,3-d)pyrimidin]-4'-yl)-N,N-dimethyl-5,6,7,8-tetrahydro-4H-pyrazolo[1,5-a][1,4]diazepine-2-carboxamide

HCl (350 µL, 4 M, 14 *equiv.,* 1.40 mmol) was added to *tert*-butyl (4'-(2-(dimethylcarbamoyl)-7,8-dihydro-4*H*-pyrazolo[1,5-*a*][1,4]diazepin-5(6*H*)-yl)-2'-(((2*R*,7a*S*)-2-fluorotetrahydro-1*H*-pyrrolizin-7a(5*H*)-yl)methoxy)-4-methyl-3,4,5',8'-tetrahydro-2*H-*spiro[naphthalene-1,7'-pyrano[4,3-*d*]pyrimidin]-7-yl)carbamate (75 mg, 1 *equiv.,* 0.10 mmol) at 0 °C. The mixture was stirred for 30 minutes, and the reaction mixture was concentrated to afford 5-(7-amino-2'-(((2*R*,7a*S*)-2-fluorotetrahydro-1*H*-pyrrolizin-7a(5*H*)-yl)methoxy)-4-methyl-3,4,5',8'-tetrahydro-2*H*-spiro[naphthalene-1,7'-pyrano[4,3-*d*]pyrimidin]-4'-yl)-*N,N-*dimethyl-5,6,7,8-tetrahydro-4*H*-pyrazolo[1,5-*a*][1,4]diazepine-2-carboxamide, 3HCl (68 mg).
LCMS: ESI [M+H]⁺ 645.5 m/z; t_{R} = 1.16 minutes (Method C)
¹H NMR (400 MHz, MeOD) δ 7.59 - 7.42 (m, 2H), 7.39 - 7.28 (m, 1H), 6.49 (d, 1H), 5.60 (d, 1H), 5.12 - 4.88 (m, 2H), 4.81 - 4.47 (m, 5H), 4.31-4.19 (m, 1H), 4.09 - 3.83 (m, 3H), 3.49 - 3.43 (m, 2H), 3.37 - 3.32 (m, 6H), 3.17 - 3.00 (m, 6H), 2.79 - 2.52 (m, 2H), 2.53 - 1.85 (m, 9H), 1.37 (dd, 3H).
¹⁹F NMR (376 MHz, MeOD) δ -174.31.

### Example 18: 5-((1S,4S)-7-Amino-2'-(((2R,7aS)-2-fluorotetrahydro-1H-pyrrolizin-7a(5H)-yl)methoxy)-4-methyl-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide (Compound R124b)

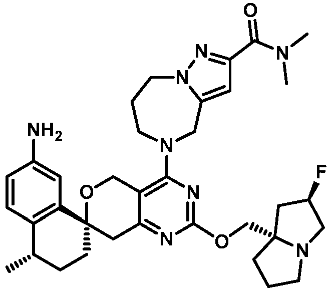

### Step 1: (4S)-7-Bromo-1-((6-chloro-5-(hydroxymethyl)-2-(methylthio)pyrimidin-4-yl)methyl)-4-methyl-1,2,3,4-tetrahydronaphthalen-1-ol

LDA solution (2.60 mL, 1 M, 2.3 *equiv.,* 2.60 mmol) was added dropwise to a solution of (4-chloro-6-methyl-2-(methylthio)pyrimidin-5-yl)methanol (231 mg, 1 *equiv.*, 1.13 mmol) in THF (4 mL) at -78 °C and stirred for 1.25 hours. To this mixture was added (S)-7-bromo-4-methyl-3,4-dihydronaphthalen-1(2*H*)-one (2^{nd} eluting peak) **(Intermediate 7b** *supra*) (270 mg, 1 *equiv.,* 1.13 mmol) in THF (3 mL) dropwise, keeping the temperature below -70 °C. The reaction mixture was stirred at -78 °C for 1 hour, at which point the reaction was quenched by aqueous ammonium chloride (10 mL) at -78 °C with vigorous stirring. The reaction mixture was then diluted with EtOAc (15 mL), and the aqueous phase was extracted with EtOAc (3 × 25 mL). The combined organics were dried with sodium sulfate and concentrated to a residue, which was purified via column chromatography (SiO₂; Gradient: 100% DCM - 100% EtOAc) to give (4S)-7-bromo-1-((6-chloro-5-(hydroxymethyl)-2-(methylthio)pyrimidin-4-yl)methyl)-4-methyl-1,2,3,4-tetrahydronaphthalen-1-ol (405 mg).

¹H NMR (400 MHz, CDCl₃) δ 7.67 (m, 1H), 7.36 (m, 1H), 7.11 (m, 1H), 4.81 - 4.67 (m, 2H), 3.40 - 3.16 (m, 2H), 2.93 (m, 1H), 2.55 (m, 3H), 2.18 (m, 1H), 2.06 - 1.92 (m, 2H), 1.91 - 1.62 (m, 2H), 1.32 (m, 3H).

### Step 2: (4S)-7-Bromo-4'-chloro-4-methyl-2'-(methylthio)-3,4,5',8'-tetrahydro-2H-spiro[naphthalene-1,7'-pyrano[4,3-d]pyrimidine] - two resolved diastereomers

Phosphoric acid (658 µL, 85 wt%, 1 *equiv.,* 9.62 mmol) was added to (4.5)-7-bromo-1-((6-chloro-5-(hydroxymethyl)-2-(methylthio)pyrimidin-4-yl)methyl)-4-methyl-1,2,3,4-tetrahydronaphthalen-1-ol (4.27 g, *1 equiv.,* 9.62 mmol) in toluene (19.2 mL). The mixture was refluxed for 2 hours. Water (50 mL) and EtOAc (50 mL) were added, and the aqueous layer was extracted with EtOAc (3 x 50 mL). The combined organics were dried with sodium sulfate and concentrated to a residue, which was purified via chiral SFC (Column: Chiralpak IB-U (3.0 × 100 mm), 1.6 µm, P/N 81U83, flow rate: 1.2 mL/min, Mobile phase: (Methanol with 0.25% DEA)) to afford the two diastereomers of (4*S*)-7-bromo-4'-chloro-4-methyl-2'-(methylthio)-3,4,5',8'-tetrahydro-2*H*-spiro[naphthalene-1,7'-pyrano[4,3-*d*]pyrimidine]: (1*S*,4*S*)-7 -bromo-4'-chloro-4-methyl-2'-(methylthio)-3,4,5',8'-tetrahydro-2*H*-spiro[naphthalene-1,7'-pyrano[4,3-*d*]pyrimidine] **Peak 1** (2.02 g) and (1*R*,4*S*)-7-bromo-4'-chloro-4-methyl-2'-(methylthio)-3,4,5',8'-tetrahydro-2H-spiro[naphthalene-1,7'-pyrano[4,3-d]pyrimidine] **Peak 2** (1.78 g).
**Peak 1:** t_{R} = 1.34 minutes (Method G; 30% MeOH with 0.25% DEA)
LCMS: ESI [M+H]⁺ 427.0 m/z; t_{R} = 4.14 minutes (Method E)
**Peak 2:** t_{R} = 2.06 minutes (Method G; 30% MeOH with 0.25% DEA)
¹H NMR (400 MHz, CDCl₃) δ 7.63 (d, 1H), 7.41 (dd, 1H), 7.19 (d, 1H), 4.78 (q, 2H), 3.12 (q, 2H), 3.00 - 2.86 (m, 1H), 2.59 (s, 3H), 2.13 - 1.87 (m, 3H), 1.48 (qd, 1H), 1.34 (d, 3H).

### Step 3: 5-((1S,4S)-7-Bromo-4-methyl-2'-(methylthio)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide

DIPEA (2.31 mL, 13.3 mmol) was added to (1*S*,4*S*)-7-bromo-4'-chloro-4-methyl-2'-(methylthio)-3,4,5',8'-tetrahydro-2*H*-spiro[naphthalene-1,7'-pyrano[4,3-*d*]pyrimidine] (Peak 1) (2.02 g, 1 *equiv.,* 3.32 mmol) and *N,N*-dimethyl-5,6,7,8-tetrahydro-4*H*-pyrazolo[1,5-*a*][1,4]diazepine-2-carboxamide, 2HCl (1.74 g, 1.5 *equiv.,* 4.98 mmol) in ethanol (7 mL). The reaction mixture was heated to reflux for 3 hours. *N,N*-dimethyl-5,6,7,8-tetrahydro-4*H-*pyrazolo[1,5-*a*][1,4]diazepine-2-carboxamide (484 mg, 0.7 *equiv.,* 2.32 mmol) and DIPEA (810 µL, 1.4 *equiv.,* 4.65 mmol) were then delivered sequentially. The reaction mixture was heated to reflux for an additional 45 minutes and concentrated under reduced pressure to an oil that was partitioned between water (50 mL) and EtOAc (50 mL). The aqueous layer was extracted with EtOAc (3 × 50 mL). The combined organics were dried over sodium sulfate and concentrated to a residue, which was purified by flash chromatography (SiO₂; Gradient: 0-100% EtOAc in DCM then switched to 5% MeOH in DCM, 40 g column) to give 5-((1*S*,4*S*)-7-bromo-4-methyl-2'-(methylthio)-3,4,5',8'-tetrahydro-2*H*-spiro[naphthalene-1,7'-pyrano[4,3-*d*]pyrimidin]-4'-yl)-*N*,*N*-dimethyl-5,6,7,8-tetrahydro-4*H*-pyrazolo[1,5-*a*][1,4]diazepine-2-carboxamide (2.44 g).
LCMS: ESI [M+H]⁺ 597.3 m/z; t_{R} = 2.97 minutes (Method E)

### Step 4: tert-Butyl ((1S,4S)-4'-(2-(dimethylcarbamoyl)-7,8-dihydro-4H-pyrazolo [1,5-a][1,4]diazepin-5(6H)-yl)-4-methyl-2'-(methylthio)-3,4,5',8'-tetrahydro-2H-spiro[naphthalene-1,7'-pyrano[4,3-d]pyrimidin]-7-yl)carbamate

5-((1*S*,4*S*)-7-Bromo-4-methyl-2'-(methylthio)-3,4,5',8'-tetrahydro-2*H-*spiro[naphthalene-1,7'-pyrano[4,3-*d*]|pyrimidin]-4'-yl)-*N*,*N*-dimethyl-5,6,7,8-tetrahydro-4*H-*pyrazolo[1,5-*a*][1,4]diazepine-2-carboxamide (2.44 g, 1 *equiv.,* 2.98 mmol) was added to BrettPhos Pd G4 (411 mg, 0.15 *equiv.,* 0.45 mmol), tert-butyl carbamate (1.75 g, 5 *equiv.*, 14.9 mmol), and cesium carbonate (2.91 g, 3 *equiv.,* 8.94 mmol) in 1,4-dioxane (40 mL) and sparged for 10 minutes with nitrogen. The reaction mixture was heated to 95 °C for 16 hours and then cooled to room temperature, filtered, and concentrated to a residue, which was purified by flash chromatography (SiO₂ 220 g: Gradient: 100% DCM to 100% EtOAc then switched to MeOH and ramped from 0-7.5%) to afford *tert*-butyl ((1*S*,4*S*)-4'-(2-(dimethylcarbamoyl)-7,8-dihydro-4*H*-pyrazolo[1,5-*a*][1,4]diazepin-5(6*H*)-yl)-4-methyl-2'-(methylthio)-3,4,5',8'-tetrahydro-2*H*-spiro[naphthalene-1,7'-pyrano[4,3-*d*]pyrimidin]-7-yl)carbamate (1.61 g).
LCMS: ESI [M+H]⁺ 634.3 m/; t_{R} = 2.83 minutes (Method E)

### Step 5: tert-Butyl ((1S,4S)-4'-(2-(dimethylcarbamoyl)-7,8-dihydro-4H-pyrazolo[1,5-a] [1,4]diazepin-5(6H)-yl)-4-methyl-2'-(methylsulfonyl)-3,4,5',8'-tetrahydro-2H-spiro[naphthalene-1,7'-pyrano[4,3-d]pyrimidin]-7-yl)carbamate

*mCPBA* (1.67 g, 70 wt%, 3 *equiv.,* 6.77 mmol) was added to a solution of *tert*-butyl ((1*S*,4*S*)-4'-(2-(dimethylcarbamoyl)-7,8-dihydro-4*H*-pyrazolo[1,5-*a*][1,4]diazepin-5(6*H*)-yl)-4-methyl-2'-(methylthio)-3,4,5',8'-tetrahydro-2*H*-spiro[naphthalene-1,7'-pyrano[4,3-*d*]pyrimidin]-7-yl)carbamate (1.57 g, 1 *equiv.,* 2.25 mmol) in DCM (45 mL) at 0 °C. The reaction mixture was stirred for 10 minutes at 0 °C, removed from the ice bath, and warmed to room temperature over 55 minutes. The reaction mixture was diluted with DCM (50 mL) and quenched with sodium thiosulfate (15 mL) and saturated sodium bicarbonate (125 mL). The resulting biphasic mixture was stirred for 30 minutes. The aqueous was separated and extracted with DCM (120 mL). The combined organics were washed with saturated sodium carbonate (2 x 200 mL), dried over sodium sulfate, and concentrated to afford *tert*-butyl ((1*S*,4*S*)-4'-(2-(dimethylcarbamoyl)-7,8-dihydro-4*H*-pyrazolo[1,5-*a*][1,4]diazepin-5(6*H*)-yl)-4-methyl-2'-(methylsulfonyl)-3,4,5',8'-tetrahydro-2*H*-spiro[naphthalene-1,7'-pyrano[4,3-*d*]pyrimidin]-7-yl)carbamate (1.76 g).
LCMS: ESI [M+H]⁺ 666.2 m/z; t_{R} = 2.91 minutes (Method E)

### Step 6: tert-Butyl ((1S,4S)-4'-(2-(dimethylcarbamoyl)-7,8-dihydro-4H-pyrazolo[1,5-a][1,4]diazepin-5(6H)-yl)-2'-(((2R,7aS)-2-fluorotetrahydro-1H-pyrrolizin-7a(5H)-yl)methoxy)-4-methyl-3,4,5',8'-tetrahydro-2H-spiro[naphthalene-1,7'-pyrano[4,3-d]pyrimidin]-7-yl)carbamate

Sodium *tert*-butoxide (221 mg, 1.15 mL, 2 M, 1.21 *equiv.,* 2.30 mmol) was added dropwise to a solution of *tert*-butyl ((1*S*,4*S*)-4'-(2-(dimethylcarbamoyl)-7,8-dihydro-4*H-*pyrazolo[1,5-*a*][1,4]diazepin-5(6*H*)-yl)-4-methyl-2'-(methylsulfonyl)-3,4,5',8'-tetrahydro-2*H-*spiro[naphthalene-1,7'-pyrano[4,3-*d*]pyrimidin]-7-yl)carbamate (1.27 g, 1 *equiv.*, 1.91 mmol) and ((2*R*,7a*S*)-2-fluorotetrahydro-1*H*-pyrrolizin-7a(5*H*)-yl)methanol (303 mg, 1 *equiv.*, 1.91 mmol) in THF at 0 °C. After 10 minutes, sodium 2-methylpropan-2-olate (550 µL, 2 M, 0.58 *equiv.,* 1.10 mmol) was added dropwise. The resulting reaction mixture was stirred for 10 minutes and then quenched with HCl (1.8 mL, 2 M in diethyl ether, 1.9 *equiv.,* 3.6 mmol). The mixture was stirred for 5 minutes and then concentrated to afford *tert*-butyl ((1*S*,4*S*)-4'-(2-(dimethylcarbamoyl)-7,8-dihydro-4*H*-pyrazolo[1,5-*a*][1,4]diazepin-5(6*H*)-yl)-2'-(((2*R*,7a*S*)-2-fluorotetrahydro-1*H*-pyrrolizin-7a(5*H*)-yl)methoxy)-4-methyl-3,4,5',8'-tetrahydro-2*H-*spiro[naphthalene-1,7'-pyrano[4,3-*d*]pyrimidin]-7-yl)carbamate (2.05 g).
LCMS: ESI [M+H]⁺ 745.4 m/z; t_{R} = 2.09 minutes. (Method E)

### Step 7: 5-((1S,4S)-7-Amino-2'-(((2R,7aS)-2-fluorotetrahydro-1H-pyrrolizin-7a(5H)-yl)methoxy)-4-methyl-3,4,5',8'-tetrahydro-2H-spiro [naphthalene- 1,7'-pyrano[4,3-d)pyrimidin]-4'-yl)-N,N-dimethyl-5,6,7,8-tetrahydro-4H-pyrazolo[1,5-a][1,4]diazepine-2-carboxamide

Hydrogen chloride (15.9 mL, 2 M in diethyl ether, 15 *equiv.,* 31.8 mmol) was added to tert-butyl ((1*S*,4*S*)-4'-(2-(dimethylcarbamoyl)-7,8-dihydro-4*H*-pyrazolo[1,5-*a*][1,4]diazepin-5(6*H*)-yl)-2'-(((2*R*,7a*S*)-2-fluorotetrahydro-1*H*-pyrrolizin-7a(5*H*)-yl)methoxy)-4-methyl-3,4,5',8'-tetrahydro-2*H*-spiro[naphthalene-1,7'-pyrano[4,3-*d*]pyrimidin]-7-yl)carbamate (2.05 g, 1 *equiv.,* 2.12 mmol) in DCM (10.6 mL) and stirred for 2 hours. The reaction mixture was concentrated to a residue followed by the addition of DCM (30 mL). Sodium hydroxide (8.3 mL, 2 M, 16.6 mmol) was added, and the mixture was extracted with DCM (10 x). The combined organics were dried over sodium sulfate and concentrated to a residue, which was purified by SFC (Column: Torus 2-PIC (19 × 100 mm, 5 µm, PN 186008586) Mobile phase: MeOH with 0.25% DEA; Gradient: 5% to 40% at 0.6 mL/min over 10 minutes; backpressure = 120 bar) to give 5-((1*S*,4*S*)-7-amino-2'-(((2*R*,7a*S*)-2-fluorotetrahydro-1*H*-pyrrolizin-7a(5*H*)-yl)methoxy)-4-methyl-3,4,5',8'-tetrahydro-2*H*-spiro[naphthalene-1,7'-pyrano[4,3-*d*]pyrimidin]-4'-yl)-*N*,*N*-dimethyl-5,6,7,8-tetrahydro-4*H*-pyrazolo[1,5-*a*][1,4]diazepine-2-carboxamide (776 mg).
LCMS: ESI [M+H]⁺ 645.3 m/z; t_{R} = 1.44 minutes. (Method E)
¹H NMR (400 MHz, CD₃OD) δ 7.02 (m, 1H), 6.76 - 6.65 (m, 2H), 6.61 (s, 1H), 5.39 - 5.12 (m, 1H), 4.93 - 4.71 (m, 3H), 4.65 (m, 1H), 4.58 - 4.45 (m, 2H), 4.14 - 4.01 (m, 2H), 3.96 (m, 2H), 3.32 (s, 3H), 3.27 - 3.12 (m, 3H), 3.08 (s, 3H), 3.06 - 2.95 (m, 2H), 2.87 (s, 2H), 2.30 - 2.02 (m, 6H), 2.03 - 1.69 (m, 6H), 1.29 (m, 3H).
¹⁹F NMR (376 MHz, CD₃OD) δ -169.56, -176.41 (m).

### Example 19: 5-(6'-Amino-2-(((2R,7aS)-2-fluorotetrahydro-1H-pyrrolizin-7a(5H)-yl)methoxy)-2',3',5,8-tetrahydro-1'H-spiro[pyrano[4,3-d]pyrimidine-7,4'-[1,3]methanonaphthalen]-4-yl)-N,N-dimethyl-5,6,7,8-tetrahydro-4H-pyrazolo[1,5-a][1,4]diazepine-2-carboxamide (Compound R172a)

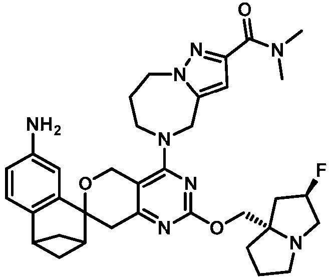

### Step 1: 6-Bromo-4-((6-chloro-5-(hydroxymethyl)-2-(methylthio)pyrimidin-4-yl)methyl)-1,2,3,4-tetrahydro-1,3-methanonaphthalen-4-ol

LDA solution (11.1 mL, 1 M, 2.3 *equiv.,* 11.1 mmol) was added to a nitrogen purged solution of (4-chloro-6-methyl-2-(methylthio)pyrimidin-5-yl)methanol (988 mg, 1 *equiv.,* 4.83 mmol) in THF (12.7 mL) at -78 °C. The internal temperature was maintained below -74 °C. The resulting orange solution was stirred for 15 minutes and then 6-bromo-2,3-dihydro-1,3-methanonaphthalen-4(1*H*)-one (1.26 g, 1.1 *equiv.,* 5.31 mmol) was added dropwise to the solution, keeping the reaction temperature below -78 °C. The reaction mixture was stirred at - 78 °C for 45 minutes and then quenched with HCl (4 mL, 4 M in 1,4-dioxane), keeping the temperature below -50 °C. Sodium bicarbonate was then added until pH = ~7 was achieved, and the mixture was extracted with DCM (3 x 25 mL). The organic layer was then concentrated to a residue, which was washed with MeOH to give 6-bromo-4-((6-chloro-5-(hydroxymethyl)-2-(methylthio)pyrimidin-4-yl)methyl)-1,2,3,4-tetrahydro-1,3-methanonaphthalen-4-ol (1.33 g), which was used without further purification.

¹H NMR (400 MHz, CDCl₃) δ 7.62 (d, 1H), 7.24 (dd, 1H), 6.87 (d, 1H), 4.84 - 4.67 (m, 2H), 3.51 (d, 1H), 3.31 (d, 1H), 3.03 (q, 1H), 2.54 - 2.42 (m, 5H), 2.35 - 2.25 (m, 1H), 1.88 - 1.76 (m, 1H), 1.70 - 1.57 (m, 1H).

### Step 2: 6'-Bromo-4-chloro-2-(methylthio)-2',3',5,8-tetrahydro-1'H-spiro[pyrano[4,3-d]pyrimidine-7,4'-[1,3]methanonaphthalene]

A solution of 6-bromo-4-((6-chloro-5-(hydroxymethyl)-2-(methylthio)pyrimidin-4-yl)methyl)-1,2,3,4-tetrahydro-1,3-methanonaphthalen-4-ol (1.33 g, 1 *equiv.,* 3.0 mmol), phosphoric acid (346 mg, 85 wt%, 1 *equiv.,* 3.0 mmol), and toluene (6.0 mL) was stirred at 100 °C for 1 hour. The reaction mixture was concentrated to give a cloudy oil, to which MeOH (4 mL) was added. The resulting suspension was filtered to afford 6'-bromo-4-chloro-2-(methylthio)-2',3',5,8-tetrahydro-1'*H*-spiro[pyrano[4,3-*d*]pyrimidine-7,4'-[1,3]methanonaphthalene] (940 mg).

¹H NMR (400 MHz, CDCl₃) δ 7.52 (d, 1H), 7.26 (dd, 1H), 6.88 (d, 1H), 4.74 (d, 1H), 4.50 (d, 1H), 3.32 (d, 1H), 3.08 (q, 1H), 2.96 (d, 1H), 2.70 (q, 1H), 2.56 - 2.45 (m, 4H), 2.41 - 2.26 (m, 1H), 1.82 (t, 1H), 1.58 (dd, 1H).

### Step 3: 5-(6'-Bromo-2-(methylthio)-2',3',5,8-tetrahydro-1'H-spiro|[pyrano[4,3-d)pyrimidine-7,4'-[1,3]methanonaphthalen]-4-yl)-N,N-dimethyl-5,6,7,8-tetrahydro-4H-pyrazolo[1,5-a][1,4]diazepine-2-carboxamide

DIPEA (1.93 mL, 5 *equiv.,* 11.1 mmol) was added to 6'-bromo-4-chloro-2-(methylthio)-2',3',5,8-tetrahydro-1'*H*-spiro[pyrano[4,3-*d*]pyrimidine-7,4'-[1,3]methanonaphthalene] (940 mg, 1 *equiv.,* 2.2 mmol) and *N,N*-dimethyl-5,6,7,8-tetrahydro-4*H*-pyrazolo[1,5-*a*][1,4]diazepine-2-carboxamide (693 mg, 1.5 *equiv.,* 3.33 mmol) in ethanol (8.9 mL). The mixture was stirred at reflux for 16 hours. The mixture was concentrated to a viscous oil, and EtOAc (20 mL) was added. The solution was washed with saturated aqueous ammonium chloride (20 mL). The organic layer was concentrated to a residue, which was purified by flash chromatography (100% DCM to 90:10 DCM:MeOH) to afford 5-(6'-bromo-2-(methylthio)-2',3',5,8-tetrahydro-1'*H*-spiro[pyrano[4,3-*d*]pyrimidine-7,4'-[1,3]methanonaphthalen]-4-yl)-*N,N*-dimethyl-5,6,7,8-tetrahydro-4*H*-pyrazolo[1,5-*a*][1,4]diazepine-2-carboxamide (1.04 g).
LCMS: ESI [M+H]⁺ 595.2 m/z; t_{R} = 1.87 minutes (Method C)

### Step 4: tert-Butyl (4-(2-(dimethylcarbamoyl)-7,8-dihydro-4H-pyrazolo[1,5-a][1,4]diazepin-5(6H)-yl)-2-(methylthio)-2',3',5,8-tetrahydro-1'H-spiro[pyrano[4,3-d]pyrimidine-7,4'-[1,3]methanonaphthalen]-6'-yl)carbamate

BrettPhos Pd G4 (241 mg, 0.15 *equiv.,* 0.26 mmol) was added to a degassed suspension of 5-(6'-bromo-2-(methylthio)-2',3',5,8-tetrahydro-1'*H*-spiro[pyrano[4,3-*d*]pyrimidine-7,4'-[1,3]methanonaphthalen]-4-yl)-*N,N*-dimethyl-5,6,7,8-tetrahydro-4*H*-pyrazolo[1,5-*a*][1,4]diazepine-2-carboxamide (1.04 g, 1 *equiv.,* 1.75 mmol), *tert*-butyl carbamate (1.03 g, 5 *equiv.,* 8.75 mmol), and cesium carbonate (1.71 g, 3 *equiv.,* 5.25 mmol) in 1,4-dioxane (7.0 mL). The mixture was stirred at 100 °C for 3 hours. The reaction mixture was cooled to room temperature, filtered, and concentrated to a residue which was purified by flash chromatography (SiO₂, 0 to 10% MeOH in DCM), to afford *tert*-butyl (4-(2-(dimethylcarbamoyl)-7,8-dihydro-4*H*-pyrazolo[1,5-*a*][1,4]diazepin-5(6*H*)-yl)-2-(methylthio)-2',3',5,8-tetrahydro-1'*H*-spiro[pyrano[4,3-*d*]pyrimidine-7,4'-[1,3]methanonaphthalen]-6'-yl)carbamate (834 mg).
LCMS: ESI [M+H]⁺ 632.3 m/z; t_{R} = 1.78 minutes. (Method C)

### Step 5: tert-Butyl (4-(2-(dimethylcarbamoyl)-7,8-dihydro-4H-pyrazolo[1,5-a][1,4]diazepin-5(6H)-yl)-2-(methylsulfonyl)-2',3',5,8-tetrahydro-1'H-spiro[pyrano[4,3-d]pyrimidine-7,4'-[1,3]methanonaphthalen]-6'-yl)carbamate

*mCPBA* (937 mg, 70 wt%, 3 *equiv.,* 3.8 mmol) was added to a solution of *tert*-butyl (4-(2-(dimethylcarbamoyl)-7,8-dihydro-4*H*-pyrazolo[1,5-*a*][1,4]diazepin-5(6*H*)-yl)-2-(methylthio)-2',3',5,8-tetrahydro-1'*H*-spiro[pyrano[4,3-*d*]pyrimidine-7,4'-[1,3]methanonaphthalen]-6'-yl)carbamate (834 mg, 1 *equiv.*, 1.27 mmol) in DCM (25 mL) at 0 °C. The reaction mixture was stirred for 10 minutes at 0 °C and then stirred at room temperature for 55 minutes. The mixture was diluted with DCM (50 mL) and quenched with aqueous sodium thiosulfate (15 mL) and saturated aqueous sodium bicarbonate (135 mL). The resulting biphasic mixture was stirred for 30 minutes, and the aqueous layer was extracted with DCM (2 x 20 mL). The combined organics were washed with aqueous sodium hydroxide (1 M, 2 x 20 mL), dried over sodium sulfate, and concentrated under reduced pressure to give *tert*-butyl (4-(2-(dimethylcarbamoyl)-7,8-dihydro-4*H*-pyrazolo[1,5-*a*][1,4]diazepin-5(6*H*)-yl)-2-(methylsulfonyl)-2',3',5,8-tetrahydro-1'H-spiro[pyrano[4,3-*d*]pyrimidine-7,4'-[1,3]methanonaphthalen]-6'-yl)carbamate (822 mg).
LCMS: ESI [M+H]⁺ 664.7 m/z; t_{R} = 2.77 minutes. (Method E)

### Step 6: 5-(6'-Amino-2-(((2R,7aS)-2-fluorotetrahydro-1H-pyrrolizin-7a(5H)-yl)methoxy)-2',3',5,8-tetrahydro-1'H-spiro[pyrano[4,3-d]pyrimidine-7,4'-[1,3]methanonaphthalen]-4-yl)-N,N-dimethyl-5,6,7,8-tetrahydro-4H-pyrazolo[1,5-a][1,4]diazepine-2-carboxamide

NaH (50 mg, 60 wt%, 1 *equiv.,* 1.24 mmol) was added to *tert*-butyl (4-(2-(dimethylcarbamoyl)-7,8-dihydro-4*H*-pyrazolo[1,5-*a*][1,4]diazepin-5(6*H*)-yl)-2-(methylsulfonyl)-2',3',5,8-tetrahydro-1'H-spiro[pyrano[4,3-*d*]pyrimidine-7,4'-[1,3]methanonaphthalen]-6'-yl)carbamate (822 mg, 1 *equiv.,* 1.24 mmol) and ((2*R*,7a*S*)-2-fluorotetrahydro-1*H*-pyrrolizin-7a(5*H*)-yl)methanol (197 mg, 1 *equiv.*, 1.24 mmol) in THF at 0°C. The reaction mixture was stirred for 10 minutes, quenched with saturated aqueous ammonium chloride (2 mL), and diluted with EtOAc (5 mL). The aqueous layer was extracted with EtOAc (2 x 20 mL). The combined organics were dried over sodium sulfate and then concentrated to give a residue, which was purified by flash chromatography (SiO₂, 0% to 20% MeOH in DCM) to afford a white foam (750 mg). The foam was dissolved in DCM (3 mL) and HCl (2.5 mL, 2 M in diethyl ether, 5.0 mmol) was added. The mixture was stirred for 1 hour, and the resulting suspension was filtered to provide 5-(6'-amino-2-(((2*R*,7a*S*)-2-fluorotetrahydro-1*H*-pyrrolizin-7a(5*H*)-yl)methoxy)-2',3',5,8-tetrahydro-1'H-spiro[pyrano[4,3-*d*]pyrimidine-7,4'-[1,3]methanonaphthalen]-4-yl)-*N,N*-dimethyl-5,6,7,8-tetrahydro-4*H*-pyrazolo[1,5-*a*][1,4]diazepine-2-carboxamide (409 mg).
LCMS: ESI [M+H]⁺ 643.3 m/z; t_{R} = 1.13 minutes. (Method C)
¹H NMR (400 MHz, MeOD) δ 7.56 (d, 1H), 7.33 - 7.20 (m, 2H), 6.76 (s, 1H), 5.61 (d, 1H), 5.25 - 5.04 (m, 2H), 5.04 - 4.94 (m, 1H), 4.90 - 4.81 (m, 3H), 4.82 - 4.71 (m, 1H), 4.72 - 4.47 (m, 2H), 4.28 - 4.08 (m, 2H), 4.08 - 3.78 (m, 3H), 3.54 - 3.42 (m, 2H), 3.42 - 3.33 (m, 5H), 3.26 (s, 1H), 3.19 - 2.99 (m, 4H), 2.84 - 2.61 (m, 2H), 2.61 - 2.46 (m, 1H), 2.43 - 2.29 (m, 3H), 2.29 - 2.14 (m, 2H), 1.86 (t, 1H), 1.69 (t, 1H).

### Example 20: (S)-2'-(((2R,7aS)-2-Fluorotetrahydro-1H-pyrrolizin-7a(5H)-yl)methoxy)-4'-morpholino-3,4,5',8'-tetrahydro-2H-spiro[naphthalene-1,7'-pyrano[4,3-d]pyrimidin]-7-amine (Compound 159a)

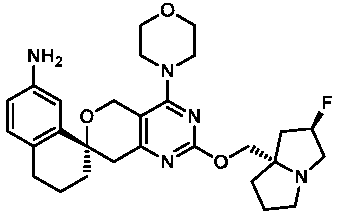

### Step 1: (S)-7-Bromo-2'-(methylthio)-4'-morpholino-3,4,5',8'-tetrahydro-2H-spiro[naphthalene-1,7'-pyrano[4,3-d]pyrimidine]

DIPEA (110 mg, 148 µL, 2 *equiv.,* 850 µmol) was added to (*S*)-7-bromo-4'-chloro-2'-(methylthio)-3,4,5',8'-tetrahydro-2*H*-spiro[naphthalene-1,7'-pyrano[4,3-*d*]pyrimidine] (175 mg, 1 *equiv.*, 0.43 mmol) and morpholine (74 mg, 2 *equiv.,* 0.85 mmol) in ethanol (850 µL). The mixture was stirred under reflux for 16 hours. The reaction mixture was then concentrated to a residue, which was purified by flash chromatography (SiO₂) to afford (S)-7-bromo-2'-(methylthio)-4'-morpholino-3,4,5',8'-tetrahydro-2*H*-spiro[naphthalene-1,7'-pyrano[4,3-*d*]pyrimidine] (185 mg).
LCMS: ESI [M+H]⁺ 463.8 m/z; t_{R} = 1.99 minutes. (Method C)

### Step 2: tert-Butyl (S)-(2'-(methylthio)-4'-morpholino-3,4,5',8'-tetrahydro-2H-spiro[naphthalene-1,7'-pyrano[4,3-d]pyrimidin]-7-yl)carbamate

Pd(OAc)₂ (9 mg, 0.10 *equiv.,* 0.04 mmol) was added to a solution of *tert*-butyl carbamate (94 mg, 2.0 *equiv.,* 0.80 mmol), (*S*)-7-bromo-2'-(methylthio)-4'-morpholino-3,4,5',8'-tetrahydro-2*H*-spiro[naphthalene-1,7'-pyrano[4,3-*d*]pyrimidine] (185 mg, 1.0 *equiv.,* 0.400 mmol), and 2-(dicyclohexylphosphanyl)-2',4',6'-tris(isopropyl)biphenyl (57 mg, 0.30 *equiv.,* 0.12 mmol) in 1,4-dioxane (3.2 mL). The reaction mixture was stirred at 100 °C for 12 hours, and then BrettPhos Pd G4 (10 mg) was added. The reaction mixture was stirred for an additional 6 hours. The reaction mixture was concentrated and washed with 90:10 heptanes:EtOAc to afford a solid, which was purified via flash chromatography (SiO₂) to provide *tert*-butyl (*S*)-(2'-(methylthio)-4'-morpholino-3,4,5',8'-tetrahydro-2*H-*spiro[naphthalene-1,7'-pyrano[4,3-*d*]pyrimidin]-7-yl)carbamate (230 mg).
LCMS: ESI [M+H]⁺ 499.8 m/z; t_{R} = 1.86 minutes. (Method C)

### Step 3: tert-Butyl (S)-(2'-(methylsulfonyl)-4'-morpholino-3,4,5',8'-tetrahydro-2H-spiro[naphthalene-1,7'-pyrano[4,3-d]pyrimidin]-7-yl)carbamate

*mCPBA* (223 mg, 3 *equiv.,* 1.29 mmol) was added to *tert*-butyl (*S*)-(2'-(methylthio)-4'-morpholino-3,4,5',8'-tetrahydro-2*H*-spiro[naphthalene-1,7'-pyrano[4,3-*d*]pyrimidin]-7-yl)carbamate (215 mg, *1 equiv.,* 0.431 mmol) in DCM (8.6 mL), and the resulting mixture was stirred at room temperature until complete conversion of the starting material was observed. The reaction mixture was diluted with saturated aqueous sodium bicarbonate followed by saturated aqueous sodium thiosulfate (0.5 mL). The reaction mixture was stirred for an additional 30 minutes and then extracted with DCM (3 x). The combined organics were concentrated under reduced pressure to provide *tert*-butyl (*S*)-(2'-(methylsulfonyl)-4'-morpholino-3,4,5',8'-tetrahydro-2*H*-spiro[naphthalene-1,7'-pyrano[4,3-*d*]pyrimidin]-7-yl)carbamate (133 mg).
LCMS: ESI [M+H]⁺ 531.4 m/z; t_{R} = 1.88 minutes. (Method C)

### Step 4: (S)-2'-(((2R,7aS)-2-Fluorotetrahydro-1H-pyrrolizin-7a(5H)-yl)methoxy)-4'-morpholino-3,4,5',8'-tetrahydro-2H-spiro[naphthalene-1,7'-pyrano[4,3-d]pyrimidin]-7-amine

NaH (10 mg, 60 wt%, 1 *equiv.*, 0.25 mmol) was added to a solution of ((2*R*,7a*S*)-2-fluorotetrahydro-1*H*-pyrrolizin-7a(5*H*)-yl)methanol (40 mg, 1 *equiv.*, 0.25 mmol) in THF (2.5 mL) at 0 °C. The mixture was stirred for 30 minutes, followed by the addition of tert-butyl (S)-(2'-(methylsulfonyl)-4'-morpholino-3,4,5',8'-tetrahydro-2*H*-spiro[naphthalene-1,7'-pyrano[4,3-*d*]pyrimidin]-7-yl)carbamate (133 mg, 1 *equiv.*, 0.251 mmol) in THF (1.0 mL) dropwise. The reaction mixture was stirred for 10 minutes. Water (10 mL) was then added, and the solution was extracted with EtOAc (3 x). The combined organics were concentrated to a residue, which was purified by flash chromatography (SiO₂) to afford the desired protected product (60 mg). The material was then dissolved in DCM, and HCl (2 M in diethyl ether) was added. The reaction mixture was stirred for 1 hour and then concentrated under reduced pressure to provide (*S*)-2'-(((2*R*,7a*S*)-2-fluorotetrahydro-1*H*-pyrrolizin-7a(5*H*)-yl)methoxy)-4'-morpholino-3,4,5',8'-tetrahydro-2 H-spiro[naphthalene-1,7'-pyrano[4,3-*d*]pyrimidin]-7-amine (40 mg).
LCMS: ESI [M+H]⁺ 510.3 m/z; t_{R} = 1.06 minutes. (Method C)
¹H NMR (400 MHz, MeOD) δ 7.51 (s, 1H), 7.31 (d, 1H), 7.23 (d, 1H), 5.56 (d, 1H), 4.93 (s, 2H), 4.64 (s, 2H), 3.89 (d, 10H), 3.42 (s, 1H), 3.27 (s, 6H), 3.10 (s, 1H), 2.88 (s, 2H), 2.60 (s, 1H), 2.50 - 1.95 (m, 6H), 1.85 (s, 1H).

### Example 21: 5-(6'-Amino-5'-cyano-2-(((2R,7aS)-2-fluorotetrahydro-1H-pyrrolizin-7a(5H)-yl)methoxy)-2',3',5,8-tetrahydro-1'H-spiro[pyrano[4,3-d]pyrimidine-7,4'-[1,3]methanonaphthalen]-4-yl)-N,N-dimethyl-5,6,7,8-tetrahydro-4H-pyrazolo[1,5-a][1,4]diazepine-2-carboxamide (Compound 504b)

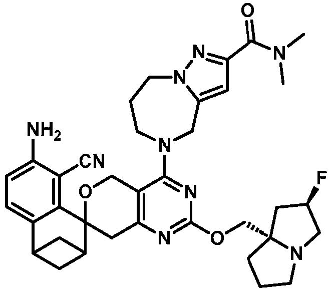

### Step 1: 6-Amino-2,3-dihydro-1,3-methanonaphthalen-4(1H)-one

To a pressure vessel charged with 6-bromo-2,3-dihydro-1,3-methanonaphthalen-4(1*H*)-one (6.61 g, 1.00 *equiv.,* 27.9 mmol) and copper(I) oxide (399 mg, 0.10 *equiv.,* 2.79 mmol), were added NMP (18.6 mL) and ammonium hydroxide (19.5 g, 21.6 mL, 20 *equiv.,* 558 mmol) under nitrogen. The pressure vessel was rapidly sealed with a teflon screw cap, and the resulting mixture was stirred at 80 °C for 17 hours. The reaction mixture was cooled to room temperature and poured in a separatory funnel charged with water (60 mL) and EtOAc (60 mL). The layers were separated, and the aqueous phase was extracted with EtOAc (x 3). The combined organic extracts were dried over sodium sulfate, filtered, and concentrated to give the crude product which was purified by flash column chromatography (gradient elution from 2:98-EtOAc:Heptane to 50:50-EtOAc:Heptane) to afford 6-amino-2,3-dihydro-1,3-methanonaphthalen-4(1*H*)-one (3.90 g), as a yellow solid.
LCMS: m/z (ESI) [M+H]⁺ 174.2, t_{R} = 1.07 minutes (Method C)
¹H NMR (400 MHz, CDCl₃) δ 7.33 - 7.28 (m, 1H), 7.04 - 6.99 (m, 1H), 6.81 - 6.74 (m, 1H), 3.19 (m, 2H), 2.90 (m, 2H), 2.35 - 2.26 (m, 2H).

### Step 2: 6-Amino-5-bromo-2,3-dihydro-1,3-methanonaphthalen-4(1H)-one

To a solution of 6-amino-2,3-dihydro-1,3-methanonaphthalen-4(1*H*)-one (3.40 g, 1 *equiv.,* 19.6 mmol) in DMF (32.7 mL) at 0 °C, was added a solution of *N*-bromosuccinimide (3.49 g, 1.00 *equiv.,* 19.6 mmol) in DMF (16.4 mL) dropwise under nitrogen. The resulting mixture was stirred at 0 °C for 5 minutes and at room temperature for 1 hour. The reaction mixture was quenched by the addition of water and diluted with EtOAc. The two layers were separated and the aqueous phase was extracted with EtOAc (x 3). The combined organic extracts were dried over sodium sulfate, filtered, and concentrated to give the crude product which was purified by flash column chromatography (SiO₂, gradient elution from 0:100-EtOAc:Heptane to 15:85-EtOAc:Heptane to 25:75-EtOAc:Heptane) to give 6-amino-5-bromo-2,3-dihydro-1,3-methanonaphthalen-4(1H)-one (2.60 g) as a yellow oil.
LCMS: m/z (ESI) [M+H]⁺ 252.0, t_{R} = 1.67 minutes (Method C)
¹H NMR (400 MHz, CDCl₃) δ 6.98 (d, 1H), 6.83 (d, 1H), 4.25 (s, 2H), 3.28 - 3.18 (m, 2H), 2.87 - 2.76 (m, 2H), 2.31 - 2.24 (m, 2H).

### Step 3: 5-Bromo-6-(dibenzylamino)-2,3-dihydro-1,3-methanonaphthalen-4(1H)-one

To a solution of 6-amino-5-bromo-2,3-dihydro-1,3-methanonaphthalen-4(1*H*)-one (2.60 g, 1.0 *equiv.,* 10.3 mmol) in MeCN (78.7 mL) at room temperature, were added benzyl bromide (4.41 g, 3.07 mL, 2.5 *equiv.,* 25.8 mmol) and potassium carbonate (4.28 g, 3.0 *equiv.,* 30.9 mmol) sequentially. The resulting mixture was stirred vigorously at reflux for 21 hours. Additional potassium carbonate (2.14 g, 1.5 *equiv.,* 15.5 mmol) and benzyl bromide (2.12 g, 1.47 mL, 1.2 *equiv.,* 12.4 mmol) were added at room temperature and the resulting reaction mixture was stirred at reflux for another 5 hours. The reaction mixture was cooled to room temperature and the solids were filtered and rinsed with EtOAc. The filtrate was concentrated to give the crude product which was purified by flash column chromatography (SiO₂; gradient elution from 0:100-EtOAc:Heptane to 25:75-EtOAc:Heptane) to give 5-bromo-6-(dibenzylamino)-2,3-dihydro-1,3-methanonaphthalen-4(1*H*)-one (4.4 g) as a white solid.
LCMS: m/z (ESI) [M+H]⁺ 432.1, t_{R} = 2.30 minutes (Method C)
¹H NMR (400 MHz, CDCl₃) δ 7.38 - 7.31 (m, 4H), 7.29 (d, J = 7.0 Hz, 4H), 7.25 - 7.18 (m, 2H), 6.96 (m, 2H), 4.17 (s, 4H), 3.30 - 3.21 (m, 2H), 2.80 (m, 2H), 2.33 - 2.24 (m, 2H).

### Step 4: 5-Bromo-4-((6-chloro-5-(hydroxymethyl)-2-(methylthio)pyrimidin-4-yl)methyl)-6-(dibenzylamino)-1,2,3,4-tetrahydro-1,3-methanonaphthalen-4-ol

LDA (1.0 M in THF/hexanes) (768 mg, 7.17 mL, 1.0 M, 3.1 *equiv.,* 7.17 mmol) was added to a stirred solution of (4-chloro-6-methyl-2-(methylthio)pyrimidin-5-yl)methanol (710 mg, 1.5 *equiv.,* 3.47 mmol) in THF (15.4 mL) at -78 °C. The resulting mixture was stirred at - 78 °C for 1 hour. 5-bromo-6-(dibenzylamino)-2,3-dihydro-1,3-methanonaphthalen-4(1H)-one (1.00 g, 1.0 *equiv.,* 2.31 mmol) was then added as a solution in THF (7.71 mL). After the reaction mixture was stirred at -78 °C for 1 hour, it was quenched at -78 °C by the addition of HCl (2.0 M in Et₂O) (270 mg, 3.70 mL, 2.0 M, 3.2 *equiv.,* 7.40 mmol). The quenched mixture was warmed to room temperature and diluted with saturated aqueous sodium bicarbonate and EtOAc. The two phases were separated and the aqueous layer was extracted with EtOAc (x 3). The combined organic extracts were dried over sodium sulfate, filtered, and concentrated to give the crude product which was purified by flash column chromatography (SiO₂, gradient elution from 0:100-EtOAc:DCM to 30:70-EtOAc:DCM) to give 5-bromo-4-((6-chloro-5-(hydroxymethyl)-2-(methylthio)pyrimidin-4-yl)methyl)-6-(dibenzylamino)-1,2,3,4-tetrahydro-1,3-methanonaphthalen-4-ol (1.4 g) as a white solid.
LCMS: m/z (ESI) [M+H]⁺ 638.1, t_{R} = 2.48 minutes (Method C)
¹H NMR (400 MHz, CDCl₃) δ 7.40 - 7.33 (m, 4H), 7.33 - 7.27 (m, 4H), 7.26 - 7.20 (m, 2H), 6.87 (m, 2H), 5.00 - 4.74 (m, 3H), 4.18 - 4.07 (m, 6H), 3.43 (m, 1H), 3.23 (d, 1H), 3.03 (m, 1H), 2.53 (s, 3H), 2.50 - 2.37 (m, 2H), 2.32 - 2.23 (m, 1H), 1.61 (m, 1H).

### Step 5: N,N-Dibenzyl-5'-bromo-4-chloro-2-(methylthio)-2',3',5,8-tetrahydro-1'H-spiro[pyrano[4,3-d]pyrimidine-7,4'-[1,3]methanonaphthalen]-6'-amine

*n*BuLi (2.5 M in hexanes) (0.28 g, 1.7 mL, 2.50 M, 2.2 *equiv.,* 4.4 mmol) was added dropwise to a stirred solution of 5-bromo-4-((6-chloro-5-(hydroxymethyl)-2-(methylthio)pyrimidin-4-yl)methyl)-6-(dibenzylamino)-1,2,3,4-tetrahydro-1,3-methanonaphthalen-4-ol (1.4 g, 90 wt%, 1 *equiv.,* 2.0 mmol) in THF (16 mL) at -78 °C. The resulting mixture was stirred at this temperature for 30 minutes at which point *p-*toluenesulfonyl chloride (0.57 g, 1.5 *equiv.,* 3.0 mmol) in THF (8.2 mL) was added dropwise to the reaction mixture. The resulting mixture was stirred at -78 °C for 15 minutes and then at room temperature for 40 minutes. The reaction mixture was quenched by the addition of a saturated aqueous solution of sodium bicarbonate and diluted with EtOAc. The two phases were separated and the aqueous layer was extracted with EtOAc (x 3). The combined organic extracts were dried over sodium sulfate, filtered, and concentrated to give the crude product which was purified by flash column chromatography (SiO₂, gradient elution from 0:100-EtOAc:Heptane to 20:80-EtOAc:Heptane) to give *N,N*-dibenzyl-5'-bromo-4-chloro-2-(methylthio)-2',3',5,8-tetrahydro-1'*H*-spiro[pyrano[4,3-*d*]pyrimidine-7,4'-[1,3]methanonaphthalen]-6'-amine (1.1 g) as a white solid.
LCMS: m/z (ESI) [M+H]⁺ 620.1, t_{R} = 4.62 minutes (Method E)
¹H NMR (400 MHz, CDCl₃) δ 7.37 - 7.26 (m, 8H), 7.25 - 7.18 (m, 2H), 6.86 (d, 1H), 6.79 (d, 1H), 4.89 (d, 1H), 4.60 (d, 1H), 4.44 (d, 1H), 4.11 (m, 4H), 3.09 (m, 1H), 2.93 - 2.79 (m, 2H), 2.58 (s, 3H), 2.52 - 2.44 (m, 1H), 2.28 (m, 1H), 1.87 (m, 1H), 1.66 (m, 1H).

### Step 6: 5-(5'-Bromo-6'-(dibenzylamino)-2-(methylthio)-2',3',5,8-tetrahydro-1'H-spiro[pyrano[4,3-d]pyrimidine-7,4'-[1,3]methanonaphthalen]-4-yl)-N,N-dimethyl-5,6,7,8-tetrahydro-4H-pyrazolo[1,5-a][1,4]diazepine-2-carboxamide

DIPEA (443 mg, 0.59 mL, 4 *equiv.,* 3.42 mmol) was added to a stirred solution of *N,N-*dimethyl-5,6,7,8-tetrahydro-4*H*-pyrazolo[1,5-*a*][1,4]diazepine-2-carboxamide (357 mg, 2 *equiv.,* 1.71 mmol) and N,N-dibenzyl-5'-bromo-4-chloro-2-(methylthio)-2',3',5,8-tetrahydro-1'*H*-spiro[pyrano[4,3-*d*]pyrimidine-7,4'-[1,3]methanonaphthalen]-6'-amine (530 mg, *1 equiv.,* 856 µmol) in DMF (8.56 mL) at room temperature. The resulting mixture was then heated to 90 °C and stirred at this temperature for 5 hours. The reaction mixture was concentrated to dryness and diluted with water and EtOAc. The two layers were separated and the aqueous phase was extracted with EtOAc (x 3). The combined organic layers were dried over sodium sulfate, filtered, and concentrated to give the crude product which was purified by flash column chromatography (SiO₂, gradient elution from 0:100-MeOH:DCM to 15:85-MeOH:DCM) to give 5-(5'-bromo-6'-(dibenzylamino)-2-(methylthio)-2',3',5,8-tetrahydro-1'*H-*spiro[pyrano[4,3-*d*]pyrimidine-7,4'-[1,3]methanonaphthalen]-4-yl)-*N,N*-dimethyl-5,6,7,8-tetrahydro-4*H*-pyrazolo[1,5-*a*][1,4]diazepine-2-carboxamide (600 mg) as a white solid.
LCMS: m/z (ESI) [M+H]⁺ 792.3, t_{R} = 3.80 minutes (Method E)
¹H NMR (400 MHz, CDCl₃) δ 7.30 (m, 6H), 7.26 - 7.17 (m, 4H), 6.83 (d, 1H), 6.74 (d, 1H), 6.55 (s, 1H), 4.79 - 4.65 (m, 3H), 4.56 - 4.38 (m, 3H), 4.20 - 4.04 (m, 4H), 4.00 (d, 1H), 3.73 (m, 1H), 3.34 (s, 3H), 3.14 - 2.98 (m, 5H), 2.83 (d, 1H), 2.51 (s, 3H), 2.38 - 2.23 (m, 2H), 2.09 (m, 1H), 1.86 (d, 1H), 1.67 (m, 1H), 1.60 (m, 2H).

### Step 7: 5-(5'-cyano-6'-(dibenzylamino)-2-(methylthio)-2',3',5,8-tetrahydro-1'H-spiro[pyrano[4,3-d]pyrimidine-7,4'-[1,3]methanonaphthalen]-4-yl)-N,N-dimethyl-5,6,7,8-tetrahydro-4H-pyrazolo[1,5-a][1,4]diazepine-2-carboxamide

Copper(I) cyanide (618 mg, 212 µL, 10 *equiv.,* 6.90 mmol) was added in one portion to a stirred solution of 5-(5'-bromo-6'-(dibenzylamino)-2-(methylthio)-2',3',5,8-tetrahydro-1'*H-*spiro[pyrano[4,3-*d*]pyrimidine-7,4'-[1,3]methanonaphthalen]-4-yl)-*N,N*-dimethyl-5,6,7,8-tetrahydro-4*H*-pyrazolo[1,5-*a*][1,4]diazepine-2-carboxamide (600 mg, 91 wt%, 1 *equiv.,* 690 µmol) in DMF (13.8 mL). The resulting light yellowish green mixture was heated to 120 °C and stirred for approximately 44 hours. The reaction mixture was cooled to room temperature and concentrated to dryness under reduced pressure. The resulting residue was diluted with water and DCM. A 1:1 mixture of ammonium hydroxide and MeOH (200 mL) was added, and the resulting mixture was stirred at room temperature for ~15 minutes to break the white solids until two clear layers were observed. The two layers were separated and the aqueous layer was extracted with EtOAc (x 3). The combined organic extracts were dried over sodium sulfate, filtered, and concentrated to give 5-(5'-cyano-6'-(dibenzylamino)-2-(methylthio)-2',3',5,8-tetrahydro-1'*H*-spiro[pyrano[4,3-*d*]pyrimidine-7,4'-[1,3]methanonaphthalen]-4-yl)-*N,N-*dimethyl-5,6,7,8-tetrahydro-4*H*-pyrazolo[1,5-*a*][1,4]diazepine-2-carboxamide (560 mg), which was used in the next step without further purification.
LCMS: m/z (ESI) [M+H]⁺ 737.3, t_{R} = 3.50 minutes (Method E)

### Step 8: 5-(5'-Cyano-6'-(dibenzylamino)-2-(methylsulfonyl)-2',3',5,8-tetrahydro-1'H-spiro[pyrano[4,3-d]pyrimidine-7,4'-[1,3]methanonaphthalen]-4-yl)-N,N-dimethyl-5,6,7,8-tetrahydro-4H-pyrazolo[1,5-a][1,4]diazepine-2-carboxamide

Oxone (1.22 g, 3 *equiv.,* 1.98 mmol) was added to a stirred solution of 5-(5'-cyano-6'-(dibenzylamino)-2-(methylthio)-2',3',5,8-tetrahydro-1'*H*-spiro[pyrano[4,3-*d*]pyrimidine-7,4'-[1,3]methanonaphthalen]-4-yl)-*N,N*-dimethyl-5,6,7,8-tetrahydro-4*H*-pyrazolo[1,5-*a*][1,4]diazepine-2-carboxamide (560 mg, 87 wt%, *1 equiv.,* 661 µmol) in THF (5.18 mL), MeOH (8.54 mL), and water (5.18 mL) at room temperature. After the reaction mixture was stirred at room temperature for 4 hours, it was quenched by the addition of water and diluted with EtOAc. The layers were separated, and the aqueous phase was extracted with EtOAc (x 3). The combined organic layers were dried over sodium sulfate, filtered, and concentrated to give 5-(5'-cyano-6'-(dibenzylamino)-2-(methylsulfonyl)-2',3',5,8-tetrahydro-1'*H-*spiro[pyrano[4,3-d]pyrimidine-7,4'-[1,3]methanonaphthalen]-4-yl)-N,N-dimethyl-5,6,7,8-tetrahydro-4*H*-pyrazolo[1,5-*a*][1,4]diazepine-2-carboxamide (410 mg), which was used in the next step without further purification.
LCMS: m/z (ESI) [M+H]⁺ 769.3, t_{R} = 3.42 minutes (Method E)
¹H NMR (400 MHz, CDCl₃) δ 7.29 - 7.18 (m, 11H), 7.05 (d, 1H), 6.82 (d, 1H), 6.61 (s, 1H), 4.86 (m, 2H), 4.74 - 4.58 (m, 2H), 4.55 - 4.40 (m, 2H), 4.26 (m, 4H), 4.07 (m, 2H), 3.86 (m, 1H), 3.31 (s, 3H), 3.23 (s, 3H), 3.15 - 3.09 (m, 1H), 3.06 (s, 3H), 2.86 (m, 1H), 2.56 (m, 1H), 2.41 (m, 1H), 2.28 (m, 1H), 2.17 - 2.05 (m, 1H), 1.82 (m, 1H), 1.67 (m, 1H).

### Step 9: 5-(5'-Cyano-6'-(dibenzylamino)-2-(((2R,7aS)-2-fluorotetrahydro-1H-pyrrolizin-7a(5H)-yl)methoxy)-2',3',5,8-tetrahydro-1'H-spiro[pyrano[4,3-d]pyrimidine-7,4'-[1,3]methanonaphthalen]-4-yl)-N,N-dimethyl-5,6,7,8-tetrahydro-4H-pyrazolo[1,5-a][1,4]diazepine-2-carboxamide

A stirred solution of ((2*R*,7a*S*)-2-fluorotetrahydro-1*H*-pyrrolizin-7a(5*H*)-yl)methanol (255 mg, 3 *equiv.,* 1.60 mmol) and 5-(5'-cyano-6'-(dibenzylamino)-2-(methylsulfonyl)-2',3',5,8-tetrahydro-1'*H*-spiro[pyrano[4,3-*d*]pyrimidine-7,4'-[1,3]methanonaphthalen]-4-yl)-*N,N*-dimethyl-5,6,7,8-tetrahydro-4*H*-pyrazolo[1,5-*a*][1,4]diazepine-2-carboxamide (410 mg, *1 equiv.,* 533 µmol) in DMF (10.7 mL) was cooled to 0 °C. LiHMDS (178 mg, 1.07 mL, 1.0 M, 2 *equiv.,* 1.07 mmol) was added dropwise to the reaction mixture at 0 °C. After the resulting mixture was stirred at 0 °C for 3 hours, it was quenched by the addition of a 1:1 mixture of water and saturated aqueous solution of ammonium chloride (3 mL). The quenched mixture was warmed to room temperature and diluted with EtOAc. The two phases were separated and the aqueous layer was extracted with EtOAc (x 3). The combined organic extracts were dried over sodium sulfate, filtered, and concentrated to give the crude product which was purified by flash column chromatography (SiO₂, gradient elution from 0:100-A:B to 40:60-A:B where A is a premixed solution of 2.5% NH4OH and 20% MeOH in DCM and B is DCM) to give 5-(5'-cyano-6'-(dibenzylamino)-2-(((2*R*,7a*S*)-2-fluorotetrahydro-1*H*-pyrrolizin-7a(5*H*)-yl)methoxy)-2',3',5,8-tetrahydro-1'*H*-spiro[pyrano[4,3-*d*]pyrimidine-7,4'-[1,3]methanonaphthalen]-4-yl)-N,N-dimethyl-5,6,7,8-tetrahydro-4*H*-pyrazolo[1,5-*a*][1,4]diazepine-2-carboxamide (420 mg) as an off-white solid.
LCMS: m/z (ESI) [M+H]⁺ 848.5, t_{R} = 2.57 minutes (Method E)
¹H NMR (400 MHz, CDCl₃) δ 7.31 - 7.27 (m, 7H), 7.22 (m, 3H), 7.03 (d, 1H), 6.81 (d, 1H), 6.56 (s, 1H), 5.32 (s, 1H), 5.18 (d, 1H), 4.87 (d, 1H), 4.75 (d, 1H), 4.58 (s, 2H), 4.52 - 4.40 (m, 2H), 4.27 (m, 4H), 4.06 (m, 1H), 3.94 (d, 1H), 3.87 (d, 1H), 3.75 (m, 1H), 3.33 (s, 3H), 3.26 - 3.12 (m, 3H), 3.07 (m, 4H), 3.00 - 2.91 (m, 2H), 2.84 (m, 1H), 2.52 (m, 1H), 2.41 - 2.32 (m, 1H), 2.33 - 2.15 (m, 3H), 2.19 - 2.02 (m, 3H), 1.99 - 1.83 (m, 3H), 1.80 (m, 1H).

### Step 10: 5-(6'-Amino-5'-cyano-2-(((2R,7aS)-2-fluorotetrahydro-1H-pyrrolizin-7a(5H)-yl)methoxy)-2',3',5,8-tetrahydro-1'H-spiro[pyrano[4,3-d]pyrimidine-7,4'-[1,3]methanonaphthalen]-4-yl)-N,N-dimethyl-5,6,7,8-tetrahydro-4H-pyrazolo[1,5-a][1,4]diazepine-2-carboxamide

A vial was charged with 5-(5'-cyano-6'-(dibenzylamino)-2-(((2*R*,7a*S*)-2-fluorotetrahydro-1*H*-pyrrolizin-7a(5*H*)-yl)methoxy)-2',3',5,8-tetrahydro-1'*H-*spiro[pyrano[4,3-*d*]pyrimidine-7,4'-[1,3]methanonaphthalen]-4-yl)-N,N-dimethyl-5,6,7,8-tetrahydro-4*H*-pyrazolo[1,5-*a*][1,4]diazepine-2-carboxamide (420 mg, 90 wt%, 1 *equiv.,* 446 µmol) and Pd(OH)₂ on carbon (782 mg, 20 wt%, 2.5 *equiv.,* 1.11 mmol). The vial headspace was evacuated and refilled with nitrogen, followed by the addition of MeOH (8.91 mL). A balloon was used to introduce an atmosphere of hydrogen into the vial. The atmosphere of nitrogen was exchanged for hydrogen gas by bubbling through the reaction mixture with hydrogen for 5 minutes, followed by the dropwise addition of acetic acid (32 drops). The resulting mixture was stirred under a positive pressure of hydrogen at room temperature for 2.5 hours. The reaction mixture was filtered through a syringe filter. The filtrate was stirred with sodium carbonate for 5 minutes. The mixture was filtered and concentrated under reduced pressure to give the crude product, which was purified by flash column chromatography (SiO₂, gradient elution from 0:100-A:B to 40:60-A:B where A is a premixed solution of 2.5% NH₄OH and 20% MeOH in DCM and B is DCM) to give 5-(6'-amino-5'-cyano-2-(((2*R*,7a*S*)-2-fluorotetrahydro-1*H*-pyrrolizin-7a(5*H*)-yl)methoxy)-2',3',5,8-tetrahydro-1'*H-*spiro[pyrano[4,3-*d*]pyrimidine-7,4'-[1,3]methanonaphthalen]-4-yl)-*N,N*-dimethyl-5,6,7,8-tetrahydro-4*H*-pyrazolo[1,5-*a*][1,4]diazepine-2-carboxamide (21 mg) as a white solid.
LCMS: m/z (ESI) [M+H]⁺ 668.3, t_{R} = 1.32 minutes (Method C)
¹H NMR (400 MHz, MeOD) δ 7.02 (d, 1H), 6.68 (d, 1H), 6.61 (d, 1H), 5.35 - 5.16 (m, 1H), 4.88 (s, 1H), 4.80 - 4.66 (m, 3H), 4.53 - 4.39 (m, 2H), 4.12 - 3.97 (m, 3H), 3.82 (m, 1H), 3.65 (d, 1H), 3.31 (s, 3H), 3.24 - 3.12 (m, 3H), 3.08 - 2.93 (m, 6H), 2.86 (d, 1H), 2.56 (m, 1H), 2.39 (m, 1H), 2.31 - 2.10 (m, 3H), 2.06 (m, 2H), 1.92 (m, 2H), 1.84 (m, 1H), 1.74 (m, 1H), 1.64 (m, 1H).

### Example 22: 5-(7-amino-8-fluoro-2'-(((2R,7aS)-2-fluorotetrahydro-1H-pyrrolizin-7a(5H)-yl)methoxy)-5-methyl-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide (Compound 501a)

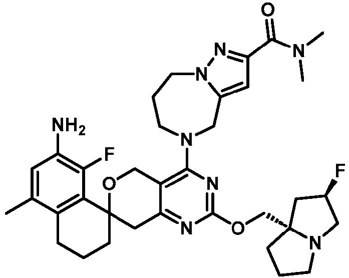

### Step 1: 4-(4-Bromo-5-fluoro-2-methylphenyl)butanal

To a flask was added Li(OAc) (1.6 g, 2.5 *equiv.,* 24 mmol), Pd(OAc)₂ (0.13 g, 0.06 *equiv.,* 0.57 mmol), tetrabutylammonium chloride, hydrate (5.6 g, 2 *equiv.,* 19 mmol), and LiCl (0.40 g, 1 *equiv.,* 9.5 mmol). These solids were evacuated and back-filled three times with nitrogen gas. DMF (24 mL) was then added followed by 1-bromo-2-fluoro-4-iodo-5-methylbenzene (3.0 g, 1 *equiv.,* 9.5 mmol) and but-3-en-1-ol (0.82 g, 0.98 mL, 1.2 *equiv.,* 11 mmol), both via syringe. This reaction mixture was stirred at 72 °C overnight. After 15 hours, TLC analysis indicated complete consumption of starting material and the reaction was quenched by addition of saturated NH₄Cl solution (100 mL) and heptane (100 mL). The layers were separated, and the aqueous layer was extracted twice more with heptane (2 x 50 mL). The combined organics were washed with 1 M HCl (100 mL) and then brine (100 mL). The organics were then dried with sodium sulfate, filtered, and concentrated to afford 4-(4-bromo-5-fluoro-2-methylphenyl)butanal (2.19 g) as an orange oil which was used without further purification.
¹H NMR (400 MHz, CDCl₃): δ 9.80 - 9.77 (m, 1H), 7.30 (d, 1H), 6.89 (d, 1H), 2.60 - 2.54 (m, 2H), 2.54 - 2.48 (m, 2H), 2.24 (s, 3H), 1.93 - 1.83 (m, 2H)

### Step 2: 4-(4-bromo-5-fluoro-2-methylphenyl)butanoic acid

To a 500 mL round bottom flask was added 4-(4-bromo-5-fluoro-2-methylphenyl)butanal (3.40 g, 1 *equiv.,* 13.1 mmol), 2-methyl-2-butene (36.7 g, 55.6 mL, 40 *equiv.,* 525 mmol), potassium phosphate, monobasic (17.9 g, 10 *equiv.,* 131 mmol), water (35 mL), and *t*-BuOH (69 mL). This mixture was placed in a 0 °C ice bath. After stirring for 30 minutes the reaction was brought to room temperature. After stirring at room temperature for 3 hours. TLC analysis indicated complete consumption of the starting material. The reaction was then poured into a separatory funnel that contained a 0.5 M HCl solution and DCM (150 mL each). The layers were separated, and the aqueous layer was extracted twice more with DCM. The combined organics were washed with brine, dried with sodium sulfate, filtered, and concentrated to yield 4-(4-bromo-5-fluoro-2-methylphenyl)butanoic acid (3.61 g), which was used without further purification.
¹H NMR (400 MHz, CDCl₃): δ 7.30 (d, 1H), 6.90 (d, 1H), 2.63 - 2.57 (m, 2H), 2.45 - 2.39 (m, 2H), 2.24 (s, 3H), 1.94 - 1.86 (m, 2H)

### Step 3: 7-Bromo-8-fluoro-5-methyl-3,4-dihydronapthalen-1(2H)-one

To a round bottom flask was added 4-(4-bromo-5-fluoro-2-methylphenyl)butanoic acid (3.61 g, 1 *equiv.,* 13.1 mmol) and dissolved in DCM (69 mL). DMF (102 µl, 0.1 *equiv., 1.31* mmol) was then added to the flask. Oxalyl chloride (3.33 g, 2.30 mL, 2 *equiv.,* 26.2 mmol) was slowly added and the resulting mixture was stirred for 1 hour. At this point the reaction mixture was concentrated and redissolved in DCM (69 mL). AlCl₃ was then added, and the reaction mixture was stirred overnight for 16 hours. The reaction was then quenched with the addition of ice water (50 mL) and the layers were separated. The aqueous layer was extracted twice more with DCM (2 x 50 mL). The combined organic layers were dried with sodium sulfate, filtered, and concentrated. The crude mixture was purified by flash column chromatography (0 to 30% EtOAc in heptane) to yield 7-bromo-8-fluoro-5-methyl-3,4-dihydronapthalen-1(2*H*)-one (1.9 g) as an off white solid.
¹H NMR (400 MHz, CDCl₃): δ 7.52 (d, 1H), 2.82 - 2.76 (m, 2H), 2.66 - 2.60 (m, 2H), 2.26 (s, 3H), 2.17 - 2.09 (m, 2H)

### Step 4: 7-Bromo-1-((6-chloro-5-(hydroxymethyl)-2-(methylthio)pyrimidin-4-yl)methyl)-8-fluoro-5-methyl-1,2,3,4-tetrahydronaphthalen-1-ol

To a 40 mL vial was added 4-chloro-6-methyl-2-(methylthio)pyrimidin-5-yl)methanol (358 mg, 1.5 *equiv.,* 1.75 mmol) and THF (10 mL). This solution was cooled to -78 °C. A 1 M LDA solution (3.6 mL, 3.1 *equiv.,* 3.62 mmol) was added dropwise to maintain an internal temperature of -78 °C. This mixture was stirred for 60 minutes at -78 °C. After 60 minutes, a solution of 7-bromo-8-fluoro-5-methyl-3,4-dihydronapthalen-1(2*H*)-one (300 mg, 1.0 *equiv.,* 1.17 mmol) in THF (6.0 mL) was added to the mixture dropwise and the resulting reaction mixture was stirred at -78 °C until complete conversion of the starting tetralone was observed. At this point a 2 M solution of HCl in Et₂O (2.04 mL, 3.5 *equiv.,* 4.08 mmol) was added to the mixture at -78 °C. The mixture was warmed to room temperature and diluted with EtOAc and water. The layers were separated, and the aqueous layer was extracted once with EtOAc and then once with DCM. The combined organic layers were dried over sodium sulfate, filtered, and concentrated to a residue which was purified by column chromatography (25 g silica gel column eluting with a gradient of 0 to 40% EtOAc in DCM) to yield 7-bromo-1-((6-chloro-5-(hydroxymethyl)-2-(methylthio)pyrimidin-4-yl)methyl)-8-fluoro-5-methyl-1,2,3,4-tetrahydronaphthalen-1-ol (315 mg).
LCMS: m/z (ESI) [M+H]⁺ 463.0, t_{R} = 3.44 minutes (Method C)

### Step 5: 7-Bromo-4'-chloro-8-fluoro-5-methyl-2'-(methylthio)-3,4,5',8'-tetrahydro-2H-spiro[naphthalene-1,7'-pyrano[4,3-d]pyrimidine]

To a vial was added 7-bromo-1-((6-chloro-5-(hydroxymethyl)-2-(methylthio)pyrimidin-4-yl)methyl)-8-fluoro-5-methyl-1 ,2,3,4-tetrahydronaphthalen-1-ol (315 mg, *1 equiv.,* 682.2 µmol). The vial was evacuated and back filled with nitrogen gas and then degassed. Chlorobenzene (5.0 mL) was then added followed by tetrafluoroboric acid diethyl ether complex (131.7 mg, 111.2 µl, 1.2 *equiv.,* 819 µl) dropwise. The reaction mixture was then stirred at room temperature for 70 minutes. At this point the mixture was diluted with EtOAc and water and the layers were separated. The aqueous layer was extracted twice more with EtOAc. The combined organic layers were dried with sodium sulfate, filtered, and concentrated to a residue which was purified by column chromatography (25 g silica gel column eluting with a gradient of 0 to 40% EtOAc in heptane) to yield 7-bromo-4'-chloro-8-fluoro-5-methyl-2'-(methylthio)-3,4,5',8'-tetrahydro-2*H*-spiro[naphthalene-1,7'-pyrano[4,3-*d*]pyrimidine] (253 mg).
LCMS: m/z (ESI) [M+H]⁺ 443.0 t_{R} = 4.14 minutes (Method C)

### Step 6: 5-(7-Bromo-8-fluoro-5-methyl-2'-(methylthio)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide

To a vial was added 7-bromo-4'-chloro-8-fluoro-5-methyl-2'-(methylthio)-3,4,5',8'-tetrahydro-2*H*-spiro[naphthalene-1,7'-pyrano[4,3-*d*]pyrimidine] (253 mg, *1 equiv.,* 570 µmol), *N,N*-dimethyl-2,4,5,6,7,8-hexahydro-1*H*-pyrazolo[1,5-*a*][1,4]diazepine-2-carboxamide, HCl (422.0 mg, 3.0 *equiv.,* 1.710 mmol), DIPEA(1.59 mL, 16.0 *equiv.,* 9.12 mmol), and MeCN (5.0 mL). The vial was sealed, and the resulting mixture was stirred at 80 °C for 18 hours. At this point the reaction mixture was diluted with water and EtOAc. The layers were separated, and the aqueous layer was extracted twice more with EtOAc. The combined organics were dried with sodium sulfate, filtered, and concentrated to a residue which was purified by column chromatography (25 g silica gel column eluting with a gradient of 0 to 10% MeOH in DCM) to yield 5-(7-bromo-8-fluoro-5-methyl-2'-(methylthio)-3,4,5',8'-tetrahydro-2H-spiro[naphthalene-1,7'-pyrano[4,3-*d*]pyrimidin]-4'-yl)-*N,N*-dimethyl-5,6,7,8-tetrahydro-4*H-*pyrazolo[1,5*-a*][1,4]diazepine-2-carboxamide (401 mg).
LCMS: m/z (ESI) [M+H]⁺ 615.2 t_{R} = 2.99 minutes (Method C)

### Step 7: 5-(7-Bromo-8-fluoro-5-methyl-2'-(methylsulfonyl)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide

To a vial was added 5-(7-bromo-8-fluoro-5-methyl-2'-(methylthio)-3,4,5',8'-tetrahydro-2*H*-spiro[naphthalene-1,7'-pyrano[4,3-d]pyrimidin]-4'-yl)-*N,N*-dimethyl-5,6,7,8-tetrahydro-4*H*-pyrazolo[1,5-*a*][1,4]diazepine-2-carboxamide (400 mg, 650 µmol) and Oxone (2.66 g, 3 *equiv.,* 1.95 mmol). These were dissolved in water (6 mL), MeOH (6 mL), and THF (6 mL). The resulting reaction mixture was stirred at room temperature for 2 hours. The reaction was quenched by the addition of water (20 mL) and EtOAc (30 mL) followed by separation of the layers. The aqueous layer was then extracted three times with EtOAc (20 mL each). The combined organics were dried over sodium sulfate, filtered, and concentrated to yield 5-(7-bromo-8-fluoro-5-methyl-2'-(methylsulfonyl)-3,4,5',8'-tetrahydro-2*H*-spiro[naphthalene-1,7'-pyrano[4,3-*d*]pyrimidin]-4'-yl)-*N,N*-dimethyl-5,6,7,8-tetrahydro-4*H*-pyrazolo[1,5-*a*][1,4]diazepine-2-carboxamide (287 mg), which was used without further purification.
LCMS: m/z (ESI) [M+H]⁺ 649.2 t_{R} = 2.95 minutes (Method C)

### Step 8: 5-(7-Bromo-8-fluoro-2'-(((2R,7aS)-2-fluorotetrahydro-1H-pyrrolizin-7a(5H)-yl)methoxy)-5-methyl-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide

To a flask was added ((2*R*,7a*S*)-2-fluorotetrahydro-1*H*-pyrrolizin-7a(5*H*)-yl)methanol (350 mg, 5.0 *equiv.),* 2.20 mmol) and 5-(7-bromo-8-fluoro-5-methyl-2'-(methylsulfonyl)-3,4,5',8'-tetrahydro-2*H*-spiro[naphthalene-1,7'-pyrano[4,3-*d*]pyrimidin]-4'-yl)-*N,N*-dimethyl-5,6,7,8-tetrahydro-4*H*-pyrazolo[1,5-*a*][1,4]diazepine-2-carboxamide (285 mg, 1.0 *equiv., 440* µmol). These were dissolved in THF (5 mL) under nitrogen at room temperature followed by the dropwise addition of 1 M KOtBu in THF (880 µl, 2.0 *equiv.,* 880 µmol). The reaction mixture was stirred at room temperature for 30 minutes and then diluted with EtOAc and water. The aqueous layer was extracted twice more with EtOAc and the combined organics were dried over sodium sulfate, filtered, and concentrated to a residue which was purified by column chromatography (25 g silica gel column eluting with a gradient of 0 to 20% MeOH in DCM) to yield 5-(7-bromo-8-fluoro-2'-(((2*R*,7a*S*)-2-fluorotetrahydro-1*H*-pyrrolizin-7a(5*H*)-yl)methoxy)-5-methyl-3,4,5',8'-tetrahydro-2 H-spiro[naphthalene-1,7'-pyrano[4,3-*d*]pyrimidin]-4'-yl)-*N,N-*dimethyl-5,6,7,8-tetrahydro-4*H*-pyrazolo[1,5-*a*][1,4]diazepine-2-carboxamide (277 mg).
LCMS: m/z (ESI) [M+H]⁺ 726.2 t_{R} = 2.12 minutes (Method C)

### Step 9: tert-Butyl (4'-(2-(dimethylcarbamoyl)-7,8-dihydro-4H-pyrazolo[1,5-a][1,4]diazepin-5(6H)-yl)-8-fluoro-2'-(((2R,7aS)-2-fluorotetrahydro-1H-pyrrolizin-7a(5H)-yl)methoxy)-5-methyl-3,4,5',8'-tetrahydro-2H-spiro[naphthalene-1,7'-pyrano[4,3-d]pyrimidin]-7-yl)carbamate

To a vial was added *tert*-butyl carbamate (27.8 mg, 1.5 *equiv.,* 237 µmol), 5-(7-bromo-8-fluoro-2'-(((2*R*,7a*S*)-2-fluorotetrahydro-1*H*-pyrrolizin-7a(5*H*)-yl)methoxy)-5-methyl-3,4,5',8'-tetrahydro-2*H*-spiro[naphthalene-1,7'-pyrano[4,3-*d*]pyrimidin]-4'-yl)-*N,N*-dimethyl-**5,6,7,8-tetrahydro-4*H*-pyrazolo[1,5-*a*][1,4]diazepine-2-carboxamide** (115 mg, 1.0 *equiv.,* 158 µmol), cesium carbonate (115 mg, 3.0 *equiv.,* 475 µmol), and BrettPhos Pd G4 (21.9 mg, 0.15 *equiv.,* 23.7 µmol). The vial was degassed and backfilled with nitrogen, and dry 1,4 dioxane (2.0 mL) was added. The mixture was heated to 80 °C and stirred for 30 minutes then the mixture was heated to 100 °C for 3 hours. At this point, the reaction mixture was stored at -26 °C overnight. The reaction mixture was then diluted with EtOAc and filtered through celite. The filtered cake was rinsed with EtOAc. The crude mixture was then concentrated and purified by column chromatography (20 g silica gel column eluting with a gradient of 0 to 20% MeOH in DCM) to yield *tert*-butyl (4'-(2-(dimethylcarbamoyl)-7,8-dihydro-4*H*-pyrazolo[1,5-*a*][1,4]diazepin-5(6*H*)-yl)-8-fluoro-2'-(((2*R*,7a*S*)-2-fluorotetrahydro-1*H*-pyrrolizin-7a(5*H*)-yl)methoxy)-5-methyl-3,4,5',8'-tetrahydro-2*H*-spiro[naphthalene-1,7'-pyrano[4,3-*d*]pyrimidin]-7-yl)carbamate (102 mg).
LCMS: m/z (ESI) [M+H]⁺ 763.4 t_{R} = 2.17 minutes (Method C)

### Step 10: 5-(7-Amino-8-fluoro-2'-(((2R,7aS)-2-fluorotetrahydro-1H-pyrrolizin-7a(5H)-yl)methoxy)-5-methyl-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide

To a vial was added *tert*-butyl (4'-(2-(dimethylcarbamoyl)-7,8-dihydro-4*H-*pyrazolo[1,5-*a*][1,4]diazepin-5(6*H*)-yl)-8-fluoro-2'-(((2*R*,7a*S*)-2-fluorotetrahydro-1*H-*pyrrolizin-7a(5*H*)-yl)methoxy)-5-methyl-3,4,5',8'-tetrahydro-2H-spiro[naphthalene-1,7'-pyrano[4,3-*d*]pyrimidin]-7-yl)carbamate (100 mg, 1.0 *equiv.,* 0.131 mmol) followed by DCM (3.0 mL) and HCl as a 2 M solution in Et₂O (1.97 mL, 30 *equiv.,* 3.93 mmol). The reaction mixture was then stirred for 1 hour at room temperature. At this point, a precipitate had formed and the filtrate was removed with a pipette. The precipitate was triturated three times with 3 mL of Et₂O. The crude precipitate was then purified via reverse phase chromatography (XSelect ^{®} CSH C18 QBD Prep column 130 Å, 5 µm, 10 mm x 150 mm, eluting with a gradient of 15 to 35% MeCN in water with 0.1% TFA additive to both mobile phases) to yield 5-(7-amino-8-fluoro-2'-(((2*R*,7a*S*)-2-fluorotetrahydro-1*H*-pyrrolizin-7a(5*H*)-yl)methoxy)-5-methyl-3,4,5',8'-tetrahydro-2*H*-spiro[naphthalene-1,7'-pyrano[4,3-*d*]pyrimidin]-4'-yl)*-N,N-*dimethyl-5,6,7,8-tetrahydro-4*H*-pyrazolo[1,5-*a*][1,4]diazepine-2-carboxamide (11.1 mg).
LCMS: m/z (ESI) [M+H]⁺ 663.3 t_{R} = 1.71 minutes (Method C)
¹H NMR (400 MHz, CD₃OD): δ 6.70 (d, 1H), 6.61 (s, 1H), 5.28 (d, 1H), 4.90 (d, 1H), 4.78 (s, 2H), 4.67 (d, 1H), 4.55 - 4.43 (m, 2H), 4.11 (d, 1H), 4.03 (d, 1H), 4.02 - 3.86 (m, 2H), 3.38 -3.33 (m, 1H), 3.32 - 3.30 (m, 3H), 3.29 - 3.13 (m, 3H), 3.09 (s, 3H), 3.05 - 2.94 (m, 1H), 2.92 - 2.78 (m, 2H), 2.67 - 2.50 (m, 2H), 2.33 - 1.80 (m, 15H), 1.80 - 1.65 (m, 1H)

### Example 23: 5-((7S)-6'-amino-5'-cyano-2-(((2R,7aS)-2-fluorotetrahydro-1H-pyrrolizin-7a(5H)-yl)methoxy)-2',3',5,8-tetrahydro-1'H-spiro[pyrano[4,3-d]pyrimidine-7,4'-[1,3]methanonaphthalen]-4-yl)-N,N-dimethyl-5,6,7,8-tetrahydro-4H-pyrazolo[1,5-a][1,4]diazepine-2-carboxamide (Compound 504c)

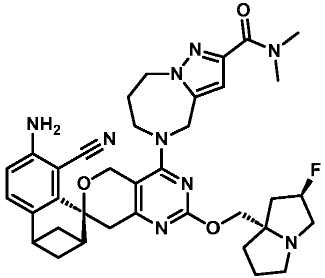

The diastereomers of 5-(6'-amino-5'-cyano-2-(((2*R*,7a*S*)-2-fluorotetrahydro-1*H-*pyrrolizin-7a(5*H*)-yl)methoxy)-2',3',5,8-tetrahydro-1'*H*-spiro[pyrano[4,3-*d*]pyrimidine-7,4'-[1,3]methanonaphthalen]-4-yl)-*N,N*-dimethyl-5,6,7,8-tetrahydro-4H-pyrazolo[1,5-*a*][1,4]diazepine-2-carboxamide (100 mg) were separated by preparative SFC chromatography (Column: ChiralPak IA 21mm ID x 250 mm L (mobile phase: 40% methanol, 0.25% diethylamine in CO₂)) to yield 5-((7*S*)-6'-amino-5'-cyano-2-(((2*R*,7a*S*)-2-fluorotetrahydro-1*H-*pyrrolizin-7a(5*H*)-yl)methoxy)-2',3',5,8-tetrahydro-1'*H*-spiro[pyrano[4,3-*d*]pyrimidine-7,4'-[1,3]methanonaphthalen]-4-yl)-*N,N*-dimethyl-5,6,7,8-tetrahydro-4H-pyrazolo[1,5-*a*][1,4]diazepine-2-carboxamide as **Peak 1** (20 mg)..
**Peak 1:** LCMS: m/z (ESI) [M+H]⁺ 668.5, t_{R} = 4.03 minutes (ChiralPak IA 3 mm ID x 100 mm L, mobile phase: 40% methanol, 0.25% diethylamine in CO₂)
¹H NMR (400 MHz, CD₃OD) δ 7.04 (d, 1H), 6.69 (d, 1H), 6.62 (s, 1H), 5.27 (d, 1H), 4.86 - 4.67 (m, 4H), 4.58 - 4.38 (m, 2H), 4.16 - 3.97 (m, 3H), 3.90 - 3.79 (m, 1H), 3.66 (d, 1H), 3.35 (s, 3H), 3.29 - 3.12 (m, 3H), 3.12 - 2.94 (m, 6H), 2.87 (d, 1H), 2.59 (m, 1H), 2.41 (m, 1H), 2.31 - 2.12 (m, 3H), 2.11 - 2.00 (m, 2H), 2.00 - 1.88 (m, 2H), 1.89 - 1.80 (m, 1H), 1.76 (m, 1H), 1.66 (m, 1H).

### Example 24: 5-((7R)-6'-amino-5'-cyano-2-(((2R,7aS)-2-fluorotetrahydro-1H-pyrrolizin-7a(5H)-yl)methoxy)-2',3',5,8-tetrahydro-1'H-spiro[pyrano[4,3-d]pyrimidine-7,4'-[1,3]methanonaphthalen]-4-yl)-N,N-dimethyl-5,6,7,8-tetrahydro-4H-pyrazolo[1,5-a][1,4]diazepine-2-carboxamide (Compound 504a)

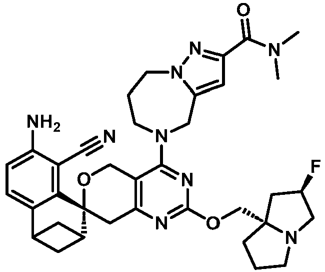

5-((7*R*)-6'-amino-5'-cyano-2-(((2*R*,7a*S*)-2-fluorotetrahydro-1*H*-pyrrolizin-7a(5*H*)-yl)methoxy)-2',3',5,8-tetrahydro-1'*H*-spiro[pyrano[4,3-d]pyrimidine-7,4'-[1,3]methanonaphthalen]-4-yl)-*N,N*-dimethyl-5,6,7,8-tetrahydro-4*H*-pyrazolo[1,5-*a*][1,4]diazepine-2-carboxamide was obtained from **Example 23** as **Peak 2** (29 mg).
**Peak 2:** LCMS: m/z (ESI) [M+H]⁺ 668.4, t_{R} = 5.98 minutes (ChiralPak IA 3 mm ID x 100 mm L, mobile phase: 40% methanol, 0.25% diethylamine in CO₂)
¹H NMR (400 MHz, CD₃OD) δ 7.05 (d, 1H), 6.70 (d, 1H), 6.63 (s, 1H), 5.37 - 5.19 (m, 1H), 4.86 - 4.67 (m, 4H), 4.58 - 4.42 (m, 2H), 4.07 (m, 3H), 3.85 (m, 1H), 3.67 (d, 1H), 3.33 (s, 3H), 3.24 - 3.14 (m, 3H), 3.08 (s, 3H), 3.06 - 2.95 (m, 3H), 2.88 (d, 1H), 2.59 (m, 1H), 2.41 (m, 1H), 2.31 - 2.19 (m, 2H), 2.18 - 2.12 (m, 1H), 2.12 - 2.03 (m, 2H), 2.01 - 1.91 (m, 2H), 1.88 (m, 1H), 1.77 (m, 1H), 1.67 (m, 1H).

### Example 25: (1S,4S)-7-amino-4'-((S*)-4-cyanoazepan-1-yl)-2'-(((2R,7aS)-2-fluorotetrahydro-1H-pyrrolizin-7a(5H)-yl)methoxy)-4-methyl-3,4,5',8'-tetrahydro-2H-spiro[naphthalene-1,7'-pyrano[4,3-d]pyrimidine]-8-carbonitrile (Compound 502b)

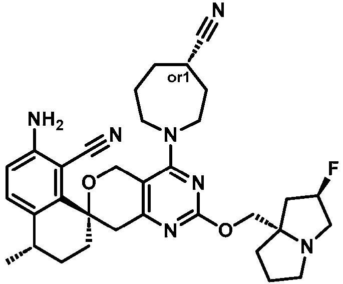

### Step 1: (1S,4S)-4'-(Benzyloxy)-7-(dibenzylamino)-2'-(((2R,7aS)-2-fluorotetrahydro-1H-pyrrolizin-7a(5H)-yl)methoxy)-4-methyl-3,4,5',8'-tetrahydro-2H-spiro[naphthalene-1,7'-pyrano[4,3-d]pyrimidine]-8-carbonitrile

Phenylmethanol (546 mg, 525 µL, 5.5 *equiv.,* 5.05 mmol) was added dropwise to a stirred solution of NaH (143 mg, 6.5 *equiv.,* 5.97 mmol) in THF (3.06 mL) at room temperature. The resulting mixture was stirred for 5 minutes. The benzylalkoxide mixture was then added dropwise to a stirring solution of (1*S*,4*S*)-*N,N*-dibenzyl-8-bromo-4'-chloro-4-methyl-2'-(methylthio)-3,4,5',8'-tetrahydro-2*H*-spiro[naphthalene-1,7'-pyrano[4,3-*d*]pyrimidin]-7-amine (600 mg, *1 equiv.,* 966 µmol)) in THF (6.12 mL) and stirred at room temperature. The reaction mixture was monitored through LCMS. After the reaction mixture was stirred at room temperature for 15 minutes, LCMS showed consumption of starting material and formation of desired product. The reaction mixture was quenched by the addition of a solution of saturated aqueous ammonium chloride (10 mL). The ice bath was removed, and the resulting mixture was diluted with EtOAc (20 mL). The two phases were separated and the aqueous layer was extracted with EtOAc (90 mL). The combined organic layers were dried over sodium sulfate, filtered, and concentrated to residue. The crude product was purified using a RediSep Rf Gold 40 g silica gel cartridge (liquid load in DCM with gradient elution from 0:100-DCM:Heptane to 100:0-DCM:Heptane) to give 672 mg of solid crude material. The major component of which was consistent with the desired product.

The above crude material was taken up in DMF (17.8 mL) and the solution was added under nitrogen to a flask containing copper(I) cyanide (799 mg, 8.92 mmol) at ambient temperature. The resulting mixture was heated to 120 °C and stirred for 2 hours, cooled to room temperature, and diluted sequentially with DCM (100 mL), water (100 ml), MeOH (20 mL), and NH₄OH (30 mL). The solution was stirred until a blue biphasic mixture was observed. The reaction mixture was transferred into a separatory funnel and the two phases were separated. The aqueous layer was extracted with DCM (100 mL x 3). The combined organic layers were dried over sodium sulfate, filtered, and concentrated to give 637 mg of solid crude material.

The above crude material was taken up in THF (11 mL), MeOH (7 mL), and water (7 mL). To this solution was delivered Oxone (1.89 g, 3.07 mmol). The resulting mixture was stirred for 1 hour at ambient temperature. The reaction mixture was diluted sequentially with water (30 mL) and EtOAc (30 mL). The aqueous layer was extracted with EtOAc (3 x 40 mL). The organic layers were combined and dried over sodium sulfate. The dried organic layers were filtered, and concentrated to give 747 mg of crude residue.

The above crude residue (747 mg) was taken up in DMF (17.8 mL) and charged to a flask containing ((2*R*,7a*S*)-2-fluorotetrahydro-1*H*-pyrrolizin-7a(5*H*)-yl)methanol (709 mg, 4.45 mmol), the reaction mixture was cooled to 0°C and LiHMDS (1.5 M in THF) (224 mg, 891 µL, 1.34 mmol) was added dropwise to the stirring solution under nitrogen. The ice/water bath was removed, and the reaction mixture was stirred for 20 minutes. The reaction was quenched by the slow addition of a 1:1 mixture of a saturated ammonium chloride solution and water (24 mL). EtOAc (25 mL) was added and the layers were separated and the aqueous phase was extracted with EtOAc (3 x 50 mL). The combined organic extracts were dried over sodium sulfate, filtered, and concentrated. The crude material was purified by silica gel chromatography (load: CH₂Cl₂, column: RediSep Rf Gold 80 g, 60 mL/min, CH₂Cl₂/[CH₂Cl₂/MeOH/NH₄OH, 75:22.5:2.5], 0 → 100% over 30 min) to afford (1*S*,4*S*)-4'-(benzyloxy)-7-(dibenzylamino)-2'-(((2*R*,7a*S*)-2-fluorotetrahydro-1*H*-pyrrolizin-7a(5*H*)-yl)methoxy)-4-methyl-3,4,5',8'-tetrahydro-2*H*-spiro[naphthalene-1,7'-pyrano[4,3-*d*]pyrimidine]-8-carbonitrile (285 mg).
¹H NMR (400 MHz, CDCl₃) δ 7.47 - 7.16 (m, 15H), 7.09 (d, 1H), 6.87 (d, 1H), 5.47 - 5.35 (m, 2H), 5.17 (d, 1H), 4.96 (d, 1H), 4.77 - 4.65 (m, 1H), 4.34 - 4.18 (m, 4H), 4.12 (d, 1H), 4.03 (d, 1H), 3.41 (d, 1H), 3.28 - 3.09 (m, 3H), 2.98 - 2.86 (m, 2H), 2.31 - 1.78 (m, 10H), 1.67 - 1.55 (m, 1H), 1.23 (d, 3H).
19% Residual DMF present in NMR: 7.98 (s, 1H), 2.92 (s, 3H), 2.85 (s, 3H)
LCMS: m/z (ESI) [M+H]⁺ 750.4, t_{R} = 3.02 min, (Method E)

### Step 2: (1S,4S)-7-Amino-2'-(((2R,7aS)-2-fluorotetrahydro-1H-pyrrolizin-7a(5H)-yl)methoxy)-4'-hydroxy-4-methyl-3,4,5',8'-tetrahydro-2H-spiro[naphthalene-1,7'-pyrano[4,3-d]pyrimidine]-8-carbonitrile

MeOH (6.16 mL) was delivered dropwise to a mixture of (1*S*,4*S*)-4'-(Benzyloxy)-7-(dibenzylamino)-2'-(((2*R*,7a*S*)-2-fluorotetrahydro-1*H*-pyrrolizin-7a(5*H*)-yl)methoxy)-4-methyl-3,4,5',8'-tetrahydro-2*H*-spiro[naphthalene-1,7'-pyrano[4,3-*d*]pyrimidine]-8-carbonitrile (285 mg, 81 wt%, *1 equiv.,* 308 µmol) and Pd(OH)₂ on carbon (108 mg, 31.4 µL, *2.5 equiv.,* 770 µmol) in MeOH (6.16 mL) under N₂. Hydrogen gas was bubbled through the reaction mixture for 10 minutes before the dropwise delivery of acetic acid (21 mg, .020 mL, *1.1 equiv.,* 0.35 mmol). The needle was removed from solution and the reaction was stirred at ambient temperature under H₂ for 2 hours. The reaction mixture was filtered, and concentrated under reduced pressure to give (1*S*,4*S*)-7-amino-2'-(((2*R*,7a*S*)-2-fluorotetrahydro-1*H-*pyrrolizin-7a(5*H*)-yl)methoxy)-4'-hydroxy-4-methyl-3,4,5',8'-tetrahydro-2H-spiro[naphthalene-1,7'-pyrano[4,3-*d*]pyrimidine]-8-carbonitrile (133 mg).
LCMS: m/z (ESI) [M+H]⁺ 480.3, t_{R} = 1.61 minutes (Method E)

### Step 3: (1S,4S)-7-amino-4'-((S*)-4-cyanoazepan-1-yl)-2'-(((2R,7aS)-2-fluorotetrahydro-1H-pyrrolizin-7a(5H)-yl)methoxy)-4-methyl-3,4,5',8'-tetrahydro-2H-spiro[naphthalene-1,7'-pyrano[4,3-d]pyrimidine]-8-carbonitrile

DIPEA (114 mg, 153 µL, 8 *equiv.,* 879 µmol) was delivered to a 20 dram vial charged with (1*S*,4*S*)-7-amino-2'-(((2*R*,7a*S*)-2-fluorotetrahydro-1*H*-pyrrolizin-7a(5*H*)-yl)methoxy)-4'-hydroxy-4-methyl-3,4,5',8'-tetrahydro-2*H*-spiro[naphthalene-1,7'-pyrano[4,3-*d*]pyrimidine]-8-carbonitrile (52.7 mg, 1 *equiv.,* 110 µmol), azepane-4-carbonitrile **(Intermediate 17a,** 42.0 mg, 3.08 *equiv.,* 338 µmol), PyBOP (80 mg, 1.4 *equiv.,* 0.15 mmol), and DMF (2.20 mL) at ambient temperature. The reaction mixture was heated at 80 °C and stirred for 2.5 hours. The reaction mixture was cooled to ambient temperature and diluted sequentially with a 1:1 mixture of water and saturated aqueous sodium chloride solution (5.0 mL) and EtOAc (10.0 mL). The product mixture was transferred to a separatory funnel. The aqueous layer was extracted with EtOAc (3 x 10 mL). The organic layers were combined, dried over sodium sulfate, and concentrated to residue. The residue was purified first by SFC (Torus 2-PIC (19 x 100 mm, 5 µm, PN 186008586) with MeOH co-solvent with 0.25% diethylamine) then by reverse phase HPLC (C18 10X150 mm, eluting with a 25-100% MeCN in water gradient, 0.1% formic acid additive)) to give (1*S*,4*S*)-7-amino-4'-((*S**)-4-cyanoazepan-1-yl)-2'-(((2*R*,7a*S*)-2-fluorotetrahydro-1*H*-pyrrolizin-7a(5*H*)-yl)methoxy)-4-methyl-3,4,5',8'-tetrahydro-2H-spiro[naphthalene-1,7'-pyrano[4,3-d]pyrimidine]-8-carbonitrile (3.7 mg).
¹H NMR (400 MHz, MeOD) δ 7.14 (d, 1H), 6.77 (d, 1H), 5.46 (d, 1H), 5.08 (dd, 1H), 4.91 (s, 1H), 4.39 (d, 1H), 4.30 (d, 1H), 3.93 - 3.55 (m, 7H), 3.17 (t, 2H), 3.01 (m, 1H), 2.91 (d, 2H), 2.61 - 2.33 (m, 2H), 2.33 - 1.81 (m, 13H), 1.77 - 1.69 (m, 1H), 1.25 (d, 3H).
LCMS: m/z (ESI) [M+H]⁺ 586.3, t_{R} = 1.40 minutes (Method E)

### Example 26: (1S,4S)-7-amino-4'-((R*)-4-cyanoazepan-1-yl)-2'-(((2R,7aS)-2-fluorotetrahydro-1H-pyrrolizin-7a(5H)-yl)methoxy)-4-methyl-3,4,5',8'-tetrahydro-2H-spiro[naphthalene-1,7'-pyrano[4,3-d]pyrimidine]-8-carbonitrile (Compound 502a)

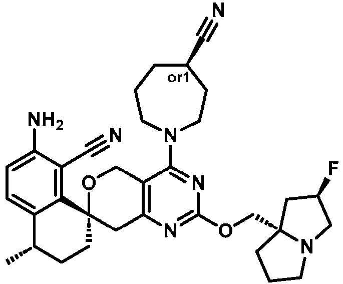

DIPEA (99.6 mg, 134 µL, 8 *equiv.,* 771 µmol) was delivered to a 20 dram vial charged with (1*S*,4*S*)-7-amino-2'-(((2*R*,7a*S*)-2-fluorotetrahydro-1*H*-pyrrolizin-7a(5*H*)-yl)methoxy)-4'-hydroxy-4-methyl-3,4,5',8'-tetrahydro-2*H*-spiro[naphthalene-1,7'-pyrano[4,3-*d*]pyrimidine]-8-carbonitrile (46.2 mg, *1 equiv.,* 96.3 µmol), rel-(R)-azepane-4-carbonitrile **(Intermediate 17b,** 45 mg, 3.8 *equiv.,* 0.36 mmol), PyBOP (61 mg, 1.2 *equiv.,* 0.12 mmol), and DMF (1.93 mL) at ambient temperature. The reaction mixture was heated at 80 °C and stirred at this temperature for 2.5 hours. The reaction mixture was cooled to ambient temperature and diluted sequentially with a 1:1 mixture of water and saturated aqueous sodium chloride solution (5.0 mL) and EtOAc (10.0 mL). The product mixture was transferred to a separatory funnel and the aqueous layer was extracted with EtOAc (3 x 10 mL). The organic layers were combined, dried over sodium sulfate, and concentrated to a residue. The residue was purified first by SFC (Torus 2-PIC (19 x 100 mm, 5 µm, PN 186008586) with MeOH cosolvent with 0.25% diethylamine) then by reverse phase HPLC (C18 10X150 mm, eluting with a 25-100% MeCN in water gradient, 0.1% formic acid additive)) to give (1*S*,4*S*)-7-amino-4'-((*R**)-4-cyanoazepan-1-yl)-2'-(((2*R*,7a*S*)-2-fluorotetrahydro-1*H*-pyrrolizin-7a(5*H*)-yl)methoxy)-4-methyl-3,4,5',8'-tetrahydro-2H-spiro[naphthalene-1,7'-pyrano[4,3-d]pyrimidine]-8-carbonitrile (10.2 mg).
¹H NMR (400 MHz, MeOD) δ 7.03 (d, J = 8.7 Hz, 1H), 6.67 (d, J = 8.6 Hz, 1H), 5.44 - 5.27 (m, 1H), 5.03 - 4.93 (m, 1H), 4.83 - 4.77 (m, 1H), 4.32 - 4.24 (m, 1H), 4.24 - 4.16 (m, 1H), 3.84 - 3.44 (m, 7H), 3.20 - 3.00 (m, 3H), 2.95 - 2.75 (m, 2H), 2.49 - 2.24 (m, 2H), 2.22 - 1.70 (m, 13H), 1.66 - 1.58 (m, 1H), 1.15 (d, J = 7.1 Hz, 3H).
LCMS: m/z (ESI) [M+H]⁺ 586.4, t_{R} = 1.78 minutes (Method E)

### Example 27: 8-Fluoro-2'-(((2R,7aS)-2-fluorotetrahydro-1H-pyrrolizin-7a(5H)-yl)methoxy)-5-methyl-4'-(1,4-oxazepan-4-yl)-3,4,5',8'-tetrahydro-2H-spiro[naphthalene-1,7'-pyrano[4,3-d]pyrimidin]-7-amine (Compound 506a)

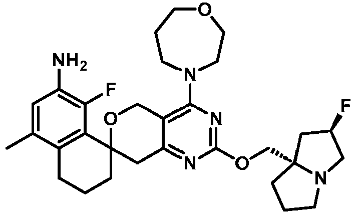

### Step 1: (4-Methyl-2-(methylthio)-6-(1,4-oxazepan-4-yl)pyrimidin-5-yl)methanol

To a 40 mL vial was combined 1,4-oxazepane, HCl (740mg, 1.1 *equiv.,* 5.38 mmol), (4-chloro-6-methyl-2-(methylthio)pyrimidin-5-yl)methanol (1000 mg, *1 equiv.,* 4.89 mmol), MeCN (12.2 mL), and DIPEA (1.90 g, 2.55 mL, 3 *equiv.,* 14.7 mmol) to yield a clear solution. The reaction was heated at 65 °C for 16 hours. At this point, the reaction was quenched with saturated sodium bicarbonate solution (30 mL) and extracted 5 times with EtOAc (5 x 30 mL). The organic layer was concentrated to yield (4-methyl-2-(methylthio)-6-(1,4-oxazepan-4-yl)pyrimidin-5-yl)methanol as a tan oil which was used directly in the next step (1.7 g).
¹H NMR (400 MHz, CDCl₃): δ 4.58 (d, 2H), 3.92 - 3.83 (m, 6H), 3.81 - 3.75 (m, 2H), 2.48 (s, 3H), 2.45 (s, 3H), 2.10 - 2.02 (m, 2H)

### Step 2: 4-(5-(((tert-Butyldimethylsilyl)oxy)methyl)-6-methyl-2-(methylthio)pyrimidin-4-yl)-1,4-oxazepane

To a 20 ml vial was combined (4-methyl-2-(methylthio)-6-(1,4-oxazepan-4-yl)pyrimidin-5-yl)methanol (500 mg, *1 equiv.,* 1.86 mmol), DCM (6.2 mL), imidazole (253 mg, 2 *equiv.,* 3.71 mmol), DMAP (22.7 mg, 0.1 *equiv.,* 186 µmol), and tert-butyl dimethylchlorosilane (308 mg, 340 µL, 1.1 *equiv.,* 2.04 mmol). The suspension was sealed and stirred at room temperature for 3 hours. The reaction was quenched with water (10 mL) and extracted 3 times with DCM (10 mL). The combined organics were concentrated and purified by silica gel chromatography (20% EtOAc in heptane) to yield 4-(5-(((*tert-*butyldimethylsilyl)oxy)methyl)-6-methyl-2-(methylthio)pyrimidin-4-yl)-1,4-oxazepane (500 mg) as a white solid.
¹H NMR (400 MHz, CDCl₃): δ 4.48 (s, 2H), 3.89 - 3.74 (m, 8H), 2.47 (s, 3H), 2.40 (s, 3H), 2.07 - 1.99 (m, 2H), 0.90 (s, 9H), 0.12 (s, 6H)

### Step 3: 4-(5-(((tert-Butyldimethylsilyl)oxy)methyl)-6-methyl-2-(methylsulfonyl)pyrimidin-4-yl)-1,4-oxazepane

To a 4 mL vial was combined 4-(5-(((*tert*-butyldimethylsilyl)oxy)methyl)-6-methyl-2-(methylthio)pyrimidin-4-yl)-1,4-oxazepane (500 mg, *1 equiv.,* 1.30 mmol), DCM (6.52 mL), and mCPBA (730 mg, 77 wt%, 2.5 *equiv.,* 3.26 mmol). The reaction was stirred at room temperature for 3 hours. The reaction was quenched with 3:1 saturated sodium bicarbonate:saturated sodium thiosulfate (5 mL) and extracted four times with DCM (5 mL). The combined organic layers were concentrated to yield 4-(5-(((*tert-*butyldimethylsilyl)oxy)methyl)-6-methyl-2-(methylsulfonyl)pyrimidin-4-yl)-1,4-oxazepane (500 mg).
¹H NMR (400 MHz, CDCl₃): δ 4.48 (s, 2H), 3.89 - 3.81 (m, 6H), 3.79 - 3.74 (m, 2H), 2.47 (s, 3H), 2.40 (s, 3H), 2.06 - 1.99 (m, 2H), 0.90 (s, 9H), 0.12 (s, 6H)

### Step 4: 4-(5-(((tert-Butyldimethylsilyl)oxy)methyl)-2-(((2R,7aS)-2-fluorotetrahydro-1H-pyrrolizin-7a(5H)-yl)methoxy)-6-methylpyrimidin-4-yl)-1,4-oxazepane

4-(5-(((*tert*-Butyldimethylsilyl)oxy)methyl)-6-methyl-2-(methylsulfonyl)pyrimidin-4-yl)-1,4-oxazepane (500 mg, 1 *equiv.,* 1.20 mmol) and ((2*S*,3a*R*)-2-fluorohexahydropentalen-3a(1*H*)-yl)methanol (190 mg, 1 *equiv.,* 1.20 mmol) were azeotroped with toluene two times to remove trace water. The resulting mixture was dissolved in THF (6.0 mL) and cooled to 0 °C. Sodium *tert*-pentoxide (159 mg, 1.44 mL, 1 M, 1.2 *equiv.,* 1.44 mmol) was then added dropwise. The resulting mixture was stirred for 1 hour at room temperature. At this point, the reaction mixture was quenched with HCl (4 M dioxane) (52.6 mg, 361 µL, 1.2 *equiv.,* 1*.*44 mmol), washed with sodium bicarbonate, and extracted three times with EtOAc. The combined organic layers were concentrated and purified by silica column (10% MeOH in DCM) to yield 4-(5-(((*tert*-butyldimethylsilyl)oxy)methyl)-2-(((2*R*,7a*S*)-2-fluorotetrahydro-1*H-*pyrrolizin-7a(5H)-yl)methoxy)-6-methylpyrimidin-4-yl)-1,4-oxazepane (500 mg) as a white solid.
LCMS: m/z (ESI) [M+H]⁺ 495.6, t_{R} = 1.40 minutes (Method M)

### Step 5: (2-(((2R,7aS)-2-Fluorotetrahydro-1H-pyrrolizin-7a(5H)-yl)methoxy)-4-methyl-6-(1,4-oxazepan-4-yl)pyrimidin-5-yl)methanol

4-(5-(((*tert*-Butyldimethylsilyl)oxy)methyl)-2-(((2*R*,7a*S*)-2-fluorotetrahydro-1*H-*pyrrolizin-7a(5*H*)-yl)methoxy)-6-methylpyrimidin-4-yl)-1,4-oxazepane (450 mg, *1 equiv.,* 910 µmol) was dissolved in MeOH (4.55 mL) and HCl (199 mg, 455 µL, 12 M, 6 *equiv., 5.46* mmol). The resulting mixture was stirred at room temperature for 16 hours. The mixture was concentrated to remove MeOH, quenched with saturated sodium bicarbonate solution, and extracted with 3:1 DCM:IPA. The combined organic layers were concentrated and suspended in EtOAc. The filtrate was then concentrated and purified on a silica gel chromatography (20 % MeOH in DCM) to yield (2-(((2*R*,7a*S*)-2-fluorotetrahydro-1*H*-pyrrolizin-7a(5H)-yl)methoxy)-4-methyl-6-(1,4-oxazepan-4-yl)pyrimidin-5-yl)methanol (140 mg) as a white solid.
LCMS: m/z (ESI) [M+H]⁺ 381.4, t_{R} = 0.68 minutes (Method M)

### Step 6: 7-Bromo-8-fluoro-1-((2-(((2R,7aS)-2-fluorotetrahydro-1H-pyrrolizin-7a(5H)-yl)methoxy)-5-(hydroxymethyl)-6-(1,4-oxazepan-4-yl)pyrimidin-4-yl)methyl)-5-methyl-1,2,3,4-tetrahydronaphthalen-1-ol

To an 8 mL vial was added (2-(((2*R*,7a*S*)-2-fluorotetrahydro-1*H*-pyrrolizin-7a(5*H*)-yl)methoxy)-4-methyl-6-(1,4-oxazepan-4-yl)pyrimidin-5-yl)methanol (100 mg, 1 *equiv.,* 0.26 mmol) in THF (0.8 mL). The resulting solution was cooled to -78 °C and a 1 M solution of LDA in THF (580 µL, 2.2 *equiv.,* 0.58 mmol) was added dropwise. This solution was stirred for 45 minutes at -78 °C. A solution of 7-bromo-8-fluoro-5-methyl-3,4-dihydronapthalen-1(2*H*)-one (67 mg, *1 equiv.,* 0.26 mmol) in THF (0.25 mL) was then added dropwise at -78 °C. The resulting reaction mixture was then stirred at -78 °C for 1 hour and quenched with the addition of saturated sodium bicarbonate solution and EtOAc (2 mL each). The layers were separated, and the aqueous layer was extracted five times with EtOAc (5 x 2 mL) and two times with 3:1 DCM: IPA solution (2 mL each). The combined organics are then dried with sodium sulfate, filtered, and concentrated. The crude mixture was purified on a silica gel chromatography (0 to 15% MeOH in DCM) to yield 7-bromo-8-fluoro-1-((2-(((2*R*,7a*S*)-2-fluorotetrahydro-1*H*-pyrrolizin-7a(5*H*)-yl)methoxy)-5-(hydroxymethyl)-6-(1,4-oxazepan-4-yl)pyrimidin-4-yl)methyl)-5-methyl-1,2,3,4-tetrahydronaphthalen-1-ol (58.7 mg) as an off white solid.
LCMS: m/z (ESI) [M+H]⁺ 639.2, t_{R} = 1.96 minutes (Method E)

### Step 7: 7-Bromo-8-fluoro-2'-(((2R,7aS)-2-fluorotetrahydro-1H-pyrrolizin-7a(5H)-yl)methoxy)-5-methyl-4'-(1,4-oxazepan-4-yl)-3,4,5',8'-tetrahydro-2H-spiro[naphthalene-1,7'-pyrano[4,3-d]pyrimidine]

To a 4 mL vial was added 7-bromo-8-fluoro-1-((2-(((2*R*,7a*S*)-2-fluorotetrahydro-1*H-*pyrrolizin-7a(5*H*)-yl)methoxy)-5-(hydroxymethyl)-6-(1,4-oxazepan-4-yl)pyrimidin-4-yl)methyl)-5-methyl-1,2,3,4-tetrahydronaphthalen-1-ol (52.2 mg, *1 equiv.,* 0.082 mmol), toluene (0.5 mL), and phosphoric acid (15 µl, 3 *equiv.,* 0.24 mmol). This mixture was heated to 100 °C for 3 hours at which point saturated sodium bicarbonate solution and EtOAc were added (3 mL each). The layers were separated, and the aqueous layer was extracted four more times with EtOAc (3 mL each). The combined organic layers were dried with sodium sulfate, filtered, and concentrated to yield 7-bromo-8-fluoro-2'-(((2*R*,7a*S*)-2-fluorotetrahydro-1*H-*pyrrolizin-7a(5*H*)-yl)methoxy)-5-methyl-4'-(1,4-oxazepan-4-yl)-3,4,5',8'-tetrahydro-2*H-*spiro[naphthalene-1,7'-pyrano[4,3-d]pyrimidine] (50 mg) as a white solid.
LCMS: m/z (ESI) [M+H]⁺ 619.2, t_{R} = 2.01 minutes (Method E)

### Step 8: tert-Butyl (8-fluoro-2'-(((2R,7aS)-2-fluorotetrahydro-1H-pyrrolizin-7a(5H)-yl)methoxy)-5-methyl-4'-(1,4-oxazepan-4-yl)-3,4,5',8'-tetrahydro-2H-spiro[naphthalene-1,7'-pyrano[4,3-d]pyrimidin]-7-yl)carbamate

A solution of 7-bromo-8-fluoro-2'-(((2*R*,7a*S*)-2-fluorotetrahydro-1*H*-pyrrolizin-7a(5*H*)-yl)methoxy)-5-methyl-4'-(1,4-oxazepan-4-yl)-3,4,5',8'-tetrahydro-2H-spiro[naphthalene-1,7'-pyrano[4,3-d]pyrimidine] (46 mg, *1 equiv.,* 0.074 mmol) in 1,4 dioxane (1.25 mL) was degassed by bubbling nitrogen through for 15 minutes. To a separated vial was weighed BrettPhos Pd G4 (7.5 mg, 0.11 *equiv.,* 0.0082 mmol), *tert*-butyl carbamate (9.6 mg, *1.1 equiv.,* 0.082 mmol), and cesium carbonate (25 mg, 1.03 *equiv.,* 0.076 mmol). This 8 mL vial was evacuated and back filled with nitrogen three times. A solution of 7-bromo-8-fluoro-2'-(((2*R*,7a*S*)-2-fluorotetrahydro-1*H*-pyrrolizin-7a(5*H*)-yl)methoxy)-5-methyl-4'-(1,4-oxazepan-4-yl)-3,4,5',8'-tetrahydro-2*H*-spiro[naphthalene-1,7'-pyrano[4,3-d]pyrimidine] was then transferred to the vial with the solids and purged again with nitrogen for 15 minutes. The mixture was then stirred at 80 °C for 3 hours. At this point, an aliquot was analyzed by LCMS which indicated complete consumption of starting material. The reaction was then left in a -26 °C freezer for 16 hours. The mixture was then diluted with water and extracted with EtOAc twice (5 mL each). The combined organics were dried with sodium sulfate, filtered, and concentrated to a residue which was purified by silica gel column (eluting with 0 to 10% MeOH) to yield *tert*-butyl (8-fluoro-2'-(((2*R*,7a*S*)-2-fluorotetrahydro-1*H*-pyrrolizin-7a(5*H*)-yl)methoxy)-5-methyl-4'-(1,4-oxazepan-4-yl)-3,4,5',8'-tetrahydro-2*H*-spiro[naphthalene-1,7'-pyrano[4,3-*d*]pyrimidin]-7-yl)carbamate (30.9 mg) as a white solid.
LCMS: m/z (ESI) [M+H]⁺ 656.4, t_{R} = 2.09 minutes (Method E)

### Step 9: 8-Fluoro-2'-(((2R,7aS)-2-fluorotetrahydro-1H-pyrrolizin-7a(5H)-yl)methoxy)-5-methyl-4'-(1,4-oxazepan-4-yl)-3,4,5',8'-tetrahydro-2H-spiro[naphthalene-1,7'-pyrano[4,3-d]pyrimidin]-7-amine

To a vial was added *tert*-butyl (8-fluoro-2'-(((2*R*,7a*S*)-2-fluorotetrahydro-1*H-*pyrrolizin-7a(5*H*)-yl)methoxy)-5-methyl-4'-(1,4-oxazepan-4-yl)-3,4,5',8'-tetrahydro-2H-spiro[naphthalene-1,7'-pyrano[4,3-*d*]pyrimidin]-7-yl)carbamate (25.9 mg, 1 *equiv.,* .0395 mmol) in DCM (1 mL). HCl as a 1 M solution in Et₂O was added (0.59 mL, 30 *equiv.,* 1.18 mmol). This mixture was stirred at 25 °C for 4 hours during which time a precipitate began forming. After four hours, the filtrate was removed, and the solid was triturated three times with 3 mL of Et₂O. This light yellow solid was then purified via reverse phase chromatography (XSelect CSH Prep C18 5 µm OBD, 19x150 mm Column eluting with 5 to 25% MeCN in H₂O (0.1% TFA additive to both solvents)). The fractions containing the desired product were collected and concentrated. These were then resuspended in a sodium bicarbonate solution (20 mL) which was extracted with EtOAc three times (20 mL each). The combined organic layers were then dried with sodium sulfate, filtered, and concentrated to yield 8-fluoro-2'-(((2R,7aS)-2-fluorotetrahydro-1*H*-pyrrolizin-7a(5*H*)-yl)methoxy)-5-methyl-4'-(1,4-oxazepan-4-yl)-3,4,5',8'-tetrahydro-2*H*-spiro[naphthalene-1,7'-pyrano[4,3-*d*]pyrimidin]-7-amine (8 mg) as a white solid.
LCMS: m/z (ESI) [M+H]⁺ 556.5, t_{R} = 1.52 minutes (Method E)
¹H NMR (400 MHz, CDCl₃): δ 6.60 (d, 1H), 5.27 (d, 1H), 4.81 (d, 1H), 4.73 (d, 1H), 4.15-3.96 (m, 2H), 3.90 - 3.57 (m, 8H), 3.58 - 3.40 (m, 2H), 3.40 - 3.16 (m, 2H), 3.03 - 2.86 (m, 2H), 2.61 - 2.45 (m, 2H), 2.37 - 2.14 (m, 3H), 2.11 (s, 3H), 2.07 - 1.63 (m, 11H)

### Example 28: 7-Amino-2'-(((2R,7aS)-2-fluorotetrahydro-1H-pyrrolizin-7a(5H)-yl)methoxy)-5-methyl-4'-(1,4-oxazepan-4-yl)-3,4,5',8'-tetrahydro-2H-spiro[naphthalene-1,7'-pyrano[4,3-d]pyrimidine]-8-carbonitrile (Compound 505a)

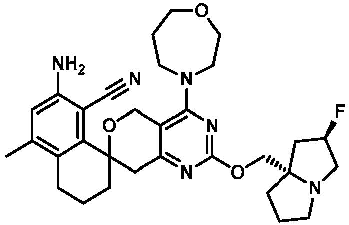

### Step 1: 7-Amino-5-methyl-3,4-dihydronaphthalen-1(2H)-one

7-Bromo-5-methyl-3,4-dihydronaphthalen-1(2H)-one (2.46 g, 1 *equiv.,* 10.3 mmol), cuprous oxide (147 mg, 0.1 *equiv.,* 1.03 mmol), NH₄OH (8.20 mL, 20 *equiv.,* 206 mmol), and NMP (7.7 mL) were combined in a 150 mL pressure vial and stirred at 85 °C for 18 hours. At this point, a second portion of cuprous oxide (147 mg, 0.1 *equiv.,* 1.03 mmol) and NH₄OH (8.20 mL, 20 *equiv.,* 206 mmol) were added and the resulting mixture was stirred again at 85 °C for another 24 hours. The reaction was then quenched by the addition of water (100 mL) and EtOAc (100 mL). The layers were separated, and the aqueous layer was then extracted twice more with EtOAc (2 x 100 mL). The combined organic layers were washed with brine and dried with sodium sulfate. The organic layer was then concentrated to a sticky brown solid. The solid was triturated with Et₂O three times to give 7-amino-5-methyl-3,4-dihydronaphthalen-1(2*H*)-one (1.26 g) as a brown solid which was used without further purification.

¹H NMR (400 MHz, CDCl₃): δ 7.29 - 7.26 (m, 1H), 6.82 - 6.77 (m, 1H), 2.80 - 2.72 (m, 2H), 2.62 - 2.56 (m, 2H), 2.24 (s, 3H), 2.14 - 2.06 (m, 2H).

### Step 2: 7-Amino-8-bromo-5-methyl-3,4-dihydronaphthalen-1(2H)-one

To a flask was added 7-amino-5-methyl-3,4-dihydronaphthalen-1(2*H*)-one (1.26 g, 1 *equiv.,* 7.19 mmol) and DMF (12.8 mL). *N*-bromosuccinimide (1.28 g, 1 *equiv.,* 7.19 mmol) was added as a solution in DMF (6.3 mL) at 0 °C dropwise via syringe. The reaction was then stirred for 5 minutes at 0 °C and then warmed to room temperature. The reaction was stirred for 90 minutes at which point TLC analysis indicated complete consumption of starting material. The reaction was quenched by the addition of water (30 mL) and EtOAc (30 mL). The mixture was extracted twice more with EtOAc (2 x 30 mL). The combined organic layers were then washed three times with a freshly prepared 10 % lithium chloride solution in water (3 x 30 mL). The organic layer was then washed with brine (50 mL), dried over sodium sulfate, and concentrated to give 7-amino-8-bromo-5-methyl-3,4-dihydronaphthalen-1(2H)-one (1.61 g) as a red solid which was used without further purification.
¹H NMR (400 MHz, CDCl₃): δ 6.90 - 6.84 (m, 1H), 2.81 - 2.74 (m, 2H), 2.69 - 2.61 (m, 2H), 2.20 (s, 3H), 2.14 - 2.05 (m, 2H)

### Step 3: 8-Bromo-7-(dibenzylamino)-5-methyl-3,4-dihydronaphthalen-1(2H)-one

To a 100 mL flask was added 7-amino-8-bromo-5-methyl-3,4-dihydronaphthalen-1(2*H*)-one (1.45 g, 1 *equiv.,* 5.71 mmol), potassium carbonate (2.76 g, 3.5 *equiv.,* 20 mmol), and DMF (28.5 mL). To this solution, benzyl bromide (3.42 g, 2.38 mL, 3.5 *equiv.,* 20 mmol) was added dropwise. The resulting mixture was stirred at 80 °C for 24 hours. At this point, a second portion of potassium carbonate (2.76 g, 3.5 *equiv.,* 20 mmol) and benzyl bromide (3.42 g, 2.38 mL, 3.5 *equiv.,* 20 mmol) were added and the resulting reaction was stirred at 90 °C for another 21 hours. The reaction was then quenched with water (100 mL) and EtOAc (100 mL). The layers were separated, and the aqueous layer was extracted twice more with EtOAc (100 mL). The combined organics were then washed three times with freshly prepared 10% lithium chloride solution (3 x 50 mL) and then brine (50 mL). The organics were then dried with sodium sulfate, filtered, and concentrated to a residue which was purified by column chromatography (150 g C18 gold column eluting with 10 to 95% MeCN (0.1% formic acid) in H₂O (0.1% formic acid)). Fractions containing the desired product were combined and diluted with saturated sodium bicarbonate solution and EtOAc (50 mL each). The layers were separated, and the organics washed with saturated sodium bicarbonate solution (50 mL). The combined aqueous layers were then back extracted once with EtOAc (50 mL). The combined organics were dried with sodium sulfate, filtered, and concentrated to yield 8-bromo-7-(dibenzylamino)-5-methyl-3,4-dihydronaphthalen-1(2*H*)-one (1.35 g) as a brown solid.
¹H NMR (400 MHz, CDCN): δ 7.45 - 7.40 (m, 4H), 7.30 - 7.25 (m, 4H), 7.24 - 7.18 (m, 2H), 7.16 (s, 1H), 4.14 (s, 4H), 2.79 - 2.73 (m, 2H), 2.62 - 2.56 (m, 2H), 2.14 (s, 3H), 2.07 - 1.99 (m, 2H)

### Step 4: 8-Bromo-7-(dibenzylamino)-1-((2-(((2R,7aS)-2-fluorotetrahydro-1H-pyrrolizin-7a(5H)-yl)methoxy)-5-(hydroxymethyl)-6-(1,4-oxazepan-4-yl)pyrimidin-4-yl)methyl)-5-methyl-1,2,3,4-tetrahydronaphthalen-1-ol

To an 8 mL vial was added (2-(((2*R*,7a*S*)-2-fluorotetrahydro-1*H*-pyrrolizin-7a(5*H*)-yl)methoxy)-4-methyl-6-(1,4-oxazepan-4-yl)pyrimidin-5-yl)methanol (100 mg, 1 *equiv.,* 0.26 mmol) in THF (0.8 mL). The resulting solution was cooled to -78 °C and a 1 M solution of LDA in THF (580 µL, 2.2 *equiv.,* 0.58 mmol) was added dropwise. This solution was stirred for 45 minutes at -78 °C. A solution of 8-bromo-7-(dibenzylamino)-5-methyl-3,4-dihydronaphthalen-1(2*H*)-one (114 mg, *1 equiv.,* 0.26 mmol) in THF (0.25 mL) was then added dropwise at -78 °C. The reaction mixture was then stirred at -78 °C for 45 minutes and quenched with the addition of saturated sodium bicarbonate solution and EtOAc (2 mL each). The layers were separated, and the aqueous layer was extracted five times with EtOAc (5 x 2 mL) and once with a 10% MeOH in DCM solution (2 mL each). The combined organics are then dried with sodium sulfate, filtered, and concentrated to a residue which was purified by silica gel chromatography (0 to 10% MeOH in DCM) to yield 8-bromo-7-(dibenzylamino)-1-((2-(((2*R*,7a*S*)-2-fluorotetrahydro-1*H*-pyrrolizin-7a(5*H*)-yl)methoxy)-5-(hydroxymethyl)-6-(1,4-oxazepan-4-yl)pyrimidin-4-yl)methyl)-5-methyl-1,2,3,4-tetrahydronaphthalen-1-ol (79.2 mg) as an off white solid.
LCMS: m/z (ESI) [M+H]⁺ 816.4, t_{R} = 2.63 minutes (Method E)

### Step 5: N,N-Dibenzyl-8-bromo-2'-(((2R,7aS)-2-fluorotetrahydro-1H-pyrrolizin-7a(5H)-yl)methoxy)-5-methyl-4'-(1,4-oxazepan-4-yl)-3,4,5',8'-tetrahydro-2H-spiro[naphthalene-1,7'-pyrano[4,3-d]pyrimidin]-7-amine

To a 4 mL vial was added 8-bromo-7-(dibenzylamino)-1-((2-(((2R,7aS)-2-fluorotetrahydro-1*H*-pyrrolizin-7a(5*H*)-yl)methoxy)-5-(hydroxymethyl)-6-(1,4-oxazepan-4-yl)pyrimidin-4-yl)methyl)-5-methyl-1,2,3,4-tetrahydronaphthalen-1-ol (59.2 mg, *1 equiv.,* 0.073 mmol), toluene (0.5 mL), and phosphoric acid (13.7 µl, 3 *equiv.,* 0.22 mmol). This mixture was heated to 100 °C and stirred for 2.5 hours at which point saturated sodium bicarbonate solution and EtOAc were added (3 mL each). The layers were separated, and the aqueous layer was extracted four more times with EtOAc (3 mL each). The combined organic layers were dried with sodium sulfate, filtered, and concentrated to yield N,N-dibenzyl-8-bromo-2'-(((2*R*,7a*S*)-2-fluorotetrahydro-1*H*-pyrrolizin-7a(5*H*)-yl)methoxy)-5-methyl-4'-(1,4-oxazepan-4-yl)-3,4,5',8'-tetrahydro-2H-spiro[naphthalene-1,7'-pyrano[4,3-d]pyrimidin]-7-amine (69.6 mg) as a white solid which was used without further purification.
LCMS: m/z (ESI) [M+H]⁺ 796.4, t_{R} = 2.93 minutes (Method E)

### Step 6: 7-(Dibenzylamino)-2'-(((2R,7aS)-2-fluorotetrahydro-1H-pyrrolizin-7a(5H)-yl)methoxy)-5-methyl-4'-(1,4-oxazepan-4-yl)-3,4,5',8'-tetrahydro-2H-spiro[naphthalene-1,7'-pyrano[4,3-d]pyrimidine]-8-carbonitrile

To a vial was added *N,N*-dibenzyl-8-bromo-2'-(((2*R*,7a*S*)-2-fluorotetrahydro-1*H-*pyrrolizin-7a(5*H*)-yl)methoxy)-5-methyl-4'-(1,4-oxazepan-4-yl)-3,4,5',8'-tetrahydro-2H-spiro[naphthalene-1,7'-pyrano[4,3-d]pyrimidin]-7-amine (57.5 mg, *1 equiv.,* 0.073 mmol) and copper(I) cyanide (78 mg, 12 *equiv.,* 0.87 mmol). DMF (1.7 mL) was then added and the resulting mixture was heated to 100 °C for four hours. The reaction was then diluted with 10% NH₄OH solution in water (5 mL) and a 10% MeOH in DCM (3 mL). The layers were separated, and the aqueous layer was extracted three more times with a 10% MeOH in DCM solution (3 mL each). The combined organics were washed with brine, dried with sodium sulfate, filtered, and concentrated to a residue which was purified by silica gel chromatography (0 to 10% MeOH in DCM) to yield 7-(dibenzylamino)-2'-(((2*R*,7a*S*)-2-fluorotetrahydro-1*H*-pyrrolizin-7a(5*H*)-yl)methoxy)-5-methyl-4'-(1,4-oxazepan-4-yl)-3,4,5',8'-tetrahydro-2*H-*spiro[naphthalene-1,7'-pyrano[4,3-d]pyrimidine]-8-carbonitrile (18.4 mg) as an off white solid.
LCMS: m/z (ESI) [M+H]⁺ 743.5, t_{R} = 2.64 minutes (Method E)

### Step 7: 7-Amino-2'-(((2R,7aS)-2-fluorotetrahydro-1H-pyrrolizin-7a(5H)-yl)methoxy)-5-methyl-4'-(1,4-oxazepan-4-yl)-3,4,5',8'-tetrahydro-2H-spiro[naphthalene-1,7'-pyrano[4,3-d]pyrimidine]-8-carbonitrile

To a vial was added 7-(dibenzylamino)-2'-(((2*R*,7a*S*)-2-fluorotetrahydro-1*H-*pyrrolizin-7a(5*H*)-yl)methoxy)-5-methyl-4'-(1,4-oxazepan-4-yl)-3,4,5',8'-tetrahydro-2*H-*spiro[naphthalene-1,7'-pyrano[4,3-d]pyrimidine]-8-carbonitrile (16.7 mg, 1 *equiv.,* 0.023 mmol) and Pd(OH)₂ on carbon (39.5 mg, 20 wt%, 2.5 *equiv.,* 0.056 mmol). The vial was then evacuated and back filled with nitrogen three times. MeOH (0.5 mL) was then added, and a hydrogen balloon was introduced. Hydrogen was bubbled through the solution for 5 minutes and then acetic acid (5 µl, 4 *equiv.,* 0.090 mmol) was added. The reaction mixture was stirred at room temperature for two hours under the hydrogen balloon. After 2 hours, LCMS indicated complete consumption of starting material. The reaction was diluted with more MeOH (2 mL) and filtered through a pipette with a Kim wipe plug. The reaction mixture was then stirred with sodium carbonate (100 mg) for 5 minutes. The mixture was then filtered again and concentrated to a white solid. This crude solid was purified via reverse phase chromatography (XSelect CSH Prep C18 5 µm OBD, 19x150 mm Column eluting with 15 to 35% MeCN in H₂O (0.1% TFA additive)). The fractions containing the desired product were collected and concentrated. These were then re-suspended in a sodium bicarbonate solution (20 mL) which was extracted with EtOAc three times (20 mL each). The combined organic layers were then dried with sodium sulfate, filtered, and concentrated to yield 7-amino-2'-(((2*R*,7a*S*)-2-fluorotetrahydro-1*H-*pyrrolizin-7a(5*H*)-yl)methoxy)-5-methyl-4'-(1,4-oxazepan-4-yl)-3,4,5',8'-tetrahydro-2*H-*spiro[naphthalene-1,7'-pyrano[4,3-d]pyrimidine]-8-carbonitrile (5.2 mg).
LCMS: m/z (ESI) [M+H]⁺ 563.4, t_{R} = 1.70 minutes (Method E)
¹H NMR (400 MHz, CDCN): δ 6.64 (s, 1H), 5.26 (d, 1H), 4.92 (d, 1H), 4.76 - 4.66 (m, 3H), 4.13 - 3.96 (m, 2H), 3.85 - 3.53 (m, 8H), 3.32 - 3.09 (m, 4H), 3.03 - 2.85 (m, 2H), 2.67 - 2.57 (m, 1H), 2.53 - 2.40 (m, 1H), 2.30 - 2.21 (m, 3H), 2.20 (s, 3H), 2.19 - 1.82 (m, 7H), 1.81 - 1.69 (m, 2H)

### Example 29: 5-Chloro-8-fluoro-2'-(((2R,7aS)-2-fluorotetrahydro-1H-pyrrolizin-7a(5H)-yl)methoxy)-4-methyl-4'-(1,4-oxazepan-4-yl)-3,4,5',8'-tetrahydro-2H-spiro[naphthalene-1,7'-pyrano[4,3-d]pyrimidin]-7-amine (Compound 508a)

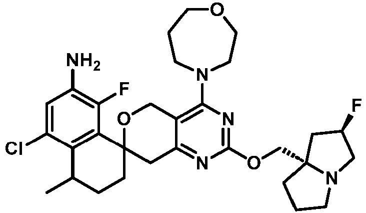

### Step 1: 7-Bromo-5-chloro-8-fluoro-4-methyl-3,4-dihydronaphthalen-1(2H)-one

To a 40 mL vial was added allyl acetic acid (0.20 mL, *1 equiv.,* 2.0 mmol) and 2-bromo-4-chloro-1-fluorobenzene (2.0 g, 4.8 *equiv.,* 9.5 mmol). AlCl₃ (2.7 g, 10 *equiv.,* 20 mmol) was added and the mixture was stirred at 110 °C for 12 hours. At this point, the reaction mixture was quenched with a 1:1 mixture of saturated sodium bicarbonate and saturated Rochelle's salt (100 mL). The aqueous layer was extracted twice with EtOAc (2 x 100 mL) and the combined organics were washed once with brine, dried with sodium sulfate, filtered, and concentrated to an orange oil. The crude mixture was purified by silica gel chromatography (0 to 50% DCM in heptane) to yield 7-bromo-5-chloro-8-fluoro-4-methyl-3,4-dihydronaphthalen-1(2H)-one (188 mg) as a pink solid.
¹H NMR (400 MHz, CDCl₃): δ 7.75 (d, 1H), 3.54 - 3.44 (m, 1H), 2.91 - 2.79 (m, 1H), 2.69 - 2.59 (m, 1H), 2.33 - 2.19 (m, 1H), 2.07 - 1.97 (m, 1H), 1.34 (d, 3H)

### Step 2: 7-Bromo-5-chloro-8-fluoro-1-((2-(((2R,7aS)-2-fluorotetrahydro-1H-pyrrolizin-7a(5H)-yl)methoxy)-5-(hydroxymethyl)-6-(1,4-oxazepan-4-yl)pyrimidin-4-yl)methyl)-4-methyl-1,2,3,4-tetrahydronaphthalen-1-ol

To an 8 mL vial was added (2-(((2*R*,7a*S*)-2-fluorotetrahydro-1*H*-pyrrolizin-7a(5*H*)-yl)methoxy)-4-methyl-6-(1,4-oxazepan-4-yl)pyrimidin-5-yl)methanol (95 mg, 1 *equiv.,* 0.25 mmol) and THF (0.8 mL). The resulting solution was cooled to -78 °C and a 1 M solution of LDA in THF (550 µL, 2.2 *equiv.,* 0.55 mmol) was added dropwise. This solution was stirred for 45 minutes at -78 °C. A solution of 7-bromo-5-chloro-8-fluoro-4-methyl-3,4-dihydronaphthalen-1(2*H*)-one (73 mg, *1 equiv.,* 0.25 mmol) in THF (0.25 mL) was then added dropwise at -78 °C. The reaction mixture was then stirred at -78 °C for 1 hour and quenched with the addition of saturated sodium bicarbonate solution and EtOAc (2 mL each). The layers were separated, and the aqueous layer was extracted four times with EtOAc (4 x 3 mL). The combined organics were then dried with sodium sulfate, filtered, and concentrated. The crude mixture was purified by silica gel chromatography (0 to 10% MeOH in DCM) to yield 7-bromo-5-chloro-8-fluoro-1-((2-(((2*R*,7a*S*)-2-fluorotetrahydro-1*H*-pyrrolizin-7a(5*H*)-yl)methoxy)-5-(hydroxymethyl)-6-(1,4-oxazepan-4-yl)pyrimidin-4-yl)methyl)-4-methyl-1,2,3,4-tetrahydronaphthalen-1-ol (73.8 mg) as a yellow solid.
LCMS: m/z (ESI) [M+H]⁺ 671.1, t_{R} = 2.09 minutes (Method E)

### Step 3: 7-Bromo-5-chloro-8-fluoro-2'-(((2R,7aS)-2-fluorotetrahydro-1H-pyrrolizin-7a(5H)-yl)methoxy)-4-methyl-4'-(1,4-oxazepan-4-yl)-3,4,5',8'-tetrahydro-2H-spiro[naphthalene-1,7'-pyrano[4,3-d]pyrimidine]

To an 8 mL vial was added 7-bromo-5-chloro-8-fluoro-1-((2-(((2*R*,7a*S*)-2-fluorotetrahydro-1*H*-pyrrolizin-7a(5*H*)-yl)methoxy)-5-(hydroxymethyl)-6-(1,4-oxazepan-4-yl)pyrimidin-4-yl)methyl)-4-methyl-1,2,3,4-tetrahydronaphthalen-1-ol (65.5 mg, *1 equiv.,* 0.098 mmol) in toluene (0.5 mL). Phosphoric acid (18 µl, 885 wt%, 3 *equiv.,* 0.29 mmol) was then added and the resulting reaction mixture was stirred for 3 hours. At which point, the reaction was quenched with saturated sodium bicarbonate solution and extracted five times with EtOAc (5 x 2 mL). The combined organics were dried with sodium sulfate, filtered, and concentrated to yield 7-bromo-5-chloro-8-fluoro-2'-(((2*R*,7a*S*)-2-fluorotetrahydro-1*H-*pyrrolizin-7a(5*H*)-yl)methoxy)-4-methyl-4'-(1,4-oxazepan-4-yl)-3,4,5',8'-tetrahydro-2*H-*spiro[naphthalene-1,7'-pyrano[4,3-*d*]pyrimidine] (62 mg) as an off white solid, which was used without further purification.
LCMS: m/z (ESI) [M+H]⁺ 653.1, t_{R} = 2.47 minutes (Method E)

### Step 4: tert-Butyl (5-chloro-8-fluoro-2'-(((2R,7aS)-2-fluorotetrahydro-1H-pyrrolizin-7a(5H)-yl)methoxy)-4-methyl-4'-(1,4-oxazepan-4-yl)-3,4,5',8'-tetrahydro-2H-spiro[naphthalene-1,7'-pyrano[4,3-d]pyrimidin]-7-yl)carbamate

A solution of 7-bromo-5-chloro-8-fluoro-2'-(((2*R*,7a*S*)-2-fluorotetrahydro-1*H-*pyrrolizin-7a(5*H*)-yl)methoxy)-4-methyl-4'-(1,4-oxazepan-4-yl)-3,4,5',8'-tetrahydro-2*H-*spiro[naphthalene-1,7'-pyrano[4,3-d]pyrimidine] (60 mg, *1 equiv.,* 0.092 mmol) in 1,4 dioxane (1.6 mL) was degassed by bubbling nitrogen through for 30 minutes. To a separated vial was added BrettPhos Pd G4 (9.3 mg, 0.11 *equiv.,* 0.010 mmol), tert-butyl carbamate (12 mg, 1.1 *equiv.,* 0.010 mmol), and cesium carbonate (31 mg, 1.03 *equiv.,* 0.094 mmol). This 8 mL vial was evacuated and back filled with nitrogen three times. The solution of 7-bromo-5-chloro-8-fluoro-2'-(((2*R*,7a*S*)-2-fluorotetrahydro-1*H*-pyrrolizin-7a(5*H*)-yl)methoxy)-4-methyl-4'-(1,4-oxazepan-4-yl)-3,4,5',8'-tetrahydro-2H-spiro[naphthalene-1,7'-pyrano[4,3-d]pyrimidine] was then transferred to the vial with the solids and degassed again with nitrogen for 10 minutes. The mixture was then stirred at 80 °C for 2 hours. At this point an aliquot was analyzed by LCMS which indicated some starting material remaining. Dioxane (800 µL) was added to the mixture along with a second portion BrettPhos Pd G4 (9.3 mg, 0.11 *equiv.,* 0.010 mmol). The mixture was degassed again for 10 minutes and stirred at 80 °C. After 3 hours, the starting material was completely consumed by LCMS. The reaction was then left in a -26 °C freezer for 16 hours. The mixture was then diluted with water and extracted with EtOAc twice (3 mL each). The combined organics were dried with sodium sulfate, filtered, and concentrated to a residue which was purified on a silica gel (column eluting with 0 to 10% MeOH) to yield *tert-*butyl (5-chloro-8-fluoro-2'-(((2*R*,7a*S*)-2-fluorotetrahydro-1*H*-pyrrolizin-7a(5*H*)-yl)methoxy)-4-methyl-4'-(1,4-oxazepan-4-yl)-3,4,5',8'-tetrahydro-2*H*-spiro[naphthalene-1,7'-pyrano[4,3-*d*]pyrimidin]-7-yl)carbamate (37.1 mg).
LCMS: m/z (ESI) [M+H]⁺ 690.3, t_{R} = 2.48 minutes (Method E)

### Step 5: 5-Chloro-8-fluoro-2'-(((2R,7aS)-2-fluorotetrahydro-1H-pyrrolizin-7a(5H)-yl)methoxy)-4-methyl-4'-(1,4-oxazepan-4-yl)-3,4,5',8'-tetrahydro-2H-spiro[naphthalene-1,7'-pyrano[4,3-d]pyrimidin]-7-amine

To a vial was added tert-butyl (5-chloro-8-fluoro-2'-(((2*R*,7a*S*)-2-fluorotetrahydro-1*H-*pyrrolizin-7a(5*H*)-yl)methoxy)-4-methyl-4'-(1,4-oxazepan-4-yl)-3,4,5',8'-tetrahydro-2*H-*spiro[naphthalene-1,7'-pyrano[4,3-d]pyrimidin]-7-yl)carbamate (34.5 mg, 1 *equiv.,* 0.050 mmol), which was dissolved in DCM (1 mL). A solution of 2 M HCl in Et₂O (0.75 mL, 30 *equiv.,* 1.50 mmol) was then added and the resulting mixture was stirred at room temperature. A precipitate began to form, after stirring for 3 hours. LCMS indicated complete consumption of starting material. The filtrate was then removed, and the resulting solid was triturated three times with 3 mL of Et₂O. The resulting white solid was left to dry to yield 5-chloro-8-fluoro-2'-(((2*R*,7a*S*)-2-fluorotetrahydro-1*H*-pyrrolizin-7a(5*H*)-yl)methoxy)-4-methyl-4'-(1,4-oxazepan-4-yl)-3,4,5',8'-tetrahydro-2H-spiro[naphthalene-1,7'-pyrano[4,3-*d*]pyrimidin]-7-amine (HCl salt) (27.1 mg).
¹H NMR (400 MHz, CDCl₃): δ 7.46 - 7.19 (m, 1H), 5.61 (d, 1H), 4.97 - 4.70 (m, 1H), 4.75 (s, 3H), 4.20 - 3.80 (m, 11H), 3.81 - 3.70 (m, 1H), 3.52 - 3.41 (m, 1H), 3.38 - 3.21 (m, 2H), 3.14 - 3.01 (m, 1H), 2.77 - 2.57 (m, 2H), 2.54 -1.83 (m, 11H), 1.39 - 1.25 (m, 3H)
LCMS: m/z (ESI) [M+H]⁺ 590.3, t_{R} = 1.96 minutes (Method E)

### Example 30: 5-((R)-7-amino-8-fluoro-2'-(((2R,7aS)-2-fluorotetrahydro-1H-pyrrolizin-7a(5H)-yl)methoxy)-5-methyl-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide (Compound 501c)

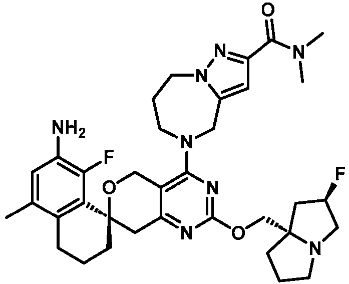

The diastereomers of 5-(7-amino-8-fluoro-2'-(((2*R*,7a*S*)-2-fluorotetrahydro-1*H-*pyrrolizin-7a(5*H*)-yl)methoxy)-5-methyl-3,4,5',8'-tetrahydro-2H-spiro[naphthalene-1,7'-pyrano[4,3-*d*]pyrimidin]-4'-yl)-*N,N*-dimethyl-5,6,7,8-tetrahydro-4*H*-pyrazolo[1,5-*a*][1,4]diazepine-2-carboxamide **(Compound 501a)** (8 mg) were separated via SFC (Chiralpak IB-N (21 x 250 mm, 5 µm, P/N 88445), 40% isocratic MeOH with 0.25% diethylamine) to afford 5-((*R*)-7-amino-8-fluoro-2'-(((2*R*,7a*S*)-2-fluorotetrahydro-1*H*-pyrrolizin-7a(5*H*)-yl)methoxy)-5-methyl-3,4,5',8'-tetrahydro-2*H*-spiro[naphthalene-1,7'-pyrano[4,3-d*]*pyrimidin]-4'-yl)-*N,N-*dimethyl-5,6,7,8-tetrahydro-4*H*-pyrazolo[1,5-*a*][1,4]diazepine-2-carboxamide **(Peak 1)** (1.5 mg).
**Peak 1:** LCMS: m/z (ESI) [M+H]⁺ 663.6, t_{R} = 3.97 min, Chiralpak IB-U (3.0 x 100 mm, 1.6 µm, P/N 81U83) with 40% isocratic cosolvent (methanol with 0.25% diethylamine).
¹H NMR (400 MHz, CD₃OD) δ 6.68 (d, 1H), 6.60 (s, 1H), 5.27 (m, 1H), 4.76 (m, 2H), 4.66 (m, 1H), 4.49 (m, 2H), 4.10 (m, 1H), 4.02 (m, 1H), 3.97 - 3.86 (m, 2H), 3.34 (m, 3H), 3.25 - 3.14 (m, 3H), 3.06 (s, 3H), 3.02 - 2.94 (m, 1H), 2.85 (m, 2H), 2.57 (m, 2H), 2.31 - 2.15 (m, 3H), 2.12 (m, 4H), 2.07 (m, 2H), 1.97 (m, 5H), 1.86 (m, 1H), 1.70 (m, 1H).

### Example 31: 5-((S)-7-amino-8-fluoro-2'-(((2R,7aS)-2-fluorotetrahydro-1H-pyrrolizin-7a(5H)-yl)methoxy)-5-methyl-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide (Compound 501b)

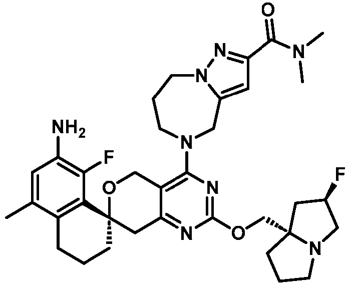

5-((*S*)-7-amino-8-fluoro-2'-(((2*R*,7a*S*)-2-fluorotetrahydro-1*H*-pyrrolizin-7a(5*H*)-yl)methoxy)-5-methyl-3,4,5',8'-tetrahydro-2*H*-spiro[naphthalene-1,7'-pyrano[4,3-*d*]pyrimidin]-4'-yl)-*N,N*-dimethyl-5,6,7,8-tetrahydro-4H-pyrazolo[1,5-a][1,4]diazepine-2-carboxamide was obtained from **Example 30** and **Peak 2** (1.9 mg) via SFC (Chiralpak IB-N (21 x 250 mm, 5 µm, P/N 88445), 40% isocratic MeOH with 0.25% diethylamine).
**Peak 2:** LCMS: t_{R} = 5.79 min, >95% de., 663.6 m/z: [M+H]⁺. Chiralpak IB-U (3.0 x 100 mm, 1.6 µm, P/N 81U83) with 40% isocratic cosolvent (methanol with 0.25% diethylamine).
¹H NMR (400 MHz, CD₃OD) δ 6.69 (d, 1H), 6.60 (s, 1H), 5.27 (d, 1H), 4.91 (s, 1H), 4.77 (s, 2H), 4.67 (m, 1H), 4.49 (m, 2H), 4.09 (m, 1H), 4.03 (m, 1H), 3.94 (m, 2H), 3.35 (m, 2H), 3.25 - 3.14 (m, 3H), 3.06 (s, 3H), 2.99 (m, 1H), 2.90 - 2.81 (m, 2H), 2.64 - 2.51 (m, 2H), 2.27 (m, 1H), 2.22 - 2.15 (m, 2H), 2.13 (s, 4H), 2.11 - 2.06 (m, 2H), 2.02 - 1.92 (m, 5H), 1.90 - 1.81 (m, 1H), 1.71 (m, 1H).

### Example 32: (S)-5-chloro-8-fluoro-2'-(((2R,7aS)-2-fluorotetrahydro-1H-pyrrolizin-7a(5H)-yl)methoxy)-4'-(1,4-oxazepan-4-yl)-3,4,5',8'-tetrahydro-2H-spiro[naphthalene-1,7'-pyrano[4,3-d]pyrimidin]-7-amine (Compound R181b)

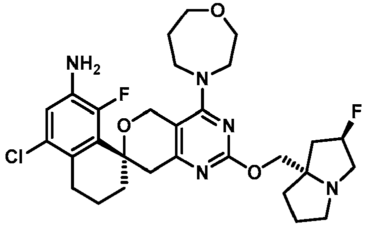

### Step 1: 7-Bromo-5-chloro-8-fluoro-2'-(methylthio)-4'-(1,4-oxazepan-4-yl)-3,4,5',8'-tetrahydro-2H-spiro[naphthalene-1,7'-pyrano[4,3-d]pyrimidine]

A mixture of 1,4-oxazepane (196 mg, 1.2 *equiv.,* 1.94 mmol) 7-bromo-4',5-dichloro-8-fluoro-2'-(methylthio)-3,4,5',8'-tetrahydro-2*H*-spiro[naphthalene-1,7'-pyrano[4,3-*d*]pyrimidine] (750 mg, *1 equiv.,* 1.62 mmol), DIPEA (1.25 g, 1.69 mL, 6 *equiv.,* 9.69 mmol), and 1,4-dioxane (20 mL) was heated at 110 °C and stirred for 4 hours. The reaction mixture was cooled to 22 °C and diluted with CHCl₃ (20 mL) and water (20 mL). The organic layer was separated, and the aqueous layer was extracted with CHCl₃/MeOH (4:1) (4 x 15 mL). The combined organic layer was washed with brine (3 x 10 mL), dried over sodium sulfate, and filtered. The solvent was evaporated under reduced pressure and the residual mass was purified by flash column chromatography (24 g cartridge, dry injection) with EtOAc/DCM (0-30%) to yield 7-bromo-5-chloro-8-fluoro-2'-(methylthio)-4'-(1,4-oxazepan-4-yl)-3,4,5',8'-tetrahydro-2*H*-spiro[naphthalene-1,7'-pyrano[4,3-d]pyrimidine] (773 mg).
LCMS: m/z (ESI) [M+H]⁺ 528.2, t_{R} = 2.06 minutes (Method B)

### Step 2: tert-Butyl (5-chloro-8-fluoro-2'-(methylthio)-4'-(1,4-oxazepan-4-yl)-3,4,5',8'-tetrahydro-2H-spiro[naphthalene-1,7'-pyrano[4,3-d]pyrimidin]-7-yl)carbamate

Into a flame dried 100 mL pressure vessel, a mixture of 7-bromo-5-chloro-8-fluoro-2'-(methylthio)-4'-(1,4-oxazepan-4-yl)-3,4,5',8'-tetrahydro-2*H*-spiro[naphthalene-1,7'-pyrano[4,3-*d*]pyrimidine] (305 mg, *1 equiv.,* 577 µmol), tert-butyl carbamate (108 mg, 1.6 *equiv.,* 923 µmol), and cesium carbonate (564 mg, 3 *equiv.,* 1.73 mmol) was purged with nitrogen. DMF (20 mL) was added via syringe. The resulting mixture was degassed with nitrogen for 5 minutes then BrettPhos Pd G4 (53.1 mg, 0.10 *equiv.,* 57.7 µmol) was added. The resulting mixture was degassed with nitrogen for another 3 minutes. The pressure vessel was capped and heated at 80 °C for 15 hours. Upon cooling to 22 °C, the solid was removed by vacuum filtration on celite and the filtrate was collected with EtOAc (20 mL). The organic filtrate was treated with EtOAc (60 mL) and was washed with brine (3 x 15 mL), dried over sodium sulfate, and filtered. The solvent was evaporated and the crude material was purified by flash column chromatography (25 g cartridge, dry injection) with EtOAc/hexanes (10-70%) to afford tert-butyl (5-chloro-8-fluoro-2'-(methylthio)-4'-(1,4-oxazepan-4-yl)-3,4,5',8'-tetrahydro-2*H*-spiro[naphthalene-1,7'-pyrano[4,3-d]pyrimidin]-7-yl)carbamate (163 mg).
LCMS: m/z (ESI) [M+H]⁺ 565.3, t_{R} = 5.67 minutes (Method I)

### Step 3: tert-Butyl (5-chloro-8-fluoro-2'-(methylsulfonyl)-4'-(1,4-oxazepan-4-yl)-3,4,5',8'-tetrahydro-2H-spiro[naphthalene-1,7'-pyrano[4,3-d]pyrimidin]-7-yl)carbamate

Hydrogen peroxide (320 µL, 30 wt%, 7 *equiv.,* 3.11 mmol) was added to a mixture of *tert*-butyl (5-chloro-8-fluoro-2'-(methylthio)-4'-(1,4-oxazepan-4-yl)-3,4,5',8'-tetrahydro-2H-spiro[naphthalene-1,7'-pyrano[4,3-*d*]pyrimidin]-7-yl)carbamate (251 mg, 1 *equiv.,* 444 µmol), sodium tungstate dihydrate (15.0 mg, 0.10 *equiv.,* 44.0 µmol), hydrogen tetra(but-1-yl)ammonium sulfate (24.0 mg, 0.16 *equiv.,* 71.0 µmol) in EtOAc (10 mL) and the resulting mixture was heated at 40 °C for 40 minutes. The reaction mixture was cooled to ambient temperature and was diluted with water (10 mL). The organics were extracted with EtOAc (4 x 15 mL). The combined organics were washed with brine (3 x 15 mL), dried over sodium sulfate and filtered. The solvent was evaporated to yield *tert*-butyl (5-chloro-8-fluoro-2'-(methylsulfonyl)-4'-(1,4-oxazepan-4-yl)-3,4,5',8'-tetrahydro-2*H*-spiro[naphthalene-1,7'-pyrano[4,3-*d*]pyrimidin]-7-yl)carbamate (260 mg).
LCMS: m/z (ESI) [M+H]⁺ 597.4, t_{R} = 1.77 minutes (Method B)

### Step 4: tert-Butyl (5-chloro-8-fluoro-2'-(((2R,7aS)-2-fluorotetrahydro-1H-pyrrolizin-7a(5H)-yl)methoxy)-4'-(1,4-oxazepan-4-yl)-3,4,5',8'-tetrahydro-2H-spiro [naphthalene-1,7'-pyrano[4,3-d]pyrimidin]-7-yl)carbamate

Potassium tert-butoxide in THF (1.09 mL, 1.0 M, 2.5 *equiv.,* 1.09 mmol) was added to a solution of ((2*R*,7a*S*)-2-fluorotetrahydro-1*H*-pyrrolizin-7a(5*H*)-yl)methanol (104 mg, 1.5 *equiv.,* 653 µmol) in anhydrous THF (160 µL) at 0 °C. After 5 minutes, *tert*-butyl (5-chloro-8-fluoro-2'-(methylsulfonyl)-4'-(1,4-oxazepan-4-yl)-3,4,5',8'-tetrahydro-2*H*-spiro[naphthalene-1,7'-pyrano[4,3-*d*]pyrimidin]-7-yl)carbamate (260 mg, *1 equiv.,* 435 µmol) was added and the contents were stirred for another 10 minutes. The reaction mixture was quenched with EtOAc (10 mL) and aqueous NH₄Cl (10 mL). The organic layer was separated, and the aqueous layer was extracted with EtOAc (4 X 15 mL). The combined organics were washed with brine (3 x 10 mL), dried over sodium sulfate, and filtered. The solvent was evaporated to yield tert-butyl (5-chloro-8-fluoro-2'-(((2*R*,7a*S*)-2-fluorotetrahydro-1*H*-pyrrolizin-7a(5*H*)-yl)methoxy)-4'-(1,4-oxazepan-4-yl)-3,4,5',8'-tetrahydro-2*H*-spiro[naphthalene-1,7'-pyrano[4,3-*d*]pyrimidin]-7-yl)carbamate which was used as crude material in the subsequent step assuming quantitative conversion to product.
LCMS: m/z (ESI) [M+H]⁺ 676.4, t_{R} = 5.39 minutes (Method I)

### Step 5: (S)-5-chloro-8-fluoro-2'-(((2R,7aS)-2-fluorotetrahydro-1H-pyrrolizin-7a(5H)-yl)methoxy)-4'-(1,4-oxazepan-4-yl)-3,4,5',8'-tetrahydro-2H-spiro[naphthalene-1,7'-pyrano[4,3-d]pyrimidin]-7-amine

A mixture was prepared with *tert*-butyl (5-chloro-8-fluoro-2'-(((2*R*,7a*S*)-2-fluorotetrahydro-1*H*-pyrrolizin-7a(5*H*)-yl)methoxy)-4'-(1,4-oxazepan-4-yl)-3,4,5',8'-tetrahydro-2*H*-spiro[naphthalene-1,7'-pyrano[4,3-*d*]pyrimidin]-7-yl)carbamate (260 mg, 1 *equiv.,* 385 µmol), p-toluenesulfonic acid (199 mg, 3 *equiv.,* 1.15 mmol) and MeCN (3 mL). The mixture was heated to 40 °C for 4 hours. The contents were then diluted with EtOAc (15 mL) and aqueous sodium bicarbonate (15 mL). The organic layer was separated, and the aqueous layer was extracted further with EtOAc (4 x 15 mL). The combined organics were washed with brine (3 x 15 mL), dried over sodium sulfate and filtered. The solvent was evaporated and the residue was purified by flash column chromatography (25 g cartridge, dry injection) with DCM/EtOAc (1-75%) to yield 5-chloro-8-fluoro-2'-(((2R,7aS)-2-fluorotetrahydro-1*H*-pyrrolizin-7a(5*H*)-yl)methoxy)-4'-(1,4-oxazepan-4-yl)-3,4,5',8'-tetrahydro-2*H*-spiro[naphthalene-1,7'-pyrano[4,3-*d*]pyrimidin]-7-amine (153 mg) as a mixture of diastereomers. The diastereomers were separated by reverse-phase prep-HPLC Gemini 5um NX-C18 110 Å, 100 x 30 mm (45 mL/min, 40-100% MeOH in water with ammonium bicarbonate buffer, pH = 10.0) to afford (S)-5-chloro-8-fluoro-2'-(((2R,7aS)-2-fluorotetrahydro-1*H*-pyrrolizin-7a(5*H*)-yl)methoxy)-4'-(1,4-oxazepan-4-yl)-3,4,5',8'-tetrahydro-2*H*-spiro[naphthalene-1,7'-pyrano[4,3-*d*]pyrimidin]-7-amine (30 mg) as **Peak 2.**
**Peak 2:** LCMS: m/z (ESI) [M+H]⁺ 576.5, t_{R} = 4.43 minutes (Method I)
¹H NMR (400 MHz, DMSO-*d*₆): δ 6.81 (d, *J =* 7.7 Hz, 1H), 5.31 (s, 1H), 5.15 (s, 2H), 4.77 (m, 1H), 4.55 (m, 1H), 3.93 (m, 1H), 3.82 (m, 1H), 3.57-3.75 (m, 8H), 2.97-3.14 (m, 4H), 2.78 (m, 2H), 2.52-2.64 (m, 2H), 2.06-2.08 (m, 1H), 1.70-1.97 (m, 11H).
¹⁹F NMR (DMSO, 376 MHz): δ -131.2,172.1.

### Example 33: 5-(7-Amino-8-cyano-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)-3-chloro-N,N-dimethyl-5,6,7,8-tetrahydro-4H-pyrazolo[1,5-a][1,4]diazepine-2-carboxamide (Compound 507a)

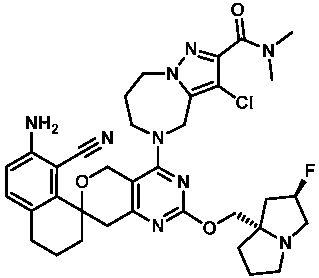

### Step 1: 3-Chloro-5-(8-cyano-7-(dibenzylamino)-2'-(methylsulfonyl)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide

5-(8-Cyano-7-(dibenzylamino)-2'-(methylthio)-3,4,5',8'-tetrahydro-2*H-*spiro[naphthalene-1,1'-pyrano[4,3-*d*]pyrimidin]-4'-yl)-*N,N*-dimethyl-5,6,7,8-tetrahydro-4*H-*pyrazolo[1,5*-a*][1,4]diazepine-2-carboxamide (200 mg, *1 equiv.,* 276 µmol) was dissolved in a mixture of THF (2 mL), MeOH (1.8 mL), and water (1.1 mL) at 25 °C. To this mixture was added Oxone (1.13 g, 0.83 mmol) in one portion. After 2.5 hours, ammonium chloride (15 mg) was added, and the mixture was stirred at 25 °C for an additional 75 minutes. Another portion of ammonium chloride (15 mg) was added, and the reaction mixture was stirred for 5 minutes. The mixture was then treated with a saturated aqueous solution of sodium thiosulfate (10 mL) and the resulting mixture was stirred for 5 minutes. The mixture was extracted with EtOAc (2 x 15 mL). The combined organic phases were washed with water (20 mL), dried over magnesium sulfate, filtered, and concentrated to give 3-chloro-5-(8-cyano-7-(dibenzylamino)-2'-(methylsulfonyl)-3,4,5',8'-tetrahydro-2*H*-spiro[naphthalene-1,7'-pyrano[4,3-*d*]pyrimidin]-4'-yl)-*N,N*-dimethyl-5,6,7,8-tetrahydro-4*H*-pyrazolo[1,5-*a*][1,4]diazepine-2-carboxamide (215 mg) which was used without further purification.
LCMS: m/z (ESI) [M+H]⁺ 791.5, t_{R} = 2.02 minutes (Method C)

### Step 2: 3-Chloro-5-(8-cyano-7-(dibenzylamino)-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide

((2*R*,7a*S*)-2-Fluorotetrahydro-1*H*-pyrrolizin-7a(5*H*)-yl)methanol (216 mg, 5 *equiv.,* 1.36 mmol) and 3-chloro-5-(8-cyano-7-(dibenzylamino)-2'-(methylsulfonyl)-3,4,5',8'-tetrahydro-2*H*-spiro[naphthalene-1,1'-pyrano[4,3-d]pyrimidin]-4'-yl)-*N,N*-dimethyl-5,6,7,8-tetrahydro-4*H*-pyrazolo[1,5-*a*][1,4]diazepine-2-carboxamide (215 mg, 1 *equiv.,* 272 µmol) were dissolved in DMF (5.4 mL) under nitrogen. The reaction mixture was cooled to 0 °C and a 1 M solution of LiHMDS in THF (0.54 mL, 0.54 mmol) was added. The mixture was warmed to 25 °C and stirred for 30 minutes at which point additional LiHMDS (0.54 mL, 0.54 mmol) was added at 25 °C. After an hour, the mixture was treated with saturated aqueous NH₄Cl (10 mL) and diluted with EtOAc (10 mL). The layers were separated, and the aqueous layer was extracted with EtOAc (3 x 5 mL). The combined organic layers were dried over magnesium sulfate, filtered, and concentrated to a residue which was purified by column chromatography (4 g silica, 0-5% (2.5% NH₄OH and 20% MeOH in DCM) in DCM) to afford 3-chloro-5-(8-cyano-7-(dibenzylamino)-2'-(((2*R*,7a*S*)-2-fluorotetrahydro-1*H*-pyrrolizin-7a(5*H*)-yl)methoxy)-3,4,5',8'-tetrahydro-2*H*-spiro[naphthalene-1,7'-pyrano[4,3-*d*]pyrimidin]-4'-yl)-*N,N*-dimethyl-5,6,7,8-tetrahydro-4*H*-pyrazolo[1,5-*a*][1,4]diazepine-2-carboxamide (93 mg).
LCMS: m/z (ESI) [M+H]⁺ 870.6, t_{R} = 1.62 minutes (Method C)

### Step 3: 5-(7-Amino-8-cyano-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)-3-chloro-N,N-dimethyl-5,6,7,8-tetrahydro-4H-pyrazolo[1,5-a][1,4]diazepine-2-carboxamide

3-Chloro-5-(8-cyano-7-(dibenzylamino)-2'-(((2*R*,7a*S*)-2-fluorotetrahydro-1H-pyrrolizin-7a(5*H*)-yl)methoxy)-3,4,5',8'-tetrahydro-2H-spiro[naphthalene-1,7'-pyrano[4,3-*d*]pyrimidin]-4'-yl)-*N,N*-dimethyl-5,6,7,8-tetrahydro-4*H*-pyrazolo[1,5-*a*][1,4]diazepine-2-carboxamide (92.9 mg, *1 equiv.,* 107 µmol) and Pd(OH)₂ on carbon (187 mg, 0.27 mmol) were combined and put under a nitrogen atmosphere. MeOH (2.1 mL) was then added under nitrogen and the resulting solution was sparged with H₂ for 5 minutes, followed by the addition of acetic acid (120 µL, 0.70 mmol). The reaction mixture was stirred vigorously under an atmosphere of hydrogen for 8 hours. The reaction mixture was then filtered through a syringe filter and the filtrate was stirred with sodium bicarbonate (500 mg) for 5 minutes. The mixture was filtered, and concentrated to a residue which was purified via column chromatography (12 g silica gel; 0-100% (60/10/10/10 EtOAc:MeOH:MeCN:H₂O)/EtOAc] to give 5-(7-amino-8-cyano-2'-(((2*R*,7a*S*)-2-fluorotetrahydro-1*H*-pyrrolizin-7a(5*H*)-yl)methoxy)-3,4,5',8'-tetrahydro-2*H*-spiro[naphthalene-1,1'-pyrano[4,3-*d*]pyrimidin]-4'-yl)-3-chloro-*N,N*-dimethyl-5,6,7,8-tetrahydro-4*H*-pyrazolo[1,5-*a*][1,4]diazepine-2-carboxamide (31.8 mg).
LCMS: m/z (ESI) [M+H]⁺ 690.3, t_{R} = 1.90 minutes (Method C)
¹H NMR (400 MHz, CDCl₃) δ 7.01 (d, *J* = 8.5 Hz, 1H), 6.64 (d, *J* = 8.5 Hz, 1H), 5.44 - 5.19 (m, 1H), 5.06 (dd, *J* = 14.2, 3.8 Hz, 1H), 4.83 (dd, *J* = 14.0, 6.1 Hz, 1H), 4.70 (t, *J* = 16.2 Hz, 1H), 4.56 - 4.31 (m, 5H), 4.16 (s, 2H), 4.00 (dq, *J* = 9.4, 4.6 Hz, 1H), 3.61 (ddt, *J* = 14.0, 8.9, 3.9 Hz, 1H), 3.47 (s, 1H), 3.34 - 3.15 (m, 2H), 3.13 - 2.83 (m, 8H), 2.72 - 2.58 (m, 2H), 2.40 - 2.11 (m, 6H), 2.06 - 1.60 (m, 6H).
¹⁹F NMR (376 MHz, CDCl₃) δ -172.60

### Example 34. 6'-Amino-2-(((2R,7aS)-2-fluorotetrahydro-1H-pyrrolizin-7a(5H)-yl)methoxy)-4-(1,4-oxazepan-4-yl)-2',3',5,8-tetrahydro-1'H-spiro[pyrano[4,3-d]pyrimidine-7,4'-[1,3]methanonaphthalene]-5'-carbonitrile (Compound 503a)

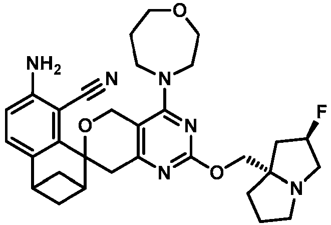

### Step 1: N,N-dibenzyl-5'-bromo-2-(methylthio)-4-(1,4-oxazepan-4-yl)-2',3',5,8-tetrahydro-1'H-spiro[pyrano[4,3-d]pyrimidine-7,4'-[1,3]methanonaphthalen]-6'-amine

1,4-Oxazepane (199 mg, 2 *equiv.,* 1.97 mmol) and *N,N*-dibenzyl-5'-bromo-4-chloro-2-(methylthio)-2',3',5,8-tetrahydro-1'*H*-spiro[pyrano[4,3-*d*]pyrimidine-7,4'-[1,3]methanonaphthalen]-6'-amine (610 mg, *1 equiv.,* 985 µmol) were dissolved in DMF (4.93 mL). DIPEA (509 mg, 0.68 mL, 4 *equiv.,* 3.94 mmol) was then added to the solution which was then heated to 90 °C and stirred for 1.5 hours. The reaction mixture was concentrated to dryness and diluted with water (5 mL) and EtOAc (5 mL). The organic layer was separated, and the aqueous phase was extracted with EtOAc (3 x 5 mL). The organic phases were combined, dried over sodium sulfate, filtered, and concentrated to a residue which was purified via column chromatography (24 g SiO₂, 0-40% EtOAc in heptanes) to give *N,N-*dibenzyl-5'-bromo-2-(methylthio)-4-(1,4-oxazepan-4-yl)-2',3',5,8-tetrahydro-1'*H-*spiro[pyrano[4,3-*d*]pyrimidine-7,4'-[1,3]methanonaphthalen]-6'-amine (610 mg).
LCMS: m/z (ESI) [M+H]⁺ 685.2, t_{R} = 3.91 minutes (Method E)

### Step 2: 6'-(Dibenzylamino)-2-(methylthio)-4-(1,4-oxazepan-4-yl)-2',3',5,8-tetrahydro-1'H-spiro[pyrano[4,3-d]pyrimidine-7,4'-[1,3]methanonaphthalene]-5'-carbonitrile

*N,N*-dibenzyl-5'-bromo-2-(methylthio)-4-(1,4-oxazepan-4-yl)-2',3',5,8-tetrahydro-1'*H-*spiro[pyrano[4,3-*d*]pyrimidine-7,4'-[1,3]methanonaphthalen]-6'-amine (610 mg, 0.83 mmol) was dissolved in DMF (16.6 mL). Copper(I) cyanide (743 mg, 8.3 mmol) was added in one portion and the resulting reaction mixture was stirred at 120 °C for 44 hours. The reaction mixture was cooled to 25 °C and concentrated to dryness under reduced pressure. The resulting residue was diluted with water (20 mL) and DCM (20 mL). A 1:1 mixture of ammonium hydroxide and MeOH (200 mL) was then added, and the resulting mixture was stirred at 25 °C for 15 minutes. The reaction mixture was extracted with EtOAc (3 x 20 mL). The combined organic layers were dried over sodium sulfate, filtered, and concentrated to give 6'-(dibenzylamino)-2-(methylthio)-4-(1,4-oxazepan-4-yl)-2',3',5,8-tetrahydro-1'*H-*spiro[pyrano[4,3-*d*]pyrimidine-7,4'-[1,3]methanonaphthalene]-5'-carbonitrile (650 mg), which was used without further purification.
LCMS: m/z (ESI) [M+H]⁺ 631.4, t_{R} = 3.56 minutes (Method E)

### Step 3: 6'-(Dibenzylamino)-2-(methylsulfonyl)-4-(1,4-oxazepan-4-yl)-2',3',5,8-tetrahydro-1'H-spiro[pyrano[4,3-d]pyrimidine-7,4'-[1,3]methanonaphthalene]-5'-carbonitrile

6'-(dibenzylamino)-2-(methylthio)-4-(1,4-oxazepan-4-yl)-2',3',5,8-tetrahydro-1'*H-*spiro[pyrano[4,3-*d*]pyrimidine-7,4'-[1,3]methanonaphthalene]-5'-carbonitrile (650 mg, 77 wt%, *1 equiv.,* 795 µmol) was dissolved in a mixture of THF (6.22 mL) and MeOH (10.3 mL) and water (6.22 mL) at 25 °C. Oxone (1.47 g, 3 *equiv.,* 2.38 mmol) was added to the reaction mixture in one portion and the reaction mixture was stirred for 15 hours. After 15 hours, the reaction was diluted with water (15 mL) and EtOAc (15 mL). The organic layer was separated, and the aqueous layer was extracted with EtOAc (3 x 15 mL). The combined organic phases were dried over sodium sulfate, filtered, and concentrated to give 6'-(dibenzylamino)-2-(methylsulfonyl)-4-(1,4-oxazepan-4-yl)-2',3',5,8-tetrahydro-1'H-spiro[pyrano[4,3-d]pyrimidine-7,4'-[1,3]methanonaphthalene]-5'-carbonitrile (450 mg).
LCMS: m/z (ESI) [M+H]⁺ 663.3, t_{R} = 3.70 minutes (Method E)

### Step 4: 6'-(Dibenzylamino)-2-(((2R,7aS)-2-fluorotetrahydro-1H-pyrrolizin-7a(5H)-yl)methoxy)-4-(1,4-oxazepan-4-yl)-2',3',5,8-tetrahydro-1'H-spiro[pyrano[4,3-d]pyrimidine-7,4'-[1,3]methanonaphthalene]-5'-carbonitrile

((2*R*,7a*S*)-2-Fluorotetrahydro-1*H*-pyrrolizin-7a(5*H*)-yl)methanol (325 mg, 3 *equiv.,* 2.04 mmol) and 6'-(dibenzylamino)-2-(methylsulfonyl)-4-(1,4-oxazepan-4-yl)-2',3',5,8-tetrahydro-1'*H*-spiro[pyrano[4,3-d]pyrimidine-7,4'-[1,3]methanonaphthalene]-5'-carbonitrile (450 mg, *1 equiv.,* 680 µmol) were dissolved in DMF (13.6 mL) at 0 °C. A 1 M solution of LiHMDS in THF (1.36 mL, 1.36 mmol) was then added dropwise to the reaction mixture at 0 °C. The resulting mixture was stirred at 0 °C for 3 hours then warmed to 25 °C. After 2.5 hours, a 1:1 mixture of water and saturated aqueous solution of ammonium chloride (6 mL) was added. The reaction mixture was diluted with EtOAc (20 mL). The organic phase was separated, and the aqueous layer was extracted with EtOAc (3 x 20 mL). The combined organic phases were dried over sodium sulfate, filtered, and concentrated to a residue which was purified via column chromatography (4 g silica, 0-40% (2.5% NH₄OH and 20% MeOH in DCM) in DCM) to give 6'-(dibenzylamino)-2-(((2*R*,7a*S*)-2-fluorotetrahydro-1*H*-pyrrolizin-7a(5*H*)-yl)methoxy)-4-(1,4-oxazepan-4-yl)-2',3',5,8-tetrahydro-1'*H*-spiro[pyrano[4,3-*d*]pyrimidine-7,4'-[1,3]methanonaphthalene]-5'-carbonitrile (250 mg).
LCMS: m/z (ESI) [M+H]⁺ 741.3, t_{R} = 2.62 minutes (Method E)

### Step 5: 6'-Amino-2-(((2R,7aS)-2-fluorotetrahydro-1H-pyrrolizin-7a(5H)-yl)methoxy)-4-(1,4-oxazepan-4-yl)-2',3',5,8-tetrahydro-1'H-spiro[pyrano[4,3-d]pyrimidine-7,4'-[1,3]methanonaphthalene]-5'-carbonitrile

6'-(Dibenzylamino)-2-(((2R,7aS)-2-fluorotetrahydro-1*H*-pyrrolizin-7a(5*H*)-yl)methoxy)-4-(1,4-oxazepan-4-yl)-2',3',5,8-tetrahydro-1*'H*-spiro[pyrano[4,3-*d*]pyrimidine-7,4'-[1,3]methanonaphthalene]-5'-carbonitrile (250 mg, *1 equiv.,* 337 µmol) and Pd(OH)₂ on carbon (592 mg, 0.84 mmol) were combined and placed under a nitrogen atmosphere. MeOH (6.75 mL) was then added, and the reaction was sparged with hydrogen for 5 minutes, followed by the dropwise addition of acetic acid (120 µL, 2.09 mmol). The reaction mixture was stirred under a positive pressure of hydrogen for 2.5 hours. After 2.5 hours the reaction mixture was filtered, and the filtrate was stirred with sodium carbonate (750 mg) for 5 minutes. The mixture was filtered, and concentrated under reduced pressure to give a residue which was purified via column chromatography (4 g silica, 0-40% (2.5% NH₄OH and 20% MeOH in DCM) in DCM) to give 6'-amino-2-(((2*R*,7a*S*)-2-fluorotetrahydro-1*H*-pyrrolizin-7a(5*H*)-yl)methoxy)-4-(1,4-oxazepan-4-yl)-2',3',5,8-tetrahydro-1'*H*-spiro[pyrano[4,3-*d*]pyrimidine-7,4'-[1,3]methanonaphthalene]-5'-carbonitrile (136 mg).
¹H NMR (400 MHz, MeOD) δ 7.04 (d, 1H), 6.69 (d, 1H), 6.63 (d1H), 5.41 - 5.16 (m, 1H), 4.88 (d, 1H), 4.78 (s, 2H), 4.71 (dd, 1H), 4.57 - 4.40 (m, 2H), 4.15 - 3.99 (m, 3H), 3.84 (m, 1H), 3.67 (d, 1H), 3.33 (s, 3H), 3.30 - 3.13 (m, 3H), 3.09 - 2.94 (m, 5H), 2.88 (d, 1H), 2.57 (m, 1H), 2.40 (m, 1H), 2.31 - 1.82 (m, 6H), 1.76 (m, 1H), 1.66 (m, 1H).
¹⁹F NMR (376 MHz, MeOD) δ -176.5 - -174.6 (m).
LCMS: m/z (ESI) [M+H]⁺ 561.3, t_{R} = 1.30 minutes (Method C)

### Example 35: (7S)-6'-Amino-2-(((2R,7aS)-2-fluorotetrahydro-1H-pyrrolizin-7a(5H)-yl)methoxy)-4-(1,4-oxazepan-4-yl)-2',3',5,8-tetrahydro-1'H-spiro[pyrano[4,3-d]pyrimidine-7,4'-[1,3]methanonaphthalene]-5'-carbonitrile (Compound 503b)

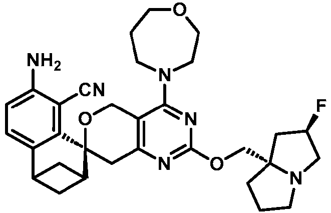

The diastereomers of 6'-amino-2-(((2*R*,7a*S*)-2-fluorotetrahydro-1*H*-pyrrolizin-7a(5*H*)-yl)methoxy)-4-(1,4-oxazepan-4-yl)-2',3',5,8-tetrahydro-1'*H*-spiro[pyrano[4,3-*d*]pyrimidine-7,4'-[1,3]methanonaphthalene]-5'-carbonitrile (70 mg) were separated by chiral SFC using Diacel ChiralPak IA 21mm ID x 250 mm L (mobile phase: 40% methanol, 0.25% diethylamine in CO₂) to give **Peak 1:** (7*S*)-6'-amino-2-(((2*R*,7a*S*)-2-fluorotetrahydro-1*H*-pyrrolizin-7a(5*H*)-yl)methoxy)-4-(1,4-oxazepan-4-yl)-2',3',5,8-tetrahydro-1'*H*-spiro[pyrano[4,3-*d*]pyrimidine-7,4'-[1,3]methanonaphthalene]-5'-carbonitrile (24 mg) and **Peak 2:** ((7*R*)-6'-amino-2-(((2*R*,7a*S*)-2-fluorotetrahydro-1*H*-pyrrolizin-7a(5*H*)-yl)methoxy)-4-(1,4-oxazepan-4-yl)-2',3',5,8-tetrahydro-1'*H*-spiro[pyrano[4,3-*d*]pyrimidine-7,4'-[1,3]methanonaphthalene]-5'-carbonitrile (26 mg).
**Peak 1:** LCMS: m/z (ESI) [M+H]⁺ 561.5, t_{R} = 1.01 minutes (Diacel ChiralPak IA 4.6 mm ID x 100 mm L, mobile phase: 40% methanol, 0.25% diethylamine in CO₂)
¹H NMR (400 MHz, MeOD) δ 7.03 (m, 1H), 6.68 (m, 1H), 5.38 - 5.17 (m, 1H), 4.85 - 4.68 (m, 2H), 4.17 - 4.09 (m, 1H), 4.04 (m, 1H), 3.94 - 3.72 (m, 6H), 3.68 (m, 3H), 3.28 - 3.12 (m, 3H), 2.99 (m, 3H), 2.86 (d, 1H), 2.56 (m, 1H), 2.42 - 2.13 (m, 3H), 2.08 (m, 2H), 2.01 - 1.77 (m, 4H), 1.69 (m, 2H).
¹⁹F NMR (376 MHz, MeOD) δ -173.52.

### Example 36: (7R)-6'-Amino-2-(((2R,7aS)-2-fluorotetrahydro-1H-pyrrolizin-7a(5H)-yl)methoxy)-4-(1,4-oxazepan-4-yl)-2',3',5,8-tetrahydro-1'H-spiro[pyrano[4,3-d]pyrimidine-7,4'-[1,3]methanonaphthalene]-5'-carbonitrile (Compound 503c)

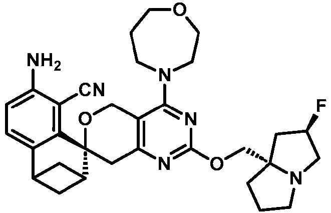

(7*R*)-6'-amino-2-(((2*R*,7a*S*)-2-fluorotetrahydro-1*H*-pyrrolizin-7a(5*H*)-yl)methoxy)-4-(1,4-oxazepan-4-yl)-2',3',5,8-tetrahydro-1'*H*-spiro[pyrano[4,3-*d*]pyrimidine-7,4'-[1,3]methanonaphthalene]-5'-carbonitrile (26 mg) was obtained from **Example 35, as Peak 2:** Diacel ChiralPak IA 21mm ID x 250 mm L (mobile phase: 40% methanol, 0.25% diethylamine in CO₂).
**Peak 2:** LCMS: m/z (ESI) [M+H]⁺ 561.5, t_{R} = 1.68 minutes (Diacel ChiralPak IA 4.5 mm ID x 100 mm L, mobile phase: 40% methanol, 0.25% diethylamine in CO₂)
¹H NMR (400 MHz, MeOD) δ 7.03 (d, 1H), 6.69 (d, 1H), 5.37 - 5.18 (m, 1H), 4.84 - 4.68 (m, 2H), 4.13 (d, 1H), 4.03 (d, 1H), 3.94 - 3.72 (m, 6H), 3.75 - 3.61 (m, 3H), 3.28 - 3.17 (m, 2H), 3.15 (s, 1H), 3.00 (m, 3H), 2.87 (d, 1H), 2.61 - 2.53 (m, 1H), 2.36 (m, 1H), 2.27 (m, 1H), 2.19 - 2.14 (m, 1H), 2.14 - 2.01 (m, 2H), 1.91 (m, 4H), 1.69 (m, 2H).
¹⁹F NMR (376 MHz, MeOD) δ -173.69.

### Example 37: 5-((S)-7-Amino-8-cyano-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)-3-chloro-N,N-dimethyl-5,6,7,8-tetrahydro-4H-pyrazolo[1,5-a][1,4]diazepine-2-carboxamide (Compound 507b)

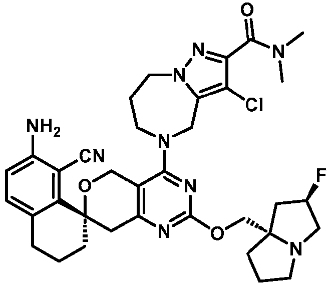

The diastereomers of 5-(7-Amino-8-cyano-2'-(((2*R*,7a*S*)-2-fluorotetrahydro-1H-pyrrolizin-7*a*(5*H*)-yl)methoxy)-3,4,5',8'-tetrahydro-2*H*-spiro[naphthalene-1,7'-pyrano[4,3-*d*]pyrimidin]-4'-yl)-3-chloro-N,N-dimethyl-5,6,7,8-tetrahydro-4H-pyrazolo[1,5-*a*][1,4]diazepine-2-carboxamide (27.5 mg, 39.8 µmol) were purified by chiral SFC using ChiralCel OJ-H 21 x 250 mm (Mobile Phase: 25% ethanol, 0.25% diethylamine in CO₂; back pressure = 100 bar) to give 5-((*S*)-7-amino-8-cyano-2'-(((2*R*,7a*S*)-2-fluorotetrahydro-1*H-*pyrrolizin-7a(5*H*)-yl)methoxy)-3,4,5',8'-tetrahydro-2*H*-spiro[naphthalene-1,7'-pyrano[4,3-d]pyrimidin]-4'-yl)-3-chloro-*N,N*-dimethyl-5,6,7,8-tetrahydro-4*H*-pyrazolo[1,5-*a*][1,4]diazepine-2-carboxamide **(Peak 1,** 10.1 mg) and 5-((*R*)-7-amino-8-cyano-2'-(((2R,7aS)-2-fluorotetrahydro-1*H*-pyrrolizin-7a(5*H*)-yl)methoxy)-3,4,5',8'-tetrahydro-2H-spiro[naphthalene-1,1'-pyrano[4,3-*d*]pyrimidin]-4'-yl)-3-chloro-*N,N*-dimethyl-5,6,7,8-tetrahydro-4*H*-pyrazolo[1,5-*a*][1,4]diazepine-2-carboxamide **(Peak 2,** 11.0 mg).
**Peak 1:** LCMS: m/z (ESI) [M+H]⁺ 690.3, t_{R} = 2.25 minutes (ChiralCel OJ-H 4.6 x 100 mm; Mobile Phase: 25% ethanol, 0.25% diethylamine in CO₂; back pressure = 140 bar)
¹H NMR (400 MHz, CDCl₃) δ 7.04 (d, 1H), 6.64 (d, 1H), 5.44 - 5.01 (m, 2H), 4.90 - 4.65 (m, 2H), 4.60 - 4.34 (m, 4H), 4.26 - 4.15 (m, 1H), 4.11 - 3.88 (m, 3H), 3.69 - 3.58 (m, 1H), 3.42 - 3.14 (m, 3H), 3.16 - 3.06 (m, 6H), 3.05 - 2.88 (m, 2H), 2.72 - 2.64 (m, 2H), 2.39 - 2.11 (m, 5H), 1.93 - 1.59 (m, 8H).

### Example 38: 5-((R)-7-Amino-8-cyano-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)-3-chloro-N,N-dimethyl-5,6,7,8-tetrahydro-4H-pyrazolo[1,5-a][1,4]diazepine-2-carboxamide (Compound 507c)

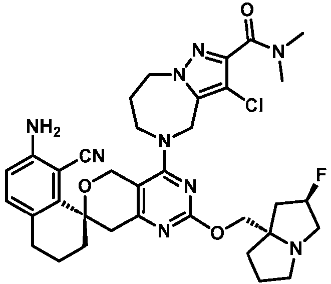

5-((*R*)-7-amino-8-cyano-2'-(((2*R*,7a*S*)-2-fluorotetrahydro-1*H*-pyrrolizin-7a(5*H*)-yl)methoxy)-3,4,5',8'-tetrahydro-2*H*-spiro[naphthalene-1,1'-pyrano[4,3-*d*]pyrimidin]-4'-yl)-3-chloro-*N,N*-dimethyl-5,6,7,8-tetrahydro-4*H*-pyrazolo[1,5-*a*][1,4]diazepine-2-carboxamide (11.0 mg) was obtained from **Example 37,** as **Peak 2:** LCMS: m/z (ESI) [M+H]⁺ 690.3, t_{R} = 2.80 minutes (ChiralCel OJ-H 4.6 x 100 mm; Mobile Phase: 25% Ethanol, 0.25% diethylamine in CO₂; back pressure = 140 bar)

¹H NMR (400 MHz, CDCl₃) δ 7.04 (d, 1H), 6.64 (d, 1H), 5.40 - 5.02 (m, 2H), 4.89 - 4.66 (m, 2H), 4.61 - 4.34 (m, 4H), 4.26 - 4.16 (m, 1H), 4.08 - 3.89 (m, 3H), 3.69 - 3.59 (m, 1H), 3.45 - 3.17 (m, 3H), 3.15 - 3.05 (m, 6H), 3.03 - 2.90 (m, 2H), 2.73 - 2.64 (m, 2H), 2.40 - 2.29 (m, 1H), 2.23 - 2.09 (m, 4H), 1.92 - 1.63 (m, 8H).

### Example 39: 5-((1S,4S)-7-Amino-8-cyano-2'-(((2R,7aS)-2-fluorotetrahydro-1H-pyrrolizin-7a(5H)-yl)methoxy)-4-methyl-3,4,5',8'-tetrahydro-2H-spiro[naphthalene-1,7'-pyrano[4,3-d]pyrimidin]-4'-yl)-3-chloro-N,N-dimethyl-5,6,7,8-tetrahydro-4H-pyrazolo[1,5-a][1,4]diazepine-2-carboxamide (Compound 509a)

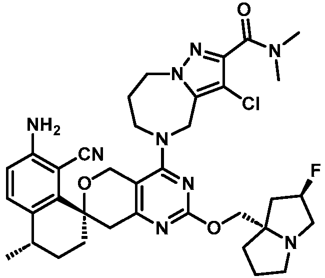

To a solution of (1*S*,4*S*)-7-amino-2'-(((2*R*,7a*S*)-2-fluorotetrahydro-1*H*-pyrrolizin-7a(5*H*)-yl)methoxy)-4'-hydroxy-4-methyl-3,4,5',8'-tetrahydro-2*H*-spiro[naphthalene-1,7'-pyrano[4,3-d]pyrimidine]-8-carbonitrile (45 mg, *1 equiv.,* 94 µmol) in DMF (1.9 mL) was added 3-chloro-N,N-dimethyl-5,6,7,8-tetrahydro-4H-pyrazolo[1,5-a][1,4]diazepine-2-carboxamide, HCl (79 mg, 3 *equiv.,* 0.28 mmol) and PyBOP (73 mg, 1.5 *equiv.,* 0.14 mmol). DIPEA (97 mg, 0.13 mL, 8 *equiv.,* 0.75 mmol) was then added dropwise. The resulting reaction mixture was heated to 80 °C and stirred for 1 hour. The reaction mixture was then cooled to room temperature and diluted with a mixture of water (5 mL), 5% lithium chloride solution (5 mL), and EtOAc (10 mL). The layers were separated, and the aqueous layer was extracted three times with EtOAc. The solution was dried over sodium sulfate, filtered, and concentrated to a red oil, which was purified by reverse phase column chromatography (0 to 100% 0.1% TFA in MeCN: 0.1% TFA in water, focused gradient 20-35%) to afford 5-((1*S*,4*S*)-7-amino-8-cyano-2'-(((2*R*,7a*S*)-2-fluorotetrahydro-1*H*-pyrrolizin-7a(5*H*)-yl)methoxy)-4-methyl-3,4,5',8'-tetrahydro-2*H*-spiro[naphthalene-1,7'-pyrano[4,3-d]pyrimidin]-4'-yl)-3-chloro-*N,N*-dimethyl-5,6,7,8-tetrahydro-4*H*-pyrazolo[1,5-*a*][1,4]diazepine-2-carboxamide, TFA salt (36.6 mg) as an off-white solid.
LCMS: m/z (ESI) [M+H]⁺ 704.3, t_{R} = 2.08 minutes (Method C)
¹H NMR (400 MHz, DMSO-*d*₆): δ 10.47 (s, 1H), 7.12 (d, 1H), 6.75 (d, 1H), 5.56 (d, 1H), 5.08 (d, 1H), 4.79 (d, 1H), 4.74 - 4.65 (m, 2H), 4.47 - 4.28 (m, 3H), 4.22 (d, 1H), 4.03 - 3.58 (m, 9H), 3.31 - 3.21 (m, 1H), 3.07 (d, 1H), 3.01 (s, 3H), 2.97 (s, 3H), 2.90 (d, 1H), 2.85 - 2.77 (m, 1H), 2.36 - 1.90 (m, 9H), 1.65 - 1.53 (m, 1H), 1.18 (d, 3H)

### Example 40: (1S,4S)-7-Amino-4'-((S)-3-(cyanomethyl)piperidin-1-yl)-2'-(((2R,7aS)-2-fluorotetrahydro-1H-pyrrolizin-7a(5H)-yl)methoxy)-4-methyl-3,4,5',8'-tetrahydro-2H-spiro[naphthalene-1,7'-pyrano[4,3-d]pyrimidine]-8-carbonitrile (Compound 511a)

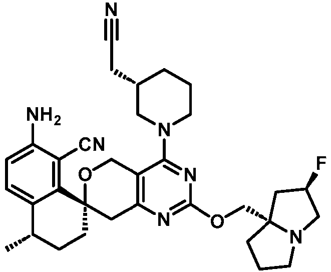

DIPEA (97 mg, 0.13 mL, 8 *equiv.,* 0.75 mmol) was added to a vial containing (S)-2-2(piperidin-3-yl)acetonitrile (78 mg, 3.5 *equiv.,* 0.33 mmol), (1*S*,4*S*)-7-amino-2'-(((2*R*,7a*S*)-2-fluorotetrahydro-1*H*-pyrrolizin-7a(5*H*)-yl)methoxy)-4'-hydroxy-4-methyl-3,4,5',8'-tetrahydro-2*H*-spiro[naphthalene-1,7'-pyrano[4,3-*d*]pyrimidine]-8-carbonitrile (45 mg, 1 *equiv.,* 94 µmol) and PyBOP (73 mg, 1.5 *equiv.,* 0.14 mmol) in DMF (1.9 mL). This vial was sealed, heated to 80 °C and stirred for 1 hour. The reaction was then diluted with water (10 mL) and extracted with EtOAc (3 x 10 mL). The combined organics were washed with 10% lithium chloride solution (4 x 20 mL) and once with brine (20 mL). The solution was dried with sodium sulfate, filtered, and concentrated. The crude mixture was purified by silica gel column chromatography (30% to 100 % 6:1:1:1 EtOAc:MeOH:MeCN:H₂O in EtOAc) to afford (1*S*,4*S*)-7-amino-4'-((*S*)-3-(cyanomethyl)piperidin-1-yl)-2'-(((2*R*,7a*S*)-2-fluorotetrahydro-1*H*-pyrrolizin-7a(5*H*)-yl)methoxy)-4-methyl-3,4,5',8'-tetrahydro-2H-spiro[naphthalene-1,7'-pyrano[4,3-*d*]pyrimidine]-8-carbonitrile (15 mg) as an off white solid.
LCMS: m/z (ESI) [M+H]⁺ 586.6, t_{R} = 1.82 minutes (Method K)
¹H NMR (400 MHz, CDCl₃): δ 7.08 (d, 1H), 6.67 (d, 1H), 5.27 (d, 1H), 4.89 (d, 1H), 4.82 (d, 1H), 4.43 (s, 2H), 4.15 - 4.08 (m, 1H), 4.06 - 3.80 (m, 2H), 3.64 (d, 1H), 3.38 - 2.73 (m, 8H), 2.40 - 2.35 (m, 2H), 2.33 - 2.09 (m, 5H), 2.03 - 1.89 (m, 7H), 1.84 - 1.75 (m, 1H) 1.71 - 1.56 (m, 2H), 1.47 - 1.33 (m, 1H), 1.23 (d, 3H)

### Example 41: (4S*)-1-(7-amino-5-chloro-8-fluoro-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)azepane-4-carbonitrile (Compound 510a)

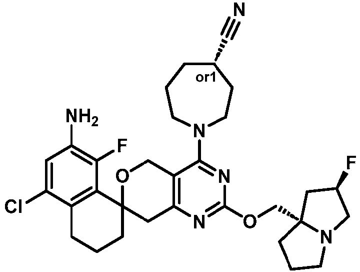

### Step 1: 7-Bromo-5-chloro-1-((6-chloro-5-(hydroxymethyl)-2-(methylthio)pyrimidin-4-yl)methyl)-8-fluoro-1,2,3,4-tetrahydronaphthalen-1-ol

LDA (1.930 g, 18.02 mL, 1 M in THF, 2.5 *equiv.,* 18.02 mmol) was added to a solution of (4-chloro-6-methyl-2-(methylthio)pyrimidin-5-yl)methanol (1.622 g, 1.1 *equiv.,* 7.927 mmol) in anhydrous THF (20 mL) at -78 °C via syringe pump over 20 minutes. The resulting mixture was stirred at this temperature for 1 hour. A solution of 7-bromo-5-chloro-8-fluoro-3,4-dihydronaphthalen-1(2*H*)-one (2000 mg, *1 equiv.,* 7.207 mmol) in THF (10 mL) was added via syringe pump over 15 minutes. The reaction mixture was stirred for 1 hour at -78 °C and quenched slowly by the addition of hydrochloric acid (840.8 mg, 5.765 mL, 4 M, 3.2 *equiv.,* 23.06 mmol) followed by aqueous ammonium chloride (20 mL) at -78 °C. The quenched mixture warmed up slowly to room temperature. The aqueous layer was extracted with EtOAc (3 x 30 mL). The combined organic layers were washed with brine (2 x 20 mL), dried over sodium sulfate, filtered, and concentrated to give the crude product which was triturated with diethyl ether to yield 7-bromo-5-chloro-1-((6-chloro-5-(hydroxymethyl)-2-(methylthio)pyrimidin-4-yl)methyl)-8-fluoro-1,2,3,4-tetrahydronaphthalen-1-ol (3.1 g) as a white solid.
LCMS: m/z (ESI) [M+H]⁺ 483.0, t_{R} = 1.84 minutes (Method B)

### Step 2: 7-Bromo-4',5-dichloro-8-fluoro-2'-(methylthio)-3,4,5',8'-tetrahydro-2H-spiro[naphthalene-1,7'-pyrano[4,3-d]pyrimidine]

Phosphoric acid (0.63 g, 0.37 mL, *1 equiv.,* 6.4 mmol) was added to a suspension of 7-bromo-5-chloro-1-((6-chloro-5-(hydroxymethyl)-2-(methylthio)pyrimidin-4-yl)methyl)-8-fluoro-1,2,3,4-tetrahydronaphthalen-1-ol (3.1 g, 1 *equiv.,* 6.4 mmol) in anhydrous toluene (30 mL). The resulting mixture was refluxed for 1 hour. The reaction mixture was cooled to room temperature and diluted with EtOAc (40 mL) and water (40 mL). The organic layer was separated, and the aqueous layer was extracted with EtOAc (4 x 15 mL). The combined organic layers were washed with brine (3 x 15 mL), dried over sodium sulfate, and filtered. The solvent was evaporated to give the crude product which was purified by silica column chromatography to yield 7-bromo-4',5-dichloro-8-fluoro-2'-(methylthio)-3,4,5',8'-tetrahydro-2*H-*spiro[naphthalene-1,7'-pyrano[4,3-d]pyrimidine] (1.48 g) as a white solid.
LCMS: m/z (ESI) [M+H]⁺ 463.0, t_{R} = 2.21 minutes (Method B)

### Step 3: (S*)-1-(7-Bromo-5-chloro-8-fluoro-2'-(methylthio)-3,4,5',8'-tetrahydro-2H-spiro[naphthalene-1,7'-pyrano[4,3-d]pyrimidin]-4'-yl)azepane-4-carbonitrile

A solution of 7-bromo-4',5-dichloro-8-fluoro-2'-(methylthio)-3,4,5',8'-tetrahydro-2*H-*spiro[naphthalene-1,7'-pyrano[4,3-*d*]pyrimidine] (150 mg, *1 equiv.,* 323 µmol) in 1,4-dioxane (5 mL) was added to a mixture of *rel*-(*S*)-azepane-4-carbonitrile hydrochloride (130 mg, 2.5 *equiv.,* 808 µmol) and DIPEA (418 mg, 563 µL, 10 *equiv.,* 3.23 mmol) in DMA (1 mL). The reaction mixture was heated at 110 °C for 7 hours. Upon completion, the reaction mixture was cooled to room temperature and diluted with CHCl₃ (20 mL) and water (20 mL). The organic layer was separated, and the aqueous layer was extracted with CHCl₃/MeOH (4:1) (4 x 15 mL). The combined organic layers were washed with brine (3 x 10 mL), dried over sodium sulfate, filtered, and concentrated under reduced pressure to give the crude product which was purified by flash column chromatography (0% EtOAc/hexane to 70% EtOAc/hexane) to yield (S*)-1-(7-bromo-5-chloro-8-fluoro-2'-(methylthio)-3,4,5',8'-tetrahydro-2*H*-spiro[naphthalene-1,7'-pyrano[4,3-*d*]pyrimidin]-4'-yl)azepane-4-carbonitrile (130 mg) as an off-white solid.
LCMS: m/z (ESI) [M+H]⁺ 551.2, t_{R} = 1.76 minutes (Method A)

### Step 4: tert-Butyl (5-chloro-4'-((S*)-4-cyanoazepan-1-yl)-8-fluoro-2'-(methylthio)-3,4,5',8'-tetrahydro-2H-spiro[naphthalene-1,7'-pyrano[4,3-d]pyrimidin]-7-yl)carbamate

The mixture of (S*)-1-(7-bromo-5-chloro-8-fluoro-2'-(methylthio)-3,4,5',8'-tetrahydro-2H-spiro[naphthalene-1,7'-pyrano[4,3-d]pyrimidin]-4'-yl)azepane-4-carbonitrile (180 mg, 1 *equiv.,* 326 µmol), tert-butyl carbamate (68.8 mg, 1.8 *equiv.,* 587 µmol), and cesium carbonate (319 mg, 3 *equiv.,* 978 µmol) in DMF (4 mL) was purged with argon and degassed for 10 minutes. To this mixture was added BrettPhos Pd G4 (60.0 mg, 0.2 *equiv.,* 65.2 µmol). The reaction mixture was again degassed with argon for another 5 minutes. The reaction vessel was capped and heated at 80 °C for 4 hours. The reaction mixture was cooled to room temperature, and the solids were filtered over a pad of celite and rinsed with EtOAc (15 mL). The organic layer was separated from the aqueous layer. The aqueous layer was extracted with EtOAc (4 x 15 mL). The combined organic layers were washed with brine (3 x 15 mL), dried over sodium sulfate, and filtered. The solvent was evaporated to give the crude product which was purified by flash column chromatography (10% EtOAc/hexanes to 70% EtOAc/hexanes) to yield *tert-*butyl (5-chloro-4'-((*S**)-4-cyanoazepan-1-yl)-8-fluoro-2'-(methylthio)-3,4,5',8'-tetrahydro-2*H*-spiro[naphthalene-1,7'-pyrano[4,3-d]pyrimidin]-7-yl)carbamate (50 mg) as an off-white solid.
LCMS: m/z (ESI) [M+H]⁺ 588.3, t_{R} = 5.68 minutes (Method B)

### Step 5: tert-Butyl (5-chloro-4'-((S*)-4-cyanoazepan-1-yl)-8-fluoro-2'-(methylsulfonyl)-3,4,5',8'-tetrahydro-2H-spiro[naphthalene-1,7'-pyrano[4,3-d]pyrimidin]-7-yl)carbamate

To a solution of *tert-*butyl (5-chloro-4'-((*S**)-4-cyanoazepan-1-yl)-8-fluoro-2'-(methylthio)-3,4,5',8'-tetrahydro-2*H*-spiro[naphthalene-1,7'-pyrano[4,3-*d*]pyrimidin]-7-yl)carbamate (50 mg, *1 equiv.,* 85 µmol), tetrabutylammonium hydrogen sulfate (4.6 mg, 0.16 *equiv.,* 14 µmol), and sodium tungstate dihydrate (2.8 mg, 0.1 *equiv.,* 8.5 µmol) in EtOAc (2 mL) was added hydrogen peroxide (67 mg, 61 µL, 30 wt%, 7 *equiv.,* 0.60 mmol) dropwise. The resulting reaction mixture was stirred at 40 °C for 45 minutes. The reaction mixture was then quenched by the addition of water (1 mL) and 5% sodium bisulfate (1 mL) and diluted with EtOAc. The organic layer was separated, dried over sodium sulfate, and filtered. The solvent was evaporated *in vacuo* to afford *tert*-butyl (5-chloro-4'-((*S**)-4-cyanoazepan-1-yl)-8-fluoro-2'-(methylsulfonyl)-3,4,5',8'-tetrahydro-2*H*-spiro[naphthalene-1,7'-pyrano[4,3-d]pyrimidin]-7-yl)carbamate (53 mg) which was used in the next step without further purification.
LCMS: m/z (ESI) [M+H]⁺ 620.3, t_{R} = 1.80 minutes (Method B)

### Step 6: tert-Butyl (5-chloro-4'-((S*)-4-cyanoazepan-1-yl)-8-fluoro-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-7-yl)carbamate

((2*R*,7a*S*)-2-fluorotetrahydro-1*H*-pyrrolizin-7a(5*H*)-yl)methanol (41 mg, 3 *equiv., 0.26* mmol) and NaH (10 mg, 60 wt%, 3 *equiv.,* 0.26 mmol) in DMF (1 mL) was stirred at room temperature for 5 minutes. A solution of *tert*-butyl (5-chloro-4'-((*S**)-4-cyanoazepan-1-yl)-8-fluoro-2'-(methylsulfonyl)-3,4,5',8'-tetrahydro-2*H*-spiro[naphthalene-1,1'-pyrano[4,3-*d*]pyrimidin]-7-yl)carbamate (53 mg, *1 equiv.,* 85 µmol) in DMF (1 mL) was added dropwise. The resulting mixture was stirred at room temperature for 1 hour. Upon completion, the reaction mixture was cooled to 0 °C and quenched by the addition of ammonium chloride (5 mL). The aqueous layer was extracted with EtOAc (2 x 10 mL) and with DCM (2 x 20 mL). The combined organic layers were washed with brine, dried over anhydrous sodium sulfate, and concentrated under vacuum to afford crude product. The crude product was purified by flash chromatography on a silica gel column (methanol/DCM/NH₄OH (9:91:1)) to give *tert-*butyl (5-chloro-4'-((*S**)-4-cyanoazepan-1-yl)-8-fluoro-2'-(((2*R*,7a*S*)-2-fluorotetrahydro-1*H-*pyrrolizin-7a(5*H*)-yl)methoxy)-3,4,5',8'-tetrahydro-2H-spiro[naphthalene-1,7'-pyrano[4,3-*d*]pyrimidin]-7-yl)carbamate (40 mg).
LCMS: m/z (ESI) [M+H]⁺ 699.4, t_{R} = 5.45 minutes (Method B)

### Step 7: (4S*)-1-(7-amino-5-chloro-8-fluoro-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)azepane-4-carbonitrile

*p*-Toluenesulfonic acid monohydrate (41 mg, 5 *equiv.,* 0.21 mmol) was added to a solution of *tert-*butyl (5-chloro-4'-((*S**)-4-cyanoazepan-1-yl)-8-fluoro-2'-(((2*R*,7a*S*)-2-fluorotetrahydro-1*H*-pyrrolizin-7a(5*H*)-yl)methoxy)-3,4,5',8'-tetrahydro-2H-spiro[naphthalene-1,7'-pyrano[4,3-*d*]pyrimidin]-7-yl)carbamate (30 mg, *1 equiv.,* 43 µmol) in MeCN (2 mL) at room temperature. The reaction mixture was heated to 60 °C and stirred for 0.5 hours. Upon completion, the solvent was removed under reduced pressure. The crude material was purified using a C18 Biotage column reverse prep with 10 mM ammonium bicarbonate solution and MeCN to give (4*S**)-1-(7-amino-5-chloro-8-fluoro-2'-(((2*R*,7a*S*)-2-fluorotetrahydro-1*H*-pyrrolizin-7a(5*H*)-yl)methoxy)-3,4,5',8'-tetrahydro-2*H-*spiro[naphthalene-1,7'-pyrano[4,3-*d*]pyrimidin]-4'-yl)azepane-4-carbonitrile (18 mg) as a white solid.
LCMS: m/z (ESI) [M+H]⁺ 599.3, t_{R} = 4.60 minutes (Method B)
¹H NMR (400 MHz, DMSO-*d*₆) δ 6.83 (d, 1H), 5.32 (s, 1H), 4.82 (d, 1H), 4.61 (d, 1H), 3.95 (dd, 1H), 3.85 (d, 1H), 3.54 (d, 4H), 3.16 - 2.98 (m, 5H), 2.78 (d, 2H), 2.64 (d, 1H), 2.55 (s, 1H), 2.14 - 1.61 (m, 18H).
¹⁹F NMR (376 MHz, DMSO) δ -131.16, -172.06.

### Example 42: (1S*,4S)-7-Amino-2'-(((2R,7aS)-2-fluorotetrahydro-1H-pyrrolizin-7a(5H)-yl)methoxy)-4'-(1,4-oxazepan-4-yl)-4-(trifluoromethyl)-3,4,5',8'-tetrahydro-2H-spiro[naphthalene-1,7'-pyrano[4,3-d]pyrimidine]-8-carbonitrile (Compound 512b)

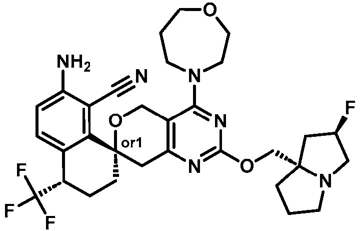

### Step 1: Methyl 4-(4-bromophenyl)-5,5-difluoropent-4-enoate

To a 250 mL round bottom flask was added methyl-4-(4-bromophenyl)-4-oxobutanoate (16 g, 1 *equiv.,* 59.0 mmol) and 2,2-difluoro-2-(tris(dimethylamino)phosphonio)acetate (15.0 g, 1.0 *equiv.,* 59.0 mmol). The round bottom flask was degassed before dry toluene (120 mL) and DMA (40.0 mL) were added. The reaction mixture was then stirred at 100 °C for 4 hours. The contents were then cooled to ambient temperature and the mixture was treated with EtOAc (150 mL) and water (150 mL). The layers were then separated, and the organics were collected along with additional EtOAc (2 x 50 mL). The pooled organic contents were filtered over sodium sulfate and were concentrated *in vacuo.* The crude residue was further purified over a 220 g SiO₂ column (0-30% EtOAc in heptanes) to yield methyl 4-(4-bromophenyl)-5,5-difluoropent-4-enoate (10.0 g).

¹H NMR (400 MHz, CDCl₃) δ 7.52 - 7.45 (m, 2H), 7.20 - 7.14 (m, 2H), 3.63 (s, 3H), 2.72 (m, 2H), 2.35 (m, 2H).

### Step 2: 4-(4-Bromophenyl)-5,5,5-trifluoropentanoic acid

A 1 L 3-necked round bottom flask charged with a condenser and a magnetic stir bar was purged with nitrogen and charged with dry toluene (180 mL) and dry DMA (60.0 mL). The contents were heated to 100 °C before the addition of methyl 4-(4-bromophenyl)-5,5-difluoropent-4-enoate (20.0 g, 1.0 *equiv.,* 65.5 mmol). After confirming internal temperature as 97 °C, TBAF (200 mL, 1.0 M in THF, 3.0 *equiv.,* 200 mmol) was added quickly (within 1.5 mins) via 250 mL addition funnel. The contents were then stirred for 25 minutes. The reaction mixture was cooled to room temperature by placing the reaction vessel in an ice bath. To the mixture was then added water (200 mL) and EtOAc (200 mL). The aqueous layer was then removed and was back extracted with additional EtOAc (2 x 50 mL). The organics were then pooled, filtered over sodium sulfate, and concentrated *in vacuo* to afford crude product (26.9 g) which was used directly in the next step.

Lithium hydroxide monohydrate (5.50 g, 2.0 *equiv.,* 131 mmol), THF (80.0 mL), and water (100 mL) were added to the above crude product (26.9 g) and the resulting reaction mixture was stirred and warmed to 50 °C for 40 minutes. The contents were then cooled to ambient temperature followed by the addition of saturated sodium bicarbonate solution (200 mL). The contents were washed with ether (2 x 40 mL). The ether washes were discarded, and the aqueous layer was acidified to pH ~ 1 with concentrated HCl. The aqueous layer was then extracted with DCM (3 x 80 mL), and the combined organics were filtered over sodium sulfate and concentrated *in vacuo* to afford 4-(4-bromophenyl)-5,5,5-trifluoropentanoic acid (19.0 g) which was used without further purification.

LCMS: m/z (ESI) [M-H]⁻ 308.8, t_{R} = 2.96 minutes (Acquity UPLC, CSH C18, 1.7 µm, 2.1 x 30 mm, Mobile phase A: 0.1% TFA n H₂O; Mobile phase B: 0.1% formic acid in MeCN (v/v); Gradient: 95% H₂O/5% MeCN linear to 5% H₂O/95% MeCN in 4.0 minutes, hold at 5% H₂O/95% MeCN to 4.50 minutes. Then 5% H₂O/95% MeCN linear to 95% H₂O/5% MeCN in 0.5 minutes. Flow rate: 0.6 mL/min.)

### Step 3: 7-Bromo-4-(trifluoromethyl)-3,4-dihydronaphthalen-1(2H)-one

Oxalyl chloride (16.0 mL, 3 *equiv.,* 182 mmol) was added slowly to a light yellow solution of 4-(4-bromophenyl)-5,5,5-trifluoropentanoic acid (19.0 g, 1 *equiv.,* 60.0 mmol)) and DMF (0.5 mL, 0.1 *equiv.,* 6.00 mmol) in DCM (200 mL) under N₂. The resulting solution was stirred at ambient temperature for 1 hour. The contents were then concentrated to dryness and the residue was re-dissolved in DCM (200 mL). Aluminum chloride (24.3 g, 3 *equiv.,* 182 mmol) was added portion-wise at ambient temperature for over 2 minutes. The reaction mixture was quenched with water (200 mL) cooled over ice, diluted with DCM (100 mL), and extracted with DCM (3 x 80 mL). The organics were collected, dried over sodium sulfate, filtered, and concentrated. The crude residue was taken up in DCM and purified by normal phase column chromatography 330 g SiO₂ column (0-30% EtOAc in heptanes) to afford 7-bromo-4-(trifluoromethyl)-3,4-dihydronaphthalen-1(2H)-one (7.43 g).

¹H NMR (400 MHz, CDCl₃) δ 8.24 (d, 1H), 7.69 (dd, 1H), 7.32 (d, 1H), 3.63 (m, 1H), 2.95 - 2.81 (m, 1H), 2.68 (m, 1H), 2.52 (m, 1H), 2.43 - 2.28 (m, 1H).

The enantiomers then separated utilizing chiral prep SFC. Purified on Chiralpak IG (21 x 250 mm, 5 µm, P/N 87445), with isocratic 20% MeOH with 0.25% diethylamine. Enantiopurity assessed via CHIRALPAK IG (4.6 x 100 mm, 5 µm, P/N 87423) isocratic 10% MeOH with 0.25% diethylamine over 2 min. Loaded 4.2 grams of the racemate.
**Peak 1: (*R*)-7-bromo-4-(trifluoromethyl)-3,4-dihydronaphthalen-1(2*H*)-one;**
Chiral SFC rt: 0.84 min; > 99% ee
Mass: 1.41 g (67% recovery)
**Peak 2: (*S*)-7-bromo-4-(trifluoromethyl)-3,4-dihydronaphthalen-1(2*H*)-one:**
   Chiral SFC rt: 1.07 min; 92% ee
   Mass: 1.80 g (86% recovery)

### Step 4: (S)-7-Amino-4-(trifluoromethyl)-3,4-dihydronaphthalen-1(2H)-one

A vial containing tert-butyl carbamate (1.20 g, 3 *equiv.,* 10.2 mmol), (S)-7-bromo-4-(trifluoromethyl)-3,4-dihydronaphthalen-1(2*H*)-one **(Step 3, Peak 2,** 1.00 g, 1 *equiv.,* 3.41 mmol)) cesium carbonate (3.34 g, 3.0 *equiv.,* 10.2 mmol) and BrettPhos Pd-G4 (157 mg, 0.05 *equiv.,* 171 µmol) was degassed and backfilled with nitrogen before dry 1,4-dioxane (20 mL) was added. The mixture was then heated to 80 °C for 3 hours. The reaction mixture was filtered through celite and SiO₂ with EtOAc (100 mL) and DCM (100 mL). The volatiles were removed *in vacuo.* The residue was then dissolved in MeOH (30 mL), treated with TFA (0.78 mL, 3.0 *equiv.,* 10.2 mmol), and HCl (17.0 mL, 2.0 M in ether, 10.0 *equiv.,* 34.1 mmol). The contents were stirred at room temperature for 16 hours. The mixture was then concentrated *in vacuo,* and organics were extracted with EtOAc (2 x 100 mL) and saturated sodium bicarbonate (100 mL). The aqueous layer was removed and was back extracted with DCM (2 x 30 mL). The combined organics were dried over sodium sulfate, filtered, and concentrated under reduced pressure to yield a residue which was further purified via 50 g SiO₂ column eluting 0-60% EtOAc in heptanes to yield (S)-7-amino-4-(trifluoromethyl)-3,4-dihydronaphthalen-1(2*H*)-one (681 mg).
LCMS: m/z (ESI) [M+H]⁺ 230.1, t_{R} = 2.19 minutes (Method K)

### Step 5: (S)-7-Amino-8-bromo-4-(trifluoromethyl)-3,4-dihydronaphthalen-1(2H)-one

To a solution of (*S*)-7-amino-4-(trifluoromethyl)-3,4-dihydronaphthalen-1(2*H*)-one (680 mg, 1.00 *equiv.,* 2.97 mmol) in DMF (10 mL) at 0 °C, was dropwise added a solution of N-bromosuccinimide (554 mg, 1.05 *equiv.,* 3.12 mmol) in DMF (5.0 mL) under nitrogen. The resulting mixture was stirred at 0 °C for 1 hour. The reaction was quenched with water (60 mL) and extracted with DCM (3 x 40 mL). The organics were pooled and dried over sodium sulfate and filtered. The volatiles were removed to yield a residue which was further purified via 50 g SiO₂ column (0-50% EtOAc in heptanes) to afford (*S*)-7-amino-8-bromo-4-(trifluoromethyl)-3,4-dihydronaphthalen-1(2*H*)-one (836 mg).
LCMS: m/z (ESI) [M+H]⁺ 308.2, t_{R} = 2.56 minutes (Method K)

### Step 6: (S)-8-Bromo-7-(dibenzylamino)-4-(trifluoromethyl)-3,4-dihydronaphthalen-1(2H)-one

To a solution of (*S*)-7-amino-8-bromo-4-(trifluoromethyl)-3,4-dihydronaphthalen-1(2H)-one (836 mg, 1.00 *equiv.,* 2.71 mmol) in MeCN (10 mL) at room temperature was added potassium carbonate (938 mg, 2.5 *equiv.,* 6.78 mmol) followed by benzyl bromide (7.10 mL, *22.0 equiv.,* 59.7 mmol). The contents were stirred at ambient temperature then heated to 80 °C and stirred for 18 hours. The reaction was treated with EtOAc (60 mL) and water (40 mL). The aqueous layer was removed and back-extracted with DCM (2 x 50 mL). The combined organic layers were dried over sodium sulfate then filtered. The volatiles were then removed, and residue was purified further via 50 g SiO₂ column (0-30% EtOAc in heptanes) to afford (*S*)-8-bromo-7-(dibenzylamino)-4-(trifluoromethyl)-3,4-dihydronaphthalen-1(2*H*)-one (750 mg).
LCMS: m/z (ESI) [M+H]⁺ 487.8, t_{R} = 3.83 minutes (Method K)

### Step 7: (1S*,4S)-8-Bromo-1-((6-chloro-5-(hydroxymethyl)-2-(methylthio)pyrimidin-4-yl)methyl)-7-(dibenzylamino)-4-(trifluoromethyl)-1,2,3,4-tetrahydronaphthalen-1-ol

To a 250 mL round bottom flask was added (4-chloro-6-methyl-2-(methylthio)pyrimidin-5-yl)methanol (1.26 g, 4.0 *equiv.,* 6.14 mmol) and THF (100 mL). The mixture was cooled to -78 °C, and degassed. LDA solution (1.35 g, 12.6 mL, 1.0 M in THF, *8.2 equiv.,* 12.6 mmol) was added dropwise, while maintaining an internal reaction temperature below - 70 °C. The contents were stirred for 1.5 hours before a separate THF solution of (S)-8-bromo-7-(dibenzylamino)-4-(trifluoromethyl)-3,4-dihydronaphthalen-1(2*H*)-one (750 mg, *1.0 equiv.,* 1.54 mmol) in THF (15.0 mL) was added dropwise, while keeping the internal temperature below - 70 °C. The reaction was stirred at -78 °C for 20 minutes. While cold, the reaction contents were quickly poured into a saturated ammonium chloride solution (approx. 200 mL). The organics were extracted with EtOAc (50 mL) then DCM (2 x 50 mL). The organics were then pooled, dried over sodium sulfate, filtered, and concentrated *in vacuo.* The resulting residue was further purified via 50 g SiO₂ column (eluting 0-40% EtOAc in heptanes) to afford (1*S**,4*S*)-8-bromo-1-((6-chloro-5-(hydroxymethyl)-2-(methylthio)pyrimidin-4-yl)methyl)-7-(dibenzylamino)-4-(trifluoromethyl)-1,2,3,4-tetrahydronaphthalen-1-ol (955 mg) as a diastereopure compound (> 50:1 based on ¹⁹F NMR) compound.
¹⁹F NMR (376 MHz, CDCl₃) δ -66.86 (d, *J =* 9.7 Hz).
LCMS: m/z (ESI) [M+H]⁺ 691.7, t_{R} = 4.13 minutes (Method K)

### Step 8: (1S*,4S)-N,N-Dibenzyl-8-bromo-4'-chloro-2'-(methylthio)-4-(trifluoromethyl)-3,4,5',8'-tetrahydro-2H-spiro[naphthalene-1,7'-pyrano[4,3-d]pyrimidin]-7-amine

To a 250 mL round bottom flask was added (1*S**,4*S*)-8-bromo-1-((6-chloro-5-(hydroxymethyl)-2-(methylthio)pyrimidin-4-yl)methyl)-7-(dibenzylamino)-4-(trifluoromethyl)-1,2,3,4-tetrahydronaphthalen-1-ol (955 mg, 1 *equiv.,* 1.38 mmol) and triphenylphosphine (1.08 g, 3.0 *equiv.,* 4.13 mmol). The vessel was degassed and backfilled with nitrogen and dry toluene (100 mL) was added. DIAD (804 µL, 3.0 *equiv.,* 4.13 mmol) was then added dropwise to the mixture via solution in toluene (20 mL), while monitoring internal temperature. Internal temperature was kept below 2 °C. After addition, the reaction mixture was stirred overnight and gradually warmed to ambient temperature over 18 hours. The reaction contents were concentrated *in vacuo* and loaded onto a 50 SiO₂ column with minimal DCM (elute 0-30% EtOAc in heptanes over 30 minutes) to afford (1*S**,4*S*)-*N,N*-dibenzyl-8-bromo-4'-chloro-2'-(methylthio)-4-(trifluoromethyl)-3,4,5',8'-tetrahydro-2*H-*spiro[naphthalene-1,7'-pyrano[4,3-*d*]pyrimidin]-7-amine (195 mg).
¹⁹F NMR (376 MHz, CDCl₃) δ -66.50 (d, *J =* 9.7 Hz).
LCMS: m/z (ESI) [M+H]⁺ 674.0, t_{R} = 4.47 minutes (Method K)

### Step 9: (1S*,4S)-N,N-Dibenzyl-8-bromo-2'-(methylthio)-4'-(1,4-oxazepan-4-yl)-4-(trifluoromethyl)-3,4,5',8'-tetrahydro-2H-spiro[naphthalene-1,7'-pyrano[4,3-d]pyrimidin]-7-amine

To a vial was added (1*S**,4*S*)-*N,N*-dibenzyl-8-bromo-4'-chloro-2'-(methylthio)-4-(trifluoromethyl)-3,4,5',8'-tetrahydro-2*H*-spiro[naphthalene-1,7'-pyrano[4,3-*d*]pyrimidin]-7-amine (195 mg, 1.0 *equiv.,* 288 µmol), 1,4-oxazepane, HCl (159 mg, 4.0 *equiv.,* 1.15 mmol), MeCN (3.0 mL), and DIPEA (500 µL, 10.0 *equiv.,* 2.88 mmol). The vial was capped and stirred at 90 °C for 4 hours. The reaction mixture was cooled, and EtOAc (30 mL) and water (30 mL) were added. The aqueous layer was removed, then was back extracted with DCM (2 x 30 mL). The organic layers were pooled, dried over sodium sulfate, filtered, and dried *in vacuo.* The material was loaded with DCM onto a 25 g silica column (0-10% MeOH in DCM) to afford (1*S**,4*S*)-*N,N*-dibenzyl-8-bromo-2'-(methylthio)-4'-(1,4-oxazepan-4-yl)-4-(trifluoromethyl)-3,4,5',8'-tetrahydro-2*H*-spiro[naphthalene-1,7'-pyrano[4,3-*d*]pyrimidin]-7-amine (180 mg).
LCMS: m/z (ESI) [M+H]⁺ 739.1 m/z, t_{R} = 3.74 minutes (Method K)

### Step 10: (1S*,4S)-7-(Dibenzylamino)-2'-(methylthio)-4'-(1,4-oxazepan-4-yl)-4-(trifluoromethyl)-3,4,5',8'-tetrahydro-2H-spiro[naphthalene-1,7'-pyrano[4,3-d]pyrimidine]-8-carbonitrile

To a vial charged with copper(I) cyanide (436 mg, 20.0 *equiv.,* 4.87 mmol), was added (1*S**,4*S*)-*N,N*-dibenzyl-8-bromo-2'-(methylthio)-4'-(1,4-oxazepan-4-yl)-4-(trifluoromethyl)-3,4,5',8'-tetrahydro-2*H*-spiro[naphthalene-1,7'-pyrano[4,3-d]pyrimidin]-7-amine (180 mg, 1 *equiv.,* 243 µmol) and dry DMF (6.0 mL) under nitrogen. The resulting mixture was stirred at 120 °C for 2 hours. The reaction mixture was cooled to room temperature. Water (50 mL), DCM (50 mL), NH₄OH(aq) (30 mL), and MeOH (30 mL) were added. The resulting mixture was stirred for 10 minutes vigorously, then the bottom (organic) layer was collected and combined with further DCM washes (2 x 40 mL). The organic layers were pooled, dried over sodium sulfate, filtered, and concentrated *in vacuo.* The residue was then placed on a hot plate set to 38 °C overnight under vacuum to remove residual DMF and to yield (1*S**,4*S*)-7-(dibenzylamino)-2'-(methylthio)-4'-(1,4-oxazepan-4-yl)-4-(trifluoromethyl)-3,4,5',8'-tetrahydro-2*H*-spiro[naphthalene-1,7'-pyrano[4,3-d]pyrimidine]-8-carbonitrile (182 mg), which was used without further purification.
LCMS: m/z (ESI) [M+H]⁺ 686.2 m/z, t_{R} = 3.48 minutes (Method K)

### Step 11: (1S*,4S)-7-(Dibenzylamino)-2'-(methylsulfinyl)-4'-(1,4-oxazepan-4-yl)-4-(trifluoromethyl)-3,4,5',8'-tetrahydro-2H-spiro[naphthalene-1,7'-pyrano[4,3-d]pyrimidine]-8-carbonitrile

To a vial was added (1*S**,4*S*)-7-(dibenzylamino)-2'-(methylthio)-4'-(1,4-oxazepan-4-yl)-4-(trifluoromethyl)-3,4,5',8'-tetrahydro-2*H*-spiro[naphthalene-1,7'-pyrano[4,3-*d*]pyrimidine]-8-carbonitrile (165 mg, *1 equiv.,* 241 µmol), THF (6 mL), MeOH (2 mL), and water (2 mL). Oxone (197 mg, 45 wt%, 0.6 *equiv.,* 144 µmol) was then added in one portion, and the vial was then capped and stirred at 25 °C for 50 minutes. The reaction was quenched by the addition of water (20 mL). EtOAc (30 mL) was then added and the layers were separated. The aqueous phase was extracted further with EtOAc (3 x 20 mL). The combined organic extracts were dried over sodium sulfate, filtered, and concentrated *in vacuo* to afford (1*S**,4*S*)-7-(dibenzylamino)-2'-(methylsulfinyl)-4'-(1,4-oxazepan-4-yl)-4-(trifluoromethyl)-3,4,5',8'-tetrahydro-2*H*-spiro[naphthalene-1,7'-pyrano[4,3-d]pyrimidine]-8-carbonitrile (150 mg).
LCMS: m/z (ESI) [M+H]⁺ 702.2 m/z, t_{R} = 3.40 minutes (Method K)

### Step 12: (1S*,4S)-7-(dibenzylamino)-2'-(((2R,7aS)-2-fluorotetrahydro-1H-pyrrolizin-7a(5H)-yl)methoxy)-4'-(1,4-oxazepan-4-yl)-4-(trifluoromethyl)-3,4,5',8'-tetrahydro-2H-spiro[naphthalene-1,7'-pyrano[4,3-d]pyrimidine]-8-carbonitrile

To a flask charged with ((2*R*,7a*S*)-2-fluorotetrahydro-1*H*-pyrrolizin-7a(5*H*)-yl)methanol (170 mg, 5.0 *equiv.,* 1.07 mmol) and (1*S**,4*S*)-7-(dibenzylamino)-2'-(methylsulfinyl)-4'-(1,4-oxazepan-4-yl)-4-(trifluoromethyl)-3,4,5',8'-tetrahydro-2*H-*spiro[naphthalene-1,7'-pyrano[4,3-*d*]pyrimidine]-8-carbonitrile (150 mg, *1 equiv.,* 214 µmol) was added DMF (3.0 mL) under nitrogen, at room temperature. To this solution was then dropwise added lithium bis(trimethylsilyl)amide (320 µL, 1.0 M in THF, 1.5 *equiv.,* 320 µmol) while the mixture was cooled to 0 °C. The contents were stirred over ice for an additional 10 minutes then stirred at ambient temperature for 2 hours. The reaction mixture was treated with EtOAc (30 mL) and water (20 mL). The aqueous layer was then removed and back extracted with DCM (2 x 20 mL). The organics were then pooled, dried over sodium sulfate, filtered, and concentrated. The residue was further purified via 25 g SiO₂ column (0-100%; 2.5% NH₄OH 20% MeOH 77.5% DCM, in DCM) to afford a complex mixture that contained (1*S**,4*S*)-7-(dibenzylamino)-2'-(((2*R*,7a*S*)-2-fluorotetrahydro-1*H*-pyrrolizin-7a(5*H*)-yl)methoxy)-4'-(1,4-oxazepan-4-yl)-4-(trifluoromethyl)-3,4,5',8'-tetrahydro-2*H-*spiro[naphthalene-1,7'-pyrano[4,3-d]pyrimidine]-8-carbonitrile (60 mg), which was used without further purification.
LCMS: m/z (ESI) [M+H]⁺ 797.4 m/z, t_{R} = 2.59 minutes (Method K)

### Step 13: (1S*,4S)-7-Amino-2'-(((2R,7aS)-2-fluorotetrahydro-1H-pyrrolizin-7a(5H)-yl)methoxy)-4'-(1,4-oxazepan-4-yl)-4-(trifluoromethyl)-3,4,5',8'-tetrahydro-2H-spiro[naphthalene-1,7'-pyrano[4,3-d]pyrimidine]-8-carbonitrile

To a vial charged with (1*S**,4*S*)-7-(dibenzylamino)-2'-(((2*R*,7a*S*)-2-fluorotetrahydro-1*H*-pyrrolizin-7a(5*H*)-yl)methoxy)-4'-(1,4-oxazepan-4-yl)-4-(trifluoromethyl)-3,4,5',8'-tetrahydro-2*H*-spiro[naphthalene-1,7'-pyrano[4,3-*d*]pyrimidine]-8-carbonitrile (60.0 mg, 25 wt%, 1 *equiv.,* 18.8 µmol) was added Pd(OH)₂ on carbon (20 wt%) (106 mg, 8.0 *equiv.,* 150 µmol). MeOH (3 mL) was added along with acetic acid (3 drops), and the vial headspace sparged with hydrogen for 15 minutes. The mixture was then stirred vigorously under an atmosphere of hydrogen (balloon) at room temperature for 18 hours. The contents were then filtered over celite with MeOH (50 mL) and the volatiles were removed *in vacuo.* The residue was dissolved in 3.0 mL of DMF before being purified via reverse-phase prep-HPLC (C₁₈ over three injections, elute 15-35% MeCN (0.1% FA additive) in Water (0.1% FA additive)) to afford (1*S**,4*S*)-7-Amino-2'-(((2*R*,7a*S*)-2-fluorotetrahydro-1*H*-pyrrolizin-7a(5*H*)-yl)methoxy)-4'-(1,4-oxazepan-4-yl)-4-(trifluoromethyl)-3,4,5',8'-tetrahydro-2*H-*spiro[naphthalene-1,7'-pyrano[4,3-*d*]pyrimidine]-8-carbonitrile (2.1 mg).
LCMS: m/z (ESI) [M+H]⁺ 617.3 m/z, t_{R} = 1.74 minutes (Method K)
¹H NMR (400 MHz, MeOD) δ 7.27 (d, *J* = 8.8 Hz, 1H), 6.81 (d, *J* = 8.8 Hz, 1H), 5.32 (d, *J* = 53.5 Hz, 1H), 5.01 (m, 1H), 4.19 (m, 1H), 4.10 (m, 1H), 3.97 - 3.64 (m, 8H), 3.54 (m, 1H), 3.38 (m, 1H), 3.27 (m, 2H), 3.14 (m, 2H), 2.92 (m, 1H), 2.40 - 2.21 (m, 3H), 2.20 - 1.84 (m, 10H).
¹⁹F NMR (376 MHz, MeOD) δ -68.8 (d, *J =* 10.1 Hz), -173.8 (m).

### Example 43: (1R*,4R)-7-amino-2'-(((2R,7aS)-2-fluorotetrahydro-1H-pyrrolizin-7a(5H)-yl)methoxy)-4'-(1,4-oxazepan-4-yl)-4-(trifluoromethyl)-3,4,5',8'-tetrahydro-2H-spiro[naphthalene-1,7'-pyrano[4,3-d]pyrimidine]-8-carbonitrile (Compound 512a)

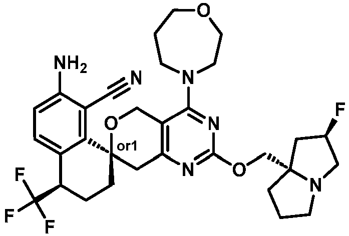

### Step 1: (1R*,4R)-7-(dibenzylamino)-2'-(methylthio)-4'-(1,4-oxazepan-4-yl)-4-(trifluoromethyl)-3,4,5',8'-tetrahydro-2H-spiro[naphthalene-1,7'-pyrano[4,3-d]pyrimidine]-8-carbonitrile

(1*R**,4*R*)-7-(dibenzylamino)-2'-(methylthio)-4'-(1,4-oxazepan-4-yl)-4-(trifluoromethyl)-3,4,5',8'-tetrahydro-2*H*-spiro[naphthalene-1,7'-pyrano[4,3-*d*]pyrimidine]-8-carbonitrile was prepared following **Steps 3-10** from **Example 42** by taking forward (*R*)-7-bromo-4-(trifluoromethyl)-3,4-dihydronaphthalen-1(2*H*)-one **(Step 3, Peak 1** of **Example 42,** 1.0 g, 3.41 mmol) to afford (1*R**,4*R*)-7-(dibenzylamino)-2'-(methylthio)-4'-(1,4-oxazepan-4-yl)-4-(trifluoromethyl)-3,4,5',8'-tetrahydro-2*H*-spiro[naphthalene-1,7'-pyrano[4,3-*d*]pyrimidine]-8-carbonitrile (232 mg).
LCMS: m/z (ESI) [M+H]⁺ 686.6, t_{R} = 2.90 minutes (Method K)

### Step 2: (1R*,4R)-7-(dibenzylamino)-2'-(methylsulfonyl)-4'-(1,4-oxazepan-4-yl)-4-(trifluoromethyl)-3,4,5',8'-tetrahydro-2H-spiro[naphthalene-1,7'-pyrano[4,3-d]pyrimidine]-8-carbonitrile

To a vial was added (1*R**,4*R*)-7-(dibenzylamino)-2'-(methylthio)-4'-(1,4-oxazepan-4-yl)-4-(trifluoromethyl)-3,4,5',8'-tetrahydro-2*H*-spiro[naphthalene-1,7'-pyrano[4,3-*d*]pyrimidine]-8-carbonitrile (232 mg, 1 *equiv.,* 338 µmol), Oxone (693 mg, 45 wt%, 1.5 *equiv.,* 507 µmol), THF (2 mL), MeOH (2 mL), and water (2 mL). The vial was then capped and stirred at 25 °C for 1 hour. The reaction was quenched by the addition of water (20 mL). EtOAc (30 mL) was added, and the layers were separated. The aqueous phase was back-extracted with EtOAc (3 x 20 mL). The combined organic extracts were dried over sodium sulfate, filtered, and concentrated to afford (1*R**,4*R*)-7-(dibenzylamino)-2'-(methylsulfonyl)-4'-(1,4-oxazepan-4-yl)-4-(trifluoromethyl)-3,4,5',8'-tetrahydro-2*H*-spiro[naphthalene-1,7'-pyrano[4,3-*d*]pyrimidine]-8-carbonitrile (205 mg).
LCMS: m/z (ESI) [M+H]⁺ 718.3 m/z, t_{R} = 3.48 minutes (Method K)

### Step 3: (1R*,4R)-7-(Dibenzylamino)-2'-(((2R,7aS)-2-fluorotetrahydro-1H-pyrrolizin-7a(5H)-yl)methoxy)-4'-(1,4-oxazepan-4-yl)-4-(trifluoromethyl)-3,4,5',8'-tetrahydro-2H-spiro[naphthalene-1,7'-pyrano[4,3-d]pyrimidine]-8-carbonitrile

To a flask charged with (2*R*,7a*S*)-2-fluorotetrahydro-1*H*-pyrrolizin-7a(5*H*)-yl)methanol (136 mg, 3.0 *equiv.,* 857 µmol) and (1*R**,4*R*)-7-(dibenzylamino)-2'-(methylsulfonyl)-4'-(1,4-oxazepan-4-yl)-4-(trifluoromethyl)-3,4,5',8'-tetrahydro-2*H-*spiro[naphthalene-1,7'-pyrano[4,3*-d*]pyrimidine]-8-carbonitrile (205 mg, 1 *equiv.,* 286 µmol) was added DMF (3.0 mL) under nitrogen, at room temperature. To this solution was dropwise added lithium bis(trimethylsilyl)amide (71.7 mg, 428 µL, 1.0 M in THF, 1.5 *equiv.,* 428 µmol) at ambient temperature. The contents were then stirred for 70 minutes, after which time, additional (2*R*,7a*S*)-2-fluorotetrahydro-1*H*-pyrrolizin-7a(5*H*)-yl)methanol (80 mg, 1.8 *equiv.,* 504 µmol) was added. The contents were then stirred for an additional 40 minutes. The mixture was then treated with EtOAc (30 mL) and water (20 mL), the aqueous layer was removed and back-extracted with DCM (2 x 20 mL). The combined organic layers were dried over sodium sulfate, filtered, and concentrated *in vacuo* to afford a residue which was purified via column chromatography (0-100%; 2.5% NH₄OH 20% MeOH 77.5% DCM, in DCM) to afford (1*R**,4*R*)-7-(dibenzylamino)-2'-(((2*R*,7a*S*)-2-fluorotetrahydro-1*H*-pyrrolizin-7a(5*H*)-yl)methoxy)-4'-(1,4-oxazepan-4-yl)-4-(trifluoromethyl)-3,4,5',8'-tetrahydro-2*H-*spiro[naphthalene-1,7'-pyrano[4,3-*d*]pyrimidine]-8-carbonitrile (191 mg) which was used without further purification.
LCMS: m/z (ESI) [M+H]⁺ 797.6 m/z, t_{R} = 2.58 minutes (Method K)

### Step 4: (1R*,4R)-7-Amino-2'-(((2R,7aS)-2-fluorotetrahydro-1H-pyrrolizin-7a(5H)-yl)methoxy)-4'-(1,4-oxazepan-4-yl)-4-(trifluoromethyl)-3,4,5',8'-tetrahydro-2H-spiro[naphthalene-1,7'-pyrano[4,3-d]pyrimidine]-8-carbonitrile

To a vial charged with (1*R**,4*R*)-7-(dibenzylamino)-2'-(((2*R*,7a*S*)-2-fluorotetrahydro-1*H*-pyrrolizin-7a(5*H*)-yl)methoxy)-4'-(1,4-oxazepan-4-yl)-4-(trifluoromethyl)-3,4,5',8'-tetrahydro-2*H*-spiro[naphthalene-1,7'-pyrano[4,3-d]pyrimidine]-8-carbonitrile (180 mg, 1 *equiv.,* 226 µmol) was added Pd(OH)₂ on carbon (317 mg, 20 wt%, 2.0 *equiv.,* 452 µmol). To this vial was added MeOH (15 mL), and the resulting mixture was sparged with hydrogen from a balloon, followed by the addition of acetic acid (8 drops). The reaction mixture was stirred vigorously under an atmosphere of hydrogen (balloon) at room temperature for 16 hours. The contents were then filtered over celite with DCM (100 mL) and the volatiles were removed by rotary evaporation. The residue was then dissolved in 2.5 mL DMF and purified via reverse-phase chromatography (C₁₈ over three injections, 200 µL, 1.0 mL, 1.0 mL eluting 5-40% MeCN (0.1% FA additive) in water (0.1% FA additive)) to afford (1*R**,4*R*)-7-amino-2'-(((2*R*,7a*S*)-2-fluorotetrahydro-1*H*-pyrrolizin-7a(5*H*)-yl)methoxy)-4'-(1,4-oxazepan-4-yl)-4-(trifluoromethyl)-3,4,5',8'-tetrahydro-2*H*-spiro[naphthalene-1,7'-pyrano[4,3-*d*]pyrimidine]-8-carbonitrile (8.0 mg).
¹H NMR (400 MHz, MeOD) δ 7.26 (d, *J* = 8.8 Hz, 1H), 6.81 (d, *J* = 8.8 Hz, 1H), 5.28 (d, *J =* 53.3 Hz, 1H), 5.01 (m, 1H), 4.15 (m, 1H), 4.06 (m, 1H), 3.95 - 3.78 (m, 6H), 3.71 (m, 2H), 3.53 (m, 1H), 3.29 - 3.10 (m, 4H), 3.01 (m, 1H), 2.90 (m, 1H), 2.35 - 1.81 (m, 13H).
¹⁹F NMR (376 MHz, MeOD) δ -68.85 (d, J = 10.1 Hz), -173.7 (m).
LCMS: m/z (ESI) [M+H]⁺ 617.4 m/z, t_{R} = 1.80 minutes (Method K)

### Example 44: 5-(7-amino-8-cyano-2'-(((S)-1-methylpyrrolidin-2-yl)methoxy)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide (Compound 513a)

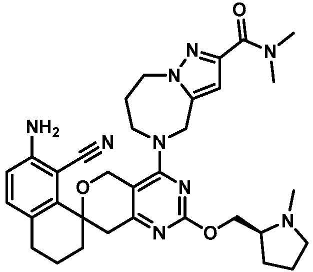

### Step 1: 5-(8-cyano-7-(dibenzylamino)-2'-(((S)-1-methylpyrrolidin-2-yl)methoxy)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide

LiHMDS solution 1.0 M in THF (240 µL, 1.8 *equiv.,* 0.24 mmol) was added to a solution of (*S*)-(1-methylpyrrolidin-2-yl)methanol (78.0 mg, 5 *equiv.,* 0.67 mmol) in anhydrous THF (2.0 mL). To this solution, was added 5-(8-cyano-7-(dibenzylamino)-2'-(methylsulfonyl)-3,4,5',8'-tetrahydro-2*H*-spiro[naphthalene-1,7'-pyrano[4,3-*d*]pyrimidin]-4'-yl)-*N*,*N*-dimethyl-5,6,7,8-tetrahydro-4*H*-pyrazolo[1,5-*a*][1,4]diazepine-2-carboxamide (120 mg, 85 wt%, *1 equiv.,* 0.13 mmol) in anhydrous THF (2.04 mL) over 1 minute at 0°C. The contents were warmed to ambient temperature and stirred for1 hour. Water (1 mL) was added to the mixture and the volatiles were then removed under reduced pressure and the contents were treated with a 1:1 mixture of a saturated aqueous ammonium chloride solution and water at 0 °C. EtOAc was added, and the layers were separated. The organic layers were combined, dried, and concentrated *in vacuo* to give a residue, which was purified by column chromatography (13 g C₁₈ column, liquid loading, 0-100% MeCN in water) to afford 5-(8-cyano-7-(dibenzylamino)-2'-(((*S*)-1-methylpyrrolidin-2-yl)methoxy)-3,4,5',8'-tetrahydro-2*H-*spiro[naphthalene-1,7'-pyrano[4,3-d]pyrimidin]-4'-yl)-*N*,*N*-dimethyl-5,6,7,8-tetrahydro-4*H-*pyrazolo[1,5-*a*][1,4]diazepine-2-carboxamide (68 mg).
LCMS: m/z (ESI) [M+H]⁺ 792.5; t_{R} = 1.38 minutes (Method A)

### Step 2: 5-(7-amino-8-cyano-2'-(((S)-1-methylpyrrolidin-2-yl)methoxy)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide

To a 5 mL flame dried flask, charged with a stirring solution of 5-(8-cyano-7-(dibenzylamino)-2'-(((*S*)-1-methylpyrrolidin-2-yl)methoxy)-3,4,5',8'-tetrahydro-2*H-*spiro[naphthalene-1,7'-pyrano[4,3-*d*]pyrimidin]-4'-yl)-*N*,*N*-dimethyl-5,6,7,8-tetrahydro-4*H-*pyrazolo[1,5-*a*][1,4]diazepine-2-carboxamide (66 mg, 95 wt%, 1 *equiv.,* 0.079 mmol) in MeOH (1.6 mL) sparged with argon, was added Pd(OH)₂ (0.14 g, 20 wt%, 2.5 *equiv.,* 0.20 mmol). The inert atmosphere was exchanged with hydrogen. Three drops of acetic acid were added to the reaction mixture and the contents were stirred overnight under hydrogen atmosphere at room temperature. The suspension was filtered over polytetrafluoroethylene filter and washed with EtOAc, DCM, and MeOH. The filtrate was concentrated to a residue which was purified by reverse phase column chromatography (12 g C₁₈, eluting 0-100% 10 mM Ammonium bicarbonate in water to MeCN followed by 1:1 MeCN/MeOH solution). The product was further purified via preparative HPLC; phase A: 10 mM Ammonium Bicarbonate pH = 10.0, B: MeOH; Flow rate: 45mL/min Column: Gemini^{®} 5 µm NX-C18 110 Å, 100 x 30 mm; Pre-run: 1.5 min at initial condition (55% A, 45% B) to afford 5-(7-amino-8-cyano-2'-(((*S*)-1-methylpyrrolidin-2-yl)methoxy)-3,4,5',8'-tetrahydro-2*H*-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide (10 mg).
LCMS: m/z (ESI) [M+H]⁺ 612.5; t_{R} = 3.43 minutes (Method I)
¹H NMR (400 MHz, DMSO-*d₆*): 7.03 (d, 1 H), 6.71 (d, 1 H), 6.52 (s, 1 H), 5.72 (s, 2 H), 4.97 (m, 1 H), 4.70 (s, 2 H), 4.59 (m, 1 H); 4.45 (s, 2 H); 4.17-4.12 (m, 1 H); 3.98-4.02 (m, 1 H); 3.81-3.92 (m, 2 H); 3.23 (s, 3 H); 3.06 (s, 1 H); 2.91 (s, 3 H); 2.81 (s, 1 H); 2.57-2.64 (m, 3 H); 2.30 (s, 3 H); 2.11-2.17 (m, 3 H); 1.52-2.00 (m, 9 H).

### Example 45: 5-(7-Amino-8-cyano-2'-(((R)-1-methylpyrrolidin-3-yl)methoxy)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide (Compound 514a)

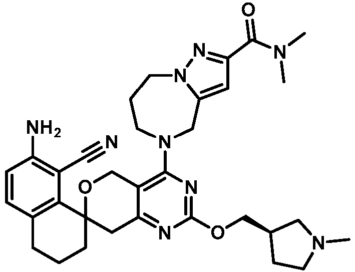

### Step 1: 5-(8-Cyano-7-(dibenzylamino)-2'-(((R)-1-methylpyrrolidin-3-yl)methoxy)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide

LiHMDS solution (1.0 M in THF) (280 µL, 1.8 *equiv.,* 280 µmol) was added to a solution of (*R*)-(1-methylpyrrolidin-3-yl)methanol (90.0 mg, 5 *equiv.,* 0.79 mmol) in anhydrous THF (2.3 mL). A solution of 5-(8-cyano-7-(dibenzylamino)-2'-(methylsulfonyl)-3,4,5',8'-tetrahydro-2*H*-spiro[naphthalene-1,7'-pyrano[4,3-*d*]pyrimidin]-4'-yl)-*N*,*N*-dimethyl-5,6,7,8-tetrahydro-4*H*-pyrazolo[1,5-*a*][1,4]diazepine-2-carboxamide (140 mg, 85 wt%, 1 *equiv.,* 160 µmol) in anhydrous THF (2.3 mL) was added over 1 minute at 0°C. The contents were then warmed to ambient temperature over the course of 5 hours with stirring. The reaction was then heated at 60°C and stirred for 1 hour. Water (1 mL) was added then the volatiles were removed under reduced pressure. The contents were treated by the slow addition of a 1:1 mixture of a saturated aqueous ammonium chloride solution and water at 0 °C. To this mixture was added EtOAc and the layers were separated and extracted. The combined organic layer was dried, concentrated *in vacuo* and purified by column chromatography (reverse phase, 13 g C₁₈, liquid loading, 0-100% MeCN in water) to afford 5-(8-cyano-7-(dibenzylamino)-2'-(((*R*)-1-methylpyrrolidin-3-yl)methoxy)-3,4,5',8'-tetrahydro-2*H*-spiro[naphthalene-1,7'-pyrano[4,3-*d*]pyrimidin]-4'-yl)-*N,N*-dimethyl-5,6,7,8-tetrahydro-4*H*-pyrazolo[1,5-*a*][1,4]diazepine-2-carboxamide (70 mg).
LCMS: m/z (ESI) [M+H]⁺ 792.7; t_{R} = 1.37 minutes (Method A)

### Step 2: 5-(7-Amino-8-cyano-2'-(((R)-1-methylpyrrolidin-3-yl)methoxy)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide

To a 5 mL flame dried flask, charged with a stirring solution of 5-(8-cyano-7-(dibenzylamino)-2'-(((*R*)-1-methylpyrrolidin-3-yl)methoxy)-3,4,5',8'-tetrahydro-2*H-*spiro[naphthalene-1,7'-pyrano[4,3-*d*]pyrimidin]-4'-yl)-*N,N*-dimethyl-5,6,7,8-tetrahydro-4*H-*pyrazolo[1,5-*a*][1,4]diazepine-2-carboxamide (70 mg, 80 wt%., *1 equiv.,* 71 µmol) in MeOH (1.4 mL) sparged with argon, was added Pd(OH)₂ on carbon (120 mg, 20 wt%, 2.5 *equiv.,* 0.18 mmol). The inert atmosphere was exchanged with hydrogen from a balloon. Three drops of acetic acid were added to the reaction mixture and the contents were stirred overnight under a hydrogen atmosphere at room temperature. The suspension was filtered over a polytetrafluoroethylene filter with EtOAc, MeOH, and MeOH/DCM. The filtrate was then concentrated at room temperature under reduced pressure. The crude residue was subjected to reverse phase flash column chromatography (13 g C₁₈, 0-100% aqueous ammonium bicarbonate solution (10mM) to pure acetonitrile). The compound was further purified with preparative HPLC (Mobile phase A: 10 mM Ammonium Bicarbonate pH = 10.0; B: MeOH; Flow rate: 45mL/min; Column: Gemini^{®} 5 µm NX-C18 110 Å, 100 x 30 mm; Pre-run: 1.5 min at initial condition (60% A, 40% B) to afford 5-(7-amino-8-cyano-2'-(((*R*)-1-methylpyrrolidin-3-yl)methoxy)-3,4,5',8'-tetrahydro-2*H*-spiro[naphthalene-1,7'-pyrano[4,3-*d*]pyrimidin]-4'-yl)-*N,N*-dimethyl-5,6,7,8-tetrahydro-4*H*-pyrazolo[1,5-*a*][1,4]diazepine-2-carboxamide (14 mg).
LCMS: m/z (ESI) [M+H]⁺ 612.5; t_{R} = 3.35 minutes (Method I)
¹H NMR (400 MHz, DMSO-*d₆*): 7.03 (d, 1 H); 6.70-6.74 (m, 1 H); 6.53-6.53 (m, 1 H); 5.73-5.75 (m, 2 H); 4.94-4.99 (m, 1 H); 4.68-4.74 (m, 2 H); 4.56-4.63 (m, 1 H); 4.41-4.46 (m, 2 H); 3.99-4.07 (m, 2 H); 3.74-3.89 (m, 2 H); 3.23 (s, 3 H); 3.04-3.09 (m, 1 H); 2.91 (s, 3 H); 2.83 (m, 1 H); 2.55-2.64 (m, 2 H); 2.25-2.35 (m, 2 H); 2.20 (m, 3 H); 2.10-2.15 (m, 1 H); 1.33-1.73 (m, 9 H).

### Example 46: 5-(7-amino-8-cyano-2'-(((S)-1-methylpyrrolidin-3-yl)methoxy)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide (Compound 514b)

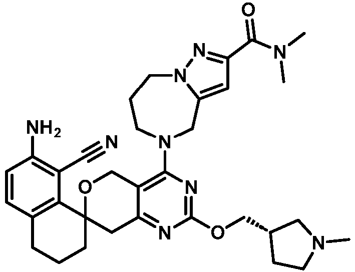

### Step 1: 5-(8-cyano-7-(dibenzylamino)-2'-(((S)-1-methylpyrrolidin-3-yl)methoxy)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide

NaH 60% in mineral oil (3.2 mg, 60 wt%, 1.8 *equiv.,* 81 µmol) was added to a solution of (*S*)-(1-methylpyrrolidin-3-yl)methanol (26 mg, 5 *equiv.,* 0.22 mmol) in anhydrous THF (0.58 mL). A solution of 5-(8-cyano-7-(dibenzylamino)-2'-(methylsulfonyl)-3,4,5',8'-tetrahydro-2*H*-spiro[naphthalene-1,7'-pyrano[4,3-*d*]pyrimidin]-4'-yl)-*N*,*N*-dimethyl-5,6,7,8-tetrahydro-4*H*-pyrazolo[1,5-*a*][1,4]diazepine-2-carboxamide (40 mg, 85 wt%, 1 *equiv.,* 45 µmol) in anhydrous THF (0.58 mL) was then added over 1 minute at 0°C. The contents were then warmed to ambient temperature and stirred for 1 hour. Water (1 mL) was then added to the mixture and the volatiles were removed under reduced pressure. The contents were then subjected to flash column purification (C₁₈, 26 g silicycle cartridge, 0-100% MeCN in water) to afford 5-(8-cyano-7-(dibenzylamino)-2'-(((*S*)-1-methylpyrrolidin-3-yl)methoxy)-3,4,5',8'-tetrahydro-2*H*-spiro[naphthalene-1,7'-pyrano[4,3-*d*]pyrimidin]-4'-yl)-*N*,*N*-dimethyl-5,6,7,8-tetrahydro-4*H*-pyrazolo[1,5-*a*][1,4]diazepine-2-carboxamide (19 mg) which was used without further purification.
LCMS: m/z (ESI) [M+H]⁺ 792.5; t_{R} = 1.38, 1.42 minutes (Method A)

### Step 2: 5-(7-amino-8-cyano-2'-(((S)-1-methylpyrrolidin-3-yl)methoxy)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide

To a 5 mL flame dried flask charged with a stirring solution of 5-(8-cyano-7-(dibenzylamino)-2'-(((*S*)-1-methylpyrrolidin-3-yl)methoxy)-3,4,5',8'-tetrahydro-2*H-*spiro[naphthalene-1,7'-pyrano[4,3-d]pyrimidin]-4'-yl)-*N*,*N*-dimethyl-5,6,7,8-tetrahydro-4*H-*pyrazolo[1,5-*a*][1,4]diazepine-2-carboxamide (45 mg, *1 equiv.,* 57 µmol) in MeOH (1.1 mL) sparged with argon was added Pd(OH)₂ on carbon (0.10 g, 20 wt%, 2.5 *equiv., 0.14* mmol). The inert atmosphere was exchanged with hydrogen from a balloon. Three drops of acetic acid were added to the reaction mixture and the contents were stirred overnight under a hydrogen atmosphere at room temperature. The suspension was then filtered over a polytetrafluoroethylene filter with EtOAc, MeOH, and MeOH/DCM. The volatiles were removed under reduced pressure and the crude residue was subjected to reverse phase flash column chromatography (13 g C₁₈ cartridge, eluting 0-100% aqueous ammonium bicarbonate solution (10 mM) to pure acetonitrile). The compound was further purified via preparative HPLC (Mobile phase A: 10mM Ammonium Bicarbonate pH = 10.0 ; B: MeOH; Flow rate: 45mL/min; Column: Gemini^{®} 5 µm NX-C18 110 Å, 100 x 30 mm; Pre-run: 1.5 min at initial condition (60% A, 40% B) to afford 5-(7-amino-8-cyano-2'-(((*S*)-1-methylpyrrolidin-3-yl)methoxy)-3,4,5',8'-tetrahydro-2*H*-spiro[naphthalene-1,7'-pyrano[4,3-*d*]pyrimidin]-4'-yl)-*N,N*-dimethyl-5,6,7,8-tetrahydro-4*H*-pyrazolo[1,5-*a*][1,4]diazepine-2-carboxamide (3.0 mg).
LCMS: m/z (ESI) [M+H]⁺ 612.5; t_{R} = 3.39 minutes (Method I)
¹H NMR (400 MHz, DMSO-*d₆*): 7.03 (d, 1 H), 6.71 (d, 1 H), 6.53 (s, 1 H), 5.72 (s, 2 H), 4.97 (m, 1 H), 4.70 (m, 2 H), 4.60 (m, 1 H), 4.44 (s, 2H), 4.03 (m, 2 H), 3.81 (s, 2H), 3.23 (s, 3 H), 3.07 (m, 1 H), 2.91 (s, 3 H), 2.83 (m, 1 H), 2.58 (s, 2 H), 2.31-2.35 (m, 1 H), 2.26 (s, 1 H), 2.20 (s, 3 H), 2.12 (s, 1 H), 1.18-2.01 (m, 10H).

### Example 47: (S)-4'-(6-(1H-pyrazol-1-yl)-1,4-oxazepan-4-yl)-7-amino-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidine]-8-carbonitrile (Compound 516a)

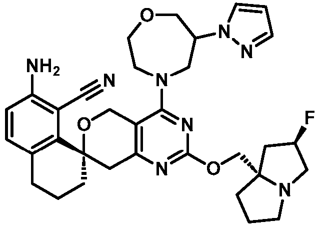

### Step 1: 6-(1H-pyrazol-1-yl)-1,4-oxazepane

Methanesulfonyl chloride (137 mg, 92.6 µL, 1.3 *equiv.,* 1.20 mmol) was added dropwise to a stirred solution of *tert*-butyl 6-hydroxy-1,4-oxazepane-4-carboxylate (200 mg, 1 *equiv.,* 921 µmol) and TEA (279 mg, 385 µL, 3 *equiv.,* 2.76 mmol) in DCM (3.07 mL) at 0 °C. The resulting mixture was stirred at 0 °C for 1 hour. The reaction mixture was diluted with water and DCM. The two phases were separated and the aqueous layer was extracted with DCM (x 3). The combined organic extracts were dried over sodium sulfate, filtered, and concentrated to give *tert*-butyl-6-((methylsulfonyl)oxy)-1,4-oxazepane-4-carboxylate (270 mg), which was used in the next step without further purification.

*tert*-Butyl-6-((methylsulfonyl)oxy)-1,4-oxazepane-4-carboxylate (136 mg, *1 equiv.,* 460 µmol) was dissolved in DMF (2.30 mL). 1H-pyrazole (40.8 mg, 1.3 *equiv.,* 599 µmol) and NaH (36.8 mg, 60 wt%, 2 *equiv.,* 921 µmol) were added and the resulting reaction mixture was heated to 80 °C and stirred overnight. The reaction mixture was then cooled to room temperature and diluted with water and EtOAc. The two phases were separated and the aqueous layer was extracted with EtOAc (x 3). The combined organic extracts were dried over sodium sulfate, filtered, and concentrated to give the crude product which was purified via silica gel chromatography (gradient elution from 0:100-EtOAc:DCM to 40:60-EtOAc:DCM) to give *tert*-butyl-6-(1*H*-pyrazol-1-yl)-1,4-oxazepane-4-carboxylate (63 mg) as a clear colorless oil.

HCl (0.17 g, 1.2 mL, 4.0 M, 20 *equiv.,* 4.7 mmol) in dioxane was added to a stirred solution of *tert*-butyl 6-(1*H*-pyrazol-1-yl)-1,4-oxazepane-4-carboxylate (63 mg, *1 equiv.,* 0.24 mmol) in DCM (2.9 mL). The resulting mixture was stirred at room temperature for 1 hour. The reaction mixture was concentrated to dryness to give the crude product 6-(1*H*-pyrazol-1-yl)-1,4-oxazepane which was used in the next step without further purification.
LCMS: m/z (ESI) [M+H]⁺ 168.2, t_{R} = 0.22 minutes (Method C)
¹H NMR (400 MHz, MeOD) δ 7.78 (d, 1H), 7.63 (s, 1H), 6.40 - 6.33 (m, 1H), 5.01 (s, 1H), 4.24 (m, 1H), 4.10 - 3.98 (m, 3H), 3.86 (m, 1H), 3.75 (m, 1H), 3.51 (m, 2H).

### Step 2: (S)-4'-(6-(1H-pyrazol-1-yl)-1,4-oxazepan-4-yl)-7-amino-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidine]-8-carbonitrile

A solution of (*S*)-7-amino-2'-(((2*R*,7a*S*)-2-fluorotetrahydro-1*H*-pyrrolizin-7a(5*H*)-yl)methoxy)-4'-hydroxy-3,4,5',8'-tetrahydro-2*H*-spiro[naphthalene-1,7'-pyrano[4,3-*d*]pyrimidine]-8-carbonitrile (20 mg, 0.86 mL,1 *equiv.,* 43 µmol) in DMF (0.86 mL) and DIPEA (44 mg, 60 µL, 8 *equiv.,* 0.34 mmol) was added to a vial containing 6-(1*H*-pyrazol-1-yl)-1,4-oxazepane, HCl (18 mg, 2 *equiv.,* 86 µmol) and PyBOP (29 mg, 1.3 *equiv.,* 56 µmol). The resulting mixture was heated to 80 °C and stirred overnight. The reaction mixture was cooled to room temperature and diluted with water and EtOAc. The two phases were separated and the aqueous layer was extracted with EtOAc (x 3). The combined organic extracts were washed with an aqueous solution of 10% LiCl (x 3) to remove residual DMF. The organic layer was dried over sodium sulfate, filtered, and concentrated to give the crude product as a brown solid. The crude product was purified using silica gel chromatography (gradient elution 0:100-A:B to 20:60-A:B where A is a premixed solution of 2.5% NH₄OH and 20% MeOH in DCM and B is DCM) to give (*S*)-4'-(6-(1*H*-pyrazol-1-yl)-1,4-oxazepan-4-yl)-7-amino-2'-(((2*R*,7a*S*)-2-fluorotetrahydro-1*H*-pyrrolizin-7a(5*H*)-yl)methoxy)-3,4,5',8'-tetrahydro-2H-spiro[naphthalene-1,7'-pyrano[4,3-*d*]pyrimidine]-8-carbonitrile (3 mg) as a yellow solid.
LCMS: m/z (ESI) [M+H]⁺ 615.3, t_{R} = 1.27 minutes (Method C)
¹H NMR (400 MHz, CDCl₃) δ 7.62 (d, 1H), 7.53 (d, 1H), 7.04 (d, 1H), 6.68 - 6.61 (m, 1H), 6.31 - 6.23 (m, 1H), 5.28 (d, 1H), 5.09 - 4.78 (m, 3H), 4.43 (d, 2H), 4.34 - 4.24 (m, 1H), 4.24 - 4.17 (m, 1H), 4.17 - 3.87 (m, 5H), 3.84 - 3.60 (m, 2H), 3.40 - 3.19 (m, 3H), 2.99 (d, 2H), 2.68 (d, 2H), 2.34 - 2.05 (m, 4H), 2.01 - 1.90 (m, 3H), 1.84 - 1.70 (m, 3H).

### Example 48: 5-((1aSR,3RS,7bSR)-5-amino-2'-(((2R,7aS)-2-fluorotetrahydro-1H-pyrrolizin-7a(5H)-yl)methoxy)-1,1a,2,5',7b,8'-hexahydrospiro[cyclopropa[a]naphthalene-3,7'-pyrano[4,3-d]pyrimidin]-4'-yl)-N,N-dimethyl-5,6,7,8-tetrahydro-4H-pyrazolo[1,5-a][1,4]diazepine-2-carboxamide (Compound 521a)

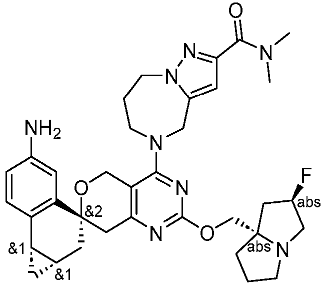

### Step 1: 5-chloro-2-vinylbenzaldehyde

To a 250 mL round bottom flask was added a mixture of 2-bromo-5-chlorobenzaldehyde (2.00 g, 1 *equiv.,* 9.11 mmol), potassium vinyltrifluoroborate (1.83 g, 1.5 *equiv.,* 13.7 mmol), and cesium carbonate (8.91 g, 3 *equiv.,* 27.3 mmol) in 1,4-dioxane (82 mL). The mixture was degassed with nitrogen for 5 minutes, followed by the addition of Pd(PPh₃)₄ (527 mg, 0.05 *equiv.,* 456 µmol). The mixture was heated to 100 °C and stirred for 7 hours. The mixture was cooled to room temperature, quenched with brine (5 mL), and extracted with Et₂O (3 x 10 mL). The organic phase was dried with sodium sulfate, filtered, and concentrated to afford crude product, which was purified via silica gel chromatography (0-40% DCM/Heptanes) to afford 5-chloro-2-vinylbenzaldehyde (1.01 g) as a clear oil.

¹H NMR (400 MHz, CDCl₃) δ 10.24 (s, 1H), 7.78 (s, 1H), 7.55 - 7.49 (m, 2H), 7.48 - 7.37 (m, 1H), 5.68 (d, 1H), 5.54 (d, 1H).

### Step 2: 7-chloro-1,2-dihydronaphthalen-1-ol

To a solution of 5-chloro-2-vinylbenzaldehyde (1.019 g, 1 *equiv.,* 6.116 mmol) in toluene (24 mL) was added allylboronic acid pinacol ester (1.5 g, 1.7 mL, 1.5 *equiv.,* 9.0 mmol). The reaction mixture was stirred at room temperature for 100 minutes. The mixture was diluted with additional toluene (120 mL) and degassed with nitrogen for 5 minutes. Grubbs catalyst (1:1) (260 mg, 0.0501 *equiv.,* 306 µmol) was added and the mixture was stirred overnight at room temperature. The mixture was then concentrated and directly purified via silica gel chromatography (0-20% EtOAc/Heptanes) to afford 7-chloro-1,2-dihydronaphthalen-1-ol (1.09 g) as a brown solid.
¹H NMR (400 MHz, CDCl₃) δ 7.39 (d, 1H), 7.31 - 7.20 (m, 1H), 7.04 (d, 1H), 6.51 (d, 1H), 6.10 - 5.93 (m, 1H), 4.92 - 4.66 (m, 1H), 2.72 - 2.47 (m, 2H).

### Step 3: (1aSR,7bSR)-5-chloro-1a,2,3,7b-tetrahydro-1H-cyclopropa[a]naphthalen-3-ol

Diethylzinc (1.0 M in hexanes) (472 mg, 3.82 mL, 3 *equiv.,* 3.82 mmol) was added to a stirring solution of 7-chloro-1,2-dihydronaphthalen-1-ol (230 mg, *1 equiv.,* 1.27 mmol) in toluene (4.24 mL) at room temperature. After the reaction mixture was stirred for 15 minutes, diiodomethane (1.36 g, 410 µL, 4 *equiv.,* 5.09 mmol) was delivered to the reaction mixture. The resulting mixture was stirred at 80 °C for 3 hours. The reaction mixture was cooled to room temperature and poured into a flask containing a saturated aqueous solution of ammonium chloride. The reaction vessel was rinsed with EtOAc. The two layers were separated, and the aqueous phase was extracted with EtOAc (x 3). The combined organic layers were dried over sodium sulfate, filtered, and concentrated to give the crude product which was purified by silica gel chromatography (gradient elution from 0:100-EtOAc:Heptane to 30:70-EtOAc:Heptane) to provide (1a*SR*,7b*SR*)-5-chloro-1a,2,3,7b-tetrahydro-1*H-*cyclopropa[a]naphthalen-3-ol (180 mg) as a clear colorless oil.

¹H NMR (400 MHz, CDCl₃) δ 7.30 (d, 1H), 7.24 - 7.17 (m, 2H), 4.97 - 4.67 (m, 1H), 2.30 - 2.18 (m, 1H), 2.04 - 1.93 (m, 2H), 1.57 (s, 1H), 1.17 - 1.04 (m, 1H), 1.02 - 0.90 (m, 1H).

### Step 4: (1aSR,7bSR)-5-chloro-1,1a,2,7b-tetrahydro-3H-cyclopropa[a]naphthalen-3-one

Dess-Martin periodinane (471 mg, 345 µL, 1.2 *equiv.,* 1.11 mmol) and sodium bicarbonate (311 mg, 144 µL, 4 *equiv.,* 3.70 mmol) were added sequentially to a stirred solution of (1a*SR*,7b*SR*)-5-chloro-1a,2,3,7b-tetrahydro-1*H*-cyclopropa[a]naphthalen-3-ol (180 mg, 1 *equiv.,* 925 µmol) in DCM (4.62 mL). The resulting mixture was stirred at room temperature for 2 hours. The reaction mixture was quenched by the addition of water and diluted with DCM. The two layers were separated and the aqueous phase was extracted with DCM (x 3). The combined organic layers were dried over sodium sulfate, filtered, and concentrated to give the crude product as a yellow solid. The crude product was purified by silica gel chromatography (gradient elution from 0:100-EtOAc:Heptane to 20:80-EtOAc:Heptane) to give (1a*SR*,7b*SR*)-5-chloro-1,1a,2,7b-tetrahydro-3H-cyclopropa[a]naphthalen-3-one (120 mg) as a clear colorless oil.
LCMS: ESI [M+H]⁺ 193.0 t_{R} = 1.86 minutes (Method A)

### Step 5: (1aSR,3RS,7bSR)-5-chloro-3-((6-chloro-5-(hydroxymethyl)-2-(methylthio)pyrimidin-4-yl)methyl)-1a,2,3,7b-tetrahydro-1H-cyclopropa[a]naphthalen-3-ol

LDA (1.0 M in THF/hexanes) (164 mg, 1.53 mL, 2.5 *equiv.,* 1.53 mmol) was added dropwise to a stirred solution of (4-chloro-6-methyl-2-(methylthio)pyrimidin-5-yl)methanol (150 mg, 1.2 *equiv.,* 735 µmol) in THF (4.08 mL) at -78 °C. The resulting orange solution was stirred at -78 °C for 1 hour. (1a*SR*,7b*SR*)-5-chloro-1,1a,2,7b-tetrahydro-3H-cyclopropa[a]naphthalen-3-one (118 mg, 1.0 *equiv.,* 613 µmol) was then added as a solution in THF (2.04 mL) at -78 °C. The resulting mixture was stirred at -78 °C for 1 hour. The reaction mixture was poured into a flask containing a saturated aqueous solution of ammonium chloride. The quenched mixture was warmed to room temperature and diluted with water and EtOAc. The two phases were separated and the aqueous layer was extracted with EtOAc (x 3). The combined organic extracts were dried over sodium sulfate, filtered, and concentrated to give the crude product as a yellow solid. The crude product was purified by silica gel chromatography (gradient elution 0:100-EtOAc:DCM to 20:80-EtOAc:DCM) to give (1a*SR*,3*RS*,7b*SR*)-5-chloro-3-((6-chloro-5-(hydroxymethyl)-2-(methylthio)pyrimidin-4-yl)methyl)-1a,2,3,7b-tetrahydro-1*H*-cyclopropa[a]naphthalen-3-ol (163 mg) as a white solid.
LCMS: m/z (ESI) [M+H]⁺ 397.1 t_{R} = 2.08 minutes (Method A)

### Step 6: (1aSR,3RS,7bSR)-4',5-dichloro-2'-(methylthio)-1,1a,2,5',7b,8'-hexahydrospiro[cyclopropa[a]naphthalene-3,7'-pyrano[4,3-d]pyrimidine]

To a stirring solution of (1a*SR*,3*RS*,7b*SR*)-5-chloro-3-((6-chloro-5-(hydroxymethyl)-2-(methylthio)pyrimidin-4-yl)methyl)-1a,2,3,7b-tetrahydro-1*H*-cyclopropa[a]naphthalen-3-ol (82 mg, 1 *equiv.,* 0.21 mmol) in DCM (2.1 mL) and THF (2.1 mL) was added polymer-bound triphenylphosphine (0.17 g, 1.3 *equiv.,* 0.27 mmol) in one portion. The resulting mixture was cooled to 0 °C, and DIAD (54 mg, 52 µL, 1.3 *equiv.,* 0.27 mmol) was added dropwise. The cold bath was removed, and the reaction mixture was stirred for 1 hour. The reaction mixture was filtered to remove triphenylphosphine oxide and rinsed with DCM. The filtrate was concentrated to dryness to give the crude product, which was purified by silica gel chromatography (gradient elution from 0:100-EtOAc:Heptane to 100:0-EtOAc:Heptane) to give (1a*SR*,3*RS*,7b*SR*)-4',5-dichloro-2'-(methylthio)-1,1a,2,5',7b,8'-hexahydrospiro[cyclopropa[a]naphthalene-3,7'-pyrano[4,3-*d*]pyrimidine] (38 mg) as a white solid.
LCMS: m/z (ESI) [M+H]⁺ 379.2 t_{R} = 2.46 minutes (Method A)

### Step 7: 5-((1aSR,3RS,7bSR)-5-chloro-2'-(methylthio)-1,1a,2,5',7b,8'-hexahydrospiro[cyclopropa[a]naphthalene-3,7'-pyrano[4,3-d]pyrimidin]-4'-yl)-N,N-dimethyl-5,6,7,8-tetrahydro-4H-pyrazolo[1,5-a][1,4]diazepine-2-carboxamide

DIPEA (77 mg, 0.10 mL, 5 *equiv.,* 0.59 mmol) was added to a stirred solution of *N,N-*dimethyl-5,6,7,8-tetrahydro-4*H*-pyrazolo[1,5-a][1,4]diazepine-2-carboxamide (49 mg, 2 *equiv.,* 0.24 mmol) and (1a*SR*,3*RS*,7b*SR*)-4',5-dichloro-2'-(methylthio)-1,1a,2,5',7b,8'-hexahydrospiro[cyclopropa[a]naphthalene-3,7'-pyrano[4,3-*d*]pyrimidine] (45 mg, 1 *equiv.,* 0.12 mmol) in MeCN (1.2 mL) at room temperature. The resulting mixture was heated to 80 °C and stirred for 16 hours. The reaction mixture was cooled to room temperature and diluted with water and EtOAc. The two layers were separated and the aqueous phase was extracted with EtOAc (x 3). The combined organic layers were dried over sodium sulfate, filtered, and concentrated to give the crude product. The crude product was purified using silica gel chromatography (gradient elution from 0:100-EtOAc:Heptane to 100:0-EtOAc:Heptane) to give 5-((1a*SR*,3*RS*,7b*SR*)-5-chloro-2'-(methylthio)-1,1a,2,5',7b,8'-hexahydrospiro[cyclopropa[a]naphthalene-3,7'-pyrano[4,3-d]pyrimidin]-4'-yl)-*N,N*-dimethyl-5,6,7,8-tetrahydro-4*H*-pyrazolo[1,5-a][1,4]diazepine-2-carboxamide (75 mg) as a white solid.
LCMS: m/z (ESI) [M+H]⁺ 551.2 t_{R} = 1.89 minutes (Method A)

### Step 8: tert-butyl ((1aSR,3RS,7bSR)-4'-(2-(dimethylcarbamoyl)-7,8-dihydro-4H-pyrazolo[1,5-a][1,4]diazepin-5(6H)-yl)-2'-(methylthio)-1,1a,2,5',7b,8'-hexahydrospiro[cyclopropa[a]naphthalene-3,7'-pyrano[4,3-d]pyrimidin]-5-yl)carbamate

To an 8 mL vial was added 5-((1a*SR*,3*RS*,7b*SR*)-5-chloro-2'-(methylthio)-1,1a,2,5',7b,8'-hexahydrospiro[cyclopropa[a]naphthalene-3,7'-pyrano[4,3-*d*]pyrimidin]-4'-yl)-*N*,*N*-dimethyl-5,6,7,8-tetrahydro-4*H*-pyrazolo[1,5-*a*][1,4]diazepine-2-carboxamide (75.0 mg, *1 equiv.,* 136 µmol), *tert*-butyl carbamate (79.7 mg, 5 *equiv.,* 680 µmol), cesium carbonate (133 mg, 3 *equiv.,* 408 µmol), and BrettPhos Pd G4 (18.8 mg, 0.15 *equiv.,* 20.4 µmol). The vial was then flushed with nitrogen and degassed. To this mixture, 1,4-dioxane (2.72 mL) was added. The resulting mixture was heated to 80 °C and stirred at this temperature for 16 hours. The reaction mixture was cooled to room temperature, filtered, and concentrated to give the crude product which was purified by silica gel chromatography (gradient elution from 0:100-EtOAc:DCM to 60:40-EtOAc:DCM to 100:0-EtOAc:DCM) to give *tert*-butyl ((1a*SR*,3*RS*,7b*SR*)-4'-(2-(dimethylcarbamoyl)-7,8-dihydro-4*H*-pyrazolo[1,5-*a*][1,4]diazepin-5(6*H*)-yl)-2'-(methylthio)-1,1a,2,5',7b,8'-hexahydrospiro[cyclopropa[a]naphthalene-3,7'-pyrano[4,3-*d*]pyrimidin]-5-yl)carbamate (80 mg) as a white solid.
LCMS: m/z (ESI) [M+H]⁺ 632.3 t_{R} = 1.84 minutes (Method A)

### Step 9: tert-butyl ((1aSR,3RS,7bSR)-4'-(2-(dimethylcarbamoyl)-7,8-dihydro-4H-pyrazolo[1,5-a][1,4]diazepin-5(6H)-yl)-2'-(methylsulfinyl)-1,1a,2,5',7b,8'-hexahydrospiro[cyclopropa[a]naphthalene-3,7'-pyrano[4,3-d]pyrimidin]-5-yl)carbamate

Oxone (73 mg, 1 *equiv.,* 0.12 mmol) was added to a stirred solution of *tert-*butyl ((1a*SR*,3*RS*,7b*SR*)-4'-(2-(dimethylcarbamoyl)-7,8-dihydro-4*H*-pyrazolo[1,5-*a*][1,4]diazepin-5(6*H*)-yl)-2'-(methylthio)-1,1a,2,5',7b,8'-hexahydrospiro[cyclopropa[a]naphthalene-3,7'-pyrano[4,3*-d*]pyrimidin]-5-yl)carbamate (80 mg, 1 *equiv.,* 0.12 mmol) in THF (0.93 mL), water (0.93 mL), and MeOH (1.5 mL). The resulting mixture was stirred at room temperature for 3 hours. The reaction mixture was diluted with water and EtOAc. The two phases were separated and the aqueous layer was extracted with EtOAc (x 3). The combined organic extracts were dried over sodium sulfate, filtered, and concentrated to give *tert-*butyl ((1a*SR*,3*RS*,7b*SR*)-4'-(2-(dimethylcarbamoyl)-7,8-dihydro-4*H*-pyrazolo[1,5-*a*][1,4]diazepin-5(6*H*)-yl)-2'-(methylsulfinyl)-1,1a,2,5',7b,8'-hexahydrospiro[cyclopropa[a]naphthalene-3,7'-pyrano[4,3-*d*]pyrimidin]-5-yl)carbamate as a white solid, which was used in the next step without further purification.
LCMS: m/z (ESI) [M+H]⁺ 648.3 t_{R} = 1.77 minutes (Method A)

### Step 10: tert-butyl ((1aSR,3RS,7bSR)-4'-(2-(dimethylcarbamoyl)-7,8-dihydro-4H-pyrazolo[1,5-a][1,4]diazepin-5(6H)-yl)-2'-(((2R,7aS)-2-fluorotetrahydro-1H-pyrrolizin-7a(5H)-yl)methoxy)-1,1a,2,5',7b,8'-hexahydrospiro[cyclopropa[a]naphthalene-3,7'-pyrano[4,3-d]pyrimidin]-5-yl)carbamate

A stirred solution of ((2*R*,7a*S*)-2-fluorotetrahydro-1*H*-pyrrolizin-7a(5*H*)-yl)methanol (21 mg, 1.1 *equiv.,* 0.13 mmol) and *tert*-butyl ((1a*SR*,3*RS*,7b*SR*)-4'-(2-(dimethylcarbamoyl)-7,8-dihydro-4H-pyrazolo[1,5-*a*][1,4]diazepin-5(6*H*)-yl)-2'-(methylsulfinyl)-1,1a,2,5',7b,8'-hexahydrospiro[cyclopropa[a]naphthalene-3,7'-pyrano[4,3-*d*]pyrimidin]-5-yl)carbamate (79 mg, *1 equiv.,* 0.12 mmol) in DMF (2.4 mL) was cooled to 0 °C. Lithium bis(trimethylsilyl)amide (41 mg, 0.24 mL, 1.0 M, 2 *equiv.,* 0.24 mmol) was added dropwise to the reaction mixture at 0 °C. The ice bath was removed, and the resulting mixture was stirred at room temperature for 2 hours. The reaction mixture was quenched by the addition of a saturated aqueous solution of sodium bicarbonate. The quenched mixture was warmed to room temperature and diluted with EtOAc. The two phases were separated and the aqueous layer was extracted with EtOAc (x 3). The combined organic extracts were dried over sodium sulfate, filtered, and concentrated to give the crude product, which was purified via silica gel chromatography (gradient elution 0:100-A:B to 20:60-A:B where A is a premixed solution of 2.5% NH₄OH and 20% MeOH in DCM and B is DCM) to give *tert-*butyl ((1a*SR*,3*RS*,7b*SR*)-4'-(2-(dimethylcarbamoyl)-7,8-dihydro-4*H*-pyrazolo[1,5-*a*][1,4]diazepin-5(6*H*)-yl)-2'-(((2*R*,7a*S*)-2-fluorotetrahydro-1*H*-pyrrolizin-7a(5*H*)-yl)methoxy)-1,1a,2,5',7b,8'-hexahydrospiro[cyclopropa[a]naphthalene-3,7'-pyrano[4,3-*d*]pyrimidin]-5-yl)carbamate (54 mg) as a white solid.
LCMS: m/z (ESI) [M+H]⁺ 743.4 t_{R} = 1.43 minutes (Method A)

### Step 11: 5-((1aSR,3RS,7bSR)-5-amino-2'-(((2R,7aS)-2-fluorotetrahydro-1H-pyrrolizin-7a(5H)-yl)methoxy)-1,1a,2,5',7b,8'-hexahydrospiro[cyclopropa[a]naphthalene-3,7'-pyrano[4,3-d]pyrimidin]-4'-yl)-N,N-dimethyl-5,6,7,8-tetrahydro-4H-pyrazolo[1,5-a][1,4]diazepine-2-carboxamide

Hydrogen chloride (2 M) in ether (29 mg, 0.40 mL, 20 *equiv.,* 0.81 mmol) was added to a stirred solution of *tert*-butyl ((1a*SR*,3*RS*,7b*SR*)-4'-(2-(dimethylcarbamoyl)-7,8-dihydro-4*H*-pyrazolo[1,5-*a*][1,4]diazepin-5(6*H*)-yl)-2'-(((2*R*,7a*S*)-2-fluorotetrahydro-1*H*-pyrrolizin-7a(5*H*)-yl)methoxy)-1,1a,2,5',7b,8'-hexahydrospiro[cyclopropa[a]naphthalene-3,7'-pyrano[4,3-*d*]pyrimidin]-5-yl)carbamate (30 mg, *1 equiv.,* 40 µmol) in DCM (0.40 mL). The resulting mixture was stirred at room temperature for 1 hour. The reaction mixture was concentrated to dryness to give the crude product as a white solid. To the crude product was added DCM and a solution of saturated aqueous sodium bicarbonate. The two phases were separated and the aqueous layer was extracted with DCM (x 3). The combined organic extracts were dried over sodium sulfate, filtered, and concentrated to give 5-((1a*SR,*3*RS*,7b*SR*)-5-amino-2'-(((2*R*,7a*S*)-2-fluorotetrahydro-1*H*-pyrrolizin-7a(5*H*)-yl)methoxy)-1,1a,2,5',7b,8'-hexahydrospiro[cyclopropa[a]naphthalene-3,7'-pyrano[4,3-*d*]pyrimidin]-4'-yl)-*N,N*-dimethyl-5,6,7,8-tetrahydro-4*H*-pyrazolo[1,5-*a*][1,4]diazepine-2-carboxamide (12 mg) as a white solid.
LCMS: m/z (ESI) [M+H]⁺ 643.3 t_{R} = 1.17 minutes (Method A)
¹H NMR (400 MHz, CDCl₃) δ 7.16 (d, 1H), 6.65 - 6.57 (m, 1H), 6.38 - 6.32 (m, 1H), 6.26 (d, 1H), 5.35 - 5.06 (m, 2H), 4.54 - 4.39 (m, 2H), 4.39 - 4.21 (m, 3H), 4.19 - 4.13 (m, 1H), 4.13 - 3.85 (m, 4H), 3.58 (s, 2H), 3.51 - 3.37 (m, 1H), 3.36 - 3.06 (m, 4H), 3.04 - 2.84 (m, 6H), 2.38 - 2.03 (m, 5H), 2.02 - 1.72 (m, 4H), 1.71 - 1.46 (m, 1H), 1.42 - 1.13 (m, 2H), 1.01 - 0.93 (m, 1H), 0.93 - 0.80 (m, 1H).
¹⁹F NMR (376 MHz, CDCl₃) δ -172.99.

### Example 49: 4-((S)-7-amino-8-cyano-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)-N,N-dimethyl-1,4-oxazepane-6-carboxamide (Compound 519a)

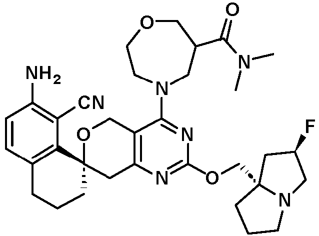

### Step 1: tert-butyl 6-(dimethylcarbamoyl)-1,4-oxazepane-4-carboxylate

DIPEA (371 mg, 500 µL, 3.01 *equiv.,* 2.87 mmol) was added dropwise to a solution of (*R*)-4-(*tert*-butoxycarbonyl)-1,4-oxazepane-6-carboxylic acid (233.8 mg, *1 equiv.,* 953.2 µmol) and HATU (478 mg, 1.32 *equiv.,* 1.26 mmol) in DCM (4.8 mL) at room temperature. The mixture was stirred for 10 minutes and dimethylamine hydrochloride (161 mg, 2.07 *equiv.,* 1.97 mmol) was added. The mixture was stirred for 2 hours. The mixture was quenched with saturated aqueous sodium carbonate. The layers were separated and washed with ammonium chloride and brine. The organics were separated, combined, dried over magnesium sulfate, filtered, and concentrated. The residue was purified by silica gel column chromatography (0-20% MeOH in DCM) to afford *tert*-butyl 6-(dimethylcarbamoyl)-1,4-oxazepane-4-carboxylate (202 mg) as an orange oil.
LCMS: m/z (ESI) [M+H-Boc] 173.3 t_{R} = 1.43 minutes (Method A)

### Step 2: N,N-dimethyl-1,4-oxazepane-6-carboxamide

Hydrogen chloride (4 M) in dioxane (0.38 g, 2.6 mL, 15 *equiv.,* 10 mmol) was added to a solution of *tert*-butyl 6-(dimethylcarbamoyl)-1,4-oxazepane-4-carboxylate (194 mg, 1 *equiv.,* 712 µmol) in DCM (1.4 mL) at room temperature. The reaction mixture was stirred for 4 hours. The mixture was directly concentrated and triturated with ether to yield *N,N-*dimethyl-1,4-oxazepane-6-carboxamide (152 mg, HCl salt) as an orange solid.

¹H NMR (400 MHz, MeOD) δ 4.22 - 4.15 (m, 1H), 4.08 - 3.98 (m, 1H), 3.97 - 3.89 (m, 1H), 3.87 - 3.80 (m, 1H), 3.79 - 3.54 (m, 3H), 3.50 - 3.36 (m, 3H), 3.12 (s, 3H), 2.98 (s, 3H).

### Step 3: 4-((S)-7-amino-8-cyano-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)-N,N-dimethyl-1,4-oxazepane-6-carboxamide

*N*,*N*-dimethyl-1,4-oxazepane-6-carboxamide, HCl (31 mg, 3.5 *equiv.,* 0.15 mmol) and PyBOP (29 mg, 1.3 *equiv.,* 56 µmol) were added to a solution of (*S*)-7-amino-2'-(((2*R*,7a*S*)-2-fluorotetrahydro-1*H*-pyrrolizin-7a(5*H*)-yl)methoxy)-4'-hydroxy-3,4,5',8'-tetrahydro-2*H-*spiro[naphthalene-1,7'-pyrano[4,3-*d*]pyrimidine]-8-carbonitrile (20 mg, 0.86 mL, 0.05 M, 1 *equiv.,* 43 µmol) in DMF (0.86 mL). DIPEA (44 mg, 60 µL, 8 *equiv.,* 0.34 mmol) was then added dropwise and the resulting reaction mixture was heated to 80 °C. After 1 hour, additional (*R*)*-N*,*N*-dimethyl-1,4-oxazepane-6-carboxamide (17 mg, 2.3 *equiv.,* 99 µmol) with DIPEA (0.03 g, 0.04 mL, 5 *equiv.,* 0.2 mmol) was added. The resulting mixture was stirred at 80 °C for 16 hours. The reaction mixture was then cooled to room temperature and diluted with water and EtOAc. The organic layers were extracted with EtOAc three times and washed with 5% aqueous LiCl solution. The resulting organic layers were separated, combined, dried over magnesium sulfate, filtered, and concentrated to an orange oil. The oil was triturated with ether and the residue obtained was purified by silica column chromatography to afford 4-((*S*)-7-amino-8-cyano-2'-(((2*R*,7a*S*)-2-fluorotetrahydro-1*H*-pyrrolizin-7a(5*H*)-yl)methoxy)-3,4,5',8'-tetrahydro-2*H*-spiro[naphthalene-1,7'-pyrano[4,3-*d*]pyrimidin]-4'-yl)-*N*,*N*-dimethyl-1,4-oxazepane-6-carboxamide (3.3 mg) as a white solid.
LCMS: m/z (ESI) [M+H]⁺ 620.3 t_{R} =1.23 minutes (Method A)
¹H NMR (400 MHz, CDCl₃) δ 7.08 - 6.85 (m, 1H), 6.74 - 6.48 (m, 1H), 5.35 - 5.07 (m, 1H), 4.97 (d, 1H), 4.88 - 4.69 (m, 1H), 4.36 (d, 2H), 4.27 - 3.99 (m, 1H), 3.83 (qd, 3H), 3.74 - 3.39 (m, 3H), 3.32 - 3.19 (m, 1H), 3.12 (d, 3H), 2.97 - 2.82 (m, 4H), 2.61 (s, 2H), 2.23 - 1.58 (m, 10H), 1.32 - 1.09 (m, 6H), 0.87 - 0.71 (m, 2H).
¹⁹F NMR (376 MHz, CDCl₃) δ -173.3

### Example 50: (S)-7-Amino-2'-(((2R,7aS)-2-fluorotetrahydro-1H-pyrrolizin-7a(5H)-yl)methoxy)-4'-(2,6-dioxa-9-azaspiro[3.6]decan-9-yl)-3,4,5',8'-tetrahydro-2H-spiro[naphthalene-1,7'-pyrano[4,3-d]pyrimidine]-8-carbonitrile (Compound 518a)

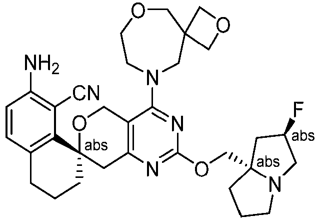

To a solution of (*S*)-7-amino-2'-(((2*R*,7a*S*)-2-fluorotetrahydro-1*H*-pyrrolizin-7a(5*H*)-yl)methoxy)-4'-hydroxy-3,4,5',8'-tetrahydro-2*H*-spiro[naphthalene-1,7'-pyrano[4,3-*d*]pyrimidine]-8-carbonitrile in DMF (0.80 mL, 0.05 M, 0.040 mmol) was added 2,6-dioxa-9-azaspiro[3.6]decane, HCl (26.6 mg, 0.148 mmol) and PyBOP (30 mg, 0.058 mmol). DIPEA (0.056 mL, 0.32 mmol) was then added dropwise, and the resulting mixture was stirred at 80 °C for 1 hour. The reaction mixture was cooled to room temperature and diluted with a mixture of water (5 mL), 5% aqueous LiCl solution (5 mL), and EtOAc (10 mL). The aqueous layer was extracted with EtOAc (3 x 10 mL). The combined organic layers were dried over sodium sulfate, filtered, and concentrated to a dark orange solid. The crude residue was taken up in DMSO and purified by reverse phase column chromatography (XSelect^{®} CSH^{™} Prep C18 5 µm OBD^{™} 19x150 mm, 0-100% 0.1% NH₄OH in MeCN: 0.1% NH₄OH in Water, focused gradient 45-55%) to yield (*S*)-7-amino-2'-(((2*R*,7a*S*)-2-fluorotetrahydro-1*H*-pyrrolizin-7a(5*H*)-yl)methoxy)-4'-(2,6-dioxa-9-azaspiro[3.6]decan-9-yl)-3,4,5',8'-tetrahydro-2*H-*spiro[naphthalene-1,7'-pyrano[4,3-*d*]pyrimidine]-8-carbonitrile (12.2 mg) as an off-white solid.
LCMS: m/z (ESI) [M+H]⁺ 591.352 m/z, t_{R} = 1.22 minutes (Method C).
¹H NMR (400 MHz, DMSO-*d₆*) δ 7.04 (d, 1H), 6.72 (d, 1H), 5.74 (s, 2H), 5.36 - 5.15 (m, 1H), 4.83 (d1H), 4.67 - 4.59 (m, 2H), 4.42 (d, 1H), 4.30 - 4.20 (m, 2H), 4.05 (d, 1H), 3.95 (d1H), 3.92 - 3.81 (m, 2H), 3.77 (m, 2H), 3.68 (d, 1H), 3.60 - 3.51 (m, 3H), 3.15 - 3.03 (m, 3H), 2.99 (s, 1H), 2.90 - 2.78 (m, 2H), 2.63 - 2.56 (m, 2H), 2.21 - 2.14 (m, 1H), 2.10 - 2.04 (m, 1H), 2.03 - 1.92 (m, 2H), 1.87 - 1.64 (m, 6H).

### Example 51: (R*)-1-(7-amino-5-chloro-8-fluoro-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)azepane-4-carbonitrile (Compound 510b)

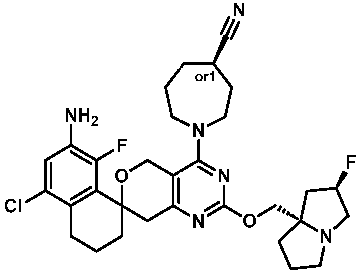

### Step 1: (4R*)-1-(7-Bromo-5-chloro-8-fluoro-2'-(methylthio)-3,4,5',8'-tetrahydro-2H-spiro[naphthalene-1,7'-pyrano[4,3-d]pyrimidin]-4'-yl)azepane-4-carbonitrile

To a 20 mL screw-capped vial fitted with a septum, a mixture of *rel*-(*R*)-azepane-4-carbonitrile hydrochloride (128 mg, *1 equiv.,* 797 µmol), and DIPEA (1.39 mL, 10 *equiv.,* 7.97 mmol) were dissolved in DMA (3 mL). 7-Bromo-4',5-dichloro-8-fluoro-2'-(methylthio)-3,4,5',8'-tetrahydro-2*H*-spiro[naphthalene-1,7'-pyrano[4,3-*d*]pyrimidine] (370 mg, *1 equiv.,* 797 µmol) in dioxane (3 mL) was then added. The resulting mixture was heated at 120 °C and stirred for 6 hours. The reaction mixture was then brought to ambient temperature, and the volatiles were removed *in vacuo.* The crude residue was purified by flash column chromatography (24 g cartridge, liquid load, eluting with Hexanes/EtOAc (0-70%)) to afford (4*R**)-1-(7-bromo-5-chloro-8-fluoro-2'-(methylthio)-3,4,5',8'-tetrahydro-2*H-*spiro[naphthalene-1,7'-pyrano[4,3-*d*]pyrimidin]-4'-yl)azepane-4-carbonitrile (210 mg).
LCMS: m/z (ESI) [M+H]⁺ 551.1, t_{R}= 2.04 minutes (Method B)

### Step 2: tert-butyl (5-chloro-4'-((R*)-4-cyanoazepan-1-yl)-8-fluoro-2'-(methylthio)-3,4,5',8'-tetrahydro-2H-spiro[naphthalene-1,7'-pyrano[4,3-d]pyrimidin]-7-yl)carbamate

Into a flame-dried 5 mL microwave vial, a mixture of (4*R**)-1-(7-bromo-5-chloro-8-fluoro-2'-(methylthio)-3,4,5',8'-tetrahydro-2*H*-spiro[naphthalene-1,7'-pyrano[4,3-*d*]pyrimidin]-4'-yl)azepane-4-carbonitrile (50 mg, 1 *equiv.,* 91 µmol), *tert*-butyl carbamate (19 mg, 1.8 *equiv.,* 0.16 mmol), and cesium carbonate (74 mg, 2.5 *equiv.,* 0.23 mmol) was dissolved in anhydrous DMF (2 mL). The vial was purged with argon and sparged for 10 minutes. To this mixture was added BrettPhos Pd G4 (13 mg, 0.15 *equiv.,* 14 µmol) in DMF (1 mL). The reaction mixture was again sparged with argon for 5 minutes. The reaction vessel was then heated at 90 °C for 3 hours. Upon cooling to 22 °C, the contents were then filtered through celite, and the solid was further washed with EtOAc (15 mL). The organic filtrate was collected and washed with brine (10 mL), dried over sodium sulfate, and filtered. The volatiles were evaporated *in vacuo* and the residue was purified by flash column chromatography (24 g cartridge, dry injection, eluting with EtOAc/hexanes (10-70%)) to yield *tert*-butyl (5-chloro-4'-((*R**)-4-cyanoazepan-1-yl)-8-fluoro-2'-(methylthio)-3,4,5',8'-tetrahydro-2*H-*spiro[naphthalene-1,7'-pyrano[4,3-*d*]pyrimidin]-7-yl)carbamate (23 mg).
LCMS: m/z (ESI) [M+H]⁺ 588.3 t_{R} = 5.65, 5.66 minutes (Method I)

### Step 3: tert-Butyl (5-chloro-4'-((R*)-4-cyanoazepan-1-yl)-8-fluoro-2'-(methylsulfonyl)-3,4,5',8'-tetrahydro-2H-spiro[naphthalene-1,7'-pyrano[4,3-d]pyrimidin]-7-yl)carbamate

Into a 10 mL screw-capped vial with a septum was added *tert-*butyl (5-chloro-4'-((*R**)-4-cyanoazepan-1-yl)-8-fluoro-2'-(methylthio)-3,4,5',8'-tetrahydro-2*H*-spiro[naphthalene-1,7'-pyrano[4,3-*d*]pyrimidin]-7-yl)carbamate (50 mg, 1 *equiv.,* 85 µmol), sodium tungstate dihydrate (2.8 mg, 0.1 *equiv.,* 8.5 µmol), tetrabutylammonium hydrogen sulfate (4.6 mg, 0.16 *equiv.,* 14 µmol), and EtOAc (3 mL). Hydrogen peroxide (61 µL, 30 wt%, 7 *equiv.,* 0.60 mmol) was then added dropwise to the resulting reaction mixture. The reaction mixture was heated to 50 °C and stirred for 45 minutes. The reaction was then quenched with 1 mL of water and 1 mL of 5% aqueous sodium bisulfate solution. Additional EtOAc (5 mL) was then added and the layers were separated. The organic layers were combined, dried over sodium sulfate, and evaporated *in vacuo* to afford crude *tert*-butyl (5-chloro-4'-((*R**)-4-cyanoazepan-1-yl)-8-fluoro-2'-(methylsulfonyl)-3,4,5',8'-tetrahydro-2*H*-spiro[naphthalene-1,7'-pyrano[4,3-*d*]pyrimidin]-7-yl)carbamate (53 mg). The crude residue was used directly in the next step without further purification.
LCMS: m/z (ESI) [M+H]⁺ 620.3 t_{R} = 1.80 minutes (Method B)

### Step 4: tert-butyl (5-chloro-4'-((R*)-4-cyanoazepan-1-yl)-8-fluoro-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-7-yl)carbamate

A mixture of (2*R*,7a*S*)-2-fluorotetrahydro-1*H*-pyrrolizin-7a-(5*H*)-yl)methanol (41 mg, 3 *equiv.,* 0.26 mmol) and NaH (10 mg, 60 wt%, 3 *equiv.,* 0.26 mmol) in THF (2 mL) was stirred at 25 °C for 5 minutes. Then a second solution of *tert*-butyl (5-chloro-4'-((*R**)-4-cyanoazepan-1-yl)-8-fluoro-2'-(methylsulfonyl)-3,4,5',8'-tetrahydro-2*H*-spiro[naphthalene-1,7'-pyrano[4,3-*d*]pyrimidin]-7-yl)carbamate (53 mg, *1 equiv.,* 85 µmol) in THF (1 mL) was added dropwise. The resulting mixture was stirred at 25 °C for 1 hour. The mixture was cooled to 0 °C and quenched by the addition of saturated aqueous ammonium chloride solution (5 mL). The organic layers were then extracted with EtOAc (2 x 30 mL) then with DCM (2 x 30 mL). The organic layers were combined, dried over anhydrous sodium sulfate, and concentrated under vacuum. The residue was purified by flash chromatography (24 g silica column, eluting with Methanol/DCM/NH₄OH (9:91:1 ratio)) to afford *tert*-butyl (5-chloro-4'-((*R**)-4-cyanoazepan-1-yl)-8-fluoro-2'-(((2*R*,7a*S*)-2-fluorotetrahydro-1*H*-pyrrolizin-7a(5*H*)-yl)methoxy)-3,4,5',8'-tetrahydro-2H-spiro[naphthalene-1,7'-pyrano[4,3-*d*]pyrimidin]-7-yl)carbamate (45 mg) as an off-white solid.
LCMS: m/z (ESI) [M+H]⁺ 699.5 t_{R} = 5.43 minutes (Method I)

### Step 5: (R*)-1-(7-amino-5-chloro-8-fluoro-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)azepane-4-carbonitrile

To a 10 mL sealed vial with a septum was added *tert*-butyl (5-chloro-4'-((*R**)-4-cyanoazepan-1-yl)-8-fluoro-2'-(((2*R*,7a*S*)-2-fluorotetrahydro-1*H*-pyrrolizin-7a(5*H*)-yl)methoxy)-3,4,5',8'-tetrahydro-2H-spiro[naphthalene-1,7'-pyrano[4,3-*d*]pyrimidin]-7-yl)carbamate (45 mg, *1 equiv.,* 64 µmol), p-toluenesulfonic acid monohydrate (61 mg, 5 *equiv.,* 0.32 mmol), and MeCN (3 mL). The reaction mixture was heated to 60 °C and stirred for 30 minutes. The volatiles were then removed under reduced pressure. The crude residue was purified using a C-18 Biotage column (12 g cartridge, liquid injection) reverse prep with 10 mM ammonium bicarbonate solution and MeCN to afford (*R**)-1-(7-amino-5-chloro-8-fluoro-2'-(((2*R*,7a*S*)-2-fluorotetrahydro-1*H*-pyrrolizin-7a(5*H*)-yl)methoxy)-3,4,5',8'-tetrahydro-2H-spiro[naphthalene-1,7'-pyrano[4,3-*d*]pyrimidin]-4'-yl)azepane-4-carbonitrile (25 mg) as a white solid.
LCMS: m/z (ESI) [M+H]⁺ 599.4, t_{R} = 4.50, 4.53 minutes (Method I)
¹H NMR (400 MHz, DMSO-*d₆*): δ 6.80 (d, J = 7.63 Hz; 1 H); 5.12-5.28 (m, 3 H); 4.79 (m, 1 H); 4.58 (m, 1 H); 3.92 (m, 1 H); 3.83 (m, 1 H); 3.44-3.60 (m, 4 H); 2.97-3.13 (m, 5 H); 2.74-2.82 (m, 2 H); 2.59 (m, 2 H); 2.05-2.09 (m, 2 H); 1.92-2.01 (m, 4 H); 1.70-1.87 (m, 9 H); 1.60 (m, 1 H).
¹⁹F NMR (376 MHz, DMSO): δ-131.2 - -131.1 (d), -172.0 - -171.9 (m).

### Example 52: (R*)-1-((R)-7-amino-5-chloro-8-fluoro-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)azepane-4-carbonitrile (Compound 510e)

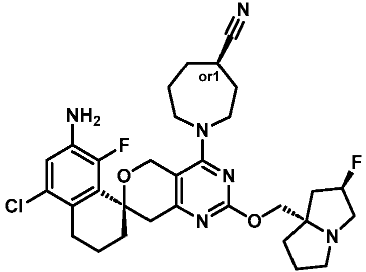

The diastereomers of (*R**)-1-(7-amino-5-chloro-8-fluoro-2'-(((2*R*,7a*S*)-2-fluorotetrahydro-1*H*-pyrrolizin-7a(5*H*)-yl)methoxy)-3,4,5',8'-tetrahydro-2H-spiro[naphthalene-1,7'-pyrano[4,3-*d*]pyrimidin]-4'-yl)azepane-4-carbonitrile were separated by chiral-SFC (Cellulose-1; (30 x 250 mm, 5µm) column, eluting with isocratic 50% (Ethanol + 0.1% DEA) as a co-solvent; Injection volume: 3 mL; Heat Exchanger temperature: 40 °C; BPR: 100 bar; Concentration: 4.4 mg/mL; Total flow: 100 mL/min; Equilibration time: 1 min; Run time: 37.8 minutes) to afford (*R**)-1-((*R*)-7-amino-5-chloro-8-fluoro-2'-(((2*R*,7a*S*)-2-fluorotetrahydro-1*H*-pyrrolizin-7a(5*H*)-yl)methoxy)-3,4,5',8'-tetrahydro-2H-spiro[naphthalene-1,7'-pyrano[4,3-*d*]pyrimidin]-4'-yl)azepane-4-carbonitrile **(Peak 1)** and (*R**)-1-((*S*)-7-amino-5-chloro-8-fluoro-2'-(((2*R*,7a*S*)-2-fluorotetrahydro-1*H*-pyrrolizin-7a(5*H*)-yl)methoxy)-3,4,5',8'-tetrahydro-2H-spiro[naphthalene-1,7'-pyrano[4,3-*d*]pyrimidin]-4'-yl)azepane-4-carbonitrile **(Peak 2).**

Analytical SFC was then used to quantify diastereomeric excess in each case according to the following method: Cellulose-1; (100 x 4.6 mm) column, eluting with isocratic 50% (Ethanol + 0.1% DEA) as a co-solvent; Injection volume: 14 µL; Heat Exchanger temperature: 40 °C; BPR: 100 bar; Total flow: 4 mL/min; Run time: 10 minutes.
**Peak 1:** SFC t_{R}: 2.8 minutes; > 99% de
¹H NMR (400 MHz, DMSO-*d₆*) δ 6.82 (d, 1H), 5.25 (d2H), 4.81 (m, 1H), 4.61 (m, 1H), 3.97 - 3.84 (m, 2H), 3.61 (m, 3H), 3.45 (m, 1H), 3.16 - 2.96 (m, 6H), 2.85 - 2.75 (m, 2H), 2.64 (m, 1H), 2.10 - 1.72 (m, 17H).
¹⁹F NMR (376 MHz, DMSO): δ-131.0 - -131.2 (d), -171.8 - -172.3 (m).

### Example 53: (R*)-1-((S)-7-amino-5-chloro-8-fluoro-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)azepane-4-carbonitrile (Compound 510f)

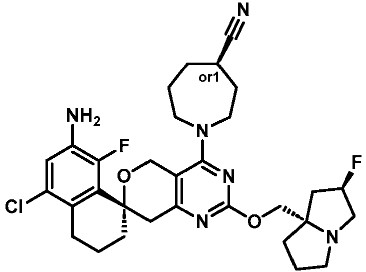

(*R**)-1-((*S*)-7-amino-5-chloro-8-fluoro-2'-(((2*R*,7a*S*)-2-fluorotetrahydro-1*H-*pyrrolizin-7a(5*H*)-yl)methoxy)-3,4,5',8'-tetrahydro-2*H*-spiro[naphthalene-1,7'-pyrano[4,3-*d*]pyrimidin]-4'-yl)azepane-4-carbonitrile was obtained from **Example 52,** as **Peak 2.**
**Peak 2:** SFC t_{R}: 4.2 minutes; 97% de
¹H NMR (400 MHz, DMSO-*d₆*) δ 6.83 (d, 1H), 5.26 (d, 1H), 4.82 (m, 1H), 4.61 (m, 1H), 3.98 - 3.85 (m, 2H), 3.70 - 3.49 (m, 5H), 3.17 - 2.99 (m, 6H), 2.85 - 2.76 (m, 2H), 2.68 - 2.60 (m, 1H), 2.12 - 1.74 (m, 17H).
¹⁹F NMR (376 MHz, DMSO): δ-131.2 - -131.2 (m), -172.3 - -172.1 (m).

### Example 54: 2-((S)-4-((S)-7-amino-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)morpholin-2-yl)acetonitrile (Compound 515a)

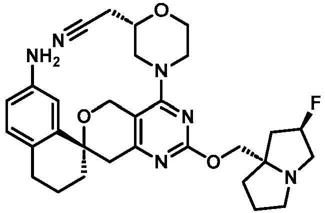

### Step 1: tert-Butyl ((S)-4'-((S)-2-(cyanomethyl)morpholino)-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-7-yl)carbamate

To a solution of tert-butyl ((S)-4'-((1H-benzo[d][1,2,3]triazol-1-yl)oxy)-2'-(((2R,7aS)-2-fluorotetrahydro-1*H*-pyrrolizin-7a(5*H*)-yl)methoxy)-3,4,5',8'-tetrahydro-2H-spiro[naphthalene-1,7'-pyrano[4,3-d]pyrimidin]-7-yl)carbamate (80 mg, 0.12 mmol) in DMF (0.5 mL), were added DIPEA (0.11 mL, 0.61 mmol, 5 *equiv*.) and (*S*)-2-(morpholin-2-yl)acetonitrile (31 mg, 0.24 mmol, 2.0 *equiv*.). The resulting reaction mixture was stirred at 90°C for 5 hours. The reaction mixture was cooled to room temperature and concentrated to get a crude material, which was purified by reverse phase chromatography (column: C18 Biotage 12 g, 10 mL/min, 10 mM ammonium bicarbonate aqueous solution and acetonitrile, 0 → 100%) to afford *tert-*butyl ((*S*)-4'-((*S*)-2-(cyanomethyl)morpholino)-2'-(((2*R*,7a*S*)-2-fluorotetrahydro-1*H*-pyrrolizin-7a(5*H*)-yl)methoxy)-3,4,5',8'-tetrahydro-2*H-*spiro[naphthalene-1,7'-pyrano[4,3-*d*]pyrimidin]-7-yl)carbamate (35 mg) as an off-white solid.
LCMS: m/z (ESI) [M+H]⁺ 649.4, t_{R} = 1.73 minutes (Method B)
¹H NMR (400 MHz, DMSO-*d₆*) δ 9.17 (br s, 1H), 7.46 (br s, 1H), 7.25 (br d, 1H), 6.99 (d, 1H), 5.26 (d, 1H), 4.66 (d, 1H), 4.42 (d, 1H), 3.99 (d, 1H), 3.94 - 3.75 (m, 4H), 3.66 - 3.51 (m, 2H), 3.13 - 2.97 (m, 4H), 2.95 - 2.65 (m, 8H), 2.14 - 2.04 (m, 1H), 2.03 - 1.66 (m, 9H), 1.42 (s, 9H).
¹⁹F NMR (376 MHz, DMSO) δ -172.08.

### Step 2: 2-((S)-4-((S)-7-amino-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)morpholin-2-yl)acetonitrile

To a solution of *tert*-butyl ((*S*)-4'-((*S*)-2-(cyanomethyl)morpholino)-2'-(((2*R,*7a*S*)-2-fluorotetrahydro-1*H*-pyrrolizin-7a(5*H*)-yl)methoxy)-3,4,5',8'-tetrahydro-2H-spiro[naphthalene-1,7'-pyrano[4,3-*d*]pyrimidin]-7-yl)carbamate (30 mg, 46 µmol) in MeCN (2.0 mL) was added *p*-toluenesulfonic acid monohydrate (35 mg, 0.18 mmol, 4.0 *equiv*.)*.* The reaction mixture was stirred at 50 °C for 1 hour. The reaction mixture was cooled to room temperature and concentrated to give crude material, which was purified by reverse phase chromatography (column: C18 Biotage 12 g, 10 mL/min, 10 mM ammonium bicarbonate aqueous solution and acetonitrile, 0 → 100%) to afford 2-((*S*)-4-((*S*)-7-amino-2'-(((2*R*,7a*S*)-2-fluorotetrahydro-1*H*-pyrrolizin-7a(5*H*)-yl)methoxy)-3,4,5',8'-tetrahydro-2*H-*spiro[naphthalene-1,7'-pyrano[4,3-*d*]pyrimidin]-4'-yl)morpholin-2-yl)acetonitrile (16 mg) as an off-white solid.
LCMS: m/z (ESI) [M+H]⁺ 549.4, t_{R} = 1.42 minutes (Method B)
¹H NMR (400 MHz, DMSO-*d₆*) δ 6.76 (d, 1H), 6.54 (d, 1H), 6.44 (dd, 1H), 5.26 (br d, 1H), 4.79 (br s, 2H), 4.65 (d, 1H), 4.40 (d, 1H), 3.98 (d, 1H), 3.94 - 3.76 (m, 4H), 3.67 - 3.57 (m, 1H), 3.54 (br d, 1H), 3.13 - 2.97 (m, 4H), 2.97 - 2.86 (m, 2H), 2.86 - 2.73 (m, 4H), 2.68 - 2.54 (m, 2H), 2.14 - 1.58 (m, 10H).
¹⁹F NMR (376 MHz, DMSO) δ -172.07.

### Example 55: (S)-7-Amino-4'-((S)-3-(cyanomethyl)-1,4-oxazepan-4-yl)-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidine]-8-carbonitrile (Compound 522a)

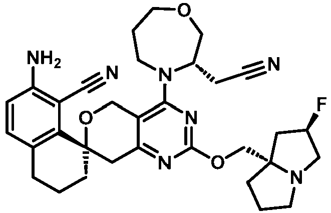

### Step 1: tert-butyl (S)-3-(hydroxymethyl)-1,4-oxazepane-4-carboxylate

Borane tetrahydrofuran complex (1 M, 5.3 mL, 5.3 mmol) was added dropwise to a stirred solution of (*R*)-4-(*tert*-butoxycarbonyl)-1,4-oxazepane-3-carboxylic acid (997 mg, 4.06 mmol) in THF (8.1 mL). The resulting mixture was stirred at room temperature for 2 days. The reaction mixture was then quenched by the addition of water (10 mL) at 0 °C and treated with EtOAc (10 mL). The two phases were separated, and the aqueous layer was extracted with EtOAc (3 x 10 mL). The combined organic extracts were dried over sodium sulfate, filtered, and concentrated to afford a clear oil, which was purified via flash chromatography (0-80% EtOAc/Heptanes) to afford *tert-*butyl (*S*)-3-(hydroxymethyl)-1,4-oxazepane-4-carboxylate (922.3 mg) as a clear oil.

¹H NMR (400 MHz, CDCl₃) δ 4.37 - 4.15 (m, 1H), 4.02 (dd, 1H), 3.98 - 3.90 (m, 1H), 3.85 - 3.76 (m, 1H), 3.71 - 3.55 (m, 2H), 3.55 - 3.41 (m, 2H), 3.28 - 3.11 (m, 1H), 2.93 - 2.77 (m, 1H), 1.96 - 1.74 (m, 1H), 1.74 - 1.64 (m, 1H), 1.54 - 1.43 (m, 9H).

### Step 2: tert-Butyl (S)-3-(cyanomethyl)-1,4-oxazepane-4-carboxylate

To a solution of *tert-*butyl (*S*)-3-(hydroxymethyl)-1,4-oxazepane-4-carboxylate (922 mg, 3.99 mmol) and TEA (0.87 g, 1.2 mL, 8.6 mmol) in DCM (8 mL) was added MsCl (450 µL, 5.78 mmol) at 0 °C. The mixture was stirred at 0 °C for 1 hour. The reaction mixture was added to water (5 mL) at 0 °C. The mixture was extracted with DCM (3 × 10 mL). The combined organic extracts were dried over sodium sulfate, filtered, and concentrated to afford *tert-*butyl (*R*)-3-(((methylsulfonyl)oxy)methyl)-1,4-oxazepane-4-carboxylate as a light brown oil.

A mixture of *tert*-butyl (*R*)-3-(((methylsulfonyl)oxy)methyl)-1,4-oxazepane-4-carboxylate (254.5 mg, 822.6 µmol) and sodium cyanide (161 mg, 3.29 mmol) in DMSO (4 mL) was heated at 50 °C overnight. The reaction mixture was treated with saturated aqueous sodium bicarbonate (100 mL) at room temperature. The mixture was extracted with Et₂O (3 × 20 mL). The combined organic extracts were washed with 5% LiCl aqueous solution (3 x 20 mL), dried over sodium sulfate, filtered, and concentrated to afford a yellow oil, which was purified via flash chromatography (0-40% EtOAc/Heptanes) to afford *tert-*butyl (*S*)-3-(cyanomethyl)-1,4-oxazepane-4-carboxylate (30.9 mg).
¹H NMR (400 MHz, CDCl₃) δ 4.50 - 4.30 (m, 1H), 4.09 - 3.83 (m, 3H), 3.73 - 3.59 (m, 1H), 3.59 - 3.47 (m, 1H), 3.36 - 3.18 (m, 1H), 2.76 (dd, 0.5H), 2.59 (dd, 0.5H), 2.51 - 2.39 (m, 1H), 1.93 - 1.76 (m, 1H), 1.75 - 1.64 (m, 1H), 1.54 - 1.39 (m, 9H).
¹³C NMR (101 MHz, CDCl₃) δ 155.37, 154.82, 117.56, 117.24, 81.08, 80.66, 72.83, 72.00, 53.48, 52.77, 42.42, 41.46, 31.75, 31.39, 28.46, 19.41, 18.82.
FTIR (neat) cm-1: 2969 (m), 2951 (m), 2359 (w), 2340 (w), 1737 (s), 1682 (s).

### Step 3: (S)-2-(1,4-Oxazepan-3-yl)acetonitrile hydrochloride

A mixture of *tert-*butyl (*S*)-3-(cyanomethyl)-1,4-oxazepane-4-carboxylate (90.9 mg, 378 µmol) in DCM (4 mL) was treated with 2M HCl in Et₂O (1 mL) at 0 °C then warmed to room temperature. The resulting mixture was stirred for 60 minutes. The mixture was concentrated to afford an orange oil. The crude was treated with DCM, at which point a precipitate formed and was filtered to afford (*S*)-2-(1,4-oxazepan-3-yl)acetonitrile hydrochloride (30 mg) as an off-white precipitate.

¹H NMR (400 MHz, MeOD-*d*₄) δ 4.07 - 3.98 (m, 1H), 3.93 - 3.74 (m, 4H), 3.50 (ddd, 1H), 3.39 (ddd, 1H), 3.09 - 3.01 (m, 1H), 3.01 - 2.89 (m, 1H), 2.22 - 2.02 (m, 2H).

¹³C NMR (101 MHz, MeOD-*d*₄) δ 116.7, 70.6, 69.2, 56.4, 45.2, 28.6, 19.1.

### Step 4: (S)-7-Amino-4'-((S)-3-(cyanomethyl)-1,4-oxazepan-4-yl)-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidine]-8-carbonitrile

(*S*)-2-(1,4-Oxazepan-3-yl)acetonitrile hydrochloride (40.7 mg, 230 µmol) and PyBOP (28 mg, 54 µmol) were added to a solution of (*S*)-7-Amino-2'-(((2*R*,7a*S*)-2-fluorotetrahydro-1H-pyrrolizin-7a(5*H*)-yl)methoxy)-4'-hydroxy-3,4,5',8'-tetrahydro-2*H*-spiro[naphthalene-1,7'-pyrano[4,3-*d]*pyrimidine]-8-carbonitrile (20 mg, 43 µmol) in DMF (0.86 mL) at room temperature. The mixture was treated with DIPEA (60 µL, 0.34 mmol) then heated at 80 °C and stirred for 1 hour. The mixture was cooled to 60 °C and stirred overnight. Afterwards, the mixture was treated with 5% LiCl solution (10 mL) then extracted with EtOAc (3 x 5 mL). The combined organic layer was washed with brine (10 mL), dried over sodium sulfate, filtered, and concentrated to afford a dark brown oil, which was purified via flash chromatography [0-20% (2.5% NH4OH, 20% MeOH in DCM)/DCM]. The relevant fractions were pooled together and re-subjected to purification via reverse phase chromatography [ACCQ Prep; 5-55% (NH₄OH in MeCN)/(NH₄OH in water)] to afford (S)-7-amino-4'-((*S*)-3-(cyanomethyl)-1,4-oxazepan-4-yl)-2'-(((2*R*,7a*S*)-2-fluorotetrahydro-1*H*-pyrrolizin-7a(5*H*)-yl)methoxy)-3,4,5',8'-tetrahydro-2*H*-spiro[naphthalene-1,7'-pyrano[4,3-*d*]pyrimidine]-8-carbonitrile (1.3 mg) as an off-white solid.
¹H NMR (400 MHz, MeOD-*d*₄) δ 6.97 (d, 1H), 6.64 (d, 1H), 5.28 - 5.09 (m, 1H), 4.99 (d, 1H), 4.73 - 4.68 (m, 1H), 4.08 (d, 1H), 4.00 (d, 1H), 3.94 (dd, 1H), 3.87 - 3.76 (m, 2H), 3.70 (ddd, 1H), 3.67 - 3.58 (m, 1H), 3.55 - 3.42 (m, 1H), 3.19 - 3.05 (m, 4H), 2.93 - 2.78 (m, 4H), 2.62 - 2.53 (m, 2H), 2.26 - 1.62 (m, 13H).
¹⁹F NMR (376 MHz, MeOD) δ -173.53.
LCMS: m/z (ESI) [M+H]⁺ 588.3, t_{R} = 1.25 minutes (Method C)

### Example 56: (S*)-1-((R)-7-amino-5-chloro-8-fluoro-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)azepane-4-carbonitrile (Compound 510c)

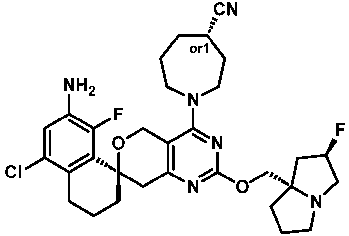

The diastereomers of (4*S**)-1-(7-amino-5-chloro-8-fluoro-2'-(((2*R*,7a*S*)-2-fluorotetrahydro-1*H*-pyrrolizin-7a(5*H*)-yl)methoxy)-3,4,5',8'-tetrahydro-2*H-*spiro[naphthalene-1,7'-pyrano[4,3*-d*]pyrimidin]-4'-yl)azepane-4-carbonitrile were separated by chiral-SFC (Cellulose; (3-30 mm) column, eluting with isocratic 30% (MeOH + 0.1% DEA) as a co-solvent; Injection volume: 2 mL; Heat Exchanger temperature: 40 °C; BPR: 100 bar; Total flow: 100 mL/min; Equilibration time: 1 min; Run time: 37.8 minutes) to afford (*S**)-1-((*R*)-7-amino-5-chloro-8-fluoro-2'-(((2*R*,7a*S*)-2-fluorotetrahydro-1*H*-pyrrolizin-7a(5*H*)-yl)methoxy)-3,4,5',8'-tetrahydro-2*H*-spiro[naphthalene-1,7'-pyrano[4,3-*d*]pyrimidin]-4'-yl)azepane-4-carbonitrile **(Peak 1)** and (*S**)-1-((*S*)-7-amino-5-chloro-8-fluoro-2'-(((2*R*,7a*S*)-2-fluorotetrahydro-1*H*-pyrrolizin-7a(5*H*)-yl)methoxy)-3,4,5',8'-tetrahydro-2*H-*spiro[naphthalene-1,7'-pyrano[4,3-*d*]pyrimidin]-4'-yl)azepane-4-carbonitrile **(Peak 2).**

Analytical chiral HPLC was then used to quantify diastereomeric excess in each case according to the following method: ChiralPak IB; (4.6 x 250mm, 5 µm) column, eluting with isocratic 50:50 hexanes/EtOH (0.1% DEA) as the eluent; column temperature: 40 °C; Total flow: 1.0 mL/min; Run time: 20 minutes.
**Peak 1:** SFC: 10.9 min, 61% de
¹H NMR (400 MHz, DMSO-*d₆*): δ 6.80 (d, 1 H), 5.08-5.26 (m, 3 H), 4.79 (m, 1 H), 4.58 (m, 1 H), 3.91 (m, 1 H), 3.82 (m, 1 H), 3.41-3.60 (m, 4 H), 2.96-3.12 (m, 5 H), 2.72-2.79 (m, 2 H), 2.59-2.63 (m, 1 H), 1.71-2.09 (m, 16 H), 1.59-1.64 (m, 1 H).
¹⁹F NMR (376 MHz, DMSO): δ-131.1 - -131.2 (d), -172.1 - -172.3 (m).

### Example 57: (S*)-1-((S)-7-amino-5-chloro-8-fluoro-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)azepane-4-carbonitrile (Compound 510d)

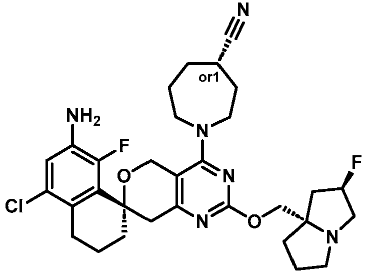

(*S**)-1-((*S*)-7-amino-5-chloro-8-fluoro-2'-(((2*R*,7a*S*)-2-fluorotetrahydro-1*H-*pyrrolizin-7a(5*H*)-yl)methoxy)-3,4,5',8'-tetrahydro-2*H*-spiro[naphthalene-1,7'-pyrano[4,3-*d*]pyrimidin]-4'-yl)azepane-4-carbonitrile was obtained from **Example 56,** as **Peak 2.**
**Peak 2:** SFC: 16.0 min, > 99% de
¹H NMR (400 MHz, DMSO-*d₆*): δ 6.80 (d, 1 H), 5.09-5.27 (m, 3 H), 4.79 (m, 1 H), 4.58 (m, 1 H), 3.92 (m, 1 H), 3.83 (m, 1 H), 3.45-3.60 (m, 4 H), 3.26 (s, 1 H), 2.97-3.13 (m, 5 H), 2.78 (m, 2 H), 2.57-2.63 (m, 1 H), 2.05 (s, 2 H), 1.92 - 1.97 (m, 4 H), 1.71 - 1.84 (m, 9 H), 1.59 -1.64 (m, 1 H).
¹⁹F NMR (376 MHz, DMSO): δ-131.2 - -131.2 (d), -172.3 - -172.1 (m).

### Example 58: 5-(7-Amino-8-cyano-2'-(((2S,4R)-4-fluoro-1-methylpyrrolidin-2-yl)methoxy)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide (Compound 523a)

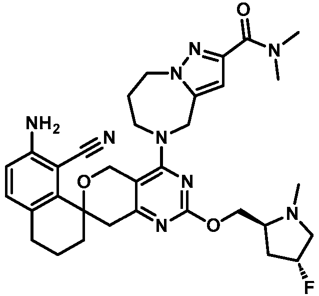

### Step 1: 5-(8-cyano-7-(dibenzylamino)-2'-(((2S,4R)-4-fluoro-1-methylpyrrolidin-2-yl)methoxy)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide

A LiHMDS solution (1 M in THF, 280 µL, 1.8 *equiv.,* 280 µmol) was added to a solution of ((2*S*,4*R*)-4-fluoro-1-methylpyrrolidin-2-yl)methanol (80 mg, 3.8 *equiv., 0.60* mmol) in anhydrous THF (2.38 mL). To this mixture was added a solution of 5-(8-cyano-7-(dibenzylamino)-2'-(methylsulfonyl)-3,4,5',8'-tetrahydro-2*H*-spiro[naphthalene-1,7'-pyrano[4,3-*d*]pyrimidin]-4'-yl)-*N,N*-dimethyl-5,6,7,8-tetrahydro-4*H*-pyrazolo[1,5-*a*][1,4]diazepine-2-carboxamide (140 mg, 85 wt%, *1 equiv.,* 157 µmol) in anhydrous THF (2.38 mL) over 1 minute at 0°C. The mixture was warmed to ambient temperature. After 2 hours of stirring, the reaction was heated at 60°C for 30 minutes. The contents were then cooled to ambient temperature and water (1 mL) was added. The volatiles were then removed under reduced pressure. The contents were then treated with a 1:1 mixture of saturated aqueous ammonium chloride solution/water (10 mL) at 0 °C. EtOAc (15 mL) was then added, and the layers were separated and the organic layers were combined, dried, and concentrated to yield a residue, which was purified by flash column chromatography (reverse phase C₁₈, 13g, liquid loading, 0-100% MeCN in water) to afford 5-(8-cyano-7-(dibenzylamino)-2'-(((2*S*,4*R*)-4-fluoro-1-methylpyrrolidin-2-yl)methoxy)-3,4,5',8'-tetrahydro-2*H*-spiro[naphthalene-1,7'-pyrano[4,3-*d*]pyrimidin]-4'-yl)-*N*,*N*-dimethyl-5,6,7,8-tetrahydro-4*H*-pyrazolo[1,5-*a*][1,4]diazepine-2-carboxamide (72 mg).
LCMS: m/z (ESI) [M+H]⁺ 810.5; t_{R} = 1.39 minutes (Method A)

### Step 2: 5-(7-amino-8-cyano-2'-(((2S,4R)-4-fluoro-1-methylpyrrolidin-2-yl)methoxy)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide

To a 5 mL flame dried flask, charged with a stirring solution of 5-(8-cyano-7-(dibenzylamino)-2'-(((2*S*,4*R*)-4-fluoro-1-methylpyrrolidin-2-yl)methoxy)-3,4,5',8'-tetrahydro-2*H*-spiro[naphthalene-1,7'-pyrano[4,3-*d*]pyrimidin]-4'-yl)-*N*,*N*-dimethyl-5,6,7,8-tetrahydro-4*H*-pyrazolo[1,5-*a*][1,4]diazepine-2-carboxamide (72 mg, 95 wt%, 1 *equiv.,* 84 µmol) in MeOH (1.7 mL), Pd(OH)₂ on carbon (0.15 g, 20 wt%, 2.5 *equiv.,* 0.21 mmol) was added. The atmosphere was exchanged with hydrogen by degassing the reaction mixture under reduced pressure with a backflow of hydrogen from a balloon. Three drops of acetic acid were added to the reaction mixture. The mixture was then stirred for 18 hours under a hydrogen atmosphere at ambient temperature. The suspension was then filtered over a polytetrafluoroethylene filter and the residue was further washed with EtOAc, DCM, and MeOH. The filtrate was concentrated under reduced pressure, which was purified by reverse phase column chromatography (12g C₁₈ column, eluting 0-100%, aqueous ammonium bicarbonate (10 mM) to MeCN and then flushing with 1/1 MeCN/MeOH). The product was further purified via preparative HPLC (Mobile phase A: 10 mM Ammonium Bicarbonate pH = 10.0 ; B: MeOH; Flow rate: 45mL/min; Column: Gemini^{®} 5 µm NX-C18 110 Å, 100 x 30 mm; Pre-run: 1.5 min at initial condition (60% A, 40% B)) to afford 5-(7-amino-8-cyano-2'-(((2*S*,4*R*)-4-fluoro-1-methylpyrrolidin-2-yl)methoxy)-3,4,5',8'-tetrahydro-2*H-*spiro[naphthalene-1,7'-pyrano[4,3-*d*]pyrimidin]-4'-yl)-*N,N*-dimethyl-5,6,7,8-tetrahydro-4*H-*pyrazolo[1,5-*a*][1,4]diazepine-2-carboxamide (15 mg).
LCMS: m/z (ESI) [M+H]⁺ 630.5; t_{R} = 3.42 minutes (Method I)
¹H NMR (400 MHz, DMSO-*d₆*): 7.03 (d, 1H), 6.71 (d, 1H), 6.53 (m, 1H), 5.72 (m, 2H), 5.07-5.24 (m, 1H), 4.94-4.99 (m, 1H), 4.69-4.71 (m, 2H), 4.60 (m, 1H), 4.42-4.45 (m, 2H), 4.09-4.24 (m, 2H), 3.81-3.93 (m, 2H), 3.21-3.24 (m, 3H), 3.06-3.11 (m, 1H), 2.91 (m, 3H), 2.81-2.86 (m, 1H), 2.65 (s, 1H), 2.57-2.61 (m, 2H), 2.30-2.34 (m, 4H), 1.65-2.15 (m, 9H).

**Example 59: 7-Amino-2'-(((*S*)-1-methylpyrrolidin-2-yl)methoxy)-4'-(1,4-oxazepan-4-yl)-3,4,5',8'-tetrahydro-2*H*-spiro[naphthalene-1,7'-pyrano[4,3-*d*]pyrimidine]-8-carbonitrile (Compound 524a)**

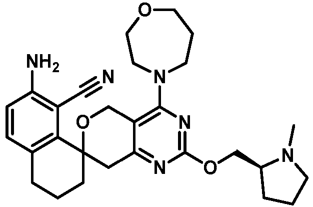

### Step 1: 7-(dibenzylamino)-2'-(((S)-1-methylpyrrolidin-2-yl)methoxy)-4'-(1,4-oxazepan-4-yl)-3,4,5',8'-tetrahydro-2H-spiro[naphthalene-1,7'-pyrano[4,3-d]pyrimidine]-8-carbonitrile

A LiHMDS solution (1.0 M in THF, 285 µL, 1.8 *equiv.,* 285 µmol) was added to a solution of (*S*)-(1-methylpyrrolidin-2-yl)methanol (91.0 mg, 5 *equiv.,* 790 µmol) in anhydrous THF (2.40 mL). A solution of 7-(dibenzylamino)-2'-(methylsulfonyl)-4'-(1,4-oxazepan-4-yl)-3,4,5',8'-tetrahydro-2*H*-spiro[naphthalene-1,7'-pyrano[4,3-*d*]pyrimidine]-8-carbonitrile (103 mg, *1 equiv.,* 159 µmol) in anhydrous THF (2.40 mL) was then added over 1 minute at 0°C. The mixture was warmed to ambient temperature and stirred for 4 hours. The reaction was then quenched by the slow addition of a 1:1 mixture of saturated aqueous ammonium chloride solution and water at 0 °C. EtOAc (2 mL) was then added, and the layers were separated and the organic layers were further extracted with EtOAc (3 x 20 mL). The combined organic phases were dried over sodium sulfate, filtered, and concentrated under reduced pressure to afford a crude residue, which was purified by normal phase chromatography (silica, 12 g RediSep Gold cartridge, solid loading, gradient of MeOH/NH₄OH/DCM (13.5/1.5/85) in DCM, 0% to 100%). Fractions containing the desired product were combined and concentrated under reduced pressure to give a crude residue which was repurified by flash column chromatography (reverse phase, 6g C₁₈, liquid loading, using MeOH in water, 5-100%, 10CV) to give 7-(dibenzylamino)-2'-(((*S*)-1-methylpyrrolidin-2-yl)methoxy)-4'-(1,4-oxazepan-4-yl)-3,4,5',8'-tetrahydro-2*H*-spiro[naphthalene-1,7'-pyrano[4,3-*d*]pyrimidine]-8-carbonitrile (43 mg).
LCMS: m/z (ESI) [M+H]⁺ 685.3; t_{R} = 1.35 minutes (Method A)

### Step 2: 7-amino-2'-(((S)-1-methylpyrrolidin-2-yl)methoxy)-4'-(1,4-oxazepan-4-yl)-3,4,5',8'-tetrahydro-2H-spiro[naphthalene-1,7'-pyrano[4,3-d]pyrimidine]-8-carbonitrile

To a 5 mL flame dried flask, charged with a stirring solution of 7-(dibenzylamino)-2'-(((*S*)-1-methylpyrrolidin-2-yl)methoxy)-4'-(1,4-oxazepan-4-yl)-3,4,5'8'-tetrahydro-2*H-*spiro[naphthalene-1,7'-pyrano[4,3-*d*]pyrimidine]-8-carbonitrile (43 mg, 1 *equiv.,* 63 µmol) in MeOH (1.5 mL), Pd(OH)₂ on carbon (0.11 g, 20 wt%, 2.5 *equiv.,* 0.16 mmol) and acetic acid (10 µL, 2.8 *equiv.,* 0.17 mmol) were added. The atmosphere was exchanged with hydrogen by degassing the reaction mixture with a backflow of hydrogen from a balloon. The reaction mixture was stirred for 16 hours under hydrogen atmosphere at ambient temperature. The suspension was then filtered over celite with MeOH (5 mL), and the filtrate was stirred with sodium carbonate (200 mg) for 5 minutes. The contents were filtered, and concentrated under reduced pressure. The crude residue was purified by reverse phase chromatography (C₁₈ 6 g RediSep Gold C18 cartridge, liquid loading in DMSO:H₂O 9:1 (1 ml), gradient of MeOH in aqueous 10 mM ammonium bicarbonate buffer, 5-100%, 10CV). Fractions containing the desired product were combined, and concentrated under reduced pressure to give a solid which was purified by reverse-phase accqPrep chromatography (MeOH in aqueous 10 mM ammonium bicarbonate buffer, 5-100% 10CV) to give 7-amino-2'-(((*S*)-1-methylpyrrolidin-2-yl)methoxy)-4'-(1,4-oxazepan-4-yl)-3,4,5',8'-tetrahydro-2*H-*spiro[naphthalene-1,7'-pyrano[4,3-*d*]pyrimidine]-8-carbonitrile (10 mg).
LCMS: m/z (ESI) [M+H]⁺ 505.5; t_{R} =1.38 minutes (Method B)
¹H NMR (CH₃OH-*d*₄, 400 MHz): δ 7.07 (m, 1H), 6.74 (m, 1H), 5.00 (m, 1H), 4.37-4.24 (m, 2H), 3.87-3.79 (m, 7H), 3.74-3.69 (m, 3H), 3.24 (m, 1H), 3.10-3.04 (m, 1H), 2.91 (m, 1H), 2.80-2.71 (m, 1H), 2.70-2.65 (m, 2H), 2.50 (m, 3H), 2.38-2.31 (m, 1H), 2.29-2.21 (m, 1H), 2.11-2.04 (m, 2H), 1.98-1.90 (m, 2H), 1.85-1.78 (m, 3H), 1.72-.1.62 (1H, m).

### Example 60: 7-Amino-2'-(((R)-1-methylpyrrolidin-3-yl)methoxy)-4'-(1,4-oxazepan-4-yl)-3,4,5',8'-tetrahydro-2H-spiro[naphthalene-1,7'-pyrano[4,3-d]pyrimidine]-8-carbonitrile (Compound 525a)

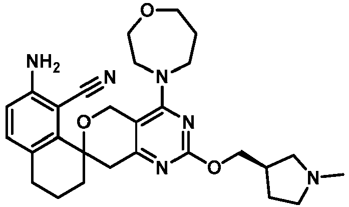

### Step 1: 7-(dibenzylamino)-2'-(((R)-1-methylpyrrolidin-3-yl)methoxy)-4'-(1,4-oxazepan-4-yl)-3,4,5',8'-tetrahydro-2H-spiro[naphthalene-1,7'-pyrano[4,3-d]pyrimidine]-8-carbonitrile

A LiHMDS solution (1.0 M in THF, 280 µL, 1.8 *equiv.,* 280 µmol) was added to a solution of (*R*)-(1-methylpyrrolidin-3-yl)methanol (89.0 mg, 5 *equiv.,* 770 µmol) in anhydrous THF (2 mL) at 0 °C. To this mixture, a solution of 7-(dibenzylamino)-2'-(methylsulfonyl)-4'-(1,4-oxazepan-4-yl)-3,4,5',8'-tetrahydro-2*H*-spiro[naphthalene-1,7'-pyrano[4,3-*d*]pyrimidine]-8-carbonitrile (100 mg, 1 *equiv.,* 154 µmol) in anhydrous THF (2 mL) was added dropwise over 1 minute at 0 °C. The mixture was warmed to room temperature over 1 hour. The resulting reaction was stirred at 60 °C for 1 hour. Water (1 mL) was then added, and the volatiles were removed under reduced pressure. The reaction was quenched by the slow addition of a 1:1 mixture of saturated aqueous ammonium chloride solution and water at 0 °C. EtOAc was added, and the layers were separated and the organic layer was dried and concentrated *in vacuo,* and the resulting residue was purified by flash column chromatography (reverse phase, 12g C₁₈, liquid loading, MeCN in water, 5-100%, 10 CV) to give 7-(dibenzylamino)-2'-(((*R*)-1-methylpyrrolidin-3-yl)methoxy)-4'-(1,4-oxazepan-4-yl)-3,4,5',8'-tetrahydro-2*H*-spiro[naphthalene-1,7'-pyrano[4,3-*d*]pyrimidine]-8-carbonitrile (108 mg).
LCMS: m/z (ESI) [M+H]⁺ 685.4; t_{R} = 1.31 minutes (Method A)

### Step 2: 7-amino-2'-(((R)-1-methylpyrrolidin-3-yl)methoxy)-4'-(1,4-oxazepan-4-yl)-3,4,5',8'-tetrahydro-2H-spiro[naphthalene-1,7'-pyrano[4,3-d]pyrimidine]-8-carbonitrile

To a 10 mL round bottom flask charged with a stirring solution of 7-(dibenzylamino)-2'-(((*R*)-1-methylpyrrolidin-3-yl)methoxy)-4'-(1,4-oxazepan-4-yl)-3,4,5',8'-tetrahydro-2*H-*spiro[naphthalene-1,7'-pyrano[4,3-*d*]pyrimidine]-8-carbonitrile (108 mg, 1 *equiv.,* 158 µmol) in MeOH (3 mL)was added Pd(OH)₂ on carbon (277 mg, 20 wt%, 2.5 *equiv.,* 394 µmol). The atmosphere was exchanged with hydrogen from a balloon. Three drops of acetic acid were then added to the reaction mixture and the resulting mixture was stirred for 18 hours under a hydrogen atmosphere at ambient temperature. The suspension was filtered over celite with MeOH (5 mL), and the filtrate was stirred with sodium carbonate (500 mg) for 5 minutes. The mixture was filtered, and concentrated under reduced pressure. The crude residue was then purified by reverse phase chromatography (C₁₈ 6 g RediSep Gold cartridge, liquid loading in DMSO:H₂O 9:1 (1 ml), gradient of MeOH in aqueous 10 mM ammonium bicarbonate buffer, 5-100%, 10CV). Fractions containing the desired product were combined, and concentrated under reduced pressure to give a residue, which was re-purified by accqPrep (C₁₈) using a gradient of MeOH in aqueous 10 mM ammonium bicarbonate buffer, 5-100% 10CV). The solid was re-purified by Prep HPLC (Mobile phase A: 10mM Ammonium Bicarbonate pH = 10.0; B: MeOH; Flow rate: 45mL/min; Column: Gemini^{®} 5 µm NX-C18 110 Å, 100 x 30 mm; Pre-run: 1.5 min at initial condition (55% A, 45% B); Gradient (45-100% B in A)) to afford 7-amino-2'-(((*R*)-1-methylpyrrolidin-3-yl)methoxy)-4'-(1,4-oxazepan-4-yl)-3,4,5',8'-tetrahydro-2H-spiro[naphthalene-1,7'-pyrano[4,3-*d*]pyrimidine]-8-carbonitrile (7 mg).
LCMS: m/z (ESI) [M+H]⁺ 505.3; t_{R} = 1.34 min. (Method B)
¹H NMR (400 MHz, DMSO-*d₆*): δ 7.04 (m, 1H), 6.72 (m, 1H), 5.73 (s, 2H), 4.88 (m, 1H), 4.66 (m, 1H), 4.08-4.04 (m, 2H), 3.77-3.67 (m, 6H), 3.62-3.54 (m, 3H), 3.09 (m, 1H), 2.84 (m, 1H), 2.62-2.55 (m, 1H), 2.41-2.30 (m, 3H), 2.23 (s, 3H), 2.19-2.16 (m, 2H), 2.01-1.81 (m, 5H), 1.71-1.65 (m, 2H), 1.49-1.43 (1H, m).

### Example 61: (S)-4'-((S)-6-(1H-pyrazol-1-yl)-1,4-oxazepan-4-yl)-7-amino-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidine]-8-carbonitrile (Compound 516c)

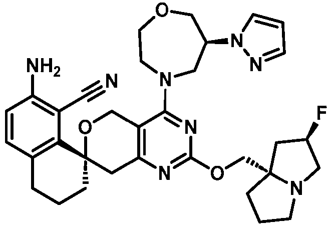

### Step 1: (S)-6-(1H-pyrazol-1-yl)-1,4-oxazepane

Methanesulfonyl chloride (343 mg, 232 µL, 1.3 *equiv.,* 2.99 mmol) was added dropwise to a stirring solution of *tert*-butyl (*R*)-6-hydroxy-1,4-oxazepane-4-carboxylate (500 mg, 1 *equiv.,* 2.30 mmol) and TEA (699 mg, 962 µL, 3 *equiv.,* 6.90 mmol) in DCM (7.67 mL) at 0 °C. The ice bath was removed, and the resulting mixture was stirred at room temperature for 1 hour. The reaction mixture was diluted with water and DCM. The two phases were separated and the aqueous layer was extracted with DCM (x 3). The combined organic extracts were dried over sodium sulfate, filtered, and concentrated to give *tert-*butyl (*R*)-6-((methylsulfonyl)oxy)-1,4-oxazepane-4-carboxylate (676 mg), which was used in the next step without further purification.

*tert*-Butyl (*R*)-6-((methylsulfonyl)oxy)-1,4-oxazepane-4-carboxylate (676 mg, 1 *equiv.,* 2.29 mmol) was dissolved in DMF (11.4 mL). 1*H*-pyrazole (203 mg, 1.3 *equiv.,* 2.98 mmol) and NaH (183 mg, 60 wt%, 2 *equiv.,* 4.58 mmol) were added and the resulting reaction mixture was heated to 80 °C and stirred for 4 hours. The reaction mixture was cooled to room temperature and diluted with water and EtOAc. The two phases were separated and the aqueous layer was extracted with EtOAc (x 3). The combined organic extracts were dried over sodium sulfate, filtered, and concentrated to give the crude product which was purified via silica gel chromatography (gradient elution from 0:100-EtOAc:DCM to 40:60-EtOAc:DCM) to give *tert*-butyl (*S*)-6-(1*H*-pyrazol-1-yl)-1,4-oxazepane-4-carboxylate (300 mg) as a clear colorless oil.

HCl (758 mg, 5.20 mL, 4.0 M, 20 *equiv.,* 20.8 mmol) in dioxane was added to a stirred solution of *tert*-butyl (*S*)-6-(1*H*-pyrazol-1-yl)-1,4-oxazepane-4-carboxylate (278 mg, *1 equiv.,* 1.04 mmol) in DCM (5.20 mL). The resulting mixture was stirred at room temperature for 2 hours. The reaction mixture was concentrated to dryness to give the crude product (*S*)-6-(1*H-*pyrazol-1-yl)-1,4-oxazepane (200 mg), which was used in the next step without further purification.
LCMS: m/z (ESI) [M+H]⁺ 168.2, t_{R} = 0.26 minutes (Method C)
¹H NMR (400 MHz, MeOD) δ 7.78 (d, 1H), 7.63 (d, 1H), 6.38 (m, 1H), 5.07 - 4.97 (m, 1H), 4.24 (m, 1H), 4.10 - 3.98 (m, 3H), 3.86 (m, 1H), 3.79 - 3.72 (m, 1H), 3.60 - 3.41 (m, 2H).

### Step 2: (S)-4'-((S)-6-(1H-pyrazol-1-yl)-1,4-oxazepan-4-yl)-7-amino-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidine]-8-carbonitrile

DIPEA (40 mg, 54 µL, 6 *equiv.,* 0.31 mmol) was added to a stirred solution of (*S*)-7-amino-2'-(((2*R*,7a*S*)-2-fluorotetrahydro-1*H*-pyrrolizin-7a(5*H*)-yl)methoxy)-4'-hydroxy-3,4,5',8'-tetrahydro-2*H*-spiro[naphthalene-1,7'-pyrano[4,3*-d*]pyrimidine]-8-carbonitrile (24 mg, 1 *equiv.,* 52 µmol) and PyBOP (28.6 mg, 1.1 *equiv.,* 55.0 µmol) in MeCN (0.26 mL). The resulting mixture was heated to 70 °C for 15 minutes. The reaction mixture was then cooled to approximately 50 °C followed by the addition of (*S*)-6-(1*H*-pyrazol-1-yl)-1,4-oxazepane (26 mg, 3 *equiv.,* 0.15 mmol) in one portion. The reaction mixture was stirred at 70 °C for 4 hours. The reaction mixture was then cooled to room temperature and diluted with a saturated aqueous solution of sodium bicarbonate and EtOAc. The two phases were separated and the aqueous layer was extracted with EtOAc (x 3). The combined organic extracts were dried over sodium sulfate, filtered, and concentrated. The resulting residue was diluted with DCM and a solution of saturated aqueous sodium bicarbonate. The two phases were separated and the aqueous layer was extracted with DCM (x 3). The combined organic extracts were dried over sodium sulfate, filtered, and concentrated to give the crude product, which was purified using silica gel cartridge chromatography (gradient elution 0: 100-A:B to 20:60-A:B where A is a premixed solution of 2.5% NH₄OH and 20% MeOH in DCM and B is DCM) to give a yellow solid which was triturated with diethyl ether to give (*S*)-4'-((*S*)-6-(1*H*-pyrazol-1-yl)-1,4-oxazepan-4-yl)-7-amino-2'-(((2*R*,7a*S*)-2-fluorotetrahydro-1*H*-pyrrolizin-7a(5*H*)-yl)methoxy)-3,4,5',8'-tetrahydro-2*H*-spiro[naphthalene-1,7'-pyrano[4,3-*d*]pyrimidine]-8-carbonitrile (4 mg) as an off-white solid.
LCMS: m/z (ESI) [M+H]⁺ 615.4, t_{R} = 1.41 minutes (Method C)
¹H NMR (400 MHz, CDCl₃) δ 7.57 (d, 1H), 7.53 (d, 1H), 7.04 (d, 1H), 6.64 (d, 1H), 6.26 (m, 1H), 5.36 - 5.15 (m, 1H), 5.00 (m, 1H), 4.83 (d, 2H), 4.43 (s, 2H), 4.24 (m, 2H), 4.15 - 4.03 (m, 2H), 4.03 - 3.92 (m, 3H), 3.80 (m, 2H), 3.64 (m, 1H), 3.34 (d, 1H), 3.29 - 3.07 (m, 3H), 3.05 - 2.91 (m, 2H), 2.69 (m, 2H), 2.29 - 2.05 (m, 4H), 1.97 - 1.71 (m, 6H).

### Example 62: (S)-4'-((R)-6-(1H-pyrazol-1-yl)-1,4-oxazepan-4-yl)-7-amino-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidine]-8-carbonitrile (Compound 516b)

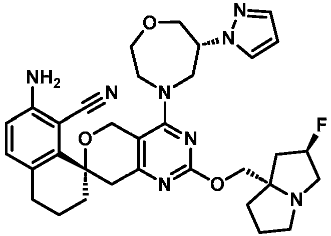

### Step 1: tert-butyl (S)-6-((methylsulfonyl)oxy)-1,4-oxazepane-4-carboxylate

Methanesulfonyl chloride (427 µL, 5.52 mmol) was added dropwise to a stirring solution of *tert*-butyl (*S*)-6-hydroxy-1,4-oxazepane-4-carboxylate (923 mg, 4.25 mmol) and TEA (1.78 mL, 12.7 mmol) in DCM (14.2 mL) at 0 °C. The mixture was warmed to room temperature and stirred for 1 hour. The reaction mixture was diluted with water and DCM. The two phases were separated, and the aqueous layer was extracted with DCM (x 3). The combined organic extracts were dried over sodium sulfate, filtered, and concentrated to afford *tert*-butyl (*S*)-6-((methylsulfonyl)oxy)-1,4-oxazepane-4-carboxylate (1.3 g) as an orange oil, which was used in the next step without further purification.
LCMS: m/z (ESI) [M+H]⁺ 240.1, t_{R} = 1.62 minutes (Method C)

### Step 2: tert-Butyl (R)-6-(1H-pyrazol-1-yl)-1,4-oxazepane-4-carboxylate

*Tert*-butyl (*S*)-6-((methylsulfonyl)oxy)-1,4-oxazepane-4-carboxylate (1.25 g, 4.23 mmol) was dissolved in DMF (21.2 mL). 1*H*-pyrazole (375 mg, 1.3 *equiv.,* 5.50 mmol) and NaH (339 mg, 60 wt%, 8.46 mmol) were added to the mixture. The resulting reaction mixture was heated to 80 °C and stirred overnight. The reaction mixture was cooled to room temperature and diluted with water and EtOAc. The two phases were separated and the aqueous layer was extracted with EtOAc (x 3). The combined organic extracts were dried over sodium sulfate, filtered, and concentrated to a residue which was purified via flash chromatography (0-40% EtOAc/DCM) to afford *tert*-butyl (*R*)-6-(1*H*-pyrazol-1-yl)-1,4-oxazepane-4-carboxylate (620 mg) as a clear colorless oil.
LCMS: m/z (ESI) [M+H]⁺ 268.2, t_{R} = 1.73 minutes (Method C)

### Step 3: (R)-6-(1H-pyrazol-1-yl)-1,4-oxazepane hydrochloride

A solution of *tert*-butyl (*R*)-6-(1*H*-pyrazol-1-yl)-1,4-oxazepane-4-carboxylate (100 mg, 374 µmol) in DCM (1.41 mL) was treated with 4M HCl in dioxane (470 µL) at 0 °C. The mixture was warmed to room temperature and stirred overnight. After 20 hours, the reaction mixture was concentrated to afford (*R*)-6-(1*H*-pyrazol-1-yl)-1,4-oxazepane hydrochloride (96.3 mg) as a colorless oil.
LCMS: m/z (ESI) [M+H]⁺ 168.0, t_{R} = 0.27 minutes (Method C)

### Step 4: (S)-4'-((R)-6-(1H-pyrazol-1-yl)-1,4-oxazepan-4-yl)-7-amino-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidine]-8-carbonitrile

DIPEA (30 µL, 0.17 mmol) was added to a solution of (*S*)-7-amino-2'-(((2*R*,7a*S*)-2-fluorotetrahydro-1*H*-pyrrolizin-7a(5*H*)-yl)methoxy)-4'-hydroxy-3,4,5',8'-tetrahydro-2*H-*spiro[naphthalene-1,7'-pyrano[4,3-d]pyrimidine]-8-carbonitrile (24.3 mg, 52.2 µmol) and PyBOP (35.7 mg, 68.6 µmol) in MeCN (260 µL) at room temperature. The reaction was stirred at 70 °C for 10 minutes giving an orange homogeneous solution. This solution was then cooled back to room temperature. (*R*)-6-(1*H*-pyrazol-1-yl)-1,4-oxazepane hydrochloride and DIPEA (30 µL, 0.17 mmol) were added and the resulting mixture was heated at 55 °C and stirred for 19 hours then at room temperature for 2 days. The mixture was concentrated and partitioned between a saturated solution of sodium bicarbonate (3 mL) and EtOAc (1 mL). The phases were separated, and the organic phase was washed with a saturated solution of sodium bicarbonate (2 x 3 mL). The organic phase was washed with brine (3 mL), dried over sodium sulfate, filtered, and concentrated to afford a brown solid. The crude was dissolved in DCM and washed with 1N HCl (3 x 3 mL). The aqueous phase was basified with 1 M sodium hydroxide until pH = 13. The aqueous phase was washed with DCM (3 x 3 mL) and the resulting layers were separated and the pooled organic phase was dried over sodium sulfate, filtered, and concentrated to afford a brown solid. The crude was purified via reverse phase chromatography [ACCQ Prep; 5-55% (NH₄OH in MeCN)/(NH₄OH in water)] to afford (*S*)-4'-((*R*)-6-(1*H*-pyrazol-1-yl)-1,4-oxazepan-4-yl)-7-amino-2'-(((2*R*,7a*S*)-2-fluorotetrahydro-1*H-*pyrrolizin-7a(5*H*)-yl)methoxy)-3,4,5',8'-tetrahydro-2*H*-spiro[naphthalene-1,7'-pyrano[4,3-*d*]pyrimidine]-8-carbonitrile as an off-white solid (15.2 mg).
¹H NMR (400 MHz, CDCl₃) δ 7.64 (brs, 1H), 7.52 (d, 1H), 7.04 (d, 1H), 6.65 (d, 1H), 6.31 - 6.26 (m, 1H), 5.44 - 5.22 (m, 1H), 4.98 - 4.87 (m, 2H), 4.87 - 4.79 (m, 1H), 4.51 - 4.37 (m, 3H), 4.28 - 4.06 (m, 5H), 4.00 - 3.87 (m, 2H), 3.72 (dd, 1H), 3.56 - 3.46 (m, 2H), 3.37 (d, 1H), 3.33 - 3.20 (m, 1H), 3.09 - 2.91 (m, 2H), 2.72 - 2.61 (m, 2H), 2.41 - 2.16 (m, 3H), 2.16 - 2.05 (m, 1H), 2.05 - 1.85 (m, 4H), 1.84 - 1.64 (m, 2H).
¹⁹F NMR (376 MHz, CDCl₃) δ -172.68.
LCMS: m/z (ESI) [M+H]⁺ 615.4, t_{R} = 1.38 minutes (Method C)

### Example 63: (S)-7-Amino-2'-(((2R,7aS)-2-fluorotetrahydro-1H-pyrrolizin-7a(5H)-yl)methoxy)-4'-((SR)-6-(2-methyloxazol-5-yl)-1,4-oxazepan-4-yl)-3,4,5',8'-tetrahydro-2H-spiro[naphthalene-1,7'-pyrano[4,3-d]pyrimidine]-8-carbonitrile (Compound 529a)

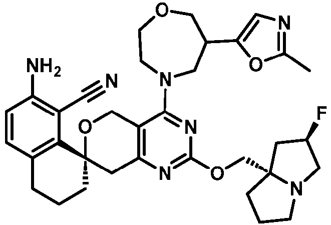

### Step 1: tert-Butyl (R)-6-formyl-1,4-oxazepane-4-carboxylate

Dess-Martin periodinane (1.61 g, 1.1 *equiv.,* 3.80 mmol) was added to a stirred solution of *tert*-butyl (*R*)-6-(hydroxymethyl)-1,4-oxazepane-4-carboxylate (800 mg, *1 equiv.,* 3.46 mmol) in DCM (17.3 mL). The reaction mixture was stirred at ambient temperature for 2 hours. The reaction mixture was diluted with water and DCM. The two phases were separated and the aqueous layer was extracted with DCM (3 x 20 mL). The combined organic extracts were dried over sodium sulfate, filtered, and concentrated to give the crude product, which was purified by flash column chromatography (0:100-EtOAc:Heptane to 60:40-EtOAc:Heptane) to give *tert*-butyl (*R*)-6-formyl-1,4-oxazepane-4-carboxylate (516 mg).

¹H NMR (400 MHz, CDCl₃) δ 9.79 (d, *J* = 8.6 Hz, 1H), 4.19-4.06 (m, 1H), 3.99 - 3.46 (m, 7H), 3.01-2.89 (m, 1H), 1.45 (s, 9H).

### Step 2: tert-Butyl (R)-6-((S)-1-hydroxy-2-nitroethyl)-1,4-oxazepane-4-carboxylate

KOtBu (294 mg, 2.62 mmol) was added to a stirred solution of *tert*-butyl (*R*)-6-formyl-1,4-oxazepane-4-carboxylate (600 mg, 1 *equiv.,* 2.62 mmol) and nitromethane (282 µL, 2 *equiv.,* 5.23 mmol) in THF (1.45 mL) and t-BuOH (1.45 mL) at ambient temperature for 3 hours. The reaction mixture was acidified with AcOH to pH = 6. The resulting mixture was extracted with EtOAc (3 x 20 mL). The combined organic extracts were dried over sodium sulfate, filtered, and concentrated to give the crude product, which was purified by flash column chromatography (10:90-EtOAc:Heptane to 80:20-EtOAc:Heptane) to give *tert*-butyl (*R*)-6-((*S*)-1-hydroxy-2-nitroethyl)-1,4-oxazepane-4-carboxylate (518 mg).
LCMS: m/z (ESI) [M-Boc+H]⁺ = 191.1 t_{R} = 1.57 minutes (Method K)

### Step 3: tert-Butyl (R)-6-((S)-2-acetamido-1-hydroxyethyl)-1,4-oxazepane-4-carboxylate

Pd on carbon (77.7 mg, 10 wt%, 0.1 *equiv.,* 73.0 µmol) was added to a stirred solution of *tert*-butyl (*R*)-6-((*S*)-1-hydroxy-2-nitroethyl)-1,4-oxazepane-4-carboxylate (212 mg, 1 *equiv.,* 730 µmol) in MeOH (1.46 mL). The reaction mixture was purged with nitrogen. A balloon was used to introduce a hydrogen atmosphere. Hydrogen was bubbled through the reaction mixture for 4 minutes. The reaction mixture was stirred at ambient temperature for 16 hours. The reaction mixture was filtered, and the filtrate was concentrated to dryness to give the crude intermediate product as a colorless solid which was used in the nest step without further purification.

DIPEA (145 mg, 196 µL, 1.5 *equiv.,* 1.12 mmol) was added to a stirred solution of HATU (342 mg, 1.2 *equiv.,* 900 µmol) and acetic acid (45.0 mg, 43.0 µL, 1 *equiv.,* 750 µmol) in DCM (5 mL). The resulting mixture was stirred at ambient temperature for 15 minutes at which point *tert*-butyl (6*S*)-6-(2-amino-1-hydroxyethyl)-1,4-oxazepane-4-carboxylate (195 mg, *1 equiv.,* 750 µmol) in DCM (2 mL) was added. The reaction mixture was stirred at ambient temperature for 2 hours. The reaction mixture was then diluted with water (30 mL) and DCM (30 mL). The two phases were separated and the aqueous layer was extracted with DCM (3 x 20 mL). The combined organic extracts were dried over sodium sulfate, filtered, and concentrated to give *tert*-butyl (R)-6-((S)-2-acetamido-1-hydroxyethyl)-1,4-oxazepane-4-carboxylate (220 mg).
LCMS: m/z (ESI) [M-Boc+H]⁺ = 203.2 m/z, t_{R} = 1.30 minutes (Method K)

### Step 4: tert-Butyl (R)-6-(acetylglycyl)-1,4-oxazepane-4-carboxylate

Dess-Martin periodinane (370 mg, 1.2 *equiv.,* 875 µmol) was added to a stirred solution of *tert*-butyl (*R*)-6-((*S*)-2-acetamido-1-hydroxyethyl)-1,4-oxazepane-4-carboxylate (220 mg, 1 *equiv.,* 730 µmol) in DCM (3.64 mL). The resulting mixture was stirred at ambient temperature for 2 hours. The reaction mixture was quenched by the addition of a saturated aqueous sodium bicarbonate (30 mL). The mixture was diluted with DCM and the two phases were separated and the aqueous layer was extracted with DCM (3 x 20 mL). The combined organic extracts were dried over sodium sulfate, filtered, and concentrated to give the crude product which was purified by flash column chromatography (0:100-EtOAc:Heptane to 100:0-EtOAc:Heptane then from 0:100-MeOH:DCM to 10:90-MeOH:DCM) to give *tert*-butyl (*R*)-6-(acetylglycyl)-1,4-oxazepane-4-carboxylate (94 mg).
LCMS: m/z (ESI) [M-Boc+H]⁺ = 201.2 m/z; t_{R} = 1.38 min; (Method C)

### Step 5: tert-Butyl (R)-6-(2-methyloxazol-5-yl)-1,4-oxazepane-4-carboxylate

Burgess reagent (224 mg, 3 *equiv.,* 940 µmol) was added to a stirred solution *tert*-butyl (*R*)-6-(acetylglycyl)-1,4-oxazepane-4-carboxylate (94.0 mg, 1 *equiv.,* 315 µmol) in THF (5.20 mL) at ambient temperature. The reaction mixture was stirred for 3 hours then concentrated to a brown oil, which was purified using a RediSep Rf Gold silica gel cartridge (liquid load in DCM with gradient elution from 0:100-EtOAc:DCM to 100:0-EtOAc:DCM) to afford *tert-*butyl (*R*)-6-(2-methyloxazol-5-yl)-1,4-oxazepane-4-carboxylate (24 mg).
LCMS: m/z (ESI) [M+H]⁺ = 283.3 m/z; t_{R} = 1.59 minutes (Method C)

### Step 6: (R)-6-(2-Methyloxazol-5-yl)-1,4-oxazepane

HCl (0.43 mL, 4.0 M, 20 *equiv.,* 1.7 mmol) in dioxane was added to a stirred solution of *tert*-butyl (*R*)-6-(2-methyloxazol-5-yl)-1,4-oxazepane-4-carboxylate (24 mg, 1 *equiv.,* 85 µmol) in DCM (0.85 mL). The resulting mixture was stirred at ambient temperature for 2 hours. The reaction mixture was concentrated to dryness to give (*R*)-6-(2-methyloxazol-5-yl)-1,4-oxazepane which was used without further purification.
LCMS: m/z (ESI) [M+H]⁺ 183.5 m/z; t_{R} = 0.27 min; (Method C)

### Step 7: (S)-7-amino-2'-(((2R,7aS)-2-fluorotetrahydro-1H-pyrrolizin-7a(5H)-yl)methoxy)-4'-((SR)-6-(2-methyloxazol-5-yl)-1,4-oxazepan-4-yl)-3,4,5',8'-tetrahydro-2H-spiro[naphthalene-1,7'-pyrano[4,3-d]pyrimidine]-8-carbonitrile

(*S*)-7-Amino-2'-(((2*R*,7a*S*)-2-fluorotetrahydro-1*H*-pyrrolizin-7a(5*H*)-yl)methoxy)-4'-hydroxy-3,4,5',8'-tetrahydro-2*H*-spiro[naphthalene-1,7'-pyrano[4,3-*d*]pyrimidine]-8-carbonitrile (20 mg, 1 *equiv.,* 43 µmol), (*R*)-6-(2-methyloxazol-5-yl)-1,4-oxazepane (16 mg, 2 *equiv.,* 86 µmol), DIPEA (33 mg, 45 µL, 6 *equiv.,* 0.26 mmol), PyBOP (27 mg, 1.2 *equiv., 52* µmol), and MeCN (0.21 mL) were combined in a vial. The resulting mixture was heated to 70 °C and stirred for 7 hours. The reaction mixture was cooled to ambient temperature and diluted with EtOAc (20 mL) and saturated aqueous sodium bicarbonate solution (20 mL). The two phases were separated and the aqueous layer was extracted with EtOAc (3 x 20mL). The combined organic extracts were dried over sodium sulfate, filtered, and concentrated to give the crude product. The crude residue was re-dissolved in DCM (20 mL). A solution of saturated aqueous sodium bicarbonate was added (20 mL). The two phases were separated and the aqueous layer was extracted with DCM (3 x 20 mL). The combined organic extracts were washed with 1M HCl (2 x 10 mL). The aqueous layer was basified slowly until pH = 13. To this mixture, DCM (20 mL) was added. The aqueous layer was extracted with DCM (3 x 10 mL). The combined organic layers were dried over sodium sulfate, filtered, and concentrated to give the crude product. The crude product was purified using a RediSep Rf Gold silica gel cartridge (liquid load in DCM with gradient elution 0:100-A:B to 20:60-A:B where A is a premixed solution of 2.5% NH₄OH and 20% MeOH in DCM and B is DCM) to give (*S*)-7-amino-2'-(((2*R*,7a*S*)-2-fluorotetrahydro-1*H*-pyrrolizin-7a(5*H*)-yl)methoxy)-4'-((*SR*)-6-(2-methyloxazol-5-yl)-1,4-oxazepan-4-yl)-3,4,5',8'-tetrahydro-2*H*-spiro[naphthalene-1,7'-pyrano[4,3-*d*]pyrimidine]-8-carbonitrile (15.9 mg).
¹H NMR (400 MHz, CDCl₃) δ 7.03 (d, 1H), 6.75 - 6.70 (m, 1H), 6.66-6.62 (m, 1H), 5.34 - 5.15 (m, 1H), 5.05 - 4.80 (m, 2H), 4.45-4.44 (m, 2H), 4.26-4.17 (m, 1H), 4.14 - 3.83 (m, 7H), 3.71 - 3.47 (m, 2H), 3.47 - 3.29 (m, 2H), 3.27 - 3.05 (m, 3H), 3.02 - 2.89 (m, 2H), 2.70-2.65 (m, 2H), 2.42 (d, 3H), 2.30 - 2.16 (m, 2H), 2.15 - 2.03 (m, 2H), 1.96 - 1.84 (m, 3H), 1.84 - 1.68 (m, 3H).
¹⁹F NMR (376 MHz, CDCl₃) δ -172.99, -173.06.
LCMS: m/z (ESI) [M+H]⁺ 630.4 m/z; t_{R} = 1.84 min; (Method E)

### Example 64: 5-((S*)-7-amino-8-cyano-2'-((S)-1-((S)-1-methylpyrrolidin-2-yl)ethoxy)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide (Compound 527a)

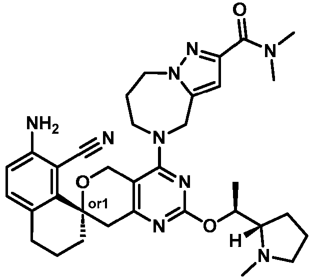

### Step 1: 5-(8-cyano-7-(dibenzylamino)-2'-((S)-1-((S)-1-methylpyrrolidin-2-yl)ethoxy)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide

A LiHMDS solution (1M in THF, 260 µL, 1.8 *equiv.,* 260 µmol) was added to a solution of (*S*)-1-((*S*)-1-methylpyrrolidin-2-yl)ethan-1-ol (94.0 mg, 5 *equiv.,* 730 µmol) in anhydrous THF (2.20 mL). To this mixture, a solution of 5-(8-cyano-7-(dibenzylamino)-2'-(methylsulfonyl)-3,4,5',8'-tetrahydro-2*H*-spiro[naphthalene-1,7'-pyrano[4,3-*d*]pyrimidin]-4'-yl)-*N*,*N*-dimethyl-5,6,7,8-tetrahydro-4*H*-pyrazolo[1,5-*a*][1,4]diazepine-2-carboxamide (130 mg, 85 wt%, *1 equiv.,* 146 µmol) in anhydrous THF (2.21 mL) was added over 1 minute at 0°C. The mixture was warmed to ambient temperature. The reaction was then heated to 60°C for 1 hour. Water (1 ml) was then added at ambient temperature and the volatiles were removed under reduced pressure. The contents were treated with the slow addition of a 1:1 mixture of a saturated aqueous ammonium chloride solution and water at 0 °C. EtOAc (10 mL) was then added, and the layers were separated and the organics were then dried, concentrated, and the crude residue was purified by flash column chromatography (reverse phase, 13 g, liquid loading, pure water to pure acetonitrile) to afford 5-(8-cyano-7-(dibenzylamino)-2'-((*S*)-1-((*S*)-1-methylpyrrolidin-2-yl)ethoxy)-3,4,5',8'-tetrahydro-2*H*-spiro[naphthalene-1,7'-pyrano[4,3-*d*]pyrimidin]-4'-yl)-*N,N*-dimethyl-5,6,7,8-tetrahydro-4*H*-pyrazolo[1,5-*a*][1,4]diazepine-2-carboxamide (70 mg).
LCMS: m/z (ESI) [M+H]⁺ 806.5; t_{R}= 1.40; 1.43 minutes (Method A)

### Step 2: 5-((S*)-7-amino-8-cyano-2'-((S)-1-((S)-1-methylpyrrolidin-2-yl)ethoxy)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide

To a 5 mL flame dried vial, charged with a stirring solution of 5-(8-cyano-7-(dibenzylamino)-2'-((*S*)-1-((*S*)-1-methylpyrrolidin-2-yl)ethoxy)-3,4,5',8'-tetrahydro-2*H-*spiro[naphthalene-1,7'-pyrano[4,3-*d*]pyrimidin]-4'-yl)-*N,N*-dimethyl-5,6,7,8-tetrahydro-4*H-*pyrazolo[1,5-*a*][1,4]diazepine-2-carboxamide (70 mg, 88 wt%, *1 equiv.,* 76 µmol) in MeOH (1.5 mL) sparged with argon, Pd(OH)₂ on carbon (0.13 g, 20 wt%, 2.5 *equiv.,* 0.19 mmol) was added. The inert atmosphere was exchanged under reduced pressure with hydrogen from a balloon. Three drops of acetic acid were added to the reaction mixture and the contents were then stirred overnight. The suspension was then filtered over a polytetrafluoroethylene filter with EtOAc, DCM, and MeOH. The mixture was concentrated to a residue which was purified by reverse phase column chromatography (12 g, ammonium bicarbonate 10 mM to MeCN and then 1/1 MeCN/MeOH). The compound was then further purified via preparative HPLC (Mobile phase Solvent A: 10mM Ammonium Formate pH = 3.8; Solvent B: Acetonitrile; Flow rate: 45mL/min; Column: Gemini^{®} 5 µm NX-C18 110 Å, 100 x 30 mm; Pre-run: 1.5 min at initial condition (85% A, 15% B)) to afford 5-((*S**)-7-amino-8-cyano-2'-((*S*)-1-((*S*)-1-methylpyrrolidin-2-yl)ethoxy)-3,4,5',8'-tetrahydro-2*H*-spiro[naphthalene-1,7'-pyrano[4,3-*d*]pyrimidin]-4'-yl)-*N,N*-dimethyl-5,6,7,8-tetrahydro-4H-pyrazolo[1,5-a][1,4]diazepine-2-carboxamide (11 mg) **(Peak 1)** and 5-((*R**)-7-amino-8-cyano-2'-((*S*)-1-((*S*)-1-methylpyrrolidin-2-yl)ethoxy)-3,4,5',8'-tetrahydro-2*H*-spiro[naphthalene-1,7'-pyrano[4,3-*d*]pyrimidin]-4'-yl)-*N,N*-dimethyl-5,6,7,8-tetrahydro-4*H*-pyrazolo[1,5-*a*][1,4]diazepine-2-carboxamide (7 mg) **(Peak 2).**

### Peak 1:

LCMS: m/z (ESI) [M+H]⁺ 626.5; t_{R} = 3.59 minutes (Method I)
¹H NMR (400 MHz, DMSO-*d₆*): 7.03 (d, 1 H); 6.71 (d, 1 H); 6.51 (s, 1 H); 5.72 (s, 2 H); 5.01-4.97 (m, 2 H); 4.69 (s, 2 H); 4.61-4.57 (m, 1 H); 4.47-4.44 (m, 2 H); 3.78-3.89 (m, 1 H); 3.23 (s, 3 H); 3.03-3.10 (m, 1 H); 2.91 (s, 4 H); 2.85 (s, 1 H); 2.54-2.66 (m, 2 H); 2.29-2.31 (m, 3 H); 2.08-2.15 (m, 3 H); 1.50-2.04 (m, 9 H); 1.16-1.21 (m, 3 H).

### Example 65: 5-((R*)-7-amino-8-cyano-2'-((S)-1-((S)-1-methylpyrrolidin-2-yl)ethoxy)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide (Compound 527b)

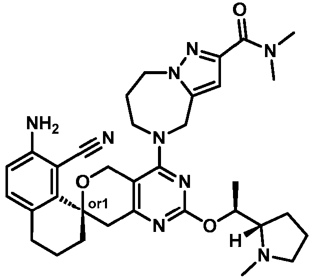

5-((*R**)-7-amino-8-cyano-2'-((*S*)-1-((*S*)-1-methylpyrrolidin-2-yl)ethoxy)-3,4,5',8'-tetrahydro-2*H*-spiro[naphthalene-1,7'-pyrano[4,3-*d*]pyrimidin]-4'-yl)-*N,N*-dimethyl-5,6,7,8-tetrahydro-4*H*-pyrazolo[1,5-*a*][1,4]diazepine-2-carboxamide was obtained from **Example 64,** as **Peak 2:** prep HPLC (Mobile phase: - A: 10mM Ammonium Bicarbonate pH = 10.0 - B: MeOH Flow rate: 45mL/min Column: Gemini^{®} 5 µm NX-C18 110 Å, 100 x 30 mm Pre-run: 1.5 min at initial condition (55% A, 45% B) 2nd time : Mobile phase: - A: 10mM Ammonium Bicarbonate pH = 10.0 - B: MeCN Flow rate: 45mL/min Column: Gemini^{®} 5 µm NX-C18 110 Å, 100 x 30 mm Pre-run: 1.5 min at initial condition (75% A, 25% B)).
LCMS: m/z (ESI) [M+H]⁺ 626.5; t_{R}= 3.61 minutes (Method I)
¹H NMR (CHCl₃-*d*, 400 MHz): 7.04 (d, 1H), 6.64 (d, 1H), 6.58 (s, 1H), 4.90 (s, 2H), 4.41-4.72 (m, 6H), 4.03-3.98 (m, 1H), 3.71 (br s, 1H), 3.31-3.34 (m, 4H), 3.07 (s, 3H), 2.99-2.94 (m, 1H), 1.59-2.58 (m, 16H), 1.28-1.25 (m, 6H).

### Example 66: 5-((1R,4S)-7-Amino-8-cyano-2'-(((2R,7aS)-2-fluorotetrahydro-1H-pyrrolizin-7a(5H)-yl)methoxy)-4-methyl-3,4,5',8'-tetrahydro-2H-spiro[naphthalene-1,7'-pyrano[4,3-d]pyrimidin]-4'-yl)-3-chloro-N,N-dimethyl-5,6,7,8-tetrahydro-4H-pyrazolo[1,5-a][1,4]diazepine-2-carboxamide (Compound 509b)

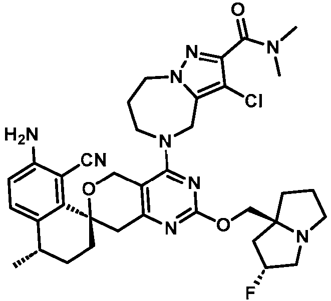

### Step 1: 5-((1R,4S)-8-Bromo-7-(dibenzylamino)-4-methyl-2'-(methylthio)-3,4,5',8'-tetrahydro-2H-spiro[naphthalene-1,7'-pyrano[4,3-d]pyrimidin]-4'-yl)-3-chloro-N,N-dimethyl-5,6,7,8-tetrahydro-4H-pyrazolo[1,5-a][1,4]diazepine-2-carboxamide

To a 4 dram vial containing (1*R*,4*S*)-*N,N*-dibenzyl-8-bromo-4'-chloro-4-methyl-2'-(methylthio)-3,4,5',8'-tetrahydro-2*H*-spiro[naphthalene-1,7'-pyrano[4,3-*d*]pyrimidin]-7-amine (803 mg, 1 *equiv.,* 1.29 mmol) was added 3-chloro-*N*,*N*-dimethyl-5,6,7,8-tetrahydro-4H-pyrazolo[1,5-*a*][1,4]diazepine-2-carboxamide, 3HCl (347.6 mg, 0.76 *equiv.,* 987.3 µmol), in MeCN (12.9 mL). The reaction mixture was heated to 70 °C and stirred for 16 hours. The mixture was then cooled to room temperature followed by the addition of 3-chloro-*N*,*N-*dimethyl-5,6,7,8-tetrahydro-4*H*-pyrazolo[1,5-*a*][1,4]diazepine-2-carboxamide, 3HCl (231 mg, 0.50 *equiv.,* 656 µmol) and DIPEA (501 mg, 676 µL, 3 *equiv.,* 3.88 mmol). The reaction was again heated to 70 °C and stirred for 2 hours then cooed to room temperature followed by the addition of 3-chloro-*N,N*-dimethyl-5,6,7,8-tetrahydro-4*H*-pyrazolo[1,5-*a*][1,4]diazepine-2-carboxamide, HCl (230 mg, 0.637 *equiv.,* 824 µmol) and DIPEA (742 mg, 1.00 mL, 4.44 *equiv.,* 5.74 mmol). The resulting reaction was stirred overnight at 70 °C then cooled before the addition of DIPEA (371 mg, 500 µL, 2.22 *equiv.,* 2.87 mmol). The reaction was then refluxed again overnight under a nitrogen atmosphere. The reaction was then cooled, diluted with water (5 mL) and EtOAc (5 mL). The aqueous phase was separated, extracted EtOAc (2 x 5 mL), and the organic phase was dried over sodium sulfate, filtered, and concentrated under reduced pressure to give 1.21 g of crude solid, which was purified by column chromatography (0-->100%) EtOAc in DCM to give 5-((1*R*,4*5*)-8-bromo-7-(dibenzylamino)-4-methyl-2'-(methylthio)-3,4,5',8'-tetrahydro-2*H*-spiro[naphthalene-1,7'-pyrano[4,3-*d*]pyrimidin]-4'-yl)-3-chloro-*N*,*N*-dimethyl-5,6,7,8-tetrahydro-4*H*-pyrazolo[1,5-*a*][1,4]diazepine-2-carboxamide (1.00 g).
LCMS: m/z (ESI) [M+H]⁺ 826.3 t_{R} = 4.09 minutes (Method E)

### Step 2: 3-chloro-5-((1R,4S)-8-cyano-7-(dibenzylamino)-2'-(((2R,7aS)-2-fluorotetrahydro-1H-pyrrolizin-7a(5H)-yl)methoxy)-4-methyl-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide

To a 50 mL round bottom flask was added copper(I) cyanide (512 mg, 5 *equiv.,* 5.71 mmol) followed by 5-((1*R*,4*S*)-8-bromo-7-(dibenzylamino)-4-methyl-2'-(methylthio)-3,4,5',8'-tetrahydro-2*H*-spiro[naphthalene-1,7'-pyrano[4,3-*d*]pyrimidin]-4'-yl)-3-chloro-*N,N*-dimethyl-5,6,7,8-tetrahydro-4*H*-pyrazolo[1,5-*a*][1,4]diazepine-2-carboxamide (945 mg, 1 *equiv.,* 1.14 mmol) dissolved in DMF (5.7 mL). The mixture was then heated to 120 °C for 45 minutes. The reaction was cooled to room temperature followed by the addition of DCM (50 mL). The mixture was filtered, and the filtrate was washed with NH₄OH (25 mL) followed by water (50 mL). The aqueous layers were extracted to with DCM (3x50 mL). The combined organic layers were dried over sodium sulfate, filtered, and then concentrated to give a crude residue. The solid from the precipitate was dissolved in 10% MeOH in DCM (50 mL). To this mixture, water (5 mL) was added, and the organic layer was separated. The aqueous layer was extracted with a 10:1 DCM:MeOH (2 x 50 mL). The organic layers were combined, dried over sodium sulfate, filtered, and concentrated to give 792 mg of crude residue.

Oxone (3.08 g, 45 wt%, 2.25 mmol) was added to a 100 mL round bottom flask containing the crude residue from above in THF (12.0 mL), MeOH (7.50 mL), and water (7.50 mL). The reaction was stirred at room temperature for 25 minutes. The reaction was then quenched with saturated sodium thiosulfate (50 mL) and the resulting mixture was stirred for 15 minutes. The reaction was extracted with EtOAc (3 x 50 mL). The organic layers were combined and washed with additional saturated sodium thiosulfate (100 mL). The organic layer was then dried over sodium sulfate, filtered, and concentrated to give 878 mg of crude sulfone/sulfoxide.

To a vial charged with the above crude material, was added ((2*R*,7a*S*)-2-fluorotetrahydro-1*H*-pyrrolizin-7a(5*H*)-yl)methanol (195 mg, 1.22 mmol). This mixture was dried by azeotropic distillation with toluene (3 x 30 mL). The vial headspace was evacuated and refilled with nitrogen. THF (11.0 mL) was added, and the reaction mixture was cooled to 0 °C, followed by the addition of sodium 2-methylbutan-2-olate (135 mg, 1.22 mmol) in one portion, while maintaining the internal temperature below 4 °C. The resulting mixture was warmed to room temperature for 2 hours then quenched with a 1:1 mixture of ammonium chloride and water (50 mL). The organic layer was separated, and the aqueous layer was extracted with EtOAc (3 x 30 mL). The combined organics were dried over sodium sulfate, filtered, and concentrated to give 1.7 g as an orange solid which was purified using a 80g silica column (0-35% gradient of 97.5% 20% MeOH in DCM, 2.5% NH₄OH in DCM) to give 3-chloro-5-((1*R*,4*S*)-8-cyano-7-(dibenzylamino)-2'-(((2*R*,7a*S*)-2-fluorotetrahydro-1*H-*pyrrolizin-7a(5*H*)-yl)methoxy)-4-methyl-3,4,5',8'-tetrahydro-2*H*-spiro[naphthalene-1,7'-pyrano[4,3-*d*]pyrimidin]-4'-yl)-*N,N*-dimethyl-5,6,7,8-tetrahydro-4*H*-pyrazolo[1,5-*a*][1,4]diazepine-2-carboxamide (101 mg).
LCMS: m/z (ESI) [M+H]⁺ 884.5 t_{R} = 2.71 minutes (Method E)

### Step 3: 5-((1R,4S)-7-amino-8-cyano-2'-(((2R,7aS)-2-fluorotetrahydro-1H-pyrrolizin-7a(5H)-yl)methoxy)-4-methyl-3,4,5',8'-tetrahydro-2H-spiro[naphthalene-1,7'-pyrano[4,3-d]pyrimidin]-4'-yl)-3-chloro-N,N-dimethyl-5,6,7,8-tetrahydro-4H-pyrazolo[1,5-a][1,4]diazepine-2-carboxamide

3-Chloro-5-((1*R*,4*S*)-8-cyano-7-(dibenzylamino)-2'-(((2*R*,7a*S*)-2-fluorotetrahydro-1*H-*pyrrolizin-7a(5*H*)-yl)methoxy)-4-methyl-3,4,5',8'-tetrahydro-2*H*-spiro[naphthalene-1,7'-pyrano[4,3-*d*]pyrimidin]-4'-yl)-*N,N*-dimethyl-5,6,7,8-tetrahydro-4*H*-pyrazolo[1,5-*a*][1,4]diazepine-2-carboxamide (85.0 mg, *1 equiv.,* 96.1 µmol) and Pd(OH)₂ on carbon (135 mg, 39.2 µL, 20 wt%, 2 *equiv.,* 192 µmol) were charged to a vial and backfilled with nitrogen (3x). MeOH (3.0 mL) and acetic acid (300 µL) were added to the reaction and the resulting mixture was sparged with H₂ for 15 minutes. The reaction mixture was stirred at room temperature under an H₂ atmosphere for 1 hour. The reaction was filtered through celite with MeOH (10 mL) and concentrated to a colorless residue. The residue was dissolved with MeOH (5 mL) and stirred with MP carbonate resin (200 mg) for 2 hours then filtered, and concentrated to give 5-((1*R*,4*S*)-7-amino-8-cyano-2'-(((2*R*,7a*S*)-2-fluorotetrahydro-1*H*-pyrrolizin-7a(5*H*)-yl)methoxy)-4-methyl-3,4,5',8'-tetrahydro-2*H*-spiro[naphthalene-1,7'-pyrano[4,3-*d*]pyrimidin]-4'-yl)-3-chloro-*N*,*N*-dimethyl-5,6,7,8-tetrahydro-4*H*-pyrazolo[1,5-*a*][1,4]diazepine-2-carboxamide (57 mg).
¹H NMR (400 MHz, CDCl₃) δ 7.25 (d, 1H), 6.68 (d, 1H), 5.24 (d, 1H), 5.04 (d, 1H), 4.86 (d, 1H), 4.69 (d, 1H), 4.56 - 4.35 (m, 5H), 4.12 - 3.91 (m, 3H), 3.76 - 3.56 (m, 1H), 3.34 (d, 1H), 3.28 - 3.17 (m, 2H), 3.14 - 2.88 (m, 9H), 2.84 - 2.71 (m, 1H), 2.44 - 2.29 (m, 1H), 2.25 - 2.03 (m, 5H), 2.03 - 1.68 (m, 7H), 1.53 - 1.32 (m, 1H), 1.24 (d, 2H).
LCMS: m/z (ESI) [M+H]⁺ 704.2 t_{R} = 1.94 minutes (Method E)

### Example 67: 5-(7-amino-8-cyano-2'-(((S)-1,2-dimethylpyrrolidin-2-yl)methoxy)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide (Compound 526a)

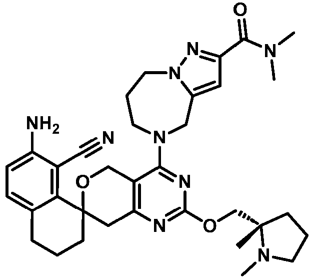

### Step 1: 5-(8-cyano-7-(dibenzylamino)-2'-(((S)-1,2-dimethylpyrrolidin-2-yl)methoxy)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide

To a solution of (*S*)-(1,2-dimethylpyrrolidin-2-yl)methanol (102 mg, 786 µmol, 5.0 *equiv.*) in THF (2.38 mL) at 0 °C, was added LiHMDS (1.0 M in THF, 0.28 mL, 0.28 mmol, 1.8 *equiv.*)*,* and a solution of 5-(8-cyano-7-(dibenzylamino)-2'-(methylsulfonyl)-3,4,5',8'-tetrahydro-2*H*-spiro[naphthalene-1,7'-pyrano[4,3-*d*]pyrimidin]-4'-yl)-*N,N*-dimethyl-5,6,7,8-tetrahydro-4*H*-pyrazolo[1,5-*a*][1,4]diazepine-2-carboxamide (140 mg, 85% purity, 157 µmol) in THF (2.38 mL) over 1 minute. The reaction mixture was stirred at room temperature for 3 hours and then at 60 °C for 1 hour. The mixture was cooled to room temperature and the reaction was quenched by the slow addition of a 1:1 mixture of saturated aqueous ammonium chloride solution and water at 0 °C. EtOAc was added, and the layers were separated and the combined organic extracts were dried over magnesium sulfate, filtered, and concentrated to a residue which was purified by reverse phase chromatography (column: C18 13 g, 10 mL/min, water and acetonitrile, 0 → 100%) to afford 5-(8-cyano-7-(dibenzylamino)-2'-(((*S*)-1,2-dimethylpyrrolidin-2-yl)methoxy)-3,4,5',8'-tetrahydro-2*H*-spiro[naphthalene-1,7'-pyrano[4,3-*d*]pyrimidin]-4'-yl)-*N*,*N*-dimethyl-5,6,7,8-tetrahydro-4*H*-pyrazolo[1,5-*a*][1,4]diazepine-2-carboxamide (80 mg).
LCMS: m/z (ESI) [M+H]⁺ 806.6, t_{R} = 1.37 minutes, (Method A)

### Step 2: 5-(7-Amino-8-cyano-2'-(((S)-1,2-dimethylpyrrolidin-2-yl)methoxy)-3,4,5',8'-tetrahydro-2H-spiro[naphthalene-1,7'-pyrano[4,3-d]pyrimidin]-4'-yl)-N,N-dimethyl-5,6,7,8-tetrahydro-4H-pyrazolo[1,5-a][1,4]diazepine-2-carboxamide

To a solution of 5-(8-cyano-7-(dibenzylamino)-2'-(((*S*)-1,2-dimethylpyrrolidin-2-yl)methoxy)-3,4,5',8'-tetrahydro-2*H*-spiro[naphthalene-1,7'-pyrano[4,3-*d*]pyrimidin]-4'-yl)-*N*,*N*-dimethyl-5,6,7,8-tetrahydro-4*H*-pyrazolo[1,5-*a*][1,4]diazepine-2-carboxamide (80 mg, 75% purity, 74 µmol) in MeOH (1.5 mL), sparged with argon, was added Pd(OH)₂ on carbon (20 wt%, 0.13 g, 0.19 mmol, 2.5 *equiv.*)*.* The reaction mixture was degassed under reduced pressure with a backflow of hydrogen (balloon). Three drops of acetic acid were added, and the resulting reaction mixture was stirred under an atmosphere of hydrogen at room temperature for 17 hours. The resulting black suspension was filtered using a polytetrafluoroethylene filter and the solids were rinsed with EtOAc, DCM, and MeOH. The filtrate was concentrated to a residue which was purified by reverse phase chromatography (column: C18 Biotage 12 g, 10 mL/min, 10 mM ammonium bicarbonate aqueous solution and acetonitrile, 0 → 100%). Further purification was achieved by reverse phase HPLC prep chromatography (column: Gemini^{®} 5 µm NX-C18 110 Å, 100 x 30 mm; mobile phase: 10 mM ammonium formate pH = 3.8 / MeCN; flow: 45 mL/min; gradient: 20% for 1.5 min then 20 → 40% over 9.5 min) to afford 5-(7-amino-8-cyano-2'-(((*S*)-1,2-dimethylpyrrolidin-2-yl)methoxy)-3,4,5',8'-tetrahydro-2*H*-spiro[naphthalene-1,7'-pyrano[4,3-*d*]pyrimidin]-4'-yl)-*N*,*N*-dimethyl-5,6,7,8-tetrahydro-4*H*-pyrazolo[1,5-*a*][1,4]diazepine-2-carboxamide (17 mg) as a solid.
LCMS: m/z (ESI) [M+H]⁺ 626.5, t_{R} = 3.53 min, (Method I)
¹H NMR (400 MHz, DMSO-*d₆*) δ 7.08 - 6.99 (m, 1H), 6.77 - 6.68 (m, 1H), 6.57 - 6.48 (m, 1H), 5.73 (br s, 2H), 5.03 - 4.93 (m, 1H), 4.82 - 4.57 (m, 3H), 4.50 - 4.39 (m, 2H), 4.03 - 3.94 (m, 2H), 3.96 - 3.75 (m, 2H), 3.24 (br s, 3H), 3.14 - 3.04 (m, 1H), 2.93 (br s, 3H), 2.90 - 2.77 (m, 2H), 2.69 - 2.57 (m, 3H), 2.23 (br s, 3H), 2.20 - 2.11 (m, 2H), 2.04 - 1.91 (m, 1H), 1.91 - 1.73 (m, 3H), 1.73 - 1.59 (m, 3H), 1.59 - 1.47 (m, 1H), 0.99 (br s, 3H).

### Example 68: 5-((S)-7-amino-8-cyano-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)-3-fluoro-N,N-dimethyl-5,6,7,8-tetrahydro-4H-pyrazolo[1,5-a][1,4]diazepine-2-carboxamide (Compound 517a)

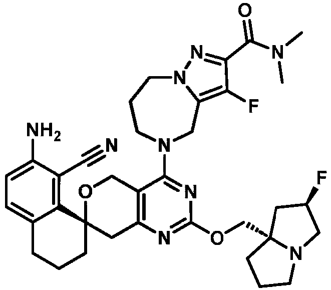

### Step 1: tert-Butyl 2-(dimethylcarbamoyl)-7,8-dihydro-4H-pyrazolo[1,5-a][1,4]diazepine-5(6H)-carboxylate

To a solution of 5-(tert-butoxycarbonyl)-5,6,7,8-tetrahydro-4*H*-pyrazolo[1,5-*a*][1,4]diazepine-2-carboxylic acid, DIPEA (37 mL, 0.21 mol), and dimethylamine, HCl (12 g, 0.14 mol) in DCM (200 mL) was added HATU (41 g, 0.11 mol) at 0 °C. The resulting mixture was stirred at 25 °C for 1 hour. The reaction mixture was concentrated under reduced pressure and the crude residue was purified by reverse phase column chromatography (C 18, 45-65% MeCN: [0.1% NH₄HCO₃] in H₂O) to afford *tert-*butyl 2-(dimethylcarbamoyl)-7,8-dihydro-4*H-*pyrazolo[1,5-*a*][1,4]diazepine-5(6*H*)-carboxylate (20.2 g) as an off white solid.
LCMS: m/z (ESI) [M+H]⁺ 309.2 t_{R} = 0.337 minutes (Method H).
¹H NMR (400 MHz, CDCl₃) δ 6.53 (s, 1H), 4.49-4.40 (m, 4H), 3.71 (s, 2H), 3.33-3.28 (m, 3H), 3.08 (s, 3H), 1.94-1.92 (m, 2H), 1.40 (s, 9H).

### Step 2: tert-Butyl 2-(dimethylcarbamoyl)-3-fluoro-7,8-dihydro-4H-pyrazolo[1,5-a][1,4]diazepine-5(6H)-carboxylate

To a heat dried flask was added *tert*-butyl 2-(dimethylcarbamoyl)-7,8-dihydro-4*H-*pyrazolo[1,5-*a*][1,4]diazepine-5(6*H*)-carboxylate (2.0 g, 6.5 mmol) and Selectfluor (11 g, 32 mmol) in MeCN (20 mL). The resulting mixture was stirred at room temperature under argon for 16 hours. The reaction mixture was concentrated and the crude residue was purified by flash chromatography (XSelect^{®} CSH^{™} Prep C18 5 µm OBD^{™} 19x150 mm, 30-90% 0.1% FA in MeCN: 0.1% FA in H₂O) to afford *tert*-butyl 2-(dimethylcarbamoyl)-3-fluoro-7,8-dihydro-4*H*-pyrazolo[1,5-*a*][1,4]diazepine-5(6*H*)-carboxylate (169.4 mg) as a white solid.

LCMS: m/z (ESI) [M+H]⁺ 327.6 t_{R} = 1.358 minutes, (Method L).

¹H NMR (400 MHz, CDCl₃) δ 4.54-4.35 (m, 4H), 3.74 (s, 2H), 3.25-3.06 (m, 6H), 1.96 (m, 2H), 1.44 (s, 9H).

### Step 3: 3-Fluoro-N,N-dimethyl-5,6,7,8-tetrahydro-4H-pyrazolo[1,5-a][1,4]diazepine-2-carboxamide, HCl

A solution of *tert*-butyl 2-(dimethylcarbamoyl)-3-fluoro-7,8-dihydro-4*H*-pyrazolo[1,5-*a*][1,4]diazepine-5(6*H*)-carboxylate (169 mg, 0.518 mmol) in DCM (2.5 mL) was treated with HCl in dioxanes (1.11 mL, 4 M, 4.45 mmol). The mixture was stirred at room temperature for 1 hour. The reaction mixture was concentrated under a stream of nitrogen. Ether (3 mL) was added, and the reaction mixture was stirred until a white precipitate was formed. The ether was then decanted and the white solid was triturated twice with ether (3 mL). The solid was then dried under vacuum to afford 3-fluoro-*N,N*-dimethyl-5,6,7,8-tetrahydro-4*H*-pyrazolo[1,5-*a*][1,4]diazepine-2-carboxamide, HCl (119.6 mg) as a white solid.

LCMS: m/z (ESI) [M+H]⁺ 227.2 t_{R} = 0.292 minutes, (Method L).

### Step 4: 5-((S)-7-Amino-8-cyano-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)-3-fluoro-N,N-dimethyl-5,6,7,8-tetrahydro-4H-pyrazolo[1,5-a][1,4]diazepine-2-carboxamide

DIPEA (0.060 mL, 0.34 mmol) was added to a vial containing 3-fluoro-*N,N*-dimethyl-5,6,7,8-tetrahydro-4*H*-pyrazolo[1,5-*a*][1,4]diazepine-2-carboxamide, HCl (56 mg, 0.21 mmol), (*S*)-7-amino-2'-(((2*R*,7a*S*)-2-fluorotetrahydro-1*H*-pyrrolizin-7a(5*H*)-yl)methoxy)-4'-hydroxy-3,4,5',8'-tetrahydro-2*H*-spiro[naphthalene-1,7'-pyrano[4,3-d]pyrimidine]-8-carbonitrile (20 mg, 0.043 mmol), and PyBOP (34 mg, 0.064 mmol) in DMF (0.86 mL). The vial was sealed, and the reaction mixture was stirred at 80 °C for 90 minutes. The reaction mixture was diluted with water, and the layers were separated and the aqueous layer was extracted with EtOAc three times. The combined organic layers were washed with 10% LiCl solution, dried over sodium sulfate, filtered, and concentrated. The crude residue was purified by flash chromatography (RediSep Rf Gold 4g, 0-30% MeOH:DCM) to afford 5-((S)-7-amino-8-cyano-2'-(((2*R*,7a*S*)-2-fluorotetrahydro-1*H*-pyrrolizin-7a(5*H*)-yl)methoxy)-3,4,5',8'-tetrahydro-2*H*-spiro[naphthalene-1,7'-pyrano[4,3-*d*]pyrimidin]-4'-yl)-3-fluoro-*N*,*N*-dimethyl-5,6,7,8-tetrahydro-4*H*-pyrazolo[1,5-*a*][1,4]diazepine-2-carboxamide (8.26 mg) as an off white solid.

LCMS: m/z (ESI) [M+H]⁺ 674.7 t_{R} = 1.717 minutes, (Method L).

¹H NMR (400 MHz, CDCl₃) δ 6.96 (d, 1H), 6.57 (d, 1H), 5.27 - 5.09 (m, 1H), 4.90 (d, 1H), 4.78 (d1H), 4.63 - 4.44 (m, 2H), 4.41 - 4.28 (m, 4H), 4.01 - 3.85 (m, 2H), 3.66 - 3.57 (m, 1H), 3.26 (d, 1H), 3.21 - 3.12 (m, 1H), 3.10 (s, 3H), 3.07 (s, 1H), 3.00 (s, 3H), 2.95 - 2.84 (m, 2H), 2.61 (mj, 2H), 2.25 - 2.14 (m, 2H), 2.12 - 2.02 (m, 2H), 1.90 - 1.48 (m, 8H).

### Example 69: 5-((S)-7-amino-8-cyano-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)-N,N,3-trimethyl-5,6,7,8-tetrahydro-4H-pyrazolo[1,5-a][1,4]diazepine-2-carboxamide (Compound 520a)

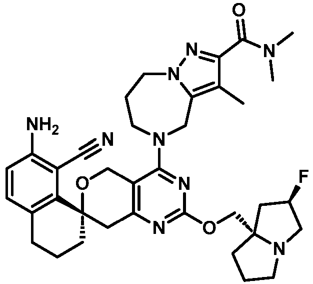

### Step 1: tert-Butyl 2-(dimethylcarbamoyl)-3-iodo-7,8-dihydro-4H-pyrazolo[1,5-a][1,4]diazepine-5(6H)-carboxylate

To a solution of *tert*-butyl 2-(dimethylcarbamoyl)-7,8-dihydro-4*H*-pyrazolo[1,5-*a*][1,4]diazepine-5(6*H*)-carboxylate (2.08 g, 6.74 mmol) in acetic acid (17.4 mL) was added NIS (3.04 g, 13.5 mmol). The resulting reaction mixture was stirred at room temperature for 2 hours. The reaction mixture was diluted with EtOAc and a mixture of saturated aqueous sodium thiosulfate solution and brine. The layers were separated, and the aqueous phase was extracted with EtOAc. The organic phases were collected, dried over magnesium sulfate, filtered, and concentrated to a reddish-brown oil, which was purified by flash chromatography (**Biotage^{®} Sfär Silica HC D** 20 µm 24 g, 50-100% EtOAc:DCM) to afford *tert*-butyl 2-(dimethylcarbamoyl)-3-iodo-7,8-dihydro-4*H*-pyrazolo[1,5-*a*][1,4]diazepine-5(6*H*)-carboxylate (1.60 g) as a dark red oil.

LCMS: m/z (ESI) [M+H]⁺ 435.2 t_{R} = 1.583 minutes, (Method L).

### Step 2: tert-Butyl 2-(dimethylcarbamoyl)-3-methyl-7,8-dihydro-4H-pyrazolo[1,5-a][1,4]diazepine-5(6H)-carboxylate

A solution of *tert*-butyl 2-(dimethylcarbamoyl)-3-iodo-7,8-dihydro-4*H*-pyrazolo[1,5-*a*][1,4]diazepine-5(6*H*)-carboxylate (1.04 g, 2.40 mmol), potassium carbonate (664 mg, 4.80 mmol), and methylboronic acid (288 mg, 4.81 mmol) in 1,4-dioxane (12.0 mL) and water (4.01 mL) was bubbled with nitrogen for 30 minutes. To this mixture, cataCXium A Pd G3 (175.0 mg, 0.240 mmol) was added, and the resulting mixture was stirred at 80 °C for 1 hour then 16 hours at 95 °C. The reaction mixture was cooled to room temperature, filtered, and concentrated. The crude residue was taken up in DMSO and subjected to reverse phase purification (XSelect^{®} CSH^{™} Prep C18 5 µm OBD^{™} 19x150 mm, 0-100% 0.1% NH₄OH in MeCN: 0.1% NH₄OH in H₂O) to afford *tert*-butyl 2-(dimethylcarbamoyl)-3-methyl-7,8-dihydro-4*H*-pyrazolo[1,5-*a*][1,4]diazepine-5(6*H*)-carboxylate (102.5 mg).

LCMS: m/z (ESI) [M+H]⁺ 323.3 t_{R} = 1.522 minutes, (Method C).

¹H NMR (400 MHz, DMSO-*d*₆) δ 4.48 - 4.32 (m, 4H), 3.66 (s, 2H), 3.01 (d, 6H), 2.03 (s, 3H), 1.79 = 1.70 (m, 2H), 1.37 (s, 9H).

### Step 3: N,N,3-Trimethyl-5,6,7,8-tetrahydro-4H-pyrazolo[1,5-a][1,4]diazepine-2-carboxamide, HCl

A solution of *tert*-butyl 2-(dimethylcarbamoyl)-3-methyl-7,8-dihydro-4*H-*pyrazolo[1,5-*a*][1,4]diazepine-5(6*H*)-carboxylate (102.5 mg, 0.318 mmol) in 1,4-dioxane (3.2 mL) was treated with HCl in dioxane (0.795 mL, 4 M, 3.18 mmol). The resulting reaction mixture was stirred at room temperature for 2 hours. The reaction mixture was concentrated under reduced pressure to yield *N,N*,3-trimethyl-5,6,7,8-tetrahydro-4*H*-pyrazolo[1,5-*a*][1,4]diazepine-2-carboxamide, HCl (65.5 mg) as a light yellow solid.

¹H NMR (400 MHz, DMSO-*d*₆) δ 4.47 - 4.26 (m, 4H), 3.39 (s, 2H), 3.01 (d, 6H), 2.04 (s, 3H), 1.98 (s, 2H).

### Step 4: 5-((S)-7-Amino-8-cyano-2'-(((2R,7aS)-2-fluorotetrahydro-1H-pyrrolizin-7a(5H)-yl)methoxy)-3,4,5',8'-tetrahydro-2H-spiro[naphthalene-1,7'-pyrano[4,3-d]pyrimidin]-4'-yl)-N,N,3-trimethyl-5,6,7,8-tetrahydro-4H-pyrazolo[1,5-a][1,4]diazepine-2-carboxamide

To a solution of (*S*)-7-amino-2'-(((2*R*,7a*S*)-2-fluorotetrahydro-1*H*-pyrrolizin-7a(5*H*)-yl)methoxy)-4'-hydroxy-3,4,5',8'-tetrahydro-2*H*-spiro[naphthalene-1,7'-pyrano[4,3-*d*]pyrimidine]-8-carbonitrile in DMF (0.70 mL, 0.05 M, 0.035 mmol) was added *N,N,*3-trimethyl-5,6,7,8-tetrahydro-4*H*-pyrazolo[1,5-*a*][1,4]diazepine-2-carboxamide, HCl (38 mg, 0.15 mmol), and PyBOP (27 mg, 0.052 mmol). DIPEA (0.049 mL, 0.28 mmol) was then added dropwise, and the resulting reaction mixture was stirred at 80 °C for 1 hour. The reaction mixture was cooled to room temperature and diluted with a mixture of water (5 mL), 5% saturated aqueous LiCl solution (5 mL), and EtOAc (10 mL). The aqueous layer was extracted with EtOAc (3 x 10 mL) and the combined organic layers were dried over sodium sulfate, filtered, and concentrated to a dark orange oil. The crude residue was taken up in DMSO and purified by reverse phase column chromatography (XSelect^{®} CSH^{™} Prep C18 5 µm OBD^{™} 19x150 mm, 0-100% 0.1% NH₄OH in MeCN: 0.1% NH₄OH in water, focused gradient 45-55%) to yield 5-((*S*)-7-amino-8-cyano-2'-(((2*R*,7a*S*)-2-fluorotetrahydro-1*H*-pyrrolizin-7a(5*H*)-yl)methoxy)-3,4,5',8'-tetrahydro-2*H*-spiro[naphthalene-1,7'-pyrano[4,3-d]pyrimidin]-4'-yl)-*N*,*N*,3-trimethyl-5,6,7,8-tetrahydro-4*H*-pyrazolo[1,5-*a*][1,4]diazepine-2-carboxamide (7.2 mg) as an off-white solid.

LCMS: m/z (ESI) [M+H]⁺ 670.3 t_{R} = 1.253 minutes, (Method C).

¹H NMR (400 MHz, DMSO-*d*₆) δ 7.04 (d, 1H), 6.72 (d, 1H), 5.74 (s, 2H), 5.34 - 5.15 (m, 1H), 4.94 (d, 1H), 4.75 - 4.62 (m, 3H), 4.44 - 4.29 (m, 2H), 3.90 - 3.76 (m, 4H), 3.13 - 3.01 (m, 6H), 2.97 (s, 1H), 2.93 (s, 3H), 2.85 (d, 1H), 2.82 - 2.76 (m, 1H), 2.59 (s, 2H), 2.19 - 1.90 (m, 10H), 1.85 - 1.67 (m, 5H).

### Example 70: (S)-7-Amino-2'-(((2R,7aS)-2-fluorotetrahydro-1H-pyrrolizin-7a(5H)-yl)methoxy)-4'-(6-oxa-1-azaspiro[3.3]heptan-1-yl)-3,4,5',8'-tetrahydro-2H-spiro[naphthalene-1,7'-pyrano[4,3-d]pyrimidine]-8-carbonitrile (Compound 550a)

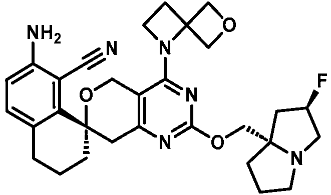

BOP (23 mg, 1.2 *equiv.,* 0.052 mmol) was added to a solution of (*S*)-7-amino-2'-(((2*R*,7a*S*)-2-fluorotetrahydro-1*H*-pyrrolizin-7a(5*H*)-yl)methoxy)-4'-hydroxy-3,4,5',8'-tetrahydro-2*H*-spiro[naphthalene-1,7'-pyrano[4,3-*d*]pyrimidine]-8-carbonitrile (20 mg, 0.043 mmol) and DIPEA (0.075 mL, 10 *equiv.,* 0.43 mmol) in DMF (0.5 mL). The resulting mixture was stirred at room temperature for 1 hour. 6-Oxa-1-azaspiro[3.3]heptane oxalate (25 mg, 2.0 *equiv.,* 0.086 mmol) was then added, and the resulting reaction mixture was stirred at room temperature for 2 hours and then at 60 °C for 1 hour. The reaction mixture was concentrated and purified by reverse phase chromatography (12g C18 silica, 0-100% water/methanol with 0.1% NaHCO₃; flow rate: 10 mL/min) to afford (*S*)-7-amino-2'-(((2*R*,7a*S*)-2-fluorotetrahydro-1*H*-pyrrolizin-7a(5*H*)-yl)methoxy)-4'-(6-oxa-1-azaspiro[3.3]heptan-1-yl)-3,4,5',8'-tetrahydro-2*H*-spiro[naphthalene-1,7'-pyrano[4,3-*d*]pyrimidine]-8-carbonitrile (9 mg).
LCMS: m/z (ESI) [M+H]⁺ 547.4 t_{R} = 1.39 minutes (Method B)
¹H NMR (400 MHz, DMSO-*d*₆): δ 7.01 (d, 1H), 6.71 (d, 1H), 5.73 (s, 2H), 5.54 (d, 1H), 5.43 (d, 1H), 5.23 (d, 1H), 4.89 - 4.73 (m, 2H), 4.52 (dd, 2H), 4.35 (q, 1H), 4.24 (q, 1H), 4.10 - 3.96 (m, 2H), 3.12 - 2.89 (m, 5H), 2.78 (q, 1H), 2.69 (d, 1H), 2.62 - 2.52 (m, 2H), 2.16 - 1.85 (m, 4H), 1.85 - 1.67 (m, 3H), 1.67 - 1.53 (m, 2H).
¹⁹F NMR (376 MHz, DMSO) δ -171.51 - -172.22 (m).

### Example 71: (1S,4S)-7-Amino-2'-(((2R,7aS)-2-fluorotetrahydro-1H-pyrrolizin-7a(5H)-yl)methoxy)-4-methyl-4'-(6-oxa-1-azaspiro[3.3]heptan-1-yl)-3,4,5',8'-tetrahydro-2H-spiro[naphthalene-1,7'-pyrano[4,3-d]pyrimidine]-8-carbonitrile (Compound 556a)

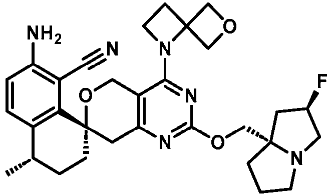

BOP (50 mg, 1.2 *equiv.,* 0.11 mmol) was added to a solution of (1*S*,4*S*)-7-amino-2'-(((2*R*,7a*S*)-2-fluorotetrahydro-1*H*-pyrrolizin-7a(5*H*)-yl)methoxy)-4'-hydroxy-4-methyl-3,4,5',8'-tetrahydro-2*H*-spiro[naphthalene-1,7'-pyrano[4,3-*d*]pyrimidine]-8-carbonitrile (45 mg, 0.094 mmol) and DIPEA (0.16 mL, 10 *equiv.*, 0.94 mmol) in DMF (0.5 mL). The mixture was stirred at room temperature for 1 hour. 6-Oxa-1-azaspiro[3.3]heptane oxalate (25 mg, 4.0 *equiv.,* 0.375 mmol) was then added, and the resulting reaction mixture was stirred at 60 °C for 2 hours. The reaction mixture was concentrated and purified by reverse phase chromatography (12 g, C18 silica, 0-100% water/methanol with 0.1% NaHCO₃; flow rate: 10 mL/min) to afford (1*S*,4*S*)-7-Amino-2'-(((2*R*,7a*S*)-2-fluorotetrahydro-1*H*-pyrrolizin-7a(5*H*)-yl)methoxy)-4-methyl-4'-(6-oxa-1-azaspiro[3.3]heptan-1-yl)-3,4,5',8'-tetrahydro-2*H*-spiro[naphthalene-1,7'-pyrano[4,3-*d*]pyrimidine]-8-carbonitrile (23 mg).
LCMS: m/z (ESI) [M+H]⁺ 561.4 t_{R} = 1.47 minutes (Method B)
¹H NMR (400 MHz, DMSO-*d*₆) δ 7.08 (d, 1H), 6.73 (d, 1H), 5.76 (s, 2H), 5.54 (d, 1H), 5.43 (d, 1H), 5.32 - 5.12 (m, 1H), 4.83 (s, 2H), 4.52 (dd, 2H), 4.36 (q, 1H), 4.24 (q, 1H), 4.10 - 3.97 (m, 2H), 3.02 (d, 2H), 2.95 (dd, 2H), 2.77 (dd, 2H), 2.68 (d, 1H), 2.54 (q, 2H), 2.15 - 1.95 (m, 2H), 1.95 - 1.66 (m, 7H), 1.53 (d, 1H), 1.13 (d, 3H)
¹⁹F NMR (376 MHz, DMSO) δ -171.56 - -172.18 (m)

### Example 112: (1S,4S)-7-amino-2'-(((2R,7aS)-2-fluorotetrahydro-1H-pyrrolizin-7a(5H)-yl)methoxy)-4-methyl-4'-((R)-6-oxa-1-azaspiro[3.5]nonan-1-yl)-3,4,5',8'-tetrahydro-2H-spiro[naphthalene-1,7'-pyrano[4,3-d]pyrimidine]-8-carbonitrile (Compound 585a)

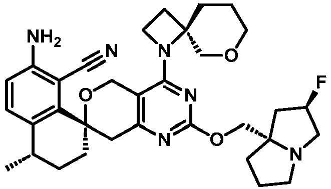

To a solution of DIPEA (81 mg, 0.11 mL, 10 *equiv.,* 0.63 mmol) and BOP (33 mg, 1.2 *equiv.,* 75 µmol) in DMF (0.5 mL) was added (1*S*,4*S*)-7-amino-2'-(((2*R*,7a*S*)-2-fluorotetrahydro-1*H*-pyrrolizin-7a(5H)-yl)methoxy)-4'-hydroxy-4-methyl-3,4,5',8'-tetrahydro-2*H*-spiro[naphthalene-1,7'-pyrano[4,3-*d*]pyrimidine]-8-carbonitrile (30 mg, 1 *equiv.,* 63 µmol). The reaction mixture was stirred for 1 hour at room temperature. 6-Oxa-1-azaspiro[3.5]nonane hydrochloride (10 mg, 1 *equiv.,* 63 µmol) was then added to the reaction mixture, and the resulting solution was stirred for 1 hour at room temperature and then 2 hours at 50 °C. The reaction mixture was concentrated to a residue and purified by reverse-phase chromatography (20 g Sentai C18 silica; flow rate = 10 mL/min; 0-100% 0.1% ammonium carbonate in water/acetonitrile) to yield (1*S*,4*S*)-7-amino-2'-(((2*R*,7a*S*)-2-fluorotetrahydro-1*H-*pyrrolizin-7a(5H)-yl)methoxy)-4-methyl-4'-(6-oxa-1-azaspiro[3.5]nonan-1-yl)-3,4,5',8'-tetrahydro-2*H*-spiro[naphthalene-1,7'-pyrano[4,3-d]pyrimidine]-8-carbonitrile (26 mg).
LCMS: m/z (ESI) [M+H]⁺ 589.4 t_{R} = 1.59 and 1.64 minutes (Method B)

The diastereomers of (1*S*,4*S*)-7-amino-2'-(((2*R*,7a*S*)-2-fluorotetrahydro-1*H*-pyrrolizin-7a(5H)-yl)methoxy)-4-methyl-4'-(6-oxa-1-azaspiro[3.5]nonan-1-yl)-3,4,5',8'-tetrahydro-2*H-*spiro[naphthalene-1,7'-pyrano[4,3-*d*]pyrimidine]-8-carbonitrile were separated by preparative HPLC (Mobile phase A: 10 mM ammonium bicarbonate pH = 10.0; B: methanol; Flow rate: 45mL/min; Column: Gemini^{®} 5 µm NX-C18 110 Å, 100 × 30 mm; Pre-run: 1.5 min at initial condition (45% A, 55% B)) to afford (1*S*,4*S*)-7-amino-2'-(((2*R*,7a*S*)-2-fluorotetrahydro-1*H-*pyrrolizin-7a(5*H*)-yl)methoxy)-4-methyl-4'-((*R*)-6-oxa-1-azaspiro[3.5]nonan-1-yl)-3,4,5',8'-tetrahydro-2*H*-spiro[naphthalene-1,7'-pyrano[4,3-*d*]pyrimidine]-8-carbonitrile (**Peak 1**) and (1*S*,4*S*)-7-amino-2'-(2*R*,7a*S*)-2-fluorotetrahydro-1*H*-pyrrolizin-7a(5*H*)-yl)methoxy)-4-methyl-4'-((*S*)-6-oxa-1-azaspiro[3.5]nonan-1-yl)-3,4,5',8'-tetrahydro-2*H*-spiro[naphthalene-1,7'-pyrano[4,3-*d*]pyrimidine]-8-carbonitrile (**Peak 2**).

### Peak 1:

LCMS: m/z (ESI) [M+H]⁺ 589.4 t_{R} = 1.59 minutes (Method B)
¹H NMR (400 MHz, DMSO-*d*₆): 7.07 (d, 1 H); 6.72 (d, 1 H); 5.75 (s, 2 H); 5.24 (d, 1 H); 4.81 (s, 2 H); 4.43 (d, 1 H); 4.24-4.32 (m, 2 H); 3.75-3.90 (m, 4 H); 2.61-3.08 (m, 9 H); 1.60-2.26 (m, 15 H); 1.13 (d, 3 H).
¹⁹F NMR (376 MHz, DMSO): -171.6--172.1.

### Example 113: (1S,4S)-7-amino-2'-(((2R,7aS)-2-fluorotetrahydro-1H-pyrrolizin-7a(5H)-yl)methoxy)-4-methyl-4'-((S)-6-oxa-1-azaspiro[3.5]nonan-1-yl)-3,4,5',8'-tetrahydro-2H-spiro[naphthalene-1,7'-pyrano[4,3-d]pyrimidine]-8-carbonitrile (Compound 585b)

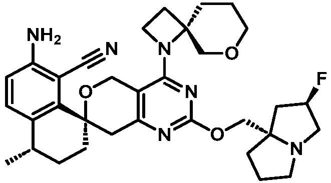

(1*S*,4*S*)-7-amino-2'-(((2*R*,7a*S*)-2-fluorotetrahydro-1*H*-pyrrolizin-7a(5*H*)-yl)methoxy)-4-methyl-4'-((*S*)-6-oxa-1-azaspiro[3.5]nonan-1-yl)-3,4,5',8'-tetrahydro-2*H*-spiro[naphthalene-1,7'-pyrano[4,3-*d*]pyrimidine]-8-carbonitrile was obtained from **Example 112** as **Peak 2.**
LCMS: m/z (ESI) [M+H]⁺ 589.4 t_{R} = 1.64 minutes (Method B)
¹H NMR (400 MHz, DMSO-*d*₆): 7.07 (d, J = 8.64 Hz; 1 H); 6.72 (d, J = 8.63 Hz; 1 H); 5.75 (s, 2 H); 5.24 (d, J = 54.41 Hz; 1 H); 4.82 (s, 2 H); 4.38 (dd, J = 48.11; 7.84 Hz; 2 H); 4.14 (d, J = 10.50 Hz; 1 H); 3.76-3.91 (m, 4 H); 2.60-3.11 (m, 9 H); 1.53-2.19 (m, 15 H); 1.13 (d, J = 6.96 Hz; 3 H).
¹⁹F NMR (376 MHz, DMSO): -171.6-172.1.

### Example 114: (1S,4S)-7-Amino-2'-(((2R,7aS)-2-fluorotetrahydro-1H-pyrrolizin-7a(5H)-yl)methoxy)-4'-((R)-2-(hydroxymethyl)azetidin-1-yl)-4-methyl-3,4,5',8'-tetrahydro-2H-spiro[naphthalene-1,7'-pyrano[4,3-d]pyrimidine]-8-carbonitrile (Compound 576a)

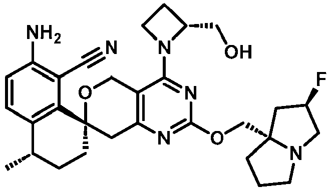

PyBOP (50 mg, 0.11 mmol) was added to a solution of (1*S*,4*S*)-7-amino-2'-(((2*R*,7a*S*)-2-fluorotetrahydro-1*H*-pyrrolizin-7a(5*H*)-yl)methoxy)-4'-hydroxy-4-methyl-3,4,5',8'-tetrahydro-2*H*-spiro[naphthalene-1,7'-pyrano[4,3-*d*]pyrimidine]-8-carbonitrile (21 mg, 0.044 mmol) and DIPEA (0.076 mL, 0.44 mmol) in DMF (0.5 mL). The mixture was stirred at room temperature for 1 hour. (*R*)-azetidin-2-ylmethanol, TFA (62 mg, 0.31 mmol) was added to the reaction mixture. The resulting mixture was stirred at room temperature for 2 hours. The reaction mixture was then concentrated and purified by reverse-phase chromatography (12 g C18 silica, 0-100% water/methanol with 0.1% NaH₄CO₃; flowrate: 10 mL/min) to afford (1*S*,4*S*)-7-amino-2'-(((2*R*,7a*S*)-2-fluorotetrahydro-1*H*-pyrrolizin-7a(5*H*)-yl)methoxy)-4'-((*R*)-2-(hydroxymethyl)azetidin-1-yl)-4-methyl-3,4,5',8'-tetrahydro-2*H*-spiro[naphthalene-1,7'-pyrano[4,3-*d*]pyrimidine]-8-carbonitrile (15 mg).
LCMS: m/z (ESI) [M+H]⁺ 549.4 t_{R} = 1.39 minutes (Method B)
¹H NMR (400 MHz, CD₃CN): 6.54 (d, 1 H); 6.19 (d, 1 H); 5.22 (s, 2 H); 4.69 (d, 1 H); 4.43 (t, 1 H); 4.33 (d, 1 H); 4.15 (d, 1 H); 3.98 (s, 1 H); 3.64-3.76 (m, 2 H); 3.19-3.33 (m, 3 H); 3.07 (d, 1 H); 2.42-2.50 (m, 3 H); 2.24 (br s, 2 H); 2.10 (d, 1 H); 1.13-1.73 (m, 12 H); 0.97 (s, 1 H); 0.59 (d, 3 H)
¹⁹F NMR (376 MHz, DMSO): -171.9-172.3 (m)

### Example 115: (1S,4S)-7-Amino-2'-(((2R,7aS)-2-fluorotetrahydro-1H-pyrrolizin-7a(5H)-yl)methoxy)-4-methyl-4'-(7-oxa-1-azaspiro[3.5]nonan-1-yl)-3,4,5',8'-tetrahydro-2H-spiro[naphthalene-1,7'-pyrano[4,3-d]pyrimidine]-8-carbonitrile (Compound 589a)

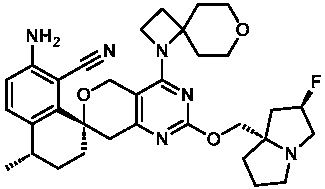

DIPEA (0.08 mL, 10 *equiv.,* 0.44 mmol) and PyBOP (23 mg, 1.2 *equiv.,* 53 µmol) were added to a solution of (1*S*,4*S*)-7-amino-2'-(((2*R*,7a*S*)-2-fluorotetrahydro-1*H*-pyrrolizin-7a(5*H*)-yl)methoxy)-4'-hydroxy-4-methyl-3,4,5',8'-tetrahydro-2*H*-spiro[naphthalene-1,7'-pyrano[4,3-*d*]pyrimidine]-8-carbonitrile (21 mg, *1 equiv.,* 44 µmol) in DMF (0.5 mL). The reaction mixture was stirred for 1 hour at room temperature. 7-Oxa-1-azaspiro[3.5]nonane (22 mg, 1.2 *equiv.,* 180 µmol) was then added to the reaction mixture, and the resulting solution was stirred for 1 hour at room temperature and then for 2 hours at 50 °C. The reaction mixture was concentrated to a residue and purified by reverse-phase chromatography (20 g Sentai C18 silica; flow rate = 10 mL/min; 0-100% 0.1% ammonium carbonate in water/acetonitrile) to yield (1*S*,4*S*)-7-amino-2'-(((2*R*,7a*S*)-2-fluorotetrahydro-1*H*-pyrrolizin-7a(5*H*)-yl)methoxy)-4-methyl-4'-(7-oxa-1-azaspiro[3.5]nonan-1-yl)-3,4,5',8'-tetrahydro-2*H*-spiro[naphthalene-1,7'-pyrano[4,3-*d*]pyrimidine]-8-carbonitrile (25 mg).
LCMS: m/z (ESI) [M+H]⁺ 589.5 t_{R} = 1.57 minutes (Method B)
¹H NMR (400 MHz, DMSO-*d*₆): 7.07 (d, 1 H); 6.72 (d, 1 H); 5.75 (s, 2 H); 5.23 (d, 1 H); 4.84 (s, 2 H); 4.44 (t, 1 H); 4.33 (t, 1 H); 3.89-3.80 (m, 4 H); 3.28-3.24 (m, 1H); 3.01-2.58 (m, 10 H); 2.12-1.61 (m, 13 H); 1.53 (s, 1H); 1.13 (d, 3 H).
¹⁹F NMR (376 MHz, DMSO): -171.8--172.2.

### Example 116: (1S,4S)-7-Amino-2'-(((2R,7aS)-2-fluorotetrahydro-1H-pyrrolizin-7a(5H)-yl)methoxy)-4'-((R)-2-(methoxymethyl)azetidin-1-yl)-4-methyl-3,4,5',8'-tetrahydro-2H-spiro[naphthalene-1,7'-pyrano[4,3-d]pyrimidine]-8-carbonitrile (Compound 578a)

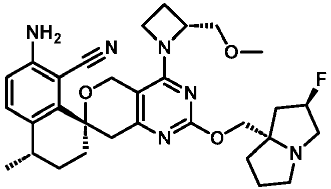

BOP (24 mg, 1.2 *equiv.,* 55 µmol) was added to a solution of (1*S*,4*S*)-7-amino-2'-(((2*R*,7a*S*)-2-fluorotetrahydro-1*H*-pyrrolizin-7a(5*H*)-yl)methoxy)-4'-hydroxy-4-methyl - 3,4,5',8'-tetrahydro-2*H*-spiro[naphthalene-1,7'-pyrano[4,3-*d*]pyrimidine]-8-carbonitrile (22 mg, 46 µmol) and DIPEA (0.08 mL, 10 *equiv.,* 460 µmol) in DMF (0.5 mL). The mixture was stirred at room temperature for 1 hour. (*R*)-2-(methoxymethyl)azetidine (20 µL, 4.0 *equiv.,* 180 µmol) was added to the reaction mixture and the resulting solution was stirred at room temperature for 2 hours. The reaction mixture was concentrated and purified by reverse phase chromatography (12 g C18 silica, 0-100% water/methanol with 0.1% NaH₄CO₃; flowrate: 10 mL/min) to afford (1*S*,4*S*)-7-amino-2'-(((2*R*,7a*S*)-2-fluorotetrahydro-1*H*-pyrrolizin-7a(5*H*)-yl)methoxy)-4'-((*R*)-2-(methoxymethyl)azetidin-1-yl)-4-methyl-3,4,5',8'-tetrahydro-2*H-*spiro[naphthalene-1,7'-pyrano[4,3-*d*]pyrimidine]-8-carbonitrile (15 mg).
LCMS: m/z (ESI) [M+H]⁺ 563.4 t_{R} = 1.57 minutes (Method B)
¹H NMR (400 MHz, DMSO-*d*₆): 7.08 (d, 1 H); 6.73 (d, 1 H); 5.76 (s, 2 H); 5.24 (d, 1H); 4.86 (d, 1 H); 4.67-4.61 (m, 2H); 4.27-4.13 (m, 2 H); 3.89 (d, 1 H); 3.81 (d, 1 H); 3.72 (dd, 1 H); 3.59 (dd, 1 H); 3.28 (s, 3 H); 3.04-2.96 (m, 4 H); 2.77 (d, 2 H); 2.65 (d, 1 H); 2.28-1.67 (m, 11 H); 1.50 (s, 1 H); 1.12 (d, 3 H).
¹⁹F NMR (376 MHz, DMSO): -171.9-172.3 (m).

### Example 147: (1S,4S)-7-Amino-2'-(((2R,7aS)-2-fluorotetrahydro-1H-pyrrolizin-7a(5H)-yl)methoxy)-4-methyl-4'-(6-oxa-1-azaspiro[3.4]octan-1-yl)-3,4,5',8'-tetrahydro-2H-spiro[naphthalene-1,7'-pyrano[4,3-d]pyrimidine]-8-carbonitrile (Compound 598a)

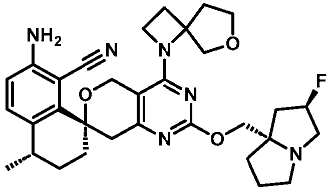

To a solution of (1*S*,4*S*)-7-amino-2'-(((2*R*,7a*S*)-2-fluorotetrahydro-1*H*-pyrrolizin-7a(5*H*)-yl)methoxy)-4'-hydroxy-4-methyl-3,4,5',8'-tetrahydro-2*H*-spiro[naphthalene-1,7'-pyrano[4,3-d]pyrimidine]-8-carbonitrile (70 mg, 150 µmol, 1 *equiv.*) and PyBOP (77 mg, 0.18 mmol, 1.2 *equiv.*) in DMF (1.3 mL) was added DIPEA (250 µL, 1.50 mmol, 10 *equiv.*)*.* The mixture was stirred at room temperature for 1 hour. To this mixture was added 6-oxa-1-azaspiro[3.4]octane hydrochloride (66 mg, 0.44 mmol, 3 *equiv.*) and the resulting mixture was stirred for 1 hour at room temperature followed by 2 hours at 50 °C. The reaction mixture was concentrated and the residue was purified by reverse phase chromatography (0.1% aqueous NH₄HCO₃/acetonitrile) to afford (1*S*,4*S*)-7-amino-2'-(((2*R*,7a*S*)-2-fluorotetrahydro-1*H-*pyrrolizin-7a(5*H*)-yl)methoxy)-4-methyl-4'-(6-oxa-1-azaspiro[3.4]octan-1-yl)-3,4,5',8'-tetrahydro-2*H*-spiro[naphthalene-1,7'-pyrano[4,3-*d*]pyrimidine]-8-carbonitrile (70 mg) as a white solid.
LCMS: m/z (ESI) [M+H]⁺ 575.3, t_{R} = 1.78 minutes (Method K)

### Example 147a: (1S,4S)-7-amino-2'-(((2R,7aS)-2-fluorotetrahydro-1H-pyrrolizin-7a(5H)-yl)methoxy)-4-methyl-4'-((S)-6-oxa-1-azaspiro[3.4]octan-1-yl)-3,4,5',8'-tetrahydro-2H-spiro[naphthalene-1,7'-pyrano[4,3-d]pyrimidine]-8-carbonitrile (Compound 598b)

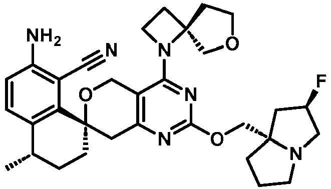

The diastereomers of (1*S*,4*S*)-7-amino-2'-(((2*R*,7a*S*)-2-fluorotetrahydro-1*H*-pyrrolizin-7a(5*H*)-yl)methoxy)-4-methyl-4'-((*RS*)-6-oxa-1-azaspiro[3.4]octan-1-yl)-3,4,5',8'-tetrahydro-2*H*-spiro[naphthalene-1,7'-pyrano[4,3-*d*]pyrimidine]-8-carbonitrile were separated by preparative HPLC (Gemini^{®} 5 µm NX-C18 110 Å, 100 x 30 mm, Flow rate = 45 mL/minute; mobile phase A: 10 mM ammonium bicarbonate; mobile phase B: Acetonitrile; 35% to 55% B over 11 minutes then 55% to 100%B for 5 minutes) to afford to (1*S*,4*S*)-7-amino-2'-(((2*R*,7a*S*)-2-fluorotetrahydro-1*H*-pyrrolizin-7a(5*H*)-yl)methoxy)-4-methyl-4'-((*S*)-6-oxa-1-azaspiro[3.4]octan-1-yl)-3,4,5',8'-tetrahydro-2*H*-spiro[naphthalene-1,7'-pyrano[4,3-*d*]pyrimidine]-8-carbonitrile (**Peak 1**) (22 mg).
LCMS: m/z (ESI) [M+H]⁺ 575.4, t_{R} = 1.52 minutes (Method B)
¹H NMR (400 MHz, DMSO-*d*₆): 7.08 (d, 1 H); 6.73 (d, 1 H); 5.74 (s, 2 H); 5.23 (d, 1 H); 4.80-4.89 (m, 2 H); 4.45 (q, 1 H); 4.25-4.35 (m, 2 H); 3.92-4.01 (m, 2 H); 3.73-3.84 (m, 2 H); 3.66 (d, 1 H); 3.05 (s, 2 H); 2.98 (d, 2 H); 2.77-2.85 (m, 3 H); 2.65 (d, 1 H); 2.37 (d, 2 H); 1.96-2.05 (m, 3 H); 1.84 (s, 5 H); 1.72 (s, 2 H); 1.52 (s, 1 H); 1.13 (d, 3 H).
¹⁹F NMR (376 MHz, DMSO): -171.6--172.0 (m)

### Example 147b: (1S,4S)-7-amino-2'-(((2R,7aS)-2-fluorotetrahydro-1H-pyrrolizin-7a(5H)-yl)methoxy)-4-methyl-4'-((R)-6-oxa-1-azaspiro[3.4]octan-1-yl)-3,4,5',8'-tetrahydro-2H-spiro[naphthalene-1,7'-pyrano[4,3-d]pyrimidine]-8-carbonitrile (Compound 598c)

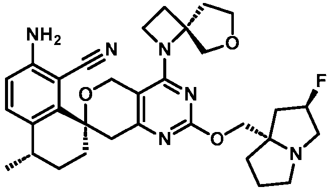

(1*S*,4*S*)-7-amino-2'-(((2*R*,7a*S*)-2-fluorotetrahydro-1*H*-pyrrolizin-7a(5*H*)-yl)methoxy)-4-methyl-4'-((*R*)-6-oxa-1-azaspiro[3.4]octan-1-yl)-3,4,5',8'-tetrahydro-2*H*-spiro[naphthalene-1,7'-pyrano[4,3-*d*]pyrimidine]-8-carbonitrile (26 mg, 45 µmol) was obtained from **Example 147a as Peak 2.**
LCMS: m/z (ESI) [M+H]⁺ 575.4, t_{R} = 1.56 minutes (Method B)
¹H NMR (400 MHz, DMSO-*d*₆): 7.08 (d, 1 H); 6.73 (d, 1 H); 5.74 (s, 2 H); 5.22 (d, 1 H); 4.84 (t, 2 H); 4.31-4.40 (m, 2 H); 4.22 (d, 1 H); 3.97-4.02 (m, 1 H); 3.71-3.90 (m, 4 H); 2.92-3.05 (m, 5 H); 2.78 (d, 2 H); 2.66 (d, 1 H); 2.31-2.42 (m, 2 H); 1.70-2.05 (m, 10 H); 1.53 (s, 1 H); 1.14 (d, 3 H).
¹⁹F NMR (376 MHz, DMSO): -171.6--172.0 (m)

### Example 156: (1S,4S)-7-Amino-2'-(((2R,7aS)-2-fluorotetrahydro-1H-pyrrolizin-7a(5H)-yl)methoxy)-4'-((R)-2-(hydroxymethyl)-2-methylazetidin-1-yl)-4-methyl-3,4,5',8'-tetrahydro-2H-spiro[naphthalene-1,7'-pyrano[4,3-d]pyrimidine]-8-carbonitrile (Compound 597a)

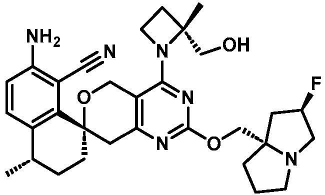

DIPEA (193 mg, 260 µL, 5.96 *equiv.,* 1.49 mmol) was added to a solution of (1*S*,4*S*)-7-amino-2'-(((2*R*,7a*S*)-2-fluorotetrahydro-1*H*-pyrrolizin-7a(5*H*)-yl)methoxy)-4'-hydroxy-4-methyl-3,4,5',8'-tetrahydro-2*H*-spiro[naphthalene-1,7'-pyrano[4,3-d]pyrimidine]-8-carbonitrile (120 mg, 1 *equiv.,* 250 µmol), (2-methylazetidin-2-yl)methanol, HCl (68.9 mg, 2 *equiv.,* 500 µmol), and PyBOP (132.5 mg, 1.20 *equiv.,* 299.6 µmol) in MeCN (1.25 mL). The reaction mixture was stirred at 70 °C for 5.5 hours. The reaction mixture was cooled to room temperature and diluted sequentially with EtOAc (2 mL) and water (2 mL). The aqueous layer was extracted with EtOAc (3 × 3 mL). The combined organic layers were washed with 10% LiCl (3 × 8 mL), the layers were separated, and the organic layer was dried over magnesium sulfate, filtered, and concentrated to a residue which was purified by reverse phase chromatography (XSelect CSH Prep C18 5 µm OBD 19×150, 15-40% MeCN in water + 0.1% ammonium hydroxide) and chiral SFC (Daicel Chiralpak IB N-5, 30 mm ID × 250 mm; Mobile Phase: 30% methanol, 0.25% diethylamine in CO₂, back pressure = 120 bar) to afford (1*S*,4*S*)-7-amino-2'-(((2*R*,7a*S*)-2-fluorotetrahydro-1*H*-pyrrolizin-7a(5*H*)-yl)methoxy)-4'-((*R*)-2-(hydroxymethyl)-2-methylazetidin-1-yl)-4-methyl-3,4,5',8'-tetrahydro-2*H*-spiro[naphthalene-1,7'-pyrano[4,3-*d*]pyrimidine]-8-carbonitrile (13.0 mg).
LCMS: m/z (ESI) [M+H]⁺ 563.5, t_{R} = 1.30 minutes (Daicel Chiralpak IB N-5, 4.6 mm ID × 100 mm; Mobile Phase: 30% methanol, 0.25% diethylamine in CO₂, back pressure = 138 bar)
¹H NMR (400 MHz, CDCl₃) δ 7.08 (d, 1H), 6.67 (d, 1H), 5.94 (s, 1H), 5.26 (d, 1H), 5.03 (d, 1H), 4.87 (d, 1H), 4.49 - 4.36 (m, 3H), 4.24 - 4.14 (m, 1H), 4.01 - 3.88 (m, 3H), 3.52 (s, 1H), 3.35 - 3.07 (m, 4H), 3.00 - 2.68 (m, 3H), 2.33 - 1.75 (m, 13H), 1.24 - 1.21 (m, 3H).

### Example 157: (1S,4S)-7-Amino-4'-(2,2-bis(hydroxymethyl)azetidin-1-yl)-2'-(((2R,7aS)-2-fluorotetrahydro-1H-pyrrolizin-7a(5H)-yl)methoxy)-4-methyl-3,4,5',8'-tetrahydro-2H-spiro[naphthalene-1,7'-pyrano[4,3-d]pyrimidine]-8-carbonitrile (Compound 592a)

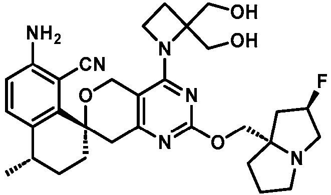

To a solution of (1*S*,4*S*)-7-amino-2'-(((2*R*,7a*S*)-2-fluorotetrahydro-1*H*-pyrrolizin-7a(5*H*)-yl)methoxy)-4'-hydroxy-4-methyl-3,4,5',8'-tetrahydro-2*H*-spiro[naphthalene-1,7'-pyrano[4,3-*d*]pyrimidine]-8-carbonitrile (35.0 mg, 73.0 µmol, 1 *equiv.*) and DIPEA (94.3 mg, 127 µL, 730 µmol, 10 *equiv.*) in DMF (811 µL) was added BOP (38.7 mg, 87.6 µmol, 1.2 *equiv.*)*.* The reaction mixture was stirred at room temperature for 1 hour, then azetidine-2,2-diyldimethanol hydrochloride (33.6 mg, 219 µmol, 3 *equiv.*) was added. The mixture was stirred for an additional hour, after which more azetidine-2,2-diyldimethanol hydrochloride (33.63 mg, 219 µmol, 3 *equiv.*) was added. The resulting reaction mixture was then stirred at 50 °C for 16 hours and concentrated under reduced pressure to give a residue. The residue was purified by reverse phase chromatography (0.1% aqueous NH₄HCO₃/Methanol) to afford (1*S*,4*S*)-7-amino-4'-(2,2-bis(hydroxymethyl)azetidin-1-yl)-2'-(((2*R*,7a*S*)-2-fluorotetrahydro-1*H*-pyrrolizin-7a(5*H*)-yl)methoxy)-4-methyl-3,4,5',8'-tetrahydro-2*H*-spiro[naphthalene-1,7'-pyrano[4,3-*d*]pyrimidine]-8-carbonitrile (6 mg) as a white solid.
LCMS: m/z (ESI) [M+H]⁺ 579.4 t_{R} = 1.31 minutes (Method B)
¹H NMR (400 MHz, *d*-DMSO) δ 7.07 (d, 1H), 6.72 (d, 1H), 5.72 (d, 2H), 5.22 (d, 1H), 5.11-4.91 (br s, 2H), 4.84 (s, 2H), 4.33 - 4.23 (m, 1H), 4.22 - 4.12 (m, 1H), 3.96 (t, 2H), 3.83 - 3.69 (m, 2H), 3.61 - 3.49 (m, 2H), 3.09 - 3.00 (m, 2H), 3.00 - 2.89 (m, 2H), 2.84 - 2.70 (m, 2H), 2.62 (d, 1H), 2.25 - 2.08 (m, 1H), 2.07 - 1.99 (m, 1H), 1.99 - 1.92 (m, 1H), 1.92 - 1.77 (m, 5H), 1.77 - 1.62 (m, 2H), 1.58 - 1.47 (m, 1H), 1.13 (d, 3H).
¹⁹F NMR (376 MHz, DMSO): δ -171.98. (m)

### Example 158: (1S,4S)-7-Amino-2'-(((2R,7aS)-2-fluorotetrahydro-1H-pyrrolizin-7a(5H)-yl)methoxy)-4'-(3-(hydroxymethyl)azetidin-1-yl)-4-methyl-3,4,5',8'-tetrahydro-2H-spiro[naphthalene-1,7'-pyrano[4,3-d]pyrimidine]-8-carbonitrile (Compound 607a)

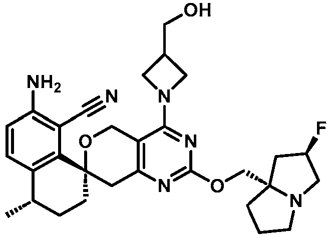

### Step 1: tert-Butyl (tert-butoxycarbonyl)((1S,4S)-8-cyano-2'-(((2R,7aS)-2-fluorotetrahydro-1H-pyrrolizin-7a(5H)-yl)methoxy)-4'-(3-(hydroxymethyl)azetidin-1-yl)-4-methyl-3,4,5',8'-tetrahydro-2H-spiro[naphthalene-1,7'-pyrano[4,3-d]pyrimidin]-7-yl)carbamate

Azetidin-3-ylmethanol hydrochloride (13 mg, 0.11 mmol, 2.0 *equiv.)* was added to a solution of *tert*-butyl (tert-butoxycarbonyl)((1*S*,4*S*)-4'-chloro-8-cyano-2'-(((2*R*,7a*S*)-2-fluorotetrahydro-1*H*-pyrrolizin-7a(5*H*)-yl)methoxy)-4-methyl-3,4,5',8'-tetrahydro-2*H-*spiro[naphthalene-1,7'-pyrano[4,3-*d*]pyrimidin]-7-yl)carbamate (40 mg, 0.054 mmol, 1 *equiv.*) in ethanol (0.11 mL). The reaction mixture was stirred at 70 °C for 1 hour and then cooled to room temperature. EtOAc was added and the resulting suspension was filtered. The filtrate was concentrated to a residue, which was purified by column chromatography (4g silica; 10% (2.5% NH₄OH in 20% MeOH in DCM) in DCM) to afford *tert*-butyl (tert-butoxycarbonyl)((1*S*,4*S*)-8-cyano-2'-((2*R*,7a*S*)-2-fluorotetrahydro-1*H*-pyrrolizin-7a(5*H*)-yl)methoxy)-4'-(3-(hydroxymethyl)azetidin-1-yl)-4-methyl-3,4,5',8'-tetrahydro-2*H*-spiro[naphthalene-1,7'-pyrano[4,3-*d*]pyrimidin]-7-yl)carbamate (19.8 mg) as a white solid.
LCMS: m/z (ESI) [M+H]⁺ 749.5, t_{R} = 1.54 minutes (Method L)

### Step 2: (1S,4S)-7-Amino-2'-(((2R,7aS)-2-fluorotetrahydro-1H-pyrrolizin-7a(5H)-yl)methoxy)-4'-(3-(hydroxymethyl)azetidin-1-yl)-4-methyl-3,4,5',8'-tetrahydro-2H-spiro[naphthalene-1,7'-pyrano[4,3-d]pyrimidine]-8-carbonitrile

TFA (41 µL, 0.53 mmol, 20 *equiv.)* was added to a solution of *tert-*butyl (tert-butoxycarbonyl)((1*S*,4*S*)-8-cyano-2'-(((2*R*,7a*S*)-2-fluorotetrahydro-1*H*-pyrrolizin-7a(5*H*)-yl)methoxy)-4'-(3-(hydroxymethyl)azetidin-1-yl)-4-methyl-3,4,5',8'-tetrahydro-2*H-*spiro[naphthalene-1,7'-pyrano[4,3-*d*]pyrimidin]-7-yl)carbamate (19.8 mg, 0.026 mmol) in DCM (0.13 mL) at 0°C. The resulting mixture was stirred for 30 minutes. The pH was adjusted to pH = 8 with saturated sodium bicarbonate, and the aqueous layer was extracted with DCM (3 x 5 mL). The combined organic layers were washed with saturated sodium bicarbonate (3 x 5 mL), dried over sodium sulfate, filtered, and concentrated to a residue, which was purified by column chromatography (4g silica eluting with 0-10% (2.5% NH₄OH in 20% MeOH in DCM) in DCM) to yield (1*S*,4*S*)-7-amino-2'-(((2*R*,7a*S*)-2-fluorotetrahydro-1*H*-pyrrolizin-7a(5*H*)-yl)methoxy)-4'-(3-(hydroxymethyl)azetidin-1-yl)-4-methyl-3,4,5',8'-tetrahydro-2*H-*spiro[naphthalene-1,7'-pyrano[4,3-*d*]pyrimidine]-8-carbonitrile (12.5 mg) as a yellow solid.
LCMS: m/z (ESI) [M+H]⁺ 549.4, t_{R} = 1.28 minutes (Method L)
¹H NMR (400 MHz, CDCl₃) δ 7.07 (d, 1H), 6.66 (d, 1H), 5.37 - 5.10 (m, 1H), 5.05 - 4.75 (m, 2H), 4.45 (s, 2H), 4.35 - 4.21 (m, 2H), 4.12 - 3.91 (m, 4H), 3.83 (d, 2H), 3.29 - 3.18 (m, 3H), 3.18 - 3.05 (m, 1H), 2.99 - 2.77 (m, 4H), 2.34 - 2.06 (m, 4H), 2.01 - 1.74 (m, 6H), 1.65 - 1.55 (m, 1H), 1.22 (d, 3H).

### Example 234: (1S,4S)-7-Amino-2'-(((2R,7aS)-2-fluorotetrahydro-1H-pyrrolizin-7a(5H)-yl)methoxy)-4'-((2R,4R)-2-(hydroxymethyl)-4-methylazetidin-1-yl)-4-methyl-3,4,5',8'-tetrahydro-2H-spiro[naphthalene-1,7'-pyrano[4,3-d]pyrimidine]-8-carbonitrile (Compound 640a)

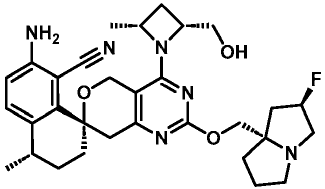

### Step 1: tert-Butyl (2R,4R)-2-(hydroxymethyl)-4-methylazetidine-1-carboxylate

Borane-tetrahydrofuran complex solution (187 mg, 2.17 mL, 1 M, 2.5 *equiv.,* 2.17 mmol) was added to a solution of (2*R*,4*R*)-4-methylazetidine-2-carboxylic acid (100 mg, 1 *equiv.,* 869 µmol) in THF (1.2 mL). The mixture was refluxed for 1.5 hours followed by the addition of potassium hydrogen sulfate (10% solution in water, 1.1 mL). The resulting mixture was refluxed for 15 minutes. The mixture was concentrated under reduced pressure to afford a white solid. Water (1 mL), 1,4-dioxane (1.5 mL), sodium hydroxide (1 M solution in water, 1 mL), and di-*tert*-butyldicarbonate (209 mg, 220 µL, 1.1 *equiv.,* 955 µmol) at 0 °C was added to the residue. The resulting mixture was stirred at 0 °C for 30 minutes then stirred at room temperature for 16 hours. Saturated aqueous ammonium chloride solution was added to the mixture and the resulting reaction mixture was extracted with EtOAc. The combined organic layers were dried with sodium sulfate and concentrated to give *tert*-butyl (2*R,*4*R*)-2-(hydroxymethyl)-4-methylazetidine-1-carboxylate (180 mg), which was used without further purification.

¹H NMR (400 MHz, CDCl₃) δ 4.26 (d, 1H), 4.22 - 4.07 (m, 2H), 3.73 - 3.59 (m, 2H), 2.38 - 2.25 (m, 1H), 1.45 (s, 9H), 1.43 - 1.36 (m, 1H), 1.33 (d, 3H).

### Step 2: ((2R,4R)-4-Methylazetidin-2-yl)methanol

Hydrogen chloride (4 M in dioxane, 161 mg, 1.11 mL, 5 *equiv.,* 4.42 mmol) was added at 0 °C to a solution of *tert*-butyl (2R,4R)-2-(hydroxymethyl)-4-methylazetidine-1-carboxylate (178 mg, 1 *equiv.,* 884 µmol) in DCM (1 mL). The resulting mixture was stirred at room temperature for 1 hour then concentrated to afford crude ((2*R*,4*R*)-4-methylazetidin-2-yl)methanol hydrochloride (153 mg).

¹H NMR (400 MHz, CDCl₃) δ 9.66 (s, 1H), 8.71 (s, 1H), 4.47 (s, 2H), 3.96 (d, 1H), 3.83 - 3.55 (m, 2H), 2.62 - 2.28 (m, 2H), 1.61 (d, 3H).

### Step 3: (1S,4S)-7-Amino-2'-(((2R,7aS)-2-fluorotetrahydro-1H-pyrrolizin-7a(5H)-yl)methoxy)-4'-((2R,4R)-2-(hydroxymethyl)-4-methylazetidin-1-yl)-4-methyl-3,4,5',8'-tetrahydro-2H-spiro[naphthalene-1,7'-pyrano[4,3-d]pyrimidine]-8-carbonitrile

BOP (33 mg, 1.2 *equiv.,* 75 µmol) was added to a solution of (1*S*,4*S*)-7-amino-2'-(((2*R*,7a*S*)-2-fluorotetrahydro-1*H*-pyrrolizin-7a(5*H*)-yl)methoxy)-4'-hydroxy-4-methyl - 3,4,5',8'-tetrahydro-2*H*-spiro[naphthalene-1,7'-pyrano[4,3-d]pyrimidine]-8-carbonitrile (30 mg, 1 *equiv.,* 63 µmol) and DIPEA (0.13 mL, 12 *equiv.,* 0.75 mmol) in MeCN (0.5 mL). The mixture was stirred at room temperature for 1 hour. ((2*R*,4*R*)-4-methylazetidin-2-yl)methanol hydrochloride (39 mg, 4.5 *equiv.,* 0.28 mmol) in MeCN (0.5 mL) was added to the reaction mixture and stirred at 50 °C for 3 hours. The reaction mixture was concentrated to a residue and purified by reverse phase chromatography (12 g biotage C18, flow rate 10 mL/min, 0-100% water/methanol) to provide (1*S*,4*S*)-7-amino-2'-(((2*R*,7a*S*)-2-fluorotetrahydro-1H-pyrrolizin-7a(5H)-yl)methoxy)-4'-((2*R*,4*R*)-2-(hydroxymethyl)-4-methylazetidin-1-yl)-4-methyl-3,4,5',8'-tetrahydro-2H-spiro[naphthalene-1,7'-pyrano[4,3-*d*]pyrimidine]-8-carbonitrile (20.2 mg).
LCMS: m/z (ESI) [M+H]⁺ 563.4 t_{R} = 1.49 minutes (Method B)
¹H NMR (400 MHz, CD₃OD) δ 7.12 (d, 1H), 6.77 (d, 1H), 5.27 (d, 1H), 5.03 - 4.93 (m, 1H), 4.78 - 4.64 (m, 1H), 4.62 - 4.46 (m, 1H), 4.14 - 4.00 (m, 2H), 3.92 (dd, 1H), 3.79 (dd, 1H), 3.25 - 3.09 (m, 4H), 3.04 - 2.93 (m, 1H), 2.92 - 2.77 (m, 2H), 2.77 - 2.59 (m, 1H), 2.36 - 1.61 (m, 12H), 1.57 (d, 3H), 1.23 (d, 3H).

### Example 376: (S)-7-amino-2'-(((2R,7aS)-2-fluorotetrahydro-1H-pyrrolizin-7a(5H)-yl)methoxy)-4'-((R)-2-(hydroxymethyl)azetidin-1-yl)-3,4,5',8'-tetrahydro-2H-spiro[naphthalene-1,7'-pyrano[4,3-d]pyrimidine]-8-carbonitrile (Compound 751a)

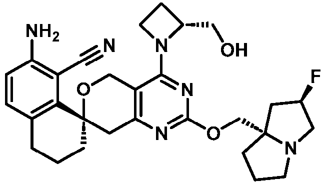

### Step 1: (S)-7-amino-2'-(((2R,7aS)-2-fluorotetrahydro-1H-pyrrolizin-7a(5H)-yl)methoxy)-4'-((R)-2-(hydroxymethyl)azetidin-1-yl)-3,4,5',8'-tetrahydro-2H-spiro[naphthalene-1,7'-pyrano[4,3-d]pyrimidine]-8-carbonitrile

BOP (143 mg, 0.32 mmol) was added to a solution of (*S*)-7-amino-2'-(((2*R*,7a*S*)-2-fluorotetrahydro-1*H*-pyrrolizin-7a(5*H*)-yl)methoxy)-4'-hydroxy-3,4,5',8'-tetrahydro-2*H-*spiro[naphthalene-1,7'-pyrano[4,3-*d*]pyrimidine]-8-carbonitrile (100 mg, 0.215 mmol) and DIPEA (374 µL, 2.15 mmol) in MeCN (2 mL). The mixture was stirred at room temperature for 1 hour followed by the addition of (*R*)-azetidin-2-ylmethanol (47 mg, 0.54 mmol). The reaction mixture was stirred at 50 °C for 1 hour then concentrated to a residue which was purified by reverse phase chromatography (26 g Biotage C18, liquid injection with DMSO, flow rate 10 mL/min, 0-100% 10 mM ammonium bicarbonate/MeCN) to afford (*S*)-7-amino-2'-(((2*R*,7a*S*)-2-fluorotetrahydro-1*H*-pyrrolizin-7a(5*H*)-yl)methoxy)-4'-((*R*)-2-(hydroxymethyl)azetidin-1-yl)-3,4,5',8'-tetrahydro-2*H*-spiro[naphthalene-1,7'-pyrano[4,3-*d*]pyrimidine]-8-carbonitrile (80 mg).
LCMS: m/z (ESI) [M+H]⁺ 535.4, t_{R} = 3.62 minutes (Method I)
¹H NMR (400 MHz, CDCl₃) δ 7.02 (d, 1H), 6.63 (d, 1H), 5.69 (s, 1H), 5.25 (d, 1H), 5.08 (d, 1H), 4.83 - 4.67 (m, 2H), 4.47 (s, 2H), 4.42 - 4.33 (m, 1H), 4.22 - 4.12 (m, 1H), 4.09 - 3.90 (m, 2H), 3.86 - 3.76 (m, 1H), 3.76 - 3.66 (m, 1H), 3.37 - 3.06 (m, 4H), 3.00 - 2.89 (m, 1H), 2.82 (d, 1H), 2.67 (d, 2H), 2.56 - 2.38 (m, 1H), 2.38 - 2.25 (m, 1H), 2.17 (d, 2H), 2.12 - 1.98 (m, 3H), 1.97 - 1.78 (m, 3H), 1.70 (d, 2H).
¹⁹F NMR (376 MHz, CDCl₃) δ -171.78 - -172.40 (m).

The following examples were synthesized using methods similar to those described in the examples above:

| **Example No.** | **Compound No.** | **LCMS (ESI)** | **Method** |
|---|---|---|---|
| **72** | **535a** | LCMS: m/z (ESI) [M+H]⁺ 519.4, t_{R} = 3.95 minutes | Method I |
| **73** | **534a** | LCMS: m/z (ESI) [M+H]⁺ 505.4, t_{R} = 1.50 minutes | Method B |
| **74** | **526c** | LCMS: m/z (ESI) [M+H]⁺ 626.5, t_{R} = 3.61 minutes | Method I |
| **75** | **526b** | LCMS: m/z (ESI) [M+H]⁺ 626.5, t_{R} = 3.61 minutes | Method I |
| **76** | **533a** | LCMS: m/z (ESI) [M+H]⁺ 565.4, t_{R} = 1.59 minutes | Method B |
| **77** | **538a** | LCMS: m/z (ESI) [M+H]⁺ 561.4, t_{R} = 1.43 minutes | Method B |
| **78** | **537a** | LCMS: m/z (ESI) [M+H]⁺ 548.5, t_{R} = 1.41 minutes | Method B |
| **79** | **536a** | LCMS: m/z (ESI) [M+H]⁺ 708.4, t_{R} = 4.04 minutes | Method I |
| **80** | **544a** | LCMS: m/z (ESI) [M+H]⁺ 597.4, t_{R} = 1.54 minutes | Method B |
| **81** | **543a** | LCMS: m/z (ESI) [M+H]⁺ 718.4, t_{R} = 1.40 minutes | Method B |
| **82** | **542a** | LCMS: m/z (ESI) [M+H]⁺ 582.4, t_{R} = 1.44 minutes | Method B |
| **83** | **541a** | LCMS: m/z (ESI) [M+H]⁺ 571.4, t_{R} = 1.44 minutes | Method B |
| **84** | **540a** | LCMS: m/z (ESI) [M+H]⁺ 523.4, t_{R} = 1.47 minutes | Method B |
| **85** | **539a** | LCMS: m/z (ESI) [M+H]⁺ 590.4, t_{R} = 3.67 minutes | Method I |
| **86** | **552a** | LCMS: m/z (ESI) [M+H]⁺ 574.4, t_{R} = 1.22 minutes | Method B |
| **87** | **551a** | LCMS: m/z (ESI) [M+H]⁺ 574.3, t_{R} = 1.25 minutes | Method B |
| **88** | **549a** | LCMS: m/z (ESI) [M+H]⁺ 563.4, t_{R} = 1.39 minutes | Method B |
| **89** | **548a** | LCMS: m/z (ESI) [M+H]⁺ 567.3, t_{R} = 1.61 minutes | Method B |
| **90** | **547a** | LCMS: m/z (ESI) [M+H]⁺ 547.3, t_{R} = 1.40 minutes | Method B |
| **91** | **546a** | LCMS: m/z (ESI) [M+H]⁺ 722.4, t_{R} = 4.18 minutes | Method I |
| **92** | **545a** | LCMS: m/z (ESI) [M+H]⁺ 523.4, t_{R} = 1.36 minutes | Method B |
| **93** | **554a** | LCMS: m/z (ESI) [M+H]⁺ 631.4/631.5, t_{R} = 1.25/1.31 minutes | Method B |
| **94** | **553a** | LCMS: m/z (ESI) [M+H]⁺ 519.4, t_{R} = 1.56 minutes | Method B |
| **95** | **565a** | LCMS: m/z (ESI) [M+H]⁺ 633.3, t_{R} = 1.33 minutes | Method L |
| **96** | **564a** | LCMS: m/z (ESI) [M+H]⁺ 633.3, t_{R} = 1.36 and 1.29 minutes | Method L |
| **97** | **563a** | LCMS: m/z (ESI) [M+H]⁺ 632.4; t_{R} = 1.76 and 1.78 minutes | Method K |
| **98** | **562a** | LCMS: m/z (ESI) [M+H]⁺ 629.5, t_{R} = 1.65 minutes | Method K |
| **99** | **561a** | LCMS: m/z (ESI) [M+H]⁺ 596.4, t_{R} = 1.48 minutes | Method B |
| **100** | **560a** | LCMS: m/z (ESI) [M+H]⁺ 537.4, t_{R} = 1.55 minutes | Method B |
| **101** | **559a** | LCMS: m/z (ESI) [M+H]⁺ 633.3, t_{R} = 1.29 minutes | Method C |
| **102** | **554c** | LCMS: m/z (ESI) [M+H]⁺ 631.5, t_{R} = 1.30 minutes | Method B |
| **103** | **554b** | LCMS: m/z (ESI) [M+H]⁺ 631.4, t_{R} = 1.25 minutes | Method B |
| **104** | **536c** | LCMS: m/z (ESI) [M+H]⁺ 708.4, t_{R} = 3.93 minutes | Method I |
| **105** | **536b** | LCMS: m/z (ESI) [M+H]⁺ 708.4, t_{R} = 3.93 minutes | Method I |
| **106** | **558a** | LCMS: m/z (ESI) [M+H]⁺ 519.4, t_{R} = 3.89 minutes | Method I |
| **107** | **555a** | LCMS: m/z (ESI) [M+H]⁺ 589.3, t_{R} = 1.18 minutes | Method C |
| **108** | **530a** | LCMS: m/z (ESI) [M+H]⁺ 684.5, t_{R} = 1.32 minutes | Method L |
| **109** | **528a** | LCMS: m/z (ESI) [M+H]⁺ 616.3, t_{R} = 1.31 minutes | Method C |
| **110** | **531a** | LCMS: m/z (ESI) [M+H]+ 688.5 t_{R} = 1.84 minutes | Method K |
| **111** | **532a** | LCMS: m/z (ESI) [M+H]+ 629.4 t_{R} = 1.30 minutes | Method C |
| **117** | **584a** | LCMS: m/z (ESI) [M+H]⁺ 702.5, t_{R} = 4.15 minutes | Method I |
| **118** | **583a** | LCMS: m/z (ESI) [M+H]⁺ 559.4, t_{R} = 1.82 minutes | Method B |
| **119** | **566a** | LCMS: m/z (ESI) [M+H]⁺ 544.4, t_{R} = 1.49 minutes | Method B |
| **120** | **567b** | LCMS: m/z (ESI) [M+H]⁺ 562.4, t_{R} = 1.31 minutes | Method B |
| **121** | **567a** | LCMS: m/z (ESI) [M+H]⁺ 562.4, t_{R} = 1.21 minutes | Method B |
| **122** | **566b** | LCMS: m/z (ESI) [M+H]⁺ 544.4, t_{R} = 1.49 minutes | Method B |
| **123** | **573a** | LCMS: m/z (ESI) [M+H]⁺ 561.4, t_{R} = 1.47 minutes | Method B |
| **124** | **568b** | LCMS: m/z (ESI) [M+H]⁺ 576.4, t_{R} = 1.29 minutes | Method B |
| **125** | **569a** | LCMS: m/z (ESI) [M+H]⁺ 558.4, t_{R} = 1.53 minutes | Method B |
| **126** | **582a** | LCMS: m/z (ESI) [M+H]⁺ 602.5, t_{R} = 1.33 minutes | Method B |
| **127** | **571a** | LCMS: m/z (ESI) [M+H]⁺ 575.4, t_{R} = 1.58 minutes | Method B |
| **128** | **574a** | LCMS: m/z (ESI) [M+H]⁺ 704.4, t_{R} = 1.52 minutes | Method B |
| **129** | **568a** | LCMS: m/z (ESI) [M+H]⁺ 576.4, t_{R} = 1.29 minutes | Method B |
| **130** | **572a** | LCMS: m/z (ESI) [M+H]⁺ 570.4, t_{R} = 1.53 minutes | Method B |
| **131** | **570a** | LCMS: m/z (ESI) [M+H]⁺ 575.4, t_{R} = 1.49 minutes | Method B |
| **132** | **574c** | LCMS: m/z (ESI) [M+H]⁺ 704.4, t_{R} = 1.52 minutes | Method B |
| **133** | **574b** | LCMS: m/z (ESI) [M+H]⁺ 704.4, t_{R} = 1.52 minutes | Method B |
| **134** | **591a** | LCMS: m/z (ESI) [M+H]⁺ 688.5, t_{R} = 3.97 minutes | Method I |
| **135** | **590a** | LCMS: m/z (ESI) [M+H]⁺ 706.5, t_{R} = 4.11 minutes | Method I |
| **136** | **588a** | LCMS: m/z (ESI) [M+H]⁺ 674.3 | Method L |
| **137** | **587a** | LCMS: m/z (ESI) [M+H]⁺ 574.3, t_{R} = 1.21 minutes | Method L |
| **138** | **586a** | LCMS: m/z (ESI) [M+H]⁺ 555.5, t_{R} = 1.87 minutes | Method K |
| **139** | **581a** | LCMS: m/z (ESI) [M+H]⁺ 630.4, t_{R} = 1.27 minutes | Method C |
| **140** | **580a** | LCMS: m/z (ESI) [M+H]⁺ 561.3, t_{R} = 1.16 minutes | Method C |
| **141** | **579a** | LCMS: m/z (ESI) [M+H]⁺ 563.2, t_{R} = 1.27 minutes | Method C |
| **142** | **577a** | LCMS: m/z (ESI) [M+H]⁺ 584.4, t_{R} = 1.55 minutes | Method B |
| **143** | **575b** | LCMS: m/z (ESI) [M+H]⁺ 590.4, t_{R} = 1.39 minutes | Method B |
| **144** | **575a** | LCMS: m/z (ESI) [M+H]⁺ 590.4, t_{R} = 1.32 minutes | Method B |
| **145** | **599b** | LCMS: m/z (ESI) [M+H]⁺ 573.5, t_{R} = 1.74 minutes | Method K |
| **146** | **599a** | LCMS: m/z (ESI) [M+H]⁺ 573.4, t_{R} = 1.72 minutes | Method K |
| **148** | **597b** | LCMS: m/z (ESI) [M+H]⁺ 563.4, t_{R} = 1.71 minutes | Method K |
| **149** | **596a** | LCMS: m/z (ESI) [M+H]⁺ 690.5, t_{R} = 1.29 minutes | Method B |
| **150** | **578b** | LCMS: m/z (ESI) [M+H]⁺ 563.4, t_{R} = 1.59 minutes | Method B |
| **151** | **576b** | LCMS: m/z (ESI) [M+H]⁺ 549.4, t_{R} = 1.46 minutes | Method B |
| **152** | **595b** | LCMS: m/z (ESI) [M+H]⁺ 533.4, t_{R} = 1.43 minutes | Method L |
| **153** | **595a** | LCMS: m/z (ESI) [M+H]⁺ 533.2, t_{R} = 1.46 minutes | Method L |
| **154** | **594a** | LCMS: m/z (ESI) [M+H]⁺ 692.4, t_{R} = 3.93 minutes | Method I |
| **155** | **593a** | LCMS: m/z (ESI) [M+H]⁺ 574.4, t_{R} = 1.27 minutes | Method B |
| **159** | **632a** | LCMS: m/z (ESI) [M+H]⁺ 688.4, t_{R} = 1.38 minutes | Method L |
| **160** | **631a** | LCMS: m/z (ESI) [M+H]⁺ 708.42, t_{R} = 1.37 minutes | Method L |
| **161** | **630a** | LCMS: m/z (ESI) [M+H]⁺ 686.5, t_{R} = 1.45 minutes | Method B |
| **162** | **629a** | LCMS: m/z (ESI) [M+H]⁺ 682.5, t_{R} = 3.87 minutes | Method I |
| **163** | **628a** | LCMS: m/z (ESI) [M+H]⁺ 716.5, t_{R} = 4.08 minutes | Method I |
| **164** | **627a** | LCMS: m/z (ESI) [M+H]⁺ 547.3, t_{R} = 1.48 minutes | Method B |
| **165** | **626a** | LCMS: m/z (ESI) [M+H]⁺ 577.4, t_{R} = 1.44 minutes | Method B |
| **166** | **625a** | LCMS: m/z (ESI) [M+H]⁺ 561.5, t_{R} = 1.47 minutes | Method B |
| **167** | **624b** | LCMS: m/z (ESI) [M+H]⁺ 593.4, t_{R} = 1.43 minutes | Method B |
| **168** | **624a** | LCMS: m/z (ESI) [M+H]⁺ 593.4, t_{R} = 1.49 minutes | Method B |
| **169** | **623a** | LCMS: m/z (ESI) [M+H]⁺ 547.4, t_{R} = 1.76 minutes | Method B |
| **170** | **622a** | LCMS: m/z (ESI) [M+H]⁺ 579.4, t_{R} = 1.32 minutes | Method B |
| **171** | **612b** | LCMS: m/z (ESI) [M+H]⁺ 533.3, t_{R} = 1.69 minutes | Method B |
| **172** | **621b** | LCMS: m/z (ESI) [M+H]⁺ 586.3, t_{R} = 1.32 minutes | Method C |
| **173** | **621a** | LCMS: m/z (ESI) [M+H]⁺ 586.4, t_{R} = 1.87 minutes | Method K |
| **174** | **620a** | LCMS: m/z (ESI) [M+H]⁺ 577.4, t_{R} = 1.43 minutes | Method L |
| **175** | **619a** | LCMS: m/z (ESI) [M+H]⁺ 602.4, t_{R} = 1.95 minutes | Method K |
| **176** | **618a** | LCMS: m/z (ESI) [M+H]⁺ 563.4, t_{R} = 1.20 minutes | Method C |
| **177** | **617a** | LCMS: m/z (ESI) [M+H]⁺ 674.5, t_{R} = 4.38 minutes | Method I |
| **178** | **616a** | LCMS: m/z (ESI) [M+H]⁺ 600.5, t_{R} = 1.28 minutes | Method B |
| **179** | **615a** | LCMS: m/z (ESI) [M+H]⁺ 634.5, t_{R} = 1.34 minutes | Method B |
| **180** | **614a** | LCMS: m/z (ESI) [M+H]⁺ 718.5, t_{R} = 4.40 minutes | Method I |
| **181** | **613a** | LCMS: m/z (ESI) [M+H]⁺ 547.4, t_{R} = 1.78 minutes | Method B |
| **182** | **612a** | LCMS: m/z (ESI) [M+H]⁺ 533.4, t_{R} = 1.71 minutes | Method B |
| **183** | **611b** | LCMS: m/z (ESI) [M+H]⁺ 585.3, t_{R} = 1.94 minutes | Method K |
| **184** | **611a** | LCMS: m/z (ESI) [M+H]⁺ 585.4, t_{R} = 1.94 minutes | Method K |
| **185** | **610a** | LCMS: m/z (ESI) [M+H]⁺ 563.4, t_{R} = 1.29 minutes | Method C |
| **186** | **600b** | LCMS: m/z (ESI) [M+H]⁺ 603.3, t_{R} = 2.14 minutes | Method L |
| **187** | **609b** | LCMS: m/z (ESI) [M+H]⁺ 593.4, t_{R} = 4.00 minutes | Method I |
| **188** | **609a** | LCMS: m/z (ESI) [M+H]⁺ 593.4, t_{R} = 3.93 minutes | Method I |
| **189** | **608b** | LCMS: m/z (ESI) [M+H]⁺ 589.4, t_{R} = 1.86 minutes | Method K |
| **190** | **608a** | LCMS: m/z (ESI) [M+H]⁺ 589.4, t_{R} = 1.83 minutes | Method K |
| **191** | **606b** | LCMS: m/z (ESI) [M+H]⁺ 575.4, t_{R} = 4.41 minutes | Method I |
| **192** | **606a** | LCMS: m/z (ESI) [M+H]⁺ 575.4, t_{R} = 4.46 minutes | Method I |
| **193** | **601a** | LCMS: m/z (ESI) [M+H]⁺ 587.5, t_{R} = 1.94 minutes | Method B |
| **194** | **600a** | LCMS: m/z (ESI) [M+H]⁺ 603.4, t_{R} = 2.16 minutes | Method K |
| **195** | **556b** | LCMS: m/z (ESI) [M+H]⁺ 561.4, t_{R} = 1.73 minutes | Method K |
| **196** | **605c** | LCMS: m/z (ESI) [M+H]⁺ 562.4, t_{R} = 1.18 minutes | Method B |
| **197** | **605b** | LCMS: m/z (ESI) [M+H]⁺ 562.4, t_{R} = 1.16 minutes | Method B |
| **198** | **605a** | LCMS: m/z (ESI) [M+H]⁺ 562.3, t_{R} = 3.09 minutes | Method I |
| **199** | **604a** | LCMS: m/z (ESI) [M+H]⁺ 705.5, t_{R} = 3.13 minutes | Method I |
| **200** | **603b** | LCMS: m/z (ESI) [M+H]⁺ 573.4, t_{R} = 1.51 minutes | Method B |
| **201** | **603a** | LCMS: m/z (ESI) [M+H]⁺ 573.4, t_{R} = 1.48 minutes | Method B |
| **202** | **602a** | LCMS: m/z (ESI) [M+H]⁺ 716.4, t_{R} = 1.49 minutes | Method B |
| **203** | **604c** | LCMS: m/z (ESI) [M+H]⁺ 705.4, t_{R} = 3.11 minutes | Method I |
| **204** | **654b** | LCMS: m/z (ESI) [M+H]⁺ 587.4, t_{R} = 4.26 minutes | Method I |
| **205** | **654a** | LCMS: m/z (ESI) [M+H]⁺ 587.5, t_{R} = 4.30 minutes | Method I |
| **206** | **653a** | LCMS: m/z (ESI) [M+H]⁺ 563.4, t_{R} = 1.53 minutes | Method B |
| **207** | **652a** | LCMS: m/z (ESI) [M+H]⁺ 575.4, t_{R} = 4.60 minutes | Method I |
| **208** | **651a** | LCMS: m/z (ESI) [M+H]⁺ 592.4, t_{R} = 3.51 minutes | Method I |
| **209** | **650a** | LCMS: m/z (ESI) [M+H]⁺ 574.4, t_{R} = 3.98 minutes | Method B |
| **210** | **649b** | LCMS: m/z (ESI) [M+H]⁺ 563.5, t_{R} = 1.40 minutes | Method B |
| **211** | **649a** | LCMS: m/z (ESI) [M+H]⁺ 563.5, t_{R} = 1.41 minutes | Method B |
| **212** | **648b** | LCMS: m/z (ESI) [M+H]⁺ 551.4, t_{R} = 1.59 minutes | Method B |
| **213** | **648a** | LCMS: m/z (ESI) [M+H]⁺ 551.4, t_{R} = 1.55 minutes | Method B |
| **214** | **647b** | LCMS: m/z (ESI) [M+H]⁺ 549.4, t_{R} = 1.36 minutes | Method B |
| **215** | **647a** | LCMS: m/z (ESI) [M+H]⁺ 549.4, t_{R} = 1.36 minutes | Method B |
| **216** | **646a** | LCMS: m/z (ESI) [M+H]⁺ 575.5, t_{R} = 1.52 minutes | Method B |
| **217** | **645a** | LCMS: m/z (ESI) [M+H]⁺ 558.5, t_{R} = 1.55 minutes | Method B |
| **218** | **644a** | LCMS: m/z (ESI) [M+H]⁺ 575.5, t_{R} = 1.49 minutes | Method B |
| **219** | **643b** | LCMS: m/z (ESI) [M+H]⁺ 547.4, t_{R} = 1.75 minutes | Method B |
| **220** | **643a** | LCMS: m/z (ESI) [M+H]⁺ 547.4, t_{R} = 1.75 minutes | Method B |
| **221** | **641a** | LCMS: m/z (ESI) [M+H]⁺ 722.5, t_{R} = 1.50 minutes | Method I |
| **222** | **639a** | LCMS: m/z (ESI) [M+H]⁺ 577.4, t_{R} = 1.60 minutes | Method B |
| **223** | **636a** | LCMS: m/z (ESI) [M+H]⁺ 577.4, t_{R} = 4.06 minutes | Method I |
| **224** | **613c** | LCMS: m/z (ESI) [M+H]⁺ 547.4, t_{R} = 1.77 minutes | Method B |
| **225** | **635a** | LCMS: m/z (ESI) [M+H]⁺ 563.4, t_{R} = 4.15 minutes | Method I |
| **226** | **613b** | LCMS: m/z (ESI) [M+H]⁺ 547.4, t_{R} = 1.79 minutes | Method B |
| **227** | **604b** | LCMS: m/z (ESI) [M+H]⁺ 705.5, t_{R} = 3.13 minutes | Method I |
| **228** | **614c** | LCMS: m/z (ESI) [M+H]⁺ 718.5, t_{R} = 4.47 minutes | Method I |
| **229** | **614b** | LCMS: m/z (ESI) [M+H]⁺ 718.5, t_{R} = 4.44 minutes | Method I |
| **230** | **637a** | LCMS: m/z (ESI) [M+H]⁺ 577.419, t_{R} = 1.184 minutes | Method C |
| **231** | **638a** | LCMS: m/z (ESI) [M+H]⁺ 575.389, t_{R} = 1.263 minutes | Method C |
| **232** | **642a** | LCMS: m/z (ESI) [M+H]⁺ 589.440, t_{R} = 1.253 minutes | Method C |
| **233** | **637b** | LCMS: m/z (ESI) [M+H]⁺ 577.466, t_{R} = 1.217 minutes | Method C |
| **235** | **610b** | LCMS: m/z (ESI) [M+H]⁺ 563.367, t_{R} = 1.163 minutes | Method C |
| **236** | **651b** | LCMS: m/z (ESI) [M+H]⁺ 592.5, t_{R} = 1.21 minutes | Method B |
| **237** | **654c** | LCMS: m/z (ESI) [M+H]⁺ 587.4, t_{R} = 4.07 minutes | Method I |
| **238** | **654d** | LCMS: m/z (ESI) [M+H]⁺ 587.4, t_{R} = 4.12 minutes | Method I |
| **239** | **655a** | LCMS: m/z (ESI) [M+H]⁺ 561.4, t_{R} = 1.83 minutes | Method B |
| **240** | **656a** | LCMS: m/z (ESI) [M+H]⁺ 576.5, t_{R} = 4.13 minutes | Method I |
| **241** | **657a** | LCMS: m/z (ESI) [M+H]⁺ 577.368, t_{R} = 1.248 minutes | Method C |
| **242** | **641c** | LCMS: m/z (ESI) [M+H]⁺ 722.6, t_{R} = 1.90 minutes | Method E |
| **243** | **658a** | LCMS: m/z (ESI) [M+H]⁺ 533.5, t_{R} = 1.67 minutes | Method B |
| **244** | **659a** | LCMS: m/z (ESI) [M+H]⁺ 549.4, t_{R} = 1.39 minutes | Method B |
| **245** | **576c** | LCMS: m/z (ESI) [M+H]⁺ 549.5, t_{R} = 1.46 minutes | Method B |
| **246** | **576d** | LCMS: m/z (ESI) [M+H]⁺ 549.5, t_{R} = 1.43 minutes | Method B |
| **247** | **660a** | LCMS: m/z (ESI) [M+H]⁺ 545.4, t_{R} = 1.72 minutes | Method B |
| **248** | **661a** | LCMS: m/z (ESI) [M+H]⁺ 558.4, t_{R} = 4.07 minutes | Method M |
| **249** | **661b** | LCMS: m/z (ESI) [M+H]⁺ 558.4, t_{R} = 4.02 minutes | Method I |
| **250** | **650b** | LCMS: m/z (ESI) [M+H]⁺ 574.4, t_{R} = 3.58 minutes | Method M |
| **251** | **650c** | LCMS: m/z (ESI) [M+H]⁺ 574.4, t_{R} = 3.68 minutes | Method B |
| **252** | **652b** | LCMS: m/z (ESI) [M+H]⁺ 575.4, t_{R} = 4.08 minutes | Method I |
| **253** | **652c** | LCMS: m/z (ESI) [M+H]⁺ 575.4, t_{R} = 4.11 minutes | Method I |
| **254** | **662a** | LCMS: m/z (ESI) [M+H]⁺ 601.4, t_{R} = 4.02 minutes | Method I |
| **255** | **662b** | LCMS: m/z (ESI) [M+H]⁺ 601.5, t_{R} = 4.08 minutes | Method I |
| **256** | **662c** | LCMS: m/z (ESI) [M+H]⁺ 601.6, t_{R} = 4.27 minutes | Method I |
| **257** | **662d** | LCMS: m/z (ESI) [M+H]⁺ 601.5, t_{R} = 4.33 minutes | Method I |
| **258** | **653b** | LCMS: m/z (ESI) [M+H]⁺ 563.5, t_{R} = 1.49 minutes | Method B |
| **259** | **641b** | LCMS: m/z (ESI) [M+H]⁺ 722.2, t_{R} = 2.36 minutes | Method K |
| **260** | **706d** | LCMS: m/z (ESI) [M+H]⁺ 506.5, t_{R} = 1.88 minutes | Method K |
| **261** | **706c** | LCMS: m/z (ESI) [M+H]⁺ 506.5, t_{R} = 1.87 minutes | Method K |
| **262** | **706b** | LCMS: m/z (ESI) [M+H]⁺ 506.6, t_{R} = 1.89 minutes | Method K |
| **263** | **706a** | LCMS: m/z (ESI) [M+H]⁺ 506.4, t_{R} = 1.89 minutes | Method K |
| **264** | **702b** | LCMS: m/z (ESI) [M+H]⁺ 563.4, t_{R} = 3.82 minutes | Method I |
| **265** | **680c** | LCMS: m/z (ESI) [M+H]⁺ 589.4, t_{R} = 1.48 minutes | Method B |
| **266** | **689b** | LCMS: m/z (ESI) [M+H]⁺ 577.5, t_{R} = 1.46 minutes | Method B |
| **267** | **705a** | LCMS: m/z (ESI) [M+H]⁺ 629.4, t_{R} = 1.64 minutes | Method B |
| **268** | **704a** | LCMS: m/z (ESI) [M+H]⁺ 617.5, t_{R} = 1.75 minutes | Method B |
| **269** | **703a** | LCMS: m/z (ESI) [M+H]⁺ 567.4, t_{R} = 1.38 minutes | Method B |
| **270** | **702a** | LCMS: m/z (ESI) [M+H]⁺ 563.4, t_{R} = 3.62 minutes | Method I |
| **271** | **701a** | LCMS: m/z (ESI) [M+H]⁺ 614.3, t_{R} = 1.54 minutes | Method B |
| **272** | **700a** | LCMS: m/z (ESI) [M+H]⁺ 575.5, t_{R} = 1.47 minutes | Method B |
| **273** | **699a** | LCMS: m/z (ESI) [M+H]⁺ 604.4, t_{R} = 1.44 minutes | Method B |
| **274** | **698a** | LCMS: m/z (ESI) [M+H]⁺ 603.4, t_{R} = 1.71 minutes | Method B |
| **275** | **697a** | LCMS: m/z (ESI) [M+H]⁺ 618.5, t_{R} = 1.49 minutes | Method B |
| **276** | **696a** | LCMS: m/z (ESI) [M+H]⁺ 587.4, t_{R} = 1.68 minutes | Method B |
| **277** | **695a** | LCMS: m/z (ESI) [M+H]⁺ 591.4, t_{R} = 1.72 minutes | Method B |
| **278** | **694a** | LCMS: m/z (ESI) [M+H]⁺ 563.4, t_{R} = 1.66 minutes | Method B |
| **279** | **693a** | LCMS: m/z (ESI) [M+H]⁺ 575.4, t_{R} = 1.53 minutes | Method B |
| **280** | **692a** | LCMS: m/z (ESI) [M+H]⁺ 589.5, t_{R} = 1.56 minutes | Method B |
| **281** | **680b** | LCMS: m/z (ESI) [M+H]⁺ 589.4, t_{R} = 1.46 minutes | Method B |
| **282** | **691a** | LCMS: m/z (ESI) [M+H]⁺ 599.4, t_{R} = 1.45 minutes | Method B |
| **283** | **690a** | LCMS: m/z (ESI) [M+H]⁺ 599.5, t_{R} = 1.50 minutes | Method B |
| **284** | **689a** | LCMS: m/z (ESI) [M+H]⁺ 577.5, t_{R} = 1.42 minutes | Method B |
| **285** | **688a** | LCMS: m/z (ESI) [M+H]⁺ 591.5, t_{R} = 4.68 minutes | Method I |
| **286** | **687a** | LCMS: m/z (ESI) [M+H]⁺ 599.5, t_{R} = 3.73 minutes | Method I |
| **287** | **686a** | LCMS: m/z (ESI) [M+H]⁺ 577.4, t_{R} = 3.96 minutes | Method I |
| **288** | **685a** | LCMS: m/z (ESI) [M+H]⁺ 561.4, t_{R} = 4.05 minutes | Method I |
| **289** | **684a** | LCMS: m/z (ESI) [M+H]⁺ 563.4, t_{R} = 4.02 minutes | Method I |
| **290** | **628c** | LCMS: m/z (ESI) [M+H]⁺ 716.5, t_{R} = 2.21 minutes | Method K |
| **291** | **628b** | LCMS: m/z (ESI) [M+H]⁺ 716.8, t_{R} = 1.89 minutes | Method K |
| **292** | **683a** | LCMS: m/z (ESI) [M+H]⁺ 591.4, t_{R} = 1.67 minutes | Method B |
| **293** | **682a** | LCMS: m/z (ESI) [M+H]⁺ 602.4, t_{R} = 1.39 minutes | Method B |
| **294** | **681a** | LCMS: m/z (ESI) [M+H]⁺ 561.4, t_{R} = 1.50 minutes | Method B |
| **295** | **680a** | LCMS: m/z (ESI) [M+H]⁺ 589.5, t_{R} = 3.95 minutes | Method I |
| **296** | **679a** | LCMS: m/z (ESI) [M+H]⁺ 589.4, t_{R} = 1.69 minutes | Method B |
| **297** | **668d** | LCMS: m/z (ESI) [M+H]⁺ 549.4, t_{R} = 1.40 minutes | Method B |
| **298** | **678a** | LCMS: m/z (ESI) [M+H]⁺ 589.4, t_{R} = 1.56 minutes | Method B |
| **299** | **677a** | LCMS: m/z (ESI) [M+H]⁺ 563.4, t_{R} = 1.44 minutes | Method B |
| **300** | **676a** | LCMS: m/z (ESI) [M+H]⁺ 603.5, t_{R} = 4.71 minutes | Method I |
| **301** | **675a** | LCMS: m/z (ESI) [M+H]⁺ 599.4, t_{R} = 1.47 minutes | Method B |
| **302** | **674a** | LCMS: m/z (ESI) [M+H]⁺ 599.4, t_{R} = 1.39 minutes | Method B |
| **303** | **668c** | LCMS: m/z (ESI) [M+H]⁺ 549.4, t_{R} = 1.39 minutes | Method B |
| **304** | **673a** | LCMS: m/z (ESI) [M+H]⁺ 603.5, t_{R} = 2.11 minutes | Method I |
| **305** | **672a** | LCMS: m/z (ESI) [M+H]⁺ 603.5, t_{R} = 1.47 minutes | Method B |
| **306** | **671b** | LCMS: m/z (ESI) [M+H]⁺ 577.5, t_{R} = 1.56 minutes | Method B |
| **307** | **671a** | LCMS: m/z (ESI) [M+H]⁺ 577.5, t_{R} = 1.56 minutes | Method B |
| **308** | **670a** | LCMS: m/z (ESI) [M+H]⁺ 591.5, t_{R} = 1.65 minutes | Method B |
| **309** | **669a** | LCMS: m/z (ESI) [M+H]⁺ 563.4, t_{R} = 1.38 minutes | Method B |
| **310** | **668b** | LCMS: m/z (ESI) [M+H]⁺ 549.4, t_{R} = 1.46 minutes | Method B |
| **311** | **668a** | LCMS: m/z (ESI) [M+H]⁺ 549.4, t_{R} = 1.37 minutes | Method B |
| **312** | **645b** | LCMS: m/z (ESI) [M+H]⁺ 558.5, t_{R} = 4.17 minutes | Method I |
| **313** | **667b** | LCMS: m/z (ESI) [M+H]⁺ 563.5, t_{R} = 1.42 minutes | Method B |
| **314** | **667a** | LCMS: m/z (ESI) [M+H]⁺ 563.5, t_{R} = 1.42 minutes | Method B |
| **315** | **666a** | LCMS: m/z (ESI) [M+H]⁺ 579.3, t_{R} = 1.33 minutes | Method K |
| **316** | **665b** | LCMS: m/z (ESI) [M+H]⁺ 534.4, t_{R} = 1.88 minutes | Method E |
| **317** | **665a** | LCMS: m/z (ESI) [M+H]⁺ 534.4, t_{R} = 1.86 minutes | Method E |
| **318** | **664b** | LCMS: m/z (ESI) [M+H]⁺ 520.5, t_{R} = 2.04 minutes | Method K |
| **319** | **664a** | LCMS: m/z (ESI) [M+H]⁺ 520.3, t_{R} = 2.02 minutes | Method K |
| **320** | **663b** | LCMS: m/z (ESI) [M+H]⁺ 520.3, t_{R} = 1.92 minutes | Method K |
| **321** | **663a** | LCMS: m/z (ESI) [M+H]⁺ 520.4, t_{R} = 1.89 minutes | Method K |
| **322** | **708a** | LCMS: m/z (ESI) [M+H]⁺ 629.4, t_{R} = 1.63 minutes | Method B |
| **323** | **734a** | LCMS: m/z (ESI) [M+H]⁺ 580.3, t_{R} = 1.32 minutes | Method B |
| **324** | **733a** | LCMS: m/z (ESI) [M+H]⁺ 562.4, t_{R} = 1.59 minutes | Method B |
| **325** | **732a** | LCMS: m/z (ESI) [M+H]⁺ 617.5, t_{R} = 4.06 minutes | Method B |
| **326** | **731a** | LCMS: m/z (ESI) [M+H]⁺ 638.5, t_{R} = 3.89 minutes | Method B |
| **327** | **730a** | LCMS: m/z (ESI) [M+H]⁺ 544.4, t_{R} = 0.88 minutes | Method B |
| **328** | **729a** | LCMS: m/z (ESI) [M+H]⁺ 575.4, t_{R} = 1.43 minutes | Method B |
| **329** | **728a** | LCMS: m/z (ESI) [M+H]⁺ 577.5, t_{R} = 4.62 minutes | Method I |
| **330** | **727a** | LCMS: m/z (ESI) [M+H]⁺ 603.5, t_{R} = 4.68 minutes | Method I |
| **331** | **726a** | LCMS: m/z (ESI) [M+H]⁺ 584.4, t_{R} = 4.28 minutes, t_{R} = 4.31 minutes | Method I |
| **332** | **725a** | LCMS: m/z (ESI) [M+H]⁺ 601.5, t_{R} = 4.04 minutes | Method I |
| **333** | **660b** | LCMS: m/z (ESI) [M+H]⁺ 545.5, t_{R} = 1.70 minutes | Method B |
| **334** | **724a** | LCMS: m/z (ESI) [M+H]⁺ 577.4, t_{R} = 1.67 minutes | Method B |
| **335** | **723a** | LCMS: m/z (ESI) [M+H]⁺ 575.4, t_{R} = 1.48 minutes | Method B |
| **336** | **722a** | LCMS: m/z (ESI) [M+H]⁺ 562.4, t_{R} = 1.22 minutes, t_{R} = 1.25 minutes | Method B |
| **337** | **721a** | LCMS: m/z (ESI) [M+H]⁺ 577.4, t_{R} = 1.71 minutes | Method B |
| **338** | **720a** | LCMS: m/z (ESI) [M+H]⁺ 589.4, t_{R} = 1.68 minutes, t_{R} = 1.70 minutes | Method B |
| **339** | **719a** | LCMS: m/z (ESI) [M+H]⁺ 563.5, t_{R} = 1.60 minutes | Method B |
| **340** | **718a** | LCMS: m/z (ESI) [M+H]⁺ 575.4, t_{R} = 1.45 minutes | Method B |
| **341** | **717a** | LCMS: m/z (ESI) [M+H]⁺ 617.5, t_{R} = 1.62 minutes | Method B |
| **342** | **716a** | LCMS: m/z (ESI) [M+H]⁺ 618.5, t_{R} = 1.47 minutes | Method B |
| **343** | **715a** | LCMS: m/z (ESI) [M+H]⁺ 589.4, t_{R} = 1.52 minutes | Method B |
| **344** | **714a** | LCMS: m/z (ESI) [M+H]⁺ 602.5, t_{R} = 1.38 minutes | Method B |
| **345** | **713a** | LCMS: m/z (ESI) [M+H]⁺ 610.4, t_{R} = 1.60 minutes | Method B |
| **346** | **712a** | LCMS: m/z (ESI) [M+H]⁺ 639.5, t_{R} = 1.56 minutes | Method B |
| **347** | **711a** | LCMS: m/z (ESI) [M+H]⁺ 618.5, t_{R} = 1.53 minutes | Method B |
| **348** | **710a** | LCMS: m/z (ESI) [M+H]⁺ 613.5, t_{R} = 1.64 minutes | Method B |
| **349** | **709a** | LCMS: m/z (ESI) [M+H]⁺ 612.5, t_{R} = 4.10 minutes | Method I |
| **350** | **707a** | LCMS: m/z (ESI) [M+H]⁺ 625.5, t_{R} = 1.51 minutes | Method B |
| **351** | **650d** | LCMS: m/z (ESI) [M+H]⁺ 574.4, t_{R} = 3.50 minutes | Method I |
| **352** | **748a** | LCMS: m/z (ESI) [M+H]⁺ 585.4, t_{R} = 4.12 minutes | Method I |
| **353** | **741b** | LCMS: m/z (ESI) [M+H]⁺ 563.4, t_{R} = 3.96 minutes | Method I |
| **354** | **745b** | LCMS: m/z (ESI) [M+H]⁺ 563.4, t_{R} = 3.82 minutes | Method I |
| **355** | **747a** | LCMS: m/z (ESI) [M+H]⁺ 575.5, t_{R} = 1.50 minutes | Method B |
| **356** | **746a** | LCMS: m/z (ESI) [M+H]⁺ 558.4, t_{R} = 1.47 minutes | Method B |
| **357** | **745a** | LCMS: m/z (ESI) [M+H]⁺ 563.4, t_{R} = 1.42 minutes | Method I |
| **358** | **744a** | LCMS: m/z (ESI) [M+H]⁺ 575.4, t_{R} = 3.98 minutes | Method I |
| **359** | **743a** | LCMS: m/z (ESI) [M+H]⁺ 577.5, t_{R} = 4.04 minutes | Method I |
| **360** | **742a** | LCMS: m/z (ESI) [M+H]⁺ 572.4, t_{R} = 4.15 minutes | Method I |
| **361** | **741a** | LCMS: m/z (ESI) [M+H]⁺ 563.4, t_{R} = 1.48 minutes | Method B |
| **362** | **740a** | LCMS: m/z (ESI) [M+H]⁺ 591.5, t_{R} = 3.95 minutes | Method I |
| **363** | **729b** | LCMS: m/z (ESI) [M+H]⁺ 575.5, t_{R} = 3.84 minutes | Method I |
| **364** | **739a** | LCMS: m/z (ESI) [M+H]⁺ 589.3, t_{R} = 4.38 minutes | Method I |
| **365** | **738a** | LCMS: m/z (ESI) [M+H]⁺ 613.4, t_{R} = 1.53 minutes | Method B |
| **366** | **737a** | LCMS: m/z (ESI) [M+H]⁺ 589.3, t_{R} = 4.04 minutes | Method I |
| **367** | **736a** | LCMS: m/z (ESI) [M+H]⁺ 572.3, t_{R} = 1.53 minutes | Method B |
| **368** | **735a** | LCMS: m/z (ESI) [M+H]⁺ 604.4, t_{R} = 1.33 minutes | Method B |
| **369** | **749a** | LCMS: m/z (ESI) [M+H]⁺ 576.3, t_{R} = 1.53 minutes | Method B |
| **370** | **747b** | LCMS: m/z (ESI) [M+H]⁺ 575.4, t_{R} = 4.08 minutes | Method I |
| **371** | **750a** | LCMS: m/z (ESI) [M+H]⁺ 585.5, t_{R} = 3.71 minutes | Method I |
| **372** | **752a** | LCMS: m/z (ESI) [M+H]⁺ 604.4, t_{R} = 4.03 minutes | Method I |
| **373** | **753a** | LCMS: m/z (ESI) [M+H]⁺ 575.4, t_{R} = 3.93 and 3.96 minutes | Method I |
| **374** | **754a** | LCMS: m/z (ESI) [M+H]⁺ 575.4, t_{R} = 4.56 minutes | Method I |
| **375** | **737b** | LCMS: m/z (ESI) [M+H]⁺ 589.5, t_{R} = 4.04 minutes | Method M |
| **377** | **640b** | LCMS: m/z (ESI) [M+H]⁺ 563.4, t_{R} = 1.48 minutes | Method B |
| **378** | **557a** | LCMS: m/z (ESI) [M+H]⁺ 524.4, t_{R} = 1.39 minutes | Method B |

### Example B1. Surface Plasmon Resonance

Methods herein pertain to Cytiva (formerly Biacore) instrumentation and consumables and are applicable to other SPR-based systems. A Series S Streptavidin or Neutravidin chip is docked into a Biacore 8K. The instrument is then primed into an appropriate running buffer (for example, 20 mM HEPES, pH 7.4, 150 mM NaCl, 5 mM MgCl₂, 1 mM TCEP, 0.005% Tween-20, and 2% DMSO) supplemented with either 5 µM GDP or GMPPNP to match the nucleotide state of the KRas state being tested (GDP for KRas in the GDP-bound state; and GMPPNP for KRas in the GTP-bound state). Biotinylated-KRas loaded with the appropriate nucleotide state (e.g., GDP or GMPPNP) is then captured to between 500-2000 RU to provide adequate signal for small molecules of interest, typically in a range of 300-600 Da, as well as to accommodate a range of potencies, when appropriate. The remaining biotin binding sites can be blocked with Biocytin. Compounds are injected over both a reference surface (Streptavidin or Neutravidin alone) as well as the active surface (KRas captured to Neutravidin or Streptavidin), and all curves are double-referenced before analysis (specifically, a signal subtraction of the reference flow cell from the active flow cell as well as a buffer subtraction). Where appropriate, a solvent correction is applied to all curves to account for differential bulk effects from DMSO on the reference and active surfaces. Data is then analyzed using the Biacore software fitting kinetics if resolvable, or fitting via a Langmuir isotherm model if no kinetics are resolvable, to report a binding affinity as a dissociation constant, K_{D}.

### Example B2. SOS1-Catalyzed Nucleotide Exchange Assay

**KRas G12R and WT:** Compounds are pre-dispensed using acoustic transfer technology into a black, low volume 384-well assay plate. A 10-point dose response of each compound is performed with a 30 µM top dose. Biotinylated KRas WT(1-169) or KRas G12R (1-169) loaded with GDP nucleotide is mixed with Streptavidin-Tb cryptate (Cisbio) in assay buffer (20 mM HEPES pH 7.4, 150 mM NaCl, 2 mM MgCl₂, and 0.005% CA680) to produce a 1.5x solution. 10 µL of the 1.5x KRas-cryptate solution is added to wells of a black, low-volume 384-well assay plate. The KRas/cryptate-compound mixture is incubated for 1 hour at room temperature. A 3x solution of SOS1 (564-1049) and EDA-GTP-DY-647P1 (Jena Bioscience) is prepared in assay buffer. 5 µL of the SOS1-labeled GTP solution is added to the wells to initiate the nucleotide exchange reaction. The final concentration of KRas G12R and SOS1 are 10 nM and 200 nM, respectively. The final concentration of KRas WT and SOS1 are 20 nM and 10 nM, respectively. The plate is allowed to incubate for 1 hr, then time resolved fluorescence is read on a PHERAstar plate reader equipped with a filter module with excitation = 337 nm and emission 1 = 620 nm, emission 2 = 665 nm. The TR-FRET signal is calculated as the ratio of fluorescence intensity [emission 665 nm]/[emission 620 nm]. IC₅₀ values are calculated using a four-parameter, variable response sigmoidal dose response curve fit in PerkinElmer Signals VitroVivo.

**KRas G12V:** Compounds were pre-dispensed via Echo liquid handling (acoustic, touch-free) to generate assay ready plates (ARP). More precisely, a 10-point concentration response curve for each compound was prepared with appropriate top concentration (1 µM, 10 µM, 100 µM). Biotinylated KRas G12V (aa 1-169) loaded with GDP nucleotide and Streptavidin-Europium (SA-EU, Columbia Biosciences) were preincubated in assay buffer (20 mM HEPES pH 7.4, 150 mM NaCl, 5 mM MgCl₂, 1 mM DTT, 0.01% Brij-35, 0.3 mg/mL BSA) on ice for 30 min. Separately SOS1 (aa 546-1049) and EDA-GTP-DY647P1 (Jena Bioscience) were preincubated in assay buffer on ice for 30 min. After 30 minutes of incubation for the assay components, 5 µL buffer and 5 µL of KRas G12V-SA-Eu were added to each well of the ARP. After a further 30 min, 5 µL of SOS1-EDA-GTP-D467P1 was added to start the nucleotide exchange reaction. Final concentration in the assay were 5 nM KRas G12V, 50 nM SOS1 (aa 564-1049). The reaction was allowed to proceed for 60-90 minutes after which the plate was read on a plate reader. After normalization of the raw data, the data was fit to a four-parameter logistic curve. For some compounds, data was collected at multiple sites, and the geometric mean value is presented in **Table B1** below with two significant figures.

The results are summarized in **Table B1,** below:

**Table B1.**

| **Compound No.** | **KRas G12V IC₅₀ (µM)** |
|---|---|
| **181b*** | 0.33 |
| **501a** | 0.022 |
| **501b** | 0.023 |
| **501c** | 6.0 |
| **502a** | 0.0033 |
| **502b** | 0.0038 |
| **503a** | 2.0 |
| **503b** | >10 |
| **503c** | 0.86 |
| **504a** | 0.0098 |
| **504b** | 0.015 |
| **504c** | 2.0 |
| **505a** | 0.23 |
| **506a** | 0.64 |
| **507a** | 0.0050 |
| **507b** | 0.0029 |
| **507c** | 0.066 |
| **508a** | 0.15 |
| **509a** | 0.0030 |
| **509b** | 0.043 |
| **510a** | 0.67 |
| **510b** | 0.37 |
| **510c** | >10 |
| **510d** | 0.45 |
| **510e** | >10 |
| **510f** | 0.18 |
| **511a** | <0.0025 |
| **512a** | 1.8 |
| **512b** | 0.0062 |
| **513a** | 0.012 |
| **514a** | 0.022 |
| **514b** | 0.042 |
| **515a** | 0.46 |
| **516a** | 0.0040 |
| **516b** | 0.58 |
| **516c** | 0.0029 |
| **517a** | 0.0026 |
| **518a** | 0.0094 |
| **519a** | 0.018 |
| **520a** | <0.0025 |
| **521a** | 0.019 |
| **522a** | 0.017 |
| **523a** | 0.0067 |
| **524a** | 0.12 |
| **525a** | 1.4 |
| **526a** | 0.0080 |
| **526b** | >10 |
| **526c** | 0.0068 |
| **527a** | 0.55 |
| **528a** | 0.0068 |
| **529a** | 0.016 |
| **530a** | 0.0029 |
| **531a** | <0.0025 |
| **532a** | <0.0025 |
| **533a** | 0.68 |
| **534a** | 0.010 |
| **536a** | 0.0033 |
| **536b** | 8.3 |
| **536c** | 0.0033 |
| **537a** | 4.9 |
| **538a** | 0.43 |
| **539a** | >10 |
| **540a** | 0.016 |
| **541a** | 0.18 |
| **542a** | 0.0066 |
| **543a** | 0.12 |
| **544a** | 0.58 |
| **546a** | <0.0025 |
| **547a** | 0.19 |
| **548a** | 0.12 |
| **549a** | 5.9 |
| **550a** | 0.0027 |
| **551a** | 0.13 |
| **552a** | 0.027 |
| **553a** | 0.0027 |
| **554a** | 0.0037 |
| **554b** | <0.0025 |
| **554c** | <0.0025 |
| **555a** | 0.60 |
| **556a** | 0.0026 |
| **556b** | 2.2 |
| **558a** | 0.42 |
| **559a** | 0.0037 |
| **560a** | 0.0027 |
| **561a** | <0.0025 |
| **562a** | 0.0083 |
| **563a** | 0.0039 |
| **564a** | 0.0026 |
| **565a** | 0.0039 |
| **566a** | 0.0039 |
| **566b** | 0.0030 |
| **567a** | 0.0036 |
| **567b** | 0.0032 |
| **568a** | 0.016 |
| **568b** | 0.0043 |
| **569a** | 0.0035 |
| **570a** | 0.033 |
| **571a** | 0.012 |
| **572a** | 0.054 |
| **573a** | 0.0036 |
| **574a** | 0.035 |
| **574b** | 0.024 |
| **574c** | 2.8 |
| **575a** | 0.035 |
| **575b** | 0.046 |
| **576a** | 0.0029 |
| **576b** | 0.0035 |
| **576c** | 0.27 |
| **576d** | 0.39 |
| **577a** | 0.094 |
| **578a** | 0.0053 |
| **578b** | 0.0039 |
| **579a** | 0.011 |
| **580a** | 0.0027 |
| **581a** | 0.0034 |
| **582a** | 0.013 |
| **583a** | <0.0025 |
| **584a** | <0.0025 |
| **585a** | <0.0025 |
| **585b** | <0.0025 |
| **586a** | 0.0056 |
| **587a** | 0.038 |
| **588a** | 0.012 |
| **589a** | 0.0028 |
| **590a** | <0.0025 |
| **591a** | <0.0025 |
| **592a** | <0.0025 |
| **593a** | 0.16 |
| **594a** | <0.0025 |
| **595a** | 0.0068 |
| **595b** | 0.0055 |
| **596a** | 0.0045 |
| **597a** | 0.0029 |
| **597b** | 0.0032 |
| **598a** | 0.0037 |
| **598b** | 0.0025 |
| **598c** | 0.0031 |
| **599a** | 0.066 |
| **599b** | 0.0029 |
| **600a** | 0.0054 |
| **600b** | 0.0044 |
| **601a** | 0.0028 |
| **602a** | 0.0052 |
| **603a** | >10 |
| **603b** | 0.0060 |
| **604a** | 0.0035 |
| **604b** | 0.56 |
| **604c** | <0.0025 |
| **605a** | 0.011 |
| **605b** | 3.2 |
| **605c** | 0.0077 |
| **606a** | 0.0039 |
| **606b** | 6.0 |
| **607a** | 0.0093 |
| **608a** | <0.0025 |
| **608b** | 0.0029 |
| **609a** | 0.0037 |
| **609b** | 0.022 |
| **610a** | 0.0045 |
| **610b** | 0.0025 |
| **611a** | <0.0025 |
| **611b** | 0.0041 |
| **612a** | <0.0025 |
| **612b** | <0.0025 |
| **613a** | <0.0025 |
| **613b** | <0.0025 |
| **613c** | 0.0031 |
| **614a** | <0.0025 |
| **614b** | <0.0025 |
| **614c** | 1.0 |
| **615a** | 0.0057 |
| **616a** | 0.012 |
| **617a** | <0.0025 |
| **618a** | <0.0025 |
| **619a** | 0.0054 |
| **620a** | 0.0045 |
| **621a** | <0.0025 |
| **621b** | 0.066 |
| **622a** | <0.0025 |
| **623a** | <0.0025 |
| **624a** | 0.12 |
| **624b** | <0.0025 |
| **625a** | 0.014 |
| **626a** | 0.0056 |
| **627a** | 0.0048 |
| **628a** | <0.0025 |
| **628b** | <0.0025 |
| **628c** | <0.0025 |
| **629a** | 0.0026 |
| **630a** | 0.0035 |
| **631a** | 0.0076 |
| **632a** | 0.041 |
| **635a** | 0.0037 |
| **636a** | 0.036 |
| **637a** | <0.0025 |
| **637b** | 0.0033 |
| **638a** | 0.0037 |
| **639a** | 0.0067 |
| **640a** | <0.0025 |
| **641a** | 0.0031 |
| **641b** | <0.0025 |
| **641c** | 0.044 |
| **642a** | 0.0029 |
| **643a** | 0.0025 |
| **643b** | 0.0028 |
| **644a** | 0.0092 |
| **645a** | 0.0048 |
| **645b** | 0.0039 |
| **646a** | 0.0087 |
| **647a** | 0.0049 |
| **647b** | 0.0037 |
| **648a** | 0.0039 |
| **648b** | 0.0039 |
| **649a** | 0.0049 |
| **649b** | 0.0051 |
| **650a** | 0.0037 |
| **650b** | 0.0037 |
| **650c** | 0.017 |
| **650d** | 0.008 |
| **651a** | 0.0054 |
| **651b** | 0.0093 |
| **652a** | 0.045 |
| **652b** | 0.11 |
| **652c** | 0.028 |
| **653a** | 0.0029 |
| **653b** | 0.0027 |
| **654a** | <0.0025 |
| **654b** | 0.0029 |
| **654c** | <0.0025 |
| **654d** | <0.0025 |
| **655a** | 0.0025 |
| **656a** | 0.0028 |
| **657a** | 0.029 |
| **658a** | 0.0059 |
| **659a** | 0.0075 |
| **660a** | 0.0028 |
| **660b** | 0.0033 |
| **661a** | 0.0068 |
| **661b** | 0.0035 |
| **662a** | 0.0027 |
| **662b** | <0.0025 |
| **662c** | 0.0048 |
| **662d** | 0.0035 |
| **663a** | 0.021 |
| **663b** | 0.080 |
| **664a** | 0.010 |
| **664b** | 0.072 |
| **665a** | 0.0031 |
| **665b** | 0.0096 |
| **666a** | 0.0059 |
| **667a** | 0.045 |
| **667b** | 0.013 |
| **668a** | <0.0025 |
| **668b** | 0.0025 |
| **668c** | <0.0025 |
| **668d** | 0.0029 |
| **669a** | 0.021 |
| **670a** | >1.0 |
| **671a** | 0.0027 |
| **671b** | 0.0030 |
| **672a** | 0.89 |
| **673a** | <0.0025 |
| **674a** | 0.051 |
| **675a** | 0.0063 |
| **676a** | 0.14 |
| **677a** | 0.041 |
| **678a** | 0.80 |
| **679a** | 0.16 |
| **680a** | 0.0056 |
| **680b** | 0.059 |
| **680c** | 0.0033 |
| **681a** | 0.0026 |
| **682a** | 0.025 |
| **683a** | 0.026 |
| **684a** | 0.039 |
| **685a** | 0.0028 |
| **686a** | 0.034 |
| **687a** | <0.0025 |
| **688a** | 0.0036 |
| **689a** | <0.0025 |
| **689b** | 0.0053 |
| **690a** | 0.011 |
| **691a** | 0.048 |
| **692a** | 0.19 |
| **693a** | 0.0058 |
| **694a** | 0.022 |
| **695a** | 0.0087 |
| **696a** | 0.30 |
| **697a** | >1.0 |
| **698a** | 0.90 |
| **699a** | >1.0 |
| **700a** | 0.059 |
| **701a** | <0.0025 |
| **702a** | <0.0025 |
| **702b** | <0.0025 |
| **703a** | 0.013 |
| **704a** | 0.47 |
| **705a** | 0.075 |
| **706a** | 0.10 |
| **706b** | >1.0 |
| **706c** | >1.0 |
| **706d** | 0.020 |
| **707a** | 0.47 |
| **708a** | 0.089 |
| **709a** | <0.0025 |
| **710a** | 0.19 |
| **711a** | >1.0 |
| **712a** | 0.028 |
| **713a** | 0.0045 |
| **714a** | 0.022 |
| **715a** | 0.12 |
| **716a** | 0.016 |
| **717a** | 0.18 |
| **718a** | 0.064 |
| **719a** | 0.16 |
| **720a** | 0.0025 |
| **721a** | <0.0025 |
| **722a** | 0.0045 |
| **723a** | 0.18 |
| **724a** | 0.13 |
| **725a** | 0.38 |
| **726a** | 0.034 |
| **727a** | 0.24 |
| **728a** | 0.28 |
| **729a** | 0.0032 |
| **729b** | 0.0052 |
| **730a** | 0.0031 |
| **731a** | 0.0048 |
| **732a** | 0.12 |
| **733a** | 0.021 |
| **734a** | 0.0053 |
| **735a** | 0.68 |
| **736a** | <0.0025 |
| **737a** | 0.0030 |
| **738a** | 0.0081 |
| **739a** | 0.33 |
| **740a** | 0.011 |
| **741a** | <0.0025 |
| **741b** | 0.0033 |
| **742a** | 0.024 |
| **743a** | >1.0 |
| **744a** | 0.028 |
| **745a** | 0.043 |
| **745b** | 0.10 |
| **746a** | 0.011 |
| **747a** | <0.0025 |
| **747b** | <0.0025 |
| **748a** | 0.015 |
| **749a** | 0.016 |
| **750a** | <0.0025 |
| **751a** | <0.0025 |
| **752a** | 0.18 |
| **753a** | 0.016 |
| **754a** | 0.0075 |

| | |
|---|---|
| **Notes:** **(1)** '*' indicates that data for this compound was previously presented as being associated with compound **500a** in U.S. Provisional Application Serial No. 63/542,178. | |

### Example B3. KRas-cRAF Protein-Protein Interaction (PPI) Assay

Compounds are pre-dispensed using acoustic transfer technology into a black, low volume 384-well assay plate. A 10-point dose response of each compound is performed with a 30µM top dose. Biotinylated KRas protein (e.g., KRas G12R(1-169)) is loaded with GppNHp (i.e., GMPPNP) nucleotide and GST-cRAF(1-149) are diluted to 90 nM and 30 nM, respectively, in assay buffer (20 mM HEPES pH 7.4, 150 mM NaCl, 5 mM MgCl₂, and 0.005% CA680). 5 µL of KRas protein is added to wells of a black, low-volume 384-well assay plate. The KRas-compound mixture is incubated for 30 minutes at room temperature. 5 µL of GST-cRAF protein is added to the KRas-compound mixture and incubated for 30 minutes at room temperature. 100x stocks of Tb cryptate-labeled anti-GST antibody (Anti-GST-Tb) (Cisbio) and Streptavidin-XL655 (Cisbio) are used to make a 3x detection mixture in a total volume of 5 µL of assay buffer. The detection mixture is added to the assay wells and incubated an additional 1 hour at room temperature. Time resolved fluorescence is read on a PHERAstar plate reader equipped with a filter module with excitation = 337 nm and emission 1 = 620 nm, emission 2 = 665 nm. The TR-FRET signal is calculated as the ratio of fluorescence intensity [emission 665 nm]/[excitation 337 nm]. IC₅₀ values are calculated using a four-parameter, variable response sigmoidal dose response curve fit in PerkinElmer Signals VitroVivo.

### Example B4. KRas-cRAF Protein-Protein Interaction (PPI) Assay

**Version 1:** Compounds are pre-dispensed via Echo liquid handling (acoustic, touch-free) to generate assay ready plates (ARP). A 10-point dose response of each compound is performed with a 30µM top dose. Biotinylated KRas G12V (aa 1-169) loaded with GMPPNP nucleotide and Streptavidin-Europium (SA-EU, Columbia Biosciences) are preincubated in assay buffer (20 mM HEPES pH 7.4, 150 mM NaCl, 5 mM MgCl₂, 1 mM DTT, 0.01% Brij-35, 0.3 mg/ml BSA) on ice for 30 min. Separately GST-tagged cRAF(1-149) and anti-GST-APC antibody (Columbia Biosciences) are preincubated in assay buffer on ice for 30 min. After incubation, 5 µL buffer and 5 µL of KRas G12V-SA-Eu are added to each well of the ARP. After a 30-minute equilibration phase at room temperature, 5 uL of GST-cRAF-anti-GST-APC is added to each well. The final concentrations in the assay are 10 nM KRas G12V, 10 nM cRAF. The reaction is allowed to proceed for 60-90 minutes after which the plate is read on a PHERAstar FSX plate reader (exc: 337 nm, em 1: 665 nm, em 2: 620 nm). After normalization of the raw data (ratio of Signal₆₆₅ₙₘ/Signal₆₂₀ₙₘ) the data is fit to a four-parameter logistic curve in GraphPad Prism (v9.4.1).

**Version 2:** Compounds are pre-dispensed via Echo liquid handling (acoustic, touch-free) to generate assay ready plates (ARP). More precisely, a 10-point concentration response curve for each compound is prepared with appropriate top concentration (e.g., 1 µM or 30 µM). Biotinylated KRas G12V (aa 1-169) loaded with GMPPNP nucleotide and Streptavidin-Europium (SA-EU, Columbia Biosciences) are preincubated in assay buffer (20 mM HEPES pH 7.4, 150 mM NaCl, 5 mM MgCl₂, 1 mM DTT, 0.01% Brij-35, 0.3 mg/ml BSA) on ice for 30 min. Separately, GST-tagged cRAF(1-149) and anti-GST-APC antibody (Columbia Biosciences) are preincubated in assay buffer on ice for 30 min. After incubation, 5 µL of KRas G12V-SA-Eu is added to each well of the ARP. After a 30 min equilibration phase at room temperature, 5 µL of GST-cRAF-anti-GST-APC is added to each well. Final concentrations in the assay are 5 nM KRas G12V, 5 nM cRAF. The reaction is allowed to proceed for 60-90 minutes after which the plate is read on a PHERAstar FSX plate reader (exc: 337 nm, em 1: 665 nm, em 2: 620 nm). After normalization of the raw data (ratio of Signal₆₆₅ₙₘ/Signal₆₂₀ₙₘ) the data is fit to a four-parameter logistic curve.

**Version 3:** Compounds are pre-dispensed via Echo liquid handling (acoustic, touch-free) to generate assay ready plates (ARP). More precisely, a 10-point concentration response curve for each compound is prepared with appropriate top concentration (e.g., 1 µM or 30 µM). Biotinylated KRas G12D (aa 1-169) loaded with GMPPNP nucleotide and Streptavidin-Europium (SA-EU, Columbia Biosciences) are preincubated in assay buffer (20 mM HEPES pH 7.4, 150 mM NaCl, 5 mM MgCl2, 1 mM DTT, 0.01% Brij-35, 0.3 mg/ml BSA) on ice for 30 min. Separately, GST-tagged cRAF(1-149) and anti-GST-APC antibody (Columbia Biosciences) are preincubated in assay buffer on ice for 30 min. After incubation, 5 µL of KRas G12D-SA-Eu is added to each well of the ARP. After a 30 min equilibration phase at room temperature, 5 µL of GST-cRAF-anti-GST-APC is added to each well. Final concentrations in the assay are 4 nM KRas G12D, 4 nM cRAF. The reaction is allowed to proceed for 60-90 minutes after which the plate is read on a PHERAstar FSX plate reader (exc: 337 nm, em 1: 665 nm, em 2: 620 nm). After normalization of the raw data (ratio of Signal₆₆₅ₙₘ/Signal₆₂₀ₙₘ) the data is fit to a four-parameter logistic curve.

### Example B5 - Surface Plasmon Resonance (SPR)

Biacore 8K buffer line was placed into 1 L of Immobilization buffer (20mM HEPES pH 7.5 / 150 mM NaCl / 5 mM MgCl₂ / 0.5 mM TCEP / 0.005% P20 / 500 nM GDP) and Change Solutions was performed. A Biacore Series S SA chip (Cytiva 29104992) was inserted and Change Solutions was performed 3 times. The chip surface was conditioned by injecting conditioning solution (40 mM NaOH / 1 mM NaCl) in four pulses of 30 seconds at 30 uL/min. These pulses were followed by pulses of running buffer injected for 30 seconds at 30 uL/min. Normalization was run using the BiaNormalize solution (Cytiva 29207950). 300 nM Biotinylated KRas G12V 1-169 was injected over all active flow cells at a flow rate of 5 uL/min until reaching a response of 1000 RUs. The buffer line was switched into Running Buffer (20 mM HEPES pH 7.5 / 150 mM NaCl / 5 mM MgCl₂ / 0.5mM TCEP / 0.005% P20 / 500 nM GDP / 2% DMSO) and Change Solutions was performed. Compounds were plated into a Greiner v-bottom 384-well Microplate (Greiner 781280) in 8-point dose-response with a log-fold dilution with a final volume of 2 uL in DMSO. 98uL of non-DMSO containing buffer was backfilled into all compound wells (final volume of 100 uL with 2% DMSO). Solvent corrections of 1%, 1.5%, 2%, 2.5%, and 3% DMSO were plated into a Greiner U-bottom 96-well microplate (Greiner 650201). The SPR method was run with both flow cell and sample compartment at 20 °C or 37 °C with 5 startup cycles of 30 second association, and 30 second dissociation at a flow rate of 70 uL/min. An eight-point single cycle kinetics (SCK) analysis was run with 30 second association time, 1600 second dissociation time, 70 uL/min flow rate, 1600 sec stability period, and a 50% DMSO wash after injection. Solvent corrections were run at the end of the method. Results were analyzed with the Biacore Insights Evaluation Software using the Single Cycle Kinetics analysis method. Sensorgrams were fit using a 1:1 binding model. K_{D} values, as an average of multiple runs, if applicable, are presented in **Table B2** with two significant figures.

**Table B2.**

| **Compound No.** | **KRas G12V GDP K_{D} (nM), 20 °C** | **KRas G12V GDP K_{D} (nM), 37 °C** |
|---|---|---|
| **181b** | 250 | |
| **501a** | 34 | |
| **501b** | 19 | |
| **501c** | >5000 | |
| **502a** | 0.24 | 4.1 |
| **502b** | 0.34 | 3.9 |
| **503a** | 970 | |
| **503b** | >5000 | |
| **503c** | 580 | |
| **504a** | 8.4 | |
| **504b** | 6.0 | |
| **504c** | >5000 | |
| **505a** | 170 | |
| **506a** | >5000 | |
| **507a** | 0.082 | 0.24 |
| **507b** | 0.058 | 0.11 |
| **507c** | 54 | |
| **508a** | 140 | |
| **509a** | 0.030 | 0.023 |
| **509b** | 8.2 | |
| **510a** | >5000 | |
| **510b** | 330 | |
| **510c** | >5000 | |
| **510d** | 1000 | |
| **510e** | >5000 | |
| **510f** | 210 | |
| **511a** | 0.12 | 2.4 |
| **512a** | >5000 | |
| **512b** | 3.8 | |
| **513a** | 4.5 | |
| **514a** | 18 | |
| **514b** | 33 | |
| **515a** | 400 | |
| **516a** | 0.68 | 6.6 |
| **516b** | 380 | |
| **516c** | 0.36 | 5.0 |
| **517a** | 0.027 | 0.18 |
| **518a** | 7.8 | |
| **519a** | 17 | |
| **520a** | 0.028 | 0.17 |
| **521a** | 15 | |
| **522a** | 12 | |
| **523a** | 1.6 | |
| **524a** | 86 | |
| **525a** | 880 | |
| **526a** | 2.5 | |
| **526b** | >5000 | |
| **526c** | 2.1 | 19 |
| **527a** | 610 | |
| **528a** | 1.5 | 16 |
| **529a** | 6.3 | 70 |
| **530a** | 0.023 | 0.046 |
| **531a** | 0.020 | 0.031 |
| **532a** | 0.065 | 0.75 |
| **533a** | 120 | |
| **534a** | 6.7 | 46 |
| **535a** | >5000 | |
| **536a** | 0.24 | 2.7 |
| **536b** | >5000 | |
| **536c** | 0.24 | 2.5 |
| **537a** | >5000 | |
| **538a** | 240 | |
| **539a** | >5000 | |
| **540a** | 7.2 | |
| **541a** | 110 | |
| **542a** | 3.4 | 45 |
| **544a** | 340 | |
| **545a** | 45 | |
| **546a** | 0.10 | 0.10 |
| **547a** | 180 | |
| **548a** | 260 | |
| **549a** | >5000 | |
| **550a** | 0.27 | 2.6 |
| **551a** | 110 | |
| **552a** | 20 | |
| **553a** | 0.75 | 8.8 |
| **554a** | 0.46 | 4.7 |
| 554b | 0.27 | 3.3 |
| 554c | 0.12 | 1.9 |
| 555a | 380 | |
| 556a | 0.048 | 0.33 |
| **556b** | >500 | >500 |
| **557a** | | 71 |
| **558a** | 380 | |
| **559a** | 0.56 | 7.6 |
| **560a** | 0.68 | 7.5 |
| **561a** | 0.31 | 3.3 |
| **562a** | 5.5 | 49 |
| **563a** | 0.64 | 7.1 |
| **564a** | 0.55 | 6.9 |
| **565a** | 0.94 | 9.3 |
| **566a** | 0.22 | 3.1 |
| **566b** | 0.68 | 11 |
| **567a** | 0.66 | 6.6 |
| **567b** | 0.36 | 4.7 |
| **568a** | 16 | 140 |
| **568b** | 1.0 | 14 |
| **569a** | 1.6 | 18 |
| **570a** | 39 | 280 |
| **571a** | 15 | 110 |
| **572a** | 38 | 250 |
| **573a** | 1.3 | 13 |
| **574a** | 25 | 140 |
| **574b** | 11 | 65 |
| **574c** | >5000 | >5000 |
| **575a** | 33 | |
| **575b** | 34 | |
| **576a** | 0.037 | 0.19 |
| **576b** | 0.29 | 3.6 |
| **576c** | | >500 |
| **576d** | | >500 |
| **577a** | 79 | |
| **578a** | 2.0 | 26 |
| **578b** | 0.20 | 3.4 |
| **579a** | 8.2 | 74 |
| **580a** | 0.36 | 1.8 |
| **581a** | 0.11 | 1.4 |
| **582a** | 6.8 | 68 |
| **583a** | 0.068 | 0.71 |
| **584a** | 0.088 | 0.25 |
| **585a** | 0.029 | 0.12 |
| **585b** | 0.42 | 7.3 |
| **586a** | 1.5 | 23 |
| **587a** | 24 | 190 |
| **588a** | 0.33 | 13 |
| **589a** | 0.41 | 4.3 |
| **590a** | 0.043 | 0.30 |
| **591a** | 0.12 | 1.0 |
| **592a** | 0.026 | 0.17 |
| **593a** | 97 | 630 |
| **594a** | 0.10 | 2.1 |
| **595a** | 0.39 | 7.2 |
| **595b** | 0.43 | 8.2 |
| **596a** | 1.0 | 15 |
| **597a** | 0.017 | 0.068 |
| **597b** | 0.051 | 0.75 |
| **598a** | 0.087 | 0.19 |
| **598b** | 0.033 | 0.10 |
| **598c** | 0.13 | 1.6 |
| **599a** | | 45 |
| **599b** | 0.15 | 0.81 |
| **600a** | 0.92 | 10 |
| **600b** | 0.078 | 0.61 |
| **601a** | 1.5 | 4.9 |
| **602a** | 0.063 | 0.90 |
| **603a** | >500 | >500 |
| **603b** | 1.9 | 17 |
| **604a** | 0.15 | 1.8 |
| **604b** | | >500 |
| **604c** | | 1.6 |
| **605a** | 6.6 | 58 |
| **605b** | >500 | >500 |
| **605c** | 4.1 | 57 |
| **606a** | 1.0 | 14 |
| **606b** | >500 | >500 |
| **607a** | 2.9 | 35 |
| **608a** | 0.050 | 0.39 |
| **608b** | 0.18 | 2.5 |
| **609a** | 0.32 | 4.3 |
| **609b** | | 21 |
| **610a** | 0.99 | 7.8 |
| **610b** | | 1.5 |
| **611a** | 0.080 | 0.58 |
| **611b** | 1.1 | 10 |
| **612a** | | 1.4 |
| **612b** | | 3.1 |
| **613a** | | 1.1 |
| **613b** | | 0.68 |
| **613c** | | 7.5 |
| **614a** | | 0.59 |
| **614b** | | 0.69 |
| **614c** | | >500 |
| **615a** | | 23 |
| **616a** | | 54 |
| **617a** | | 0.94 |
| **618a** | | 0.64 |
| **619a** | | 2.2 |
| **620a** | | 7.5 |
| **621a** | | 0.57 |
| **621b** | | 470 |
| **622a** | | 1.9 |
| **623a** | | 0.33 |
| **624a** | | >500 |
| **624b** | | 0.12 |
| **625a** | | 61 |
| **626a** | | 28 |
| **627a** | | 20 |
| **628b** | | 0.033 |
| **628c** | | 0.050 |
| **631a** | | 2.0 |
| **632a** | | 9.0 |
| **635a** | | 14 |
| **636a** | | 140 |
| **637a** | | 1.3 |
| **637b** | | 4.4 |
| **638a** | | 6.7 |
| **639a** | | 32 |
| **640a** | | 0.091 |
| **640b** | | 0.24 |
| **641a** | | 0.49 |
| **641b** | | 0.26 |
| **641c** | | 9.0 |
| **642a** | | 1.0 |
| **643a** | | 3.3 |
| **643b** | | 8.4 |
| **644a** | | 72 |
| **645a** | | 16 |
| **645b** | | 7.9 |
| **646a** | | 55 |
| **647a** | | 26 |
| **647b** | | 17 |
| **648a** | | 18 |
| **648b** | | 19 |
| **649a** | | 25 |
| **649b** | | 21 |
| **650a** | | 20 |
| **650b** | | 16 |
| **650c** | | 120 |
| **650d** | | 40 |
| **651a** | | 34 |
| **651b** | | 56 |
| **652a** | | 320 |
| **652b** | | >500 |
| **652c** | | 210 |
| **653a** | | 9.2 |
| **653b** | | 1.1 |
| **654a** | | 2.5 |
| **654b** | | 8.7 |
| **654c** | | 0.83 |
| **654d** | | 0.18 |
| **655a** | | 3.4 |
| **656a** | | 6.3 |
| **657a** | | 210 |
| **658a** | | 34 |
| **659a** | | 45 |
| **660a** | | 5.1 |
| **660b** | | 5.3 |
| **661a** | | 35 |
| **661b** | | 13 |
| **662a** | | 1.1 |
| **662b** | | 0.19 |
| **662c** | | 28 |
| **662d** | | 9.0 |
| **663a** | 13 | 130 |
| **663b** | 43 | 410 |
| **664a** | 4 | 44 |
| **664b** | 42 | 270 |
| **665a** | 0.18 | 2.1 |
| **665b** | 3.8 | 32 |
| **666a** | | 10 |
| **667a** | | 240 |
| **667b** | | 83 |
| **668a** | | 5.5 |
| **668b** | | 3.9 |
| **668c** | | 2.6 |
| **668d** | | 4.4 |
| **669a** | | 130 |
| **670a** | | >500 |
| **671a** | | 5.7 |
| **671b** | | 7.4 |
| **672a** | | >500 |
| **673a** | | 4.0 |
| **674a** | | 260 |
| **675a** | | 33 |
| **676a** | | 1300 |
| **677a** | | 220 |
| **678a** | | >500 |
| **679a** | | 400 |
| **680a** | | 20 |
| **680b** | | 360 |
| **680c** | | 14 |
| **681a** | | 2.3 |
| **682a** | | 120 |
| **683a** | | 170 |
| **684a** | | 270 |
| **685a** | | 9.0 |
| **686a** | | 240 |
| **687a** | | 1.1 |
| **688a** | | 13 |
| **689a** | | 0.68 |
| **689b** | | 26 |
| **690a** | | 59 |
| **691a** | | 360 |
| **692a** | | 770 |
| **693a** | | 14 |
| **694a** | | 150 |
| **695a** | | 46 |
| **696a** | | >500 |
| **697a** | | >500 |
| **698a** | | >500 |
| **699a** | | >500 |
| **700a** | | 390 |
| **701a** | | 3.7 |
| **702a** | | 1.2 |
| **702b** | | 0.99 |
| **703a** | | 81 |
| **704a** | | >500 |
| **705a** | | 350 |
| **706a** | | 540 |
| **706b** | | >500 |
| **706c** | | >500 |
| **706d** | | 130 |
| **707a** | | >500 |
| **708a** | | 270 |
| **709a** | | 3.3 |
| **710a** | | 710 |
| **711a** | | >500 |
| **712a** | | 120 |
| **713a** | | 26 |
| **714a** | | 110 |
| **715a** | | 480 |
| **716a** | | 96 |
| **717a** | | 650 |
| **718a** | | 310 |
| **719a** | | 680 |
| **720a** | | 0.54 |
| **721a** | | 0.65 |
| **722a** | | 14 |
| **723a** | | 600 |
| **724a** | | >500 |
| **725a** | | >500 |
| **726a** | | 170 |
| **727a** | | >500 |
| **728a** | | >500 |
| **729a** | | 8.9 |
| **729b** | | 14 |
| **730a** | | 3.5 |
| **731a** | | 19 |
| **732a** | | 650 |
| **733a** | | 24 |
| **734a** | | 22 |
| **735a** | | >500 |
| **736a** | | 1.3 |
| **737a** | | 2.3 |
| **738a** | | 52 |
| **739a** | | >500 |
| **740a** | | 72 |
| **741a** | | 3.7 |
| **741b** | | 7.4 |
| **742a** | | 140 |
| **743a** | | >500 |
| **745b** | | 660 |
| **747a** | | 0.33 |
| **747b** | | 0.26 |
| **748a** | | 120 |
| **749a** | | 130 |
| **750a** | | 0.42 |
| **751a** | | 2.4 |
| **752a** | | >500 |
| **753a** | | 84 |
| **754a** | | 46 |

### Example B6 - Measurement of cellular phospho- and total ERK1/2

In a white, opaque-bottom Revvity CulturPlate-384, SW620 ([SW-620] (ATCC CCL-227)) or GP2d (CB_95090714) cells were seeded at previously determined seeding densities such that the well would be approximately 80% confluent at the end of the assay, and incubated overnight in a standard 37 °C, 5% CO₂ humidified incubator. The day after seeding, compounds were dispensed into the treatment plates using a Tecan D300e compound printer in 9-point DRC format, 10-micromolar top concentration, in triplicate. Treatment plates were then returned to a 37 °C, 5% CO₂ humidified incubator for 2 hours unless otherwise stated. Following compound treatment, phosphoERK and total ERK were measured using the Alpha SureFire Ultra Multiplex Phospho/Total ERK1/2 Assay Kit (Revvity). All media was removed from the treatment plate(s) and the cells subsequently lysed using 1X Lysis Buffer in accordance with manufacturer protocol. Next, the *Acceptor Mix* (prepared in accordance with manufacturers protocol) was added to each well of the assay plate and incubated on an orbital shaker at room temperature for 2 hours. Following Acceptor incubation, the *Donor Mix* (prepared in accordance with manufacturer protocol) was added to each well of the assay plate, covered to protect from light and incubated on an orbital shaker at room temperature overnight. Assay plates were read the following day on a BMG Labtech PHERAstar FSX microplate reader. Data were then analyzed by calculating the ratio of ERK1/2-phosphorylation relative to Total ERK1/2 for each individual well. $Ratio pERK 1 / 2 = \frac{615 nm Signal \left(pERK1/2\right)}{545 nm Signal \left(Total ERK1/2\right)}$

The replicate ratios for each concentration were averaged and normalized to DMSO control or other corresponding co-treatment before performing a variable slope (4-parameter), non-linear regression curve fit for each compound of interest. IC₅₀ values, as a geometric mean of multiple runs, if applicable, are presented in from this assay are presented in **Table B3** with two significant figures.

**Table B3.**

| **Compound No.** | **pERK SW620 IC₅₀ (µM)** | **pERK GP2d IC₅₀ (µM)** |
|---|---|---|
| **181b** | 4.2 | 4.3 |
| **501a** | 0.019 | 0.038 |
| **501b** | 1.5 | 0.041 |
| **502a** | 0.029 | 0.011 |
| **502b** | 0.040 | 0.0070 |
| **504a** | 3.8 | 0.16 |
| **504b** | 2.1 | 0.83 |
| **505a** | | 3.4 |
| **506a** | 1.5 | 3.2 |
| **507a** | 0.018 | <0.0026 |
| **507b** | 0.0099 | <0.0026 |
| **507c** | 0.21 | 0.029 |
| **508a** | 0.97 | 2.0 |
| **509a** | 0.0036 | 0.00041 |
| **509b** | 0.12 | |
| **511a** | 0.13 | 0.29 |
| **512b** | 0.20 | |
| **513a** | 7.9 | |
| **516a** | 0.55 | |
| **516c** | 0.098 | |
| **517a** | 0.077 | |
| **518a** | 0.20 | |
| **519a** | 0.87 | |
| **520a** | 0.011 | |
| **521a** | 1.8 | |
| **522a** | 1.1 | |
| **523a** | 0.96 | |
| **526a** | 3.7 | |
| **526c** | 1.4 | |
| **528a** | 0.10 | |
| **529a** | 0.35 | |
| **530a** | 0.0029 | |
| **531a** | 0.0041 | |
| **532a** | 0.062 | |
| **534a** | 0.12 | |
| **536a** | 0.10 | |
| **536c** | 0.039 | |
| **540a** | 0.25 | |
| **542a** | 0.34 | |
| **546a** | 0.0030 | |
| **550a** | 0.0095 | |
| **552a** | >10 | |
| **553a** | 0.016 | |
| **554a** | 0.47 | |
| **554b** | 0.36 | |
| **554c** | 0.37 | |
| **556a** | 0.0021 | 0.00083 |
| **559a** | 0.079 | |
| **560a** | 0.011 | |
| **561a** | 0.0069 | |
| **562a** | 0.60 | |
| **563a** | 0.12 | |
| **564a** | 0.063 | |
| **565a** | 0.057 | |
| **566a** | 0.014 | |
| **566b** | 0.031 | |
| **567a** | 0.077 | |
| **567b** | 0.29 | |
| **568a** | 0.42 | |
| **568b** | 0.58 | |
| **569a** | 0.028 | |
| **570a** | 0.51 | |
| **571a** | 0.14 | |
| **572a** | 0.64 | |
| **573a** | 0.026 | |
| **574a** | 0.57 | |
| **574b** | 0.40 | |
| **575a** | 2.1 | |
| **575b** | 7.1 | |
| **576a** | 0.00048 | 0.00059 |
| **576b** | 0.012 | 0.013 |
| **577a** | 1.2 | |
| **578a** | 0.026 | 0.11 |
| **578b** | 0.035 | |
| **579a** | 0.10 | |
| **580a** | 0.0052 | |
| **581a** | 0.034 | |
| **582a** | 1.1 | |
| **583a** | 0.011 | 0.013 |
| **584a** | 0.0037 | |
| **585a** | 0.00033 | 0.00046 |
| **585b** | 0.024 | |
| **586a** | 0.16 | |
| 587a | 0.92 | |
| 588a | 0.12 | |
| 589a | 0.0063 | 0.0059 |
| 590a | 0.0042 | 0.00069 |
| **591a** | 0.014 | 0.0061 |
| **592a** | 0.0011 | 0.00036 |
| **594a** | 0.018 | |
| **595a** | 0.013 | 0.062 |
| **595b** | 0.062 | |
| **596a** | 0.41 | |
| **597a** | 0.00044 | 0.00021 |
| **597b** | 0.0022 | 0.00067 |
| **598a** | 0.0033 | 0.0013 |
| **598b** | 0.0029 | |
| **598c** | 0.0010 | |
| **599a** | 0.15 | |
| **599b** | 0.0070 | |
| **600a** | 0.44 | |
| **600b** | 0.017 | |
| **601a** | 0.0065 | |
| **602a** | 0.013 | |
| **603b** | 0.020 | |
| **604a** | 0.95 | |
| **604c** | 0.31 | |
| **605a** | 1.3 | |
| **605c** | 0.50 | |
| **606a** | 0.063 | |
| **607a** | 0.088 | |
| **608a** | 0.036 | |
| **608b** | 0.18 | |
| **609a** | 0.27 | |
| **609b** | 0.43 | |
| **610a** | 0.091 | |
| **610b** | 0.0038 | |
| **611a** | 0.0017 | |
| **611b** | 0.042 | |
| **612a** | 0.0089 | |
| **612b** | 0.026 | |
| **613a** | 0.022 | |
| **613b** | 0.0023 | |
| **613c** | 0.053 | |
| **614a** | 0.064 | |
| **614b** | 0.0069 | |
| **615a** | 3.5 | |
| **616a** | >10 | |
| **617a** | 0.13 | |
| **619a** | 0.40 | |
| **620a** | 0.041 | |
| **621a** | 0.054 | |
| **621b** | 0.36 | |
| **622a** | 0.057 | |
| **623a** | 0.0061 | |
| **624b** | 0.0018 | |
| **625a** | 0.72 | |
| **626a** | 0.15 | |
| **627a** | 0.22 | |
| **628a** | 0.034 | |
| **628b** | 0.0093 | |
| **628c** | 0.02 | |
| **629a** | 0.20 | |
| **630a** | 0.36 | |
| **631a** | 0.12 | |
| **632a** | 0.12 | |
| **635a** | 0.059 | |
| **636a** | 0.60 | |
| **637a** | 0.0032 | |
| **637b** | 0.017 | |
| **638a** | 0.061 | |
| **639a** | 0.067 | |
| **640a** | 0.00024 | 0.00032 |
| **641a** | 0.0025 | |
| **641b** | 0.0024 | |
| **641c** | 0.049 | |
| **642a** | 0.012 | |
| **643a** | 0.0043 | |
| **643b** | 0.15 | |
| **644a** | 0.13 | |
| **645a** | 0.056 | |
| **645b** | 0.030 | |
| **646a** | 0.066 | |
| **647a** | 0.11 | |
| **647b** | 0.051 | |
| **648a** | 0.035 | |
| **648b** | 0.041 | |
| **649a** | 0.099 | |
| **649b** | 0.083 | |
| **650a** | 0.69 | |
| **650b** | 0.5 | |
| **650c** | 0.58 | |
| **650d** | 0.52 | |
| **651a** | >10 | |
| **651b** | >10 | |
| **652a** | 0.18 | |
| **652b** | 1.5 | |
| **652c** | 0.27 | |
| **653a** | 0.020 | |
| **653b** | 0.0035 | |
| **654a** | 0.0072 | |
| **654b** | 0.017 | |
| **654c** | 0.0017 | |
| **654d** | 0.0029 | |
| **655a** | 0.0068 | |
| **656a** | 0.36 | |
| **657a** | 0.040 | |
| **658a** | 0.045 | |
| **659a** | 0.13 | |
| **660a** | 0.037 | |
| **660b** | 0.025 | |
| **661a** | 0.054 | |
| **661b** | 0.050 | |
| **662a** | 0.0017 | |
| **662b** | 0.00048 | |
| **662c** | 0.036 | |
| **662d** | 0.026 | |
| **663a** | 0.37 | |
| **663b** | 1.6 | |
| **664a** | 0.074 | |
| **664b** | 0.67 | |
| **665a** | 0.0020 | |
| **665b** | 0.023 | |
| **666a** | 0.064 | |
| **667a** | 0.42 | |
| **667b** | 0.23 | |
| **668a** | 0.0096 | |
| **668b** | 0.011 | |
| **668c** | 0.0069 | |
| **668d** | 0.014 | |
| **669a** | 0.34 | |
| **671a** | 0.0050 | |
| **671b** | 0.011 | |
| **673a** | 0.0053 | |
| **674a** | 1.1 | |
| **675a** | 0.045 | |
| **677a** | 0.89 | |
| **680a** | 0.065 | |
| **680b** | 0.75 | |
| **680c** | 0.11 | |
| **681a** | 0.0035 | |
| **682a** | 0.39 | |
| **683a** | 0.33 | |
| **684a** | 0.54 | |
| **685a** | 0.019 | |
| **686a** | 0.49 | |
| **687a** | 0.0096 | |
| **688a** | 0.016 | |
| **689a** | 0.0013 | |
| **689b** | 0.027 | |
| **690a** | 0.12 | |
| **691a** | 0.75 | |
| **693a** | 0.010 | |
| **694a** | 0.33 | |
| **695a** | 0.047 | |
| **700a** | 0.33 | |
| **701a** | 0.0047 | |
| **702a** | <0.0026 | |
| **702b** | 0.0018 | |
| **703a** | 0.27 | |
| **705a** | 0.26 | |
| **706a** | 0.58 | |
| **706d** | 0.17 | |
| **708a** | 0.43 | |
| **709a** | 0.0080 | |
| **712a** | 0.36 | |
| **713a** | 0.065 | |
| **714a** | 0.71 | |
| **716a** | 0.37 | |
| **718a** | 0.45 | |
| **720a** | 0.00080 | |
| **721a** | 0.0047 | |
| **722a** | 0.31 | |
| **726a** | 0.56 | |
| **729a** | 0.033 | |
| **729b** | 0.20 | |
| **730a** | 0.0051 | |
| **731a** | 0.061 | |
| **733a** | 0.05 | |
| **734a** | 0.44 | |
| **736a** | 0.0038 | |
| **737a** | 0.0016 | |
| **737b** | 0.045 | |
| **738a** | 1.2 | |
| **740a** | >10 | |
| **741a** | 0.0071 | |
| **741b** | 0.0082 | |
| **742a** | 0.14 | |
| **744a** | 0.64 | |
| **745a** | 0.54 | |
| **745b** | 2.0 | |
| **746a** | 0.48 | |
| **747a** | 0.00047 | |
| **748a** | 0.19 | |
| **749a** | 0.47 | |

### Example B7 - Measurement of cellular proliferation

In a black, clear-bottom 384-well tissue culture treated plates, SW620 or MIAPaCa-2 (Sigma cb_85062806) cells were seeded at previously determined seeding densities such that the well would be approximately 80% confluent at the end of the assay. Immediately after seeding, compounds were dispensed into the treatment plates using a Tecan D300e compound printer in 9-point DRC format, 10- micromolar top concentration, in triplicate. Treatment plates were then transferred to a 37 °C, 5% CO₂ humidified incubator for 72 hours unless otherwise stated. After the completion of treatment, CELLTITER-GLO^{®} 2.0 reagent (Promega) was added directly to each well. The plates were covered to protect from light and incubated on an orbital shaker at room temperature for 30 minutes. Immediately before reading the assay plates, an opaque seal was attached to the bottom of each clear-bottom plate; assay plates were then read on a BMG Labtech PHERAstar FSX microplate reader. Data were analyzed by calculating the ratio of signal from treated wells relative to negative control-treated wells. The replicates were averaged and a variable slope (4-parameter), non-linear regression curve was fitted for each compound of interest. IC₅₀ values, as a geometric mean of multiple runs, if applicable, are presented in from this assay are presented in **Table B4** with two significant figures.

**Table B4.**

| **Compound No.** | **Proliferation SW620 IC₅₀ (µM)** | **Proliferation MIAPaCa-2 IC₅₀ (µM)** |
|---|---|---|
| **501a*** | 0.31 | |
| **501b** | 6.0 | |
| **501c** | >10 | |
| **502a** | 0.49 | 1.1 |
| **502b** | 0.49 | 1.1 |
| **504a** | >10 | |
| **504b** | >10 | |
| **504c** | >10 | |
| **505a** | >10 | |
| **506a** | >10 | |
| **507a** | 0.035 | 0.077 |
| **507b** | 0.017 | 0.039 |
| **509a** | 0.0026 | 0.0053 |
| **509b** | 0.024 | 0.059 |
| **511a** | 0.77 | 1.2 |
| **512b** | 2.3 | 3.2 |
| **516a** | 1.1 | 1.0 |
| **516c** | 0.90 | 0.73 |
| **517a** | 0.045 | 0.10 |
| **518a** | 4.2 | 2.1 |
| **519a** | 3.3 | 2.2 |
| **520a** | 0.024 | 0.020 |
| **521a** | 9.4 | 8.7 |
| **522a** | 6.7 | 6.6 |
| **523a** | 4.2 | 7.3 |
| **526a** | >10 | >10 |
| **526c** | >10 | >10 |
| **528a** | 1.4 | 1.7 |
| **529a** | 5.2 | 7.0 |
| **530a** | 0.0063 | 0.0096 |
| **531a** | 0.0089 | 0.021 |
| **532a** | 0.48 | 0.53 |
| **534a** | 3.0 | 8.1 |
| **536a** | 0.15 | 0.35 |
| **536c** | 0.31 | 1.1 |
| **540a** | 2.3 | >10 |
| **542a** | 1.1 | 2.7 |
| **546a** | 0.015 | 0.080 |
| **550a** | 0.17 | 0.59 |
| **552a** | >10 | >10 |
| **553a** | 0.43 | 3.2 |
| **554a** | 6.0 | >10 |
| **554b** | 3.3 | >10 |
| **554c** | 2.5 | 9.0 |
| **556a** | 0.026 | 0.12 |
| **559a** | 1.0 | 2.2 |
| **560a** | 0.17 | 0.91 |
| **561a** | 0.14 | 0.22 |
| **562a** | 4.6 | 8.0 |
| **563a** | 1.9 | 2.5 |
| **564a** | 1.1 | 1.8 |
| **565a** | 0.84 | 2.8 |
| **566a** | 0.22 | 3.7 |
| **567a** | 0.56 | 5.7 |
| **567b** | 1.2 | 7.9 |
| **568a** | 5.2 | >10 |
| **568b** | 2.4 | 6.9 |
| **569a** | 0.30 | 4.9 |
| **570a** | | >10 |
| **571a** | | >10 |
| **572a** | >10 | >10 |
| **573a** | | 1.5 |
| **574a** | 2.6 | 3.3 |
| **574b** | 3.2 | 4.3 |
| **575a** | >10 | >10 |
| **575b** | >10 | >10 |
| **576a** | 0.0055 | 0.012 |
| **576b** | 0.14 | 0.23 |
| **577a** | >10 | >10 |
| **578a** | 0.70 | 0.47 |
| **578b** | 0.49 | 3.8 |
| **579a** | 2.4 | >10 |
| **580a** | 0.070 | 0.19 |
| **581a** | 0.37 | 0.45 |
| **583a** | 0.10 | |
| **585a** | 0.0033 | 0.010 |
| **588a** | 1.4 | 1.9 |
| **589a** | 0.16 | 0.16 |
| **590a** | 0.051 | 0.20 |
| **591a** | 0.24 | 0.43 |
| **592a** | 0.015 | 0.014 |
| **594a** | 0.27 | 0.59 |
| **595a** | >10 | >10 |
| **595b** | 3.8 | >10 |
| **596a** | 1.6 | 8.8 |
| **597a** | 0.0021 | 0.0069 |
| **597b** | 0.043 | 0.076 |
| **598a** | 0.054 | 0.088 |
| **598b** | 0.012 | 0.045 |
| **598c** | 0.052 | 0.13 |
| **599a** | 3.1 | >10 |
| **599b** | 0.074 | 0.48 |
| **600a** | 3.6 | >10 |
| **600b** | 0.45 | 0.33 |
| **601a** | 0.54 | 1.2 |
| **602a** | 0.13 | 0.49 |
| **603b** | 0.97 | 3.2 |
| **604a** | 5.3 | >10 |
| **604c** | 8.8 | >10 |
| **605a** | >10 | >10 |
| **605c** | 3.5 | >10 |
| **606a** | 0.87 | 3.6 |
| **607a** | 4.4 | >10 |
| **608a** | 0.21 | 0.39 |
| **608b** | 0.37 | 0.55 |
| **609a** | 0.22 | 0.21 |
| **609b** | 1.6 | 1.6 |
| **610a** | 0.30 | 0.37 |
| **610b** | 0.050 | 0.097 |
| **611a** | 0.010 | 0.015 |
| **611b** | 0.16 | 0.33 |
| **612a** | 0.090 | 0.13 |
| **612b** | 0.43 | 1.2 |
| **613a** | 0.20 | 0.27 |
| **613b** | 0.21 | 0.59 |
| **613c** | 2.2 | 7.2 |
| **614a** | 0.091 | 0.16 |
| **614b** | 0.72 | 0.84 |
| **615a** | 7.2 | 8.8 |
| **616a** | >10 | >10 |
| **617a** | 0.27 | 0.53 |
| **618a** | 0.017 | 0.037 |
| **619a** | 1.9 | 2.4 |
| **620a** | 0.76 | 0.63 |
| **621a** | 0.42 | 0.94 |
| **621b** | >10 | >10 |
| **622a** | 0.30 | 0.29 |
| **623a** | 0.047 | 0.082 |
| **624b** | 0.0035 | 0.0092 |
| **625a** | 5.1 | >10 |
| **626a** | 2.4 | 4.7 |
| **627a** | 2.3 | 5.1 |
| **628a** | 0.018 | 0.029 |
| **628b** | 0.052 | 0.075 |
| **628c** | 0.025 | 0.08 |
| **629a** | 0.13 | 0.24 |
| **630a** | 0.10 | 0.14 |
| **631a** | 0.55 | 0.84 |
| **632a** | 0.73 | 1.5 |
| **635a** | 4.1 | 6.4 |
| **636a** | >10 | >10 |
| **637a** | 0.28 | 2.0 |
| **637b** | 0.29 | 0.55 |
| **638a** | 1.8 | 4.3 |
| **639a** | 1.8 | 1.6 |
| **640a** | 0.0054 | 0.0069 |
| **641a** | 0.026 | 0.099 |
| **641b** | 0.0099 | 0.052 |
| **641c** | 0.47 | 0.67 |
| **642a** | 0.35 | 0.70 |
| **643a** | 0.18 | 0.28 |
| **643b** | 0.94 | 1.2 |
| **644a** | 2.5 | 2.1 |
| **645a** | 0.38 | 1.0 |
| **645b** | 0.60 | 1.2 |
| **646a** | 1.6 | 1.7 |
| **647a** | 1.6 | 8.2 |
| **647b** | 0.47 | 0.47 |
| **648a** | 0.43 | 0.77 |
| **648b** | 0.66 | 0.99 |
| **649a** | 0.85 | 0.92 |
| **649b** | 0.88 | 0.71 |
| **650a** | >10 | >10 |
| **650b** | 4.5 | 4.8 |
| **650c** | 8.4 | >10 |
| **650d** | 9.3 | 7.2 |
| **651a** | >10 | >10 |
| **651b** | >10 | >10 |
| **652a** | >10 | >10 |
| **652b** | >10 | >10 |
| **652c** | 4.5 | 4.1 |
| **653a** | 0.51 | 0.60 |
| **653b** | 0.022 | 0.044 |
| **654a** | 0.080 | 0.10 |
| **654b** | 0.26 | 0.27 |
| **654c** | 0.035 | 0.12 |
| **654d** | 0.032 | 0.078 |
| **655a** | 0.17 | 0.35 |
| **656a** | 2.1 | 2.0 |
| **657a** | 1.7 | >10 |
| **658a** | 0.80 | 3.0 |
| **659a** | 2.1 | 2.9 |
| **660a** | 0.39 | 0.69 |
| **660b** | 0.58 | 1.4 |
| **661a** | 0.99 | 1.8 |
| **661b** | 0.47 | 2.2 |
| **662a** | 0.0099 | 0.013 |
| **662b** | 0.0056 | 0.0063 |
| **662c** | 0.63 | 0.78 |
| **662d** | 0.18 | 0.26 |
| **663a** | 4.2 | 5.8 |
| **663b** | >10 | >10 |
| **664a** | 1.9 | >10 |
| **664b** | >10 | >10 |
| **665a** | 0.053 | 0.47 |
| **665b** | 0.71 | 3.5 |
| **666a** | 0.30 | 0.89 |
| **667a** | 7.0 | 7.4 |
| **667b** | 2.5 | 4.5 |
| **668a** | 0.19 | 0.64 |
| **668b** | 0.12 | 0.18 |
| **668c** | 0.17 | 0.68 |
| **668d** | 0.16 | 0.44 |
| **669a** | 2.9 | 6.3 |
| **671a** | 0.13 | 0.42 |
| **671b** | 0.21 | 0.84 |
| **673a** | 0.17 | 0.40 |
| **674a** | >10 | 10 |
| **675a** | 1.4 | 3.1 |
| **677a** | 9.5 | >10 |
| **680a** | 1.5 | >10 |
| **680b** | >10 | >10 |
| **680c** | 0.96 | >10 |
| **681a** | 0.10 | 0.32 |
| **682a** | 4.0 | >10 |
| **683a** | 5.2 | 6.8 |
| **684a** | 8.7 | >10 |
| **685a** | 0.24 | 1.0 |
| **686a** | 5.5 | 6.5 |
| **687a** | 0.14 | 0.25 |
| **688a** | 0.40 | 0.65 |
| **689a** | 0.014 | 0.043 |
| **689b** | 0.96 | 1.4 |
| **690a** | 2.5 | 4.9 |
| **691a** | >10 | >10 |
| **693a** | 0.42 | 1.1 |
| **694a** | 8.8 | >10 |
| **695a** | 1.6 | 1.5 |
| **700a** | >10 | >10 |
| **701a** | 0.13 | 0.18 |
| **702a** | 0.026 | 0.10 |
| **702b** | 0.028 | 0.078 |
| **703a** | 2.5 | 8.7 |
| **705a** | 5.7 | 4.9 |
| **706a** | >10 | >10 |
| **706d** | 3.7 | >10 |
| **708a** | 5.6 | 6.6 |
| **709a** | 0.20 | 1.2 |
| **712a** | 5.6 | 3.6 |
| **713a** | 2.1 | 7.5 |
| **714a** | >10 | >10 |
| **716a** | 3.9 | >10 |
| **718a** | >10 | >10 |
| **720a** | 0.025 | 0.037 |
| **721a** | 0.18 | 0.38 |
| **722a** | 8.9 | 8.8 |
| **726a** | >10 | >10 |
| **729a** | 1.9 | 1.4 |
| **729b** | 0.62 | 1.7 |
| **730a** | 0.16 | 0.50 |
| **731a** | 2.6 | 1.3 |
| **733a** | >10 | 8.5 |
| **734a** | 2.4 | 3.6 |
| **736a** | 0.12 | 0.54 |
| **737a** | 0.021 | 0.026 |
| **737b** | 0.095 | 0.10 |
| **738a** | 3.0 | 7.8 |
| **740a** | 2.0 | 3.4 |
| **741a** | 0.18 | 0.91 |
| **741b** | 0.20 | 0.36 |
| **742a** | 6.5 | 9.0 |
| **744a** | 8.1 | 6.5 |
| **745a** | 5.5 | 7.7 |
| **745b** | >10 | >10 |
| **746a** | 2.2 | 5.9 |
| **747a** | 0.014 | 0.029 |
| **747b** | 0.058 | 0.020 |
| **748a** | 6.4 | 6.7 |
| **750a** | 0.21 | 0.032 |
| **751a** | 0.28 | 0.085 |

| | | |
|---|---|---|
| **Notes:** *This value was inadvertently reported incorrectly in U.S. Provisional Application Serial No. 63/545,535. | | |

### Example B8 - Protein Production

A KRas construct of amino acid residues 1-169, harboring the G12V mutation, a C-terminal Avi tag, and an N-terminal 6x Histidine tag followed by a tobacco etch virus (TEV) protease site was expressed in *E. coli* BL21(DE3). A single colony was inoculated in 100 mL LB with 100 µg/mL ampicillin and incubated at 37 °C overnight on an orbital shaker at 225 rpm. The overnight strain was inoculated into 2L TB with 100 µg/mL ampicillin at a ratio of 1:100. The culture was grown at 37 °C on an orbital shaker until the OD₆₀₀ reached 0.8. The culture was cooled to 18 °C, induced with IPTG at a final concentration of 0.2 mM and grown 16 hours at 18 °C on an orbital shaker at 180 rpm. Cells were harvested at 13,600 x g for 5 minutes at 4 °C.

The KRas G12V protein was purified by affinity chromatography using NiNTA resin. The cell pellet resuspended in 20 mM Tris pH 8.0, 500 mM NaCl, 2 mM beta-mercaptoethanol supplemented with EDTA-free protease inhibitor (Roche). Cells were sonicated at a power of 350 W with 3 s on and 3 s off pulses for a total of 300 cycles. The lysate was centrifuged at 13,600 x g at 4 °C for 30 min. The supernatant was collected and centrifuged a second time with the same parameters. 5 mL NiNTA resin was pre-equilibrated with 20 mM Tris pH 8.0, 500 mM NaCl, 2 mM beta-mercaptoethanol, added to the cleared lysate, and incubated for 2 h at 4 °C on a rotator. The resin was washed in succession with 20 mM Tris pH 8.0, 500 mM NaCl, 10 mM imidazole, 2 mM beta-mercaptoethanol, and then 20 mM Tris pH 8.0, 500 mM NaCl, 20 mM imidazole, 2 mM beta-mercaptoethanol. Finally, the protein was eluted from the resin using 20 mM Tris pH 8.0, 500 mM NaCl, 250 mM imidazole, 2 mM beta-mercaptoethanol. The 6x Histidine tag was removed from the KRas G12V protein by overnight incubation with TEV protease at 4 °C while dialyzing against 20 mM HEPES pH 7.5, 150 mM NaCl. The Avi tag on the C terminus was specifically labeled with biotin using recombinant *BirA* enzyme during the tag cleavage process. 16 mg of recombinant *BirA* enzyme, 1 mM biotin, 7 mM ATP, and 7.5 mM MgCl₂ was added to the 4 °C overnight reaction. KRas G12V protein was subsequently mixed with NiNTA resin pre-equilibrated with 20 mM HEPES pH 7.5, 150 mM NaCl - the cleaved and biotinylated protein was collected in the flow-through.

KRas G12V protein was subsequently loaded with nucleotide by adding 10 mM EDTA and 2 mM GDP. The mixture was incubated on a rotator overnight at 4 °C. 20 mM MgCl₂ was added to quench loading and stabilize the protein. KRas G12V was further purified and excess GDP nucleotide was removed by size exclusion chromatography. The protein was loaded on a Superdex 75 pg 16/600 column equilibrated with 20 mM HEPES pH 7.5, 150 mM NaCl and eluted with an isocratic flow. Biotin incorporation and GDP loading was confirmed by mass spectrometry on an Agilent 1290 Infinity II UPLC connected to an Agilent 6545XT qTOF.

### EMBODIMENTS

### P01 EXEMPLARY EMBODIMENTS

**Embodiment 1.** A compound of Formula **(II):** 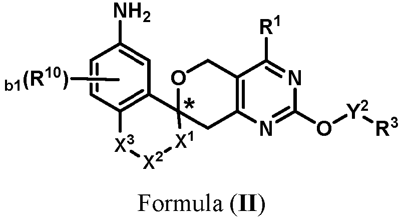 or a pharmaceutically acceptable salt thereof, wherein:
**R¹** is selected from the group consisting of:
   **(i)** a 4-10 membered heterocyclyl optionally substituted with 1-4 **R⁷**;
   **(ii)** an 8-12 membered bicyclic heterocyclyl, wherein the heterocyclyl comprises an endocyclic group selected from the group consisting of C(=O)NH and S(O)₂NH, and wherein the heterocyclyl is further optionally substituted with 1-3 **R⁷** at one or more ring carbon atoms; and
   **(iii)** 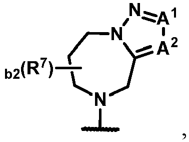 wherein **b2** is 0, 1, 2, or 3; and **A¹** and **A²** are independently selected from the group consisting of: N, CH, and C**R⁷**;
each **R⁷** is independently selected from the group consisting of **R^{a}** and **R^{b}**;
**X¹** is selected from the group consisting of a bond, S(O)₀₋₂, CH₂, CH**R^{L}**, C(**R^{L}**)₂, and O;
**X²** and **X³** are independently selected from the group consisting of: CH₂, CH**R^{L}**, C(**R^{L}**)₂, O, and S(O)₀₋₂, provided that no more than one of **X¹**, **X²**, and **X³** is selected from the group consisting of: O and S(O)₀₋₂;
**b1** is 1 or 2;
each **R¹⁰** is independently selected from the group consisting of **R^{a}** and **R^{b}**;
each **R^{L}** is independently selected from the group consisting of C₁₋₃ alkoxy, -F, CN, and C₁₋₃ alkyl optionally substituted with 1-3 **R^{c}**; or
one pair of **R^{L}** on the same or different ring carbon atom(s) taken together with the ring atom(s) connecting them form a C₃₋₆ cycloalkyl ring;
**Y²** is a bond or a straight-chain C₁₋₆ alkylene optionally substituted with 1-6 **R^{Y}**;
each **R^{Y}** is independently selected from the group consisting of: halo, cyano, -OH, oxo, C₁₋₆ alkoxy, C₁₋₆ haloalkoxy, C₁₋₆ alkyl, and C₁₋₆ haloalkyl, or
one pair of **R^{Y}** on the same or different carbon atom(s) taken together with the atom(s) connecting them forms a C₃₋₆ cycloalkyl ring or 4-6 membered heterocyclyl ring, each of which is optionally substituted with 1-3 independently selected C₁₋₃ alkyl;
**R³** is selected from the group consisting of:
   **(a)** 4-15 membered heterocyclyl optionally substituted with 1-6 substituents independently selected from the group consisting of: **R^{a}** and **R^{b}**; and
   **(b)** -N**R^{d}R^{e}**;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   **(f)** -C₁₋₆ haloalkoxy;
   **(g)** -N**R^{d}R^{e}**;
   **(h)** C(=O)C₁₋₆ alkyl;
   **(i)** C(=O)C₁₋₆ haloalkyl;
   **(j)** C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   **(o)** S(O)₀₋₂(C₁₋₆ haloalkyl);
   **(p)** S(O)₁₋₂N(**R^{f}**)₂; and
   **(q)** C₁₋₆ alkyl, C₂₋₆ alkenyl, or C₂₋₆ alkynyl, each optionally substituted with 1-6 **R^{c}**;
each **R^{b}** is independently selected from the group consisting of: **-(L^{b})_{b}-R^{b1}** and **-R^{b1}**, wherein:
   **b is** 1, 2, or 3;
   each **-L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e}**, C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)₁₋₂N(**R^{f}**)₂;
   each **R^{d}** and **R^{e}** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂₋.

**Embodiment 2.** The compound of Embodiment 1, wherein the compound is a compound of Formula (**II-a**): 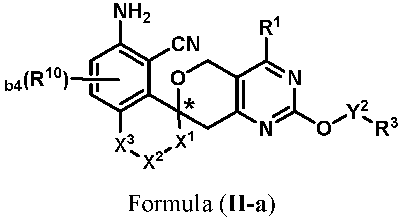 or a pharmaceutically acceptable salt thereof, wherein:
**b4** is 0 or 1; and
each **R¹⁰** is independently selected from the group consisting of: -Cl, -F, -CN, and C₁₋₃ alkyl optionally substituted with 1-3 **R^{c}**.

**Embodiment 3.** A compound of Formula (**III**): 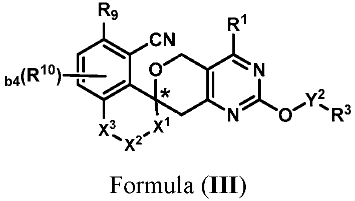 or a pharmaceutically acceptable salt thereof, wherein:
**R¹** is selected from the group consisting of:
   **(i)** a 4-10 membered heterocyclyl optionally substituted with 1-4 **R⁷**;
   **(ii)** an 8-12 membered bicyclic heterocyclyl, wherein the heterocyclyl comprises an endocyclic group selected from the group consisting of C(=O)NH and S(O)₂NH, and wherein the heterocyclyl is further optionally substituted with 1-3 **R⁷** at one or more ring carbon atoms; and
   **(iii)** 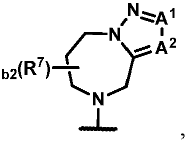 wherein **b2** is 0, 1, 2, or 3; and **A¹** and **A²** are independently selected from the group consisting of: N, CH, and C**R⁷**;
each **R⁷** is independently selected from the group consisting of **R^{a}** and **R^{b}**;
**X¹** is selected from the group consisting of a bond, S(O)₀₋₂, CH₂, CH**R^{L}**, C(**R^{L}**)₂, and O;
**X²** and **X³** are independently selected from the group consisting of: CH₂, CH**R^{L}**, C(**R^{L}**)₂, O, and S(O)₀₋₂, provided that no more than one of **X¹**, **X²**, and **X³** is selected from the group consisting of: O and S(O)₀₋₂;
**R⁹** is selected from the group consisting of: H, N**R^{d}R^{e}**, -OH, and halo;
**b4** is 0 or 1;
each **R¹⁰** is independently selected from the group consisting of **R^{a}** and **R^{b}**;
each **R^{L}** is independently selected from the group consisting of C₁₋₃ alkoxy, -F, CN, and C₁₋₃ alkyl optionally substituted with 1-3 **R^{c}**; or
one pair of **R^{L}** on the same or different ring carbon atom(s) taken together with the ring atom(s) connecting them form a C₃₋₆ cycloalkyl ring;
**Y²** is a bond or a straight-chain C₁₋₆ alkylene optionally substituted with 1-6 **R^{Y}**;
each **R^{Y}** is independently selected from the group consisting of: halo, cyano, -OH, oxo, C₁₋₆ alkoxy, C₁₋₆ haloalkoxy, C₁₋₆ alkyl, and C₁₋₆ haloalkyl, or
one pair of **R^{Y}** on the same or different carbon atom(s) taken together with the atom(s) connecting them forms a C₃₋₆ cycloalkyl ring or 4-6 membered heterocyclyl ring, each of which is optionally substituted with 1-3 independently selected C₁₋₃ alkyl;
**R³** is selected from the group consisting of:
   **(a)** 4-15 membered heterocyclyl optionally substituted with 1-6 substituents independently selected from the group consisting of: **R^{a}** and **R^{b}**; and
   **(b)** -N**R^{d}R^{e}**;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   **(f)** -C₁₋₆ haloalkoxy;
   **(g)** -N**R^{d}R^{e}**;
   **(h)** C(=O)C₁₋₆ alkyl;
   **(i)** C(=O)C₁₋₆ haloalkyl;
   **(j)** C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   **(o)** S(O)₀₋₂(C₁₋₆ haloalkyl);
   **(p)** S(O)₁₋₂N(**R^{f}**)₂; and
   **(q)** C₁₋₆ alkyl, C₂₋₆ alkenyl, or C₂₋₆ alkynyl, each optionally substituted with 1-6 **R^{c}**;
each **R^{b}** is independently selected from the group consisting of: **-(L^{b})_{b}-R^{b1}** and **-R^{b1}**, wherein:
   **b is** 1, 2, or 3;
   each **-L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e}**, C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)₁₋₂N(**R^{f}**)₂;
   each **R^{d}** and **R^{e}** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂₋.

**Embodiment 4.** The compound of Embodiment 3, wherein **R⁹** is -NH₂; and each **R¹⁰** is independently selected from the group consisting of: -Cl, -F, -CN, and C₁₋₃ alkyl optionally substituted with 1-3 **R^{c}**.

**Embodiment 5.** The compound of any one of Embodiments 1-4, wherein **X¹** is CH₂ or CH**R^{L}**.

**Embodiment 6.** The compound of any one of Embodiments 1-5, wherein **X²** and **X³** are independently selected from the group consisting of: CH₂, CH**R^{L}**, and C(**R^{L}**)₂.

**Embodiment 7.** The compound of any one of Embodiments 1-6, wherein **X¹** is CH₂; and **X²** and **X³** are both CH₂.

**Embodiment 8.** The compound of any one of Embodiments 1-6, wherein at least one (e.g., one) of **X¹**, **X²**, and **X³** is selected from the group consisting of: CH**R^{L}** and C(**R^{L}**)₂.

**Embodiment** 9. The compound of any one of Embodiments 1-6 or 8, wherein **X¹** is CH₂; and **X²** and **X³** are independently selected from the group consisting of: CH₂, CH**R^{L}**, and C(**R^{L}**)₂, provided that 1-2 of **X²** and **X³** is independently CH**R^{L}** or C(**R^{L}**)₂.

**Embodiment 10.** The compound of any one of Embodiments 1-6 or 8-9, wherein **X¹** is CH₂; **X²** is CH₂; and **X³** is CH**R^{L}**.

**Embodiment 11.** The compound of any one of Embodiments 1-5, wherein one of **X²** and **X³** is -O-; and the other of **X²** and **X³** is selected from the group consisting of: CH₂, CH**R^{L}**, and C(**R^{L}**)₂.

**Embodiment 12.** The compound of any one of Embodiments 1-5 or 11, wherein **X²** is -O-; and **X³** is selected from the group consisting of: CHz, CH**R^{L}**, and C(**R^{L}**)₂.

**Embodiment 13.** The compound of any one of Embodiments 1-12, wherein each **R^{L}** is independently selected from the group consisting of: CH₃, CF₃, CHF₂, and CH₂F.

**Embodiment 14.** The compound of Embodiment 1, wherein the 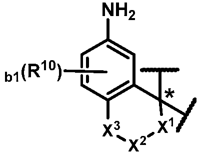 moiety is selected from the group consisting of: 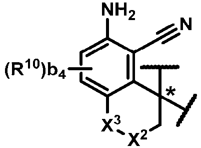 and 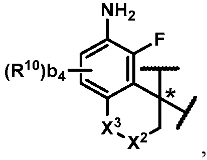 wherein:
**b4** is 0 or 1;
**X²** is -O- or -CH₂-;
**X³** is -CH₂- or -CH**R^{L}**-, wherein **R^{L}** is C₁₋₃ alkyl (e.g., methyl); and
each **R¹⁰** is independently selected from the group consisting of: -Cl, -F, -CN, and C₁₋₃ alkyl optionally substituted with 1-3 **R^{c}**.

**Embodiment 15.** The compound of Embodiment 14, wherein the 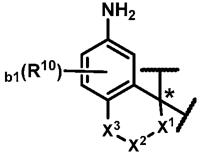 moiety is selected from the group consisting of:

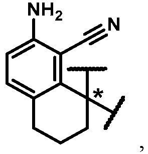

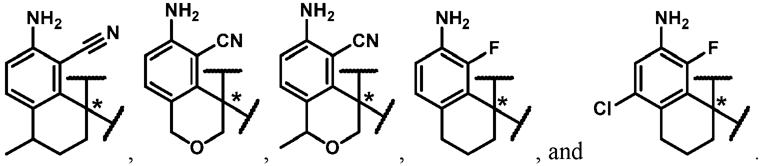

**Embodiment 16.** A compound of Formula **(IV):** 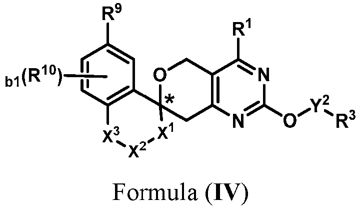 or a pharmaceutically acceptable salt thereof, wherein:
**X¹** is selected from the group consisting of a bond, S(O)₀₋₂, CH₂, CH**R^{L}**, C(**R^{L}**)₂, and O;
**X²** and **X³** are independently selected from the group consisting of: CH₂, CH**R^{L}**, C(**R^{L}**)₂, O, and S(O)₀₋₂, provided that at least one of **X¹**, **X²**, and **X³** is CH**R^{L}** or C(**R^{L}**)₂;
further provided that no more than one of **X¹**, **X²**, and **X³** is selected from the group consisting of: O and S(O)₀₋₂;
**b1** is 0, 1 or 2;
**R⁹** is selected from the group consisting of: H, OH, N**R^{d}R^{e}**, and halo;
each **R¹⁰** is independently selected from the group consisting of **R^{a}** and **R^{b}**;
each **R^{L}** is independently selected from the group consisting of C₁₋₃ alkoxy, -F, CN, and C₁₋₃ alkyl optionally substituted with 1-3 **R^{c}**;
**R¹** is selected from the group consisting of:
   **(i)** a 4-10 membered heterocyclyl optionally substituted with 1-4 **R⁷**;
   **(ii)** an 8-12 membered bicyclic heterocyclyl, wherein the heterocyclyl comprises an endocyclic group selected from the group consisting of C(=O)NH and S(O)₂NH, and wherein the heterocyclyl is further optionally substituted with 1-3 **R⁷** at one or more ring carbon atoms; and
   **(iii)** 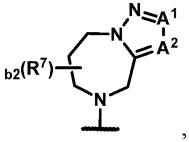 wherein **b2** is 0, 1, 2, or 3; and **A¹** and **A²** are independently selected from the group consisting of: N, CH, and C**R⁷**;
each **R⁷** is independently selected from the group consisting of **R^{a}** and **R^{b}**;
**Y²** is a bond or a straight-chain C₁₋₆ alkylene optionally substituted with 1-6 **R^{Y}**;
each **R^{Y}** is independently selected from the group consisting of: halo, cyano, -OH, oxo, C₁₋₆ alkoxy, C₁₋₆ haloalkoxy, C₁₋₆ alkyl, and C₁₋₆ haloalkyl, or
one pair of **R^{Y}** on the same or different carbon atom(s) taken together with the atom(s) connecting them forms a C₃₋₆ cycloalkyl ring or 4-6 membered heterocyclyl ring, each of which is optionally substituted with 1-3 independently selected C₁₋₃ alkyl;
**R³** is selected from the group consisting of:
   **(a)** 4-15 membered heterocyclyl optionally substituted with 1-6 substituents independently selected from the group consisting of: **R^{a}** and **R^{b}**; and
   **(b)** -N**R^{d}R^{e}**;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   **(f)** -C₁₋₆ haloalkoxy;
   **(g)** -N**R^{d}R^{e}**;
   **(h)** C(=O)C₁₋₆ alkyl;
   **(i)** C(=O)C₁₋₆ haloalkyl;
   **(j)** C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   **(o)** S(O)₀₋₂(C₁₋₆ haloalkyl);
   **(p)** S(O)₁₋₂N(**R^{f}**)₂; and
   **(q)** C₁₋₆ alkyl, C₂₋₆ alkenyl, or C₂₋₆ alkynyl, each optionally substituted with 1-6 **R^{c}**;
each **R^{b}** is independently selected from the group consisting of: **-(L^{b})_{b}-R^{b1}** and **-R^{b1}**, wherein:
   **b is** 1, 2, or 3;
   each **-L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e}**, C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)₁₋₂N(**R^{f}**)₂;
   each **R^{d}** and **R^{e}** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂₋.

**Embodiment 17.** The compound of Embodiment 16, wherein the compound is a compound of Formula **(IV-a):** 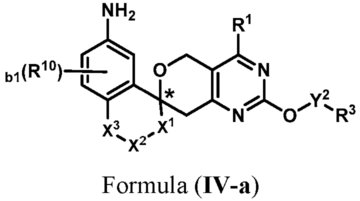 or a pharmaceutically acceptable salt thereof, wherein:
each **R¹⁰** is independently selected from the group consisting of: -Cl, -F, -CN, and C₁₋₃ alkyl optionally substituted with 1-3 **R^{c}**.

**Embodiment 18.** The compound of Embodiments 16, wherein the compound is a compound of Formula **(IV-b):** 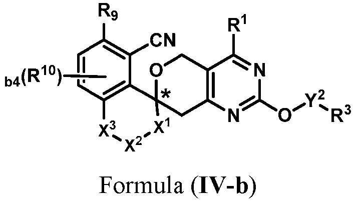 or a pharmaceutically acceptable salt thereof, wherein:
**b4** is 0 or 1; and
each **R¹⁰** is independently selected from the group consisting of: -Cl, -F, -CN, and C₁₋₃ alkyl optionally substituted with 1-3 **R^{c}.**

**Embodiment 19.** The compound of any one of Embodiments 16-18, wherein **X¹** is CH₂.

**Embodiment 20.** The compound of any one of Embodiments 16-19, wherein **X²** is CH₂; and **X³** is CH**R^{L}**.

**Embodiment 21.** The compound of any one of Embodiments 16-19, wherein **X²** is -O-; and **X³** is selected from the group consisting of: CH**R^{L}** and C(**R^{L}**)₂.

**Embodiment 22.** The compound of any one of Embodiments 16-21, wherein each **R^{L}** is independently selected from the group consisting of: CH₃, CF₃, CHF₂, and CH₂F.

**Embodiment 23.** The compound of any one of Embodiments 16-18, wherein the compound is a compound of Formula **(IV-c):** 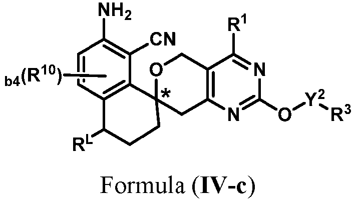 or a pharmaceutically acceptable salt thereof, wherein:
**b4 is** 0 or 1; and
each **R¹⁰** is independently selected from the group consisting of: -Cl, -F, -CN, and C₁₋₃ alkyl optionally substituted with 1-3 **R^{c}.**

**Embodiment 24.** The compound of any one of Embodiments 1-23, wherein **Y²** is -CH₂-; and **R³** is a 4-10 membered heterocyclyl having one ring nitrogen atom and 0-1 additional ring heteroatom selected from the group consisting of oxygen and nitrogen, wherein the heterocyclyl is optionally substituted with 1-6 **R^{a}**.

**Embodiment 25.** The compound of any one of Embodiments 1-24, wherein **R³** is 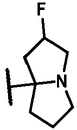 (e.g., 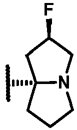 ).

**Embodiment 26.** The compound of any one of Embodiments 1-25, wherein **R¹** is 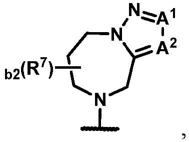 wherein b2 is 0, 1, or 2, and **A¹** and **A²** are independently selected from the group consisting of: N, CH, and C**R⁷**.

**Embodiment 27.** The compound of any one of Embodiments 1-26, wherein **R¹** is 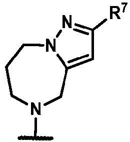 or

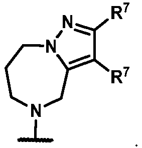

**Embodiment 28.** The compound of any one of Embodiments 1-27, wherein **R¹** is 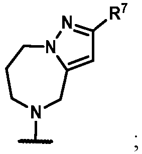 and **R⁷** is selected from the group consisting of: C(=O)N(**R^{f}**)₂, C(O)N(C₁₋₃ alkyl)**R^{b1}**, -C(O)N(H)**R^{b1}**, **R^{b1}**, and C(O)**R^{b1}**.

**Embodiment 29.** The compound of Embodiment 28, wherein **R⁷** is selected from the group consisting of:
**(a)** C(=O)N(**R^{f}**)₂, wherein each **R^{f}** is independently H or C₁₋₃ alkyl optionally substituted with 1-3 **R^{h}**;
**(b)** C(O)N(C₁₋₃ alkyl)**R^{b1}** or -C(O)N(H)**R^{b1}**, wherein: **R^{b1}** is C₃₋₆ cycloalkyl or 4-6 membered heterocyclyl, each of which is optionally substituted with 1-3 **R^{g}**; and
**(c)** C(O)**R^{b1}**, wherein **R^{b1}** is heterocyclyl optionally substituted with 1-3 **R^{g}**, wherein **R^{b1}** is attached to the C(O) via a ring nitrogen atom.

**Embodiment 30.** The compound of any one of Embodiments 27-29, wherein **R⁷** is C(=O)N(**R^{f}**)₂, wherein each **R^{f}** is independently H or C₁₋₃ alkyl optionally substituted with 1-3 **R^{h}**.

**Embodiment 31.** The compound of any one of Embodiments 1-27, wherein **R¹** is 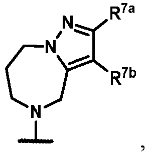 wherein **R^{7a}** and **R^{7b}** are independently selected **R⁷**.

**Embodiment 32.** The compound of Embodiment 31, wherein **R^{7a}** is selected from the group consisting of: C(=O)N(**R^{f}**)₂, C(O)N(C₁₋₃ alkyl)**R^{b1}**, -C(O)N(H)**R^{b1}**, **R^{b1}**, and C(O)**R^{b1}**; and
**R^{7b}** is -halo, -CN, and C₁₋₃ alkyl optionally substituted with 1-3 **R^{c}.**

**Embodiment 33.** The compound of Embodiment 32, wherein **R^{7a}** is selected from the group consisting of:
**(d)** C(=O)N(**R^{f}**)₂, wherein each **R^{f}** is independently H or C₁₋₃ alkyl optionally substituted with 1-3 **R^{h}**;
**(e)** C(O)N(C₁₋₃ alkyl)**R^{b1}** or -C(O)N(H)**R^{b1}**, wherein: **R^{b1}** is C₃₋₆ cycloalkyl or 4-6 membered heterocyclyl, each of which is optionally substituted with 1-3 **R^{g}**; and
**(f)** C(O)**R^{b1}**, wherein **R^{b1}** is heterocyclyl optionally substituted with 1-3 **R^{g}**, wherein **R^{b1}** is attached to the C(O) via a ring nitrogen atom.

**Embodiment 34.** The compound of any one of Embodiments 1-25, wherein **R¹** is a 7-10 (e.g., 7) membered heterocyclyl having one ring nitrogen atom, one ring oxygen atom, and no additional ring heteroatoms, wherein the 7-10 membered heterocyclyl is optionally substituted with 1-4 **R⁷**.

**Embodiment 35.** The compound of Embodiment 34, wherein **R¹** is 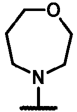 optionally substituted with 1-4 **R⁷** at one or more ring carbon atoms (e.g., **R¹** is 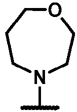 ).

**Embodiment 36.** The compound of Embodiment 35, wherein each **R⁷** is independently selected from the group consisting of: -OH; -CN; -F; and C₁₋₃ alkyl optionally substituted with 1-3 **R^{c}**, wherein:
each **R^{c}** is independently selected from the group consisting of: -F, -OH, and -CN.

**Embodiment 37.** The compound of Embodiment 1, wherein the compound is a compound of Formula (**II-a1**) or (**II-b1**): 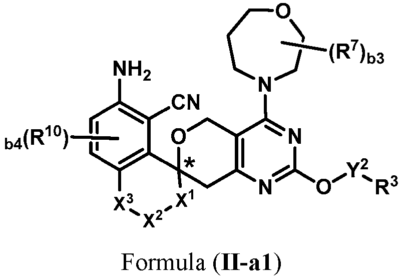 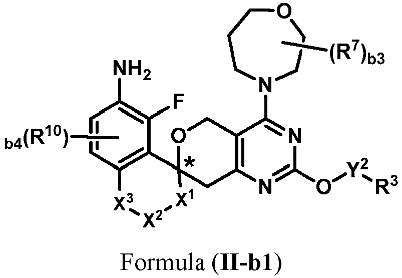 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c}**;
**b3** is 0, 1, 2, or 3;
**X¹** is CH₂;
**X²** and **X³** are independently selected from the group consisting of: O, CH₂, CH**R^{L}**, and C(**R^{L}**)₂; and
**b1** is 0, 1, or 2.

**Embodiment 38.** The compound of Embodiment 3, wherein the compound is a compound of Formula (**III-1**): 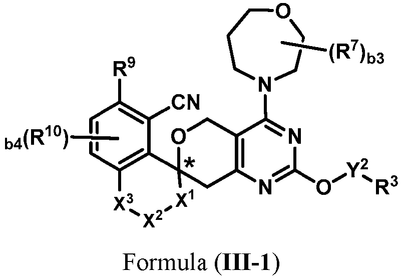 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c}**;
**b3** is 0, 1, 2, or 3;
**X¹** is CH₂;
**X²** and **X³** are independently selected from the group consisting of: O, CH₂, CH**R^{L}**, and C(**R^{L}**)₂; and
**b1** is 0, 1, or 2.

**Embodiment 39.** The compound of Embodiment 38, wherein **R⁹** is N**R^{d}R^{e}** (e.g., - NH₂).

**Embodiment 40.** The compound of Embodiment 16, wherein the compound is a compound of Formula (**IV-a1**) or **(IV-b1):** 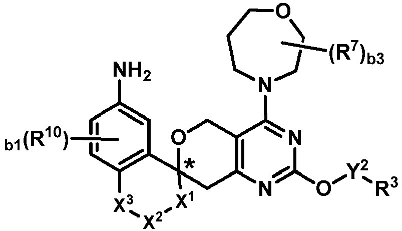 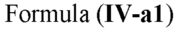 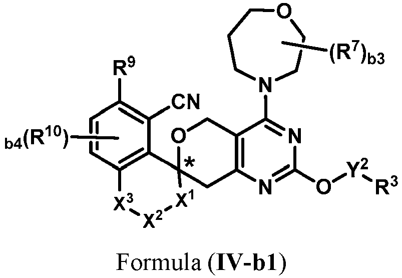 or a pharmaceutically acceptable salt thereof, wherein:
**b4** is 0 or 1;
**b1** is 0, 1, or 2;
each **R¹⁰** is independently selected from the group consisting of: -Cl, -F, -CN, and C₁₋₃ alkyl optionally substituted with 1-3 **R^{c}**;
**b3** is 0, 1, 2, or 3;
**X¹** is CH₂;
one of **X²** and **X³** is independently selected from the group consisting of: CH₂, CH**R^{L}**, and C(**R^{L}**)₂; and
the other of **X²** and **X³** is CH₂ or O.

**Embodiment 41.** The compound of Embodiment 40, wherein **X²** is CH₂; and **X³** is CH**R^{L}**.

**Embodiment 42.** The compound of Embodiment 1, wherein the compound is a compound of Formula (**II-a2**) or (**II-b2**): 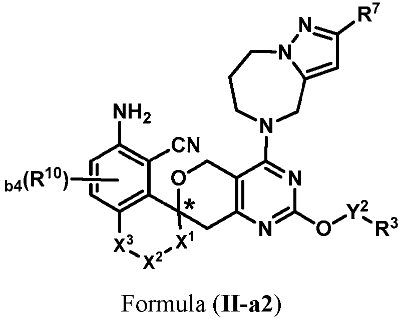 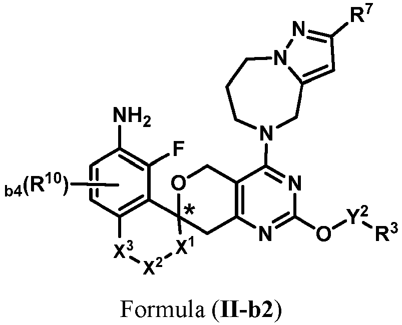 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c}**;
**R⁷** is selected from the group consisting of: C(=O)N(**R^{f}**)₂, C(O)N(C₁₋₃ alkyl)**R^{b1}**, - C(O)N(H)**R^{b1}**, **R^{b1}**, and C(O)**R^{b1}**;
**X¹** is CH₂;
**X²** and **X³** are independently selected from the group consisting of: O, CH₂, CH**R^{L}**, and C(**R^{L}**)₂; and
**b1** is 0, 1, or 2.

**Embodiment 43.** The compound of Embodiment 3, wherein the compound is a compound of Formula (**III-2**): 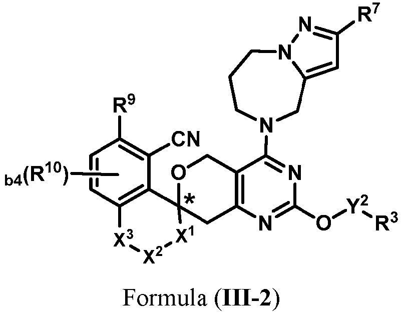 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c}**;
**R⁷** is selected from the group consisting of: C(=O)N(**R^{f}**)₂, C(O)N(C₁₋₃ alkyl)**R^{b1}**, - C(O)N(H)**R^{b1}**, **R^{b1}**, and C(O)**R^{b1}**;
**X¹** is CH₂;
**X²** and **X³** are independently selected from the group consisting of: O, CH₂, CH**R^{L}**, and C(**R^{L}**)₂; and
**b1** is 0, 1, or 2.

**Embodiment 44.** The compound of Embodiment 43, wherein **R⁹** is N**R^{d}R^{e}** (e.g., - NH₂).

**Embodiment 45.** The compound of Embodiment 16, wherein the compound is a compound of Formula (**IV-a2**) or **(IV-b2):** 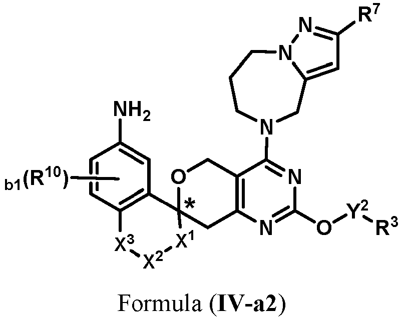 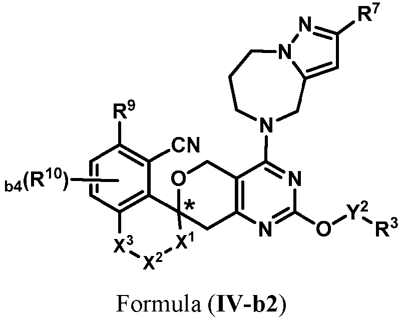 or a pharmaceutically acceptable salt thereof, wherein:
**b4** is 0 or 1;
**b1** is 0, 1, or 2;
each **R¹⁰** is independently selected from the group consisting of: -Cl, -F, -CN, and C₁₋₃ alkyl optionally substituted with 1-3 **R^{c}**;
**R⁷** is selected from the group consisting of: C(=O)N(**R^{f}**)₂, C(O)N(C₁₋₃ alkyl)**R^{b1}**, - C(O)N(H)**R^{b1}**, **R^{b1}**, and C(O)**R^{b1}**;
**X¹** is CH₂;
one of **X²** and **X³** is independently selected from the group consisting of: CH₂, CH**R^{L}**, and C(**R^{L}**)₂; and
the other of **X²** and **X³** is CH₂ or O.

**Embodiment 46.** The compound of Embodiment 45, wherein **X²** is CH₂; and **X³** is CH**R^{L}**.

**Embodiment 47.** The compound of any one of Embodiments 42-46, wherein **R⁷** is selected from the group consisting of:
**(a)** C(=O)N(**R^{f}**)₂, wherein each **R^{f}** is independently H or C₁₋₃ alkyl optionally substituted with 1-3 **R^{h}**;
**(b)** C(O)N(C₁₋₃ alkyl)**R^{b1}** or -C(O)N(H)**R^{b1}**, wherein: **R^{b1}** is C₃₋₆ cycloalkyl or 4-6 membered heterocyclyl, each of which is optionally substituted with 1-3 **R^{g}**; and
**(c)** C(O)**R^{b1}**, wherein **R^{b1}** is heterocyclyl optionally substituted with 1-3 **R^{g}**, wherein **R^{b1}** is attached to the C(O) via a ring nitrogen atom.

**Embodiment 48.** The compound of any one of Embodiments 37-47, wherein **Y²** is -CH₂-; and **R³** is a 4-10 membered heterocyclyl having one ring nitrogen atom and 0-1 additional ring heteroatom selected from the group consisting of oxygen and nitrogen, wherein the heterocyclyl is optionally substituted with 1-6 **R^{a}**.

**Embodiment 49.** The compound of Embodiment 48, wherein **R³** is 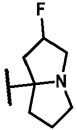 (e.g., 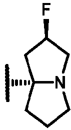 ).

**Embodiment 50.** The compound of any one of Embodiments 1-49, wherein the ring carbon atom labelled with * has (S)-stereochemistry.

**Embodiment 51.** The compound of Embodiment 1, wherein the compound of Formula (**II**) is selected from the group consisting of Compound Nos. **139, 139a, 139b, 139c, 158, 158a, 158b, 158c, 160, 160a, 161, 161a, 164, 164a, 164b, 170, 170a, 171, 171a, 176, 176a, 176b, 176c, 176d, 178, 178a, 178b, 179, 179a, 180, 180a, 181,** and **181a of Table C1,** or a pharmaceutically acceptable salt thereof.

**Embodiment 52.** The compound of Embodiment 3, wherein the compound of Formula (**III**) is selected from the group consisting of Compound Nos. **158, 158a, 158b, 158c, 161, 161a, 176, 176a, 176b, 176c, 176d, 177, 177a, 178, 178a, 178b, 179, 179a, 180,** and **180a of Table C1,** or a pharmaceutically acceptable salt thereof.

**Embodiment 53.** The compound of Embodiment 16, wherein the compound of Formula (IV) is selected from the group consisting of Compound Nos. **124, 124a, 124b, 125, 125a, 130, 130a, 131, 131a, 133, 133a, 133b, 134, 134a, 138, 138a, 148, 148a, 162, 162a, 163, 163a, 175,** and **175a** of **Table C1,** or a pharmaceutically acceptable salt thereof.

### P06 EXEMPLARY EMBODIMENTS

**Embodiment 1.** A compound of Formula **(II):** 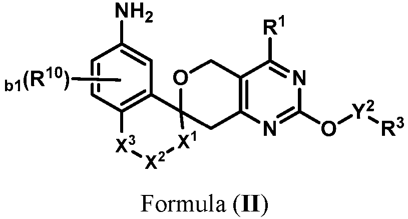 or a pharmaceutically acceptable salt thereof, wherein:
**R¹** is selected from the group consisting of:
   **(i)** a 4-10 membered heterocyclyl optionally substituted with 1-4 **R⁷**;
   **(ii)** an 8-12 membered bicyclic heterocyclyl, wherein the heterocyclyl comprises an endocyclic group selected from the group consisting of C(=O)NH and S(O)₂NH, and wherein the heterocyclyl is further optionally substituted with 1-3 **R⁷** at one or more ring carbon atoms; and
   **(iii)** 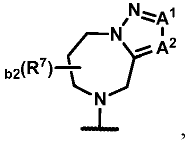 wherein **b2** is 0, 1, 2, or 3; and **A¹** and **A²** are independently selected from the group consisting of: N, CH, and C**R⁷**;
each **R⁷** is independently selected from the group consisting of **R^{a}** and **R^{b}**;
**X¹** is selected from the group consisting of a bond, S(O)₀₋₂, CH₂, CH**R^{L}**, C(**R^{L}**)₂, and O;
**X²** and **X³** are independently selected from the group consisting of: CH₂, CH**R^{L}**, C(**R^{L}**)₂, O, and S(O)₀₋₂, provided that no more than one of **X¹**, **X²**, and **X³** is selected from the group consisting of: O and S(O)₀₋₂;
**b1** is 1 or 2;
each **R¹⁰** is independently selected from the group consisting of **R^{a}** and **R^{b}**;
each **R^{L}** is independently selected from the group consisting of C₁₋₃ alkoxy, -F, CN, and C₁₋₃ alkyl optionally substituted with 1-3 **R^{c}**; or
one pair of **R^{L}** on the same or different ring carbon atom(s) taken together with the ring atom(s) connecting them form a C₃₋₆ cycloalkyl ring;
**Y²** is a bond or a straight-chain C₁₋₆ alkylene optionally substituted with 1-6 **R^{Y}**;
each **R^{Y}** is independently selected from the group consisting of: halo, cyano, -OH, oxo, C₁₋₆ alkoxy, C₁₋₆ haloalkoxy, C₁₋₆ alkyl, and C₁₋₆ haloalkyl, or
one pair of **R^{Y}** on the same or different carbon atom(s) taken together with the atom(s) connecting them forms a C₃₋₆ cycloalkyl ring or 4-6 membered heterocyclyl ring, each of which is optionally substituted with 1-3 independently selected C₁₋₃ alkyl;
**R³** is selected from the group consisting of:
   **(a)** 4-15 membered heterocyclyl optionally substituted with 1-6 substituents independently selected from the group consisting of: **R^{a}** and **R^{b}**; and
   **(b)** -N**R^{d}R^{e}**;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   **(f)** -C₁₋₆ haloalkoxy;
   **(g)** -N**R^{d}R^{e}**;
   **(h)** C(=O)C₁₋₆ alkyl;
   **(i)** C(=O)C₁₋₆ haloalkyl;
   **(j)** C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   **(o)** S(O)₀₋₂(C₁₋₆ haloalkyl);
   **(p)** S(O)₁₋₂N(**R^{f}**)₂; and
   **(q)** C₁₋₆ alkyl, C₂₋₆ alkenyl, or C₂₋₆ alkynyl, each optionally substituted with 1-6 **R^{c}**;
each **R^{b}** is independently selected from the group consisting of: **-(L^{b})_{b}-R^{b1}** and **-R^{b1}**, wherein:
   **b** is 1, 2, or 3;
   each **-L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e},** C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)₁₋₂N(**R^{f}**)₂;
   each **R^{d}** and **R^{e}** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂-.

**Embodiment 2.** The compound of Embodiment 1, wherein the compound is a compound of Formula (**II-a**): 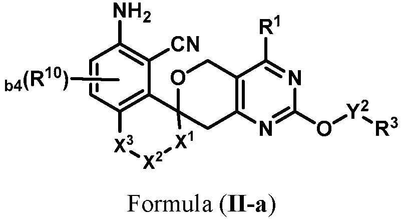 or a pharmaceutically acceptable salt thereof, wherein:
**b4** is 0 or 1; and
each **R¹⁰** is independently selected from the group consisting of: -Cl, -F, -CN, and C₁₋₃ alkyl optionally substituted with 1-3 **R^{c}.**

**Embodiment 3.** The compound of Embodiment 1, wherein the compound is a compound of Formula (**II-b**): 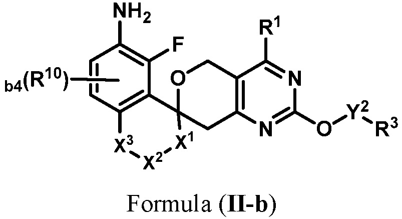 or a pharmaceutically acceptable salt thereof, wherein:
**b4** is 0 or 1; and
each **R¹⁰** is independently selected from the group consisting of: -Cl, -F, -CN, and C₁₋₃ alkyl optionally substituted with 1-3 **R^{c}.**

**Embodiment 4.** The compound of Embodiment 2 or 3, wherein **b4** is 1.

**Embodiment 5.** The compound of Embodiment 4, wherein **R¹⁰** is *ortho* to **X³**.

**Embodiment 6.** A compound of Formula (**III**): 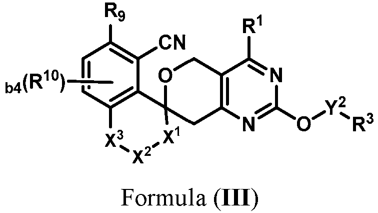 or a pharmaceutically acceptable salt thereof, wherein:
**R¹** is selected from the group consisting of:
   **(i)** a 4-10 membered heterocyclyl optionally substituted with 1-4 **R⁷**;
   **(ii)** an 8-12 membered bicyclic heterocyclyl, wherein the heterocyclyl comprises an endocyclic group selected from the group consisting of C(=O)NH and S(O)₂NH, and wherein the heterocyclyl is further optionally substituted with 1-3 **R⁷** at one or more ring carbon atoms; and
   **(iii)** 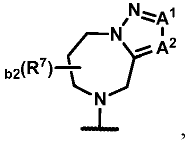 wherein **b2** is 0, 1, 2, or 3; and **A¹** and **A²** are independently selected from the group consisting of: N, CH, and C**R⁷**;
each **R⁷** is independently selected from the group consisting of **R^{a}** and **R^{b}**;
**X¹** is selected from the group consisting of a bond, S(O)₀₋₂, CH₂, CH**R^{L}**, C(**R^{L}**)₂, and O;
**X²** and **X³** are independently selected from the group consisting of: CH₂, CH**R^{L}**, C(**R^{L}**)₂, O, and S(O)₀₋₂, provided that no more than one of **X¹**, **X²**, and **X³** is selected from the group consisting of: O and S(O)₀₋₂;
**R⁹** is selected from the group consisting of: H, N**R^{d}R^{e}**, -OH, and halo;
**b4** is 0 or 1;
each **R¹⁰** is independently selected from the group consisting of **R^{a}** and **R^{b}**;
each **R^{L}** is independently selected from the group consisting of C₁₋₃ alkoxy, -F, CN, and C₁₋₃ alkyl optionally substituted with 1-3 **R^{c}**; or
one pair of **R^{L}** on the same or different ring carbon atom(s) taken together with the ring atom(s) connecting them form a C₃₋₆ cycloalkyl ring;
**Y²** is a bond or a straight-chain C₁₋₆ alkylene optionally substituted with 1-6 **R^{Y}**;
each **R^{Y}** is independently selected from the group consisting of: halo, cyano, -OH, oxo, C₁₋₆ alkoxy, C₁₋₆ haloalkoxy, C₁₋₆ alkyl, and C₁₋₆ haloalkyl, or
one pair of **R^{Y}** on the same or different carbon atom(s) taken together with the atom(s) connecting them forms a C₃₋₆ cycloalkyl ring or 4-6 membered heterocyclyl ring, each of which is optionally substituted with 1-3 independently selected C₁₋₃ alkyl;
**R³** is selected from the group consisting of:
   **(a)** 4-15 membered heterocyclyl optionally substituted with 1-6 substituents independently selected from the group consisting of: **R^{a}** and **R^{b}**; and
   **(b)** -N**R^{d}R^{e}**;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   **(f)** -C₁₋₆ haloalkoxy;
   **(g)** -N**R^{d}R^{e}**;
   **(h)** C(=O)C₁₋₆ alkyl;
   **(i)** C(=O)C₁₋₆ haloalkyl;
   **(j)** C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   **(o)** S(O)₀₋₂(C₁₋₆ haloalkyl);
   **(p)** S(O)₁₋₂N(**R^{f}**)₂; and
   **(q)** C₁₋₆ alkyl, C₂₋₆ alkenyl, or C₂₋₆ alkynyl, each optionally substituted with 1-6 **R^{c}**;
each **R^{b}** is independently selected from the group consisting of: **-(L^{b})_{b}-R^{b1}** and -**R^{b1}**, wherein:
   **b** is 1, 2, or 3;
   each **-L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e},** C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)₁₋₂N(**R^{f}**)₂;
   each **R^{d}** and **R^{e}** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂-.

**Embodiment 7.** The compound of Embodiment 6, wherein **R⁹** is -NH₂; and each **R¹⁰** is independently selected from the group consisting of: -Cl, -F, -CN, and C₁₋₃ alkyl optionally substituted with 1-3 **R^{c}.**

**Embodiment 8.** The compound of any one of Embodiments 1-7, wherein **X¹** is CH₂ or CH**R^{L}**.

**Embodiment 9.** The compound of any one of Embodiments 1-8, wherein **X²** and **X³** are independently selected from the group consisting of: CH₂, CH**R^{L}**, and C(**R^{L}**)₂.

**Embodiment 10.** The compound of any one of Embodiments 1-9, wherein **X¹** is CH₂; and **X²** and **X³** are both CH₂.

**Embodiment 11.** The compound of any one of Embodiments 1-9, wherein at least one (e.g., one) of **X¹**, **X²**, and **X³** is selected from the group consisting of: CH**R^{L}** and C(**R^{L}**)₂.

**Embodiment 12.** The compound of any one of Embodiments 1-9 or 11, wherein **X¹** is CH₂; and **X²** and **X³** are independently selected from the group consisting of: CH₂, CH**R^{L}**, and C(**R^{L}**)₂, provided that 1-2 of **X²** and **X³** is independently CH**R^{L}** or C(**R^{L}**)₂.

**Embodiment 13.** The compound of any one of Embodiments 1-9 or 11-12, wherein **X¹** is CH₂; **X²** is CH₂; and **X³** is CH**R^{L}** (e.g., **X³** is C(H)CF₃; or e.g., **X³** is C(H)CH₃).

**Embodiment 14.** The compound of any one of Embodiments 1-13, wherein each **R^{L}** is independently selected from the group consisting of: CH₃, CF₃, CHF₂, and CH₂F (e.g., CH₃).

**Embodiment 15.** The compound of Embodiment 1, wherein the 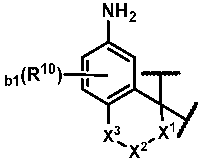 moiety is selected from the group consisting of: 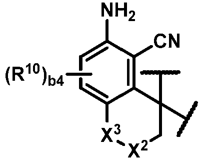 and 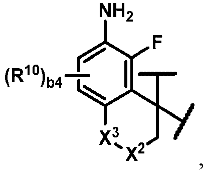 wherein:
**b4** is 0 or 1;
**X²** is -O- or -CH₂-;
**X³** is -CH₂- or -CH**R^{L}**-, wherein **R^{L}** is C₁₋₃ alkyl (e.g., methyl); and
each **R¹⁰** is independently selected from the group consisting of: -Cl, -F, -CN, and C₁₋₃ alkyl optionally substituted with 1-3 **R^{c}.**

**Embodiment 16.** The compound of Embodiment 15, wherein the 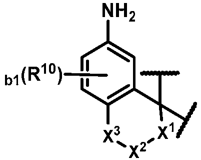 moiety is selected from the group consisting of:

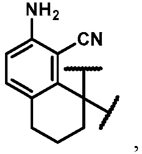

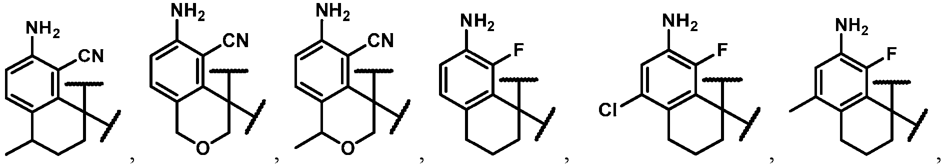

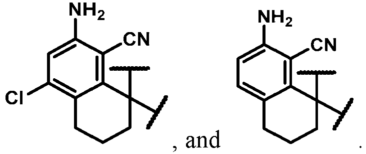

**Embodiment** 17. A compound of Formula (IV): 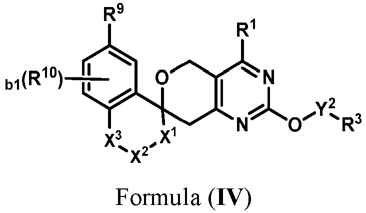 or a pharmaceutically acceptable salt thereof, wherein:
**X¹** is selected from the group consisting of a bond, S(O)₀₋₂, CH₂, CH**R^{L}**, C(**R^{L}**)₂, and O;
**X²** and **X³** are independently selected from the group consisting of: CH₂, CH**R^{L}**, C(**R^{L}**)₂, O, and S(O)₀₋₂, provided that at least one of **X¹**, **X²**, and **X³** is CH**R^{L}** or C(**R^{L}**)₂;
further provided that no more than one of **X¹**, **X²**, and **X³** is selected from the group consisting of: O and S(O)₀₋₂;
**b1** is 0, 1 or 2;
**R⁹** is selected from the group consisting of: H, OH, N**R^{d}R^{e}**, and halo;
each **R¹⁰** is independently selected from the group consisting of **R^{a}** and **R^{b}**;
each **R^{L}** is independently selected from the group consisting of C₁₋₃ alkoxy, -F, CN, and C₁₋₃ alkyl optionally substituted with 1-3 **R^{c}**;
**R¹** is selected from the group consisting of:
   **(i)** a 4-10 membered heterocyclyl optionally substituted with 1-4 **R⁷**;
   **(ii)** an 8-12 membered bicyclic heterocyclyl, wherein the heterocyclyl comprises an endocyclic group selected from the group consisting of C(=O)NH and S(O)₂NH, and wherein the heterocyclyl is further optionally substituted with 1-3 **R⁷** at one or more ring carbon atoms; and
   **(iii)** 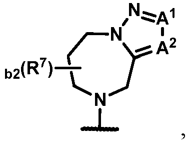 wherein **b2** is 0, 1, 2, or 3; and **A¹** and **A²** are independently selected from the group consisting of: N, CH, and C**R⁷**;
each **R⁷** is independently selected from the group consisting of **R^{a}** and **R^{b}**;
**Y²** is a bond or a straight-chain C₁₋₆ alkylene optionally substituted with 1-6 **R^{Y}**;
each **R^{Y}** is independently selected from the group consisting of: halo, cyano, -OH, oxo, C₁₋₆ alkoxy, C₁₋₆ haloalkoxy, C₁₋₆ alkyl, and C₁₋₆ haloalkyl, or
one pair of **R^{Y}** on the same or different carbon atom(s) taken together with the atom(s) connecting them forms a C₃₋₆ cycloalkyl ring or 4-6 membered heterocyclyl ring, each of which is optionally substituted with 1-3 independently selected C₁₋₃ alkyl;
**R³** is selected from the group consisting of:
   **(a)** 4-15 membered heterocyclyl optionally substituted with 1-6 substituents independently selected from the group consisting of: **R^{a}** and **R^{b}**; and
   **(b)** -N**R^{d}R^{e}**;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   **(f)** -C₁₋₆ haloalkoxy;
   **(g)** -N**R^{d}R^{e}**;
   **(h)** C(=O)C₁₋₆ alkyl;
   **(i)** C(=O)C₁₋₆ haloalkyl;
   **(j)** C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   **(o)** S(O)₀₋₂(C₁₋₆ haloalkyl);
   **(p)** S(O)₁₋₂N(**R^{f}**)₂; and
   **(q)** C₁₋₆ alkyl, C₂₋₆ alkenyl, or C₂₋₆ alkynyl, each optionally substituted with 1-6 **R^{c}**;
each **R^{b}** is independently selected from the group consisting of: **-(L^{b})_{b}-R^{b1}** and **-R^{b1}**, wherein:
   **b** is 1, 2, or 3;
   each **-L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e},** C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)₁₋₂N(**R^{f}**)₂;
   each **R^{d}** and **R^{e}** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂-.

**Embodiment 18.** The compound of Embodiment 17, wherein the compound is a compound of Formula **(IV-a):** 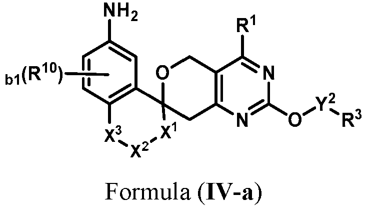 or a pharmaceutically acceptable salt thereof, wherein:
each **R¹⁰** is independently selected from the group consisting of: -Cl, -F, -CN, and C₁₋₃ alkyl optionally substituted with 1-3 **R^{c}.**

**Embodiment 19.** The compound of Embodiment 17, wherein the compound is a compound of Formula **(IV-b):** 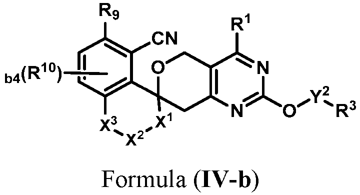 or a pharmaceutically acceptable salt thereof, wherein:
**b4** is 0 or 1; and
each **R¹⁰** is independently selected from the group consisting of: -Cl, -F, -CN, and C₁₋₃ alkyl optionally substituted with 1-3 **R^{c}.**

**Embodiment 20.** The compound of Embodiment 19, wherein **X¹** is CH₂.

**Embodiment 21.** The compound of any one of Embodiments 19-20, wherein **X²** is CH₂; and **X³** is CH**R^{L}**.

**Embodiment 22.** The compound of any one of Embodiments 19-21, wherein each **R^{L}** is independently selected from the group consisting of: CH₃, CF₃, CHF₂, and CH₂F (e.g., CH₃).

**Embodiment 23.** The compound of any one of Embodiments 19-22, wherein one **R^{L}** is CF₃; or wherein one **R^{L}** is CH₃.

**Embodiment 24.** The compound of any one of Embodiments 17-19, wherein the compound is a compound of Formula **(IV-c):** 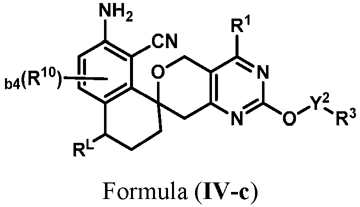 or a pharmaceutically acceptable salt thereof, wherein:
**b4** is 0 or 1; and
each **R¹⁰** is independently selected from the group consisting of: -Cl, -F, -CN, and C₁₋₃ alkyl optionally substituted with 1-3 **R^{c}.**

**Embodiment 25.** The compound of Embodiment 24, wherein **R^{L}** is CF₃; or wherein **R^{L}** is CH₃.

**Embodiment 26.** The compound of any one of Embodiments 1-25, wherein **Y²** is -CH₂-; and **R³** is a 4-10 membered heterocyclyl having one ring nitrogen atom and 0-1 additional ring heteroatom selected from the group consisting of oxygen and nitrogen, wherein the heterocyclyl is optionally substituted with 1-6 **R^{a}**.

**Embodiment 27.** The compound of any one of Embodiments 1-26, wherein **Y²** is -CH₂-; and **R³** is 1-2 -F). 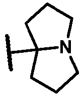 optionally substituted with 1-2 **R^{a}** (e.g., optionally substituted with

**Embodiment 28.** The compound of any one of Embodiments 1-27, wherein **R³** is 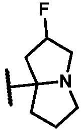 (e.g., 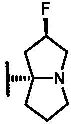 ).

**Embodiment 29.** The compound of any one of Embodiments 1-27, wherein **R³** is

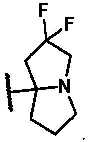

**Embodiment 30.** The compound of any one of Embodiments 1-29, wherein **R¹** is 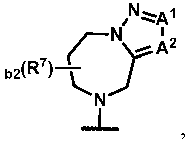 wherein b2 is 0, 1, or 2, and **A¹** and **A²** are independently selected from the group consisting of: N, CH, and C**R⁷**.

**Embodiment 31.** The compound of any one of Embodiments 1-30, wherein **R¹** is 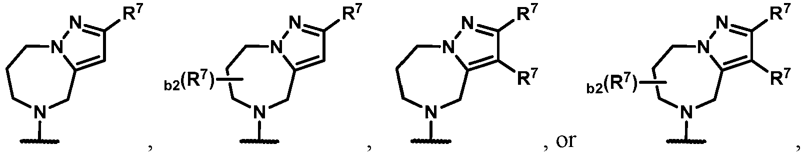 wherein b2 is 1 or 2.

**Embodiment 32.** The compound of any one of Embodiments 1-31, wherein **R¹** is 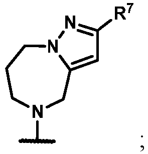 and **R⁷** is selected from the group consisting of: C(=O)N(**R^{f}**)₂, C(O)N(C₁₋₃ alkyl)**R^{b1}**, -C(O)N(H)**R^{b1}**, **R^{b1}**, and C(O)**R^{b1}**.

**Embodiment 33.** The compound of Embodiment 32, wherein **R⁷** is selected from the group consisting of:
**(a)** C(=O)N(**R^{f}**)₂, wherein each **R^{f}** is independently H or C₁₋₃ alkyl optionally
   substituted with 1-3 **R^{h}**;
**(b)** C(O)N(C₁₋₃ alkyl)**R^{b1}** or -C(O)N(H)**R^{b1},** wherein **R^{b1}** is C₃₋₆ cycloalkyl or 4-6 membered heterocyclyl, each of which is optionally substituted with 1-3 **R^{g}**; and
**(c)** C(O)**R^{b1}**, wherein **R^{b1}** is 4-10 membered heterocyclyl optionally substituted with 1-3 **R^{g}**, wherein **R^{b1}** is attached to the C(O) via a ring nitrogen atom.

**Embodiment 34.** The compound of any one of Embodiments 31-33, wherein **R⁷** is C(=O)N(**R^{f}**)₂, wherein each **R^{f}** is independently H or C₁₋₃ alkyl optionally substituted with 1-3 **R^{h}** (e.g., **R⁷** is C(=O)N(Me)₂).

**Embodiment 35.** The compound of Embodiment 32, wherein **R⁷** is **R^{b1}**, wherein the **R^{b1}** is a 5-6 membered heteroaryl optionally substituted with 1-2 **R^{g}**, or wherein the **R^{b1}** is oxetanyl optionally substituted with **R^{g}**.

**Embodiment 36.** The compound of any one of Embodiments 1-31, wherein **R¹** is 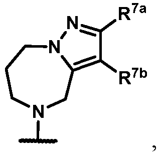 wherein **R^{7a}** and **R^{7b}** are independently selected **R⁷**.

**Embodiment 37.** The compound of Embodiment 36, wherein **R^{7a}** is selected from the group consisting of: C(=O)N(**R^{f}**)₂, C(O)N(C₁₋₃ alkyl)**R^{b1}**, -C(O)N(H)**R^{b1}, R^{b1}**, and C(O)**R^{b1}**; and
**R^{7b}** is -halo, -CN, and C₁₋₃ alkyl optionally substituted with 1-3 **R^{c}.**

**Embodiment 38.** The compound of Embodiment 36 or 37, wherein **R^{7a}** is selected from the group consisting of:
**(a)** C(=O)N(**R^{f}**)₂, wherein each **R^{f}** is independently H or C₁₋₃ alkyl optionally substituted with 1-3 **R^{h}**;
**(b)** C(O)N(C₁₋₃ alkyl)**R^{b1}** or -C(O)N(H)**R^{b1},** wherein **R^{b1}** is C₃₋₆ cycloalkyl or 4-6 membered heterocyclyl, each of which is optionally substituted with 1-3 **R^{g}**; and
**(c)** C(O)**R^{b1}**, wherein **R^{b1}** is 4-10 membered heterocyclyl optionally substituted with 1-3 **R^{g}**, wherein **R^{b1}** is attached to the C(O) via a ring nitrogen atom.

**Embodiment 39.** The compound of any one of Embodiments 36-38, wherein **R^{7a}** is -C(=O)N(**R^{f}**)₂, wherein each **R^{f}** is independently H or C₁₋₃ alkyl optionally substituted with 1-3 **R^{h}** (e.g., **R^{7a}** is C(=O)N(Me)₂); and **R^{7b}** is -Cl, -F, or methyl.

**Embodiment 40.** The compound of Embodiment 36 or 37, wherein **R^{7a}** is **R^{b1}**, wherein the **R^{b1}** is a 5-6 membered heteroaryl optionally substituted with 1-2 **R^{g}**, or wherein the **R^{b1}** is oxetanyl optionally substituted with **R^{g}**.

**Embodiment 41.** The compound of any one of Embodiments 1-31, wherein **R¹** is 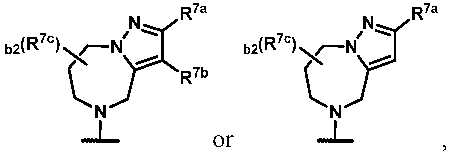 wherein b2 is 1 or 2; and each **R^{7a}**, **R^{7b}**, and **R^{7c}** is an independently selected **R⁷**.

**Embodiment 42.** The compound of any one of Embodiments 1-31 or 41, wherein **R¹** is 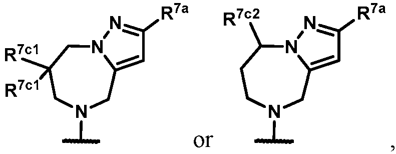 wherein **R^{7a}** is selected from the group consisting of: C(=O)N(**R^{f}**)₂, C(O)N(C₁₋₃ alkyl)**R^{b1}**, -C(O)N(H)**R^{b1}, R^{b1}**, and C(O)**R^{b1}**;
one **R^{7c1}** is CN, and the other **R^{7c1}** is C₁₋₃ alkyl optionally substituted with 1-3 **R^{c}**; and
**R^{7c2}** is C₁₋₃ alkyl substituted with CN and further optionally substituted with 1-3 **R^{c}.**

**Embodiment 43.** The compound of Embodiment 42, wherein **R^{7a}** is C(=O)N(**R^{f}**)₂, wherein each **R^{f}** is independently H or C₁₋₃ alkyl optionally substituted with 1-3 **R^{h}**;
one **R^{7c1}** is CN, and the other **R^{7c1}** is C₁₋₃ alkyl optionally substituted with 1-3 F; and
**R^{7c2}** is C₁₋₃ alkyl substituted with CN (e.g., CH₂CN).

**Embodiment 44.** The compound of any one of Embodiments 1-29, wherein **R¹** is a 7-10 (e.g., 7) membered heterocyclyl having one ring nitrogen atom, one ring oxygen atom, and no additional ring heteroatoms, wherein the 7-10 membered heterocyclyl is optionally substituted with 1-4 (e.g., 1-2) **R⁷**.

**Embodiment 45.** The compound of any one of Embodiments 1-29 or 44, wherein **R¹** is a 7-10 (e.g., 7) membered monocyclic heterocyclyl having one ring nitrogen atom, one ring oxygen atom, and no additional ring heteroatoms, wherein the 7-10 membered monocyclic heterocyclyl is optionally substituted with 1-4 (e.g., 1-2) **R⁷**.

**Embodiment 46.** The compound of any one of Embodiments 1-29 or 44-45, wherein **R¹** is 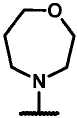 optionally substituted with 1-4 **R⁷** at one or more ring carbon atoms (e.g., **R¹** is 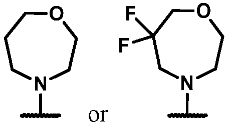 ).

**Embodiment 47.** The compound of Embodiment 45 or 46, wherein each **R⁷** is independently selected from the group consisting of: -OH; -CN; -F; and C₁₋₃ alkyl optionally substituted with 1-3 **R^{c}**, wherein:
each **R^{c}** is independently selected from the group consisting of: -F, -OH, and -CN.

**Embodiment 48.** The compound of any one of Embodiments 1-29 or 44-45, wherein **R¹** is 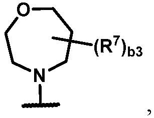 wherein **b3** is 1, 2, or 3.

**Embodiment 49.** The compound of Embodiment 48, wherein one occurrence of **R⁷** is **R^{b}** (e.g., one occurrence of **R⁷** is **R^{b1}**).

**Embodiment 50.** The compound of Embodiment 48 or 49, wherein one occurrence of **R⁷** is a 5-6 membered heteroaryl optionally substituted with 1-3 **R^{g}**.

**Embodiment 51.** The compound of any one of Embodiments 48-50, wherein one occurrence of **R⁷** is a 5-membered heteroaryl optionally substituted with 1-3 **R^{g}**.

**Embodiment 52.** The compound of any one of Embodiments 48-51, wherein one occurrence of **R⁷** is selected from the group consisting of pyrazolyl and oxazolyl, each of which is optionally substituted with 1-2 **R^{g}** (e.g., **R⁷** is 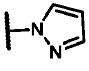 ).

**Embodiment 53.** The compound of any one of Embodiments 48-52, wherein b3 is 1.

**Embodiment 54.** The compound of any one of Embodiments 1-29 or 44-45, wherein **R¹** is 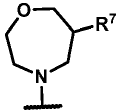 (e.g., 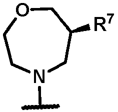 ), wherein **R⁷** is a 5-membered heteroaryl optionally substituted with 1-3 **R^{g}**.

**Embodiment 55.** The compound of Embodiment 54, wherein **R⁷** is selected from the group consisting of pyrazolyl and oxazolyl, each of which is optionally substituted with 1-2 **R^{g}**.

**Embodiment 56.** The compound of Embodiment 54 or 55, wherein **R⁷** is pyrazolyl optionally substituted with 1-2 **R^{g}** (e.g., **R⁷** is 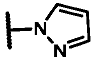 optionally substituted with one **R^{g}**); or wherein **R⁷** is oxazolyl optionally substituted with one **R^{g}** (e.g., **R⁷** is 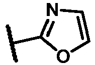 optionally substituted with one **R^{g}**).

**Embodiment 57.** The compound of any one of Embodiments 54-56, wherein **R⁷** is

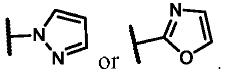

**Embodiment 58.** The compound of any one of Embodiments 1-29 or 44-46, wherein **R¹** is a 7-10 (e.g., 7) membered bicyclic heterocyclyl having one ring nitrogen atom, one ring oxygen atom, and no additional ring heteroatoms, wherein the 7-10 membered bicyclic heterocyclyl is optionally substituted with 1-4 (e.g., 1-2) **R⁷**.

**Embodiment 59.** The compound of any one of Embodiments 1-29, 44-46, or 58, wherein **R¹** is a 7-10 (e.g., 7) membered spirocyclic bicyclic heterocyclyl having one ring nitrogen atom, one ring oxygen atom, and no additional ring heteroatoms, wherein the 7-10 membered spirocyclic bicyclic heterocyclyl is optionally substituted with 1-4 (e.g., 1-2) **R⁷**.

**Embodiment 60.** The compound of any one of Embodiments 1-29, 44-46, or 58-59, wherein **R¹** is 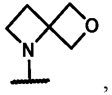 which is optionally substituted with 1-2 **R⁷**.

**Embodiment 61.** The compound of any one of Embodiments 1-29, 44-46, or 58-60, wherein **R¹** is

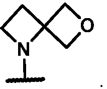

**Embodiment 62.** The compound of any one of Embodiments 1-29, wherein **R¹** is a 4-membered heterocyclyl optionally substituted with 1-2 **R⁷**.

**Embodiment 63.** The compound of any one of Embodiments 1-29 or 62, wherein **R¹** is 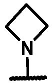 optionally substituted with 1-2 **R⁷**.

**Embodiment 64.** The compound of any one of Embodiments 1-29 or 62-63, wherein **R¹** is selected from the group consisting of:

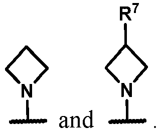

**Embodiment 65.** The compound of any one of Embodiments 62-64, wherein each **R⁷** is independently selected from the group consisting of: -F, -OH, **-R^{b1}**, and C₁₋₃ alkyl optionally substituted with 1-3 F, wherein the **R^{b1}** is a 5-6 membered heteroaryl optionally substituted with 1-2 **R^{g}**.

**Embodiment 66.** The compound of Embodiment 1, wherein the compound is a compound of Formula (**II-a1**) or (**II**-**b1**): 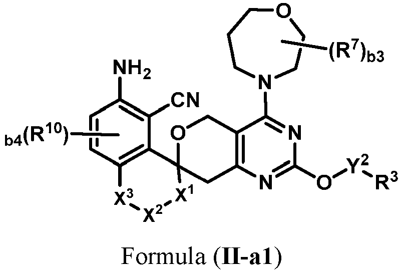 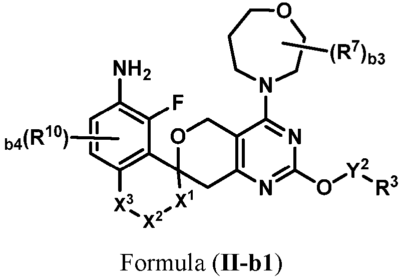 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 R**^{c}**;
**b3** is 0, 1, 2, or 3;
**X¹** is CH₂; and
**X²** and **X³** are independently selected from the group consisting of: O, CH₂, CH**R^{L}**, and C(**R^{L}**)₂.

**Embodiment 67.** The compound of Embodiment 66, wherein **b4** is 1; and **R¹⁰** is *ortho* to **X³**.

**Embodiment 68.** The compound of Embodiment 6, wherein the compound is a compound of Formula (**III-1**): 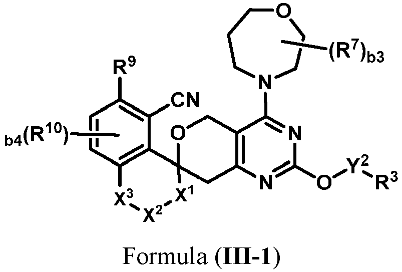 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c}**;
**b3** is 0, 1, 2, or 3;
**X¹** is CH₂; and
**X²** and **X³** are independently selected from the group consisting of: O, CH₂, CH**R^{L}**, and C(**R^{L}**)₂.

**Embodiment 69.** The compound of Embodiment 68, wherein **R⁹** is N**R^{d}R^{e}** (e.g., - NH₂).

**Embodiment 70.** The compound of Embodiment 17, wherein the compound is a compound of Formula (**IV-a1**) or (**IV-b1**): 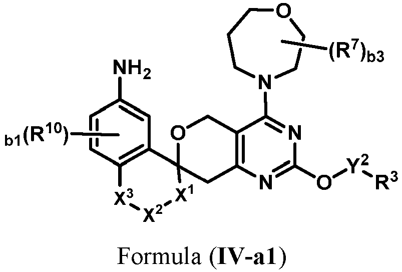 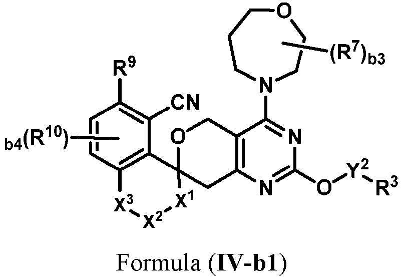 or a pharmaceutically acceptable salt thereof, wherein:
**b4** is 0 or 1;
**b1** is 0, 1, or 2;
each **R¹⁰** is independently selected from the group consisting of: -Cl, -F, -CN, and C₁₋₃ alkyl optionally substituted with 1-3 **R^{c}**;
**b3** is 0, 1, 2, or 3;
**X¹** is CH₂;
one of **X²** and **X³** is independently selected from the group consisting of: CH**R^{L}** and C(**R^{L}**)₂; and
the other of **X²** and **X³** is CH₂ or O.

**Embodiment 71.** The compound of Embodiment 70, wherein the compound is a compound of Formula (**IV-b1**), or a pharmaceutically acceptable salt thereof, wherein **b4** is 1; and **R¹⁰** is *ortho* to **X³**.

**Embodiment 72.** The compound of Embodiment 70, wherein the compound is a compound of Formula (**IV-a1**), or a pharmaceutically acceptable salt thereof, wherein **b1** is 1 or 2; and the 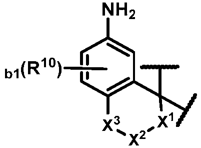 moiety is

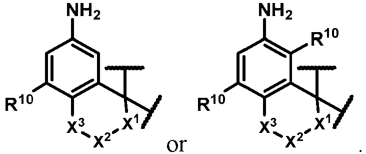

**Embodiment 73.** The compound of any one of Embodiments 70-72, wherein **X²** is CH₂; and **X³** is CH**R^{L}**.

**Embodiment 74.** The compound of Embodiment 73, wherein **R^{L}** is CF₃; or wherein **R^{L}** is CH₃.

**Embodiment 75.** The compound of any one of Embodiments 66-74, wherein **b3** is 0.

**Embodiment 76.** The compound of any one of Embodiments 66-74, wherein **b3** is 1 or 2; and each **R⁷** is independently selected from the group consisting of: -OH; -CN; -F; and C₁₋₃ alkyl optionally substituted with 1-3 **R^{c}**, wherein each **R^{c}** is independently selected from the group consisting of: -F, -OH, and -CN.

**Embodiment 77.** The compound of any one of Embodiments 66-74, wherein **b3** is 1; and the 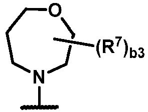 moiety is 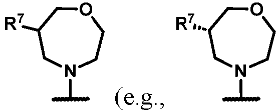 ), wherein **R⁷** is a 5-membered heteroaryl optionally substituted with 1-3 **R^{g}**.

**Embodiment 78.** The compound of Embodiment 77, wherein **R⁷** is pyrazolyl optionally substituted with 1-2 **R^{g}** (e.g., **R⁷** is 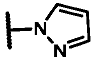 optionally substituted with one **R^{g}**).

**Embodiment 79.** The compound of Embodiment 77 or 78, wherein **R⁷** is

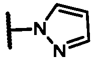

**Embodiment 80.** The compound of Embodiment 1, wherein the compound is a compound of Formula (**II-a2**) or (**II-b2**): 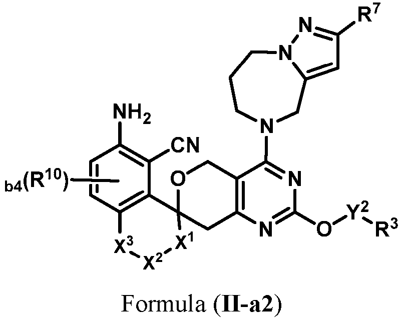 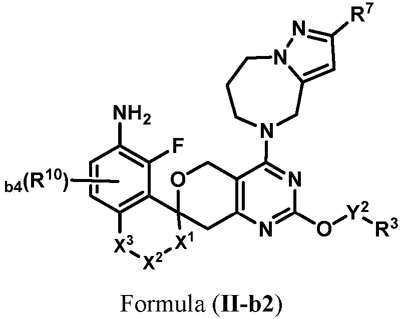 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c};**
**R⁷** is selected from the group consisting of: C(=O)N(**R^{f}**)₂, C(O)N(C₁₋₃ alkyl)**R^{b1}**, - C(O)N(H)**R^{b1}**, **R^{b1}**, and C(O)R**^{b1}**;
**X¹** is CH₂; and
**X²** and **X³** are independently selected from the group consisting of: O, CH₂, CH**R^{L}**, and C(**R^{L}**)₂.

**Embodiment 81.** The compound of Embodiment 6, wherein the compound is a compound of Formula (**III-2**): 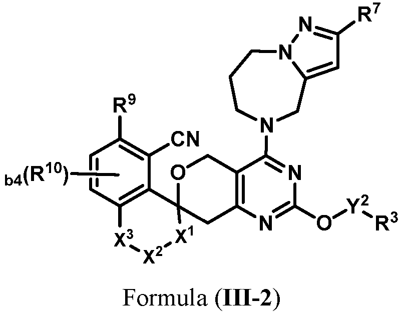 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c};**
**R⁷** is selected from the group consisting of: C(=O)N(**R^{f}**)₂, C(O)N(C₁₋₃ alkyl)**R^{b1}**, - C(O)N(H)**R^{b1}**, **R^{b1}**, and C(O)R**^{b1}**;
**X¹** is CH₂; and
**X²** and **X³** are independently selected from the group consisting of: O, CH₂, CH**R^{L}**, and C(**R^{L}**)₂.

**Embodiment 82.** The compound of Embodiment 81, wherein **R⁹** is N**R^{d}R^{e}** (e.g., - NH₂).

**Embodiment 83.** The compound of Embodiment 17, wherein the compound is a compound of Formula (**IV-a2**) or (**IV-b2**): 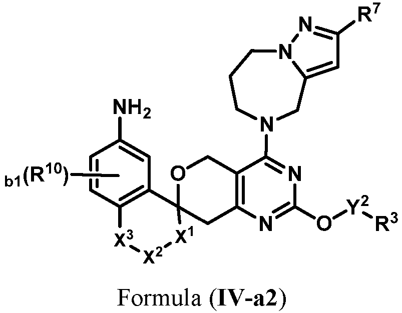 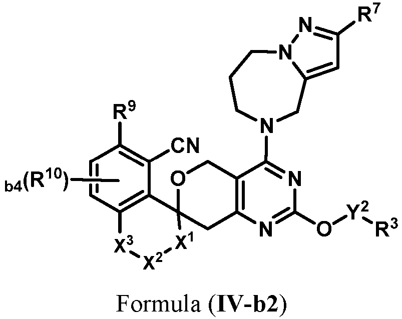 or a pharmaceutically acceptable salt thereof, wherein:
**b4** is 0 or 1;
**b1** is 0, 1, or 2;
each **R¹⁰** is independently selected from the group consisting of: -Cl, -F, -CN, and C₁₋₃ alkyl optionally substituted with 1-3 **R^{c};**
**R⁷** is selected from the group consisting of: C(=O)N(**R^{f}**)₂, C(O)N(C₁₋₃ alkyl)**R^{b1}**, - C(O)N(H)**R^{b1}**, **R^{b1}**, and C(O)R**^{b1}**;
**X¹** is CH₂;
one of **X²** and **X³** is independently selected from the group consisting of: CH**R^{L}** and C(**R^{L}**)₂; and
the other of **X²** and **X³** is CH₂ or O.

**Embodiment 84.** The compound of Embodiment 83, wherein the compound is a compound of Formula (**IV-a2**), or a pharmaceutically acceptable salt thereof, wherein **b1** is 1 or 2; and the 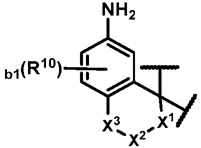 moiety is 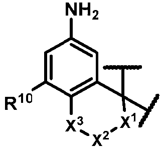 or

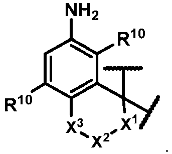

**Embodiment 85.** The compound of Embodiment 83 or 84, wherein **X²** is CH₂; and **X³** is CH**R^{L}**.

**Embodiment 86.** The compound of Embodiment 85, wherein **R^{L}** is CF₃; or wherein **R^{L}** is CH₃.

**Embodiment 87.** The compound of any one of Embodiments 80-87, wherein **R⁷** is selected from the group consisting of:
**(a)** C(=O)N(**R^{f}**)₂, wherein each **R^{f}** is independently H or C₁₋₃ alkyl optionally substituted with 1-3 **R^{h}**;
**(b)** C(O)N(C₁₋₃ alkyl)**R^{b1}** or -C(O)N(H)**R^{b1}**, wherein: **R^{b1}** is C₃₋₆ cycloalkyl or 4-6 membered heterocyclyl, each of which is optionally substituted with 1-3 **R^{g}**; and
**(c)** C(O)**R^{b1}**, wherein **R^{b1}** is 4-10 membered heterocyclyl optionally substituted with 1-3 **R^{g}**, wherein **R^{b1}** is attached to the C(O) via a ring nitrogen atom.

**Embodiment 88.** The compound of Embodiment 1, wherein the compound is a compound of Formula (**II-3**): 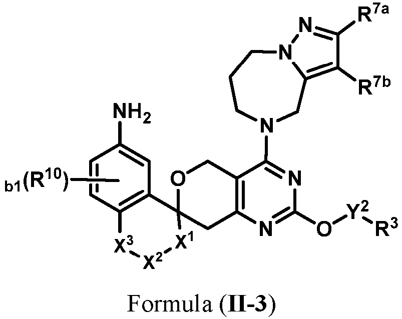 or a pharmaceutically acceptable salt thereof, wherein:
**b1** is 1 or 2;
each **R¹⁰** is independently selected from the group consisting of: -Cl, -F, -CN, and C₁₋₃ alkyl optionally substituted with 1-3 **R^{c};**
**R^{7a}** is selected from the group consisting of:
   **(a)** C(=O)N(**R^{f}**)₂, wherein each **R^{f}** is independently H or C₁₋₃ alkyl optionally substituted with 1-3 **R^{h}**;
   **(b)** C(O)N(C₁₋₃ alkyl)**R^{b1}** or -C(O)N(H)**R^{b1}**, wherein: **R^{b1}** is C₃₋₆ cycloalkyl or 4-6 membered heterocyclyl, each of which is optionally substituted with 1-3 **R^{g}**; and
   **(c)** C(O)**R^{b1}**, wherein **R^{b1}** is 4-10 membered heterocyclyl optionally substituted with 1-3 **R^{g}**, wherein **R^{b1}** is attached to the C(O) via a ring nitrogen atom;
**R^{7b}** is -halo or C₁₋₃ alkyl;
**X¹** is CH₂; and
**X²** and **X³** are independently selected from the group consisting of: O, CH₂, CH**R^{L}**, and C(**R^{L}**)₂.

**Embodiment 89.** The compound of Embodiment 1, wherein the compound is a compound of Formula **(II-a3):** 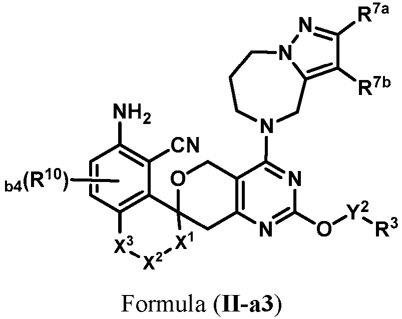 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c};**
**R^{7a}** is selected from the group consisting of:
   **(a)** C(=O)N(**R^{f}**)₂, wherein each **R^{f}** is independently H or C₁₋₃ alkyl optionally substituted with 1-3 **R^{h}**;
   **(b)** C(O)N(C₁₋₃ alkyl)**R^{b1}** or -C(O)N(H)**R^{b1}**, wherein: **R^{b1}** is C₃₋₆ cycloalkyl or 4-6 membered heterocyclyl, each of which is optionally substituted with 1-3 **R^{g}**; and
   **(c)** C(O)**R^{b1}**, wherein **R^{b1}** is 4-10 membered heterocyclyl optionally substituted with 1-3 **R^{g}**, wherein **R^{b1}** is attached to the C(O) via a ring nitrogen atom;
**R^{7b}** is -halo or C₁₋₃ alkyl;
**X¹** is CH₂; and
**X²** and **X³** are independently selected from the group consisting of: O, CH₂, CH**R^{L}**, and C(**R^{L}**)₂.

**Embodiment 90.** The compound of Embodiment 89, wherein **b4** is 0.

**Embodiment 91.** The compound of Embodiment 6, wherein the compound is a compound of Formula **(III-3):** 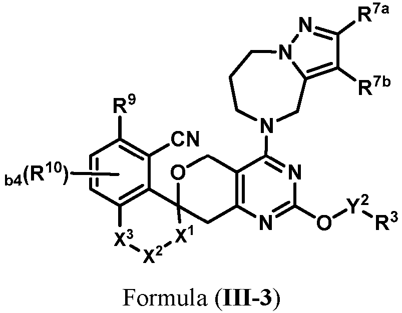 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c};**
**R^{7a}** is selected from the group consisting of:
   **(a)** C(=O)N(**R^{f}**)₂, wherein each **R^{f}** is independently H or C₁₋₃ alkyl optionally substituted with 1-3 **R^{h}**;
   **(b)** C(O)N(C₁₋₃ alkyl)**R^{b1}** or -C(O)N(H)**R^{b1}**, wherein: **R^{b1}** is C₃₋₆ cycloalkyl or 4-6 membered heterocyclyl, each of which is optionally substituted with 1-3 **R^{g}**; and
   **(c)** C(O)**R^{b1}**, wherein **R^{b1}** is 4-10 membered heterocyclyl optionally substituted with 1-3 **R^{g}**, wherein **R^{b1}** is attached to the C(O) via a ring nitrogen atom;
**R^{7b}** is -halo or C₁₋₃ alkyl;
**X¹** is CH₂; and
**X²** and **X³** are independently selected from the group consisting of: O, CH₂, CH**R^{L}**, and C(**R^{L}**)₂.

**Embodiment 92.** The compound of Embodiment 91, wherein **b4** is 0.

**Embodiment 93.** The compound of Embodiment 91 or 92, wherein **R⁹** is N**R^{d}R^{e}** (e.g., -NH₂).

**Embodiment 94.** The compound of Embodiment 17, wherein the compound is a compound of Formula **(IV-a3)** or **(IV-b3):** 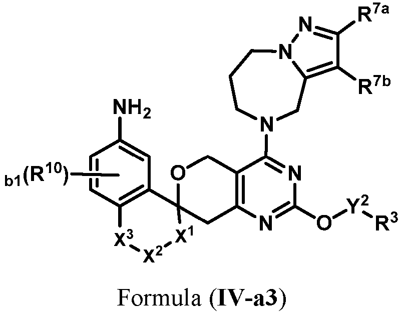 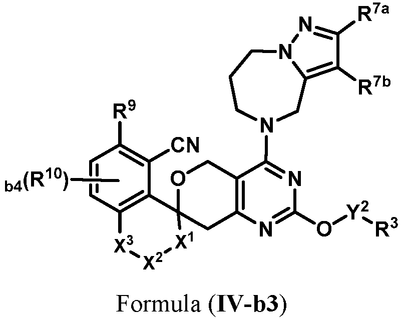 or a pharmaceutically acceptable salt thereof, wherein:
**b1** is 0, 1, or 2;
**b4** is 0 or 1;
each **R¹⁰** is independently selected from the group consisting of: -Cl, -F, -CN, and C₁₋₃ alkyl optionally substituted with 1-3 **R^{c};**
**R^{7a}** is selected from the group consisting of:
   **(a)** C(=O)N(**R^{f}**)₂, wherein each **R^{f}** is independently H or C₁₋₃ alkyl optionally substituted with 1-3 **R^{h}**;
   **(b)** C(O)N(C₁₋₃ alkyl)**R^{b1}** or -C(O)N(H)**R^{b1}**, wherein: **R^{b1}** is C₃₋₆ cycloalkyl or 4-6 membered heterocyclyl, each of which is optionally substituted with 1-3 **R^{g}**; and
   **(c)** C(O)**R^{b1}**, wherein **R^{b1}** is 4-10 membered heterocyclyl optionally substituted with 1-3 **R^{g}**, wherein **R^{b1}** is attached to the C(O) via a ring nitrogen atom;
**R^{7b}** is -halo or C₁₋₃ alkyl;
**X¹** is CHz;
one of **X²** and **X³** is independently selected from the group consisting of: CH**R^{L}** and C(**R^{L}**)₂; and
the other of **X²** and **X³** is CH₂ or O.

**Embodiment 95.** The compound of Embodiment 94, wherein the compound is a compound of Formula (**IV-b3**), or a pharmaceutically acceptable salt thereof, wherein **b4** is 0.

**Embodiment 96.** The compound of Embodiment 94 or 95, wherein **X²** is CH₂; and **X³** is CH**R^{L}**.

**Embodiment 97.** The compound of Embodiment 96, wherein **R^{L}** is CF₃ or CH₃ (e.g., CH₃).

**Embodiment 98.** The compound of any one of Embodiments 88-97, wherein **R^{7a}** is C(=O)N(**R^{f}**)₂, wherein each **R^{f}** is independently C₁₋₃ alkyl optionally substituted with 1-3 **R^{h}** (e.g., **R^{7a}** is C(=O)N(Me)₂).

**Embodiment 99.** The compound of any one of Embodiments 88-98, wherein **R^{7b}** is -Cl, -F, or methyl.

**Embodiment 100.** The compound of any one of Embodiments 88-99, wherein **R^{7b}** is -Cl.

**Embodiment 101.** The compound of any one of Embodiments 88-100, wherein the 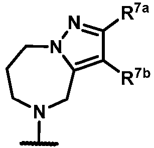 moiety is

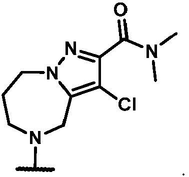

**Embodiment 102.** The compound of Embodiment 1, wherein the compound is a compound of Formula (**II-4**): 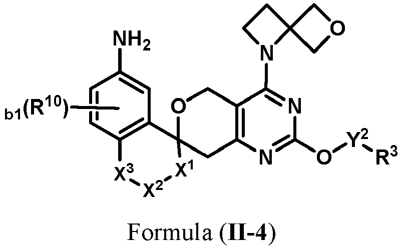 or a pharmaceutically acceptable salt thereof, wherein:
**b1** is 1 or 2;
each **R¹⁰** is independently selected from the group consisting of: -Cl, -F, -CN, and C₁₋₃ alkyl optionally substituted with 1-3 **R^{c};**
**X¹** is CH₂; and
**X²** and **X³** are independently selected from the group consisting of: O, CH₂, CH**R^{L}**, and C(**R^{L}**)₂.

**Embodiment 103.** The compound of Embodiment 1, wherein the compound is a compound of Formula (**II-a4**): 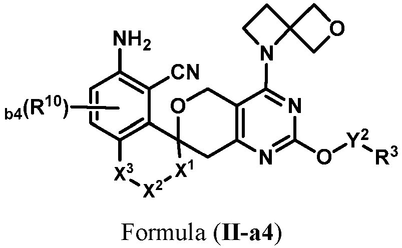 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c};**
**X¹** is CH₂; and
**X²** and **X³** are independently selected from the group consisting of: O, CH₂, CH**R^{L}**, and C(**R^{L}**)₂.

**Embodiment 104.** The compound of Embodiment 103, wherein **b4** is 0.

**Embodiment 105.** The compound of Embodiment 6, wherein the compound is a compound of Formula (**III-4**): 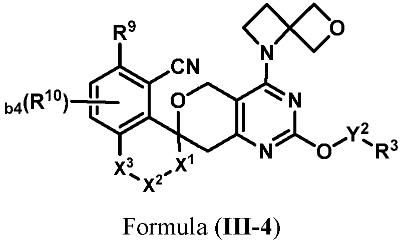 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c};**
**X¹** is CH₂; and
**X²** and **X³** are independently selected from the group consisting of: O, CH₂, CH**R^{L}**, and C(**R^{L}**)₂.

**Embodiment 106.** The compound of Embodiment 105, wherein **b4** is 0.

**Embodiment 107.** The compound of Embodiment 105 or 106, wherein **R⁹** is N**R^{d}R^{e}** (e.g., -NH₂).

**Embodiment 108.** The compound of Embodiment 17, wherein the compound is a compound of Formula (**IV-a4**) or (**IV-b4**): 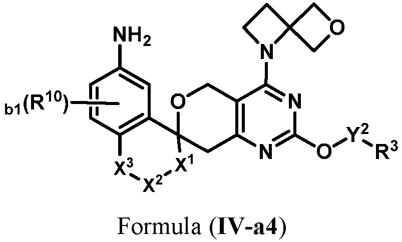 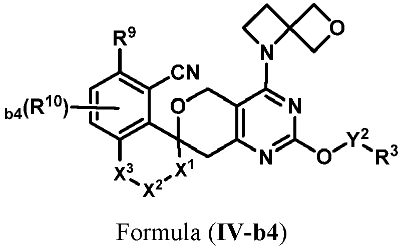 or a pharmaceutically acceptable salt thereof, wherein:
**b1** is 0, 1, or 2;
**b4** is 0 or 1;
each **R¹⁰** is independently selected from the group consisting of: -Cl, -F, -CN, and C₁₋₃ alkyl optionally substituted with 1-3 **R^{c};**
**X¹** is CH₂;
one of **X²** and **X³** is independently selected from the group consisting of: CH**R^{L}** and C(**R^{L}**)₂; and
the other of **X²** and **X³** is CH₂ or O.

**Embodiment 109.** The compound of Embodiment 108, wherein the compound is a compound of Formula (**IV-a4**), or a pharmaceutically acceptable salt thereof, wherein **b1** is 1; and the 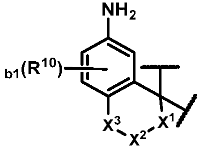 moiety is 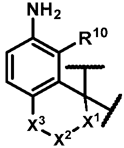 (e.g., 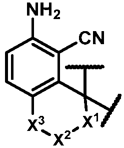 ).

**Embodiment 110.** The compound of Embodiment 108, wherein the compound is a compound of Formula (**IV-b4**), or a pharmaceutically acceptable salt thereof, wherein **b4** is 0.

**Embodiment 111.** The compound of any one of Embodiments 102-110, wherein **X²** is CH₂; and **X³** is CH**R^{L}** (e.g., CH(CH₃)).

**Embodiment 112.** The compound of any one of Embodiments 102-107, wherein **X²** is CH₂; and **X³** is CH₂.

**Embodiment 113.** The compound of any one of Embodiments 66-112, wherein **Y²** is -CH₂-; and **R³** is a 4-10 membered heterocyclyl having one ring nitrogen atom and 0-1 additional ring heteroatom selected from the group consisting of oxygen and nitrogen, wherein the heterocyclyl is optionally substituted with 1-6 **R^{a}**.

**Embodiment 114.** The compound of any one of Embodiments 66-113, wherein **Y²** is -CH₂-; and **R³** is 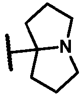 optionally substituted with 1-2 -F (e.g., **R³** is 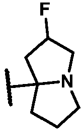 or 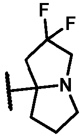 ).

**Embodiment 115.** The compound of Embodiment 113 or 114, wherein **R³** is 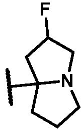 (e.g., 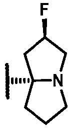 ).

**Embodiment 116.** The compound of any one of Embodiments 1-115, wherein the 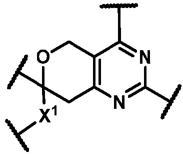 moiety is

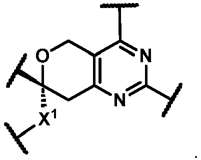

**Embodiment 117.** The compound of Embodiment 1, wherein the compound of Formula (**II**) is selected from the group consisting of Compound Nos. **181b, 501, 501a, 501b, 501c, 502, 502a, 502b, 503, 503a, 503b, 503c, 504, 504a, 504b, 504c, 505, 505a, 506, 506a, 507, 507a, 507b, 507c, 508, 508a, 509, 509a, 510, 510a, 510b, 511, 511a, 512, 512a, 512b, 513, 513a, 514, 514a, 514b, 516, 516a, 516b, 516c, 517, 517a, 518, 518a, 519, 519a, 520, 520a, 522, 522a, 523, 523a, 524, 524a, 525, 525a, 526, 526a, 526b, 526c, 527, 527a, 527b, 528, 528a, 529, 529a, 530, 530a, 531, 531a, 532, 532a, 534, 534a, 535, 535a, 536, 536a, 536b, 536c, 537, 537a, 538, 538a, 539, 539a, 540, 540a, 541, 541a, 542, 542a, 543, 543a, 544, 544a, 545, 545a, 546, 546a, 547, 547a, 548, 548a, 549, 549a, 550, 550a, 551, 551a, 552, 552a, 553, 553a, 554, 554a, 554b, 554c, 555, 555a, 556, 556a, 558, 558a, 559, 559a, 560, 560a, 561, 561a, 562, 562a, 563, 563a, 564, 564a, 565,** and **565a** as depicted in **Table C3,** or a pharmaceutically acceptable salt thereof.

**Embodiment 118.** The compound of Embodiment 6, wherein the compound of Formula (**III**) is selected from the group consisting of Compound Nos. **502, 502a, 502b, 503, 503a, 503b, 503c, 504, 504a, 504b, 504c, 505, 505a, 507, 507a, 507b, 507c, 509, 509a, 511, 511a, 512, 512a, 512b, 513, 513a, 514, 514a, 514b, 516, 516a, 516b, 516c, 517, 517a, 518, 518a, 519, 519a, 520, 520a, 522, 522a, 523, 523a, 524, 524a, 525, 525a, 526, 526a, 526b, 526c, 527, 527a, 527b, 528, 528a, 529, 529a, 530, 530a, 531, 531a, 532, 532a, 534, 534a, 535, 535a, 536, 536a, 536b, 536c, 537, 537a, 538, 538a, 539, 539a, 540, 540a, 541, 541a, 542, 542a, 543, 543a, 544, 544a, 545, 545a, 546, 546a, 547, 547a, 548, 548a, 549, 549a, 550, 550a, 551, 551a, 552, 552a, 553, 553a, 554, 554a, 554b, 554c, 555, 555a, 556, 556a, 558, 558a, 559, 559a, 560, 560a, 561, 561a, 562, 562a, 563, 563a, 564, 564a, 565,** and **565a** as depicted in **Table C3,** or a pharmaceutically acceptable salt thereof.

**Embodiment 119.** The compound of Embodiment 17, wherein the compound of Formula (**IV**) is selected from the group consisting of Compound Nos. **502, 502a, 502b, 508, 508a, 509, 509a, 511, 511a, 512, 512a, 512b, 530, 530a, 531, 531a, 532, 532a, 546, 546a, 553, 553a, 554, 554a, 554b, 554c, 555, 555a, 556, 556a, 560, 560a, 561,** and **561a** as depicted in **Table C3,** or a pharmaceutically acceptable salt thereof.

### P07 EXEMPLARY EMBODIMENTS

**Embodiment 1.** A compound of Formula (**II**): 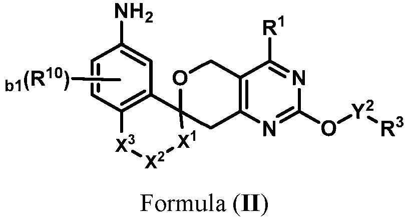 or a pharmaceutically acceptable salt thereof, wherein:
**R¹** is selected from the group consisting of:
   **(i)** a 4-10 membered heterocyclyl optionally substituted with 1-4 **R⁷**;
   **(ii)** an 8-12 membered bicyclic heterocyclyl, wherein the heterocyclyl comprises an endocyclic group selected from the group consisting of C(=O)NH and S(O)₂NH, and wherein the heterocyclyl is further optionally substituted with 1-3 **R⁷** at one or more ring carbon atoms; and
   (iii) 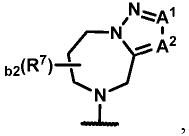 wherein **b2** is 0, 1, 2, or 3; and **A¹** and **A²** are independently selected from the group consisting of: N, CH, and C**R⁷**;
each **R⁷** is independently selected from the group consisting of **R^{a}** and **R^{b}**;
**X¹** is selected from the group consisting of a bond, S(O)₀₋₂, CH₂, CH**R^{L}**, C(**R^{L}**)₂, and O;
**X²** and **X³** are independently selected from the group consisting of: CH₂, CH**R^{L}**, C(**R^{L}**)₂, O, and S(O)₀₋₂, provided that no more than one of **X¹**, **X²**, and **X³** is selected from the group consisting of: O and S(O)₀₋₂;
**b1** is 1 or 2;
each **R¹⁰** is independently selected from the group consisting of **R^{a}** and **R^{b}**;
each **R^{L}** is independently selected from the group consisting of C₁₋₃ alkoxy, -F, CN, and C₁₋₃ alkyl optionally substituted with 1-3 **R^{c};** or
one pair of **R^{L}** on the same or different ring carbon atom(s) taken together with the ring atom(s) connecting them form a C₃₋₆ cycloalkyl ring;
**Y²** is a bond or a straight-chain C₁₋₆ alkylene optionally substituted with 1-6 **R^{Y}**;
each **R^{Y}** is independently selected from the group consisting of: halo, cyano, -OH, oxo, C₁₋₆ alkoxy, C₁₋₆ haloalkoxy, C₁₋₆ alkyl, and C₁₋₆ haloalkyl, or
one pair of **R^{Y}** on the same or different carbon atom(s) taken together with the atom(s) connecting them forms a C₃₋₆ cycloalkyl ring or 4-6 membered heterocyclyl ring, each of which is optionally substituted with 1-3 independently selected C₁₋₃ alkyl;
**R³** is selected from the group consisting of:
   **(a)** 4-15 membered heterocyclyl optionally substituted with 1-6 substituents independently selected from the group consisting of: **R^{a}** and **R^{b}**; and
   **(b)** - N**R^{d}R^{e}**;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   **(f)** -C₁₋₆ haloalkoxy;
   **(g)** -N**R^{d}R^{e}**;
   **(h)** C(=O)C₁₋₆ alkyl;
   **(i)** C(=O)C₁₋₆ haloalkyl;
   **(j)** C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   **(o)** S(O)₀₋₂(C₁₋₆ haloalkyl);
   **(p)** S(O)₁₋₂N(**R^{f}**)₂; and
   **(q)** C₁₋₆ alkyl, C₂₋₆ alkenyl, or C₂₋₆ alkynyl, each optionally substituted with 1-6 **R^{c};**
each **R^{b}** is independently selected from the group consisting of: -(**L^{b}**)**_{b}**-**R^{b1}** and -**R^{b1}**, wherein:
   **b is** 1, 2, or 3;
   each -**L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e}**, C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)₁₋₂N(**R^{f}**)₂;
   each **R^{d}** and **R^{e}** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂₋.

**Embodiment 2.** The compound of Embodiment 1, wherein the compound is a compound of Formula (**II-a**): 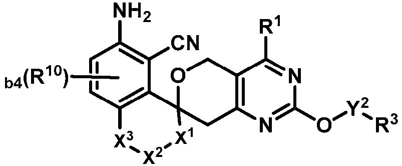 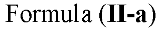 or a pharmaceutically acceptable salt thereof, wherein:
**b4** is 0 or 1; and
each **R¹⁰** is independently selected from the group consisting of: -Cl, -F, -CN, and C₁₋₃ alkyl optionally substituted with 1-3 **R^{c}**.

**Embodiment 3.** The compound of Embodiment 1, wherein the compound is a compound of Formula (**II-b**): 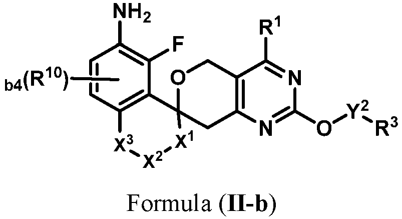 or a pharmaceutically acceptable salt thereof, wherein:
**b4** is 0 or 1; and
each **R¹⁰** is independently selected from the group consisting of: -Cl, -F, -CN, and C₁₋₃ alkyl optionally substituted with 1-3 **R^{c}**.

**Embodiment 4.** A compound of Formula (**III**): 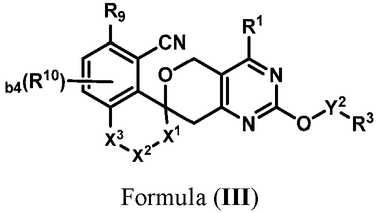 or a pharmaceutically acceptable salt thereof, wherein:
**R¹** is selected from the group consisting of:
   **(i)** a 4-10 membered heterocyclyl optionally substituted with 1-4 **R⁷**;
   **(ii)** an 8-12 membered bicyclic heterocyclyl, wherein the heterocyclyl comprises an endocyclic group selected from the group consisting of C(=O)NH and S(O)₂NH, and wherein the heterocyclyl is further optionally substituted with 1-3 **R⁷** at one or more ring carbon atoms; and
   **(iii)** 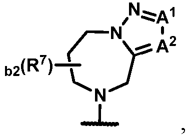 wherein **b2** is 0, 1, 2, or 3; and **A¹** and **A²** are independently selected from the group consisting of: N, CH, and C**R⁷**;
each **R⁷** is independently selected from the group consisting of **R^{a}** and **R^{b}**;
**X¹** is selected from the group consisting of a bond, S(O)₀₋₂, CH₂, CH**R^{L}**, C(**R^{L}**)₂, and O;
**X²** and **X³** are independently selected from the group consisting of: CH₂, CH**R^{L}**, C(**R^{L}**)₂, O, and S(O)₀₋₂, provided that no more than one of **X¹**, **X²**, and **X³** is selected from the group consisting of: O and S(O)₀₋₂;
**R⁹** is selected from the group consisting of: H, N**R^{d}R^{e}**, -OH, and halo;
**b4** is 0 or 1;
each **R¹⁰** is independently selected from the group consisting of **R^{a}** and **R^{b}**;
each **R^{L}** is independently selected from the group consisting of C₁₋₃ alkoxy, -F, CN, and C₁₋₃ alkyl optionally substituted with 1-3 **R^{c};** or
one pair of **R^{L}** on the same or different ring carbon atom(s) taken together with the ring atom(s) connecting them form a C₃₋₆ cycloalkyl ring;
**Y²** is a bond or a straight-chain C₁₋₆ alkylene optionally substituted with 1-6 **R^{Y}**;
each **R^{Y}** is independently selected from the group consisting of: halo, cyano, -OH, oxo, C₁₋₆ alkoxy, C₁₋₆ haloalkoxy, C₁₋₆ alkyl, and C₁₋₆ haloalkyl, or
one pair of **R^{Y}** on the same or different carbon atom(s) taken together with the atom(s) connecting them forms a C₃₋₆ cycloalkyl ring or 4-6 membered heterocyclyl ring, each of which is optionally substituted with 1-3 independently selected C₁₋₃ alkyl;
**R³** is selected from the group consisting of:
   **(a)** 4-15 membered heterocyclyl optionally substituted with 1-6 substituents independently selected from the group consisting of: **R^{a}** and **R^{b}**; and
   **(b)** -N**R^{d}R^{e}**;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   **(f)** -C₁₋₆ haloalkoxy;
   **(g)** -N**R^{d}R^{e}**;
   **(h)** C(=O)C₁₋₆ alkyl;
   **(i)** C(=O)C₁₋₆ haloalkyl;
   **(j)** C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   **(o)** S(O)₀₋₂(C₁₋₆ haloalkyl);
   **(p)** S(O)₁₋₂N(**R^{f}**)₂; and
   **(q)** C₁₋₆ alkyl, C₂₋₆ alkenyl, or C₂₋₆ alkynyl, each optionally substituted with 1-6 **R^{c};**
each **R^{b}** is independently selected from the group consisting of: -(**L^{b}**)**_{b}**-**R^{b1}** and -**R^{b1}**, wherein:
   **b** is 1, 2, or 3;
   each -**L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e}**, C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)₁₋₂N(**R^{f}**)₂;
   each **R^{d}** and **R^{e}** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂₋.

**Embodiment 5.** The compound of Embodiment 4, wherein **R⁹** is -NH₂; and each **R¹⁰** is independently selected from the group consisting of: -Cl, -F, -CN, and C₁₋₃ alkyl optionally substituted with 1-3 **R^{c}**.

**Embodiment 6.** The compound of any one of Embodiments 1-5, wherein **X¹** is CH₂ or CH**R^{L}**.

**Embodiment 7.** The compound of any one of Embodiments 1-6, wherein **X²** and **X³** are independently selected from the group consisting of: CH₂, CH**R^{L}**, and C(**R^{L}**)₂.

**Embodiment 8.** The compound of any one of Embodiments 1-7, wherein **X¹** is CH₂; and **X²** and **X³** are both CH₂.

**Embodiment 9.** The compound of any one of Embodiments 1-7, wherein at least one (e.g., one) of **X¹**, **X²**, and **X³** is selected from the group consisting of: CH**R^{L}** and C(**R^{L}**)₂.

**Embodiment 10.** The compound of any one of Embodiments 1-7 or 9, wherein **X¹** is CH₂; and **X²** and **X³** are independently selected from the group consisting of: CH₂, CH**R^{L}**, and C(**R^{L}**)₂, provided that 1-2 of **X²** and **X³** is independently CH**R^{L}** or C(**R^{L}**)₂.

**Embodiment 11.** The compound of any one of Embodiments 1-7 or 9-10, wherein **X¹** is CH₂; **X²** is CH₂; and **X³** is CH**R^{L}** (e.g., **X³** is C(H)CF₃; or e.g., **X³** is C(H)CH₃).

**Embodiment 12.** The compound of any one of Embodiments 1-11, wherein each **R^{L}** is independently selected from the group consisting of: C_{H3}, CF₃, CHF₂, and CH₂F (e.g., CH₃).

**Embodiment 13.** The compound of Embodiment 1, wherein the 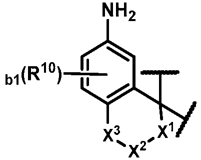 moiety is selected from the group consisting of: 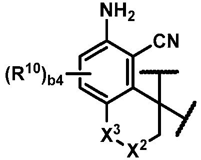 and 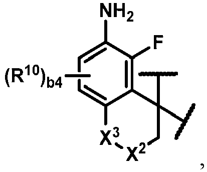 wherein:
**b4** is 0 or 1;
**X²** is -O- or -CH₂-;
**X³** is -CH₂- or -CH**R^{L}**-, wherein **R^{L}** is C₁₋₃ alkyl (e.g., methyl); and
each **R¹⁰** is independently selected from the group consisting of: -Cl, -F, -CN, and C₁₋₃ alkyl optionally substituted with 1-3 **R^{c}**.

**Embodiment 14.** The compound of Embodiment 13, wherein the 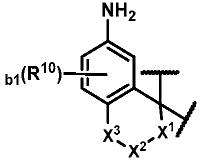 moiety is selected from the group consisting of:

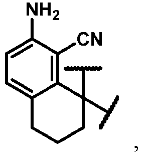

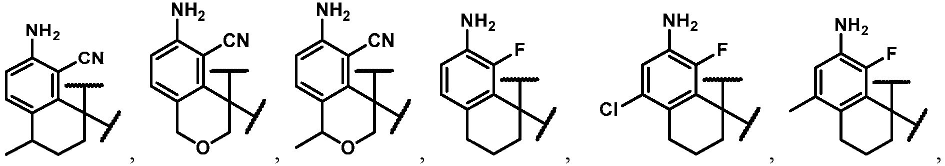

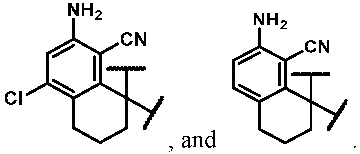

**Embodiment 15.** A compound of Formula (**IV**): 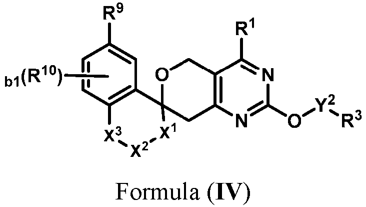 or a pharmaceutically acceptable salt thereof, wherein:
**X¹** is selected from the group consisting of a bond, S(O)₀₋₂, CH₂, CH**R^{L}**, C(**R^{L}**)₂, and O;
**X²** and **X³** are independently selected from the group consisting of: CH₂, CH**R^{L}**, C(**R^{L}**)₂, O, and S(O)₀₋₂, provided that at least one of **X¹**, **X²**, and **X³** is CH**R^{L}** or C(**R^{L}**)₂;
further provided that no more than one of **X¹**, **X²**, and **X³** is selected from the group consisting of: O and S(O)₀₋₂;
**b1** is 0, 1 or 2;
**R⁹** is selected from the group consisting of: H, OH, N**R^{d}R^{e}**, and halo;
each **R¹⁰** is independently selected from the group consisting of **R^{a}** and **R^{b}**;
each **R^{L}** is independently selected from the group consisting of C₁₋₃ alkoxy, -F, CN, and C₁₋₃ alkyl optionally substituted with 1-3 **R^{c};**
**R¹** is selected from the group consisting of:
   **(i)** a 4-10 membered heterocyclyl optionally substituted with 1-4 **R⁷**;
   **(ii)** an 8-12 membered bicyclic heterocyclyl, wherein the heterocyclyl comprises an endocyclic group selected from the group consisting of C(=O)NH and S(O)₂NH, and wherein the heterocyclyl is further optionally substituted with 1-3 **R⁷** at one or more ring carbon atoms; and
   **(iii)** 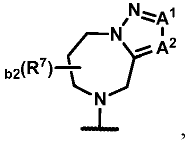 wherein **b2** is 0, 1, 2, or 3; and **A¹** and **A²** are independently selected from the group consisting of: N, CH, and C**R⁷**;
each **R⁷** is independently selected from the group consisting of **R^{a}** and **R^{b}**;
**Y²** is a bond or a straight-chain C₁₋₆ alkylene optionally substituted with 1-6 **R^{Y}**;
each **R^{Y}** is independently selected from the group consisting of: halo, cyano, -OH, oxo, C₁₋₆ alkoxy, C₁₋₆ haloalkoxy, C₁₋₆ alkyl, and C₁₋₆ haloalkyl, or
one pair of **R^{Y}** on the same or different carbon atom(s) taken together with the atom(s) connecting them forms a C₃₋₆ cycloalkyl ring or 4-6 membered heterocyclyl ring, each of which is optionally substituted with 1-3 independently selected C₁₋₃ alkyl;
**R³** is selected from the group consisting of:
   **(a)** 4-15 membered heterocyclyl optionally substituted with 1-6 substituents independently selected from the group consisting of: **R^{a}** and **R^{b}**; and
   **(b)** - N**R^{d}R^{e}**;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   **(f)** -C₁₋₆ haloalkoxy;
   **(g)** - N**R^{d}R^{e}**;
   **(h)** C(=O)C₁₋₆ alkyl;
   **(i)** C(=O)C₁₋₆ haloalkyl;
   **(j)** C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   **(o)** S(O)₀₋₂(C₁₋₆ haloalkyl);
   **(p)** S(O)₁₋₂N(**R^{f}**)₂; and
   **(q)** C₁₋₆ alkyl, C₂₋₆ alkenyl, or C₂₋₆ alkynyl, each optionally substituted with 1-6 **R^{c};**
each **R^{b}** is independently selected from the group consisting of: -(**L^{b}**)**_{b}**-**R^{b1}** and -**R^{b1}**, wherein:
   **b is** 1, 2, or 3;
   each -**L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e}**, C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)₁₋₂N(**R^{f}**)₂;
   each **R^{d}** and **R^{e}** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂₋.

**Embodiment 16.** The compound of Embodiment 15, wherein the compound is a compound of Formula (**IV-a**): 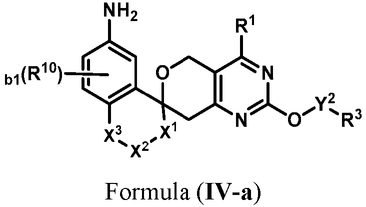 or a pharmaceutically acceptable salt thereof, wherein:
each **R¹⁰** is independently selected from the group consisting of: -Cl, -F, -CN, and C₁₋₃ alkyl optionally substituted with 1-3 **R^{c}**.

**Embodiment 17.** The compound of Embodiment 15, wherein the compound is a compound of Formula (**IV-b**)**:** 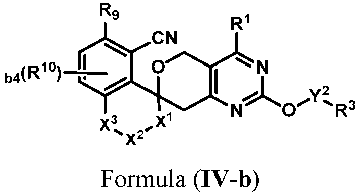 or a pharmaceutically acceptable salt thereof, wherein:
**b4** is 0 or 1; and
each **R¹⁰** is independently selected from the group consisting of: -Cl, -F, -CN, and C₁₋₃ alkyl optionally substituted with 1-3 **R^{c}**.

**Embodiment 18.** The compound of Embodiment 17, wherein **X¹** is CH₂.

**Embodiment 19.** The compound of Embodiment 17 or 18, wherein **X²** is CH₂; and **X³** is CH**R^{L}**.

**Embodiment 20.** The compound of any one of Embodiments 17-19, wherein each **R^{L}** is independently selected from the group consisting of: CH₃, CF₃, CHF₂, and CH₂F (e.g., CH₃).

**Embodiment 21.** The compound of any one of Embodiments 17-20, wherein one **R^{L}** is CF₃; or wherein one **R^{L}** is CH₃.

**Embodiment 22.** The compound of any one of Embodiments 15-17, wherein the compound is a compound of Formula (**IV-c**): 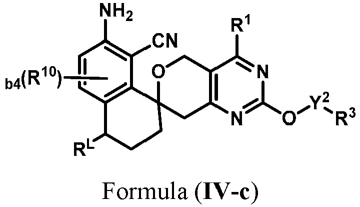 or a pharmaceutically acceptable salt thereof, wherein:
**b4** is 0 or 1; and
each **R¹⁰** is independently selected from the group consisting of: -Cl, -F, -CN, and C₁₋₃ alkyl optionally substituted with 1-3 **R^{c}**.

**Embodiment 23.** The compound of Embodiment 22, wherein **R^{L}** is CF₃; or wherein **R^{L}** is CH₃.

**Embodiment 24.** The compound of any one of Embodiments 1-23, wherein **Y²** is -CH₂-; and **R³** is a 4-10 membered heterocyclyl having one ring nitrogen atom and 0-1 additional ring heteroatom selected from the group consisting of oxygen and nitrogen, wherein the heterocyclyl is optionally substituted with 1-6 **R^{a}**.

**Embodiment 25.** The compound of any one of Embodiments 1-24, wherein **Y²** is -CH₂-; and **R³** is 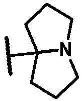 optionally substituted with 1-2 **R^{a}** (e.g., optionally substituted with 1-2 -F).

**Embodiment 26.** The compound of any one of Embodiments 1-25, wherein **R³** is 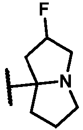 (e.g., 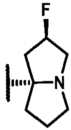 ).

**Embodiment 27.** The compound of any one of Embodiments 1-25, wherein **R³** is

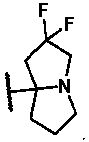

**Embodiment 28.** The compound of any one of Embodiments 1-27, wherein **R¹** is 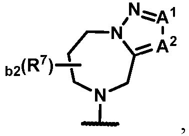 wherein **b2** is 0, 1, or 2, and **A¹** and **A²** are independently selected from the group consisting of: N, CH, and C**R⁷**.

**Embodiment 29.** The compound of any one of Embodiments 1-28, wherein **R¹** is 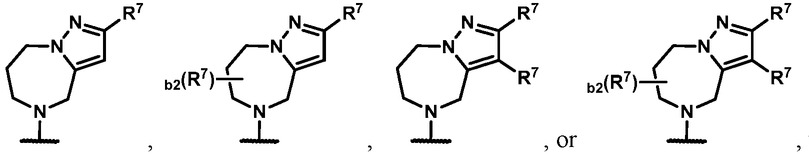 wherein b2 is 1 or 2.

**Embodiment 30.** The compound of any one of Embodiments 1-29, wherein **R¹** is 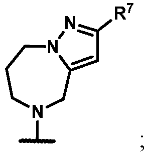 and **R⁷** is selected from the group consisting of: C(=O)N(**R^{f}**)₂, C(O)N(C₁₋₃ alkyl)**R^{b1}**, C(O)N(H)**R^{b1}**, **R^{b1}**, and C(O)**R^{b1}**.

**Embodiment 31.** The compound of Embodiment 30, wherein **R⁷** is selected from the group consisting of:
**(a)** C(=O)N(**R^{f}**)₂, wherein each **R^{f}** is independently H or C₁₋₃ alkyl optionally
   substituted with 1-3 **R^{h}**;
**(b)** C(O)N(C₁₋₃ alkyl)**R^{b1}** or -C(O)N(H)**R^{b1}**, wherein **R^{b1}** is C₃₋₆ cycloalkyl or 4-6 membered heterocyclyl, each of which is optionally substituted with 1-3 **R^{g}**; and
**(c)** C(O)**R^{b1}**, wherein **R^{b1}** is 4-10 membered heterocyclyl optionally substituted with 1-3 **R^{g}**, wherein **R^{b1}** is attached to the C(O) via a ring nitrogen atom.

**Embodiment 32.** The compound of any one of Embodiments 29-31, wherein **R⁷** is C(=O)N(**R^{f}**)₂, wherein each **R^{f}** is independently H or C₁₋₃ alkyl optionally substituted with 1-3 **R^{h}** (e.g., **R⁷** is C(=O)N(Me)₂).

**Embodiment 33.** The compound of Embodiment 30, wherein **R⁷** is **R^{b1}**, wherein the **R^{b1}** is a 5-6 membered heteroaryl optionally substituted with 1-2 **R^{g}**, or wherein the **R^{b1}** is oxetanyl optionally substituted with **R^{g}**.

**Embodiment 34.** The compound of any one of Embodiments 1-29, wherein **R¹** is 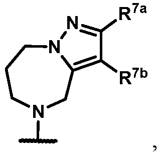 wherein **R^{7a}** and **R^{7b}** are independently selected **R⁷**.

**Embodiment 35.** The compound of Embodiment 34, wherein **R^{7a}** is selected from the group consisting of: C(=O)N(**R^{f}**)₂, C(O)N(C₁₋₃ alkyl)**R^{b1}**, -C(O)N(H)**R^{b1}**, **R^{b1}**, and C(O)**R^{b1}**; and
**R^{7b}** is -halo, -CN, and C₁₋₃ alkyl optionally substituted with 1-3 **R^{c}**.

**Embodiment 36.** The compound of Embodiment 34 or 35, wherein **R^{7a}** is selected from the group consisting of:
**(a)** C(=O)N(**R^{f}**)₂, wherein each **R^{f}** is independently H or C₁₋₃ alkyl optionally
   substituted with 1-3 **R^{h}**;
**(b)** C(O)N(C₁₋₃ alkyl)**R^{b1}** or -C(O)N(H)**R^{b1}**, wherein **R^{b1}** is C₃₋₆ cycloalkyl or 4-6 membered heterocyclyl, each of which is optionally substituted with 1-3 **R^{g}**; and
**(c)** C(O)**R^{b1}**, wherein **R^{b1}** is 4-10 membered heterocyclyl optionally substituted with 1-3 **R^{g}**, wherein **R^{b1}** is attached to the C(O) via a ring nitrogen atom.

**Embodiment 37.** The compound of any one of Embodiments 34-36, wherein **R^{7a}** is -C(=O)N(**R^{f}**)₂, wherein each **R^{f}** is independently H or C₁₋₃ alkyl optionally substituted with 1-3 **R^{h}** (e.g., **R^{7a}** is C(=O)N(Me)₂); and **R^{7b}** is -Cl, -F, or methyl.

**Embodiment 38.** The compound of Embodiment 34 or 35, wherein **R^{7a}** is **R^{b1}**, wherein the **R^{b1}** is a 5-6 membered heteroaryl optionally substituted with 1-2 **R^{g}**, or wherein the **R^{b1}** is oxetanyl optionally substituted with **R^{g}**.

**Embodiment 39.** The compound of any one of Embodiments 1-27, wherein **R¹** is a 7-10 (e.g., 7) membered heterocyclyl having one ring nitrogen atom, one ring oxygen atom, and no additional ring heteroatoms, wherein the 7-10 membered heterocyclyl is optionally substituted with 1-4 (e.g., 1-2) **R⁷**.

**Embodiment 40.** The compound of any one of Embodiments 1-27 or 39, wherein **R¹** is a 7-10 (e.g., 7) membered monocyclic heterocyclyl having one ring nitrogen atom, one ring oxygen atom, and no additional ring heteroatoms, wherein the 7-10 membered monocyclic heterocyclyl is optionally substituted with 1-4 (e.g., 1-2) **R⁷**.

**Embodiment 41.** The compound of any one of Embodiments 1-27 or 39-40, wherein **R¹** is 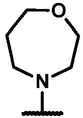 optionally substituted with 1-4 **R⁷** at one or more ring carbon atoms (e.g., **R¹** is 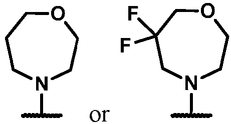 ).

**Embodiment 42.** The compound of any one of Embodiments 39-41, wherein each **R⁷** is independently selected from the group consisting of: -OH; -CN; -F; and C₁₋₃ alkyl optionally substituted with 1-3 **R^{c}**, wherein:
each **R^{c}** is independently selected from the group consisting of: -F, -OH, and -CN.

**Embodiment 43.** The compound of any one of Embodiments 1-27 or 39-40, wherein **R¹** is 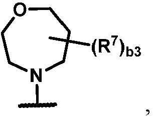 wherein b3 is 1, 2, or 3.

**Embodiment 44.** The compound of Embodiment 43, wherein one occurrence of **R⁷** is **R^{b}** (e.g., one occurrence of **R⁷** is **R^{b1}**).

**Embodiment 45.** The compound of Embodiment 43 or 44, wherein one occurrence of **R⁷** is a 5-6 membered heteroaryl optionally substituted with 1-3 **R^{g}**.

**Embodiment 46.** The compound of any one of Embodiments 43-45, wherein one occurrence of **R⁷** is a 5-membered heteroaryl optionally substituted with 1-3 **R^{g}**.

**Embodiment 47.** The compound of any one of Embodiments 43-46, wherein one occurrence of **R⁷** is selected from the group consisting of pyrazolyl and oxazolyl, each of which is optionally substituted with 1-2 **R^{g}** (e.g., **R⁷** is 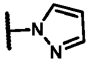 ).

**Embodiment 48.** The compound of any one of Embodiments 43-47, wherein **b3** is 1.

**Embodiment 49.** The compound of any one of Embodiments 1-27 or 39-40, wherein **R¹** is 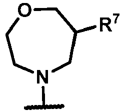 (e.g., 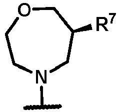 ), wherein **R⁷** is a 5-membered heteroaryl optionally substituted with 1-3 **R^{g}**.

**Embodiment 50.** The compound of Embodiment 49, wherein **R⁷** is selected from the group consisting of pyrazolyl and oxazolyl, each of which is optionally substituted with 1-2 **R^{g}**.

**Embodiment 51.** The compound of Embodiment 49 or 50, wherein **R⁷** is pyrazolyl optionally substituted with 1-2 **R^{g}** (e.g., **R⁷** is 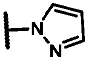 optionally substituted with one **R^{g}**); or wherein **R⁷** is oxazolyl optionally substituted with one **R^{g}** (e.g., **R⁷** is 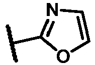 optionally substituted with one **R^{g}**).

**Embodiment 52.** The compound of any one of Embodiments 49-51, wherein **R⁷** is

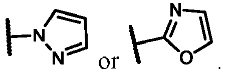

**Embodiment 53.** The compound of any one of Embodiments 1-27 or 39, wherein **R¹** is a 7-10 (e.g., 7) membered bicyclic heterocyclyl having one ring nitrogen atom, one ring oxygen atom, and no additional ring heteroatoms, wherein the 7-10 membered bicyclic heterocyclyl is optionally substituted with 1-4 (e.g., 1-2) **R⁷**.

**Embodiment 54.** The compound of any one of Embodiments 1-27, 39, or 53, wherein **R¹** is a 7-10 (e.g., 7; e.g., 9) membered spirocyclic bicyclic heterocyclyl having one ring nitrogen atom, one ring oxygen atom, and no additional ring heteroatoms, wherein the 7-10 membered spirocyclic bicyclic heterocyclyl is optionally substituted with 1-4 (e.g., 1-2) **R⁷**.

**Embodiment 55.** The compound of any one of Embodiments 1-27, 39, or 53-54, wherein **R¹** is 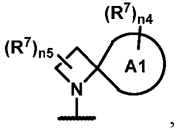 wherein: **Ring A1** is a 4-7 membered heterocyclyl ring having one ring oxygen atom and no additional ring heteroatoms; **n4** is 0, 1, or 2; and **n5** is 0, 1, or 2, provided that **n4** + **n5** is 0, 1, or 2.

**Embodiment 56.** The compound of any one of Embodiments 1-27, 39, or 53-55, wherein **R¹** is 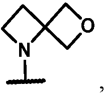 which is optionally substituted with 1-2 **R⁷**.

**Embodiment 57.** The compound of any one of Embodiments 1-27, 39, or 53-56, wherein **R¹** is

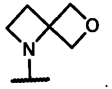

**Embodiment 58.** The compound of any one of Embodiments 1-27, 39, or 53-55, wherein **R¹** is 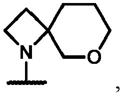 which is optionally substituted with 1-2 **R⁷**.

**Embodiment 59.** The compound of any one of Embodiments 1-27, 39, 53-55, or 58, wherein **R¹** is 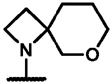 (e.g., 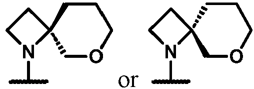 ).

**Embodiment 60.** The compound of any one of Embodiments 1-27, wherein **R¹** is a 4-membered heterocyclyl optionally substituted with 1-2 **R⁷**.

**Embodiment 61.** The compound of any one of Embodiments 1-27 or 60, wherein **R¹** is 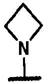 optionally substituted with 1-2 **R⁷**.

**Embodiment 62.** The compound of any one of Embodiments 1-27 or 60-61, wherein **R¹** is selected from the group consisting of: 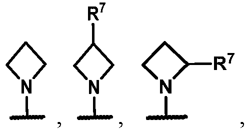 and

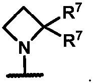

**Embodiment 63.** The compound of any one of Embodiments 1-27 or 60-62, wherein **R¹** is 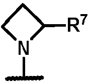 (e.g., 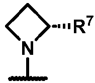 ).

**Embodiment 64.** The compound of any one of Embodiments 60-63, wherein each **R⁷** is independently selected from the group consisting of:
-F,
-cyano,
-OH,
-**R^{b1}**, wherein the **R^{b1}** is a 5-6 membered heteroaryl optionally substituted with 1-2 **R^{g}**, C₁₋₃ alkyl optionally substituted with 1-3 F,
C₁₋₃ alkyl substituted with -OH or C₁₋₃ alkoxy, and
C(=O)N(**R^{f}**)₂.

**Embodiment 65.** The compound of any one of Embodiments 1-27, wherein **R¹** is 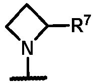 (e.g., 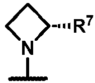 ), wherein **R⁷** is C₁₋₃ alkyl substituted with -OH or C₁₋₃ alkoxy (e.g., C₁₋₃ alkyl substituted with -OH).

Embodiment 66. The compound of any one of Embodiments 1-27 or 65, wherein **R¹** is 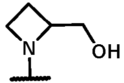 (e.g., 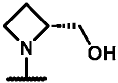 ).

**Embodiment 67.** The compound of Embodiment 1, wherein the compound is a compound of Formula (**II-a1**) or (**II-b1**): 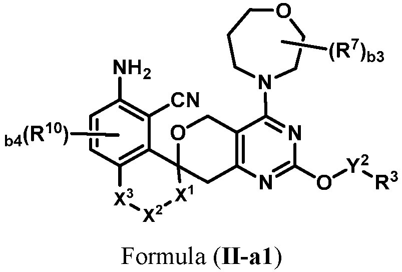 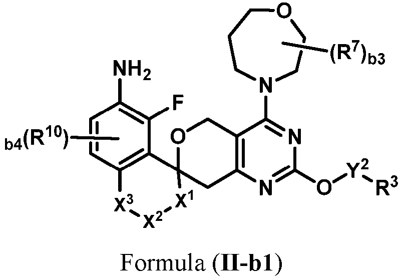 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c}**;
**b3** is 0, 1, 2, or 3;
**X¹** is CH₂; and
**X²** and **X³** are independently selected from the group consisting of: O, CH₂, CH**R^{L}**, and C(**R^{L}**)₂.

**Embodiment 68.** The compound of Embodiment 4, wherein the compound is a compound of Formula (**III-1**): 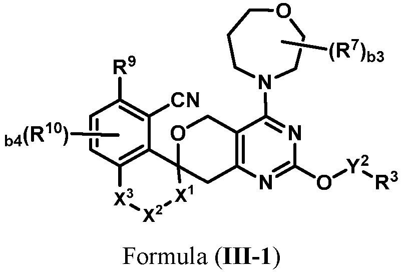 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c}**;
**b3** is 0, 1, 2, or 3;
**X¹** is CH₂; and
**X²** and **X³** are independently selected from the group consisting of: O, CH₂, CH**R^{L}**, and C(**R^{L}**)₂.

**Embodiment 69.** The compound of Embodiment 68, wherein **R⁹** is N**R^{d}R^{e}** (e.g., - NH₂).

**Embodiment 70.** The compound of Embodiment 15, wherein the compound is a compound of Formula (**IV-a1**) or (**IV-b1**): 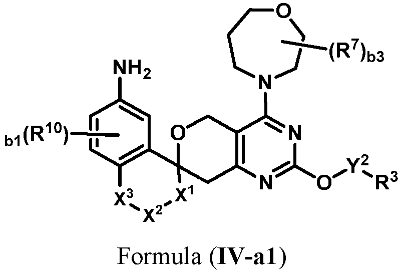 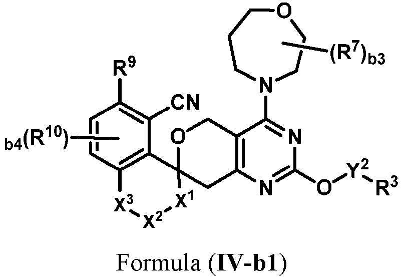 or a pharmaceutically acceptable salt thereof, wherein:
**b4** is 0 or 1;
**b1** is 0, 1, or 2;
each **R¹⁰** is independently selected from the group consisting of: -Cl, -F, -CN, and C₁₋₃ alkyl optionally substituted with 1-3 **R^{c}**;
**b3** is 0, 1, 2, or 3;
**X¹** is CHz;
one of **X²** and **X³** is independently selected from the group consisting of: CH**R^{L}** and C(**R^{L}**)₂; and
the other of **X²** and **X³** is CH₂ or O.

**Embodiment 71.** The compound of Embodiment 70, wherein **X²** is CH₂; and **X³** is CH**R^{L}**.

**Embodiment 72.** The compound of Embodiment 71, wherein **R^{L}** is CF₃; or wherein **R^{L}** is CH₃.

**Embodiment 73.** The compound of any one of Embodiments 67-72, wherein **b3** is 0.

**Embodiment 74.** The compound of any one of Embodiments 67-72, wherein **b3** is 1 or 2; and each **R⁷** is independently selected from the group consisting of: -OH; -CN; -F; and C₁₋₃ alkyl optionally substituted with 1-3 **R^{c}**, wherein each **R^{c}** is independently selected from the group consisting of: -F, -OH, and -CN.

**Embodiment 75.** The compound of any one of Embodiments 67-72, wherein **b3** is 1; and the 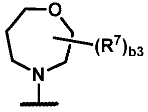 moiety is 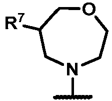 (e.g., 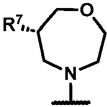 ), wherein **R⁷** is a 5-membered heteroaryl optionally substituted with 1-3 **R^{g}**.

**Embodiment 76.** The compound of Embodiment 75, wherein **R⁷** is pyrazolyl optionally substituted with 1-2 **R^{g}** (e.g., **R⁷** is 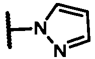 optionally substituted with one **R^{g}**).

**Embodiment 77.** The compound of Embodiment 75 or 76, wherein **R⁷** is

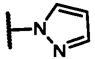

**Embodiment 78.** The compound of Embodiment 1, wherein the compound is a compound of Formula **(II-a2)** or **(II-b2):** 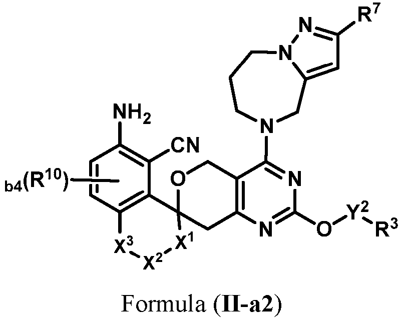 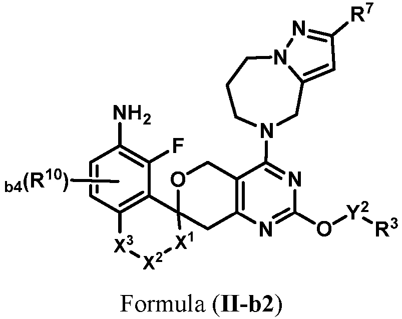 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c}**;
**R⁷** is selected from the group consisting of: C(=O)N(**R^{f}**)₂, C(O)N(C₁₋₃ alkyl)**R^{b1}**, - C(O)N(H)**R^{b1}**, **R^{b1}**, and C(O)**R^{b1}**;
**X¹** is CH₂; and
**X²** and **X³** are independently selected from the group consisting of: O, CH₂, CH**R^{L}**, and C(**R^{L}**)₂.

**Embodiment 79.** The compound of Embodiment 4, wherein the compound is a compound of Formula (**III-2**): 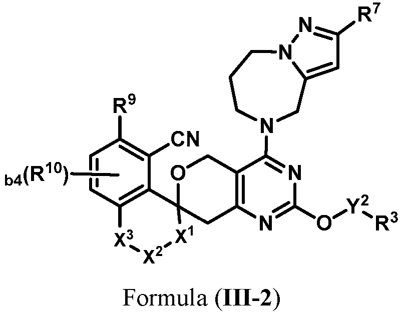 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c}**;
**R⁷** is selected from the group consisting of: C(=O)N(**R^{f}**)₂, C(O)N(C₁₋₃ alkyl)**R^{b1}**, - C(O)N(H)**R^{b1}**, **R^{b1}**, and C(O)**R^{b1}**;
**X¹** is CH₂; and
**X²** and **X³** are independently selected from the group consisting of: O, CH₂, CH**R^{L}**, and C(**R^{L}**)₂.

**Embodiment 80.** The compound of Embodiment 79, wherein **R⁹** is N**R^{d}R^{e}** (e.g., - NH₂).

**Embodiment 81.** The compound of Embodiment 15, wherein the compound is a compound of Formula **(IV-a2)** or **(IV-b2):** 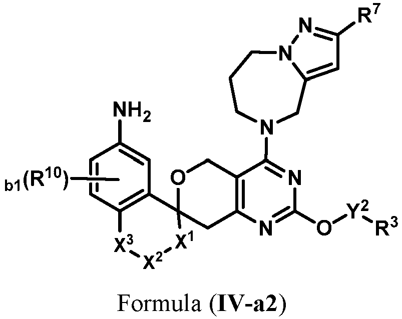 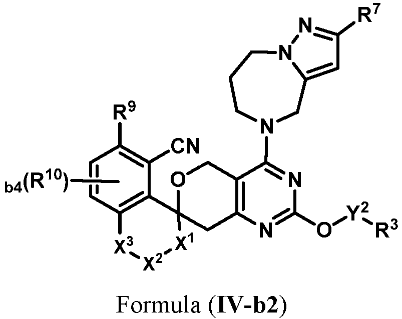 or a pharmaceutically acceptable salt thereof, wherein:
**b4** is 0 or 1;
**b1** is 0, 1, or 2;
each **R¹⁰** is independently selected from the group consisting of: -Cl, -F, -CN, and C₁₋₃ alkyl optionally substituted with 1-3 **R^{c}**;
**R⁷** is selected from the group consisting of: C(=O)N(**R^{f}**)₂, C(O)N(C₁₋₃ alkyl)**R^{b1}**, - C(O)N(H)**R^{b1}**, **R^{b1}**, and C(O)**R^{b1}**;
**X¹** is CH₂;
one of **X²** and **X³** is independently selected from the group consisting of: CH**R^{L}** and C(**R^{L}**)₂; and
the other of **X²** and **X³** is CH₂ or O.

**Embodiment 82.** The compound of Embodiment 81, wherein the compound is a compound of Formula **(IV-a2),** or a pharmaceutically acceptable salt thereof, wherein **b1** is 1 or 2; and the 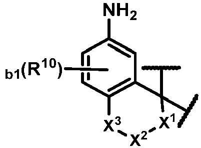 moiety is 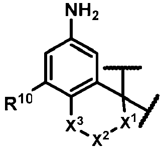 or

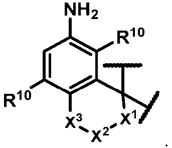

**Embodiment 83.** The compound of Embodiment 81 or 82, wherein **X²** is CH₂; and **X³** is CH**R^{L}**.

**Embodiment 84.** The compound of Embodiment 83, wherein **R^{L}** is CF₃; or wherein **R^{L}** is CH₃.

**Embodiment 85.** The compound of any one of Embodiments 78-84, wherein **R⁷** is selected from the group consisting of:
**(a)** C(=O)N(**R^{f}**)₂, wherein each **R^{f}** is independently H or C₁₋₃ alkyl optionally substituted with 1-3 **R^{h}**;
**(b)** C(O)N(C₁₋₃ alkyl)**R^{b1}** or -C(O)N(H)**R^{b1}**, wherein: **R^{b1}** is C₃₋₆ cycloalkyl or 4-6 membered heterocyclyl, each of which is optionally substituted with 1-3 **R^{g}**; and
**(c)** C(O)**R^{b1}**, wherein **R^{b1}** is 4-10 membered heterocyclyl optionally substituted with 1-3 **R^{g}**, wherein **R^{b1}** is attached to the C(O) via a ring nitrogen atom.

**Embodiment 86.** The compound of Embodiment 1, wherein the compound is a compound of Formula (**II-3**): 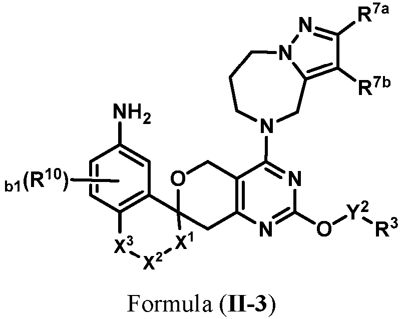 or a pharmaceutically acceptable salt thereof, wherein:
**b1** is 1 or 2;
each **R¹⁰** is independently selected from the group consisting of: -Cl, -F, -CN, and C₁₋₃ alkyl optionally substituted with 1-3 **R^{c}**;
**R^{7a}** is selected from the group consisting of:
   **(a)** C(=O)N(**R^{f}**)₂, wherein each **R^{f}** is independently H or C₁₋₃ alkyl optionally
      substituted with 1-3 **R^{h}**;
   **(b)** C(O)N(C₁₋₃ alkyl)**R^{b1}** or -C(O)N(H)**R^{b1}**, wherein: **R^{b1}** is C₃₋₆ cycloalkyl or 4-6 membered heterocyclyl, each of which is optionally substituted with 1-3 **R^{g}**; and
   **(c)** C(O)**R^{b1}**, wherein **R^{b1}** is 4-10 membered heterocyclyl optionally substituted with 1-3 **R^{g}**, wherein **R^{b1}** is attached to the C(O) via a ring nitrogen atom;
**R^{7b}** is -halo or C₁₋₃ alkyl;
**X¹** is CH₂; and
**X²** and **X³** are independently selected from the group consisting of: O, CH₂, CH**R^{L}**, and C(**R^{L}**)₂.

**Embodiment 87.** The compound of Embodiment 86, wherein **b1** is 1.

**Embodiment 88.** The compound of Embodiment 1, wherein the compound is a compound of Formula (**II-a3**): 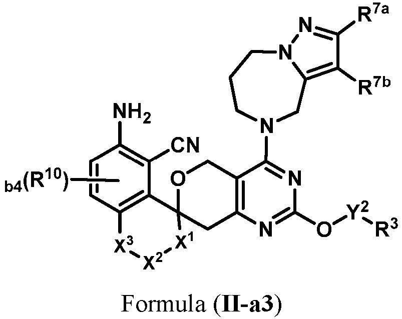 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c}**;
**R^{7a}** is selected from the group consisting of:
   **(a)** C(=O)N(**R^{f}**)₂, wherein each **R^{f}** is independently H or C₁₋₃ alkyl optionally
      substituted with 1-3 **R^{h}**;
   **(b)** C(O)N(C₁₋₃ alkyl)**R^{b1}** or -C(O)N(H)**R^{b1}**, wherein: **R^{b1}** is C₃₋₆ cycloalkyl or 4-6 membered heterocyclyl, each of which is optionally substituted with 1-3 **R^{g}**; and
   **(c)** C(O)**R^{b1}**; wherein **R^{b1}** is 4-10 membered heterocyclyl optionally substituted with 1-3 **R^{g}**, wherein **R^{b1}** is attached to the C(O) via a ring nitrogen atom;
**R^{7b}** is -halo or C₁₋₃ alkyl;
**X¹** is CH₂; and
**X²** and **X³** are independently selected from the group consisting of: O, CH₂, CH**R^{L}**, and C(**R^{L}**)₂.

**Embodiment 89.** The compound of Embodiment 88, wherein **b4** is 0.

**Embodiment 90.** The compound of Embodiment 4, wherein the compound is a compound of Formula (**III-3**): 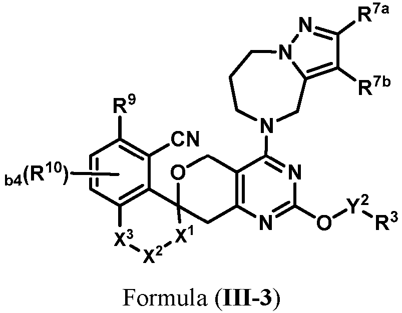 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c}**;
**R^{7a}** is selected from the group consisting of:
   **(a)** C(=O)N(**R^{f}**)₂, wherein each **R^{f}** is independently H or C₁₋₃ alkyl optionally
      substituted with 1-3 **R^{h}**;
   **(b)** C(O)N(C₁₋₃ alkyl)**R^{b1}** or -C(O)N(H)**R^{b1}**, wherein: **R^{b1}** is C₃₋₆ cycloalkyl or 4-6 membered heterocyclyl, each of which is optionally substituted with 1-3 **R^{g}**; and
   **(c)** C(O)**R^{b1}**, wherein **R^{b1}** is 4-10 membered heterocyclyl optionally substituted with 1-3 **R^{g}**, wherein **R^{b1}** is attached to the C(O) via a ring nitrogen atom;
**R^{7b}** is -halo or C₁₋₃ alkyl;
**X¹** is CH₂; and
**X²** and **X³** are independently selected from the group consisting of: O, CH₂, CH**R^{L}**, and C(**R^{L}**)₂.

**Embodiment** 91. The compound of Embodiment 90, wherein b4 is 0.

**Embodiment 92.** The compound of Embodiment 90 or 91, wherein **R⁹** is N**R^{d}R^{e}** (e.g., -NH₂).

**Embodiment 93.** The compound of Embodiment 15, wherein the compound is a compound of Formula (IV-a3) or (IV-b3): 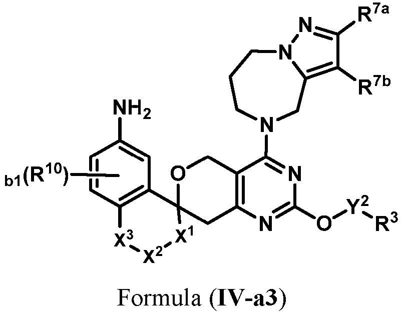 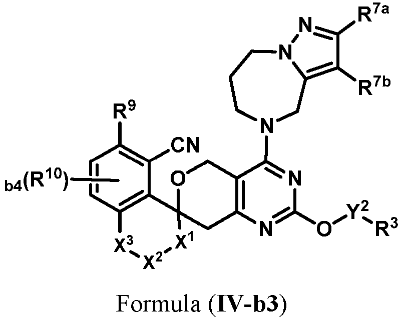 or a pharmaceutically acceptable salt thereof, wherein:
**b1** is 0, 1, or 2;
**b4** is 0 or 1;
each **R¹⁰** is independently selected from the group consisting of: -Cl, -F, -CN, and C₁₋₃ alkyl optionally substituted with 1-3 **R^{c}**;
**R^{7a}** is selected from the group consisting of:
   **(a)** C(=O)N(**R^{f}**)₂, wherein each **R^{f}** is independently H or C₁₋₃ alkyl optionally
      substituted with 1-3 **R^{h}**;
   **(b)** C(O)N(C₁₋₃ alkyl)**R^{b1}** or -C(O)N(H)**R^{b1}**, wherein: **R^{b1}** is C₃₋₆ cycloalkyl or 4-6 membered heterocyclyl, each of which is optionally substituted with 1-3 **R^{g}**; and
   **(c)** C(O)**R^{b1}**; wherein **R^{b1}** is 4-10 membered heterocyclyl optionally substituted with 1-3 **R^{g}**, wherein **R^{b1}** is attached to the C(O) via a ring nitrogen atom;
**R^{7b}** is -halo or C₁₋₃ alkyl;
**X¹** is CHz;
one of **X²** and **X³** is independently selected from the group consisting of: CH**R^{L}** and C(**R^{L}**)₂; and
the other of **X²** and **X³** is CH₂ or O.

**Embodiment 94.** The compound of Embodiment 93, wherein the compound is a compound of Formula **(IV-b3),** or a pharmaceutically acceptable salt thereof, wherein **b4** is 0.

**Embodiment 95.** The compound of Embodiment 93, wherein the compound is a compound of Formula **(IV-a3),** or a pharmaceutically acceptable salt thereof, wherein **b1** is 1 or 2; and the 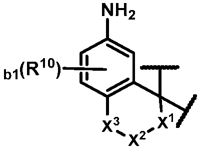 moiety is

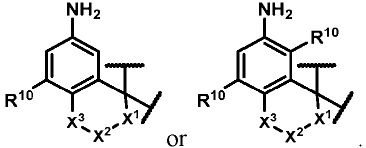

**Embodiment 96.** The compound of Embodiment 93, wherein the compound is a compound of Formula **(IV-a3),** or a pharmaceutically acceptable salt thereof, wherein **b1** is 1; and the 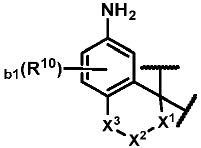 moiety is

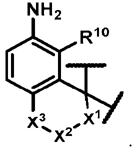

**Embodiment 97.** The compound of any one of Embodiments 93-96, wherein **X²** is CH₂; and **X³** is CH**R^{L}**.

**Embodiment 98.** The compound of Embodiment 97, wherein **R^{L}** is CF₃ or CH₃ (e.g., CH₃).

**Embodiment** 99. The compound of any one of Embodiments 86-98, wherein **R^{7a}** is C(=O)N(**R^{f}**)₂, wherein each **R^{f}** is independently C₁₋₃ alkyl optionally substituted with 1-3 **R^{h}** (e.g., **R^{7a}** is C(=O)N(Me)₂).

**Embodiment 100.** The compound of any one of Embodiments 86-99, wherein **R^{7b}** is -Cl, -F, or methyl.

**Embodiment 101.** The compound of any one of Embodiments 86-100, wherein **R^{7b}** is -Cl.

**Embodiment 102.** The compound of any one of Embodiments 86-98, wherein the 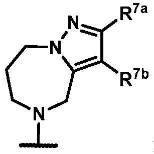 moiety is

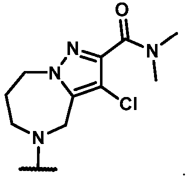

**Embodiment 103.** The compound of Embodiment 1, wherein the compound is a compound of Formula (**II-4**) or (**II-5**): 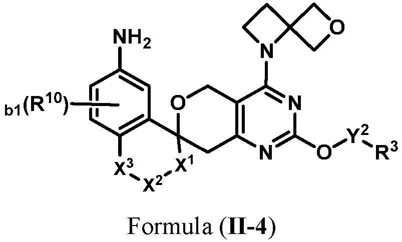 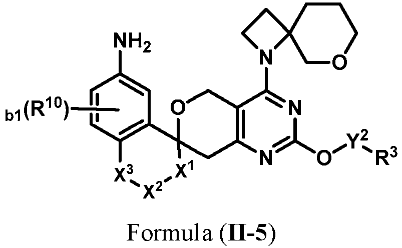 or a pharmaceutically acceptable salt thereof, wherein:
**b1** is 1 or 2;
each **R¹⁰** is independently selected from the group consisting of: -Cl, -F, -CN, and C₁₋₃ alkyl optionally substituted with 1-3 **R^{c}**;
**X¹** is CH₂; and
**X²** and **X³** are independently selected from the group consisting of: O, CH₂, CH**R^{L}**, and C(**R^{L}**)₂.

**Embodiment 104.** The compound of Embodiment 1, wherein the compound is a compound of Formula **(II-a4)** or **(II-a5):** 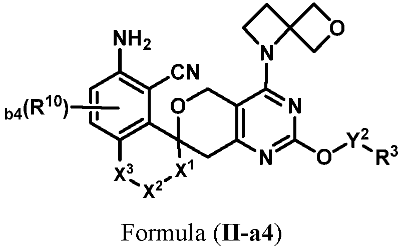 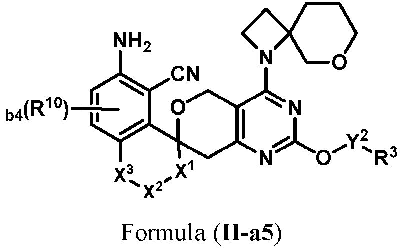 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c}**;
**X¹** is CH₂; and
**X²** and **X³** are independently selected from the group consisting of: O, CH₂, CH**R^{L}**, and C(**R^{L}**)₂.

**Embodiment 105.** The compound of Embodiment 104, wherein **b4** is 0.

**Embodiment 106.** The compound of Embodiment 4, wherein the compound is a compound of Formula **(III-4)** or **(III-5):** 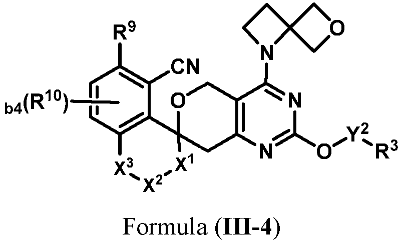 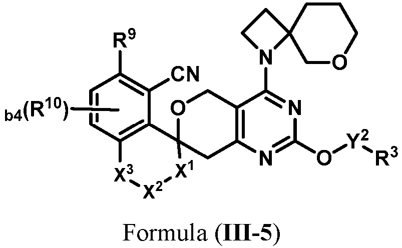 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c}**;
**X¹** is CH₂; and
**X²** and **X³** are independently selected from the group consisting of: O, CH₂, CH**R^{L}**, and C(**R^{L}**)₂.

**Embodiment 107.** The compound of Embodiment 106, wherein **b4** is 0.

**Embodiment 108.** The compound of Embodiment 106 or 107, wherein **R⁹** is N**R^{d}R^{e}** (e.g., -NH₂).

**Embodiment 109.** The compound of Embodiment 15, wherein the compound is a compound of Formula **(IV-a4), (IV-b4), (IV-a5),** or **(IV-b5):** 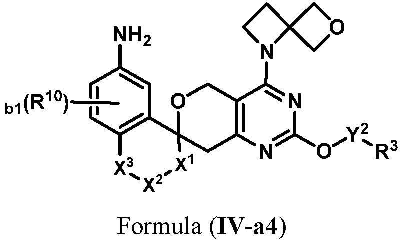 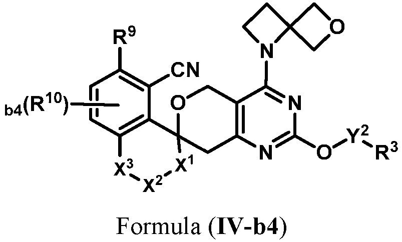 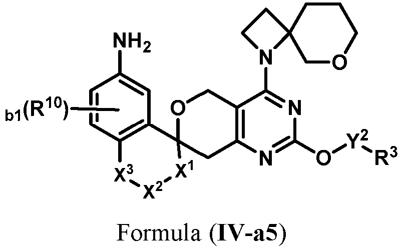 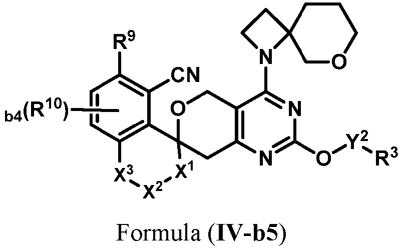 or a pharmaceutically acceptable salt thereof, wherein:
**b1** is 0, 1, or 2;
**b4** is 0 or 1;
each **R¹⁰** is independently selected from the group consisting of: -Cl, -F, -CN, and C₁₋₃ alkyl optionally substituted with 1-3 **R^{c}**;
**X¹** is CH₂;
one of **X²** and **X³** is independently selected from the group consisting of: CH**R^{L}** and C(**R^{L}**)₂; and
the other of **X²** and **X³** is CH₂ or O.

**Embodiment 110.** The compound of Embodiment 109, wherein the compound is a compound of Formula **(IV-a4)** or **(IV-a5),** or a pharmaceutically acceptable salt thereof, wherein **b1** is 1; and the 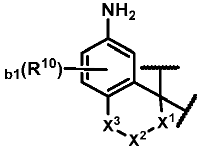 moiety is 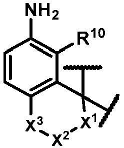 (e.g., 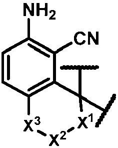 ).

**Embodiment 111.** The compound of Embodiment 109, wherein the compound is a compound of Formula **(IV-b4)** or **(IV-b5),** or a pharmaceutically acceptable salt thereof, wherein b4 is 0.

**Embodiment 112.** The compound of Embodiment 1, wherein the compound is a compound of Formula **(II-6):** 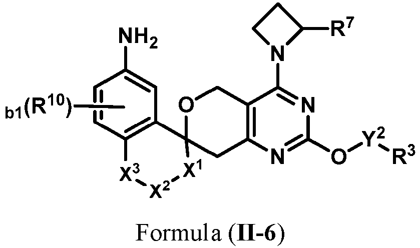 or a pharmaceutically acceptable salt thereof, wherein:
**R⁷** is C₁₋₃ alkyl substituted with -OH or C₁₋₃ alkoxy;
**b1** is 1 or 2;
each **R¹⁰** is independently selected from the group consisting of: -Cl, -F, -CN, and C₁₋₃ alkyl optionally substituted with 1-3 **R^{c}**;
**X¹** is CH₂; and
**X²** and **X³** are independently selected from the group consisting of: O, CH₂, CH**R^{L}**, and C(**R^{L}**)₂.

**Embodiment 113.** The compound of Embodiment 1, wherein the compound is a compound of Formula **(II-a6):** 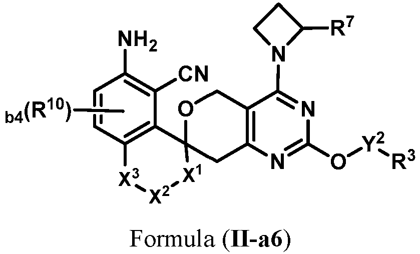 or a pharmaceutically acceptable salt thereof, wherein:
**R⁷** is C₁₋₃ alkyl substituted with -OH or C₁₋₃ alkoxy;
**b4** is 0 or 1;
each **R¹⁰** is independently selected from the group consisting of: -Cl, -F, -CN, and C₁₋₃ alkyl optionally substituted with 1-3 **R^{c}**;
**X¹** is CH₂; and
**X²** and **X³** are independently selected from the group consisting of: O, CH₂, CH**R^{L}**, and C(**R^{L}**)₂.

**Embodiment 114.** The compound of Embodiment 113, wherein **b4** is 0.

**Embodiment 115.** The compound of Embodiment 4, wherein the compound is a compound of Formula (**III-6**): 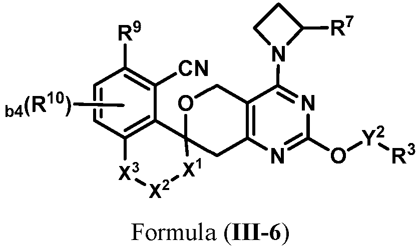 or a pharmaceutically acceptable salt thereof, wherein:
**R⁷** is C₁₋₃ alkyl substituted with -OH or C₁₋₃ alkoxy;
**b4** is 0 or 1;
each **R¹⁰** is independently selected from the group consisting of: -Cl, -F, -CN, and C₁₋₃ alkyl optionally substituted with 1-3 **R^{c}**;
**X¹** is CH₂; and
**X²** and **X³** are independently selected from the group consisting of: O, CH₂, CH**R^{L}**, and C(**R^{L}**)₂.

**Embodiment 116.** The compound of Embodiment 115, wherein **b4** is 0.

**Embodiment 117.** The compound of Embodiment 115 or 116, wherein **R⁹** is N**R^{d}R^{e}** (e.g., -NH₂).

**Embodiment 118.** The compound of Embodiment 15, wherein the compound is a compound of Formula **(IV-a6)** or **(IV-b6):** 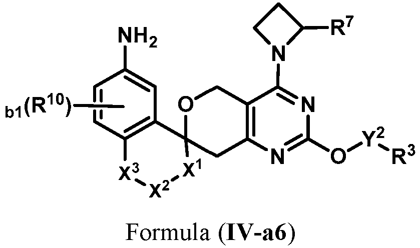 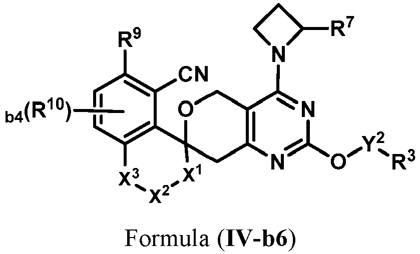 or a pharmaceutically acceptable salt thereof, wherein:
**R⁷** is C₁₋₃ alkyl substituted with -OH or C₁₋₃ alkoxy;
**b1** is 0, 1, or 2;
**b4** is 0 or 1;
each **R¹⁰** is independently selected from the group consisting of: -Cl, -F, -CN, and C₁₋₃ alkyl optionally substituted with 1-3 **R^{c}**;
**X¹** is CH₂;
one of **X²** and **X³** is independently selected from the group consisting of: CH**R^{L}** and C(**R^{L}**)₂; and
the other of **X²** and **X³** is CH₂ or O.

**Embodiment 119.** The compound of Embodiment 118, wherein the compound is a compound of Formula **(IV-a6),** or a pharmaceutically acceptable salt thereof, wherein **b1** is 1; and the 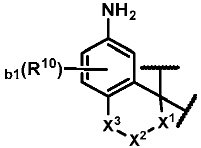 moiety is 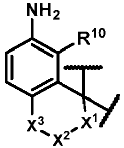 (e.g., 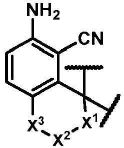 ).

**Embodiment 120.** The compound of Embodiment 118, wherein the compound is a compound of Formula **(IV-b6),** or a pharmaceutically acceptable salt thereof, wherein **b4** is 0.

**Embodiment 121.** The compound of any one of Embodiments 112-120, wherein the 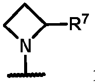 moiety is

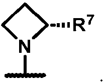

**Embodiment 122.** The compound of any one of Embodiments 112-121, wherein **R⁷** is C₁₋₃ alkyl substituted with -OH.

**Embodiment 123.** The compound of any one of Embodiments 112-122, wherein **R⁷** is -CH₂OH.

**Embodiment 124.** The compound of any one of Embodiments 103-123, wherein **X²** is CH₂; and **X³** is CH**R^{L}** (e.g., CH(CH₃)).

**Embodiment 125.** The compound of any one of Embodiments 103-108 or 112-117, wherein **X²** is CH₂; and **X³** is CH₂.

**Embodiment 126.** The compound of any one of Embodiments 67-125, wherein **Y²** is -CH₂-; and **R³** is a 4-10 membered heterocyclyl having one ring nitrogen atom and 0-1 additional ring heteroatom selected from the group consisting of oxygen and nitrogen, wherein the heterocyclyl is optionally substituted with 1-6 **R^{a}**.

**Embodiment 127.** The compound of any one of Embodiments 67-126, wherein **Y²** is -CH₂-; and **R³** is 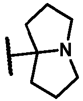 optionally substituted with 1-2 -F (e.g., **R³** is 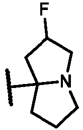 or 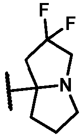 ).

**Embodiment 128.** The compound of Embodiment 126 or 127, wherein **R³** is 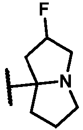 (e.g., 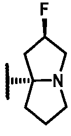 ).

**Embodiment 129.** The compound of any one of Embodiments 1-128, wherein the 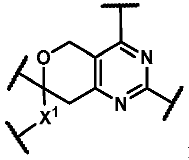 moiety is

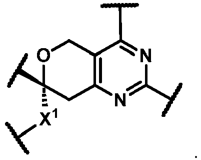

**Embodiment 130.** The compound of Embodiment 1, wherein the compound of Formula (**II**) is selected from the group consisting of Compound Nos. **181b, 501, 501a, 501b, 501c**, **502, 502a, 502b, 503, 503a, 503b, 503c, 504, 504a, 504b, 504c, 505, 505a, 506, 506a, 507, 507a, 507b, 507c, 508, 508a, 509, 509a, 510, 510a, 510b, 511, 511a, 512, 512a, 512b, 513, 513a, 514, 514a, 514b, 516, 516a, 516b, 516c, 517, 517a, 518, 518a, 519, 519a, 520, 520a, 522, 522a, 523, 523a, 524, 524a, 525, 525a, 526, 526a, 526b, 526c, 527, 527a, 527b, 528, 528a, 529, 529a, 530, 530a, 531, 531a, 532, 532a, 534, 534a, 535, 535a, 536, 536a, 536b, 536c, 537, 537a, 538, 538a, 539, 539a, 540, 540a, 541, 541a, 542, 542a, 543, 543a, 544, 544a, 545, 545a, 546, 546a, 547, 547a, 548, 548a, 549, 549a, 550, 550a, 551, 551a, 552, 552a, 553, 553a, 554, 554a, 554b, 554c, 555, 555a, 556, 556a, 558, 558a, 559, 559a, 560, 560a, 561, 561a, 562, 562a, 563, 563a, 564, 564a, 565, 565a, 566, 566a, 566b, 567, 567a, 567b, 568, 568a, 568b, 569, 569a, 570, 570a, 571, 571a, 572, 572a, 573, 573a, 575, 575a, 575b, 576, 576a, 577, 577a, 578, 578a, 579, 579a, 580, 580a, 581, 581a, 582, 582a, 583, 583a, 584, 584a, 585, 585a, 585b, 586, 586a, 587, 587a, 588, 588a, 589, 589a, 590, 590a, 591, 591a, 592,** and **592a,** as depicted in **Table C3,** or a pharmaceutically acceptable salt thereof.

**Embodiment 131.** The compound of Embodiment 4, wherein the compound of Formula **(III)** is selected from the group consisting of Compound Nos. **502, 502a, 502b, 503, 503a, 503b, 503c, 504, 504a, 504b, 504c, 505, 505a, 507, 507a, 507b, 507c, 509, 509a, 511, 511a, 512, 512a, 512b, 513, 513a, 514, 514a, 514b, 516, 516a, 516b, 516c, 517, 517a, 518, 518a, 519, 519a, 520, 520a, 522, 522a, 523, 523a, 524, 524a, 525, 525a, 526, 526a, 526b, 526c, 527, 527a, 527b, 528, 528a, 529, 529a, 530, 530a, 531, 531a, 532, 532a, 534, 534a, 535, 535a, 536, 536a, 536b, 536c, 537, 537a, 538, 538a, 539, 539a, 540, 540a, 541, 541a, 542, 542a, 543, 543a, 544, 544a, 545, 545a, 546, 546a, 547, 547a, 548, 548a, 549, 549a, 550, 550a, 551, 551a, 552, 552a, 553, 553a, 554, 554a, 554b, 554c, 555, 555a, 556, 556a, 558, 558a, 559, 559a, 560, 560a, 561, 561a, 562, 562a, 563, 563a, 564, 564a, 565, 565a, 566, 566a, 566b, 567, 567a, 567b, 568, 568a, 568b, 569, 569a, 570, 570a, 571, 571a, 572, 572a, 573, 573a, 574, 574a, 574b, 574c, 575, 575a, 575b, 576, 576a, 577, 577a, 578, 578a, 579, 579a, 580, 580a, 581, 581a, 582, 582a, 583, 583a, 584, 584a, 585, 585a, 585b, 586, 586a, 587, 587a, 588, 588a, 589, 589a, 590, 590a, 591, 591a, 592,** and **592a,** as depicted in **Table C3,** or a pharmaceutically acceptable salt thereof.

**Embodiment 132.** The compound of Embodiment 15, wherein the compound of Formula **(IV)** is selected from the group consisting of Compound Nos. **502, 502a, 502b, 508, 508a, 509, 509a, 511, 511a, 512, 512a, 512b, 530, 530a, 531, 531a, 532, 532a, 546, 546a, 553, 553a, 554, 554a, 554b, 554c, 555, 555a, 556, 556a, 560, 560a, 561, 561a, 566, 566a, 566b, 567, 567a, 567b, 568, 568a, 568b, 569, 569a, 570, 570a, 571, 571a, 572, 572a, 573, 573a, 575, 575a, 575b, 576, 576a, 577, 577a, 578, 578a, 581, 581a, 582, 582a, 583, 583a, 584, 584a, 585, 585a, 585b, 589, 589a, 590, 590a, 592,** and **592a,** as depicted in **Table C3,** or a pharmaceutically acceptable salt thereof.

### P09 EXEMPLARY EMBODIMENTS

**Embodiment 1.** A compound of Formula **(II):** 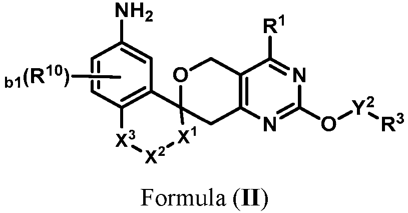 or a pharmaceutically acceptable salt thereof, wherein:
**R¹** is selected from the group consisting of:
   **(i)** a 4-10 membered heterocyclyl optionally substituted with 1-4 **R⁷**;
   **(ii)** an 8-12 membered bicyclic heterocyclyl, wherein the heterocyclyl comprises an endocyclic group selected from the group consisting of C(=O)NH and S(O)₂NH, and wherein the heterocyclyl is further optionally substituted with 1-3 **R⁷** at one or more ring carbon atoms; and
   (iii) 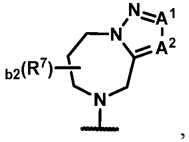 wherein **b2** is 0, 1, 2, or 3; and **A¹** and **A²** are independently selected from the group consisting of: N, CH, and C**R⁷**;
each **R⁷** is independently selected from the group consisting of **R^{a}** and **R^{b}**;
**X¹** is selected from the group consisting of a bond, S(O)₀₋₂, CH₂, CH**R^{L}**, C(**R^{L}**)₂, and O;
**X²** and **X³** are independently selected from the group consisting of: CH₂, CH**R^{L}**, C(**R^{L}**)₂, O, and S(O)₀₋₂, provided that no more than one of **X¹**, **X²**, and **X³** is selected from the group consisting of: O and S(O)₀₋₂;
**b1** is 1 or 2;
each **R¹⁰** is independently selected from the group consisting of **R^{a}** and **R^{b}**;
each **R^{L}** is independently selected from the group consisting of C₁₋₃ alkoxy, -F, CN, and C₁₋₃ alkyl optionally substituted with 1-3 **R^{c}**; or
one pair of **R^{L}** on the same or different ring carbon atom(s) taken together with the ring atom(s) connecting them form a C₃₋₆ cycloalkyl ring;
**Y²** is a bond or a straight-chain C₁₋₆ alkylene optionally substituted with 1-6 **R^{Y}**;
each **R^{Y}** is independently selected from the group consisting of: halo, cyano, -OH, oxo, C₁₋₆ alkoxy, C₁₋₆ haloalkoxy, C₁₋₆ alkyl, and C₁₋₆ haloalkyl, or
one pair of **R^{Y}** on the same or different carbon atom(s) taken together with the atom(s) connecting them forms a C₃₋₆ cycloalkyl ring or 4-6 membered heterocyclyl ring, each of which is optionally substituted with 1-3 independently selected C₁₋₃ alkyl;
**R³** is selected from the group consisting of:
   **(a)** 4-15 membered heterocyclyl optionally substituted with 1-6 substituents independently selected from the group consisting of: **R^{a}** and **R^{b}**; and
   **(b)** -N**R^{d}R^{e}**;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   **(f)** -C₁₋₆ haloalkoxy;
   **(g)** -N**R^{d}R^{e}**;
   **(h)** C(=O)C₁₋₆ alkyl;
   **(i)** C(=O)C₁₋₆ haloalkyl;
   **(j)** C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   **(o)** S(O)₀₋₂(C₁₋₆ haloalkyl);
   **(p)** S(O)₁₋₂N(**R^{f}**)₂; and
   **(q)** C₁₋₆ alkyl, C₂₋₆ alkenyl, or C₂₋₆ alkynyl, each optionally substituted with 1-6 **R^{c}**;
each **R^{b}** is independently selected from the group consisting of: -**(L^{b})_{b}-R^{b1}** and **-R^{b1}**, wherein:
   **b is** 1, 2, or 3;
   each **-L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e}**, C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)₁₋₂N(**R^{f}**)₂;
   each **R^{d}** and **R^{e}** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂-.

**Embodiment 2.** The compound of Embodiment 1, wherein the compound is a compound of Formula (**II-a**): 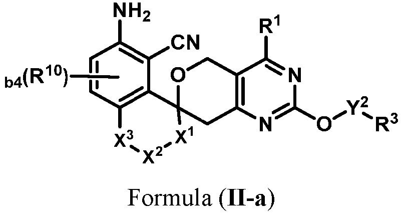 or a pharmaceutically acceptable salt thereof, wherein:
**b4** is 0 or 1; and
each **R¹⁰** is independently selected from the group consisting of: -Cl, -F, -CN, and C₁₋₃ alkyl optionally substituted with 1-3 **R^{c}**.

**Embodiment 3.** The compound of Embodiment 1, wherein the compound is a compound of Formula **(II-b):** 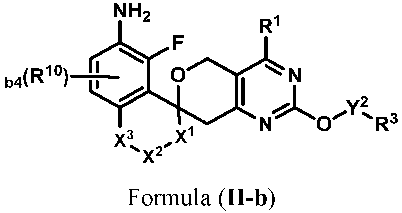 or a pharmaceutically acceptable salt thereof, wherein:
**b4** is 0 or 1; and
each **R¹⁰** is independently selected from the group consisting of: -Cl, -F, -CN, and C₁₋₃ alkyl optionally substituted with 1-3 **R^{c}**.

**Embodiment 4.** A compound of Formula **(III):** 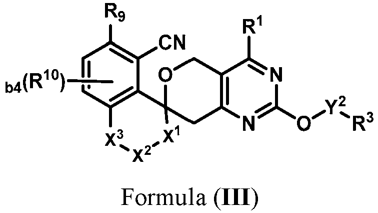 or a pharmaceutically acceptable salt thereof, wherein:
**R¹** is selected from the group consisting of:
   **(i)** a 4-10 membered heterocyclyl optionally substituted with 1-4 **R⁷**;
   **(ii)** an 8-12 membered bicyclic heterocyclyl, wherein the heterocyclyl comprises an endocyclic group selected from the group consisting of C(=O)NH and S(O)₂NH, and wherein the heterocyclyl is further optionally substituted with 1-3 **R⁷** at one or more ring carbon atoms; and
   (iii) 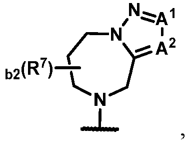 wherein **b2** is 0, 1, 2, or 3; and **A¹** and **A²** are independently selected from the group consisting of: N, CH, and C**R⁷**;
each **R⁷** is independently selected from the group consisting of **R^{a}** and **R^{b}**;
**X¹** is selected from the group consisting of a bond, S(O)₀₋₂, CH₂, CH**R^{L}**, C(**R^{L}**)₂, and O;
**X²** and **X³** are independently selected from the group consisting of: CH₂, CH**R^{L}**, C(**R^{L}**)₂, O, and S(O)₀₋₂, provided that no more than one of **X¹**, **X²**, and **X³** is selected from the group consisting of: O and S(O)₀₋₂;
**R⁹** is selected from the group consisting of: H, N**R^{d}R^{e}**, -OH, and halo;
**b4 is 0 or** 1;
each **R¹⁰** is independently selected from the group consisting of **R^{a}** and **R^{b}**;
each **R^{L}** is independently selected from the group consisting of C₁₋₃ alkoxy, -F, CN, and C₁₋₃ alkyl optionally substituted with 1-3 **R^{c}**; or
one pair of **R^{L}** on the same or different ring carbon atom(s) taken together with the ring atom(s) connecting them form a C₃₋₆ cycloalkyl ring;
**Y²** is a bond or a straight-chain C₁₋₆ alkylene optionally substituted with 1-6 **R^{Y}**;
each **R^{Y}** is independently selected from the group consisting of: halo, cyano, -OH, oxo, C₁₋₆ alkoxy, C₁₋₆ haloalkoxy, C₁₋₆ alkyl, and C₁₋₆ haloalkyl, or
one pair of **R^{Y}** on the same or different carbon atom(s) taken together with the atom(s) connecting them forms a C₃₋₆ cycloalkyl ring or 4-6 membered heterocyclyl ring, each of which is optionally substituted with 1-3 independently selected C₁₋₃ alkyl;
**R³** is selected from the group consisting of:
   **(a)** 4-15 membered heterocyclyl optionally substituted with 1-6 substituents independently selected from the group consisting of: **R^{a}** and **R^{b}**; and
   **(b)** -N**R^{d}R^{e}**;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   **(f)** -C₁₋₆ haloalkoxy;
   **(g)** -N**R^{d}R^{e}**;
   **(h)** C(=O)C₁₋₆ alkyl;
   **(i)** C(=O)C₁₋₆ haloalkyl;
   **(j)** C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   **(o)** S(O)₀₋₂(C₁₋₆ haloalkyl);
   **(p)** S(O)₁₋₂N(**R^{f}**)₂; and
   **(q)** C₁₋₆ alkyl, C₂₋₆ alkenyl, or C₂₋₆ alkynyl, each optionally substituted with 1-6 **R^{c}**;
each **R^{b}** is independently selected from the group consisting of: -**(L^{b})_{b}-R^{b1}** and -**R^{b1}**, wherein:
   **b** is 1, 2, or 3;
   each **-L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e}**, C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)₁₋₂N(**R^{f}**)₂;
   each **R^{d}** and **R^{e}** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂-.

**Embodiment 5.** The compound of Embodiment 4, wherein **R⁹** is -NH₂; and each **R¹⁰** is independently selected from the group consisting of: -Cl, -F, -CN, and C₁₋₃ alkyl optionally substituted with 1-3 **R^{c}**.

**Embodiment 6.** The compound of any one of Embodiments 1-5, wherein **X¹** is CH₂ or CH**R^{L}**.

**Embodiment 7.** The compound of any one of Embodiments 1-6, wherein **X²** and **X³** are independently selected from the group consisting of: CH₂, CH**R^{L}**, and C(**R^{L}**)₂.

**Embodiment 8.** The compound of any one of Embodiments 1-7, wherein **X¹** is CH₂; and **X²** and **X³** are both CH₂.

**Embodiment 9.** The compound of any one of Embodiments 1-7, wherein at least one (e.g., one) of **X¹**, **X²**, and **X³** is selected from the group consisting of: CH**R^{L}** and C(**R^{L}**)₂.

**Embodiment 10.** The compound of any one of Embodiments 1-7 or 9, wherein **X¹** is CH₂; and **X²** and **X³** are independently selected from the group consisting of: CH₂, CH**R^{L}**, and C(**R^{L}**)₂, provided that 1-2 of **X²** and **X³** is independently CH**R^{L}** or C(**R^{L}**)₂.

**Embodiment 11.** The compound of any one of Embodiments 1-7 or 9-10, wherein **X¹** is CH₂; **X²** is CH₂; and **X³** is CH**R^{L}** (e.g., **X³** is C(H)CF₃; or e.g., **X³** is C(H)CH₃).

**Embodiment 12.** The compound of any one of Embodiments 1-11, wherein each **R^{L}** is independently selected from the group consisting of: CH₃, CF₃, CHF₂, and CH₂F (e.g., CH₃).

**Embodiment 13.** The compound of Embodiment 1, wherein the 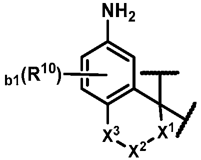 moiety is selected from the group consisting of: 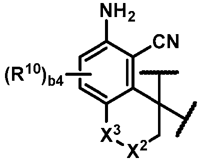 and 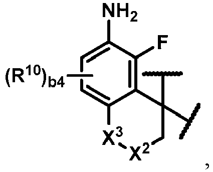 wherein:
**b4** is 0 or 1;
**X²** is -O- or -CH₂-;
**X³** is -CH₂- or -CH**R^{L}**-, wherein **R^{L}** is C₁₋₃ alkyl (e.g., methyl); and
each **R¹⁰** is independently selected from the group consisting of: -Cl, -F, -CN, and C₁₋₃ alkyl optionally substituted with 1-3 **R^{c}**.

**Embodiment 14.** The compound of Embodiment 13, wherein the 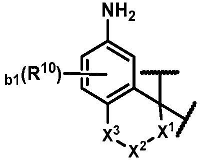 moiety is selected from the group consisting of:

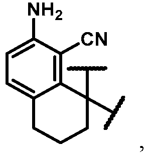

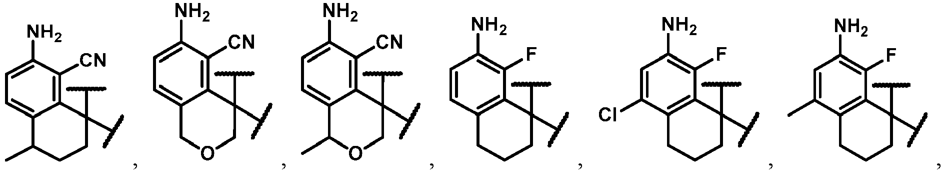

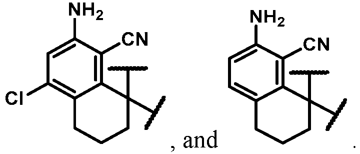

**Embodiment 15.** A compound of Formula (IV): 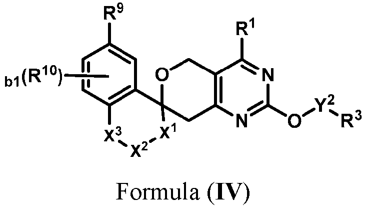 or a pharmaceutically acceptable salt thereof, wherein:
**X¹** is selected from the group consisting of a bond, S(O)₀₋₂, CH₂, CH**R^{L}**, C(**R^{L}**)₂, and O;
**X²** and **X³** are independently selected from the group consisting of: CH₂, CH**R^{L}**, C(**R^{L}**)₂, O, and S(O)₀₋₂, provided that at least one of **X¹**, **X²**, and **X³** is CH**R^{L}** or C(**R^{L}**)₂;
further provided that no more than one of **X¹**, **X²**, and **X³** is selected from the group consisting of: O and S(O)₀₋₂;
**b1** is 0, 1 or 2;
**R⁹** is selected from the group consisting of: H, OH, N**R^{d}R^{e}**, and halo;
each **R¹⁰** is independently selected from the group consisting of **R^{a}** and **R^{b}**;
each **R^{L}** is independently selected from the group consisting of C₁₋₃ alkoxy, -F, CN, and C₁₋₃ alkyl optionally substituted with 1-3 **R^{c}**;
**R¹** is selected from the group consisting of:
   **(i)** a 4-10 membered heterocyclyl optionally substituted with 1-4 **R⁷**;
   **(ii)** an 8-12 membered bicyclic heterocyclyl, wherein the heterocyclyl comprises an endocyclic group selected from the group consisting of C(=O)NH and S(O)₂NH, and wherein the heterocyclyl is further optionally substituted with 1-3 **R⁷** at one or more ring carbon atoms; and
   **(iii)** 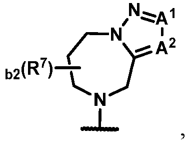 wherein **b2** is 0, 1, 2, or 3; and **A¹** and **A²** are independently selected from the group consisting of: N, CH, and C**R⁷**;
each **R⁷** is independently selected from the group consisting of **R^{a}** and **R^{b}**;
**Y²** is a bond or a straight-chain C₁₋₆ alkylene optionally substituted with 1-6 **R^{Y}**;
each **R^{Y}** is independently selected from the group consisting of: halo, cyano, -OH, oxo, C₁₋₆ alkoxy, C₁₋₆ haloalkoxy, C₁₋₆ alkyl, and C₁₋₆ haloalkyl, or
one pair of **R^{Y}** on the same or different carbon atom(s) taken together with the atom(s) connecting them forms a C₃₋₆ cycloalkyl ring or 4-6 membered heterocyclyl ring, each of which is optionally substituted with 1-3 independently selected C₁₋₃ alkyl;
**R³** is selected from the group consisting of:
   **(a)** 4-15 membered heterocyclyl optionally substituted with 1-6 substituents independently selected from the group consisting of: **R^{a}** and **R^{b}**; and
   **(b)** -N**R^{d}R^{e}**;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   **(f)** -C₁₋₆ haloalkoxy;
   **(g)** -N**R^{d}R^{e}**;
   **(h)** C(=O)C₁₋₆ alkyl;
   **(i)** C(=O)C₁₋₆ haloalkyl;
   **(j)** C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   **(o)** S(O)₀₋₂(C₁₋₆ haloalkyl);
   **(p)** S(O)₁₋₂N(**R^{f}**)₂; and
   **(q)** C₁₋₆ alkyl, C₂₋₆ alkenyl, or C₂₋₆ alkynyl, each optionally substituted with 1-6 **R^{c}**;
each **R^{b}** is independently selected from the group consisting of: -**(L^{b})_{b}-R^{b1}** and -**R^{b1}**, wherein:
   **b is** 1, 2, or 3;
   each **-L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e}**, C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)₁₋₂N(**R^{f}**)₂;
   each **R^{d}** and **R^{e}** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂-.

**Embodiment 16.** The compound of Embodiment 15, wherein the compound is a compound of Formula **(IV-a):** 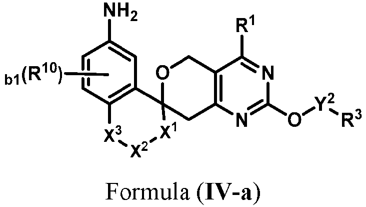 or a pharmaceutically acceptable salt thereof, wherein:
each **R¹⁰** is independently selected from the group consisting of: -Cl, -F, -CN, and C₁₋₃ alkyl optionally substituted with 1-3 **R^{c}**.

**Embodiment 17.** The compound of Embodiment 15, wherein the compound is a compound of Formula **(IV-b):** 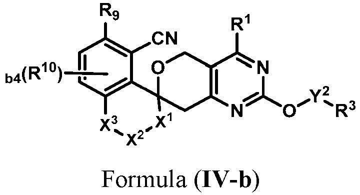 or a pharmaceutically acceptable salt thereof, wherein:
**b4** is 0 or 1; and
each **R¹⁰** is independently selected from the group consisting of: -Cl, -F, -CN, and C₁₋₃ alkyl optionally substituted with 1-3 **R^{c}**.

**Embodiment 18.** The compound of any one of Embodiments 15-17, wherein **X¹** is CH₂.

**Embodiment 19.** The compound of any one of Embodiments 15-18, wherein **X²** is CH₂; and **X³** is CH**R^{L}**.

**Embodiment 20.** The compound of any one of Embodiments 15-19, wherein each **R^{L}** is independently selected from the group consisting of: CH₃, CF₃, CHF₂, and CH₂F (e.g., CH₃).

**Embodiment 21.** The compound of any one of Embodiments 15-20, wherein one **R^{L}** is CF₃; or wherein one **R^{L}** is CH₃.

**Embodiment 22.** The compound of any one of Embodiments 15-17, wherein the compound is a compound of Formula **(IV-c):** 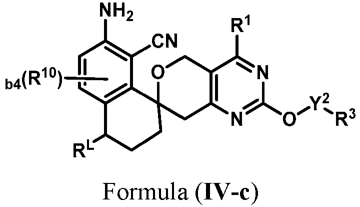 or a pharmaceutically acceptable salt thereof, wherein:
**b4** is 0 or 1; and
each **R¹⁰** is independently selected from the group consisting of: -Cl, -F, -CN, and C₁₋₃ alkyl optionally substituted with 1-3 **R^{c}**.

**Embodiment 23.** The compound of Embodiment 22, wherein **R^{L}** is CF₃; or wherein **R^{L}** is CH₃.

**Embodiment 24.** The compound of any one of Embodiments 1-23, wherein **Y²** is -CH₂-; and **R³** is a 4-10 membered heterocyclyl having one ring nitrogen atom and 0-1 additional ring heteroatom selected from the group consisting of oxygen and nitrogen, wherein the heterocyclyl is optionally substituted with 1-6 **R^{a}**.

**Embodiment 25.** The compound of any one of Embodiments 1-24, wherein **Y²** is -CH₂-; and **R³** is 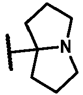 optionally substituted with 1-2 **R^{a}**.

**Embodiment 26.** The compound of any one of Embodiments 1-25, wherein **Y²** is -CH₂-; and **R³** is 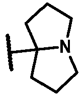 optionally substituted with 1-2 substituents each independently selected from the group consisting of: -F, -C₁₋₃ alkoxy, and -C₁₋₃ haloalkoxy (e.g., **R³** is 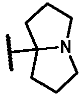 optionally substituted with 1-2 -F).

**Embodiment 27.** The compound of any one of Embodiments 1-26, wherein **R³** is 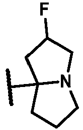 (e.g., 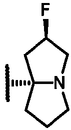 ).

**Embodiment 28.** The compound of any one of Embodiments 1-26, wherein **R³** is

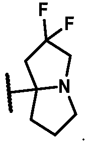

**Embodiment 29.** The compound of any one of Embodiments 1-26, wherein **R³** is

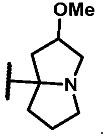

**Embodiment 30.** The compound of any one of Embodiments 1-29, wherein **R¹** is 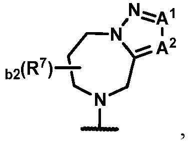 wherein **b2** is 0, 1, or 2, and **A¹** and **A²** are independently selected from the group consisting of: N, CH, and **CR⁷**.

**Embodiment 31.** The compound of any one of Embodiments 1-30, wherein **R¹** is 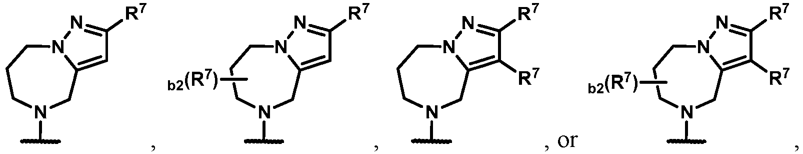 wherein b2 is 1 or 2.

**Embodiment 32.** The compound of any one of Embodiments 1-31, wherein **R¹** is 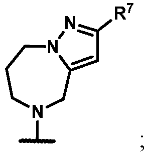 and **R⁷** is selected from the group consisting of: C(=O)N(**R^{f}**)₂, C(O)N(C₁₋₃ alkyl)**R^{b1}**, C(O)N(H)**R^{b1}**, **R^{b1}**, and C(O)**R^{b1}**.

**Embodiment 33.** The compound of Embodiment 32, wherein **R⁷** is selected from the group consisting of:
**(a)** C(=O)N(**R^{f}**)₂, wherein each **R^{f}** is independently H or C₁₋₃ alkyl optionally
   substituted with 1-3 **R^{h}**;
**(b)** C(O)N(C₁₋₃ alkyl)**R^{b1}** or -C(O)N(H)**R^{b1}**, wherein **R^{b1}** is C₃₋₆ cycloalkyl or 4-6 membered heterocyclyl, each of which is optionally substituted with 1-3 **R^{g}**; and
**(c)** C(O)**R^{b1}**, wherein **R^{b1}** is 4-10 membered heterocyclyl optionally substituted with 1-3 **R^{g}**, wherein **R^{b1}** is attached to the C(O) via a ring nitrogen atom.

**Embodiment 34.** The compound of any one of Embodiments 31-33, wherein **R⁷** is C(=O)N(**R^{f}**)₂, wherein each **R^{f}** is independently H or C₁₋₃ alkyl optionally substituted with 1-3 **R^{h}** (e.g., **R⁷** is C(=O)N(Me)₂).

**Embodiment 35.** The compound of Embodiment 32, wherein **R⁷** is **R^{b1}**, wherein the **R^{b1}** is a 5-6 membered heteroaryl optionally substituted with 1-2 **R^{g}**, or wherein the **R^{b1}** is oxetanyl optionally substituted with **R^{g}**.

Embodiment 36. The compound of any one of Embodiments 1-31, wherein **R¹** is 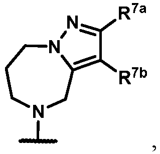 wherein **R^{7a}** and **R^{7b}** are independently selected **R⁷**.

**Embodiment** 37. The compound of Embodiment 36, wherein **R^{7a}** is selected from the group consisting of: C(=O)N(**R^{f}**)₂, C(O)N(C₁₋₃ alkyl)**R^{b1}**, -C(O)N(H)**R^{b1}**, **R^{b1}**, and C(O)**R^{b1}**; and
**R^{7b}** is -halo, -CN, and C₁₋₃ alkyl optionally substituted with 1-3 **R^{c}.**

**Embodiment 38.** The compound of Embodiment 36 or 37, wherein **R^{7a}** is selected from the group consisting of:
(a) C(=O)N(**R^{f}**)₂, wherein each **R^{f}** is independently H or C₁₋₃ alkyl optionally substituted with 1-3 **R^{h}**;
(b) C(O)N(C₁₋₃ alkyl)**R^{b1}** or -C(O)N(H)**R^{b1}**, wherein **R^{b1}** is C₃₋₆ cycloalkyl or 4-6 membered heterocyclyl, each of which is optionally substituted with 1-3 **R^{g}**; and
**(c)** C(O)**R^{b1}**, wherein **R^{b1}** is 4-10 membered heterocyclyl optionally substituted with 1-3 **R^{g}**, wherein **R^{b1}** is attached to the C(O) via a ring nitrogen atom.

**Embodiment 39.** The compound of any one of Embodiments 36-38, wherein **R^{7a}** is -C(=O)N(**R^{f}**)₂, wherein each **R^{f}** is independently H or C₁₋₃ alkyl optionally substituted with 1-3 **R^{h}** (e.g., **R^{7a}** is C(=O)N(Me)₂); and **R^{7b}** is -Cl, -F, or methyl.

**Embodiment 40.** The compound of Embodiment 36 or 37, wherein **R^{7a}** is **R^{b1}**, wherein the **R^{b1}** is a 5-6 membered heteroaryl optionally substituted with 1-2 **R^{g}**, or wherein the **R^{b1}** is oxetanyl optionally substituted with **R^{g}**.

**Embodiment 41.** The compound of any one of Embodiments 1-29, wherein **R¹** is a 7-10 (e.g., 7) membered heterocyclyl having one ring nitrogen atom, one ring oxygen atom, and no additional ring heteroatoms, wherein the 7-10 membered heterocyclyl is optionally substituted with 1-4 (e.g., 1-2) **R⁷**.

**Embodiment 42.** The compound of any one of Embodiments 1-29 or 41, wherein **R¹** is a 7-10 (e.g., 7) membered monocyclic heterocyclyl having one ring nitrogen atom, one ring oxygen atom, and no additional ring heteroatoms, wherein the 7-10 membered monocyclic heterocyclyl is optionally substituted with 1-4 (e.g., 1-2) **R⁷**.

**Embodiment 43.** The compound of any one of Embodiments 1-29 or 41-42, wherein **R¹** is 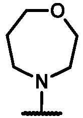 optionally substituted with 1-4 **R⁷** at one or more ring carbon atoms (e.g., **R¹** is 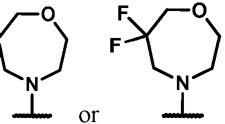 ).

**Embodiment 44.** The compound of any one of Embodiments 41-43, wherein each **R⁷** is independently selected from the group consisting of: -OH; -CN; -F; and C₁₋₃ alkyl optionally substituted with 1-3 **R^{c}**, wherein:
each **R^{c}** is independently selected from the group consisting of: -F, -OH, and -CN.

**Embodiment 45.** The compound of any one of Embodiments 1-29 or 41-42, wherein **R¹** is 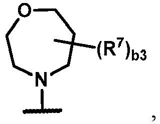 wherein b3 is 1, 2, or 3.

**Embodiment 46.** The compound of Embodiment 45, wherein one occurrence of **R⁷** is **R^{b}** (e.g., one occurrence of **R⁷** is **R^{b1}**).

**Embodiment 47.** The compound of Embodiment 45 or 46, wherein one occurrence of **R⁷** is a 5-6 membered heteroaryl optionally substituted with 1-3 **R^{g}**.

**Embodiment 48.** The compound of any one of Embodiments 45-47, wherein one occurrence of **R⁷** is a 5-membered heteroaryl optionally substituted with 1-3 **R^{g}**.

**Embodiment 49.** The compound of any one of Embodiments 45-48, wherein one occurrence of **R⁷** is selected from the group consisting of pyrazolyl and oxazolyl, each of which is optionally substituted with 1-2 **R^{g}** (e.g., **R⁷** is 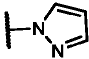 ).

**Embodiment 50.** The compound of any one of Embodiments 45-49, wherein b3 is 1.

**Embodiment 51.** The compound of any one of Embodiments 1-29 or 41-42, wherein **R¹** is 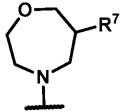 (e.g., 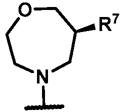 ), wherein **R⁷** is a 5-membered heteroaryl optionally substituted with 1-3 **R^{g}**.

**Embodiment 52.** The compound of Embodiment 51, wherein **R⁷** is selected from the group consisting of pyrazolyl and oxazolyl, each of which is optionally substituted with 1-2 **R^{g}**.

**Embodiment 53.** The compound of Embodiment 51 or 52, wherein **R⁷** is pyrazolyl optionally substituted with 1-2 **R^{g}** (e.g., **R⁷** is 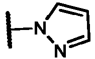 optionally substituted with one **R^{g}**); or wherein **R⁷** is oxazolyl optionally substituted with one **R^{g}** (e.g., **R⁷** is 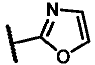 optionally substituted with one **R^{g}**).

**Embodiment 54.** The compound of any one of Embodiments 51-53, wherein **R⁷** is

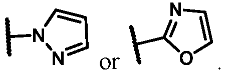

**Embodiment 55.** The compound of any one of Embodiments 1-29 or 41, wherein **R¹** is a 7-10 (e.g., 7) membered bicyclic heterocyclyl having one ring nitrogen atom, one ring oxygen atom, and no additional ring heteroatoms, wherein the 7-10 membered bicyclic heterocyclyl is optionally substituted with 1-4 (e.g., 1-2) **R⁷**.

**Embodiment 56.** The compound of any one of Embodiments 1-29, 41, or 55, wherein **R¹** is a 7-10 (e.g., 7; e.g., 9) membered spirocyclic bicyclic heterocyclyl having one ring nitrogen atom, one ring oxygen atom, and no additional ring heteroatoms, wherein the 7-10 membered spirocyclic bicyclic heterocyclyl is optionally substituted with 1-4 (e.g., 1-2) **R⁷**.

**Embodiment 57.** The compound of any one of Embodiments 1-29, 41, or 55-56, wherein **R¹** is 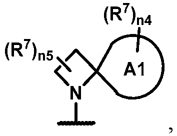 wherein: **Ring A¹** is a 4-7 membered heterocyclyl ring having one ring oxygen atom and no additional ring heteroatoms; **n4** is 0, 1, or 2; and **n5** is 0, 1, or 2, provided that **n4 + n5** is 0, 1, or 2.

**Embodiment 58.** The compound of any one of Embodiments 1-29, 41, or 55-57, wherein **R¹** is 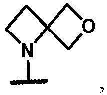 which is optionally substituted with 1-2 **R⁷**.

**Embodiment 59.** The compound of any one of Embodiments 1-29, 41, or 55-58, wherein **R¹** is

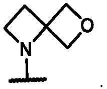

**Embodiment 60.** The compound of any one of Embodiments 1-29, 41, or 55-57, wherein **R¹** is 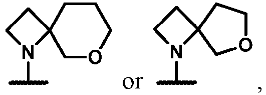 each of which is optionally substituted with 1-2 **R⁷**.

**Embodiment 61.** The compound of any one of Embodiments 1-29, 41, 55-57, or 60, wherein **R¹** is 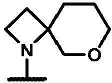 (e.g., 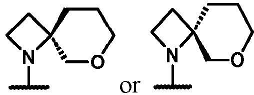 ) or 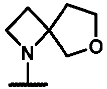 (e.g., 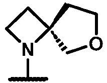 or 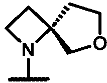 ).

**Embodiment 62.** The compound of any one of Embodiments 1-29, wherein **R¹** is a 4-membered heterocyclyl optionally substituted with 1-2 **R⁷**.

**Embodiment 63.** The compound of any one of Embodiments 1-29 or 62, wherein **R¹** is 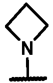 optionally substituted with 1-2 **R⁷**.

**Embodiment 64.** The compound of any one of Embodiments 1-29 or 62-63, wherein **R¹** is selected from the group consisting of:

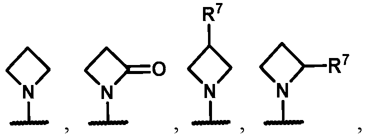

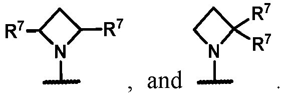

**Embodiment 65.** The compound of any one of Embodiments 1-29 or 62-64, wherein **R¹** is 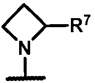 (e.g., 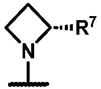 ), 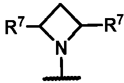 (e.g., 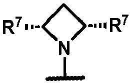 ), or

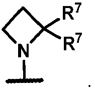

**Embodiment 66.** The compound of any one of Embodiments 62-65, wherein each **R⁷** is independently selected from the group consisting of:
-F,
-cyano,
-OH,
-**R^{b1}**, wherein the **R^{b1}** is a 5-6 membered heteroaryl optionally substituted with 1-2 **R^{g}**,
C₁₋₃ alkyl optionally substituted with 1-3 F,
C₁₋₃ alkyl substituted with -OH or C₁₋₃ alkoxy, and
C(=O)N(**R^{f}**)₂.

**Embodiment 67.** The compound of any one of Embodiments 1-29, wherein **R¹** is 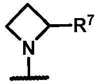 (e.g., 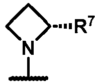 ), wherein **R⁷** is C₁₋₃ alkyl substituted with -OH or C₁₋₃ alkoxy (e.g., C₁₋₃ alkyl substituted with -OH).

**Embodiment 68.** The compound of any one of Embodiments 1-29 or 67, wherein **R¹** is 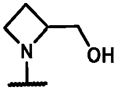 (e.g., 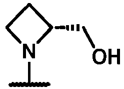 ).

**Embodiment 69.** The compound of any one of Embodiments 1-29, wherein **R¹** is 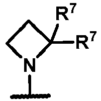 or 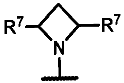 (e.g., 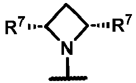 ), wherein each **R⁷** is independently selected from the group consisting of:
C₁₋₃ alkyl optionally substituted with 1-3 F; and
C₁₋₃ alkyl substituted with -OH or C₁₋₃ alkoxy.

**Embodiment 70.** The compound of any one of Embodiments 1-29 or 69, wherein **R¹** is 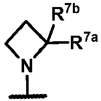 (e.g., 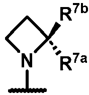 ) or 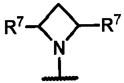 (e.g., 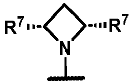 ), wherein **R^{7a}** is C₁₋₃ alkyl substituted with -OH (e.g., -CH₂OH); and
**R^{7b}** is selected from the group consisting of:
C₁₋₃ alkyl optionally substituted with 1-3 F (e.g., methyl), and
C₁₋₃ alkyl substituted with -OH or C₁₋₃ alkoxy.

**Embodiment 71.** The compound of any one of Embodiments 1-27 or 69-70, wherein **R¹** is 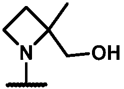 (e.g., 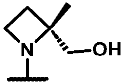 ).

**Embodiment 72.** The compound of any one of Embodiments 1-27 or 69-70, wherein **R¹** is 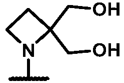 or 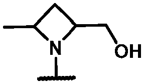 (e.g.,

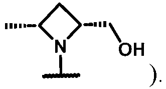

**Embodiment 73.** The compound of Embodiment 1, wherein the compound is a compound of Formula (**II-a1**) or (**II-b1**): 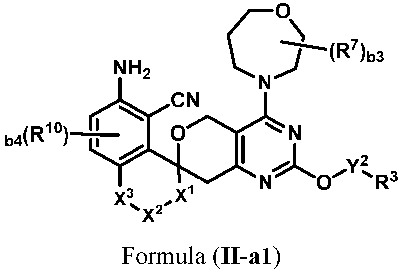 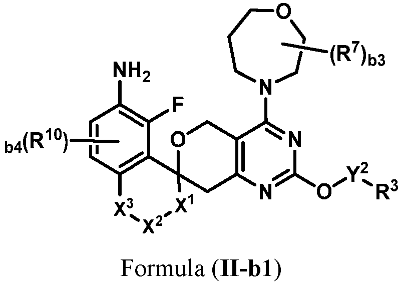 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c}**;
**b3** is 0, 1, 2, or 3;
**X¹** is CH₂; and
**X²** and **X³** are independently selected from the group consisting of: O, CH₂, CH**R^{L}**, and C(**R^{L}**)₂.

**Embodiment 74.** The compound of Embodiment 4, wherein the compound is a compound of Formula (**III-1**): 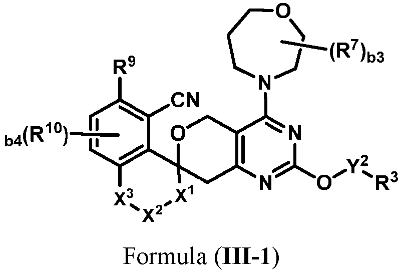 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c}**;
**b3** is 0, 1, 2, or 3;
**X¹** is CH₂; and
**X²** and **X³** are independently selected from the group consisting of: O, CH₂, CH**R^{L}**, and C(**R^{L}**)₂.

**Embodiment 75.** The compound of Embodiment 74, wherein **R⁹** is N**R^{d}R^{e}** (e.g., - NH₂).

**Embodiment 76.** The compound of Embodiment 15, wherein the compound is a compound of Formula (**IV-a1**) or (**IV-b1**): 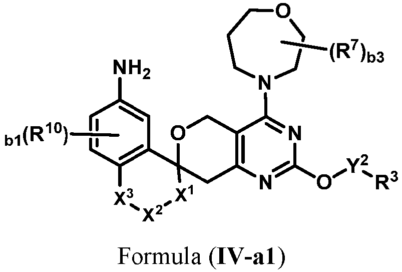 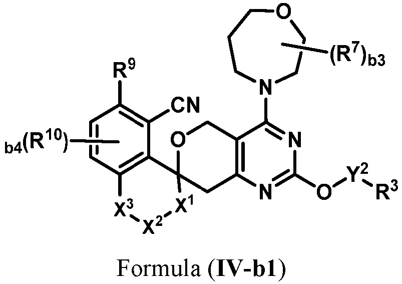 or a pharmaceutically acceptable salt thereof, wherein:
**b4** is 0 or 1;
**b1** is 0, 1, or 2;
each **R¹⁰** is independently selected from the group consisting of: -Cl, -F, -CN, and C₁₋₃ alkyl optionally substituted with 1-3 **R^{c}**;
**b3** is 0, 1, 2, or 3;
**X¹** is CHz;
one of **X²** and **X³** is independently selected from the group consisting of: CH**R^{L}** and C(**R^{L}**)₂; and
the other of **X²** and **X³** is CH₂ or O.

**Embodiment 77.** The compound of Embodiment 76, wherein **X²** is CH₂; and **X³** is CH**R^{L}**.

**Embodiment 78.** The compound of Embodiment 77, wherein **R^{L}** is CF₃; or wherein **R^{L}** is CH₃.

**Embodiment 79.** The compound of Embodiment 77 or 78, wherein **b3** is 0.

**Embodiment 80.** The compound of Embodiment 77 or 78, wherein **b3** is 1 or 2; and each **R⁷** is independently selected from the group consisting of: -OH; -CN; -F; and C₁₋₃ alkyl optionally substituted with 1-3 **R^{c}**, wherein each **R^{c}** is independently selected from the group consisting of: -F, -OH, and -CN.

**Embodiment 81.** The compound of Embodiment 77 or 78, wherein **b3** is 1; and the 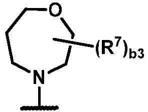 moiety is 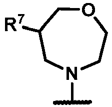 (e.g., 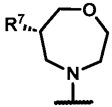 ), wherein **R⁷** is a 5-membered heteroaryl optionally substituted with 1-3 **R^{g}**.

**Embodiment 82.** The compound of Embodiment 81, wherein **R⁷** is pyrazolyl optionally substituted with 1-2 **R^{g}** (e.g., **R⁷** is 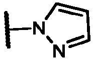 optionally substituted with one **R^{g}**).

**Embodiment 83.** The compound of Embodiment 81 or 82, wherein **R⁷** is

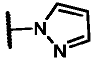

**Embodiment 84.** The compound of Embodiment 1, wherein the compound is a compound of Formula **(II-a2)** or **(II-b2):** 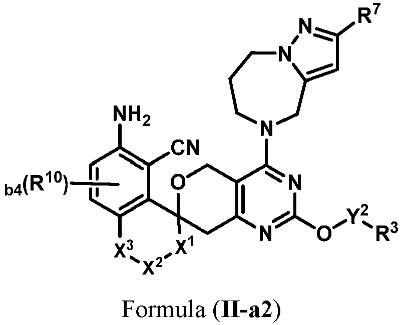 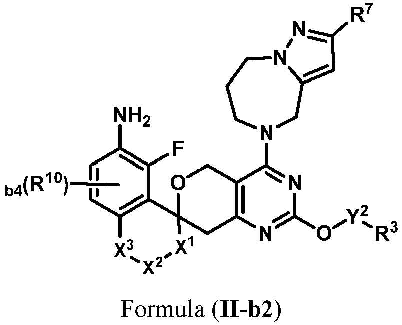 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c}**;
**R⁷** is selected from the group consisting of: C(=O)N(**R^{f}**)₂, C(O)N(C₁₋₃ alkyl)**R^{b1}**, - C(O)N(H)**R^{b1}**, **R^{b1}**, and C(O)**R^{b1}**;
**X¹** is CH₂; and
**X²** and **X³** are independently selected from the group consisting of: O, CH₂, CH**R^{L}**, and C(**R^{L}**)₂.

**Embodiment 85.** The compound of Embodiment 4, wherein the compound is a compound of Formula (**III-2**): 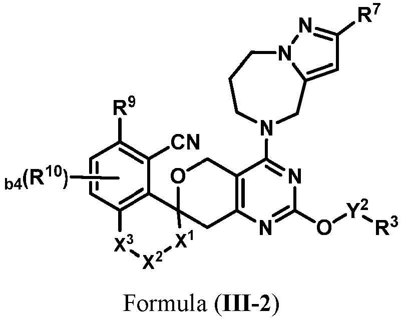 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c}**;
**R⁷** is selected from the group consisting of: C(=O)N(**R^{f}**)₂, C(O)N(C₁₋₃ alkyl)**R^{b1}**, - C(O)N(H)**R^{b1}**, **R^{b1}**, and C(O)**R^{b1}**;
**X¹** is CH₂; and
**X²** and **X³** are independently selected from the group consisting of: O, CH₂, CH**R^{L}**, and C(**R^{L}**)₂.

**Embodiment 86.** The compound of Embodiment 85, wherein **R⁹** is N**R^{d}R^{e}** (e.g., - NH₂).

**Embodiment 87.** The compound of Embodiment 15, wherein the compound is a compound of Formula **(IV-a2)** or **(IV-b2):** 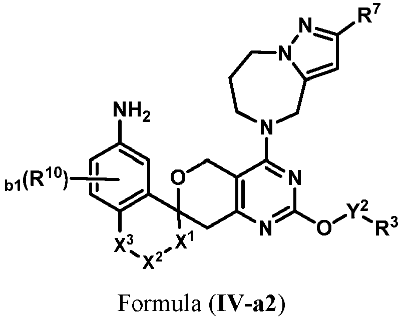 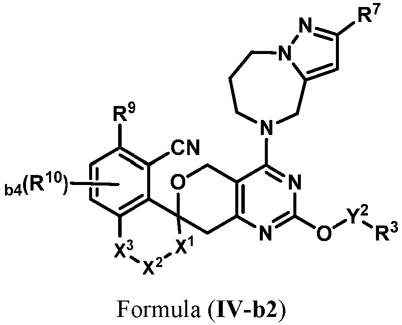 or a pharmaceutically acceptable salt thereof, wherein:
**b4** is 0 or 1;
**b1** is 0, 1, or 2;
each **R¹⁰** is independently selected from the group consisting of: -Cl, -F, -CN, and C₁₋₃ alkyl optionally substituted with 1-3 **R^{c}**;
**R⁷** is selected from the group consisting of: C(=O)N(**R^{f}**)₂, C(O)N(C₁₋₃ alkyl)**R^{b1}**, - C(O)N(H)**R^{b1}**, **R^{b1}**, and C(O)**R^{b1}**;
**X¹** is CH₂;
one of **X²** and **X³** is independently selected from the group consisting of: CH**R^{L}** and C(**R^{L}**)₂; and
the other of **X²** and **X³** is CH₂ or O.

**Embodiment 88.** The compound of Embodiment 87, wherein **X²** is CH₂; and **X³** is CH**R^{L}**.

**Embodiment 89.** The compound of Embodiment 88, wherein **R^{L}** is CF₃; or wherein **R^{L}** is CH₃.

**Embodiment 90.** The compound of any one of Embodiments 84-89, wherein **R⁷** is selected from the group consisting of:
**(a)** C(=O)N(**R^{f}**)₂, wherein each **R^{f}** is independently H or C₁₋₃ alkyl optionally substituted with 1-3 **R^{h}**;
**(b)** C(O)N(C₁₋₃ alkyl)**R^{b1}** or -C(O)N(H)**R^{b1}**, wherein: **R^{b1}** is C₃₋₆ cycloalkyl or 4-6 membered heterocyclyl, each of which is optionally substituted with 1-3 **R^{g}**; and
**(c)** C(O)**R^{b1}**, wherein **R^{b1}** is 4-10 membered heterocyclyl optionally substituted with 1-3 **R^{g}**, wherein **R^{b1}** is attached to the C(O) via a ring nitrogen atom.

**Embodiment 91.** The compound of Embodiment 1, wherein the compound is a compound of Formula (**II-3**): 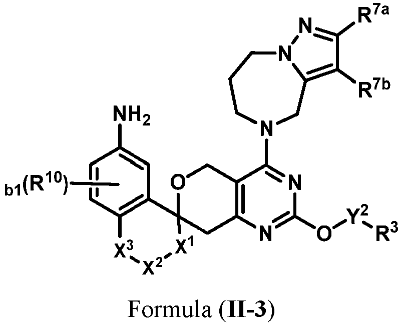 or a pharmaceutically acceptable salt thereof, wherein:
**b1** is 1 or 2;
each **R¹⁰** is independently selected from the group consisting of: -Cl, -F, -CN, and C₁₋₃ alkyl optionally substituted with 1-3 **R^{c}**;
**R^{7a}** is selected from the group consisting of:
   **(a)** C(=O)N(**R^{f}**)₂, wherein each **R^{f}** is independently H or C₁₋₃ alkyl optionally substituted with 1-3 **R^{h}**;
   **(b)** C(O)N(C₁₋₃ alkyl)**R^{b1}** or -C(O)N(H)**R^{b1}**, wherein: **R^{b1}** is C₃₋₆ cycloalkyl or 4-6 membered heterocyclyl, each of which is optionally substituted with 1-3 **R^{g}**; and
   **(c)** C(O)**R^{b1}**, wherein **R^{b1}** is 4-10 membered heterocyclyl optionally substituted with 1-3 **R^{g}**, wherein **R^{b1}** is attached to the C(O) via a ring nitrogen atom;
**R^{7b}** is -halo or C₁₋₃ alkyl;
**X¹** is CH₂; and
**X²** and **X³** are independently selected from the group consisting of: O, CH₂, CH**R^{L}**, and C(**R^{L}**)₂.

**Embodiment 92.** The compound of Embodiment 1, wherein the compound is a compound of Formula (**II-a3**): 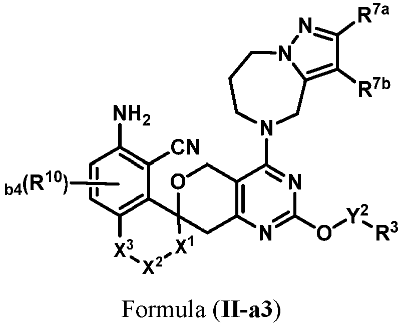 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c}**;
**R^{7a}** is selected from the group consisting of:
   **(a)** C(=O)N(**R^{f}**)₂, wherein each **R^{f}** is independently H or C₁₋₃ alkyl optionally substituted with 1-3 **R^{h}**;
   **(b)** C(O)N(C₁₋₃ alkyl)**R^{b1}** or -C(O)N(H)**R^{b1}**, wherein: **R^{b1}** is C₃₋₆ cycloalkyl or 4-6 membered heterocyclyl, each of which is optionally substituted with 1-3 **R^{g}**; and
   **(c)** C(O)**R^{b1}**, wherein **R^{b1}** is 4-10 membered heterocyclyl optionally substituted with 1-3 **R^{g}**, wherein **R^{b1}** is attached to the C(O) via a ring nitrogen atom;
**R^{7b}** is -halo or C₁₋₃ alkyl;
**X¹** is CH₂; and
**X²** and **X³** are independently selected from the group consisting of: O, CH₂, CH**R^{L}**, and C(**R^{L}**)₂.

**Embodiment 93.** The compound of Embodiment 92, wherein **b4** is 0.

**Embodiment 94.** The compound of Embodiment 4, wherein the compound is a compound of Formula (**III-3**): 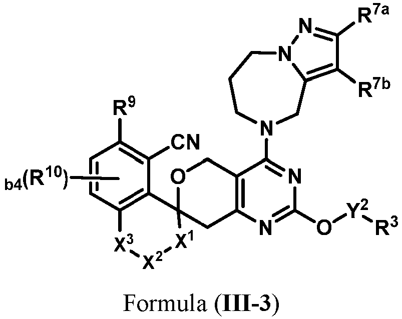 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c}**;
**R^{7a}** is selected from the group consisting of:
   **(a)** C(=O)N(**R^{f}**)₂, wherein each **R^{f}** is independently H or C₁₋₃ alkyl optionally substituted with 1-3 **R^{h}**;
   **(b)** C(O)N(C₁₋₃ alkyl)**R^{b1}** or -C(O)N(H)**R^{b1}**, wherein: **R^{b1}** is C₃₋₆ cycloalkyl or 4-6 membered heterocyclyl, each of which is optionally substituted with 1-3 **R^{g}**; and
   **(c)** C(O)**R^{b1}**, wherein **R^{b1}** is 4-10 membered heterocyclyl optionally substituted with 1-3 **R^{g}**, wherein **R^{b1}** is attached to the C(O) via a ring nitrogen atom;
**R^{7b}** is -halo or C₁₋₃ alkyl;
**X¹** is CH₂; and
**X²** and **X³** are independently selected from the group consisting of: O, CH₂, CH**R^{L}**, and C(**R^{L}**)₂.

**Embodiment 95.** The compound of Embodiment 94, wherein **b4** is 0.

**Embodiment 96.** The compound of Embodiment 94 or 95, wherein **R⁹** is N**R^{d}R^{e}** (e.g., -NH₂).

**Embodiment 97.** The compound of Embodiment 15, wherein the compound is a compound of Formula **(IV-a3)** or **(IV-b3):** 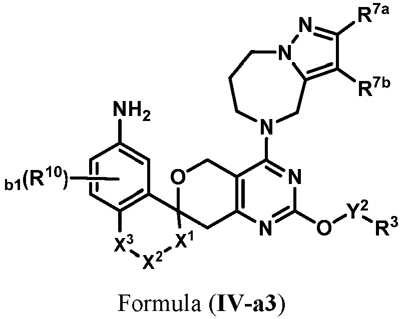 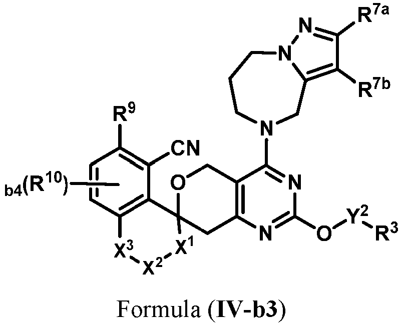 or a pharmaceutically acceptable salt thereof, wherein:
**b1** is 0, 1, or 2;
**b4** is 0 or 1;
each **R¹⁰** is independently selected from the group consisting of: -Cl, -F, -CN, and C₁₋₃ alkyl optionally substituted with 1-3 **R^{c}**;
**R^{7a}** is selected from the group consisting of:
   **(a)** C(=O)N(**R^{f}**)₂, wherein each **R^{f}** is independently H or C₁₋₃ alkyl optionally substituted with 1-3 **R^{h}**;
   **(b)** C(O)N(C₁₋₃ alkyl)**R^{b1}** or -C(O)N(H)**R^{b1}**, wherein: **R^{b1}** is C₃₋₆ cycloalkyl or 4-6 membered heterocyclyl, each of which is optionally substituted with 1-3 **R^{g}**; and
   **(c)** C(O)**R^{b1}**, wherein **R^{b1}** is 4-10 membered heterocyclyl optionally substituted with 1-3 **R^{g}**, wherein **R^{b1}** is attached to the C(O) via a ring nitrogen atom;
**R^{7b}** is -halo or C₁₋₃ alkyl;
**X¹** is CH₂;
one of **X²** and **X³** is independently selected from the group consisting of: CH**R^{L}** and C(**R^{L}**)₂; and
the other of **X²** and **X³** is CH₂ or O.

**Embodiment 98.** The compound of Embodiment 97, wherein the compound is a compound of Formula **(IV-b3),** or a pharmaceutically acceptable salt thereof, wherein **b4** is 0.

**Embodiment 99.** The compound of Embodiment 97, wherein the compound is a compound of Formula **(IV-a3),** or a pharmaceutically acceptable salt thereof, wherein **b1** is 1; and the 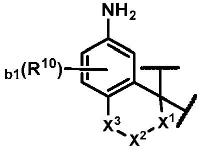 moiety is

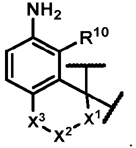

**Embodiment 100.** The compound of any one of Embodiments 97-99, wherein **X²** is CH₂; and **X³** is CH**R^{L}**.

**Embodiment 101.** The compound of Embodiment 100, wherein **R^{L}** is CF₃ or CH₃ (e.g., CH₃).

**Embodiment 102.** The compound of any one of Embodiments 91-101, wherein **R^{7a}** is C(=O)N(**R^{f}**)₂, wherein each **R^{f}** is independently C₁₋₃ alkyl optionally substituted with 1-3 **R^{h}** (e.g., **R^{7a}** is C(=O)N(Me)₂).

**Embodiment 103.** The compound of any one of Embodiments 91-102, wherein **R^{7b}** is -Cl, -F, or methyl.

**Embodiment 104.** The compound of any one of Embodiments 91-103, wherein **R^{7b}** is -Cl.

**Embodiment 105.** The compound of any one of Embodiments 91-104, wherein the 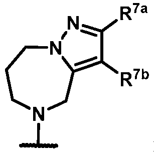 moiety is

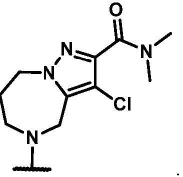

**Embodiment 106.** The compound of Embodiment 1, wherein the compound is a compound of Formula (**II-4**), (**II-5**), or **(II-8):** 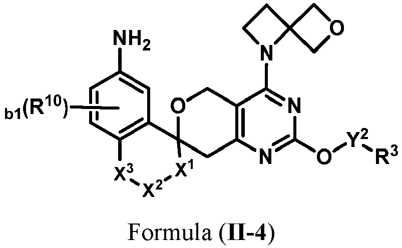 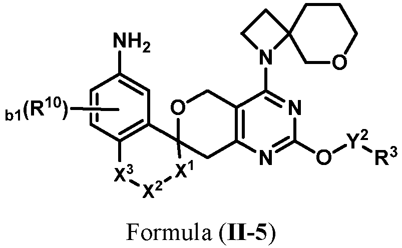 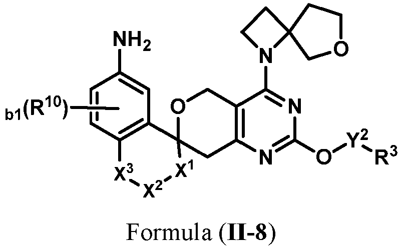 or a pharmaceutically acceptable salt thereof, wherein:
**b1** is 1 or 2;
each **R¹⁰** is independently selected from the group consisting of: -Cl, -F, -CN, and C₁₋₃ alkyl optionally substituted with 1-3 **R^{c}**;
**X¹** is CH₂; and
**X²** and **X³** are independently selected from the group consisting of: O, CH₂, CH**R^{L}**, and C(**R^{L}**)₂.

**Embodiment 107.** The compound of Embodiment 1, wherein the compound is a compound of Formula **(II-a4), (II-a5),** or **(II-a8):** 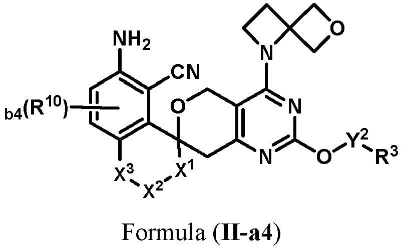 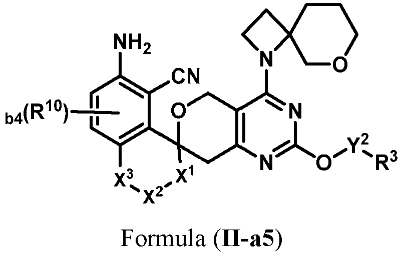 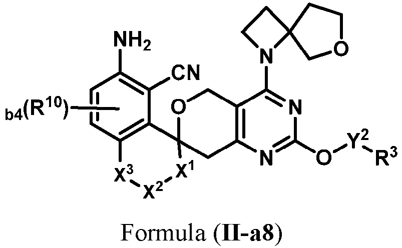 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c}**;
**X¹** is CH₂; and
**X²** and **X³** are independently selected from the group consisting of: O, CH₂, CH**R^{L}**, and C(**R^{L}**)₂.

**Embodiment 108.** The compound of Embodiment 107, wherein **b4** is 0.

**Embodiment 109.** The compound of Embodiment 4, wherein the compound is a compound of Formula (**III-4**), (**III-5**), or (**III-8**): 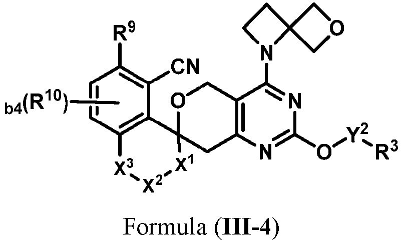 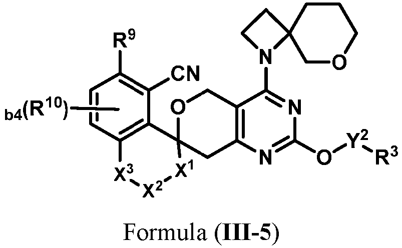 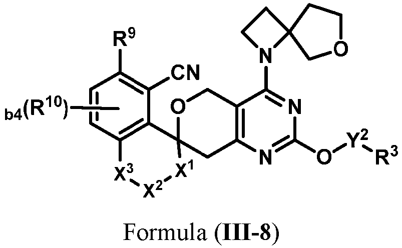 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c}**;
**X¹** is CH₂; and
**X²** and **X³** are independently selected from the group consisting of: O, CH₂, CH**R^{L}**, and C(**R^{L}**)₂.

**Embodiment 110.** The compound of Embodiment 109, wherein **b4** is 0.

**Embodiment 111.** The compound of Embodiment 109 or 110, wherein **R⁹** is N**R^{d}R^{e}** (e.g., -NH₂).

**Embodiment 112.** The compound of Embodiment 15, wherein the compound is a compound of Formula **(IV-a4), (IV-b4), (IV-a5), (IV-b5), (IV-a8),** or **(IV-b8):** 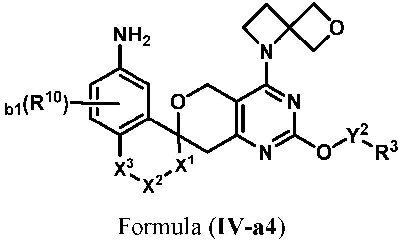 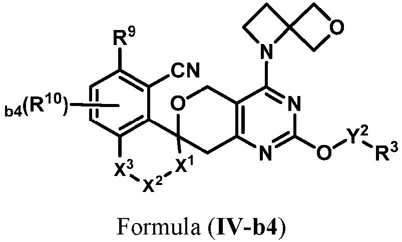 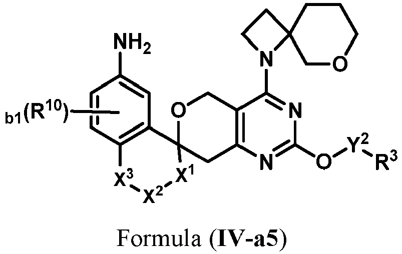 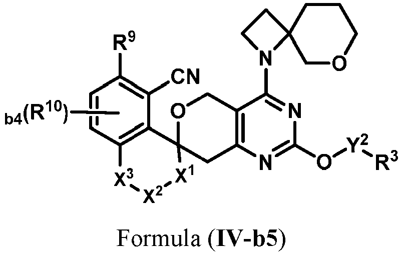 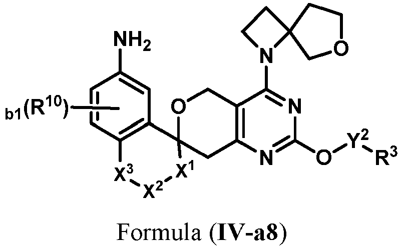 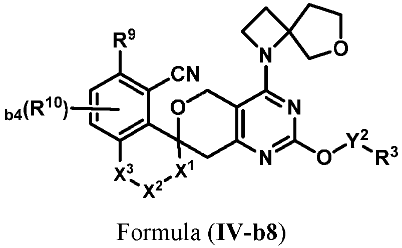 or a pharmaceutically acceptable salt thereof, wherein:
**b1** is 0, 1, or 2;
**b4** is 0 or 1;
each **R¹⁰** is independently selected from the group consisting of: -Cl, -F, -CN, and C₁₋₃ alkyl optionally substituted with 1-3 **R^{c}**;
**X¹** is CHz;
one of **X²** and **X³** is independently selected from the group consisting of: CH**R^{L}** and C(**R^{L}**)₂; and
the other of **X²** and **X³** is CH₂ or O.

**Embodiment 113.** The compound of Embodiment 112, wherein the compound is a compound of Formula **(IV-a4)** or **(IV-a5),** or a pharmaceutically acceptable salt thereof, wherein **b1** is 1; and the 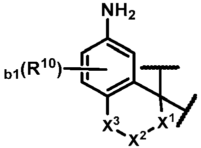 moiety is 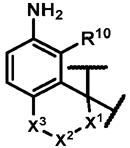 (e.g., 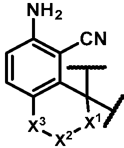 ).

**Embodiment 114.** The compound of Embodiment 112, wherein the compound is a compound of Formula **(IV-b4)** or **(IV-b5),** or a pharmaceutically acceptable salt thereof, wherein **b4** is 0.

**Embodiment 115.** The compound of Embodiment 1, wherein the compound is a compound of Formula (**II-6**) or (**II-7**): 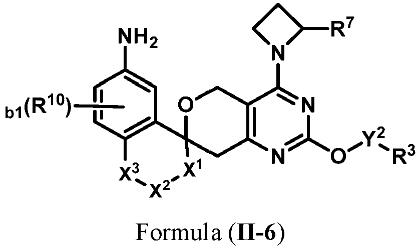 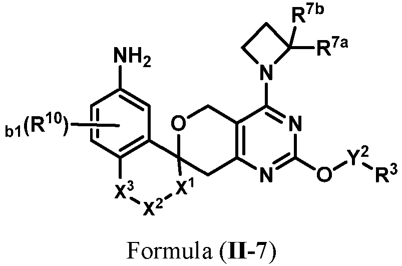 or a pharmaceutically acceptable salt thereof, wherein:
**R⁷** is C₁₋₃ alkyl substituted with -OH or C₁₋₃ alkoxy;
**R^{7a}** is C₁₋₃ alkyl substituted with -OH (e.g., -CH₂OH);
**R^{7b}** is selected from the group consisting of:
   C₁₋₃ alkyl optionally substituted with 1-3 F (e.g., methyl), and
   C₁₋₃ alkyl substituted with -OH or C₁₋₃ alkoxy;
   **b1** is 1 or 2;
   each **R¹⁰** is independently selected from the group consisting of: -Cl, -F, -CN, and C₁₋₃ alkyl optionally substituted with 1-3 **R^{c}**;
   **X¹** is CH₂; and
   **X²** and **X³** are independently selected from the group consisting of: O, CH₂, CH**R^{L}**, and C(**R^{L}**)₂.

**Embodiment 116.** The compound of Embodiment 1, wherein the compound is a compound of Formula **(II-a6)** or **(II-a7):** 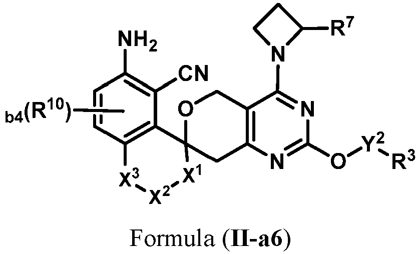 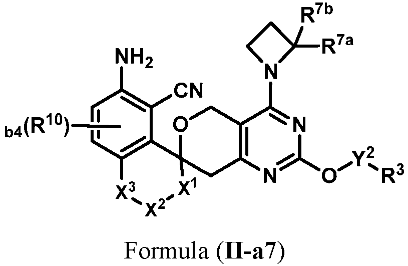 or a pharmaceutically acceptable salt thereof, wherein:
**R⁷** is C₁₋₃ alkyl substituted with -OH or C₁₋₃ alkoxy;
**R^{7a}** is C₁₋₃ alkyl substituted with -OH (e.g., -CH₂OH);
**R^{7b}** is selected from the group consisting of:
   C₁₋₃ alkyl optionally substituted with 1-3 F (e.g., methyl), and
   C₁₋₃ alkyl substituted with -OH or C₁₋₃ alkoxy;
   **b4** is 0 or 1;
   each **R¹⁰** is independently selected from the group consisting of: -Cl, -F, -CN, and C₁₋₃ alkyl optionally substituted with 1-3 **R^{c}**;
   **X¹** is CH₂; and
   **X²** and **X³** are independently selected from the group consisting of: O, CH₂, CH**R^{L}**, and C(**R^{L}**)₂.

**Embodiment 117.** The compound of Embodiment 117, wherein **b4** is 0.

**Embodiment 118.** The compound of Embodiment 4, wherein the compound is a compound of Formula (**III-6**) or (**III-7**): 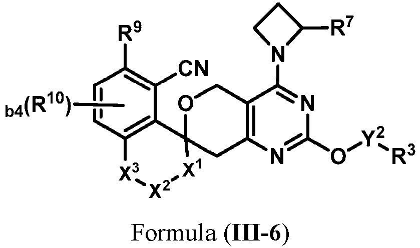 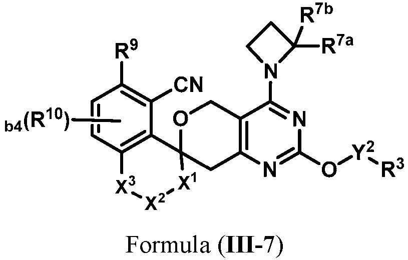 or a pharmaceutically acceptable salt thereof, wherein:
**R⁷** is C₁₋₃ alkyl substituted with -OH or C₁₋₃ alkoxy;
**R^{7a}** is C₁₋₃ alkyl substituted with -OH (e.g., -CH₂OH);
**R^{7b}** is selected from the group consisting of:
   C₁₋₃ alkyl optionally substituted with 1-3 F (e.g., methyl), and
   C₁₋₃ alkyl substituted with -OH or C₁₋₃ alkoxy;
   **b4** is 0 or 1;
   each **R¹⁰** is independently selected from the group consisting of: -Cl, -F, -CN, and C₁₋₃ alkyl optionally substituted with 1-3 **R^{c}**;
   **X¹** is CH₂; and
   **X²** and **X³** are independently selected from the group consisting of: O, CH₂, CH**R^{L}**, and C(**R^{L}**)₂.

**Embodiment 119.** The compound of Embodiment 118, wherein **b4** is 0.

**Embodiment 120.** The compound of Embodiment 118 or 119, wherein **R⁹** is N**R^{d}R^{c}** (e.g., -NH₂).

**Embodiment 121.** The compound of Embodiment 15, wherein the compound is a compound of Formula (IV-a6), **(IV-b6), (IV-a7),** or **(IV-b7):** 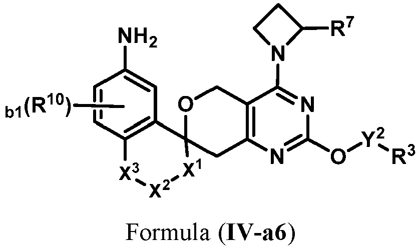 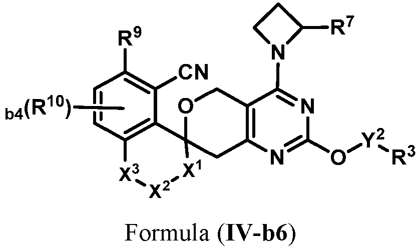 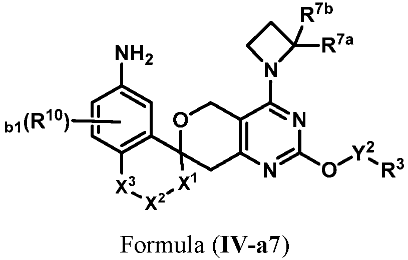 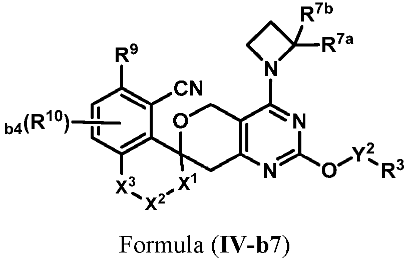 or a pharmaceutically acceptable salt thereof, wherein:
**R⁷** is C₁₋₃ alkyl substituted with -OH or C₁₋₃ alkoxy;
**R^{7a}** is C₁₋₃ alkyl substituted with -OH (e.g., -CH₂OH);
**R^{7b}** is selected from the group consisting of:
   C₁₋₃ alkyl optionally substituted with 1-3 F (e.g., methyl), and
   C₁₋₃ alkyl substituted with -OH or C₁₋₃ alkoxy;
   **b1** is 0, 1, or 2;
   **b4** is 0 or 1;
   each **R¹⁰** is independently selected from the group consisting of: -Cl, -F, -CN, and C₁₋₃ alkyl optionally substituted with 1-3 **R^{c}**;
   **X¹** is CHz;
   one of **X²** and **X³** is independently selected from the group consisting of: CH**R^{L}** and C(**R^{L}**)₂; and
   the other of **X²** and **X³** is CH₂ or O.

**Embodiment 122.** The compound of Embodiment 121, wherein the compound is a compound of Formula **(IV-a6)** or **(IV-a7),** or a pharmaceutically acceptable salt thereof, wherein **b1** is 1; and the 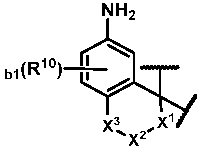 moiety is 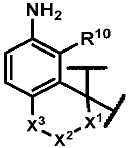 (e.g., 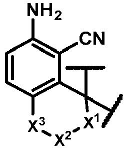 ).

**Embodiment 123.** The compound of Embodiment 121, wherein the compound is a compound of Formula **(IV-b6)** or **(IV-b7),** or a pharmaceutically acceptable salt thereof, wherein **b4** is 0.

**Embodiment 124.** The compound of any one of Embodiments 115-123, wherein the 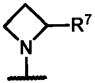 moiety is

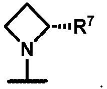

**Embodiment 125.** The compound of any one of Embodiments 115-124, wherein **R⁷** is C₁₋₃ alkyl substituted with -OH.

**Embodiment 126.** The compound of any one of Embodiments 115-125, wherein **R⁷** is -CH₂OH.

**Embodiment 127.** The compound of any one of Embodiments 115-123, wherein the 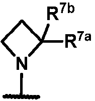 moiety is

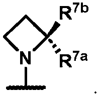

**Embodiment 128.** The compound of any one of Embodiments 115-123 or 127, wherein **R^{7a}** is -CH₂OH.

**Embodiment 129.** The compound of any one of Embodiments 115-123 or 128-129, wherein **R^{7b}** is C₁₋₃ alkyl optionally substituted with 1-3 -F.

**Embodiment 130.** The compound of Embodiment 129, wherein **R^{7b}** is methyl.

**Embodiment 131.** The compound of any one of Embodiments 106-130, wherein **X²** is CH₂; and **X³** is CH**R^{L}** (e.g., CH(CH₃)).

**Embodiment 132.** The compound of any one of Embodiments 106-111 or 115-120, wherein **X²** is CH₂; and **X³** is CH₂.

**Embodiment 133.** The compound of any one of Embodiments 73-132, wherein **Y²** is -CH₂-; and **R³** is a 4-10 membered heterocyclyl having one ring nitrogen atom and 0-1 additional ring heteroatom selected from the group consisting of oxygen and nitrogen, wherein the heterocyclyl is optionally substituted with 1-6 **R^{a}**.

**Embodiment 134.** The compound of any one of Embodiments 73-133, wherein **Y²** is -CH₂-; and **R³** is 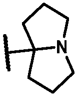 optionally substituted with 1-2 substituents each independently selected from the group consisting of: -F, -C₁₋₃ alkoxy, and -C₁₋₃ haloalkoxy.

**Embodiment 135.** The compound of any one of Embodiments 73-134, wherein **Y²** is -CH₂-; and **R³** is 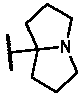 optionally substituted with 1-2 -F (e.g., **R³** is 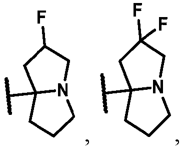 or 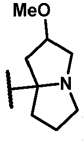 ).

**Embodiment 136.** The compound of any one of Embodiments 133-135, wherein **R³** is 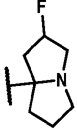 (e.g., 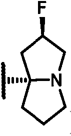 ).

**Embodiment 137.** The compound of any one of Embodiments 1-136, wherein the 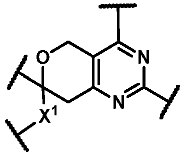 moiety is

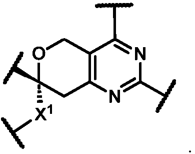

**Embodiment 138.** The compound of Embodiment 1, wherein the compound of Formula (**II**) is selected from the group consisting of Compound Nos. **181b, 501, 501a, 501b, 501c, 502, 502a, 502b, 503, 503a, 503b, 503c, 504, 504a, 504b, 504c, 505, 505a, 506, 506a, 507, 507a, 507b, 507c, 508, 508a, 509, 509a, 509b, 510, 510a, 510b, 510c, 510d, 510e, 510f, 511, 511a, 512, 512a, 512b, 513, 513a, 514, 514a, 514b, 516, 516a, 516b, 516c, 517, 517a, 518, 518a, 519, 519a, 520, 520a, 522, 522a, 523, 523a, 524, 524a, 525, 525a, 526, 526a, 526b, 526c, 527, 527a, 527b, 528, 528a, 529, 529a, 530, 530a, 531, 531a, 532, 532a, 534, 534a, 535, 535a, 536, 536a, 536b, 536c, 537, 537a, 538, 538a, 539, 539a, 540, 540a, 541, 541a, 542, 542a, 543, 543a, 544, 544a, 545, 545a, 546, 546a, 547, 547a, 548, 548a, 549, 549a, 550, 550a, 551, 551a, 552, 552a, 553, 553a, 554, 554a, 554b, 554c, 555, 555a, 556, 556a, 556b, 558, 558a, 559, 559a, 560, 560a, 561, 561a, 562, 562a, 563, 563a, 564, 564a, 565, 565a, 566, 566a, 566b, 567, 567a, 567b, 568, 568a, 568b, 569, 569a, 570, 570a, 571, 571a, 572, 572a, 573, 573a, 575, 575a, 575b, 576, 576a, 576b, 577, 577a, 578, 578a, 578b, 579, 579a, 580, 580a, 581, 581a, 582, 582a, 583, 583a, 584, 584a, 585, 585a, 585b, 586, 586a, 587, 587a, 588, 588a, 589, 589a, 590, 590a, 591, 591a, 592, 592a, 593 , 593a, 594, 594a, 595, 595a, 595b, 596, 596a, 597, 597a, 597b, 598, 598a, 598b, 598c, 599, 599a, 599b, 600, 600a, 600b, 601, 601a, 602, 602a, 603, 603a, 603b, 606, 606a, 606b, 607, 607a, 608, 608a, 608b, 609, 609a, 609b, 610, 610a, 611, 611a, 611b, 612, 612a, 612b, 613, 613a, 614, 614a, 615, 615a, 616, 616a, 617, 617a, 618, 618a, 619, 619a, 620, 620a, 621, 621a, 621b, 622, 622a, 623, 623a, 624, 624a, 624b, 625, 625a, 626, 626a, 627, 627a, 628, 628a, 629, 629a, 630, 630a, 631, 631a, 632, 632a, 633, 633a, 634, 634a, 635, 635a, 636, 636a, 637, 637a, 637b, 638, 638a, 639, 639a, 640, 640a, 641, 641a, 642, 642a, 643, 643a, 643b, 644, 644a, 645, 645a, 646, 646a, 647, 647a, 647b, 648, 648a, 648b, 649, 649a, 649b, 650, 650a, 651, 651a, 651b, 652, 652a, 653, 653a, 654, 654a, 654b, 654c, 654d, 655, 655a, 656,** and **656a** as depicted in **Table C3,** or a pharmaceutically acceptable salt thereof.

**Embodiment 139.** The compound of Embodiment 4, wherein the compound of Formula (**III**) is selected from the group consisting of Compound Nos. **502, 502a, 502b, 503, 503a, 503b, 503c, 504, 504a, 504b, 504c, 505, 505a, 507, 507a, 507b, 507c, 509, 509a, 509b, 511, 511a, 512, 512a, 512b, 513, 513a, 514, 514a, 514b, 516, 516a, 516b, 516c, 517, 517a, 518, 518a, 519, 519a, 520, 520a, 522, 522a, 523, 523a, 524, 524a, 525, 525a, 526, 526a, 526b, 526c, 527, 527a, 527b, 528, 528a, 529, 529a, 530, 530a, 531, 531a, 532, 532a, 534, 534a, 535, 535a, 536, 536a, 536b, 536c, 537, 537a, 538, 538a, 539, 539a, 540, 540a, 541, 541a, 542, 542a, 543, 543a, 544, 544a, 545, 545a, 546, 546a, 547, 547a, 548, 548a, 549, 549a, 550, 550a, 551, 551a, 552, 552a, 553, 553a, 554, 554a, 554b, 554c, 555, 555a, 556, 556a, 556b, 558, 558a, 559, 559a, 560, 560a, 561, 561a, 562, 562a, 563, 563a, 564, 564a, 565, 565a, 566, 566a, 566b, 567, 567a, 567b, 568, 568a, 568b, 569, 569a, 570, 570a, 571, 571a, 572, 572a, 573, 573a, 574, 574a, 574b, 574c, 575, 575a, 575b, 576, 576a, 576b, 577, 577a, 578, 578a, 578b, 579, 579a, 580, 580a, 581, 581a, 582, 582a, 583, 583a, 584, 584a, 585, 585a, 585b, 586, 586a, 587, 587a, 588, 588a, 589, 589a, 590, 590a, 591, 591a, 592, 592a, 593 , 593a, 594, 594a, 595, 595a, 595b, 596, 596a, 597, 597a, 597b, 598, 598a, 598b, 598c, 599, 599a, 599b, 600, 600a, 600b, 601, 601a, 602, 602a, 603, 603a, 603b, 604, 604a, 605, 605a, 605b, 605c, 606, 606a, 606b, 607, 607a, 608, 608a, 608b, 609, 609a, 609b, 610, 610a, 611, 611a, 611b, 612, 612a, 612b, 613, 613a, 614, 614a, 615, 615a, 616, 616a, 617, 617a, 618, 618a, 619, 619a, 620, 620a, 621, 621a, 621b, 622, 622a, 623, 623a, 624, 624a, 624b, 625, 625a, 626, 626a, 627, 627a, 628, 628a, 629, 629a, 630, 630a, 631, 631a, 632, 632a, 633, 633a, 634, 634a, 635, 635a, 636, 636a, 637, 637a, 637b, 638, 638a, 639, 639a, 640, 640a, 641, 641a, 642, 642a, 643, 643a, 643b, 644, 644a, 645, 645a, 646, 646a, 647, 647a, 647b, 648, 648a, 648b, 649, 649a, 649b, 650, 650a, 651, 651a, 651b, 652, 652a, 653, 653a, 654, 654a, 654b, 654c, 654d, 655, 655a, 656,** and **656a** as depicted in **Table C3,** or a pharmaceutically acceptable salt thereof.

**Embodiment 140.** The compound of Embodiment 15, wherein the compound of Formula **(IV)** is selected from the group consisting of Compound Nos. **502, 502a, 502b, 508, 508a, 509, 509a, 509b, 511, 511a, 512, 512a, 512b, 530, 530a, 531, 531a, 532, 532a, 546, 546a, 553, 553a, 554, 554a, 554b, 554c, 555, 555a, 556, 556a, 556b, 560, 560a, 561, 561a, 566, 566a, 566b, 567, 567a, 567b, 568, 568a, 568b, 569, 569a, 570, 570a, 571, 571a, 572, 572a, 573, 573a, 575, 575a, 575b, 576, 576a, 576b, 577, 577a, 578, 578a, 578b, 581, 581a, 582, 582a, 583, 583a, 584, 584a, 585, 585a, 585b, 589, 589a, 590, 590a, 592, 592a, 595, 595a, 595b, 596, 596a, 597, 597a, 597b, 598, 598a, 598b, 598c, 599, 599a, 599b, 600, 600a, 600b, 601, 601a, 604, 604a, 605, 605a, 605b, 605c, 606, 606a, 606b, 607, 607a, 608, 608a, 608b, 609, 609a, 609b, 610, 610a, 611, 611a, 611b, 612, 612a, 612b, 613, 613a, 614, 614a, 615, 615a, 616, 616a, 617, 617a, 618, 618a, 619, 619a, 620, 620a, 621, 621a, 621b, 622, 622a, 623, 623a, 624, 624a, 624b, 625, 625a, 626, 626a, 627, 627a, 628, 628a, 629, 629a, 630, 630a, 632, 632a, 633, 633a, 634, 634a, 635, 635a, 636, 636a, 637, 637a, 637b, 638, 638a, 639, 639a, 640, 640a, 641, 641a, 642, 642a, 643, 643a, 643b, 644, 644a, 645, 645a, 646, 646a, 647, 647a, 647b, 648, 648a, 648b, 649, 649a, 649b, 650, 650a, 651, 651a, 651b, 652, 652a, 653, 653a, 654, 654a, 654b, 654c, 654d, 655, 655a, 656,** and **656a** as depicted in **Table C3,** or a pharmaceutically acceptable salt thereof.

**Embodiment 141.** The compound of Embodiment 1, wherein the compound of Formula (**II**) is other than Compound Nos. **R139, R139a, R139b, R139c, R158, R158a**, **R158b, R158c, R160, R160a, R161, R161a, R161b, R161c, R164, R164a, R164b, R170, R170a, R171, R171a, R176, R176a, R176b, R176c, R176d, R178, R178a, R178b, R179, R179a, R179b, R179d, R179e, R179f, R180, R180a, R181,** and **R181a** as depicted in **Table C1,** or a pharmaceutically acceptable salt thereof.

**Embodiment 142.** The compound of Embodiment 4, wherein the compound of Formula **(III)** is other than Compound Nos. **R158, R158a, R158b, R158c, R161, R161a, R161b, R161c, R176, R176a, R176b, R176c, R176d, R177, R177a, R178, R178a, R178b, R179, R179a, R179b, R179d, R179e, R179f, R180,** and **R180a** as depicted in **Table C1,** or a pharmaceutically acceptable salt thereof.

**Embodiment 143.** The compound of Embodiment 15, wherein the compound of Formula **(IV)** is other than Compound Nos. **R124, R124a, R124b, R124c, R124d, R124e, R124f, R125, R125a, R130, R130a, R131, R131a, R133, R133a, R133b, R134, R134a, R138, R138a, R148, R148a, R162, R162a, R163, R163a, R175,** and **R175a** as depicted in **Table C1,** or a pharmaceutically acceptable salt thereof.

### P11 EXEMPLARY EMBODIMENTS

**Embodiment 1.** A compound of Formula **(II):** 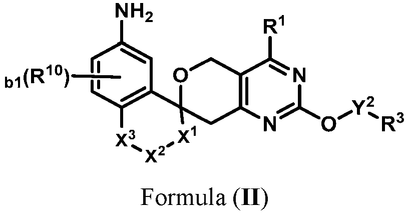 or a pharmaceutically acceptable salt thereof, wherein:
**R¹** is selected from the group consisting of:
   **(i)** a 4-10 membered heterocyclyl optionally substituted with 1-4 **R⁷**;
   **(ii)** an 8-12 membered bicyclic heterocyclyl, wherein the heterocyclyl comprises an endocyclic group selected from the group consisting of C(=O)NH and S(O)₂NH, and wherein the heterocyclyl is further optionally substituted with 1-3 **R⁷** at one or more ring carbon atoms; and
   **(iii)** 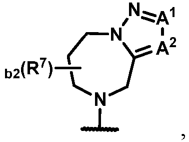 wherein **b2** is 0, 1, 2, or 3; and **A¹** and **A²** are independently selected from the group consisting of: N, CH, and C**R⁷**;
each **R⁷** is independently selected from the group consisting of **R^{a}** and **R^{b}**;
**X¹** is selected from the group consisting of a bond, S(O)₀₋₂, CH₂, CH**R^{L}**, C(**R^{L}**)₂, and O;
**X²** and **X³** are independently selected from the group consisting of: CH₂, CH**R^{L}**, C(**R^{L}**)₂, O, and S(O)₀₋₂, provided that no more than one of **X¹**, **X²**, and **X³** is selected from the group consisting of: O and S(O)₀₋₂;
**b1** is 1 or 2;
each **R¹⁰** is independently selected from the group consisting of **R^{a}** and **R^{b}**;
each **R^{L}** is independently selected from the group consisting of C₁₋₃ alkoxy, -F, CN, and C₁₋₃ alkyl optionally substituted with 1-3 **R^{c}**; or
one pair of **R^{L}** on the same or different ring carbon atom(s) taken together with the ring atom(s) connecting them form a C₃₋₆ cycloalkyl ring;
**Y²** is a bond or a straight-chain C₁₋₆ alkylene optionally substituted with 1-6 **R^{Y}**;
each **R^{Y}** is independently selected from the group consisting of: halo, cyano, -OH, oxo, C₁₋₆ alkoxy, C₁₋₆ haloalkoxy, C₁₋₆ alkyl, and C₁₋₆ haloalkyl, or
one pair of **R^{Y}** on the same or different carbon atom(s) taken together with the atom(s) connecting them forms a C₃₋₆ cycloalkyl ring or 4-6 membered heterocyclyl ring, each of which is optionally substituted with 1-3 independently selected C₁₋₃ alkyl;
**R³** is selected from the group consisting of:
   **(a)** 4-15 membered heterocyclyl optionally substituted with 1-6 substituents independently selected from the group consisting of: **R^{a}** and **R^{b}**; and
   **(b)** -N**R^{d}R^{e}**;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   **(f)** -C₁₋₆ haloalkoxy;
   **(g)** -N**R^{d}R^{e}**;
   **(h)** C(=O)C₁₋₆ alkyl;
   **(i)** C(=O)C₁₋₆ haloalkyl;
   **(j)** C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   **(o)** S(O)₀₋₂(C₁₋₆ haloalkyl);
   **(p)** S(O)₁₋₂N(**R^{f}**)₂; and
   **(q)** C₁₋₆ alkyl, C₂₋₆ alkenyl, or C₂₋₆ alkynyl, each optionally substituted with 1-6 **R^{c}**;
each **R^{b}** is independently selected from the group consisting of: **-(L^{b})_{b}-R^{b2}** and -**R^{b1}**, wherein:
   **b** is 1, 2, or 3;
   each **-L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e}**, C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)₁₋₂N(**R^{f}**)₂;
   each **R^{d}** and **R^{c}** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂₋.

**Embodiment 2.** The compound of Embodiment 1, wherein the compound of Formula (**II**) is a compound of Formula (II-a): 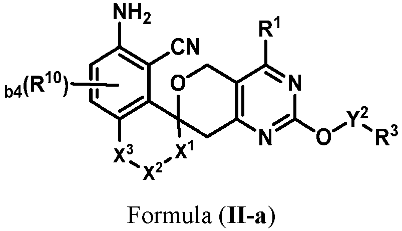 or a pharmaceutically acceptable salt thereof, wherein:
**b4** is 0 or 1; and
each **R¹⁰** is independently selected from the group consisting of: -Cl, -F, -CN, and C₁₋₃ alkyl optionally substituted with 1-3 **R^{c}.**

**Embodiment 3.** The compound of Embodiment 2, wherein **b4** is 0.

**Embodiment 4.** A compound of Formula **(III):** 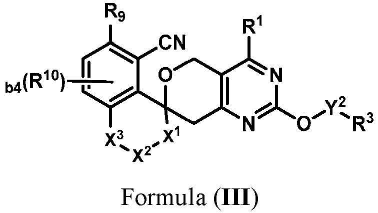 or a pharmaceutically acceptable salt thereof, wherein:
**R¹** is selected from the group consisting of:
   **(i)** a 4-10 membered heterocyclyl optionally substituted with 1-4 **R⁷**;
   **(ii)** an 8-12 membered bicyclic heterocyclyl, wherein the heterocyclyl comprises an endocyclic group selected from the group consisting of C(=O)NH and S(O)₂NH, and wherein the heterocyclyl is further optionally substituted with 1-3 **R⁷** at one or more ring carbon atoms; and
   **(iii)** 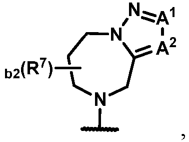 wherein **b2** is 0, 1, 2, or 3; and **A¹** and **A²** are independently selected from the group consisting of: N, CH, and C**R⁷**;
each **R⁷** is independently selected from the group consisting of **R^{a}** and **R^{b}**;
**X¹** is selected from the group consisting of a bond, S(O)₀₋₂, CH₂, CH**R^{L}**, C(**R^{L}**)₂, and O;
**X²** and **X³** are independently selected from the group consisting of: CH₂, CH**R^{L}**, C(**R^{L}**)₂, O, and S(O)₀₋₂, provided that no more than one of **X¹**, **X²**, and **X³** is selected from the group consisting of: O and S(O)₀₋₂;
**R⁹** is selected from the group consisting of: H, N**R^{d}R^{e}**, -OH, and halo;
**b4 is 0 or** 1;
each **R¹⁰** is independently selected from the group consisting of **R^{a}** and **R^{b}**;
each **R^{L}** is independently selected from the group consisting of C₁₋₃ alkoxy, -F, CN, and C₁₋₃ alkyl optionally substituted with 1-3 **R^{c}**; or
one pair of **R^{L}** on the same or different ring carbon atom(s) taken together with the ring atom(s) connecting them form a C₃₋₆ cycloalkyl ring;
**Y²** is a bond or a straight-chain C₁₋₆ alkylene optionally substituted with 1-6 **R^{Y}**;
each **R^{Y}** is independently selected from the group consisting of: halo, cyano, -OH, oxo, C₁₋₆ alkoxy, C₁₋₆ haloalkoxy, C₁₋₆ alkyl, and C₁₋₆ haloalkyl, or
one pair of **R^{Y}** on the same or different carbon atom(s) taken together with the atom(s) connecting them forms a C₃₋₆ cycloalkyl ring or 4-6 membered heterocyclyl ring, each of which is optionally substituted with 1-3 independently selected C₁₋₃ alkyl;
**R³** is selected from the group consisting of:
   **(a)** 4-15 membered heterocyclyl optionally substituted with 1-6 substituents independently selected from the group consisting of: **R^{a}** and **R^{b}**; and
   **(b)** -N**R^{d}R^{e}**;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   **(f)** -C₁₋₆ haloalkoxy;
   **(g)** -N**R^{d}R^{e}**;
   **(h)** C(=O)C₁₋₆ alkyl;
   **(i)** C(=O)C₁₋₆ haloalkyl;
   **(j)** C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   **(o)** S(O)₀₋₂(C₁₋₆ haloalkyl);
   **(p)** S(O)₁₋₂N(**R^{f}**)₂; and
   **(q)** C₁₋₆ alkyl, C₂₋₆ alkenyl, or C₂₋₆ alkynyl, each optionally substituted with 1-6 **R^{c}**;
each **R^{b}** is independently selected from the group consisting of: -**(L^{b})_{b}-R^{b1}** and -**R^{b1}**, wherein:
   **b** is 1, 2, or 3;
   each **-L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e}**, C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)₁₋₂N(**R^{f}**)₂;
   each **R^{d}** and **R^{c}** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂-.

**Embodiment 5.** The compound of Embodiment 4, wherein **b4** is 0.

**Embodiment 6.** The compound of Embodiment 4 or 5, wherein **R⁹** is -NH₂; and each **R¹⁰** is independently selected from the group consisting of: -Cl, -F, -CN, and C₁₋₃ alkyl optionally substituted with 1-3 **R^{c}.**

**Embodiment 7.** The compound of any one of Embodiments 1-6, wherein **X¹** is CH₂ or CH**R^{L}**.

**Embodiment 8.** The compound of any one of Embodiments 1-7, wherein **X²** and **X³** are independently selected from the group consisting of: CH₂, CH**R^{L}**, and C(**R^{L}**)₂.

**Embodiment 9.** The compound of any one of Embodiments 1-8, wherein **X¹** is CH₂; and **X²** and **X³** are both CH₂.

**Embodiment 10.** The compound of any one of Embodiments 1-8, wherein at least one (e.g., one) of **X¹**, **X²**, and **X³** is selected from the group consisting of: CH**R^{L}** and C(**R^{L}**)₂.

**Embodiment 11.** The compound of any one of Embodiments 1-8 or 10, wherein **X¹** is CH₂; and **X²** and **X³** are independently selected from the group consisting of: CH₂, CH**R^{L}**, and C(**R^{L}**)₂, provided that 1-2 of **X²** and **X³** is independently CH**R^{L}** or C(**R^{L}**)₂.

**Embodiment 12.** The compound of any one of Embodiments 1-8 or 10-11, wherein **X¹** is CH₂; **X²** is CH₂; and **X³** is CH**R^{L}** (e.g., **X³** is C(H)CH₃).

**Embodiment 13.** The compound of any one of Embodiments 1-12, wherein each **R^{L}** is independently selected from the group consisting of: CH₃, CF₃, CHF₂, and CH₂F (e.g., each **R^{L}** is CH₃).

**Embodiment 14.** The compound of Embodiment 1, wherein the 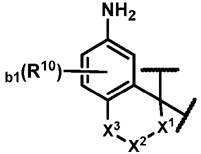 moiety is 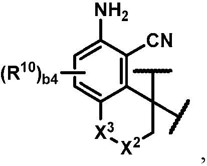 wherein:
**b4** is 0 or 1;
**X²** is -O- or -CH₂-;
**X³** is -CH₂- or -CH**R^{L}**-, wherein **R^{L}** is C₁₋₃ alkyl (e.g., methyl); and
each **R¹⁰** is independently selected from the group consisting of: -Cl, -F, -CN, and C₁₋₃ alkyl optionally substituted with 1-3 **R^{c}**.

**Embodiment 15.** The compound of Embodiment 14, wherein the 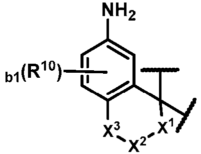 moiety is selected from the group consisting of: 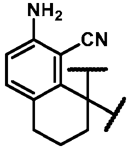 and

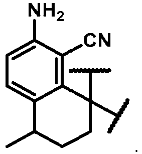

**Embodiment 16.** A compound of Formula (**IV**): 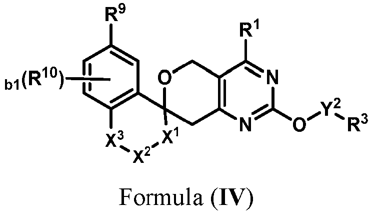 or a pharmaceutically acceptable salt thereof, wherein:
**X¹** is selected from the group consisting of a bond, S(O)₀₋₂, CH₂, CH**R^{L}**, C(**R^{L}**)₂, and O;
**X²** and **X³** are independently selected from the group consisting of: CH₂, CH**R^{L}**, C(**R^{L}**)₂, O, and S(O)₀₋₂, provided that at least one of **X¹**, **X²**, and **X³** is CH**R^{L}** or C(**R^{L}**)₂;
further provided that no more than one of **X¹**, **X²**, and **X³** is selected from the group consisting of: O and S(O)₀₋₂;
**b1** is 0, 1 or 2;
**R⁹** is selected from the group consisting of: H, OH, N**R^{d}R^{e}**, and halo;
each **R¹⁰** is independently selected from the group consisting of **R^{a}** and **R^{b}**;
each **R^{L}** is independently selected from the group consisting of C₁₋₃ alkoxy, -F, CN, and C₁₋₃ alkyl optionally substituted with 1-3 **R^{c}**;
**R¹** is selected from the group consisting of:
   **(i)** a 4-10 membered heterocyclyl optionally substituted with 1-4 **R⁷**;
   **(ii)** an 8-12 membered bicyclic heterocyclyl, wherein the heterocyclyl comprises an endocyclic group selected from the group consisting of C(=O)NH and S(O)₂NH, and wherein the heterocyclyl is further optionally substituted with 1-3 **R⁷** at one or more ring carbon atoms; and
   **(iii)** 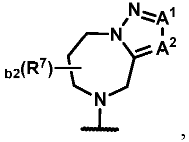 wherein **b2** is 0, 1, 2, or 3; and **A¹** and **A²** are independently selected from the group consisting of: N, CH, and C**R⁷**;
each **R⁷** is independently selected from the group consisting of **R^{a}** and **R^{b}**;
**Y²** is a bond or a straight-chain C₁₋₆ alkylene optionally substituted with 1-6 **R^{Y}**;
each **R^{Y}** is independently selected from the group consisting of: halo, cyano, -OH, oxo, C₁₋₆ alkoxy, C₁₋₆ haloalkoxy, C₁₋₆ alkyl, and C₁₋₆ haloalkyl, or
one pair of **R^{Y}** on the same or different carbon atom(s) taken together with the atom(s) connecting them forms a C₃₋₆ cycloalkyl ring or 4-6 membered heterocyclyl ring, each of which is optionally substituted with 1-3 independently selected C₁₋₃ alkyl;
**R³** is selected from the group consisting of:
   **(a)** 4-15 membered heterocyclyl optionally substituted with 1-6 substituents independently selected from the group consisting of: **R^{a}** and **R^{b}**; and
   **(b)** -N**R^{d}R^{e}**;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   **(f)** -C₁₋₆ haloalkoxy;
   **(g)** -N**R^{d}R^{e}**;
   **(h)** C(=O)C₁₋₆ alkyl;
   **(i)** C(=O)C₁₋₆ haloalkyl;
   **(j)** C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   **(o)** S(O)₀₋₂(C₁₋₆ haloalkyl);
   **(p)** S(O)₁₋₂N(**R^{f}**)₂; and
   **(q)** C₁₋₆ alkyl, C₂₋₆ alkenyl, or C₂₋₆ alkynyl, each optionally substituted with 1-6 **R^{c}**;
each **R^{b}** is independently selected from the group consisting of: -(**L^{b}**)**_{b}**-**R^{b1}** and -**R^{b1}**, wherein:
   **b is** 1, 2, or 3;
   each -**L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e}**, C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)₁₋₂N(**R^{f}**)₂;
   each **R^{d}** and **R^{c}** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋6 alkoxy, -C₁₋₆ haloalkoxy, -NH₂, -N(H)(C₁₋₃ alkyl), and -N(C₁₋₃ alkyl)₂₋.

**Embodiment 17.** The compound of Embodiment 16, wherein the compound of Formula (**IV**) is a compound of Formula (**IV-a**): 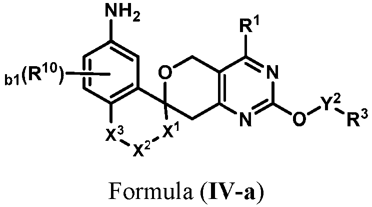 or a pharmaceutically acceptable salt thereof, wherein:
each **R¹⁰** is independently selected from the group consisting of: -Cl, -F, -CN, and C₁₋₃ alkyl optionally substituted with 1-3 **R^{c}**.

**Embodiment 18.** The compound of Embodiment 16, wherein the compound of Formula (**IV**) is a compound of Formula (**IV-b**): 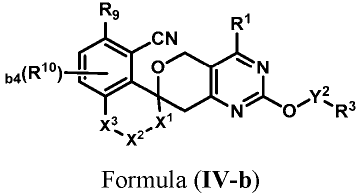 or a pharmaceutically acceptable salt thereof, wherein:
**b4** is 0 or 1; and
each **R¹⁰** is independently selected from the group consisting of: -Cl, -F, -CN, and C₁₋₃ alkyl optionally substituted with 1-3 **R^{c}**.

**Embodiment 19.** The compound of Embodiment 18, wherein **b4** is 0.

**Embodiment 20.** The compound of any one of Embodiments 16-19, wherein **X¹** is CH₂.

**Embodiment 21.** The compound of any one of Embodiments 16-20, wherein **X²** is CH₂; and **X³** is CH**R^{L}**.

**Embodiment 22.** The compound of any one of Embodiments 16-21, wherein each **R^{L}** is independently selected from the group consisting of: CH₃, CF₃, CHF₂, and CH₂F (e.g., each **R^{L}** is CH₃).

**Embodiment 23.** The compound of any one of Embodiments 16-22, wherein one **R**^{L} is CH₃.

**Embodiment 24**. The compound of any one of Embodiments 16-18, wherein the compound is a compound of Formula (IV-c): 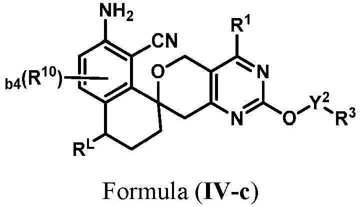 or a pharmaceutically acceptable salt thereof, wherein:
**b4** is 0 or 1; and
each **R¹⁰** is independently selected from the group consisting of: -Cl, -F, -CN, and C₁₋₃ alkyl optionally substituted with 1-3 **R^{c}**.

**Embodiment 25.** The compound of Embodiment 24, wherein **b4** is 0.

**Embodiment 26.** The compound of Embodiment 24 or 25, wherein **R^{L}** is CH₃.

**Embodiment 27.** The compound of any one of Embodiments 1-26, wherein **Y²** is -CH₂-; and **R³** is a 4-10 membered heterocyclyl having one ring nitrogen atom and 0-1 additional ring heteroatom selected from the group consisting of oxygen and nitrogen, wherein the heterocyclyl is optionally substituted with 1-6 **R^{a}**.

**Embodiment 28.** The compound of any one of Embodiments 1-27, wherein **Y²** is -CH₂-; and **R³** is 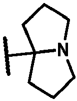 optionally substituted with 1-2 **R^{a}**.

**Embodiment 29.** The compound of any one of Embodiments 1-28, wherein **Y²** is -CH₂-; and **R³** is 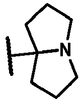 optionally substituted with 1-2 substituents each independently selected from the group consisting of: -F, -C₁₋₃ alkoxy, and -C₁₋₃ haloalkoxy (e.g., **R³** is 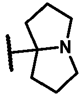 optionally substituted with 1-2 -F).

**Embodiment 30.** The compound of any one of Embodiments 1-29, wherein **R³** is 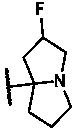 (e.g., 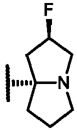 ).

**Embodiment 31.** The compound of any one of Embodiments 1-29, wherein **R³** is

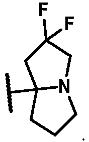

**Embodiment 32.** The compound of any one of Embodiments 1-29, wherein **R³** is

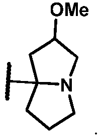

**Embodiment 33.** The compound of any one of Embodiments 1-32, wherein **R¹** is 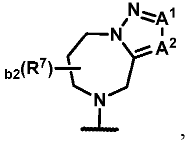 wherein **b2** is 0, 1, or 2, and **A¹** and **A²** are independently selected from the group consisting of: N, CH, and C**R⁷**.

**Embodiment 34.** The compound of any one of Embodiments 1-33, wherein **R¹** is

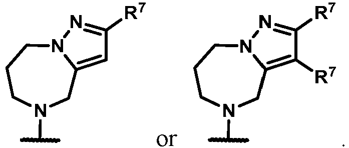

**Embodiment 35.** The compound of any one of Embodiments 1-34, wherein **R¹** is 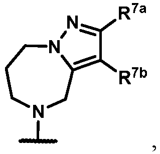 wherein **R^{7a}** and **R^{7b}** are independently selected **R⁷**.

**Embodiment 36.** The compound of Embodiment 35, wherein **R^{7a}** is selected from the group consisting of: C(=O)N(**R^{f}**)₂, C(=O)N(C₁₋₃ alkyl)**R^{b1}**, C(=O)N(H)**R^{b1}**, **R^{b1}**, and C(=O)**R^{b1}**; and
**R^{7b}** is -halo, -CN, and C₁₋₃ alkyl optionally substituted with 1-3 **R^{c}**.

**Embodiment 37.** The compound of Embodiment 35 or 36, wherein **R^{7a}** is selected from the group consisting of:
**(a)** C(=O)N(**R^{f}**)₂, wherein each **R^{f}** is independently H or C₁₋₃ alkyl optionally substituted with 1-3 **R^{h}**;
**(b)** C(=O)N(C₁₋₃ alkyl)**R^{b1}** or C(=O)N(H)**R^{b1}**, wherein **R^{b1}** is C₃₋₆ cycloalkyl or 4-6 membered heterocyclyl, each of which is optionally substituted with 1-3 **R^{g}**; and
**(c)** C(=O)**R^{b1}**, wherein **R^{b1}** is a 4-10 membered heterocyclyl optionally substituted with 1-3 **R^{g}**, wherein **R^{b1}** is attached to the C(=O) via a ring nitrogen atom.

**Embodiment 38.** The compound of any one of Embodiments 35-37, wherein **R^{7a}** is C(=O)N(**R^{f}**)₂, wherein each **R^{f}** is independently H or C₁₋₃ alkyl optionally substituted with 1-3 **R^{h}** (e.g., **R^{7a}** is C(=O)N(Me)₂); and **R^{7b}** is -Cl, -F, or methyl.

**Embodiment 39.** The compound of Embodiment 35 or 36, wherein **R^{7a}** is **R^{b1}**, wherein the **R^{b1}** is a 5-6 membered heteroaryl optionally substituted with 1-2 **R^{g}**, or wherein the **R^{b1}** is oxetanyl optionally substituted with **R^{g}.**

**Embodiment 40.** The compound of any one of Embodiments 1-32, wherein **R¹** is a 4-10 membered heterocyclyl optionally substituted with 1-4 **R⁷**.

**Embodiment 41.** The compound of Embodiment 40, wherein each **R⁷** is independently selected from the group consisting of: halo; cyano; -OH; oxo; -C₁₋₆ alkoxy; C(=O)N(**R^{f}**)₂; **R^{b1}**; -(C₁₋₃ alkylene)-**R^{b1}**; -O-**R^{b1}**; and C₁₋₆ alkyl optionally substituted with 1-3 **R^{c7}**, wherein:
each **R^{b1}** is independently selected from the group consisting of: C₃₋₆ cycloalkyl, phenyl, 4-6 membered heterocyclyl, and 5-6 membered heteroaryl, each of which is optionally substituted with 1-3 **R^{g}** (e.g., each **R^{g}** is independently selected from the group consisting of: halo and C₁₋₃ alkyl); and
each **R^{c7}** is independently selected from the group consisting of: halo, cyano, -OH, and -C₁₋₆ alkoxy.

**Embodiment 42.** The compound of any one of Embodiments 1-32, wherein **R¹** is a 7-10 (e.g., 7) membered heterocyclyl having one ring nitrogen atom, one ring oxygen atom, and no additional ring heteroatoms, wherein the 7-10 membered heterocyclyl is optionally substituted with 1-4 (e.g., 1-2) **R⁷**.

**Embodiment 43.** The compound of any one of Embodiments 1-32 or 42, wherein **R¹** is a 7-10 (e.g., 7) membered monocyclic heterocyclyl having one ring nitrogen atom, one ring oxygen atom, and no additional ring heteroatoms, wherein the 7-10 membered monocyclic heterocyclyl is optionally substituted with 1-4 (e.g., 1-2) **R⁷**.

**Embodiment 44.** The compound of any one of Embodiments 1-32 or 42-43, wherein **R¹** is 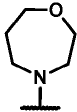 optionally substituted with 1-4 **R⁷** at one or more ring carbon atoms.

**Embodiment 45.** The compound of any one of Embodiments 1-32 or 42-44, wherein **R¹** is 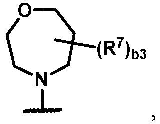 wherein **b3** is 1, 2, or 3.

**Embodiment 46.** The compound of Embodiment 45, wherein one occurrence of **R⁷** is **R^{b}** (e.g., one occurrence of **R⁷** is **R^{b1}**).

**Embodiment 47.** The compound of Embodiment 45 or 46, wherein one occurrence **of R⁷** is a 5-6 membered heteroaryl optionally substituted with 1-3 **R^{g}.**

**Embodiment 48.** The compound of any one of Embodiments 45-47, wherein one occurrence of **R⁷** is a 5-membered heteroaryl optionally substituted with 1-3 **R^{g}.**

**Embodiment 49.** The compound of any one of Embodiments 45-48, wherein one occurrence of **R⁷** is selected from the group consisting of pyrazolyl and oxazolyl, each of which is optionally substituted with 1-2 **R^{g}** (e.g., **R⁷** is 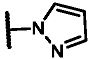 ).

**Embodiment 50.** The compound of any one of Embodiments 45-49, wherein **b3** is 1.

**Embodiment 51.** The compound of any one of Embodiments 1-32 or 44-45, wherein **R¹** is 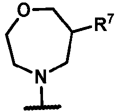 (e.g., 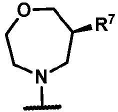 ), wherein **R⁷** is a 5-membered heteroaryl optionally substituted with 1-3 **R^{g}.**

**Embodiment 52.** The compound of Embodiment 51, wherein **R⁷** is selected from the group consisting of pyrazolyl and oxazolyl, each of which is optionally substituted with 1-2 **R^{g}.**

**Embodiment 53.** The compound of Embodiment 51 or 52, wherein **R⁷** is pyrazolyl optionally substituted with 1-2 **R^{g}** (e.g., **R⁷** is 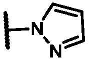 optionally substituted with one **R^{g}**); or wherein **R⁷** is oxazolyl optionally substituted with one **R^{g}** (e.g., **R⁷** is 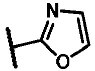 optionally substituted with one **R^{g}**).

**Embodiment 54.** The compound of any one of Embodiments 51-53, wherein **R⁷** is

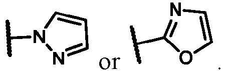

**Embodiment 55.** The compound of any one of Embodiments 1-32 or 42, wherein **R¹** is a 7-10 (e.g., 7) membered bicyclic heterocyclyl having one ring nitrogen atom, one ring oxygen atom, and no additional ring heteroatoms, wherein the 7-10 membered bicyclic heterocyclyl is optionally substituted with 1-4 (e.g., 1-2) **R⁷**.

**Embodiment 56.** The compound of any one of Embodiments 1-32, 42, or 55, wherein **R¹** is a 7-10 (e.g., 7; e.g., 9) membered spirocyclic bicyclic heterocyclyl having one ring nitrogen atom, one ring oxygen atom, and no additional ring heteroatoms, wherein the 7-10 membered spirocyclic bicyclic heterocyclyl is optionally substituted with 1-4 (e.g., 1-2) **R⁷**.

**Embodiment 57.** The compound of any one of Embodiments 1-32, 42, or 55-56, wherein **R¹** is 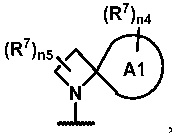 wherein: **Ring A1** is a 4-7 membered heterocyclyl ring having one ring oxygen atom and no additional ring heteroatoms; **n4** is 0, 1, or 2; and **n5** is 0, 1, or 2, provided that **n4** + **n5** is 0, 1, or 2.

**Embodiment 58.** The compound of any one of Embodiments 1-32, 42, or 55-57, wherein **R¹** is 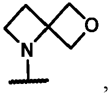 which is optionally substituted with 1-2 **R⁷**.

**Embodiment 59.** The compound of any one of Embodiments 1-32, 42, or 55-58, wherein **R¹** is

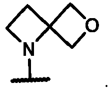

**Embodiment 60.** The compound of any one of Embodiments 1-32, 42, or 55-57, wherein **R¹** is 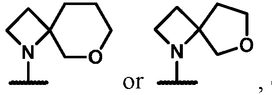 each of which is optionally substituted with 1-2 **R⁷**.

**Embodiment 61.** The compound of any one of Embodiments 1-32, 42, 55-57, or 60, wherein **R¹** is 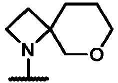 (e.g., 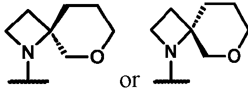 ) or 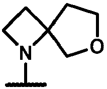 (e.g., 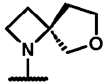 or 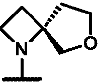 ).

**Embodiment 62.** The compound of any one of Embodiments 1-32, wherein **R¹** is a 4-5 membered heterocyclyl (e.g., azetidinyl or pyrrolidinyl) optionally substituted with 1-4 (e.g., 1-2) **R⁷**.

**Embodiment 63.** The compound of any one of Embodiments 1-32 or 62, wherein **R¹** is a 4-membered heterocyclyl optionally substituted with 1-4 (e.g., 1-2) **R⁷**.

**Embodiment 64.** The compound of any one of Embodiments 1-32 or 62-63, wherein **R¹** is 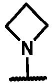 optionally substituted with 1-4 (e.g., 1-2) **R⁷**.

**Embodiment 65.** The compound of any one of Embodiments 1-32 or 62-64, wherein **R¹** is selected from the group consisting of:

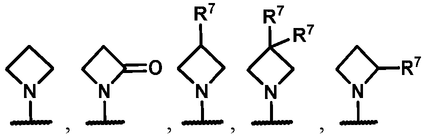

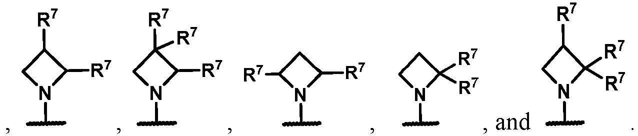

**Embodiment 66.** The compound of any one of Embodiments 1-32 or 62-65, wherein **R¹** is 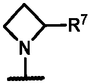 (e.g., 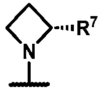 ), 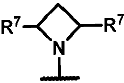 (e.g., 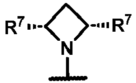 ), or

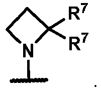

**Embodiment 67.** The compound of any one of Embodiments 62-66, wherein each **R⁷** is independently selected from the group consisting of: halo; cyano; -OH; oxo; -C₁₋₆ alkoxy; C(=O)N(**R^{f}**)₂; **R^{b1}**; -(C₁₋₃ alkylene)-**R^{b1}**; -O-**R^{b1}**; and C₁₋₆ alkyl optionally substituted with 1-3 **R^{c7}**, wherein:
each **R^{b1}** is independently selected from the group consisting of: C₃₋₆ cycloalkyl, phenyl, 4-6 membered heterocyclyl, and 5-6 membered heteroaryl, each of which is optionally substituted with 1-3 **R^{g}** (e.g., each **R^{g}** is independently selected from the group consisting of: halo and C₁₋₃ alkyl); and
each **R^{c7}** is independently selected from the group consisting of: halo, cyano, -OH, and -C₁₋₆ alkoxy.

**Embodiment 68.** The compound of any one of Embodiments 62-67, wherein each **R⁷** is independently selected from the group consisting of:
-F;
-cyano;
-OH;
-**R^{b1}**, wherein the **R^{b1}** is a 5-6 membered heteroaryl optionally substituted with 1-2 **R^{g}**,
C₁₋₃ alkyl optionally substituted with 1-3 F;
C₁₋₃ alkyl substituted with -OH or C₁₋₃ alkoxy; and
C(=O)N(**R^{f}**)₂.

**Embodiment 69.** The compound of Embodiment 67 or 68, wherein at least one **R⁷** is C₁₋₃ alkyl substituted with -OH.

**Embodiment 70.** The compound of any one of Embodiments 1-32, wherein **R¹** is 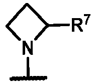 (e.g., 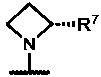 ), wherein **R⁷** is C₁₋₃ alkyl substituted with -OH or C₁₋₃ alkoxy (e.g., **R⁷** is C₁₋₃ alkyl substituted with -OH).

**Embodiment 71.** The compound of any one of Embodiments 1-32 or 70, wherein **R¹** is 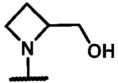 (e.g., 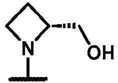 ).

**Embodiment 72.** The compound of any one of Embodiments 1-32, wherein **R¹** is 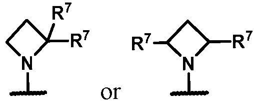 (e.g., 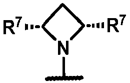 ), wherein each **R⁷** is independently selected from the group consisting of:
C₁₋₃ alkyl optionally substituted with 1-3 F; and
C₁₋₃ alkyl substituted with -OH or C₁₋₃ alkoxy.

**Embodiment 73.** The compound of any one of Embodiments 1-32 or 72, wherein **R¹** is 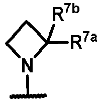 (e.g., 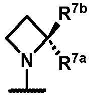 ) or 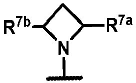 (e.g., 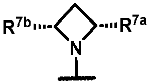 ), wherein **R^{7a}** is C₁₋₃ alkyl substituted with -OH (e.g., -CH₂OH); and
**R^{7b}** is selected from the group consisting of:
C₁₋₃ alkyl optionally substituted with 1-3 F (e.g., methyl), and
C₁₋₃ alkyl substituted with -OH or C₁₋₃ alkoxy.

**Embodiment 74.** The compound of any one of Embodiments 1-32 or 72-73, wherein **R¹** is 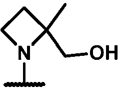 (e.g., 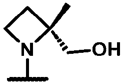 ).

**Embodiment 75.** The compound of any one of Embodiments 1-32 or 72-73, wherein **R¹** is 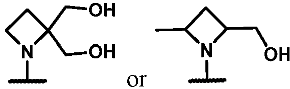 (e.g., 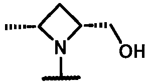 ). 1.

**Embodiment 76.** The compound of Embodiment 1, wherein the compound is a compound of Formula (**II-3**): 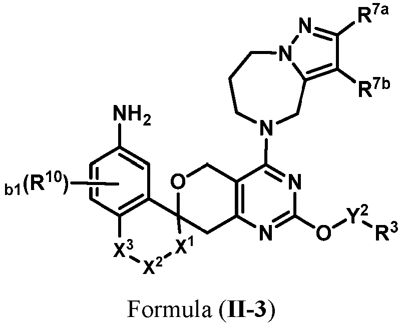 or a pharmaceutically acceptable salt thereof, wherein:
**b1** is 1 or 2;
each **R¹⁰** is independently selected from the group consisting of: -Cl, -F, -CN, and C₁₋₃ alkyl optionally substituted with 1-3 **R^{c}**;
**R^{7a}** is selected from the group consisting of:
   **(a)** C(=O)N(**R^{f}**)₂, wherein each **R^{f}** is independently H or C₁₋₃ alkyl optionally substituted with 1-3 **R^{h}**;
   **(b)** C(=O)N(C₁₋₃ alkyl)**R^{b1}** or C(=O)N(H)**R^{b1}**, wherein: **R^{b1}** is C₃₋₆ cycloalkyl or 4-6 membered heterocyclyl, each of which is optionally substituted with 1-3 **R^{g}**; and
   **(c)** C(=O)**R^{b1}**, wherein **R^{b1}** is 4-10 membered heterocyclyl optionally substituted with 1-3 **R^{g}**, wherein **R^{b1}** is attached to the C(=O) via a ring nitrogen atom;
**R^{7b}** is -halo or C₁₋₃ alkyl;
**X¹** is CH₂; and
**X²** and X³ are independently selected from the group consisting of: O, CH₂, CH**R^{L}**, and C(**R^{L}**)₂.

**Embodiment 77.** The compound of Embodiment 76, wherein **b1** is 1.

**Embodiment 78.** The compound of Embodiment 1, wherein the compound of Formula (**II**) is a compound of Formula (**II-a3**): 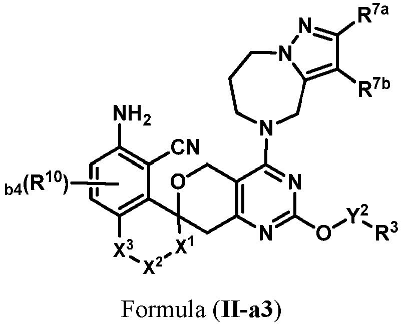 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c}**;
**R^{7a}** is selected from the group consisting of:
   (a) C(=O)N(**R^{f}**)₂, wherein each **R^{f}** is independently H or C₁₋₃ alkyl optionally substituted with 1-3 **R^{h}**;
   (b) C(=O)N(C₁₋₃ alkyl)**R^{b1}** or C(=O)N(H)**R^{b1}**, wherein: **R^{b1}** is C₃₋₆ cycloalkyl or 4-6 membered heterocyclyl, each of which is optionally substituted with 1-3 **R^{g}**; and
   **(c)** C(=O)**R^{b1}**, wherein **R^{b1}** is 4-10 membered heterocyclyl optionally substituted with 1-3 **R^{g}**, wherein **R^{b1}** is attached to the C(=O) via a ring nitrogen atom;
**R^{7b}** is -halo or C₁₋₃ alkyl;
**X¹** is CH₂; and
**X²** and **X³** are independently selected from the group consisting of: O, CH₂, CH**R^{L}**, and C(**R^{L}**)₂.

**Embodiment 79.** The compound of Embodiment 78, wherein **b4** is 0.

**Embodiment 80.** The compound of Embodiment 4, wherein the compound of Formula (**III**) is a compound of Formula **(**I**II-3**): 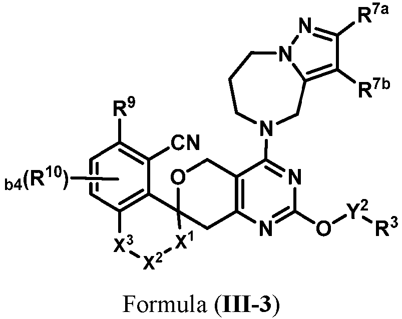 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c}**;
**R^{7a}** is selected from the group consisting of:
   **(a)** C(=O)N(**R^{f}**)₂, wherein each **R^{f}** is independently H or C₁₋₃ alkyl optionally substituted with 1-3 **R^{h}**;
   **(b)** C(=O)N(C₁₋₃ alkyl)**R^{b1}** or C(=O)N(H)**R^{b1}**, wherein: **R^{b1}** is C₃₋₆ cycloalkyl or 4-6 membered heterocyclyl, each of which is optionally substituted with 1-3 **R^{g}**; and
   **(c)** C(=O)**R^{b1}**, wherein **R^{b1}** is 4-10 membered heterocyclyl optionally substituted with 1-3 **R^{g}**, wherein **R^{b1}** is attached to the C(=O) via a ring nitrogen atom;
**R^{7b}** is -halo or C₁₋₃ alkyl;
**X¹ is** CH₂; and
**X²** and **X³** are independently selected from the group consisting of: O, CH₂, CH**R^{L}**, and C(**R^{L}**)₂.

**Embodiment 81.** The compound of Embodiment 80, wherein **b4** is 0.

**Embodiment 82.** The compound of Embodiment 80 or 81, wherein **R⁹** is N**R^{d}R^{e}** (e.g., -NH₂).

**Embodiment 83.** The compound of Embodiment 16, wherein the compound of Formula (**IV**) is a compound of Formula (**IV-a3**) or (**IV-b3**): 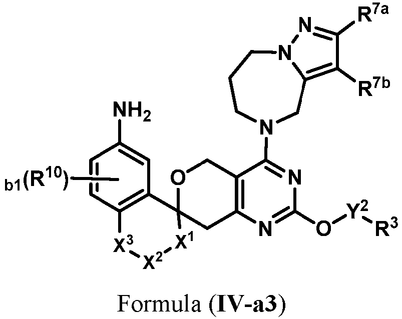 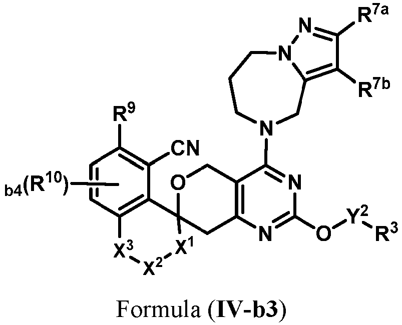 or a pharmaceutically acceptable salt thereof, wherein:
**b1** is 0, 1, or 2;
**b4** is 0 or 1;
each **R¹⁰** is independently selected from the group consisting of: -Cl, -F, -CN, and C₁₋₃ alkyl optionally substituted with 1-3 **R^{c}**;
**R^{7a}** is selected from the group consisting of:
   **(a)** C(=O)N(**R^{f}**)₂, wherein each **R^{f}** is independently H or C₁₋₃ alkyl optionally substituted with 1-3 **R^{h}**;
   **(b)** C(=O)N(C₁₋₃ alkyl)**R^{b1}** or C(=O)N(H)**R^{b1}**, wherein: **R^{b1}** is C₃₋₆ cycloalkyl or 4-6 membered heterocyclyl, each of which is optionally substituted with 1-3 **R^{g}**; and
   **(c)** C(=O)**R^{b1}**, wherein **R^{b1}** is 4-10 membered heterocyclyl optionally substituted with 1-3 **R^{g}**, wherein **R^{b1}** is attached to the C(=O) via a ring nitrogen atom;
**R^{7b}** is -halo or C₁₋₃ alkyl;
**X¹** is CH₂;
one of **X²** and **X³** is independently selected from the group consisting of: CH**R^{L}** and C(**R^{L}**)₂; and
the other of **X²** and **X³** is CH₂ or O.

**Embodiment 84.** The compound of Embodiment 83, wherein the compound is a compound of Formula (**IV-b3**), or a pharmaceutically acceptable salt thereof, wherein **b4** is 0.

**Embodiment 85.** The compound of Embodiment 83, wherein the compound is a compound of Formula (**IV-a3**), or a pharmaceutically acceptable salt thereof, wherein **b1** is 1; and the 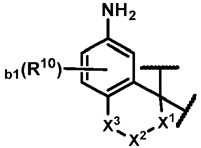 moiety is

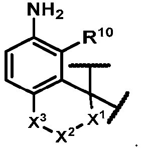

**Embodiment 86.** The compound of any one of Embodiments 76-85, wherein **X²** is CH₂; and **X³** is CH**R^{L}**.

**Embodiment 87**. The compound of Embodiment 86, wherein **R^{L}** is CF₃ or CH₃ (e.g., CH₃).

**Embodiment 88.** The compound of any one of Embodiments 76-82, wherein **X²** is CH₂; and **X³** is CH₂.

**Embodiment 89.** The compound of any one of Embodiments 76-88, wherein **R^{7a}** is C(=O)N(**R^{f}**)₂, wherein each **R^{f}** is independently C₁₋₃ alkyl optionally substituted with 1-3 **R^{h}** (e.g., **R^{7a}** is C(=O)N(Me)₂).

**Embodiment 90.** The compound of any one of Embodiments 76-89, wherein **R^{7b}** is -Cl, -F, or methyl.

**Embodiment 91.** The compound of any one of Embodiments 76-90, wherein **R^{7b}** is -Cl.

**Embodiment 92.** The compound of any one of Embodiments 76-88, wherein the 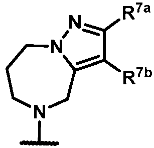 moiety is

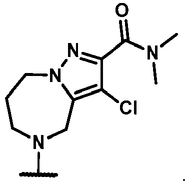

**Embodiment 93.** The compound of Embodiment 1, wherein the compound of Formula (II) is a compound of Formula (**II-4**), (**II-5**), or (**II-8**): 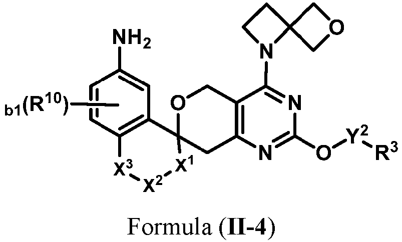 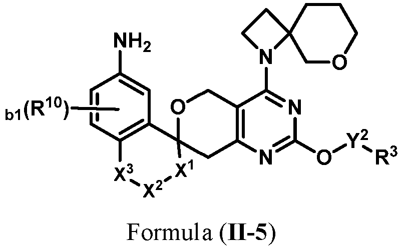 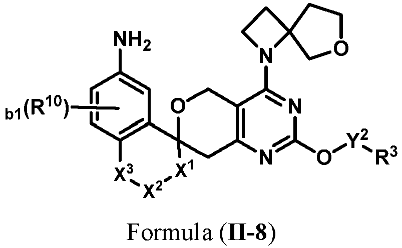 or a pharmaceutically acceptable salt thereof, wherein:
**b1** is 1 or 2;
each **R¹⁰** is independently selected from the group consisting of: -Cl, -F, -CN, and C₁₋₃ alkyl optionally substituted with 1-3 **R^{c}**;
**X¹** is CH₂; and
**X²** and **X³** are independently selected from the group consisting of: O, CH₂, CH**R^{L}**, and C(**R^{L}**)₂.

**Embodiment 94.** The compound of Embodiment 93, wherein **b1** is 1.

**Embodiment 95.** The compound of Embodiment 1, wherein the compound of Formula (**II**) is a compound of Formula (**II-a4**), (**II-a5**), or (**II-a8**): 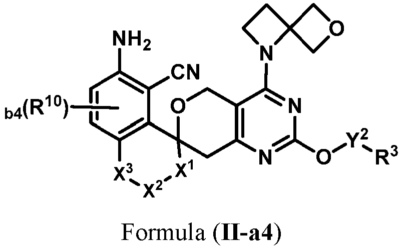 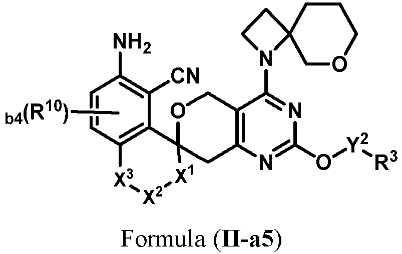 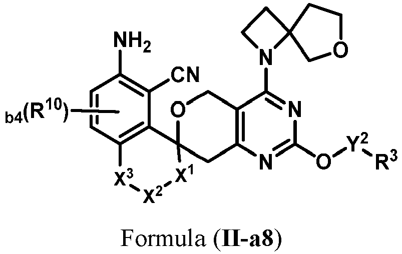 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c}**;
**X¹** is CH₂; and
**X²** and **X³** are independently selected from the group consisting of: O, CH₂, CH**R^{L}**, and C(**R^{L}**)₂.

**Embodiment 96.** The compound of Embodiment 95, wherein b4 is 0.

**Embodiment 97.** The compound of Embodiment 4, wherein the compound of Formula (**III**) is a compound of Formula (**III-4**), (**III-5**), or (**III-8**): 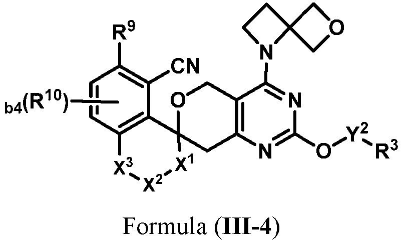 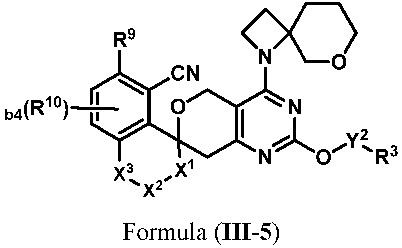 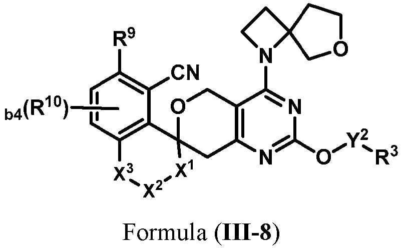 or a pharmaceutically acceptable salt thereof, wherein:
**b4** is 0 or 1;
each **R¹⁰** is independently selected from the group consisting of: -Cl, -F, -CN, and C₁₋₃ alkyl optionally substituted with 1-3 **R^{c}**;
**X¹** is CH₂; and
**X²** and **X³** are independently selected from the group consisting of: O, CH₂, CH**R^{L}**, and C(**R^{L}**)₂.

**Embodiment 98.** The compound of Embodiment 97, wherein **b4** is 0.

**Embodiment 99.** The compound of Embodiment 97 or 98, wherein **R⁹** is N**R^{d}R^{e}** (e.g., -NH₂).

**Embodiment 100.** The compound of Embodiment 16, wherein the compound is a compound of Formula (**IV-a4**), (**IV-b4**), (**IV-a5**), (**IV-b5**), (**IV-a8**), or (**IV-b8**): 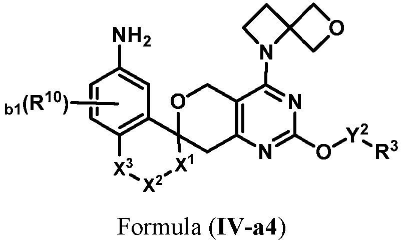 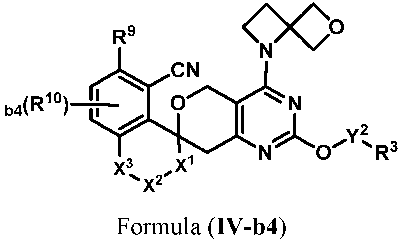 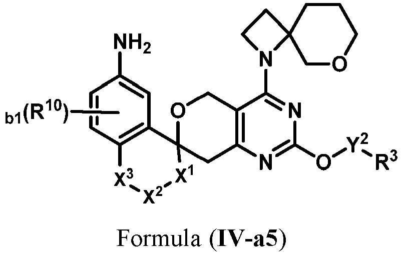 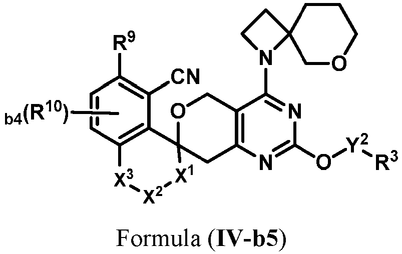 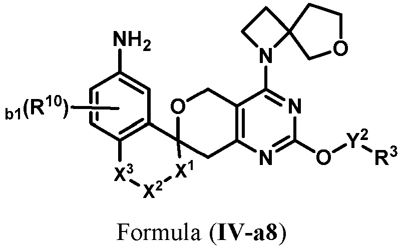 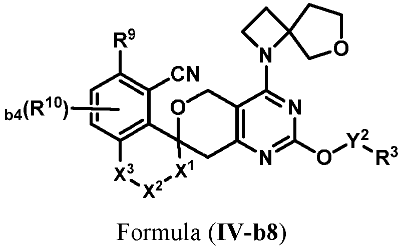 or a pharmaceutically acceptable salt thereof, wherein:
**b1** is 0, 1, or 2;
**b4** is 0 or 1;
each **R¹⁰** is independently selected from the group consisting of: -Cl, -F, -CN, and C₁₋₃ alkyl optionally substituted with 1-3 **R^{c}**;
**X¹** is CH₂;
one of **X²** and **X³** is independently selected from the group consisting of: CH**R^{L}** and C(**R^{L}**)₂; and
the other of **X²** and **X³** is CH₂ or O.

**Embodiment 101.** The compound of Embodiment 100, wherein the compound is a compound of Formula (**IV-a4**), (**IV-a5**), or (**IV-a8**), or a pharmaceutically acceptable salt thereof, wherein **b1** is 1; and the 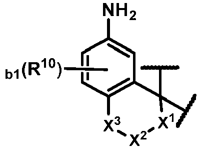 moiety is 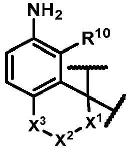 (e.g., 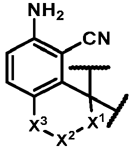 ).

**Embodiment 102**. The compound of Embodiment 100, wherein the compound is a compound of Formula (**IV-b4**), (**IV-b5**), or (**IV-b8**), or a pharmaceutically acceptable salt thereof, wherein **b4** is 0.

**Embodiment 103.** The compound of Embodiment 1, wherein the compound of Formula (**II**) is a compound of Formula (**II-6**) or (**II-7**): 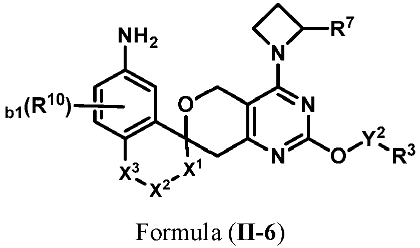 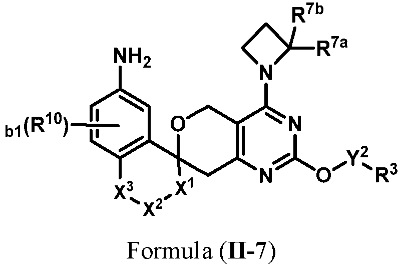 or a pharmaceutically acceptable salt thereof, wherein:
**R⁷** is C₁₋₃ alkyl substituted with -OH or C₁₋₃ alkoxy;
**R^{7a}** is C₁₋₃ alkyl substituted with -OH (e.g., -CH₂OH);
**R^{7b}** is selected from the group consisting of:
   C₁₋₃ alkyl optionally substituted with 1-3 F (e.g., methyl), and
   C₁₋₃ alkyl substituted with -OH or C₁₋₃ alkoxy;
   **b1** is 1 or 2;
   each **R¹⁰** is independently selected from the group consisting of: -Cl, -F, -CN, and C₁₋₃ alkyl optionally substituted with 1-3 **R^{c}**;
   **X¹** is CH₂; and
   **X²** and **X³** are independently selected from the group consisting of: O, CH₂, CH**R^{L}**, and C(**R^{L}**)₂.

**Embodiment 104.** The compound of Embodiment 103, wherein **b1** is 1.

**Embodiment 105.** The compound of Embodiment 1, wherein the compound of Formula (**II**) is a compound of Formula (**II-a6**) or (**II-a7**): 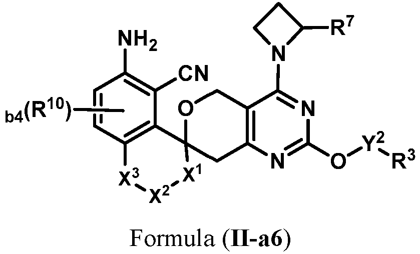 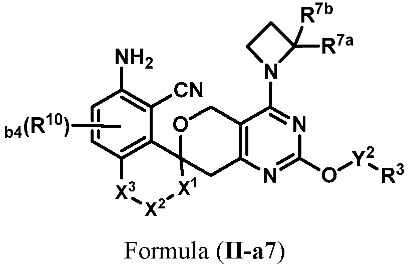 or a pharmaceutically acceptable salt thereof, wherein:
**R⁷** is C₁₋₃ alkyl substituted with -OH or C₁₋₃ alkoxy;
**R^{7a}** is C₁₋₃ alkyl substituted with -OH (e.g., -CH₂OH);
**R^{7b}** is selected from the group consisting of:
   C₁₋₃ alkyl optionally substituted with 1-3 F (e.g., methyl), and
   C₁₋₃ alkyl substituted with -OH or C₁₋₃ alkoxy;
   **b4** is 0 or 1;
   each **R¹⁰** is independently selected from the group consisting of: -Cl, -F, -CN, and C₁₋₃ alkyl optionally substituted with 1-3 **R^{c}**;
   **X¹** is CH₂; and
   **X²** and **X³** are independently selected from the group consisting of: O, CH₂, CH**R^{L}**, and C(**R^{L}**)₂.

**Embodiment 106.** The compound of Embodiment 105, wherein b4 is 0.

**Embodiment 107.** The compound of Embodiment 4, wherein the compound of Formula (**III**) is a compound of Formula (**III-6**) or (**III-7**): 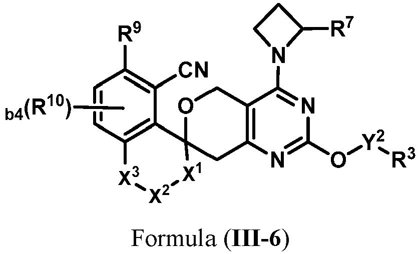 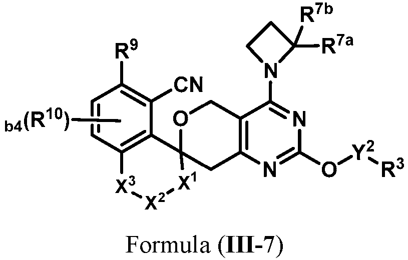 or a pharmaceutically acceptable salt thereof, wherein:
**R⁷** is C₁₋₃ alkyl substituted with -OH or C₁₋₃ alkoxy;
**R^{7a}** is C₁₋₃ alkyl substituted with -OH (e.g., -CH₂OH);
**R^{7b}** is selected from the group consisting of:
   C₁₋₃ alkyl optionally substituted with 1-3 F (e.g., methyl), and
   C₁₋₃ alkyl substituted with -OH or C₁₋₃ alkoxy;
   **b4** is 0 or 1;
   each **R¹⁰** is independently selected from the group consisting of: -Cl, -F, -CN, and C₁₋₃ alkyl optionally substituted with 1-3 **R^{c}**;
   **X¹** is CH₂; and
   **X²** and **X³** are independently selected from the group consisting of: O, CH₂, CH**R^{L}**, and C(**R^{L}**)₂.

**Embodiment 108.** The compound of Embodiment 107, wherein **b4** is 0.

**Embodiment 109.** The compound of Embodiment 107 or 108, wherein **R⁹** is N**R^{d}R^{e}** (e.g., -NH₂).

**Embodiment 110.** The compound of Embodiment 16, wherein the compound of Formula (**IV**) is a compound of Formula (**IV-a6**), (**IV-b6**), (**IV-a7**), or (**IV-b7**): 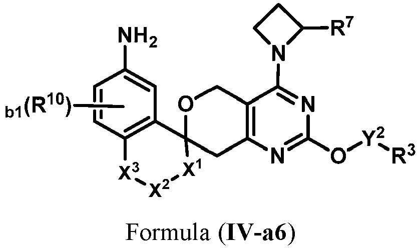 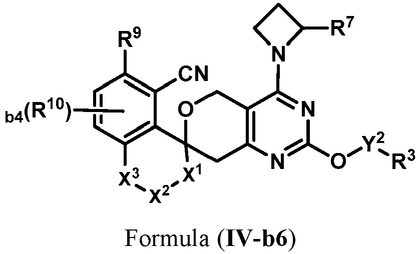 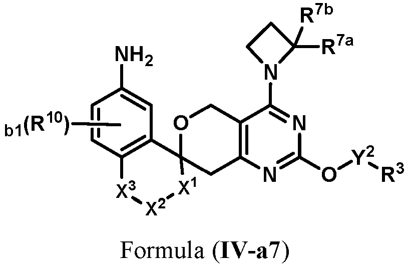 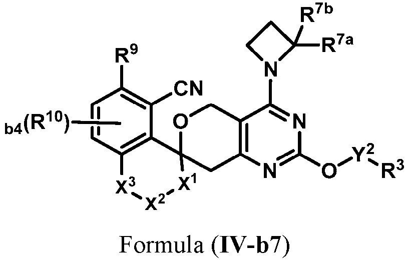 or a pharmaceutically acceptable salt thereof, wherein:
**R⁷** is C₁₋₃ alkyl substituted with -OH or C₁₋₃ alkoxy;
**R^{7a}** is C₁₋₃ alkyl substituted with -OH (e.g., -CH₂OH);
**R^{7b}** is selected from the group consisting of:
   C₁₋₃ alkyl optionally substituted with 1-3 F (e.g., methyl), and
   C₁₋₃ alkyl substituted with -OH or C₁₋₃ alkoxy;
   **b1** is 0, 1, or 2;
   **b4** is 0 or 1;
   each **R¹⁰** is independently selected from the group consisting of: -Cl, -F, -CN, and C₁₋₃ alkyl optionally substituted with 1-3 **R^{c}**;
   **X¹** is CH₂;
   one of **X²** and **X³** is independently selected from the group consisting of: CH**R^{L}** and C(**R^{L}**)₂; and
   the other of **X²** and **X³** is CH₂ or O.

**Embodiment 111.** The compound of Embodiment 110, wherein the compound is a compound of Formula (**IV-a6**) or (**IV-a7**), or a pharmaceutically acceptable salt thereof, wherein **b1** is 1; and the 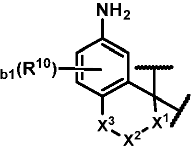 moiety is 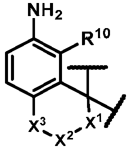 (e.g., 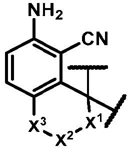 ).

**Embodiment 112.** The compound of Embodiment 110, wherein the compound is a compound of Formula (**IV-b6**) or (**IV-b7**), or a pharmaceutically acceptable salt thereof, wherein **b4** is 0.

**Embodiment 113.** The compound of any one of Embodiments 103-112, wherein the 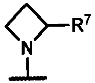 moiety is

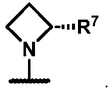

**Embodiment 114.** The compound of any one of Embodiments 103-113, wherein **R⁷** is C₁₋₃ alkyl substituted with -OH.

**Embodiment 115.** The compound of any one of Embodiments 103-114, wherein **R⁷** is -CH₂OH.

**Embodiment 116.** The compound of any one of Embodiments 103-112, wherein the 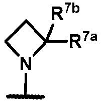 moiety is

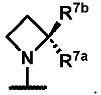

**Embodiment 117.** The compound of any one of Embodiments 103-112 or 116, wherein **R^{7a}** is -CH₂OH.

**Embodiment 118.** The compound of any one of Embodiments 103-112 or 116-117, wherein **R^{7b}** is C₁₋₃ alkyl optionally substituted with 1-3 -F.

**Embodiment 119.** The compound of Embodiment 118, wherein **R^{7b}** is methyl.

**Embodiment 120.** The compound of any one of Embodiments 93-119, wherein **X²** is CH₂; and **X³** is CH**R^{L}** (e.g., CH(CH₃)).

**Embodiment 121.** The compound of any one of Embodiments 93-99 or 103-109, wherein **X²** is CH₂; and **X³** is CH₂.

**Embodiment 122.** The compound of any one of Embodiments 76-121, wherein **Y²** is -CH₂-; and **R³** is a 4-10 membered heterocyclyl having one ring nitrogen atom and 0-1 additional ring heteroatom selected from the group consisting of oxygen and nitrogen, wherein the heterocyclyl is optionally substituted with 1-6 **R^{a}**.

**Embodiment 123.** The compound of any one of Embodiments 76-122, wherein **Y²** is -CH₂-; and **R³** is 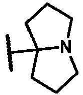 optionally substituted with 1-2 substituents each independently selected from the group consisting of: -F, -C₁₋₃ alkoxy, and -C₁₋₃ haloalkoxy.

**Embodiment 124.** The compound of any one of Embodiments 76-123, wherein **Y²** is -CH₂-; and **R³** is 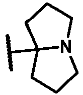 optionally substituted with 1-2 -F (e.g., **R³** is 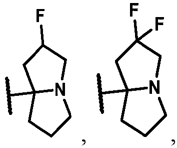 or 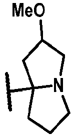 ).

**Embodiment** 125. The compound of any one of Embodiments 76-124, wherein **R³** is 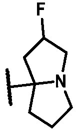 (e.g., 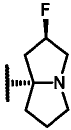 ).

**Embodiment 126.** The compound of any one of Embodiments 1-125, wherein the 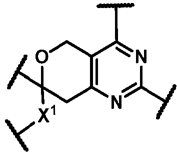 moiety is

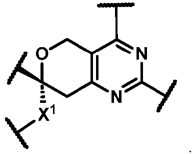

**Embodiment 127.** The compound of Embodiment 1, wherein the compound of Formula (**II**) is selected from the group consisting of Compound Nos. **181b, 501, 501a, 501b, 501c, 502, 502a, 502b, 503, 503a, 503b, 503c, 504, 504a, 504b, 504c, 505, 505a, 506, 506a, 507, 507a, 507b, 507c, 508, 508a, 509, 509a, 509b, 510, 510a, 510b, 510c, 510d, 510e, 510f, 511, 511a, 512, 512a, 512b, 513, 513a, 514, 514a, 514b, 516, 516a, 516b, 516c, 517, 517a, 518, 518a, 519, 519a, 520, 520a, 522, 522a, 523, 523a, 524, 524a, 525, 525a, 526, 526a, 526b, 526c, 527, 527a, 527b, 528, 528a, 529, 529a, 530, 530a, 531, 531a, 532, 532a, 534, 534a, 535, 535a, 536, 536a, 536b, 536c, 537, 537a, 538, 538a, 539, 539a, 540, 540a, 541, 541a, 542, 542a, 543, 543a, 544, 544a, 545, 545a, 546, 546a, 547, 547a, 548, 548a, 549, 549a, 550, 550a, 551, 551a, 552, 552a, 553, 553a, 554, 554a, 554b, 554c, 555, 555a, 556, 556a, 556b, 558, 558a, 559, 559a, 560, 560a, 561, 561a, 562, 562a, 563, 563a, 564, 564a, 565, 565a, 566, 566a, 566b, 567, 567a, 567b, 568, 568a, 568b, 569, 569a, 570, 570a, 571, 571a, 572, 572a, 573, 573a, 575, 575a, 575b, 576, 576a, 576b, 576c, 576d, 577, 577a, 578, 578a, 578b, 579, 579a, 580, 580a, 581, 581a, 582, 582a, 583, 583a, 584, 584a, 585, 585a, 585b, 586, 586a, 587, 587a, 588, 588a, 589, 589a, 590, 590a, 591, 591a, 592, 592a, 593, 593a, 594, 594a, 595, 595a, 595b, 596, 596a, 597, 597a, 597b, 598, 598a, 598b, 598c, 599, 599a, 599b, 600, 600a, 600b, 601, 601a, 602, 602a, 603, 603a, 603b, 606, 606a, 606b, 607, 607a, 608, 608a, 608b, 609, 609a, 609b, 610, 610a, 610b, 611, 611a, 611b, 612, 612a, 612b, 613, 613a, 613b, 613c, 614, 614a, 614b, 614c, 615, 615a, 616, 616a, 617, 617a, 618, 618a, 619, 619a, 620, 620a, 621, 621a, 621b, 622, 622a, 623, 623a, 624, 624a, 624b, 625, 625a, 626, 626a, 627, 627a, 628, 628a, 628b, 628c, 629, 629a, 630, 630a, 631, 631a, 632, 632a, 633, 633a, 634, 634a, 635, 635a, 636, 636a, 637, 637a, 637b, 638, 638a, 639, 639a, 640, 640a, 641, 641a, 641b, 641c, 642, 642a, 643, 643a, 643b, 644, 644a, 645, 645a, 645b, 646, 646a, 647, 647a, 647b, 648, 648a, 648b, 649, 649a, 649b, 650, 650a, 650b, 650c, 650d, 651, 651a, 651b, 652, 652a, 652b, 652c, 653, 653a, 653b, 654, 654a, 654b, 654c, 654d, 655, 655a, 656, 656a, 657, 657a, 658, 658a, 659, 659a, 660, 660a, 660b, 661, 661a, 661b, 662, 662a, 662b, 662c, 662d, 663, 663a, 663b, 664, 664a, 664b, 665, 665a, 665b, 666, 666a, 667, 667a, 667b, 668, 668a, 668b, 668c, 668d, 669, 669a, 670, 670a, 671, 671a, 671b, 672, 672a, 673, 673a, 674, 674a, 675, 675a, 676, 676a, 677, 677a, 678, 678a, 679, 679a, 680, 680a, 680b, 680c, 681, 681a, 682, 682a, 683, 683a, 684, 684a, 685, 685a, 686, 686a, 687, 687a, 688, 688a, 689, 689a, 689b, 690, 690a, 691, 691a, 692, 692a, 693, 693a, 694, 694a, 695, 695a, 696, 696a, 697, 697a, 698, 698a, 699, 699a, 700, 700a, 701, 701a, 702, 702a, 702b, 703, 703a, 704, 704a, 705, 705a, 706, 706a, 706b, 706c, 706d, 707, 707a, 708, 708a, 709, 709a, 710, 710a, 711, 711a, 712, 712a, 713, 713a, 714, 714a, 715, 715a, 716, 716a, 717, 717a, 718, 718a, 719, 719a, 720, 720a, 721, 721a, 722, 722a, 723, 723a, 724, 724a, 725, 725a, 726, 726a, 727, 727a, 728, 728a, 729, 729a, 729b, 730, 730a, 731, 731a, 732, 732a, 733, 733a, 734, 734a, 735, 735a, 736, 736a, 737, 737a, 738, 738a, 739, 739a, 740, 740a, 741, 741a, 741b, 742, 742a, 743, 743a, 744, 744a, 745, 745a, 745b, 746, 746a, 747, 747a, 748, 748a, 749,** and **749a** as depicted in **Table C3**, or a pharmaceutically acceptable salt thereof.

**Embodiment 128.** The compound of Embodiment 4, wherein the compound of Formula (**III**) is selected from the group consisting of Compound Nos. **502, 502a, 502b, 503, 503a, 503b, 503c, 504, 504a, 504b, 504c, 505, 505a, 507, 507a, 507b, 507c, 509, 509a, 509b, 511, 511a, 512, 512a, 512b, 513, 513a, 514, 514a, 514b, 516, 516a, 516b, 516c, 517, 517a, 518, 518a, 519, 519a, 520, 520a, 522, 522a, 523, 523a, 524, 524a, 525, 525a, 526, 526a, 526b, 526c, 527, 527a, 527b, 528, 528a, 529, 529a, 530, 530a, 531, 531a, 532, 532a, 534, 534a, 535, 535a, 536, 536a, 536b, 536c, 537, 537a, 538, 538a, 539, 539a, 540, 540a, 541, 541a, 542, 542a, 543, 543a, 544, 544a, 545, 545a, 546, 546a, 547, 547a, 548, 548a, 549, 549a, 550, 550a, 551, 551a, 552, 552a, 553, 553a, 554, 554a, 554b, 554c, 555, 555a, 556, 556a, 556b, 558, 558a, 559, 559a, 560, 560a, 561, 561a, 562, 562a, 563, 563a, 564, 564a, 565, 565a, 566, 566a, 566b, 567, 567a, 567b, 568, 568a, 568b, 569, 569a, 570, 570a, 571, 571a, 572, 572a, 573, 573a, 574, 574a, 574b, 574c, 575, 575a, 575b, 576, 576a, 576b, 576c, 576d, 577, 577a, 578, 578a, 578b, 579, 579a, 580, 580a, 581, 581a, 582, 582a, 583, 583a, 584, 584a, 585, 585a, 585b, 586, 586a, 587, 587a, 588, 588a, 589, 589a, 590, 590a, 591, 591a, 592, 592a, 593 , 593a, 594, 594a, 595, 595a, 595b, 596, 596a, 597, 597a, 597b, 598, 598a, 598b, 598c, 599, 599a, 599b, 600, 600a, 600b, 601, 601a, 602, 602a , 603, 603a, 603b, 604, 604a, 604b, 604c, 605, 605a, 605b, 605c, 606, 606a, 606b, 607, 607a, 608, 608a, 608b, 609, 609a, 609b, 610, 610a, 610b, 611, 611a, 611b, 612, 612a, 612b, 613, 613a, 613b, 613c, 614, 614a, 614b, 614c, 615, 615a, 616, 616a, 617, 617a, 618, 618a, 619, 619a, 620, 620a, 621, 621a, 621b, 622, 622a, 623, 623a, 624, 624a, 624b, 625, 625a, 626, 626a, 627, 627a, 628, 628a, 628b, 628c, 629, 629a, 630, 630a, 631, 631a, 632, 632a, 633, 633a, 634, 634a, 635, 635a, 636, 636a, 637, 637a, 637b, 638, 638a, 639, 639a, 640, 640a, 641, 641a, 641b, 641c, 642, 642a, 643, 643a, 643b, 644, 644a, 645, 645a, 645b, 646, 646a, 647, 647a, 647b, 648, 648a, 648b, 649, 649a, 649b, 650, 650a, 650b, 650c, 650d, 651, 651a, 651b, 652, 652a, 652b, 652c, 653, 653a, 653b, 654, 654a, 654b, 654c, 654d, 655, 655a, 656, 656a, 657, 657a, 658, 658a, 659, 659a, 660, 660a, 660b, 661, 661a, 661b, 662, 662a, 662b, 662c, 662d, 663, 663a, 663b, 664, 664a, 664b, 665, 665a, 665b, 666, 666a, 667, 667a, 667b, 668, 668a, 668b, 668c, 668d, 669, 669a, 670, 670a, 671, 671a, 671b, 672, 672a, 673, 673a, 674, 674a, 675, 675a, 676, 676a, 677, 677a, 678, 678a, 679, 679a, 680, 680a, 680b, 680c, 681, 681a, 682, 682a, 683, 683a, 684, 684a, 685, 685a, 686, 686a, 687, 687a, 688, 688a, 689, 689a, 689b, 690, 690a, 691, 691a, 692, 692a, 693, 693a, 694, 694a, 695, 695a,696, 696a,697, 697a,698, 698a, 699, 699a, 700, 700a, 701, 701a, 702, 702a, 702b, 703, 703a, 704, 704a, 705, 705a, 706, 706a, 706b, 706c, 706d, 707, 707a, 708, 708a, 709, 709a, 710, 710a, 711, 711a, 712, 712a, 713, 713a, 714, 714a, 715, 715a, 716, 716a, 717, 717a, 718, 718a, 719, 719a, 720, 720a, 721, 721a, 722, 722a, 723, 723a, 724, 724a, 725, 725a, 726, 726a, 727, 727a, 728, 728a, 729, 729a, 729b, 730, 730a, 731, 731a, 732, 732a, 733, 733a, 734, 734a, 735, 735a, 736, 736a, 737, 737a, 738, 738a, 739, 739a, 740, 740a, 741, 741a, 741b, 742, 742a, 743, 743a, 744, 744a, 745, 745a, 745b, 746, 746a, 747, 747a, 748, 748a**, **749**, and **749a** as depicted in **Table C3**, or a pharmaceutically acceptable salt thereof.

**Embodiment 129**. The compound of Embodiment 16, wherein the compound of Formula (**IV**) is selected from the group consisting of Compound Nos. **502, 502a, 502b, 508, 508a, 509, 509a, 509b, 511, 511a, 512, 512a, 512b, 530, 530a, 531, 531a, 532, 532a, 546, 546a, 553, 553a, 554, 554a, 554b, 554c, 555, 555a, 556, 556a, 556b, 560, 560a, 561, 561a, 566, 566a, 566b, 567, 567a, 567b, 568, 568a, 568b, 569, 569a, 570, 570a, 571, 571a, 572, 572a, 573, 573a, 575, 575a, 575b, 576, 576a, 576b, 576c, 576d, 577, 577a, 578, 578a, 578b, 581, 581a, 582, 582a, 583, 583a, 584, 584a, 585, 585a, 585b, 589, 589a, 590, 590a, 592, 592a, 595, 595a, 595b, 596, 596a, 597, 597a, 597b, 598, 598a, 598b, 598c, 599, 599a, 599b, 600, 600a, 600b, 601, 601a, 604, 604a, 604b, 604c, 605, 605a, 605b, 605c, 606, 606a, 606b, 607, 607a, 608, 608a, 608b, 609, 609a, 609b, 610, 610a, 610b, 611, 611a, 611b, 612, 612a, 612b, 613, 613a, 613b, 613c, 614, 614a, 614b, 614c, 615, 615a, 616, 616a, 617, 617a, 618, 618a, 619, 619a, 620, 620a, 621, 621a, 621b, 622, 622a, 623, 623a, 624, 624a, 624b, 625, 625a, 626, 626a, 627, 627a, 628, 628a, 628b, 628c, 629, 629a, 630, 630a, 632, 632a, 633, 633a, 634, 634a, 635, 635a, 636, 636a, 637, 637a, 637b, 638, 638a, 639, 639a, 640, 640a, 641, 641a, 641b, 641c, 642, 642a, 643, 643a, 643b, 644, 644a, 645, 645a, 645b, 646, 646a, 647, 647a, 647b, 648, 648a, 648b, 649, 649a, 649b, 650, 650a, 650b, 650c, 650d, 651, 651a, 651b, 652, 652a, 652b, 652c, 653, 653a, 653b, 654, 654a, 654b, 654c, 654d, 655, 655a, 656, 656a, 657, 657a, 658, 658a, 659, 659a, 660, 660a, 660b, 661, 661a, 661b, 662, 662a, 662b, 662c, 662d, 665, 665a, 665b, 666, 666a, 667, 667a, 667b, 668, 668a, 668b, 668c, 668d, 669, 669a, 670, 670a, 671, 671a, 671b, 672, 672a, 673, 673a, 674, 674a, 675, 675a, 676, 676a, 677, 677a, 678, 678a, 679, 679a, 680, 680a, 680b, 680c, 681, 681a, 682, 682a, 683, 683a, 684, 684a, 685, 685a, 686, 686a, 687, 687a, 688, 688a, 689, 689a, 689b, 690, 690a, 691, 691a, 692, 692a, 693, 693a, 694, 694a, 695, 695a, 696, 696a, 697, 697a, 698, 698a, 699, 699a, 700, 700a, 701, 701a, 702, 702a, 702b, 703, 703a, 704, 704a, 705, 705a, 707, 707a, 708, 708a, 709, 709a, 710, 710a, 711, 711a, 712, 712a, 713, 713a, 714, 714a, 715, 715a, 716, 716a, 717, 717a, 718, 718a, 719, 719a, 720, 720a, 721, 721a, 722, 722a, 723, 723a, 724, 724a, 725, 725a, 726, 726a, 727, 727a, 728, 728a, 729, 729a, 729b, 730, 730a, 731, 731a, 732, 732a, 733, 733a, 734, 734a, 735, 735a, 736, 736a, 737, 737a, 738, 738a, 739, 739a, 740, 740a, 741, 741a, 741b, 742, 742a, 743, 743a, 744, 744a, 745, 745a, 745b, 746, 746a, 747, 747a, 748, 748a, 749,** and **749a** as depicted in **Table C3,** or a pharmaceutically acceptable salt thereof.

**Embodiment 130.** The compound of Embodiment 1, wherein the compound of Formula (**II**) is other than Compound Nos. **R139, R139a, R139b, R139c, R158, R158a, R158b, R158c, R160, R160a, R161, R161a, R161b, R161c, R164, R164a, R164b, R170, R170a, R171, R171a, R176, R176a, R176b, R176c, R176d, R178, R178a, R178b, R179, R179a, R179b, R179d, R179e, R179f, R180, R180a, R181,** and **R181a** as depicted in **Table C1**, or a pharmaceutically acceptable salt thereof.

**Embodiment 131.** The compound of Embodiment 4, wherein the compound of Formula (**III**) is other than Compound Nos. **R158, R158a, R158b, R158c, R161, R161a, R161b, R161c, R176, R176a, R176b, R176c, R176d, R177, R177a, R178, R178a, R178b, R179, R179a, R179b, R179d, R179e, R179f, R180,** and **R180a** as depicted in **Table C1**, or a pharmaceutically acceptable salt thereof.

**Embodiment 132.** The compound of Embodiment 16, wherein the compound of Formula (**IV**) is other than Compound Nos. **R124, R124a, R124b, R124c, R124d, R124e, R124f, R125, R125a, R130, R130a, R131, R131a, R133, R133a, R133b, R134, R134a, R138, R138a, R148, R148a, R162, R162a, R163, R163a, R175,** and **R175a** as depicted in **Table C1,** or a pharmaceutically acceptable salt thereof.

**Embodiment 133.** A pharmaceutical composition comprising a compound of any one of Embodiments 1-132, or a pharmaceutically acceptable salt thereof, and a pharmaceutically acceptable excipient.

**Embodiment 134.** A dysregulated KRas protein non-covalently bound with a compound of any one of Embodiments 1-132, or a pharmaceutically acceptable salt thereof.

**Embodiment 135.** A method for treating a KRas-associated cancer in a subject in need thereof, the method comprising administering to the subject a therapeutically effective amount of a compound of any one of Embodiments 1-132, or a pharmaceutically acceptable salt thereof, or a pharmaceutical composition according to Embodiment 133.

**Embodiment 136.** A method for treating a KRas-associated cancer in a subject in need thereof, the method comprising (a) determining that the cancer in the subject has a KRas dysregulation; and (b) administering to the subject a therapeutically effective amount of a compound of any one of Embodiments 1-132, or a pharmaceutically acceptable salt thereof, or a pharmaceutical composition according to Embodiment 133.

**Embodiment 137.** A method of treating a KRas-associated cancer in a subject, the method comprising administering to a subject identified or diagnosed as having a cancer having a KRas dysregulation a therapeutically effective amount of a compound of any one of Embodiments 1-132 or a pharmaceutically acceptable salt thereof, or a pharmaceutical composition according to Embodiment 133.

**Embodiment 138.** A method of treating a KRas-associated cancer in a subject, the method comprising:
**(a)** determining that the cancer in the subject has a KRas dysregulation; and
**(b)** administering to the subject a therapeutically effective amount of a compound of any one of Embodiments 1-132 or a pharmaceutically acceptable salt thereof, or a pharmaceutical composition according to Embodiment 133.

**Embodiment 139.** The method of any one of Embodiments 135-138, wherein the KRas-associated cancer is a mutant KRas-associated cancer.

**Embodiment 140.** The method of Embodiment 139, wherein the mutant KRas-associated cancer is a KRas G12A-associated cancer, a KRas G12C-associated cancer, a KRas G12D-associated cancer, a KRas G12R-associated cancer, a KRas G12S-associated cancer, or a KRas G12V-associated cancer.

**Embodiment 141.** The method of Embodiment 139, wherein the mutant KRas-associated cancer is a KRas G12D-associated cancer or a KRas G12V-associated cancer.

**Embodiment 142.** The method of Embodiment 139, wherein the mutant KRas-associated cancer is a KRas G12D-associated cancer.

**Embodiment 143.** The method of Embodiment 139, wherein the mutant KRas-associated cancer is a KRas G12R-associated cancer.

**Embodiment 144.** The method of Embodiment 139, wherein the mutant KRas-associated cancer is a KRas G12V-associated cancer.

**Embodiment 145.** The method of any one of Embodiments 136 or 138, wherein the step of determining that the cancer in the subject has a KRas dysregulation includes performing an assay to detect the KRas dysregulation (e.g., a KRas mutation) in a tumor sample from the subject.

**Embodiment 146.** The method of Embodiment 145, wherein detecting the KRas dysregulation includes detecting a *KRAS* gene having a mutation corresponding to a substitution of glycine 12 in a KRas protein and/or a KRas protein having a substitution of glycine 12.

**Embodiment 147.** The method of Embodiment 146, wherein the substitution of glycine 12 is a substitution to alanine, cysteine, aspartic acid, arginine, serine, or valine.

**Embodiment 148.** The method of Embodiment 147, wherein the substitution of glycine 12 is a substitution to aspartic acid.

**Embodiment 149.** The method of Embodiment 147, wherein the substitution of glycine 12 is a substitution to arginine.

**Embodiment 150.** The method of Embodiment 147, wherein the substitution of glycine 12 is a substitution to valine.

**Embodiment 151.** The method of any one of Embodiments 145-150, comprising obtaining a tumor sample from the subject.

**Embodiment 152.** The method of Embodiment 151, wherein the tumor sample is a biopsy sample.

**Embodiment 153.** The method of any one of Embodiments 145-152, wherein the assay is selected from the group consisting of sequencing, immunohistochemistry, and enzyme-linked immunosorbent assay, and fluorescence in situ hybridization (FISH).

**Embodiment 154.** The method of Embodiment 153, wherein the sequencing is pyrosequencing or next generation sequencing.

**Embodiment 155.** The method of any one of Embodiments 135-154, wherein the KRas-associated cancer is selected from the group consisting of: a hematological cancer, a soft tissue cancer, bile duct cancer, bladder cancer, brain cancer, breast cancer, cervical cancer, colon cancer, endometrial cancer, esophageal cancer, kidney cancer, liver cancer, lung cancer, mucinous carcinoma, ovarian cancer, pancreatic cancer, prostate cancer, rectal cancer, skin cancer, stomach cancer, testicular cancer, thymus cancer, thyroid cancer, urothelial cancer, uterine cancer, and a combination thereof.

**Embodiment 156.** The method of Embodiment 155, wherein the KRas-associated cancer is pancreatic cancer.

**Embodiment 157.** The method of Embodiment 155, wherein the KRas-associated cancer is selected from the group consisting of: a hematological cancer, brain cancer, cervical cancer, colon cancer, endometrial cancer, esophageal cancer, kidney cancer, liver cancer, lung cancer, mucinous carcinoma, pancreatic cancer, prostate cancer, rectal cancer, skin cancer, stomach cancer, thymus cancer, urothelial cancer, and uterine cancer.

**Embodiment 158.** The method of Embodiment 155, wherein the KRas-associated cancer is selected from the group consisting of: a hematological cancer, bladder cancer, bile duct cancer, brain cancer, breast cancer, cervical cancer, colon cancer, endometrial cancer, esophageal cancer, kidney cancer, liver cancer, lung cancer, mucinous carcinoma, ovarian cancer, pancreatic cancer, rectal cancer, skin cancer, stomach cancer, testicular cancer (e.g., seminoma), thymus cancer, and uterine cancer.

**Embodiment 159.** The method of Embodiment 155, wherein the KRas-associated cancer is selected from the group consisting of: bladder cancer, breast cancer, cervical cancer, colorectal cancer, endometrial cancer, esophageal or stomach cancer, leukemia, lung cancer (e.g., NSCLC), pancreatic cancer, and kidney cancer.

**Embodiment 160.** The method of any one of Embodiments 135-159, comprising administering an additional therapy or therapeutic agent to the subject.

**Embodiment 161.** The method of Embodiment 160, wherein the additional therapy or therapeutic agent is selected from the group consisting of Ras pathway targeted therapeutic agents, kinase-targeted therapeutics, mTORC1 inhibitors or degraders, YAP inhibitors or degraders, proteasome inhibitors or degraders, HSP90 inhibitors or degraders, farnesyl transferase inhibitors or degraders, PTEN inhibitors or degraders, signal transduction pathway inhibitors or degraders, checkpoint inhibitors, modulators of the apoptosis pathway, chemotherapeutics, angiogenesis-targeted therapies, immune-targeted agents, radiotherapy, and combinations thereof.

**Embodiment 162.** A method for a method for modulating KRas protein activity in a mammalian cell, the method comprising contacting the mammalian cell with an effective amount of a compound of any one of Embodiments 1-132, or a pharmaceutically acceptable salt thereof.

**Embodiment 163.** The method of Embodiment 162, wherein the contacting occurs in vivo.

**Embodiment 164.** The method of Embodiment 162, wherein the contacting occurs in vitro.

**Embodiment 165.** The method of Embodiment 162, wherein the contacting occurs ex vivo.

**Embodiment 166.** The method of any one of Embodiments 162-165, wherein the mammalian cell is a mammalian cancer cell.

**Embodiment 167.** The method of any one of Embodiments 162-165, wherein the KRas protein is a mutant KRas protein.

**Embodiment 168.** The method of Embodiment 167, wherein the mutant KRas protein is a mutant KRas protein selected from the group consisting of: a KRas G12A mutant protein, a KRas G12C mutant protein, a KRas G12D mutant protein, a KRas G12R mutant protein, a KRas G12S mutant protein, and a KRas G12V mutant protein.

### FORMULA (II-a) EXEMPLARY EMBODIMENTS

**Embodiment 1.** A compound of Formula (**II-a**): 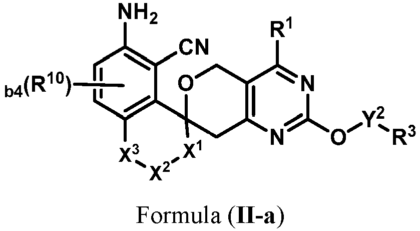 or a pharmaceutically acceptable salt thereof, wherein:
**R¹** is a 4-10 membered heterocyclyl optionally substituted with 1-4 **R⁷**;
each **R⁷** is independently selected from the group consisting of **R^{a}** and **R^{b}**;
**X¹** is CH₂;
**X²** and **X³** are independently selected from the group consisting of: CH₂, CH**R^{L}**, C(**R^{L}**)₂,
provided that 1-2 of **X²** and **X³** is independently CH**R^{L}** or C(**R^{L}**)₂;
**b4** is 0;
each **R¹⁰** is independently selected from the group consisting of: -Cl, -F, -CN, and C₁₋₃ alkyl optionally substituted with 1-3 **R^{c}**;
**R^{L}** is selected from the group consisting of C₁₋₃ alkoxy, -F, CN, and C₁₋₃ alkyl optionally substituted with 1-3 **R^{c}**;
**Y²** is -CH₂-; and
**R³** is 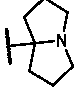 optionally substituted with 1-2 substituents each independently selected from the group consisting of: -F, -C₁₋₃ alkoxy, and -C₁₋₃ haloalkoxy;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   **(f)** -C₁₋₆ haloalkoxy;
   **(g)** -N**R^{d}R^{e}**;
   **(h)** C(=O)C₁₋₆ alkyl;
   **(i)** C(=O)C₁₋₆ haloalkyl;
   **(j)** C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   **(o)** S(O)₀₋₂(C₁₋₆ haloalkyl);
   **(p)** S(O)₁₋₂N(**R^{f}**)₂; and
   **(q)** C₁₋₆ alkyl, C₂₋₆ alkenyl, or C₂₋₆ alkynyl, each optionally substituted with 1-6 **R^{c}**;
each **R^{b}** is independently selected from the group consisting of: **-(L^{b})_{b}-R^{b1}** and -**R^{b1}**, wherein:
   **b** is 1, 2, or 3;
   each -**L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e}**, C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)₁₋₂N(**R^{f}**)₂;
   each **R^{d}** and **R^{c}** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂-.

**Embodiment 2.** The compound of any one of Embodiment 1, wherein **X¹** is CH₂; **X²** is CH₂; and **X³** is CH**R^{L}** (e.g., **X³** is C(H)CH₃).

**Embodiment 3.** The compound of Embodiment 1, wherein each **R**^{L} is independently selected from the group consisting of: CH₃, CF₃, CHF₂, and CH₂F (e.g., each **R^{L}** is CH₃).

**Embodiment 4.** The compound of any one of Embodiments 1-3, wherein **R³** is 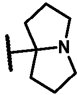 optionally substituted with 1-2 -F.

**Embodiment 5.** The compound of any one of Embodiments 1-4, wherein **R³** is 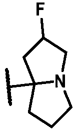 (e.g., 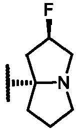 ).

**Embodiment 6.** The compound of any one of Embodiments 1-4, wherein **R³** is

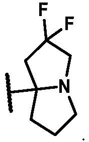

**Embodiment 7.** The compound of any one of Embodiments 1-3, wherein **R³** is

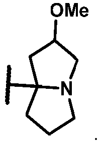

**Embodiment 8.** The compound of any one of Embodiments 1-7, wherein each **R⁷** is independently selected from the group consisting of: halo; cyano; -OH; oxo; -C₁₋₆ alkoxy; C(=O)N(**R^{f}**)₂; **R^{b1}**; -(C₁₋₃ alkylene)-**R^{b1}**; -O-**R^{b1}**; and C₁₋₆ alkyl optionally substituted with 1-3 **R^{c7}**, wherein:
each **R^{b1}** is independently selected from the group consisting of: C₃₋₆ cycloalkyl, phenyl, 4-6 membered heterocyclyl, and 5-6 membered heteroaryl, each of which is optionally substituted with 1-3 **R^{g}** (e.g., each **R^{g}** is independently selected from the group consisting of: halo and C₁₋₃ alkyl); and
each **R^{c7}** is independently selected from the group consisting of: halo, cyano, -OH, and -C₁₋₆ alkoxy.

**Embodiment 9.** The compound of any one of Embodiments 1-8, wherein **R¹** is a 7-10 (e.g., 7) membered heterocyclyl having one ring nitrogen atom, one ring oxygen atom, and no additional ring heteroatoms, wherein the 7-10 membered heterocyclyl is optionally substituted with 1-4 (e.g., 1-2) **R⁷**.

**Embodiment 10.** The compound of any one of Embodiments 1-9, wherein **R¹** is a 7-10 (e.g., 7) membered monocyclic heterocyclyl having one ring nitrogen atom, one ring oxygen atom, and no additional ring heteroatoms, wherein the 7-10 membered monocyclic heterocyclyl is optionally substituted with 1-4 (e.g., 1-2) **R⁷**.

**Embodiment 11.** The compound of any one of Embodiments 1-10, wherein **R¹** is 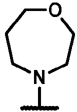 optionally substituted with 1-4 **R⁷** at one or more ring carbon atoms.

**Embodiment 12.** The compound of any one of Embodiments 1-11, wherein **R¹** is 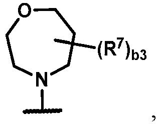 wherein **b3** is 1, 2, or 3.

**Embodiment 13.** The compound of Embodiment 12, wherein one occurrence of **R⁷** is **R^{b}** (e.g., one occurrence of **R⁷** is **R^{b1}**).

**Embodiment 14.** The compound of Embodiment 12 or 13, wherein one occurrence **of R⁷** is a 5-6 membered heteroaryl optionally substituted with 1-3 **R^{g}**.

**Embodiment 15.** The compound of any one of Embodiments 12-14, wherein one occurrence **of R⁷** is a 5-membered heteroaryl optionally substituted with 1-3 **R^{g}**.

**Embodiment 16.** The compound of any one of Embodiments 12-15, wherein one occurrence of **R⁷** is selected from the group consisting of pyrazolyl and oxazolyl, each of which is optionally substituted with 1-2 **R^{g}** (e.g., **R⁷** is

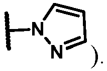

**Embodiment 17.** The compound of any one of Embodiments 12-16, wherein **b3** is 1.

**Embodiment 18.** The compound of any one of Embodiments 1-8 or 12-13, wherein **R¹** is 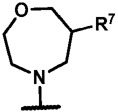 (e.g., 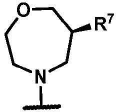 ), wherein **R⁷** is a 5-membered heteroaryl optionally substituted with 1-3 **R^{g}**.

**Embodiment 19.** The compound of Embodiment 19, wherein **R⁷** is selected from the group consisting of pyrazolyl and oxazolyl, each of which is optionally substituted with 1-2 **R^{g}**.

**Embodiment 20.** The compound of Embodiment 19 or 20, wherein **R⁷** is pyrazolyl optionally substituted with 1-2 **R^{g}** (e.g., **R⁷** is 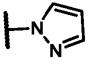 optionally substituted with one **R^{g}**); or wherein **R⁷** is oxazolyl optionally substituted with one **R^{g}** (e.g., **R⁷** is 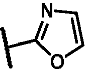 optionally substituted with one **R^{g}**).

**Embodiment 21.** The compound of any one of Embodiments 19-21, wherein **R⁷** is

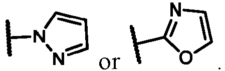

**Embodiment 22.** The compound of any one of Embodiments 1-8, wherein **R¹** is a 7-10 (e.g., 7) membered bicyclic heterocyclyl having one ring nitrogen atom, one ring oxygen atom, and no additional ring heteroatoms, wherein the 7-10 membered bicyclic heterocyclyl is optionally substituted with 1-4 (e.g., 1-2) **R⁷**.

**Embodiment 23.** The compound of any one of Embodiments 1-8 or 22, wherein **R¹** is a 7-10 (e.g., 7; e.g., 9) membered spirocyclic bicyclic heterocyclyl having one ring nitrogen atom, one ring oxygen atom, and no additional ring heteroatoms, wherein the 7-10 membered spirocyclic bicyclic heterocyclyl is optionally substituted with 1-4 (e.g., 1-2) **R⁷**.

**Embodiment 24.** The compound of any one of Embodiments 1-8 or 22-23, wherein **R¹** is 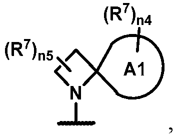 wherein: **Ring A1** is a 4-7 membered heterocyclyl ring having one ring oxygen atom and no additional ring heteroatoms; **n4** is 0, 1, or 2; and **n5** is 0, 1, or 2, provided that **n4** + **n5** is 0, 1, or 2.

**Embodiment 25.** The compound of any one of Embodiments 1-8 or 22-24, wherein **R¹** is 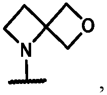 which is optionally substituted with 1-2 **R⁷**.

**Embodiment 26.** The compound of any one of Embodiments 1-8 or 22-25, wherein **R¹** is

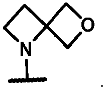

**Embodiment 27.** The compound of any one of Embodiments 1-8 or 22-24, wherein **R¹** is 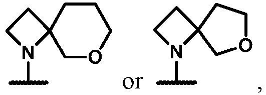 each of which is optionally substituted with 1-2 **R⁷**.

**Embodiment 28.** The compound of any one of Embodiments 1-8, 22-24, or 27, wherein **R¹** is 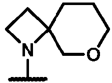 (e.g., 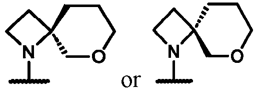 ) or 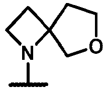 (e.g., 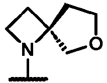 or 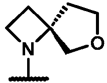 ).

**Embodiment 29.** The compound of any one of Embodiments 1-8, wherein **R¹** is a 4-7 membered heterocyclyl optionally substituted with 1-4 (e.g., 1-2) **R⁷**.

**Embodiment 30.** The compound of any one of Embodiments 1-8 or 29, wherein **R¹ is** a 4-5 membered heterocyclyl (e.g., azetidinyl or pyrrolidinyl) optionally substituted with 1-4 (e.g., 1-2) **R⁷**.

**Embodiment 31.** The compound of any one of Embodiments 1-8 or 29-30, wherein **R¹** is a 4-membered heterocyclyl optionally substituted with 1-4 (e.g., 1-2) **R⁷**.

**Embodiment 32.** The compound of any one of Embodiments 1-8 or 29-31, wherein **R¹** is 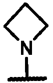 optionally substituted with 1-4 (e.g., 1-2) **R⁷**.

**Embodiment 33.** The compound of any one of Embodiments 1-8 or 29-32, wherein **R¹** is selected from the group consisting of:

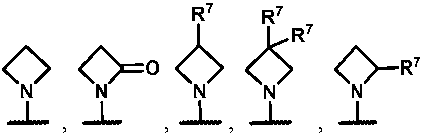

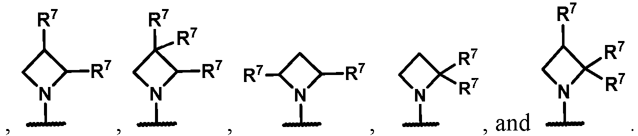

**Embodiment 34.** The compound of any one of Embodiments 1-8 or 29-33, wherein **R¹** is 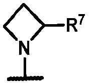 (e.g., 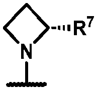 ), 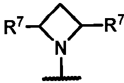 (e.g., 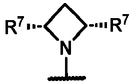 ), or

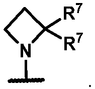

**Embodiment 35.** The compound of any one of Embodiments 29-34, wherein each **R⁷** is independently selected from the group consisting of: halo; cyano; -OH; oxo; -C₁₋₆ alkoxy; C(=O)N(**R^{f}**)₂; **R^{b1}**; -(C₁₋₃ alkylene)-**R^{b1}**; -O-**R^{b1}**; and C₁₋₆ alkyl optionally substituted with 1-3 **R^{c7}**, wherein:
each **R^{b1}** is independently selected from the group consisting of: C₃₋₆ cycloalkyl, phenyl, 4-6 membered heterocyclyl, and 5-6 membered heteroaryl, each of which is optionally substituted with 1-3 **R^{g}** (e.g., each **R^{g}** is independently selected from the group consisting of: halo and C₁₋₃ alkyl); and
each **R^{c7}** is independently selected from the group consisting of: halo, cyano, -OH, and -C₁₋₆ alkoxy.

**Embodiment 36.** The compound of any one of Embodiments 29-35, wherein each **R⁷** is independently selected from the group consisting of:
-F;
-cyano;
-OH;
-**R^{b1}**, wherein the **R^{b1}** is a 5-6 membered heteroaryl optionally substituted with 1-2 **R^{g}**,
C₁₋₃ alkyl optionally substituted with 1-3 F;
C₁₋₃ alkyl substituted with -OH or C₁₋₃ alkoxy; and
C(=O)N(**R^{f}**)₂,

**Embodiment 37.** The compound of Embodiment 35 or 36, wherein at least one **R⁷** is C₁₋₃ alkyl substituted with -OH.

**Embodiment 38.** The compound of any one of Embodiments 1-8, wherein **R¹** is 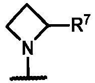 (e.g., 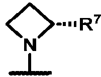 ), wherein **R⁷** is C₁₋₃ alkyl substituted with -OH or C₁₋₃ alkoxy (e.g., **R⁷** is C₁₋₃ alkyl substituted with -OH).

**Embodiment 39.** The compound of any one of Embodiments 1-8 or 38, wherein **R¹** is 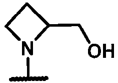 (e.g., 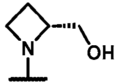 ),

**Embodiment 40.** The compound of any one of Embodiments 1-8, wherein **R¹** is 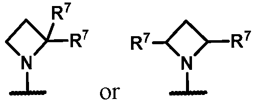 (e.g., 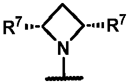 ), wherein each **R⁷** is independently selected from the group consisting of:
C₁₋₃ alkyl optionally substituted with 1-3 F; and
C₁₋₃ alkyl substituted with -OH or C₁₋₃ alkoxy.

**Embodiment 41.** The compound of any one of Embodiments 1-8 or 40, wherein **R¹** is 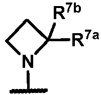 (e.g., 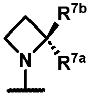 ) or 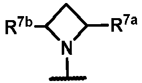 (e.g., 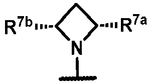 ), wherein **R^{7a}** is C₁₋₃ alkyl substituted with -OH (e.g., -CH₂OH); and
**R^{7b}** is selected from the group consisting of:
C₁₋₃ alkyl optionally substituted with 1-3 F (e.g., methyl), and
C₁₋₃ alkyl substituted with -OH or C₁₋₃ alkoxy.

**Embodiment 42.** The compound of any one of Embodiments 1-8 or 40-41, wherein **R¹** is 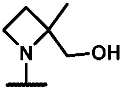 (e.g., 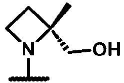 ),

**Embodiment 43.** The compound of any one of Embodiments 1-8 or 40-41, wherein **R¹** is 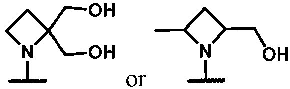 (e.g., 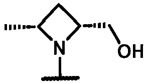 ). 2.

**Embodiment 44.** The compound of Embodiment 1, wherein the compound of Formula **(II-a)** is a compound of Formula **(II-a4), (II-a5),** or **(II-a8):** 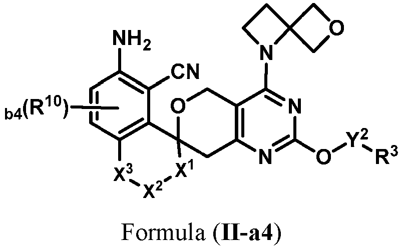 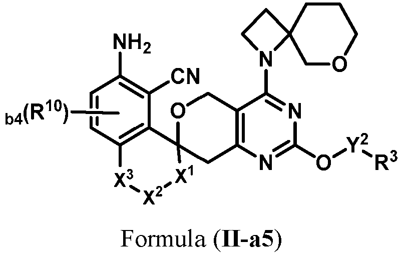 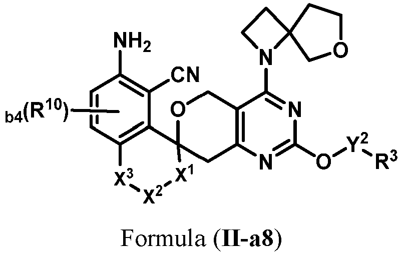 or a pharmaceutically acceptable salt thereof, wherein:
**b4** is 0;
each **R¹⁰** is independently selected from the group consisting of: -Cl, -F, -CN, and C₁₋₃ alkyl optionally substituted with 1-3 **R^{c}**;
**X¹** is CH₂;
**X²** is CH₂; and
**X³** is CH**R^{L}**.

**Embodiment 45.** The compound of Embodiment 1, wherein the compound of Formula **(II-a)** is a compound of Formula **(II-a6)** or **(II-a7):** 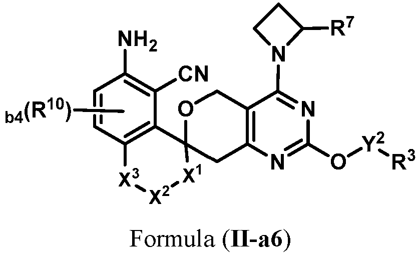 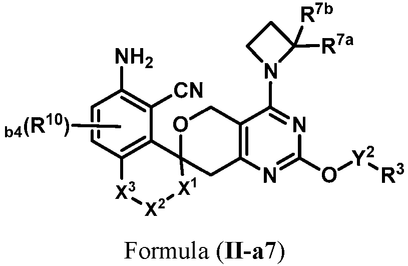 or a pharmaceutically acceptable salt thereof, wherein:
**R⁷** is C₁₋₃ alkyl substituted with -OH or C₁₋₃ alkoxy;
**R^{7a}** is C₁₋₃ alkyl substituted with -OH (e.g., -CH₂OH);
**R^{7b}** is selected from the group consisting of:
   C₁₋₃ alkyl optionally substituted with 1-3 F (e.g., methyl), and
   C₁₋₃ alkyl substituted with -OH or C₁₋₃ alkoxy;
   **b4** is 0;
   each **R¹⁰** is independently selected from the group consisting of: -Cl, -F, -CN, and C₁₋₃ alkyl optionally substituted with 1-3 **R^{c}**;
   **X¹** is CH₂;
   **X²** is CH₂;
   **X³** is CH**R^{L}**.

**Embodiment 46.** The compound of Embodiment 45, wherein the 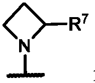 moiety is

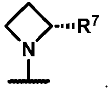

**Embodiment 47.** The compound of Embodiment 45 or 46, wherein **R⁷** is C₁₋₃ alkyl substituted with -OH.

**Embodiment 48.** The compound of any one of Embodiments 45-47, wherein **R⁷** is -CH₂OH.

**Embodiment 49.** The compound of Embodiment 45, wherein the 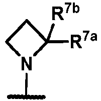 moiety is

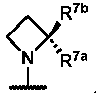

**Embodiment 50.** The compound of Embodiment 45 or 49, wherein **R^{7a}** is - CH₂OH.

**Embodiment 51.** The compound of any one of Embodiments 45 or 50-51, wherein **R^{7b}** is C₁₋₃ alkyl optionally substituted with 1-3 -F.

**Embodiment 52.** The compound of Embodiment 51, wherein **R^{7b}** is methyl.

**Embodiment 53.** The compound of any one of Embodiments 44-52, wherein **R³** is 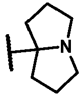 optionally substituted with 1-2 -F (e.g., **R³** is 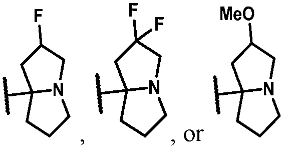 ).

**Embodiment 54.** The compound of any one of Embodiments 44-53, wherein **R³** is 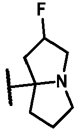 (e.g., 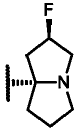 **).**

**Embodiment 55.** The compound of any one of Embodiments 1-54, wherein the 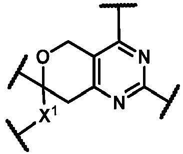 moiety is

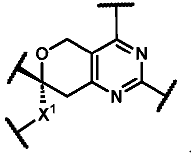

**Embodiment 56.** The compound of Embodiment 1, wherein the compound of Formula **(II-a)** is selected from the group consisting of Compound Nos. **502, 502a, 502b, 511, 511a, 512, 512a, 512b, 532, 532a, 553, 553a, 554, 554a, 554b, 554c, 555, 555a, 556, 556a, 556b, 560, 560a, 561, 561a, 566, 566a, 566b, 567, 567a, 567b, 568, 568a, 568b, 569, 569a, 570, 570a, 571, 571a, 572, 572a, 573, 573a, 575, 575a, 575b, 576, 576a, 576b, 576c, 576d, 577, 577a, 578, 578a, 578b, 581, 581a, 582, 582a, 583, 583a, 585, 585a, 585b, 589, 589a, 592, 592a, 595, 595a, 595b, 597, 597a, 597b, 598, 598a, 598b, 598c, 599, 599a, 599b, 600, 600a, 600b, 601, 601a, 606, 606a, 606b, 607, 607a, 608, 608a, 608b, 609, 609a, 609b, 610, 610a, 610b, 611, 611a, 611b, 612, 612a, 612b, 613, 613a, 613b, 613c, 618, 618a, 619, 619a, 620, 620a, 621, 621a, 621b, 622, 622a, 623, 623a, 624, 624a, 624b, 625, 625a, 626, 626a, 627, 627a, 633, 633a, 634, 634a, 635, 635a, 636, 636a, 637, 637a, 637b, 638, 638a, 639, 639a, 640, 640a, 640b, 642, 642a, 643, 643a, 643b, 644, 644a, 645, 645a, 645b, 646, 646a, 647, 647a, 647b, 648, 648a, 648b, 649, 649a, 649b, 650, 650a, 650b, 650c, 650d, 651, 651a, 651b, 652, 652a, 652b, 652c, 653, 653a, 653b, 654, 654a, 654b, 654c, 654d, 655, 655a, 656, 656a, 657, 657a, 658, 658a, 659, 659a, 660, 660a, 660b, 661, 661a, 661b, 662, 662a, 662b, 662c, 662d, 665, 665a, 665b, 666, 666a, 667, 667a, 667b, 668, 668a, 668b, 668c, 668d, 669, 669a, 670, 670a, 671, 671a, 671b, 672, 672a, 673, 673a, 674, 674a, 675, 675a, 676, 676a, 677, 677a, 678, 678a, 679, 679a, 680, 680a, 680b, 680c, 681, 681a, 682, 682a, 683, 683a, 684, 684a, 685, 685a, 686, 686a, 687, 687a, 688, 688a, 689, 689a, 689b, 690, 690a, 691, 691a, 692, 692a, 693, 693a, 694, 694a, 695, 695a, 696, 696a, 697, 697a, 698, 698a, 699, 699a, 700, 700a, 701, 701a, 702, 702a, 702b, 703, 703a, 704, 704a, 705, 705a, 707, 707a, 708, 708a, 709, 709a, 710, 710a, 711, 711a, 712, 712a, 713, 713a, 714, 714a, 715, 715a, 716, 716a, 717, 717a, 718, 718a, 719, 719a, 720, 720a, 721, 721a, 722, 722a, 723, 723a, 724, 724a, 725, 725a, 726, 726a, 727, 727a, 728, 728a, 729, 729a, 729b, 730, 730a, 731, 731a, 732, 732a, 733, 733a, 734, 734a, 735, 735a, 736, 736a, 737, 737a, 737b, 738, 738a, 739, 739a, 740, 740a, 741, 741a, 741b, 742, 742a, 743, 743a, 744, 744a, 745, 745a, 745b, 746, 746a, 747, 747a, 747b, 748, 748a, 749, 749a, 750, 750a, 752, 752a, 753, 753a, 754,** and **754a,** as depicted in **Table C3,** or a pharmaceutically acceptable salt thereof.

**Embodiment 57.** The compound of Embodiment 1, wherein the compound of Formula **(II-a)** is other than Compound Nos. **R179, R179a, R179b, R179d, R179e,** and **R179f,** as depicted in **Table C1,** or a pharmaceutically acceptable salt thereof.

**Embodiment 58.** A pharmaceutical composition comprising a compound of any one of Embodiments 1-57, or a pharmaceutically acceptable salt thereof, and a pharmaceutically acceptable excipient.

**Embodiment 59.** A method for treating cancer in a subject, the method comprising administering to a subject identified or diagnosed as having a cancer having a KRas dysregulation a therapeutically effective amount of a compound of any one of Embodiments 1-57, or a pharmaceutically acceptable salt thereof, or a pharmaceutical composition according to Embodiment 58.

**Embodiment 60.** A method for treating cancer in a subject, the method comprising (a) determining that the cancer in the subject has a KRas dysregulation; and (b) administering to the subject a therapeutically effective amount of a compound of any one of Embodiments 1-57, or a pharmaceutically acceptable salt thereof, or a pharmaceutical composition according to Embodiment 58.

**Embodiment 61.** The method of Embodiment 59 or 60, wherein the KRas dysregulation cancer is a KRas mutation.

**Embodiment 62.** The method of Embodiment 61, wherein the KRas mutation is a KRas G12A mutation, a KRas G12C mutation, a KRas G12D mutation, a KRas G12R mutation, a KRas G12S mutation, or a KRas G12V mutation.

**Embodiment 63.** The method of Embodiment 62, wherein the KRas mutation is a KRas G12C mutation, a KRas G12D mutation or a KRas G12V mutation.

**Embodiment 64.** The method of Embodiment 60, wherein the step of determining that the cancer in the subject has a KRas dysregulation includes performing an assay to detect the KRas dysregulation (e.g., a KRas mutation) in a tumor sample from the subject.

**Embodiment 65.** The method of Embodiment 64, wherein detecting the KRas dysregulation includes detecting a *KRAS* gene having a mutation corresponding to a substitution of glycine 12 in a KRas protein and/or a KRas protein having a substitution of glycine 12.

**Embodiment 66.** The method of Embodiment 65, wherein the substitution of glycine 12 is a substitution to alanine, cysteine, aspartic acid, arginine, serine, or valine.

**Embodiment 67.** The method of any one of Embodiments 59-66, wherein the cancer is selected from the group consisting of: a hematological cancer, a soft tissue cancer, bile duct cancer, bladder cancer, brain cancer, breast cancer, cervical cancer, colon cancer, endometrial cancer, esophageal cancer, kidney cancer, liver cancer, lung cancer, mucinous carcinoma, ovarian cancer, pancreatic cancer, prostate cancer, rectal cancer, skin cancer, stomach cancer, testicular cancer, thymus cancer, thyroid cancer, urothelial cancer, uterine cancer, and a combination thereof.

**Embodiment 68.** The method of Embodiment 67, wherein the cancer is selected from the group consisting of: colon cancer, endometrial cancer, lung cancer, pancreatic cancer, and uterine cancer.

**Embodiment 69.** The method of any one of Embodiments 59-68, comprising administering an additional therapy or therapeutic agent to the subject.

**Embodiment 70.** The method of Embodiment 69, wherein the additional therapy or therapeutic agent is selected from the group consisting of Ras pathway targeted therapeutic agents, kinase-targeted therapeutics, mTORC1 inhibitors or degraders, YAP inhibitors or degraders, proteasome inhibitors or degraders, HSP90 inhibitors or degraders, farnesyl transferase inhibitors or degraders, PTEN inhibitors or degraders, signal transduction pathway inhibitors or degraders, checkpoint inhibitors, modulators of the apoptosis pathway, chemotherapeutics, angiogenesis-targeted therapies, immune-targeted agents, radiotherapy, and combinations thereof.

### FORMULA (II-a) AND (IV-b) EXEMPLARY EMBODIMENTS

**Embodiment 1.** A compound of Formula (II-a): 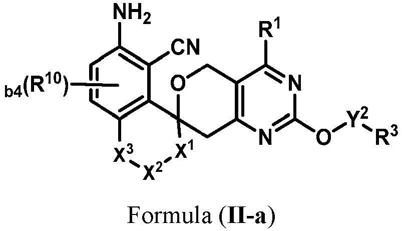 or a pharmaceutically acceptable salt thereof, wherein:
**R¹** is 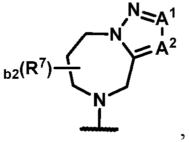 wherein **b2** is 0, 1, 2, or 3; and **A¹** and **A²** are independently selected from the group consisting of: N, CH, and C**R⁷**;
each **R⁷** is independently selected from the group consisting of **R^{a}** and **R^{b}**;
**X¹** is CH₂;
**X²** and **X³** are independently selected from the group consisting of: CH₂, CH**R^{L}**, C(**R^{L}**)₂, provided that 1-2 of **X²** and **X³** is independently CH**R^{L}** or C(**R^{L}**)₂;
**b4** is 0;
each **R¹⁰** is independently selected from the group consisting of: -Cl, -F, -CN, and C₁₋₃ alkyl optionally substituted with 1-3 **R^{c}**;
**R^{L}** is selected from the group consisting of C₁₋₃ alkoxy, -F, CN, and C₁₋₃ alkyl optionally substituted with 1-3 **R^{c}**;
**Y²** is -CH₂-; and
**R³** is 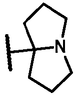 optionally substituted with 1-2 substituents each independently selected from the group consisting of: -F, -C₁₋₃ alkoxy, and -C₁₋₃ haloalkoxy;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   **(f)** -C₁₋₆ haloalkoxy;
   **(g)** -N**R^{d}R^{e}**;
   **(h)** C(=O)C₁₋₆ alkyl;
   **(i)** C(=O)C₁₋₆ haloalkyl;
   **(j)** C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   **(o)** S(O)₀₋₂(C₁₋₆ haloalkyl);
   **(p)** S(O)₁₋₂N(**R^{f}**)₂; and
   **(q)** C₁₋₆ alkyl, C₂₋₆ alkenyl, or C₂₋₆ alkynyl, each optionally substituted with 1-6 **R^{c}**;
each **R^{b}** is independently selected from the group consisting of: **-(L^{b})_{b}-R^{b1}** and -**R^{b1}**, wherein:
   **b** is 1, 2, or 3;
   each **-L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e}**, C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)₁₋₂N(**R^{f}**)₂;
   each **R^{d}** and **R^{e}** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂-.

**Embodiment 2.** The compound of Embodiment 2, wherein **X¹** is CH₂; **X²** is CH₂; and **X³** is CH**R^{L}** (e.g., **X³** is C(H)CH₃).

**Embodiment 3.** The compound of Embodiment 1 or 2, wherein each **R^{L}** is independently selected from the group consisting of: CH₃, CF₃, CHF₂, and CH₂F (e.g., each **R^{L}** is CH₃).

**Embodiment 4.** A compound of Formula **(IV-b):** 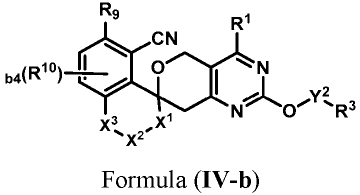 or a pharmaceutically acceptable salt thereof, wherein:
**R¹** is 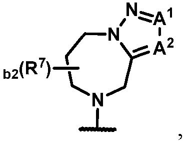 wherein **b2** is 0, 1, 2, or 3; and **A¹** and **A²** are independently selected from the group consisting of: N, CH, and C**R⁷**;
each **R⁷** is independently selected from the group consisting of **R^{a}** and **R^{b}**;
**X¹** is CH₂;
**X²** is CH₂;
**X³** is CH**R^{L}**;
**b4** is 0;
each **R¹⁰** is independently selected from the group consisting of: -Cl, -F, -CN, and C₁₋₃ alkyl optionally substituted with 1-3 **R^{c}**;
**R^{L}** is selected from the group consisting of C₁₋₃ alkoxy, -F, CN, and C₁₋₃ alkyl optionally substituted with 1-3 **R^{c}**;
**Y²** is -CH₂-; and
**R³** is 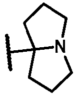 optionally substituted with 1-2 substituents each independently selected from the group consisting of: -F, -C₁₋₃ alkoxy, and -C₁₋₃ haloalkoxy;
each **R^{a}** is independently selected from the group consisting of:
   **(a)** halo;
   **(b)** cyano;
   **(c)** -OH;
   **(d)** oxo;
   **(e)** -C₁₋₆ alkoxy;
   **(f)** -C₁₋₆ haloalkoxy;
   **(g)** -N**R^{d}R^{e}**;
   **(h)** C(=O)C₁₋₆ alkyl;
   **(i)** C(=O)C₁₋₆ haloalkyl;
   **(j)** C(=O)OH;
   **(k)** C(=O)OC₁₋₆ alkyl;
   **(l)** C(=O)OC₁₋₆ haloalkyl;
   **(m)** C(=O)N(**R^{f}**)₂;
   **(n)** S(O)₀₋₂(C₁₋₆ alkyl);
   **(o)** S(O)₀₋₂(C₁₋₆ haloalkyl);
   **(p)** S(O)₁₋₂N(**R^{f}**)₂; and
   **(q)** C₁₋₆ alkyl, C₂₋₆ alkenyl, or C₂₋₆ alkynyl, each optionally substituted with 1-6 **R^{c}**;
each **R^{b}** is independently selected from the group consisting of: -(**L^{b})_{b}-R^{b1}** and **-R^{b1}**, wherein:
   **b is** 1, 2, or 3;
   each **-L^{b}** is independently selected from the group consisting of: -O-, -N(H)-, -N(C₁₋₃ alkyl)-, -S(O)₀₋₂-, C(=O), and C₁₋₃ alkylene; and
   each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
   each **R^{c}** is independently selected from the group consisting of: halo, cyano, -OH, -C₁₋₆ alkoxy, -C₁₋₆ haloalkoxy, -N**R^{d}R^{e}**, C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)₁₋₂N(**R^{f}**)₂;
   each **R^{d}** and **R^{e}** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
   each **R^{f}** is independently selected from the group consisting of: H and C₁₋₆ alkyl optionally substituted with 1-3 **R^{h}**;
   each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
   each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂-.

**Embodiment 5.** The compound of Embodiment 4, wherein **R^{L}** is independently selected from the group consisting of: CH₃, CF₃, CHF₂, and CH₂F (e.g., each **R^{L}** is CH₃).

**Embodiment 6.** The compound of Embodiment 4, wherein **R^{L}** is CH₃.

**Embodiment 7.** The compound of Embodiment 4, wherein the compound is a compound of Formula **(IV-c):** 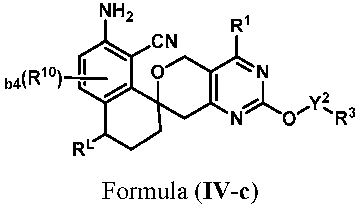 or a pharmaceutically acceptable salt thereof.

**Embodiment 8.** The compound of Embodiment 7, wherein **R^{L}** is CH₃.

**Embodiment 9.** The compound of any one of Embodiments 1-8, wherein **R³** is 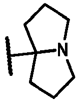 optionally substituted with 1-2 -F.

**Embodiment 10.** The compound of any one of Embodiments 1-9, wherein **R³** is 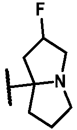 (e.g., 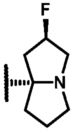 ).

**Embodiment 11.** The compound of any one of Embodiments 1-9, wherein **R³** is

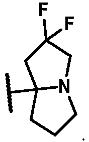

**Embodiment 12.** The compound of any one of Embodiments 1-8, wherein **R³** is

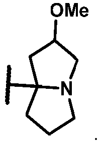

**Embodiment 13.** The compound of any one of Embodiments 1-12, wherein **R¹** is 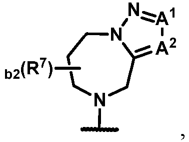 wherein **b2** is 0, 1, or 2, and **A¹** and **A²** are independently selected from the group consisting of: N, CH, and C**R⁷**.

**Embodiment 14.** The compound of any one of Embodiments 1-13, wherein **R¹** is

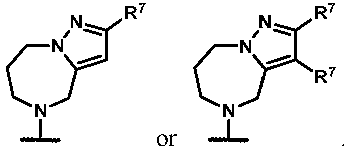

**Embodiment 15.** The compound of any one of Embodiments 1-14, wherein **R¹** is 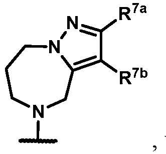 wherein **R^{7a}** and **R^{7b}** are independently selected **R⁷**.

**Embodiment 16.** The compound of Embodiment 15, wherein **R^{7a}** is selected from the group consisting of: C(=O)N(**R^{f}**)₂, C(=O)N(C₁₋₃ alkyl)**R^{b1}**, C(=O)N(H)**R^{b1}**, **R^{b1}**, and C(=O)**R^{b1}**; and
**R^{7b}** is -halo, -CN, and C₁₋₃ alkyl optionally substituted with 1-3 **R^{c}.**

**Embodiment 17.** The compound of Embodiment 15 or 16, wherein **R^{7a}** is selected from the group consisting of:
**(a)** C(=O)N(**R^{f}**)₂, wherein each **R^{f}** is independently H or C₁₋₃ alkyl optionally substituted with 1-3 **R^{h}**;
**(b)** C(=O)N(C₁₋₃ alkyl)**R^{b1}** or C(=O)N(H)**R^{b1}**, wherein **R^{b1}** is C₃₋₆ cycloalkyl or 4-6 membered heterocyclyl, each of which is optionally substituted with 1-3 **R^{g}**; and
**(c)** C(=O)**R^{b1}**, wherein **R^{b1}** is a 4-10 membered heterocyclyl optionally substituted with 1-3 **R^{g}**, wherein **R^{b1}** is attached to the C(=O) via a ring nitrogen atom.

**Embodiment 18.** The compound of any one of Embodiments 15-17, wherein **R^{7a}** is C(=O)N(**R^{f}**)₂, wherein each **R^{f}** is independently H or C₁₋₃ alkyl optionally substituted with 1-3 **R^{h}** (e.g., **R^{7a}** is C(=O)N(Me)₂); and **R^{7b}** is -Cl, -F, or methyl.

**Embodiment 19.** The compound of Embodiment 15 or 16, wherein **R^{7a}** is **R^{b1}**, wherein the **R^{b1}** is a 5-6 membered heteroaryl optionally substituted with 1-2 **R^{g}**, or wherein the **R^{b1}** is oxetanyl optionally substituted with **R^{g}**.

**Embodiment 20.** The compound of Embodiment 1, wherein the compound of Formula **(II-a)** is a compound of Formula **(II-a3):** 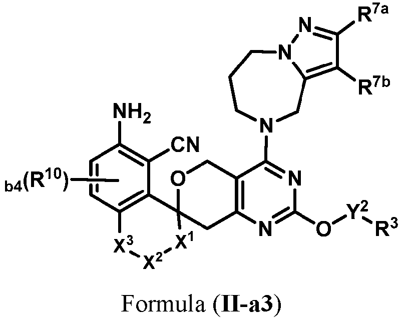 or a pharmaceutically acceptable salt thereof, wherein:
**b4** is 0;
each **R¹⁰** is independently selected from the group consisting of: -Cl, -F, -CN, and C₁₋₃ alkyl optionally substituted with 1-3 **R^{c}**;
**R^{7a}** is selected from the group consisting of:
   **(a)** C(=O)N(**R^{f}**)₂, wherein each **R^{f}** is independently H or C₁₋₃ alkyl optionally substituted with 1-3 **R^{h}**;
   **(b)** C(=O)N(C₁₋₃ alkyl)**R^{b1}** or C(=O)N(H)**R^{b1}**, wherein: **R^{b1}** is C₃₋₆ cycloalkyl or 4-6 membered heterocyclyl, each of which is optionally substituted with 1-3 **R^{g}**; and
   **(c)** C(=O)**R^{b1}**, wherein **R^{b1}** is 4-10 membered heterocyclyl optionally substituted with 1-3 **R^{g}**, wherein **R^{b1}** is attached to the C(=O) via a ring nitrogen atom; and
**R^{7b}** is -halo or C₁₋₃ alkyl.

**Embodiment 21.** The compound of Embodiment 20, wherein **R^{L}** is CF₃ or CH₃ (e.g., CH₃).

**Embodiment 22.** The compound of Embodiment 20 or 21, wherein **R^{7a}** is C(=O)N(**R^{f}**)₂, wherein each **R^{f}** is independently C₁₋₃ alkyl optionally substituted with 1-3 **R^{h}** (e.g., **R^{7a}** is C(=O)N(Me)₂).

**Embodiment 23.** The compound of any one of Embodiments 20-22, wherein **R^{7b}** is -Cl, -F, or methyl.

**Embodiment 24.** The compound of any one of Embodiments 20-23, wherein **R^{7b}** is -Cl.

**Embodiment 25.** The compound of any one of Embodiments 20-24, wherein the 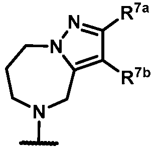 moiety is

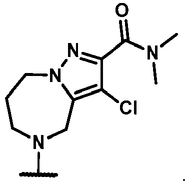

**Embodiment 26.** The compound of any one of Embodiments 20-25, wherein **R³** is

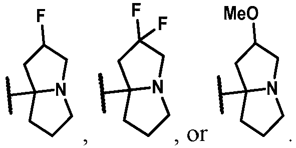

**Embodiment 27.** The compound of any one of Embodiments 20-26, wherein **R³** is 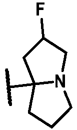 (e.g., 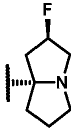 ).

**Embodiment 28.** The compound of any one of Embodiments 1-27, wherein the 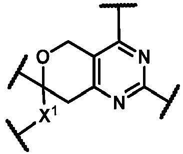 moiety is

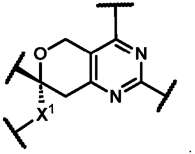

**Embodiment 29.** The compound of Embodiment 1, wherein the compound of Formula **(II-a)** is selected from the group consisting of Compound Nos. **509, 509a, 509b, 530, 530a, 531, 531a, 546, 546a, 584, 584a, 590, 590a, 614, 614a, 614b, 614c, 628, 628a, 628b, 628c, 629, 629a, 630, 630a, 632,** and **632a,** as depicted in **Table C3,** or a pharmaceutically acceptable salt thereof.

**Embodiment 30.** The compound of Embodiment 4, wherein the compound of Formula **(IV-b)** is selected from the group consisting of Compound Nos. **509, 509a, 509b, 530, 530a, 531, 531a, 546, 546a, 584, 584a, 590, 590a, 604, 604a, 604b, 604c, 614, 614a, 614b, 614c, 628, 628a, 628b, 628c, 629, 629a, 630, 630a, 632,** and **632a,** as depicted in **Table C3,** or a pharmaceutically acceptable salt thereof.

**Embodiment 31.** The compound of Embodiment 1, wherein the compound of Formula **(II-a)** is other than Compound Nos. **R176, R176a, R176b, R176c, R176d,** and **R176e,** as depicted in **Table C1,** or a pharmaceutically acceptable salt thereof.

**Embodiment 32.** The compound of Embodiment 4, wherein the compound of Formula **(IV)** is other than Compound Nos. **R176, R176a, R176b, R176c, R176d,** and **R176e,** as depicted in **Table C1,** or a pharmaceutically acceptable salt thereof.

**Embodiment 33.** A pharmaceutical composition comprising a compound of any one of Embodiments 1-32, or a pharmaceutically acceptable salt thereof, and a pharmaceutically acceptable excipient.

**Embodiment 34.** A method for treating cancer in a subject, the method comprising administering to a subject identified or diagnosed as having a cancer having a KRas dysregulation a therapeutically effective amount of a compound of any one of Embodiments 1-32, or a pharmaceutically acceptable salt thereof, or a pharmaceutical composition according to Embodiment 33.

**Embodiment 35.** A method for treating cancer in a subject, the method comprising (a) determining that the cancer in the subject has a KRas dysregulation; and (b) administering to the subject a therapeutically effective amount of a compound of any one of Embodiments 1-32, or a pharmaceutically acceptable salt thereof, or a pharmaceutical composition according to Embodiment 33.

**Embodiment 36.** The method of Embodiment 34 or 35, wherein the KRas dysregulation cancer is a KRas mutation.

**Embodiment 37.** The method of Embodiment 36, wherein the KRas mutation is a KRas G12A mutation, a KRas G12C mutation, a KRas G12D mutation, a KRas G12R mutation, a KRas G12S mutation, or a KRas G12V mutation.

**Embodiment 38.** The method of Embodiment 37, wherein the KRas mutation is a KRas G12C mutation, a KRas G12D mutation or a KRas G12V mutation.

**Embodiment 39.** The method of Embodiment 35, wherein the step of determining that the cancer in the subject has a KRas dysregulation includes performing an assay to detect the KRas dysregulation (e.g., a KRas mutation) in a tumor sample from the subject.

**Embodiment 40.** The method of Embodiment 39, wherein detecting the KRas dysregulation includes detecting a *KRAS* gene having a mutation corresponding to a substitution of glycine 12 in a KRas protein and/or a KRas protein having a substitution of glycine 12.

**Embodiment 41.** The method of Embodiment 40, wherein the substitution of glycine 12 is a substitution to alanine, cysteine, aspartic acid, arginine, serine, or valine.

**Embodiment 42.** The method of any one of Embodiments 34-41, wherein the cancer is selected from the group consisting of: a hematological cancer, a soft tissue cancer, bile duct cancer, bladder cancer, brain cancer, breast cancer, cervical cancer, colon cancer, endometrial cancer, esophageal cancer, kidney cancer, liver cancer, lung cancer, mucinous carcinoma, ovarian cancer, pancreatic cancer, prostate cancer, rectal cancer, skin cancer, stomach cancer, testicular cancer, thymus cancer, thyroid cancer, urothelial cancer, uterine cancer, and a combination thereof.

**Embodiment 43.** The method of Embodiment 42, wherein the cancer is selected from the group consisting of: colon cancer, endometrial cancer, lung cancer, pancreatic cancer, and uterine cancer.

**Embodiment 44.** The method of any one of Embodiments 34-43, comprising administering an additional therapy or therapeutic agent to the subject.

**Embodiment 45.** The method of Embodiment 44, wherein the additional therapy or therapeutic agent is selected from the group consisting of Ras pathway targeted therapeutic agents, kinase-targeted therapeutics, mTORC1 inhibitors or degraders, YAP inhibitors or degraders, proteasome inhibitors or degraders, HSP90 inhibitors or degraders, farnesyl transferase inhibitors or degraders, PTEN inhibitors or degraders, signal transduction pathway inhibitors or degraders, checkpoint inhibitors, modulators of the apoptosis pathway, chemotherapeutics, angiogenesis-targeted therapies, immune-targeted agents, radiotherapy, and combinations thereof.

### ADDITIONAL EMBODIMENTS

Although the present invention is defined in the claims, it should be understood that the present invention can also (alternatively) be defined in accordance with the following embodiments:
**1.** A compound of Formula **(IV-b):** 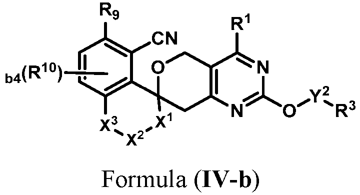 or a pharmaceutically acceptable salt thereof, wherein:
   **R¹** is a 4-10 membered heterocyclyl optionally substituted with 1-4 **R⁷**;
   each **R⁷** is independently selected from the group consisting of **R^{a}** and **R^{b}**;
   **X¹** is CH₂;
   **X²** is CH₂;
   **X³** is CH**R^{L}**
   **R⁹** is selected from the group consisting of: H, OH, N**R^{d}R^{e}**, and halo;
   **b4** is 0; and
   **R¹⁰** is selected from the group consisting of: -Cl, -F, -CN, and C₁₋₃ alkyl optionally substituted with 1-3 **R^{c}**;
   **R^{L}** is selected from the group consisting of C₁₋₃ alkoxy, -F, CN, and C₁₋₃ alkyl optionally substituted with 1-3 **R^{c}**;
   **Y²** is -CH₂-; and
   **R³** is 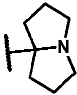 optionally substituted with 1-2 substituents each independently selected from the group consisting of: -F, -C₁₋₃ alkoxy, and -C₁₋₃ haloalkoxy;
   each **R^{a}** is independently selected from the group consisting of:
      **(a)** halo;
      **(b)** cyano;
      **(c)** -OH;
      **(d)** oxo;
      **(e)** -C₁₋₆ alkoxy;
      **(f)** -C₁₋₆ haloalkoxy;
      **(g)** -N**R^{d}R^{e}**;
      **(h)** C(=O)C₁₋₆ alkyl;
      **(i)** C(=O)C₁₋₆ haloalkyl;
      **(j)** C(=O)OH;
      **(k)** C(=O)OC₁₋₆ alkyl;
      **(l)** C(=O)OC₁₋₆ haloalkyl;
      **(m)** C(=O)N(**R^{f}**)₂;
      **(n)** S(O)₀₋₂(C₁₋₆ alkyl);
      **(o)** S(O)₀₋₂(C₁₋₆ haloalkyl);
      **(p)** S(O)₁₋₂N(**R^{f}**)₂; and
      **(q)** C₁₋₆ alkyl, C₂₋₆ alkenyl, or C₂₋₆ alkynyl, each optionally substituted with 1-6 **R^{c}**;
   each **R^{b}** is independently selected from the group consisting of: **-(L^{b})_{b}-R^{b1}** and -**R^{b1}**, wherein:
      **b is** 1, 2, or 3;
      each **-L^{b}** is independently selected from the group consisting of: -O-, -N(H)-, -N(C₁₋₃ alkyl)-, -S(O)₀₋₂-, C(=O), and C₁₋₃ alkylene; and
      each **R^{b1}** is independently selected from the group consisting of: C₃₋₁₀ cycloalkyl, 4-10 membered heterocyclyl, C₆₋₁₀ aryl, and 5-10 membered heteroaryl, each of which is optionally substituted with 1-3 **R^{g}**;
      each **R^{c}** is independently selected from the group consisting of: halo, cyano, -OH, -C₁₋₆ alkoxy, -C₁₋₆ haloalkoxy, -N**R^{d}R^{e}**, C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)₁₋₂N(**R^{f}**)₂;
      each **R^{d}** and **R^{e}** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
      each **R^{f}** is independently selected from the group consisting of: H and C₁₋₆ alkyl optionally substituted with 1-3 **R^{h}**;
      each **R^{g}** is independently selected from the group consisting of: **R^{h}**, C₁₋₃ alkyl, C₁₋₃ haloalkyl, C₃₋₅ cycloalkyl, and 4-5 membered heterocyclyl; and
      each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂-.
**2.** The compound of embodiment 1, wherein **R⁹** is N**R^{d}R^{e}** (e.g., -NH₂).
**3.** The compound of embodiment 1 or 2, wherein **R^{L}** is independently selected from the group consisting of: CH₃, CF₃, CHF₂, and CH₂F
**4.** The compound of any one of embodiments 1-3, wherein **R^{L}** is CH₃.
**5.** The compound of any one of embodiments 1-4, wherein the compound is a compound of Formula **(IV-c):** 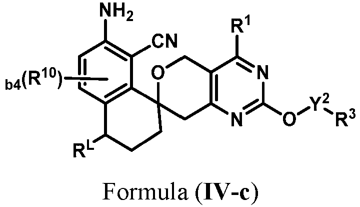 or a pharmaceutically acceptable salt thereof
**6.** The compound of embodiment 5, wherein **R^{L}** is CH₃.
**7.** The compound of any one of embodiments 1-6, wherein **R³** is 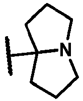 optionally substituted with 1-2 -F.
**8.** The compound of any one of embodiments 1-7, wherein **R³** is 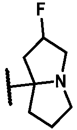 (e.g., 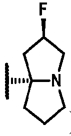 ).
**9.** The compound of any one of embodiments 1-7, wherein **R³** is 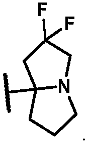
**10.** The compound of any one of embodiments 1-6, wherein **R³** is 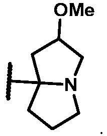
**11.** The compound of any one of embodiments 1-10, wherein each **R⁷** is independently selected from the group consisting of: halo; cyano; -OH; oxo; -C₁₋₆ alkoxy; C(=O)N(**R^{f}**)₂; **R^{b1}**; -(C₁₋₃ alkylene)-**R^{b1}**; -O-**R^{b1}**; and C₁₋₆ alkyl optionally substituted with 1-3 **R^{c7}**, wherein:
   each **R^{b1}** is independently selected from the group consisting of: C₃₋₆ cycloalkyl, phenyl, 4-6 membered heterocyclyl, and 5-6 membered heteroaryl, each of which is optionally substituted with 1-3 **R^{g}** (e.g., each **R^{g}** is independently selected from the group consisting of: halo and C₁₋₃ alkyl); and
   each **R^{c7}** is independently selected from the group consisting of: halo, cyano, -OH, and -C₁₋₆ alkoxy.
**12.** The compound of any one of embodiments 1-11, wherein **R¹** is a 7-10 (e.g., 7) membered heterocyclyl having one ring nitrogen atom, one ring oxygen atom, and no additional ring heteroatoms, wherein the 7-10 membered heterocyclyl is optionally substituted with 1-4 (e.g., 1-2) **R⁷.**
**13.** The compound of any one of embodiments 1-12, wherein **R¹** is a 7-10 (e.g., 7) membered monocyclic heterocyclyl having one ring nitrogen atom, one ring oxygen atom, and no additional ring heteroatoms, wherein the 7-10 membered monocyclic heterocyclyl is optionally substituted with 1-4 (e.g., 1-2) **R⁷.**
**14.** The compound of any one of embodiments 1-13, wherein **R¹** is 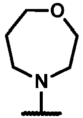 optionally substituted with 1-4 **R⁷** at one or more ring carbon atoms.
**15.** The compound of any one of embodiments 1-14, wherein **R¹** is 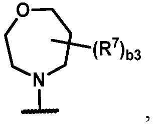 wherein **b3** is 1, 2, or 3.
**16.** The compound of embodiment 15, wherein one occurrence of **R⁷** is **R^{b}** (e.g., one occurrence of **R⁷** is **R^{b1}**).
**17.** The compound of embodiment 15 or 16, wherein one occurrence of **R⁷** is a 5-6 membered heteroaryl optionally substituted with 1-3 **R^{g}**.
**18.** The compound of any one of embodiments 1-17, wherein one occurrence of**R⁷** is a 5-membered heteroaryl optionally substituted with 1-3 **R^{g}.**
**19.** The compound of any one of embodiments 1-18, wherein one occurrence of **R⁷** is selected from the group consisting of pyrazolyl and oxazolyl, each of which is optionally substituted with 1-2 **R^{g}** (e.g., **R⁷** is 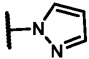 ).
**20.** The compound of any one of embodiments 1-19, wherein **b3** is 1.
**21.** The compound of any one of embodiments 1-15, wherein **R¹** is 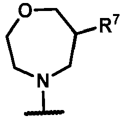 (e.g., 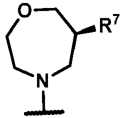 ), wherein **R⁷** is a 5-membered heteroaryl optionally substituted with 1-3 **R^{g}**.
**22.** The compound of embodiment 21, wherein **R⁷** is selected from the group consisting of pyrazolyl and oxazolyl, each of which is optionally substituted with 1-2 **R^{g}**.
**23.** The compound of embodiment 21 or 22, wherein **R⁷** is pyrazolyl optionally substituted with 1-2 **R^{g}** (e.g., **R⁷** is 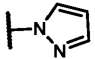 optionally substituted with one **R^{g}**); or wherein **R⁷** is oxazolyl optionally substituted with one **R^{g}** (e.g., **R⁷** is 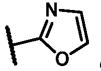 optionally substituted with one **R^{g}).**
**24.** The compound of any one of embodiments 21-23, wherein **R⁷** is 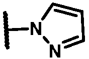 or 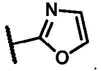
**25.** The compound of any one of embodiments 1-12, wherein **R¹** is a 7-10 (e.g., 7) membered bicyclic heterocyclyl having one ring nitrogen atom, one ring oxygen atom, and no additional ring heteroatoms, wherein the 7-10 membered bicyclic heterocyclyl is optionally substituted with 1-4 (e.g., 1-2) **R⁷.**
**26.** The compound of any one of embodiments 1-12 or 25, wherein **R¹** is a 7-10 (e.g., 7;e.g., 9) membered spirocyclic bicyclic heterocyclyl having one ring nitrogen atom, one ring oxygen atom, and no additional ring heteroatoms, wherein the 7-10 membered spirocyclic bicyclic heterocyclyl is optionally substituted with 1-4 (e.g., 1-2) **R⁷.**
**27.** The compound of any one of embodiments 1-12 or 25-26, wherein **R¹** is 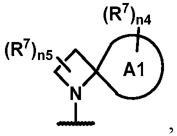 wherein: **Ring A1** is a 4-7 membered heterocyclyl ring having one ring oxygen atom and no additional ring heteroatoms; **n4** is 0, 1, or 2; and **n5** is 0, 1, or 2, provided that **n4** + **n5** is 0, 1, or 2.
**28.** The compound of any one of embodiments 1-12 or 25-27, wherein **R¹** is 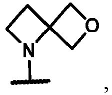 which is optionally substituted with 1-2 **R⁷.**
**29.** The compound of any one of embodiments 1-12 or 25-28, wherein **R¹** is 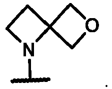
**30.** The compound of any one of embodiments 1-12 or 25-27, wherein **R¹** is 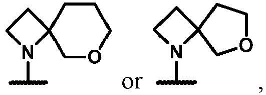 each of which is optionally substituted with 1-2 **R⁷.**
**31.** The compound of any one of embodiments 1-12, 25-27, or 30, wherein **R¹** is 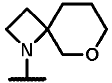 (e.g., 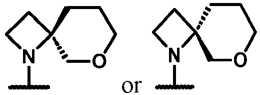 ) or 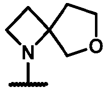 (e.g., 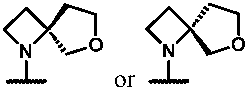 ).
**32.** The compound of any one of embodiments 1-30, wherein **R¹** is a 4-7 membered heterocyclyl optionally substituted with 1-4 (e.g., 1-2) **R⁷**.
**33.** The compound of any one of embodiments 1-32, wherein **R¹** is a 4-5 membered heterocyclyl (e.g., azetidinyl or pyrrolidinyl) optionally substituted with 1-4 (e.g., 1-2) **R⁷.**
**34.** The compound of any one of embodiments 1-10 or 32-33, wherein **R¹** is a 4-membered heterocyclyl optionally substituted with 1-4 (e.g., 1-2) **R⁷**.
**35.** The compound of any one of embodiments 1-10 or 32-34, wherein **R¹** is 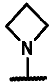 optionally substituted with 1-4 (e.g., 1-2) **R⁷.**
**36.** The compound of any one of embodiments 1-10 or 32-35, wherein **R¹** is selected from the group consisting of: 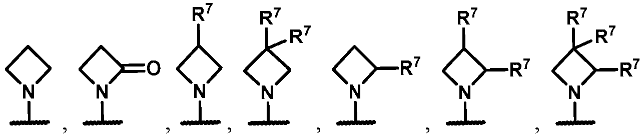 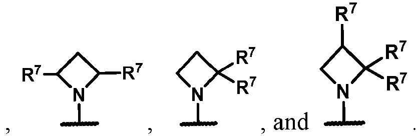
**37.** The compound of any one of embodiments 1-10 or 32-36, wherein **R¹** is 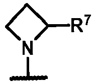 (e.g., 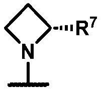 ), 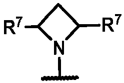 (e.g., 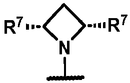 ), or 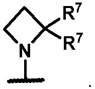
**38.** The compound of any one of embodiments 32-37, wherein each **R⁷** is independently selected from the group consisting of: halo; cyano; -OH; oxo; -C₁₋₆ alkoxy; C(=O)N(**R^{f}**)₂; **R^{b1}**; -(C₁₋₃ alkylene)-**R^{b1}**; -O-**R^{b1}**; and C₁₋₆ alkyl optionally substituted with 1-3 **R^{c7}**, wherein:
   each **R^{b1}** is independently selected from the group consisting of: C₃₋₆ cycloalkyl, phenyl, 4-6 membered heterocyclyl, and 5-6 membered heteroaryl, each of which is optionally substituted with 1-3 **R^{g}** (e.g., each **R^{g}** is independently selected from the group consisting of: halo and C₁₋₃ alkyl); and
   each **R^{c7}** is independently selected from the group consisting of: halo, cyano, -OH, and -C₁₋₆ alkoxy.
**39.** The compound of any one of embodiments 32-38, wherein each **R⁷** is independently selected from the group consisting of:
   -F;
   -cyano;
   -OH;
   -**R^{b1}**, wherein the **R^{b1}** is a 5-6 membered heteroaryl optionally substituted with 1-2 **R^{g}**,
   C₁₋₃ alkyl optionally substituted with 1-3 F;
   C₁₋₃ alkyl substituted with -OH or C₁₋₃ alkoxy; and
   C(=O)N(**R^{f}**)₂.
**40.** The compound of embodiment 38 or 39, wherein at least one **R⁷** is C₁₋₃ alkyl substituted with -OH.
**41.** The compound of any one of embodiments 1-10, wherein **R¹** is 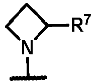 (e.g., 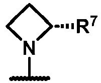 ), wherein **R⁷** is C₁₋₃ alkyl substituted with -OH or C₁₋₃ alkoxy (e.g., **R⁷** is C₁₋₃ alkyl substituted with -OH).
**42.** The compound of any one of embodiments 1-10 or 41, wherein **R¹** is 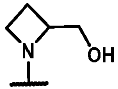 (e.g., 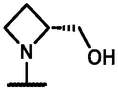 ).
**43.** The compound of any one of embodiments 1-10, wherein **R¹** is 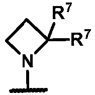 or 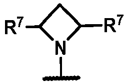 (e.g., 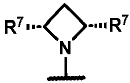 ) , wherein each **R⁷** is independently selected from the group consisting of:
   C₁₋₃ alkyl optionally substituted with 1-3 F; and
   C₁₋₃ alkyl substituted with -OH or C₁₋₃ alkoxy.
**44.** The compound of any one of embodiments 1-10 or 43, wherein **R¹** is 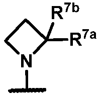 (e.g., 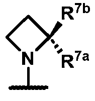 ) or 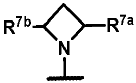 (e.g., 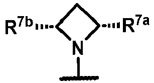 ), wherein **R^{7a}** is C₁₋₃ alkyl substituted with -OH (e.g., -CH₂OH); and
   **R^{7b}** is selected from the group consisting of:
   C₁₋₃ alkyl optionally substituted with 1-3 F (e.g., methyl), and
   C₁₋₃ alkyl substituted with -OH or C₁₋₃ alkoxy.
**45.** The compound of any one of embodiments 1-10 or 43-44, wherein **R¹** is 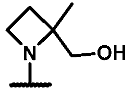 (e.g., 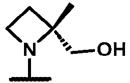 ).
**46.** The compound of any one of embodiments 1-10 or 43-44, wherein **R¹** is 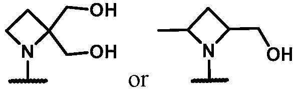 (e.g., 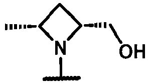 ).
**47.** The compound of embodiment 1, wherein the compound is a compound of Formula **(IV-b4), (IV-b5),** or **(IV-b8):** 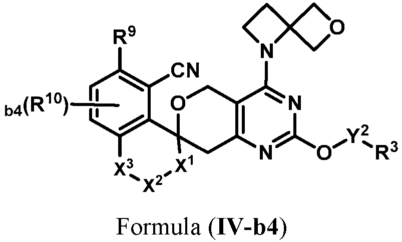 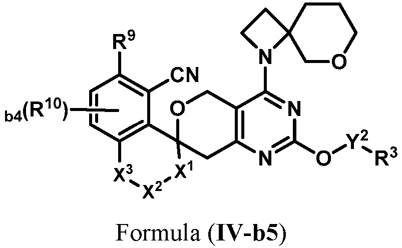 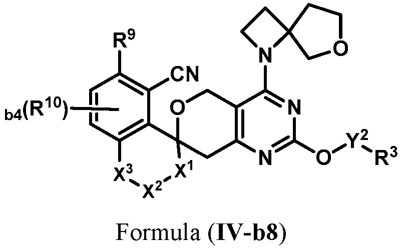 or a pharmaceutically acceptable salt thereof, wherein:
   **b4** is 0;
   each **R¹⁰** is independently selected from the group consisting of: -Cl, -F, -CN, and C₁₋₃ alkyl optionally substituted with 1-3 **R^{c}**;
   **X¹** is CH₂;
   **X²** is CH₂; and
   **X³** is CH**R^{L}**.
**48.** The compound of embodiment 1, wherein the compound of Formula **(IV)** is a compound of Formula **(IV-b6)** or **(IV-b7):** 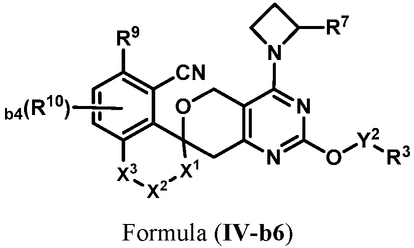 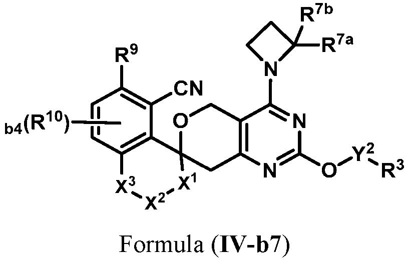 or a pharmaceutically acceptable salt thereof, wherein:
   **R⁷** is C₁₋₃ alkyl substituted with -OH or C₁₋₃ alkoxy;
   **R^{7a}** is C₁₋₃ alkyl substituted with -OH (e.g., -CH₂OH);
   **R^{7b}** is selected from the group consisting of:
      C₁₋₃ alkyl optionally substituted with 1-3 F (e.g., methyl), and
      C₁₋₃ alkyl substituted with -OH or C₁₋₃ alkoxy;
      **b4** is 0;
      each **R¹⁰** is independently selected from the group consisting of: -Cl, -F, -CN, and C₁₋₃ alkyl optionally substituted with 1-3 **R^{c}**;
      **X¹** is CH₂;
      **X²** is CH₂; and
      **X³** is CH**R^{L}**.
**49.** The compound of embodiment 47 or 48, wherein **R⁹** is N**R^{d}R^{e}** (e.g., -NH₂).
**50.** The compound of embodiment 48 or 49, wherein the 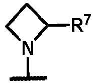 moiety is 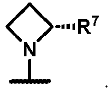
**51.** The compound of embodiment 48-50, wherein **R⁷** is C₁₋₃ alkyl substituted with -OH.
**52.** The compound of any one of embodiments 48-51, wherein **R⁷** is -CH₂OH.
**53.** The compound of embodiment 48 or 49, wherein the 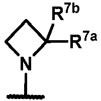 moiety is 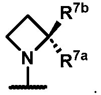
**54.** The compound of any one of embodiments 48-49 or 53, wherein **R^{7a}** is -CH₂OH.
**55.** The compound of any one of embodiments 48-49 or 53-54, wherein **R^{7b}** is C₁₋₃ alkyl optionally substituted with 1-3 -F.
**56.** The compound of embodiment 55, wherein **R^{7b}** is methyl.
**57.** The compound of any one of embodiments 47-56, wherein **X³** is CH(CH₃).
**58.** The compound of any one of embodiments 47-57, wherein **R³** is 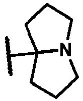 optionally substituted with 1-2 -F (e.g., **R³** is 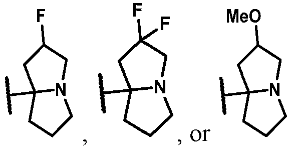 ).
**59.** The compound of any one of embodiments 47-57, wherein **R³** is 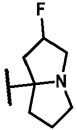 (e.g., 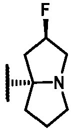 ).
**60.** The compound of any one of embodiments 1-59, wherein the 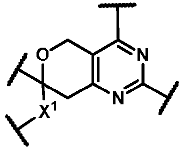 moiety is 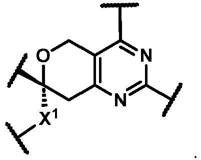
**61.** The compound of embodiment 1, wherein the compound of Formula **(IV-b)** is selected from the group consisting of Compound Nos. **502, 502a, 502b, 511, 511a, 512, 512a, 512b, 532, 532a, 553, 553a, 554, 554a, 554b, 554c, 555, 555a, 556, 556a, 556b, 560, 560a, 561, 561a, 566, 566a, 566b, 567, 567a, 567b, 568, 568a, 568b, 569, 569a, 570, 570a, 571, 571a, 572, 572a, 573, 573a, 575, 575a, 575b, 576, 576a, 576b, 576c, 576d, 577, 577a, 578, 578a, 578b, 581, 581a, 582, 582a, 583, 583a, 585, 585a, 585b, 589, 589a, 592, 592a, 595, 595a, 595b, 597, 597a, 597b, 598, 598a, 598b, 598c, 599, 599a, 599b, 600, 600a, 600b, 601, 601a, 605, 605a, 605b, 605c, 606, 606a, 606b, 607, 607a, 608, 608a, 608b, 609, 609a, 609b, 610, 610a, 610b, 611, 611a, 611b, 612, 612a, 612b, 613, 613a, 613b, 613c, 618, 618a, 619, 619a, 620, 620a, 621, 621a, 621b, 622, 622a, 623, 623a, 624, 624a, 624b, 625, 625a, 626, 626a, 627, 627a, 633, 633a, 634, 634a, 635, 635a, 636, 636a, 637, 637a, 637b, 638, 638a, 639, 639a, 640, 640a, 640b, 642, 642a, 643, 643a, 643b, 644, 644a, 645, 645a, 645b, 646, 646a, 647, 647a, 647b, 648, 648a, 648b, 649, 649a, 649b, 650, 650a, 650b, 650c, 650d, 651, 651a, 651b, 652, 652a, 652b, 652c, 653, 653a, 653b, 654, 654a, 654b, 654c, 654d, 655, 655a, 656, 656a, 657, 657a, 658, 658a, 659, 659a, 660, 660a, 660b, 661, 661a, 661b, 662, 662a, 662b, 662c, 662d, 665, 665a, 665b, 666, 666a, 667, 667a, 667b, 668, 668a, 668b, 668c, 668d, 669, 669a, 670, 670a, 671, 671a, 671b, 672, 672a, 673, 673a, 674, 674a, 675, 675a, 676, 676a, 677, 677a, 678, 678a, 679, 679a, 680, 680a, 680b, 680c, 681, 681a, 682, 682a, 683, 683a, 684, 684a, 685, 685a, 686, 686a, 687, 687a, 688, 688a, 689, 689a, 689b, 690, 690a, 691, 691a, 692, 692a, 693, 693a, 694, 694a, 695, 695a, 696, 696a, 697, 697a, 698, 698a, 699, 699a, 700, 700a, 701, 701a, 702, 702a, 702b, 703, 703a, 704, 704a, 705, 705a, 707, 707a, 708, 708a, 709, 709a, 710, 710a, 711, 711a, 712, 712a, 713, 713a, 714, 714a, 715, 715a, 716, 716a, 717, 717a, 718, 718a, 719, 719a, 720, 720a, 721, 721a, 722, 722a, 723, 723a, 724, 724a, 725, 725a, 726, 726a, 727, 727a, 728, 728a, 729, 729a, 729b, 730, 730a, 731, 731a, 732, 732a, 733, 733a, 734, 734a, 735, 735a, 736, 736a, 737, 737a, 737b, 738, 738a, 739, 739a, 740, 740a, 741, 741a, 741b, 742, 742a, 743, 743a, 744, 744a, 745, 745a, 745b, 746, 746a, 747, 747a, 747b, 748, 748a, 749, 749a, 750, 750a, 752, 752a, 753, 753a, 754,** and **754a,** as depicted in **Table C3,** or a pharmaceutically acceptable salt thereof.
**62.** The compound of embodiment 1, wherein the compound of Formula **(IV-b)** is other than Compound Nos. **R179, R179a, R179b, R179d, R179e,** and **R179f,** as depicted in **Table C1,** or a pharmaceutically acceptable salt thereof.
**63.** A pharmaceutical composition comprising a compound of any one of embodiments 1-62, or a pharmaceutically acceptable salt thereof, and a pharmaceutically acceptable excipient.
**64.** A method for treating cancer in a subject, the method comprising administering to a subject identified or diagnosed as having a cancer having a KRas dysregulation a therapeutically effective amount of a compound of any one of embodiments 1-62, or a pharmaceutically acceptable salt thereof, or a pharmaceutical composition according to embodiment 63.
**65.** A method for treating cancer in a subject, the method comprising (a) determining that the cancer in the subject has a KRas dysregulation; and (b) administering to the subject a therapeutically effective amount of a compound of any one of embodiments 1-62, or a pharmaceutically acceptable salt thereof, or a pharmaceutical composition according to embodiment 63.
**66.** The method of embodiment 64 or 65, wherein the KRas dysregulation is a KRas mutation.
**67.** The method of embodiment 66, wherein the KRas mutation is a KRas G12A mutation, a KRas G12C mutation, a KRas G12D mutation, a KRas G12R mutation, a KRas G12S mutation, or a KRas G12V mutation.
**68.** The method of embodiment 67, wherein the KRas mutation is a KRas G12C mutation, a KRas G12D mutation or a KRas G12V mutation.
**69.** The method of embodiment 65, wherein the step of determining that the cancer in the subject has a KRas dysregulation includes performing an assay to detect the KRas dysregulation (e.g., a KRas mutation) in a tumor sample from the subject.
**70.** The method of embodiment 69, wherein detecting the KRas dysregulation includes detecting a *KRAS* gene having a mutation corresponding to a substitution of glycine 12 in a KRas protein and/or a KRas protein having a substitution of glycine 12.
**71.** The method of embodiment 70, wherein the substitution of glycine 12 is a substitution to alanine, cysteine, aspartic acid, arginine, serine, or valine.
**72.** The method of any one of embodiments 64-71, wherein the cancer is selected from the group consisting of: a hematological cancer, a soft tissue cancer, bile duct cancer, bladder cancer, brain cancer, breast cancer, cervical cancer, colorectal cancer, endometrial cancer, esophageal cancer, kidney cancer, liver cancer, lung cancer, mucinous carcinoma, ovarian cancer, pancreatic cancer, prostate cancer, skin cancer, stomach cancer, testicular cancer, thymus cancer, thyroid cancer, urothelial cancer, uterine cancer, and a combination thereof.
**73.** The method of embodiment 72, wherein the cancer is selected from the group consisting of: colon cancer, endometrial cancer, lung cancer, pancreatic cancer, and uterine cancer.
**74.** The method of embodiment 72, wherein the cancer is selected from the group consisting of: colorectal cancer, endometrial cancer, lung cancer (e.g., NSCLC), ovarian cancer, and pancreatic cancer.
**75.** The method of any one of embodiments 64-74, comprising administering an additional therapy or therapeutic agent to the subject.
**76.** The method of embodiment 75, wherein the additional therapy or therapeutic agent is selected from the group consisting of Ras pathway targeted therapeutic agents, kinase-targeted therapeutics, mTORC1 inhibitors or degraders, YAP inhibitors or degraders, proteasome inhibitors or degraders, HSP90 inhibitors or degraders, farnesyl transferase inhibitors or degraders, PTEN inhibitors or degraders, signal transduction pathway inhibitors or degraders, checkpoint inhibitors, modulators of the apoptosis pathway, chemotherapeutics, angiogenesis-targeted therapies, immune-targeted agents, radiotherapy, and combinations thereof.

## Claims

1. A compound of Formula **(II-a3):** 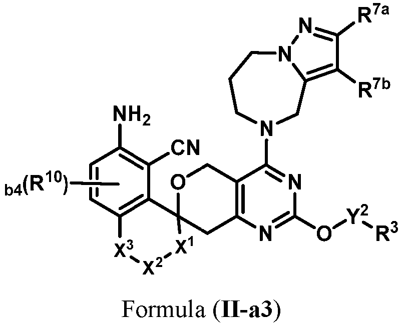 or a pharmaceutically acceptable salt thereof, wherein:
**b4** is 0;
each **R¹⁰** is independently selected from the group consisting of: -Cl, -F, -CN, and C₁₋₃ alkyl optionally substituted with 1-3 **R^{c}**;
**R^{7a}** is C(=O)N(Me)2;
**R^{7b}** is -halo or C₁₋₃ alkyl;
**X¹** is CH₂;
**X²** and **X³** are independently selected from the group consisting of: CH₂, CH**R^{L}**, C(**R^{L}**)₂, provided that 1-2 of **X²** and **X³** is independently CH**R^{L}** or C(**R^{L}**)₂;
**R^{L}** is selected from the group consisting of C₁₋₃ alkoxy, -F, CN, and C₁₋₃ alkyl optionally substituted with 1-3 **R^{c}**;
**Y²** is -CH₂-;
**R³** is 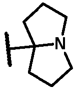 optionally substituted with 1-2 substituents each independently selected from the group consisting of: -F, -C₁₋₃ alkoxy, and -C₁₋₃ haloalkoxy;
each **R^{c}** is independently selected from the group consisting of: halo, cyano, -OH, -C₁₋₆ alkoxy, -C₁₋₆ haloalkoxy, -N**R^{d}R^{e}**, C(=O)C₁₋₆ alkyl, C(=O)C₁₋₆ haloalkyl, C(=O)OC₁₋₆ alkyl, C(=O)OC₁₋₆ haloalkyl, C(=O)OH, C(=O)N(**R^{f}**)₂, S(O)₀₋₂(C₁₋₆ alkyl), S(O)₀₋₂(C₁₋₆ haloalkyl), and S(O)₁₋₂N(**R^{f}**)₂;
each **R^{d}** and **R^{e}** is independently selected from the group consisting of: H, C(=O)C₁₋₆ alkyl, C(=O)C₁₋₆ haloalkyl, C(=O)OC₁₋₆ alkyl, C(=O)OC₁₋₆ haloalkyl, C(=O)N(**R^{f}**)₂, S(O)₁₋₂(C₁₋₆ alkyl), S(O)₁₋₂(C₁₋₆ haloalkyl), S(O)₁₋₂N(**R^{f}**)₂, and C₁₋₆ alkyl optionally substituted with 1-3 **R^{h}**;
each **R^{f}** is independently selected from the group consisting of: H and C₁₋₆ alkyl optionally substituted with 1-3 **R^{h}**; and
each **R^{h}** is independently selected from the group consisting of: halo, cyano, -OH, -C₁₋₆ alkoxy, -C₁₋₆ haloalkoxy, -NH₂, -N(H)(C₁₋₃ alkyl), and -N(C₁₋₃ alkyl)₂₋.

2. The compound of claim 1, wherein **R^{L}** is CF₃ or CH₃.

3. The compound of claim 1 or 2, wherein **R^{L}** is CH₃.

4. The compound of any one of claims 1-3, wherein **R^{7b}** is -Cl, -F, or methyl.

5. The compound of any one of claims 1-4, wherein **R^{7b}** is -Cl.

6. The compound of claim 1, wherein the 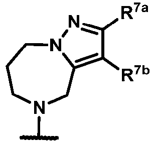 moiety is

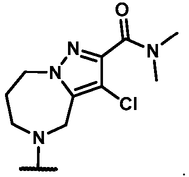

7. The compound of any one of claims 1-6, wherein **R³** is 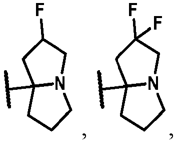 or

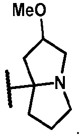

8. The compound of any one of claims 1-7, wherein **R³** is

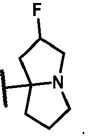

9. The compound of any one of claims 1-8, wherein **R³** is

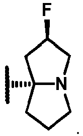

10. The compound of any one of claims 1-9, wherein the 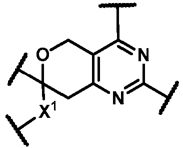 moiety is

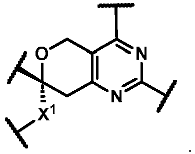

11. The compound of claim 1, wherein the compound of Formula **(II-a3)** is selected from the group consisting of:
| | |
|---|---|
| **509** | 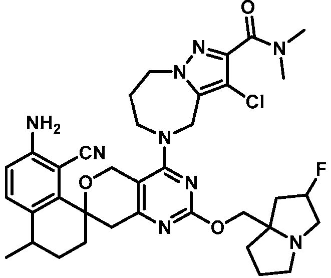 |
| **509a** | 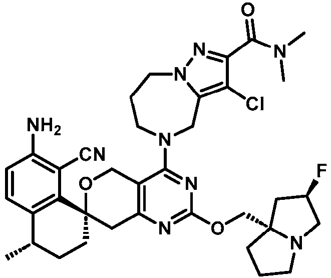 |
| **530** | 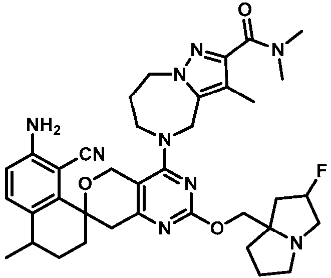 |
| **530a** | 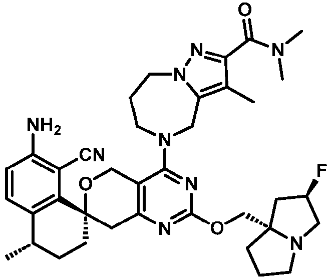 |
| **531** | 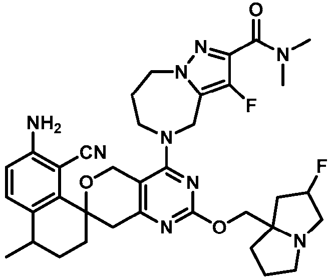 |
| **531a** | 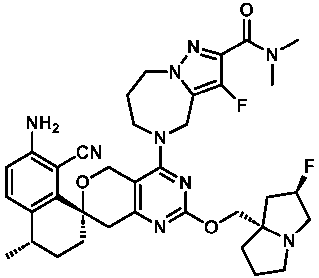 |
| **546** | 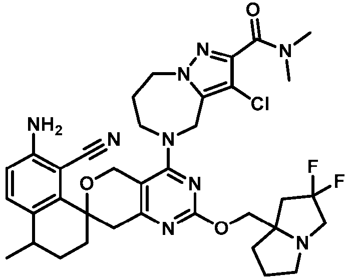 |
| **546a** | 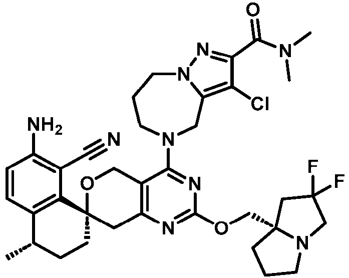 |
| **584** | 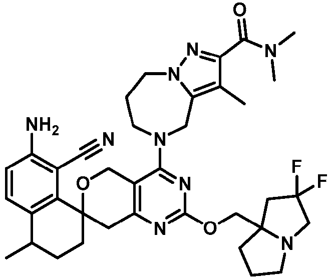 |
| **584a** | 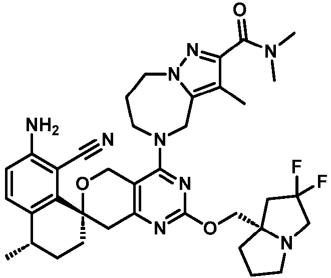 |
| **590** | 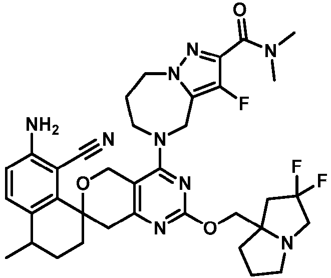 |
| **590a** | 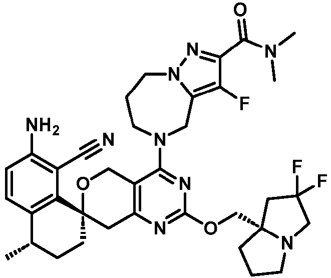 |
| **614** | 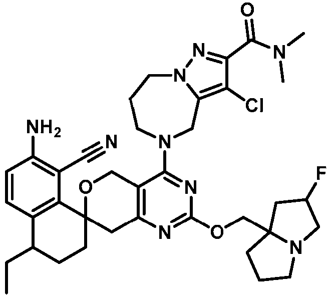 |
| **614a** | 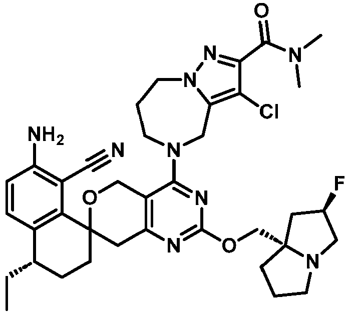 |
| **614b** | 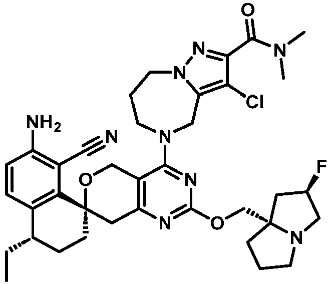 |
| **614c** | 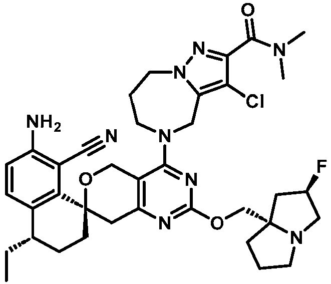 |
| **628** | 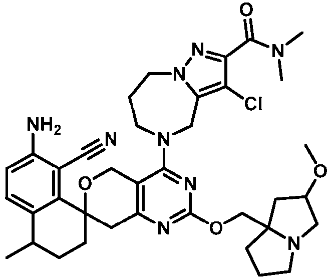 |
| **628a** | 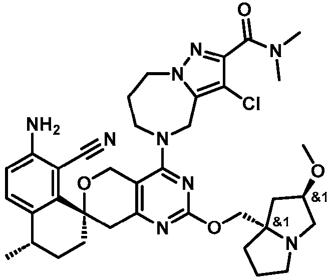 |
| **628b** | 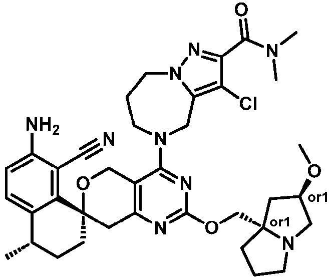 |
| **628c** | 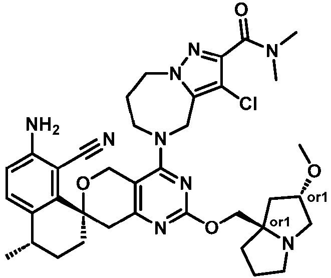 |
| **630** | 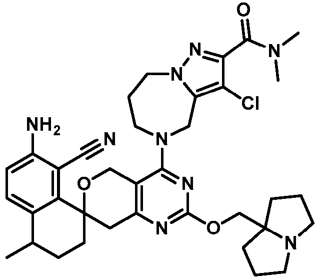 |
| **630a** | 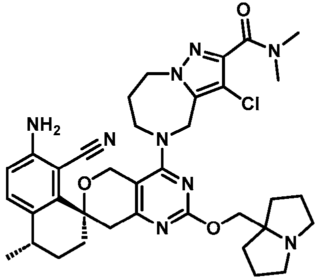 |
or a pharmaceutically acceptable salt thereof.

12. A pharmaceutical composition comprising a compound of any one of claims 1-11, or a pharmaceutically acceptable salt thereof, and a pharmaceutically acceptable excipient.

13. A compound of any one of claims 1-11, or a pharmaceutically acceptable salt thereof, or the pharmaceutical composition of claim 12, for use in treatment of a cancer having a KRas dysregulation.

14. The compound or pharmaceutical composition of claim 13, wherein the KRas dysregulation is a KRas mutation; optionally
wherein the KRas mutation is a KRas G12A mutation, a KRas G12C mutation, a KRas G12D mutation, a KRas G12R mutation, a KRas G12S mutation, or a KRas G12V mutation; or
the KRas mutation is a KRas G12C mutation, a KRas G12D mutation, or a KRas G12V mutation.

15. The compound or pharmaceutical composition of claim 13 or 14, wherein the cancer is selected from the group consisting of: a hematological cancer, a soft tissue cancer, bile duct cancer, bladder cancer, brain cancer, breast cancer, cervical cancer, colon cancer, endometrial cancer, esophageal cancer, kidney cancer, liver cancer, lung cancer, mucinous carcinoma, ovarian cancer, pancreatic cancer, prostate cancer, rectal cancer, skin cancer, stomach cancer, testicular cancer, thymus cancer, thyroid cancer, urothelial cancer, uterine cancer, and a combination thereof; or
the cancer is selected from the group consisting of: a colon cancer, endometrial cancer, lung cancer, pancreatic cancer, and uterine cancer; or
the cancer is a pancreatic cancer;
the cancer is a colorectal cancer; or
the cancer is a lung cancer; or
the cancer is an ovarian cancer; or
the cancer is selected from the group consisting of: a colorectal cancer, gastric cancer, gastroesophageal cancer, head and neck squamous carcinoma, or lung cancer.

16. The compound or pharmaceutical composition of any one of claims 13-15, wherein the treatment of cancer comprises administering an additional therapy or therapeutic agent; optionally wherein the additional therapy or therapeutic agent is selected from the group consisting of Ras pathway targeted therapeutic agents, kinase-targeted therapeutics, mTORC1 inhibitors or degraders, YAP inhibitors or degraders, proteasome inhibitors or degraders, HSP90 inhibitors or degraders, farnesyl transferase inhibitors or degraders, PTEN inhibitors or degraders, signal transduction pathway inhibitors or degraders, checkpoint inhibitors, modulators of the apoptosis pathway, chemotherapeutics, angiogenesis-targeted therapies, immune-targeted agents, radiotherapy, and combinations thereof; or the additional therapy or therapeutic agent is selected from the group consisting of kinase-targeted therapeutics, checkpoint inhibitors, chemotherapeutics, and immune-targeted agents.
